# EUROPEAN PATENT SPECIFICATION

(11) **EP 0 592 676 B1**
(45) Date of publication and mention of the grant of the patent: **30.09.1998**
(21) Application number: 93901023.7
(22) Date of filing: 25.06.1992
(51) Int. Cl.: C07D 239/26, C07D 239/28, C07D 251/14, C07D 251/26, C07D 403/12, C07D 413/12, C07D 417/12

(54) **IMINOSULFONYLUREA DERIVATIVE AND HERBICIDE**
IMINOSULFONYLHARNSTOFFDERIVATE UND HERBIZIDE
DERIVE D'IMINOSULFONYLUREE ET HERBICIDE

(30) Priority: 28.06.1991 JP 158106/91; 14.08.1991 JP 204294/91; 25.09.1991 JP 245876/91; 03.12.1991 JP 319422/91; 20.01.1992 JP 7397/92; 24.03.1992 JP 66277/92; 30.04.1992 JP 111494/92
(43) Date of publication of application: 20.04.1994
(73) Proprietor: NISSAN CHEMICAL INDUSTRIES, LIMITED, Chiyoda-ku Tokyo 101 (JP)
(72) Inventor: MAKINO, Kenzi, Nissan Chemical Industries Ltd., Funabashi-shi, Chiba 274 (JP); AKIYAMA, Shigeaki, Nissan Chemical Industries Ltd., Funabashi-shi, Chiba 274 (JP); SUZUKI, Hideaki, Nissan Chemical Industries Ltd., Funabashi-shi, Chiba 274 (JP); NAGAOKA, Takeshi, Nissan Chemical Industries Ltd., Funabashi-shi, Chiba 274 (JP); NIKI, Toshio, Nissan Chemical Industries Ltd., Funabashi-shi, Chiba 274 (JP); SUZUKI, K., Nissan Chem. Ind. Ltd, Seibutsukagaku, Minamisaitama-gun, Saitama 349-02 (JP); NAWAMAKI, T, Nissan Chem. Ind. Ltd, Seibutsukagaku, Minamisaitama-gun, Saitama 349-02 (JP); WATANABE, S, Nissan Chem. Ind. Ltd. Seibutsukagaku, Minamisaitama-gun, Saitama 349-02 (JP); ISHIKAWA, K, Nissan Chem. Ind. Ltd, Seibutsukagaku, Minamisaitama-gun, Saitama 349-02 (JP)
(74) Representative: Wächtershäuser, Günter, Prof. Dr.
(86) International application number: JP9200808
(87) International publication number: WO9300336

(56) References cited:
- JP-A-58 015 962
- JP-A-58 103 371
- CHEMICAL ABSTRACTS, vol. 104, no. 13, 31 March 1986, Columbus, Ohio, US; abstract no. 109685u, S. YAMAMOTO ET AL. 'Sulfonylurea derivatives and plant growth regulators or herbicides.' page 733 ;

## Description

### TECHNICAL FIELD

The present invention relates to novel iminosulfonylurea derivatives, and herbicides containing them as active ingredients.

### BACKGROUND TECHNIQUE

It is indispensable to use herbicides to protect important crop plants such as rice, wheat, corn, soybean, cotton and sugar beet from weeds and thereby to increase the harvest. Especially in recent years, a selective herbicide is desired which is capable of selectively killing weeds without showing any phytotoxicity against crop plants when applied to the foliages of crop plants and weeds simultaneously in a field where such useful crop plants and weeds are coexistent. Further, with a view to avoiding environmental pollution and reducing the costs for transportation and application, researches and developments have been conducted for many years for compounds having high herbicidal effects at low doses. Some of the compounds having such properties are presently used as selective herbicides. However, there still exists a need for better new compounds having such properties.

As the prior art showing a chemical structure similar to that of the compounds of the present invention, Japanese Unexamined Patent Publications No. 15962/1983, No. 103371/1983, No. 126859/1983, No. 48973/1985, No. 214785/1985, No. 134377/1986, No. 151577/1989, No. 45473/1990, No. 91060/1990, No. 7284/1991 and No. 68562/1991, and U.S Patents 4,559,081, 4,592,776, 4,602,939, 4,622,065, 4,666,508, 4,696,695 and 4,741,762, disclose compounds having sulfonylurea bonded to a nitrogen atom. However, compounds having sulfonylurea bonded to a nitrogen atom of an imino structure like the compounds of the present invention have not been known at all, and they are novel compounds.

### DISCLOSURE OF THE INVENTION

The present inventors have conducted extensive researches over years to develop selective herbicides for important crop plants and have studied herbicidal properties of many compounds with an aim to find out compounds having higher herbicidal activities as well as selectivity. As a result, it has been found that an iminosulfonylurea derivative of the formula (1) or an agriculturally suitable salt thereof: 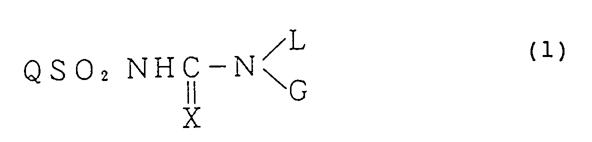
wherein Q is 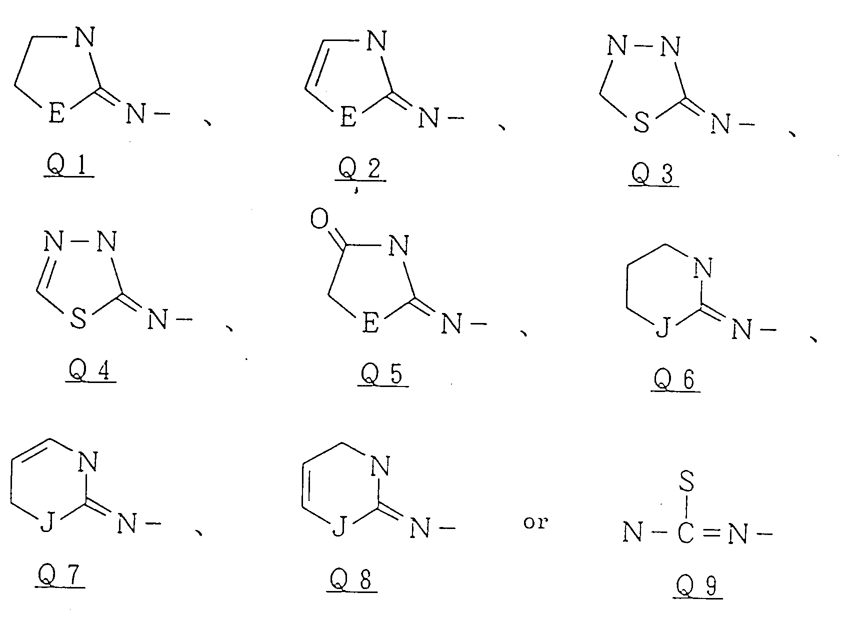 wherein in Q1, Q2 and Q5, E is a sulfur atom, an oxygen atom or a nitrogen atom mono-substituted by a substituent other than a hydrogen atom; in Q6, Q7 and Q8, J is a sulfur atom or an oxygen atom; in Q1 to Q8, a nitrogen atom in the ring of Q is substituted by a substituent other than a hydrogen atom, and a carbon atom in the ring of Q is optionally substituted; and in Q9, the sulfur atom and the nitrogen atom on the carbon atom to which the imino group of Q is bonded, are substituted by substituents other than hydrogen atoms, wherein Q is preferably 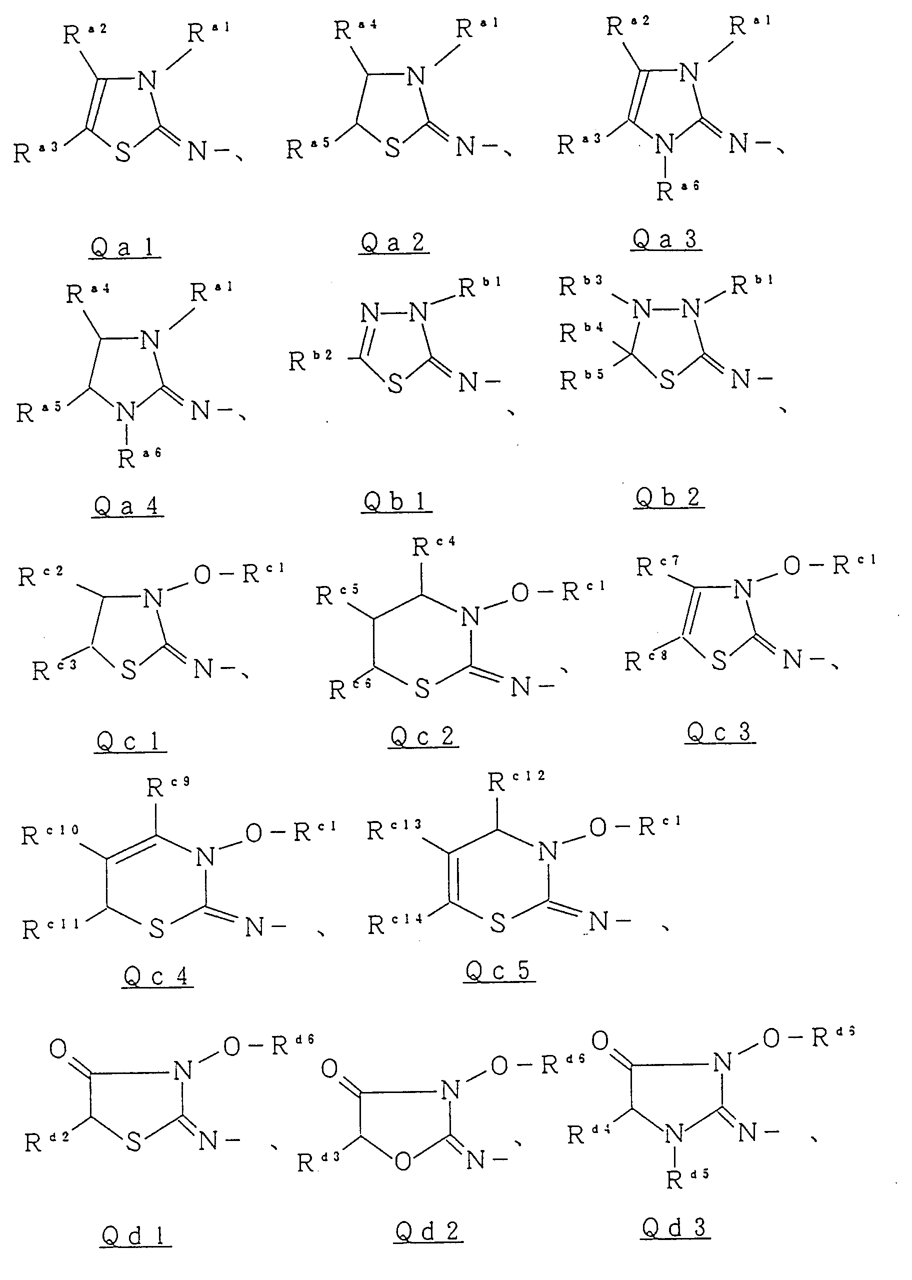 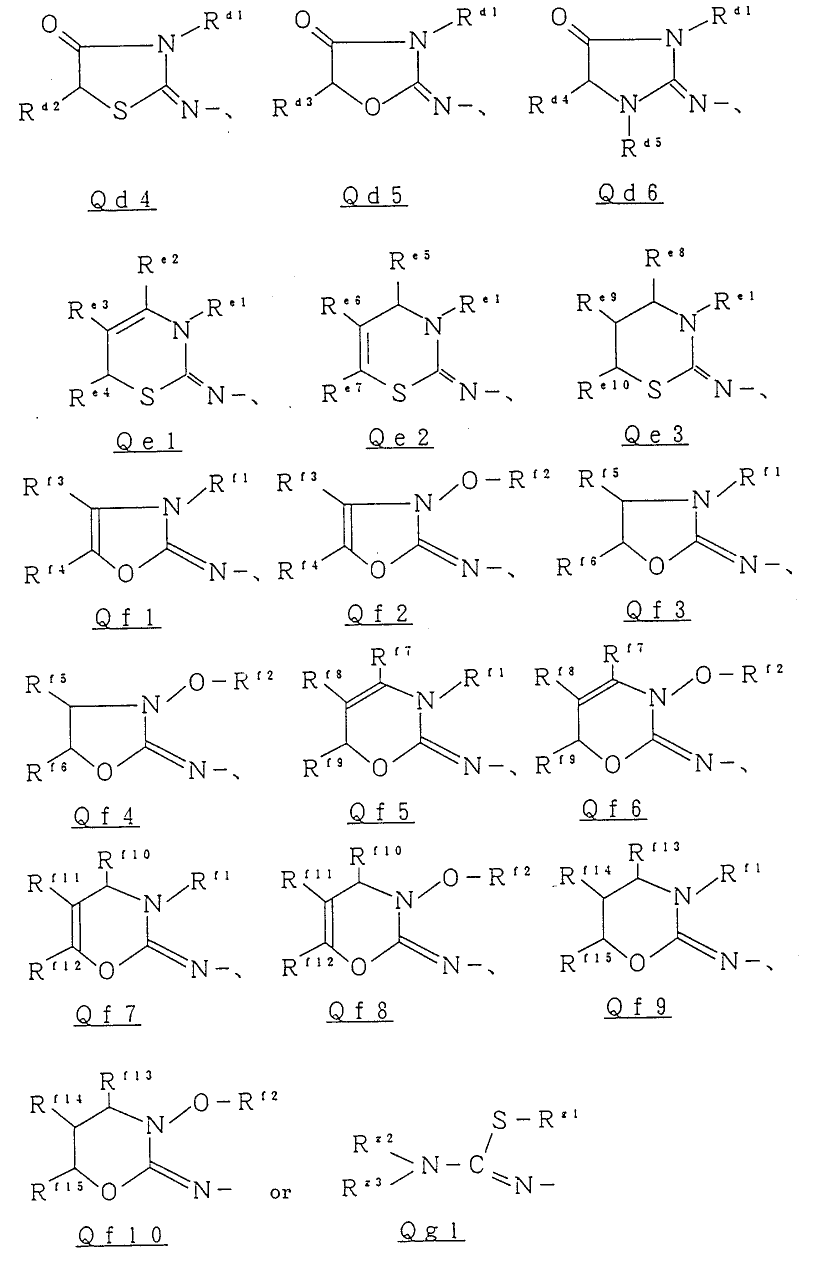
R^{a1} is a C₁₋₈ alkyl group, a C₃₋₇ cycloalkyl group, a C₁₋₆ alkyl group substituted by a C₃₋₇ cycloalkyl group, a C₃₋₇ cycloalkenyl group, a C₁₋₆ alkyl group substituted by a C₃₋₇ cycloalkenyl group, a C₂₋₈ alkenyl group, a C₂₋₈ alkynyl group, a C₁₋₆ alkyl group substituted by a C₁₋₆ alkoxy group, a C₁₋₆ alkyl group substituted by a C₂₋₆ alkenyloxy group, a C₁₋₆ alkyl group substituted by a C₂₋₆ alkynyloxy group, a C₁₋₆ alkyl group substituted by a mono-, di- or poly-halogeno C₁₋₆ alkoxy group, a C₁₋₆ alkyl group substituted by a mono-, di- or poly-halogeno C₂₋₆ alkenyloxy group, a C₁₋₆ alkyl group substituted by a mono-, di- or poly-halogeno C₂₋₆ alkynyloxy group, a C₁₋₆ alkyl group substituted by a C₁₋₆ alkylthio group, a C₁₋₆ alkyl group substituted by a C₁₋₆ alkylsulfinyl group, a C₁₋₆ alkyl group substituted by a C₁₋₆ alkylsulfonyl group, a mono-, di- or poly-halogeno C₁₋₈ alkyl group, a mono-, di- or poly-halogeno C₂₋₈ alkenyl group, a mono-, di- or poly-halogeno C₂₋₈ alkynyl group, a C₁₋₆ alkyl group substituted by a cyano group, a C₂₋₆ alkenyl group substituted by a cyano group, a C₂₋₆ alkynyl group substituted by a cyano group, a C₁₋₆ alkyl group substituted by a nitro group, a C₂₋₆ alkenyl group substituted by a nitro group, a C₂₋₆ alkynyl group substituted by a nitro group, a C₁₋₆ alkyl group substituted by a C₂₋₇ alkoxycarbonyl group, a C₂₋₆ alkenyl group substituted by a C₂₋₇ alkoxycarbonyl group, a C₂₋₆ alkynyl group substituted by a C₂₋₇ alkoxycarbonyl group, a C₁₋₆ alkyl group substituted by a C₂₋₇ alkylcarbonyl group, a C₁₋₆ alkyl group substituted by a mono-, di- or poly-halogeno C₂₋₇ alkylcarbonyl group, a C₁₋₆ alkyl group substituted by a C₃₋₇ alkenylcarbonyl group, a C₁₋₆ alkyl group substituted by a C₃₋₇ alkynylcarbonyl group, a C₁₋₆ alkyl group substituted by a C₂₋₅ alkylcarbonyl group substituted by a C₁₋₄ alkoxy group, a C₁₋₆ alkyl group substituted by a C₂₋₅ alkylcarbonyl group substituted by a C₁₋₄ alkylthio group, a C₁₋₆ alkyl group substituted by a C₂₋₅ alkylcarbonyl group substituted by a C₁₋₄ alkylsulfinyl group, a C₁₋₆ alkyl group substituted by a C₂₋₅ alkylcarbonyl group substituted by a C₁₋₄ alkylsulfonyl group, a C₂₋₆ alkenyl group substituted by a C₂₋₇ alkylcarbonyl group, a C₂₋₆ alkynyl group substituted by a C₂₋₇ alkylcarbonyl group, a C₁₋₆ alkyl group substituted by a C₁₋₆ alkylsulfamoyl group, a C₁₋₆ alkyl group substituted by a C₁₋₆ alkoxysulfamoyl group, a C₁₋₆ alkyl group substituted by a di(C₁₋₃ alkyl)sulfamoyl group, a C₁₋₆ alkyl group substituted by an N-(C₁₋₃ alkyl)-N-(C₁₋₃ alkoxy)sulfamoyl group, a C₁₋₆ alkyl group substituted by a C₂₋₇ alkylcarbamoyl group, a C₁₋₆ alkyl group substituted by a di(C₁₋₃ alkyl)carbamoyl group, a C₁₋₆ alkyl group substituted by a C₂₋₇ alkoxycarbamoyl group, a C₁₋₆ alkyl group substituted by an N-(C₁₋₃ alkyl)-N-(C₁₋₃ alkoxy)carbamoyl group, a C₁₋₆ alkyl group substituted by a C₁₋₆ alkylamino group, a C₁₋₆ alkyl group substituted by a C₁₋₆ alkoxyamino group, a C₁₋₆ alkyl group substituted by a di(C₁₋₃ alkyl)amino group, a C₁₋₆ alkyl group substituted by an N-(C₁₋₃ alkyl)-N-(C₁₋₃ alkoxy)amino group, a C₁₋₆ alkyl group substituted by an N-(C₂₋₇ alkylcarbonyl)-N-(C₁₋₆ alkyl)amino group, a C₁₋₆ alkyl group substituted by an N-(C₂₋₇ alkylcarbonyl)-N-(C₁₋₆ alkoxy)amino group, a C₁₋₆ alkyl group substituted by an N-(C₁₋₆ alkylsulfonyl)-N-(C₁₋₆ alkyl)amino group, a C₁₋₆ alkyl group substituted by an N-(C₁₋₆ alkylsulfonyl)-N-(C₁₋₆ alkoxy)amino group, a phenyl group (provided that such a phenyl group may be substituted by one or more substituents selected from the group consisting of a halogen atom, a trifluoromethyl group, a nitro group, a C₁₋₆ alkyl group, a C₁₋₆ alkoxy group and a C₂₋₇ alkoxycarbonyl group), a C₁₋₆ alkyl group substituted by a phenyl group (provided that such a phenyl group may be substituted by one or more substituents selected from the group consisting of a halogen atom, a trifluoromethyl group, a nitro group, a C₁₋₆ alkyl group, a C₁₋₆ alkoxy group and a C₂₋₇ alkoxycarbonyl group), a C₂₋₇ alkenyl group substituted by a phenyl group (provided that such a phenyl group may be substituted by one or more substituents selected from the group consisting of a halogen atom, a trifluoromethyl group, a nitro group, a C₁₋₆ alkyl group, a C₁₋₆ alkoxy group and a C₂₋₇ alkoxycarbonyl group), a C₂₋₆ alkynyl group substituted by a phenyl group (provided that such a phenyl group may be substituted by one or more substituents selected from the group consisting of a halogen atom, a trifluoromethyl group, a nitro group, a C₁₋₆ alkyl group, a C₁₋₆ alkoxy group and a C₂₋₇ alkoxycarbonyl group), a C₁₋₆ alkyl group substituted by a phenoxy group (provided that such a phenoxy group may be substituted by one or more substituents selected from the group consisting of a halogen atom, a trifluoromethyl group, a nitro group, a C₁₋₆ alkyl group, a C₁₋₆ alkoxy group and a C₂₋₇ alkoxycarbonyl group), a C₁₋₆ alkyl group substituted by a phenylthio group (provided that such a phenylthio group may be substituted by one or more substituents selected from the group consisting of a halogen atom, a trifluoromethyl group, a nitro group, a C₁₋₆ alkyl group, a C₁₋₆ alkoxy group and a C₂₋₇ alkoxycarbonyl group), a C₁₋₆ alkyl group substituted by a phenylsulfinyl group (provided that such a phenylsulfinyl group may be substituted by one or more substituents selected from the group consisting of a halogen atom, a trifluoromethyl group, a nitro group, a C₁₋₆ alkyl group, a C₁₋₆ alkoxy group and a C₂₋₇ alkoxycarbonyl group), a C₁₋₆ alkyl group substituted by a phenylsulfonyl group (provided that such a phenylsulfonyl group may be substituted by one or more substituents selected from the group consisting of a halogen atom, a trifluoromethyl group, a nitro group, a C₁₋₆ alkyl group, a C₁₋₆ alkoxy group and a C₂₋₇ alkoxycarbonyl group), a C₁₋₆ alkyl group substituted by a benzyloxy group (provided that the phenyl group of such a benzyloxy group may be substituted by one or more substituents selected from the group consisting of a halogen atom, a trifluoromethyl group, a nitro group, a C₁₋₆ alkyl group, a C₁₋₆ alkoxy group and a C₂₋₇ alkoxycarbonyl group), a C₁₋₆ alkyl group substituted by a benzylthio group (provided that the phenyl group of such a benzylthio group may be substituted by one or more substituents selected from the group consisting of a halogen atom, a trifluoromethyl group, a nitro group, a C₁₋₆ alkyl group, a C₁₋₆ alkoxy group and a C₂₋₇ alkoxycarbonyl group), a C₁₋₆ alkyl group substituted by a benzylsulfinyl group (provided that the phenyl group of such a benzylsulfinyl group may be substituted by one or more substituents selected from the group consisting of a halogen atom, a trifluoromethyl group, a nitro group, a C₁₋₆ alkyl group, a C₁₋₆ alkoxy group and a C₂₋₇ alkoxycarbonyl group), a C₁₋₆ alkyl group substituted by a benzylsulfonyl group (provided that the phenyl group of such a benzylsulfonyl group may be substituted by one or more substituents selected from the group consisting of a halogen atom, a trifluoromethyl group, a nitro group, a C₁₋₆ alkyl group, a C₁₋₆ alkoxy group and a C₂₋₇ alkoxycarbonyl group), a C₁₋₆ alkyl group substituted by a phenylcarbonyl group (provided that such a phenylcarbonyl group may be substituted by one or more substituents selected from the group consisting of a halogen atom, a trifluoromethyl group, a nitro group, a C₁₋₆ alkyl group, a C₁₋₆ alkoxy group and a C₂₋₇ alkoxycarbonyl group), a C₁-₆ alkyl group substituted by a benzylcarbonyl group (provided that the phenyl group of such a benzylcarbonyl group may be substituted by one or more substituents selected from the group consisting of a halogen atom, a trifluoromethyl group, a nitro group, a C₁₋₆ alkyl group, a C₁₋₆ alkoxy group and a C₂₋₇ alkoxycarbonyl group), a C₁₋₆ alkyl group substituted by an amino group substituted by a C₁₋₄ alkylsulfonyl group, or a C₁₋₆ alkyl group substituted by an amino group substituted by a C₂₋₄ alkylcarbonyl group,
each of R^{a2} and R^{a3} which are independent of each other, is a hydrogen atom, a C₁₋₆ alkyl group, a mono-, di- or poly-halogeno C₁₋₆ alkyl group, a C₁₋₆ alkoxy group, a mono-, di or poly-halogeno C₁₋₆ alkoxy group, a C₁₋₆ alkylthio group, a C₁₋₆ alkylsulfinyl group, a C₁₋₆ alkylsulfonyl group, a C₂₋₇ alkoxycarbonyl group, a C₂₋₇ alkylcarbonyl group, a halogen atom, a nitro group, a cyano group, or a phenyl group (provided that such a phenyl group may be substituted by one or more substituents selected from the group consisting of a halogen atom, a trifluoromethyl group, a nitro group, a C₁₋₆ alkyl group, a C₁₋₆ alkoxy group and a C₂₋₇ alkoxycarbonyl group),
each of R^{a4} and R^{a5} which are independent of each other, is a hydrogen atom, a C₁₋₈ alkyl group, a C₂₋₈ alkenyl group, a C₂₋₈ alkynyl group, or a phenyl group (provided that such a phenyl group may be substituted by one or more substituents selected from the group consisting of a halogen atom, a trifluoromethyl group, a nitro group, a C₁₋₆ alkyl group, a C₁₋₆ alkoxy group and a C₂₋₇ alkoxycarbonyl group),
R^{a6} is a C₁₋₈ alkyl group, a C₂₋₈ alkenyl group, a C₂₋₈ alkynyl group or a phenyl group (provided that such a phenyl group may be substituted by one or more substituents selected from the group consisting of a halogen atom, a trifluoromethyl group, a nitro group, a C₁₋₆ alkyl group, a C₁₋₆ alkoxy group and a C₂₋₇ alkoxycarbonyl group),
R^{b1} is a C₁₋₈ alkyl group, a C₃₋₇ cycloalkyl group, a C₁₋₆ alkyl group substituted by a C₃₋₇ cycloalkyl group, a C₃₋₇ cycloalkenyl group, a C₁₋₆ alkyl group substituted by a C₃₋₇ cycloalkenyl group, a C₂₋₈ alkenyl group, a C₂₋₈ alkynyl group, a C₁₋₆ alkyl group substituted by a C₁₋₆ alkoxy group, a C₁₋₆ alkyl group substituted by a C₂₋₆ alkenyloxy group, a C₁₋₆ alkyl group substituted by a C₂₋₆ alkynyloxy group, a C₁₋₆ alkyl group substituted by a mono-, di or poly-halogeno C₁₋₆ alkoxy group, a C₁₋₆ alkyl group substituted by a mono-, di- or poly-halogeno C₂₋₆ alkenyloxy group, a C₁₋₆ alkyl group substituted by a mono-, di- or poly-halogeno C₂₋₆ alkynyloxy group, a C₁₋₆ alkyl group substituted by a C₁₋₆ alkylthio group, a C₁₋₆ alkyl group substituted by a C₁₋₆ alkylsulfinyl group, a C₁₋₆ alkyl group substituted by a C₁₋₆ alkylsulfonyl group, a mono-, di- or poly-halogeno C₁₋₈ alkyl group, a mono-, di- or poly-halogeno C₂₋₈ alkenyl group, a mono-, di- or poly-halogeno C₂₋₈ alkynyl group, a C₁₋₆ alkyl group substituted by a cyano group, a C₂₋₆ alkenyl group substituted by a cyano group, a C₂₋₆ alkynyl group substituted by a cyano group, a C₁₋₆ alkyl group substituted by a nitro group, a C₂₋₆ alkenyl group substituted by a nitro group, a C₂₋₆ alkynyl group substituted by a nitro group, a C₁₋₆ alkyl group substituted by a C₂₋₇ alkoxycarbonyl group, a C₂₋₆ alkenyl group substituted by a C₂₋₇ alkoxycarbonyl group, a C₂₋₆ alkynyl group substituted by a C₂₋₇ alkoxycarbonyl group, a C₁₋₆ alkyl group substituted by a C₂₋₇ alkylcarbonyl group, a C₁₋₆ alkyl group substituted by a mono-, di- or poly-halogeno C₂₋₇ alkylcarbonyl group, a C₁₋₆ alkyl group substituted by a C₃₋₇ alkenylcarbonyl group, a C₁₋₆ alkyl group substituted by a C₃₋₇ alkynylcarbonyl group, a C₁₋₆ alkyl group substituted by a C₂₋₅ alkylcarbonyl group substituted by a C₁₋₄ alkoxy group, a C₁₋₆ alkyl group substituted by a C₂₋₅ alkylcarbonyl group substituted by a C₁₋₄ alkylthio group, a C₁₋₆ alkyl group substituted by a C₂₋₅ alkylcarbonyl group substituted by a C₁₋₄ alkylsulfinyl group, a C₁₋₆ alkyl group substituted by a C₂₋₅ alkylcarbonyl group substituted by a C₁₋₄ alkylsulfonyl group, a C₂₋₆ alkenyl group substituted by a C₂₋₇ alkylcarbonyl group, a C₂₋₆ alkynyl group substituted by a C₂₋₇ alkylcarbonyl group, a C₁₋₆ alkyl group substituted by a C₁₋₆ alkylsulfamoyl group, a C₁₋₆ alkyl group substituted by a C₁₋₆ alkoxysulfamoyl group, a C₁₋₆ alkyl group substituted by a di(C₁₋₃ alkyl)sulfamoyl group, a C₁₋₆ alkyl group substituted by an N-(C₁₋₃ alkyl)-N-(C₁₋₃ alkoxy)sulfamoyl group, a C₁₋₆ alkyl group substituted by a C₂₋₇ alkylcarbamoyl group, a C₁₋₆ alkyl group substituted by a di(C₁₋₃ alkyl)carbamoyl group, a C₁₋₆ alkyl group substituted by a C₂₋₇ alkoxycarbamoyl group, a C₁₋₆ alkyl group substituted by an N-(C₁₋₃ alkyl)-N-(C₁₋₃ alkoxy)carbamoyl group, a C₁₋₆ alkyl group substituted by a C₁₋₆ alkylamino group, a C₁₋₆ alkyl group substituted by a C₁₋₆ alkoxyamino group, a C₁₋₆ alkyl group substituted by a di(C₁₋₃ alkyl)amino group, a C₁₋₆ alkyl group substituted by an N-(C₁₋₃ alkyl)-N-(C₁₋₃ alkoxy)amino group, a C₁₋₆ alkyl group substituted by an N-(C₂₋₇ alkylcarbonyl)-N-(C₁₋₆ alkyl)amino group, a C₁₋₆ alkyl group substituted by an N-(C₂₋₇ alkylcarbonyl)-N-(C₁₋₆ alkoxy)amino group, a C₁₋₆ alkyl group substituted by an N-(C₁₋₆ alkylsulfonyl)-N-(C₁₋₆ alkyl)amino group, a C₁₋₆ alkyl group substituted by an N-(C₁₋₆ alkylsulfonyl)-N-(C₁₋₆ alkoxy)amino group, a phenyl group (provided that such a phenyl group may be substituted by one or more substituents selected from the group consisting of a halogen atom, a trifluoromethyl group, a nitro group, a C₁₋₆ alkyl group, a C₁₋₆ alkoxy group and a C₂₋₇ alkoxycarbonyl group), a C₁₋₆ alkyl group substituted by a phenyl group (provided that such a phenyl group may be substituted by one or more substituents selected from the group consisting of a halogen atom, a trifluoromethyl group, a nitro group, a C₁₋₆ alkyl group, a C₁₋₆ alkoxy group and a C₂₋₇ alkoxycarbonyl group), a C₂₋₇ alkenyl group substituted by a phenyl group (provided that such a phenyl group may be substituted by one or more substituents selected from the group consisting of a halogen atom, a trifluoromethyl group, a nitro group, a C₁₋₆ alkyl group, a C₁₋₆ alkoxy group and a C₂₋₇ alkoxycarbonyl group), a C₂₋₆ alkynyl group substituted by a phenyl group (provided that such a phenyl group may be substituted by one or more substituents selected from the group consisting of a halogen atom, a trifluoromethyl group, a nitro group, a C₁₋₆ alkyl group, a C₁₋₆ alkoxy group and a C₂₋₇ alkoxycarbonyl group), a C₁₋₆ alkyl group substituted by a phenoxy group (provided that such a phenoxy group may be substituted by one or more substituents selected from the group consisting of a halogen atom, a trifluoromethyl group, a nitro group, a C₁₋₆ alkyl group, a C₁₋₆ alkoxy group and a C₂₋₇ alkoxycarbonyl group), a C₁₋₆ alkyl group substituted by a phenylthio group (provided that such a phenylthio group may be substituted by one or more substituents selected from the group consisting of a halogen atom, a trifluoromethyl group, a nitro group, a C₁₋₆ alkyl group, a C₁₋₆ alkoxy group and a C₂₋₇ alkoxycarbonyl group), a C₁₋₆ alkyl group substituted by a phenylsulfinyl group (provided that such a phenylsulfinyl group may be substituted by one or more substituents selected from the group consisting of a halogen atom, a trifluoromethyl group, a nitro group, a C₁₋₆ alkyl group, a C₁₋₆ alkoxy group and a C₂₋₇ alkoxycarbonyl group), a C₁₋₆ alkyl group substituted by a phenylsulfonyl group (provided that such a phenylsulfonyl group may be substituted by one or more substituents selected from the group consisting of a halogen atom, a trifluoromethyl group, a nitro group, a C₁₋₆ alkyl group, a C₁₋₆ alkoxy group and a C₂₋₇ alkoxycarbonyl group), a C₁₋₆ alkyl group substituted by a benzyloxy group (provided that the phenyl group of such a benzyloxy group may be substituted by one or more substituents selected from the group consisting of a halogen atom, a trifluoromethyl group, a nitro group, a C₁₋₆ alkyl group, a C₁₋₆ alkoxy group and a C₂₋₇ alkoxycarbonyl group), a C₁₋₆ alkyl group substituted by a benzylthio group (provided that the phenyl group of such a benzylthio group may be substituted by one or more substituents selected from the group consisting of a halogen atom, a trifluoromethyl group, a nitro group, a C₁₋₆ alkyl group, a C₁₋₆ alkoxy group and a C₂₋₇ alkoxycarbonyl group), a C₁₋₆ alkyl group substituted by a benzylsulfinyl group (provided that the phenyl group of such a benzylsulfinyl group may be substituted by one or more substituents selected from the group consisting of a halogen atom, a trifluoromethyl group, a nitro group, a C₁₋₆ alkyl group, a C₁₋₆ alkoxy group and a C₂₋₇ alkoxycarbonyl group), a C₁₋₆ alkyl group substituted by a benzylsulfonyl group (provided that the phenyl group of such a benzylsulfonyl group may be substituted by one or more substituents selected from the group consisting of a halogen atom, a trifluoromethyl group, a nitro group, a C₁₋₆ alkyl group, a C₁₋₆ alkoxy group and a C₂₋₇ alkoxycarbonyl group), a C₁₋₆ alkyl group substituted by a phenylcarbonyl group (provided that such a phenylcarbonyl group may be substituted by one or more substituents selected from the group consisting of a halogen atom, a trifluoromethyl group, a nitro group, a C₁₋₆ alkyl group, a C₁₋₆ alkoxy group and a C₂₋₇ alkoxycarbonyl group), a C₁₋₆ alkyl group substituted by a benzylcarbonyl group (provided that the phenyl group of such a benzylcarbonyl group may be substituted by one or more substituents selected from the group consisting of a halogen atom, a trifluoromethyl group, a nitro group, a C₁₋₆ alkyl group, a C₁₋₆ alkoxy group and a C₂₋₇ alkoxycarbonyl group), a C₁₋₆ alkyl group substituted by an amino group substituted by a C₁₋₄ alkylsulfonyl group, or a C₁₋₆ alkyl group substituted by an amino group substituted by a C₂₋₄ alkylcarbonyl group,
R^{b2} is a hydrogen atom, a C₁₋₆ alkyl group, a mono-, di- or poly-halogeno C₁₋₆ alkyl group, a C₁₋₆ alkoxy group, a mono-, di- or poly-halogeno C₁₋₆ alkoxy group, a C₁₋₆ alkylthio group, a C₁₋₆ alkylsulfinyl group, a C₁₋₆ alkylsulfonyl group, a C₂₋₇ alkoxycarbonyl group, a C₂₋₇ alkylcarbonyl group, a halogen atom, a nitro group, a cyano group, or a phenyl group (provided that such a phenyl group may be substituted by one or more substituents selected from the group consisting of a halogen atom, a trifluoromethyl group, a nitro group, a C₁₋₆ alkyl group, a C₁₋₆ alkoxy group and a C₂₋₇ alkoxycarbonyl group),
R^{b3} is a C₁₋₆ alkyl group, a C₂₋₆ alkenyl group or a C₂₋₆ alkynyl group,
R^{b4} is a hydrogen atom, or a C₁₋₆ alkyl group,
R^{b5} is a hydrogen atom, or a C₁₋₆ alkyl group,
R^{c1} is a C₁₋₈ alkyl group, a C₃₋₇ cycloalkyl group, a C₁₋₆ alkyl group substituted by a C₃₋₇ cycloalkyl group, a C₃₋₇ cycloalkenyl group, a C₁₋₆ alkyl group substituted by a C₃₋₇ cycloalkenyl group, a C₂₋₈ alkenyl group, a C₂₋₈ alkynyl group, a C₁₋₆ alkyl group substituted by a C₁₋₆ alkoxy group, a C₁₋₆ alkyl group substituted by a C₂₋₆ alkenyloxy group, a C₁₋₆ alkyl group substituted by a C₂₋₆ alkynyloxy group, a C₁₋₆ alkyl group substituted by a mono-, di- or poly-halogeno C₁₋₆ alkoxy group, a C₁₋₆ alkyl group substituted by a mono-, di- or poly-halogeno C₂₋₆ alkenyloxy group, a C₁₋₆ alkyl group substituted by a mono-, di- or poly-halogeno C₂₋₆ alkynyloxy group, a C₁₋₆ alkyl group substituted by a C₁₋₆ alkylthio group, a C₁₋₆ alkyl group substituted by a C₁₋₆ alkylsulfinyl group, a C₁₋₆ alkyl group substituted by a C₁₋₆ alkylsulfonyl group, a mono-, di- or poly-halogeno C₁₋₈ alkyl group, a mono-, di- or poly-halogeno C₂₋₈ alkenyl group, a mono-, di- or poly-halogeno C₂₋₈ alkynyl group, a C₁₋₆ alkyl group substituted by a cyano group, a C₂₋₆ alkenyl group substituted by a cyano group, a C₂₋₆ alkynyl group substituted by a cyano group, a C₁₋₆ alkyl group substituted by a nitro group, a C₂₋₆ alkenyl group substituted by a nitro group, a C₂₋₆ alkynyl group substituted by a nitro group, a C₁₋₆ alkyl group substituted by a C₂₋₇ alkoxycarbonyl group, a C₂₋₆ alkenyl group substituted by a C₂₋₇ alkoxycarbonyl group, a C₂₋₆ alkynyl group substituted by a C₂₋₇ alkoxycarbonyl group, a C₁₋₆ alkyl group substituted by a C₂₋₇ alkylcarbonyl group, a C₁₋₆ alkyl group substituted by a mono-, di- or poly-halogeno C₂₋₇ alkylcarbonyl group, a C₁₋₆ alkyl group substituted by a C₃₋₇ alkenylcarbonyl group, a C₁₋₆ alkyl group substituted by a C₃₋₇ alkynylcarbonyl group, a C₁₋₆ alkyl group substituted by a C₂₋₅ alkylcarbonyl group substituted by a C₁₋₄ alkoxy group, a C₁₋₆ alkyl group substituted by a C₂₋₅ alkylcarbonyl group substituted by a C₁₋₄ alkylthio group, a C₁₋₆ alkyl group substituted by a C₂₋₅ alkylcarbonyl group substituted by a C₁₋₄ alkylsulfinyl group, a C₁₋₆ alkyl group substituted by a C₂₋₅ alkylcarbonyl group substituted by a C₁₋₄ alkylsulfonyl group, a C₂₋₆ alkenyl group substituted by a C₂₋₇ alkylcarbonyl group, a C₂₋₆ alkynyl group substituted by a C₂₋₇ alkylcarbonyl group, a C₁₋₆ alkyl group substituted by a C₁₋₆ alkylsulfamoyl group, a C₁₋₆ alkyl group substituted by a C₁₋₆ alkoxysulfamoyl group, a C₁₋₆ alkyl group substituted by a di(C₁₋₃ alkyl)sulfamoyl group, a C₁₋₆ alkyl group substituted by an N-(C₁₋₃ alkyl)-N-(C₁₋₃ alkoxy)sulfamoyl group, a C₁₋₆ alkyl group substituted by a C₂₋₇ alkylcarbamoyl group, a C₁₋₆ alkyl group substituted by a di(C₁₋₃ alkyl)carbamoyl group, a C₁₋₆ alkyl group substituted by a C₂₋₇ alkoxycarbamoyl group, a C₁₋₆ alkyl group substituted by an N-(C₁₋₃ alkyl)-N-(C₁₋₃ alkoxy)carbamoyl group, a C₁₋₆ alkyl group substituted by a C₁₋₆ alkylamino group, a C₁₋₆ alkyl group substituted by a C₁₋₆ alkoxyamino group, a C₁₋₆ alkyl group substituted by a di(C₁₋₃ alkyl)amino group, a C₁₋₆ alkyl group substituted by an N-(C₁₋₃ alkyl)-N-(C₁₋₃ alkoxy)amino group, a C₁₋₆ alkyl group substituted by an N-(C₂₋₇ alkylcarbonyl)-N-(C₁₋₆ alkyl)amino group, a C₁₋₆ alkyl group substituted by an N-(C₂₋₇ alkylcarbonyl)-N-(C₁₋₆ alkoxy)amino group, a C₁₋₆ alkyl group substituted by an N-(C₁₋₆ alkylsulfonyl)-N-(C₁₋₆ alkyl)amino group, a C₁₋₆ alkyl group substituted by an N-(C₁₋₆ alkylsulfonyl)-N-(C₁₋₆ alkoxy)amino group, a phenyl group (provided that such a phenyl group may be substituted by one or more substituents selected from the group consisting of a halogen atom, a trifluoromethyl group, a nitro group, a C₁₋₆ alkyl group, a C₁₋₆ alkoxy group and a C₂₋₇ alkoxycarbonyl group), a C₁₋₆ alkyl group substituted by a phenyl group (provided that such a phenyl group may be substituted by one or more substituents selected from the group consisting of a halogen atom, a trifluoromethyl group, a nitro group, a C₁₋₆ alkyl group, a C₁₋₆ alkoxy group and a C₂₋₇ alkoxycarbonyl group), a C₂₋₇ alkenyl group substituted by a phenyl group (provided that such a phenyl group may be substituted by one or more substituents selected from the group consisting of a halogen atom, a trifluoromethyl group, a nitro group, a C₁₋₆ alkyl group, a C₁₋₆ alkoxy group and a C₂₋₇ alkoxycarbonyl group), a C₂₋₆ alkynyl group substituted by a phenyl group (provided that such a phenyl group may be substituted by one or more substituents selected from the group consisting of a halogen atom, a trifluoromethyl group, a nitro group, a C₁₋₆ alkyl group, a C₁₋₆ alkoxy group and a C₂₋₇ alkoxycarbonyl group), a C₁₋₆ alkyl group substituted by a phenoxy group (provided that such a phenoxy group may be substituted by one or more substituents selected from the group consisting of a halogen atom, a trifluoromethyl group, a nitro group, a C₁₋₆ alkyl group, a C₁₋₆ alkoxy group and a C₂₋₇ alkoxycarbonyl group), a C₁₋₆ alkyl group substituted by a phenylthio group (provided that such a phenylthio group may be substituted by one or more substituents selected from the group consisting of a halogen atom, a trifluoromethyl group, a nitro group, a C₁₋₆ alkyl group, a C₁₋₆ alkoxy group and a C₂₋₇ alkoxycarbonyl group), a C₁₋₆ alkyl group substituted by a phenylsulfinyl group (provided that such a phenylsulfinyl group may be substituted by one or more substituents selected from the group consisting of a halogen atom, a trifluoromethyl group, a nitro group, a C₁₋₆ alkyl group, a C₁₋₆ alkoxy group and a C₂₋₇ alkoxycarbonyl group), a C₁₋₆ alkyl group substituted by a phenylsulfonyl group (provided that such a phenylsulfonyl group may be substituted by one or more substituents selected from the group consisting of a halogen atom, a trifluoromethyl group, a nitro group, a C₁₋₆ alkyl group, a C₁₋₆ alkoxy group and a C₂₋₇ alkoxycarbonyl group), a C₁₋₆ alkyl group substituted by a benzyloxy group (provided that the phenyl group of such a benzyloxy group may be substituted by one or more substituents selected from the group consisting of a halogen atom, a trifluoromethyl group, a nitro group, a C₁₋₆ alkyl group, a C₁₋₆ alkoxy group and a C₂₋₇ alkoxycarbonyl group), a C₁₋₆ alkyl group substituted by a benzylthio group (provided that the phenyl group of such a benzylthio group may be substituted by one or more substituents selected from the group consisting of a halogen atom, a trifluoromethyl group, a nitro group, a C₁₋₆ alkyl group, a C₁₋₆ alkoxy group and a C₂₋₇ alkoxycarbonyl group), a C₁₋₆ alkyl group substituted by a benzylsulfinyl group (provided that the phenyl group of such a benzylsulfinyl group may be substituted by one or more substituents selected from the group consisting of a halogen atom, a trifluoromethyl group, a nitro group, a C₁₋₆ alkyl group, a C₁₋₆ alkoxy group and a C₂₋₇ alkoxycarbonyl group), a C₁₋₆ alkyl group substituted by a benzylsulfonyl group (provided that the phenyl group of such a benzylsulfonyl group may be substituted by one or more substituents selected from the group consisting of a halogen atom, a trifluoromethyl group, a nitro group, a C₁₋₆ alkyl group, a C₁₋₆ alkoxy group and a C₂₋₇ alkoxycarbonyl group), a C₁₋₆ alkyl group substituted by a phenylcarbonyl group (provided that such a phenylcarbonyl group may be substituted by one or more substituents selected from the group consisting of a halogen atom, a trifluoromethyl group, a nitro group, a C₁₋₆ alkyl group, a C₁₋₆ alkoxy group and a C₂₋₇ alkoxycarbonyl group), a C₁₋₆ alkyl group substituted by a benzylcarbonyl group (provided that the phenyl group of such a benzylcarbonyl group may be substituted by one or more substituents selected from the group consisting of a halogen atom, a trifluoromethyl group, a nitro group, a C₁₋₆ alkyl group, a C₁₋₆ alkoxy group and a C₂₋₇ alkoxycarbonyl group), a C₁₋₆ alkyl group substituted by an amino group substituted by a C₁₋₄ alkylsulfonyl group, or a C₁₋₆ alkyl group substituted by an amino group substituted by a C₂₋₄ alkylcarbonyl group,
each of R^{c2}, R^{c3}, R^{c4}, R^{c5}, R^{c6}, R^{c11} and R^{c12} which are independent of one another, is a hydrogen atom, a C₁₋₈ alkyl group, a C₂₋₈ alkenyl group, a C₂₋₈ alkynyl group, or a phenyl group (provided that such a phenyl group may be substituted by one or more substituents selected from the group consisting of a halogen atom, a trifluoromethyl group, a nitro group, a C₁₋₆ alkyl group, a C₁₋₆ alkoxy group and a C₂₋₇ alkoxycarbonyl group),
each of R^{c7}, R^{c8}, R^{c9}, R^{c10}, R^{c13} and R^{c14} which are independent of one another, is a hydrogen atom, a C₁₋₆ alkyl group, a mono-, di- or poly-halogeno C₁₋₆ alkyl group, a C₁₋₆ alkoxy group, a mono, di- or poly-halogeno C₁₋₆ alkoxy group, a C₁₋₆ alkylthio group, a C₁₋₆ alkylsulfinyl group, a C₁₋₆ alkylsulfonyl group, a C₂₋₇ alkoxycarbonyl group, a C₂₋₇ alkylcarbonyl group, a halogen atom, a nitro group, a cyano group, or a phenyl group (provided that such a phenyl group may be substituted by one or more substituents selected from the group consisting of a halogen atom, a trifluoromethyl group, a nitro group, a C₁₋₆ alkyl group, a C₁₋₆ alkoxy group and a C₂₋₇ alkoxycarbonyl group),
R^{d1} is a C₁₋₈ alkyl group, a C₃₋₇ cycloalkyl group, a C₁₋₆ alkyl group substituted by a C₃₋₇ cycloalkyl group, a C₃₋₇ cycloalkenyl group, a C₁₋₆ alkyl group substituted by a C₃₋₇ cycloalkenyl group, a C₂₋₈ alkenyl group, a C₂₋₈ alkynyl group, a C₁₋₆ alkyl group substituted by a C₁₋₆ alkoxy group, a C₁₋₆ alkyl group substituted by a C₂₋₆ alkenyloxy group, a C₁₋₆ alkyl group substituted by a C₂₋₆ alkynyloxy group, a C₁₋₆ alkyl group substituted by a mono-, di or poly-halogeno C₁₋₆ alkoxy group, a C₁₋₆ alkyl group substituted by a mono-, di- or poly-halogeno C₂₋₆ alkenyloxy group, a C₁₋₆ alkyl group substituted by a mono-, di- or poly-halogeno C₂₋₆ alkynyloxy group, a C₁₋₆ alkyl group substituted by a C₁₋₆ alkylthio group, a C₁₋₆ alkyl group substituted by a C₁₋₆ alkylsulfinyl group, a C₁₋₆ alkyl group substituted by a C₁₋₆ alkylsulfonyl group, a mono-, di- or poly-halogeno C₁₋₈ alkyl group, a mono-, di- or poly-halogeno C₂₋₈ alkenyl group, a mono-, di- or poly-halogeno C₂₋₈ alkynyl group, a C₁₋₆ alkyl group substituted by a cyano group, a C₂₋₆ alkenyl group substituted by a cyano group, a C₂₋₆ alkynyl group substituted by a cyano group, a C₁₋₆ alkyl group substituted by a nitro group, a C₂₋₆ alkenyl group substituted by a nitro group, a C₂₋₆ alkynyl group substituted by a nitro group, a C₁₋₆ alkyl group substituted by a C₂₋₇ alkoxycarbonyl group, a C₂₋₆ alkenyl group substituted by a C₂₋₇ alkoxycarbonyl group, a C₂₋₆ alkynyl group substituted by a C₂₋₇ alkoxycarbonyl group, a C₁₋₆ alkyl group substituted by a C₂₋₇ alkylcarbonyl group, a C₁₋₆ alkyl group substituted by a mono-, di- or poly-halogeno C₂₋₇ alkylcarbonyl group, a C₁₋₆ alkyl group substituted by a C₃₋₇ alkenylcarbonyl group, a C₁₋₆ alkyl group substituted by a C₃₋₇ alkynylcarbonyl group, a C₁₋₆ alkyl group substituted by a C₂₋₅ alkylcarbonyl group substituted by a C₁₋₄ alkoxy group, a C₁₋₆ alkyl group substituted by a C₂₋₅ alkylcarbonyl group substituted by a C₁₋₄ alkylthio group, a C₁₋₆ alkyl group substituted by a C₂₋₅ alkylcarbonyl group substituted by a C₁₋₄ alkylsulfinyl group, a C₁₋₆ alkyl group substituted by a C₂₋₅ alkylcarbonyl group substituted by a C₁₋₄ alkylsulfonyl group, a C₂₋₆ alkenyl group substituted by a C₂₋₇ alkylcarbonyl group, a C₂₋₆ alkynyl group substituted by a C₂₋₇ alkylcarbonyl group, a C₁₋₆ alkyl group substituted by a C₁₋₆ alkylsulfamoyl group, a C₁₋₆ alkyl group substituted by a C₁₋₆ alkoxysulfamoyl group, a C₁₋₆ alkyl group substituted by a di(C₁₋₃ alkyl)sulfamoyl group, a C₁₋₆ alkyl group substituted by an N-(C₁₋₃ alkyl)-N-(C₁₋₃ alkoxy)sulfamoyl group, a C₁₋₆ alkyl group substituted by a C₂₋₇ alkylcarbamoyl group, a C₁₋₆ alkyl group substituted by a di(C₁₋₃ alkyl)carbamoyl group, a C₁₋₆ alkyl group substituted by a C₂₋₇ alkoxycarbamoyl group, a C₁₋₆ alkyl group substituted by an N-(C₁-₃ alkyl)-N-(C₁₋₃ alkoxy)carbamoyl group, a C₁₋₆ alkyl group substituted by a C₁₋₆ alkylamino group, a C₁₋₆ alkyl group substituted by a C₁₋₆ alkoxyamino group, a C₁₋₆ alkyl group substituted by a di(C₁₋₃ alkyl)amino group, a C₁₋₆ alkyl group substituted by an N-(C₁₋₃ alkyl)-N-(C₁₋₃ alkoxy)amino group, a C₁₋₆ alkyl group substituted by an N-(C₂₋₇ alkylcarbonyl)-N-(C₁₋₆ alkyl)amino group, a C₁₋₆ alkyl group substituted by an N-(C₂₋₇ alkylcarbonyl)-N-(C₁₋₆ alkoxy)amino group, a C₁₋₆ alkyl group substituted by an N-(C₁₋₆ alkylsulfonyl)-N-(C₁₋₆ alkyl)amino group, a C₁₋₆ alkyl group substituted by an N-(C₁₋₆ alkylsulfonyl)-N-(C₁₋₆ alkoxy)amino group, a phenyl group (provided that such a phenyl group may be substituted by one or more substituents selected from the group consisting of a halogen atom, a trifluoromethyl group, a nitro group, a C₁₋₆ alkyl group, a C₁₋₆ alkoxy group and a C₂₋₇ alkoxycarbonyl group), a C₁₋₆ alkyl group substituted by a phenyl group (provided that such a phenyl group may be substituted by one or more substituents selected from the group consisting of a halogen atom, a trifluoromethyl group, a nitro group, a C₁₋₆ alkyl group, a C₁₋₆ alkoxy group and a C₂₋₇ alkoxycarbonyl group), a C₂₋₇ alkenyl group substituted by a phenyl group (provided that such a phenyl group may be substituted by one or more substituents selected from the group consisting of a halogen atom, a trifluoromethyl group, a nitro group, a C₁₋₆ alkyl group, a C₁₋₆ alkoxy group and a C₂₋₇ alkoxycarbonyl group), a C₂₋₆ alkynyl group substituted by a phenyl group (provided that such a phenyl group may be substituted by one or more substituents selected from the group consisting of a halogen atom, a trifluoromethyl group, a nitro group, a C₁₋₆ alkyl group, a C₁₋₆ alkoxy group and a C₂₋₇ alkoxycarbonyl group), a C₁₋₆ alkyl group substituted by a phenoxy group (provided that such a phenoxy group may be substituted by one or more substituents selected from the group consisting of a halogen atom, a trifluoromethyl group, a nitro group, a C₁₋₆ alkyl group, a C₁₋₆ alkoxy group and a C₂₋₇ alkoxycarbonyl group), a C₁₋₆ alkyl group substituted by a phenylthio group (provided that such a phenylthio group may be substituted by one or more substituents selected from the group consisting of a halogen atom, a trifluoromethyl group, a nitro group, a C₁₋₆ alkyl group, a C₁₋₆ alkoxy group and a C₂₋₇ alkoxycarbonyl group), a C₁₋₆ alkyl group substituted by a phenylsulfinyl group (provided that such a phenylsulfinyl group may be substituted by one or more substituents selected from the group consisting of a halogen atom, a trifluoromethyl group, a nitro group, a C₁₋₆ alkyl group, a C₁₋₆ alkoxy group and a C₂₋₇ alkoxycarbonyl group), a C₁₋₆ alkyl group substituted by a phenylsulfonyl group (provided that such a phenylsulfonyl group may be substituted by one or more substituents selected from the group consisting of a halogen atom, a trifluoromethyl group, a nitro group, a C₁₋₆ alkyl group, a C₁₋₆ alkoxy group and a C₂₋₇ alkoxycarbonyl group), a C₁₋₆ alkyl group substituted by a benzyloxy group (provided that the phenyl group of such a benzyloxy group may be substituted by one or more substituents selected from the group consisting of a halogen atom, a trifluoromethyl group, a nitro group, a C₁₋₆ alkyl group, a C₁₋₆ alkoxy group and a C₂₋₇ alkoxycarbonyl group), a C₁₋₆ alkyl group substituted by a benzylthio group (provided that the phenyl group of such a benzylthio group may be substituted by one or more substituents selected from the group consisting of a halogen atom, a trifluoromethyl group, a nitro group, a C₁₋₆ alkyl group, a C₁₋₆ alkoxy group and a C₂₋₇ alkoxycarbonyl group), a C₁₋₆ alkyl group substituted by a benzylsulfinyl group (provided that the phenyl group of such a benzylsulfinyl group may be substituted by one or more substituents selected from the group consisting of a halogen atom, a trifluoromethyl group, a nitro group, a C₁₋₆ alkyl group, a C₁₋₆ alkoxy group and a C₂₋₇ alkoxycarbonyl group), a C₁₋₆ alkyl group substituted by a benzylsulfonyl group (provided that the phenyl group of such a benzylsulfonyl group may be substituted by one or more substituents selected from the group consisting of a halogen atom, a trifluoromethyl group, a nitro group, a C₁₋₆ alkyl group, a C₁₋₆ alkoxy group and a C₂₋₇ alkoxycarbonyl group), a C₁₋₆ alkyl group substituted by a phenylcarbonyl group (provided that such a phenylcarbonyl group may be substituted by one or more substituents selected from the group consisting of a halogen atom, a trifluoromethyl group, a nitro group, a C₁₋₆ alkyl group, a C₁₋₆ alkoxy group and a C₂₋₇ alkoxycarbonyl group), a C₁₋₆ alkyl group substituted by a benzylcarbonyl group (provided that the phenyl group of such a benzylcarbonyl group may be substituted by one or more substituents selected from the group consisting of a halogen atom, a trifluoromethyl group, a nitro group, a C₁₋₆ alkyl group, a C₁₋₆ alkoxy group and a C₂₋₇ alkoxycarbonyl group), a C₁₋₆ alkyl group substituted by an amino group substituted by a C₁₋₄ alkylsulfonyl group, or a C₁₋₆ alkyl group substituted by an amino group substituted by a C₂₋₄ alkylcarbonyl group,
each of R^{d2}, R^{d3} and R^{d4} which are independent of one another, is a hydrogen atom, a C₁₋₈ alkyl group, a C₂₋₈ alkenyl group, a C₂₋₈ alkynyl group, or a phenyl group (provided that such a phenyl group may be substituted by one or more substituents selected from the group consisting of a halogen atom, a trifluoromethyl group, a nitro group, a C₁₋₆ alkyl group, a C₁₋₆ alkoxy group and a C₂₋₇ alkoxycarbonyl group),
R^{d5} is a C₁₋₈ alkyl group, a C₂₋₈ alkenyl group, a C₂₋₈ alkynyl group, or a phenyl group (provided that such a phenyl group may be substituted by one or more substituents selected from the group consisting of a halogen atom, a trifluoromethyl group, a nitro group, a C₁₋₆ alkyl group, a C₁₋₆ alkoxy group and a C₂₋₇ alkoxycarbonyl group),
R^{d6} is a C₁₋₈ alkyl group, a C₂₋₈ alkenyl group, a C₂₋₈ alkynyl group, a C₁₋₆ alkyl group substituted by a C₁₋₆ alkylthio group, a C₁₋₆ alkyl group substituted by a C₁₋₆ alkylsulfinyl group, a C₁₋₆ alkyl group substituted by a C₁₋₆ alkylsulfonyl group, a C₁₋₆ alkyl group substituted by a C₁₋₆ alkoxy group, a C₁₋₆ alkyl group substituted by a C₂₋₇ alkoxycarbonyl group, a C₁₋₆ alkyl group substituted by a C₂₋₇ alkylcarbonyl group, a C₁₋₆ alkyl group substituted by a cyano group, a phenyl group (provided that such a phenyl group may be substituted by one or more substituents selected from the group consisting of a halogen atom, a trifluoromethyl group, a nitro group, a C₁₋₆ alkyl group, a C₁₋₆ alkoxy group and a c₂₋₇ alkoxycarbonyl group), or a C₁₋₆ alkyl group substituted by a phenyl group (provided that such a phenyl group may be substituted by one or more substituents selected from the group consisting of a halogen atom, a trifluoromethyl group, a nitro group, a C₁₋₆ alkyl group, a C₁₋₆ alkoxy group and a C₂₋₇ alkoxycarbonyl group),
R^{e1} is a C₁₋₈ alkyl group, a C₃₋₇ cycloalkyl group, a C₁₋₆ alkyl group substituted by a C₃₋₇ cycloalkyl group, a C₃₋₇ cycloalkenyl group, a C₁₋₆ alkyl group substituted by a C₃₋₇ cycloalkenyl group, a C₂₋₈ alkenyl group, a C₂₋₈ alkynyl group, a C₁₋₆ alkyl group substituted by a C₁₋₆ alkoxy group, a C₁₋₆ alkyl group substituted by a C₂₋₆ alkenyloxy group, a C₁₋₆ alkyl group substituted by a C₂₋₆ alkynyloxy group, a C₁₋₆ alkyl group substituted by a mono-, di- or poly-halogeno C₁₋₆ alkoxy group, a C₁₋₆ alkyl group substituted by a mono-, di- or poly-halogeno C₂₋₆ alkenyloxy group, a C₁₋₆ alkyl group substituted by a mono-, di- or poly-halogeno C₂₋₆ alkynyloxy group, a C₁₋₆ alkyl group substituted by a C₁₋₆ alkylthio group, a C₁₋₆ alkyl group substituted by a C₁₋₆ alkylsulfinyl group, a C₁₋₆ alkyl group substituted by a C₁₋₆ alkylsulfonyl group, a mono-, di- or poly-halogeno C₁₋₈ alkyl group, a mono-, di- or poly-halogeno C₂₋₈ alkenyl group, a mono-, di- or poly-halogeno C₂₋₈ alkynyl group, a C₁₋₆ alkyl group substituted by a cyano group, a C₂₋₆ alkenyl group substituted by a cyano group, a C₂₋₆ alkynyl group substituted by a cyano group, a C₁₋₆ alkyl group substituted by a nitro group, a C₂₋₆ alkenyl group substituted by a nitro group, a C₂₋₆ alkynyl group substituted by a nitro group, a C₁₋₆ alkyl group substituted by a C₂₋₇ alkoxycarbonyl group, a C₂₋₆ alkenyl group substituted by a C₂₋₇ alkoxycarbonyl group, a C₂₋₆ alkynyl group substituted by a C₂₋₇ alkoxycarbonyl group, a C₁₋₆ alkyl group substituted by a C₂₋₇ alkylcarbonyl group, a C₁₋₆ alkyl group substituted by a mono-, di- or poly-halogeno C₂₋₇ alkylcarbonyl group, a C₁₋₆ alkyl group substituted by a C₃₋₇ alkenylcarbonyl group, a C₁₋₆ alkyl group substituted by a C₃₋₇ alkynylcarbonyl group, a C₁₋₆ alkyl group substituted by a C₂₋₅ alkylcarbonyl group substituted by a C₁₋₄ alkoxy group, a C₁₋₆ alkyl group substituted by a C₂₋₅ alkylcarbonyl group substituted by a C₁₋₄ alkylthio group, a C₁₋₆ alkyl group substituted by a C₂₋₅ alkylcarbonyl group substituted by a C₁₋₄ alkylsulfinyl group, a C₁₋₆ alkyl group substituted by a C₂₋₅ alkylcarbonyl group substituted by a C₁₋₄ alkylsulfonyl group, a C₂₋₆ alkenyl group substituted by a C₂₋₇ alkylcarbonyl group, a C₂₋₆ alkynyl group substituted by a C₂₋₇ alkylcarbonyl group, a C₁₋₆ alkyl group substituted by a C₁₋₆ alkylsulfamoyl group, a C₁₋₆ alkyl group substituted by a C₁₋₆ alkoxysulfamoyl group, a C₁₋₆ alkyl group substituted by a di(C₁₋₃ alkyl)sulfamoyl group, a C₁₋₆ alkyl group substituted by an N-(C₁₋₃ alkyl)-N-(C₁₋₃ alkoxy)sulfamoyl group, a C₁₋₆ alkyl group substituted by a C₂₋₇ alkylcarbamoyl group, a C₁₋₆ alkyl group substituted by a di(C₁₋₃ alkyl)carbamoyl group, a C₁₋₆ alkyl group substituted by a C₂₋₇ alkoxycarbamoyl group, a C₁₋₆ alkyl group substituted by an N-(C₁₋₃ alkyl)-N-(C₁₋₃ alkoxy)carbamoyl group, a C₁₋₆ alkyl group substituted by a C₁₋₆ alkylamino group, a C₁₋₆ alkyl group substituted by a C₁₋₆ alkoxyamino group, a C₁₋₆ alkyl group substituted by a di(C₁₋₃ alkyl)amino group, a C₁₋₆ alkyl group substituted by an N-(C₁₋₃ alkyl)-N-(C₁₋₃ alkoxy)amino group, a C₁₋₆ alkyl group substituted by an N-(C₂₋₇ alkylcarbonyl)-N-(C₁₋₆ alkyl)amino group, a C₁₋₆ alkyl group substituted by an N-(C₂₋₇ alkylcarbonyl)-N-(C₁₋₆ alkoxy)amino group, a C₁₋₆ alkyl group substituted by an N-(C₁₋₆ alkylsulfonyl)-N-(C₁₋₆ alkyl)amino group, a C₁₋₆ alkyl group substituted by an N-(C₁₋₆ alkylsulfonyl)-N-(C₁₋₆ alkoxy)amino group, a phenyl group (provided that such a phenyl group may be substituted by one or more substituents selected from the group consisting of a halogen atom, a trifluoromethyl group, a nitro group, a C₁₋₆ alkyl group, a C₁₋₆ alkoxy group and a C₂₋₇ alkoxycarbonyl group), a C₁₋₆ alkyl group substituted by a phenyl group (provided that such a phenyl group may be substituted by one or more substituents selected from the group consisting of a halogen atom, a trifluoromethyl group, a nitro group, a C₁₋₆ alkyl group, a C₁₋₆ alkoxy group and a C₂₋₇ alkoxycarbonyl group), a C₂₋₇ alkenyl group substituted by a phenyl group (provided that such a phenyl group may be substituted by one or more substituents selected from the group consisting of a halogen atom, a trifluoromethyl group, a nitro group, a C₁₋₆ alkyl group, a C₁₋₆ alkoxy group and a C₂₋₇ alkoxycarbonyl group), a C₂₋₆ alkynyl group substituted by a phenyl group (provided that such a phenyl group may be substituted by one or more substituents selected from the group consisting of a halogen atom, a trifluoromethyl group, a nitro group, a C₁₋₆ alkyl group, a C₁₋₆ alkoxy group and a C₂₋₇ alkoxycarbonyl group), a C₁₋₆ alkyl group substituted by a phenoxy group (provided that such a phenoxy group may be substituted by one or more substituents selected from the group consisting of a halogen atom, a trifluoromethyl group, a nitro group, a C₁₋₆ alkyl group, a C₁₋₆ alkoxy group and a C₂₋₇ alkoxycarbonyl group), a C₁₋₆ alkyl group substituted by a phenylthio group (provided that such a phenylthio group may be substituted by one or more substituents selected from the group consisting of a halogen atom, a trifluoromethyl group, a nitro group, a C₁₋₆ alkyl group, a C₁₋₆ alkoxy group and a C₂₋₇ alkoxycarbonyl group), a C₁₋₆ alkyl group substituted by a phenylsulfinyl group (provided that such a phenylsulfinyl group may be substituted by one or more substituents selected from the group consisting of a halogen atom, a trifluoromethyl group, a nitro group, a C₁₋₆ alkyl group, a C₁₋₆ alkoxy group and a C₂₋₇ alkoxycarbonyl group), a C₁₋₆ alkyl group substituted by a phenylsulfonyl group (provided that such a phenylsulfonyl group may be substituted by one or more substituents selected from the group consisting of a halogen atom, a trifluoromethyl group, a nitro group, a C₁₋₆ alkyl group, a C₁₋₆ alkoxy group and a C₂₋₇ alkoxycarbonyl group), a C₁₋₆ alkyl group substituted by a benzyloxy group (provided that the phenyl group of such a benzyloxy group may be substituted by one or more substituents selected from the group consisting of a halogen atom, a trifluoromethyl group, a nitro group, a C₁₋₆ alkyl group, a C₁₋₆ alkoxy group and a C₂₋₇ alkoxycarbonyl group), a C₁₋₆ alkyl group substituted by a benzylthio group (provided that the phenyl group of such a benzylthio group may be substituted by one or more substituents selected from the group consisting of a halogen atom, a trifluoromethyl group, a nitro group, a C₁₋₆ alkyl group, a C₁₋₆ alkoxy group and a C₂₋₇ alkoxycarbonyl group), a C₁₋₆ alkyl group substituted by a benzylsulfinyl group (provided that the phenyl group of such a benzylsulfinyl group may be substituted by one or more substituents selected from the group consisting of a halogen atom, a trifluoromethyl group, a nitro group, a C₁₋₆ alkyl group, a C₁₋₆ alkoxy group and a C₂₋₇ alkoxycarbonyl group), a C₁₋₆ alkyl group substituted by a benzylsulfonyl group (provided that the phenyl group of such a benzylsulfonyl group may be substituted by one or more substituents selected from the group consisting of a halogen atom, a trifluoromethyl group, a nitro group, a C₁₋₆ alkyl group, a C₁₋₆ alkoxy group and a C₂₋₇ alkoxycarbonyl group), a C₁₋₆ alkyl group substituted by a phenylcarbonyl group (provided that such a phenylcarbonyl group may be substituted by one or more substituents selected from the group consisting of a halogen atom, a trifluoromethyl group, a nitro group, a C₁₋₆ alkyl group, a C₁₋₆ alkoxy group and a C₂₋₇ alkoxycarbonyl group), a C₁₋₆ alkyl group substituted by a benzylcarbonyl group (provided that the phenyl group of such a benzylcarbonyl group may be substituted by one or more substituents selected from the group consisting of a halogen atom, a trifluoromethyl group, a nitro group, a C₁₋₆ alkyl group, a C₁₋₆ alkoxy group and a C₂₋₇ alkoxycarbonyl group), a C₁₋₆ alkyl group substituted by an amino group substituted by a C₁₋₄ alkylsulfonyl group, or a C₁₋₆ alkyl group substituted by an amino group substituted by a C₂₋₄ alkylcarbonyl group,
each of R^{e2}, R^{e3}, R^{e6} and R^{e7} which are independent of one another, is a hydrogen atom, a C₁₋₆ alkyl group, a mono-, di- or poly-halogeno C₁₋₆ alkyl group, a C₁₋₆ alkoxy group, a mono-, di- or poly-halogeno C₁₋₆ alkoxy group, a C₁₋₆ alkylthio group, a C₁₋₆ alkylsulfinyl group, a C₁₋₆ alkylsulfonyl group, a C₂₋₇ alkoxycarbonyl group, a C₂₋₇ alkylcarbonyl group, a halogen atom, a nitro group, a cyano group, or a phenyl group (provided that such a phenyl group may be substituted by one or more substituents selected from the group consisting of a halogen atom, a trifluoromethyl group, a nitro group, a C₁₋₆ alkyl group, a C₁₋₆ alkoxy group and a C₂₋₇ alkoxycarbonyl group),
each of R^{e4}, R^{e5}, R^{e8}, R^{e9} and R^{e10} which are independent of one another, is a hydrogen atom, a C₁₋₈ alkyl group, a C₂₋₈ alkenyl group, a C₂₋₈ alkynyl group, or a phenyl group (provided that such a phenyl group may be substituted by one or more substituents selected from the group consisting of a halogen atom, a trifluoromethyl group, a nitro group, a C₁₋₆ alkyl group, a C₁₋₆ alkoxy group and a C₂₋₇ alkoxycarbonyl group),
R^{f1} is a C₁₋₈ alkyl group, a C₃₋₇ cycloalkyl group, a C₁₋₆ alkyl group substituted by a C₃₋₇ cycloalkyl group, a C₃₋₇ cycloalkenyl group, a C₁₋₆ alkyl group substituted by a C₃₋₇ cycloalkenyl group, a C₂₋₈ alkenyl group, a C₂₋₈ alkynyl group, a C₁₋₆ alkyl group substituted by a C₁₋₆ alkoxy group, a C₁₋₆ alkyl group substituted by a C₂₋₆ alkenyloxy group, a C₁₋₆ alkyl group substituted by a C₂₋₆ alkynyloxy group, a C₁₋₆ alkyl group substituted by a mono-, di- or poly-halogeno C₁₋₆ alkoxy group, a C₁₋₆ alkyl group substituted by a mono-, di- or poly-halogeno C₂₋₆ alkenyloxy group, a C₁₋₆ alkyl group substituted by a mono-, di- or poly-halogeno C₂₋₆ alkynyloxy group, a C₁₋₆ alkyl group substituted by a C₁₋₆ alkylthio group, a C₁₋₆ alkyl group substituted by a C₁₋₆ alkylsulfinyl group, a C₁₋₆ alkyl group substituted by a C₁₋₆ alkylsulfonyl group, a mono-, di- or poly-halogeno C₁₋₈ alkyl group, a mono-, di- or poly-halogeno C₂₋₈ alkenyl group, a mono-, di- or poly-halogeno C₂₋₈ alkynyl group, a C₁₋₆ alkyl group substituted by a cyano group, a C₂₋₆ alkenyl group substituted by a cyano group, a C₂₋₆ alkynyl group substituted by a cyano group, a C₁₋₆ alkyl group substituted by a nitro group, a C₂₋₆ alkenyl group substituted by a nitro group, a C₂₋₆ alkynyl group substituted by a nitro group, a C₁₋₆ alkyl group substituted by a C₂₋₇ alkoxycarbonyl group, a C₂₋₆ alkenyl group substituted by a C₂₋₇ alkoxycarbonyl group, a C₂₋₆ alkynyl group substituted by a C₂₋₇ alkoxycarbonyl group, a C₁₋₆ alkyl group substituted by a C₂₋₇ alkylcarbonyl group, a C₁₋₆ alkyl group substituted by a mono-, di- or poly-halogeno C₂₋₇ alkylcarbonyl group, a C₁₋₆ alkyl group substituted by a C₃₋₇ alkenylcarbonyl group, a C₁₋₆ alkyl group substituted by a C₃₋₇ alkynylcarbonyl group, a C₁₋₆ alkyl group substituted by a C₂₋₅ alkylcarbonyl group substituted by a C₁₋₄ alkoxy group, a C₁₋₆ alkyl group substituted by a C₂₋₅ alkylcarbonyl group substituted by a C₁₋₄ alkylthio group, a C₁₋₆ alkyl group substituted by a C₂₋₅ alkylcarbonyl group substituted by a C₁₋₄ alkylsulfinyl group, a C₁₋₆ alkyl group substituted by a C₂₋₅ alkylcarbonyl group substituted by a C₁₋₄ alkylsulfonyl group, a C₂₋₆ alkenyl group substituted by a C₂₋₇ alkylcarbonyl group, a C₂₋₆ alkynyl group substituted by a C₂₋₇ alkylcarbonyl group, a C₁₋₆ alkyl group substituted by a C₁₋₆ alkylsulfamoyl group, a C₁₋₆ alkyl group substituted by a C₁₋₆ alkoxysulfamoyl group, a C₁₋₆ alkyl group substituted by a di(C₁₋₃ alkyl)sulfamoyl group, a C₁₋₆ alkyl group substituted by an N-(C₁₋₃ alkyl)-N-(C₁₋₃ alkoxy)sulfamoyl group, a C₁₋₆ alkyl group substituted by a C₂₋₇ alkylcarbamoyl group, a C₁₋₆ alkyl group substituted by a di(C₁₋₃ alkyl)carbamoyl group, a C₁₋₆ alkyl group substituted by a C₂₋₇ alkoxycarbamoyl group, a C₁₋₆ alkyl group substituted by an N-(C₁₋₃ alkyl)-N-(C₁₋₃ alkoxy)carbamoyl group, a C₁₋₆ alkyl group substituted by a C₁₋₆ alkylamino group, a C₁₋₆ alkyl group substituted by a C₁₋₆ alkoxyamino group, a C₁₋₆ alkyl group substituted by a di(C₁₋₃ alkyl)amino group, a C₁₋₆ alkyl group substituted by an N-(C₁₋₃ alkyl)-N-(C₁₋₃ alkoxy)amino group, a C₁₋₆ alkyl group substituted by an N-(C₂₋₇ alkylcarbonyl)-N-(C₁₋₆ alkyl)amino group, a C₁₋₆ alkyl group substituted by an N-(C₂₋₇ alkylcarbonyl)-N-(C₁₋₆ alkoxy)amino group, a C₁₋₆ alkyl group substituted by an N-(C₁₋₆ alkylsulfonyl)-N-(C₁₋₆ alkyl)amino group, a C₁₋₆ alkyl group substituted by an N-(C₁₋₆ alkylsulfonyl)-N-(C₁₋₆ alkoxy)amino group, a phenyl group (provided that such a phenyl group may be substituted by one or more substituents selected from the group consisting of a halogen atom, a trifluoromethyl group, a nitro group, a C₁₋₆ alkyl group, a C₁₋₆ alkoxy group and a C₂₋₇ alkoxycarbonyl group), a C₁₋₆ alkyl group substituted by a phenyl group (provided that such a phenyl group may be substituted by one or more substituents selected from the group consisting of a halogen atom, a trifluoromethyl group, a nitro group, a C₁₋₆ alkyl group, a C₁₋₆ alkoxy group and a C₂₋₇ alkoxycarbonyl group), a C₂₋₇ alkenyl group substituted by a phenyl group (provided that such a phenyl group may be substituted by one or more substituents selected from the group consisting of a halogen atom, a trifluoromethyl group, a nitro group, a C₁₋₆ alkyl group, a C₁₋₆ alkoxy group and a C₂₋₇ alkoxycarbonyl group), a C₂₋₆ alkynyl group substituted by a phenyl group (provided that such a phenyl group may be substituted by one or more substituents selected from the group consisting of a halogen atom, a trifluoromethyl group, a nitro group, a C₁₋₆ alkyl group, a C₁₋₆ alkoxy group and a C₂₋₇ alkoxycarbonyl group), a C₁₋₆ alkyl group substituted by a phenoxy group (provided that such a phenoxy group may be substituted by one or more substituents selected from the group consisting of a halogen atom, a trifluoromethyl group, a nitro group, a C₁₋₆ alkyl group, a C₁₋₆ alkoxy group and a C₂₋₇ alkoxycarbonyl group), a C₁₋₆ alkyl group substituted by a phenylthio group (provided that such a phenylthio group may be substituted by one or more substituents selected from the group consisting of a halogen atom, a trifluoromethyl group, a nitro group, a C₁₋₆ alkyl group, a C₁₋₆ alkoxy group and a C₂₋₇ alkoxycarbonyl group), a C₁₋₆ alkyl group substituted by a phenylsulfinyl group (provided that such a phenylsulfinyl group may be substituted by one or more substituents selected from the group consisting of a halogen atom, a trifluoromethyl group, a nitro group, a C₁₋₆ alkyl group, a C₁₋₆ alkoxy group and a C₂₋₇ alkoxycarbonyl group), a C₁₋₆ alkyl group substituted by a phenylsulfonyl group (provided that such a phenylsulfonyl group may be substituted by one or more substituents selected from the group consisting of a halogen atom, a trifluoromethyl group, a nitro group, a C₁₋₆ alkyl group, a C₁₋₆ alkoxy group and a C₂₋₇ alkoxycarbonyl group), a C₁₋₆ alkyl group substituted by a benzyloxy group (provided that the phenyl group of such a benzyloxy group may be substituted by one or more substituents selected from the group consisting of a halogen atom, a trifluoromethyl group, a nitro group, a C₁₋₆ alkyl group, a C₁₋₆ alkoxy group and a C₂₋₇ alkoxycarbonyl group), a C₁₋₆ alkyl group substituted by a benzylthio group (provided that the phenyl group of such a benzylthio group may be substituted by one or more substituents selected from the group consisting of a halogen atom, a trifluoromethyl group, a nitro group, a C₁₋₆ alkyl group, a C₁₋₆ alkoxy group and a C₂₋₇ alkoxycarbonyl group), a C₁₋₆ alkyl group substituted by a benzylsulfinyl group (provided that the phenyl group of such a benzylsulfinyl group may be substituted by one or more substituents selected from the group consisting of a halogen atom, a trifluoromethyl group, a nitro group, a C₁₋₆ alkyl group, a C₁₋₆ alkoxy group and a C₂₋₇ alkoxycarbonyl group), a C₁₋₆ alkyl group substituted by a benzylsulfonyl group (provided that the phenyl group of such a benzylsulfonyl group may be substituted by one or more substituents selected from the group consisting of a halogen atom, a trifluoromethyl group, a nitro group, a C₁₋₆ alkyl group, a C₁₋₆ alkoxy group and a C₂₋₇ alkoxycarbonyl group), a C₁₋₆ alkyl group substituted by a phenylcarbonyl group (provided that such a phenylcarbonyl group may be substituted by one or more substituents selected from the group consisting of a halogen atom, a trifluoromethyl group, a nitro group, a C₁₋₆ alkyl group, a C₁₋₆ alkoxy group and a C₂₋₇ alkoxycarbonyl group), a C₁₋₆ alkyl group substituted by a benzylcarbonyl group (provided that the phenyl group of such a benzylcarbonyl group may be substituted by one or more substituents selected from the group consisting of a halogen atom, a trifluoromethyl group, a nitro group, a C₁₋₆ alkyl group, a C₁₋₆ alkoxy group and a C₂₋₇ alkoxycarbonyl group), a C₁₋₆ alkyl group substituted by an amino group substituted by a C₁₋₄ alkylsulfonyl group, or a C₁₋₆ alkyl group substituted by an amino group substituted by a C₂₋₄ alkylcarbonyl group,
R^{f2} is a C₁₋₈ alkyl group, a C₂₋₈ alkenyl group, a C₂₋₈ alkynyl group, a C₁₋₆ alkyl group substituted by a C₁₋₆ alkylthio group, a C₁₋₆ alkyl group substituted by a C₁₋₆ alkylsulfinyl group, a C₁₋₆ alkyl group substituted by a C₁₋₆ alkylsulfonyl group, a C₁₋₆ alkyl group substituted by a C₁₋₆ alkoxy group, a C₁₋₆ alkyl group substituted by a C₂₋₇ alkoxycarbonyl group, a C₁₋₆ alkyl group substituted by a C₂₋₇ alkylcarbonyl group, a C₁₋₆ alkyl group substituted by a cyano group, a phenyl group (provided that such a phenyl group may be substituted by one or more substituents selected from the group consisting of a halogen atom, a trifluoromethyl group, a nitro group, a C₁₋₆ alkyl group, a C₁₋₆ alkoxy group and a C₂₋₇ alkoxycarbonyl group), or a C₁₋₆ alkyl group substituted by a phenyl group (provided that such a phenyl group may be substituted by one or more substituents selected from the group consisting of a halogen atom, a trifluoromethyl group, a nitro group, a C₁₋₆ alkyl group, a C₁₋₆ alkoxy group and a C₂₋₇ alkoxycarbonyl group),
each of R^{f3}, R^{f4}, R^{f7}, R^{f8}, R^{f11} and R^{f12} which are independent of one another, is a hydrogen atom, a C₁₋₆ alkyl group, a mono-, di- or poly-halogeno C₁₋₆ alkyl group, a C₁₋₆ alkoxy group, a mono-, di- or poly-halogeno C₁₋₆ alkoxy group, a C₁₋₆ alkylthio group, a C₁₋₆ alkylsulfinyl group, a C₁₋₆ alkylsulfonyl group, a C₂₋₇ alkoxycarbonyl group, a C₂₋₇ alkylcarbonyl group, a halogen atom, a nitro group, a cyano group, or a phenyl group (provided that such a phenyl group may be substituted by one or more substituents selected from the group consisting of a halogen atom, a trifluoromethyl group, a nitro group, a C₁₋₆ alkyl group, a C₁₋₆ alkoxy group and a C₂₋₇ alkoxycarbonyl group),
each of R^{f5}, R^{f6}, R^{f9}, R^{f10}, R^{f13}, R^{f14} and R^{f15} which are independent of one another, is a hydrogen atom, a C₁₋₈ alkyl group, a C₂₋₈ alkenyl group, a C₂₋₈ alkynyl group, or a phenyl group (provided that such a phenyl group may be substituted by one or more substituents selected from the group consisting of a halogen atom, a trifluoromethyl group, a nitro group, a C₁₋₆ alkyl group, a C₁₋₆ alkoxy group and a C₂₋₇ alkoxycarbonyl group),
R^{g1} is a C₁₋₈ alkyl group, a C₃₋₇ cycloalkyl group, a C₁₋₆ alkyl group substituted by a C₃₋₇ cycloalkyl group, a C₃₋₇ cycloalkenyl group, a C₁₋₆ alkyl group substituted by a C₃₋₇ cycloalkenyl group, a C₂₋₈ alkenyl group, a C₂₋₈ alkynyl group, a C₁₋₆ alkyl group substituted by a C₁₋₆ alkoxy group, a C₁₋₆ alkyl group substituted by a C₂₋₆ alkenyloxy group, a C₁₋₆ alkyl group substituted by a C₂₋₆ alkynyloxy group, a C₁₋₆ alkyl group substituted by a mono-, di- or poly-halogeno C₁₋₆ alkoxy group, a C₁₋₆ alkyl group substituted by a mono-, di- or poly-halogeno C₂₋₆ alkenyloxy group, a C₁₋₆ alkyl group substituted by a mono-, di- or poly-halogeno C₂₋₆ alkynyloxy group, a C₁₋₆ alkyl group substituted by a C₁₋₆ alkylthio group, a C₁₋₆ alkyl group substituted by a C₁₋₆ alkylsulfinyl group, a C₁₋₆ alkyl group substituted by a C₁₋₆ alkylsulfonyl group, a mono-, di- or poly-halogeno C₁₋₈ alkyl group, a mono-, di- or poly-halogeno C₂₋₈ alkenyl group, a mono-, di- or poly-halogeno C₂₋₈ alkynyl group, a C₁₋₆ alkyl group substituted by a cyano group, a C₂₋₆ alkenyl group substituted by a cyano group, a C₂₋₆ alkynyl group substituted by a cyano group, a C₁₋₆ alkyl group substituted by a nitro group, a C₂₋₆ alkenyl group substituted by a nitro group, a C₂₋₆ alkynyl group substituted by a nitro group, a C₁₋₆ alkyl group substituted by a C₂₋₇ alkoxycarbonyl group, a C₂₋₆ alkenyl group substituted by a C₂₋₇ alkoxycarbonyl group, a C₂₋₆ alkynyl group substituted by a C₂₋₇ alkoxycarbonyl group, a C₁₋₆ alkyl group substituted by a C₂₋₇ alkylcarbonyl group, a C₁₋₆ alkyl group substituted by a mono-, di- or poly-halogeno C₂₋₇ alkylcarbonyl group, a C₁₋₆ alkyl group substituted by a C₃₋₇ alkenylcarbonyl group, a C₁₋₆ alkyl group substituted by a C₃₋₇ alkynylcarbonyl group, a C₁₋₆ alkyl group substituted by a C₂₋₅ alkylcarbonyl group substituted by a C₁₋₄ alkoxy group, a C₁₋₆ alkyl group substituted by a C₂₋₅ alkylcarbonyl group substituted by a C₁₋₄ alkylthio group, a C₁₋₆ alkyl group substituted by a C₂₋₅ alkylcarbonyl group substituted by a C₁₋₄ alkylsulfinyl group, a C₁₋₆ alkyl group substituted by a C₂₋₅ alkylcarbonyl group substituted by a C₁₋₄ alkylsulfonyl group, a C₂₋₆ alkenyl group substituted by a C₂₋₇ alkylcarbonyl group, a C₂₋₆ alkynyl group substituted by a C₂₋₇ alkylcarbonyl group, a C₁₋₆ alkyl group substituted by a C₁₋₆ alkylsulfamoyl group, a C₁₋₆ alkyl group substituted by a C₁₋₆ alkoxysulfamoyl group, a C₁₋₆ alkyl group substituted by a di(C₁₋₃ alkyl)sulfamoyl group, a C₁₋₆ alkyl group substituted by an N-(C₁₋₃ alkyl)-N-(C₁₋₃ alkoxy)sulfamoyl group, a C₁₋₆ alkyl group substituted by a C₂₋₇ alkylcarbamoyl group, a C₁₋₆ alkyl group substituted by a di(C₁₋₃ alkyl)carbamoyl group, a C₁₋₆ alkyl group substituted by a C₂₋₇ alkoxycarbamoyl group, a C₁₋₆ alkyl group substituted by an N-(C₁₋₃ alkyl)-N-(C₁₋₃ alkoxy)carbamoyl group, a C₁₋₆ alkyl group substituted by a C₁₋₆ alkylamino group, a C₁₋₆ alkyl group substituted by a C₁₋₆ alkoxyamino group, a C₁₋₆ alkyl group substituted by a di(C₁₋₃ alkyl)amino group, a C₁₋₆ alkyl group substituted by an N-(C₁₋₃ alkyl)-N-(C₁₋₃ alkoxy)amino group, a C₁₋₆ alkyl group substituted by an N-(C₂₋₇ alkylcarbonyl)-N-(C₁₋₆ alkyl)amino group, a C₁₋₆ alkyl group substituted by an N-(C₂₋₇ alkylcarbonyl)-N-(C₁₋₆ alkoxy)amino group, a C₁₋₆ alkyl group substituted by an N-(C₁₋₆ alkylsulfonyl)-N-(C₁₋₆ alkyl)amino group, a C₁₋₆ alkyl group substituted by an N-(C₁₋₆ alkylsulfonyl)-N-(C₁₋₆ alkoxy)amino group, a C₁₋₆ alkyl group substituted by a phenyl group (provided that such a phenyl group may be substituted by one or more substituents selected from the group consisting of a halogen atom, a trifluoromethyl group, a nitro group, a C₁₋₆ alkyl group, a C₁₋₆ alkoxy group and a C₂₋₇ alkoxycarbonyl group), a C₂₋₇ alkenyl group substituted by a phenyl group (provided that such a phenyl group may be substituted by one or more substituents selected from the group consisting of a halogen atom, a trifluoromethyl group, a nitro group, a C₁₋₆ alkyl group, a C₁₋₆ alkoxy group and a C₂₋₇ alkoxycarbonyl group), a C₂₋₆ alkynyl group substituted by a phenyl group (provided that such a phenyl group may be substituted by one or more substituents selected from the group consisting of a halogen atom, a trifluoromethyl group, a nitro group, a C₁₋₆ alkyl group, a C₁₋₆ alkoxy group and a C₂₋₇ alkoxycarbonyl group), a C₁₋₆ alkyl group substituted by a phenoxy group (provided that such a phenoxy group may be substituted by one or more substituents selected from the group consisting of a halogen atom, a trifluoromethyl group, a nitro group, a C₁₋₆ alkyl group, a C₁₋₆ alkoxy group and a C₂₋₇ alkoxycarbonyl group), a C₁₋₆ alkyl group substituted by a phenylthio group (provided that such a phenylthio group may be substituted by one or more substituents selected from the group consisting of a halogen atom, a trifluoromethyl group, a nitro group, a C₁₋₆ alkyl group, a C₁₋₆ alkoxy group and a C₂₋₇ alkoxycarbonyl group), a C₁₋₆ alkyl group substituted by a phenylsulfinyl group (provided that such a phenylsulfinyl group may be substituted by one or more substituents selected from the group consisting of a halogen atom, a trifluoromethyl group, a nitro group, a C₁₋₆ alkyl group, a C₁₋₆ alkoxy group and a C₂₋₇ alkoxycarbonyl group), a C₁₋₆ alkyl group substituted by a phenylsulfonyl group (provided that such a phenylsulfonyl group may be substituted by one or more substituents selected from the group consisting of a halogen atom, a trifluoromethyl group, a nitro group, a C₁₋₆ alkyl group, a C₁₋₆ alkoxy group and a C₂₋₇ alkoxycarbonyl group), a C₁₋₆ alkyl group substituted by a benzyloxy group (provided that the phenyl group of such a benzyloxy group may be substituted by one or more substituents selected from the group consisting of a halogen atom, a trifluoromethyl group, a nitro group, a C₁₋₆ alkyl group, a C₁₋₆ alkoxy group and a C₂₋₇ alkoxycarbonyl group), a C₁₋₆ alkyl group substituted by a benzylthio group (provided that the phenyl group of such a benzylthio group may be substituted by one or more substituents selected from the group consisting of a halogen atom, a trifluoromethyl group, a nitro group, a C₁₋₆ alkyl group, a C₁₋₆ alkoxy group and a C₂₋₇ alkoxycarbonyl group), a C₁₋₆ alkyl group substituted by a benzylsulfinyl group (provided that the phenyl group of such a benzylsulfinyl group may be substituted by one or more substituents selected from the group consisting of a halogen atom, a trifluoromethyl group, a nitro group, a C₁₋₆ alkyl group, a C₁₋₆ alkoxy group and a C₂₋₇ alkoxycarbonyl group), a C₁₋₆ alkyl group substituted by a benzylsulfonyl group (provided that the phenyl group of such a benzylsulfonyl group may be substituted by one or more substituents selected from the group consisting of a halogen atom, a trifluoromethyl group, a nitro group, a C₁₋₆ alkyl group, a C₁₋₆ alkoxy group and a C₂₋₇ alkoxycarbonyl group), a C₁₋₆ alkyl group substituted by a phenylcarbonyl group (provided that such a phenylcarbonyl group may be substituted by one or more substituents selected from the group consisting of a halogen atom, a trifluoromethyl group, a nitro group, a C₁₋₆ alkyl group, a C₁₋₆ alkoxy group and a C₂₋₇ alkoxycarbonyl group), or a C₁₋₆ alkyl group substituted by a benzylcarbonyl group (provided that the phenyl group of such a benzylcarbonyl group may be substituted by one or more substituents selected from the group consisting of a halogen atom, a trifluoromethyl group, a nitro group, a C₁₋₆ alkyl group, a C₁₋₆ alkoxy group and a C₂₋₇ alkoxycarbonyl group),
each of R^{g2} and R^{g3} which are independent of each other, is a C₁₋₆ alkyl group, a C₂₋₆ alkenyl group, a C₂₋₆ alkynyl group, a mono-, di- or poly-halogeno C₁₋₆ alkyl group, a C₁₋₆ alkoxy group, a C₁₋₆ alkylsulfonyl group, a C₁₋₆ alkylsulfamoyl group, a di(C₁₋₃ alkyl)sulfamoyl group, a C₂₋₇ alkoxycarbonyl group, a C₂₋₇ alkylcarbonyl group, a C₂₋₇ alkylcarbamoyl group, a di(C₁₋₃ alkyl)carbamoyl group, a phenyl group (provided that such a phenyl group may be substituted by one or more substituents selected from the group consisting of a halogen atom, a trifluoromethyl group, a nitro group, a C₁₋₆ alkyl group, a C₁₋₆ alkoxy group and a C₂₋₇ alkoxycarbonyl group), or a benzyl group (provided that the phenyl group of such a benzyl group may be substituted by one or more substituents selected from the group consisting of a halogen atom, a trifluoromethyl group, a nitro group, a C₁₋₆ alkyl group, a C₁₋₆ alkoxy group and a C₂₋₇ alkoxycarbonyl group),
or R^{g2} and R^{g3} form a saturated 3- to 7-membered heterocyclic ring together with the nitrogen atom to which they are bonded,
X is an oxygen atom or a sulfur atom,
L is a hydrogen atom, a C₁₋₆ alkyl group, a C₂₋₆ alkenyl group or a C₂₋₆ alkynyl group,
G is 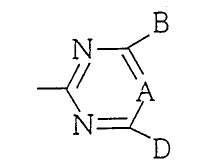
A is a CH group, or a nitrogen atom, and
each of B and D which are independent of each other, is a C₁₋₄ alkyl group, a C₁₋₄ alkoxy group, a mono-, di- or poly-halogeno C₁₋₄ alkyl group, a mono-, di- or poly-halogeno C₁₋₄ alkoxy group, a halogen atom, a C₁₋₄ alkylamino group, or a di(C₁₋₄ alkyl)amino group, (hereinafter referred to as the compound of the present invention) exhibits remarkably strong herbicidal activities against many weeds in soil treatment, in soil admixing treatment or in foliage treatment and at the same time has a high level of safety for important crop plants such as wheat, corn, cotton, soybean, sugar beet and rice, etc. The present invention has been accomplished on the basis of this discovery.

Examples for the substituents R^{a1}, R^{a2}, R^{a3}, R^{a4}, R^{a5}, R^{a6}, R^{b1}, R^{b2}, R^{b3}, R^{b4}, R^{b5}, R^{c1}, R^{c2}, R^{c3}, R^{c4}, R^{c5}, R^{c6}, R^{c7}, R^{c8}, R^{c9}, R^{c10}, R^{c11}, R^{c12}, R^{c13}, R^{c14}, R^{d1}, R^{d2}, R^{d3}, R^{d4}, R^{d5}, R^{d6}, R^{e1}, R^{e2}, R^{e3}, R^{e4}, R^{e5}, R^{e6}, R^{e7}, R^{e8}, R^{e9}, R^{e10}, R^{f1}, R^{f2}, R^{f3}, R^{f4}, R^{f5}, R^{f6}, R^{f7}, R^{f8}, R^{f9}, R^{f10}, R^{f11}, R^{f12}, R^{f13}, R^{f14}, R^{f15}, R^{g1}, R^{g2}, R^{g3} L, B and D of the compound of the present invention are as follows. However, symbols have the following meanings.

Me: methyl group, Et: ethyl group, Pr-n: n-propyl group, Pr-iso: isopropyl group, Bu-n: n-butyl group, Bu-iso: isobutyl group, Bu-sec: sec-butyl group, Bu-tert: tert-butyl group, Pen-n: n-pentyl grup, Hex-n: n-hexyl group, Hep-n: n-heptyl group, Pr-cyc: cyclopropyl group, Bu-cyc: cyclobutyl group, Pen-cyc: cyclopentyl group, Hex-cyc: cyclohexyl group, and Ph: phenyl group.

### Specific examples for the substituents R^{a1}, R^{b1}, R^{d1}, R^{e1} and R^{f1} of the compound of the present invention

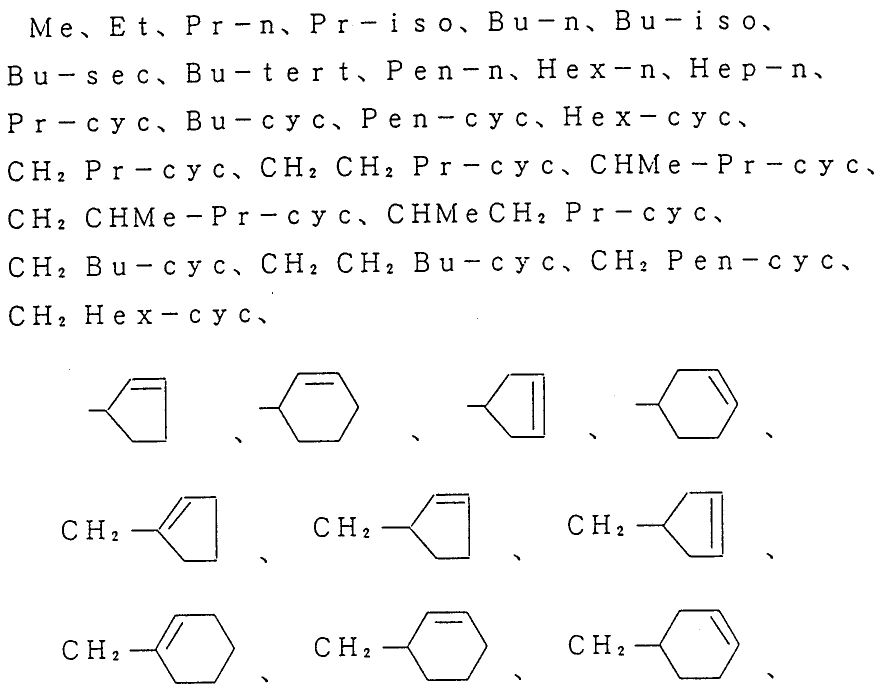

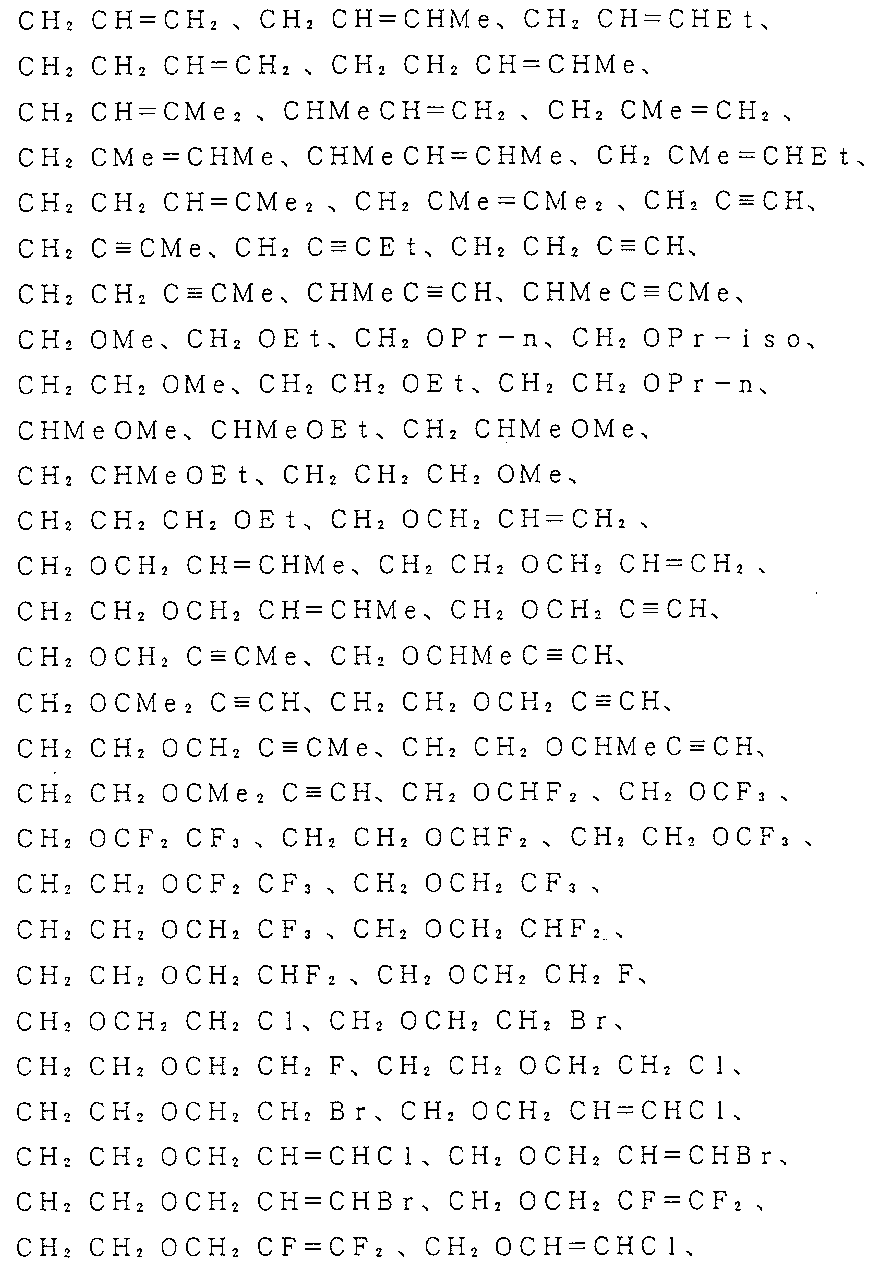

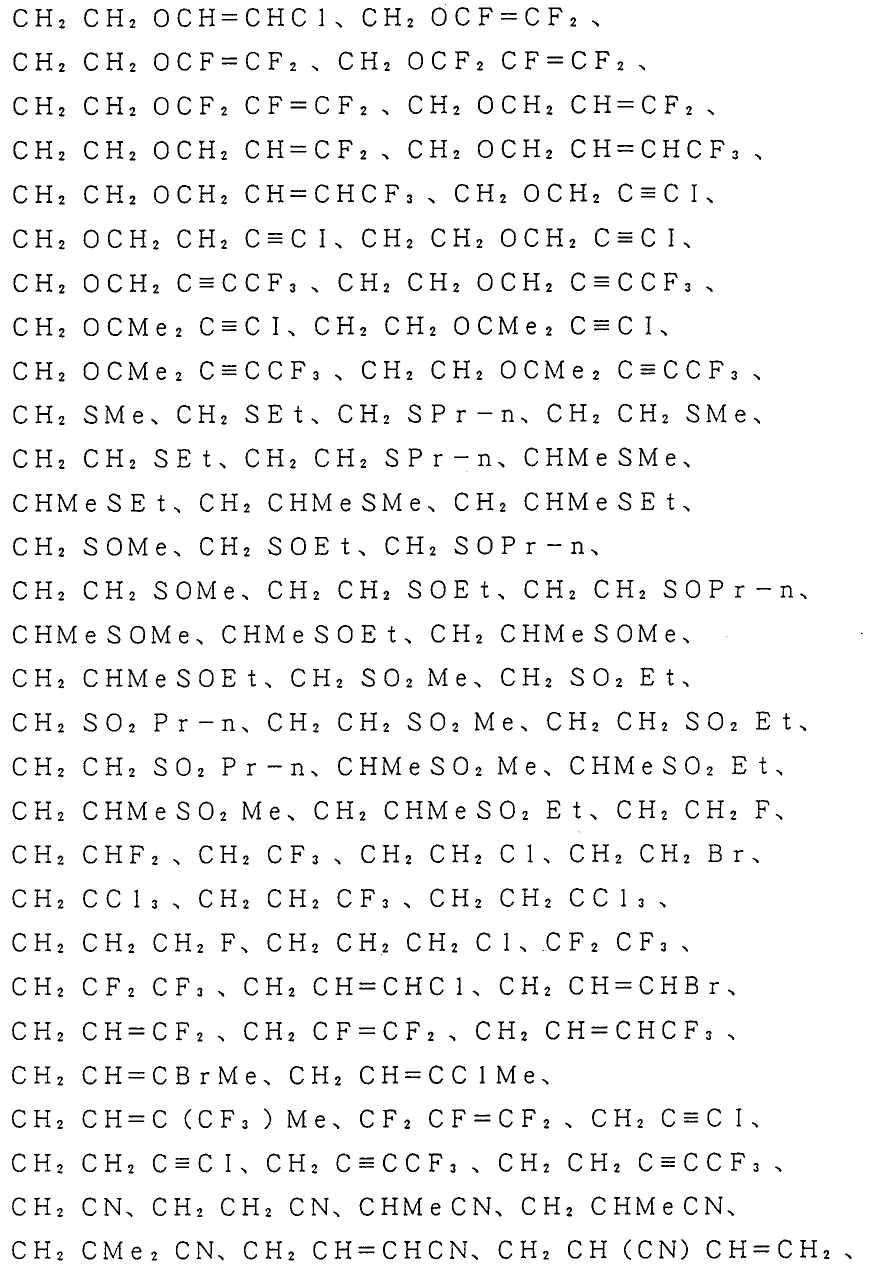

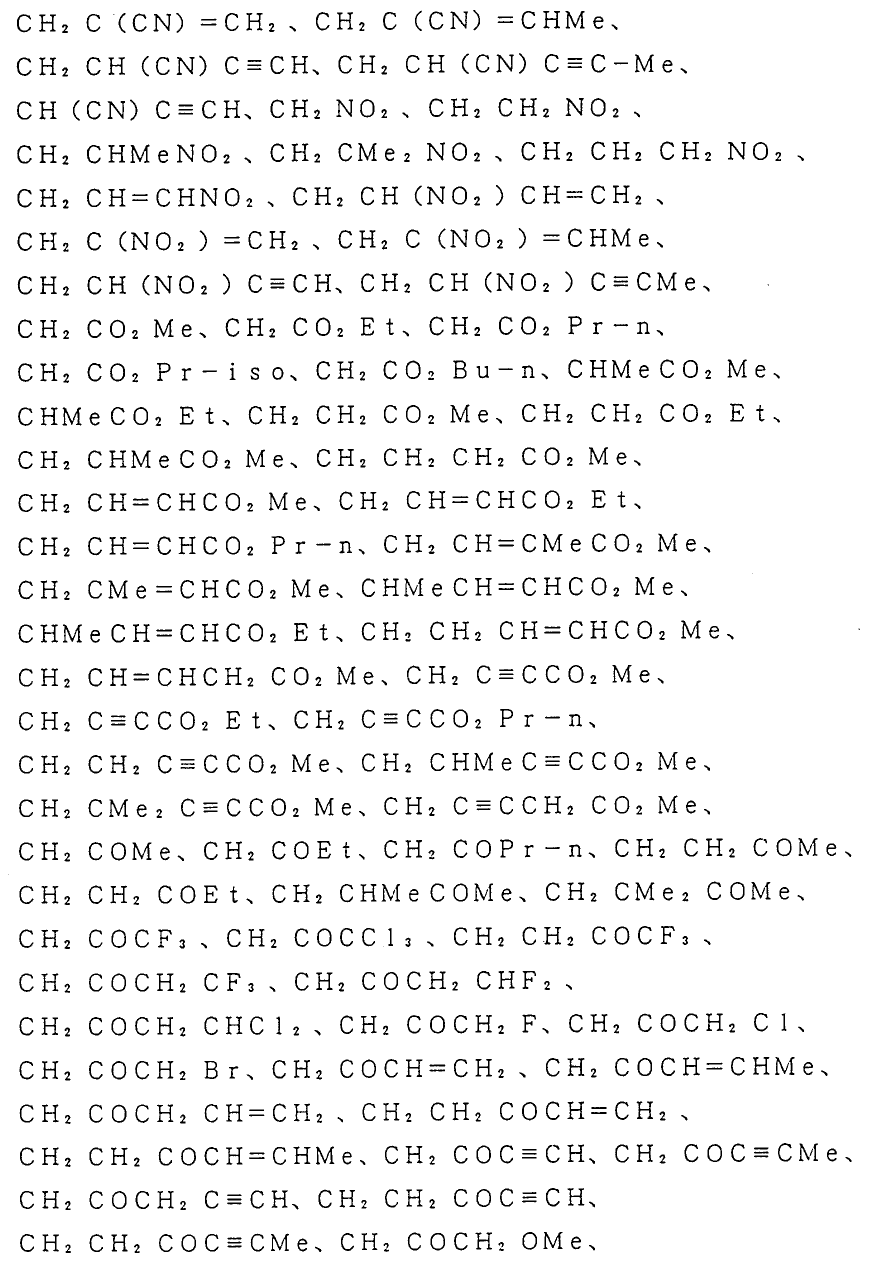

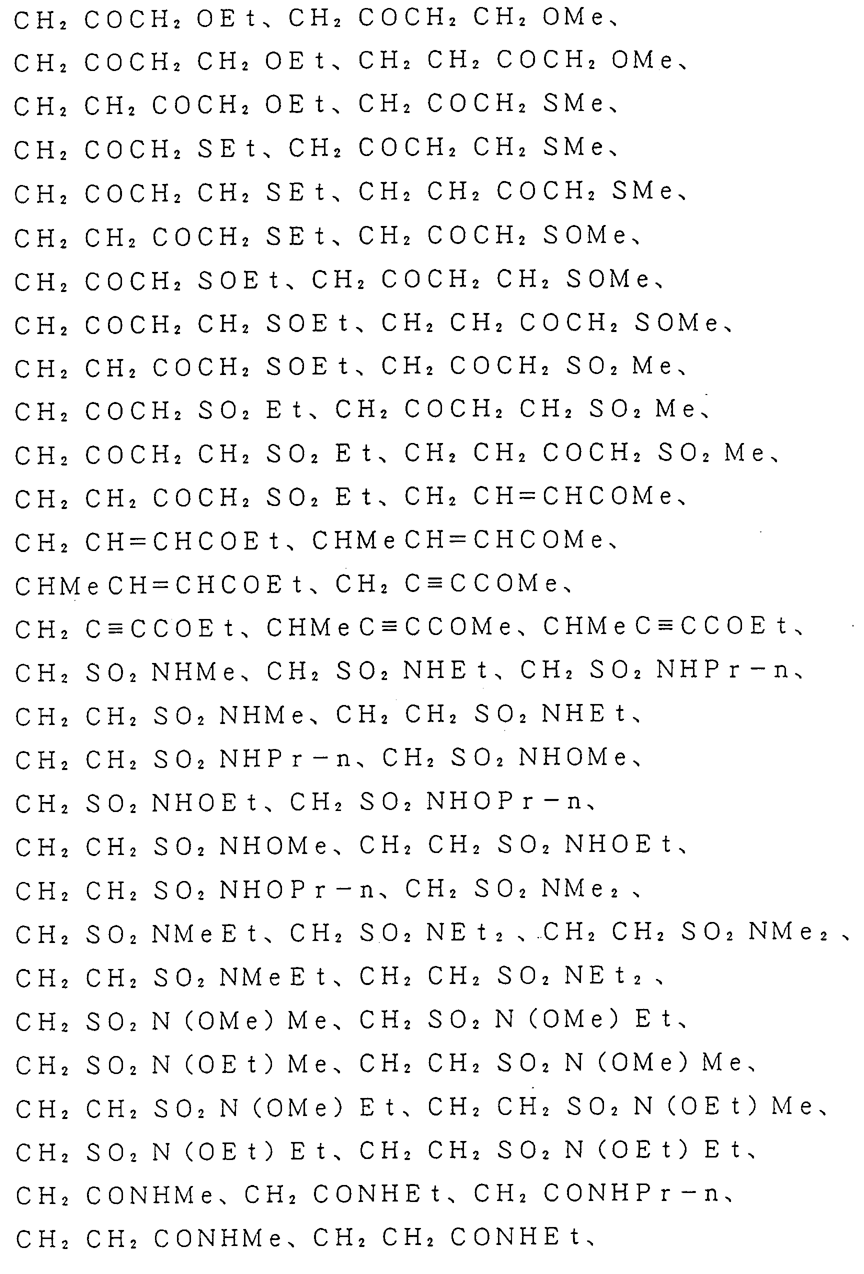

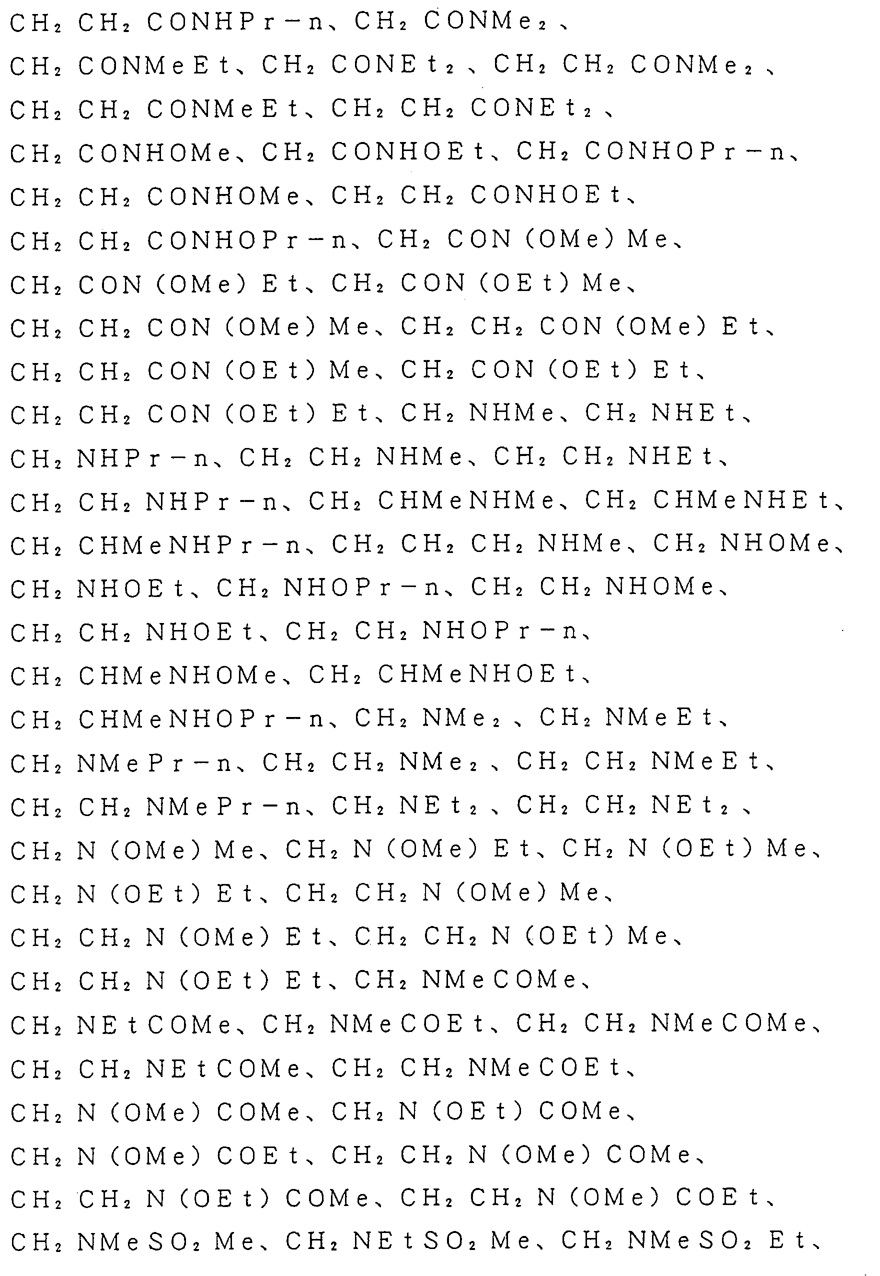

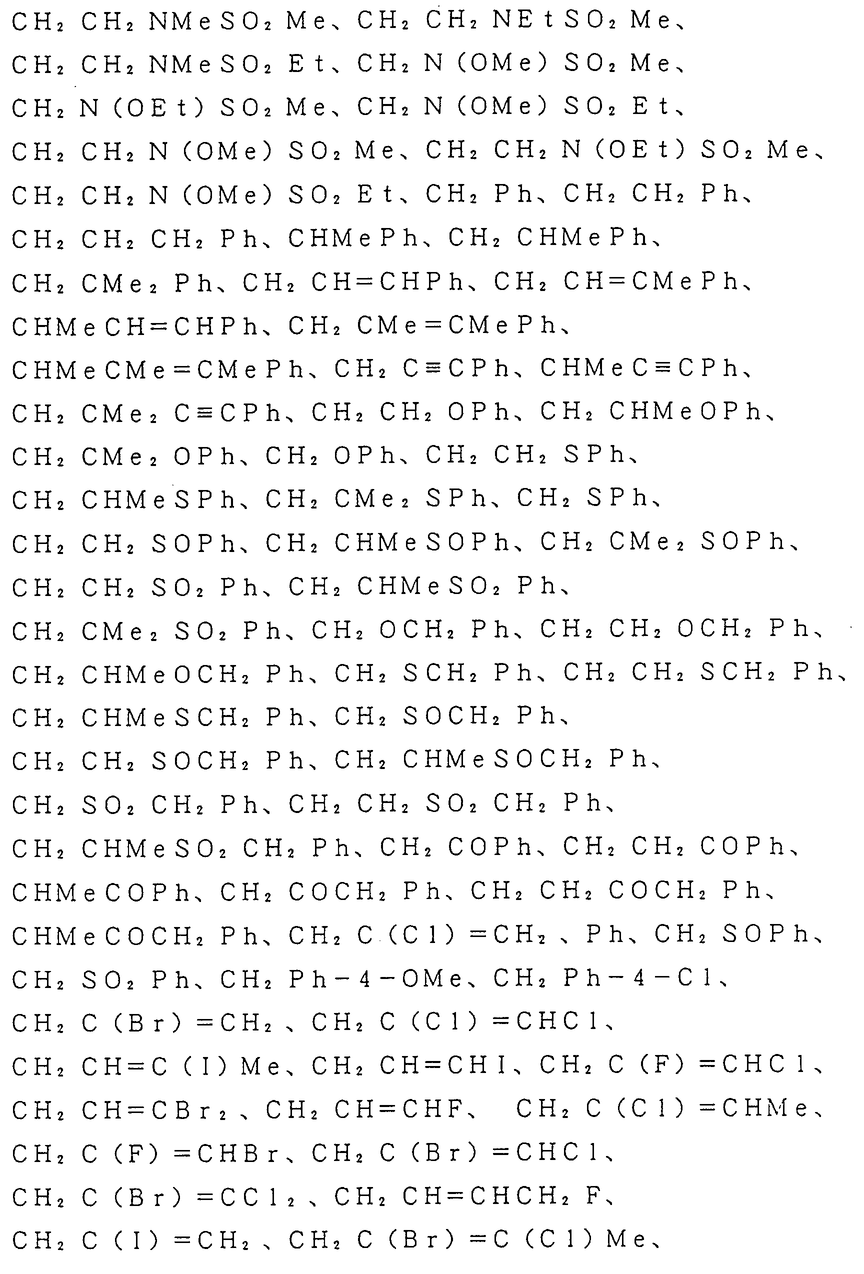

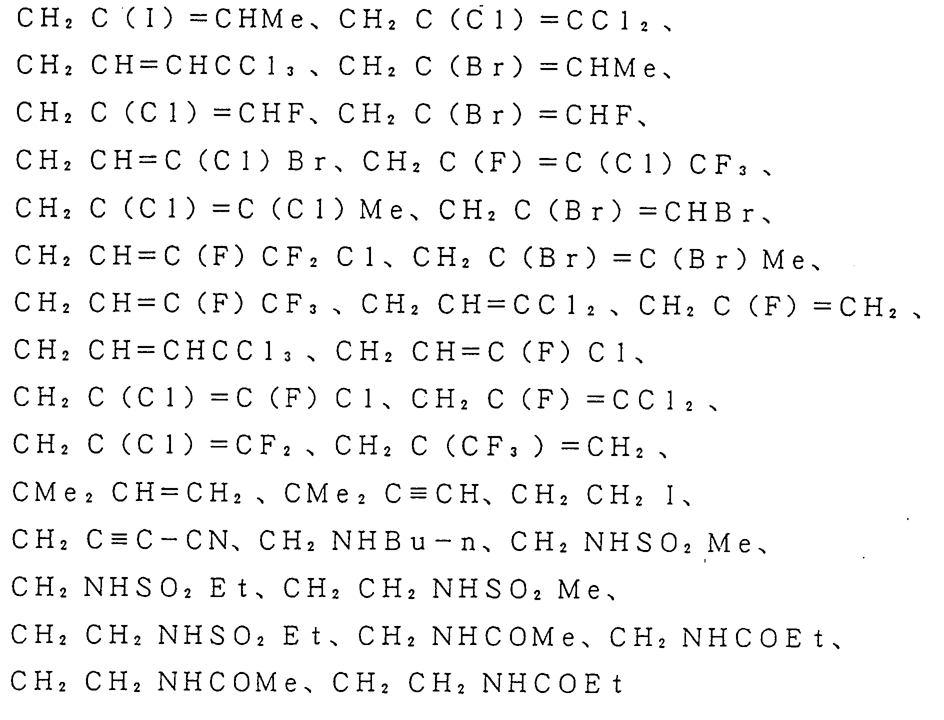

### Specific examples for the substituents R^{a2} and R^{a3} of the compound of the present invention

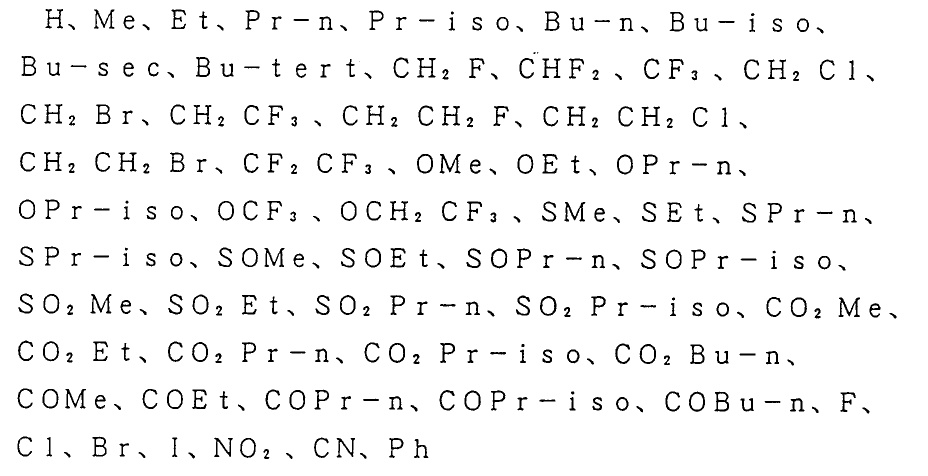

### Specific examples for the substituents R^{a4} and R^{a5} of the compound of the present invention

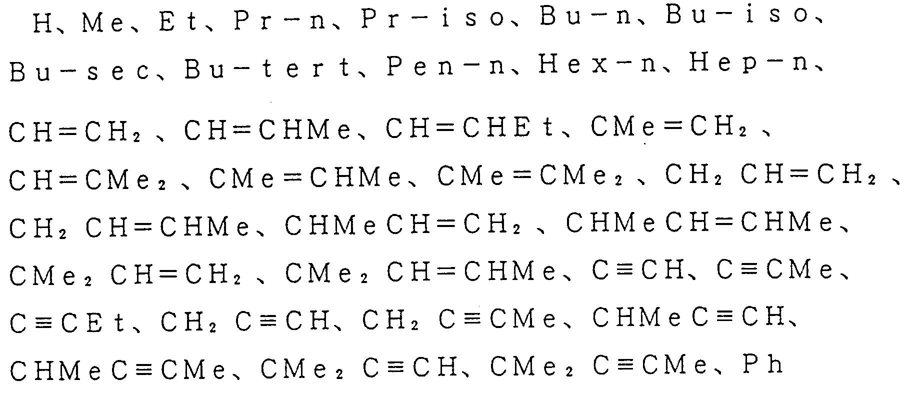

### Specific examples for the substituent R^{a6} of the compound of the present invention

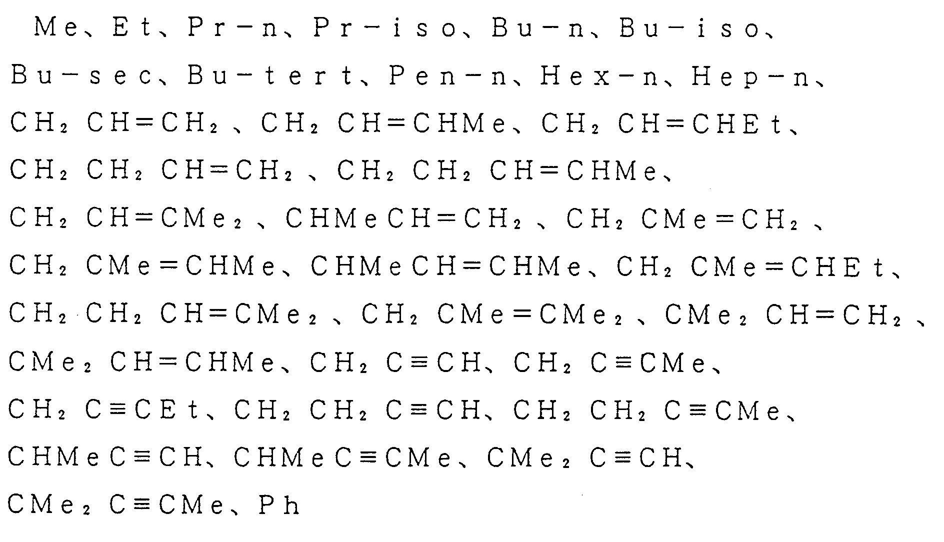

### Specific examples for the substituent R^{b2} of the compound of the present invention

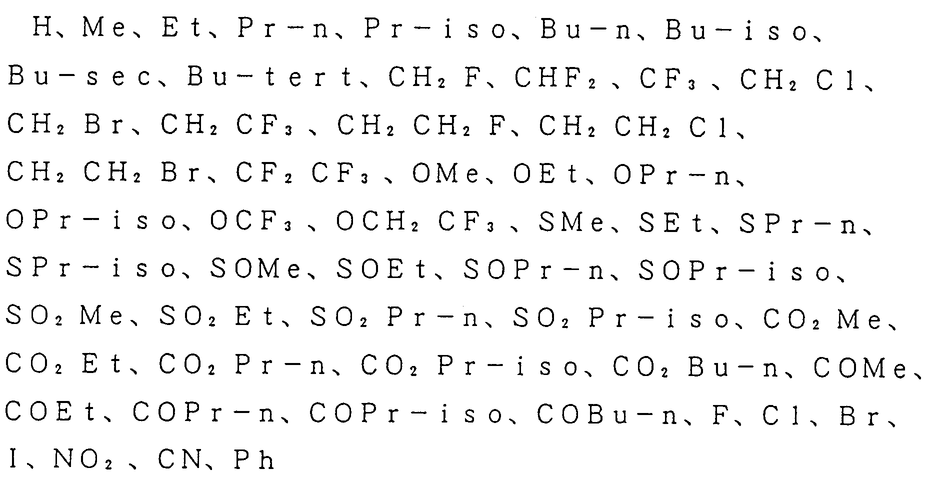

### Specific examples for the substituent R^{b3} of the compound of the present invention

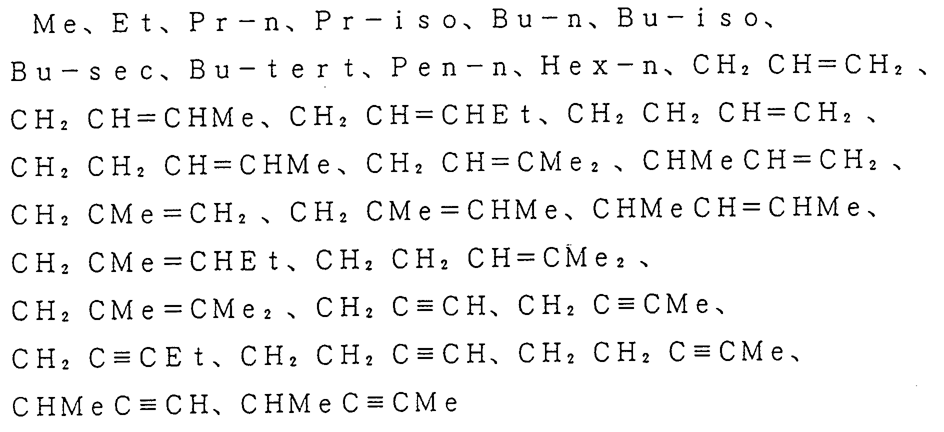

### Specific examples for the substituent R^{b4} of the compound of the present invention

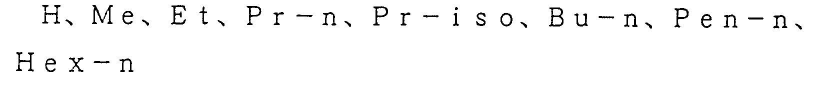

### Specific examples for the substituent R^{b5} of the compound of the present invention

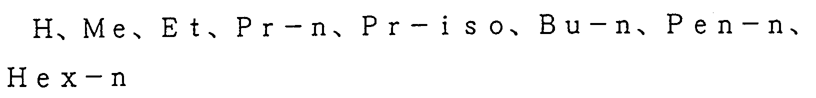

### Specific examples for the substituent R^{c1} of the compound of the present invention

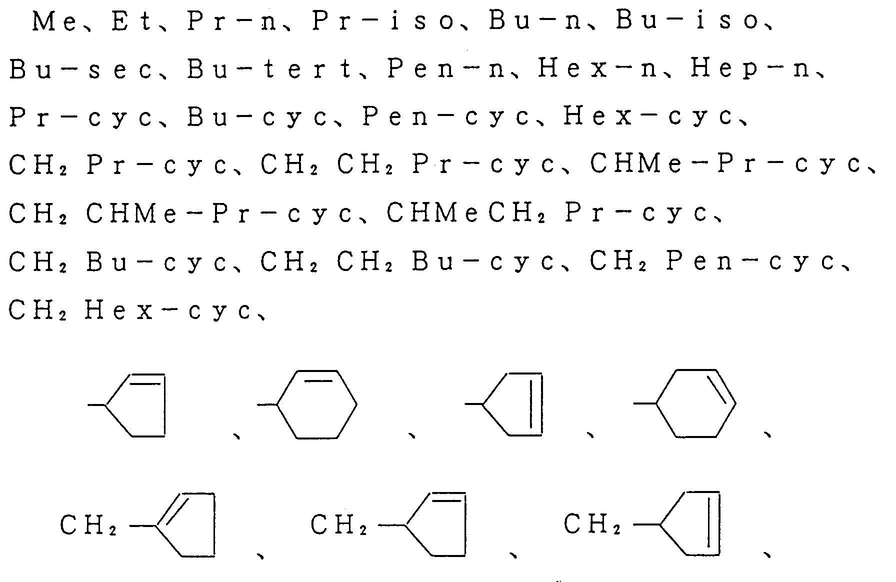

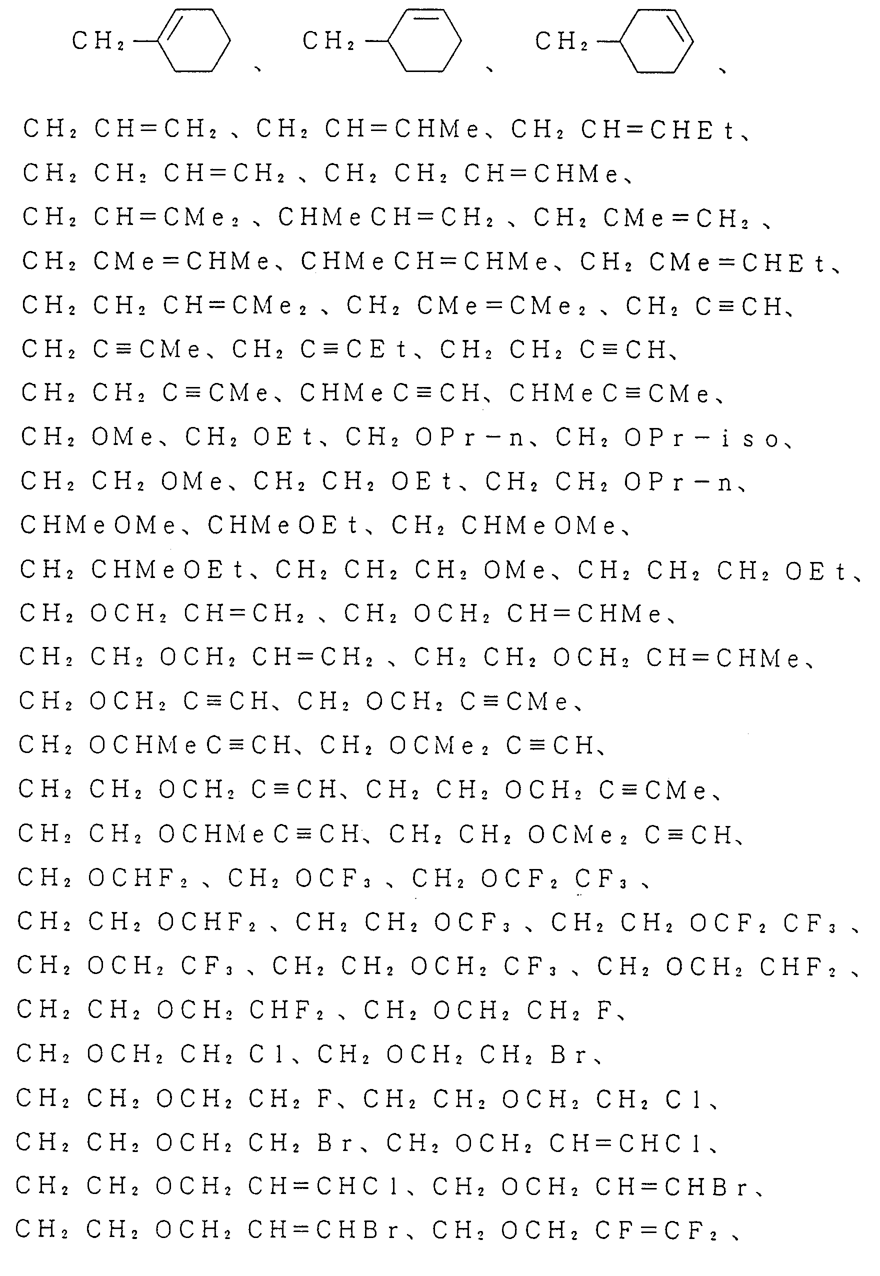

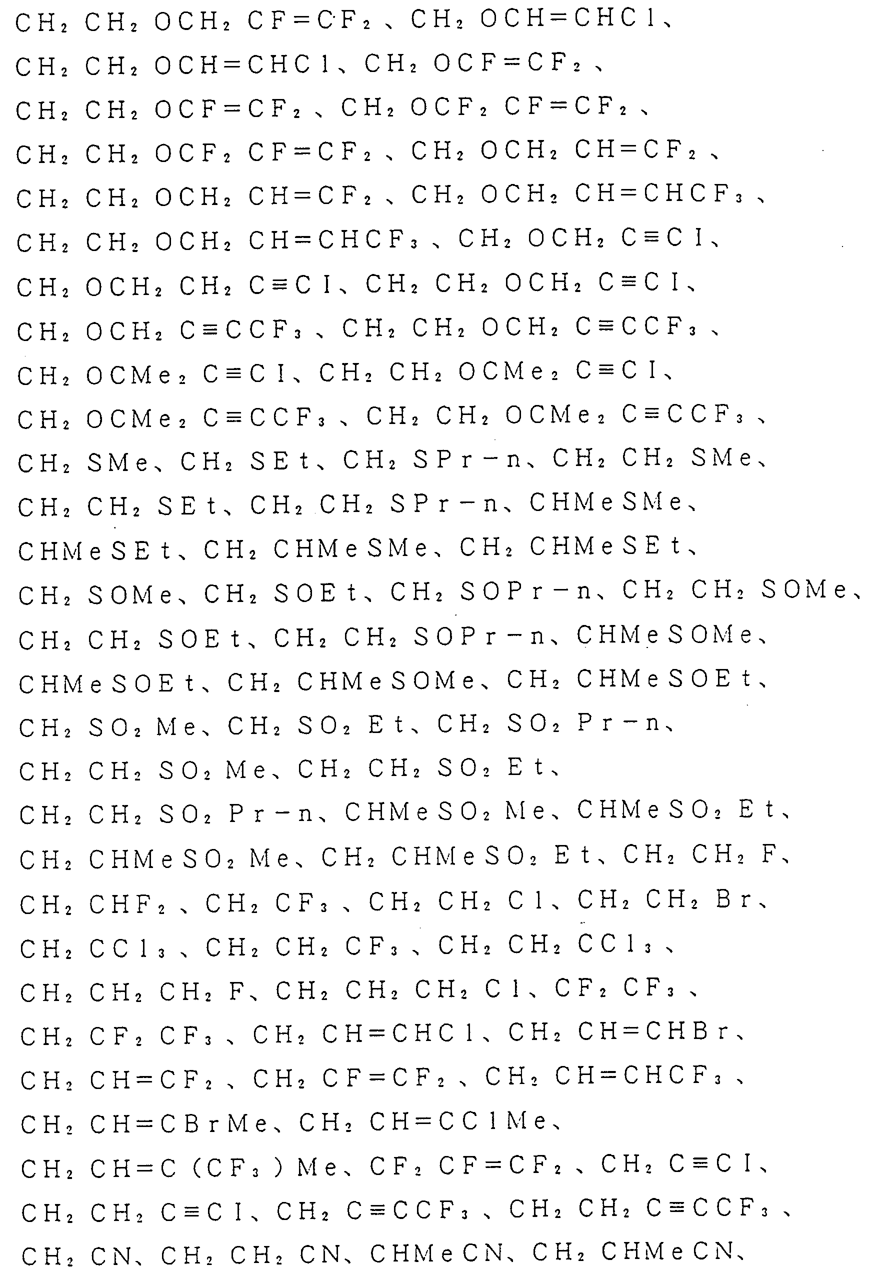

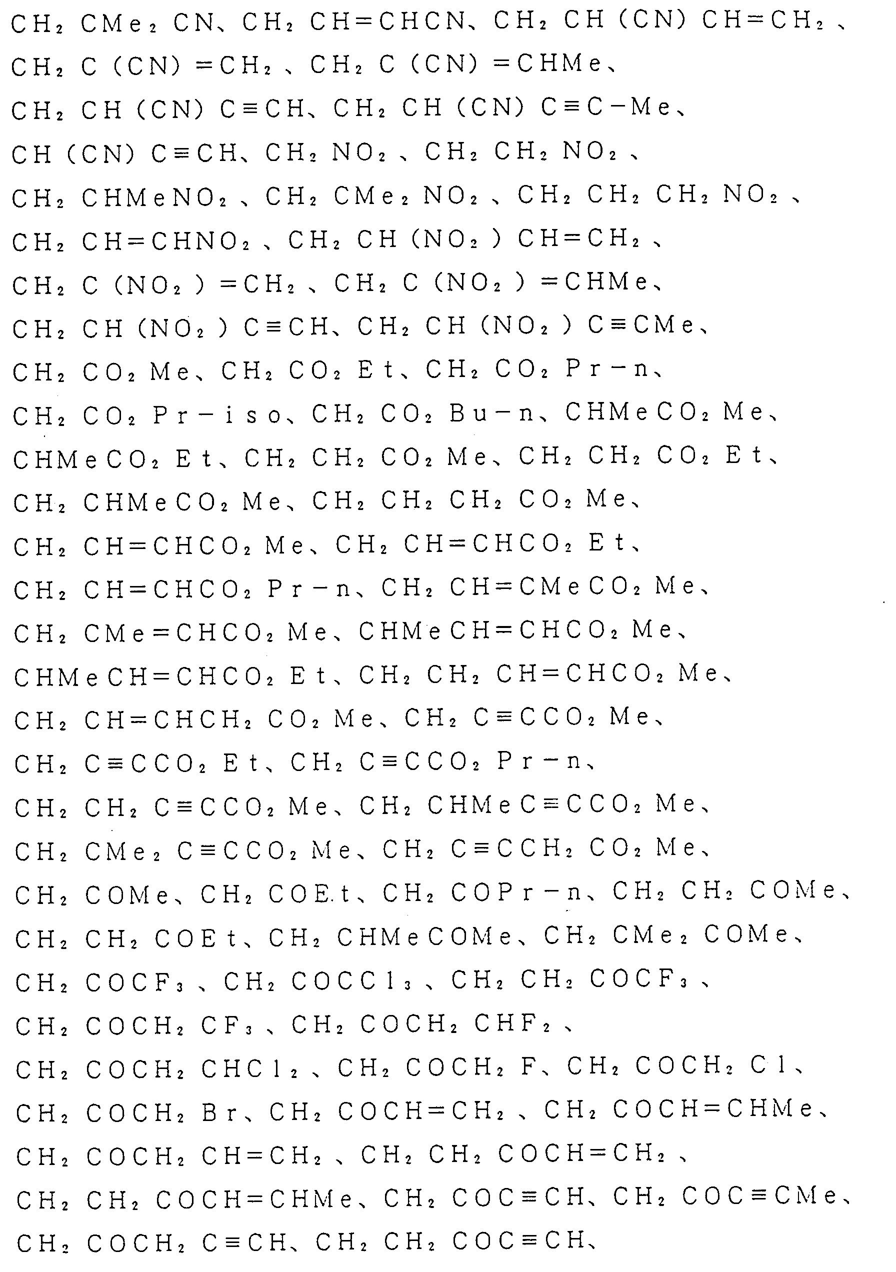

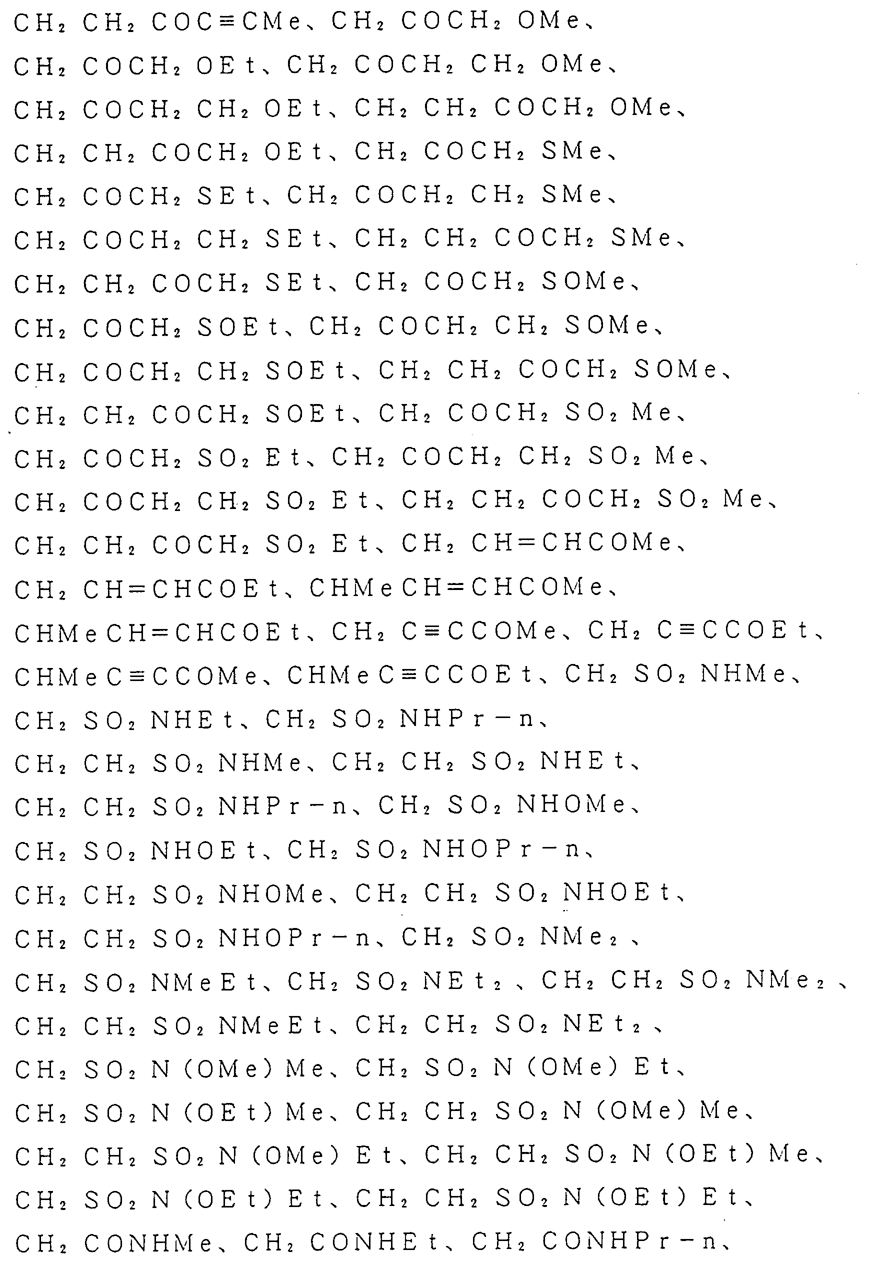

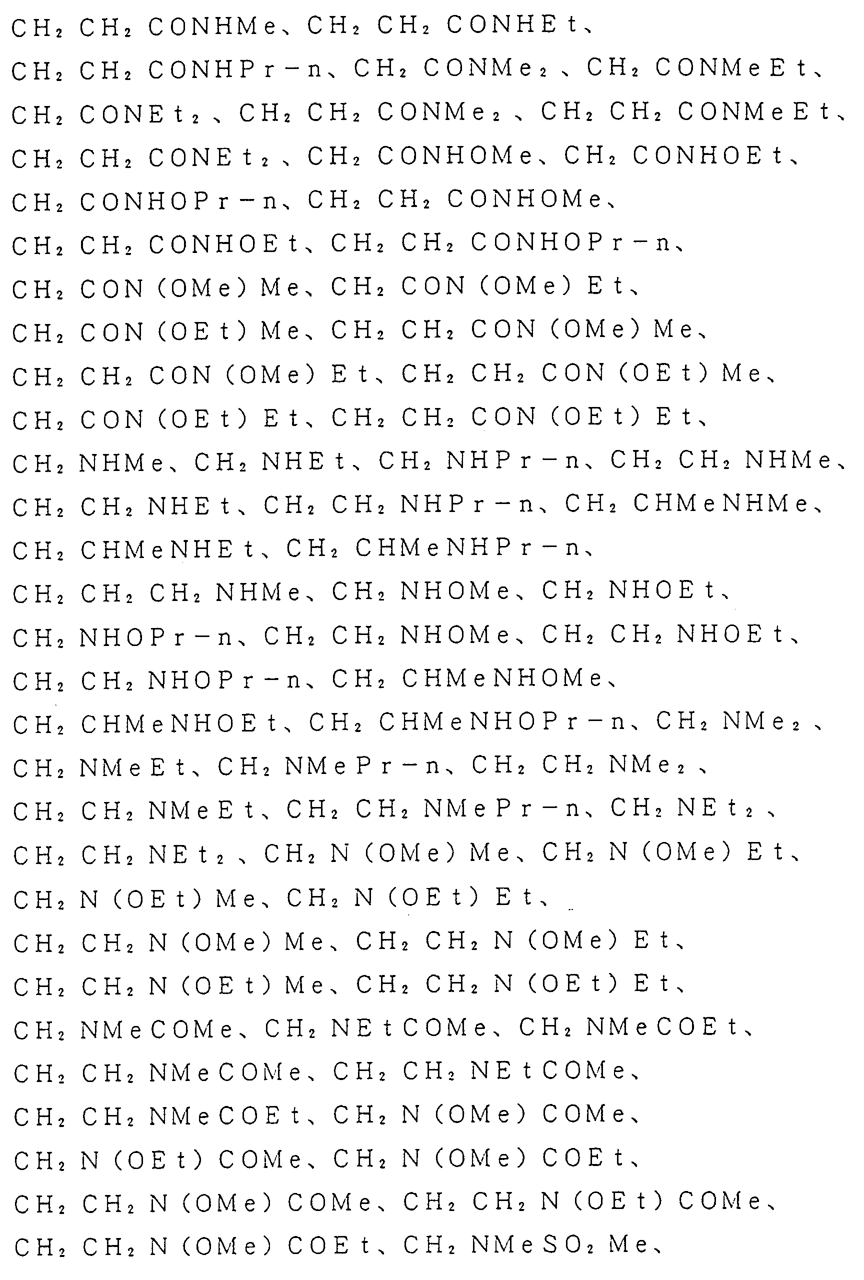

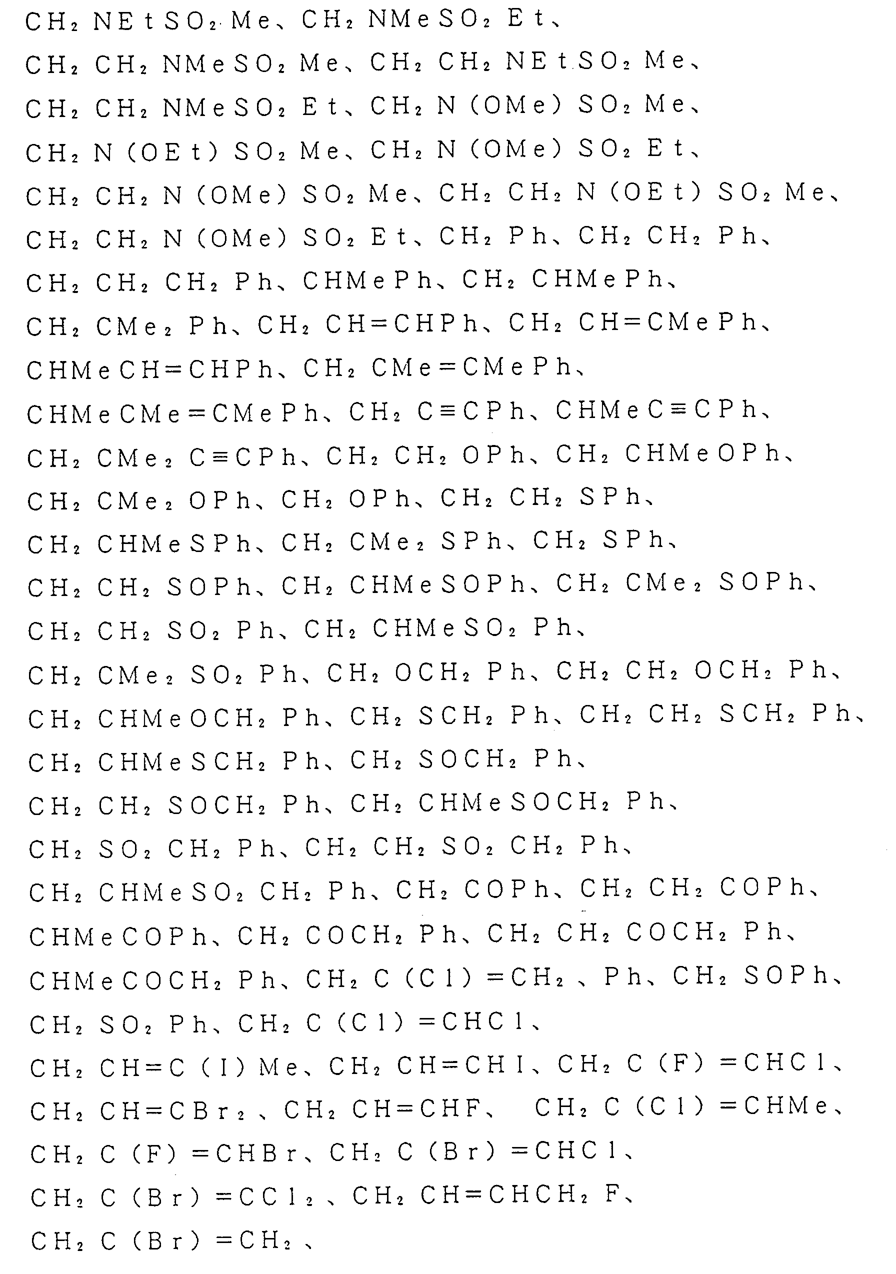

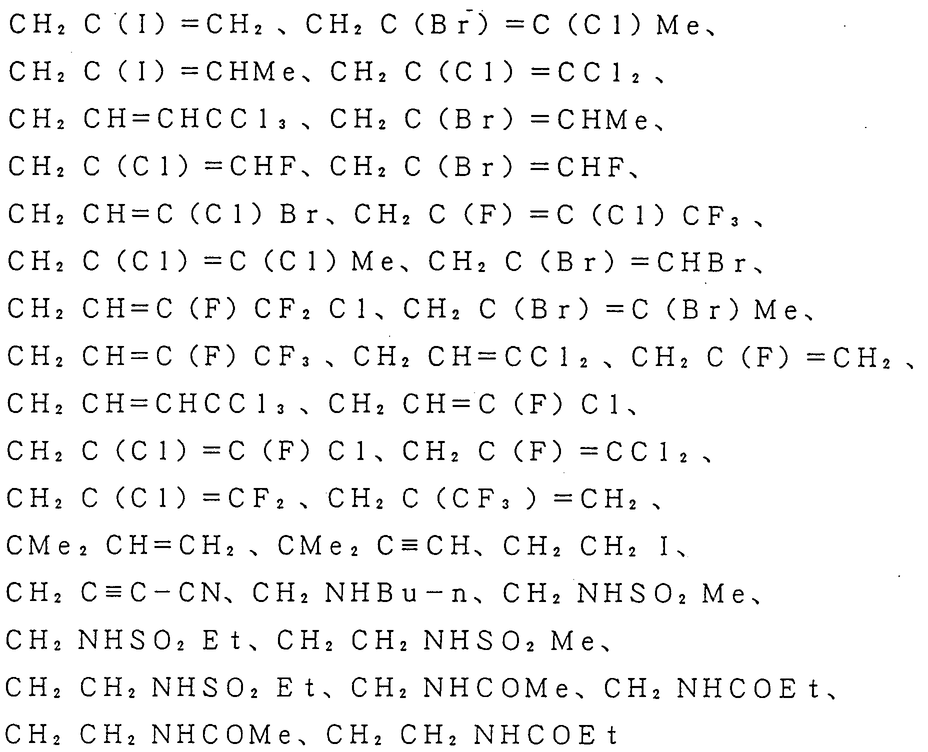

### Specific examples for the substituents R^{c2}, R^{c3}, R^{c4}, R^{c5}, R^{c6}, R^{c11}, and R^{c12} of the compound of the present invention

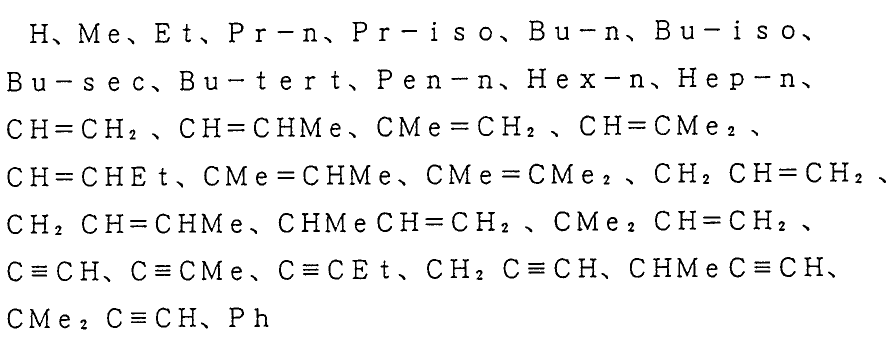

### Specific examples for the substituents R^{c7}, R^{c8}, R^{c9}, R^{c10}, R^{c13}, and R^{c14} of the compound of the present invention

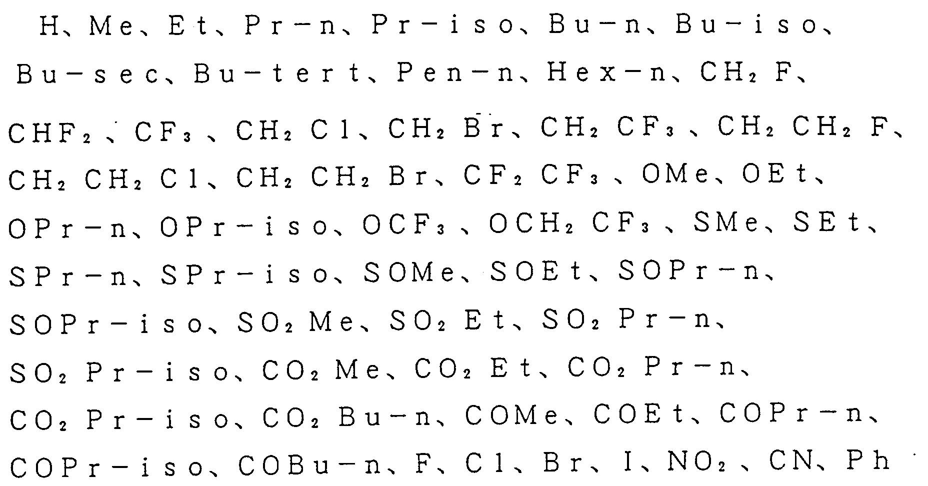

### Specific examples for the substituents R^{d2}, R^{d3} and R^{d4} of the compound of the present invention

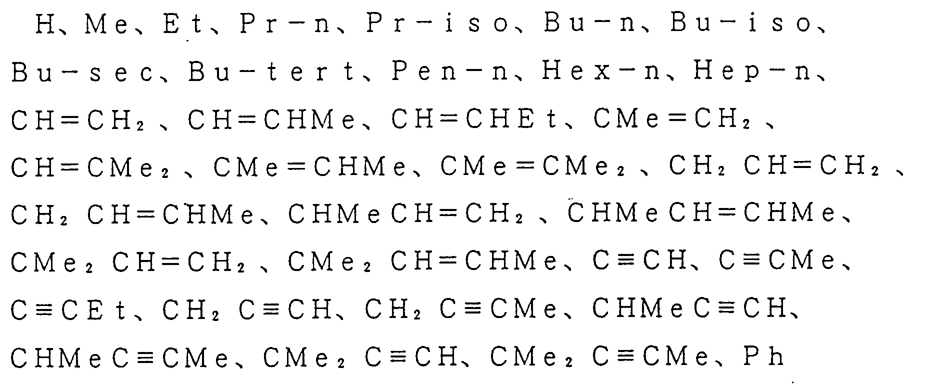

### Specific examples for the substituent R^{d5} of the compound of the present invention

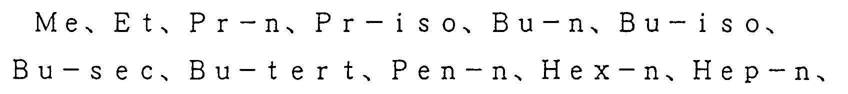

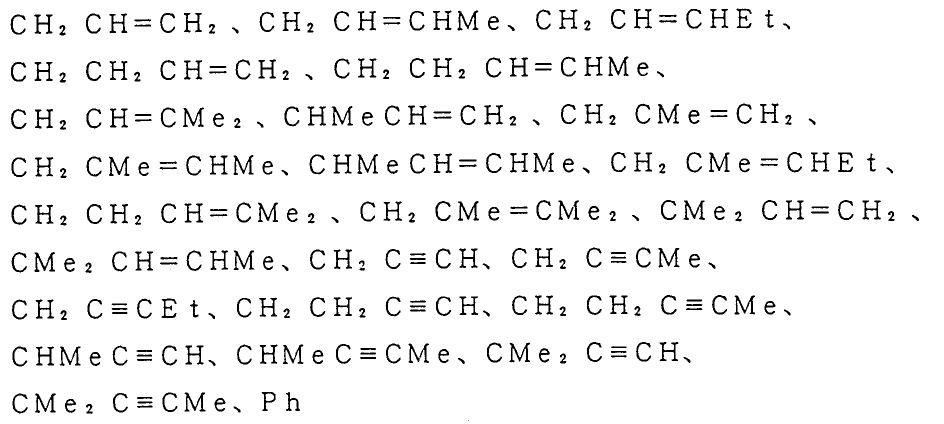

### Specific examples for the substituents R^{d6} and R^{f2} of the compound of the present invention

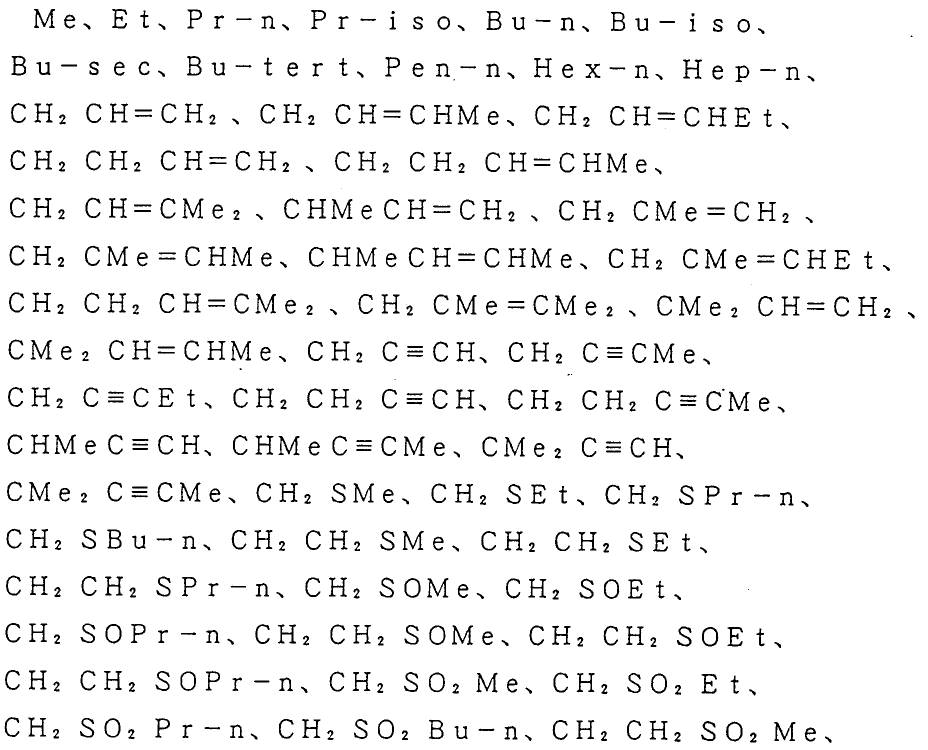

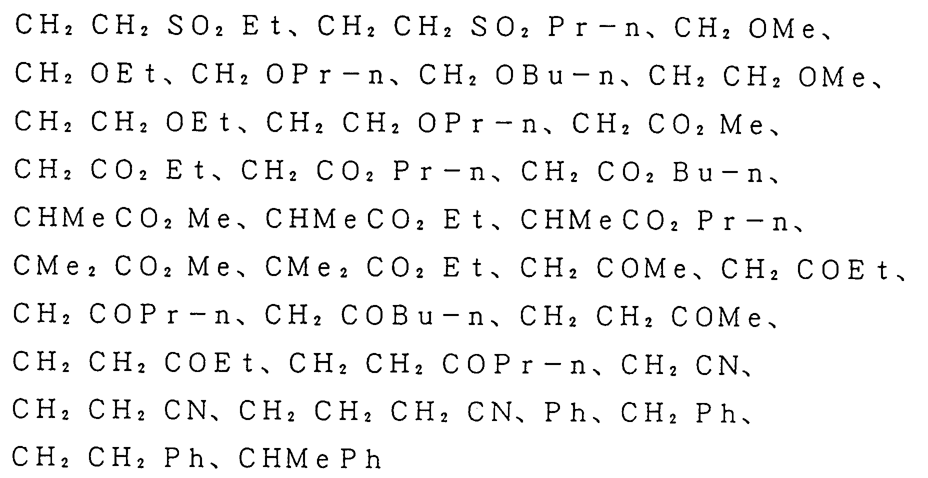

### Specific examples for the substituents R^{e2}, R^{e3}, R^{e6} and R^{e7} of the compound of the present invention

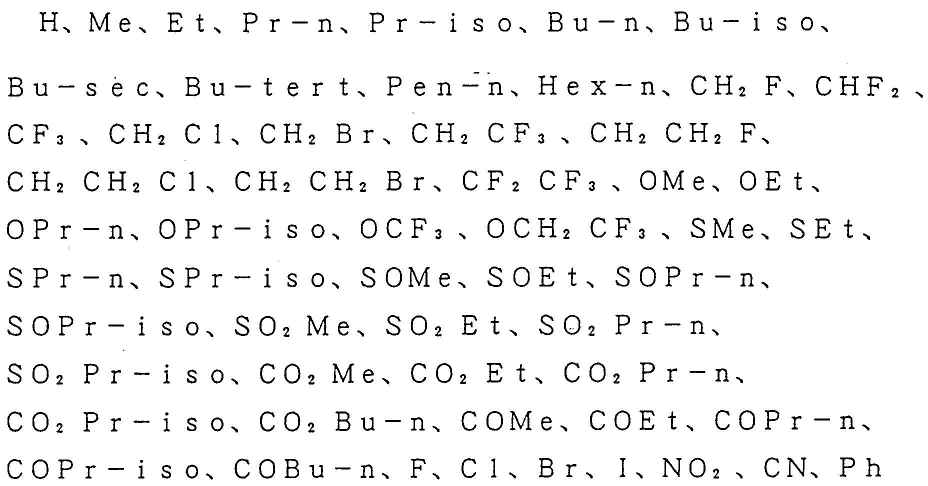

### Specific examples for the substituents R^{e4}, R^{e5}, R^{e8}, R^{e9} and R^{e10} of the compound of the present invention

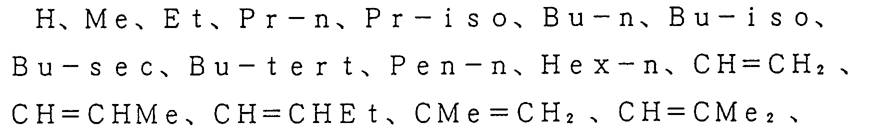

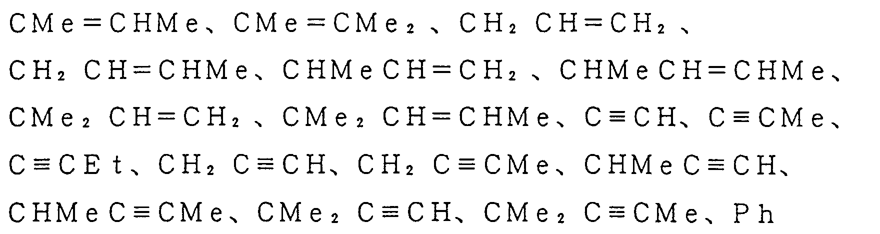

### Specific examples for the substituents R^{f3}, R^{f4}, R^{f7}, R^{f8}, R^{f11} and R^{f12} of the compound of the present invention

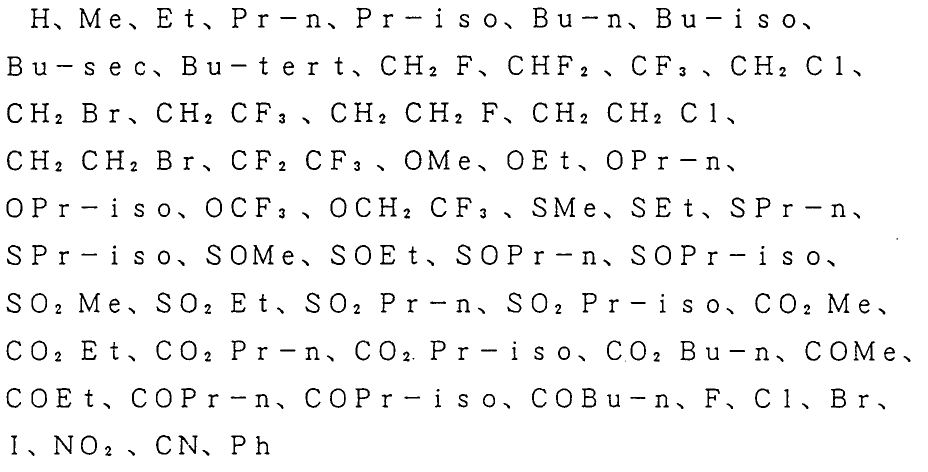

### Specific examples for the substituents R^{f5}, R^{f6}, R^{f9}, R^{f10}, R^{f13}, R^{f14} and R^{f15} of the compound of the present invention

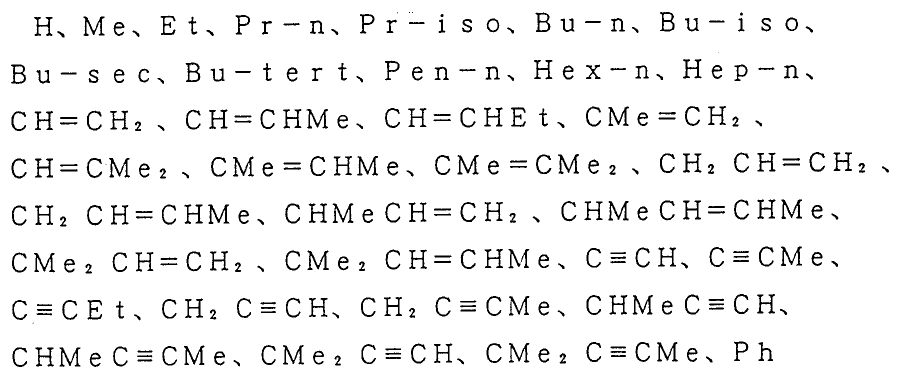

### Specific examples for the substituent R^{g1} of the compound of the present invention

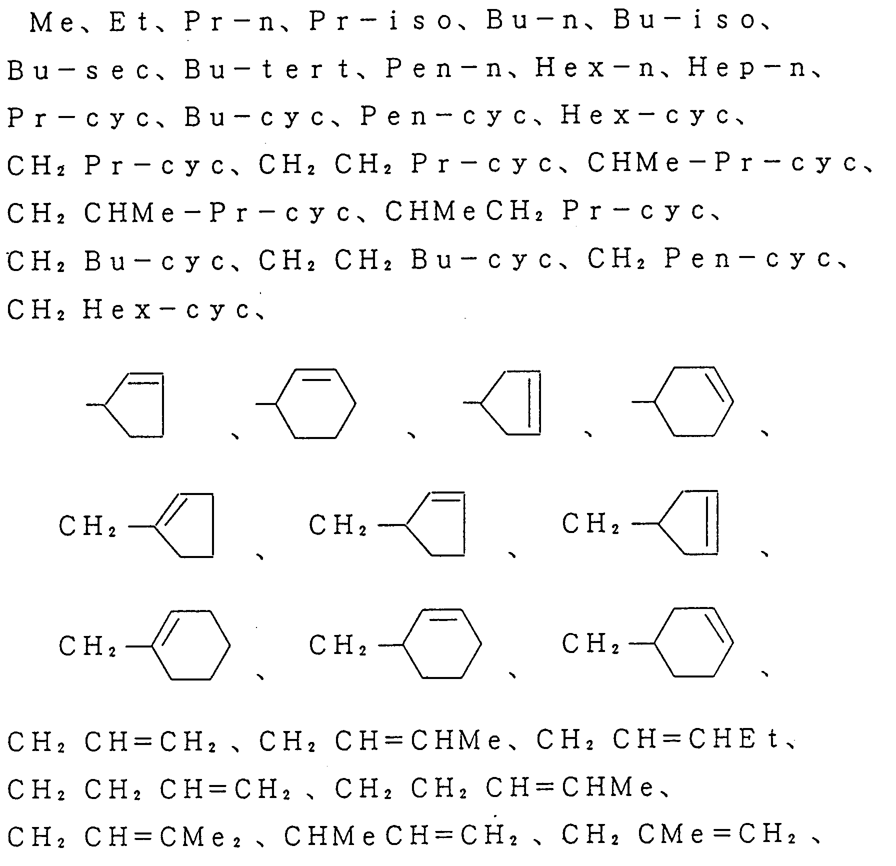

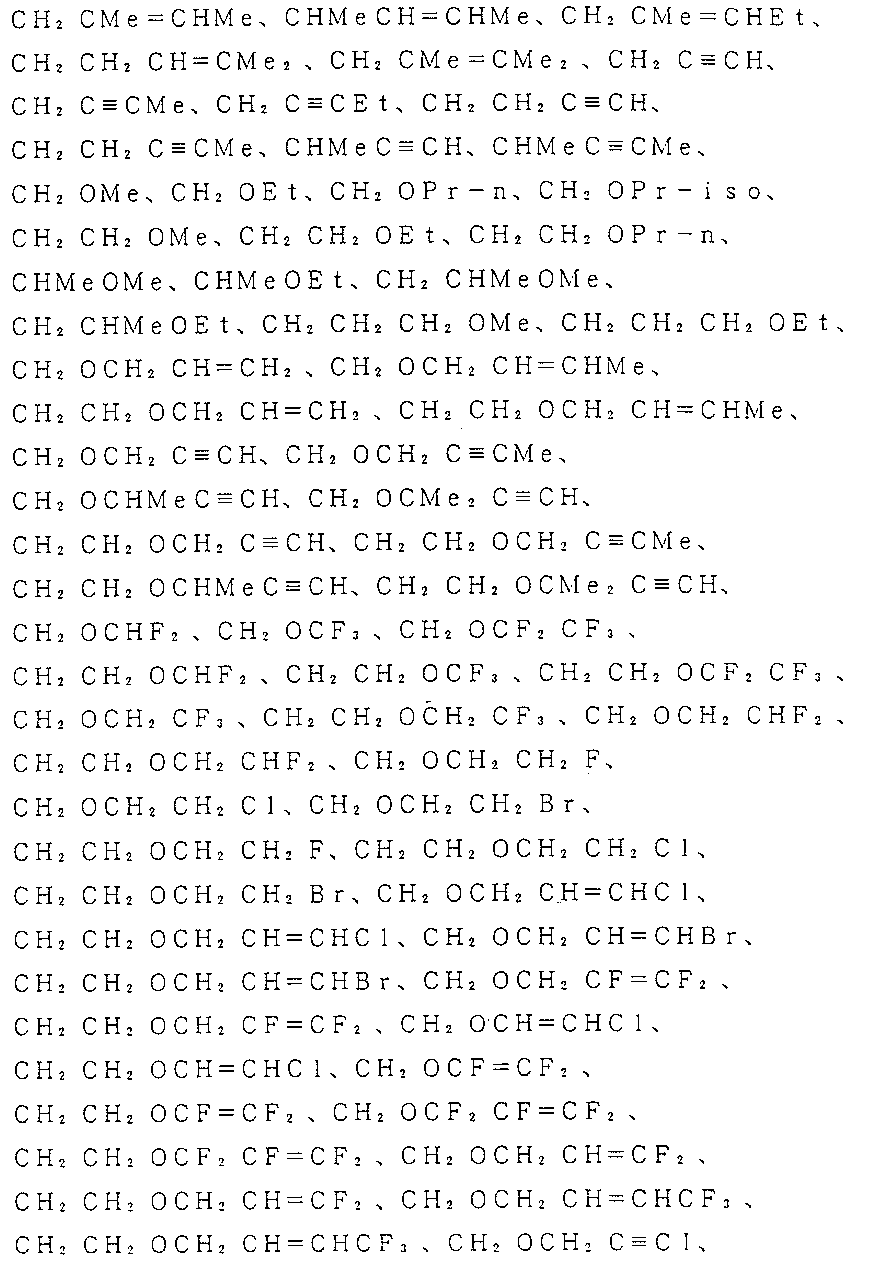

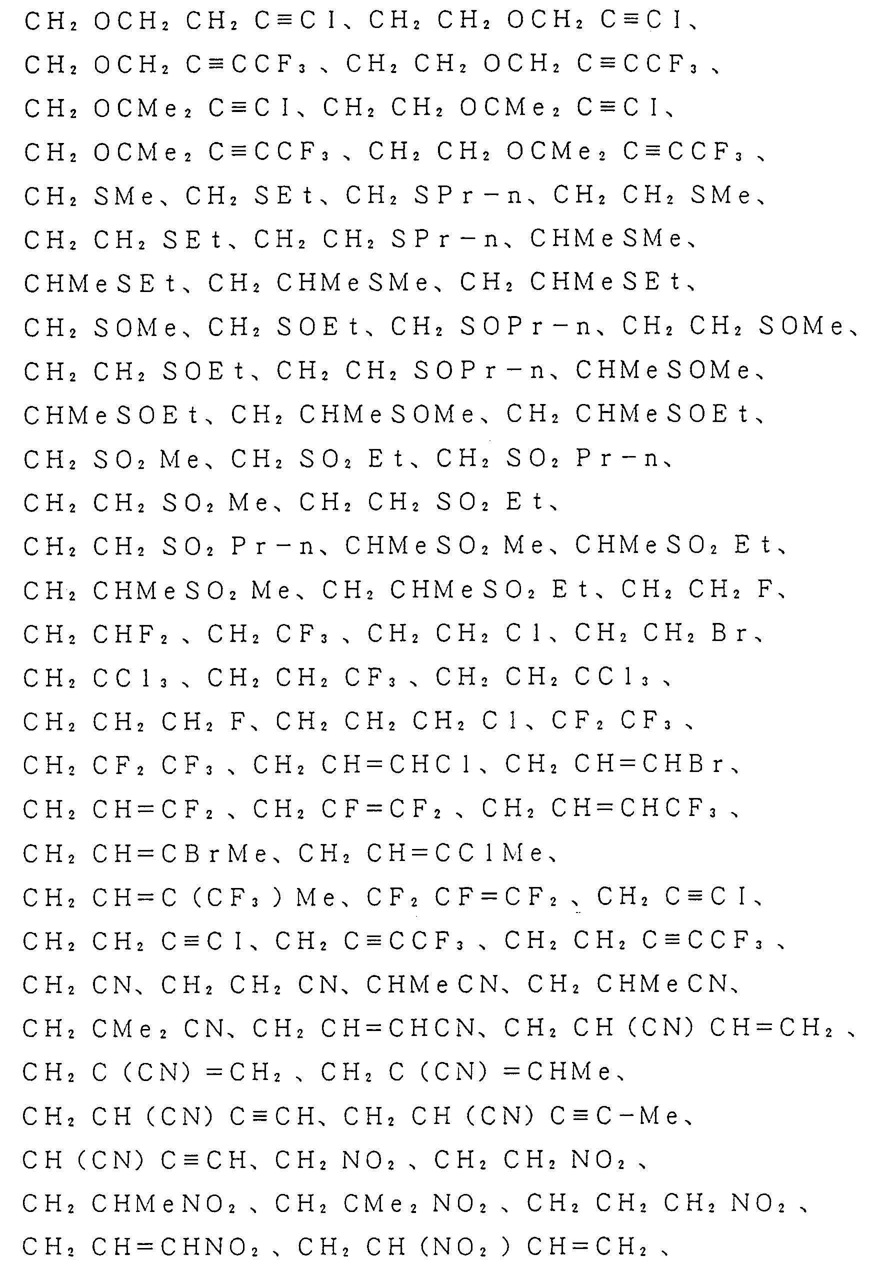

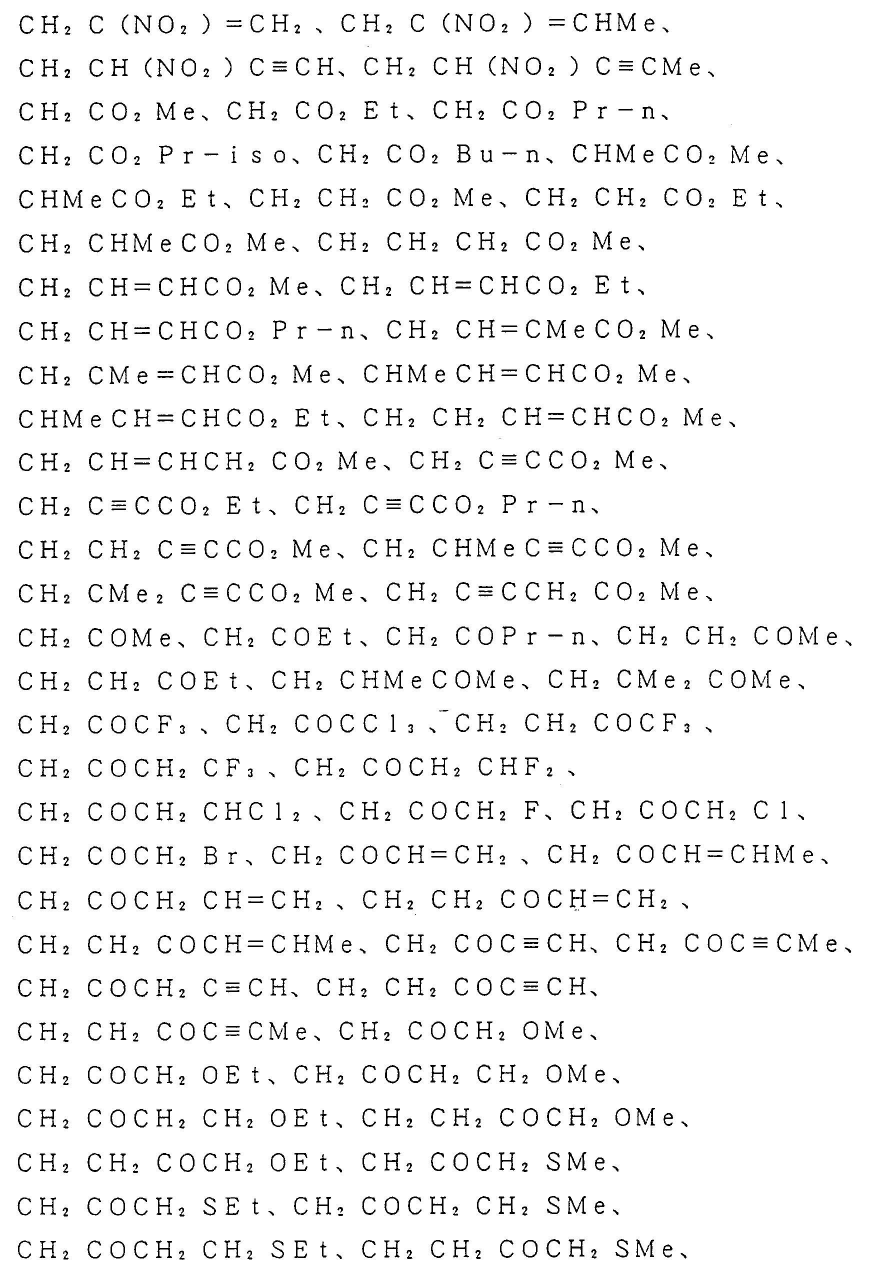

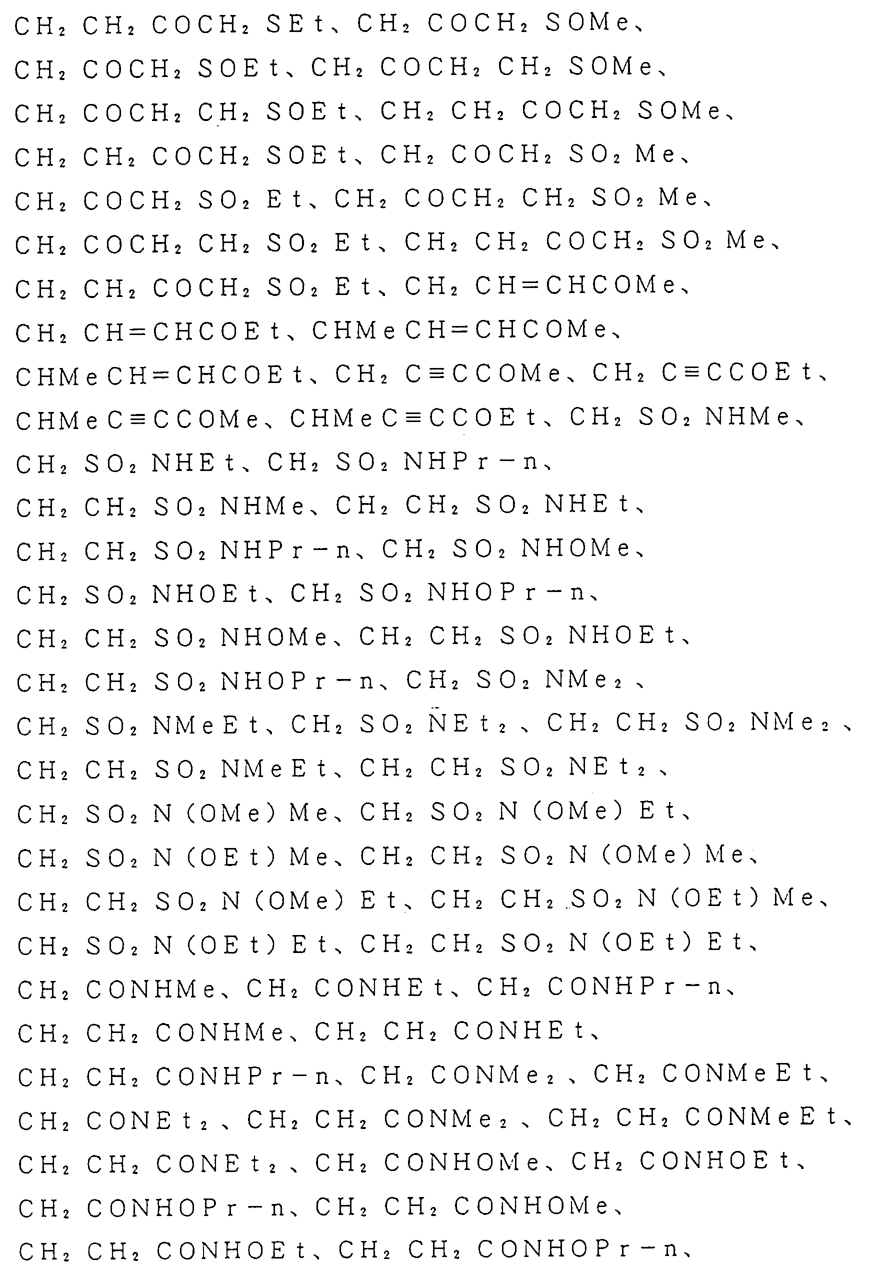

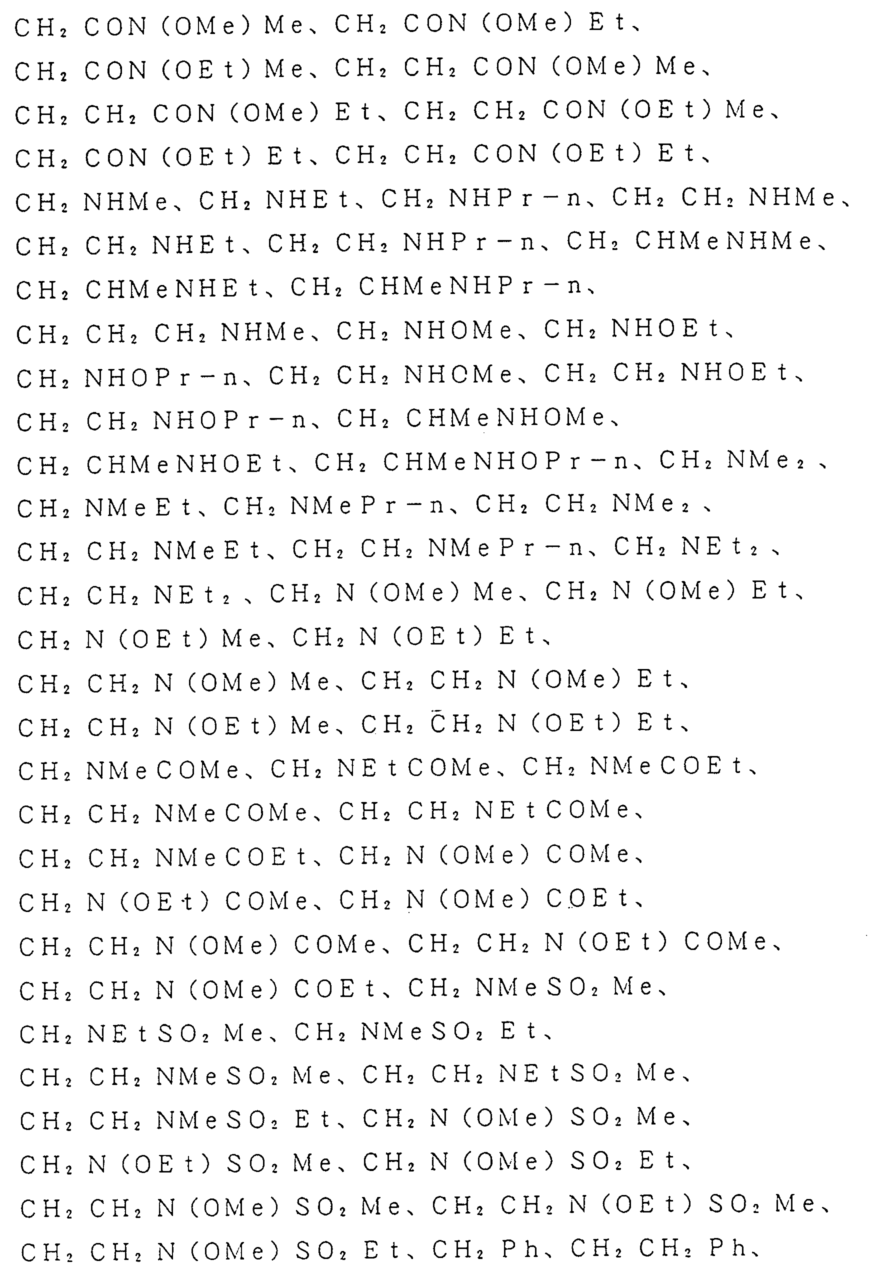

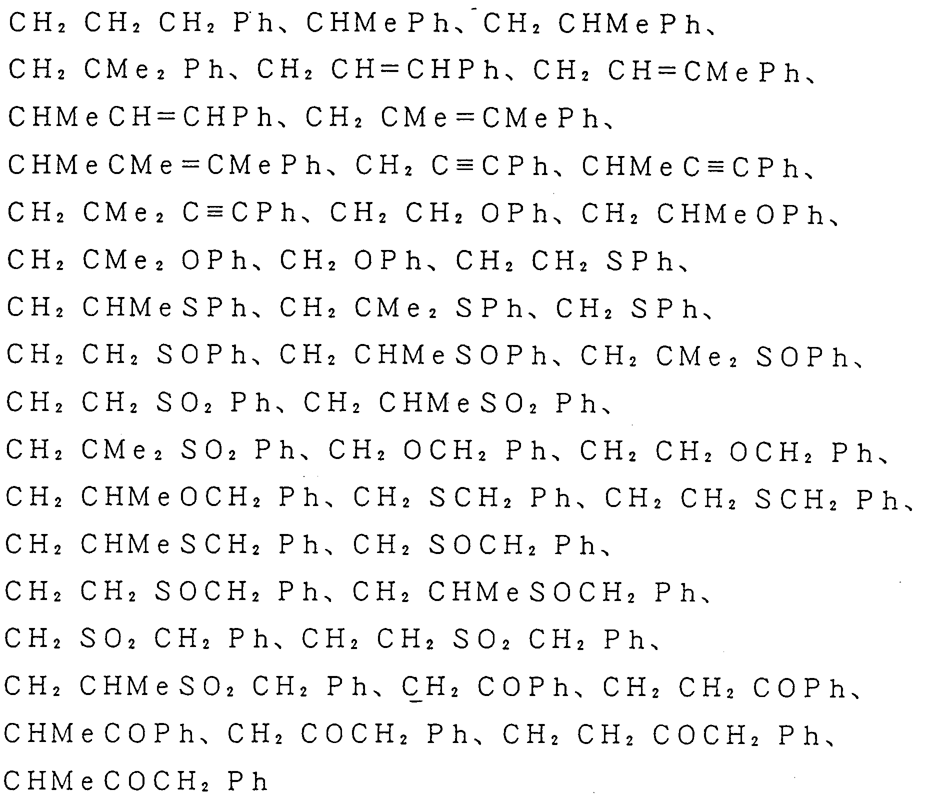

### Specific examples for the substituents R^{g2} and R^{g3} of the compound of the present invention

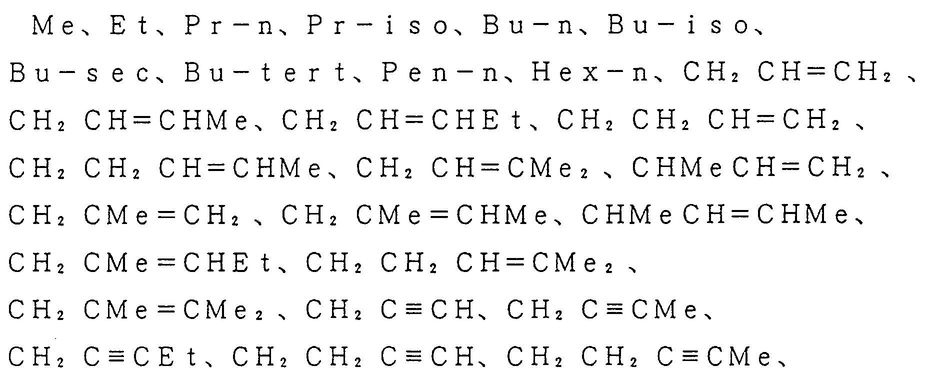

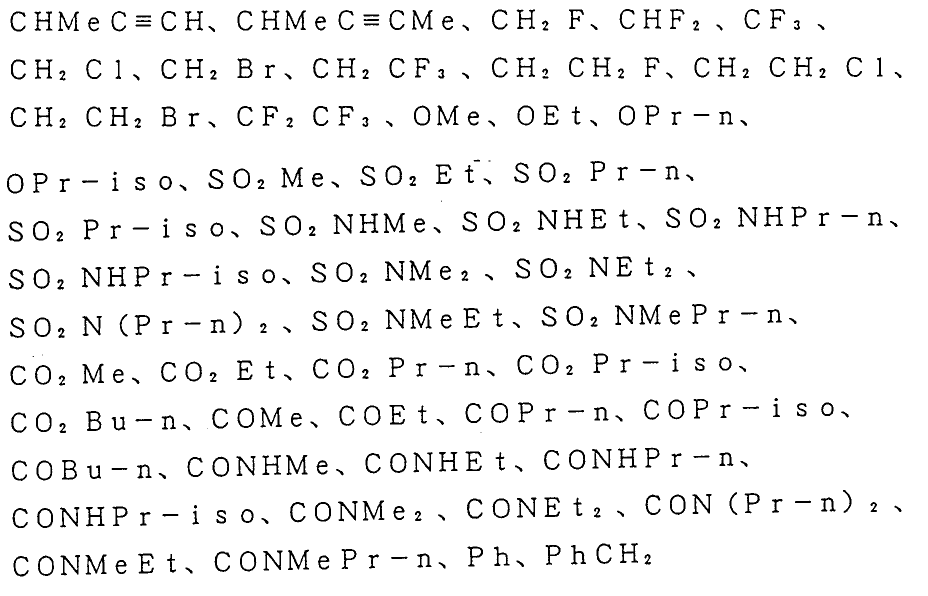

### Specific examples of -NR^{g2}R^{g3} wherein the substituents R^{g2} and R^{g3} of the compound of the present invention form a saturated 3- to 7-membered heterocyclic ring together with the nitrogen atom to which they are bonded

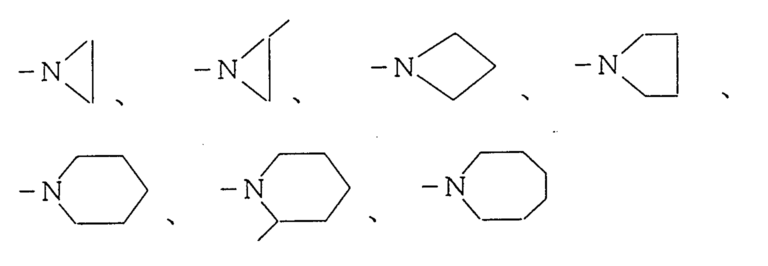

### Specific examples for the substituent L of the compound of the present invention

H, Me, Et, Pr-n, CH₂CH=CH₂, CH₂C≡CH

### Specific examples for the substituents B and D of the compound of the present invention

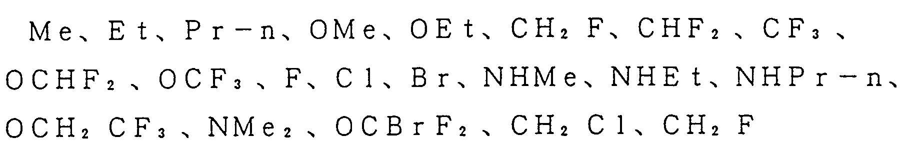

Now, examples of the compound covered by the present invention will be presented in the following Tables 1A, 1B, 1C, 2A, 2B, 2C, 3, 4A, 4B, 4C, 5, 6, 7, 8, 9, 10, 11 and 12. However, the compound of the present invention is not limited to such examples. The symbols in these Tables have the following meanings.

Me: methyl group, Et: ethyl group, Pr-n: n-propyl group, Pr-iso: isopropyl group, Bu-n: n-butyl group, Bu-iso: isobutyl group, Bu-sec: sec-butyl group, Bu-tert: tert-butyl group, Pen-n: n-pentyl grup, Hex-n: n-hexyl group, Hep-n: n-heptyl group, Pr-cyc: cyclopropyl group, Bu-cyc: cyclobutyl group, Pen-cyc: cyclopentyl group, Hex-cyc: cyclohexyl group, and Ph: phenyl group, 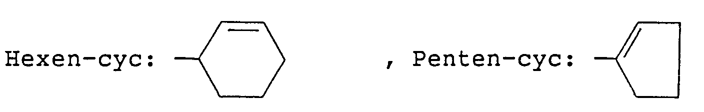 Gn is the same as above G and represents the following Ga, Gb and Gc.
Ga = G1 to G90 (i.e. represents any one of G1 to G90)
Gb = G1 to G13 (i.e. represents any one of G1 to G13)
Gc = G1 to G6 (i.e. represents any one of G1 to G6)

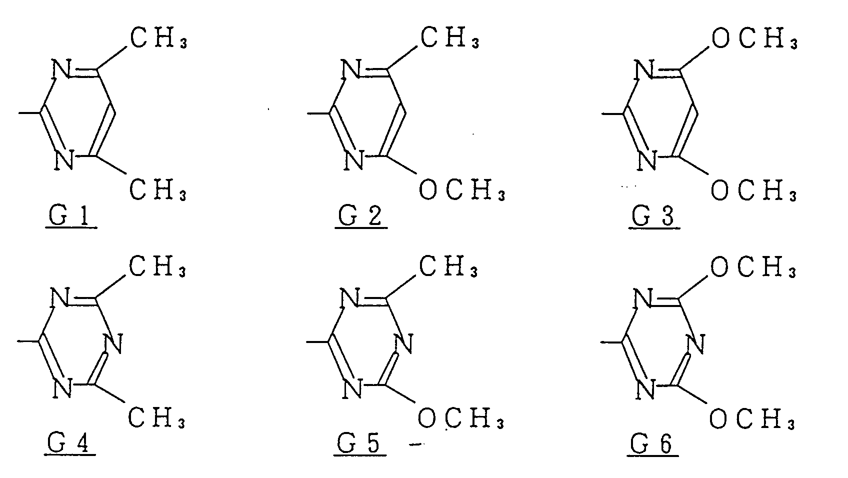

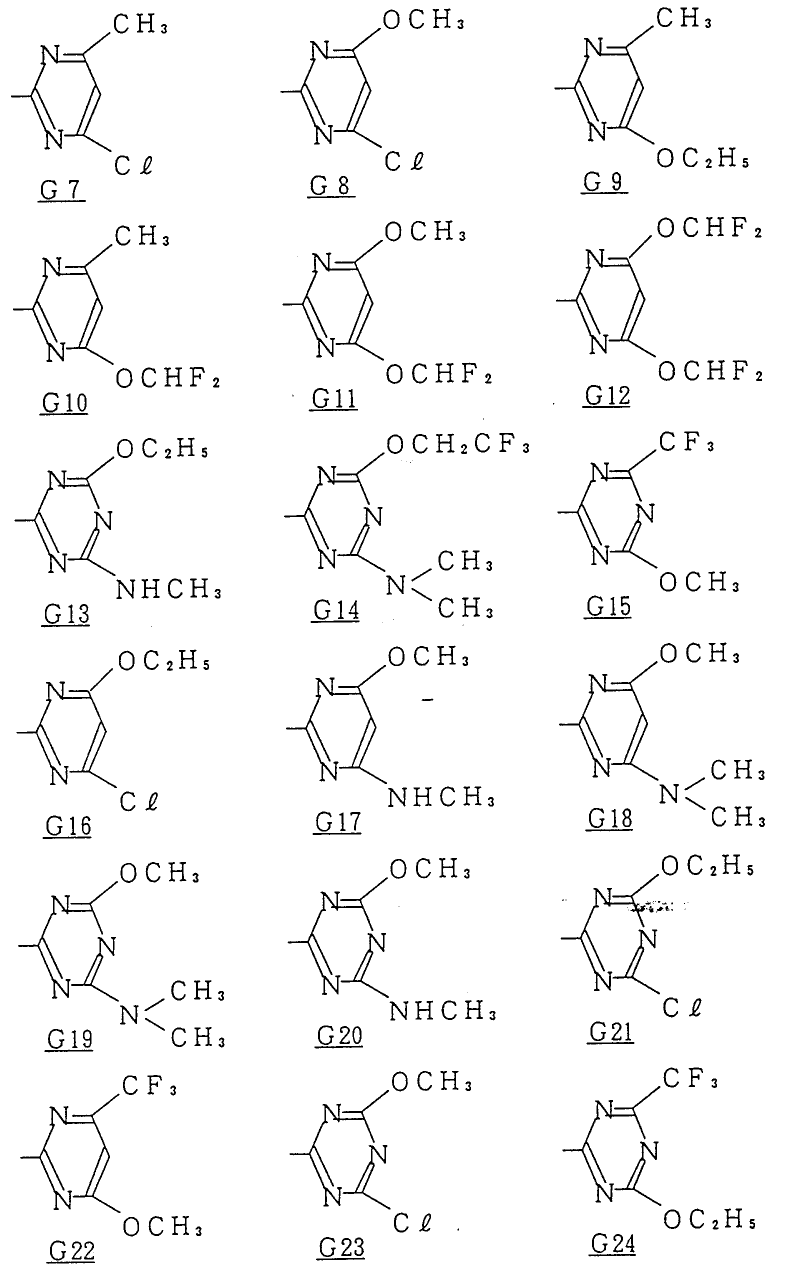

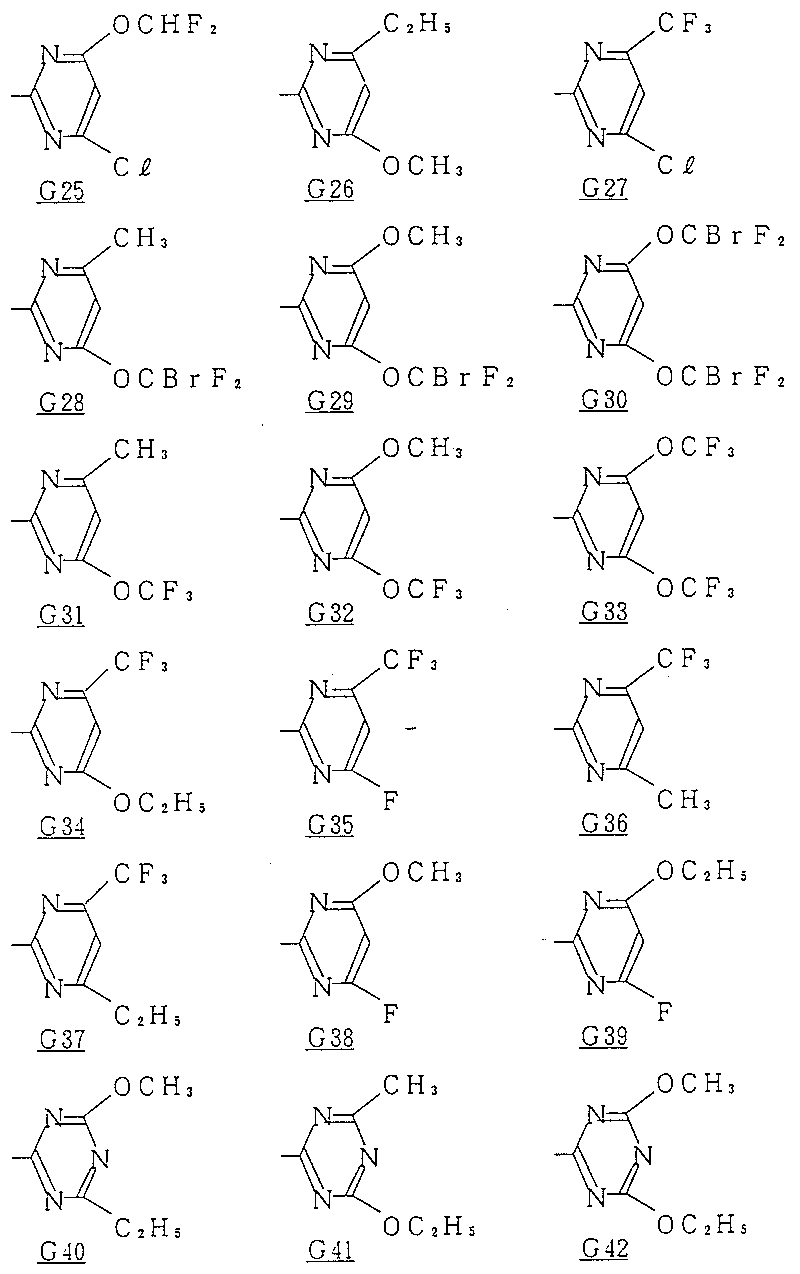

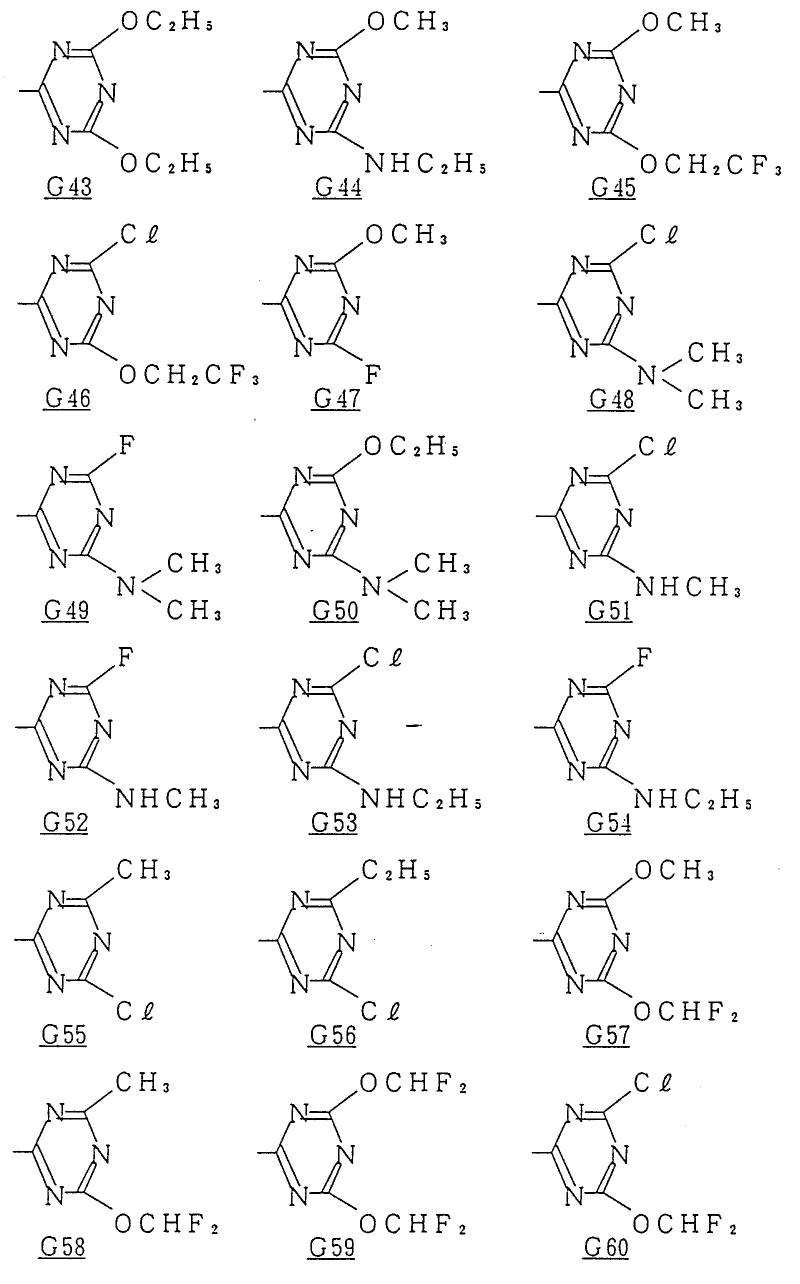

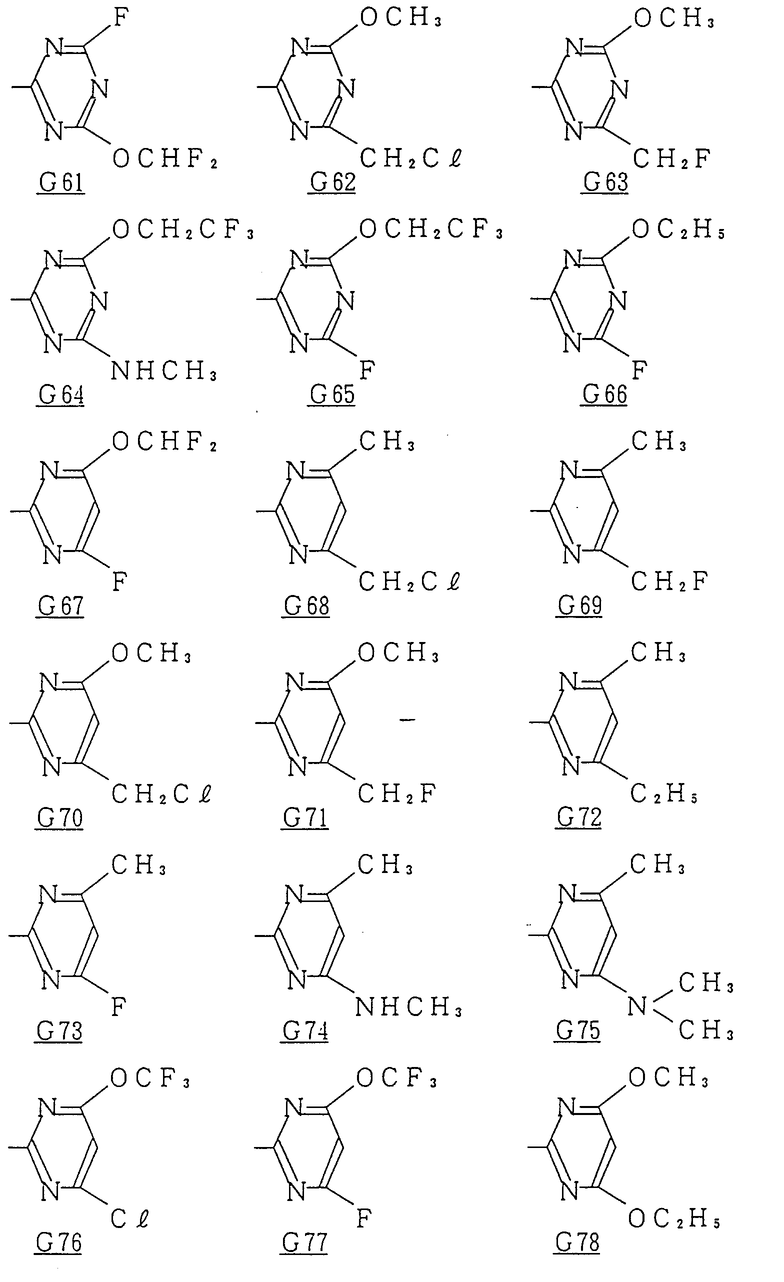

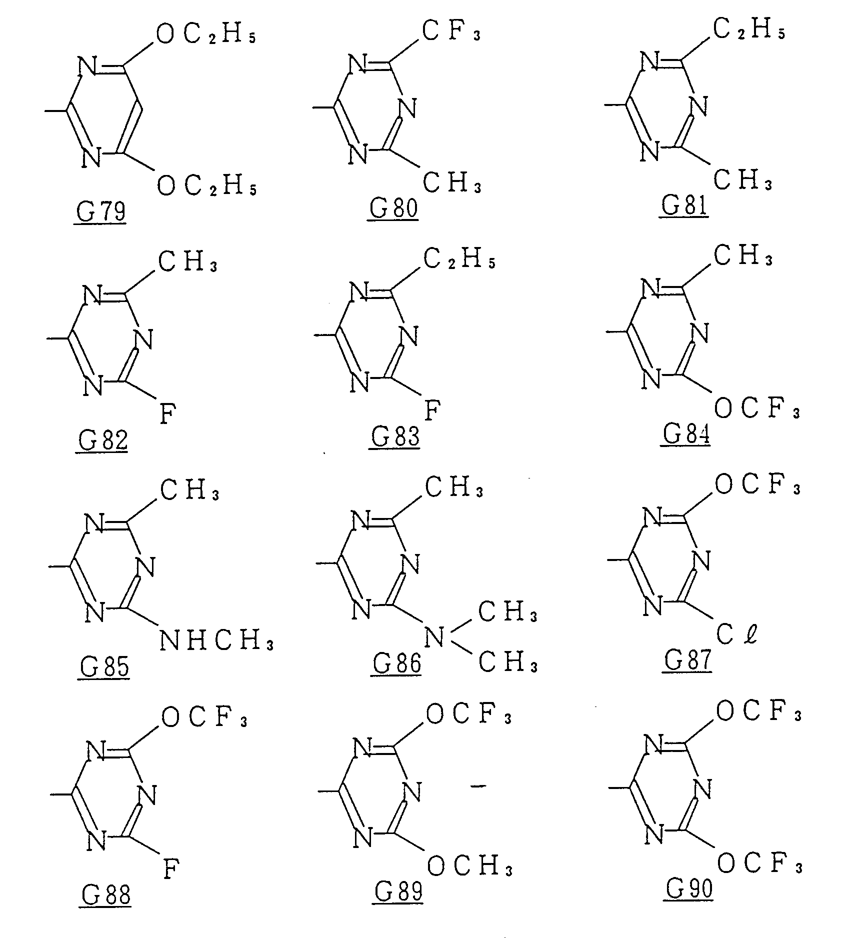

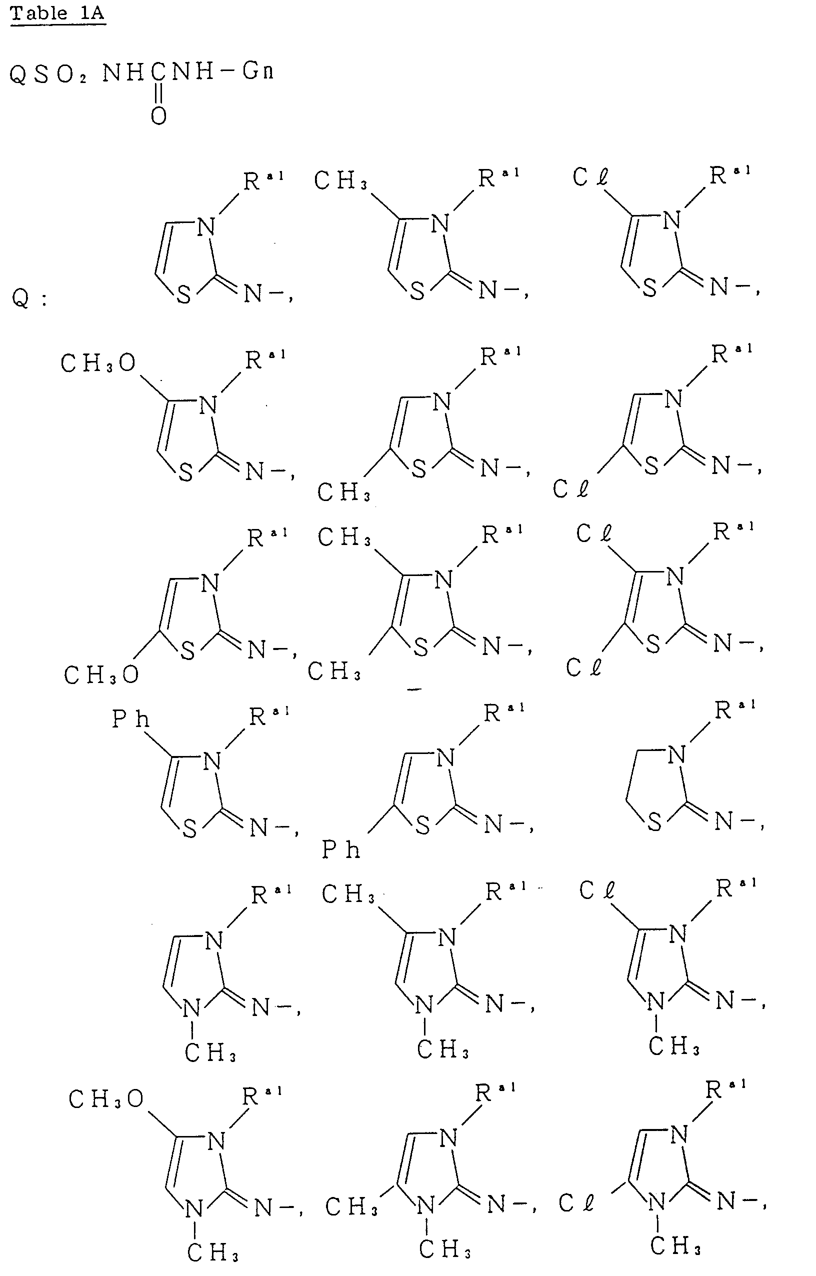

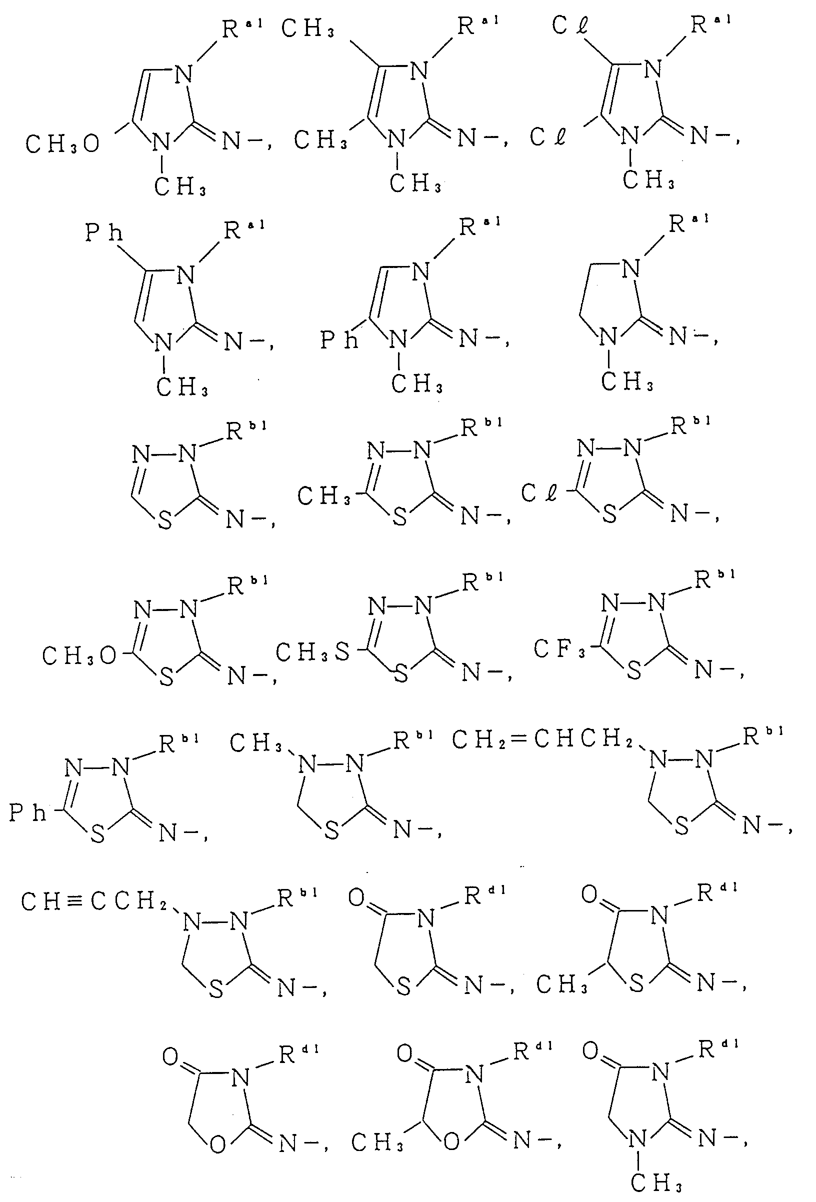

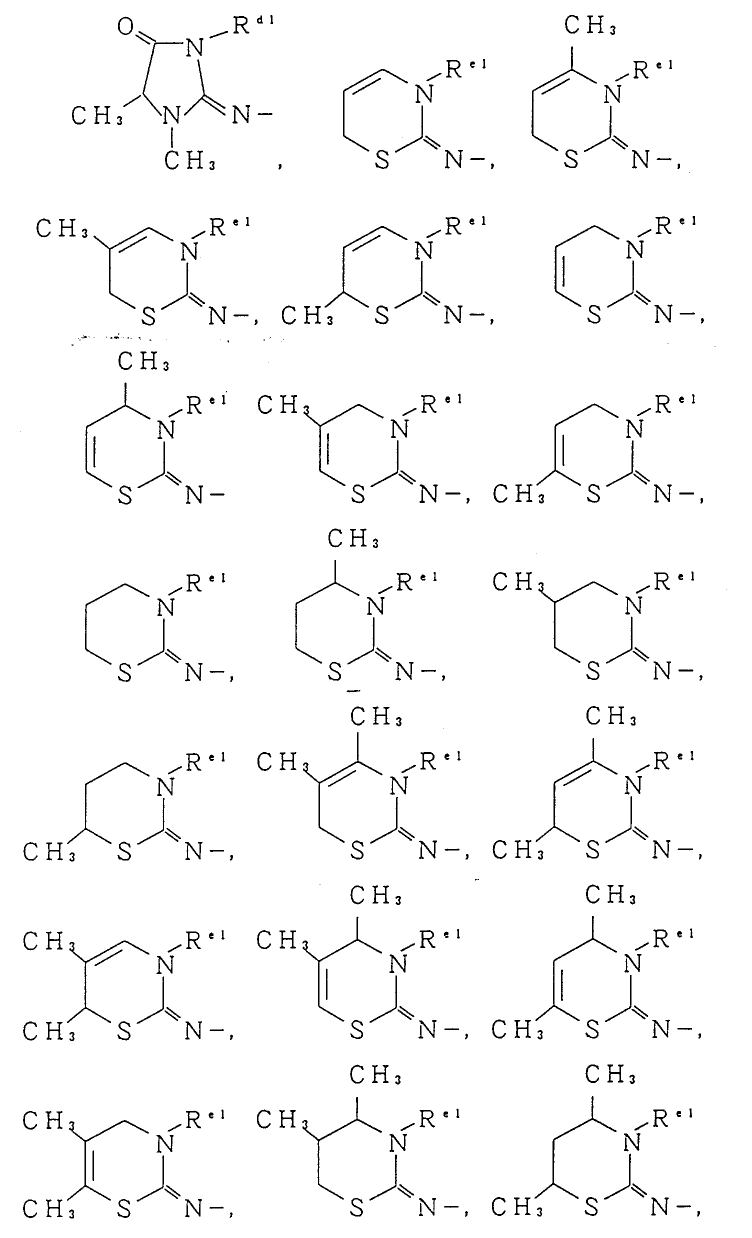

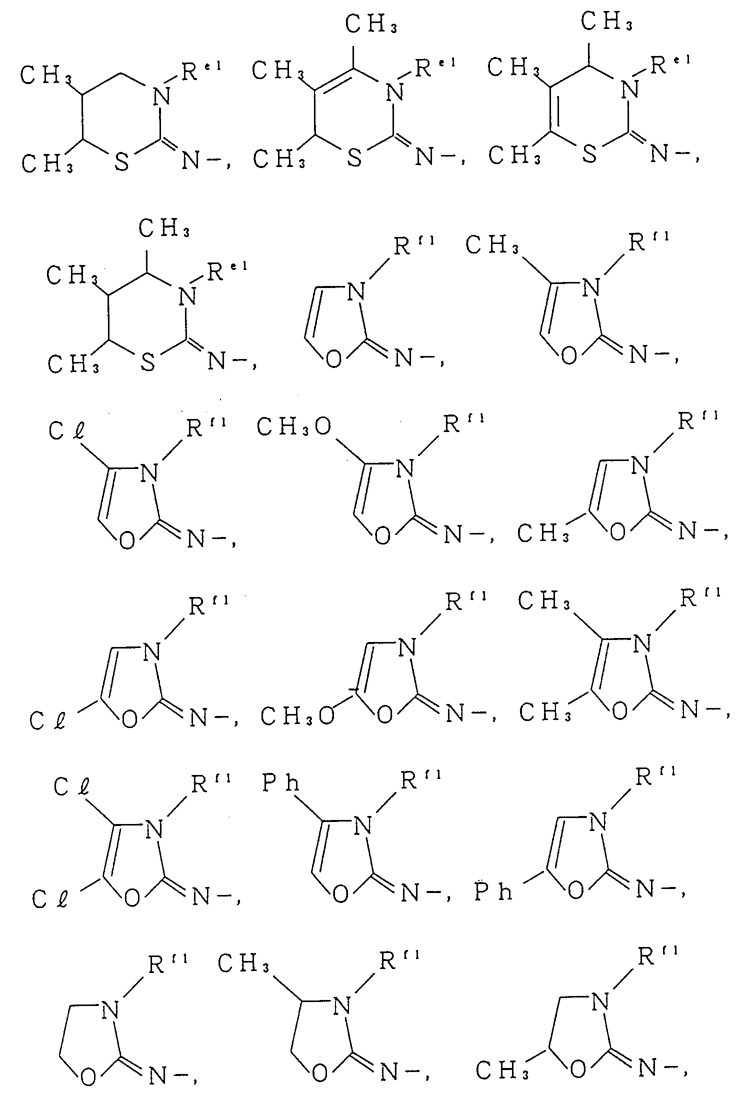

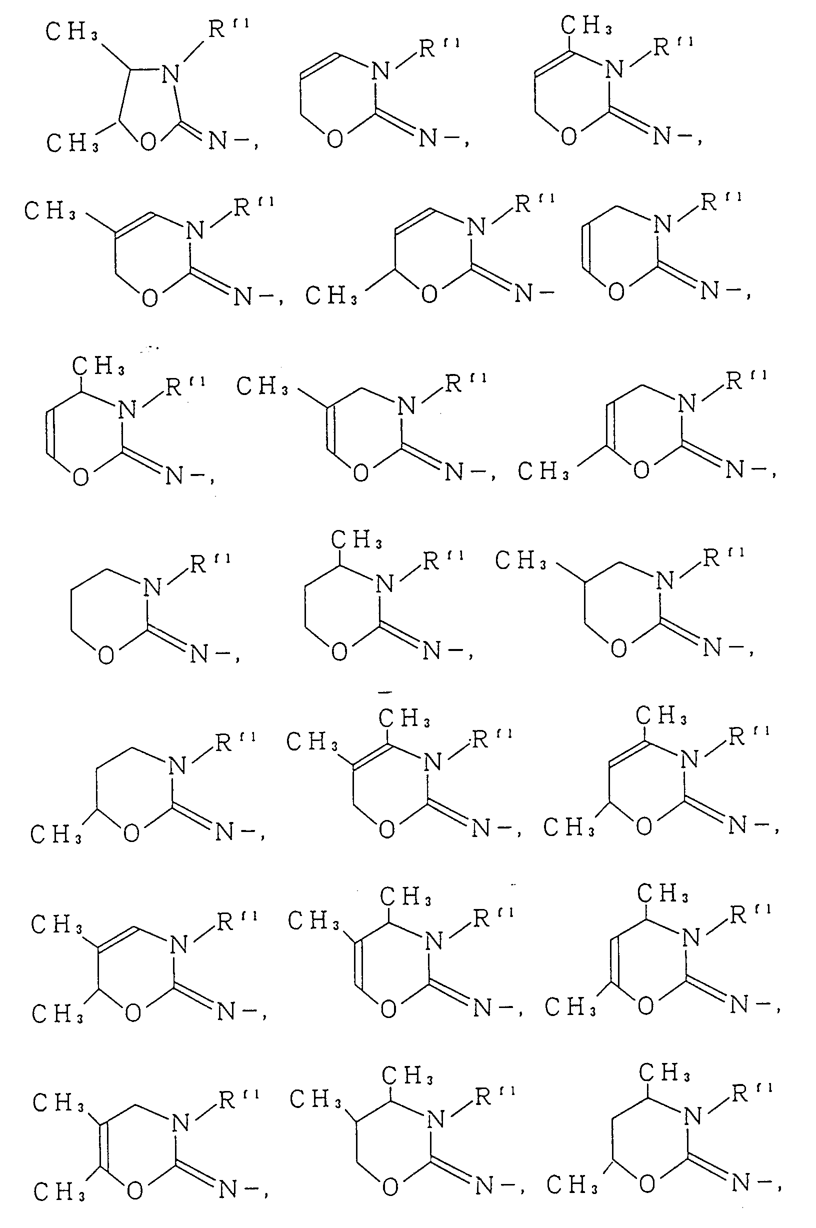

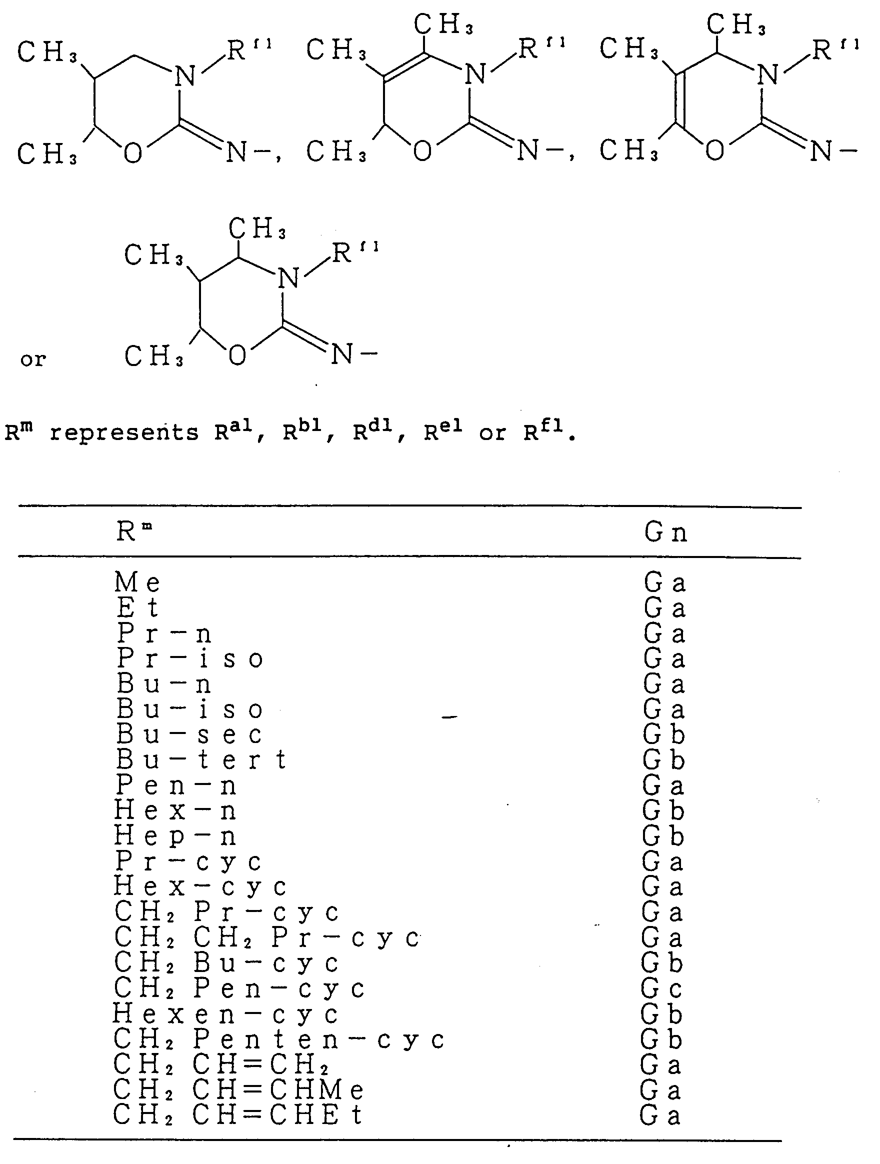

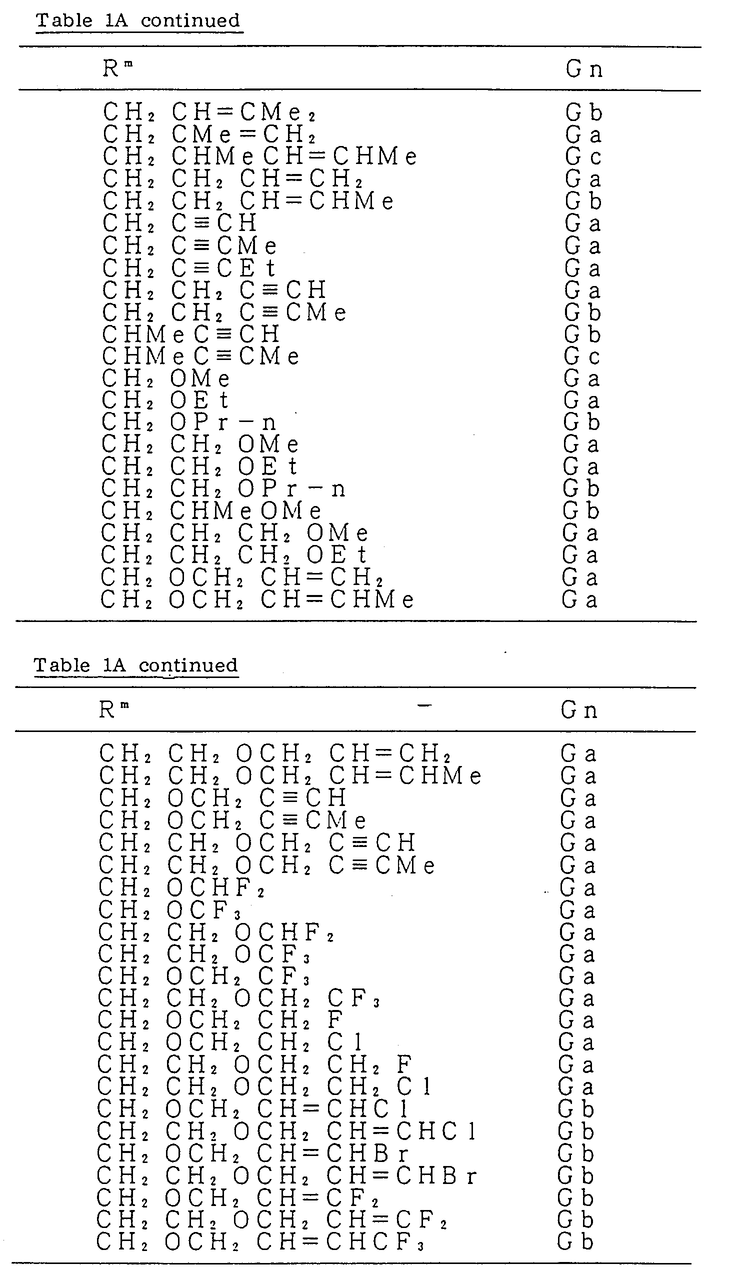

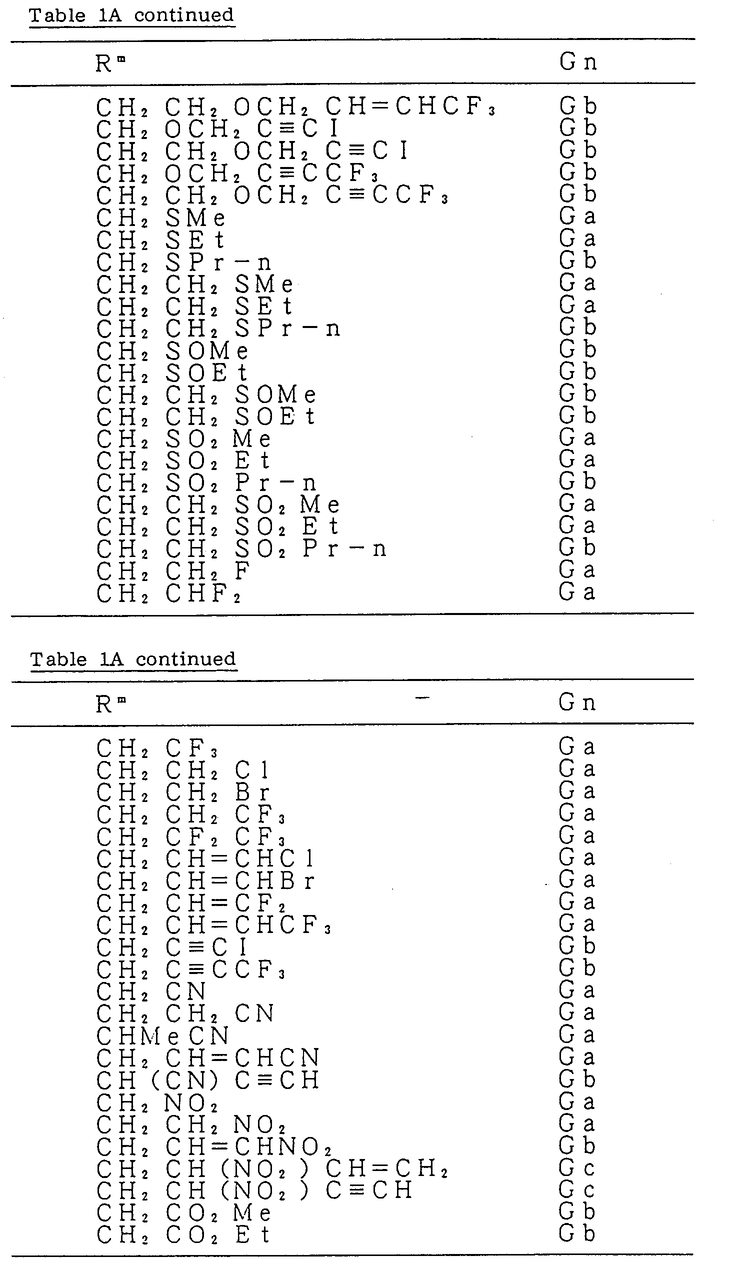

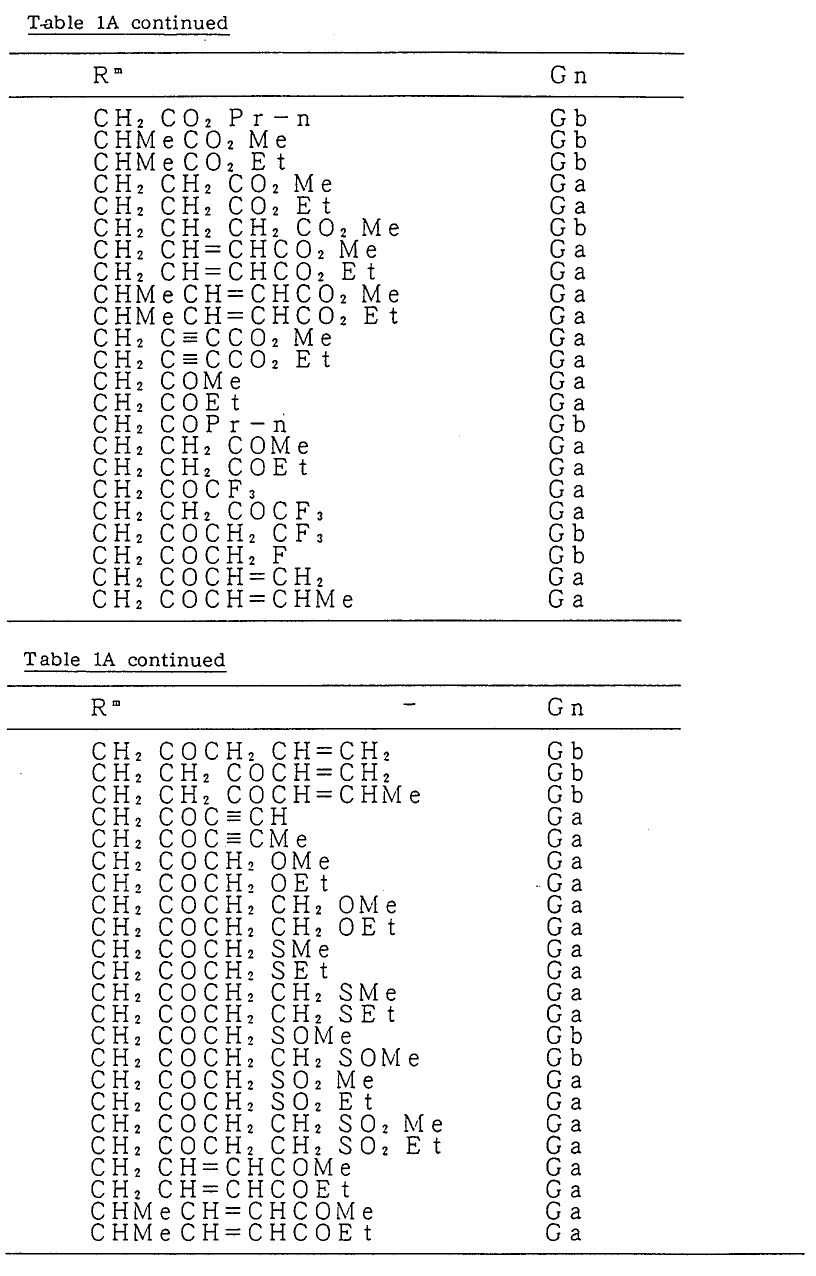

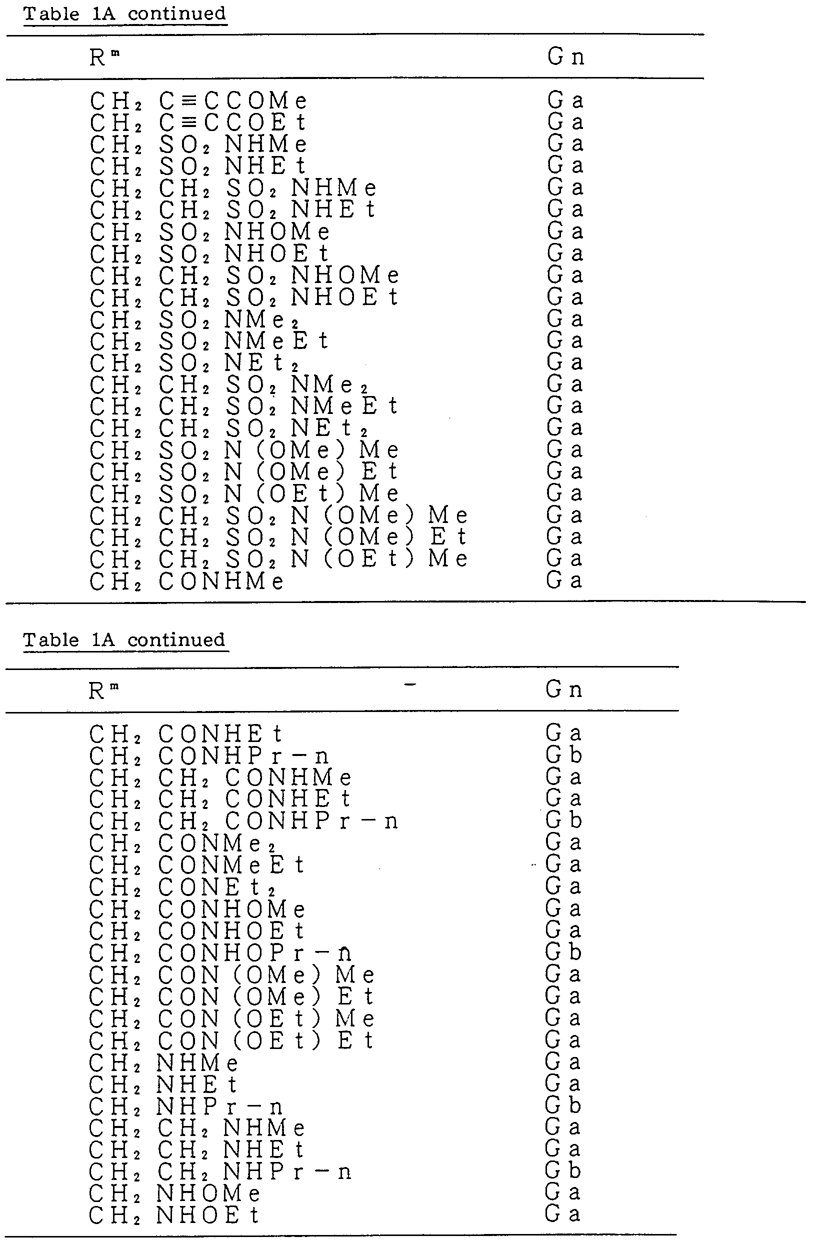

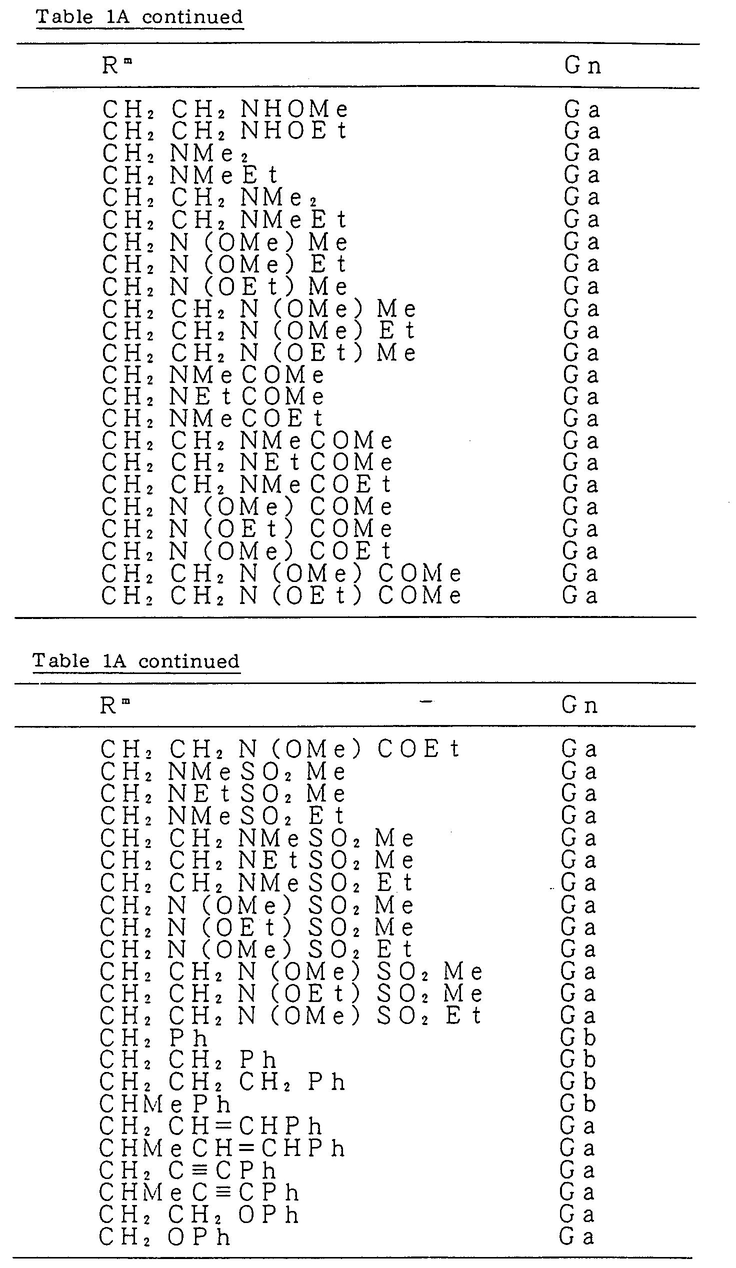

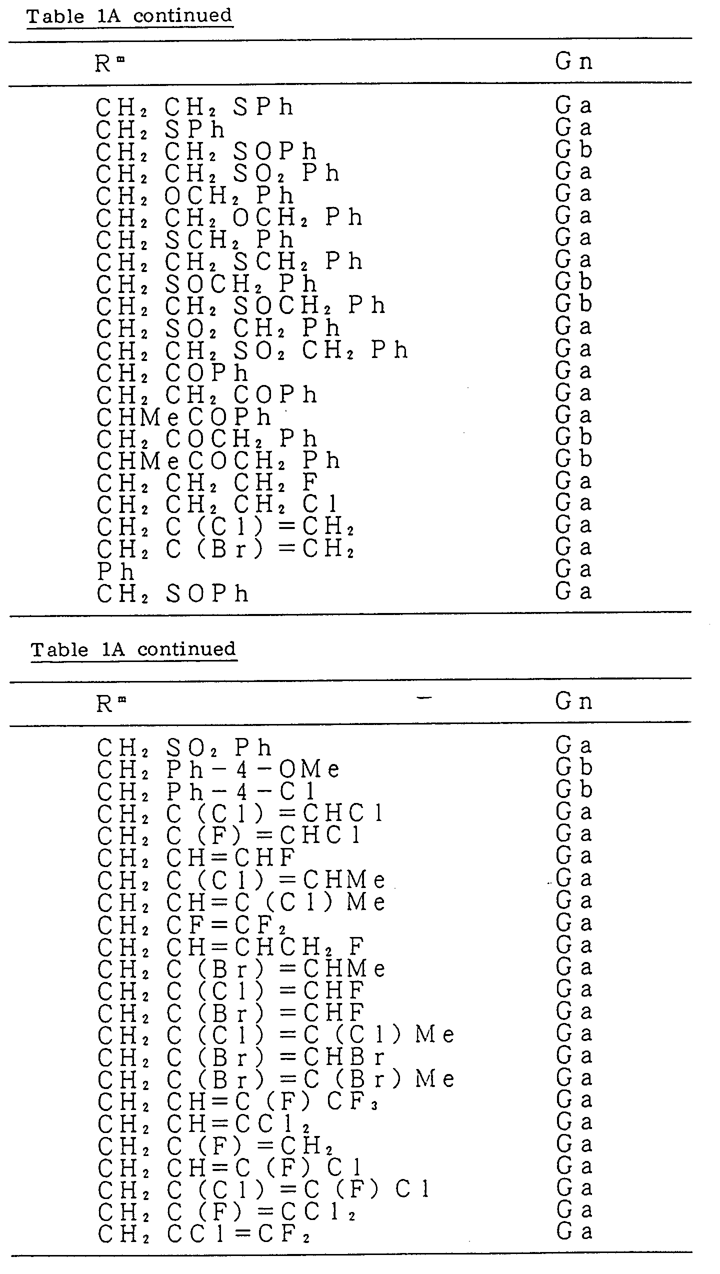

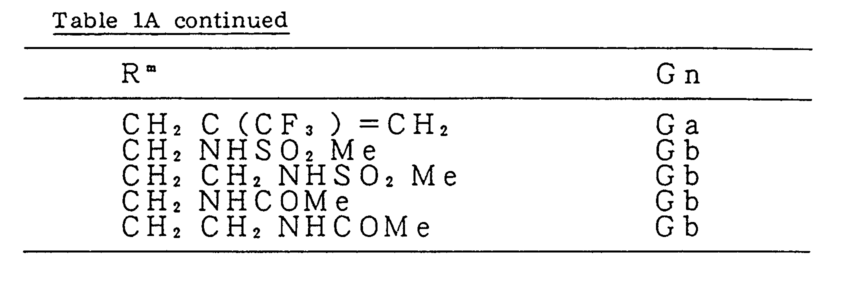

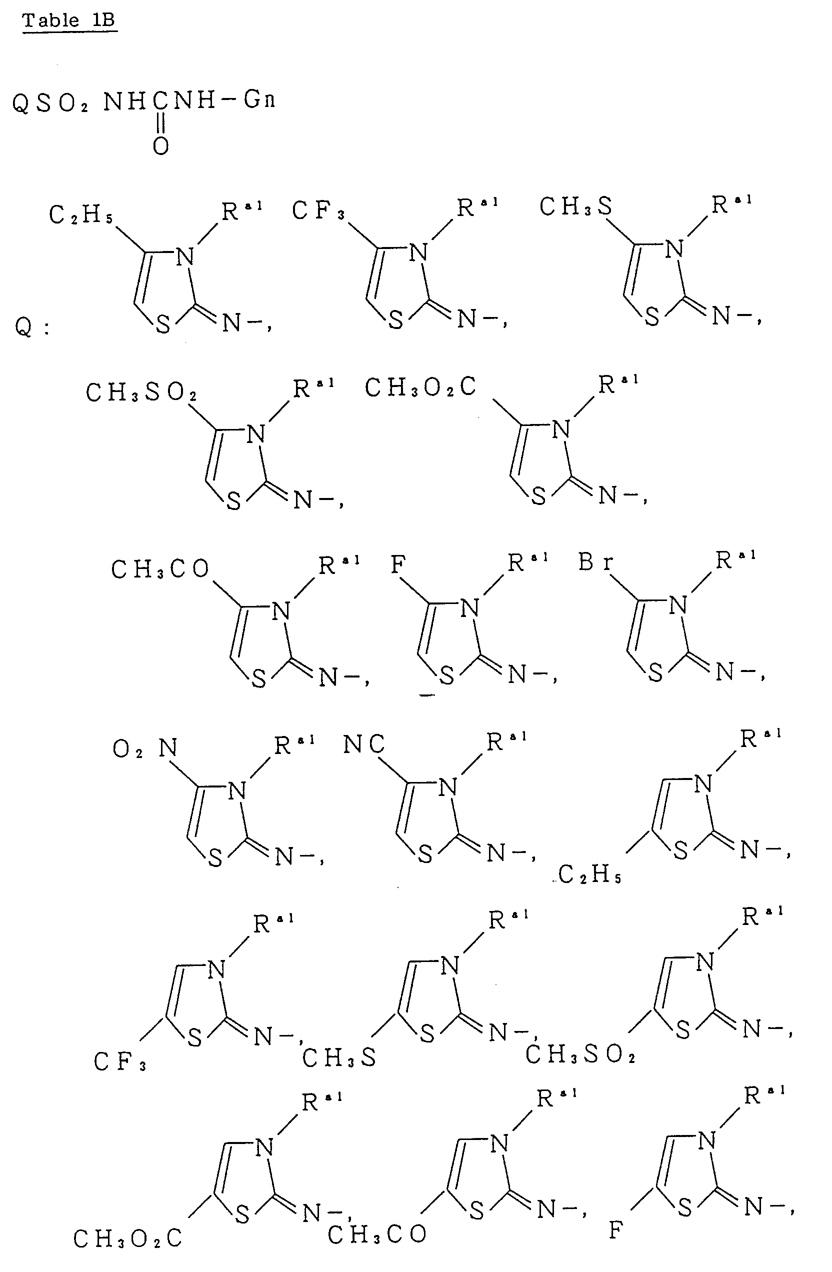

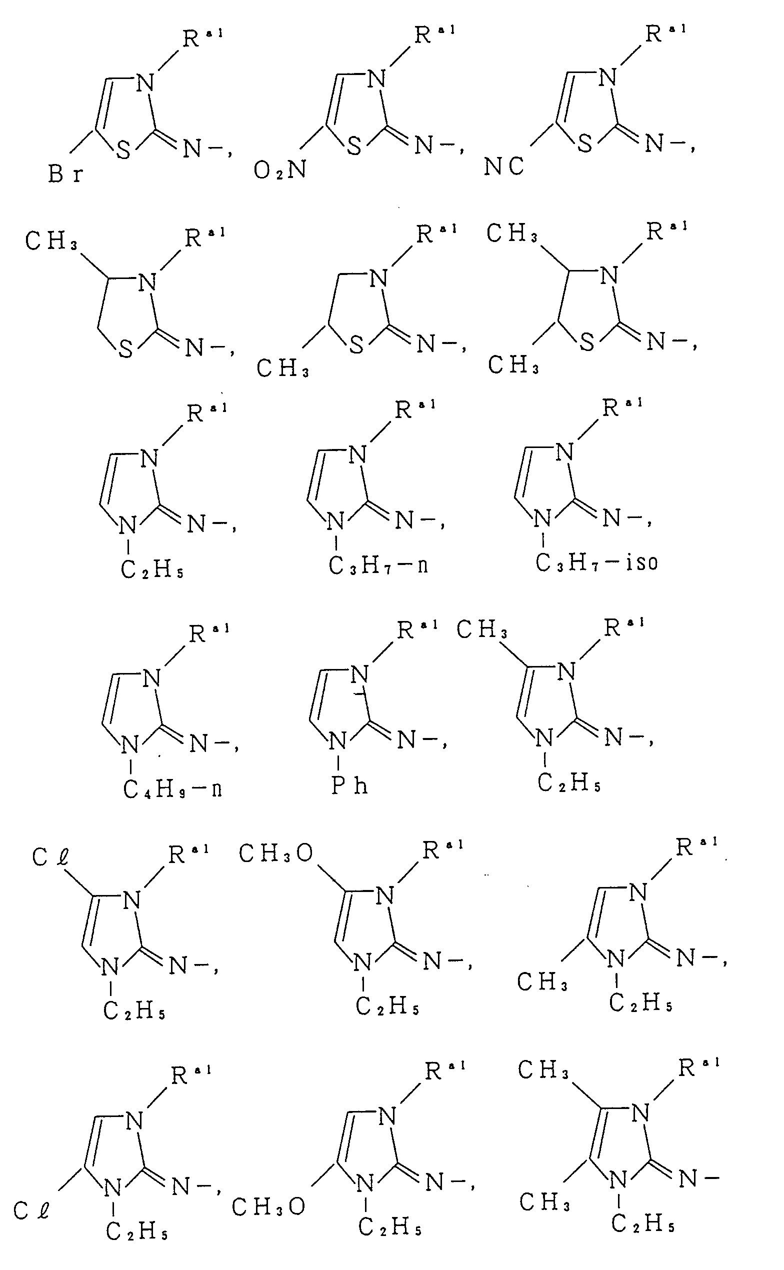

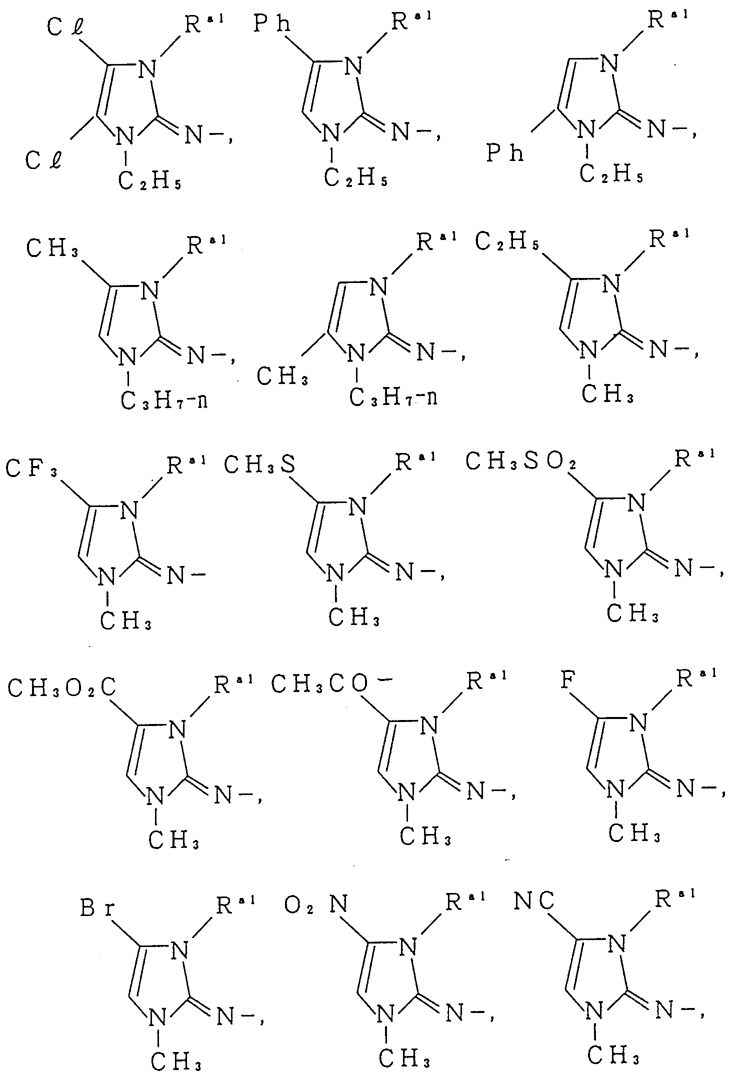

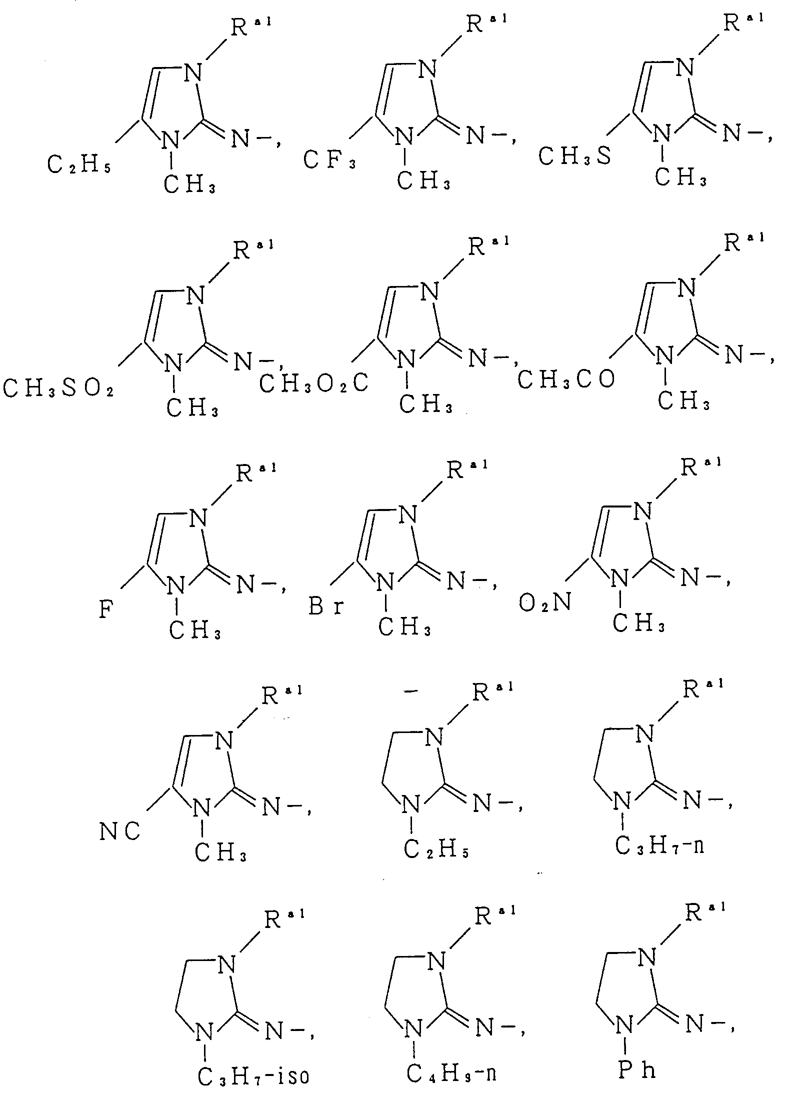

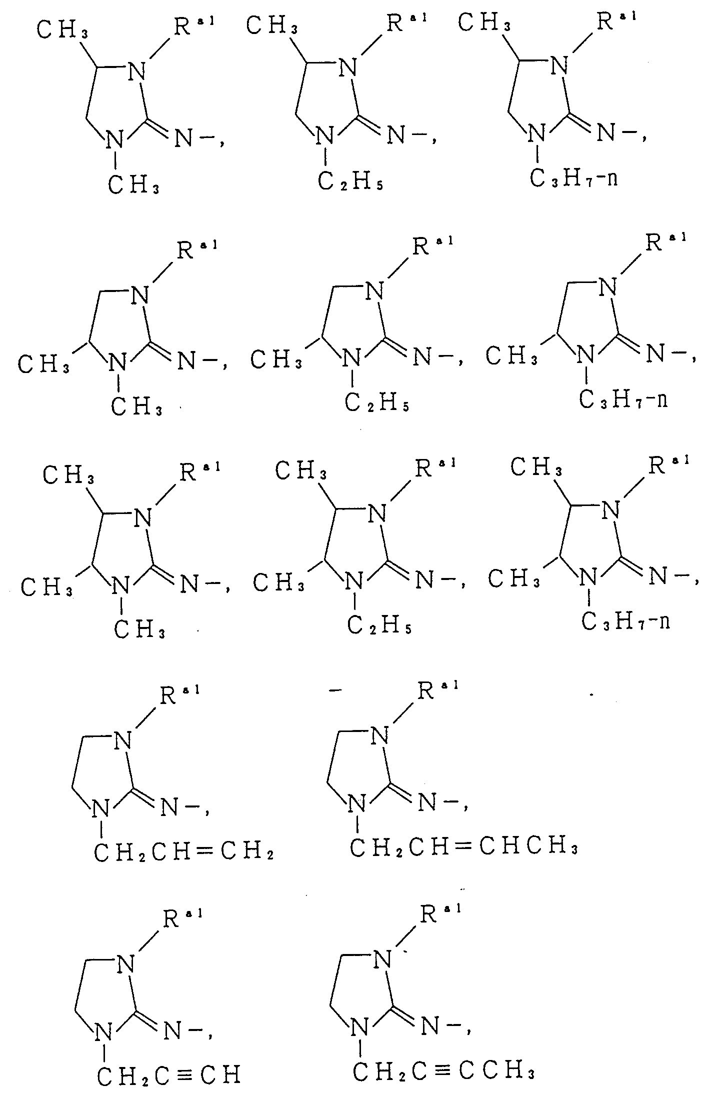

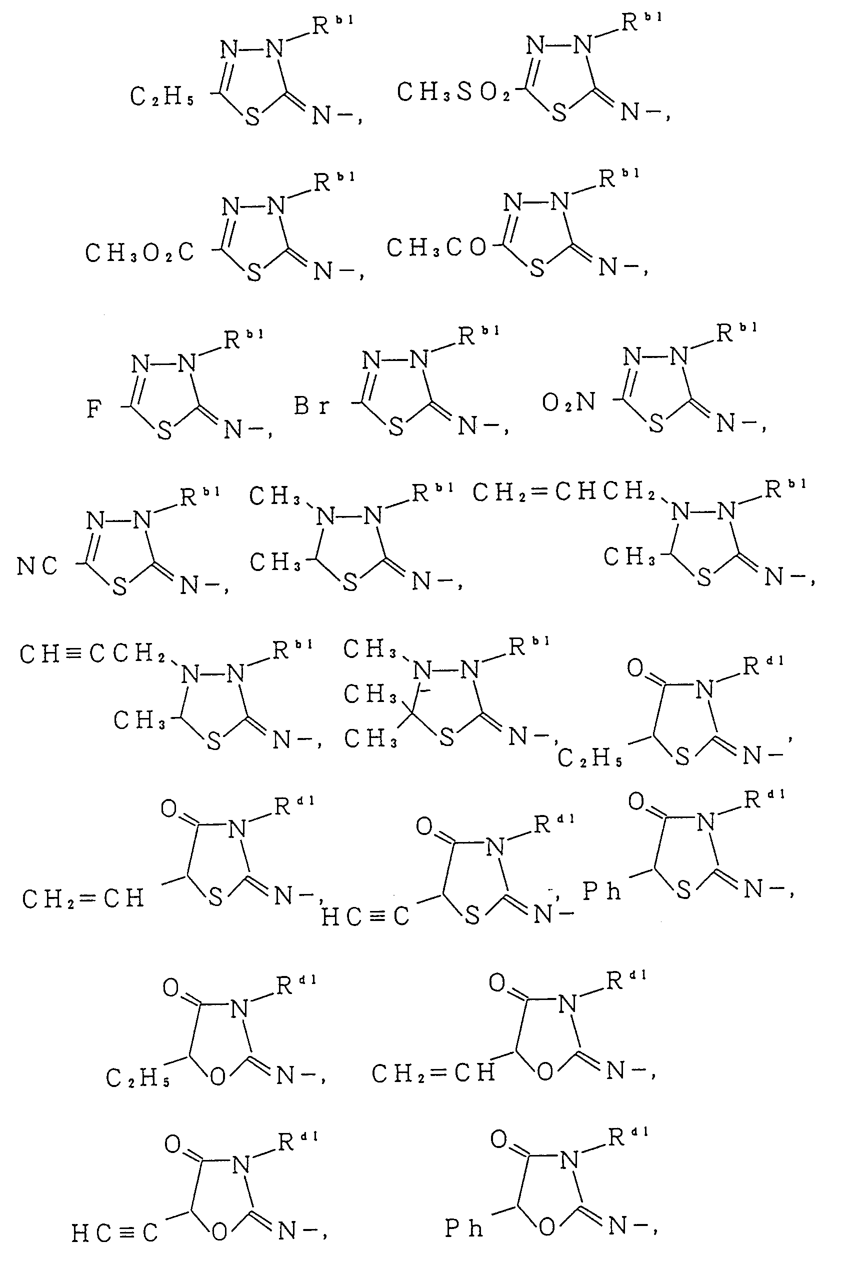

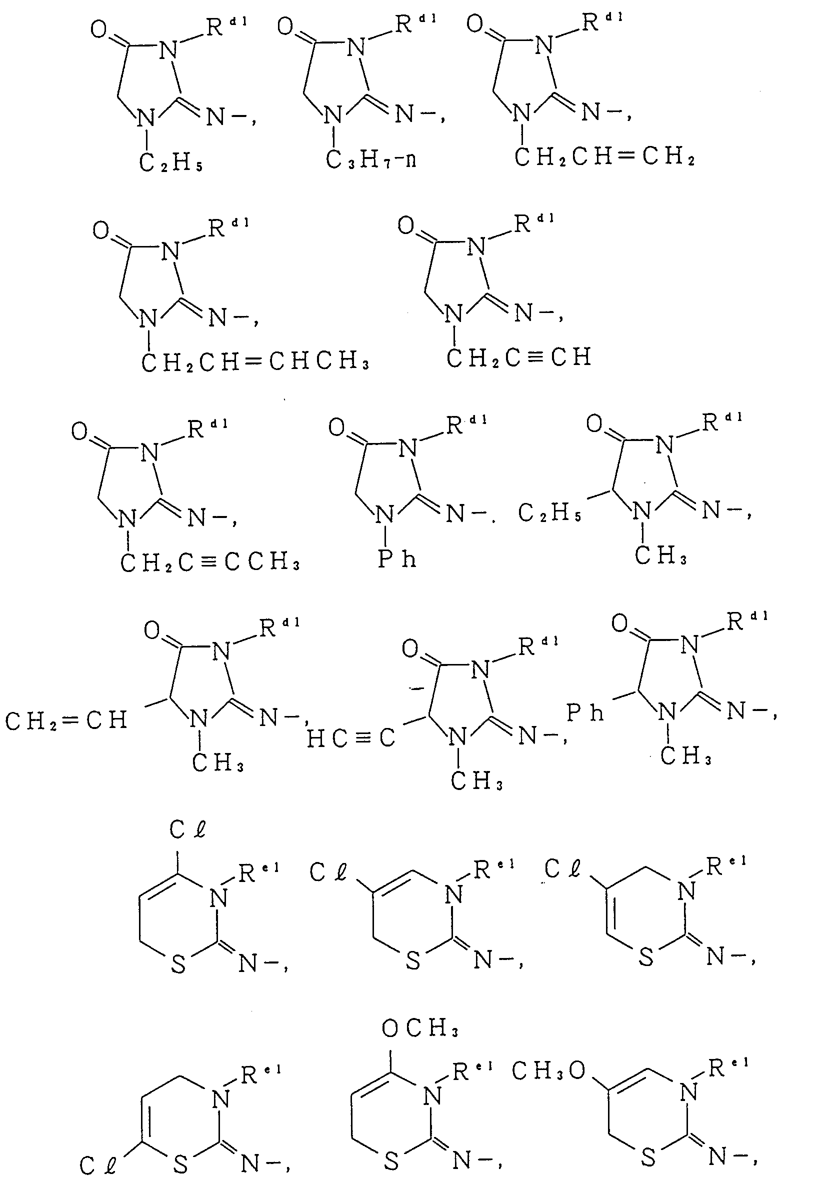

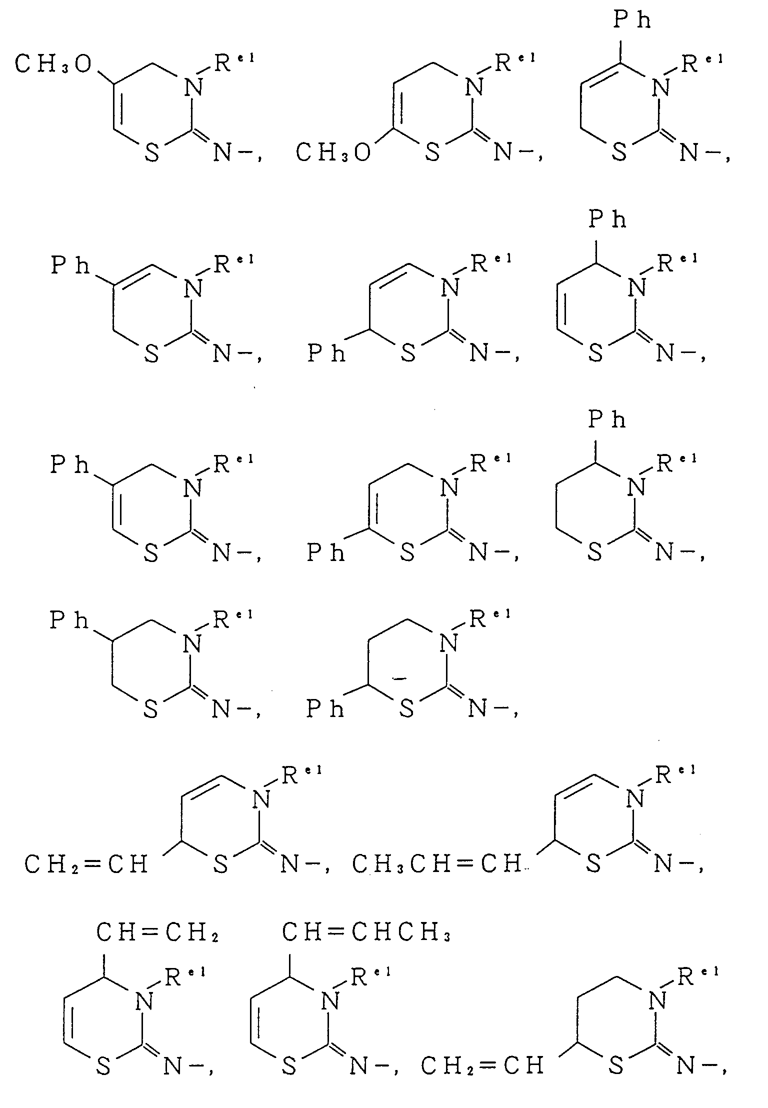

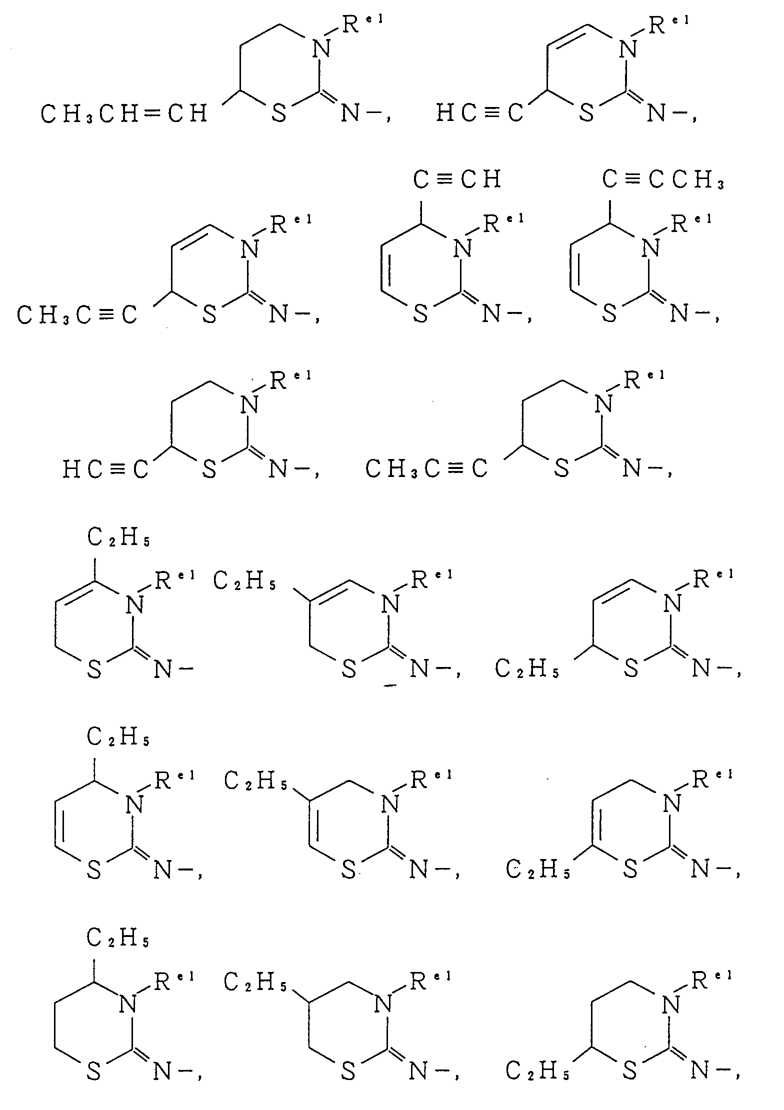

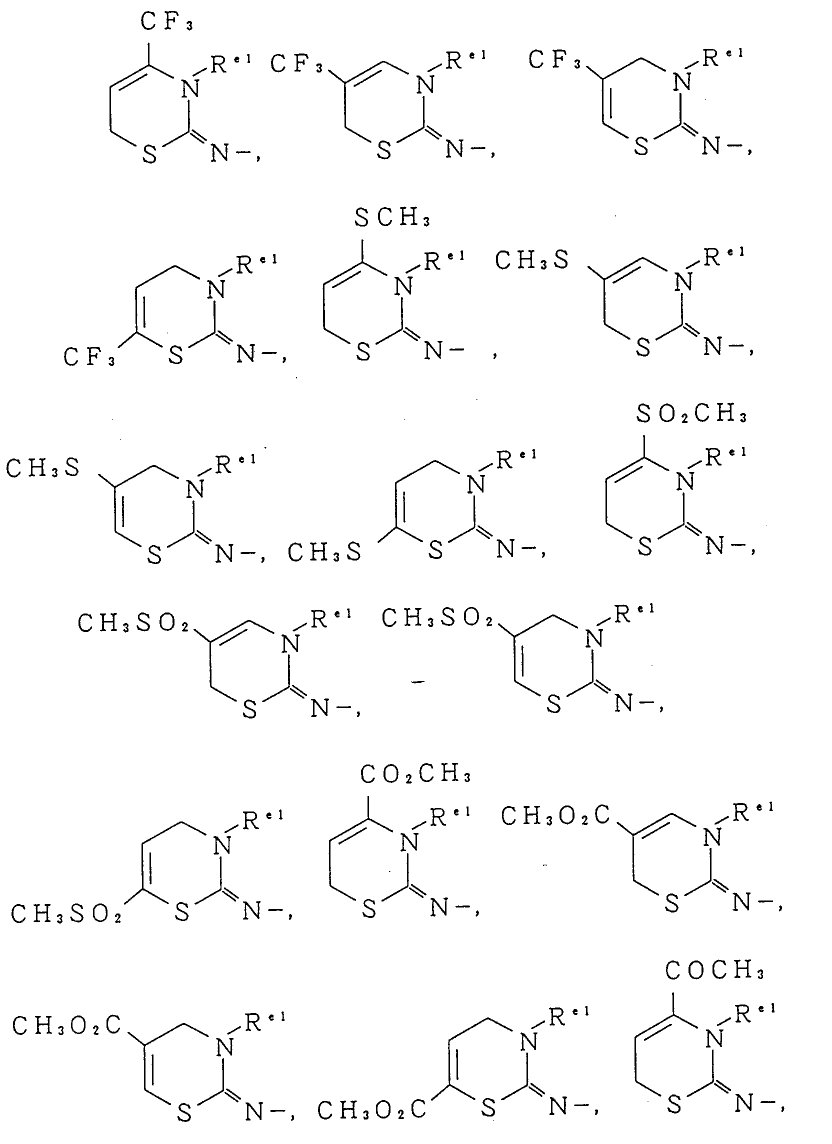

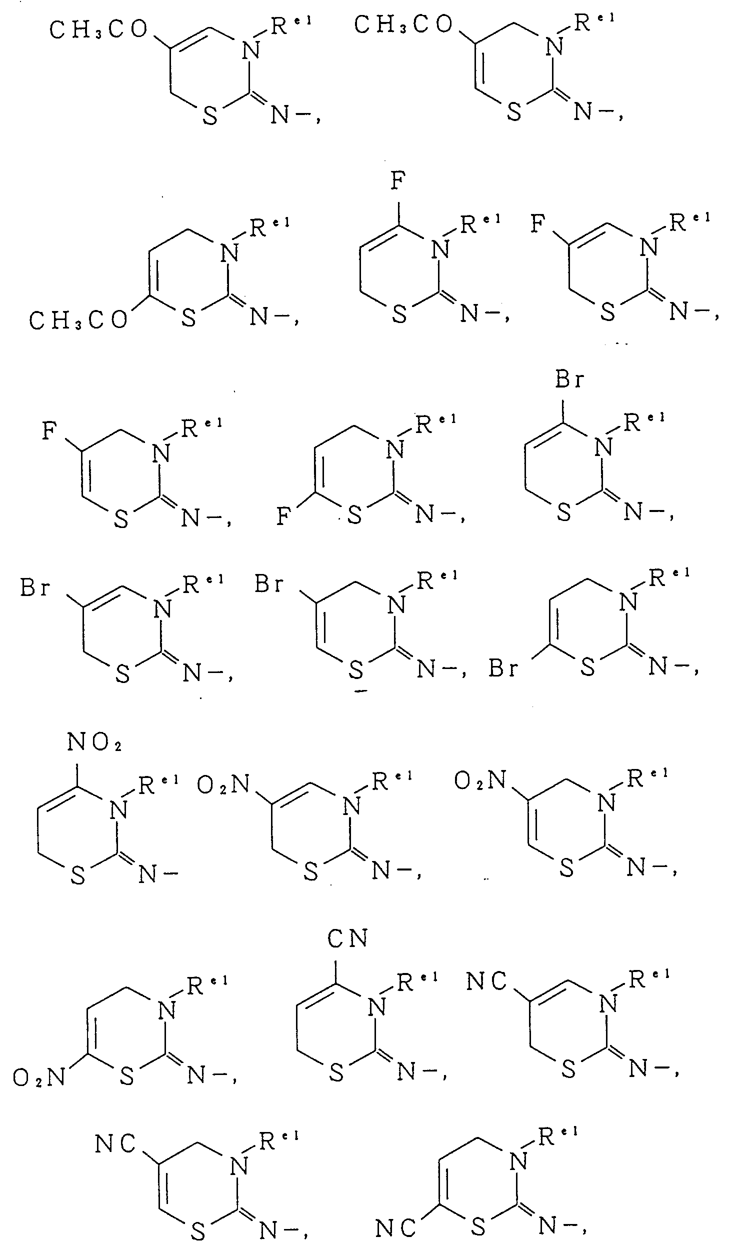

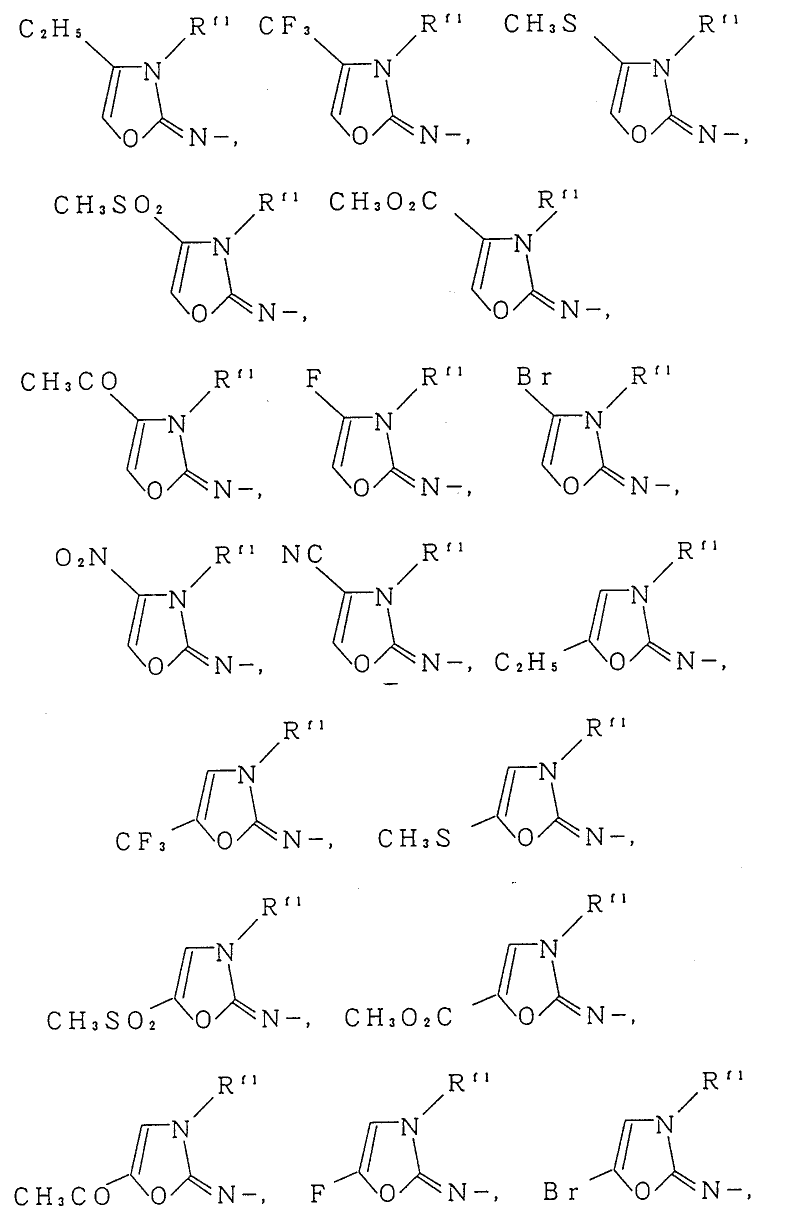

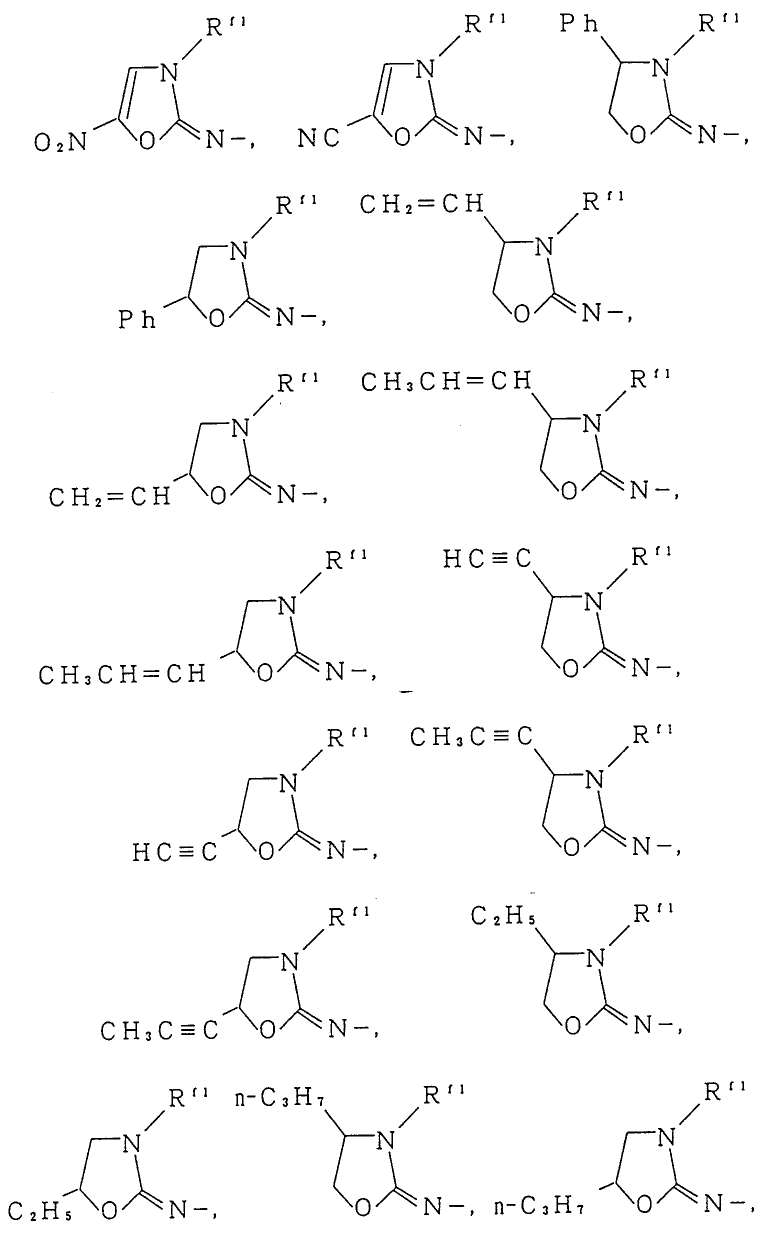

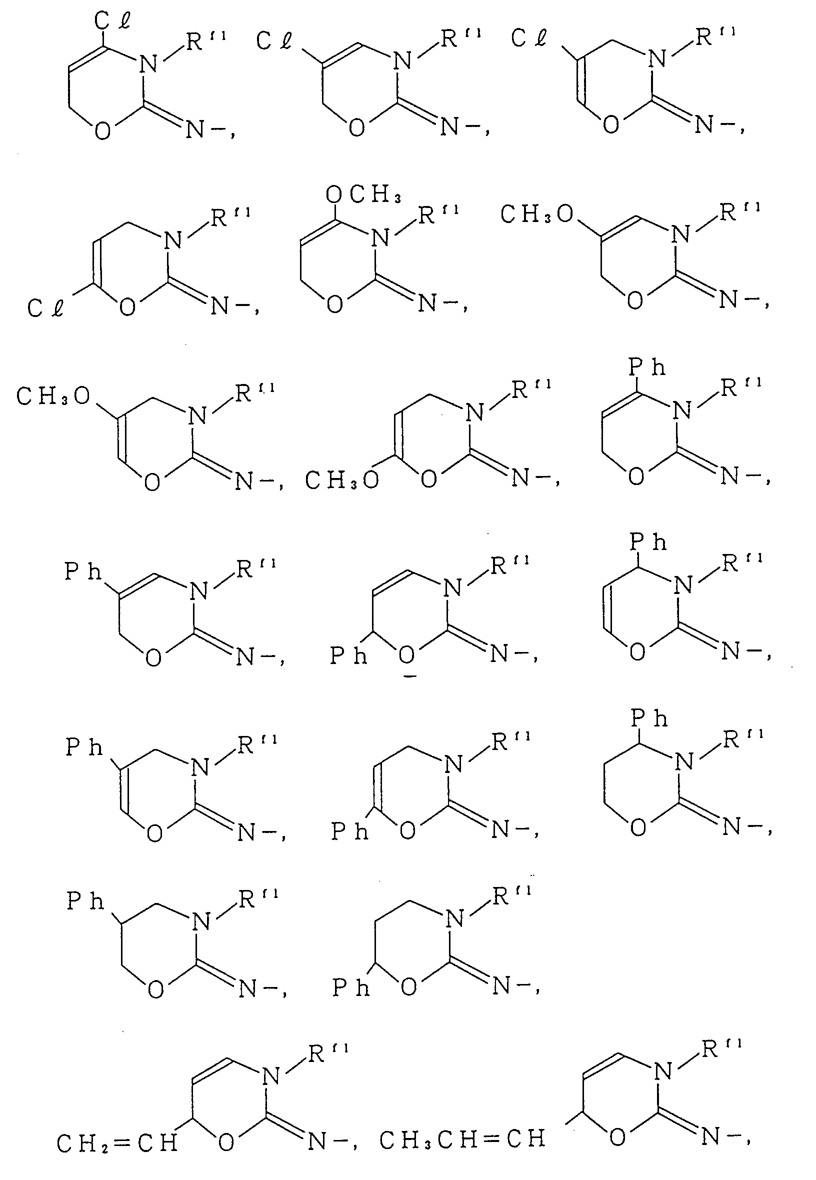

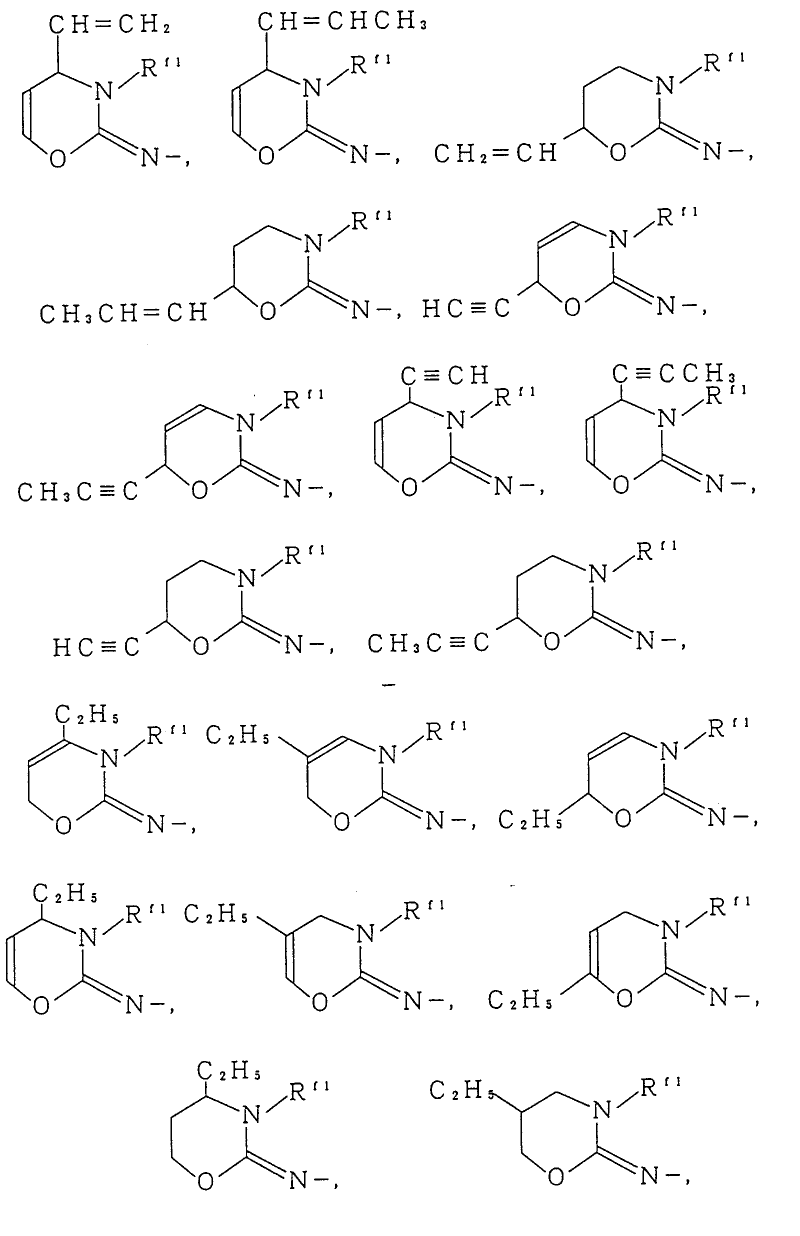

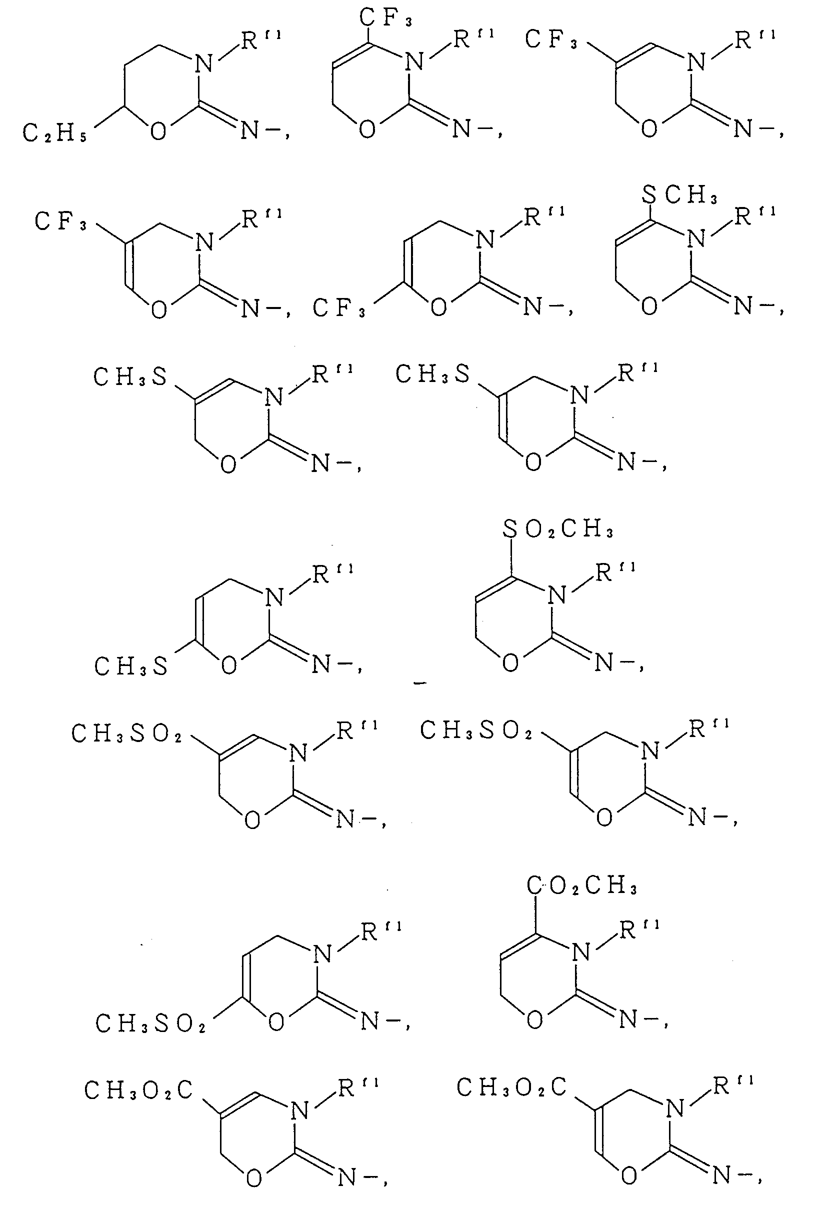

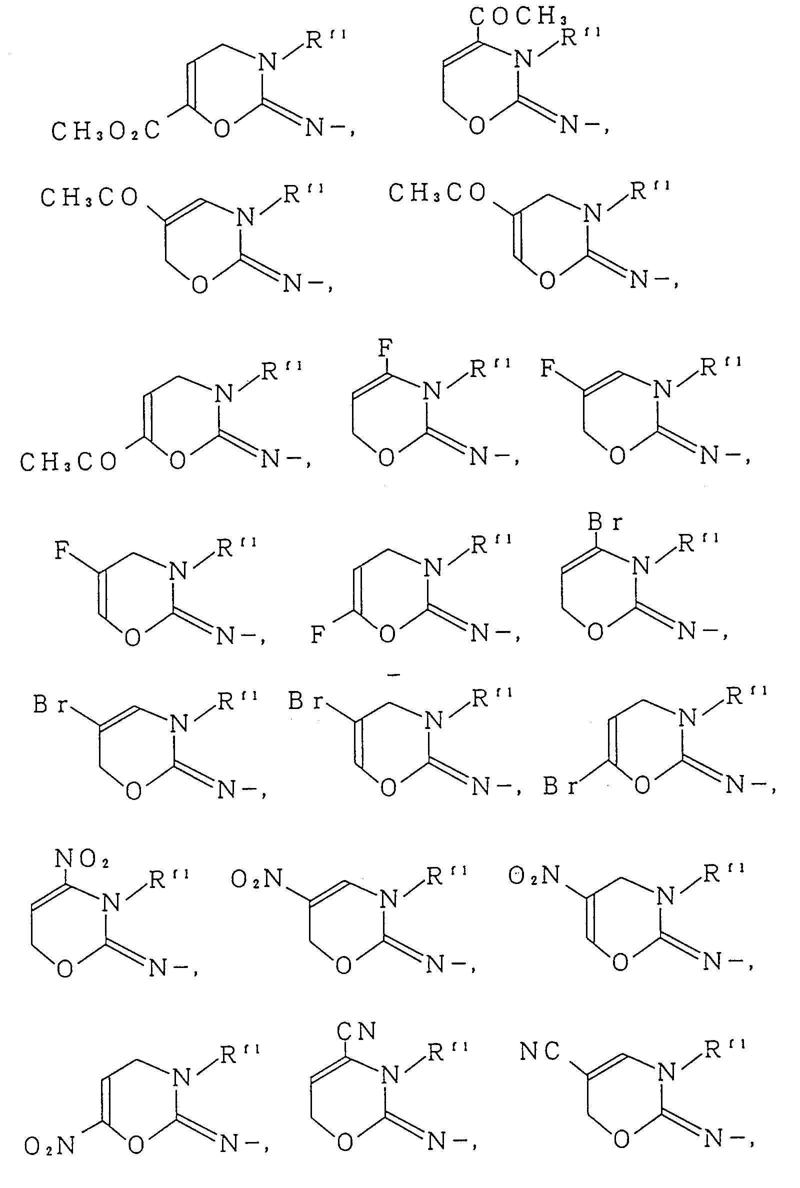

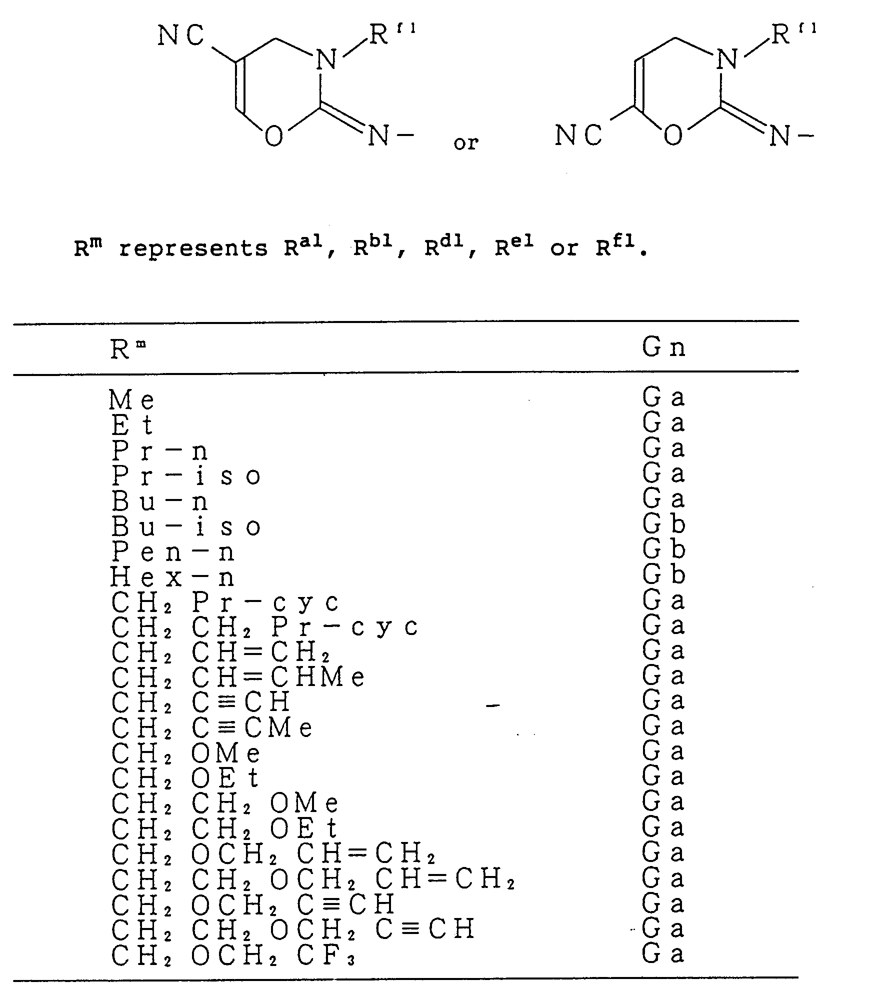

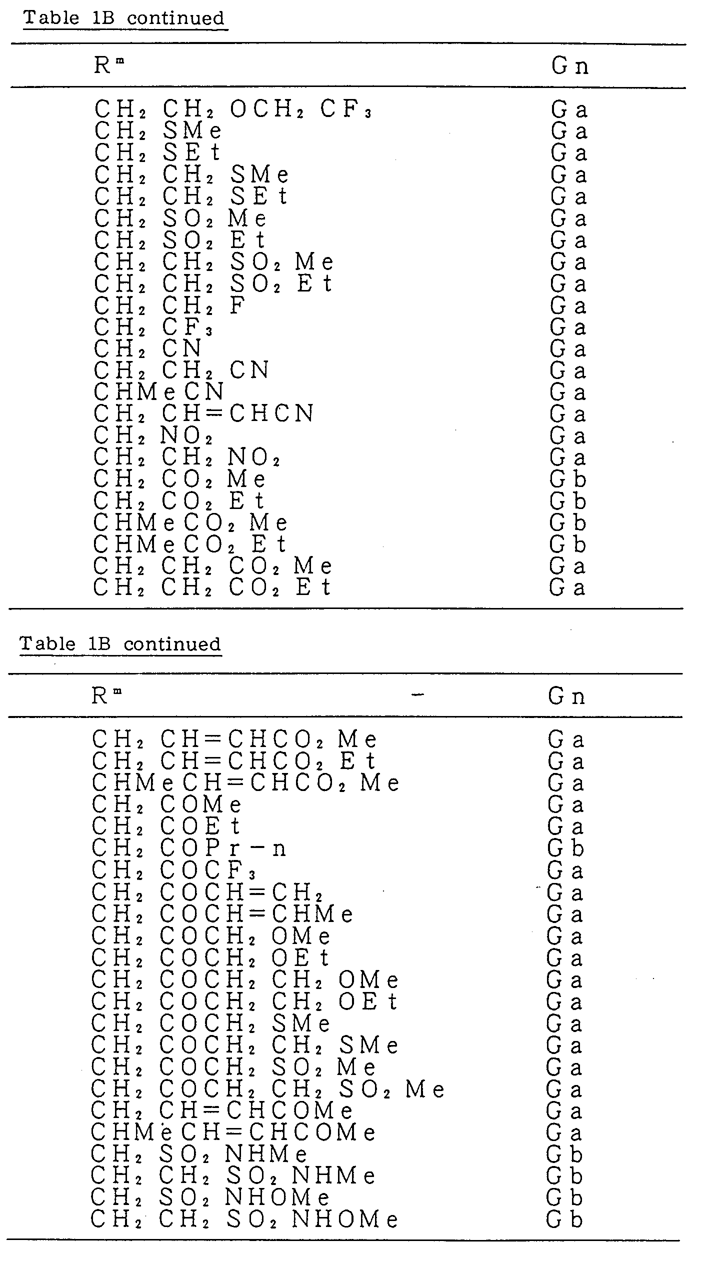

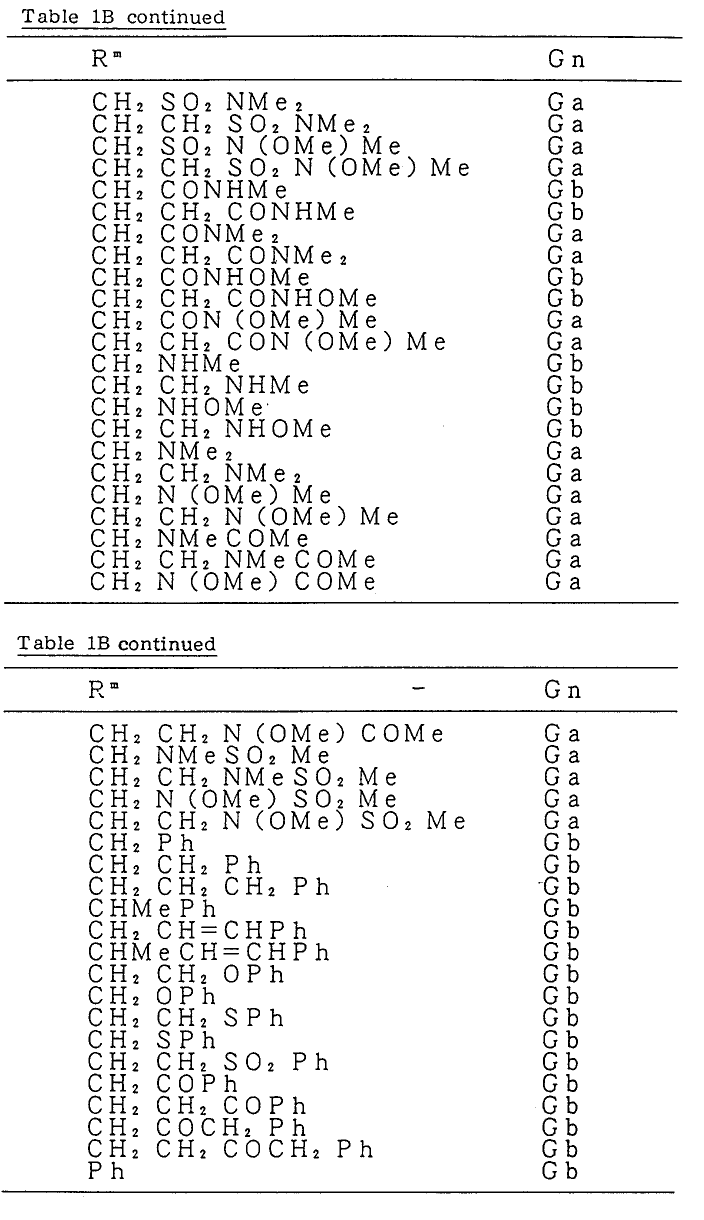

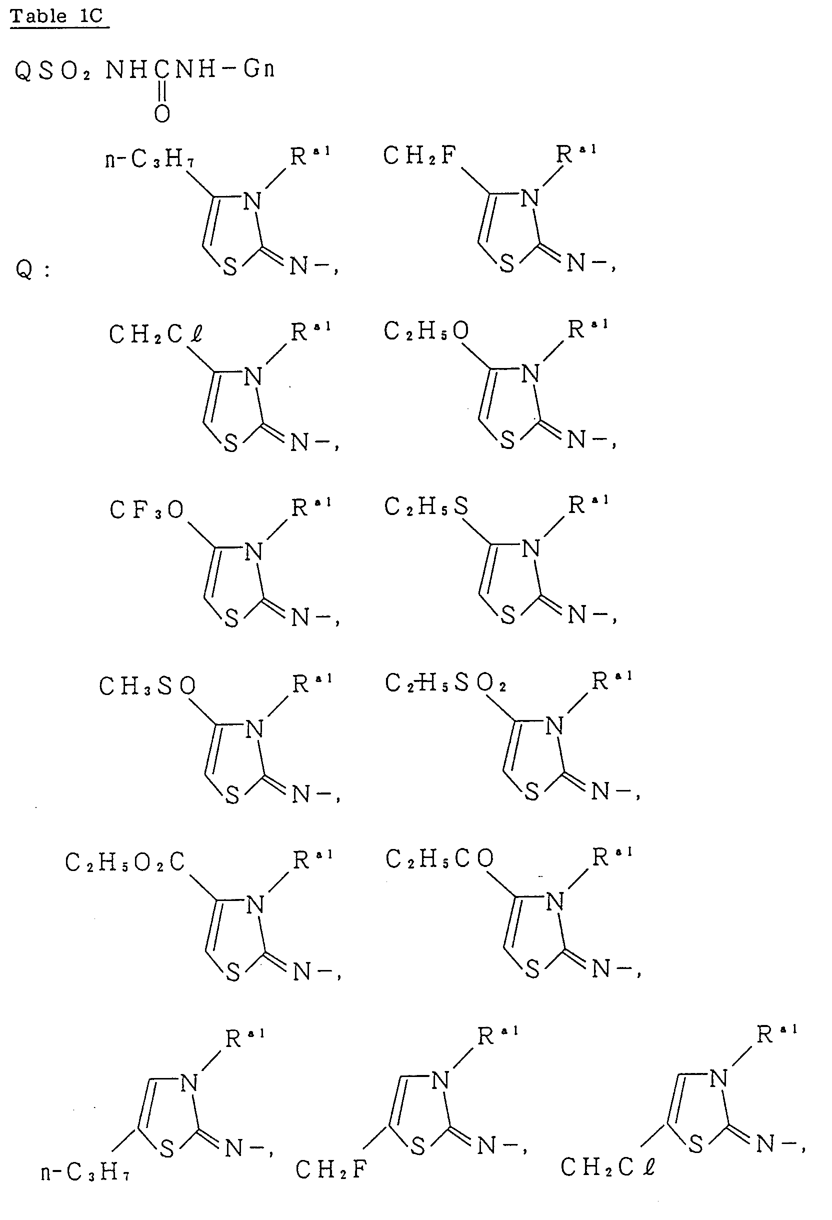

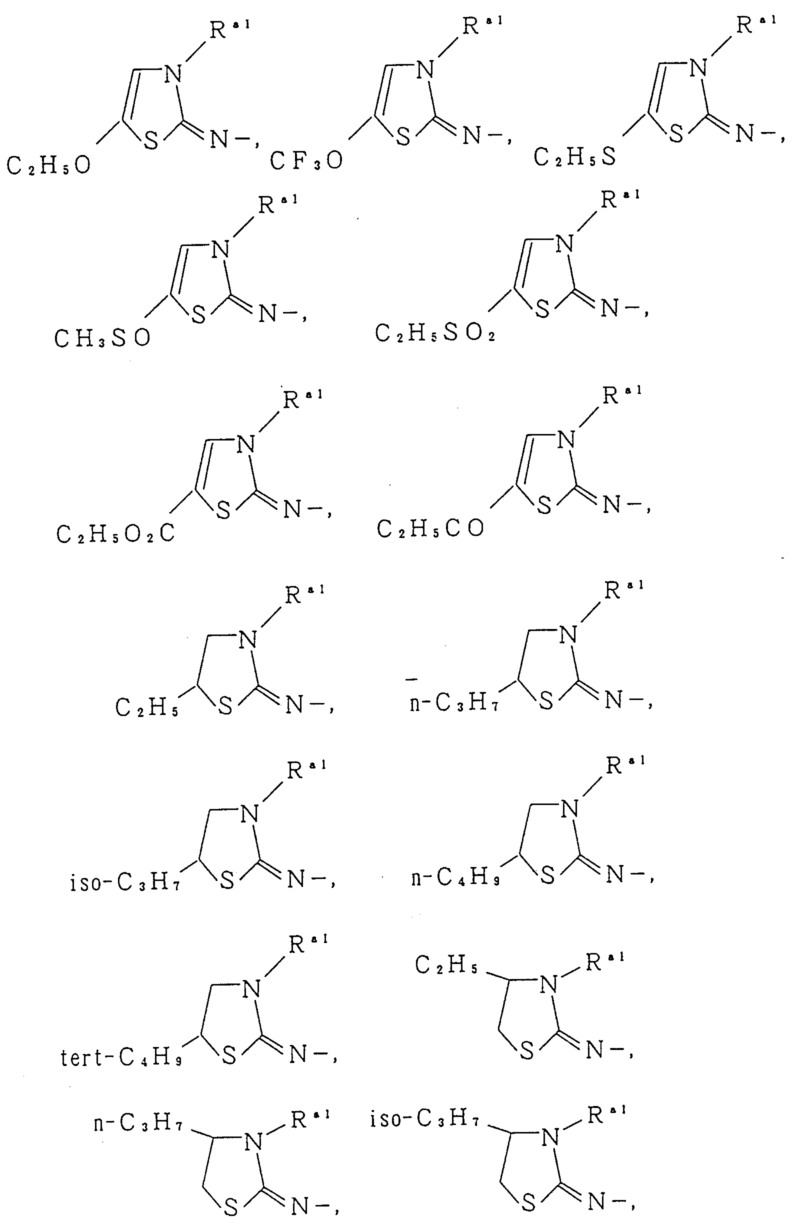

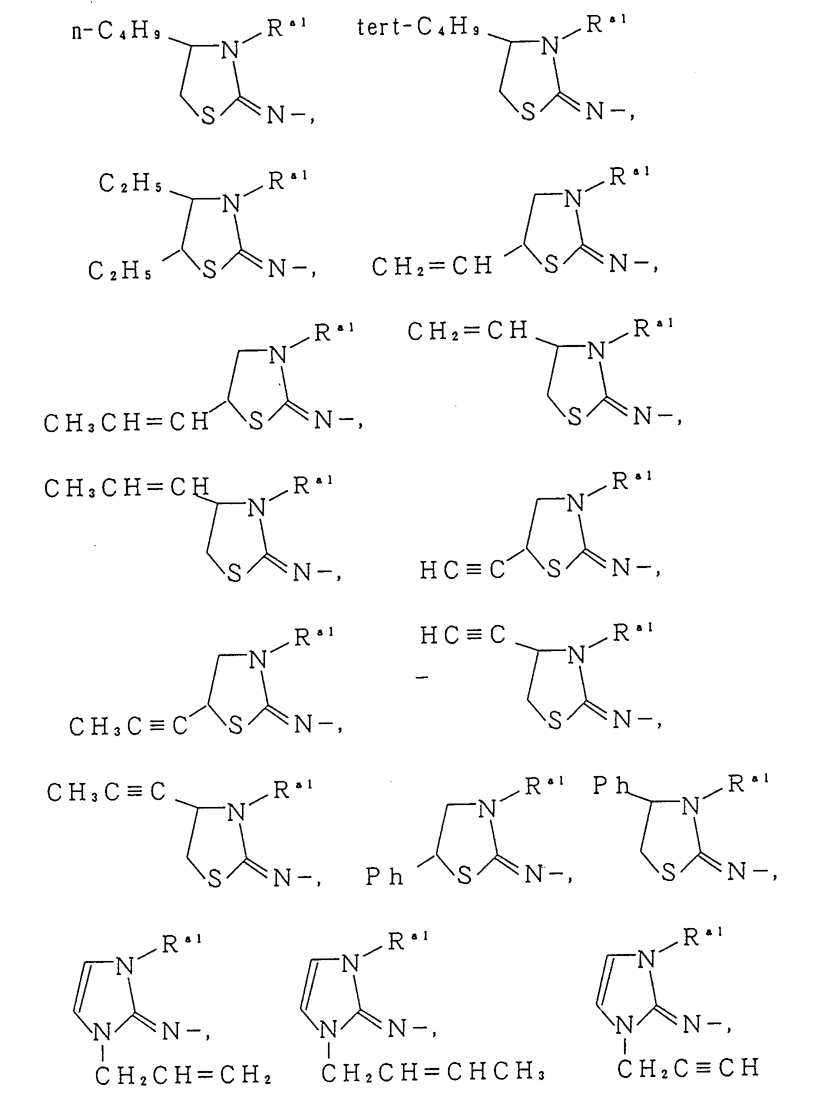

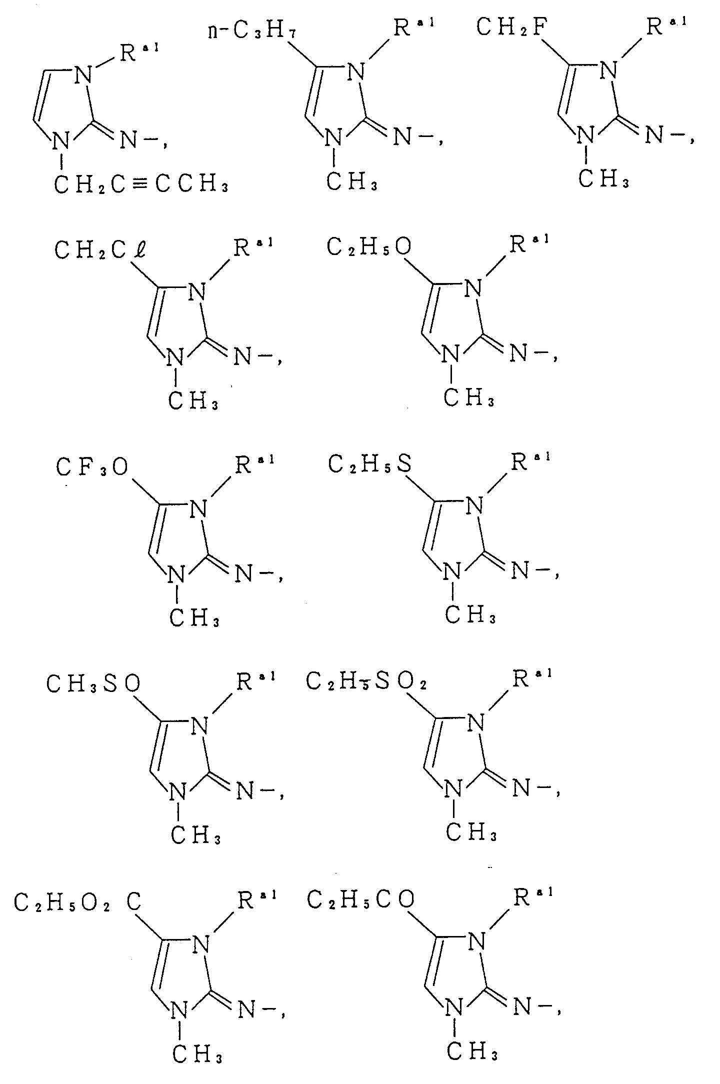

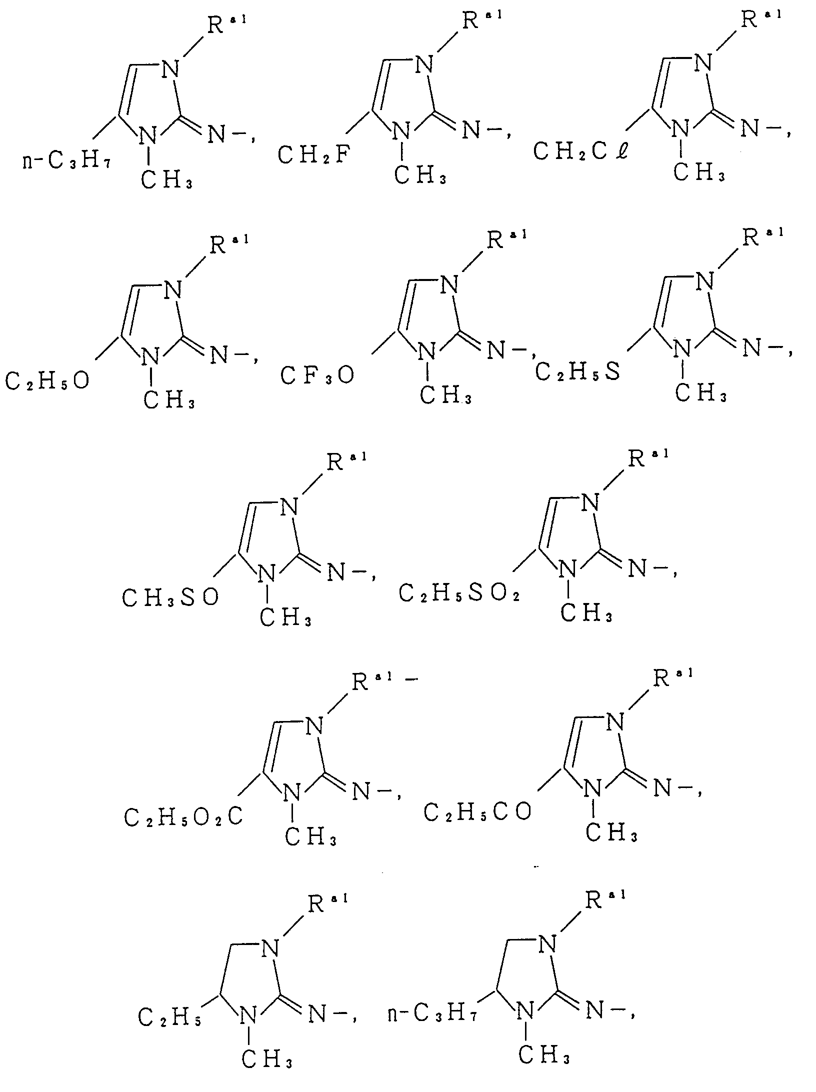

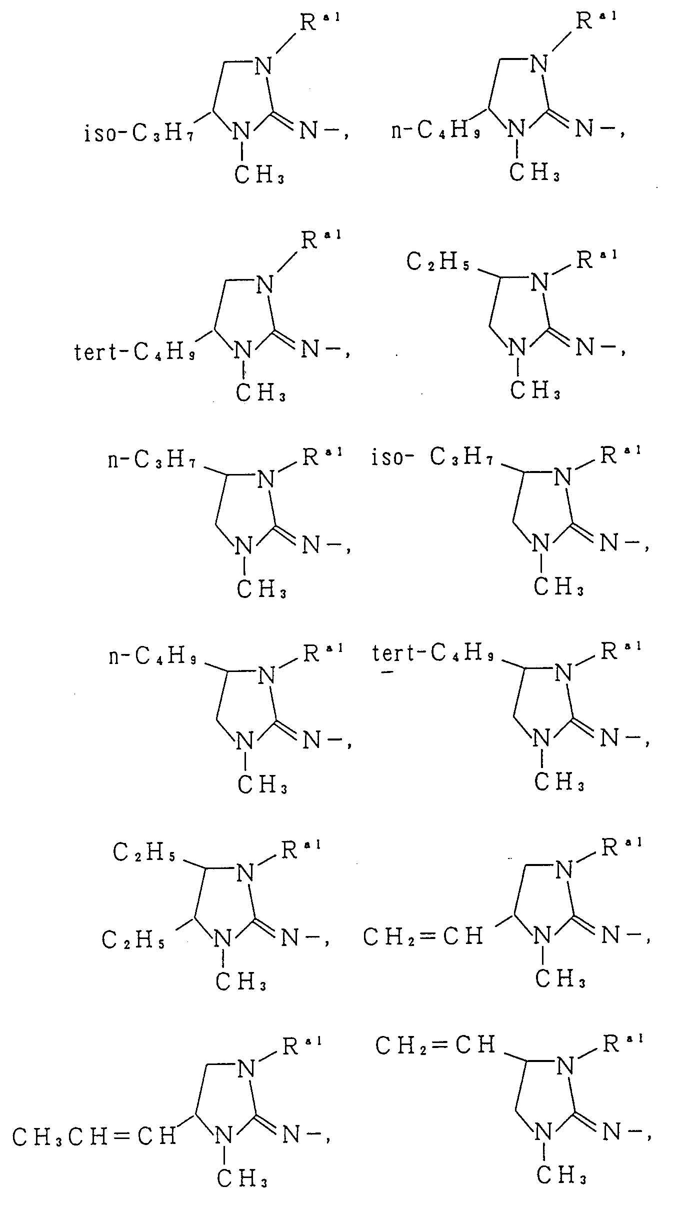

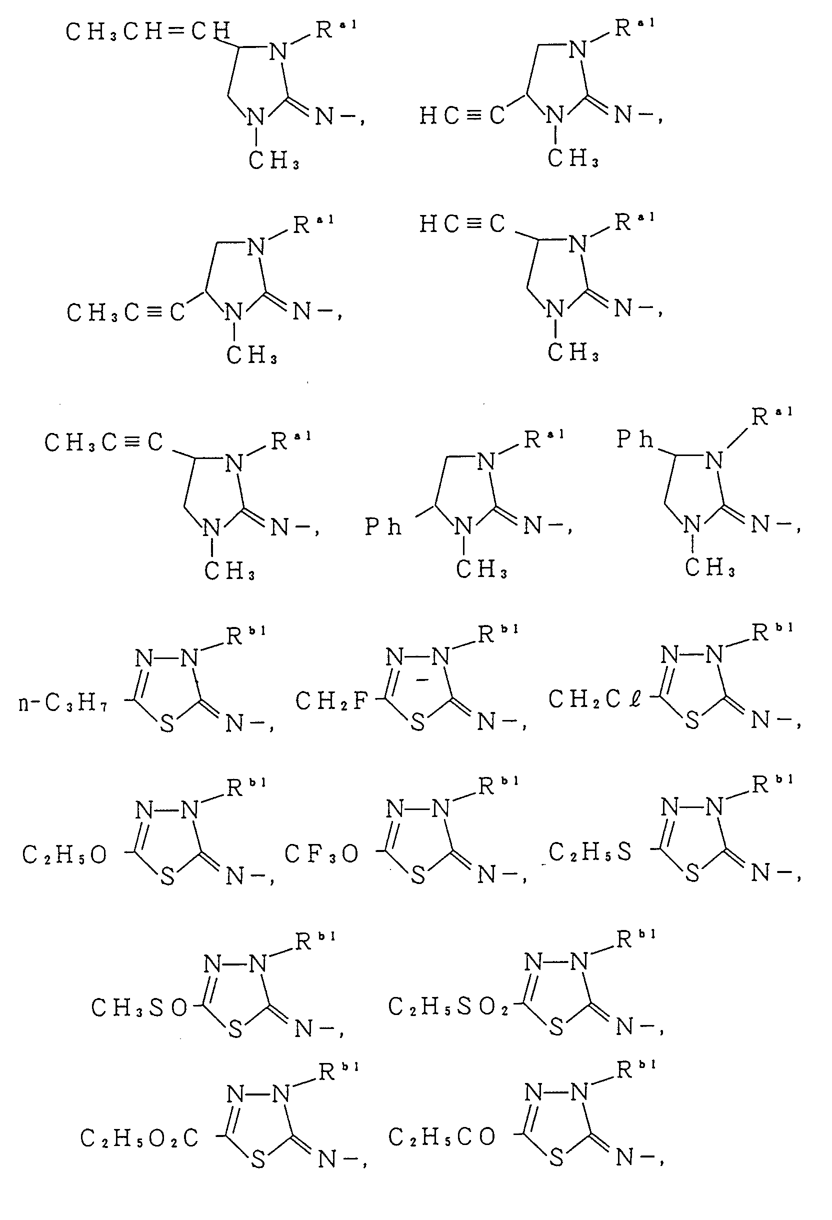

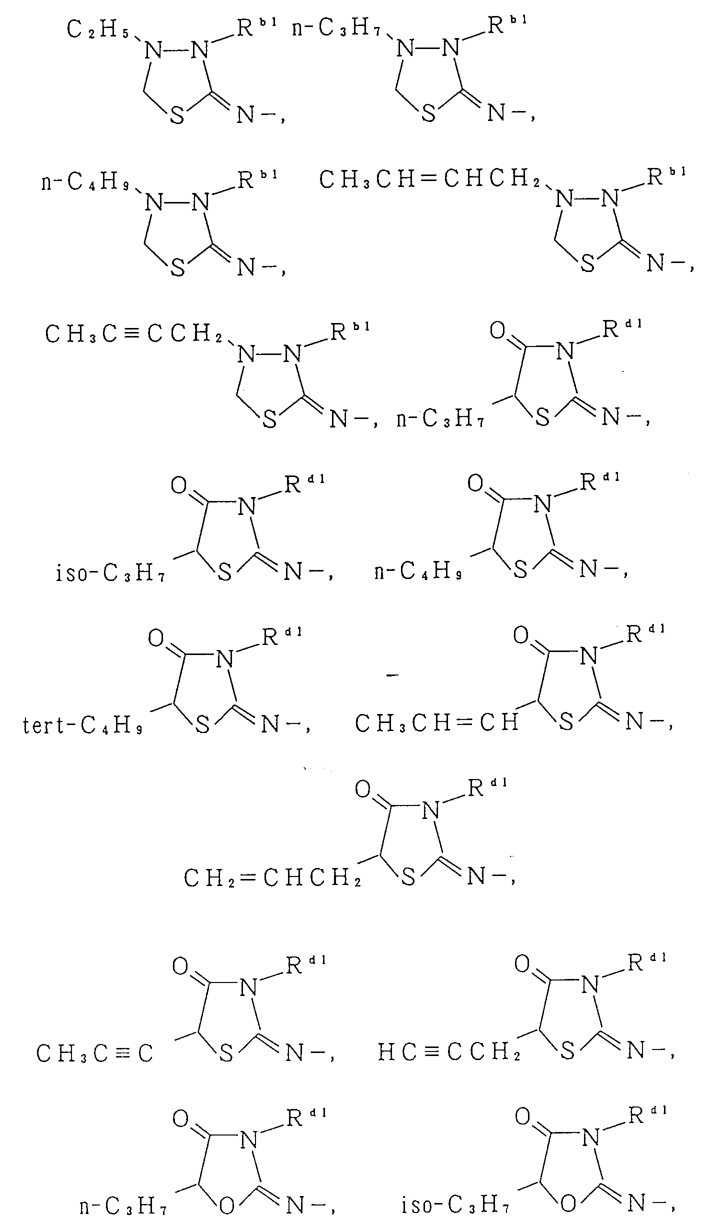

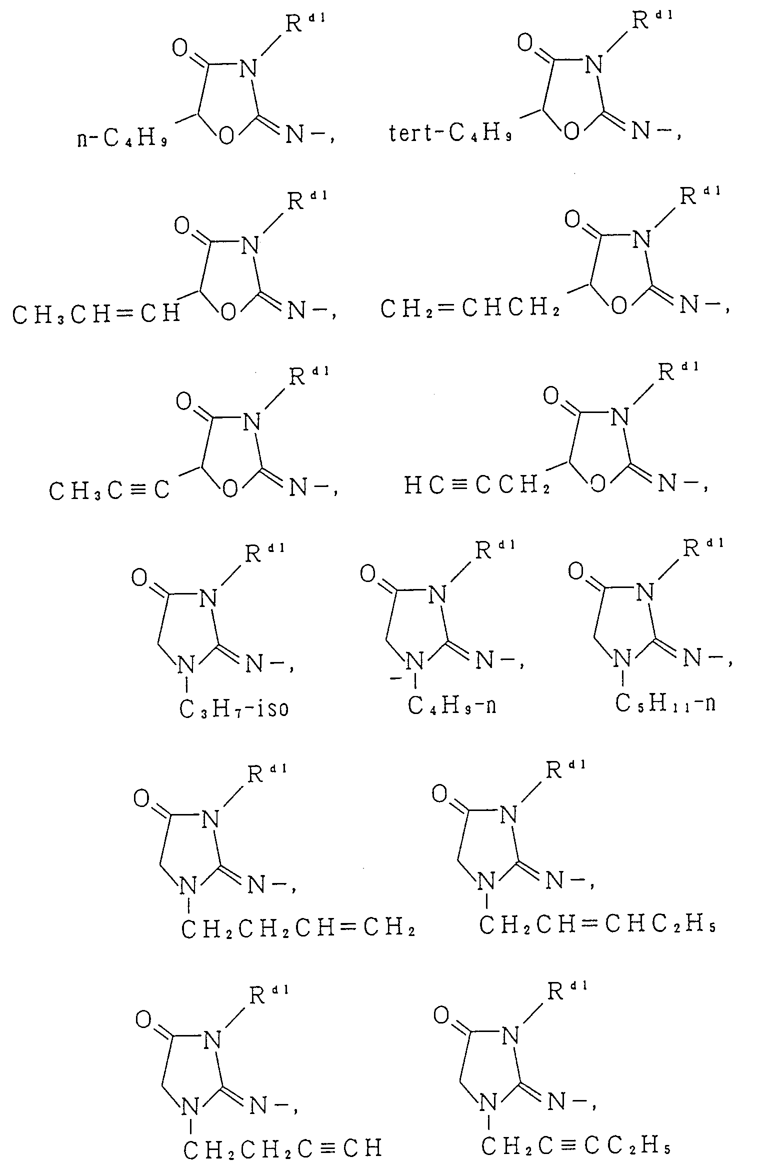

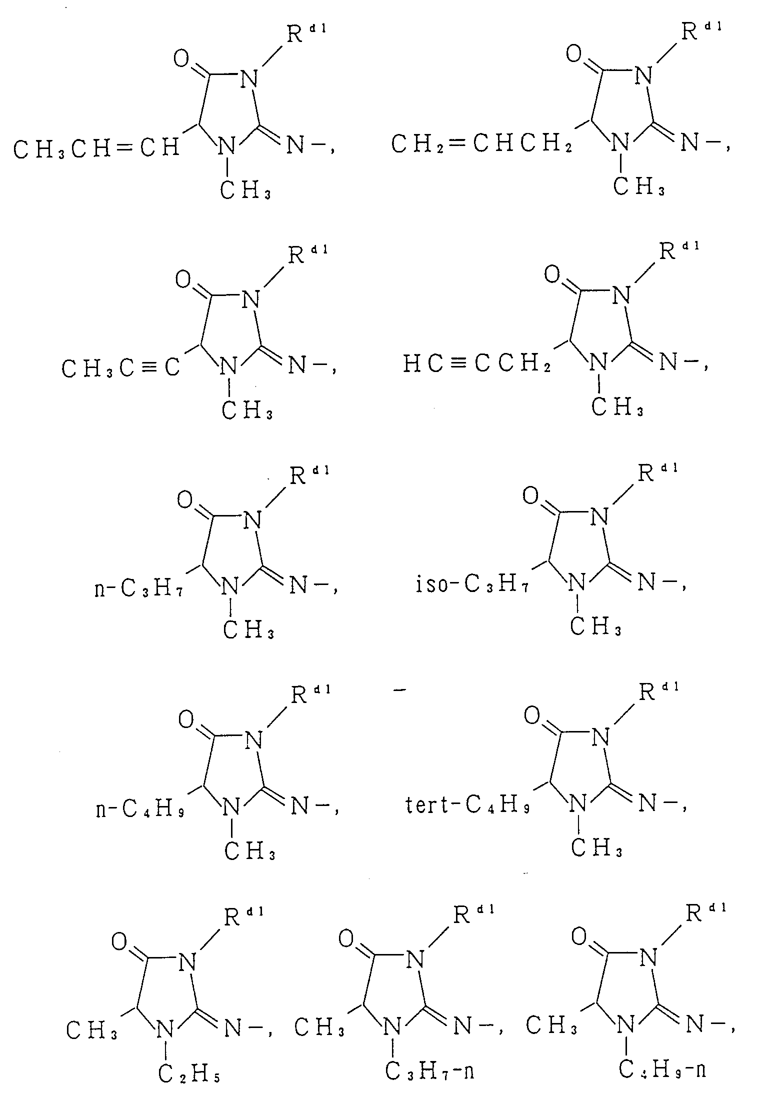

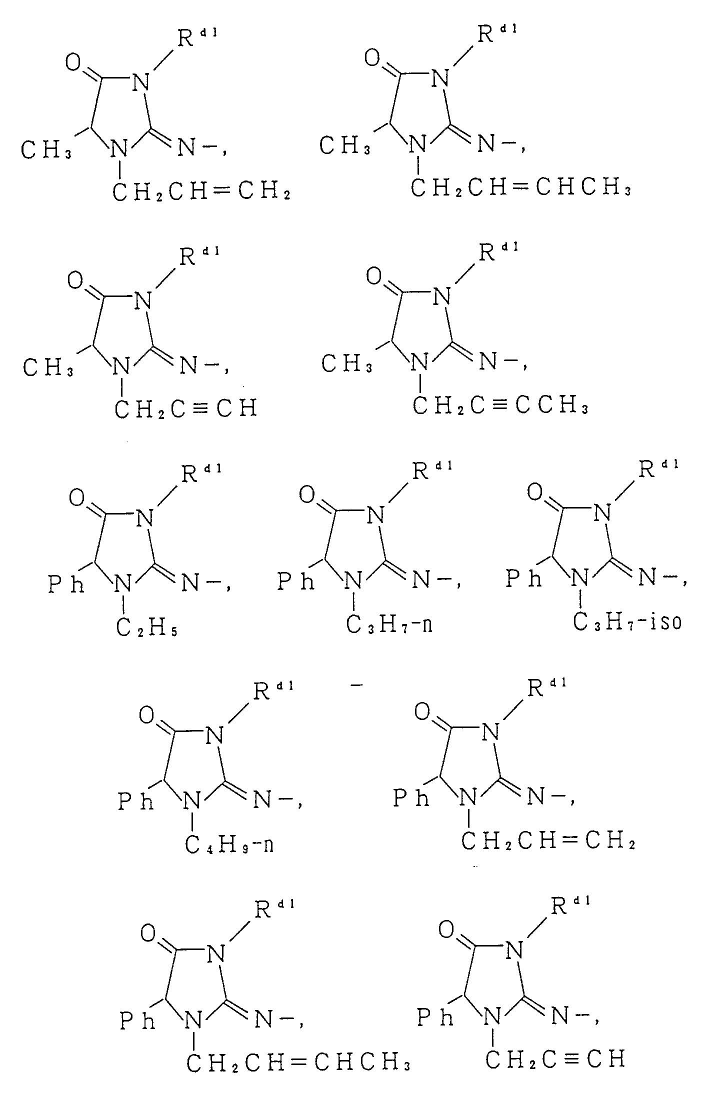

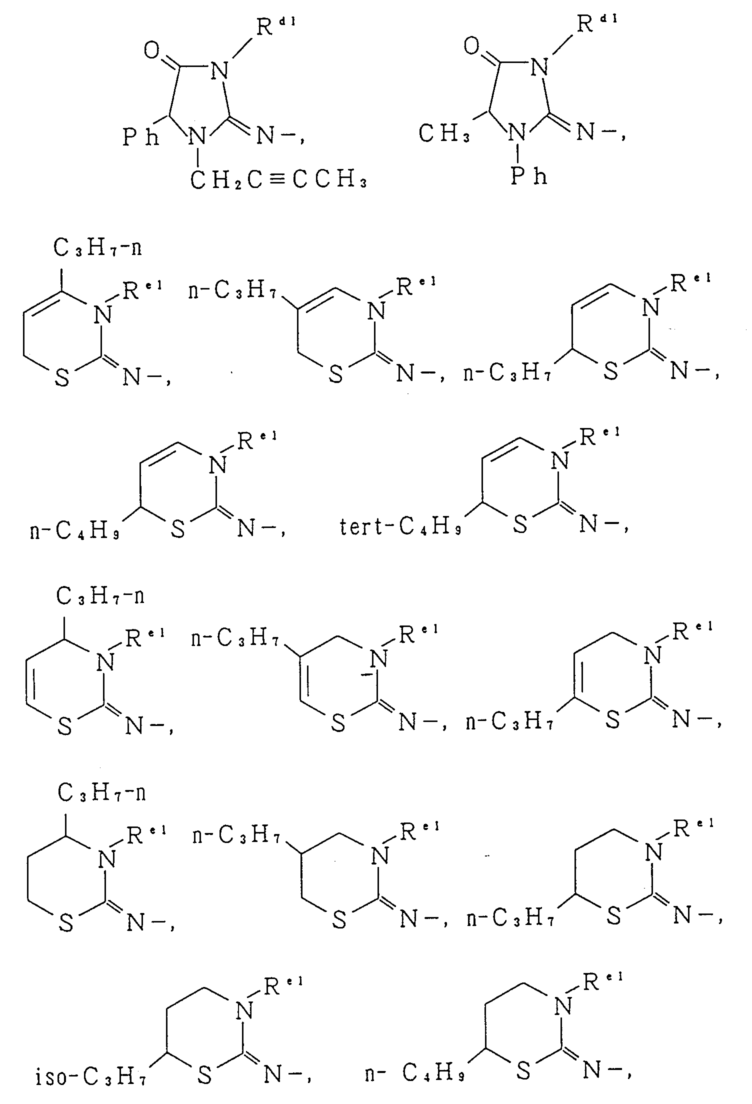

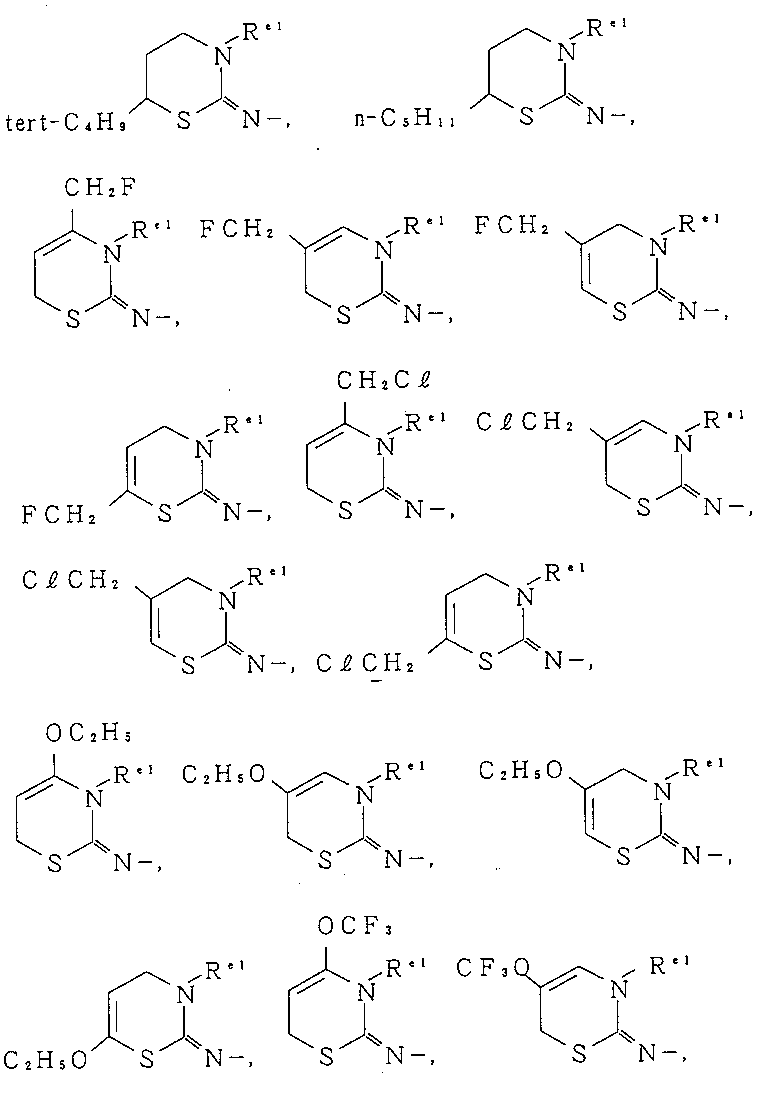

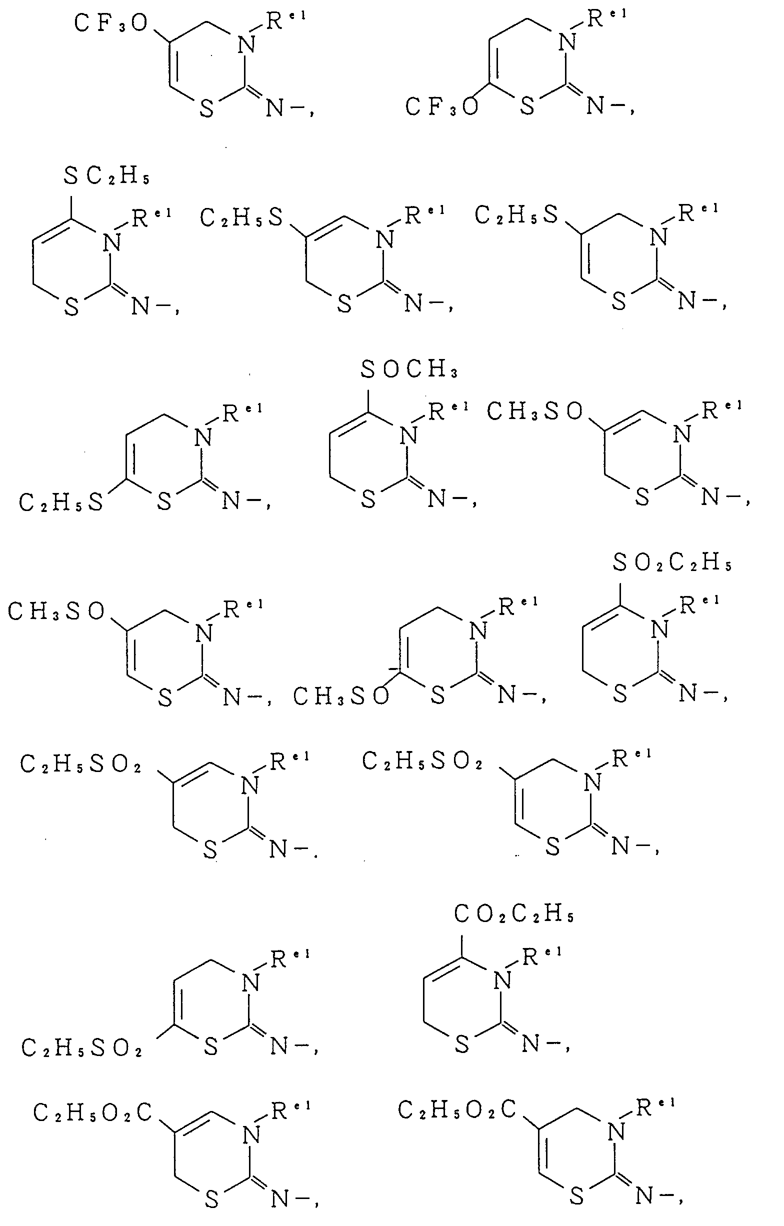

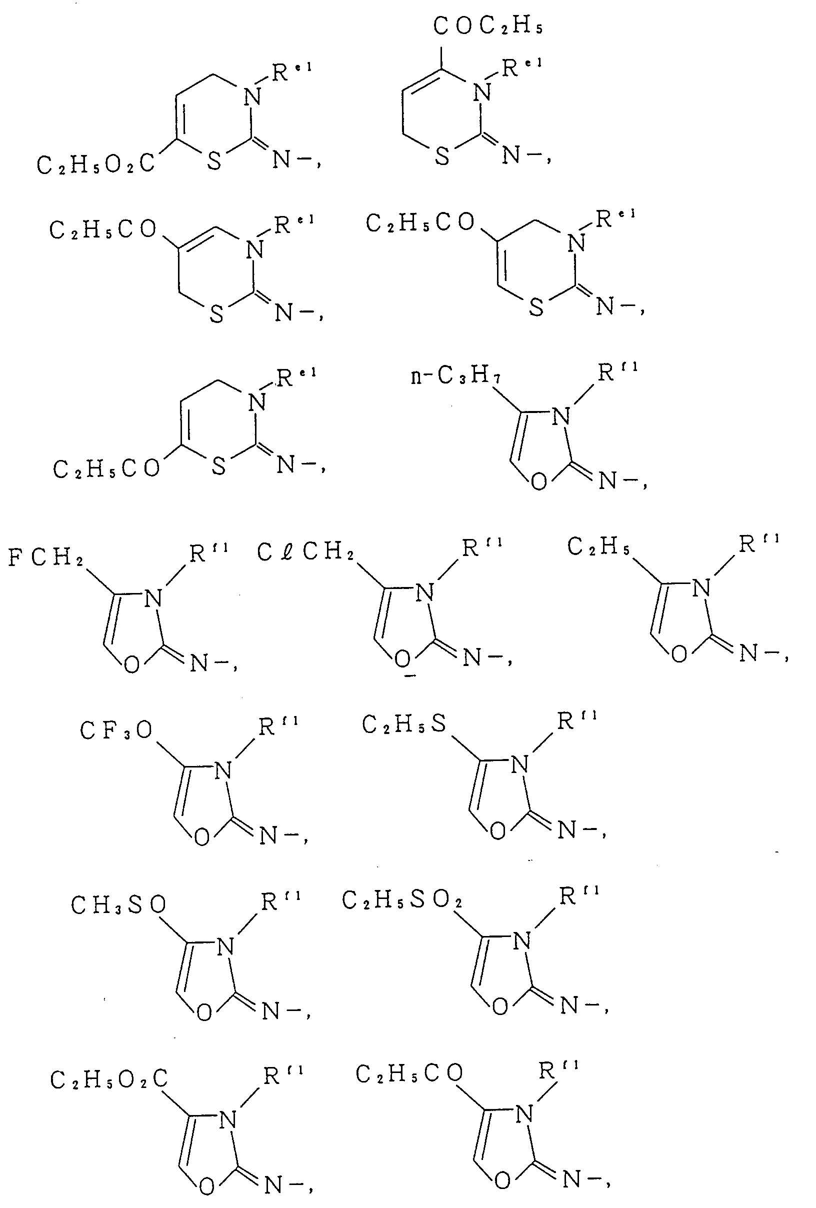

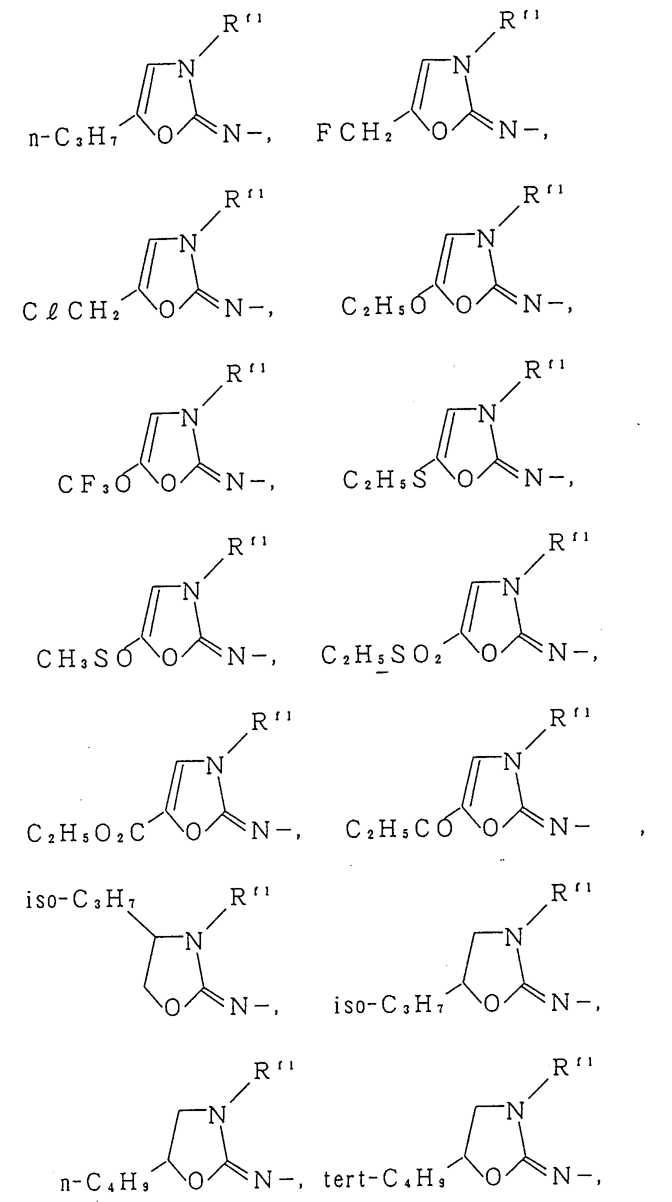

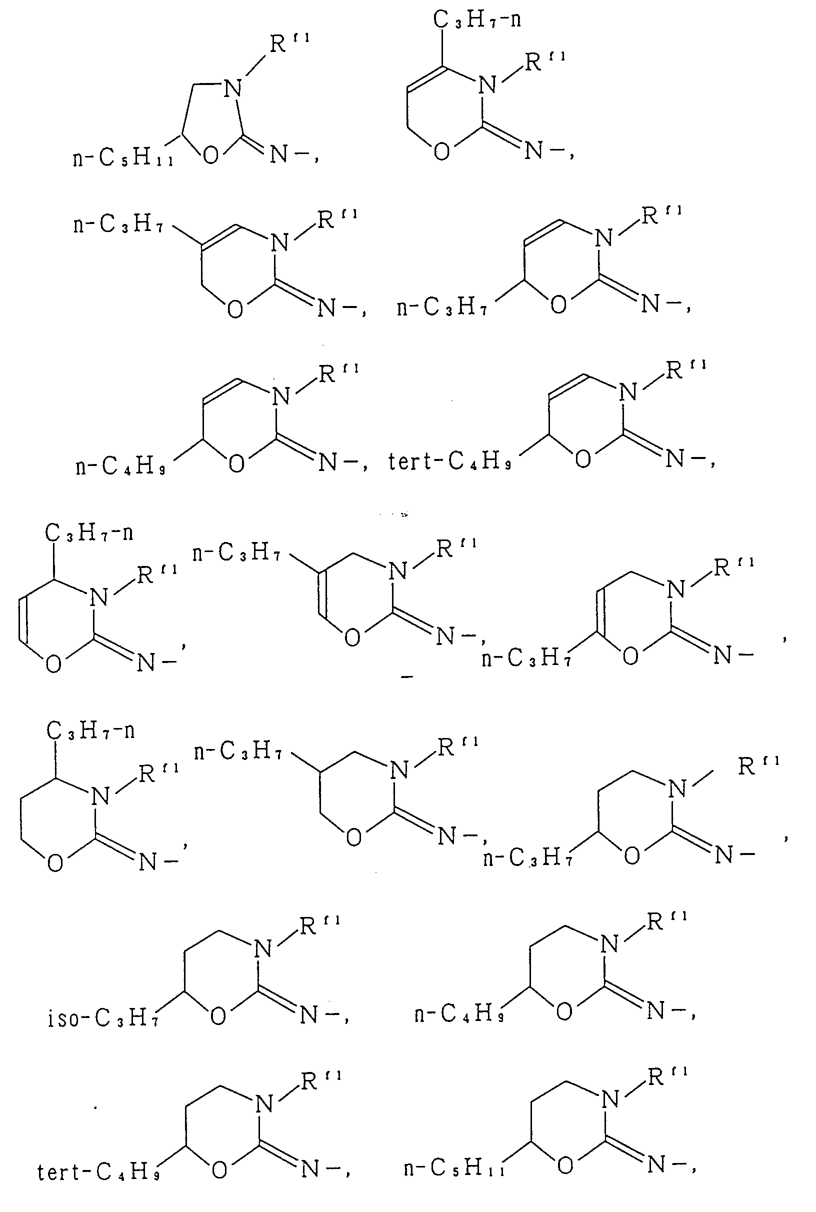

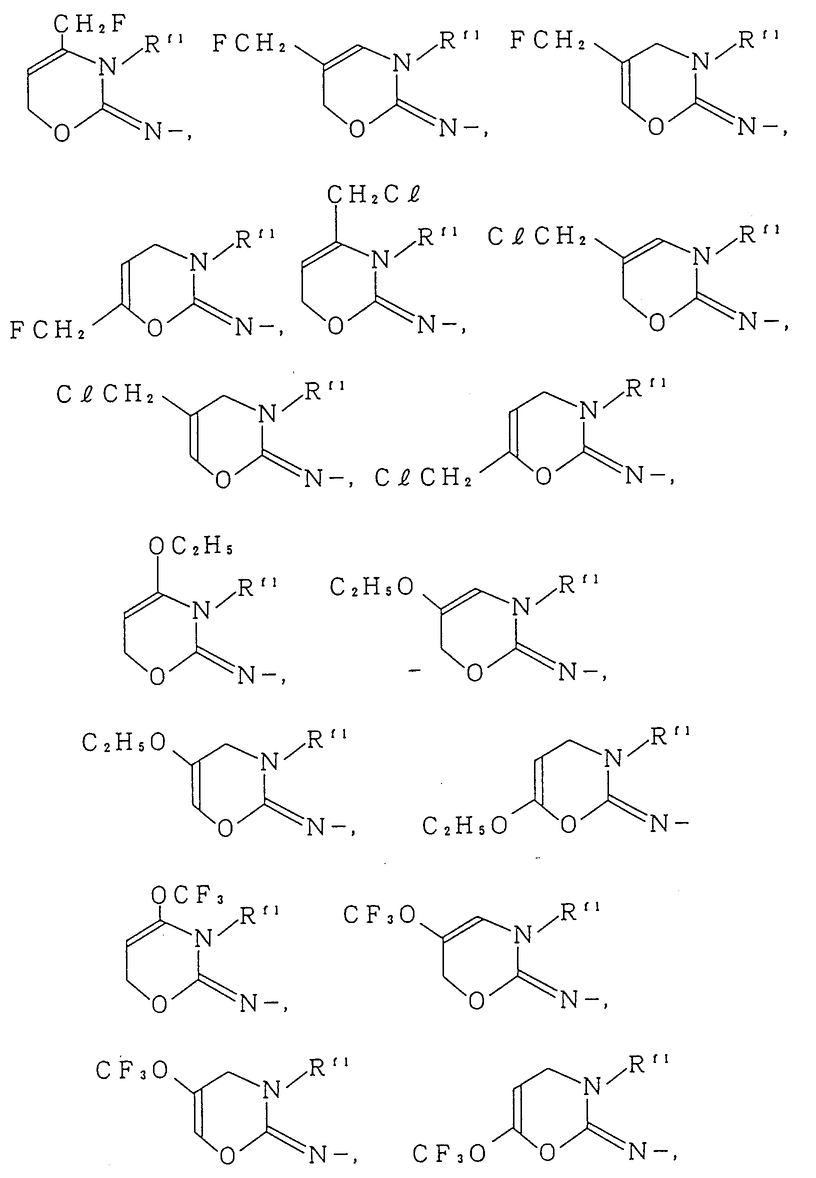

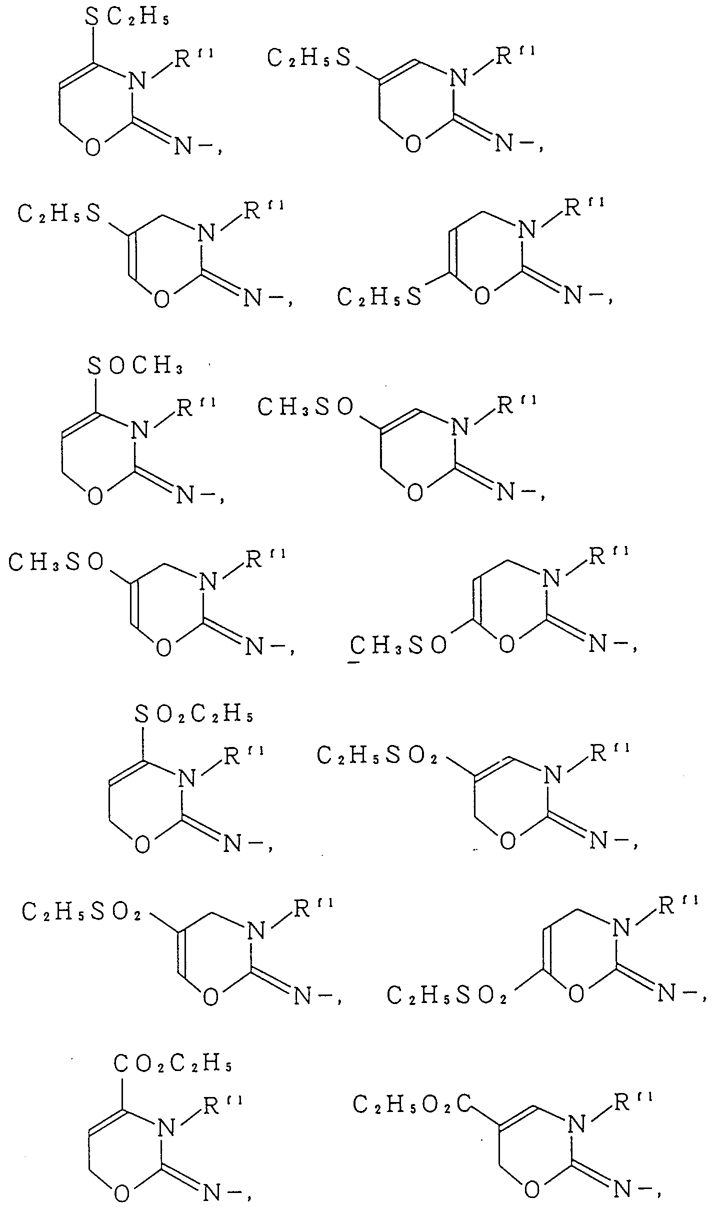

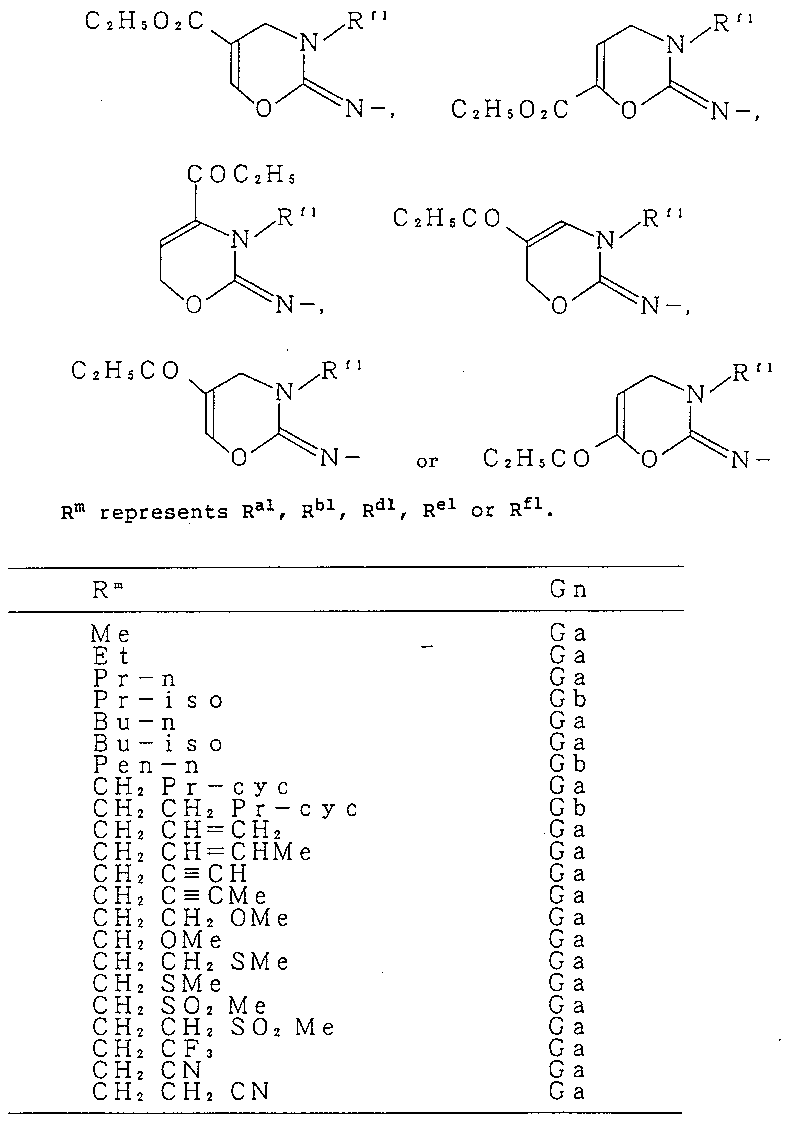

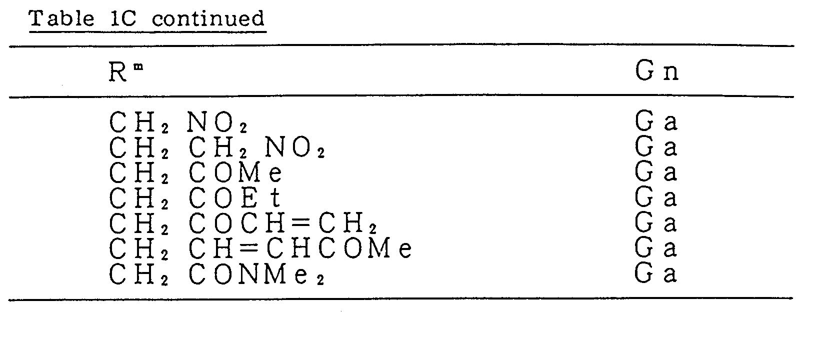

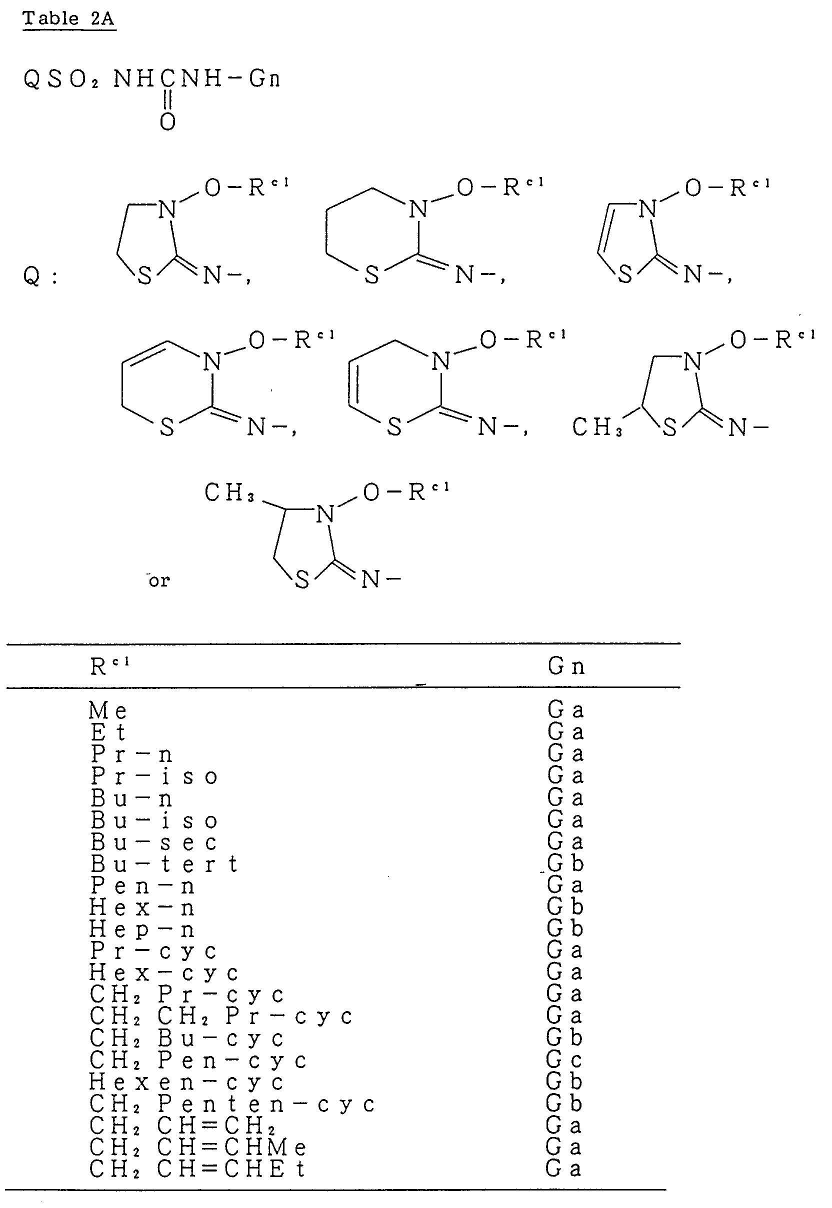

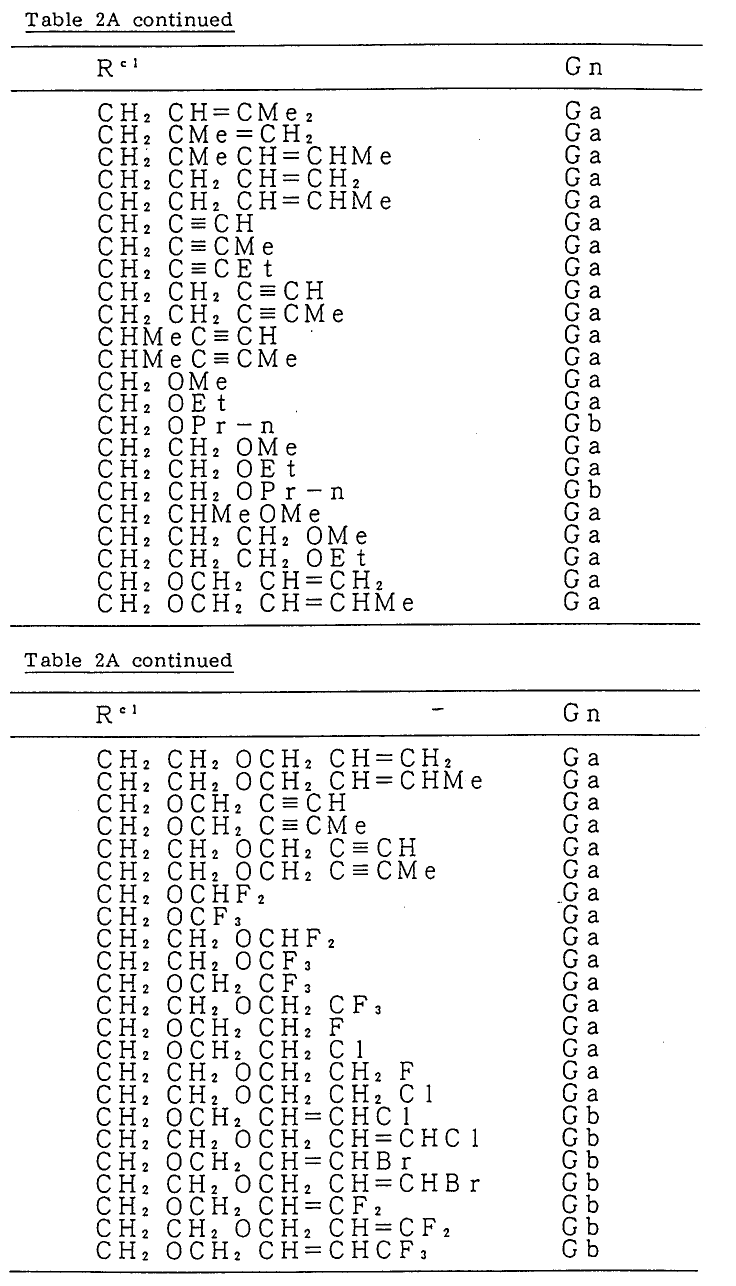

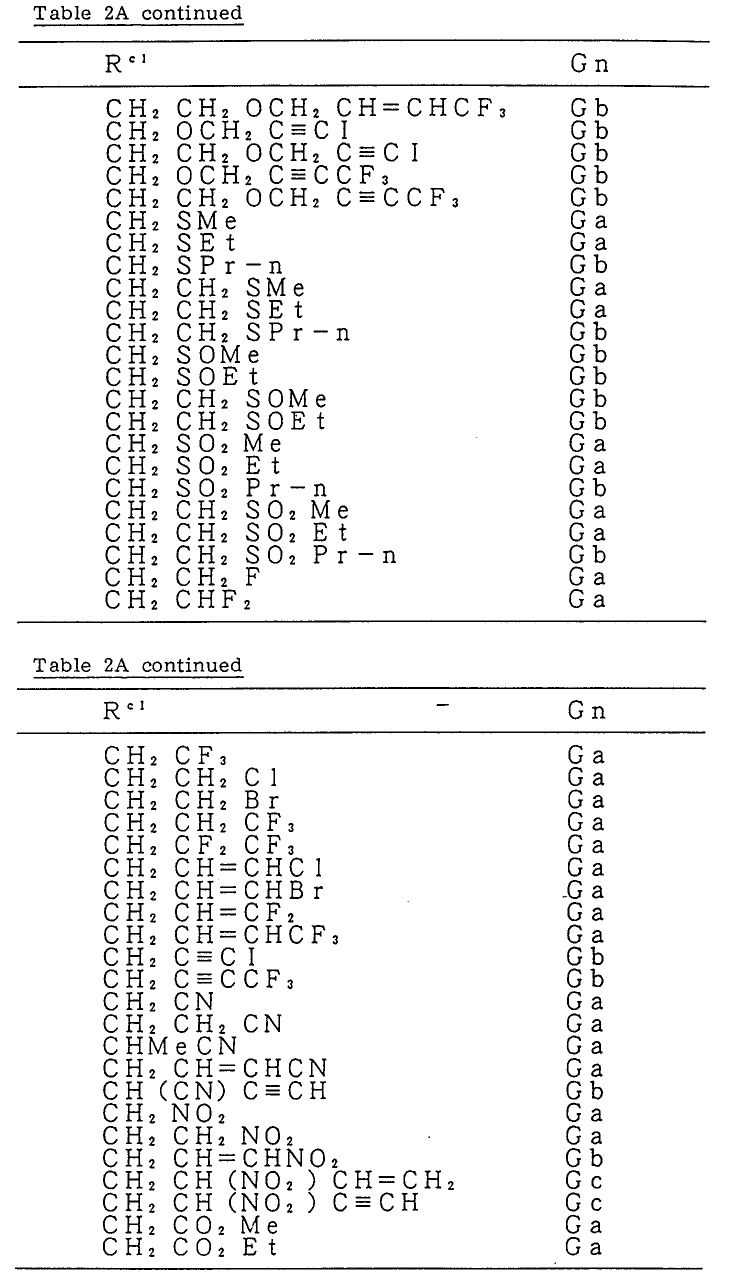

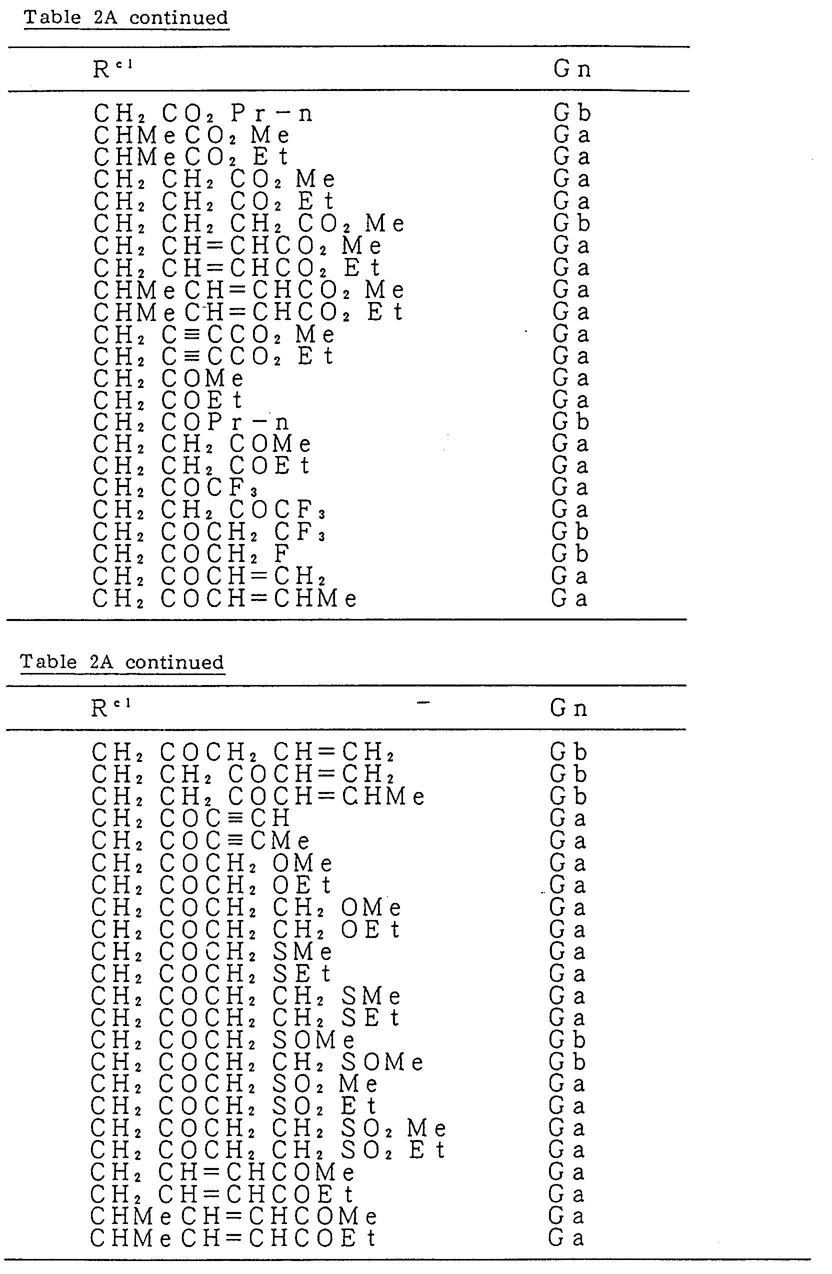

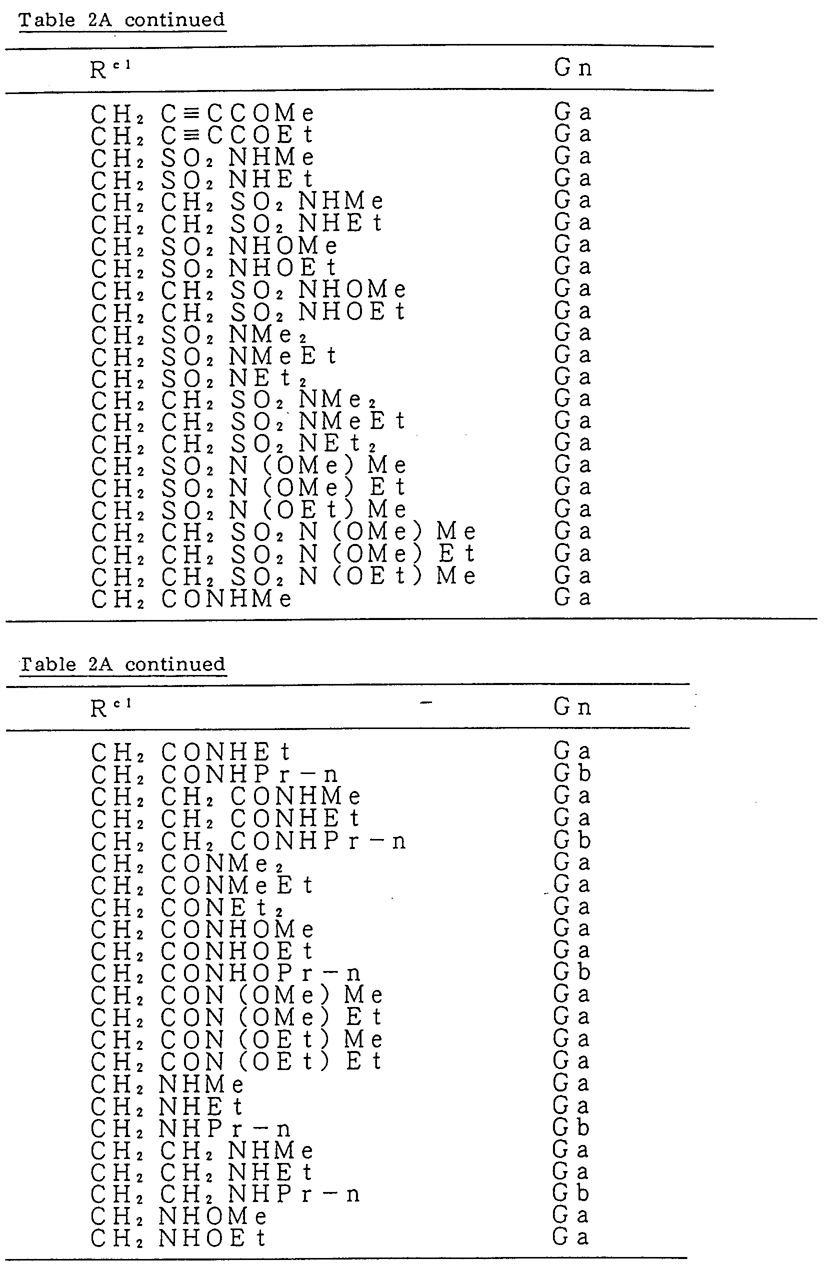

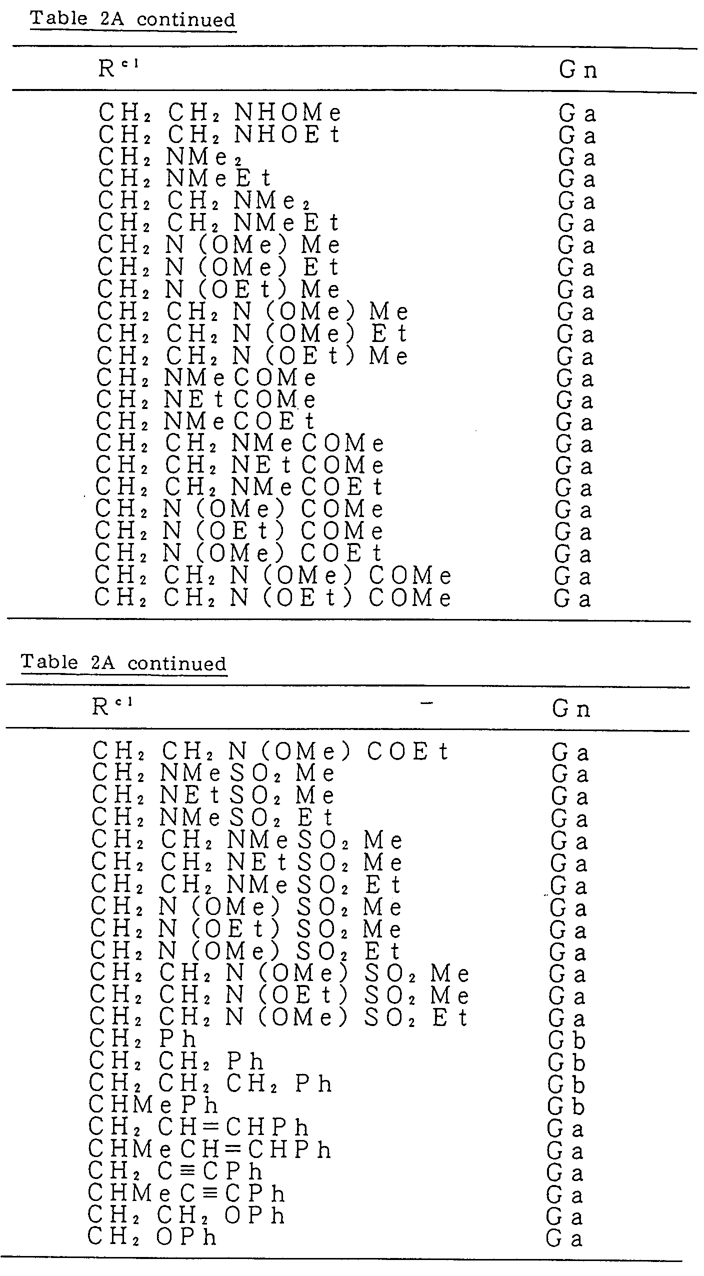

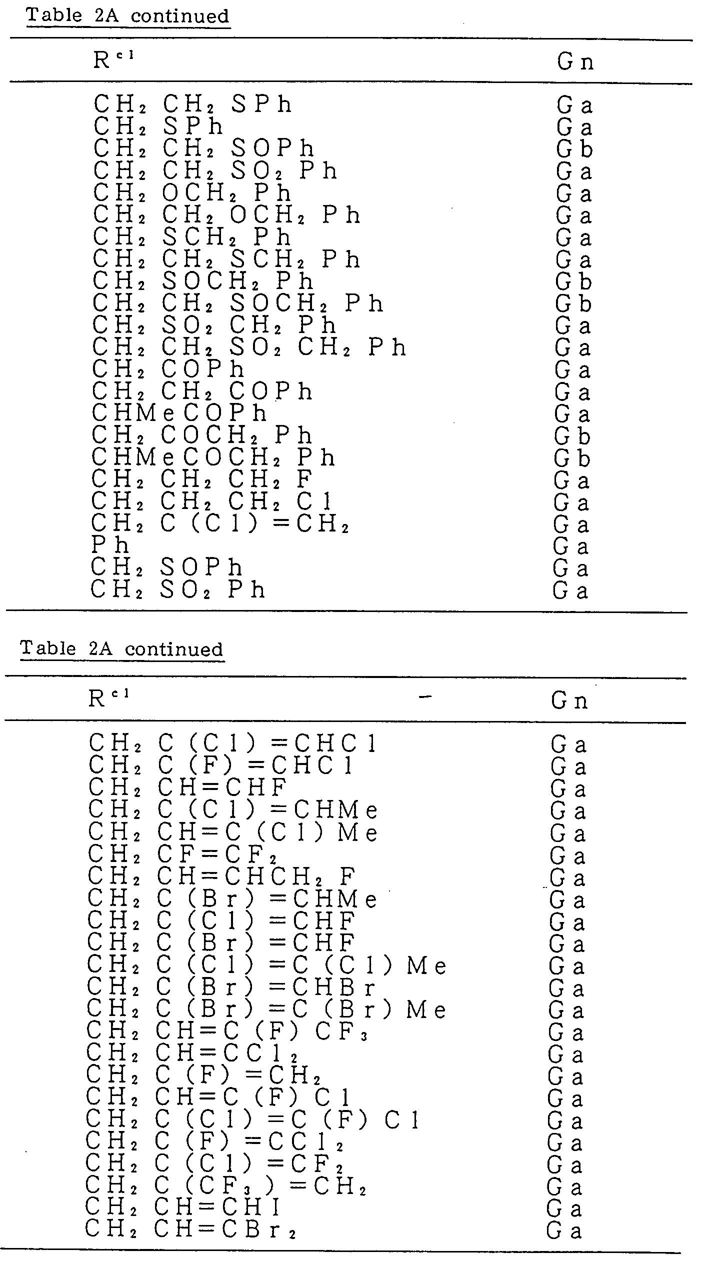

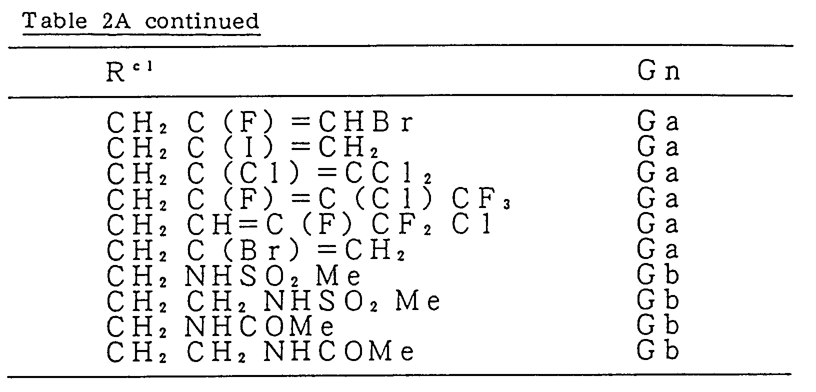

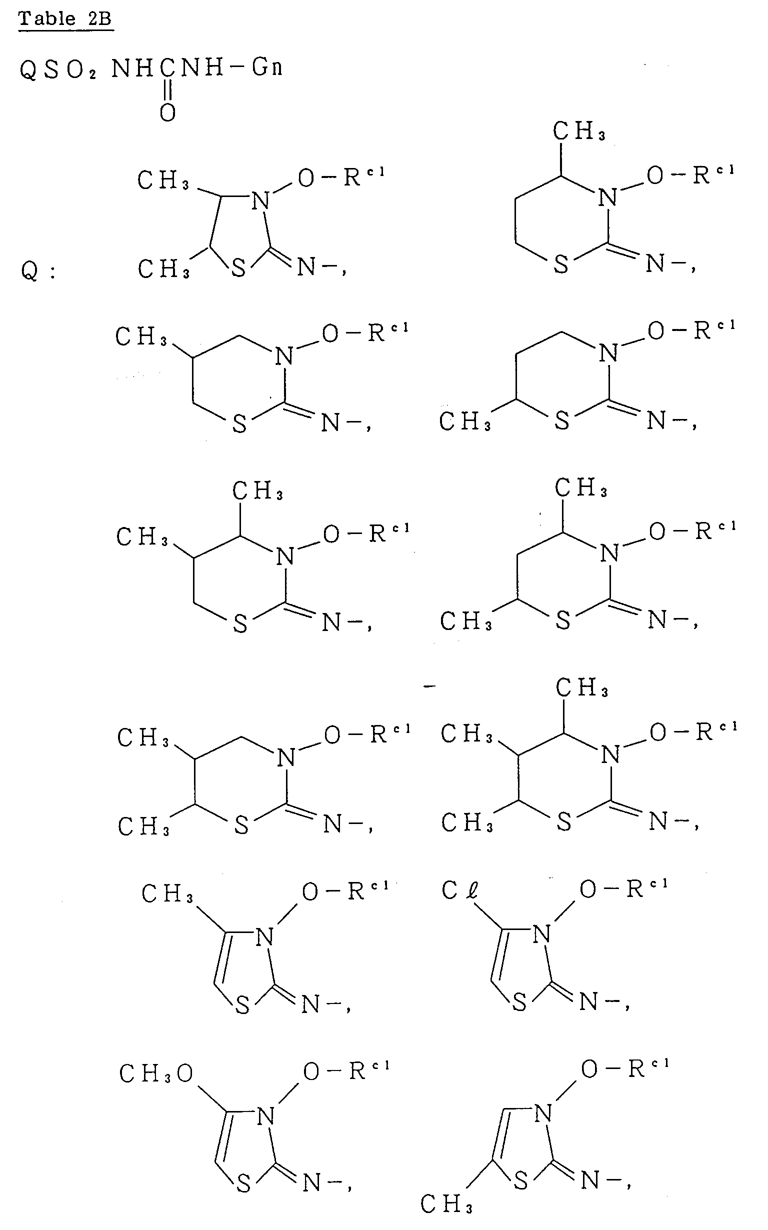

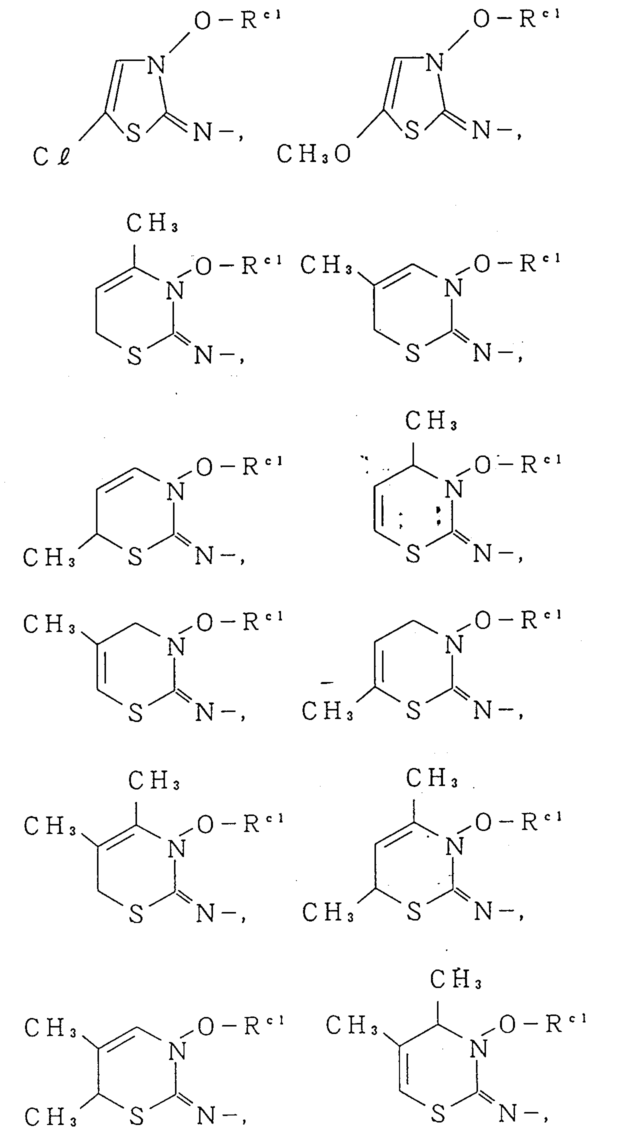

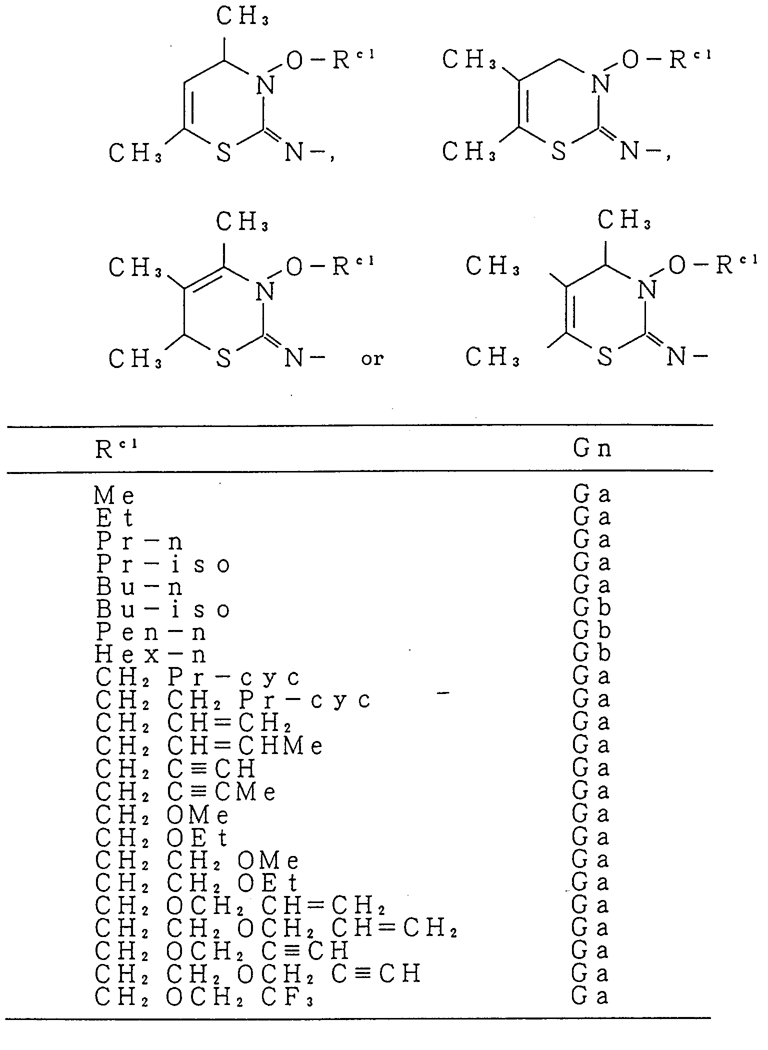

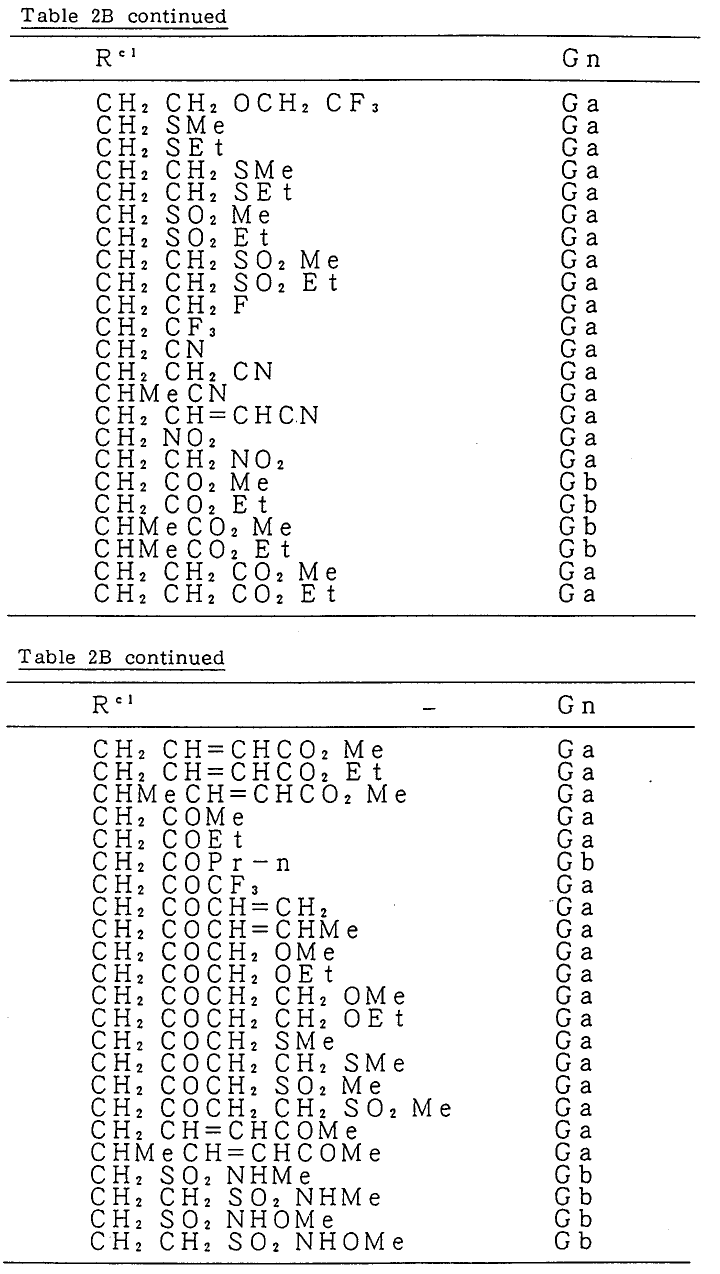

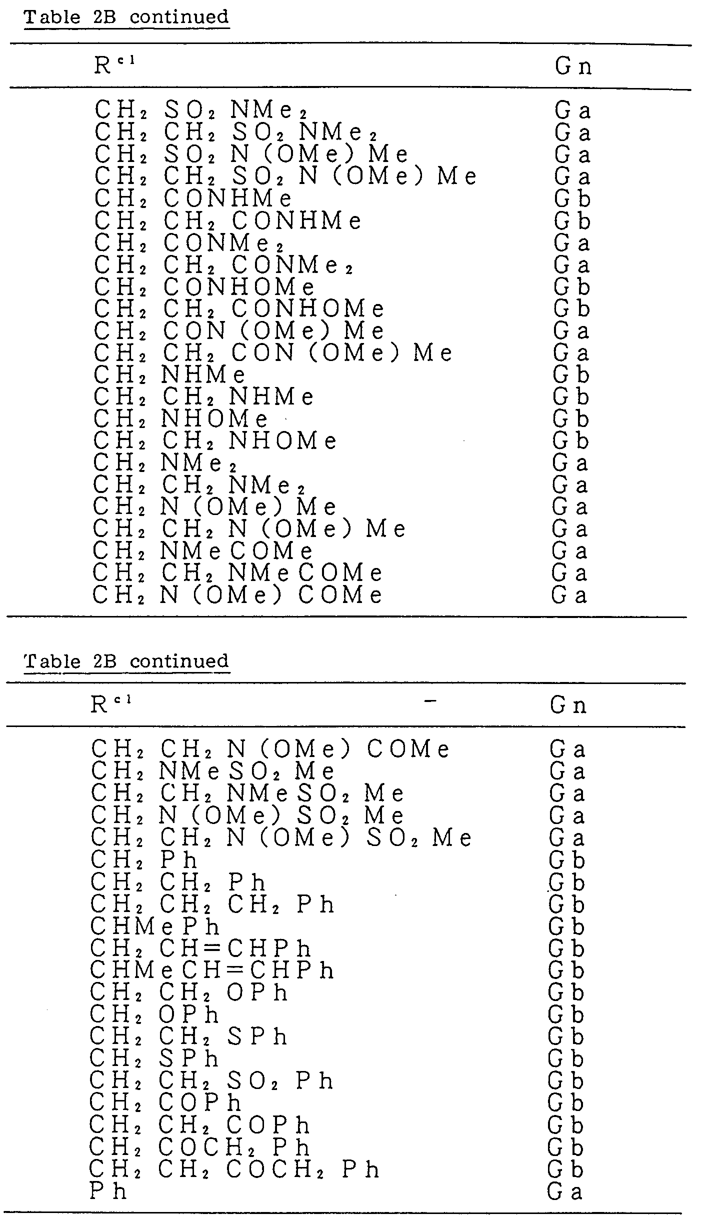

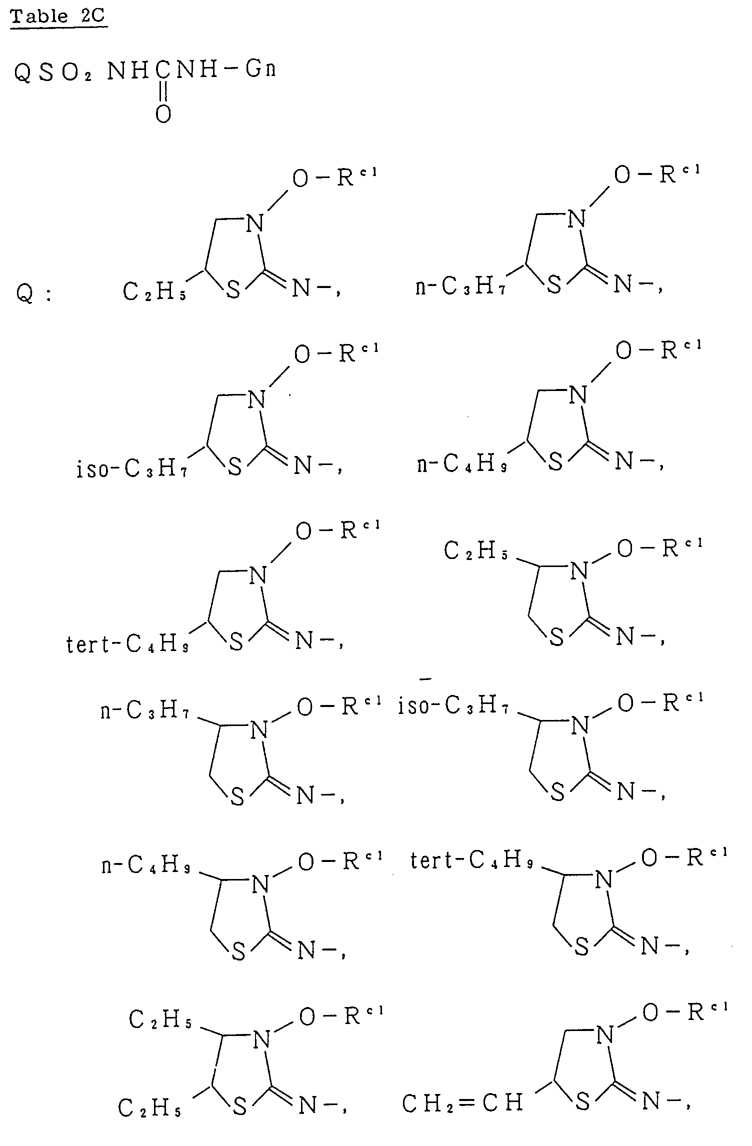

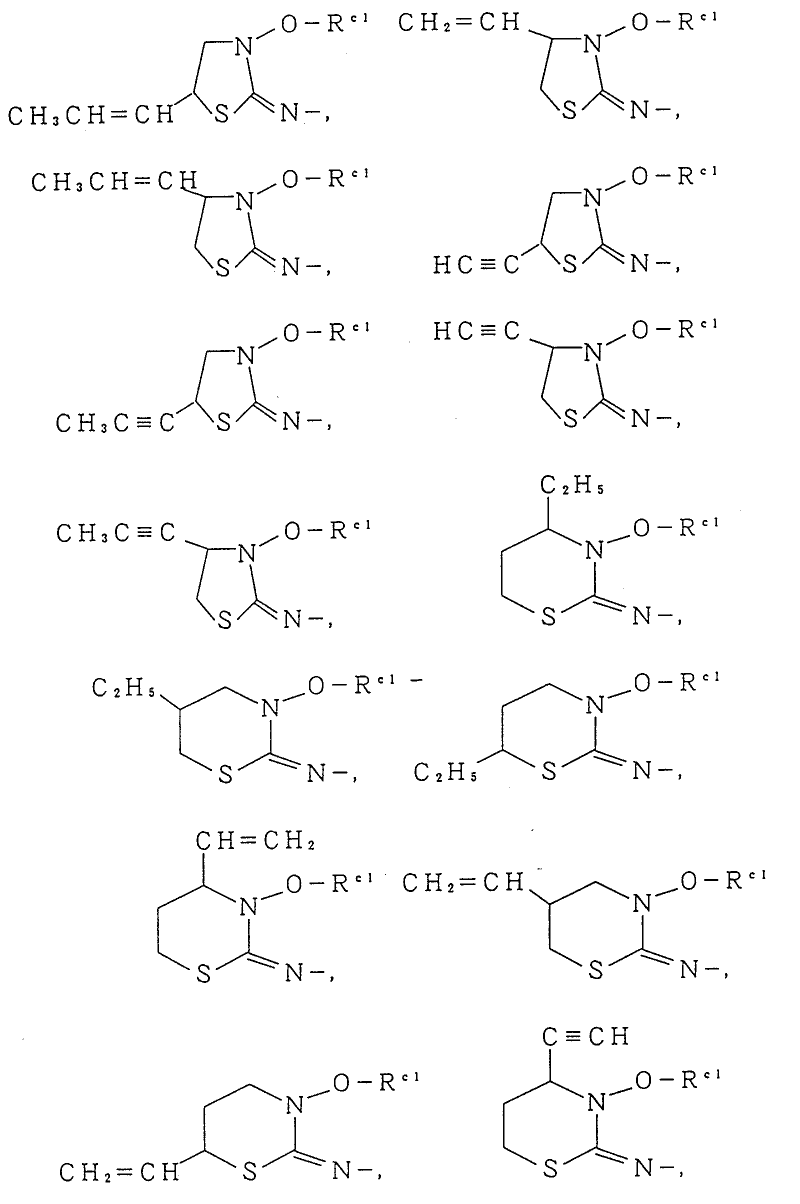

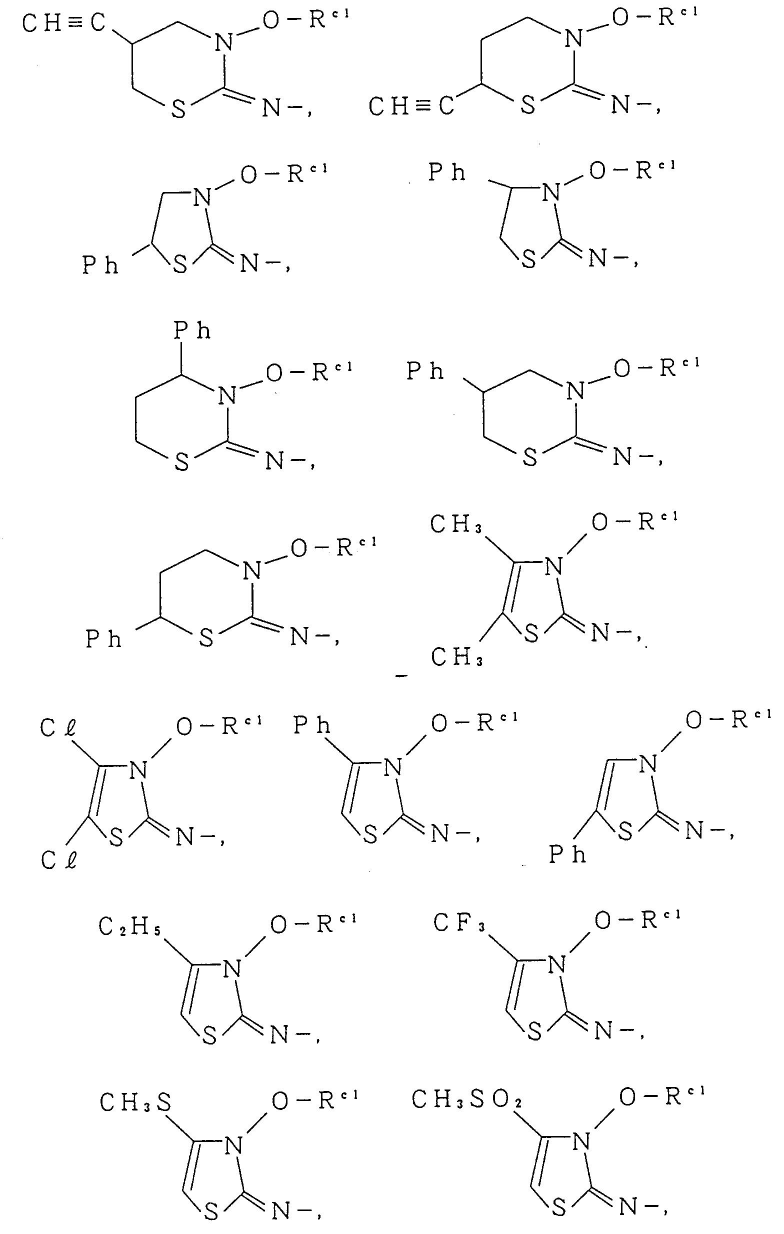

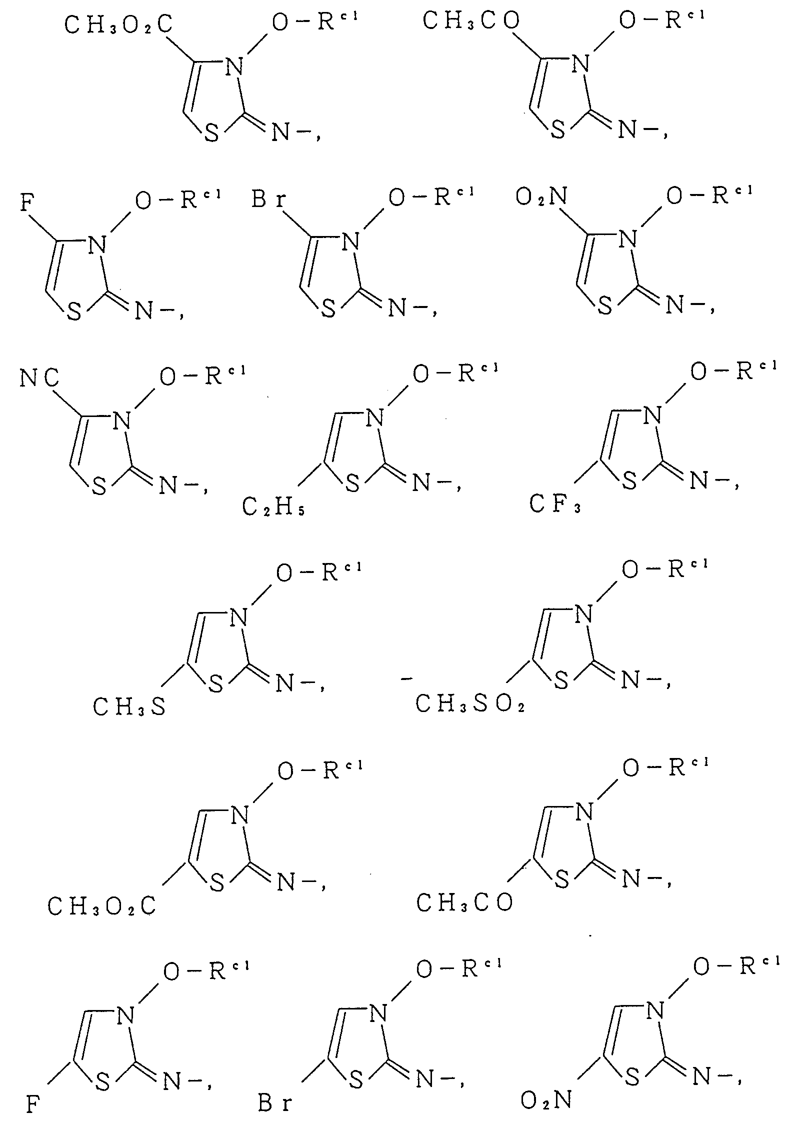

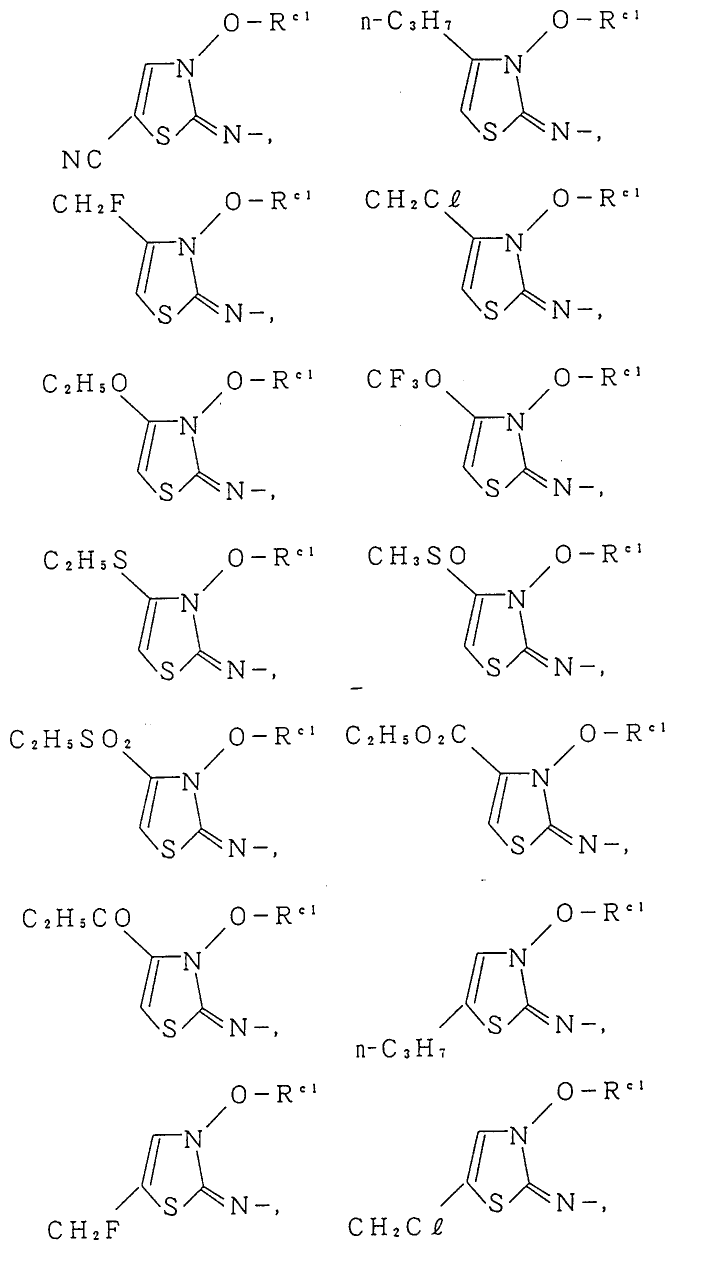

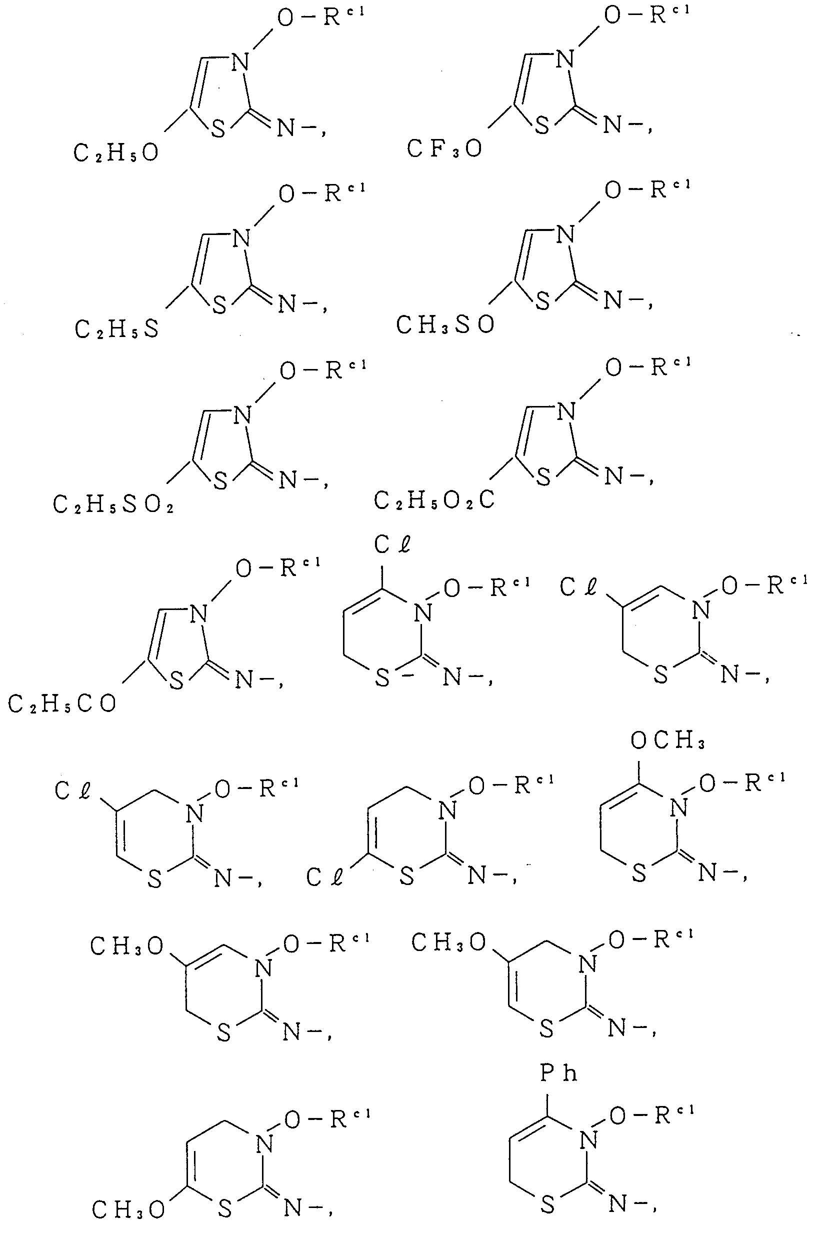

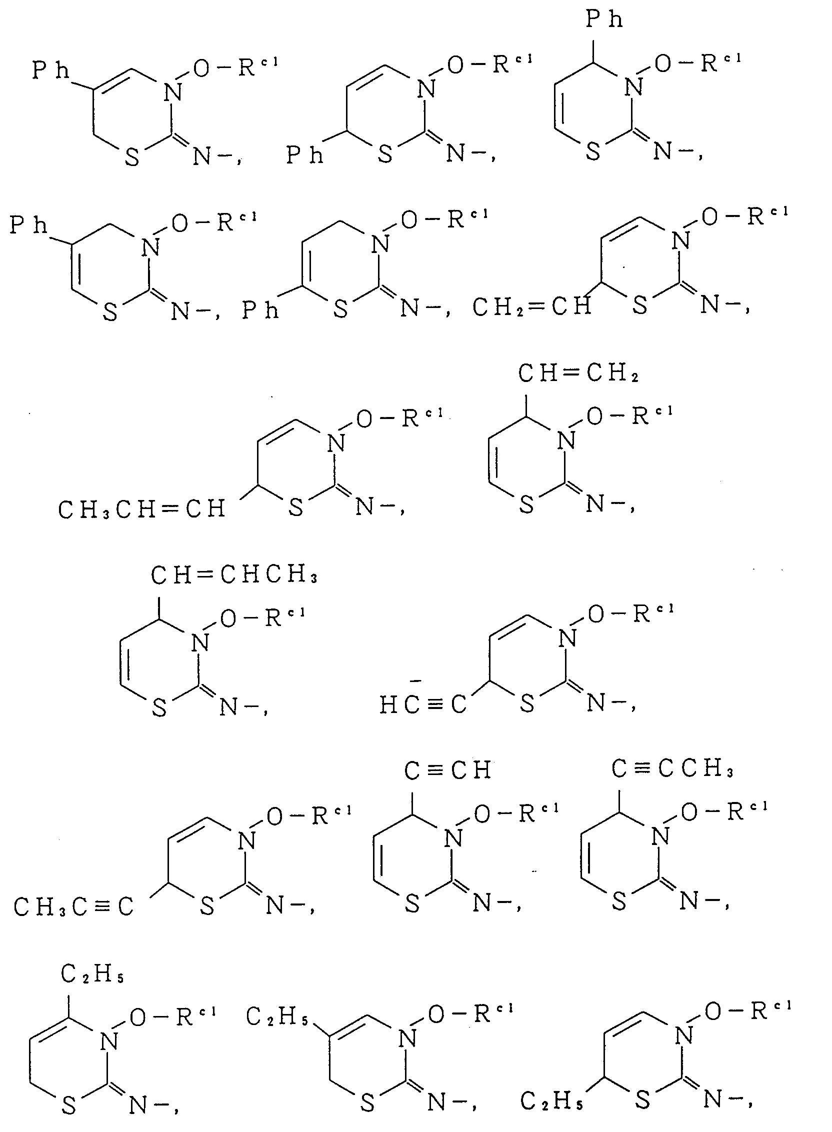

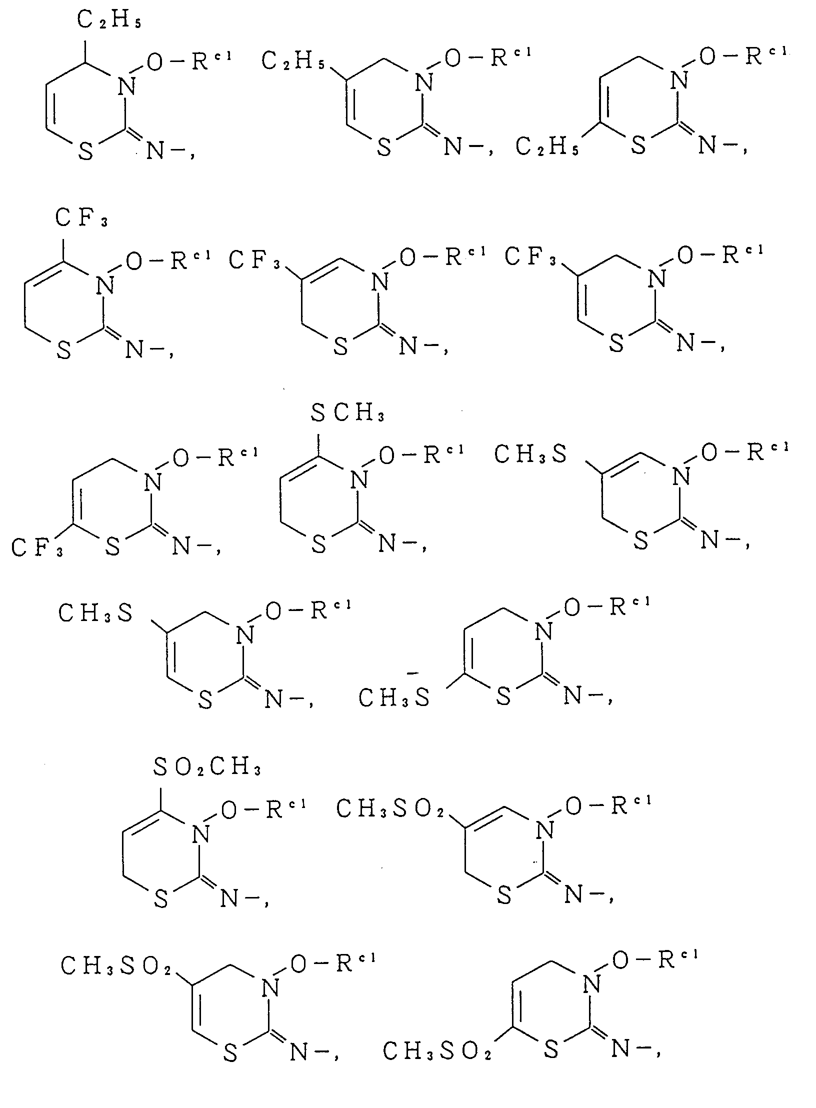

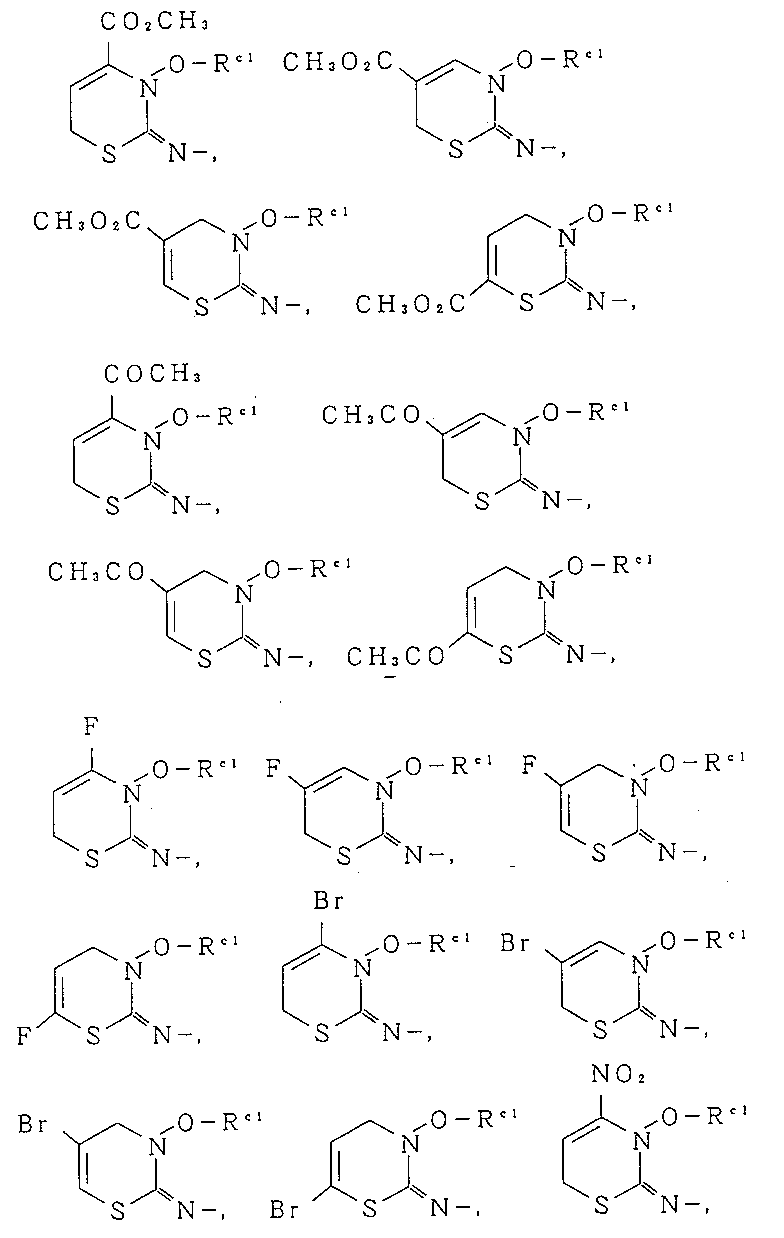

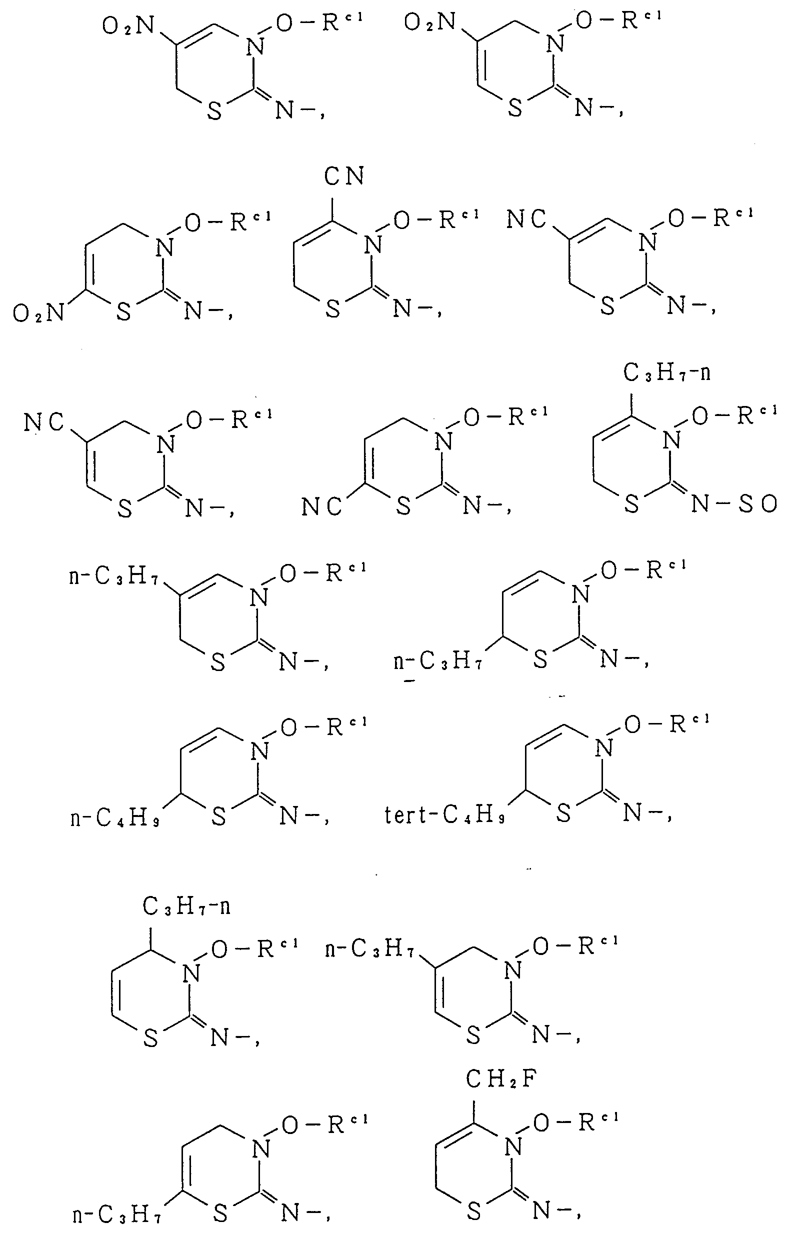

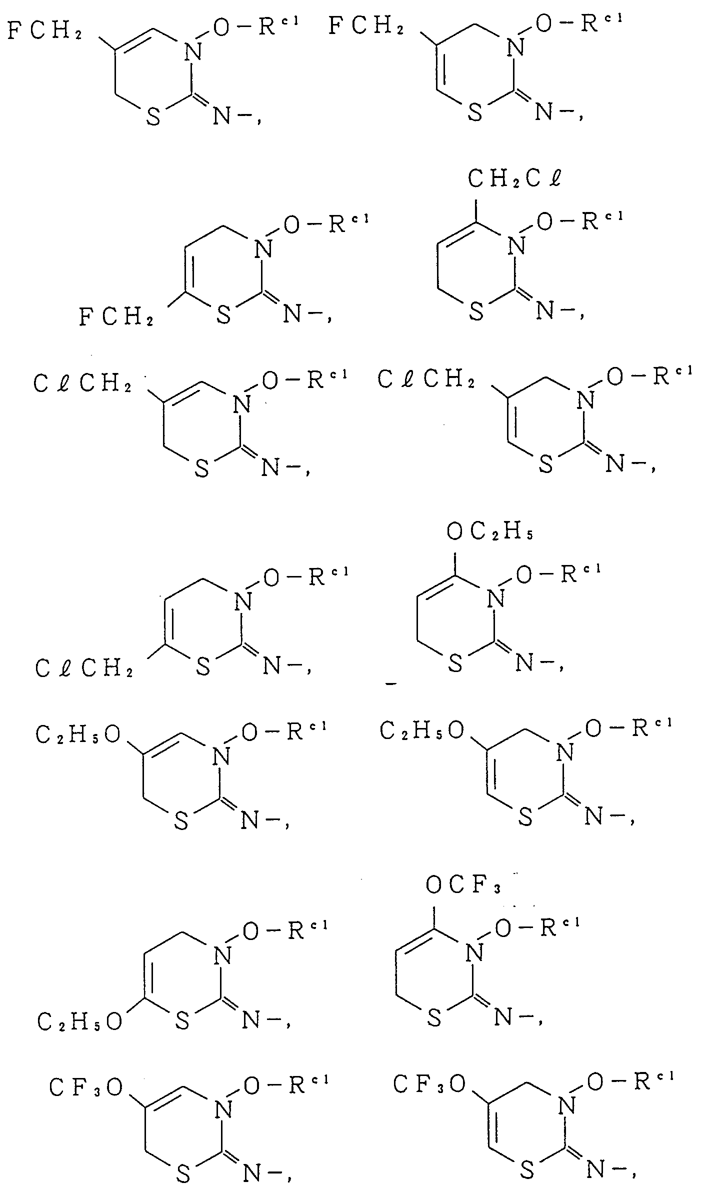

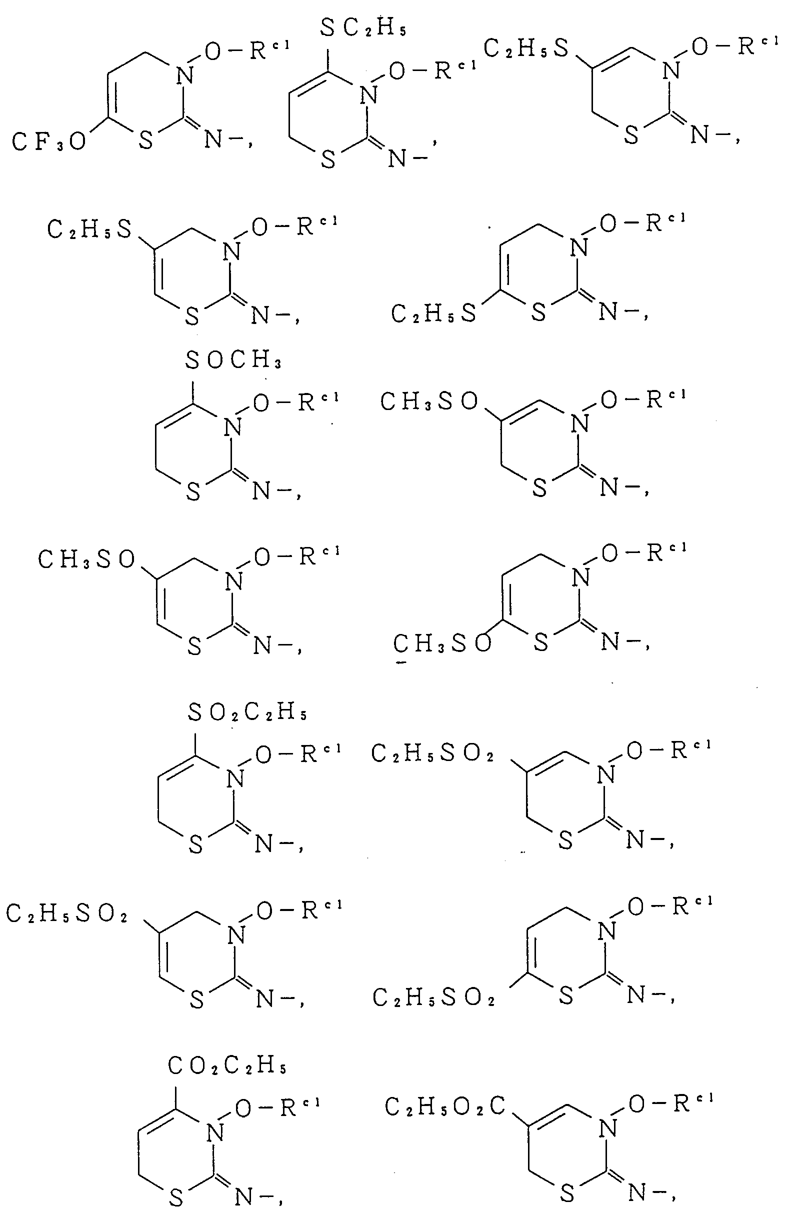

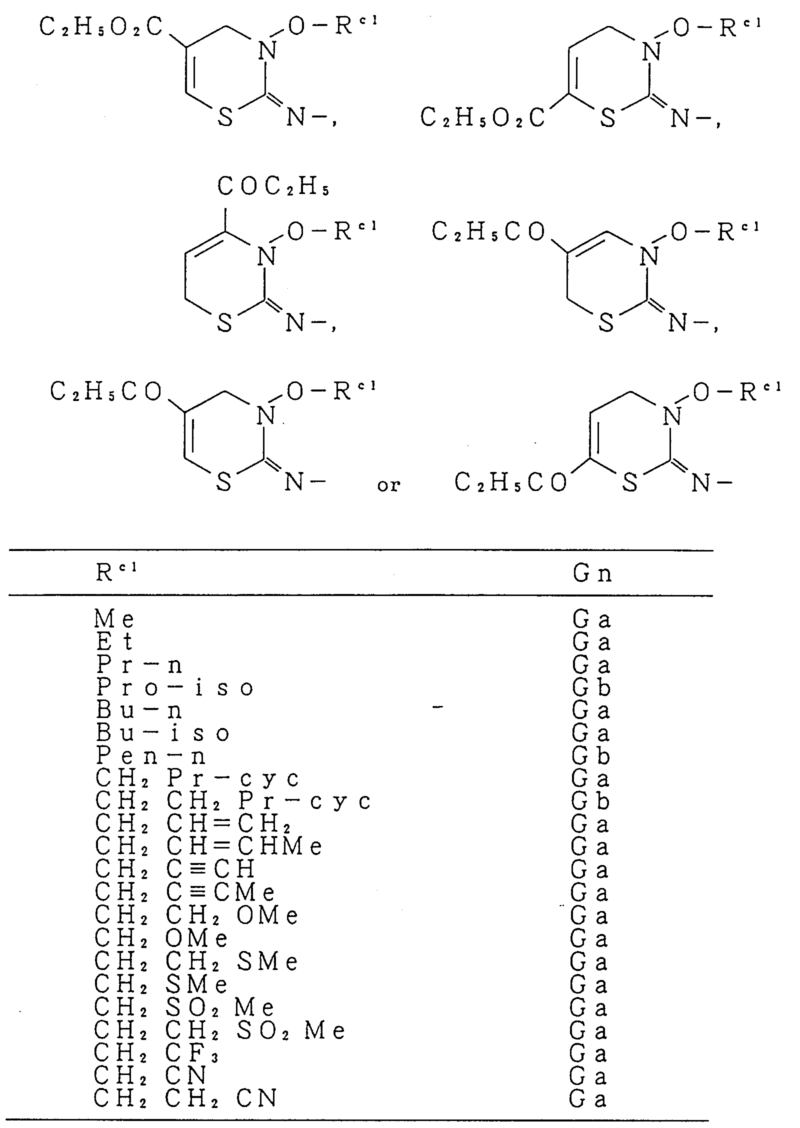

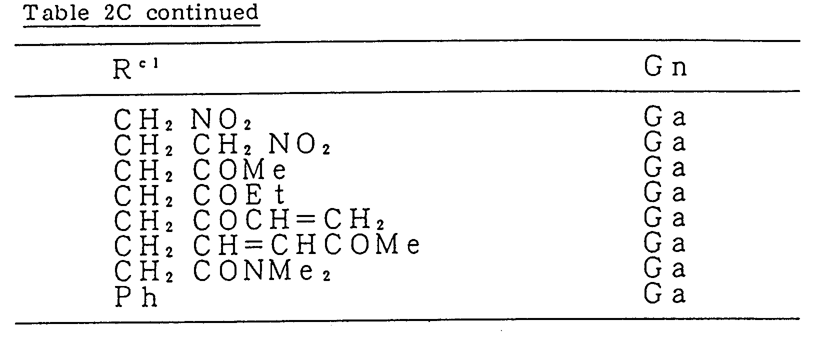

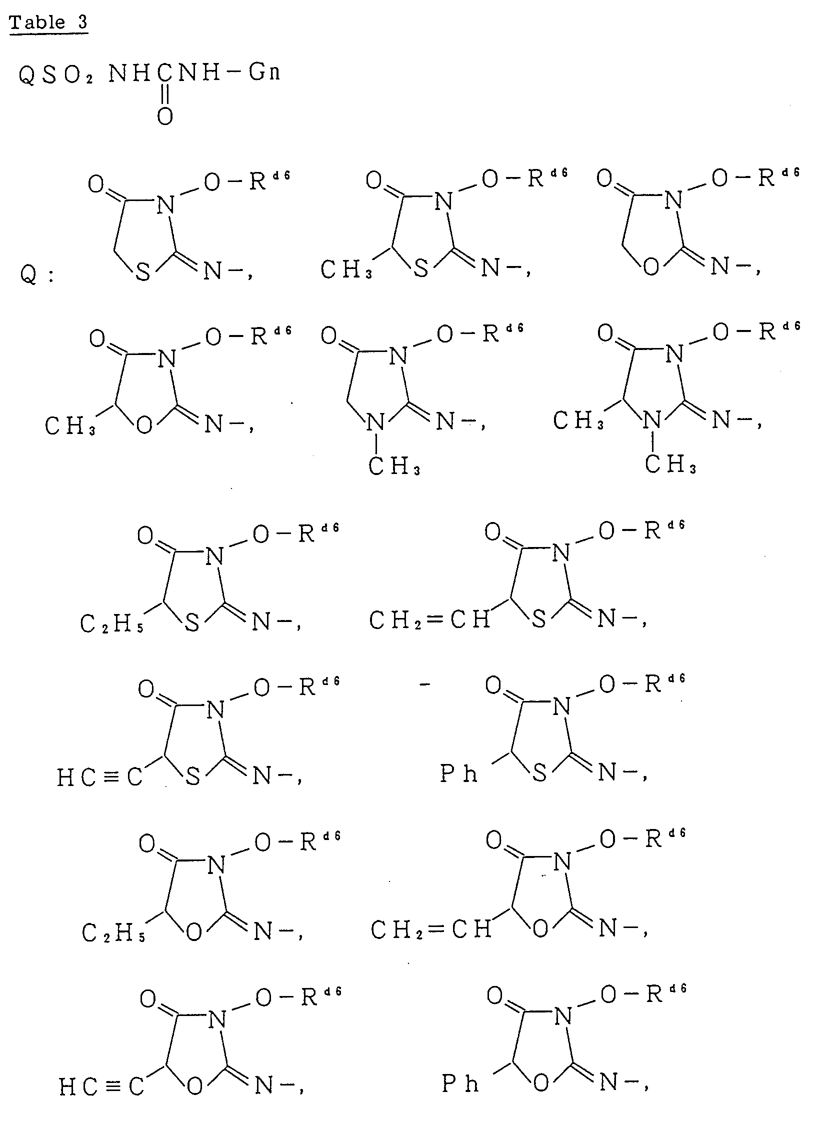

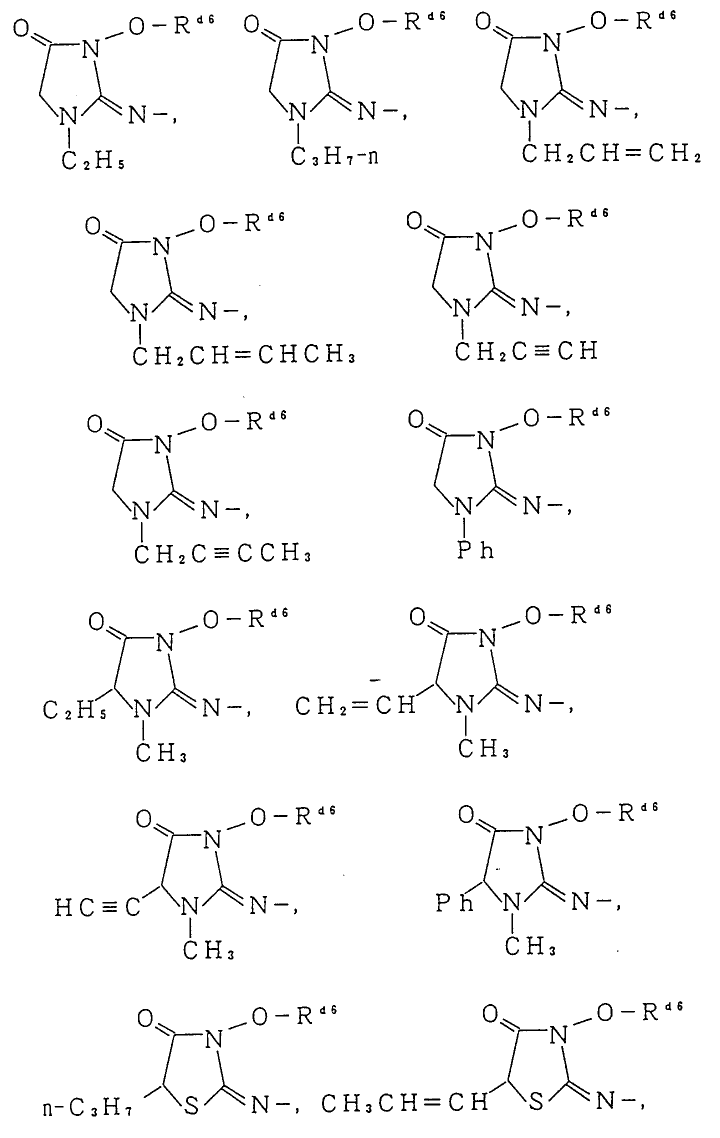

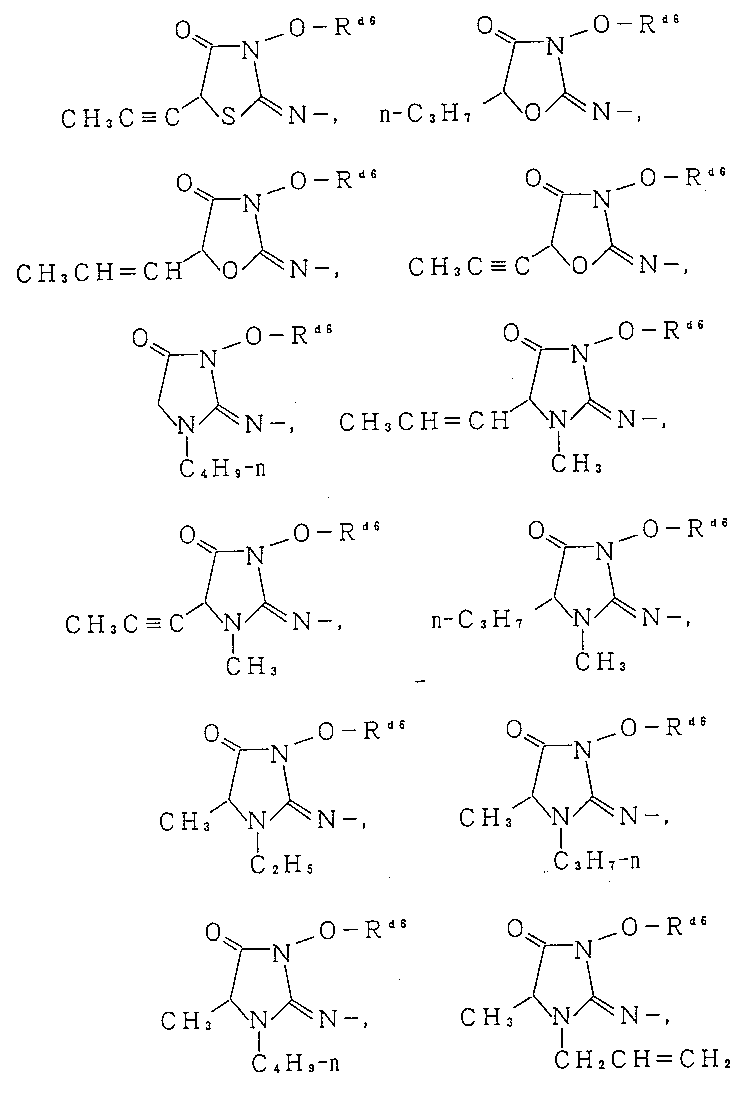

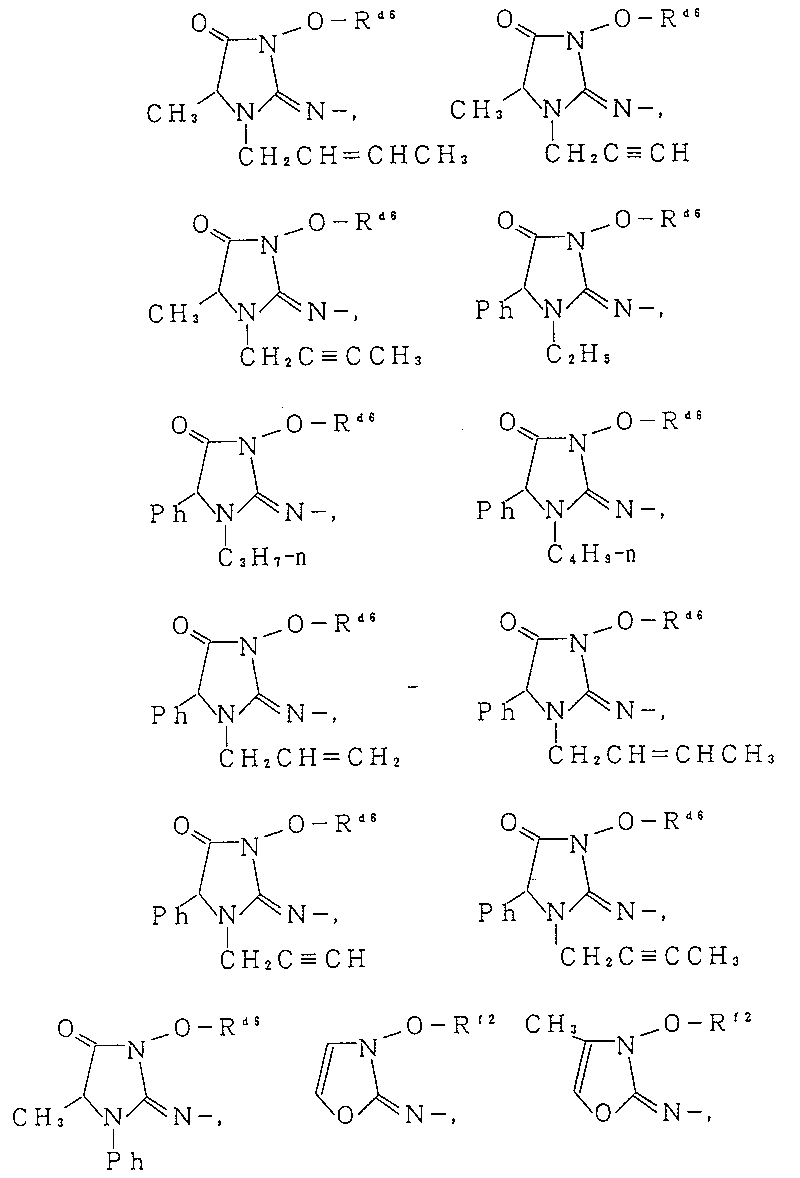

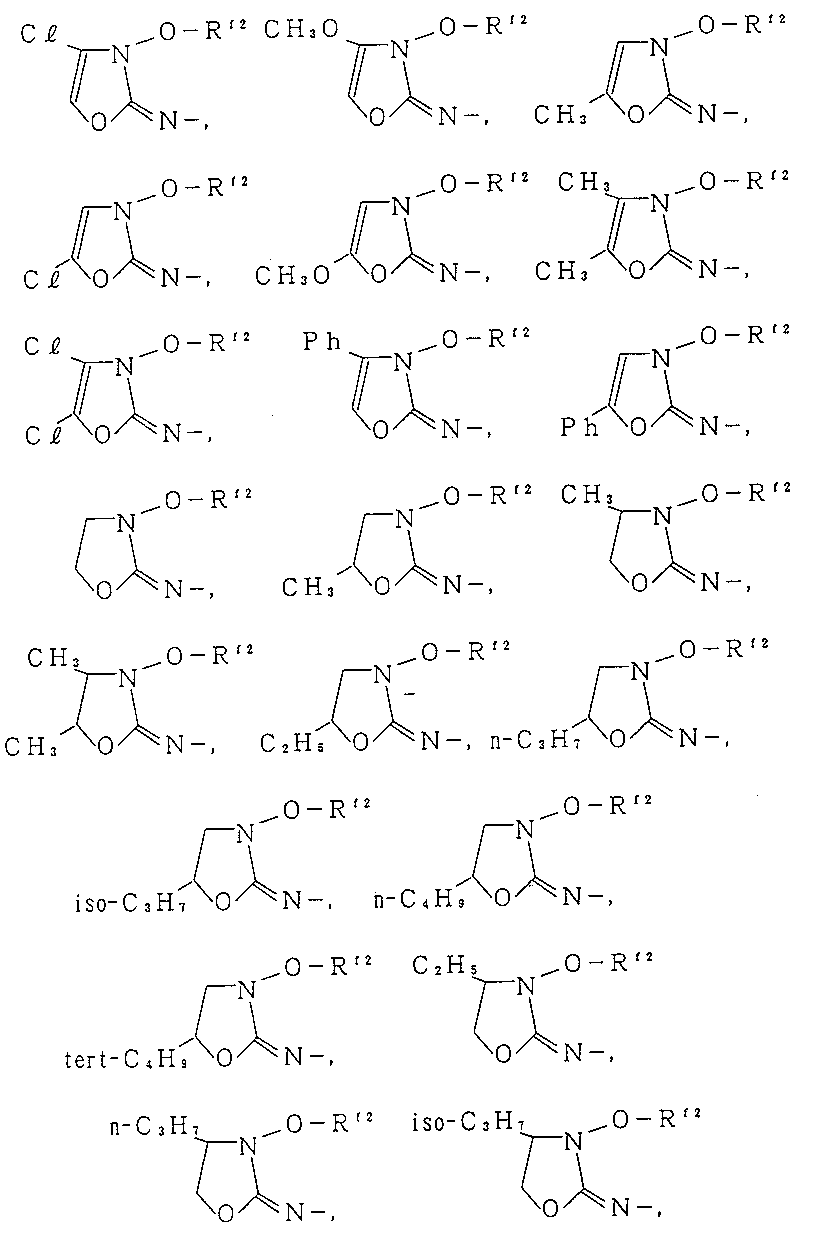

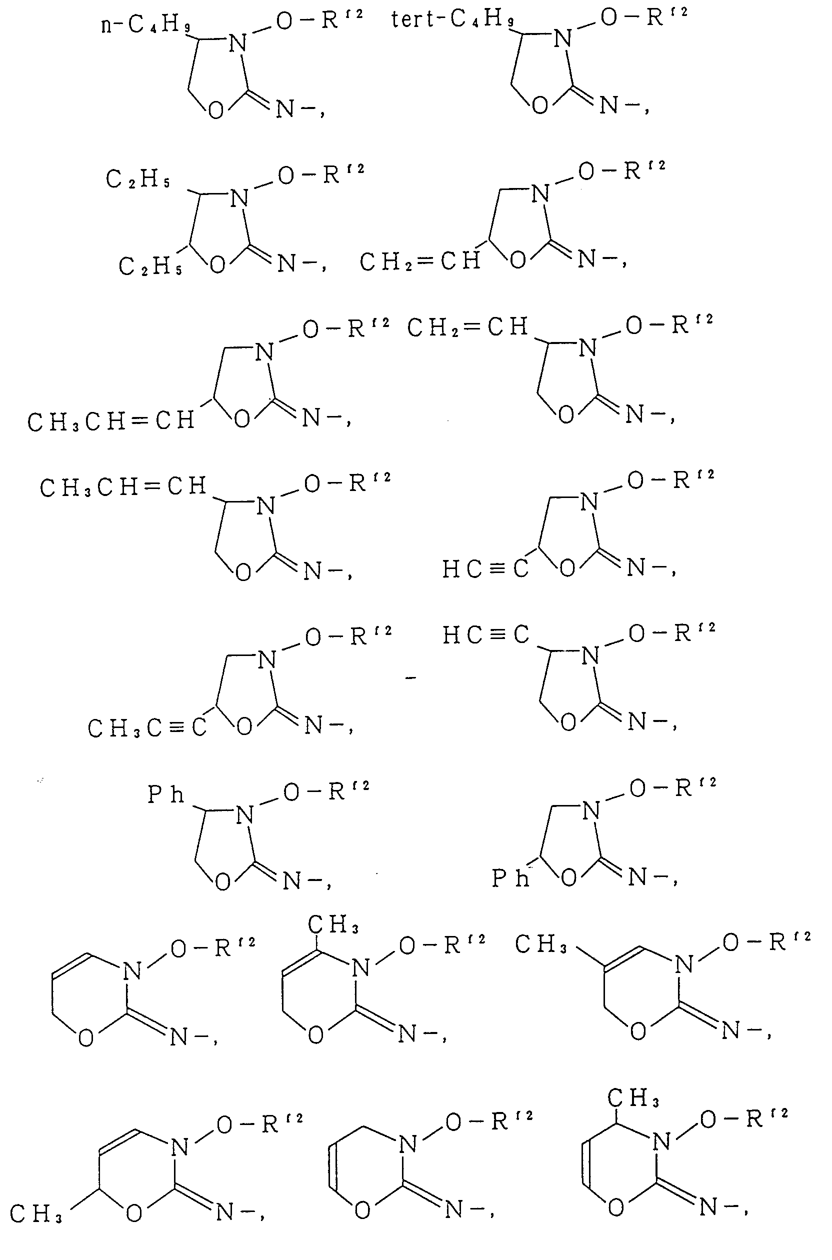

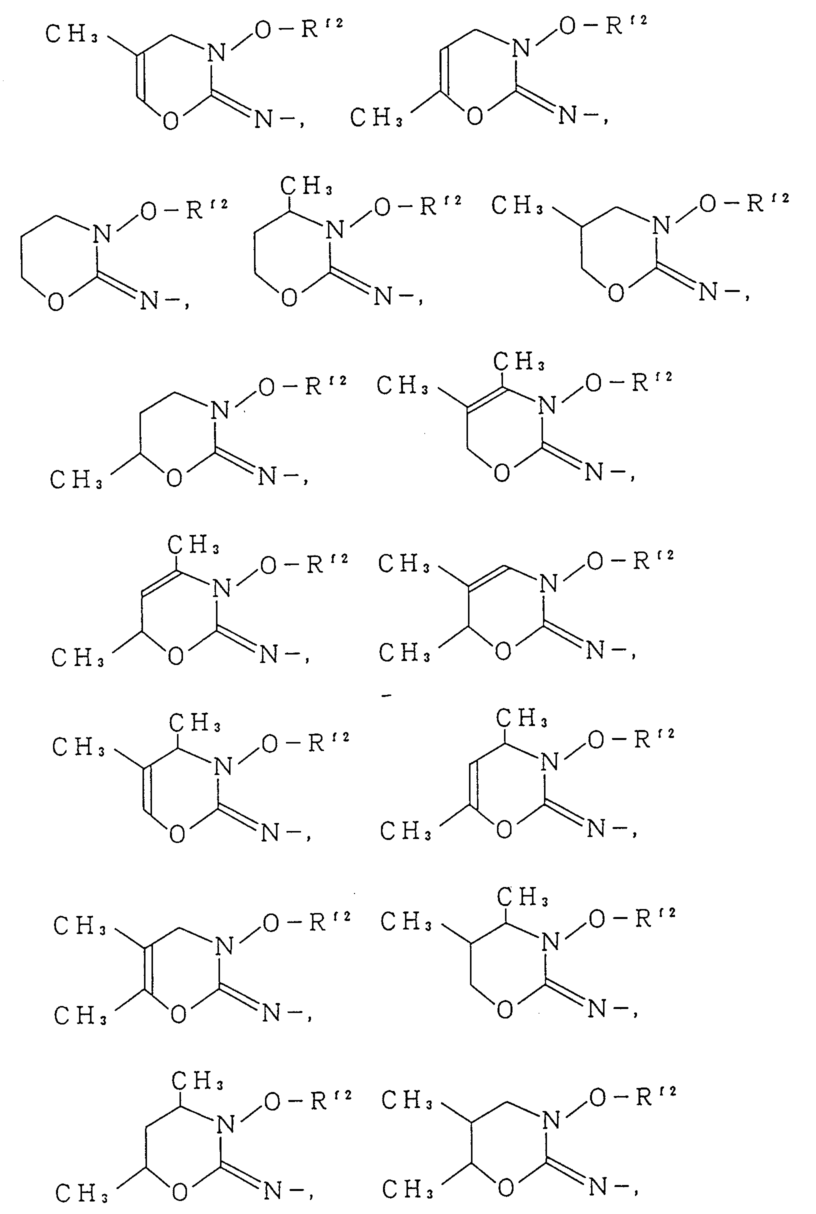

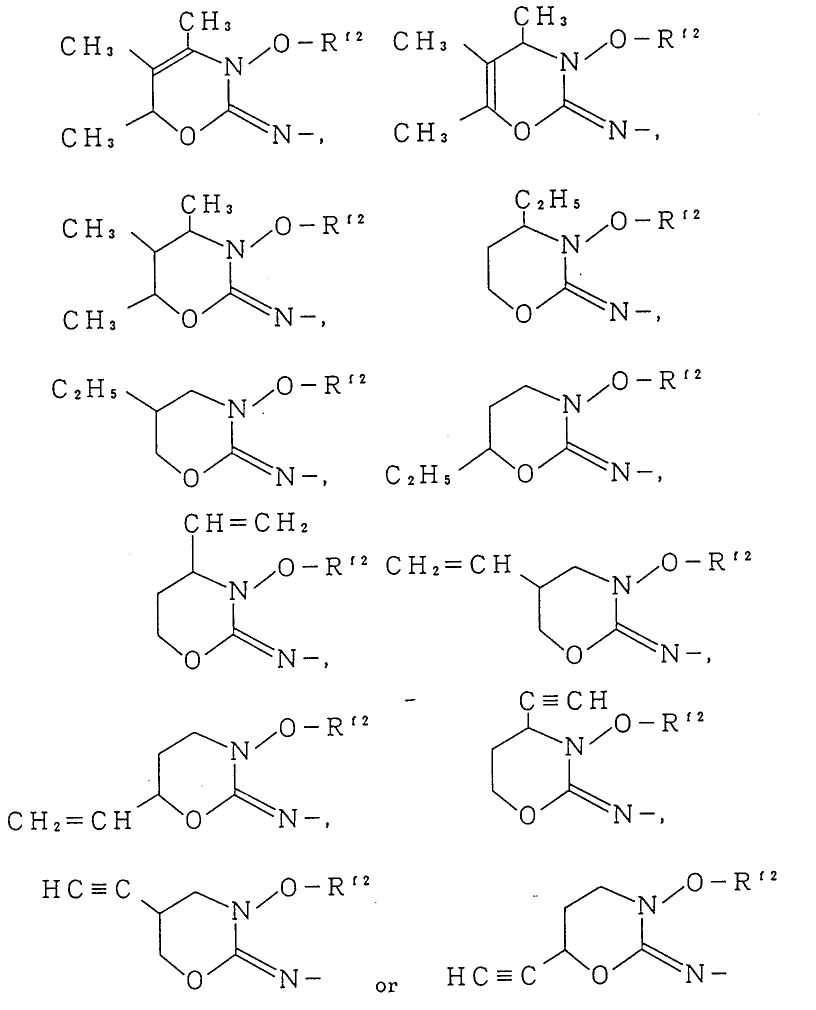

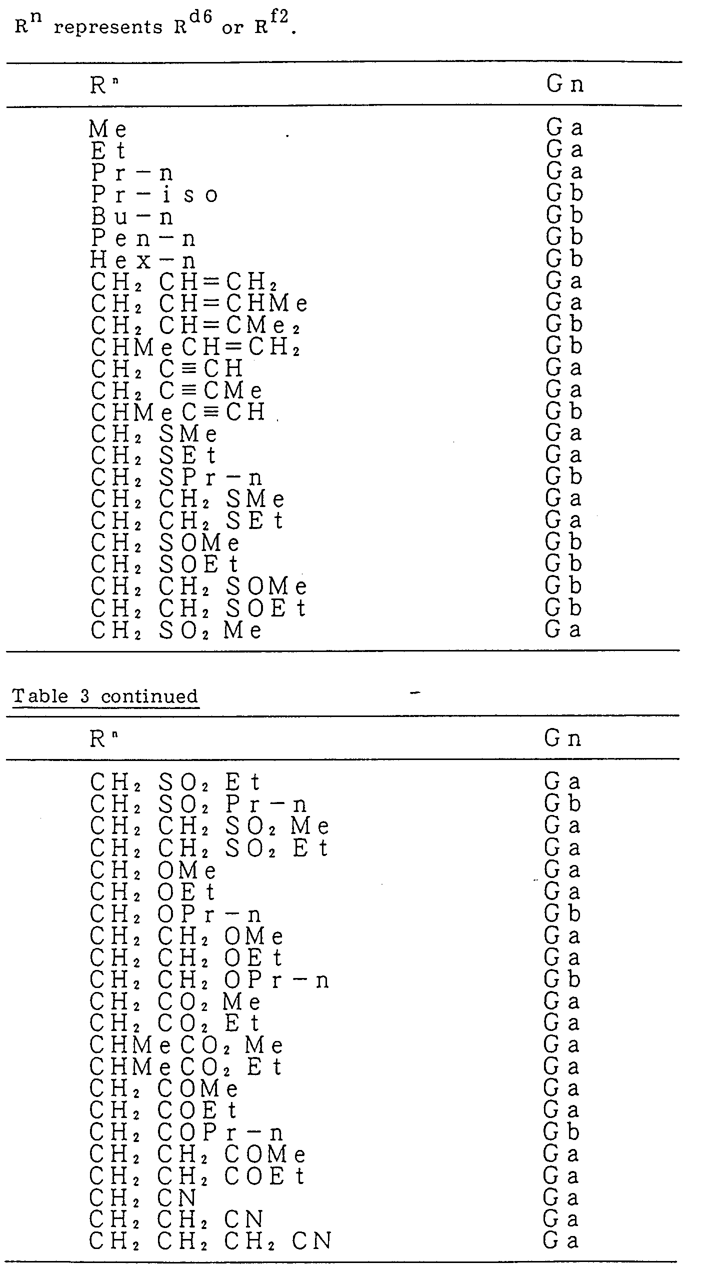

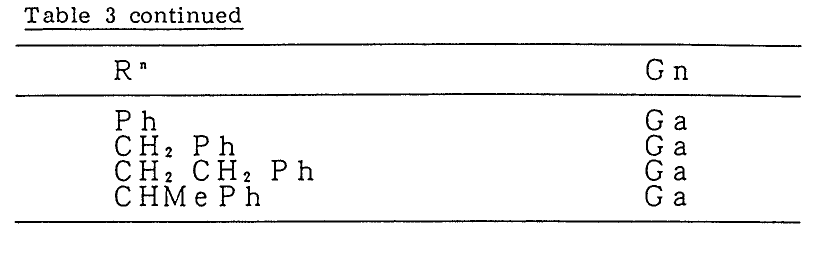

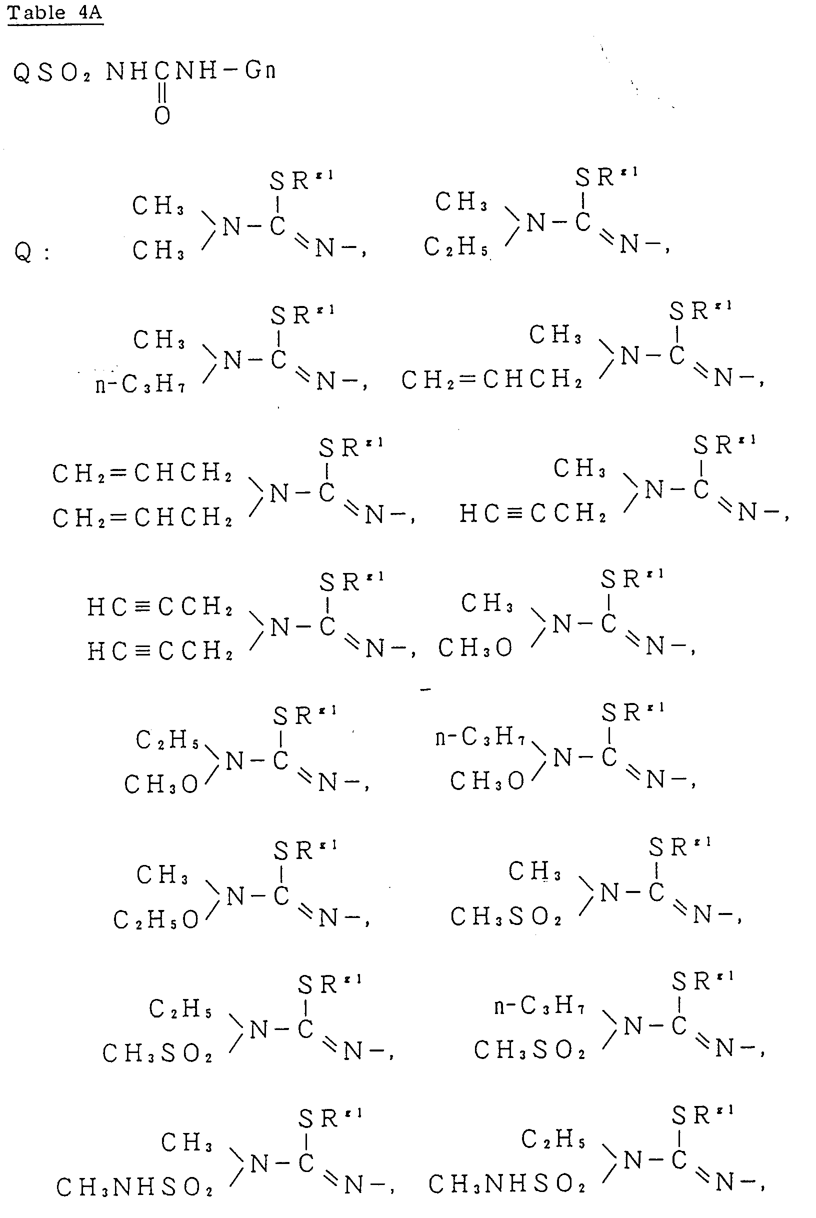

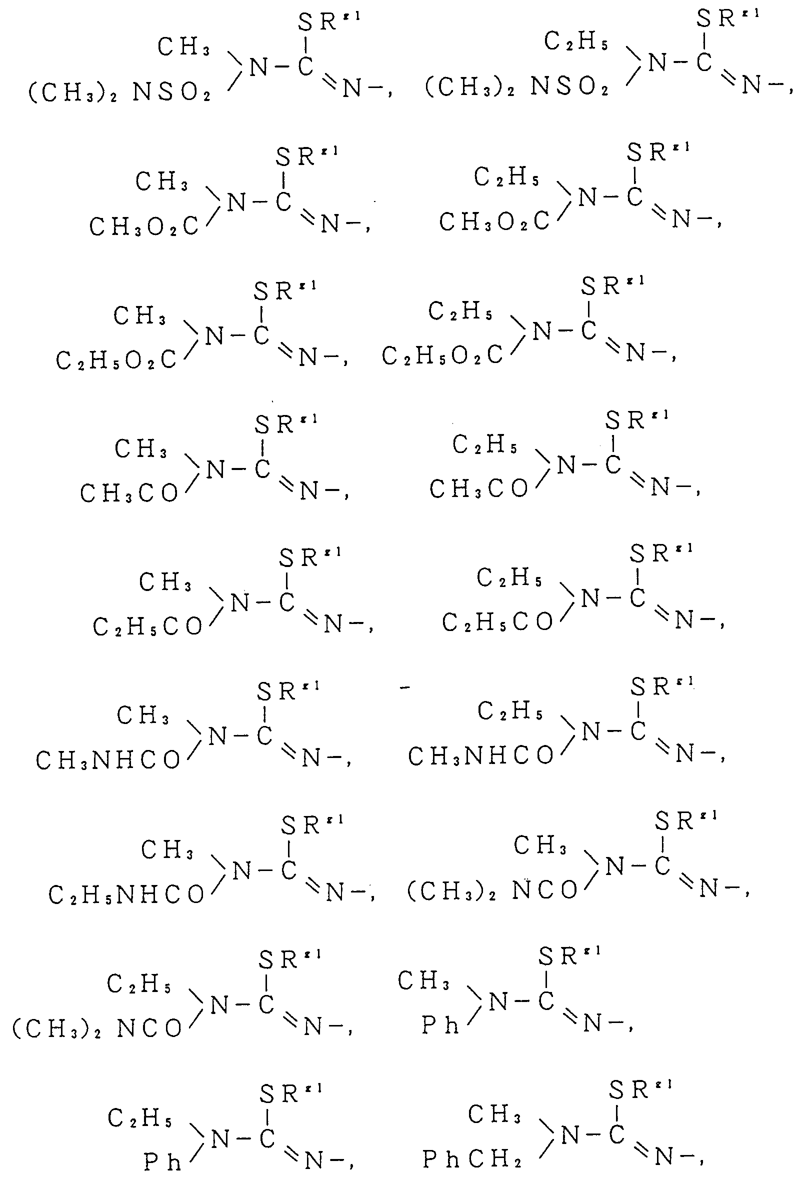

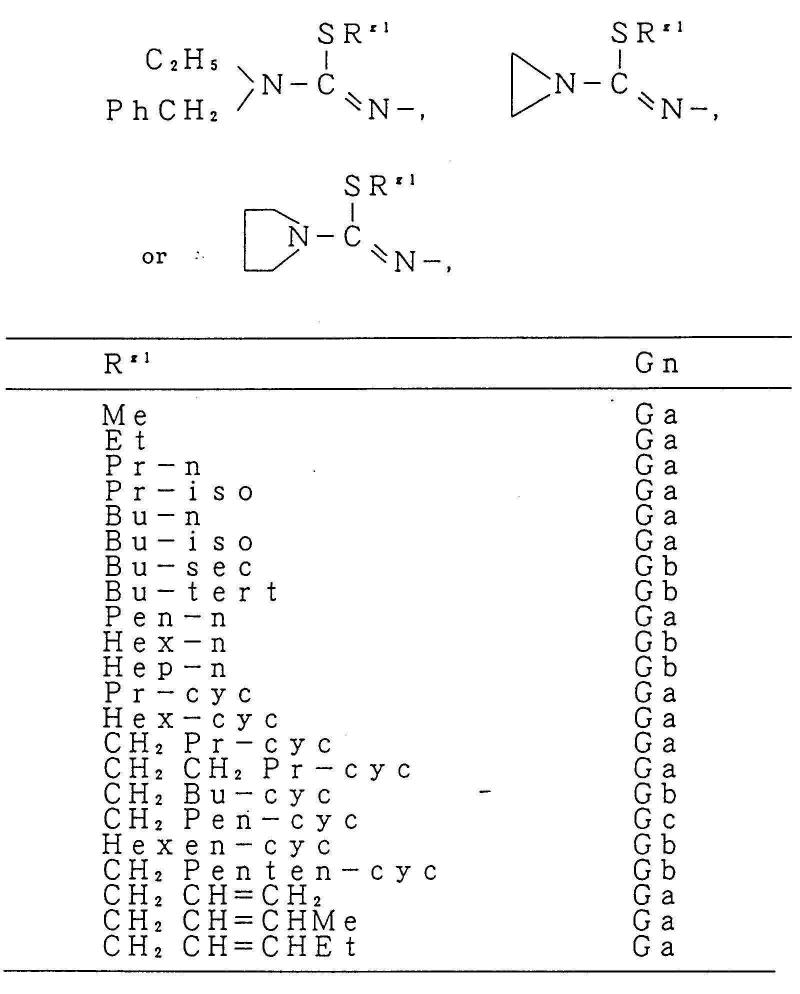

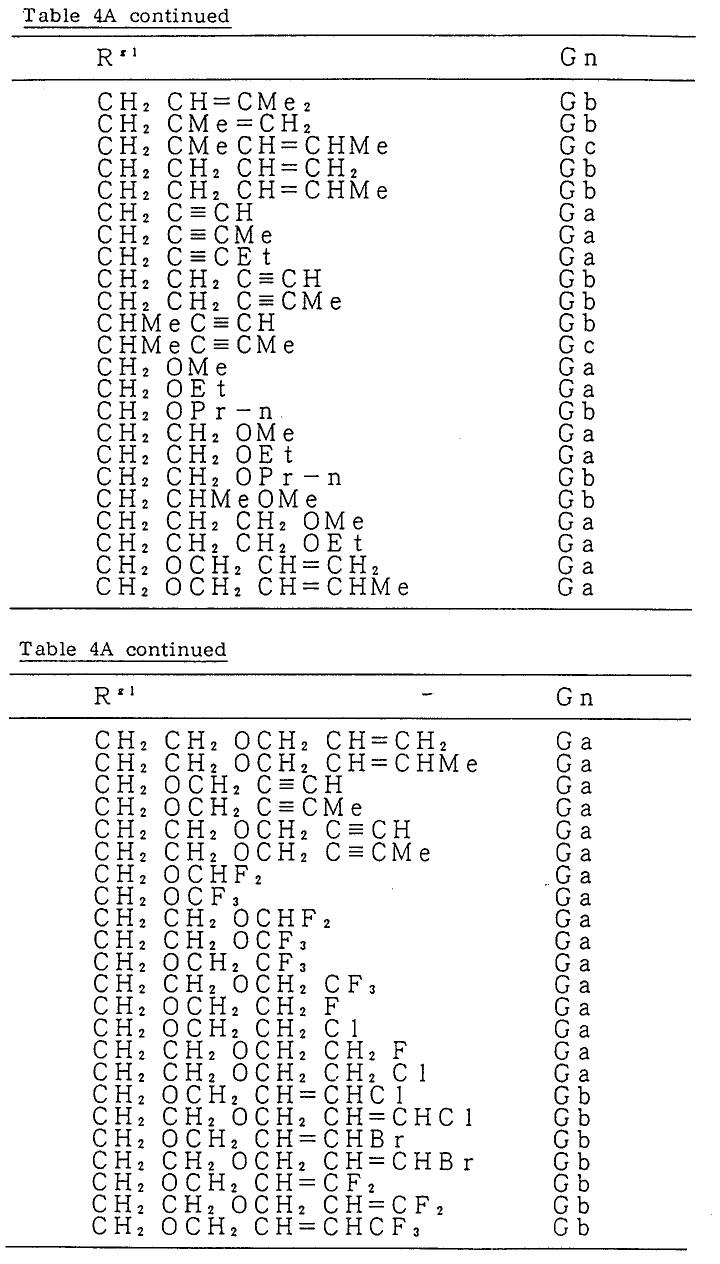

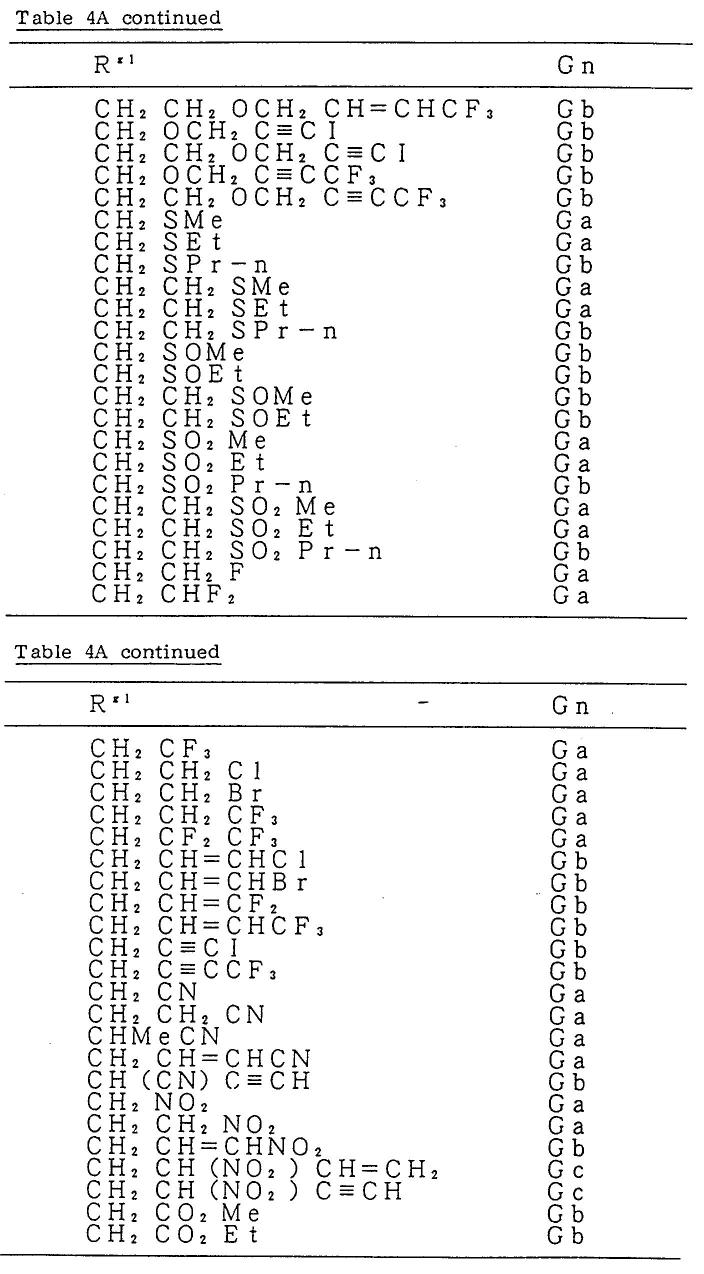

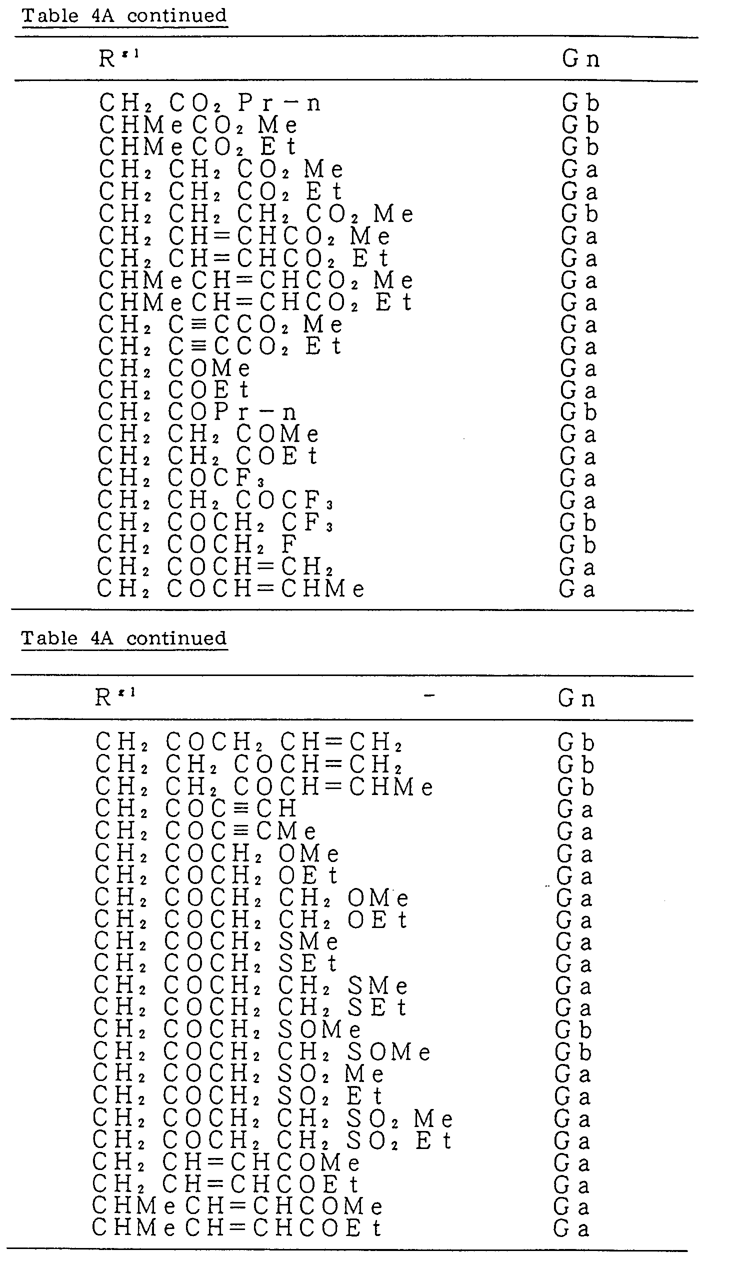

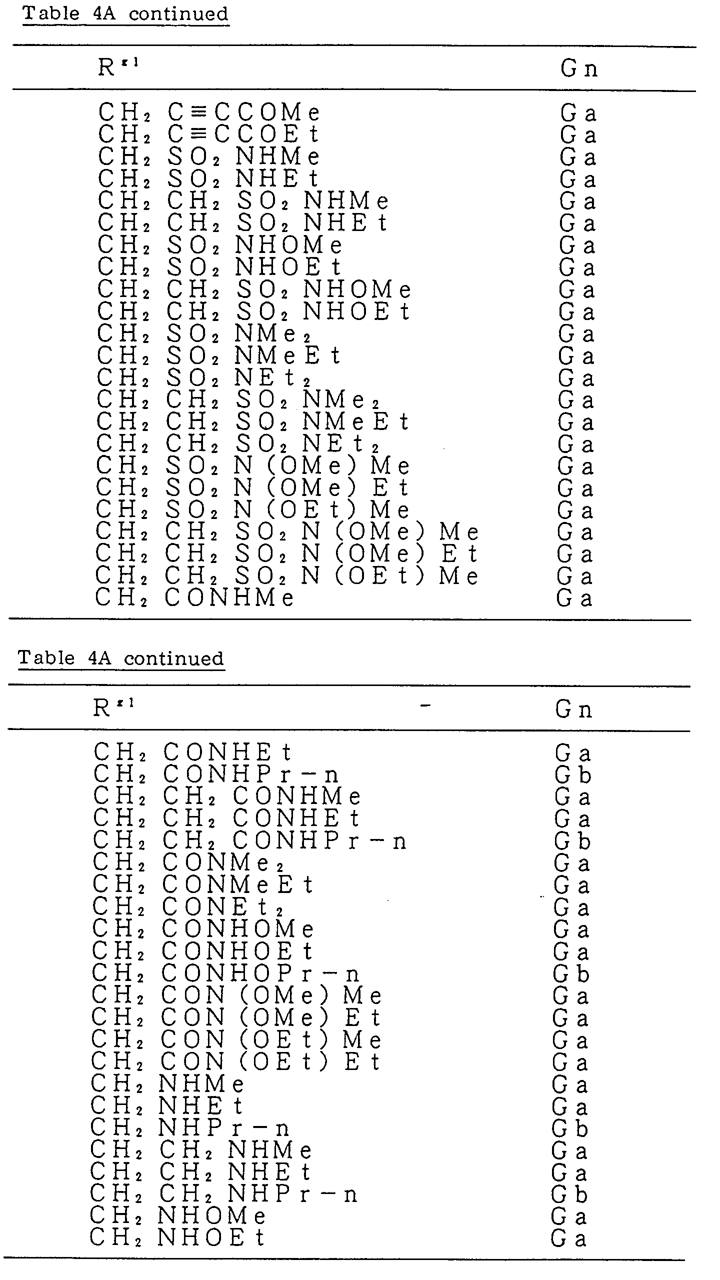

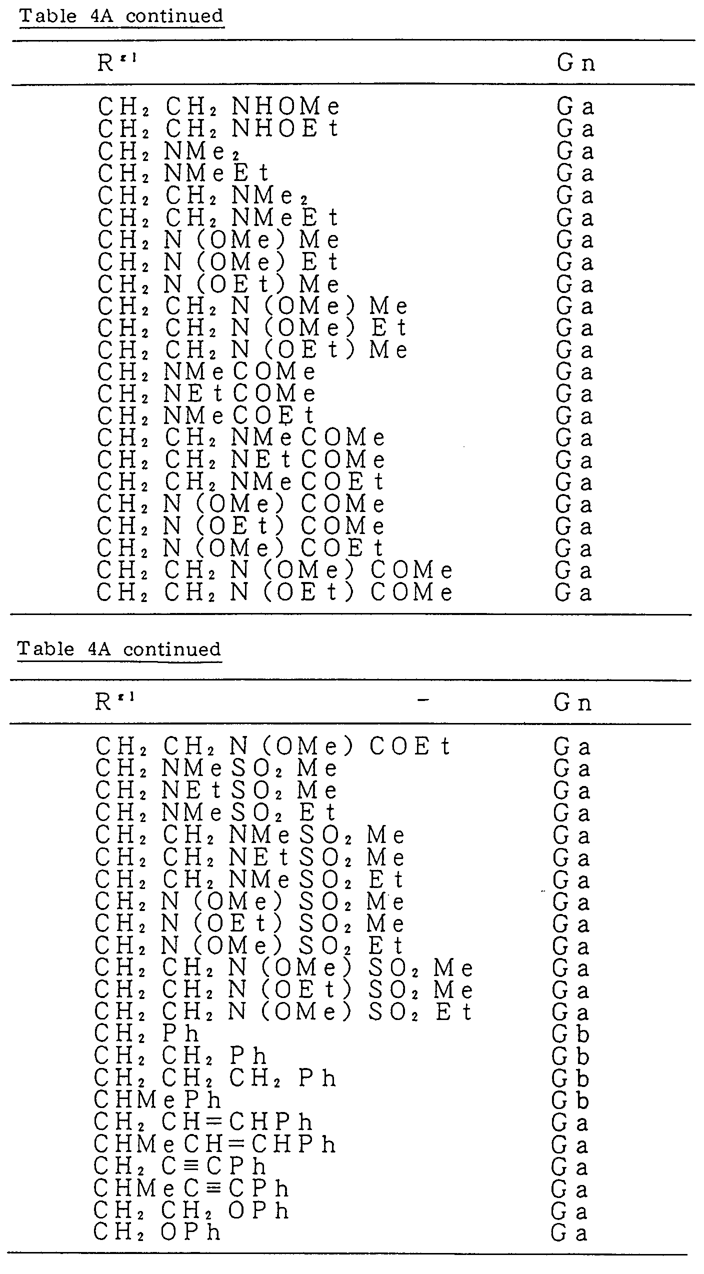

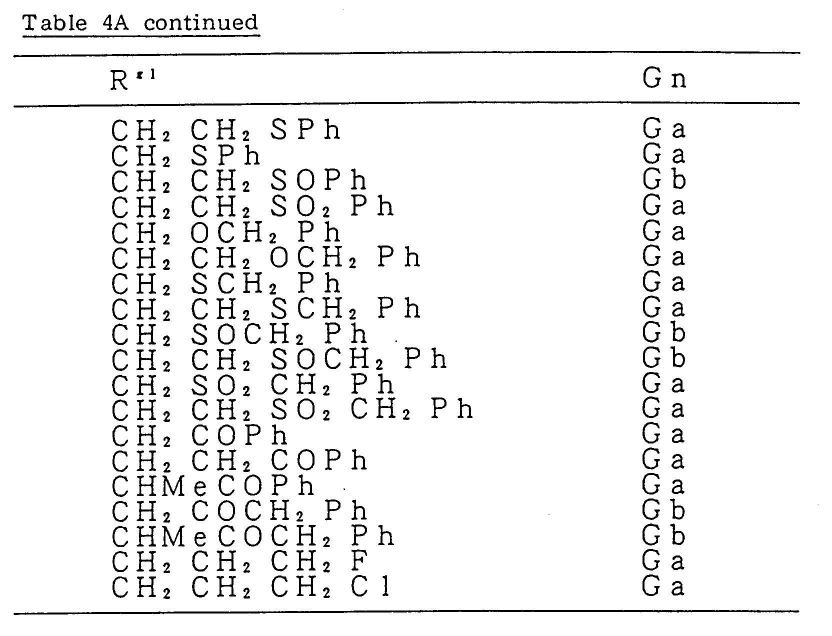

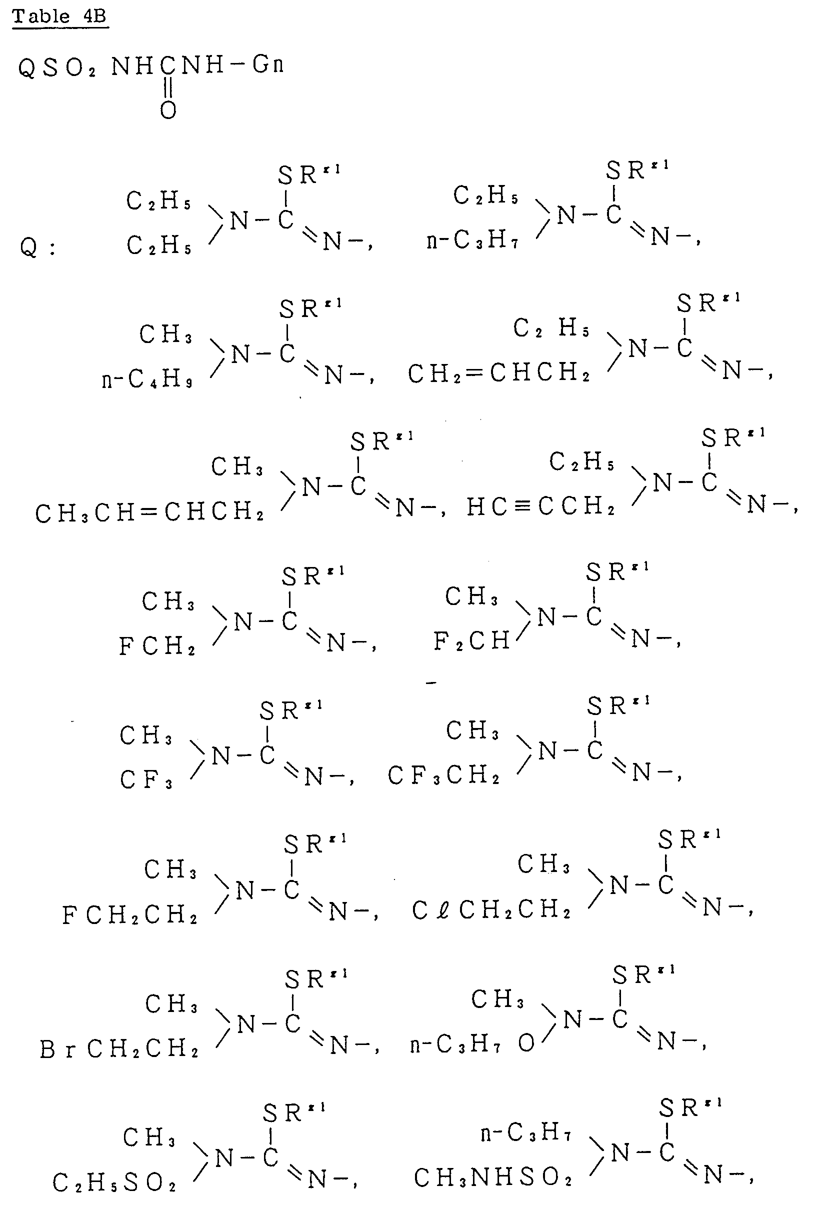

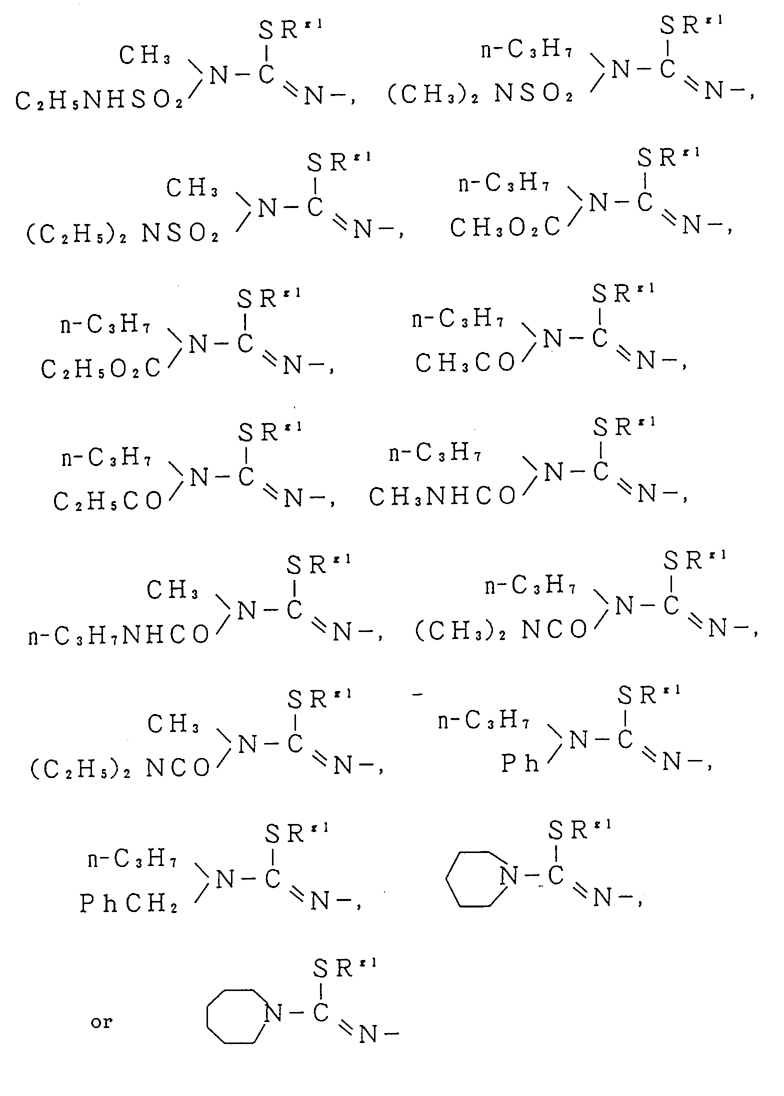

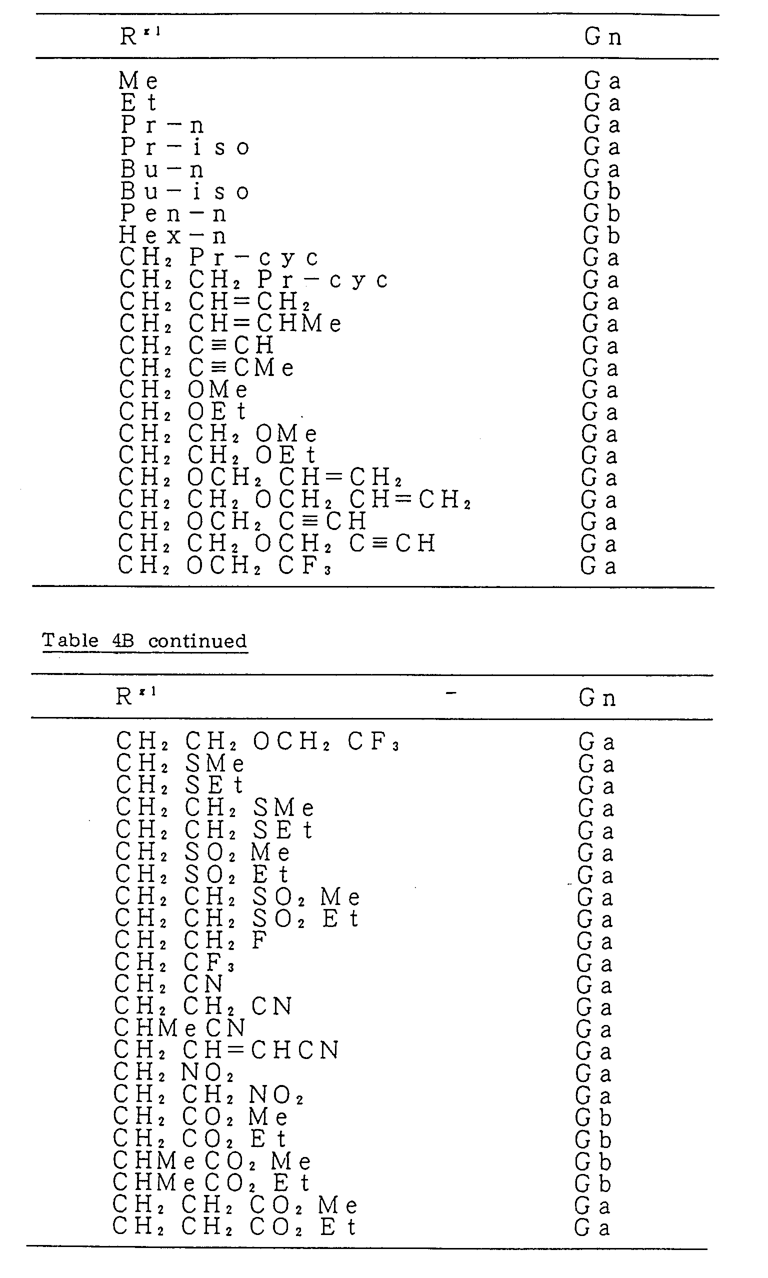

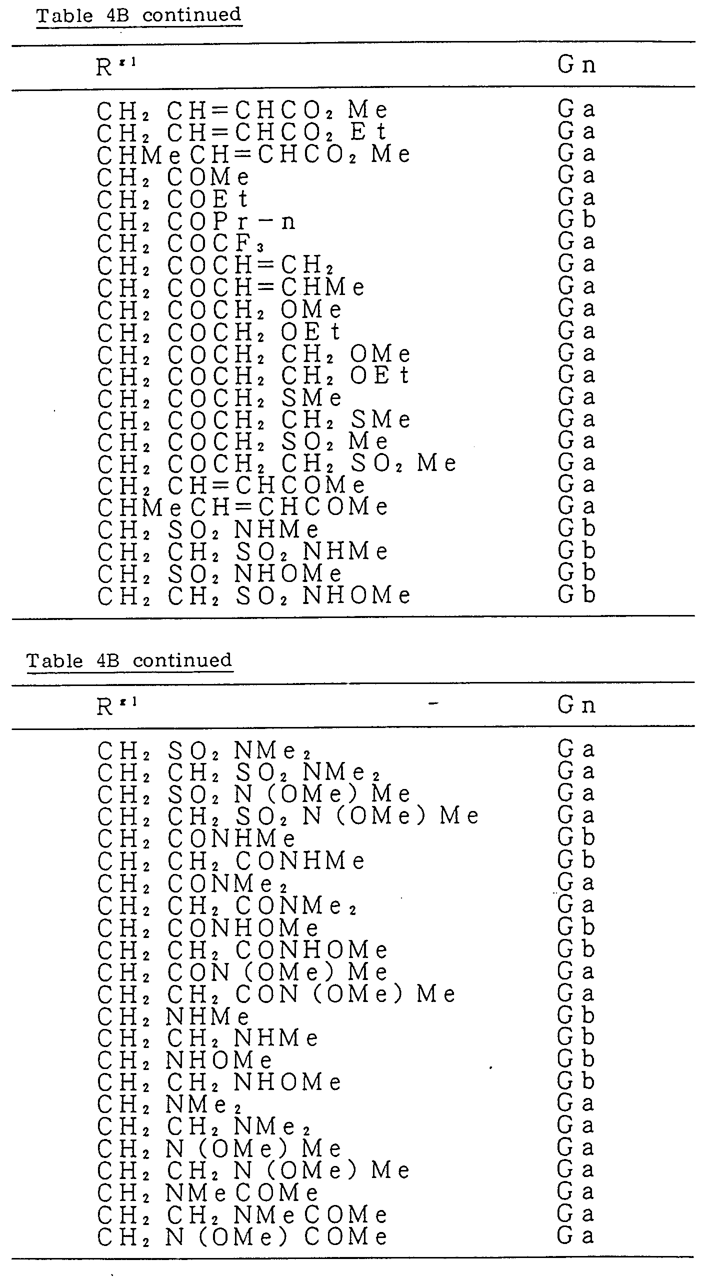

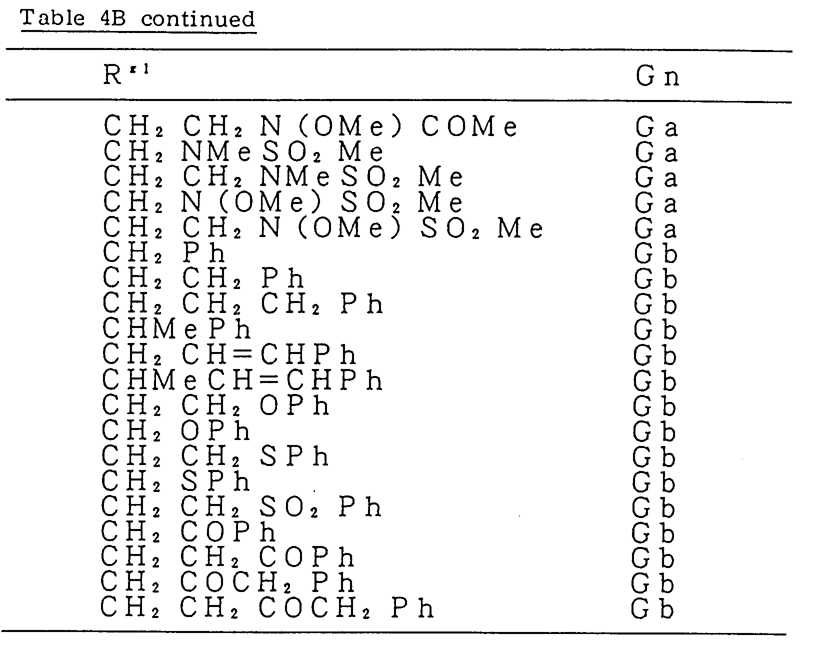

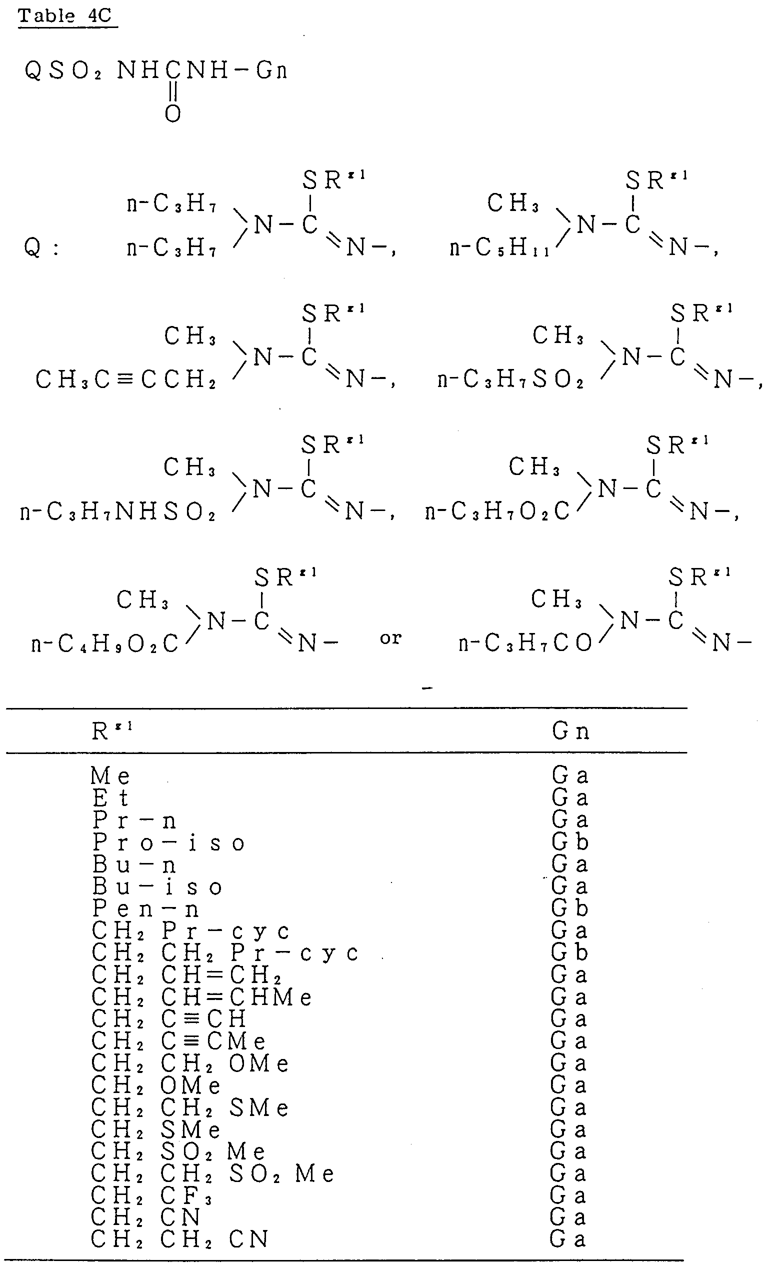

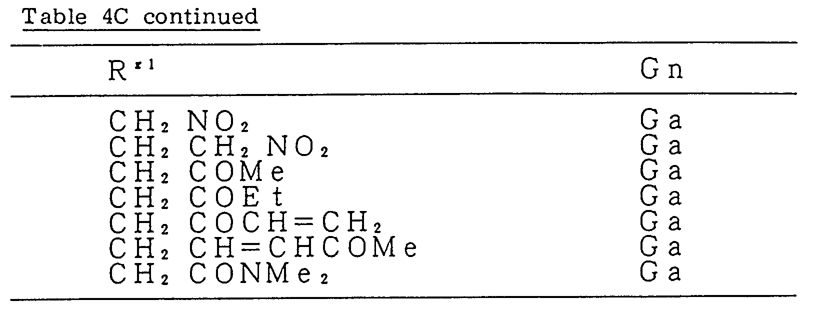

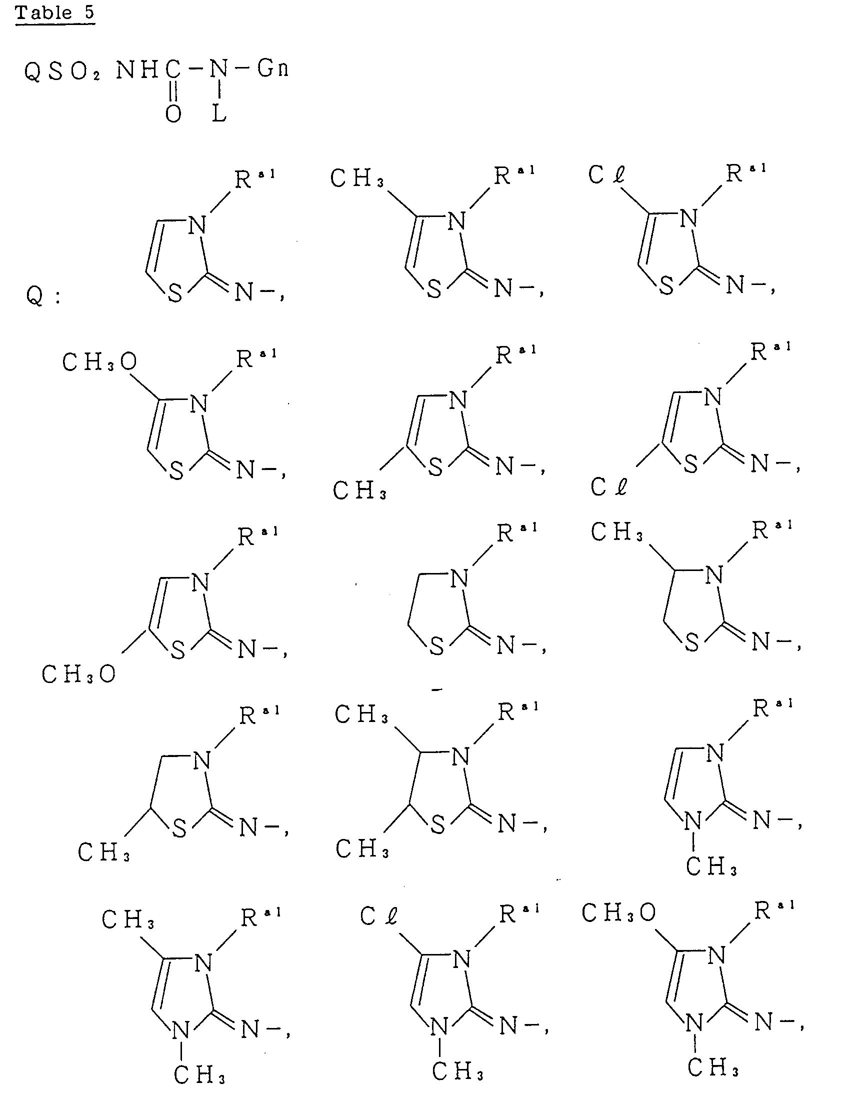

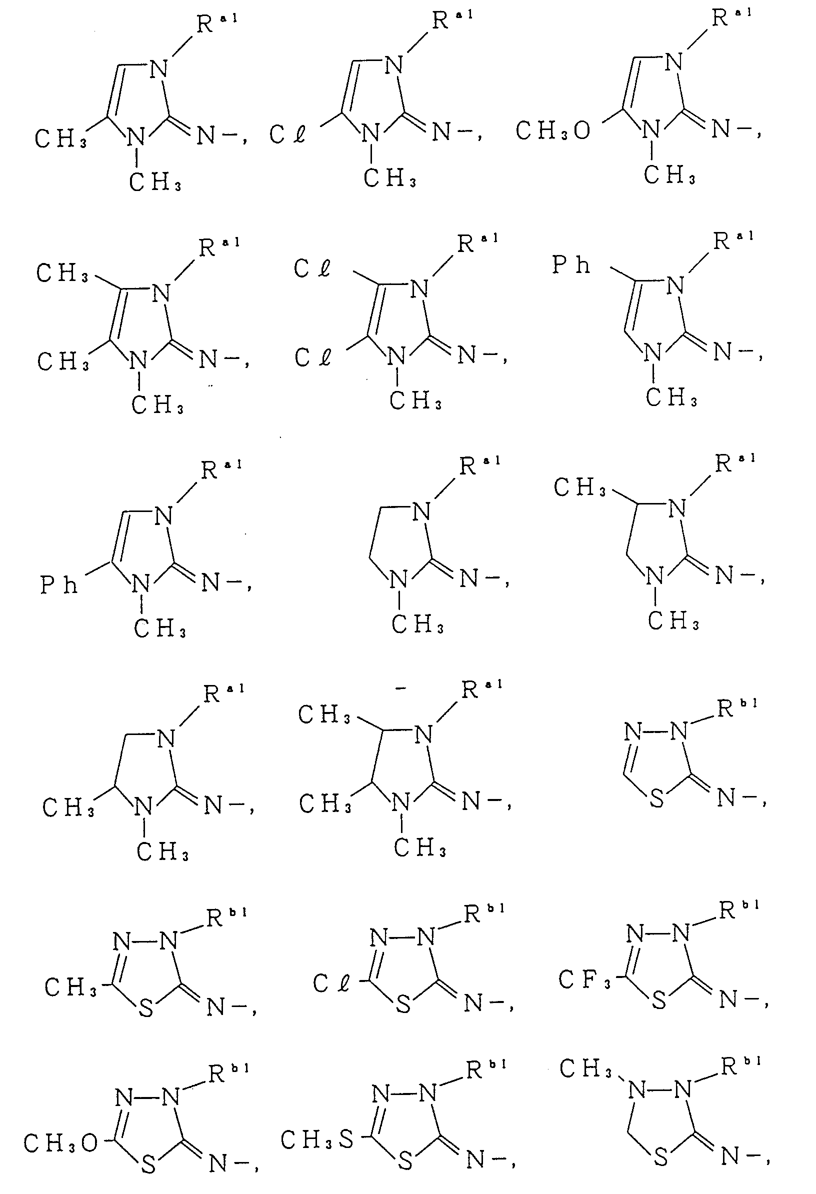

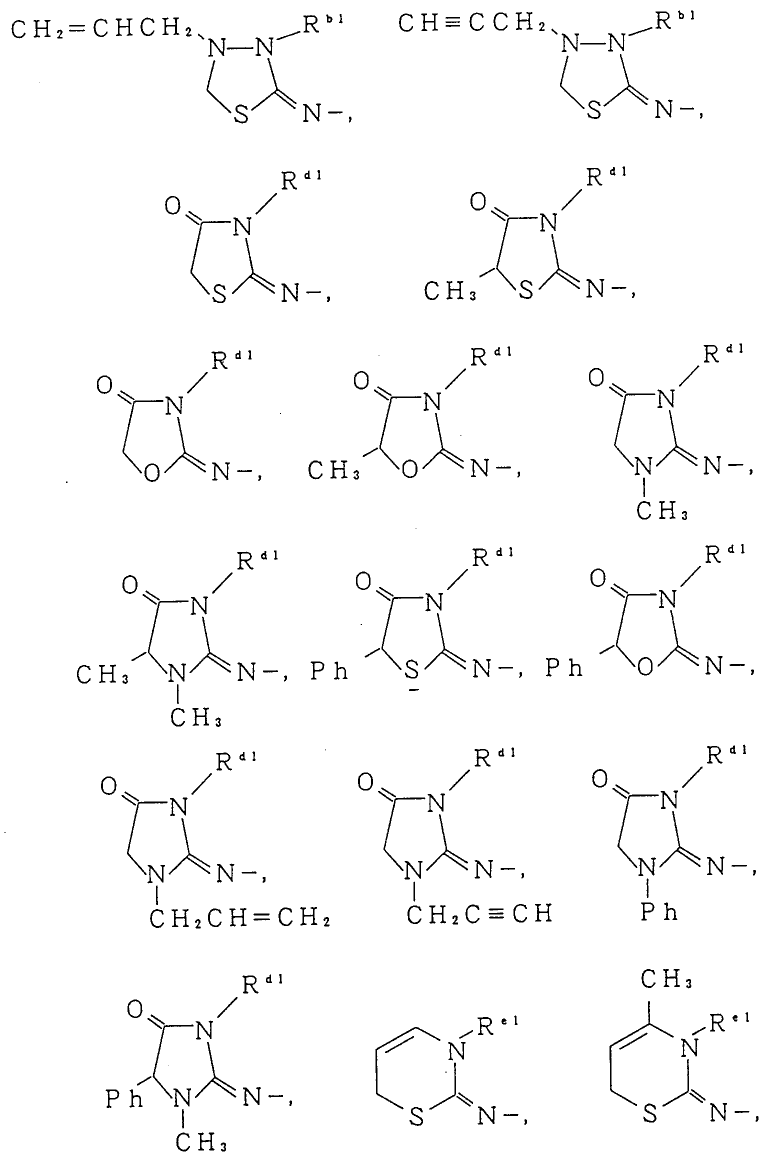

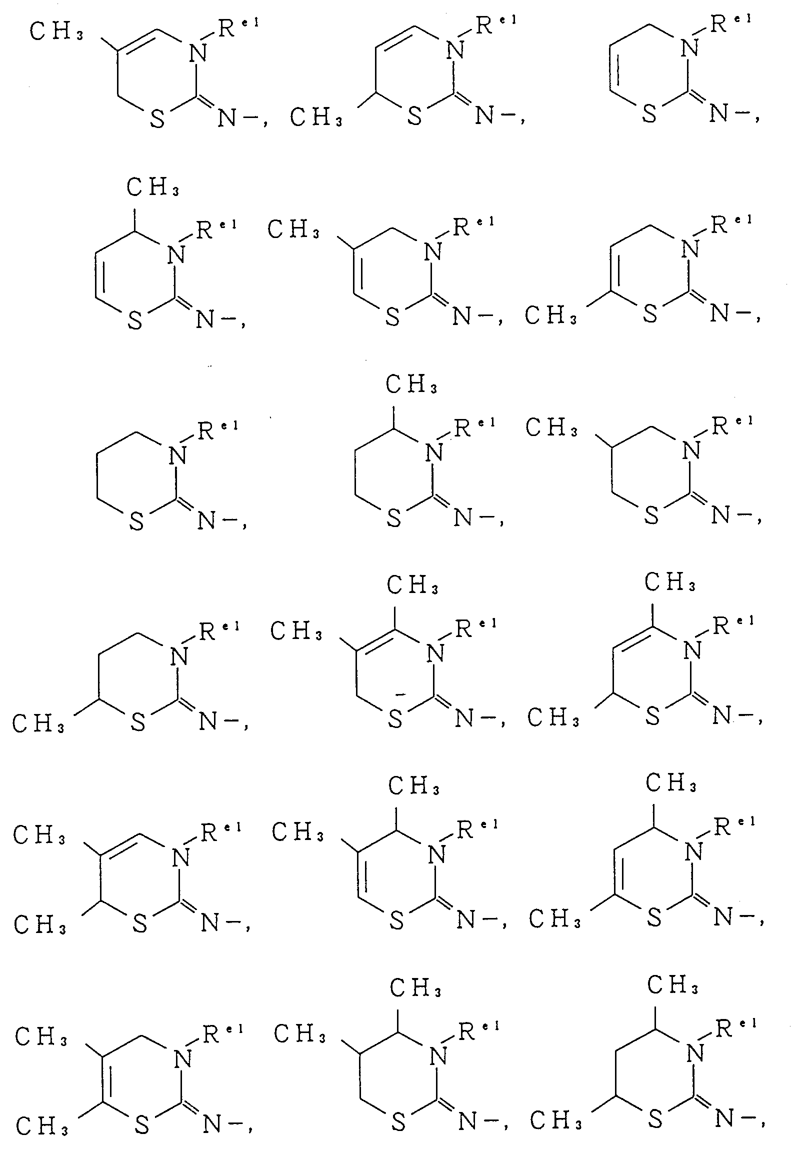

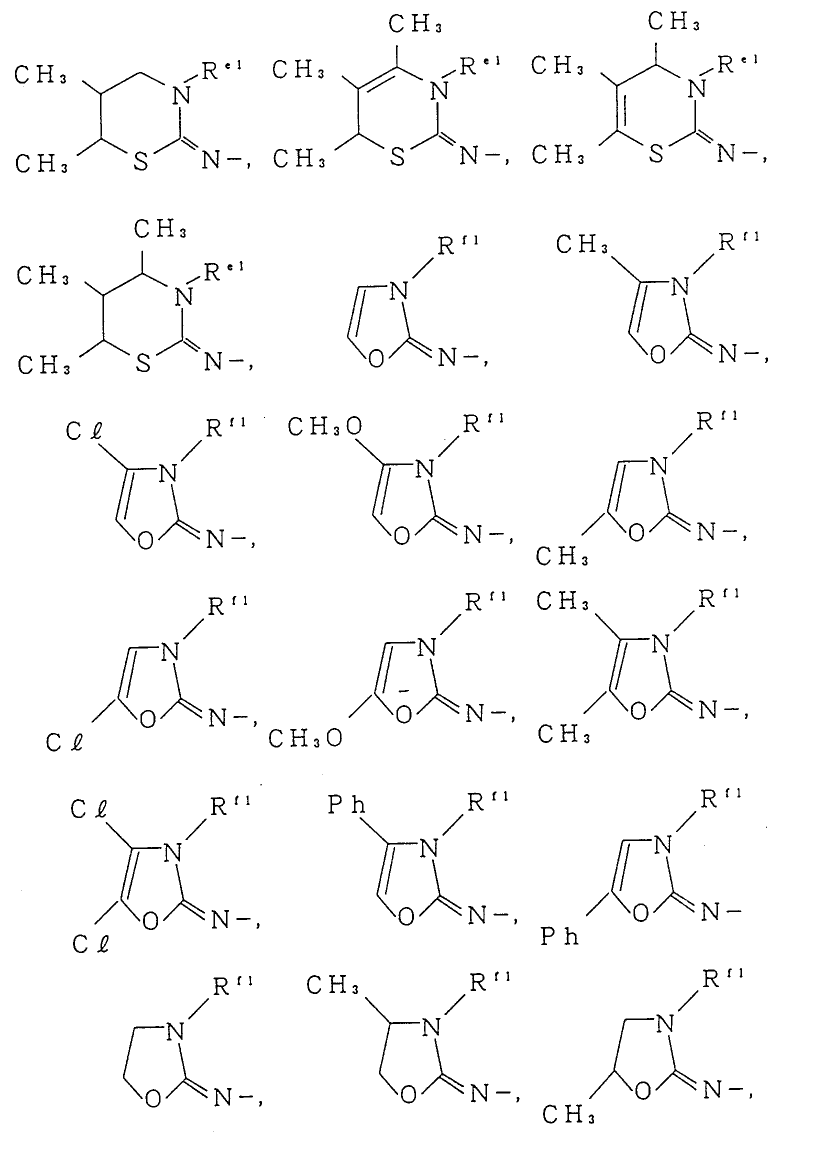

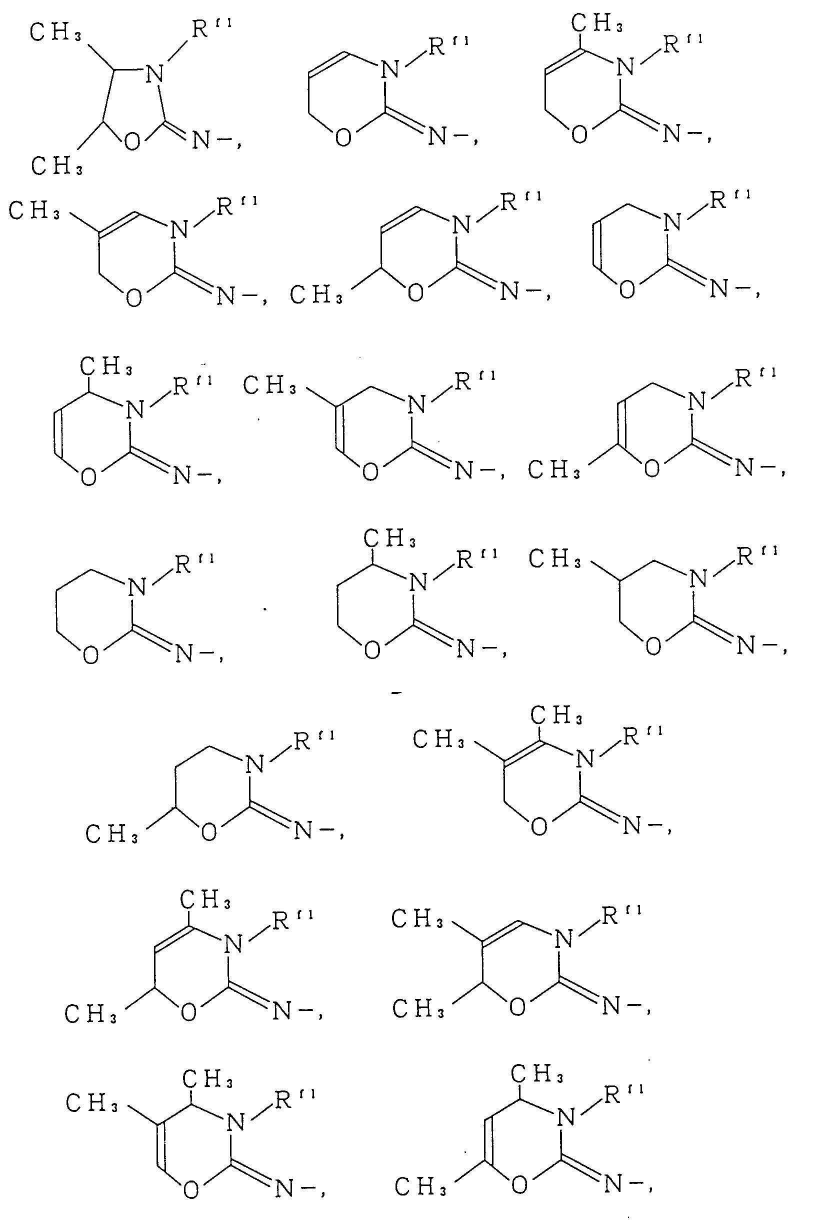

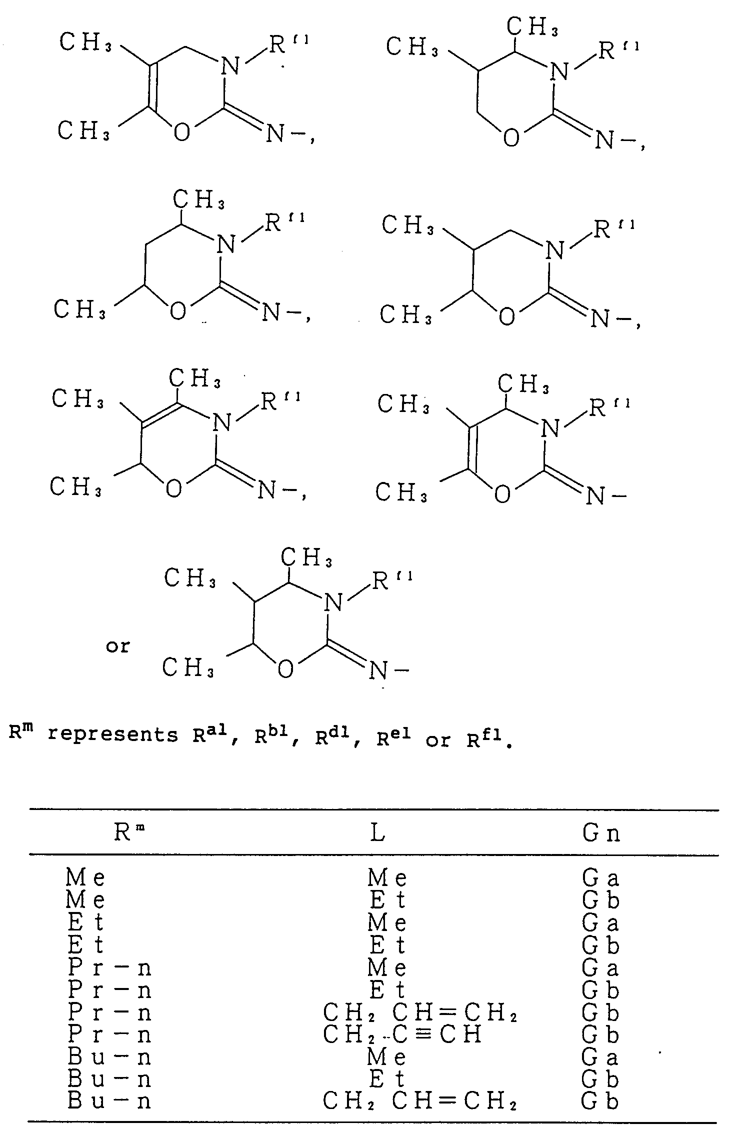

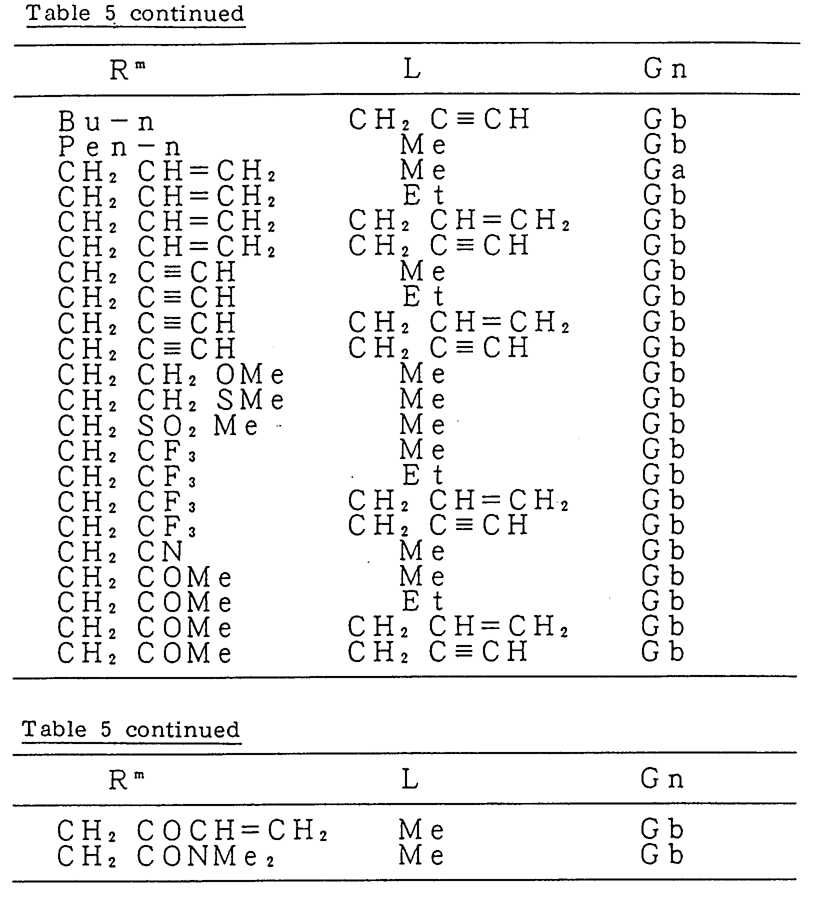

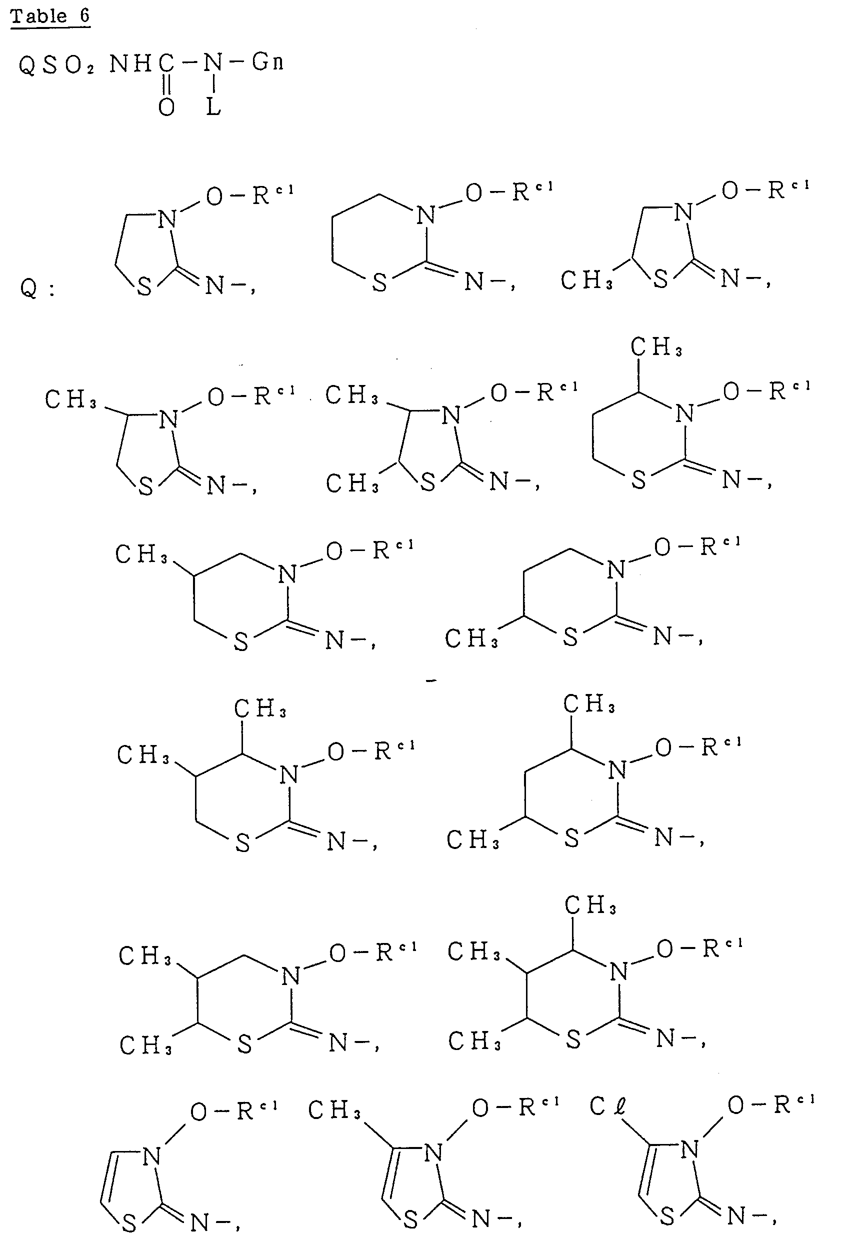

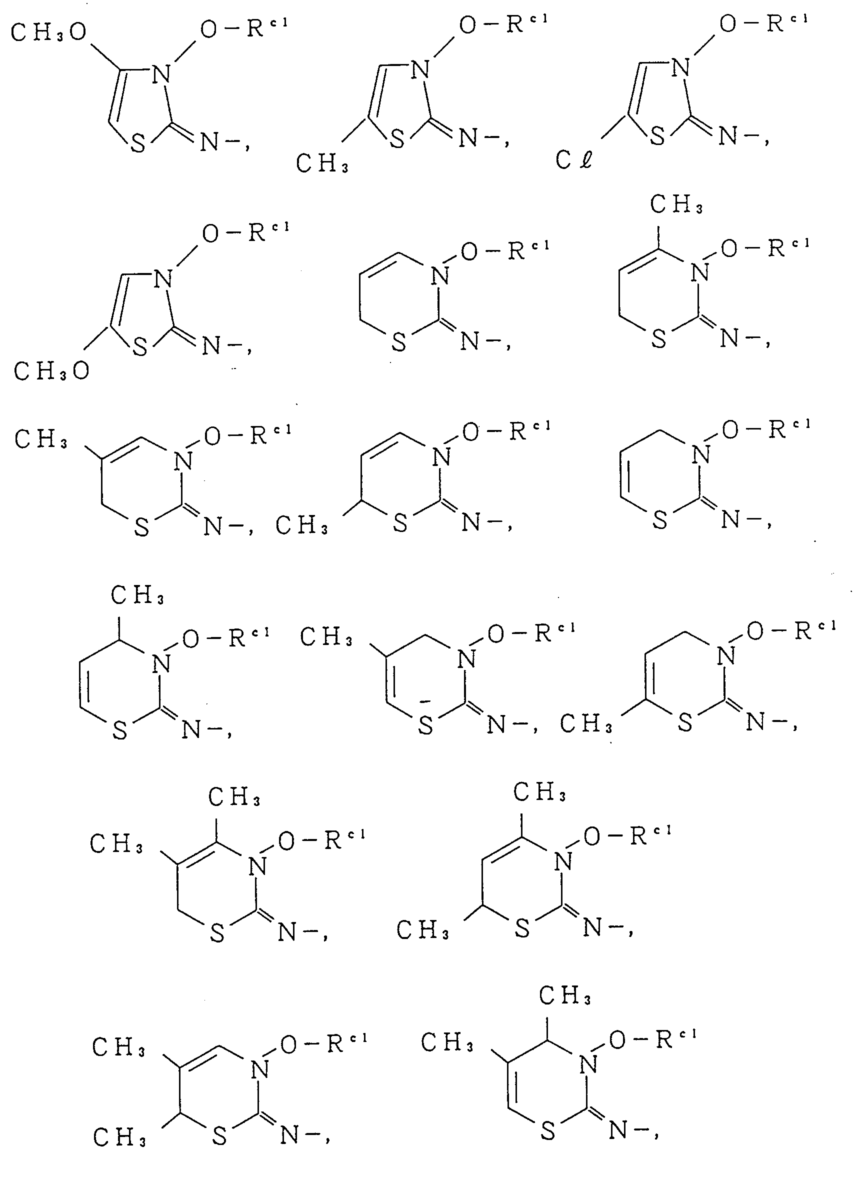

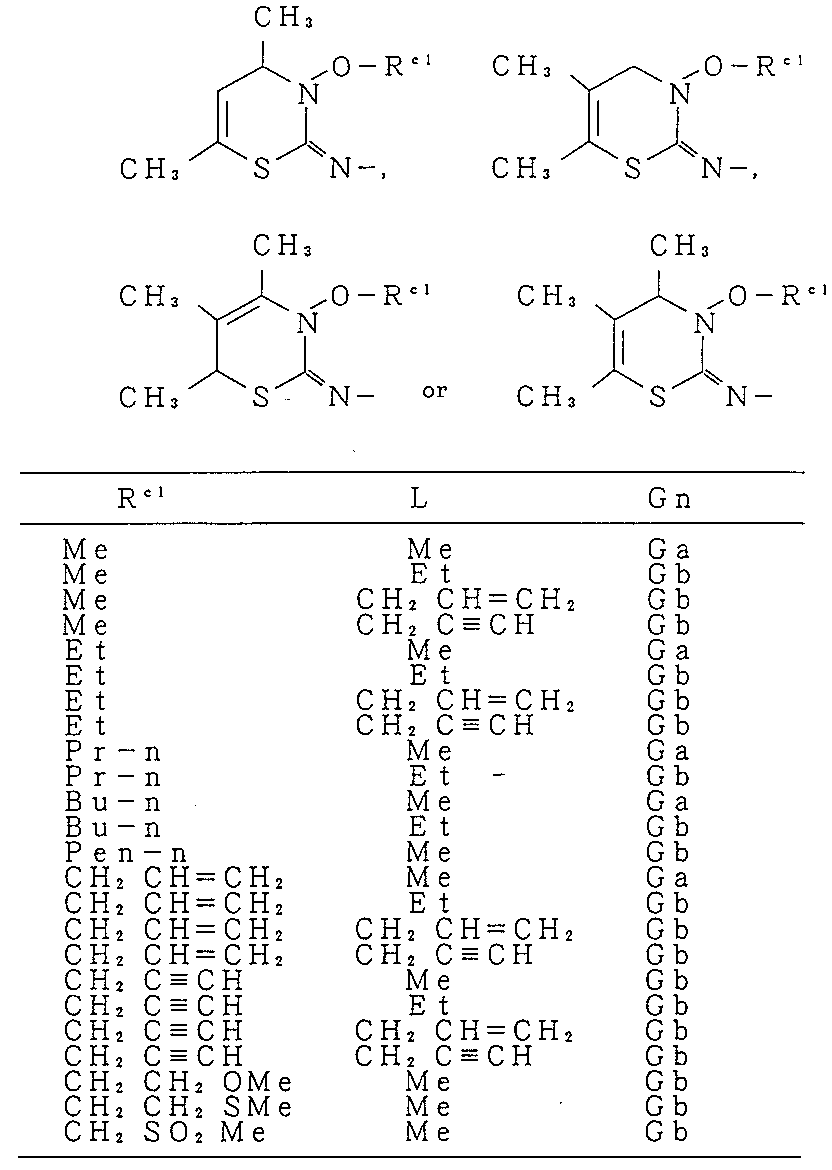

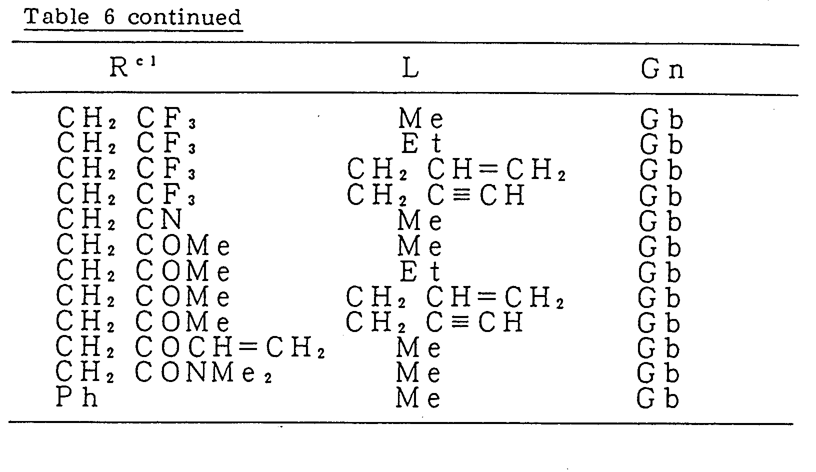

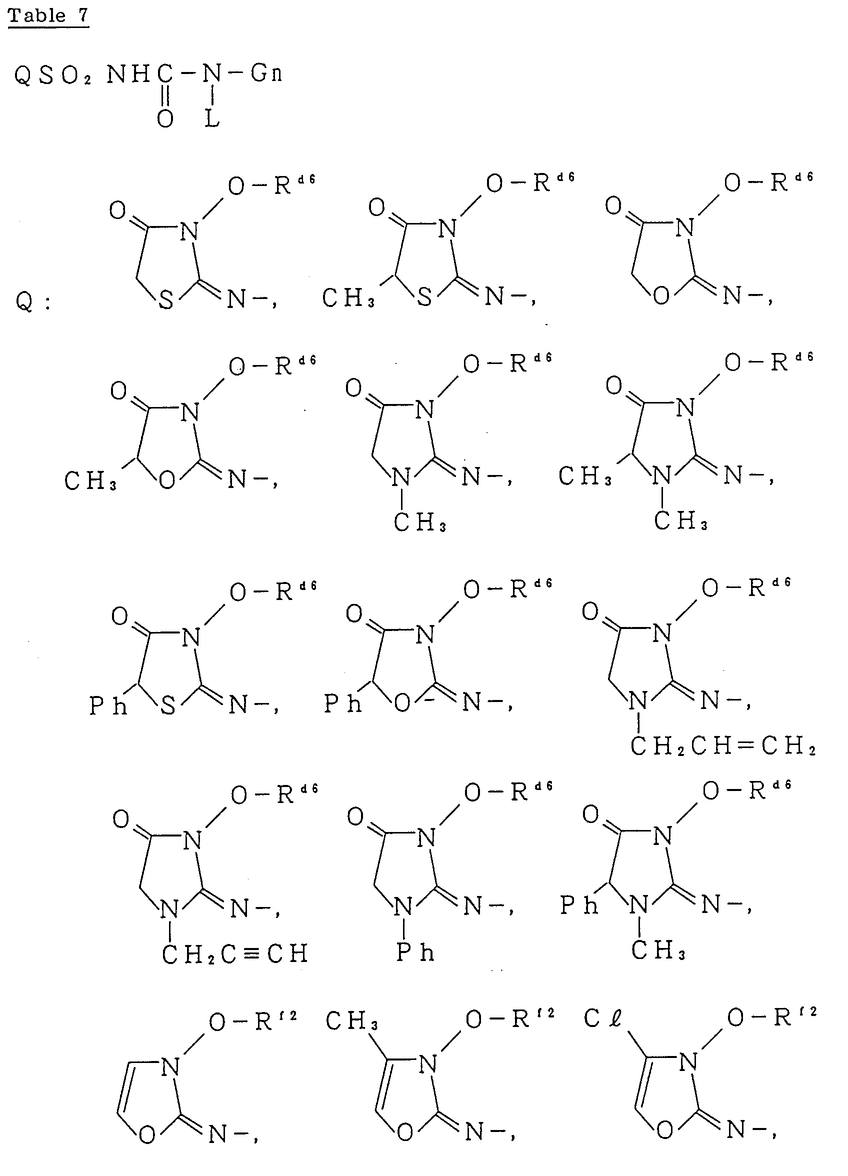

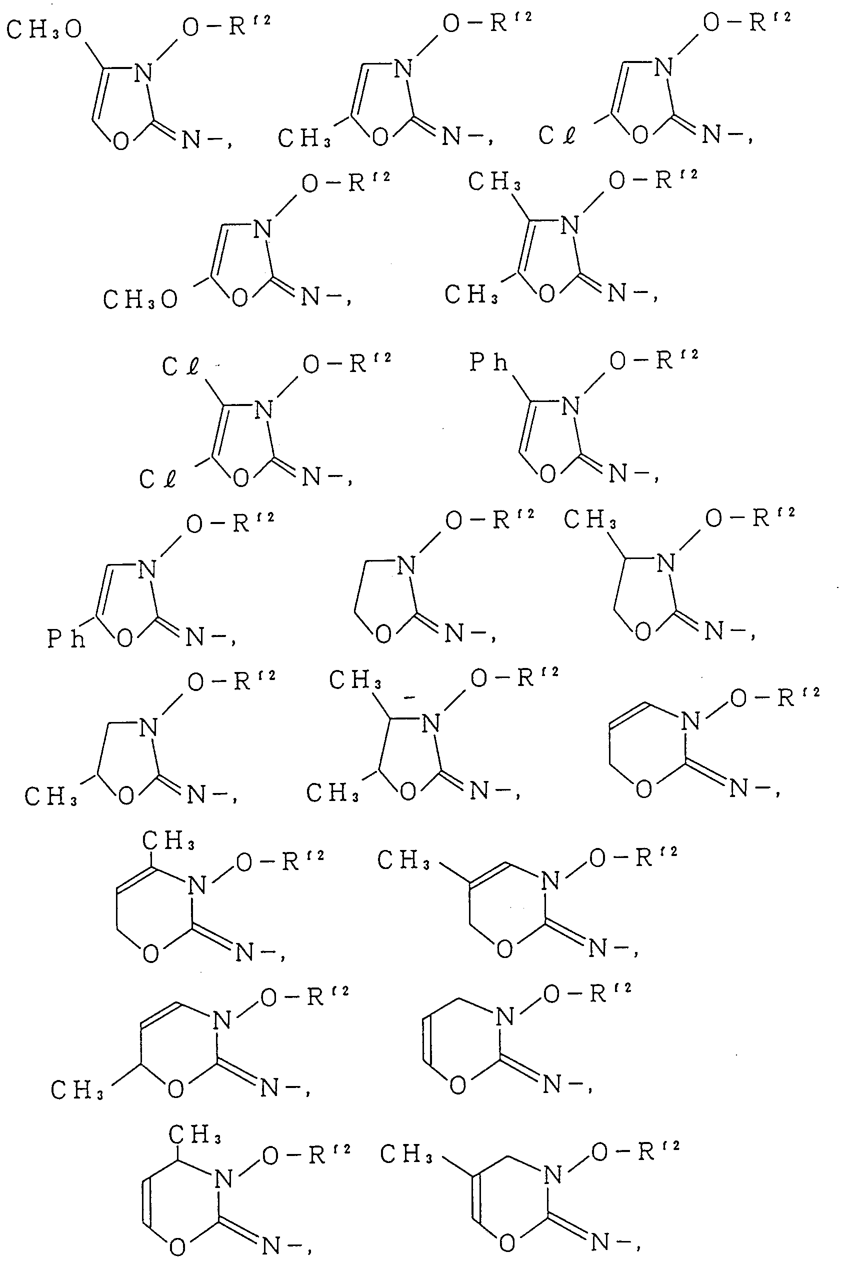

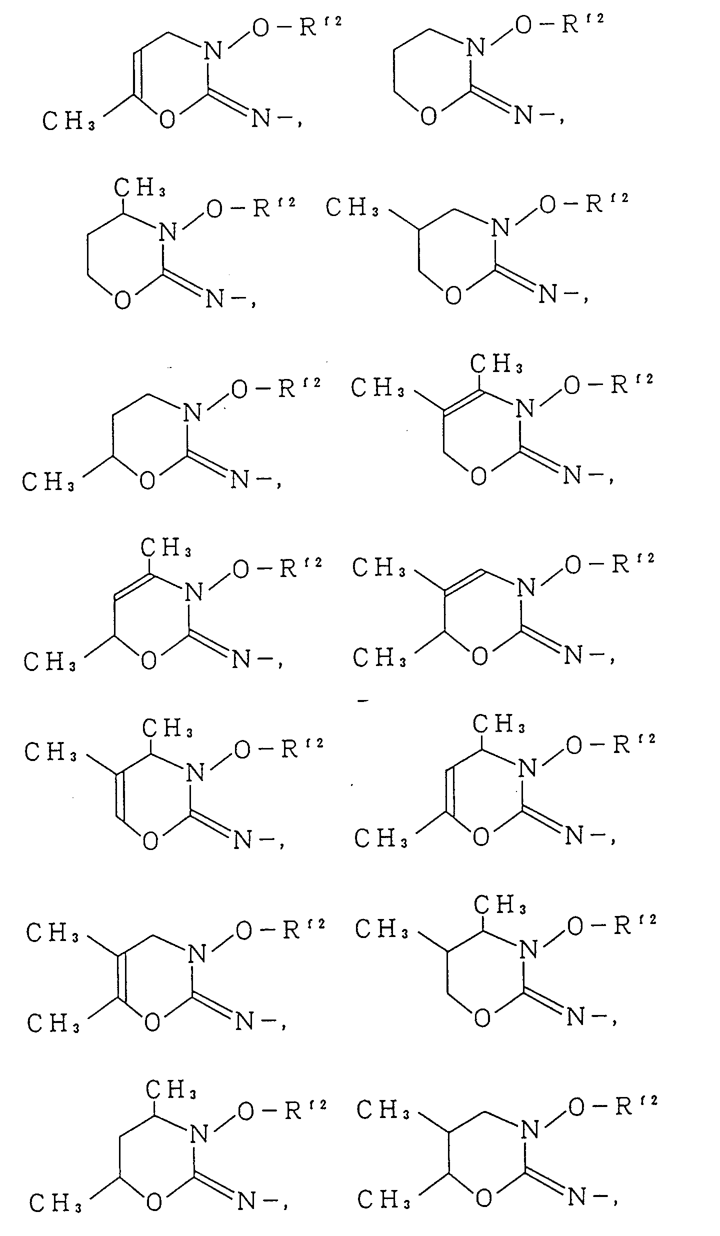

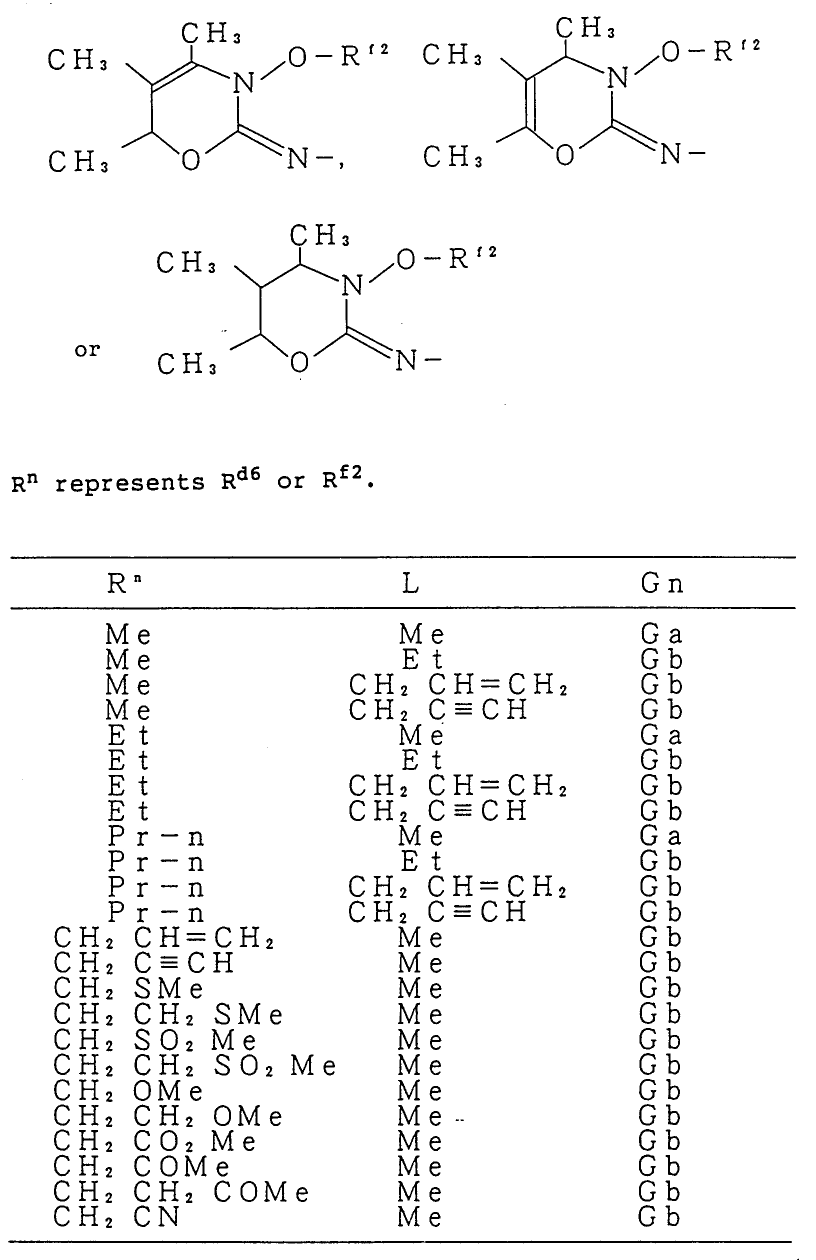

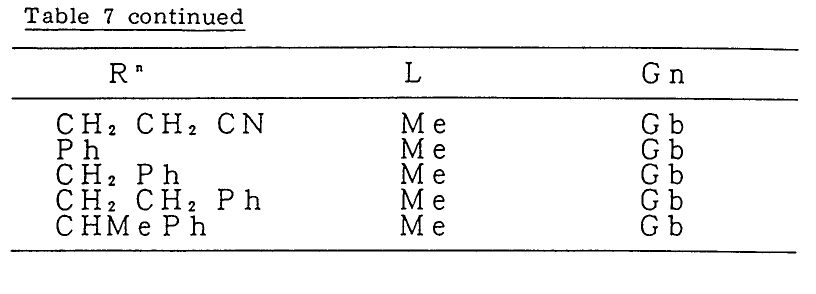

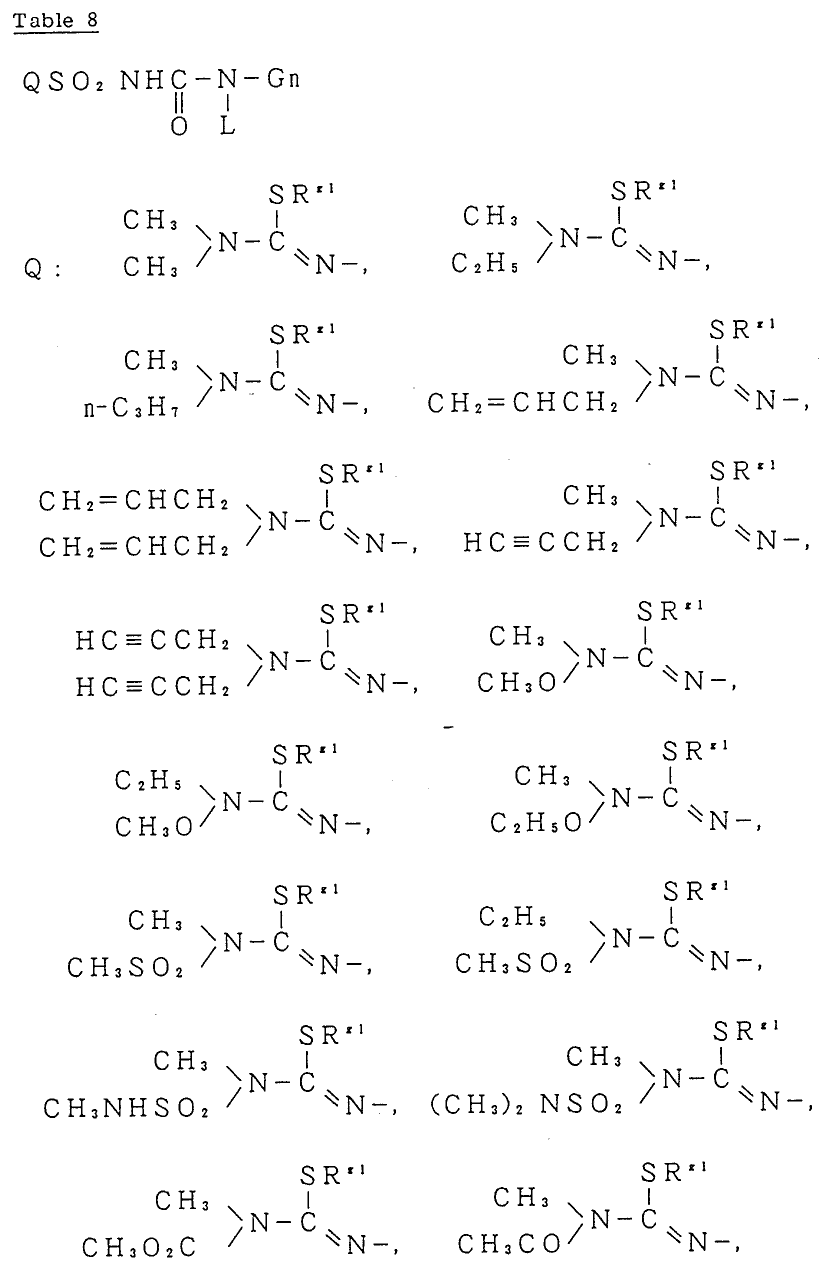

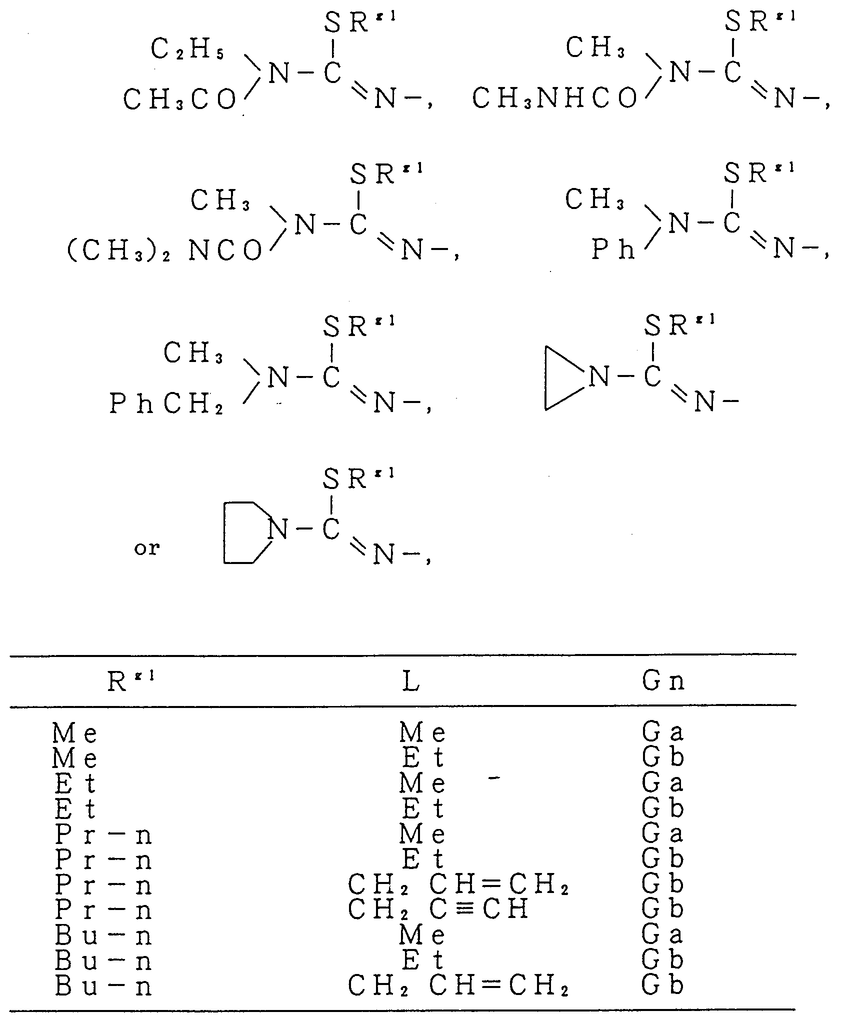

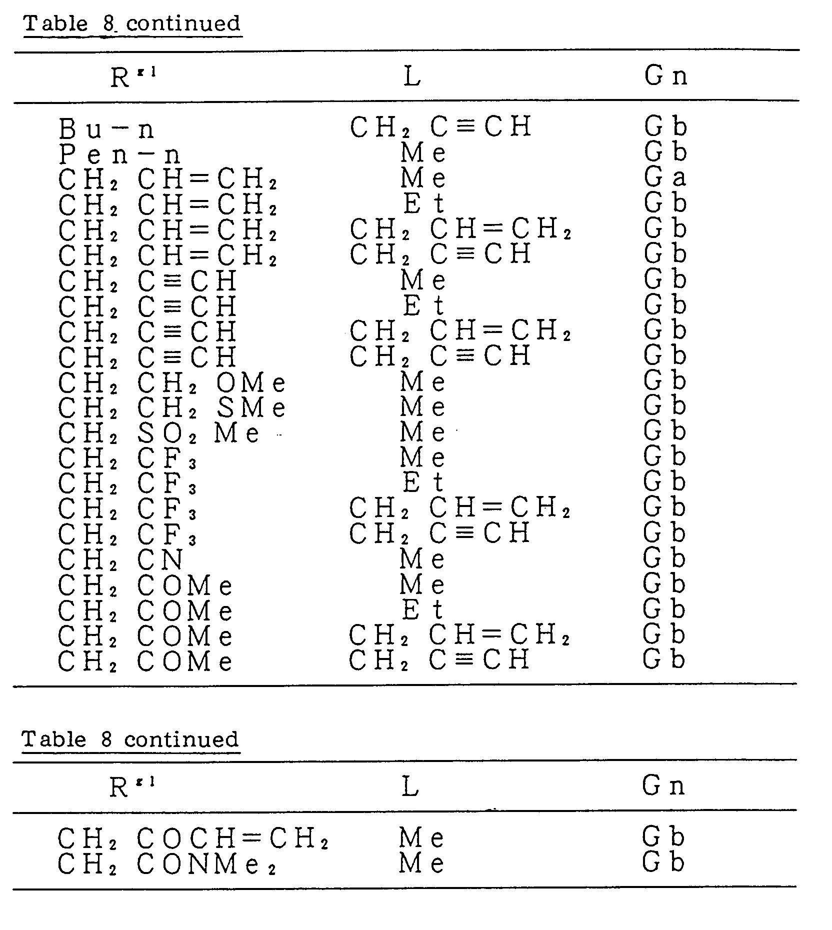

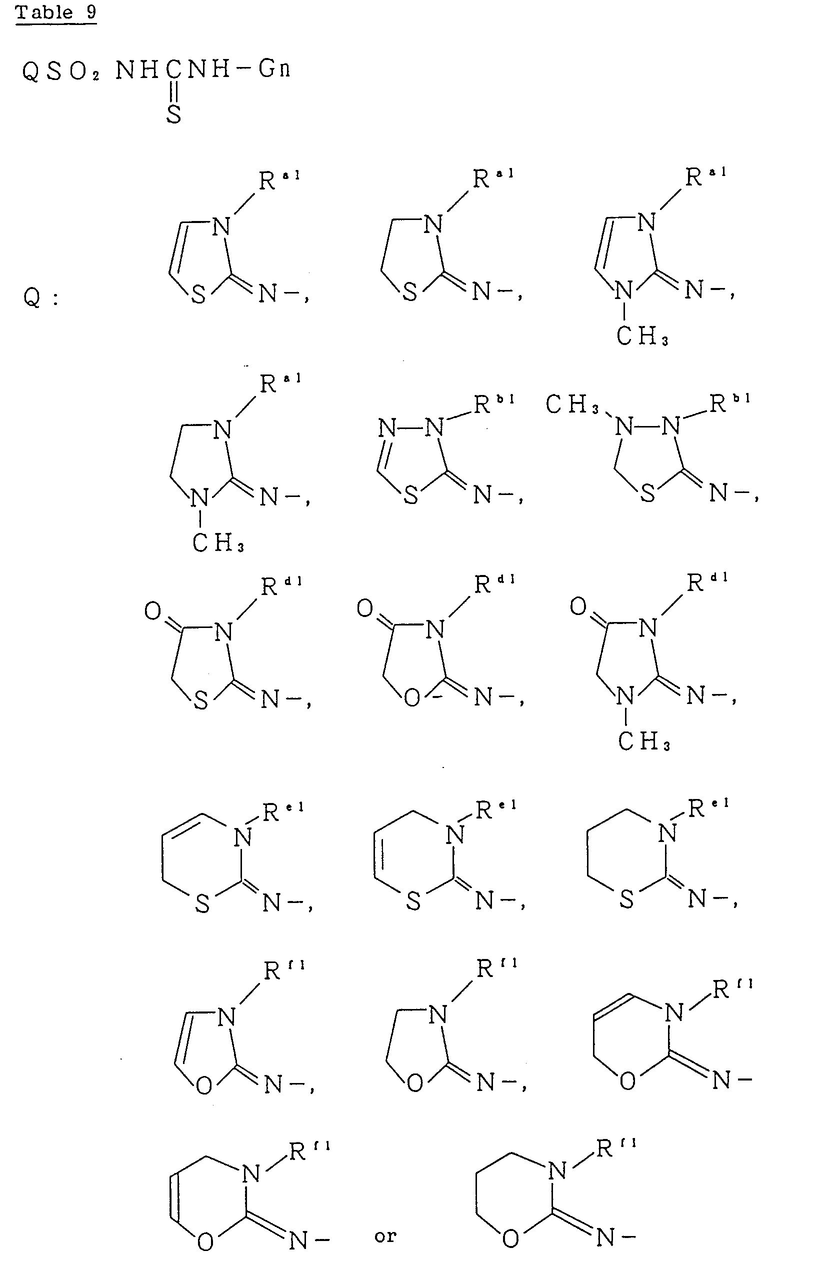

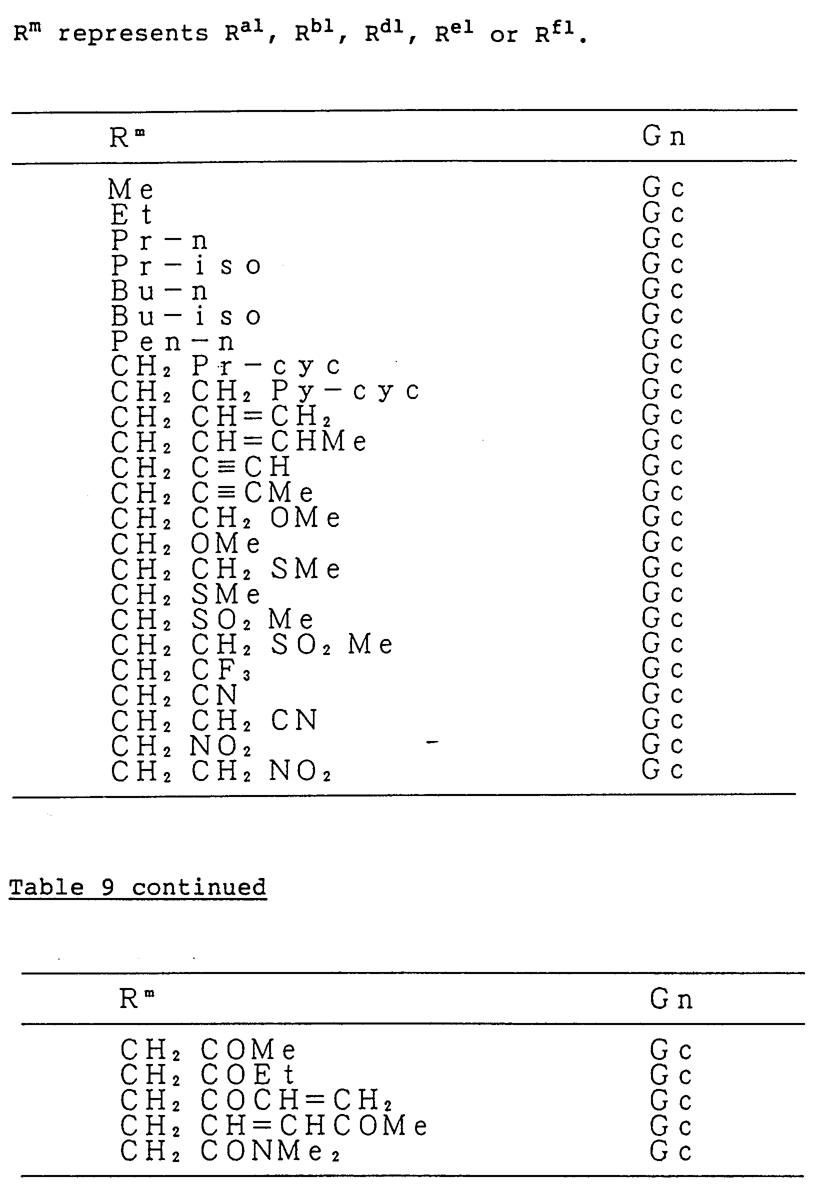

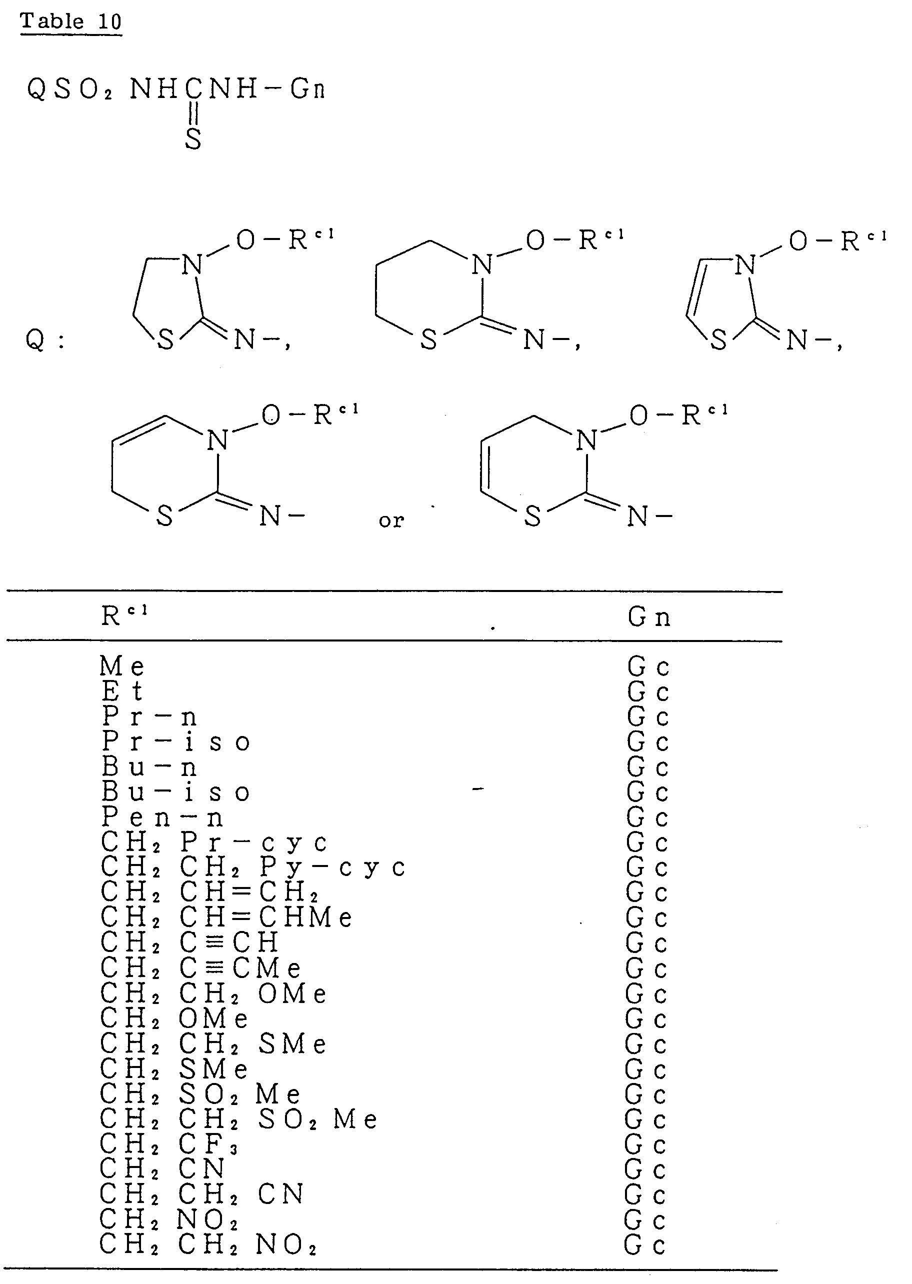

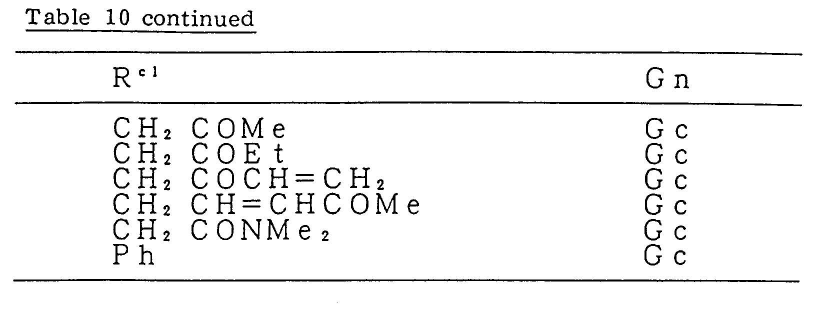

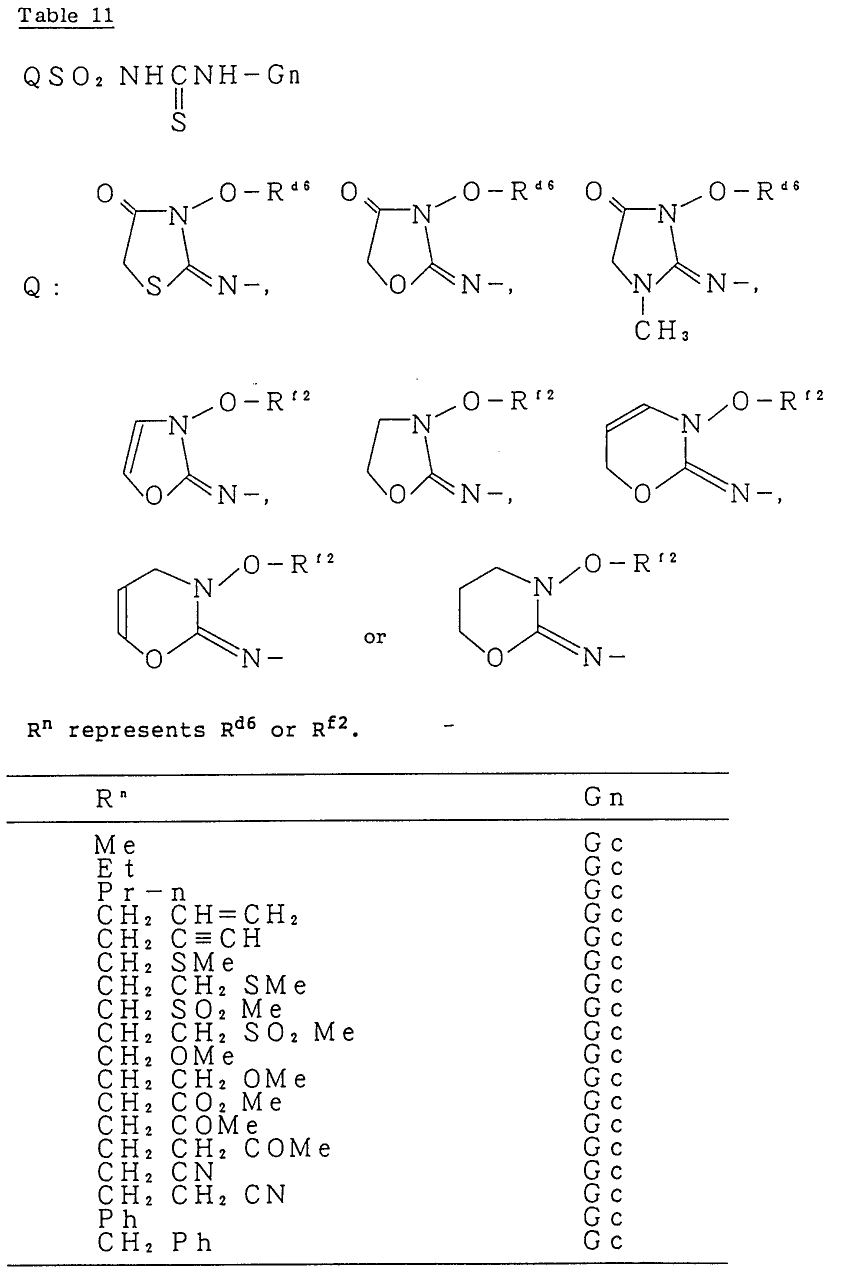

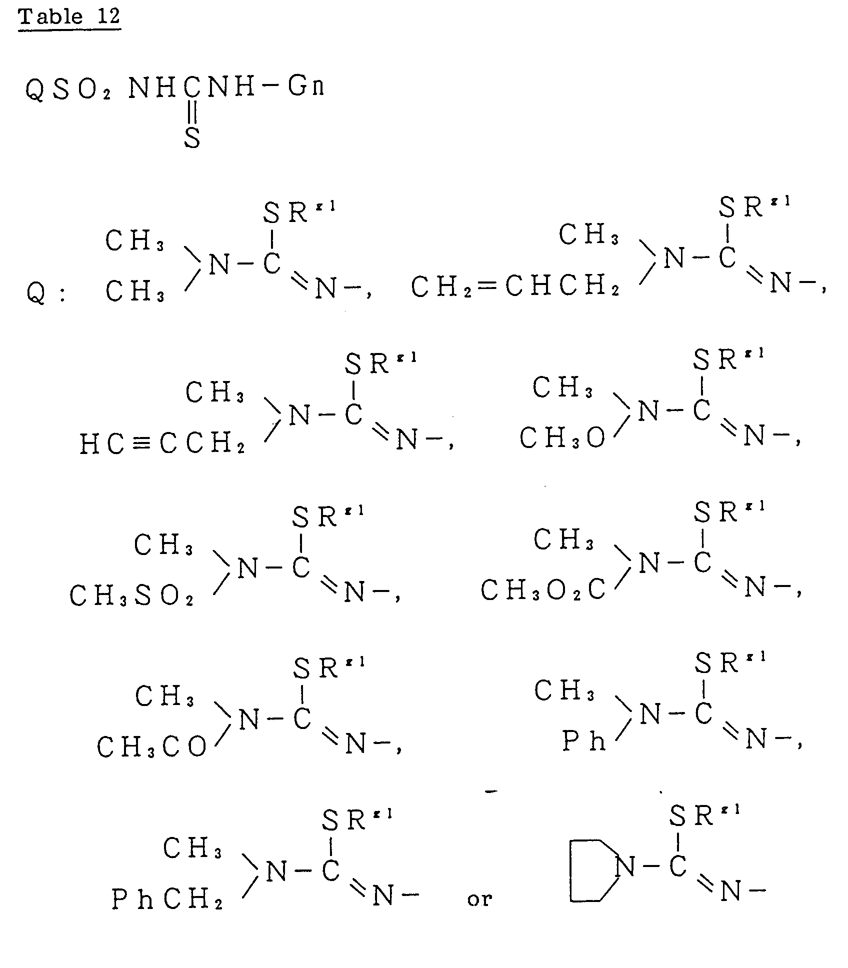

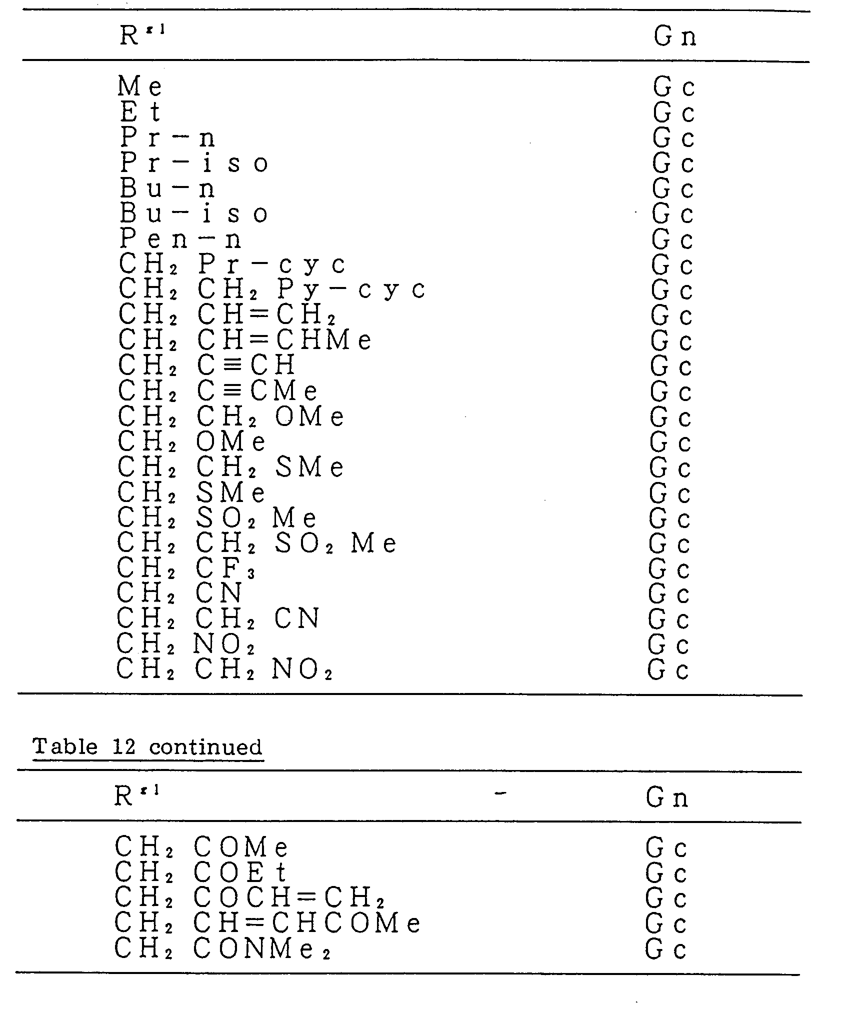

The compound of the present invention can be used as a herbicide for upland fields by any treating method such as soil treatment, soil admixing treatment or foliage treatment.

The dose of the compound of the present invention varies depending upon the application site, the season for application, the manner of application, the type of weeds to be controlled, the type of crop plants, etc. However, the dose is usually within a range of from 0.0001 to 10 kg, preferably from 0.005 to 5 kg, per hectare (ha), as the amount of the active ingredient.

Further, the compound of the present invention may be combined with other herbicides, various insecticides, fungicides, plant growth regulating agents, synergism agents and safeners at the time of the preparation of the formulations or at the time of the application, as the case requires.

Particularly, by the combined application with other herbicide, it can be expected to reduce the cost due to a decrease of the dose or to enlarge the herbicidal spectrum or obtain higher herbicidal effects due to a synergistic effect of the combined herbicides. In such a case, a plurality of known herbicides may be simultaneously combined. The herbicides of the type which can be used in combination with the compound of the present invention, may, for example, be compounds disclosed in Farm Chemicals Handbook (1990).

When the compound of the present invention is to be used as a herbicide, it is usually mixed with a suitable carrier, for instance, a solid carrier such as clay, talc, bentonite, diatomaceous earth or fine silica powder, or a liquid carrier such as water, an alcohol (such as isopropanol, butanol, benzyl alcohol or furfuryl alcohol), an aromatic hydrocarbon (such as toluene or xylene), an ether (such as anisole), a ketone (such as cyclohexanone or isophorone), an ester (such as butyl acetate), an acid amide (such as N-methylpyrrolidone), or a halogenated hydrocarbon (such as chlorobenzene). If desired, a surfactant, an emulsifier, a dispersing agent, a penetrating agent, a spreader, a thickener, an antifreezing agent, a coagulation preventing agent or a stabilizer may be added to prepare an optional formulation such as a liquid formulation, an emulsifiable concentrate, a wettable powder, a dry flowable, a flowable, a dust or a granule.

Cropland weeds to be controlled by the compound of the present invention include, for example, Solanaceae weeds such as Solanum nigrum and Datura stramonium, Malvaceae weeds such as Abutilon theophrasti and Side spinosa, Convolvulaceae weeds such as Ipomoea spps. e.g. Ipomoea purpurea, and Calystegia spps., Amaranthaceae weeds such as Amaranthus lividus and Amaranthus viridis, Compositae weeds such as Xanthium strumarium, Ambrosia artemisiaefolia, Helianthus annuu, Galinsoga ciliat, Cirsium arvense, Senecio vulgaris and Erigeron annus, Cruciferae weeds such as Rorippa indica, Sinapis arvensis and Capsella Bursapastris, Polygonaceae weeds such as Polygonum Blumei and Polygonum convolvulus, Portulacaceae weeds such as Portulaca oleracea, Chenopodiaceae weeds such as Chenopodium album, Chenopodium ficifolium and Kochia scoparis, Caryophyllaceae weeds such as Stellaria media, Scrophulariaceae weeds such as Veronica persica, Commelinaceae weeds such as Commelina communis, Labiatae weeds such as Lamium amplexicaule and Lamium purpureum, Euphorbiaceae weeds such as Euphorbia supina and Euphorbia maculata, Rubiaceae weeds such as Galium spurium, Galium aparine and Rubia akane, Violaceae weeds such as Viola arvensis, Leguminosae weeds such as Sesbania exaltata and Cassia obtusifolia, Graminaceous weeds such as Sorgham bicolor, Panicum dichotomiflorum, Sorghum halepense, Echinochloa crus-galli, Digitaria adscendens, Avena fatua, Eleusine indica, Setaria viridis and Alopecurus aegualis, and Cyperaceous weeds such as Cyperus rotundus and Cyperus esculentus.

Further, the compound of the present invention can be used as a paddy field herbicide by any treating method such as irrigated soil treatment or foliage treatment. Paddy weeds include, for example, Alismataceae weeds such as Alisma canaliculatum, Sagittaria trifolia and Sagittaria pygmaea, Cyperaceae weeds such as Cyperus difformis, Cyperus serotinus, Scirpus juncoides and Eleocharis kuroguwai, Scrothulariaceae weeds such as Lindemia pyxidaria, Potenderiaceae weeds such as Monochoria vaginalis, Potamogetonaceae weeds such as Potamogeton distinctus, Lythraceae weeds such as Rotala indica, and Gramineae weeds such as Echinochloa crus-galli.

The compound of the present invention can be applied to control various weeds not only in the agricultural and horticultural fields such as upland fields, paddy fields or orchards, but also in non-agricultural fields such as play grounds, non-used vacant fields or railway sides.

The compound of the present invention can easily be produced by selecting any one of the following reaction schemes 1 to 5. 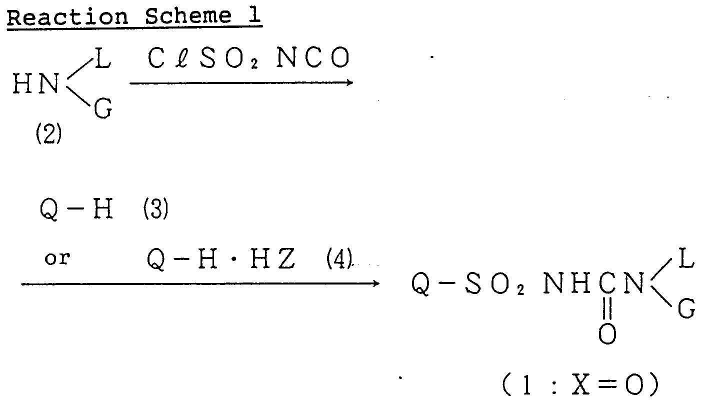 In the above formulas, Q, G and L are as defined above, and Z is a halogen atom.

Namely, the amine (2) is reacted with chlorosulfonyl isocyanate in a solvent such as tetrahydrofuran, dimethoxyethane, acetonitrile, propionitrile, dimethylformamide, dichloromethane, dichloroethane, benzene or toluene and then reacted with the imine (3) or (4) in the presence of a base such as triethylamine, pyridine, sodium hydride, sodium methoxide, sodium ethoxide, sodium hydroxide, potassium hydroxide or potassium carbonate, to obtain the compound of the present invention (1:X = O). 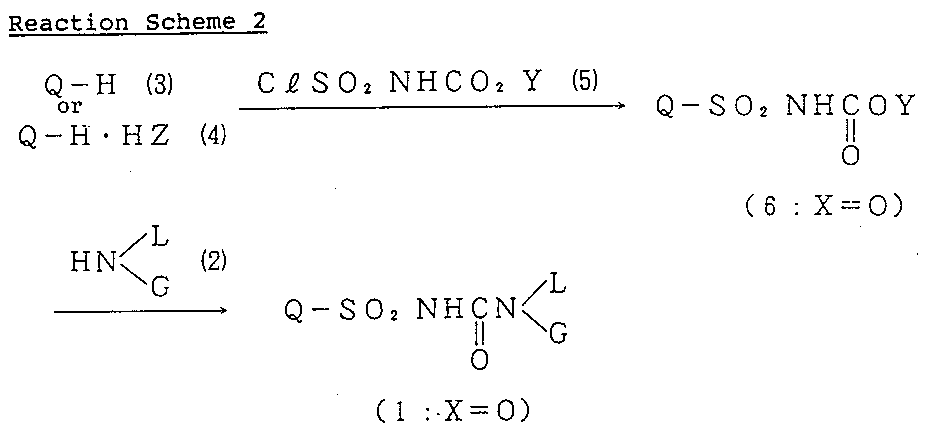 In the above formulas, Q, G, L and Z are as defined above, and Y is a C₁₋₆ alkyl group or a phenyl group.

Namely, the reaction of the imine (3) or (4) with phenyl N-chlorosulfonyl carbamate (5:Y = phenyl group) or an alkyl N-chlorosulfonyl carbamate (5:Y = lower alkyl group), is conducted by using the carbamate derivative (5) in an amount of from 0.5 to 3.0 mols, per mol of the imine (3) or (4). Preferably, the amount is within a range of from 0.9 to 1.2 mols.

The reaction temperature may be selected optionally within a range of from -50°C to 100°C, but it is preferably within a range of from -20°C to 30°C.

This reaction can be carried out by using various bases. The amount of the base is from 0.5 to 4.0 mols per mol of the imine (3) or (4).

A suitable base may, for example, be an organic base such as triethylamine or pyridine, a metal hydride such as sodium hydride, an inorganic base such as sodium hydroxide, potassium hydroxide or potassium carbonate, or a metal alkoxide such as sodium methoxide or sodium ethoxide.

A suitable solvent for this reaction is a solvent inert to this reaction, for example, an aromatic hydrocarbon such as benzene, toluene or xylene, a halogenated hydrocarbon such as dichloromethane, chloroform or carbon tetrachloride, an ether such as ethyl ether, isopropyl ether, dioxane or tetrahydrofuran, a nitrile such as acetonitrile or propionitrile, a hydrocarbon such as petroleum ether, petroleum benzine or n-hexane, a ketone such as acetone or methyl ethyl ketone, an ester such as ethyl acetate, or an amide such as dimethylformamide, dimethylacetamide or hexamethyl phosphorous triamide.

These solvents may be used alone or in combination as a mixture.

Particularly preferred is an ether or an amide.

Then, the phenyl N-substituted iminosulfonyl carbamate (6:X = O, Y = phenyl group) or the alkyl N-substituted iminosulfonyl carbamate (6:X = O, Y = lower alkyl group) and the compound (2) are heated in a solvent such as benzene, toluene or dioxane to obtain the compound of the present invention (1:X = O). 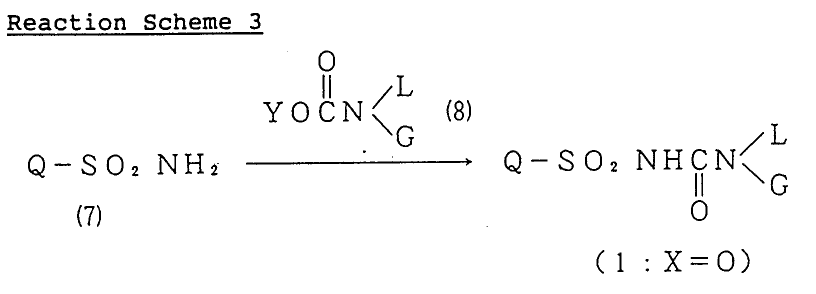 In the above formulas, Q, G, L and Y are as defined above.

Namely, the substituted iminosulfonamide derivative (7) is reacted with the carbamate derivative (8) in a solvent such as acetone, acetonitrile or dioxane in the presence of an inorganic base such as potassium carbonate or an organic base such as triethylamine or 1,8-diazabicyclo[5.4.0]-7-undecene (DBU) to obtain the compound of the present invention (1:X = O). 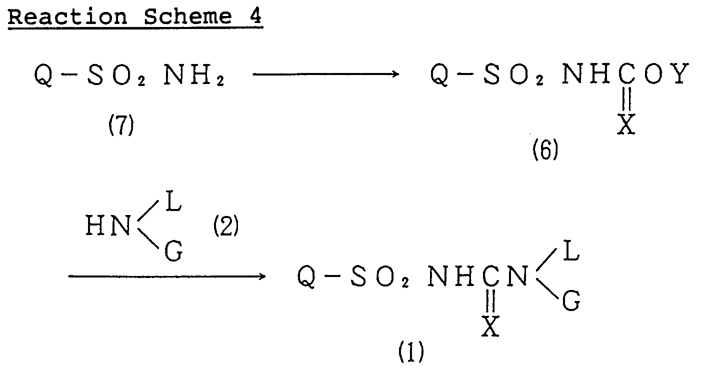 In the above formulas, Q, G, L, X and Y are as defined above.

Namely, the substituted iminosulfonamide derivative (7) is reacted with chloroformic acid (thio)ester or carbonic acid (thio)ester in a solvent such as acetone, methyl ethyl ketone, acetonitrile, dioxane or tetrahydrofuran in the presence of a base such as potassium carbonate, triethylamine or pyridine to obtain the compound (6), which is then heated together with the compound (2) in a solvent such as toluene, benzene or dioxane to obtain the compound of the present invention (1). 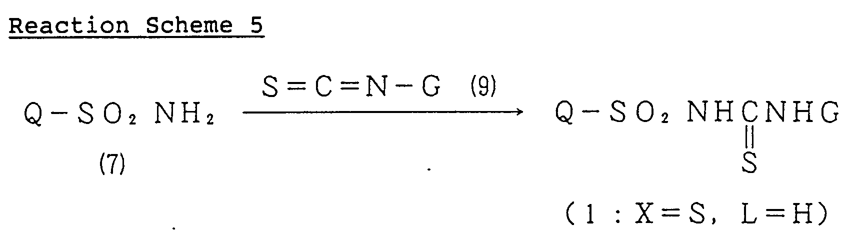 In the above formulas, Q and G are as defined above.

Namely, the substituted iminosulfonamide derivative (7) is reacted with the isothiocyanate derivative (9) in a solvent such as acetone, acetonitrile, dioxane or tetrahydrofuran in the presence of an inorganic base such as potassium carbonate or an organic base such as triethylamine or DBU, to obtain the compound of the present invention (1:X = S, L = H).

The intermediates to be used in the present invention, i.e. the substituted iminosulfonamide derivative (7), the phenyl N-substituted iminosulfonyl(thio)carbamate (6:Y = phenyl group) and the alkyl N-substituted iminosulfonyl(thio)carbamate (6: Y = C₁₋₆ alkyl group) are also novel compounds.

The substituted iminosulfonamide derivative (7) can be synthesized from an imine (3) or (4) by the methods of the following Reaction Schemes 6 and 7. 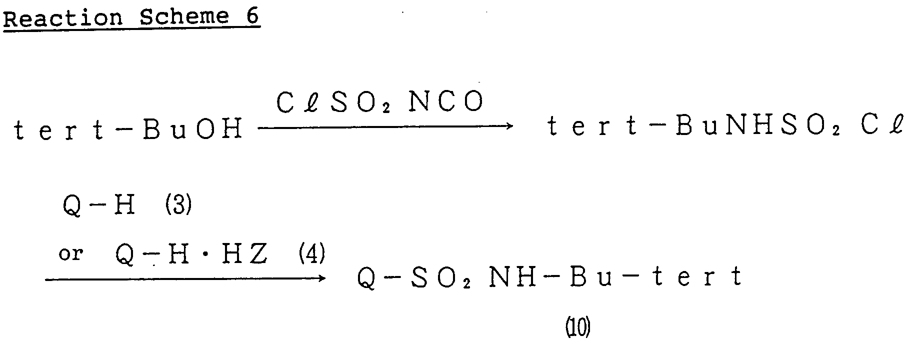 In the above formulas, Q and Z are as defined above.

In the Reaction Scheme 6, the reaction of tert-butanol with chlorosulfonyl isocyanate can be conducted by a method per se known, for example, in accordance with Japanese unexamined Patent Publication No. 101323/1975.

The reaction of the imine (3) or (4) with tert-butylsulfamoyl chloride is carried out by using tert-butylsulfamoyl chloride in an amount of from 0.5 to 3.0 mols per mol of the imine (3) or (4). Preferably the amount is within a range of from 0.9 to 1.2 mols.

The reaction temperature may be selected optionally within a range of from -50°C to 100°C. However, the temperature is preferably within a range of from -20°C to 30°C.

This reaction can be conducted by using various bases. The amount of the base is from 0.5 to 4.0 mols per mol of the imine (3) or (4). Preferably, the amount is within a range of from 0.8 to 2.5 mols. A suitable base may, for example, be a metal hydride such as sodium hydride, an organic base such as triethylamine or pyridine, an inorganic base such as sodium hydroxide, potassium hydroxide or potassium carbonate, or a metal alkoxide such as sodium methoxide or sodium ethoxide.

A suitable solvent for the reaction is a solvent inert to this reaction, for example, an aromatic hydrocarbon such as benzene, toluene or xylene, a halogenated hydrocarbon such as dichloromethane, chloroform or carbon tetrachloride, an ether such as ethyl ether, isopropyl ether, dioxane or tetrahydrofuran, a nitrile such as acetonitrile or propionitrile, a hydrocarbon such as petroleum ether, petroleum benzine or n-hexane, a ketone such as acetone or methyl ethyl ketone, an ester such as ethyl acetate, or an amide such as dimethylformamide, dimethylacetamide or hexamethylphosphorous triamide.

These solvents may be used alone or in combination as a mixture. Particularly preferred is an ether or an amide. 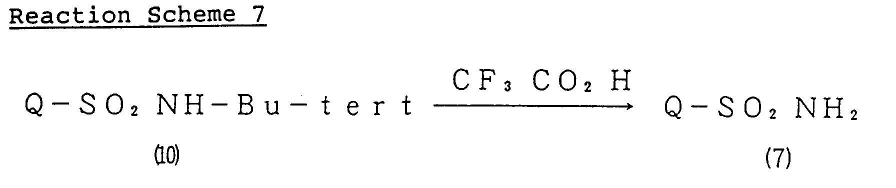 In the above formulas, Q is as defined above.

In the Reaction Scheme 7, removal of the tert-butyl group is carried out by using trifluoroacetic acid.

The amount of trifluoroacetic acid may be selected optionally within a range of an equimolar amount to an excess amount. Trifluoroacetic acid may be used as a solvent without any particular problem.

The reaction temperature may be selected optionally within a range of from -50°C to 80°C. The temperature is preferably within a range of from -20°C to 30°C.

When a solvent is used for this reaction, a solvent inert to this reaction, for example, an aromatic hydrocarbon such as benzene, toluene or xylene, a halogenated hydrocarbon such as dichloromethane, chloroform or carbon tetrachloride, an ether such as ethyl ether, isopropyl ether, dioxane or tetrahydrofuran, a nitrile such as acetonitrile or propionitrile, a hydrocarbon such as petroleum ether, petroleum benzine or n-hexane, a ketone such as acetone or methyl ethyl ketone, an ester such as ethyl acetate, or an amide such as dimethylformamide, dimethylacetamide or hexamethylphosphorous triamide, may be used. These solvents may be used alone or in combination as a mixture.

In Reaction Scheme 2, the phenyl N-chlorosulfonyl carbamate (5:Y = phenyl group) and the alkyl N-chlorosulfonyl carbamate (5:Y = lower alkyl group) can be synthesized by a method known per se, for example, in accordance with Chemische Berichte, vol. 96, p. 56 (1963).

The imines (3) and (4) to be used as the starting materials for the above reaction, can be synthesized, for example, in accordance with U.S. Patent 4,237,302, Journal of Chemical Society, p. 307 (1956), Chemical and Pharmaceutical Bulletin, vol. 26, p. 3658 (1978), Journal of Organic Chemistry, vol. 30, p. 4298 (1965), East German Patent 291,757, Journal of American Chemical Society, vol. 93, p. 5552 (1971), U.S. Patent 4,054,652, British Patent 752,003, Chemische Berichte, vol. 92, p. 1928 (1959), Journal of Medicinal Chemistry, vol. 6, p. 266 (1963), Chemical Abstracts, vol. 64, 14171e (1966), and Belgian Patent 654,416.

As representative examples, synthetic schemes for 2-imino-3-ethoxythiazolidine hydrobromide, 2-imino-3-n-propoxythiazolidine hydrobromide and 2-imino-3-methoxy-4,5-dimethylthiazoline hydrochloride will be shown as Reaction Schemes 8, 9 and 10.

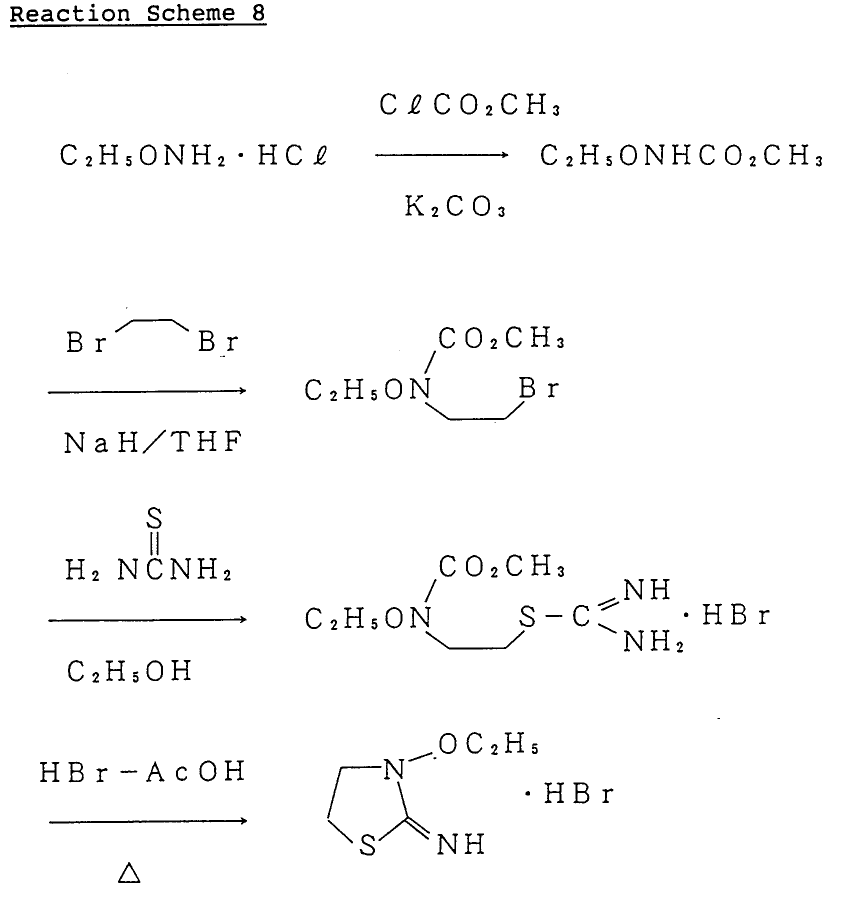

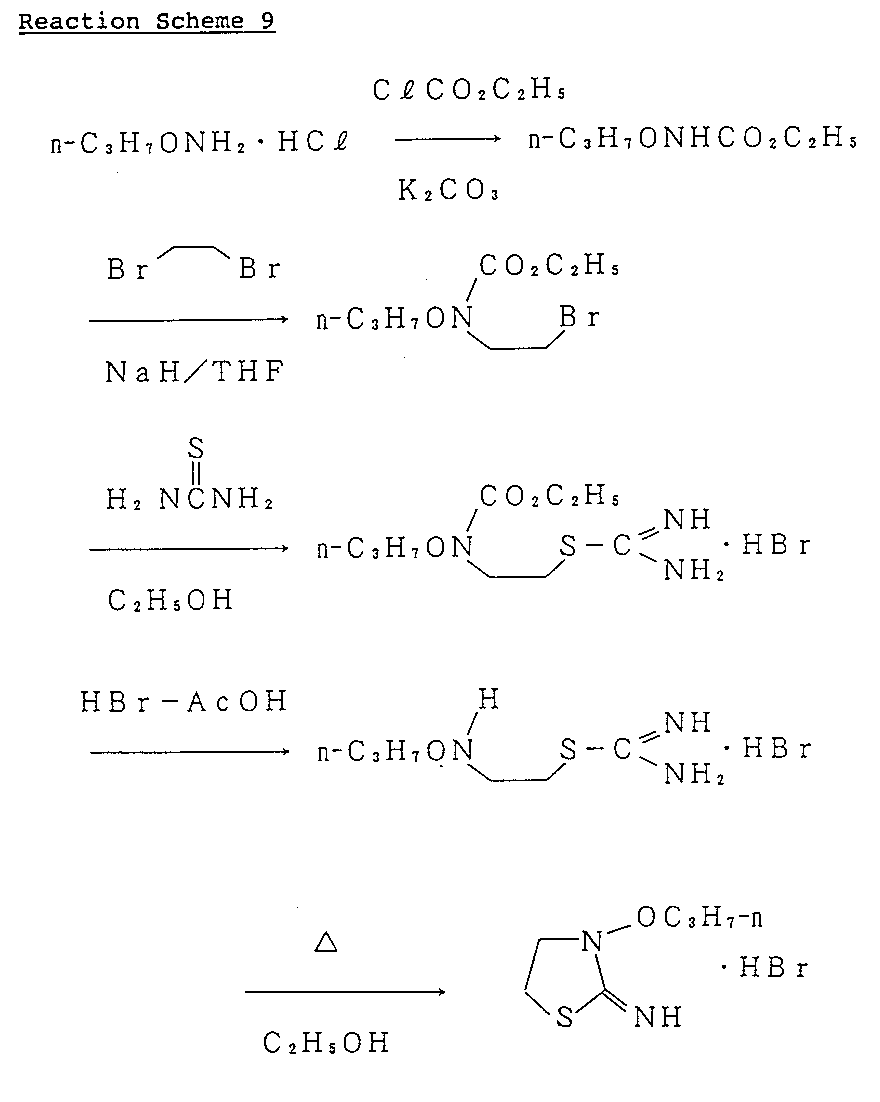

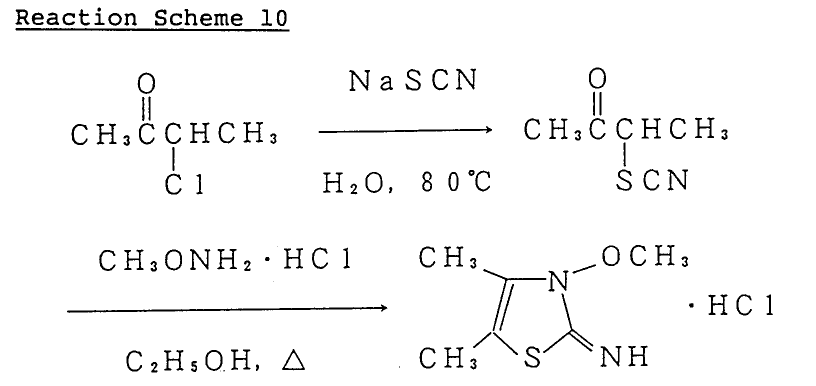

### THE BEST MODE FOR CARRYING OUT THE INVENTION

Now, syntheses of the compounds of the present invention will be described in detail as Reference Examples and working Examples. However, the present invention is by no means restricted to such specific Examples.

### REFERENCE EXAMPLE a-1

### Preparation of 2-imino-3-methylthiazol-4-ine hydroiodide

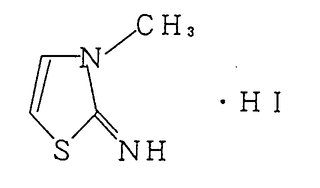

In 125 ml of dimethylformamide, 50 g (0.5 mol) of 2-aminothiazole was dissolved, and 90 g (0.63 mol) of methyl iodide was added thereto at room temperature. The mixture was further stirred at room temperature for 48 hours. Then, 1000 ml of ethyl acetate was added to the reaction mixture. Formed crystals were collected by filtration, washed with ethyl acetate and then dried to obtain 105 g of 2-imino-3-methylthiazol-4-ine hydroiodide.
Melting point: 181 - 183°C

The 2-imino-3-methylthiazol-4-ine hydroiodide was neutralized with potassium carbonate to obtain 2-imino-3-methylthiazol-4-ine.
Boiling point 55 - 60°C/1mmHg

### REFERENCE EXAMPLE a-2

### Preparation of 2-imino-3-n-butylthiazolidine

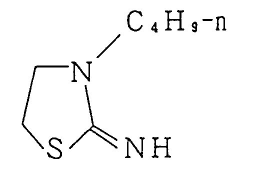

In 25 ml of dimethylformamide, 8.2 g (80 mmol) of 2-amino-2-thiazolidine was dissolved, and 18.4 g (100 mmol) of n-butyl iodide was added thereto at room temperature. The reaction mixture was heated and stirred at 60°C for 10 hours and then left to cool to room temperature. The reaction mixture was added to 300 ml of ethyl acetate, and the mixture was stirred at room temperature for 10 minutes. A formed oily substance was separated by decantation from the ethyl acetate solution, and the same operation was repeated twice. Then, ethyl acetate contained in the oily substance was distilled off under reduced pressure to obtain 2-imino-3-n-butylthiazolidine hydroiodide as a crude product.

Then, the 2-imino-3-n-butylthiazolidine hydroiodide was stirred together with 5.28 g (80 mmol) of 85% potassium hydroxide in 300 ml of methanol at room temperature for one hour. Methanol was distilled off under reduced pressure. Then, 200 ml of chloroform was added to the residue, and precipitated insoluble matters were removed by filtration. Chloroform was distilled off under reduced pressure, and 6.9 g of 2-imino-3-n-butylthiazolidine was obtained by distillation under reduced pressure.
Boiling point: 85 - 89°C/0.26 torr

### REFERENCE EXAMPLE a-3

### Preparation of 2-imino-1,3-dimethylimidazol-4-ine hydrochloride

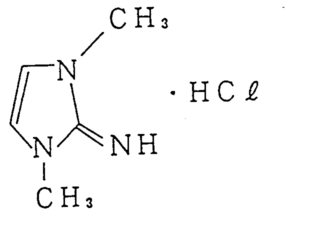

16.9 g (150 mmol) of creatinine was dissolved in 100 ml of N,N-dimethylformamide. Then, 27.6 g (194 mmol) of methyl iodide was added thereto, and the mixture was heated to 50°C and stirred at that temperature for 2 hours and further at room temperature overnight. To the reaction mixture, 500 ml of ethyl acetate was added, and crystals were collected by filtration. The obtained crystals were washed with ethyl acetate and dried to obtain 26.2 g of 2-imino-1,3-dimethylimidazolidin-4-one hydroiodide as white crystals.

Then, to 150 ml of a methanol solution containing 5.17 g (78.4 mmol) of 85% potassium hydroxide, 20 g (78.4 mmol) of 2-imino-1,3-dimethylimidazolidin-4-one hydroiodide was added, and the mixture was stirred at room temperature for 20 minutes. The solvent was distilled off under reduced pressure. Then, to the residue, 200 ml of chloroform was added, and insoluble matters were filtered off. The filtrate was dried over anhydrous magnesium sulfate, and the solvent was distilled off under reduced pressure to obtain 9.0 g of 2-imino-1,3-dimethylimidazolidin-4-one.

800 mg (21.0 mmol) of lithium aluminum hydride was suspended in 20 ml of dry tetrahydrofuran, and 200 ml of a dry tetrahydrofuran solution containing 1.5 g (11.8 mmol) of 2-imino-1,3-dimethylimidazolidin-4-one, was added thereto at room temperature. The mixture was stirred at the same temperature overnight. Then, to the reaction mixture, 10 ml of ethyl acetate and then 5 ml of water were carefully added, and insoluble matters were filtered off. The filtrate was adjusted to pH 3 with concentrated hydrochloric acid, and then the solvent was distilled off under reduced pressure. Obtained crystals were washed with a solvent mixture of ethyl ether and ethanol to obtain 1.2 g of 2-imino-1,3-dimethylimidazol-4-ine hydrochloride.
Melting point: 168 - 171°C

The structures and the physical property values or characteristics of the compounds prepared by the same methods as the above Reference Examples a-1 to a-3 are presented in Tables 13a-1, 13a-2 and 13a-3.

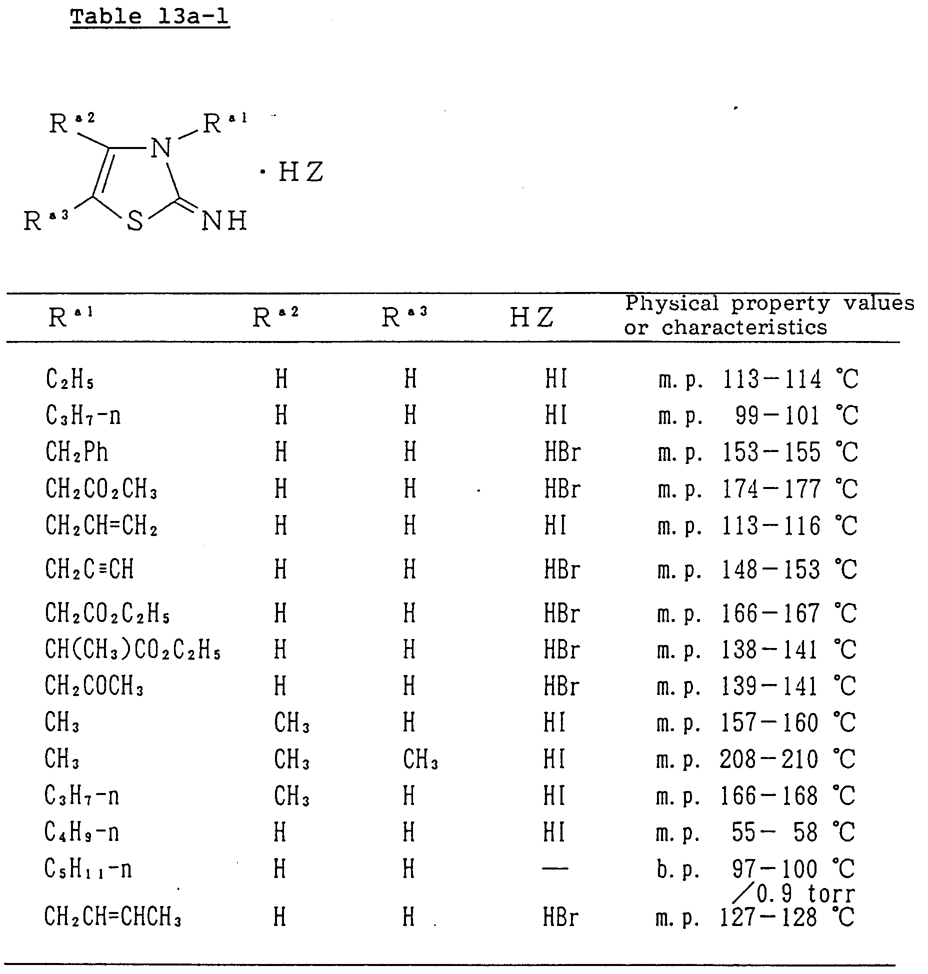

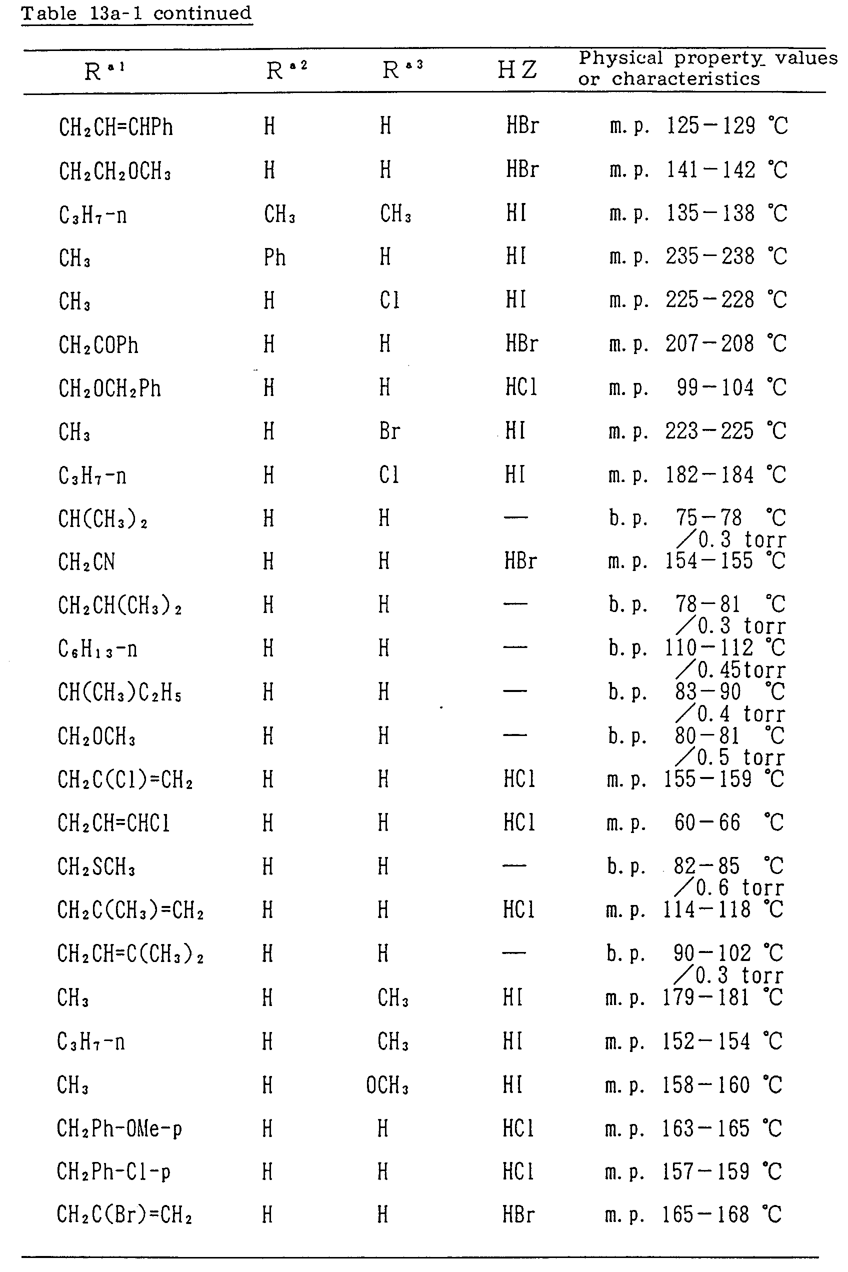

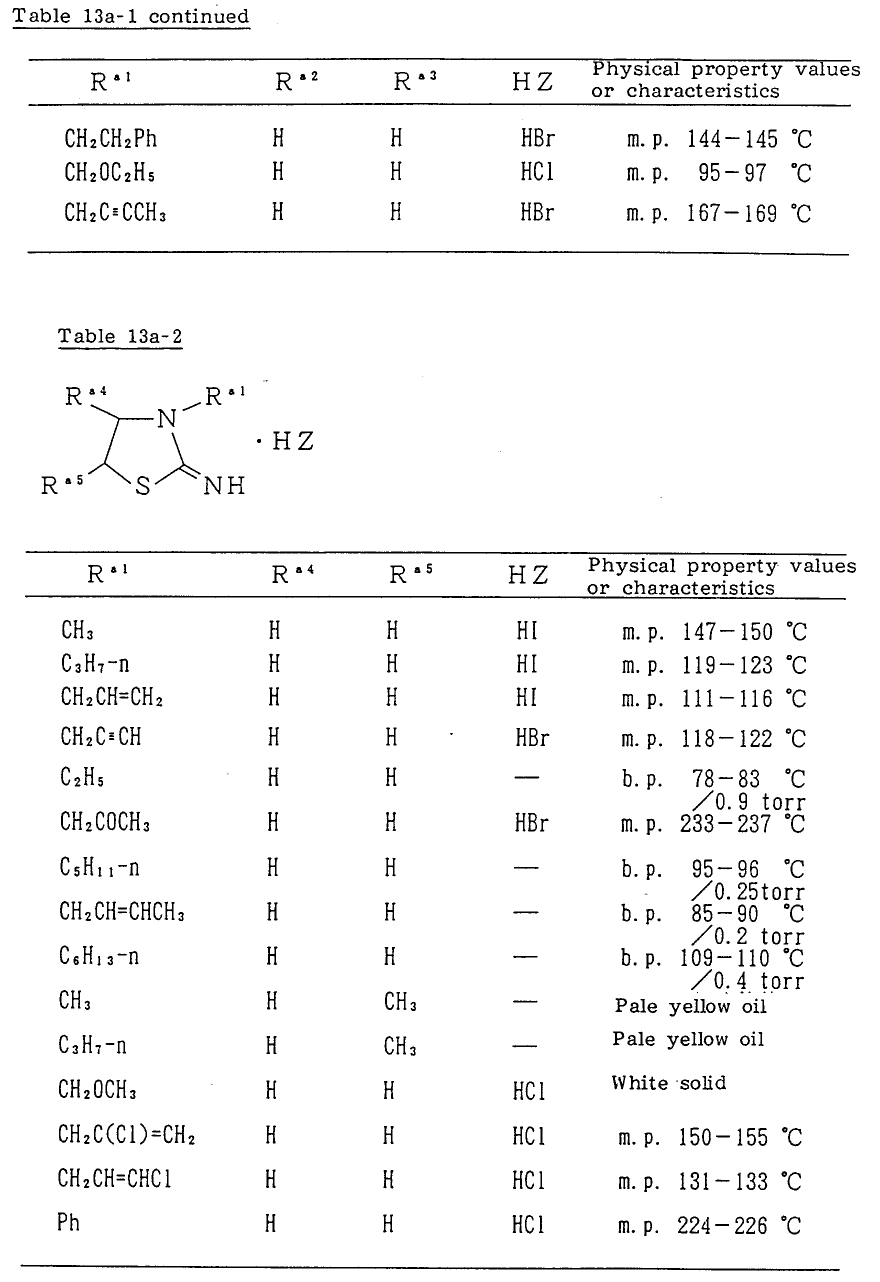

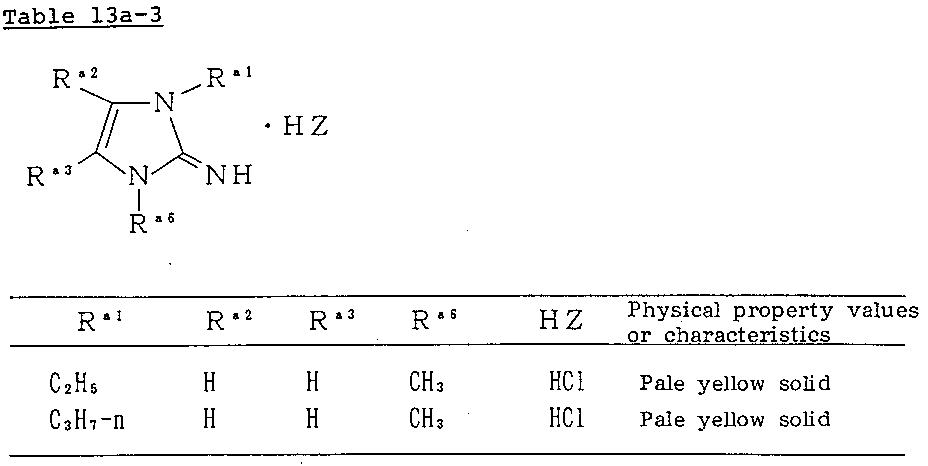

### REFERENCE EXAMPLE b-1

### Preparation of 2-imino-3-n-propylthiadiazol-4-ine hydroiodide

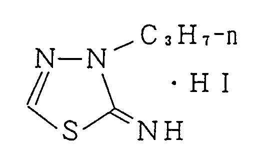

In 40 ml of dimethylformamide, 8.1 g (80 mmol) of 2-aminothiadiazole was dissolved, and 17.0 g (100 mmol) of n-propyl iodide was added thereto at room temperature. The mixture was heated at 60°C for 30 minutes and then left to cool, and then it was stirred at room temperature for 24 hours. Then, to the reaction mixture, 500 ml of ethyl acetate was added. Formed crystals were collected by filtration, washed with ethyl acetate and then dried to obtain 13.1 g of desired 2-imino-3-n-propylthiadiazol-4-ine hydroiodide.
Melting point: 121 - 124°C

The structures and the physical property values of the compounds prepared by the same method as in the above Reference Example b-1 are presented in Table 13b.

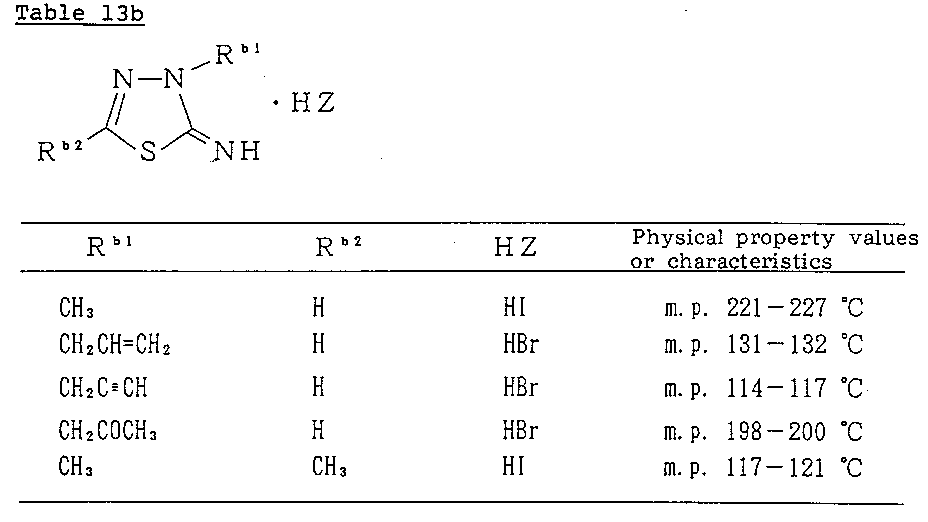

### REFERENCE EXAMPLE c-1

### Preparation of 2-imino-3-n-propoxythiazolidine hydrobromide

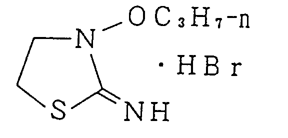

22 g (197 mmol) of n-propoxyamine hydrochloride was dissolved in 100 ml of water, and 200 ml of ethylene dichloride was added thereto. Then, 27.2 g (197 mmol) of potassium carbonate was added in several times under cooling, and then 21.3 g (196 mmol) of ethyl chloroformate was dropwise added thereto at a temperature of not higher than 10°C. After raising the temperature to room temperature, the mixture was further stirred at the same temperature for 4 hours. The ethylene dichloride layer was separated, and then the aqueous layer was extracted twice with 100 ml of chloroform. The ethylene dichloride layer and the chloroform layer were put together, and washed with a saturated sodium chloride aqueous solution, and then dried over anhydrous sodium sulfate. Then, the solvent was distilled off under reduced pressure. 27 g of ethyl N-n-propoxycarbamate was obtained by distillation under reduced pressure.
Boiling point: 88°C/2.5 mmHg

8.73 g (218 mmol) of 60% sodium hydride was suspended in 200 ml of dry tetrahydrofuran, and 50 ml of a dry tetrahydrofuran solution containing 26.7 g (182 mmol) of ethyl N-n-propoxycarbamate, was dropwise added thereto under cooling with ice at a temperature of not higher than 10°C. After raising the temperature to room temperature, the mixture was stirred at the same temperature for 20 minutes and again cooled with ice. Then, 121.4 g (646 mmol) of 1,2-dibromoethane was added all at once. The temperature was gradually raised and then the mixture was refluxed under heating for 2 hours. the mixture was left to cool to room temperature, and the solvent was partially distilled off under reduced pressure. The residue was poured into 100 ml of ice water and extracted three times with 100 ml of chloroform. The chloroform layer was washed with a saturated sodium chloride aqueous solution and then dried over anhydrous sodium sulfate. The solvent was distilled off under reduced pressure, and then 42.0 g of ethyl N-(2-bromoethyl)-N-n-propoxycarbamate was obtained by distillation under reduced pressure.
Boiling point: 97°C/0.4 mmHg

A mixture comprising 41.7 g (164 mmol) of ethyl N-(2-bromoethyl)-N-n-propoxycarbamate, 16.2 g (213 mmol) of thiourea and 200 ml of ethanol, was refluxed under heating for 5 hours. The mixture was left to cool, and then the solvent was distilled off under reduced pressure. Then, 300 ml of chloroform was added to the residue, and the mixture was stirred at room temperature for 10 minutes. After removing insoluble matters by filtration, chloroform was distilled off under reduced pressure. To the residue, ethyl ether and a small amount of water were added for crystallization. Then, the crystals were collected by filtration to obtain 50 g of S-[2-(N-ethoxycarbonyl-N-n-propoxy)aminoethyl]-isothiuronium hydrobromide.
Melting point: 74 - 76°C

5.0 g (15.2 mmol) of S-[2-(N-ethoxycarbonyl-N-n-propoxy)aminoethyl]isothiuronium hydrobromide and 0.27 g (15.0 mmol) of water were added to 30 ml of a 30% hydrogen bromide/acetic acid solution, and the mixture was heated and stirred at 55°C for 4 hours. The mixture was left to cool, and acetic acid was distilled off under reduced pressure. To the residue, ethyl ether and a small amount of ethanol were added for crystallization. The crystals were collected by filtration to obtain 3.8 g of S-[2-(N-n-propoxy)aminoethyl]isothiuronium hydrobromide.
Melting point: 112 - 113°C

3.8 g (14.7 mmol) of S-[2-(N-n-propoxy)aminoethyl]-isothiuronium hydrobromide was added to 60 ml of ethanol, and the mixture was refluxed under heating for 3 hours. The mixture was left to cool, and then ethanol was distilled off under reduced pressure. To the residue, ethyl ether and a small amount of ethanol were added for crystallization. The crystals were collected by filtration to obtain 3.2 g of 2-imino-3-n-propoxythiazolidine hydrobromide.
Melting point: 117 - 119°C

### REFERENCE EXAMPLE c-2

### Preparation of 2-imino-3-methoxy-4-methylthiazol-4-ine hydrochloride

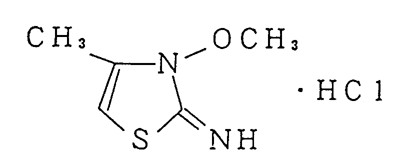

5.67 g (70 mmol) of sodium thiocyanate was dissolved in 12 ml of water, and the solution was heated to 80°C. 5.55 g (60 mmol) of chloroacetone was dropwise added thereto over a period of one hour, and the mixture was stirred at the same temperature for 3 hours. The mixture was cooled to room temperature, and then 60 ml of ethyl ether was added thereto. The aqueous layer was separated and removed. The ethyl ether layer was washed twice with 10 ml of water, and then the solvent was distilled off under reduced pressure to obtain 6.0 g of thiocyanoacetone.

2.30 g (20 mmol) of thiocyanoacetone and 1.67 g (20 mmol) of methoxyamine hydrochloride were dissolved in 10 ml of ethanol, and the solution was refluxed under heating for 5 hours. Ethanol was distilled off under reduced pressure. Then, to the obtained residue, 50 ml of ethyl acetate was added. Precipitated crystals were collected by filtration to obtain 3.34 g of 2-imino-3-methoxy-4-methylthiazol-4-ine hydrochloride.
Melting point: 145 - 155°C (decomposed)

The structures and the physical property values of the compounds prepared by the same methods as in Reference Examples c-1 and c-2 are presented in Tables 13c-1, 13c-2 and 13c-3.

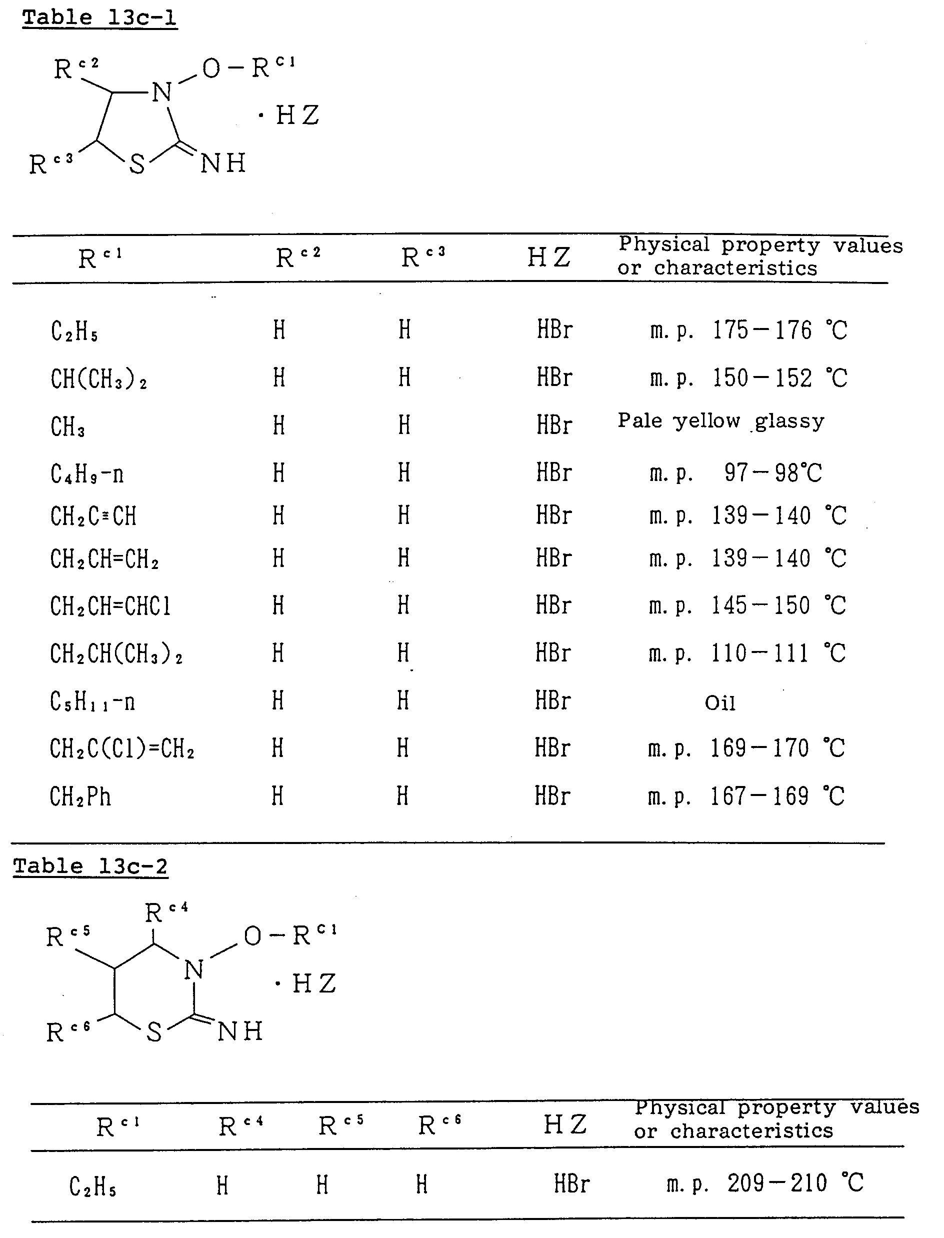

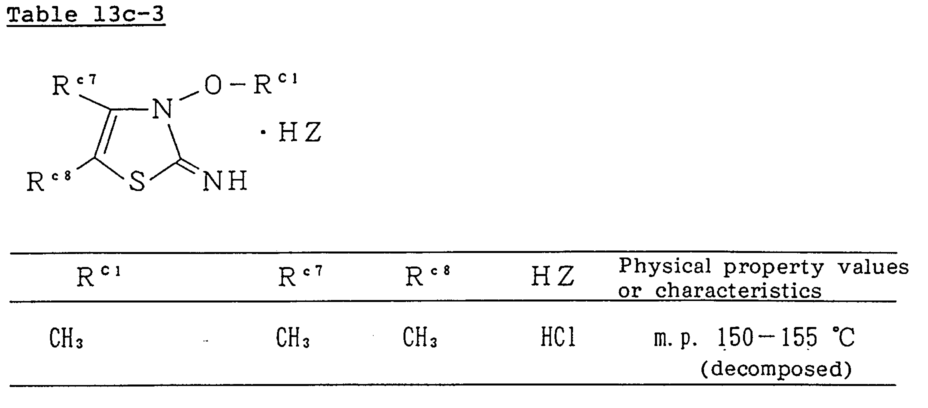

### REFERENCE EXAMPLE d-1

### Preparation of 3-methyl-2-iminothiazolidin-4-one hydroiodide

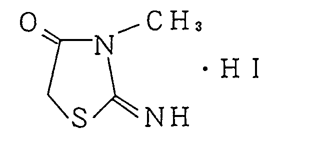

11.6 g (0.1 mol) of pseudothiohydantoin was suspended in 150 ml of dimethylformamide, and 17 g (0.12 mol) of methyl iodide was added thereto. Then, the mixture was stirred at 60°C for one hour. After being left to cool, the reaction mixture was poured into 1000 ml of ethyl acetate, and precipitated crystals were collected by filtration to obtain 15 g of 3-methyl-2-iminothiazolidin-4-one hydroiodide as pale yellow crystals. Melting point: 237 - 238°C

### REFERENCE EXAMPLE d-2

### Preparation of 1-methyl-3-n-propyl-2-iminoimidazolidin-4-one hydroiodide

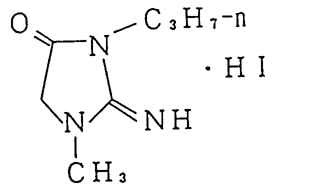

9.04 g (80 mmol) of creatinine was suspended in 50 ml of dimethylformamide, and 17.0 g (100 mmol) of n-propyl iodide was added thereto. Then, the mixture was heated and stirred within a range of from 70°C to 80°C until creatinine was completely dissolved. The mixture was left to cool, and 500 ml of ethyl acetate was added thereto. Precipitated crystals were collected by filtration to obtain 10.6 g of 1-methyl-3-n-propyl-2-iminoimidazolidin-4-one hydroiodide.
Melting point: 159 - 161°C

The 1-methyl-3-n-propyl-2-iminoimidazolidin-4-one hydroiodide was neutralized in accordance with the following method to obtain 1-methyl-3-n-propyl-2-iminoimidazolidin-4-one.

2.83 g (10 mmol) of the 1-methyl-3-n-propyl-2-iminoimidazolidin-4-one hydroiodide was added to 25 ml of methanol containing 0.66 g (10 mmol) of 85% potassium hydroxide, and the mixture was stirred at room temperature for one hour. Methanol was distilled off under reduced pressure, and chloroform was added to the residue. Precipitated crystals were removed by filtration. Chloroform was distilled off under reduced pressure to obtain 1.16 g of 1-methyl-3-n-propyl-2-iminoimidazolidin-4-one as an oily substance.

The structures and the physical property values of the compounds prepared by the same methods as in Reference Examples d-1 and d-2, are presented in Table 13d.

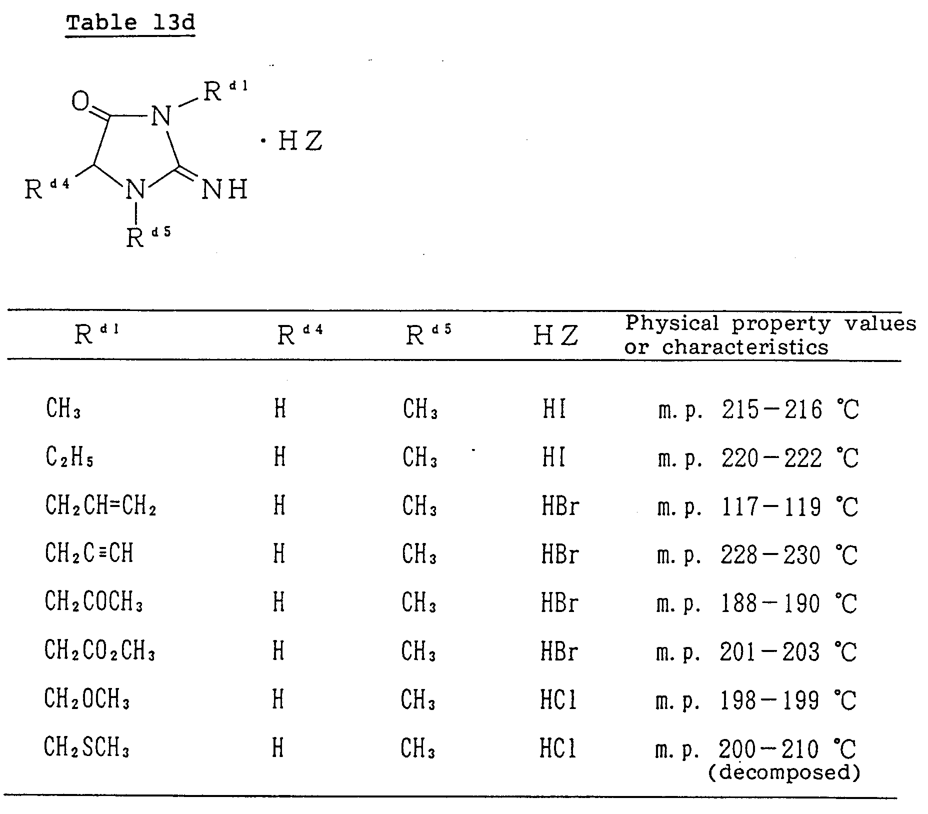

### REFERENCE EXAMPLE e-1

### Preparation of 3,6-dihydro-3-n-propyl-2H-1,3-thiazin-2-imine

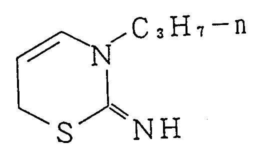

1.3 g (11.4 mmol) of 2-amino-6H-1,3-thiazine was dissolved in 4 ml of dimethylformamide, and 2.4 g (14.1 mmol) of n-propyl iodide was added thereto. The mixture was heated at 50°C for one hour and then stirred at room temperature overnight. To the reaction solution, 100 ml of ethyl acetate was added, and the mixture was stirred and then left to stand still. Then, the ethyl acetate layer was separated and removed by decantation. Then, the residual oily substance was dissolved in 50 ml of methanol, and 30 ml of a methanol solution containing 0.75 g (11.4 mmol) of 85% potassium hydroxide, was added thereto at room temperature. The mixture was further stirred at the same temperature for one hour, and then methanol was distilled off under reduced pressure. To the residue, 60 ml of chloroform was added, and insoluble matters were removed by filtration. Then, the filtrate was concentrated under reduced pressure. The residual oily substance was purified by alumina column chromatography (eluent: chloroform) to obtain 0.4 g of 3,6-dihydro-3-n-propyl-2H-1,3-thiazin-2-imine as an oily substance.

### REFERENCE EXAMPLE 2-e

### Preparation of 3,4,5,6-tetrahydro-3-methyl-2H-1,3-thiazin-2-imine

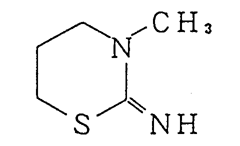

3.13 g (27 mmol) of 2-amino-4,5-dihydro-6H-1,3-thiazine was dissolved in 20 ml of isopropyl alcohol, and 4.26 g (30 mmol) of methyl iodide was added thereto. The mixture was refluxed under heating for one hour, and then left to cool. The solvent was distilled off under reduced pressure. Then, the residual oily substance was dissolved in 200 ml of methanol, and a 70 ml of a methanol solution containing 1.68 g (25.5 mmol) of 85% potassium hydroxide, was added thereto at room temperature. The mixture was stirred at the same temperature for 5 minutes, and then methanol was distilled off under reduced pressure. To the residue, 300 ml of chloroform was added and then dried over anhydrous sodium sulfate. Inorganic substances were removed by filtration, and then chloroform was distilled off under reduced pressure to obtain 3 g of 3,4,5,6-tetrahydro-3-methyl-2H-1,3-thiazin-2-imine as a pale red oily substance.

The structures and the characteristics of the compounds prepared by the same methods as in Reference Examples e-1 and e-2, are presented in Tables 13e-1 and 13e-2.

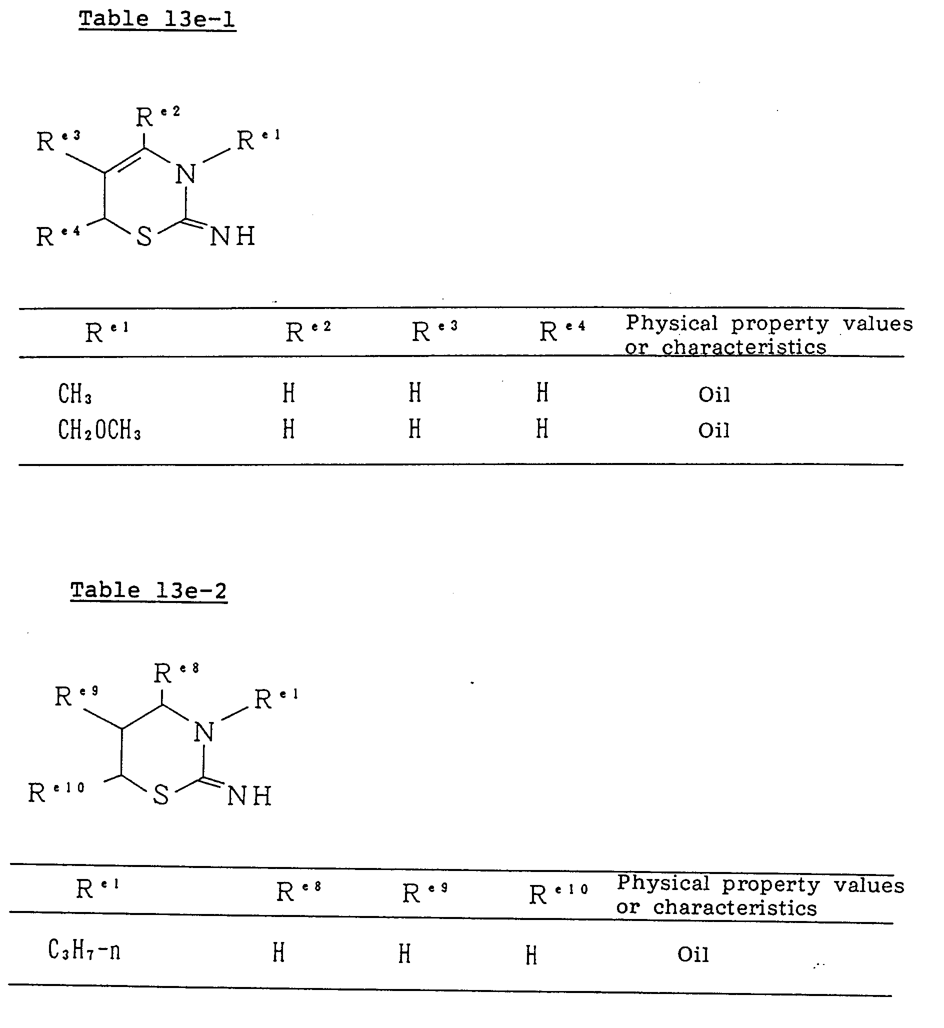

### REFERENCE EXAMPLE f-1

### Preparation of 2-imino-3-methyloxazolidine hydroiodide

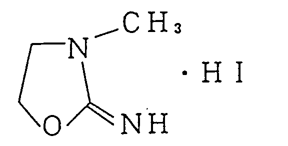

15 g (122 mmol) of 2-amino-2-oxazoline hydrochloride was stirred with 8.4 g (128 mmol) of 85% potassium hydroxide in 400 ml of methanol at room temperature for one hour. Methanol was distilled off under reduced pressure, and then 500 ml of chloroform was added. Precipitated insoluble substances were removed by filtration. Chloroform was distilled off under reduced pressure to obtain 10.5 g of 2-amino-2-oxazoline.

Then, 10.5 g of 2-amino-2-oxazoline was dissolved in 40 ml of dimethylformamide, and 22 g (155 mmol) of methyl iodide was added thereto at room temperature. The mixture was further stirred at room temperature for 48 hours. Then, 1000 ml of ethyl acetate was added to the reaction mixture. Formed crystals were collected by filtration, washed with ethyl acetate and then dried to obtain 23 g of 2-imino-3-methyloxazolidine hydroiodide.
Melting point: 165 - 169°C

The structures and characteristics of the compounds prepared by the same method as in Reference Example f-1 are presented in Table 13f.

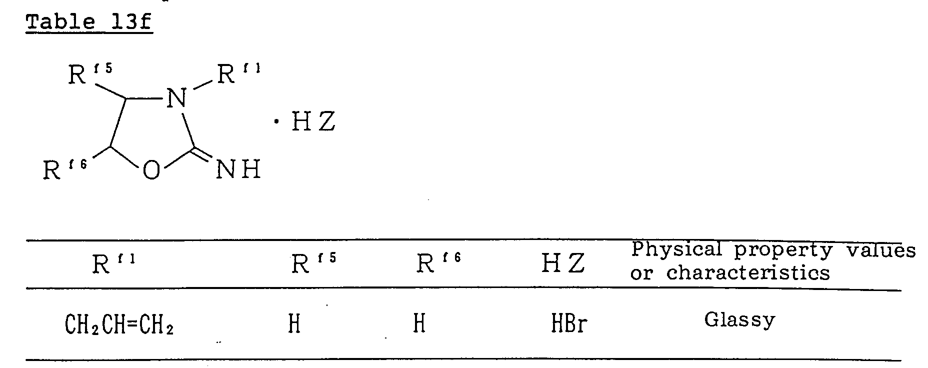

### REFERENCE EXAMPLE g-1

### Preparation of N,N-dimethyl-S-methylisothiourea hydroiodide

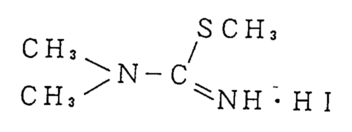

35 g (0.5 mol) of N,N-dimethylcyanamide was dissolved in a mixed solution of 70 ml of pyridine and 70 ml of triethylamine, and the solution was heated to 60°C. Hydrogen sulfide gas was introduced thereinto for 30 minutes. Then, the reaction mixture was left to cool to room temperature, and 300 ml of ethyl ether was added thereto. Precipitated crystals were collected by filtration and then washed with ethyl ether to obtain 48 g of N,N-dimethylthiourea as pale brown crystals.
Melting point: 163 - 164°C.

10.4 g (0.1 mol) of N,N-dimethylthiourea was suspended in 80 ml of ethanol, and 17 g (0.12 mol) of methyl iodide was added thereto. The mixture was refluxed under heating for 30 minutes. The reaction mixture was left to cool to room temperature, and then the solvent was distilled off under reduced pressure. The obtained crystals were washed with ethyl ether, collected by filtration and dried to obtain 20 g of N,N-dimethyl-S-methylisothiourea hydroiodide as yellow crystals.
Melting point: 84 - 85°C

### REFERENCE EXAMPLE g-2

### Preparation of N-ethyl-N-methyl-S-methylisothiourea hydroiodide

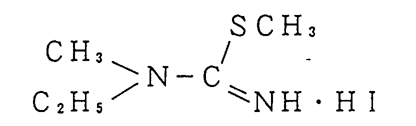

7.08 g (120 mmol) of N-ethyl-N-methylamine was dissolved in 80 ml of dry acetone, and the solution was cooled to 0°C. 13.1 g (100 mmol) of ethoxycarbonyl isothiocyanate was dropwise added thereto. Then, the reaction temperature was raised to room temperature, and the mixture was further stirred at the same temperature for 2 hours. The solvent was distilled off under reduced pressure. To the obtained residue, 80 ml of concentrated hydrochloric acid was added. The reaction temperature was raised to 80°C, and the mixture was further stirred at the same temperature for 5 hours. Then, it was cooled to 0°C, and then ammonium carbonate was gradually added to neutralize the reaction mixture (pH = 6 to 7). After adding a small amount of water, the mixture was extracted three times with 100 ml of ethyl acetate. The ethyl acetate layer was washed with water and then dried over anhydrous sodium sulfate. Then, the solvent was distilled off under reduced pressure. Obtained crystals were washed with a solvent mixture of ethanol/n-hexane to obtain 5.8 g of N-ethyl-N-methylthiourea as white crystals. Melting point: 124 - 125°C

5.8 g (49 mmol) of N-ethyl-N-methylthiourea was dissolved in 10 ml of N,N-dimethylformamide. 8.8 g (62 mmol) of methyl iodide was added at room temperature, and the mixture was stirred at room temperature for 15 hours. 500 ml of ethyl acetate was added to the mixture, and precipitated crystals were collected by filtration and then washed with ethyl acetate to obtain 3.1 g of N-ethyl-N-methyl-S-methylisothiourea hydroiodide as pale yellow crystals.
Melting point: 94 - 97°C

### REFERENCE EXAMPLE g-3

### Preparation of N-methoxy-N-methyl-S-methylisothiourea hydroiodide

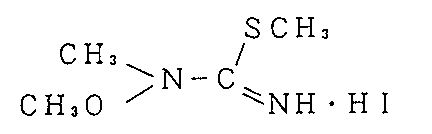

1.83 g (30 mmol) of N-methoxy-N-methylamine was dissolved in 20 ml of dichloromethane, and the solution was cooled to 0°C. 3.93 g (30 mmol) of ethoxycarbonyl isothiocyanate was dropwise added thereto. Then, the reaction temperature was raised to room temperature, and the mixture was further stirred at the same temperature for 15 hours. The solvent was distilled off under reduced pressure, and to the obtained residue, 20 ml of concentrated hydrochloric acid was added. The reaction temperature was raised to 80°C, and the mixture was further stirred at the same temperature for 5 hours. Then, the mixture was cooled to 0°C, and then ammonium carbonate was gradually added to neutralize the reaction mixture (pH = 6 to 7). After adding 10 ml of water, the mixture was extracted three times with 50 ml of ethyl acetate. The ethyl acetate layer was washed with water and then dried over anhydrous magnesium sulfate. Then, the solvent was distilled off under reduced pressure to obtain 2.0 g of N-methoxy-N-methylthiourea as pale yellow crystals. Melting point: 30 - 32°C

1.76 g (14.7 mmol) of N-methoxy-N-methylthiourea was dissolved in 5 ml of N,N-dimethylformamide. 2.09 g (14.7 mmol) of methyl iodide was added thereto at room temperature, and the mixture was stirred at room temperature for 15 hours. 500 ml of ethyl acetate was added thereto, and precipitated crystals were collected by filtration and then washed with ethyl acetate to obtain 2.7 g of N-methoxy-N-methyl-S-methylisothiourea hydroiodide as pale yellow crystals.
Melting point: 122 - 124°C

The structures and physical property values or characteristics of the compounds prepared by the same methods as in Reference Examples g-1 to g-3 are presented in Table 13g.

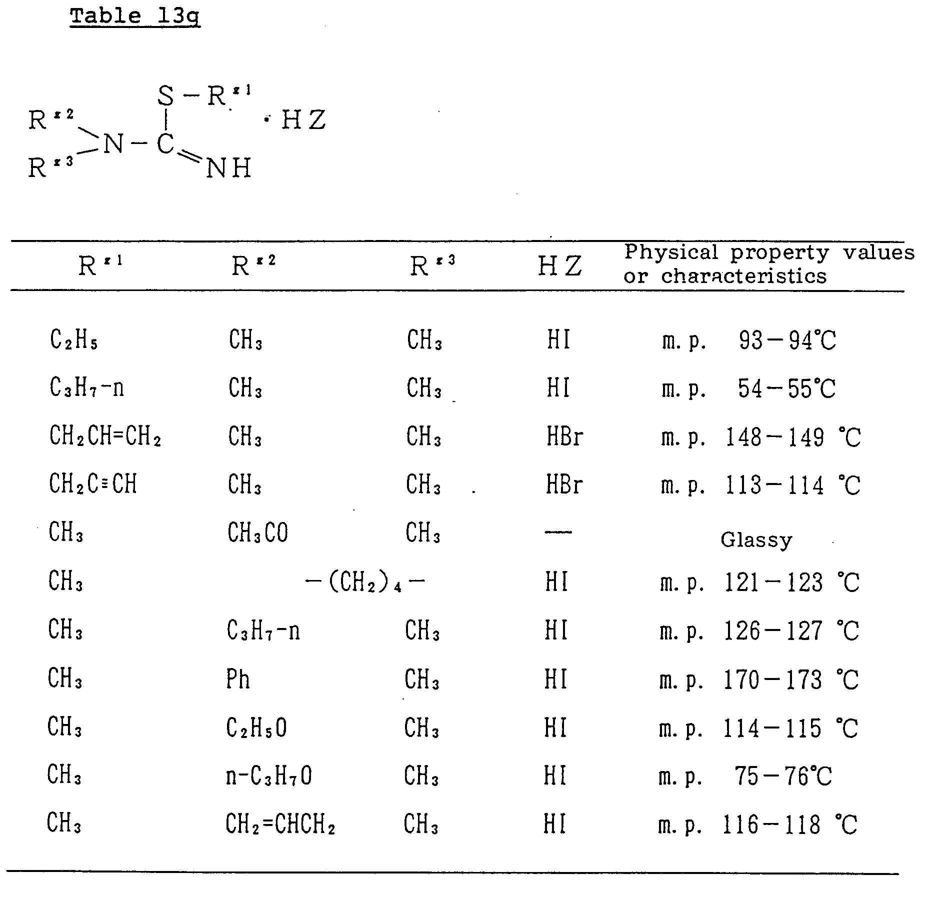

### EXAMPLE a-1

### Preparation of 1-(3-methyl-4-thiazoline-2-sulfonylimino)-3-(4,6-dimethoxypyrimidin-2-yl)urea

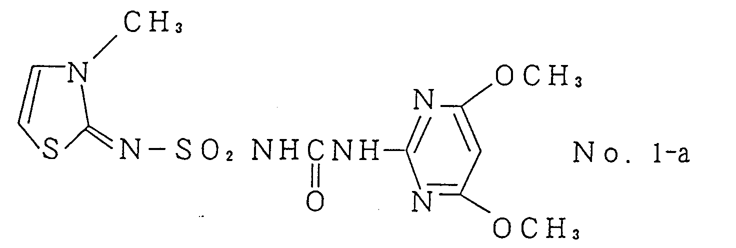

1.55 g (10 mmol) of 2-amino-4,6-dimethoxypyrimidine was dissolved in 20 ml of dry tetrahydrofuran, and 1.42 g (10 mmol) of chlorosulfonyl isocyanate was dropwise added thereto within a range of from -10°C to -5°C. The reaction temperature was raised to 0°C, and the mixture was stirred for 5 minutes. The reaction mixture was again cooled to -30°C, and 1.14 g (10 mmol) of 2-imino-3-methylthiazol-4-ine and 1.11 g (11 mmol) of triethylamine dissolved in 10 ml of dry tetrahydrofuran were dropwise added thereto. The reaction temperature was raised to room temperature, and the mixture was further stirred at the same temperature for one hour. Then, the solvent was distilled off under reduced pressure. Then, water was added to the obtained residue. Precipitated crystals were collected by filtration and washed with acetonitrile to obtain 1.5 g of desired 1-(3-methyl-4-thiazoline-2-sulfonylimino)-3-(4,6-dimethoxypyrimidin-2-yl)urea.
Melting point: 214 - 215°C

### EXAMPLE a-2

### Preparation of 1-(3-n-propyl-4-thiazoline-2-sulfonylimino)-3-(4,6-dimethoxypyrimidin-2-yl)urea

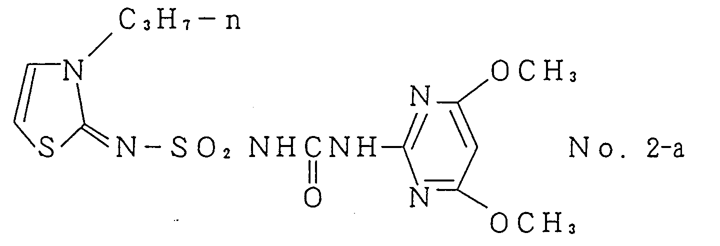

1.55 g (10 mmol) of 2-amino-4,6-dimethoxypyrimidine was dissolved in 30 ml of dry tetrahydrofuran, and 1.42 g (10 mmol) of chlorosulfonyl isocyanate was dropwise added thereto at a temperature of not higher than -20°C. The reaction temperature was raised to 0°C, and then the mixture was cooled again to a temperature of not higher than -20°C. Then, 2.70 g (10 mmol) of 2-imino-3-n-propylthiazol-4-ine hydroiodide and 2.22 g (22 mmol) of triethylamine dissolved in 30 ml of dry tetrahydrofuran were dropwise added thereto. The reaction temperature was raised to room temperature, and the mixture was further stirred at the same temperature for one hour. Then, the solvent was distilled off under reduced pressure. Then, water was added to the residue, and the mixture was extracted three times with chloroform. The chloroform layer was washed sequentially with water and a saturated sodium chloride aqueous solution and then dried over anhydrous sodium sulfate. Then, the solvent was distilled off under reduced pressure. The obtained crystals were washed with ethyl ether to obtain 3 g of desired 1-(3-n-propyl-4-thiazoline-2-sulfonylimino)-3-(4,6-dimethoxypyrimidin-2-yl)urea.
Melting point: 166 - 167°C

### EXAMPLE a-3

### Preparation of 1-(3-n-butylthiazolidine-2-sulfonylimino)-3-(4,6-dimethoxypyrimidin-2-yl)urea

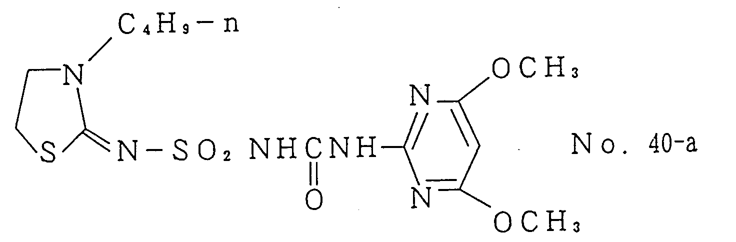

1.55 g (10 mmol) of 2-amino-4,6-dimethoxypyrimidine was dissolved in 40 ml of dry tetrahydrofuran, and 1.42 g (10 mmol) of chlorosulfonyl isocyanate was dropwise added thereto at -40°C. The reaction temperature was raised to 0°C, and then the reaction mixture was cooled again to - 60°C. Then, 1.90 g (12 mmol) of 2-imino-3-n-butylthiazolidine suspended in 40 ml of dry tetrahydrofuran containing 1.33 g (13 mmol) of triethylamine, was added thereto. The reaction temperature was raised to room temperature, and the mixture was further stirred at the same temperature for one hour. Then, the solvent was distilled off under reduced pressure. Then, water was added to the residue, and the mixture was extracted three times with chloroform. The chloroform layer was washed sequentially with water and a saturated sodium chloride aqueous solution, and then dried over anhydrous sodium sulfate. Then, the solvent was distilled off under reduced pressure. The obtained crystals were washed with ethyl ether to obtain 2.8 g of desired 1-(3-n-butylthiazolidine-2-sulfonylimino)-3-(4,6-dimethoxypyrimidin-2-yl)urea.
Melting point: 139 - 140°C

### EXAMPLE a-4

### Preparation of 1-(1,3-dimethyl-4-imidazoline-2-sulfonylimino)-3-(4,6-dimethoxypyrimidin-2-yl)urea

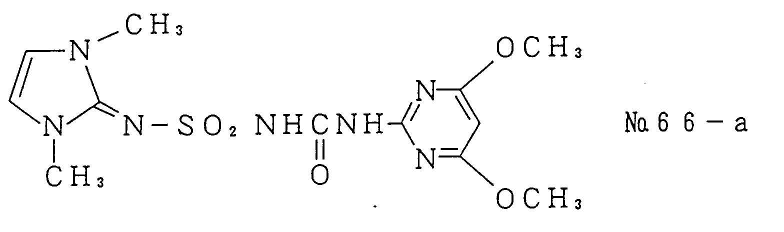

540 mg (3.46 mmol) of 2-amino-4,6-dimethoxypyrimidine was dissolved in 30 ml of dry tetrahydrofuran, and 490 mg (3.46 mmol) of chlorosulfonyl isocyanate was dropwise added in a range of from -20°C to -15°C. The reaction temperature was raised to 0°C, and then the mixture was cooled again to -20°C. Then, a mixture comprising 600 mg (4.07 mmol) of 2-imino-1,3-dimethylimidazol-4-ine hydrochloride, 820 mg (8.13 mmol) of triethylamine and 30 ml of dry tetrahydrofuran, was added thereto. The reaction temperature was raised to room temperature, and the mixture was further stirred at the same temperature for 3 hours. Then, the solvent was distilled off under reduced pressure. Then, 60 ml of water was added to the obtained residue, and crystals were collected by filtration. The obtained crystals were washed with a solvent mixture of ethyl ether, acetonitrile and acetone to obtain 200 mg of desired 1-(1,3-dimethyl-4-imidazoline-2-sulfonylimino)-3-(4,6-dimethoxypyrimidin-2-yl)urea.
Melting point: 201 - 203°C

The structures and the physical property values of the compounds prepared by the same methods as in Examples a-1 to a-4 are presented in Tables 14a-1, 14a-2 and 14a-3.

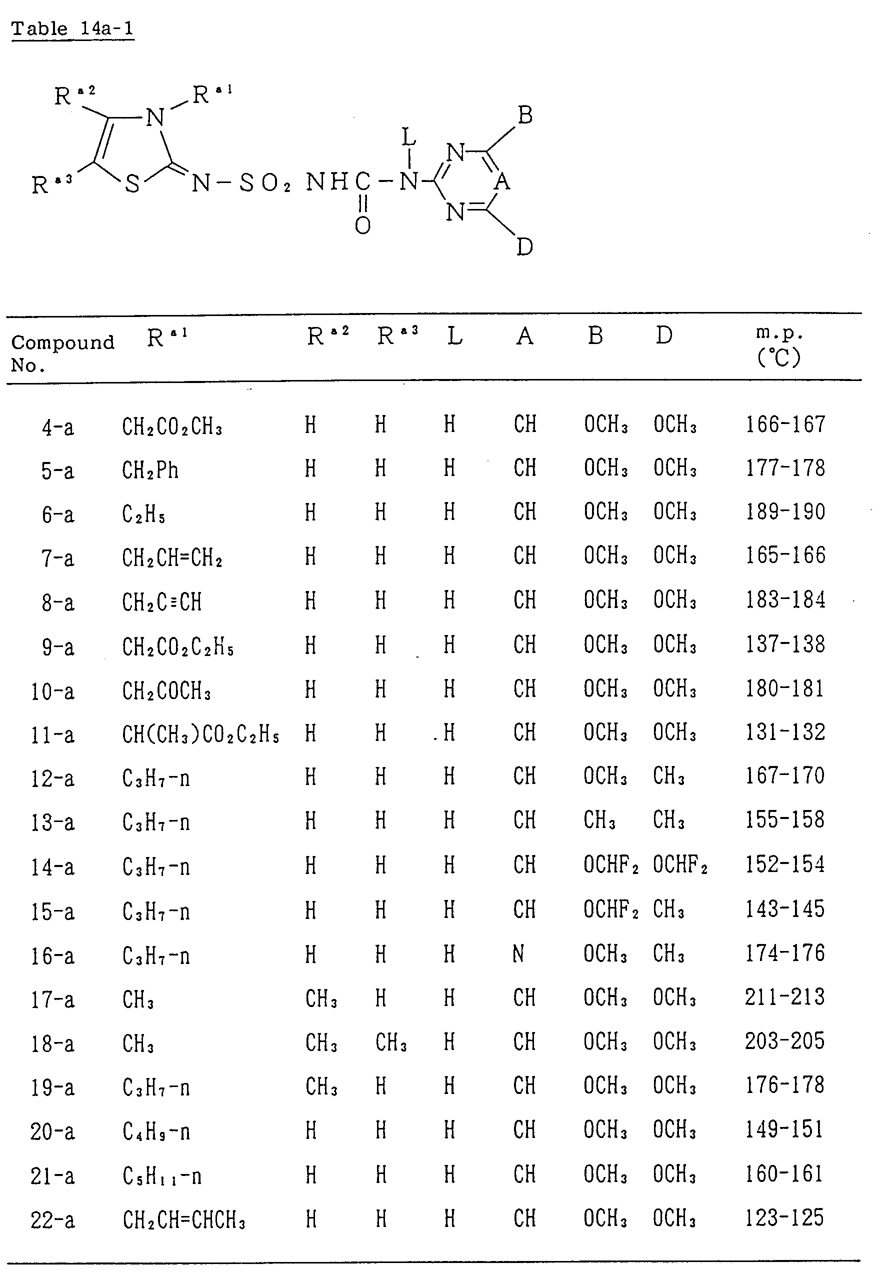

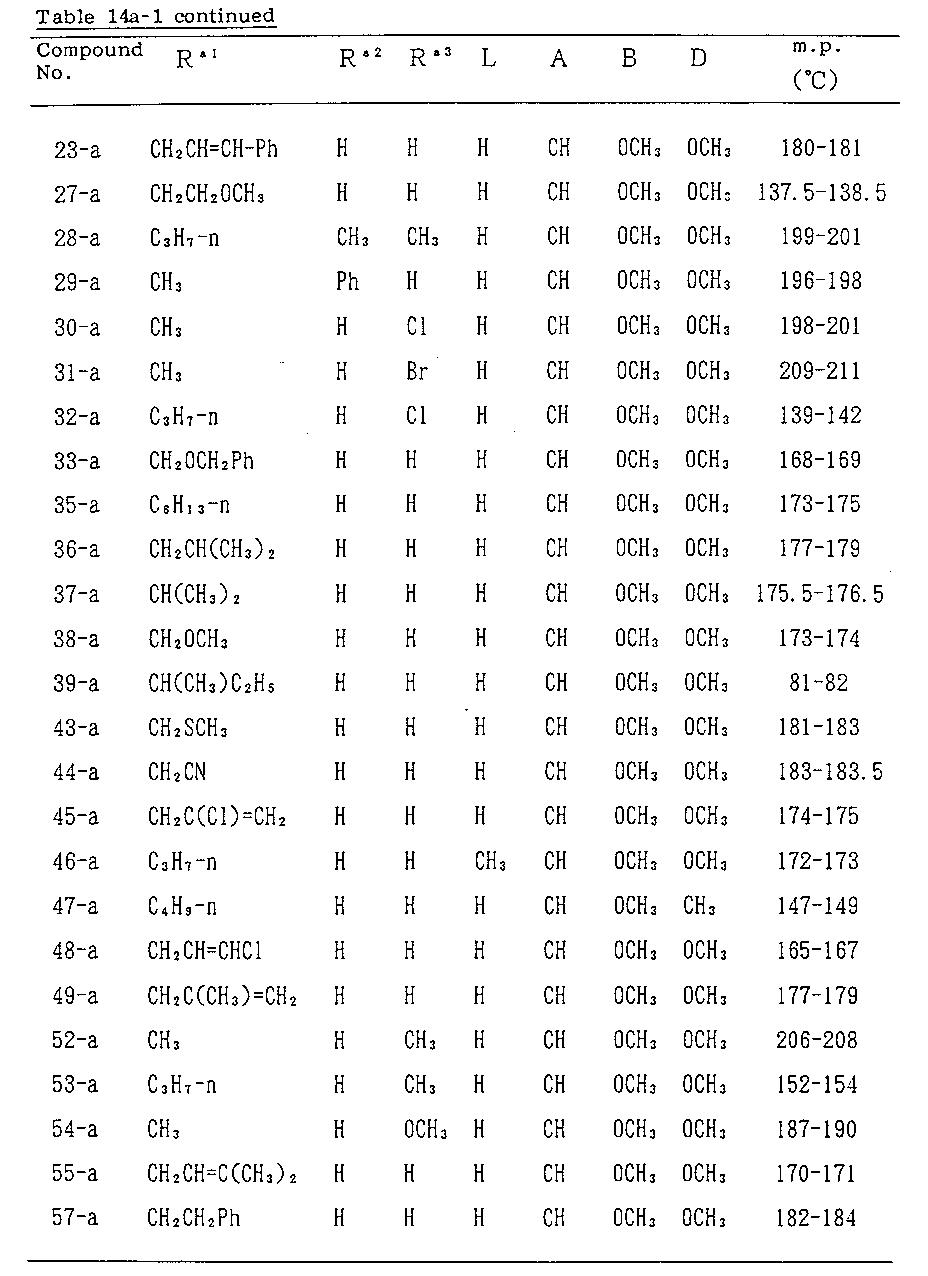

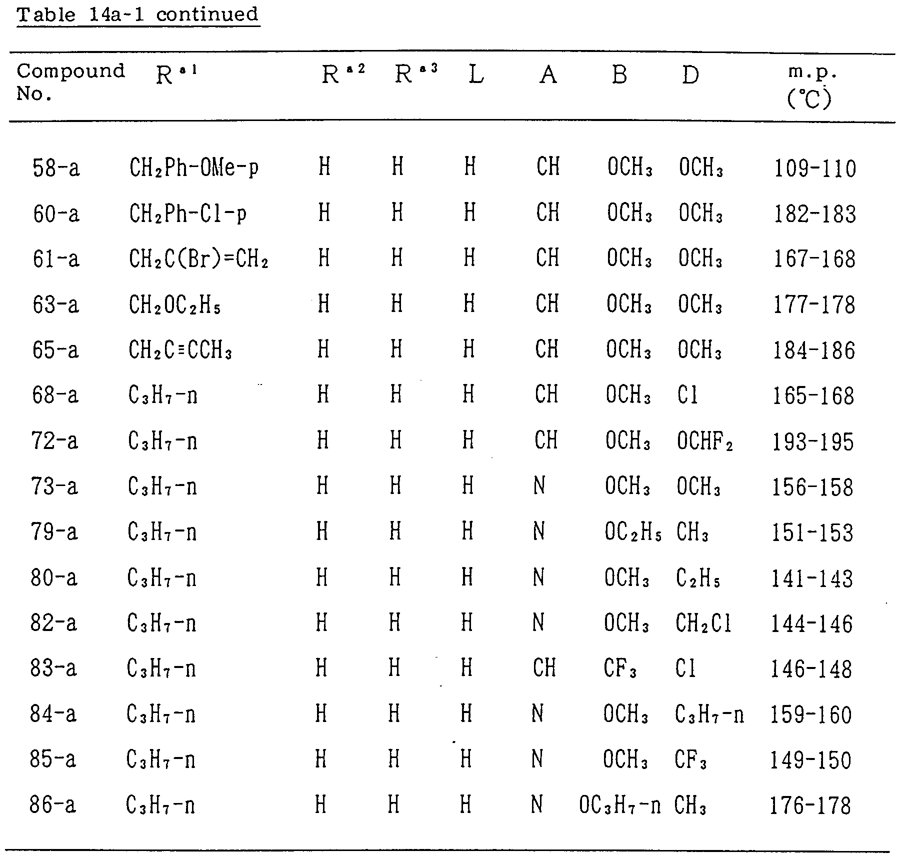

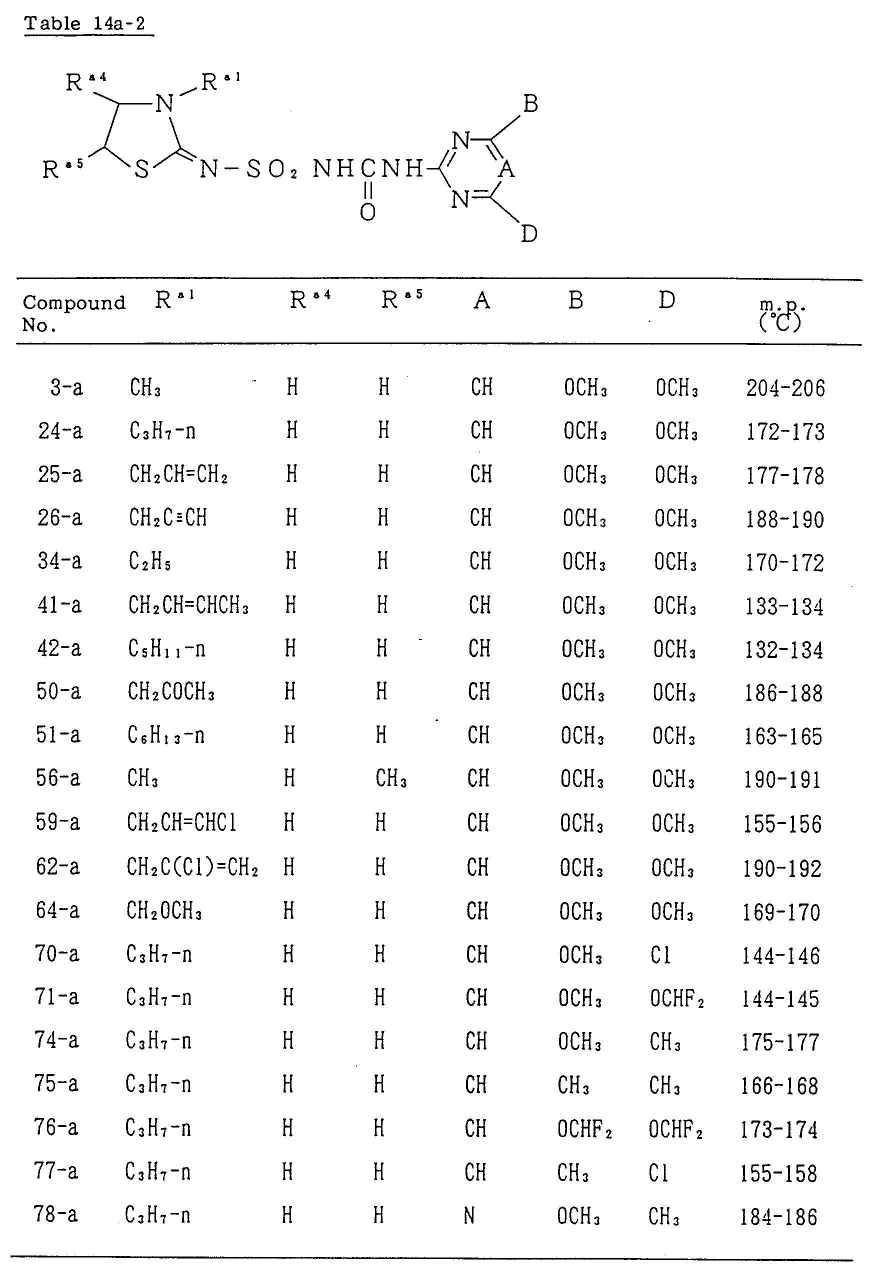

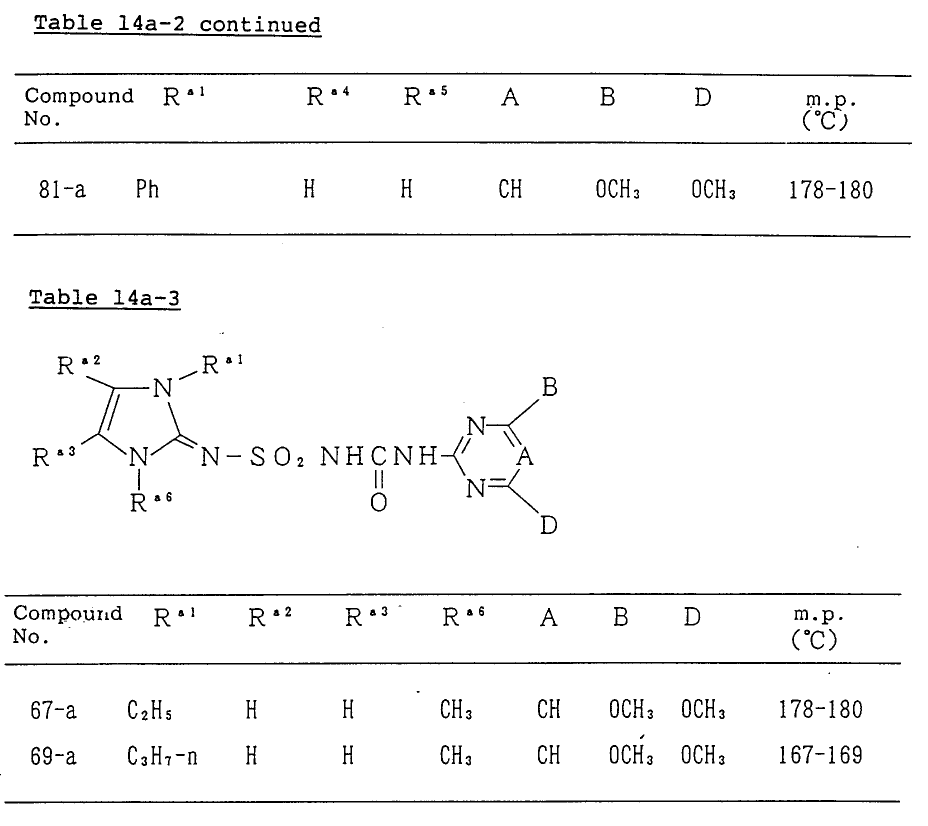

### EXAMPLE b-1

### Preparation of 1-(3-n-propyl-4-thiadiazoline-2-sulfonylimino)-3-(4,6-dimethoxypyrimidin-2-yl)urea

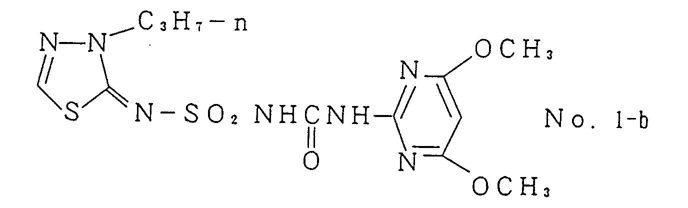

1.55 g (10 mmol) of 2-amino-4,6-dimethoxypyrimidine was dissolved in 40 ml of dry tetrahydrofuran, and 1.42 g (10 mmol) of chlorosulfonyl isocyanate was dropwise added at -40°C. The reaction temperature was raised to 0°C, and then the mixture was cooled again to -40°C. Then, 2.58 g (9.5 mmol) of 2-imino-3-n-propylthiadiazol-4-ine hydroiodide suspended in 40 ml of dry tetrahydrofuran containing 2.22 g (22 mmol) of triethylamine, was added thereto. The reaction temperature was raised to room temperature, and the mixture was further stirred at the same temperature for one hour. Then, the solvent was distilled off under reduced pressure, and then water was added to the obtained residue. The mixture was extracted three times with chloroform. The chloroform layer was washed sequentially with water and a saturated sodium chloride aqueous solution and then dried over anhydrous sodium sulfate. Then, the solvent was distilled off under reduced pressure. Obtained crystals were washed with acetonitrile to obtain 2.25 g of desired 1-(3-n-propyl-4-thiadiazoline-2-sulfonylimino)-3-(4,6-dimethoxypyrimidin-2-yl)urea.
Melting point: 189 - 190°C

The structures and the physical property values of the compounds prepared by the same method as in Example b-1 are presented in Table 14b.

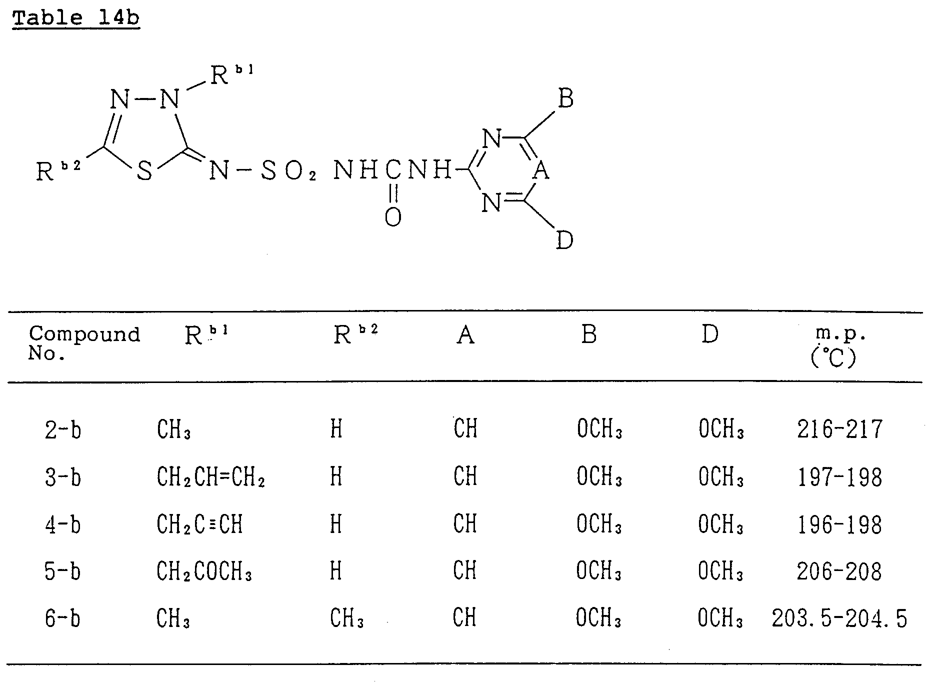

### EXAMPLE c-1

### Preparation of 1-(3-ethoxythiazolidine-2-sulfonylimino)-3-(4,6-dimethoxypyrimidin-2-yl)urea

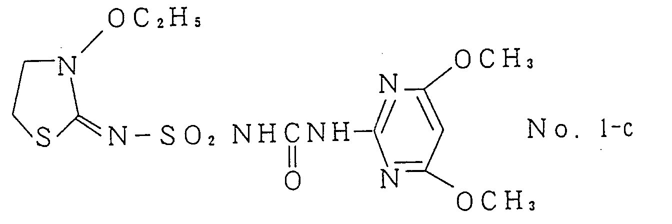

1.55 g (10 mmol) of 2-amino-4,6-dimethoxypyrimidine was dissolved in 20 ml of dry tetrahydrofuran, and 1.42 g (10 mmol) of chlorosulfonyl isocyanate was dropwise added thereto in a range of -10°C to -5°C. The reaction temperature was raised to 0°C, and the mixture was stirred for 5 minutes. The reaction mixture was cooled again to -30°C, and 2.72 g (12 mmol) of 2-imino-3-ethoxythiazolidine hydrobromide and 2.43 g (24 mmol) of triethylamine suspended in 10 ml of dry tetrahydrofuran were gradually added thereto. The reaction temperature was raised to room temperature, and the mixture was further stirred at the same temperature for 20 minutes. Then, the solvent was distilled off under reduced pressure, and then water was added to the obtained residue. Precipitated crystals were extracted three times with 100 ml of chloroform, and the extract was washed once with 100 ml of water and dried over anhydrous sodium sulfate. Then, the solvent was distilled off under reduced pressure. Obtained crystals were washed with ethyl ether and acetonitrile to obtain 2.1 g of desired 1-(3-ethoxythiazolidine-2-sulfonylimino)-3-(4,6-dimethoxypyridimin-2-yl)urea.
Melting point: 175 - 176°C

### EXAMPLE c-2

### Preparation of 1-(3-n-propoxythiazolidine-2-sulfonylimino)-3-(4,6-dimethoxypyrimidin-2-yl)urea

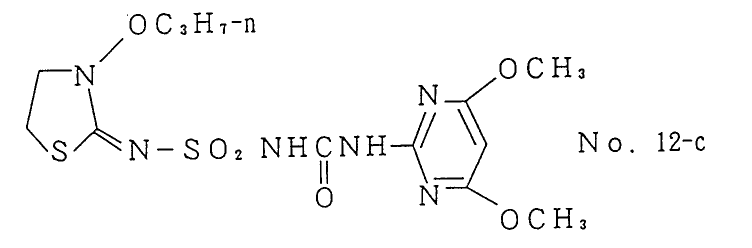

0.62 g (4.0 mmol) of 2-amino-4,6-dimethoxypyrimidine was dissolved in 30 ml of dry tetrahydrofuran, and 0.57 g (4.0 mmol) of chlorosulfonyl isocyanate was dropwise added at -50°C. The reaction temperature was raised to room temperature, and then the reaction mixture was cooled again to -50°C. Then, 1.2 g (5.0 mmol) of 2-imino-3-n-propoxythiazolidine hydrobromide suspended in 20 ml of dry tetrahydrofuran containing 1.01 g (10.0 mmol) of triethylamine, was added thereto. The reaction temperature was gradually raised to room temperature, and the mixture was further stirred at the same temperature for 10 minutes. Then, the solvent was distilled off under reduced pressure, and then water was added to the obtained residue. The mixture was extracted three times with 50 ml of chloroform. The chloroform layer was washed with a saturated sodium chloride aqueous solution and then dried over anhydrous sodium sulfate. Then, the solvent was distilled off under reduced pressure. To the obtained residue, ethyl ether and a small amount of acetonitrile were added for crystallization. The crystals were collected by filtration to obtain 0.65 g of desired 1-(3-n-propoxythiazolidine-2-sulfonylimino)-3-(4,6-dimethoxypyridimin-2-yl)urea.
Melting point: 173 - 175°C

### EXAMPLE c-3

### Preparation of 1-(3-n-propoxythiazolidine-2-sulfonylimino)-3-(4,6-dimethoxytriazin-2-yl)urea

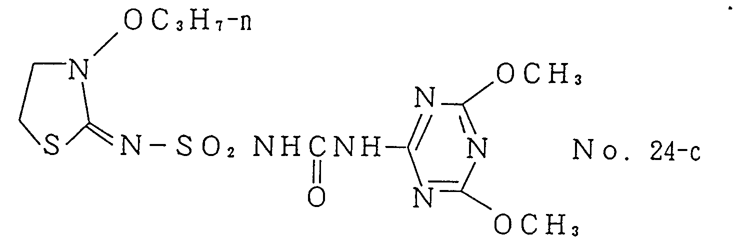

3.76 g (40.0 mmol) of phenol was dissolved in 40 ml of dry tetrahydrofuran, and 5.66 g (40.0 mmol) of chlorosulfonyl isocyanate was dropwise added thereto at -50°C. The reaction temperature was raised to room temperature, and then the reaction mixture was cooled again to -50°C. Then, 10.6 g (44 mmol) of 2-imino-3-n-propoxythiazolidine hydrobromide suspended in 20 ml of dry acetonitrile containing 8.08 g (80 mmol) of triethylamine, was added thereto. The reaction temperature was gradually raised to room temperature, and the mixture was further stirred at the same temperature for 10 minutes. Then, the solvent was distilled off under reduced pressure, and then water was added to the obtained residue. The mixture was extracted three times with 70 ml of chloroform. The chloroform layer was washed with a saturated sodium chloride aqueous solution and then dried over anhydrous sodium sulfate. Then, the solvent was distilled off under reduced pressure. To the obtained residue, ethyl ether and a small amount of acetonitrile were added for crystallization. Then, the crystals were collected by filtration to obtain 14 g of phenyl N-(3-n-propoxythiazolidine-2-sulfonylimino)carbamate. Then, 0.72 g (2.0 mmol) of the obtained carbamate was dissolved in 30 ml of dry dioxane, and 0.23 g (1.5 mmol) of 2-amino-4,6-dimethoxytriazine was added thereto. The mixture was refluxed under heating for 4 hours. The solvent was distilled off under reduced pressure, and ethyl ether and a small amount of acetonitrile were added for crystallization. Then, the crystals were collected by filtration to obtain 0.3 g of desired 1-(3-n-propoxythiazolidine-2-sulfonylimino)-3-(4,6-dimethoxytriazin-2-yl)urea.
Melting point: 166 - 167°C

### EXAMPLE c-4

### Preparation of 1-[3-(3-chloroallyloxy)thiazolidine-2-sulfonylimino]-3-(4-methyl-6-methoxytriazin-2-yl)urea

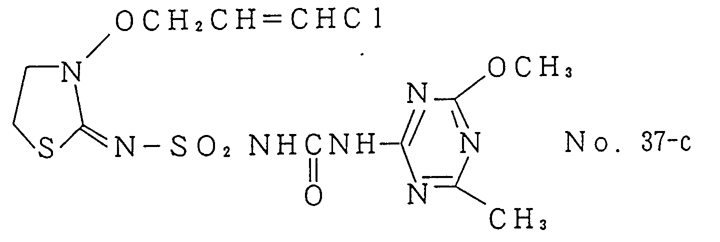

0.84 g (6 mmol) of 2-amino-4-methyl-6-methoxytriazine was suspended in 20 ml of dry tetrahydrofuran, and 0.85 g (6 mmol) of chlorosulfonyl isocyanate was dropwise added thereto at room temperature. A heat was generated mildly, and the reaction mixture turned to a pale yellow solution. Then, 1.64 g (6 mmol) of 2-imino-3-(3-chloroallyloxy)thiazolidine hydrobromide and 1.43 g (14 mmol) of triethylamine suspended in 15 ml of dry tetrahydrofuran were gradually added thereto, and the mixture was further stirred at room temperature for 30 minutes. The solvent was distilled off under reduced pressure, and then water was added to the obtained residue. The resulting oily substance was extracted three times with 80 ml of chloroform. The extract solution was washed once with 100 ml of water and dried over anhydrous sodium sulfate. Then, the solvent was distilled off under reduced pressure. Obtained crystals were washed with ethyl ether and acetonitrile to obtain 1.2 g of desired 1-[3-(3-chloroallyloxy)thiazolidine-2-sulfonylimino]-3-(4-methyl-6-methoxytriazin-2-yl)urea.
Melting point: 145 - 147°C

### EXAMPLE c-5

### Preparation of 1-(3-methoxy-4-methyl-4-thiazoline-2-sulfonylimino)-3-(4,6-dimethoxypyrimidin-2-yl)urea

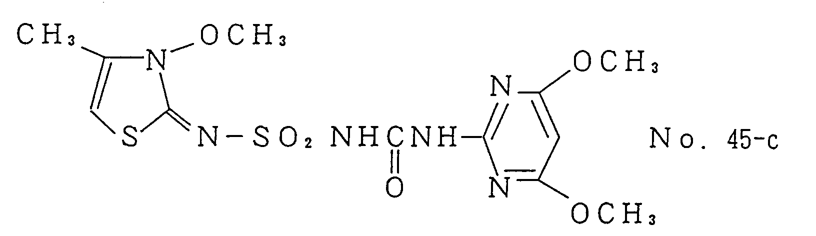

0.78 g (5 mmol) of 2-amino-4,6-dimethoxypyrimidine was dissolved in 10 ml of dry tetrahydrofuran, and 0.71 g (5 mmol) of chlorosulfonyl isocyanate was dropwise added thereto in a range of -20°C to -15°C. The reaction temperature was raised to 0°C, and the mixture was stirred for 5 minutes. The mixture was cooled again to -20°C, and 0.90 g (5 mmol) of 2-imino-3-methoxy-4-methylthiazol-4-ine hydrochloride and 1.11 g (11 mmol) of triethylamine suspended in 10 ml of dry tetrahydrofuran, was gradually added thereto. The reaction temperature was raised to room temperature, and the mixture was further stirred at the same temperature for 20 minutes. The solvent was distilled off under reduced pressure, and then water was added to the obtained residue. Precipitated crystals were extracted three times with 50 ml of chloroform. The extract solution was washed once with 50 ml of water and dried over anhydrous sodium sulfate. Then, the solvent was distilled off under reduced pressure. Obtained crystals were washed with ethyl ether and acetonitrile to obtain 1.02 g of desired 1-(3-methoxy-4-methyl-4-thiazolidine-2-sulfonylimino)-3-(4,6-dimethoxypyrimidin-2-yl)urea.
Melting point: 183 - 185°C

The structures and the physical property values of the compounds prepared by the same methods as in Examples c-1 to c-5 are presented in Tables 14c-1, 14c-2 and 14c-3.

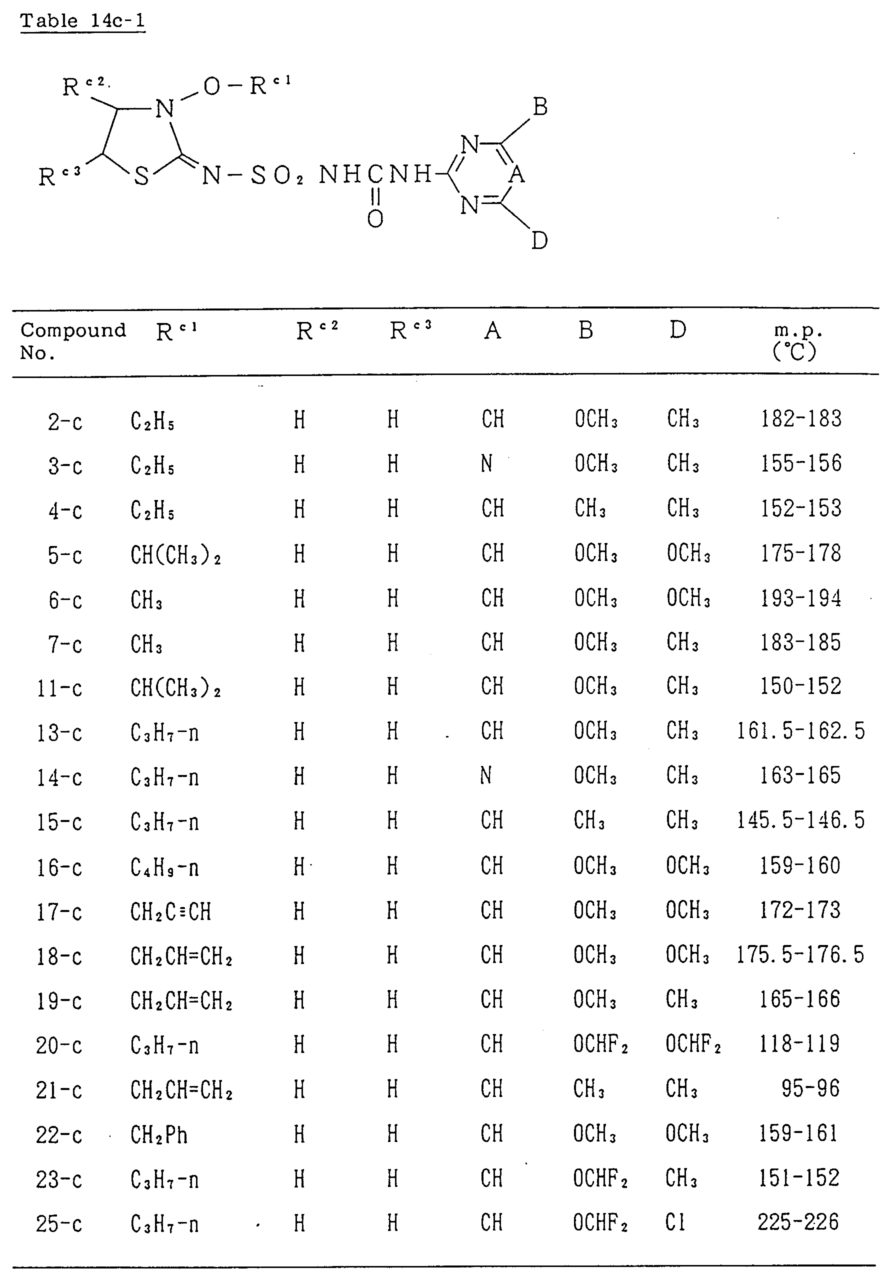

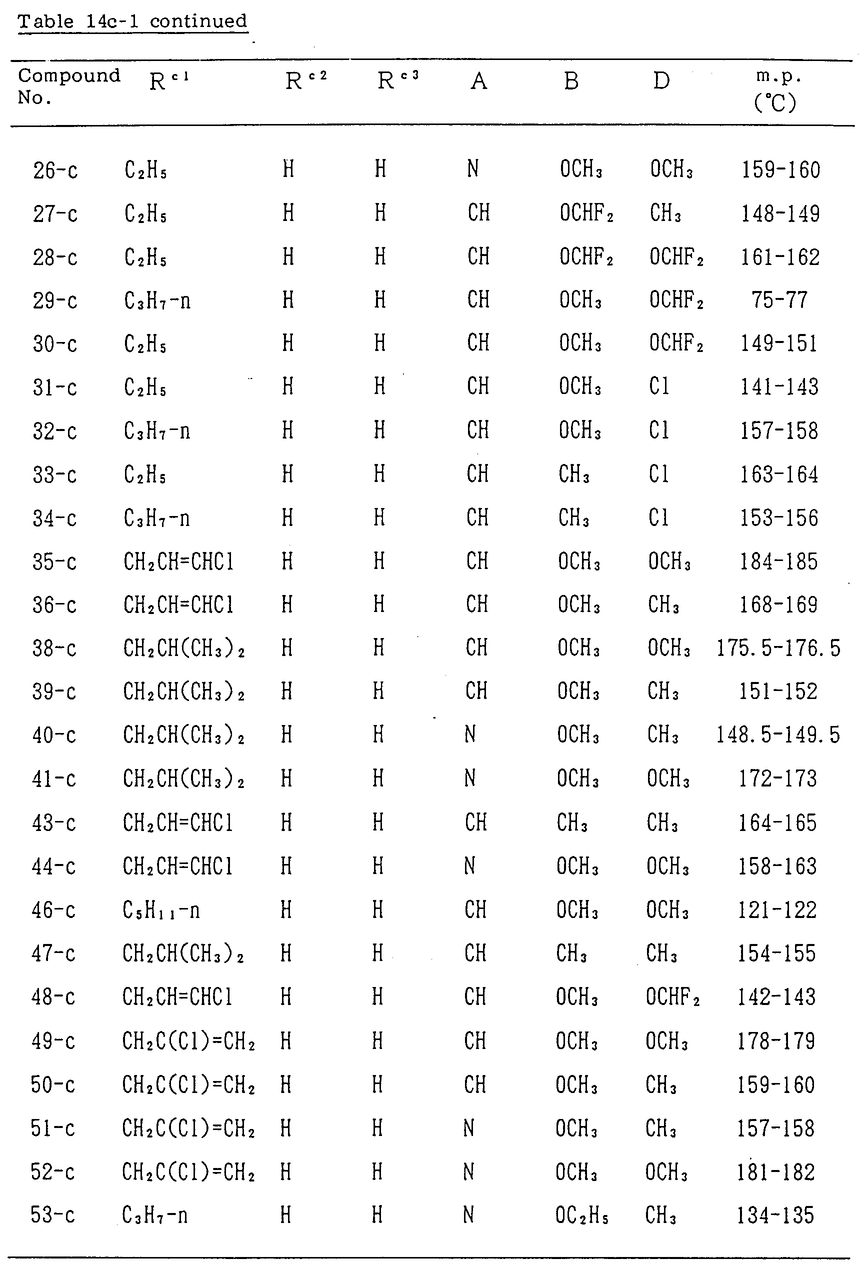

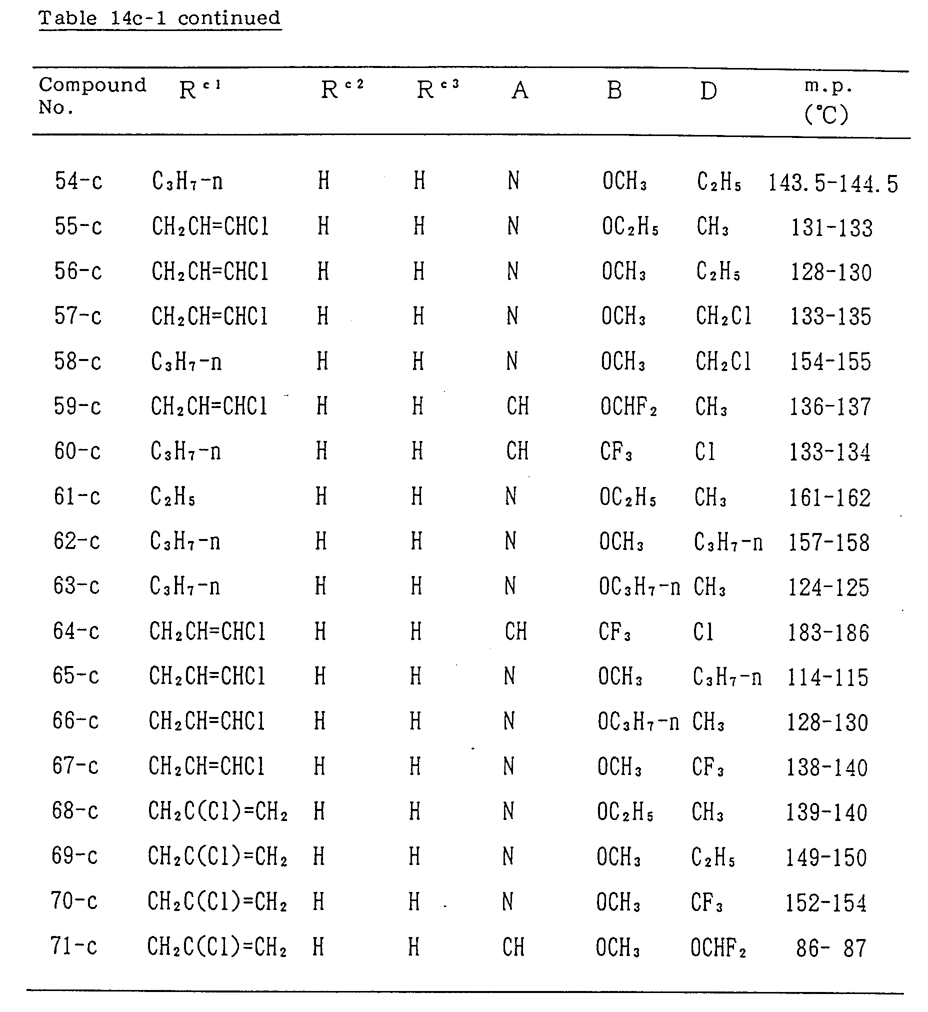

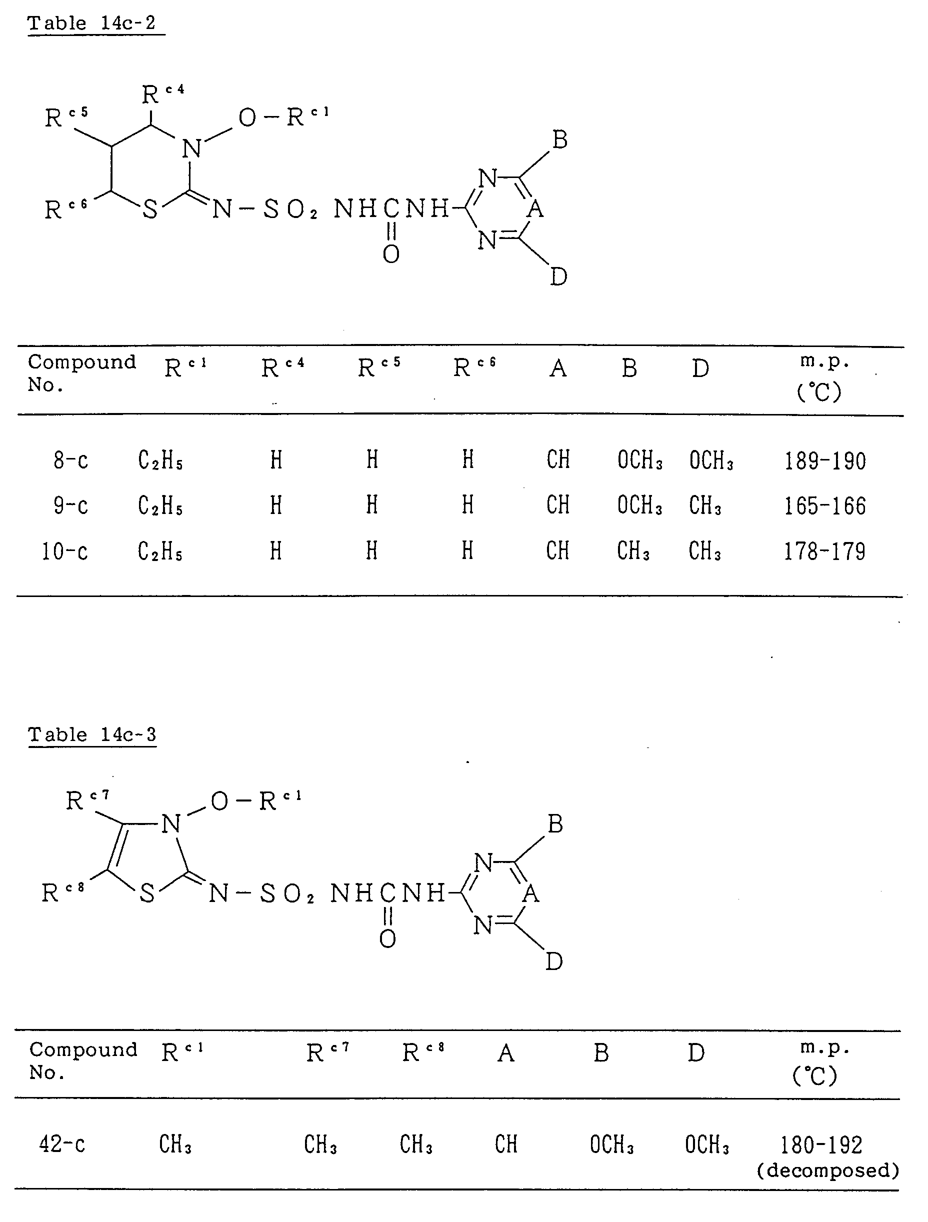

### EXAMPLE d-1

### Preparation of 1-(3-methylthiazolidin-4-one-2-sulfonylimino)-3-(4,6-dimethoxypyrimidin-2-yl)urea

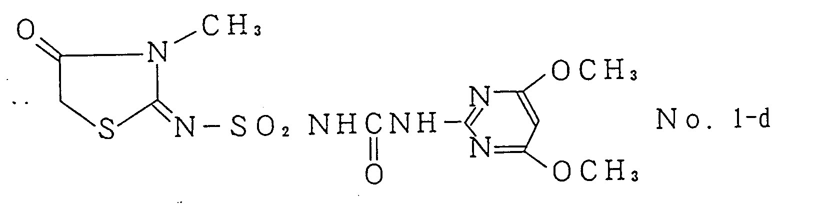

1.55 g (10 mmol) of 2-amino-4,6-dimethoxypyrimidine was dissolved in 30 ml of dry tetrahydrofuran, and 1.42 g (10 mmol) of chlorosulfonyl isocyanate was dropwise added thereto in a range of from -20°C to -15°C. The reaction temperature was raised to 0°C, and the mixture was further stirred at the same temperature for 10 minutes. The reaction mixture was cooled again to -30°C, and a mixture comprising 2.84 g (11 mmol) of 3-methyl-2-iminothiazolidin-4-one hydroiodide, 2.22 g (22 mmol) of triethylamine and 30 ml of dry tetrahydrofuran, was added thereto. The reaction temperature was raised to room temperature, and the mixture was further stirred at the same temperature for 20 minutes. Then, the solvent was distilled off under reduced pressure, and then 100 ml of water was added to the obtained residue. Precipitated crystals were extracted three times with 100 ml of chloroform. The chloroform layer was washed with water and then dried over anhydrous sodium sulfate. Then, the solvent was distilled off under reduced pressure. Obtained crystals were washed with a solvent mixture of ethyl ether/acetonitrile and collected by filtration to obtain 1.5 g of desired 1-(3-methylthiazolidin-4-one-2-sulfonylimino)-3-(4,6-dimethoxypyrimidin-2-yl)urea as colorless crystals.
Melting point: 200 - 201°C

### EXAMPLE d-2

### Preparation of 1-(1-methyl-3-n-propylimidazolidin-4-one-2-sulfonylimino)-3-(4,6-dimethoxypyrimidin-2-yl)urea

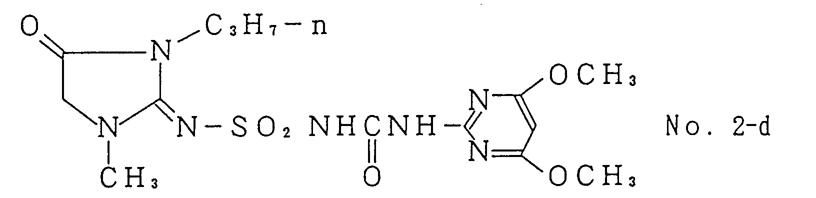

1.16 g (7.5 mmol) of 2-amino-4,6-dimethoxypyrimidine was dissolved in 20 ml of dry tetrahydrofuran, and 1.07 g (7.5 mmol) of chlorosulfonyl isocyanate was dropwise added thereto at -40°C. The reaction temperature was raised to 0°C, and then the reaction mixture was cooled again to -40°C. Then, a mixture comprising 1.16 g (7.5 mmol) of 1-methyl-3-n-propyl-2-iminoimidazolidin-4-one, 0.83 g (8.2 mmol) of triethylamine and 20 ml of dry tetrahydrofuran, was added thereto. The reaction temperature was raised to room temperature, and the mixture was further stirred at the same temperature for 2 hours. Then, the solvent was distilled off under reduced pressure, and then 100 ml of water was added to the obtained residue. The mixture was extracted three times with 100 ml of chloroform. Then, the chloroform layer was washed with water and then dried over anhydrous sodium sulfate. The solvent was distilled off under reduced pressure, and precipitated crystals were washed with a solvent mixture of ethyl ether/acetonitrile and collected by filtration to obtain 0.16 g of desired 1-(1-methyl-3-n-propylimidazolidin-4-one-2-sulfonylimino)-3-(4,6-dimethoxypyrimidin-2-yl)urea.
Melting point: 96 - 98°C

The structures and the physical property values of the compounds prepared by the same methods as in Examples d-1 and d-2 are presented in Table 14d.

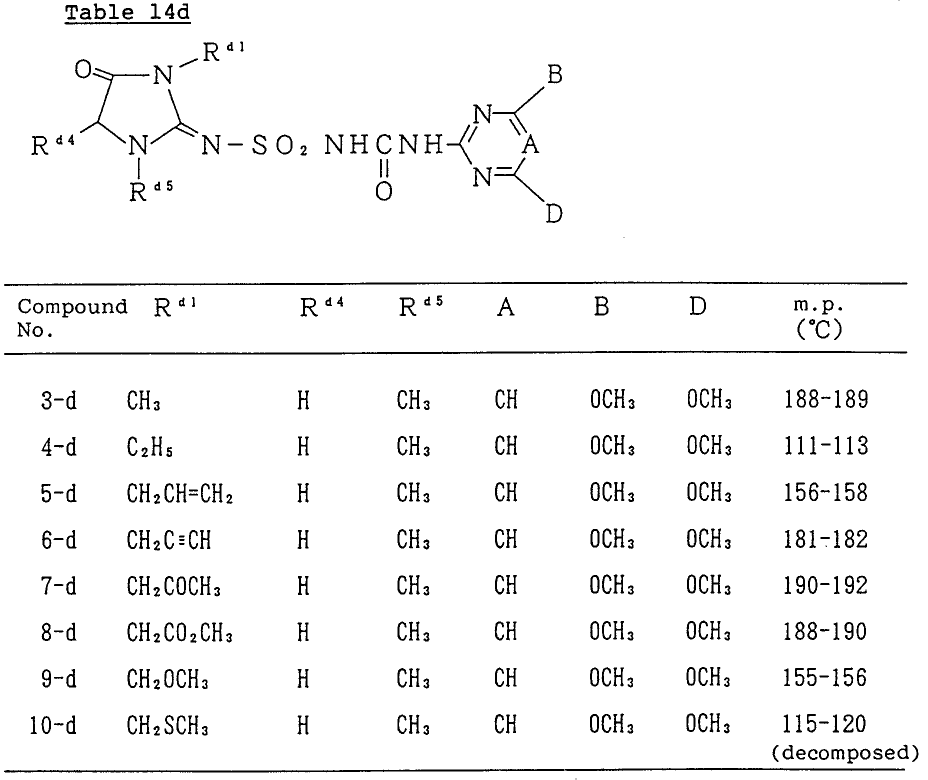

### EXAMPLE e-1

### Preparation of 1-(3,6-dihydro-3-n-propyl-2H-1,3-thiazine-2-sulfonylimino)-3-(4,6-dimethoxypyrimidin-2-yl)urea

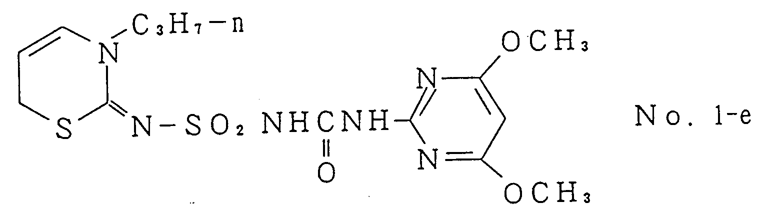

0.32 g (2.06 mmol) of 2-amino-4,6-dimethoxypyrimidine was dissolved in 40 ml of dry tetrahydrofuran, and the solution was cooled to -40°C. At the same temperature, 0.29 g (2.05 mmol) of chlorosulfonyl isocyanate was dropwise added thereto. Then, the temperature was raised to 0°C. The mixture was cooled again to -40°C, and then a mixed solution comprising 0.4 g (2.56 mmol) of 3,6-dihydro-3-n-propyl-2H-1,3-thiazin-2-imine, 0.26 g (2.57 mmol) of triethylamine and 40 ml of dry tetrahydrofuran, was dropwise added thereto. The mixture was gradually heated to room temperature with stirring, and then the solvent was distilled off under reduced pressure. 100 ml of water was added to the residue, and then the mixture was extracted twice with 50 ml of chloroform. The chloroform layer was washed with water and then dried over anhydrous sodium sulfate. Then, chloroform was distilled off under reduced pressure. Obtained crystals were washed with acetonitrile and then with ethyl ether to obtain 0.3 g of desired 1-(3,6-dihydro-3-n-propyl-2H-1,3-thiazine-2-sulfonylimino)-3-(4,6-dimethoxypyrimidin- 2-yl)urea.
Melting point: 161 - 163°C

### EXAMPLE e-2

### Preparation of 1-(3,4,5,6-tetrahydro-3-methyl-2H-1,3-thiazine-2-sulfonylimino)-3-(4,6-dimethoxypyrimidin-2-yl)urea

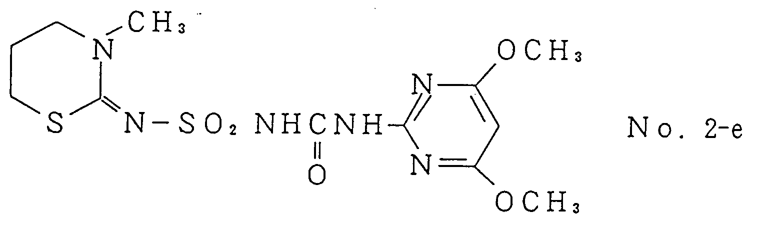

1.55 g (10 mmol) of 2-amino-4,6-dimethoxypyrimidine was dissolved in 30 ml of dry tetrahydrofuran, and 1.42 g (10 mmol) of chlorosulfonyl isocyanate was dropwise added thereto in a range of from -15°C to -5°C. The reaction solution was heated to 0°C and then further stirred at the same temperature for 15 minutes. Then, the reaction solution was cooled again to -30°C, and a mixed solution comprising 1.56 g (12 mmol) of 3,4,5,6-tetrahydro-3-methyl-2H-1,3-thiazin-2-imine, 1.21 g (12 mmol) of triethylamine and 10 ml of dry tetrahydrofuran, was dropwise added thereto. The reaction solution was gradually heated to room temperature with stirring, and then the solvent was distilled off under reduced pressure. 80 ml of water was added to the residue, and precipitated crystals were extracted three times with 60 ml of chloroform. The chloroform layer was washed with water and dried over anhydrous sodium sulfate. Then, chloroform was distilled off under reduced pressure.

Obtained crystals were washed with a solvent mixture of ethyl ether/acetonitrile to obtain 1.2 g of desired 1-(3,4,5,6-tetrahydro-3-methyl-2H-1,3-thiazine-2-sulfonylimino)-3-(4,6-dimethoxypyrimidin-2-yl)urea.
Melting point: 188 - 190°C

The structures and the physical property values of the compounds prepared by the same methods as in Examples e-1 and e-2 are presented in Tables 14e-1 and 14e-2.

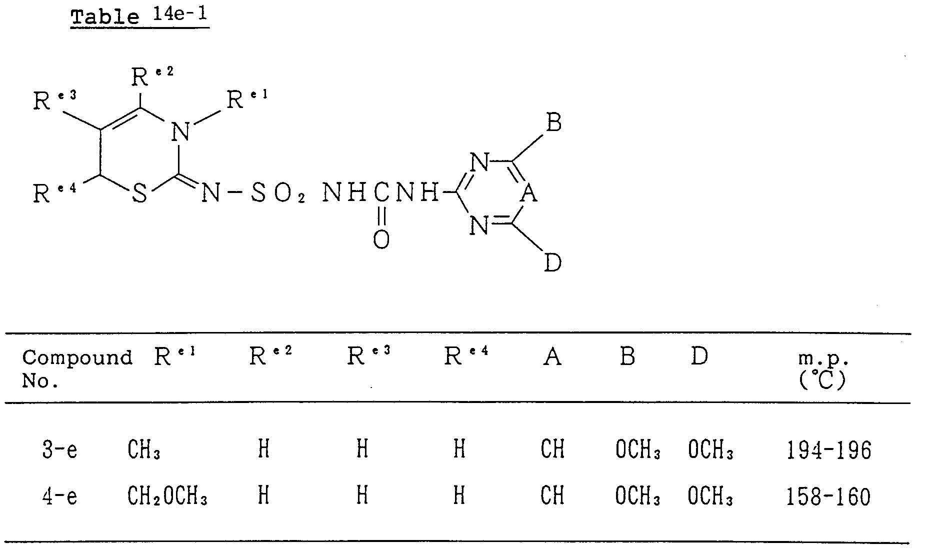

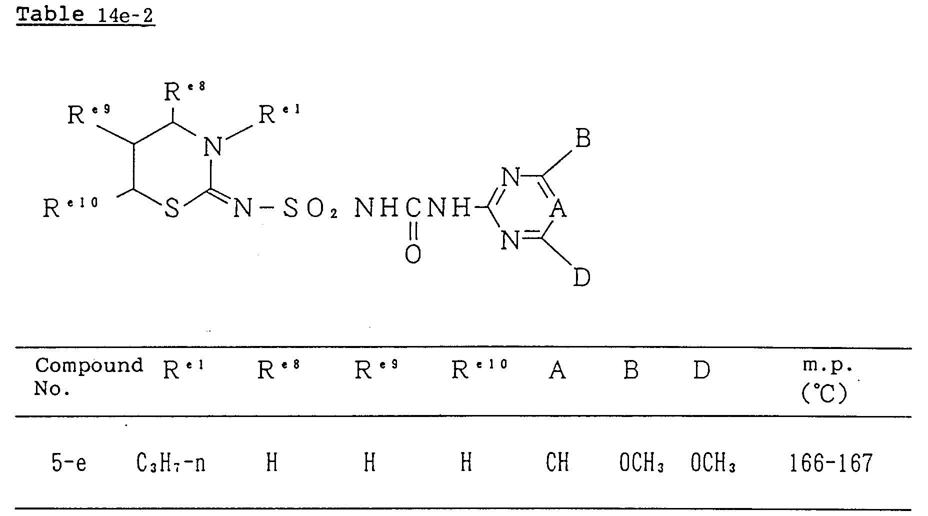

### EXAMPLE f-1

### Preparation of 1-(3-methyloxazolidine-2-sulfonylimino)-3-(4,6-dimethoxypyrimidin-2-yl)urea

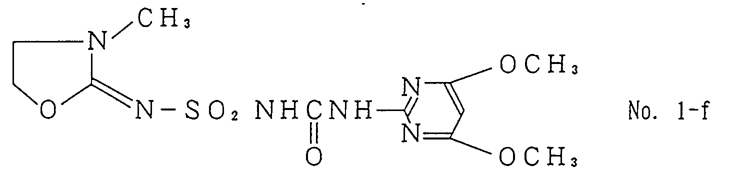

1.55 g (10 mmol) of 2-amino-4,6-dimethoxypyrimidine was dissolved in 30 ml of dry tetrahydrofuran, and 1.42 g (10 mmol) of chlorosulfonyl isocyanate was dropwise added thereto at -20°C. The reaction temperature was raised to -5°C, and the mixture was stirred at the same temperature for 5 minutes. The reaction mixture was cooled again to -20°C, and a mixture comprising 2.28 g (10 mmol) of 2-imino-3-methyloxazolidine hydroiodide, 2.22 g (22 mmol) of triethylamine and 30 ml of dry tetrahydrofuran, was added thereto. The reaction temperature was raised to room temperature, and the mixture was further stirred at the same temperature for one hour. Then, the solvent was distilled off under reduced pressure, and then 100 ml of water was added to the obtained residue. Precipitated crystals were collected by filtration, washed with water and then with a solvent mixture of ethyl ether/acetonitrile and dried to obtain 0.8 g of desired 1-(3-methyloxazolidine-2-sulfonylimino)-3-(4,6-dimethoxypyrimidin-2-yl)urea.
Melting point: 174 - 175°C

The structures and the physical property values of the compounds prepared by the same method as in Example f-1 are presented in Tables 14f.

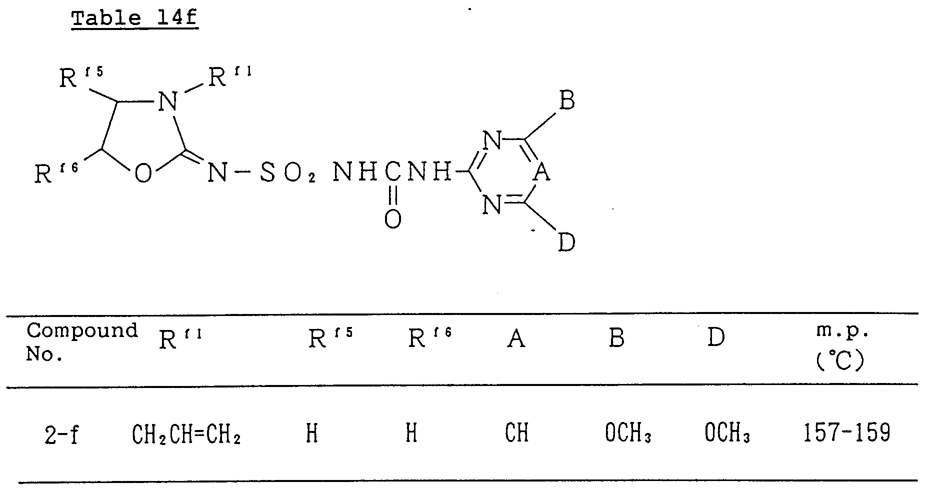

### EXAMPLE g-1

### Preparation of 1-[N-((methylthio-N,N-dimethylamino)-methylene)aminosulfonyl]-3-(4,6-dimethoxypyrimidin-2-yl)urea

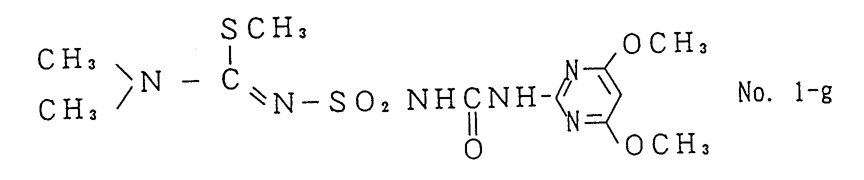

1.55 g (10 mmol) of 2-amino-4,6-dimethoxypyrimidine was dissolved in 40 ml of dry tetrahydrofuran, and 1.42 g (10 mmol) of chlorosulfonyl isocyanate was dropwise added thereto in a range of from -20°C to -15°C. The reaction temperature was raised to 0°C, and the mixture was cooled again to -20°C. Then, a mixture comprising 2.5 g (10.2 mmol) of N,N-dimethyl-S-methylisothiourea hydroiodide, 2.22 g (22 mmol) of triethylamine and 30 ml of dry tetrahydrofuran, was added thereto. The reaction temperature was raised to room temperature, and the mixture was further stirred at the same temperature for 10 minutes. Then, the solvent was distilled off under reduced pressure, and then 80 ml of water was added to the obtained residue. The mixture was extracted three times with 30 ml of chloroform. The chloroform layer was washed with water and then dried over anhydrous sodium sulfate. Then, solvent was distilled off under reduced pressure. Obtained crystals were washed with a solvent mixture of ethyl ether/acetonitrile to obtain 2 g of desired 1-[N-((methylthio-N,N-dimethylamino)methylene)-aminosulfonyl]-3-(4,6-dimethoxypyrimidin-2-yl)urea.
Melting point: 167 - 168°C

### EXAMPLE g-2

### Preparation of 1-[N-((methylthio-N-ethyl-N-methylamino)-methylene)aminosulfonyl]-3-(4,6-dimethoxypyrimidin-2-yl)urea

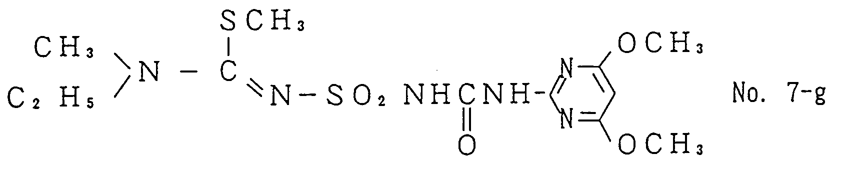

1.55 g (10 mmol) of 2-amino-4,6-dimethoxypyrimidine was dissolved in 40 ml of dry tetrahydrofuran, and 1.42 g (10 mmol) of chlorosulfonyl isocyanate was dropwise added thereto in a range of from -20°C to -15°C. The reaction temperature was raised to 0°C, and the mixture was cooled again to -20°C. Then, a mixture comprising 2.60 g (10 mmol) of N-ethyl-N-methyl-S-methylisothiourea hydroiodide, 2.22 g (22 mmol) of triethylamine and 30 ml of dry tetrahydrofuran, was added thereto. The reaction temperature was raised to room temperature, and the mixture was further stirred at the same temperature for one hour. Then, the solvent was distilled off under reduced pressure, and 80 ml of water was added to the obtained residue. The mixture was extracted three times with 50 ml of chloroform. The chloroform layer was washed with water, and then dried over anhydrous sodium sulfate. Then, the solvent was distilled off under reduced pressure. Obtained crystals were washed with acetonitrile to obtain 1.32 g of desired 1-[N-((methylthio-N-ethyl-N-methylamino)methylene)-aminosulfonyl]-3-(4,6-dimethoxypyrimidin-2-yl)urea.
Melting point: 143 - 144°C

### EXAMPLE g-3

### Preparation of 1-[N-((methylthio-N-methoxy-N-methylamino)methylene)aminosulfonyl]-3-(4,6-dimethoxypyrimidin-2-yl)urea

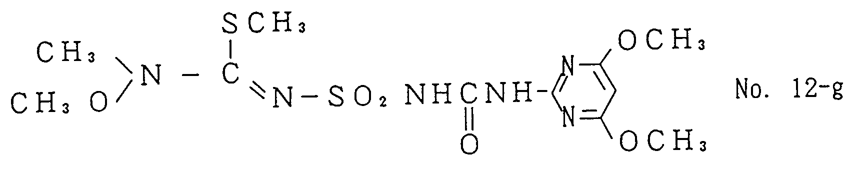

0.78 g (5 mmol) of 2-amino-4,6-dimethoxypyrimidine was dissolved in 20 ml of dry tetrahydrofuran, and 0.71 g (5 mmol) of chlorosulfonyl isocyanate was dropwise added thereto in a range of from -20°C to -15°C. The reaction temperature was raised to 0°C, and then the mixture was cooled again to -20°C. Then, a mixture comprising 1.31 g (5 mmol) of N-methoxy-N-methyl-S-methylisothiourea hydroiodide, 1.11 g (11 mmol) of triethylamine and 15 ml of dry tetrahydrofuran, was added thereto. The reaction temperature was raised to room temperature, and the mixture was further stirred at the same temperature for one hour. Then, the solvent was distilled off under reduced pressure, and then 40 ml of water was added to the obtained residue. The mixture was extracted three times with 25 ml of chloroform. The chloroform layer was washed with water, and then dried over anhydrous sodium sulfate. Then, the solvent was distilled off under reduced pressure. Obtained crystals were washed with acetonitrile to obtain 1.51 g of desired 1-[N-((methylthio-N-methoxy-N-methylamino)methylene)-aminosulfonyl]-3-(4,6-dimethoxypyrimidin-2-yl)urea.
Melting point: 165 - 167°C

The structures and the physical property values of the compounds prepared by the same methods as in Examples g-1 to g-3 are presented in Table 14g.

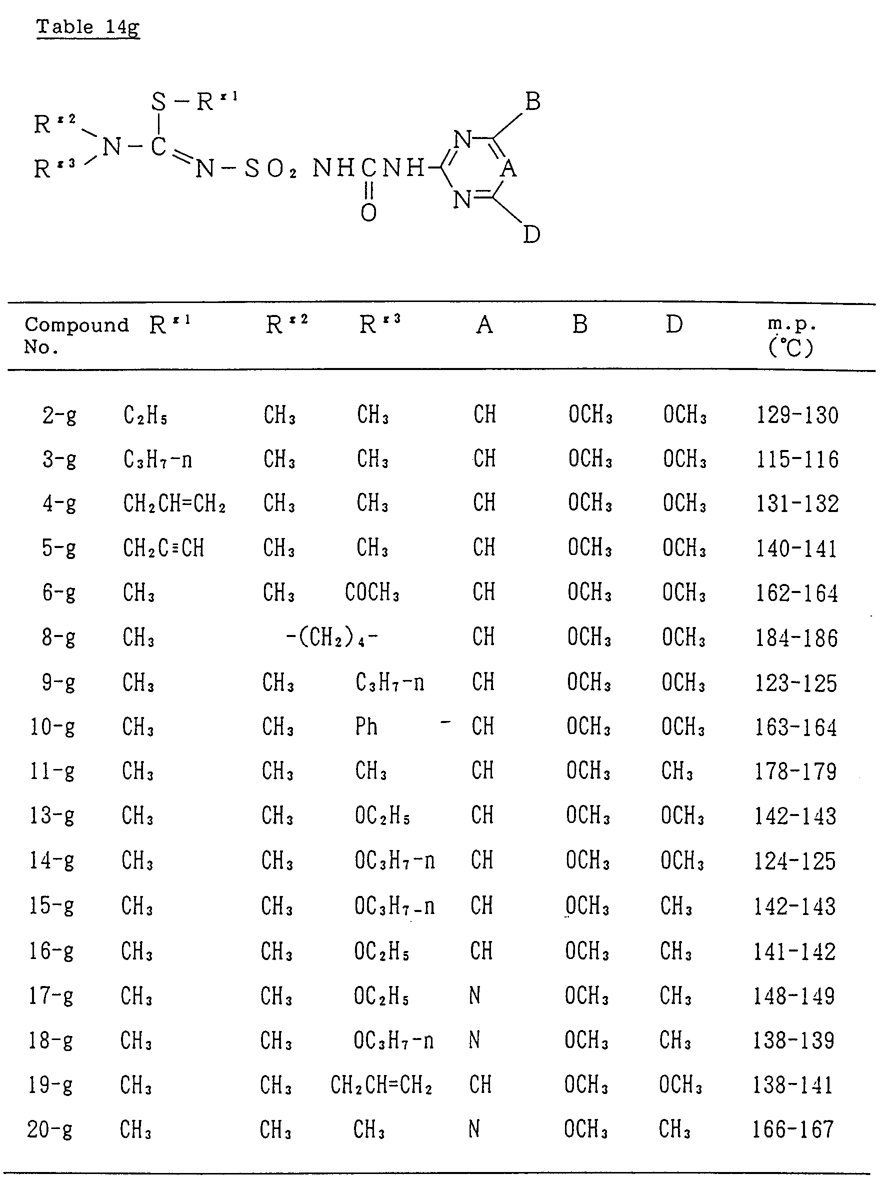

Now, Formulation Examples of the herbicides containing the compounds of the present invention will be given specifically. However, it should be understood that the present invention is by no means restricted to such specific Examples. In the following Formulation Examples, "parts" means "parts by weight".

| Wettable powder | |
|---|---|
| Compound of the present invention | 5-80 parts |
| Solid carrier | 10-85 parts |
| Surfactant | 1-10 parts |
| Other | 1-5 parts |

As other, a coagulation preventing agent may, for example, be mentioned.

| Emulsifiable concentrate | |
|---|---|
| Compound of the present invention | 1-30 parts |
| Liquid carrier | 30-95 parts |
| Surfactant | 5-15 parts |

| Flowable | |
|---|---|
| Compound of the present invention | 5-70 parts |
| Liquid carrier | 15-65 parts |
| Surfactant | 5-12 parts |
| Other | 5-30 parts |

As other, an antifreezing agent and a thickener may, for example, be mentioned.

| Granular wettable powder (dry flowable) | |
|---|---|
| Compound of the present invention | 20-90 parts |
| Solid carrier | 10-60 parts |
| Surfactant | 1-20 parts |

| Granule | |
|---|---|
| Compound of the present invention | 0.1-10 parts |
| Solid carrier | 90-99.9 parts |
| Other | 1-5 parts |

### FORMULATION EXAMPLE a-1: Wettable powder

| | |
|---|---|
| Compound No. 1-a of the present invention | 20 parts |
| Zeeklite A (tradename for a kaolin-type clay, manufactured by Zeeklite Industries, Co., Ltd.) | 76 parts |
| Sorpol 5039 (tradename for a mixture of a nonionic surfactant and an anionic surfactant, manufactured by Toho Chemical Industry Co., Ltd.) | 2 parts |
| Carplex (tradename for a coagulation-preventing agent composed of a white carbon, manufactured by Shionogi Pharmaceutical Co., Ltd.) | 2 parts |

The above ingredients are homogeneously pulverized and mixed to form a wettable powder.

### FORMULATION EXAMPLE a-2: Wettable powder

| | |
|---|---|
| Compound No. 2-a of the present invention | 40 parts |
| Zeeklite A (tradename for a kaolin-type clay, manufactured by Zeeklite Industries, Co., Ltd.) | 54 parts |
| Sorpol 5039 (tradename for a mixture of a nonionic surfactant and an anionic surfactant, manufactured by Toho Chemical Industry Co., Ltd.) | 2 parts |
| Carplex (tradename for a coagulation-preventing agent composed of a white carbon, manufactured by Shionogi Pharmaceutical Co., Ltd.) | 4 parts |

The above ingredients are homogeneously pulverized and mixed to form a wettable powder.

### FORMULATION EXAMPLE a-3: Emulsifiable concentrate

| | |
|---|---|
| Compound No. 3-a of the present invention | 5 parts |
| Xylene | 75 parts |
| N,N-dimethylformamide | 15 parts |
| Sorpol 2680 (tradename for a mixture of a nonionic surfactant and an anionic surfactant, manufactured by Toho Chemical Industry Co., Ltd.) | 5 parts |

The above ingredients are homogeneously mixed to form an emulsifiable concentrate.

### FORMULATION EXAMPLE a-4: Flowable

| | |
|---|---|
| Compound No. 7-a of the present invention | 25 parts |
| Agrizole S-710 (tradename for a nonionic surfactant, manufactured by Kao Corp.) | 10 parts |
| Runox 1000C (tradename for an anionic surfactant, manufactured by Toho Chemical Industry Co., Ltd.) | 0.5 part |
| 1% Rodopol water (tradename for a thickener, manufactured by Rhone-Poulenc) | 20 parts |
| Water | 44.5 parts |

The above ingredients were homogeneously mixed to obtain a flowable.

### FORMULATION EXAMPLE a-5: Flowable

| | |
|---|---|
| Compound No. 8-a of the present invention | 40 parts |
| Agrizole S-710 (tradename for a nonionic surfactant, manufactured by Kao Corp.) | 10 parts |
| Runox 1000C (tradename for an anionic surfactant, manufactured by Toho Chemical Industry Co., Ltd.) | 0.5 part |
| 1% Rodopol water (tradename for a thickener, manufactured by Rhone-Poulenc) | 20 parts |
| Water | 29.5 parts |

The above ingredients were homogeneously mixed to obtain a flowable.

### FORMULATION EXAMPLE a-6: Granular wettable powder (dry flowable)

| | |
|---|---|
| Compound No. 10-a of the present invention | 75 parts |
| Isoban No. 1 (tradename for an anionic surfactant, manufactured by Kuraray Isoprene Chemical Co., Ltd.) | 10 parts |
| Vanirex N (tradename for an anionic surfactant, manufactured by Sanyo Kokusaku Pulp Co., Ltd.) | 5 parts |
| Carplex #80 (tradename for a white carbon, manufactured by Shionogi Pharmaceutical Co., Ltd.) | 10 parts |

The above ingredients are uniformly mixed and pulverized to form a dry flowable.

### FORMULATION EXAMPLE a-7: Granule

| | |
|---|---|
| Compound No. 69-a of the present invention | 1 part |
| Bentonite | 55 parts |
| Talc | 44 parts |

The above ingredients were homogeneously mixed and pulverized, and after an addition of a small amount of water, the mixture was stirred, mixed and granulated by an extrusion-type granulating machine, followed by drying to obtain a granule.

### FORMULATION EXAMPLE b-1: Wettable powder

| | |
|---|---|
| Compound No. 1-b of the present invention | 20 parts |
| Zeeklite A (tradename for a kaolin-type clay, manufactured by Zeeklite Industries, Co., Ltd.) | 76 parts |
| Sorpol 5039 (tradename for a mixture of a nonionic surfactant and an anionic surfactant, manufactured by Toho Chemical Industry Co., Ltd.) | 2 parts |
| Carplex (tradename for a coagulation-preventing agent composed of a white carbon, manufactured by Shionogi Pharmaceutical Co., Ltd.) | 2 parts |

The above ingredients are homogeneously pulverized and mixed to form a wettable powder.

### FORMULATION EXAMPLE b-2: Wettable powder

| | |
|---|---|
| Compound No. 2-b of the present invention | 40 parts |
| Zeeklite A (tradename for a kaolin-type clay, manufactured by Zeeklite Industries, Co., Ltd.) | 54 parts |
| Sorpol 5039 (tradename for a mixture of a nonionic surfactant and an anionic surfactant, manufactured by Toho Chemical Industry Co., Ltd.) | 2 parts |
| Carplex (tradename for a coagulation-preventing agent composed of a white carbon, manufactured by Shionogi Pharmaceutical Co., Ltd.) | 4 parts |

The above ingredients are homogeneously pulverized and mixed to form a wettable powder.

### FORMULATION EXAMPLE b-3: Emulsifiable concentrate

| | |
|---|---|
| Compound No. 3-b of the present invention | 5 parts |
| Xylene | 75 parts |
| N,N-dimethylformamide | 15 parts |
| Sorpol 2680 (tradename for a mixture of a nonionic surfactant and an anionic surfactant, manufactured by Toho Chemical Industry Co., Ltd.) | 5 parts |

The above ingredients are homogeneously mixed to form an emulsifiable concentrate.

### FORMULATION EXAMPLE b-4: Flowable

| | |
|---|---|
| Compound No. 1-b of the present invention | 25 parts |
| Agrizole S-710 (tradename for a nonionic surfactant, manufactured by Kao Corp.) | 10 parts |
| Runox 1000C (tradename for an anionic surfactant, manufactured by Toho Chemical Industry Co., Ltd.) | 0.5 part |
| 1% Rodopol water (tradename for a thickener, manufactured by Rhone-Poulenc) | 20 parts |
| Water | 44.5 parts |

The above ingredients were homogeneously mixed to obtain a flowable.

### FORMULATION EXAMPLE b-5: Flowable

| | |
|---|---|
| Compound No. 4-b of the present invention | 40 parts |
| Agrizole S-710 (tradename for a nonionic surfactant, manufactured by Kao Corp.) | 10 parts |
| Runox 1000C (tradename for an anionic surfactant, manufactured by Toho Chemical Industry Co., Ltd.) | 0.5 part |
| 1% Rodopol water (tradename for a thickener, manufactured by Rhone-Poulenc) | 20 parts |
| Water | 29.5 parts |

The above ingredients were homogeneously mixed to obtain a flowable.

### FORMULATION EXAMPLE b-6: Granular wettable powder (dry flowable)

| | |
|---|---|
| Compound No. 5-b of the present invention | 75 parts |
| Isoban No. 1 (tradename for an anionic surfactant, manufactured by Kuraray Isoprene Chemical Co., Ltd.) | 10 parts |
| Vanirex N (tradename for an anionic surfactant, manufactured by Sanyo Kokusaku Pulp Co., Ltd.) | 5 parts |
| Carplex #80 (tradename for a white carbon, manufactured by Shionogi Pharmaceutical Co., Ltd.) | 10 parts |

The above ingredients are uniformly mixed and pulverized to form a dry flowable.

### FORMULATION EXAMPLE b-7: Granule

| | |
|---|---|
| Compound No. 6-b of the present invention | 1 part |
| Bentonite | 55 parts |
| Talc | 44 parts |

The above ingredients were homogeneously mixed and pulverized, and after an addition of a small amount of water, the mixture was stirred, mixed and granulated by an extrusion-type granulating machine, followed by drying to obtain a granule.

### FORMULATION EXAMPLE c-1: Wettable powder

| | |
|---|---|
| Compound No. 1-c of the present invention | 20 parts |
| Zeeklite A (tradename for a kaolin-type clay, manufactured by Zeeklite Industries, Co., Ltd.) | 76 parts |
| Sorpol 5039 (tradename for a mixture of a nonionic surfactant and an anionic surfactant, manufactured by Toho Chemical Industry Co., Ltd.) | 2 parts |
| Carplex (tradename for a coagulation-preventing agent composed of a white carbon, manufactured by Shionogi Pharmaceutical Co., Ltd.) | 2 parts |

The above ingredients are homogeneously pulverized and mixed to form a wettable powder.

### FORMULATION EXAMPLE c-2: Wettable powder

| | |
|---|---|
| Compound No. 2-c of the present invention | 40 parts |
| Zeeklite A (tradename for a kaolin-type clay, manufactured by Zeeklite Industries, Co., Ltd.) | 54 parts |
| Sorpol 5039 (tradename for a mixture of a nonionic surfactant and an anionic surfactant, manufactured by Toho Chemical Industry Co., Ltd.) | 2 parts |
| Carplex (tradename for a coagulation-preventing agent composed of a white carbon, manufactured by Shionogi Pharmaceutical Co., Ltd.) | 4 parts |

The above ingredients are homogeneously pulverized and mixed to form a wettable powder.

### FORMULATION EXAMPLE c-3: Emulsifiable concentrate

| | |
|---|---|
| Compound No. 12-c of the present invention | 5 parts |
| Xylene | 75 parts |
| N,N-dimethylformamide | 15 parts |
| Sorpol 2680 (tradename for a mixture of a nonionic surfactant and an anionic surfactant, manufactured by Toho Chemical Industry Co., Ltd.) | 5 parts |

The above ingredients are homogeneously mixed to form an emulsifiable concentrate.

### FORMULATION EXAMPLE c-4: Flowable

| | |
|---|---|
| Compound No. 24-c of the present invention | 25 parts |
| Agrizole S-710 (tradename for a nonionic surfactant, manufactured by Kao Corp.) | 10 parts |
| Runox 1000C (tradename for an anionic surfactant, manufactured by Toho Chemical Industry Co., Ltd.) | 0.5 part |
| 1% Rodopol water (tradename for a thickener, manufactured by Rhone-Poulenc) | 20 parts |
| Water | 44.5 parts |

The above ingredients were homogeneously mixed to obtain a flowable.

### FORMULATION EXAMPLE c-5: Flowable

| | |
|---|---|
| Compound No. 37-c of the present invention | 40 parts |
| Agrizole S-710 (tradename for a nonionic surfactant, manufactured by Kao Corp.) | 10 parts |
| Runox 1000C (tradename for an anionic surfactant, manufactured by Toho Chemical Industry Co., Ltd.) | 0.5 part |
| 1% Rodopol water (tradename for a thickener, manufactured by Rhone-Poulenc) | 20 parts |
| Water | 29.5 parts |

The above ingredients were homogeneously mixed to obtain a flowable.

### FORMULATION EXAMPLE c-6: Granular wettable powder (dry flowable)

| | |
|---|---|
| Compound No. 45-c of the present invention | 75 parts |
| Isoban No. 1 (tradename for an anionic surfactant, manufactured by Kuraray Isoprene Chemical Co., Ltd.) | 10 parts |
| Vanirex N (tradename for an anionic surfactant, manufactured by Sanyo Kokusaku Pulp Co., Ltd.) | 5 parts |
| Carplex #80 (tradename for a white carbon, manufactured by Shionogi Pharmaceutical Co., Ltd.) | 10 parts |

The above ingredients are uniformly mixed and pulverized to form a dry flowable.

### FORMULATION EXAMPLE c-7: Granule

| | |
|---|---|
| Compound No. 48-c of the present invention | 1 part |
| Bentonite | 55 parts |
| Talc | 44 parts |

The above ingredients were homogeneously mixed and pulverized, and after an addition of a small amount of water, the mixture was stirred, mixed and granulated by an extrusion-type granulating machine, followed by drying to obtain a granule.

### FORMULATION EXAMPLE d-1: Wettable powder

| | |
|---|---|
| Compound No. 1-d of the present invention | 20 parts |
| Zeeklite A (tradename for a kaolin-type clay, manufactured by Zeeklite Industries, Co., Ltd.) | 76 parts |
| Sorpol 5039 (tradename for a mixture of a nonionic surfactant and an anionic surfactant, manufactured by Toho Chemical Industry Co., Ltd.) | 2 parts |
| Carplex (tradename for a coagulation-preventing agent composed of a white carbon manufactured by Shionogi Pharmaceutical Co., Ltd.) | 2 parts |

The above ingredients are homogeneously pulverized and mixed to form a wettable powder.

### FORMULATION EXAMPLE d-2: Wettable powder

| | |
|---|---|
| Compound No. 2-d of the present invention | 40 parts |
| Zeeklite A (tradename for a kaolin-type clay, manufactured by Zeeklite Industries, Co., Ltd.) | 54 parts |
| Sorpol 5039 (tradename for a mixture of a nonionic surfactant and an anionic surfactant, manufactured by Toho Chemical Industry Co., Ltd.) | 2 parts |
| Carplex (tradename for a coagulation-preventing agent composed of a white carbon, manufactured by Shionogi Pharmaceutical Co., Ltd.) | 4 parts |

The above ingredients are homogeneously pulverized and mixed to form a wettable powder.

### FORMULATION EXAMPLE d-3: Emulsifiable concentrate

| | |
|---|---|
| Compound No. 3-d of the present invention | 5 parts |
| Xylene | 75 parts |
| N,N-dimethylformamide | 15 parts |
| Sorpol 2680 (tradename for a mixture of a nonionic surfactant and an anionic surfactant, manufactured by Toho Chemical Industry Co., Ltd.) | 5 parts |

The above ingredients are homogeneously mixed to form an emulsifiable concentrate.

### FORMULATION EXAMPLE d-4: Flowable

| | |
|---|---|
| Compound No. 4-d of the present invention | 25 parts |
| Agrizole S-710 (tradename for a nonionic surfactant, manufactured by Kao Corp.) | 10 parts |
| Runox 1000C (tradename for an anionic surfactant, manufactured by Toho Chemical Industry Co., Ltd.) | 0.5 part |
| 1% Rodopol water (tradename for a thickener, manufactured by Rhone-Poulenc) | 20 parts |
| Water | 44.5 parts |

The above ingredients were homogeneously mixed to obtain a flowable.

### FORMULATION EXAMPLE d-5: Flowable

| | |
|---|---|
| Compound No. 5-d of the present invention | 40 parts |
| Agrizole S-710 (tradename for a nonionic surfactant, manufactured by Kao Corp.) | 10 parts |
| Runox 1000C (tradename for an anionic surfactant, manufactured by Toho Chemical Industry Co., Ltd.) | 0.5 part |
| 1% Rodopol water (tradename for a thickener, manufactured by Rhone-Poulenc) | 20 parts |
| Water | 29.5 parts |

The above ingredients were homogeneously mixed to obtain a flowable.

### FORMULATION EXAMPLE d-6: Granular wettable powder (dry flowable)

| | |
|---|---|
| Compound No. 6-d of the present invention | 75 parts |
| Isoban No. 1 (tradename for an anionic surfactant, manufactured by Kuraray Isoprene Chemical Co., Ltd.) | 10 parts |
| Vanirex N (tradename for an anionic surfactant, manufactured by Sanyo Kokusaku Pulp Co., Ltd.) | 5 parts |
| Carplex #80 (tradename for a white carbon, manufactured by Shionogi Pharmaceutical Co., Ltd.) | 10 parts |

The above ingredients are uniformly mixed and pulverized to form a dry flowable.

### FORMULATION EXAMPLE d-7: Granule

| | |
|---|---|
| Compound No. 9-d of the present invention | 1 part |
| Bentonite | 55 parts |
| Talc | 44 parts |

The above ingredients were homogeneously mixed and pulverized, and after an addition of a small amount of water, the mixture was stirred, mixed and granulated by an extrusion-type granulating machine, followed by drying to obtain a granule.

### FORMULATION EXAMPLE e-1: Wettable powder

| | |
|---|---|
| Compound No. 1-e of the present invention | 20 parts |
| Zeeklite A (tradename for a kaolin-type clay, manufactured by Zeeklite Industries, Co., Ltd.) | 76 parts |
| Sorpol 5039 (tradename for a mixture of a nonionic surfactant and an anionic surfactant, manufactured by Toho Chemical Industry Co., Ltd.) | 2 parts |
| Carplex (tradename for a coagulation-preventing agent composed of a white carbon, manufactured by Shionogi Pharmaceutical Co., Ltd.) | 2 parts |

The above ingredients are homogeneously pulverized and mixed to form a wettable powder.

### FORMULATION EXAMPLE e-2: Wettable powder

| | |
|---|---|
| Compound No. 2-e of the present invention | 40 parts |
| Zeeklite A (tradename for a kaolin-type clay, manufactured by Zeeklite Industries, Co., Ltd.) | 54 parts |
| Sorpol 5039 (tradename for a mixture of a nonionic surfactant and an anionic surfactant, manufactured by Toho Chemical Industry Co., Ltd.) | 2 parts |
| Carplex (tradename for a coagulation-preventing agent composed of a white carbon, manufactured by Shionogi Pharmaceutical Co., Ltd.) | 4 parts |

The above ingredients are homogeneously pulverized and mixed to form a wettable powder.

### FORMULATION EXAMPLE e-3: Emulsifiable concentrate

| | |
|---|---|
| Compound No. 3-e of the present invention | 5 parts |
| Xylene | 75 parts |
| N,N-dimethylformamide | 15 parts |
| Sorpol 2680 (tradename for a mixture of a nonionic surfactant and an anionic surfactant, manufactured by Toho Chemical Industry Co., Ltd.) | 5 parts |

The above ingredients are homogeneously mixed to form an emulsifiable concentrate.

### FORMULATION EXAMPLE e-4: Flowable

| | |
|---|---|
| Compound No. 4-e of the present invention | 25 parts |
| Agrizole S-710 (tradename for a nonionic surfactant, manufactured by Kao Corp.) | 10 parts |
| Runox 1000C (tradename for an anionic surfactant, manufactured by Toho Chemical Industry Co., Ltd.) | 0.5 part |
| 1% Rodopol water (tradename for a thickener, manufactured by Rhone-Poulenc) | 20 parts |
| Water | 44.5 parts |

The above ingredients were homogeneously mixed to obtain a flowable.

### FORMULATION EXAMPLE e-5: Flowable

| | |
|---|---|
| Compound No. 5-e of the present invention | 40 parts |
| Agrizole S-710 (tradename for a nonionic surfactant, manufactured by Kao Corp.) | 10 parts |
| Runox 1000C (tradename for an anionic surfactant, manufactured by Toho Chemical Industry Co., Ltd.) | 0.5 part |
| 1% Rodopol water (tradename for a thickener, manufactured by Rhone-Poulenc) | 20 parts |
| Water | 29.5 parts |

The above ingredients were homogeneously mixed to obtain a flowable.

### FORMULATION EXAMPLE e-6: Granular wettable powder (dry flowable)

| | |
|---|---|
| Compound No. 1-e of the present invention | 75 parts |
| Isoban No. 1 (tradename for an anionic surfactant, manufactured by Kuraray Isoprene Chemical Co., Ltd.) | 10 parts |
| Vanirex N (tradename for an anionic surfactant, manufactured by Sanyo Kokusaku Pulp Co., Ltd.) | 5 parts |
| Carplex #80 (tradename for a white carbon, manufactured by Shionogi Pharmaceutical Co., Ltd.) | 10 parts |

The above ingredients are uniformly mixed and pulverised to form a dry flowable.

### FORMULATION EXAMPLE e-7: Granule

| | |
|---|---|
| Compound No. 5-e of the present invention | 1 part |
| Bentonite | 55 parts |
| Talc | 44 parts |

The above ingredients were homogeneously mixed and pulverized, and after an addition of a small amount of water, the mixture was stirred, mixed and granulated by an extrusion-type granulating machine, followed by drying to obtain a granule.

### FORMULATION EXAMPLE f-1: Wettable powder

| | |
|---|---|
| Compound No. 1-f of the present invention | 20 parts |
| Zeeklite A (tradename for a kaolin-type clay, manufactured by Zeeklite Industries, Co., Ltd.) | 76 parts |
| Sorpol 5039 (tradename for a mixture of a nonionic surfactant and an anionic surfactant, manufactured by Toho Chemical Industry Co., Ltd.) | 2 parts |
| Carplex (tradename for a coagulation-preventing agent composed of a white carbon, manufactured by Shionogi Pharmaceutical Co., Ltd.) | 2 parts |

The above ingredients are homogeneously pulverized and mixed to form a wettable powder.

### FORMULATION EXAMPLE f-2: Wettable powder

| | |
|---|---|
| Compound No. 2-f of the present invention | 40 parts |
| Zeeklite A (tradename for a kaolin-type clay, manufactured by Zeeklite Industries, Co., Ltd.) | 54 parts |
| Sorpol 5039 (tradename for a mixture of a nonionic surfactant and an anionic surfactant, manufactured by Toho Chemical Industry Co., Ltd.) | 2 parts |
| Carplex (tradename for a coagulation-preventing agent composed of a white carbon, manufactured by Shionogi Pharmaceutical Co., Ltd.) | 4 parts |

The above ingredients are homogeneously pulverized and mixed to form a wettable powder.

### FORMULATION EXAMPLE f-3: Emulsifiable concentrate

| | |
|---|---|
| Compound No. 1-f of the present invention | 5 parts |
| Xylene | 75 parts |
| N,N-dimethylformamide | 15 parts |
| Sorpol 2680 (tradename for a mixture of a nonionic surfactant and an anionic surfactant, manufactured by Toho Chemical Industry Co., Ltd.) | 5 parts |

The above ingredients are homogeneously mixed to form an emulsifiable concentrate.

### FORMULATION EXAMPLE f-4: Flowable

| | |
|---|---|
| Compound No. 2-f of the present invention | 25 parts |
| Agrizole S-710 (tradename for a nonionic surfactant, manufactured by Kao Corp.) | 10 parts |
| Runox 1000C (tradename for an anionic surfactant, manufactured by Toho Chemical Industry Co., Ltd.) | 0.5 part |
| 1% Rodopol water (tradename for a thickener, manufactured by Rhone-Poulenc) | 20 parts |
| Water | 44.5 parts |

The above ingredients were homogeneously mixed to obtain a flowable.

### FORMULATION EXAMPLE f-5: Flowable

| | |
|---|---|
| Compound No. 2-f of the present invention | 40 parts |
| Agrizole S-710 (tradename for a nonionic surfactant, manufactured by Kao Corp.) | 10 parts |
| Runox 1000C (tradename for an anionic surfactant, manufactured by Toho Chemical Industry Co., Ltd.) | 0.5 part |
| 1% Rodopol water (tradename for a thickener, manufactured by Rhone-Poulenc) | 20 parts |
| Water | 29.5 parts |

The above ingredients were homogeneously mixed to obtain a flowable.

### FORMULATION EXAMPLE f-6: Granular wettable powder (dry flowable)

| | |
|---|---|
| Compound No. 2-f of the present invention | 75 parts |
| Isoban No. 1 (tradename for an anionic surfactant, manufactured by Kuraray Isoprene Chemical Co., Ltd.) | 10 parts |
| Vanirex N (tradename for an anionic surfactant, manufactured by Sanyo Kokusaku Pulp Co., Ltd.) | 5 parts |
| Carplex #80 (tradename for a white carbon, manufactured by Shionogi Pharmaceutical Co., Ltd.) | 10 parts |

The above ingredients are uniformly mixed and pulverized to form a dry flowable.

### FORMULATION EXAMPLE f-7: Granule

| | |
|---|---|
| Compound No. 2-f of the present invention | 1 part |
| Bentonite | 55 parts |
| Talc | 44 parts |

The above ingredients were homogeneously mixed and pulverized, and after an addition of a small amount of water, the mixture was stirred, mixed and granulated by an extrusion-type granulating machine, followed by drying to obtain a granule.

### FORMULATION EXAMPLE g-1: Wettable powder

| | |
|---|---|
| Compound No. 1-g of the present invention | 20 parts |
| Zeeklite A (tradename for a kaolin-type clay, manufactured by Zeeklite Industries, Co., Ltd.) | 76 parts |
| Sorpol 5039 (tradename for a mixture of a nonionic surfactant and an anionic surfactant, manufactured by Toho Chemical Industry Co., Ltd.) | 2 parts |
| Carplex (tradename for a coagulation-preventing agent composed of a white carbon, manufactured by Shionogi Pharmaceutical Co., Ltd.) | 2 parts |

The above ingredients are homogeneously pulverized and mixed to form a wettable powder.

### FORMULATION EXAMPLE g-2: Wettable powder

| | |
|---|---|
| Compound No. 2-g of the present invention | 40 parts |
| Zeeklite A (tradename for a kaolin-type clay, manufactured by Zeeklite Industries, Co., Ltd.) | 54 parts |
| Sorpol 5039 (tradename for a mixture of a nonionic surfactant and an anionic surfactant, manufactured by Toho Chemical Industry Co., Ltd.) | 2 parts |
| Carplex (tradename for a coagulation-preventing agent composed of a white carbon, manufactured by Shionogi Pharmaceutical Co., Ltd.) | 4 parts |

The above ingredients are homogeneously pulverized and mixed to form a wettable powder.

### FORMULATION EXAMPLE g-3: Emulsifiable concentrate

| | |
|---|---|
| Compound No. 3-g of the present invention | 5 parts |
| Xylene | 75 parts |
| N,N-dimethylformamide | 15 parts |
| Sorpol 2680 (tradename for a mixture of a nonionic surfactant and an anionic surfactant, manufactured by Toho Chemical Industry Co., Ltd.) | 5 parts |

The above ingredients are homogeneously mixed to form an emulsifiable concentrate.

### FORMULATION EXAMPLE g-4: Flowable

| | |
|---|---|
| Compound No. 4-g of the present invention | 25 parts |
| Agrizole S-710 (tradename for a nonionic surfactant, manufactured by Kao Corp.) | 10 parts |
| Runox 1000C (tradename for an anionic surfactant, manufacture by Toho Chemical Industry Co., Ltd.) | 0.5 part |
| 1% Rodopol water (tradename for a thickener, manufactured by Rhone-Poulenc) | 20 parts |
| Water | 44.5 parts |

The above ingredients were homogeneously mixed to obtain a flowable.

### FORMULATION EXAMPLE g-5: Flowable

| | |
|---|---|
| Compound No. 5-g of the present invention | 40 parts |
| Agrizole S-710 (tradename for a nonionic surfactant, manufactured by Kao Corp.) | 10 parts |
| Runox 1000C (tradename for an anionic surfactant, manufactured by Toho Chemical Industry Co., Ltd.) | 0.5 part |
| 1% Rodopol water (tradename for a thickener, manufactured by Rhone-Poulenc) | 20 parts |
| Water | 29.5 parts |

The above ingredients were homogeneously mixed to obtain a flowable.

### FORMULATION EXAMPLE g-6: Granular wettable powder (dry flowable)

| | |
|---|---|
| Compound No. 12-g of the present invention | 75 parts |
| Isoban No. 1 (tradename for an anionic surfactant, manufactured by Kuraray Isoprene Chemical Co., Ltd.) | 10 parts |
| Vanirex N (tradename for an anionic surfactant, manufactured by Sanyo Kokusaku Pulp Co., Ltd.) | 5 parts |
| Carplex #80 (tradename for a white carbon, manufactured by Shionogi Pharmaceutical Co., Ltd.) | 10 parts |

The above ingredients are uniformly mixed and pulverized to form a dry flowable.

### FORMULATION EXAMPLE g-7: Granule

| | |
|---|---|
| Compound No. 19-g of the present invention | 1 part |
| Bentonite | 55 parts |
| Talc | 44 parts |

The above ingredients were homogeneously mixed and pulverized, and after an addition of a small amount of water, the mixture was stirred, mixed and granulated by an extrusion-type granulating machine, followed by drying to obtain a granule.

In use, the above wettable powder, emulsifiable concentrate, flowable or granular wettable powder is diluted with water from 50 to 1,000 times and applied so that the active ingredient will be from 0.0001 to 10 kg per hectare (ha).

Now, the herbicidal activities of the compounds of the present invention will be described in detail with reference to the following Test Examples.

### TEST EXAMPLE 1: Test-1 on the herbicidal effects in soil treatment

A plastic box having a length of 15 cm, a width of 22 cm and a depth of 6 cm was filled with a sterilized diluvial soil, and seeds of Echinochloa crus-galli, Digitaria adscendens, Cyperus microiria, Solanum nigrum, Galinsoga ciliata, Rorippa indica, Oryza sativa, Zea mays, Triticum aestivum, Glycine max and Gossypium spp. were sown, and the soil was covered thereon in a thickness of about 1.5 cm, and then a herbicide solution was applied onto the surface of the soil uniformly so that the active ingredient was distributed at a predetermined concentration. The herbicide solution was prepared by diluting a wettable powder prepared in accordance with the foregoing Formulation Examples with water and applied by a small spray onto the entire soil surface. Four weeks after the application of the herbicidal solution, the herbicidal effects against each weed and the phytotoxicities against each crop plant were determined on the basis of the following standard ratings. The results are shown in Table 15.

### Standard ratings:

- 5:: Growth control rate of more than 90% (almost completely withered)
- 4:: Growth control rate of from 70 to 90%
- 3:: Growth control rate of from 40 to 70%
- 2:: Growth control rate of from 20 to 40%
- 1:: Growth control rate of from 5 to 20%
- 0:: Growth control rate of less than 5% (almost non-effective)

The above growth control rates were calculated by the following equation:$\text{Growth control rate (%) = (1 -} \frac{\text{T}}{\text{N}} \text{) x 100}$ where
- T:: Weight of the weed grown above the soil surface of the treated area
- N:: Weight of the weed grown above the soil surface of the non-treated area

### TEST EXAMPLE 2: Test-1 on the herbicidal effects in foliage treatment

A plastic box having a length of 15 cm, a width of 22 cm and a depth of 6 cm was filled with a sterilized diluvial soil, and seeds of Echinochloa crus-galli, Digitaria adscendens, Avena fatua, Cyperus microiria, Solanum nigrum, Galinsoga ciliata, Rorippa indica, Oryza sativa, Zea mays, Triticum aestivum, Glycine max, Gossypium spp. and Beta vulgaris were spot-wisely sown, and the soil was covered thereon in a thickness of about 1.5 cm. When the various weeds and crop plants grew to the 2 or 3 leaf stage, a herbicidal solution was uniformly sprayed on the foliages so that the active ingredient was applied in a predetermined concentration. The herbicidal solution was prepared by diluting a wettable powder prepared in accordance with the above Formulation Examples with water and applied onto the entire surface of the foliages of the weeds and the crop plants by a small spray. Four weeks after the application of the herbicide solution, the herbicidal effects against each weed and the phytotoxicities against each crop plant were determined on the basis of the standard ratings described in Test Example 1. The results are shown in Table 16.

### TEST EXAMPLE 3: Test-1 on the herbicidal effects in irrigation treatment

Into a Wagner pot of 1/5000a, alluvial soil was put, and then water was introduced and mixed to form an irrigated state with a water depth of 4 cm. Seeds of Echinochloa crus-galli, Scirpus juncoides, Monochoria vaginalis and Rotala indica were sown in the above pot, aid tubers of Sagittaria pygmaea and Cyperus serotinus were embedded. Then, rice seedlings of 2.5 leaf stage were transplanted. The pot was placed in a greenhouse at a temperature of from 25 to 30°C, and the plants were cultured. On the third day after the seeding and plantation, a diluted solution of the herbicide was dropwise applied to the water surface by a measuring pipette, so that the dose would be a predetermined level. Three weeks after the dropwise application of the herbicide, the herbicidal effects against various weeds and rice were determined on the basis of the standard ratings described in Test Example 1. The results are shown in Table 17.

In Tables 15, 16 and 17, Compound Nos. correspond to Compound Nos. in the Examples, and symbols have the following meanings.
- A:: Echinochloa crus-galli (barnyardgrass)
- B:: Digitaria adscendens (large crabgrass)
- C:: Avena fatua (wild oat)
- D:: Cyperus microiria (annual sedge)
- E:: Solanum nigrum (black nightshade)
- F:: Galinsoga ciliata (hairy galinsoga)
- G:: Rorippa indica (fieldcress)
- H:: Scirpus juncoides (bulrush)
- I:: Monochoria vaginalis (ducksalad)
- J:: Rotala indica (toothcup)
- K:: Sagittaria pygmaea (arrowhead)
- L:: Cyperus serctinus (flat sedge)
- a:: Oryza sativa (rice)
- b:: Zea mays (corn)
- c:: Triticum aestivum (wheat)
- d:: Glycine max (soybean)
- e:: Gossypium spp. (cotton)
- f:: Beta vulgaris (sugar beet)

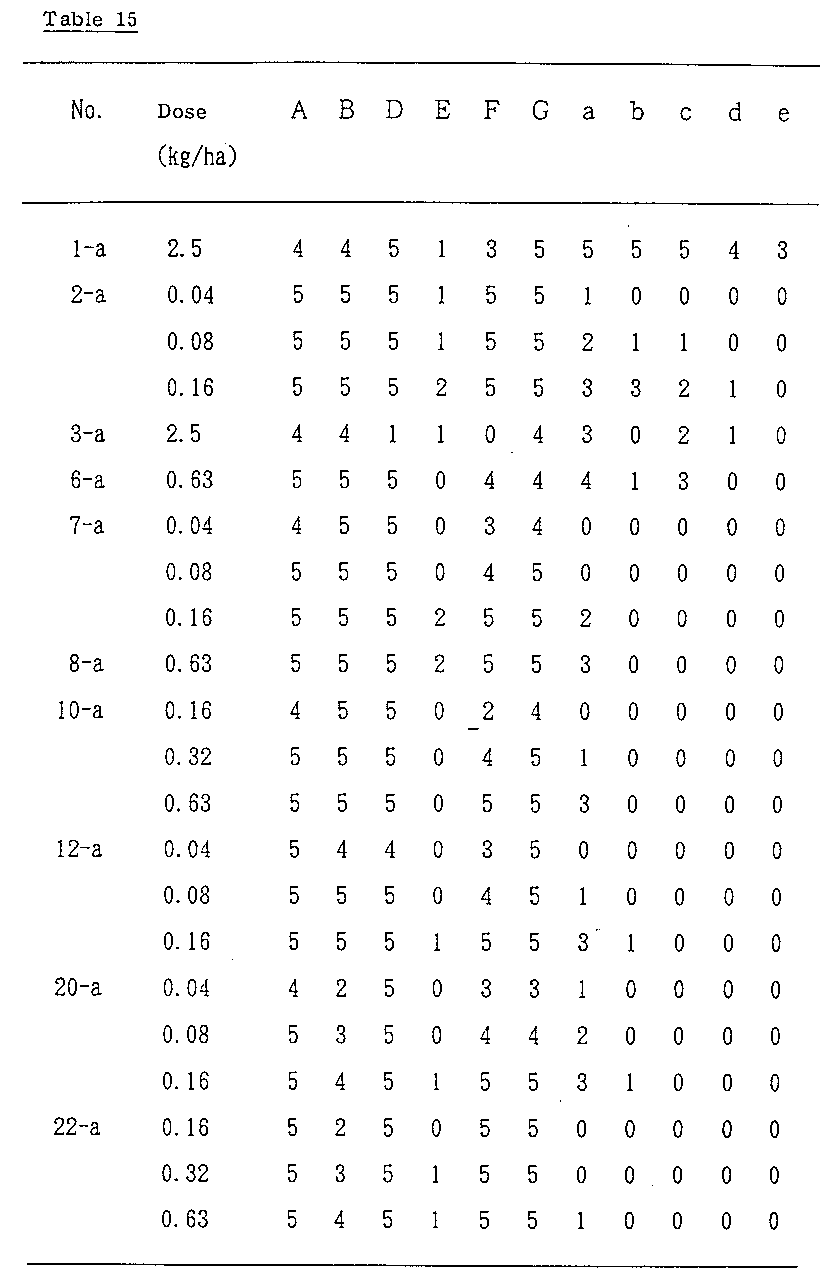

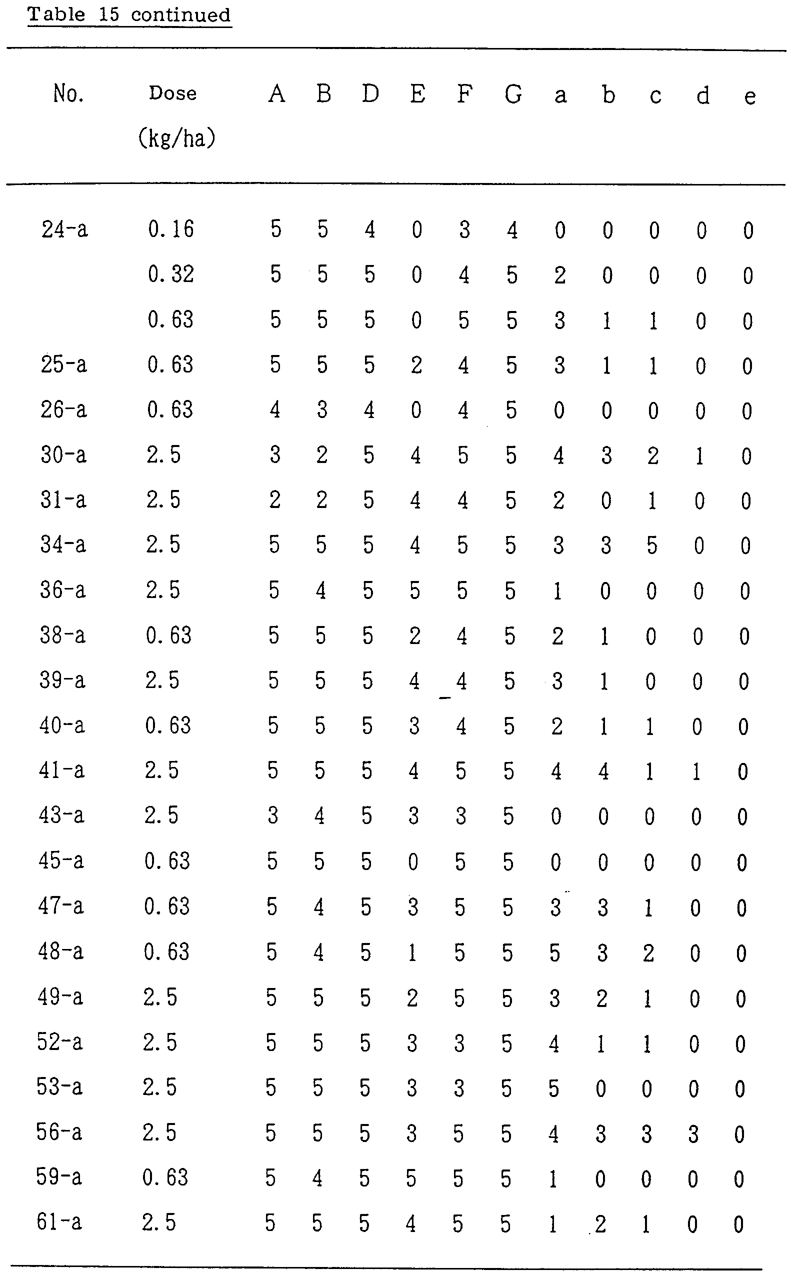

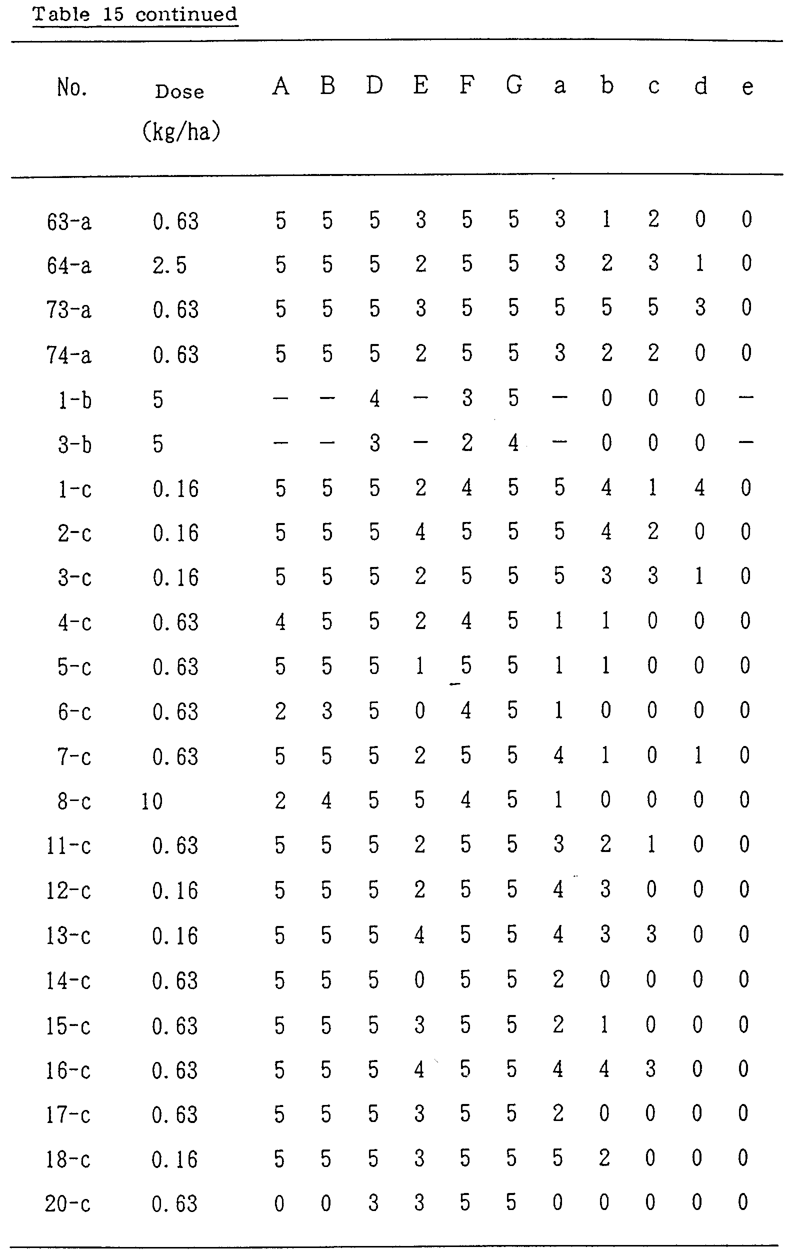

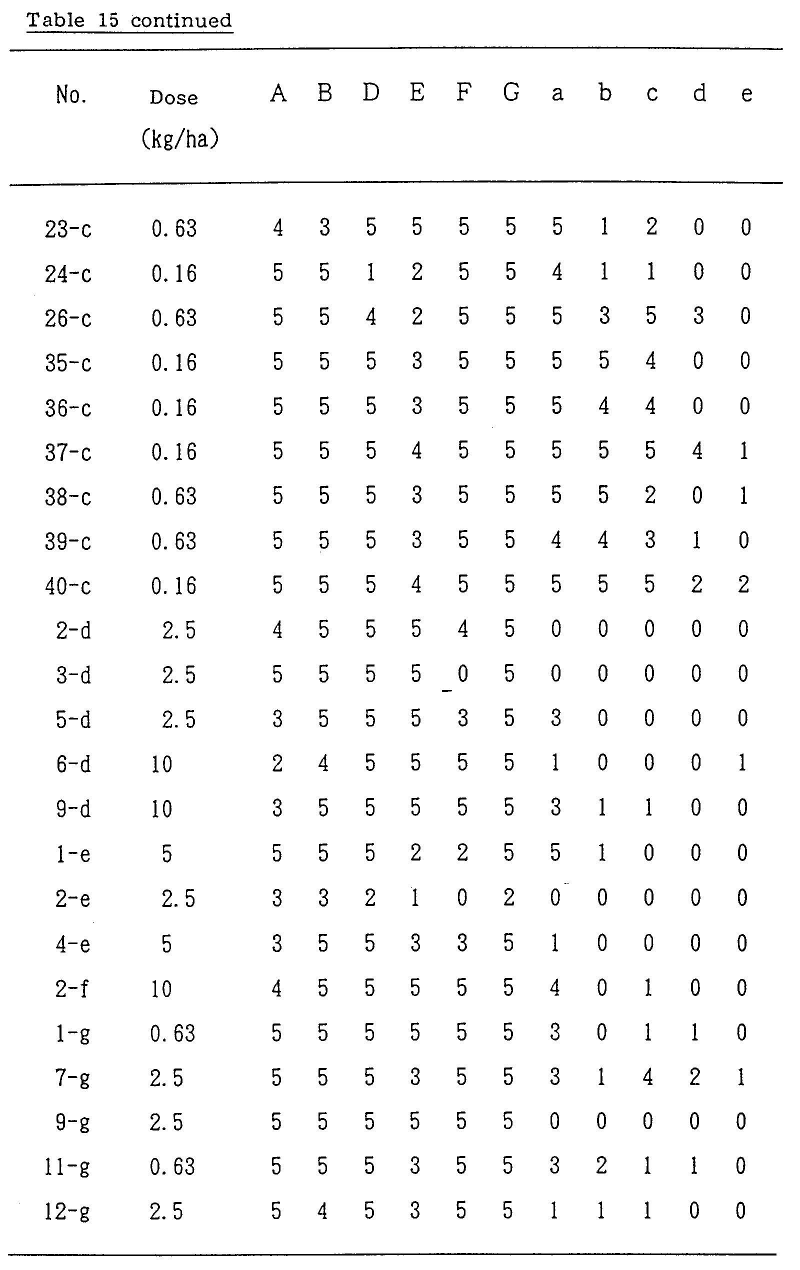

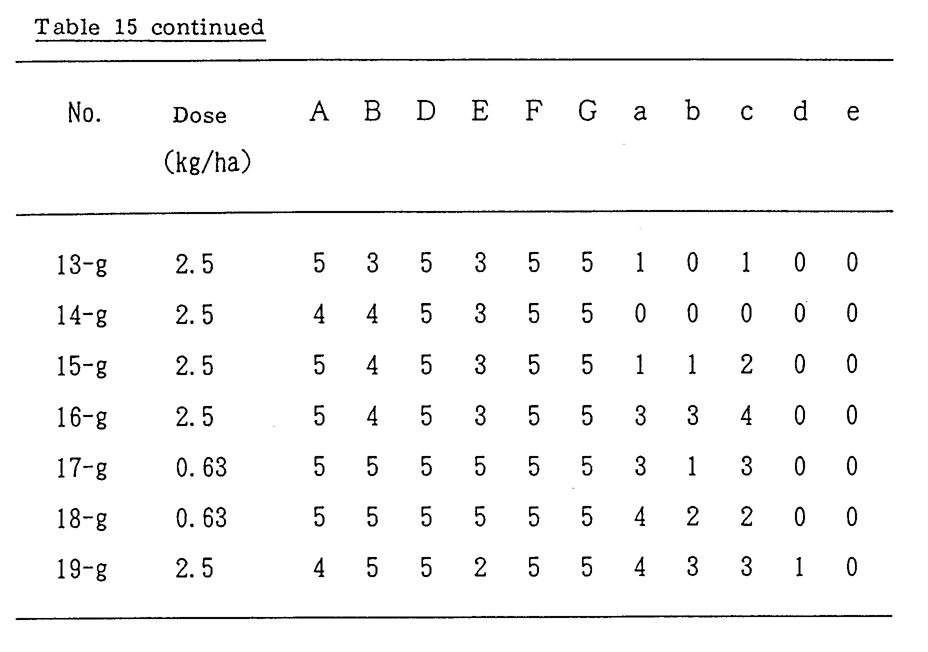

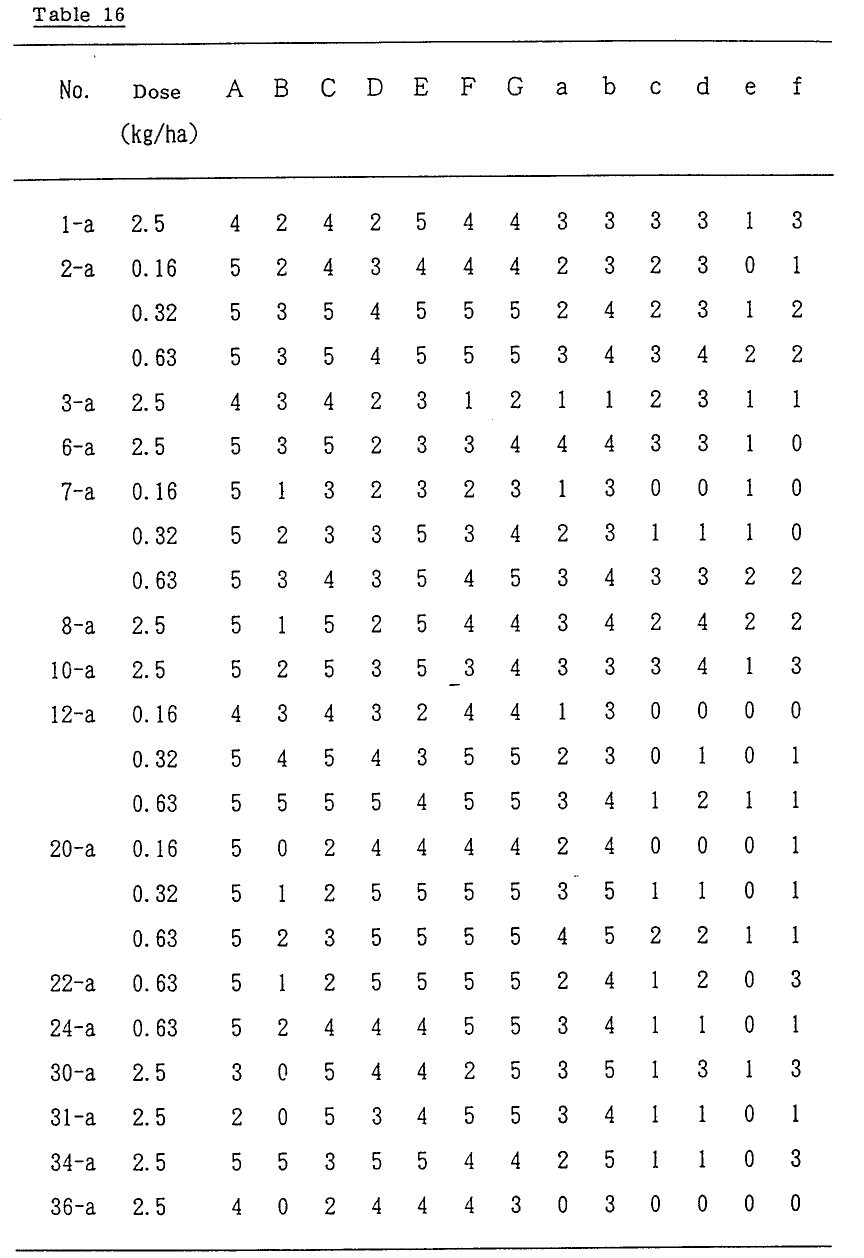

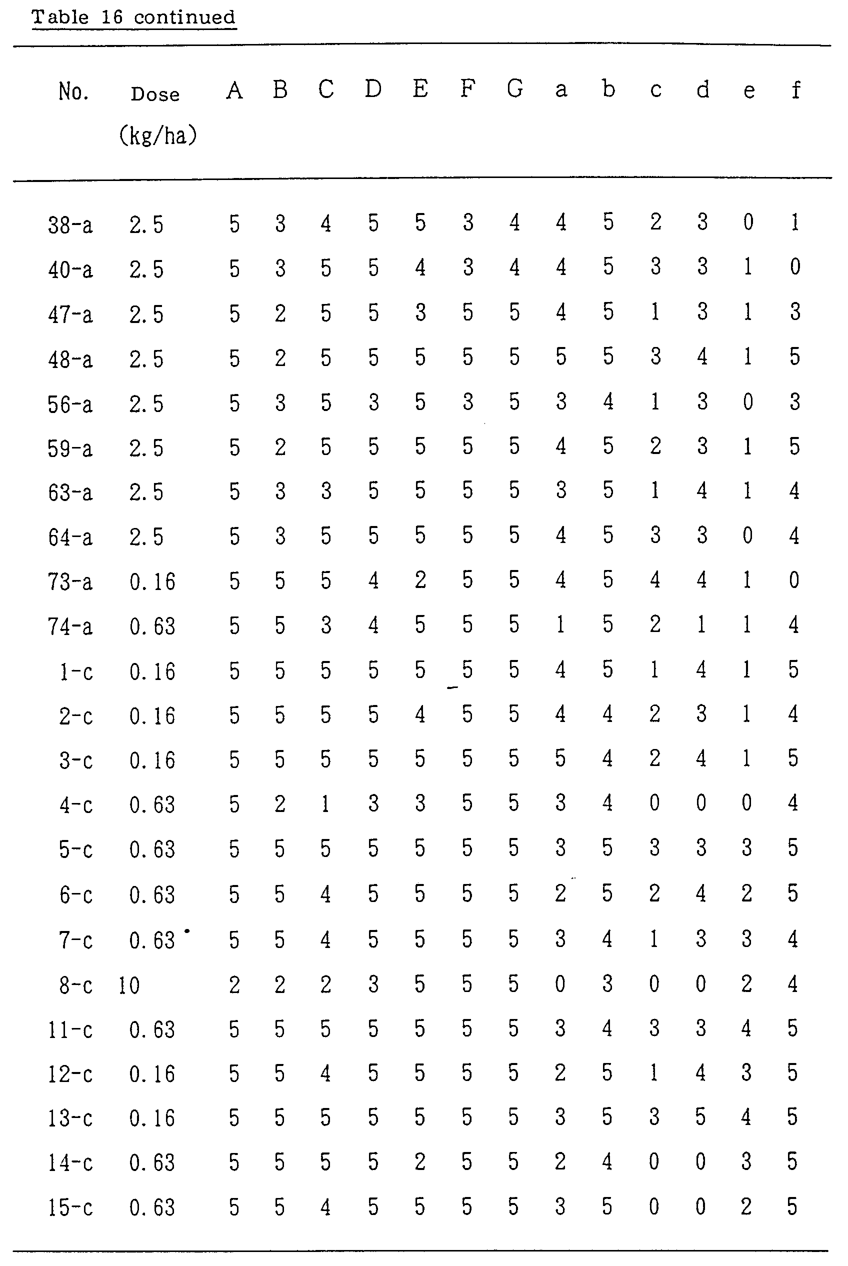

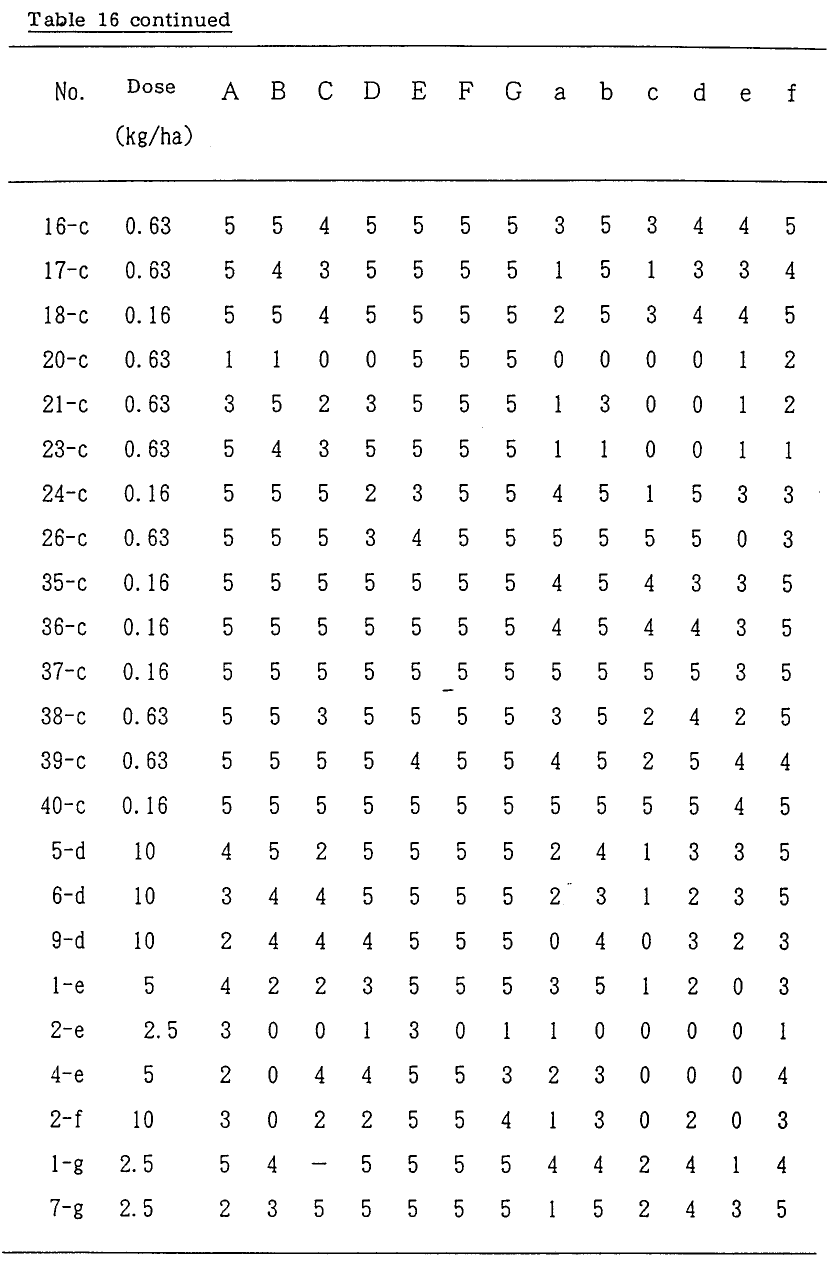

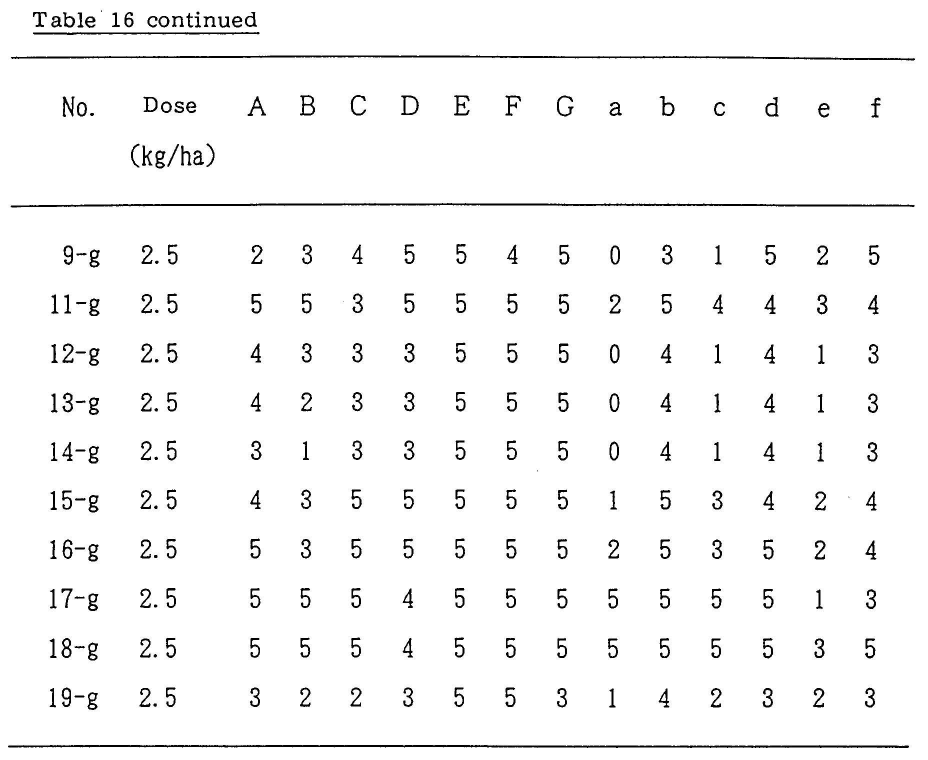

**Table 17**

| No. | Dose (kg/ha) | A | H | I | J | K | L | a |
|---|---|---|---|---|---|---|---|---|
| 74-a | 2.5 | 5 | 4 | 4 | 4 | 5 | 4 | 0 |
| 1-b | 4 | 4 | - | 5 | 5 | 5 | - | 0 |
| 1-c | 0.64 | 5 | 5 | 4 | 5 | 5 | 5 | 0 |
| 7-c | 2.5 | 5 | 0 | 4 | 4 | 4 | 4 | 0 |
| 13-c | 0.64 | 5 | 4 | 5 | 4 | 5 | 5 | 0 |
| 50-c | 0.64 | 5 | 4 | 5 | 5 | 5 | 4 | 0 |
| 53-c | 0.16 | 5 | 0 | 4 | 5 | 4 | 2 | 0 |
| 54-c | 0.16 | 5 | 2 | 4 | 4 | 5 | 5 | 0 |

### TEST EXAMPLE 4: Test-2 on the herbicidal effects in soil treatment

A plastic box having a length of 21 cm, a width of 13 cm and a depth of 7 cm was filled with a sterilized diluvial soil, and seeds of Echinochloa crus-galli, Setaria viridis, Avena fatua, Alopecurus myosuroides, Abutilon theophrasti, Xanthium strumarium, Amaranthus viridis, Ipomoea spp., Veronica persica, Stellaria media, Zea mays, Oryza sativa, Oryza sativa, Glycine max, Gossypium spp., Triticum aestivum and Beta vulgaris were spot-wisely sown, and the soil was covered thereon in a thickness of about 1.5 cm, and then a herbicide solution was applied onto the surface of the soil uniformly so that the active ingredient was distributed at a predetermined concentration. The herbicide solution was prepared by diluting a wettable powder prepared in accordance with the foregoing Formulation Examples with water and applied onto the entire soil surface by a small spray. Three weeks after the application of the herbicidal solution, the herbicidal effects against each weed and the phytotoxicities against each crop plant were visually determined on the basis of the following standard ratings. The results are shown in Table 18.

Some of the compounds of the present invention show selectivity for certain crop plants.

### Standard ratings:

- 5:: Growth control rate of more than 90% (almost completely withered)
- 4:: Growth control rate of from 70 to 90%
- 3:: Growth control rate of from 40 to 70%
- 2:: Growth control rate of from 20 to 40%
- 1:: Growth control rate of from 5 to 20%
- 0:: Growth control rate of less than 5% (almost non-effective)

### TEST EXAMPLE 5: Test-2 on the herbicidal effects in foliage treatment

A plastic box having a length of 21 cm, a width of 13 cm and a depth of 7 cm was filled with a sterilized diluvial soil, and seeds of Echinochloa crus-galli, Setaria viridis, Avena fatua, Alopecurus myosuroides, Abutilon theophrasti, Xanthium strumarium, Amaranthus viridis, Ipomoea spp., Veronica persica, Stellaria media, Zea mays, Oryza sativa, Glycine max, Gossypium spp., Triticum aestivum and Beta vulgaris were spot-wisely sown and the soil was covered thereon in a thickness of about 1.5 cm. When the various weeds and crop plants grew to the 2 or 3 leaf stage, a herbicidal solution was uniformly sprayed on the foliages so that the active ingredient was applied in a predetermined concentration. The herbicidal solution was prepared by diluting a wettable powder prepared in accordance with the above Formulation Examples with water and applied onto the entire surface of the foliages of the weeds and the crop plants by a small spray. Three weeks after the application of the herbicide solution, the herbicidal effects against each weed and the phytotoxicities against each crop plant were visually determined on the basis of the standard ratings described in Test Example 4. The results are shown in Table 19.

### TEST EXAMPLE 6: Test-2 on the herbicidal effects during the growing stage in irrigation treatment

Into a Wagner pot of 1/5000a, alluvial soil was put, and then water was introduced and mixed to form an irrigated state with a water depth of 4 cm. Seeds of Echinochloa crus-galli, Scirpus juncoides, Monochoria vaginalis and Rotala indica were sown in the above pot. The pot was placed in a greenhouse at a temperature of from 25 to 30°C, and the plants were cultured. When Echinochloa crus-galli, Scirpus juncoides, Monochoria vaginalis and Rotala indica reached 1 to 2 leaf stage, a diluted solution of the herbicide was dropwise applied to the water surface by a measuring pipette, so that the dose would be a predetermined level. Three weeks after the dropwise application of the herbicide, the herbicidal effects to various weeds were visually determined on the basis of the standard ratings described in Test Example 4. The results are shown in Table 20.

In Tables 18, 19 and 20, Nos. correspond to Compound Nos. in the Examples, and symbols have the following meanings.
- A:: Echinochloa crus-galli (barnyardgrass)
- B:: Setaria viridis (green foxtail)
- C:: Avena fatua (wild oat)
- D:: Alopecurus myosuroides (black grass)
- E:: Abutilon theophrasti (velvetleaf)
- F:: Xanthium strumarium (common cocklebur)
- G:: Amaranthus viridis (slender amaranth)
- H:: Ipomoea spp. (mornigglory)
- I:: Veronica persica (Persian speedwell)
- J:: Stellaria media (common chickweed)
- a:: Zea mays (corn)
- b:: Oryza sativa (rice)
- c:: Glycine max (soybean)
- d:: Gossypium spp. (cotton)
- e:: Triticum aestivum (wheat)
- f:: Beta vulgaris (sugar beet)
- K:: Scirpus juncoides
- L:: Monochoria vaginalis
- M:: Rotala indica

**Table 18**

| No. | Dose (kg/ha) | A | B | C | D | E | F | G | H | I | J | a | b | c | d | e | f |
|---|---|---|---|---|---|---|---|---|---|---|---|---|---|---|---|---|---|
| 41-c | 0.16 | 5 | 5 | 3 | 5 | 5 | 5 | 5 | 4 | 2 | 5 | 4 | 5 | 3 | 0 | 3 | 4 |
| 43-c | 0.63 | 5 | 5 | 3 | 5 | 3 | 1 | 5 | 3 | 5 | 5 | 5 | 5 | 1 | 0 | 4 | 4 |
| 44-c | 0.63 | 5 | 5 | 5 | 5 | 5 | 3 | 5 | 5 | 5 | 5 | 5 | 5 | 4 | 0 | 4 | 4 |
| 49-c | 0.63 | 5 | 5 | 2 | 5 | 5 | 2 | 5 | 5 | 5 | 5 | 5 | 5 | 0 | 2 | 2 | 5 |
| 50-c | 0.63 | 5 | 5 | 4 | 5 | 5 | 2 | 5 | 5 | 5 | 5 | 5 | 5 | 1 | 1 | 3 | 5 |
| 51-c | 0.16 | 5 | 5 | 4 | 5 | 5 | 5 | 5 | 5 | 5 | 5 | 5 | 5 | 3 | 1 | 2 | 4 |
| 52-c | 0.63 | 5 | 5 | 5 | 5 | 2 | 5 | 5 | 5 | 5 | 5 | 5 | 5 | 3 | 3 | 4 | 4 |
| 53-c | 0.16 | 5 | 5 | 5 | 5 | 2 | 5 | 5 | 5 | 5 | 5 | 4 | 5 | 0 | 0 | 4 | 5 |
| 54-c | 0.63 | 5 | 5 | 5 | 5 | 5 | 5 | 5 | 5 | 5 | 5 | 5 | 5 | 3 | 1 | 4 | 5 |
| 55-c | 0.63 | 5 | 5 | 5 | 5 | 4 | 5 | 5 | 5 | 5 | 5 | 5 | 5 | 1 | 0 | 5 | 5 |
| 56-c | 0.63 | 5 | 5 | 5 | 5 | 5 | 5 | 5 | 5 | 5 | 5 | 5 | 5 | 3 | 1 | 5 | 5 |
| 20-g | 0.63 | 5 | 5 | 3 | 5 | 5 | 2 | 5 | 3 | 5 | 5 | 4 | 4 | 2 | 0 | 2 | 5 |

**Table 19**

| No. | Dose (kg/ha) | A | B | C | D | E | F | G | H | I | J | a | b | c | d | e | f |
|---|---|---|---|---|---|---|---|---|---|---|---|---|---|---|---|---|---|
| 41-c | 0.16 | 5 | 5 | 4 | 5 | 5 | 5 | 5 | 5 | 1 | 5 | 5 | 3 | 4 | 2 | 2 | 5 |
| 43-c | 0.63 | 5 | 5 | 4 | 5 | 5 | 5 | 5 | 3 | 2 | 5 | 5 | 3 | 0 | 1 | 2 | 5 |
| 44-c | 0.63 | 5 | 5 | 5 | 5 | 5 | 5 | 5 | 5 | 3 | 5 | 5 | 4 | 4 | 3 | 3 | 5 |
| 49-c | 0.63 | 5 | 5 | 2 | 5 | 5 | 5 | 5 | 5 | 5 | 5 | 5 | 2 | 4 | 2 | 1 | 5 |
| 50-c | 0.63 | 5 | 5 | 3 | 5 | 5 | 5 | 5 | 5 | 4 | 5 | 5 | 3 | 4 | 3 | 1 | 5 |
| 51-c | 0.16 | 5 | 5 | 3 | 4 | 5 | 5 | 5 | 5 | 5 | 5 | 5 | 4 | 5 | 1 | 2 | 5 |
| 52-c | 0.63 | 5 | 5 | 5 | 5 | 5 | 5 | 5 | 4 | 2 | 5 | 5 | 4 | 4 | 1 | 4 | 5 |
| 53-c | 0.16 | 5 | 5 | 5 | 5 | 5 | 5 | 5 | 5 | 5 | 5 | 5 | 5 | 5 | 3 | 4 | 5 |
| 54-c | 0.16 | 5 | 5 | 5 | 5 | 5 | 5 | 5 | 5 | 5 | 5 | 5 | 5 | 5 | 3 | 3 | 5 |
| 55-c | 0.16 | 5 | 5 | 5 | 5 | 5 | 5 | 5 | 3 | 5 | 5 | 5 | 5 | 5 | 1 | 5 | 5 |
| 56-c | 0.16 | 5 | 5 | 5 | 5 | 5 | 5 | 5 | 2 | 5 | 5 | 5 | 5 | 5 | 1 | 5 | 5 |
| 20-g | 0.63 | 5 | 5 | 5 | 5 | 5 | 5 | 5 | 4 | 5 | 5 | 5 | 5 | 5 | 3 | 4 | 5 |

**Table 20**

| No. | Dose (kg/ha) | A | K | L | M |
|---|---|---|---|---|---|
| 50-c | 0.64 | 5 | 4 | 4 | 4 |
| 53-c | 0.16 | 5 | 3 | 4 | 2 |
| 54-c | 0.16 | 5 | 4 | 4 | 4 |

### INDUSTRIAL APPLICABILITY

Iminosulfonylurea derivatives of the formula (1) of the present invention can be used safely to important crop plants, and they are compounds showing high herbicidal effects against many weeds and thus useful as active ingredients of herbicides.

## Claims (Claims for the following Contracting State(s): AT, BE, CH, DE, DK, FR, GB, IT, LI, LU, MC, NL, SE)

1. An iminosulfonylurea derivative of the formula (1) or its salt: 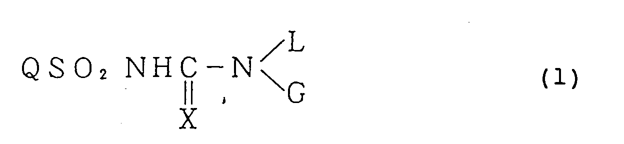
wherein Q is 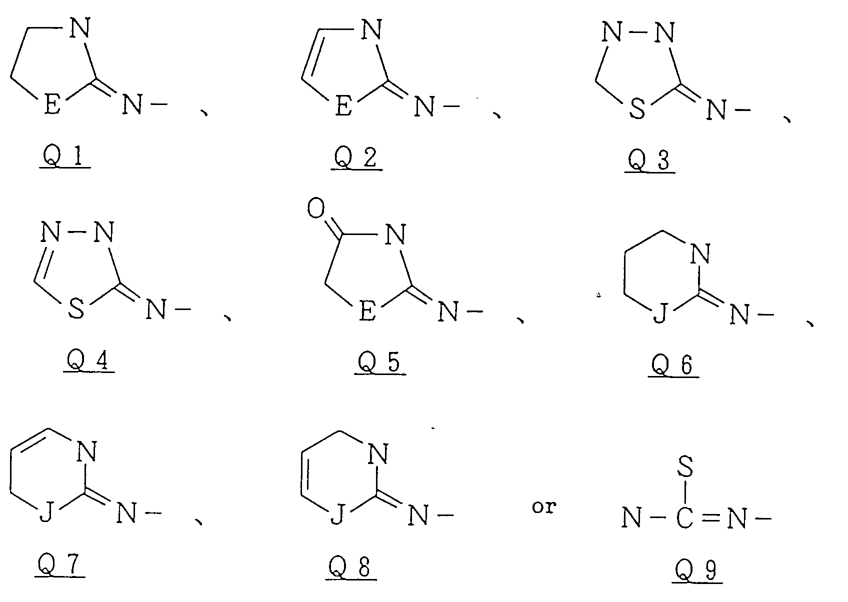 wherein in Q1, Q2 and Q5, E is a sulfur atom, an oxygen atom or a nitrogen atom mono-substituted by a substituent other than a hydrogen atom; in Q6, Q7 and Q8, J is a sulfur atom or an oxygen atom; in Q1 to Q8, a nitrogen atom in the ring of Q is substituted by a substituent other than a hydrogen atom, and a carbon atom in the ring of Q is optionally substituted; and in Q9, the sulfur atom and the nitrogen atom on the carbon atom to which the imino group of Q is bonded, are substituted by substituents other than hydrogen atoms,
X is an oxygen atom or a sulfur atom,
L is a hydrogen atom, a C₁₋₆ alkyl group, a C₂₋₆ alkenyl group or a C₂₋₆ alkynyl group,
G is 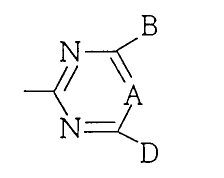
A is a CH group or a nitrogen atom, and
each of B and D which are independent of each other, is a C₁₋₄ alkyl group, a C₁₋₄ alkoxy group, a mono-, di- or poly-halogeno C₁₋₄ alkyl group, a mono-, di- or poly-halogeno C₁₋₄ alkoxy group, a halogen atom, a C₁₋₄ alkylamino group or a di(C₁₋₄ alkyl)amino group.

2. The iminosulfonylurea derivative of the formula (1) and its salt according to Claim 1: 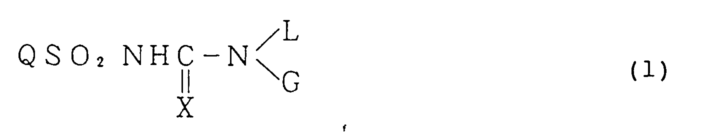
wherein Q is 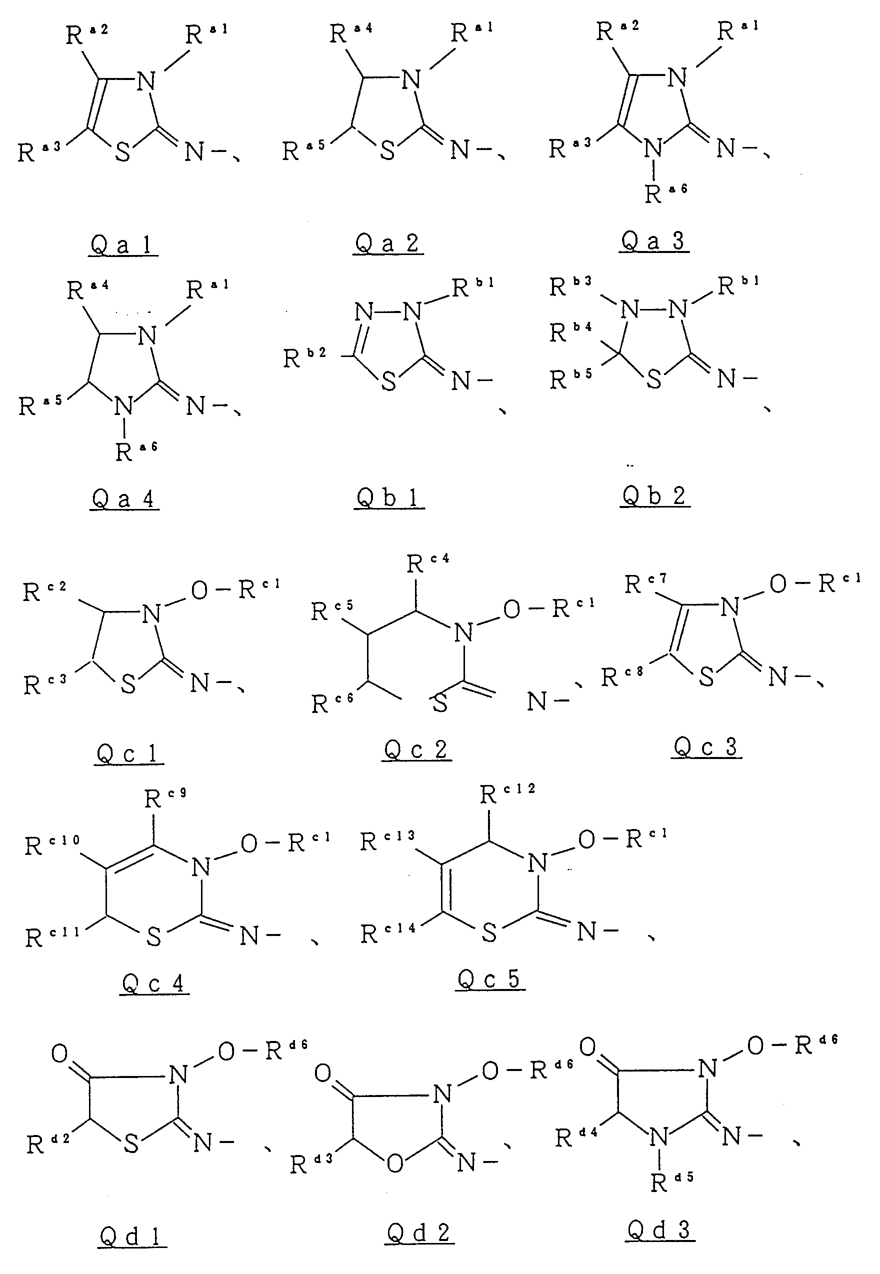 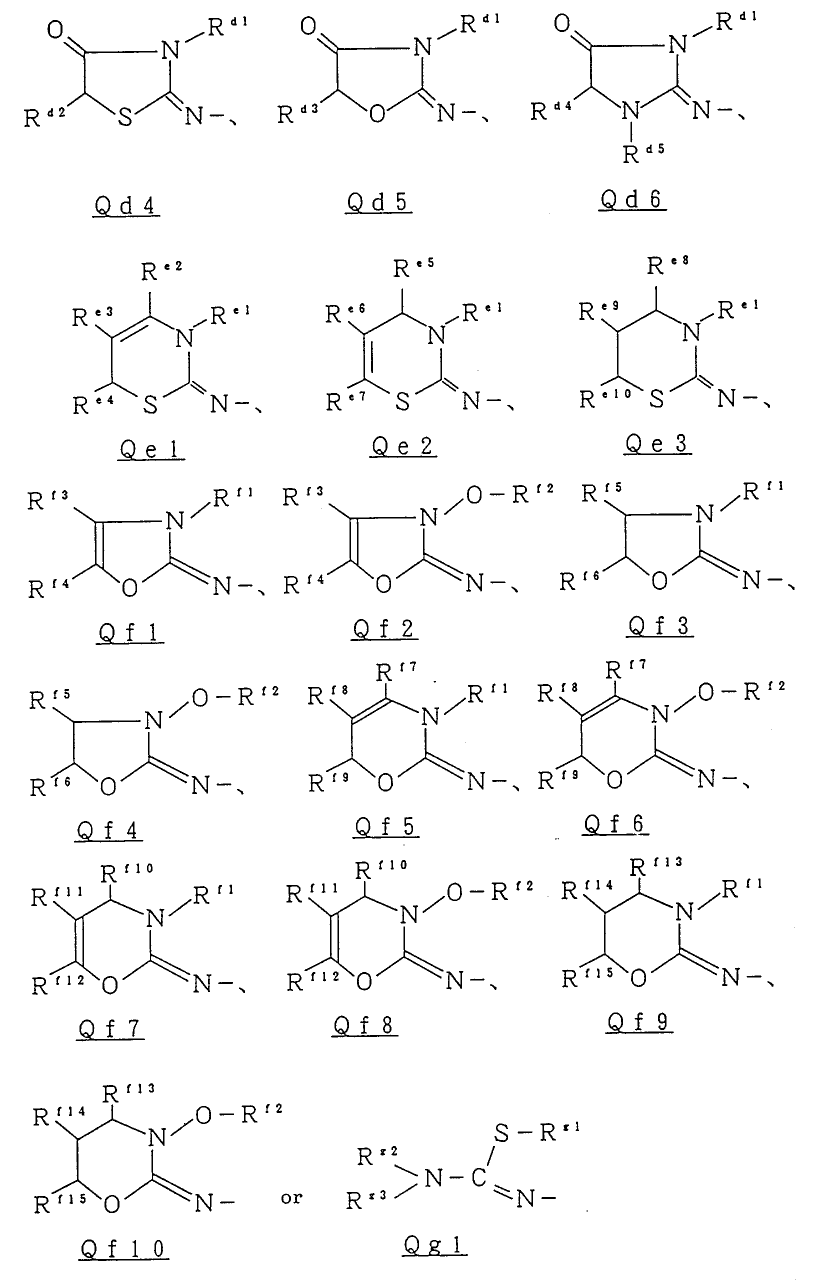
R^{a1} is a C₁₋₈ alkyl group, a C₃₋₇ cycloalkyl group, a C₁₋₆ alkyl group substituted by a C₃₋₇ cycloalkyl group, a C₃₋₇ cycloalkenyl group, a C₁₋₆ alkyl group substituted by a C₃₋₇ cycloalkenyl group, a C₂₋₈ alkenyl group, a C₂₋₈ alkynyl group, a C₁₋₆ alkyl group substituted by a C₁₋₆ alkoxy group, a C₁₋₆ alkyl group substituted by a C₂₋₆ alkenyloxy group, a C₁₋₆ alkyl group substituted by a C₂₋₆ alkynyloxy group, a C₁₋₆ alkyl group substituted by a mono-, di- or poly-halogeno C₁₋₆ alkoxy group, a C₁₋₆ alkyl group substituted by a mono-, di- or poly-halogeno C₂₋₆ alkenyloxy group, a C₁₋₆ alkyl group substituted by a mono-, di- or poly-halogeno C₂₋₆ alkynyloxy group, a C₁₋₆ alkyl group substituted by a C₁₋₆ alkylthio group, a C₁₋₆ alkyl group substituted by a C₁₋₆ alkylsulfinyl group, a C₁₋₆ alkyl group substituted by a C₁₋₆ alkylsulfonyl group, a mono-, di- or poly-halogeno C₁₋₈ alkyl group, a mono-, di- or poly-halogeno C₂₋₈ alkenyl group, a mono-, di- or poly-halogeno C₂₋₈ alkynyl group, a C₁₋₆ alkyl group substituted by a cyano group, a C₂₋₆ alkenyl group substituted by a cyano group, a C₂₋₆ alkynyl group substituted by a cyano group, a C₁₋₆ alkyl group substituted by a nitro group, a C₂₋₆ alkenyl group substituted by a nitro group, a C₂₋₆ alkynyl group substituted by a nitro group, a C₁₋₆ alkyl group substituted by a C₂₋₇ alkoxycarbonyl group, a C₂₋₆ alkenyl group substituted by a C₂₋₇ alkoxycarbonyl group, a C₂₋₆ alkynyl group substituted by a C₂₋₇ alkoxycarbonyl group, a C₁₋₆ alkyl group substituted by a C₂₋₇ alkylcarbonyl group, a C₁₋₆ alkyl group substituted by a mono-, di- or polyhalogeno C₂₋₇ alkylcarbonyl group, a C₁₋₆ alkyl group substituted by a C₃₋₇ alkenylcarbonyl group, a C₁₋₆ alkyl group substituted by a C₃₋₇ alkynylcarbonyl group, a C₁₋₆ alkyl group substituted by a C₂₋₅ alkylcarbonyl group substituted by a C₁₋₄ alkoxy group, a C₁₋₆ alkyl group substituted by a C₂₋₅ alkylcarbonyl group substituted by a C₁₋₄ alkylthio group, a C₁₋₆ alkyl group substituted by a C₂₋₅ alkylcarbonyl group substituted by a C₁₋₄ alkylsulfinyl group, a C₁₋₆ alkyl group substituted by a C₂₋₅ alkylcarbonyl group substituted by a C₁₋₄ alkylsulfonyl group, a C₂₋₆ alkenyl group substituted by a C₂₋₇ alkylcarbonyl group, a C₂₋₆ alkynyl group substituted by a C₂₋₇ alkylcarbonyl group, a C₁₋₆ alkyl group substituted by a C₁₋₆ alkylsulfamoyl group, a C₁₋₆ alkyl group substituted by a C₁₋₆ alkoxysulfamoyl group, a C₁₋₆ alkyl group substituted by a di(C₁₋₃ alkyl)sulfamoyl group, a C₁₋₆ alkyl group substituted by an N-(C₁₋₃ alkyl)-N-(C₁₋₃ alkoxy)sulfamoyl group, a C₁₋₆ alkyl group substituted by a C₂₋₇ alkylcarbamoyl group, a C₁₋₆ alkyl group substituted by a di(C₁₋₃ alkyl)carbamoyl group, a C₁₋₆ alkyl group substituted by a C₂₋₇ alkoxycarbamoyl group, a C₁₋₆ alkyl group substituted by an N-(C₁₋₃ alkyl)-N-(C₁₋₃ alkoxy)carbamoyl group, a C₁₋₆ alkyl group substituted by a C₁₋₆ alkylamino group, a C₁₋₆ alkyl group substituted by a C₁₋₆ alkoxyamino group, a C₁₋₆ alkyl group substituted by a di(C₁₋₃ alkyl)amino group, a C₁₋₆ alkyl group substituted by an N-(C₁₋₃ alkyl)-N-(C₁₋₃ alkoxy)amino group, a C₁₋₆ alkyl group substituted by an N-(C₂₋₇ alkylcarbonyl)-N-(C₁₋₆ alkyl)amino group, a C₁₋₆ alkyl group substituted by an N-(C₂₋₇ alkylcarbonyl)-N-(C₁₋₆ alkoxy)amino group, a C₁₋₆ alkyl group substituted by an N-(C₁₋₆ alkylsulfonyl)-N-(C₁₋₆ alkyl)amino group, a C₁₋₆ alkyl group substituted by an N-(C₁₋₆ alkylsulfonyl)-N-(C₁₋₆ alkoxy)amino group, a phenyl group (provided that such a phenyl group may be substituted by one or more substituents selected from the group consisting of a halogen atom, a trifluoromethyl group, a nitro group, a C₁₋₆ alkyl group, a C₁₋₆ alkoxy group and a C₂₋₇ alkoxycarbonyl group), a C₁₋₆ alkyl group substituted by a phenyl group (provided that such a phenyl group may be substituted by one or more substituents selected from the group consisting of a halogen atom, a trifluoromethyl group, a nitro group, a C₁₋₆ alkyl group, a C₁₋₆ alkoxy group and a, C₂₋₇ alkoxycarbonyl group), a C₂₋₇ alkenyl group substituted by a phenyl group (provided that such a phenyl group may be substituted by one or more substituents selected from the group consisting of a halogen atom, a trifluoromethyl group, a nitro group, a C₁₋₆ alkyl group, a C₁₋₆ alkoxy group and a C₂₋₇ alkoxycarbonyl group), a C₂₋₆ alkynyl group substituted by a phenyl group (provided that such a phenyl group may be substituted by one or more substituents selected from the group consisting of a halogen atom, a trifluoromethyl group, a nitro group, a C₁₋₆ alkyl group, a C₁₋₆ alkoxy group and a C₂₋₇ alkoxycarbonyl group), a C₁₋₆ alkyl group substituted by a phenoxy group (provided that such a phenoxy group may be substituted by one or more substituents selected from the group consisting of a halogen atom, a trifluoromethyl group, a nitro group, a C₁₋₆ alkyl group, a C₁₋₆ alkoxy group and a C₂₋₇ alkoxycarbonyl group), a C₁₋₆ alkyl group substituted by a phenylthio group (provided that such a phenylthio group may be substituted by one or more substituents selected from the group consisting of a halogen atom, a trifluoromethyl group, a nitro group, a C₁₋₆ alkyl group, a C₁₋₆ alkoxy group and a C₂₋₇ alkoxycarbonyl group), a C₁₋₆ alkyl group substituted by a phenylsulfinyl group (provided that such a phenylsulfinyl group may be substituted by one or more substituents selected from the group consisting of a halogen atom, a trifluoromethyl group, a nitro group, a C₁₋₆ alkyl group, a C₁₋₆ alkoxy group and a C₂₋₇ alkoxycarbonyl group), a C₁₋₆ alkyl group substituted by a phenylsulfonyl group (provided that such a phenylsulfonyl group may be substituted by one or more substituents selected from the group consisting of a halogen atom, a trifluoromethyl group, a nitro group, a C₁₋₆ alkyl group, a C₁₋₆ alkoxy group and a C₂₋₇ alkoxycarbonyl group), a C₁₋₆ alkyl group substituted by a benzyloxy group (provided that the phenyl group of such a benzyloxy group may be substituted by one or more substituents selected from the group consisting of a halogen atom, a trifluoromethyl group, a nitro group, a C₁₋₆ alkyl group, a C₁₋₆ alkoxy group and a C₂₋₇ alkoxycarbonyl group), a C₁₋₆ alkyl group substituted by a benzylthio group (provided that the phenyl group of such a benzylthio group may be substituted by one or more substituents selected from the group consisting of a halogen atom, a trifluoromethyl group, a nitro group, a C₁₋₆ alkyl group, a C₁₋₆ alkoxy group and a C₂₋₇ alkoxycarbonyl group), a C₁₋₆ alkyl group substituted by a benzylsulfinyl group (provided that the phenyl group of such a benzylsulfinyl group may be substituted by one or more substituents selected from the group consisting of a halogen atom, a trifluoromethyl group, a nitro group, a C₁₋₆ alkyl group, a C₁₋₆ alkoxy group and a C₂₋₇ alkoxycarbonyl group), a C₁₋₆ alkyl group substituted by a benzylsulfonyl group (provided that the phenyl group of such a benzylsulfonyl group may be substituted by one or more substituents selected from the group consisting of a halogen atom, a trifluoromethyl group, a nitro group, a C₁₋₆ alkyl group, a C₁₋₆ alkoxy group and a C₂₋₇ alkoxycarbonyl group), a C₁₋₆ alkyl group substituted by a phenylcarbonyl group (provided that such a phenylcarbonyl group may be substituted by one or more substituents selected from the group consisting of a halogen atom, a trifluoromethyl group, a nitro group, a C₁₋₆ alkyl group, a C₁₋₆ alkoxy group and a C₂₋₇ alkoxycarbonyl group), a C₁₋₆ alkyl group substituted by a benzylcarbonyl group (provided that the phenyl group of such a benzylcarbonyl group may be substituted by one or more substituents selected from the group consisting of a halogen atom, a trifluoromethyl group, a nitro group, a C₁₋₆ alkyl group, a C₁₋₆ alkoxy group and a C₂₋₇ alkoxycarbonyl group), a C₁₋₆ alkyl group substituted by an amino group substituted by a C₁₋₄ alkylsulfonyl group, or a C₁₋₆ alkyl group substituted by an amino group substituted by a C₂₋₄ alkylcarbonyl group,
each of R^{a2} and R^{a3} which are independent of each other, is a hydrogen atom, a C₁₋₆ alkyl group, a mono-, di- or poly-halogeno C₁₋₆ alkyl group, a C₁₋₆ alkoxy group, a mono-, di or poly-halogeno C₁₋₆ alkoxy group, a C₁₋₆ alkylthio group, a C₁₋₆ alkylsulfinyl group, a C₁₋₆ alkylsulfonyl group, a C₂₋₇ alkoxycarbonyl group, a C₂₋₇ alkylcarbonyl group, a halogen atom, a nitro group, a cyano group, or a phenyl group (provided that such a phenyl group may be substituted by one or more substituents selected from the group consisting of a halogen atom, a trifluoromethyl group, a nitro group, a C₁₋₆ alkyl group, a C₁₋₆ alkoxy group and a C₂₋₇ alkoxycarbonyl group),
each of R^{a4} and R^{a5} which are independent of each other, is a hydrogen atom, a C₁₋₈ alkyl group, a C₂₋₈ alkenyl group, a C₂₋₈ alkynyl group, or a phenyl group (provided that such a phenyl group may be substituted by one or more substituents selected from the group consisting of a halogen atom, a trifluoromethyl group, a nitro group, a C₁₋₆ alkyl group, a C₁₋₆ alkoxy group and a C₂₋₇ alkoxycarbonyl group),
R^{a6} is a C₁₋₈ alkyl group, a C₂₋₈ alkenyl group, a C₂₋₈ alkynyl group or a phenyl group (provided that such a phenyl group may be substituted by one or more substituents selected from the group consisting of a halogen atom, a trifluoromethyl group, a nitro group, a C₁₋₆ alkyl group, a C₁₋₆ alkoxy group and a C₂₋₇ alkoxycarbonyl group),
R^{b1} is a C₁₋₈ alkyl group, a C₃₋₇ cycloalkyl group, a C₁₋₆ alkyl group substituted by a C₃₋₇ cycloalkyl group, a C₃₋₇ cycloalkenyl group, a C₁₋₆ alkyl group substituted by a C₃₋₇ cycloalkenyl group, a C₂₋₈ alkenyl group, a C₂₋₈ alkynyl group, a C₁₋₆ alkyl group substituted by a C₁₋₆ alkoxy group, a C₁₋₆ alkyl group substituted by a C₂₋₆ alkenyloxy group, a C₁₋₆ alkyl group substituted by a C₂₋₆ alkynyloxy group, a C₁₋₆ alkyl group substituted by a mono-, di or poly-halogeno C₁₋₆ alkoxy group, a C₁₋₆ alkyl group substituted by a mono-, di- or poly-halogeno C₂₋₆ alkenyloxy group, a C₁₋₆ alkyl group substituted by a mono-, di- or poly-halogeno C₂₋₆ alkynyloxy group, a C₁₋₆ alkyl group substituted by a C₁₋₆ alkylthio group, a C₁₋₆ alkyl group substituted by a C₁₋₆ alkylsulfinyl group, a C₁₋₆ alkyl group substituted by a C₁₋₆ alkylsulfonyl group, a mono-, di- or poly-halogeno C₁₋₈ alkyl group, a mono-, di- or poly-halogeno C₂₋₈ alkenyl group, a mono-, di- or poly-halogeno C₂₋₈ alkynyl group, a C₁₋₆ alkyl group substituted by a cyano group, a C₂₋₆ alkenyl group substituted by a cyano group, a C₂₋₆ alkynyl group substituted by a cyano group, a C₁₋₆ alkyl group substituted by a nitro group, a C₂₋₆ alkenyl group substituted by a nitro group, a C₂₋₆ alkynyl group substituted by a nitro group, a C₁₋₆ alkyl group substituted by a C₂₋₇ alkoxycarbonyl group, a C₂₋₆ alkenyl group substituted by a C₂₋₇ alkoxycarbonyl group, a C₂₋₆ alkynyl group substituted by a C₂₋₇ alkoxycarbonyl group, a C₁₋₆ alkyl group substituted by a C₂₋₇ alkylcarbonyl group, a C₁₋₆ alkyl group substituted by a mono-, di- or poly-halogeno C₂₋₇ alkylcarbonyl group, a C₁₋₆ alkyl group substituted by a C₃₋₇ alkenylcarbonyl group, a C₁₋₆ alkyl group substituted by a C₃₋₇ alkynylcarbonyl group, a C₁₋₆ alkyl group substituted by a C₂₋₅ alkylcarbonyl group substituted by a C₁₋₄ alkoxy group, a C₁₋₆ alkyl group substituted by a C₂₋₅ alkylcarbonyl group substituted by a C₁₋₄ alkylthio group, a C₁₋₆ alkyl group substituted by a C₂₋₅ alkylcarbonyl group substituted by a C₁₋₄ alkylsulfinyl group, a C₁₋₆ alkyl group substituted by a C₂₋₅ alkylcarbonyl group substituted by a C₁₋₄ alkylsulfonyl group, a C₂₋₆ alkenyl group substituted by a C₂₋₇ alkylcarbonyl group, a C₂₋₆ alkynyl group substituted by a C₂₋₇ alkylcarbonyl group, a C₁₋₆ alkyl group substituted by a C₁₋₆ alkylsulfamoyl group, a C₁₋₆ alkyl group substituted by a C₁₋₆ alkoxysulfamoyl group, a C₁₋₆ alkyl group substituted by a di(C₁₋₃ alkyl)sulfamoyl group, a C₁₋₆ alkyl group substituted by an N-(C₁₋₃ alkyl)-N-(C₁₋₃ alkoxy)sulfamoyl group, a C₁₋₆ alkyl group substituted by a C₂₋₇ alkylcarbamoyl group, a C₁₋₆ alkyl group substituted by a di(C₁₋₃ alkyl)carbamoyl group, a C₁₋₆ alkyl group substituted by a C₂₋₇ alkoxycarbamoyl group, a C₁₋₆ alkyl group substituted by an N-(C₁₋₃ alkyl)-N-(C₁₋₃ alkoxy)carbamoyl group, a C₁₋₆ alkyl group substituted by a C₁₋₆ alkylamino group, a C₁₋₆ alkyl group substituted by a C₁₋₆ alkoxyamino group, a C₁₋₆ alkyl group substituted by a di(C₁₋₃ alkyl)amino group, a C₁₋₆ alkyl group substituted by an N-(C₁₋₃ alkyl)-N-(C₁₋₃ alkoxy)amino group, a C₁₋₆ alkyl group substituted by an N-(C₂₋₇ alkylcarbonyl)-N-(C₁₋₆ alkyl)amino group, a C₁₋₆ alkyl group substituted by an N-(C₂₋₇ alkylcarbonyl)-N(C₁₋₆ alkoxy)amino group, a C₁₋₆ alkyl group substituted by an N-(C₁₋₆ alkylsulfonyl)-N-(C₁₋₆ alkyl)amino group, a C₁₋₆ alkyl group substituted by an N-(C₁₋₆ alkylsulfonyl)-N-(C₁₋₆ alkoxy)amino group, a phenyl group (provided that such a phenyl group may be substituted by one or more substituents selected from the group consisting of a halogen atom, a trifluoromethyl group, a nitro group, a C₁₋₆ alkyl group, a C₁₋₆ alkoxy group and a C₂₋₇ alkoxycarbonyl group), a C₁₋₆ alkyl group substituted by a phenyl group (provided that such a phenyl group may be substituted by one or more substituents selected from the group consisting of a halogen atom, a trifluoromethyl group, a nitro group, a C₁₋₆ alkyl group, a C₁₋₆ alkoxy group and a C₂₋₇ alkoxycarbonyl group), a C₂₋₇ alkenyl group substituted by a phenyl group (provided that such a phenyl group may be substituted by one or more substituents selected from the group consisting of a halogen atom, a trifluoromethyl group, a nitro group, a C₁₋₆ alkyl group, a C₁₋₆ alkoxy group and a C₂₋₇ alkoxycarbonyl group), a C₂₋₆ alkynyl group substituted by a phenyl group (provided that such a phenyl group may be substituted by one or more substituents selected from the group consisting of a halogen atom, a trifluoromethyl group, a nitro group, a C₁₋₆ alkyl group, a C₁₋₆ alkoxy group and a C₂₋₇ alkoxycarbonyl group), a C₁₋₆ alkyl group substituted by a phenoxy group (provided that such a phenoxy group may be substituted by one or more substituents selected from the group consisting of a halogen atom, a trifluoromethyl group, a nitro group, a C₁₋₆ alkyl group, a C₁₋₆ alkoxy group and a C₂₋₇ alkoxycarbonyl group), a C₁₋₆ alkyl group substituted by a phenylthio group (provided that such a phenylthio group may be substituted by one or more substituents selected from the group consisting of a halogen atom, a trifluoromethyl group, a nitro group, a C₁₋₆ alkyl group, a C₁₋₆ alkoxy group and a C₂₋₇ alkoxycarbonyl group), a C₁₋₆ alkyl group substituted by a phenylsulfinyl group (provided that such a phenylsulfinyl group may be substituted by one or more substituents selected from the group consisting of a halogen atom, a trifluoromethyl group, a nitro group, a C₁₋₆ alkyl group, a C₁₋₆ alkoxy group and a C₂₋₇ alkoxycarbonyl group), a C₁₋₆ alkyl group substituted by a phenylsulfonyl group (provided that such a phenylsulfonyl group may be substituted by one or more substituents selected from the group consisting of a halogen atom, a trifluoromethyl group, a nitro group, a C₁₋₆ alkyl group, a C₁₋₆ alkoxy group and a C₂₋₇ alkoxycarbonyl group), a C₁₋₆ alkyl group substituted by a benzyloxy group (provided that the phenyl group of such a benzyloxy group may be substituted by one or more substituents selected from the group consisting of a halogen atom, a trifluoromethyl group, a nitro group, a C₁₋₆ alkyl group, a C₁₋₆ alkoxy group and a C₂₋₇ alkoxycarbonyl group), a C₁₋₆ alkyl group substituted by a benzylthio group (provided that the phenyl group of such a benzylthio group may be substituted by one or more substituents selected from the group consisting of a halogen atom, a trifluoromethyl group, a nitro group, a C₁₋₆ alkyl group, a C₁₋₆ alkoxy group and a C₂₋₇ alkoxycarbonyl group), a C₁₋₆ alkyl group substituted by a benzylsulfinyl group (provided that the phenyl group of such a benzylsulfinyl group may be substituted by one or more substituents selected from the group consisting of a halogen atom, a trifluoromethyl group, a nitro group, a C₁₋₆ alkyl group, a C₁₋₆ alkoxy group and a C₂₋₇ alkoxycarbonyl group), a C₁₋₆ alkyl group substituted by a benzylsulfonyl group (provided that the phenyl group of such a benzylsulfonyl group may be substituted by one or more substituents selected from the group consisting of a halogen atom, a trifluoromethyl group, a nitro group, a C₁₋₆ alkyl group, a C₁₋₆ alkoxy group and a C₂₋₇ alkoxycarbonyl group), a C₁₋₆ alkyl group substituted by a phenylcarbonyl group (provided that such a phenylcarbonyl group may be substituted by one or more substituents selected from the group consisting of a halogen atom, a trifluoromethyl group, a nitro group, a C₁₋₆ alkyl group, a C₁₋₆ alkoxy group and a C₂₋₇ alkoxycarbonyl group), a C₁₋₆ alkyl group substituted by a benzylcarbonyl group (provided that the phenyl group of such a benzylcarbonyl group may be substituted by one or more substituents selected from the group consisting of a halogen atom, a trifluoromethyl group, a nitro group, a C₁₋₆ alkyl group, a C₁₋₆ alkoxy group and a C₂₋₇ alkoxycarbonyl group), a C₁₋₆ alkyl group substituted by an amino group substituted by a C₁₋₄ alkylsulfonyl group, or a C₁₋₆ alkyl group substituted by an amino group substituted by a C₂₋₄ alkylcarbonyl group,
R^{b2} is a hydrogen atom, a C₁₋₆ alkyl group, a mono-, di- or poly-halogeno C₁₋₆ alkyl group, a C₁₋₆ alkoxy group, a mono-, di- or poly-halogeno C₁₋₆ alkoxy group, a C₁₋₆ alkylthio group, a C₁₋₆ alkylsulfinyl group, a C₁₋₆ alkylsulfonyl group, a C₂₋₇ alkoxycarbonyl group, a C₂₋₇ alkylcarbonyl group, a halogen atom, a nitro group, a cyano group, or a phenyl group (provided that such a phenyl group may be substituted by one or more substituents selected from the group consisting of a halogen atom, a trifluoromethyl group, a nitro group, a C₁₋₆ alkyl group, a C₁₋₆ alkoxy group and a C₂₋₇ alkoxycarbonyl group),
R^{b3} is a C₁₋₆ alkyl group, a C₂₋₆ alkenyl group or a C₂₋₆ alkynyl group,
R^{b4} is a hydrogen atom, or a C₁₋₆ alkyl group,
R^{b5} is a hydrogen atom, or a C₁₋₆ alkyl group,
R^{c1} is a C₁₋₈ alkyl group, a C₃₋₇ cycloalkyl group, a C₁₋₆ alkyl group substituted by a C₃₋₇ cycloalkyl group, a C₃₋₇ cycloalkenyl group, a C₁₋₆ alkyl group substituted by a C₃₋₇ cycloalkenyl group, a C₂₋₈ alkenyl group, a C₂₋₈ alkynyl group, a C₁₋₆ alkyl group substituted by a C₁₋₆ alkoxy group, a C₁₋₆ alkyl group substituted by a C₂₋₆ alkenyloxy group, a C₁₋₆ alkyl group substituted by a C₂₋₆ alkynyloxy group, a C₁₋₆ alkyl group substituted by a mono-, di- or polyhalogeno C₁₋₆ alkoxy group, a C₁₋₆ alkyl group substituted by a mono-, di- or poly-halogeno C₂₋₆ alkenyloxy group, a C₁₋₆ alkyl group substituted by a mono-, di- or polyhalogeno C₂₋₆ alkynyloxy group, a C₁₋₆ alkyl group substituted by a C₁₋₆ alkylthio group, a C₁₋₆ alkyl group substituted by a C₁₋₆ alkylsulfinyl group, a C₁₋₆ alkyl group substituted by a C₁₋₆ alkylsulfonyl group, a mono-, di- or poly-halogeno C₁₋₈ alkyl group, a mono-, di- or poly-halogeno C₂₋₈ alkenyl group, a mono-, di- or poly-halogeno C₂₋₈ alkynyl group, a C₁₋₆ alkyl group substituted by a cyano group, a C₂₋₆ alkenyl group substituted by a cyano group, a C₂₋₆ alkynyl group substituted by a cyano group, a C₁₋₆ alkyl group substituted by a nitro group, a C₂₋₆ alkenyl group substituted by a nitro group, a C₂₋₆ alkynyl group substituted by a nitro group, a C₁₋₆ alkyl group substituted by a C₂₋₇ alkoxycarbonyl group, a C₂₋₆ alkenyl group substituted by a C₂₋₇ alkoxycarbonyl group, a C₂₋₆ alkynyl group substituted by a C₂₋₇ alkoxycarbonyl group, a C₁₋₆ alkyl group substituted by a C₂₋₇ alkylcarbonyl group, a C₁₋₆ alkyl group substituted by a mono-, di- or poly-halogeno C₂₋₇ alkylcarbonyl group, a C₁₋₆ alkyl group substituted by a C₃₋₇ alkenylcarbonyl group, a C₁₋₆ alkyl group substituted by a C₃₋₇ alkynylcarbonyl group, a C₁₋₆ alkyl group substituted by a C₂₋₅ alkylcarbonyl group substituted by a C₁₋₄ alkoxy group, a C₁₋₆ alkyl group substituted by a C₂₋₅ alkylcarbonyl group substituted by a C₁₋₄ alkylthio group, a C₁₋₆ alkyl group substituted by a C₂₋₅ alkylcarbonyl group substituted by a C₁₋₄ alkylsulfinyl group, a C₁₋₆ alkyl group substituted by a C₂₋₅ alkylcarbonyl group substituted by a C₁₋₄ alkylsulfonyl group, a C₂₋₆ alkenyl group substituted by a C₂₋₇ alkylcarbonyl group, a C₂₋₆ alkynyl group substituted by a C₂₋₇ alkylcarbonyl group, a C₁₋₆ alkyl group substituted by a C₁₋₆ alkylsulfamoyl group, a C₁₋₆ alkyl group substituted by a C₁₋₆ alkoxysulfamoyl group, a C₁₋₆ alkyl group substituted by a di(C₁₋₃ alkyl)sulfamoyl group, a C₁₋₆ alkyl group substituted by an N-(C₁₋₃ alkyl)-N-(C₁₋₃ alkoxy)sulfamoyl group, a C₁₋₆ alkyl group substituted by a C₂₋₇ alkylcarbamoyl group, a C₁₋₆ alkyl group substituted by a di(C₁₋₃ alkyl)carbamoyl group, a C₁₋₆ alkyl group substituted by a C₂₋₇ alkoxycarbamoyl group, a C₁₋₆ alkyl group substituted by an N-(C₁₋₃ alkyl)-N-(C₁₋₃ alkoxy)carbamoyl group, a C₁₋₆ alkyl group substituted by a C₁₋₆ alkylamino group, a C₁₋₆ alkyl group substituted by a C₁₋₆ alkoxyamino group, a C₁₋₆ alkyl group substituted by a di(C₁₋₃ alkyl)amino group, a C₁₋₆ alkyl group substituted by an N-(C₁₋₃ alkyl)-N-(C₁₋₃ alkoxy)amino group, a C₁₋₆ alkyl group substituted by an N-(C₂₋₇ alkylcarbonyl)-N-(C₁₋₆ alkyl)amino group, a C₁₋₆ alkyl group substituted by an N-(C₂₋₇ alkylcarbonyl)-N-(C₁₋₆ alkoxy)amino group, a C₁₋₆ alkyl group substituted by an N-(C₁₋₆ alkylsulfonyl)-N-(C₁₋₆ alkyl)amino group, a C₁₋₆ alkyl group substituted by an N-(C₁₋₆ alkylsulfonyl)-N-(C₁₋₆ alkoxy)amino group, a phenyl group (provided that such a phenyl group may be substituted by one or more substituents selected from the group consisting of a halogen atom, a trifluoromethyl group, a nitro group, a C₁₋₆ alkyl group, a C₁₋₆ alkoxy group and a C₂₋₇ alkoxycarbonyl group), a C₁₋₆ alkyl group substituted by a phenyl group (provided that such a phenyl group may be substituted by one or more substituents selected from the group consisting of a halogen atom, a trifluoromethyl group, a nitro group, a C₁₋₆ alkyl group, a C₁₋₆ alkoxy group and a C₂₋₇ alkoxycarbonyl group), a C₂₋₇ alkenyl group substituted by a phenyl group (provided that such a phenyl group may be substituted by one or more substituents selected from the group consisting of a halogen atom, a trifluoromethyl group, a nitro group, a C₁₋₆ alkyl group, a C₁₋₆ alkoxy group and a C₂₋₇ alkoxycarbonyl group), a C₂₋₆ alkynyl group substituted by a phenyl group (provided that such a phenyl group may be substituted by one or more substituents selected from the group consisting of a halogen atom, a trifluoromethyl group, a nitro group, a C₁₋₆ alkyl group, a C₁₋₆ alkoxy group and a C₂₋₇ alkoxycarbonyl group), a C₁₋₆ alkyl group substituted by a phenoxy group (provided that such a phenoxy group may be substituted by one or more substituents selected from the group consisting of a halogen atom, a trifluoromethyl group, a nitro group, a C₁₋₆ alkyl group, a C₁₋₆ alkoxy group and a C₂₋₇ alkoxycarbonyl group), a C₁₋₆ alkyl group substituted by a phenylthio group (provided that such a phenylthio group may be substituted by one or more substituents selected from the group consisting of a halogen atom, a trifluoromethyl group, a nitro group, a C₁₋₆ alkyl group, a C₁₋₆ alkoxy group and a C₂₋₇ alkoxycarbonyl group), a C₁₋₆ alkyl group substituted by a phenylsulfinyl group (provided that such a phenylsulfinyl group may be substituted by one or more substituents selected from the group consisting of a halogen atom, a trifluoromethyl group, a nitro group, a C₁₋₆ alkyl group, a C₁₋₆ alkoxy group and a C₂₋₇ alkoxycarbonyl group), a C₁₋₆ alkyl group substituted by a phenylsulfonyl group (provided that such a phenylsulfonyl group may be substituted by one or more substituents selected from the group consisting of a halogen atom, a trifluoromethyl group, a nitro group, a C₁₋₆ alkyl group, a C₁₋₆ alkoxy group and a C₂₋₇ alkoxycarbonyl group), a C₁₋₆ alkyl group substituted by a benzyloxy group (provided that the phenyl group of such a benzyloxy group may be substituted by one or more substituents selected from the group consisting of a halogen atom, a trifluoromethyl group, a nitro group, a C₁₋₆ alkyl group, a C₁₋₆ alkoxy group and a C₂₋₇ alkoxycarbonyl group), a C₁₋₆ alkyl group substituted by a benzylthio group (provided that the phenyl group of such a benzylthio group may be substituted by one or more substituents selected from the group consisting of a halogen atom, a trifluoromethyl group, a nitro group, a C₁₋₆ alkyl group, a C₁₋₆ alkoxy group and a C₂₋₇ alkoxycarbonyl group), a C₁₋₆ alkyl group substituted by a benzylsulfinyl group (provided that the phenyl group of such a benzylsulfinyl group may be substituted by one or more substituents selected from the group consisting of a halogen atom, a trifluoromethyl group, a nitro group, a C₁₋₆ alkyl group, a C₁₋₆ alkoxy group and a C₂₋₇ alkoxycarbonyl group), a C₁₋₆ alkyl group substituted by a benzylsulfonyl group (provided that the phenyl group of such a benzylsulfonyl group may be substituted by one or more substituents selected from the group consisting of a halogen atom, a trifluoromethyl group, a nitro group, a C₁₋₆ alkyl group, a C₁₋₆ alkoxy group and a C₂₋₇ alkoxycarbonyl group), a C₁₋₆ alkyl group substituted by a phenylcarbonyl group (provided that such a phenylcarbonyl group may be substituted by one or more substituents selected from the group consisting of a halogen atom, a trifluoromethyl group, a nitro group, a C₁₋₆ alkyl group, a C₁₋₆ alkoxy group and a C₂₋₇ alkoxycarbonyl group), a C₁₋₆ alkyl group substituted by a benzylcarbonyl group (provided that the phenyl group of such a benzylcarbonyl group may be substituted by one or more substituents selected from the group consisting of a halogen atom, a trifluoromethyl group, a nitro group, a C₁₋₆ alkyl group, a C₁₋₆ alkoxy group and a C₂₋₇ alkoxycarbonyl group), a C₁₋₆ alkyl group substituted by an amino group substituted by a C₁₋₄ alkylsulfonyl group, or a C₁₋₆ alkyl group substituted by an amino group substituted by a C₂₋₄ alkylcarbonyl group,
each of R^{c2}, R^{c3}, R^{c4}, R^{c5}, R^{c6}, R^{c11} and R^{c12} which are independent of one another, is a hydrogen atom, a C₁₋₈ alkyl group, a C₂₋₈ alkenyl group, a C₂₋₈ alkynyl group, or a phenyl group (provided that such a phenyl group may be substituted by one or more substituents selected from the group consisting of a halogen atom, a trifluoromethyl group, a nitro group, a C₁₋₆ alkyl group, a C₁₋₆ alkoxy group and a C₂₋₇ alkoxycarbonyl group),
each of R^{c7}, R^{c8}, R^{c9}, R^{c10}, R^{c13} and R^{c14} which are independent of one another, is a hydrogen atom, a C₁₋₆ alkyl group, a mono-, di- or poly-halogeno C₁₋₆ alkyl group, a C₁₋₆ alkoxy group, a mono, di- or poly-halogeno C₁₋₆ alkoxy group, a C₁₋₆ alkylthio group, a C₁₋₆ alkylsulfinyl group, a C₁₋₆ alkylsulfonyl group, a C₂₋₇ alkoxycarbonyl group, a C₂₋₇ alkylcarbonyl group, a halogen atom, a nitro group, a cyano group, or a phenyl group (provided that such a phenyl group may be substituted by one or more substituents selected from the group consisting of a halogen atom, a trifluoromethyl group, a nitro group, a C₁₋₆ alkyl group, a C₁₋₆ alkoxy group and a C₂₋₇ alkoxycarbonyl group),
R^{d1} is a C₁₋₈ alkyl group, a C₃₋₇ cycloalkyl group, a C₁₋₆ alkyl group substituted by a C₃₋₇ cycloalkyl group, a C₃₋₇ cycloalkenyl group, a C₁₋₆ alkyl group substituted by a C₃₋₇ cycloalkenyl group, a C₂₋₈ alkenyl group, a C₂₋₈ alkynyl group, a C₁₋₆ alkyl group substituted by a C₁₋₆ alkoxy group, a C₁₋₆ alkyl group substituted by a C₂₋₆ alkenyloxy group, a C₁₋₆ alkyl group substituted by a C₂₋₆ alkynyloxy group, a C₁₋₆ alkyl group substituted by a mono-, di or poly-halogeno C₁₋₆ alkoxy group, a C₁₋₆ alkyl group substituted by a mono-, di- or poly-halogeno C₂₋₆ alkenyloxy group, a C₁₋₆ alkyl group substituted by a mono-, di- or poly-halogeno C₂₋₆ alkynyloxy group, a C₁₋₆ alkyl group substituted by a C₁₋₆ alkylthio group, a C₁₋₆ alkyl group substituted by a C₁₋₆ alkylsulfinyl group, a C₁₋₆ alkyl group substituted by a C₁₋₆ alkylsulfonyl group, a mono-, di- or poly-halogeno C₁₋₈ alkyl group, a mono-, di- or poly-halogeno C₂₋₈ alkenyl group, a mono-, di- or poly-halogeno C₂₋₈ alkynyl group, a C₁₋₆ alkyl group substituted by a cyano group, a C₂₋₆ alkenyl group substituted by a cyano group, a C₂₋₆ alkynyl group substituted by a cyano group, a C₁₋₆ alkyl group substituted by a nitro group, a C₂₋₆ alkenyl group substituted by a nitro group, a C₂₋₆ alkynyl group substituted by a nitro group, a C₁₋₆ alkyl group substituted by a C₂₋₇ alkoxycarbonyl group, a C₂₋₆ alkenyl group substituted by a C₂₋₇ alkoxycarbonyl group, a C₂₋₆ alkynyl group substituted by a C₂₋₇ alkoxycarbonyl group, a C₁₋₆ alkyl group substituted by a C₂₋₇ alkylcarbonyl group, a C₁₋₆ alkyl group substituted by a mono-, di- or poly-halogeno C₂₋₇ alkylcarbonyl group, a C₁₋₆ alkyl group substituted by a C₃₋₇ alkenylcarbonyl group, a C₁₋₆ alkyl group substituted by a C₃₋₇ alkynylcarbonyl group, a C₁₋₆ alkyl group substituted by a C₂₋₅ alkylcarbonyl group substituted by a C₁₋₄ alkoxy group, a C₁₋₆ alkyl group substituted by a C₂₋₅ alkylcarbonyl group substituted by a C₁₋₄ alkylthio group, a C₁₋₆ alkyl group substituted by a C₂₋₅ alkylcarbonyl group substituted by a C₁₋₄ alkylsulfinyl group, a C₁₋₆ alkyl group substituted by a C₂₋₅ alkylcarbonyl group substituted by a C₁₋₄ alkylsulfonyl group, a C₂₋₆ alkenyl group substituted by a C₂₋₇ alkylcarbonyl group, a C₂₋₆ alkynyl group substituted by a C₂₋₇ alkylcarbonyl group, a C₁₋₆ alkyl group substituted by a C₁₋₆ alkylsulfamoyl group, a C₁₋₆ alkyl group substituted by a C₁₋₆ alkoxysulfamoyl group, a C₁₋₆ alkyl group substituted by a di(C₁₋₃ alkyl)sulfamoyl group, a C₁₋₆ alkyl group substituted by an N-(C₁₋₃ alkyl)-N-(C₁₋₃ alkoxy)sulfamoyl group, a C₁₋₆ alkyl group substituted by a C₂₋₇ alkylcarbamoyl group, a C₁₋₆ alkyl group substituted by a di(C₁₋₃ alkyl)carbamoyl group, a C₁₋₆ alkyl group substituted by a C₂₋₇ alkoxycarbamoyl group, a C₁₋₆ alkyl group substituted by an N-(C₁₋₃ alkyl)-N-(C₁₋₃ alkoxy)carbamoyl group, a C₁₋₆ alkyl group substituted by a C₁₋₆ alkylamino group, a C₁₋₆ alkyl group substituted by a C₁₋₆ alkoxyamino group, a C₁₋₆ alkyl group substituted by a di(C₁₋₃ alkyl)amino group, a C₁₋₆ alkyl group substituted by an N-(C₁₋₃ alkyl)-N-(C₁₋₃ alkoxy)amino group, a C₁₋₆ alkyl group substituted by an N-(C₂₋₇ alkylcarbonyl)-N-(C₁₋₆ alkyl)amino group, a C₁₋₆ alkyl group substituted by an N-(C₂₋₇ alkylcarbonyl)-N-(C₁₋₆ alkoxy)amino group, a C₁₋₆ alkyl group substituted by an N-(C₁₋₆ alkylsulfonyl)-N-(C₁₋₆ alkyl)amino group, a C₁₋₆ alkyl group substituted by an N-(C₁₋₆ alkylsulfonyl)-N-(C₁₋₆ alkoxy)amino group, a phenyl group (provided that such a phenyl group may be substituted by one or more substituents selected from the group consisting of a halogen atom, a trifluoromethyl group, a nitro group, a C₁₋₆ alkyl group, a C₁₋₆ alkoxy group and a C₂₋₇ alkoxycarbonyl group), a C₁₋₆ alkyl group substituted by a phenyl group (provided that such a phenyl group may be substituted by one or more substituents selected from the group consisting of a halogen atom, a trifluoromethyl group, a nitro group, a C₁₋₆ alkyl group, a C₁₋₆ alkoxy group and a C₂₋₇ alkoxycarbonyl group), a C₂₋₇ alkenyl group substituted by a phenyl group (provided that such a phenyl group may be substituted by one or more substituents selected from the group consisting of a halogen atom, a trifluoromethyl group, a nitro group, a C₁₋₆ alkyl group, a C₁₋₆ alkoxy group and a C₂₋₇ alkoxycarbonyl group), a C₂₋₆ alkynyl group substituted by a phenyl group (provided that such a phenyl group may be substituted by one or more substituents selected from the group consisting of a halogen atom, a trifluoromethyl group, a nitro group, a C₁₋₆ alkyl group, a C₁₋₆ alkoxy group and a C₂₋₇ alkoxycarbonyl group), a C₁₋₆ alkyl group substituted by a phenoxy group (provided that such a phenoxy group may be substituted by one or more substituents selected from the group consisting of a halogen atom, a trifluoromethyl group, a nitro group, a C₁₋₆ alkyl group, a C₁₋₆ alkoxy group and a C₂₋₇ alkoxycarbonyl group), a C₁₋₆ alkyl group substituted by a phenylthio group (provided that such a phenylthio group may be substituted by one or more substituents selected from the group consisting of a halogen atom, a trifluoromethyl group, a nitro group, a C₁₋₆ alkyl group, a C₁₋₆ alkoxy group and a C₂₋₇ alkoxycarbonyl group), a C₁₋₆ alkyl group substituted by a phenylsulfinyl group (provided that such a phenylsulfinyl group may be substituted by one or more substituents selected from the group consisting of a halogen atom, a trifluoromethyl group, a nitro group, a C₁₋₆ alkyl group, a C₁₋₆ alkoxy group and a C₂₋₇ alkoxycarbonyl group), a C₁₋₆ alkyl group substituted by a phenylsulfonyl group (provided that such a phenylsulfonyl group may be substituted by one or more substituents selected from the group consisting of a halogen atom, a trifluoromethyl group, a nitro group, a C₁₋₆ alkyl group, a C₁₋₆ alkoxy group and a C₂₋₇ alkoxycarbonyl group), a C₁₋₆ alkyl group substituted by a benzyloxy group (provided that the phenyl group of such a benzyloxy group may be substituted by one or more substituents selected from the group consisting of a halogen atom, a trifluoromethyl group, a nitro group, a C₁₋₆ alkyl group, a C₁₋₆ alkoxy group and a C₂₋₇ alkoxycarbonyl group), a C₁₋₆ alkyl group substituted by a benzylthio group (provided that the phenyl group of such a benzylthio group may be substituted by one or more substituents selected from the group consisting of a halogen atom, a trifluoromethyl group, a nitro group, a C₁₋₆ alkyl group, a C₁₋₆ alkoxy group and a C₂₋₇ alkoxycarbonyl group), a C₁₋₆ alkyl group substituted by a benzylsulfinyl group (provided that the phenyl group of such a benzylsulfinyl group may be substituted by one or more substituents selected from the group consisting of a halogen atom, a trifluoromethyl group, a nitro group, a C₁₋₆ alkyl group, a C₁₋₆ alkoxy group and a C₂₋₇ alkoxycarbonyl group), a C₁₋₆ alkyl group substituted by a benzylsulfonyl group (provided that the phenyl group of such a benzylsulfonyl group may be substituted by one or more substituents selected from the group consisting of a halogen atom, a trifluoromethyl group, a nitro group, a C₁₋₆ alkyl group, a C₁₋₆ alkoxy group and a C₂₋₇ alkoxycarbonyl group), a C₁₋₆ alkyl group substituted by a phenylcarbonyl group (provided that such a phenylcarbonyl group may be substituted by one or more substituents selected from the group consisting of a halogen atom, a trifluoromethyl group, a nitro group, a C₁₋₆ alkyl group, a C₁₋₆ alkoxy group and a C₂₋₇ alkoxycarbonyl group), a C₁₋₆ alkyl group substituted by a benzylcarbonyl group (provided that the phenyl group of such a benzylcarbonyl group may be substituted by one or more substituents selected from the group consisting of a halogen atom, a trifluoromethyl group, a nitro group, a C₁₋₆ alkyl group, a C₁₋₆ alkoxy group and a C₂₋₇ alkoxycarbonyl group), a C₁₋₆ alkyl group substituted by an amino group substituted by a C₁₋₄ alkylsulfonyl group, or a C₁₋₆ alkyl group substituted by an amino group substituted by a C₂₋₄ alkylcarbonyl group,
each of R^{d2}_{,} R^{d3} and R^{d4} which are independent of one another, is a hydrogen atom, a C₁₋₈ alkyl group, a C₂₋₈ alkenyl group, a C₂₋₈ alkynyl group, or a phenyl group (provided that such a phenyl group may be substituted by one or more substituents selected from the group consisting of a halogen atom, a trifluoromethyl group, a nitro group, a C₁₋₆ alkyl group, a C₁₋₆ alkoxy group and a C₂₋₇ alkoxycarbonyl group),
R^{d5} is a C₁₋₈ alkyl group, a C₂₋₈ alkenyl group, a C₂₋₈ alkynyl group, or a phenyl group (provided that such a phenyl group may be substituted by one or more substituents selected from the group consisting of a halogen atom, a trifluoromethyl group, a nitro group, a C₁₋₆ alkyl group, a C₁₋₆ alkoxy group and a C₂₋₇ alkoxycarbonyl group),
R^{d6} is a C₁₋₈ alkyl group, a C₂₋₈ alkenyl group, a C₂₋₈ alkynyl group, a C₁₋₆ alkyl group substituted by a C₁₋₆ alkylthio group, a C₁₋₆ alkyl group substituted by a C₁₋₆ alkylsulfinyl group, a C₁₋₆ alkyl group substituted by a C₁₋₆ alkylsulfonyl group, a C₁₋₆ alkyl group substituted by a C₁₋₆ alkoxy group, a C₁₋₆ alkyl group substituted by a C₂₋₇ alkoxycarbonyl group, a C₁₋₆ alkyl group substituted by a C₂₋₇ alkylcarbonyl group, a C₁₋₆ alkyl group substituted by a cyano group, a phenyl group (provided that such a phenyl group may be substituted by one or more substituents selected from the group consisting of a halogen atom, a trifluoromethyl group, a nitro group, a C₁₋₆ alkyl group, a C₁₋₆ alkoxy group and a C₂₋₇ alkoxycarbonyl group), or a C₁₋₆ alkyl group substituted by a phenyl group (provided that such a phenyl group may be substituted by one or more substituents selected from the group consisting of a halogen atom, a trifluoromethyl group, a nitro group, a C₁₋₆ alkyl group, a C₁₋₆ alkoxy group and a C₂₋₇ alkoxycarbonyl group),
R^{e1} is a C₁₋₈ alkyl group, a C₃₋₇ cycloalkyl group, a C₁₋₆ alkyl group substituted by a C₃₋₇ cycloalkyl group, a C₃₋₇ cycloalkenyl group, a C₁₋₆ alkyl group substituted by a C₃₋₇ cycloalkenyl group, a C₂₋₈ alkenyl group, a C₂₋₈ alkynyl group, a C₁₋₆ alkyl group substituted by a C₁₋₆ alkoxy group, a C₁₋₆ alkyl group substituted by a C₂₋₆ alkenyloxy group, a C₁₋₆ alkyl group substituted by a C₂₋₆ alkynyloxy group, a C₁₋₆ alkyl group substituted by a mono-, di- or poly-halogeno C₁₋₆ alkoxy group, a C₁₋₆ alkyl group substituted by a mono-, di- or poly-halogeno C₂₋₆ alkenyloxy group, a C₁₋₆ alkyl group substituted by a mono-, di- or poly-halogeno C₂₋₆ alkynyloxy group, a C₁₋₆ alkyl group substituted by a C₁₋₆ alkylthio group, a C₁₋₆ alkyl group substituted by a C₁₋₆ alkylsulfinyl group, a C₁₋₆ alkyl group substituted by a C₁₋₆ alkylsulfonyl group, a mono-, di- or poly-halogeno C₁₋₈ alkyl group, a mono-, di- or poly-halogeno C₂₋₈ alkenyl group, a mono-, di- or poly-halogeno C₂₋₈ alkynyl group, a C₁₋₆ alkyl group substituted by a cyano group, a C₂₋₆ alkenyl group substituted by a cyano group, a C₂₋₆ alkynyl group substituted by a cyano group, a C₁₋₆ alkyl group substituted by a nitro group, a C₂₋₆ alkenyl group substituted by a nitro group, a C₂₋₆ alkynyl group substituted by a nitro group, a C₁₋₆ alkyl group substituted by a C₂₋₇ alkoxycarbonyl group, a C₂₋₆ alkenyl group substituted by a C₂₋₇ alkoxycarbonyl group, a C₂₋₆ alkynyl group substituted by a C₂₋₇ alkoxycarbonyl group, a C₁₋₆ alkyl group substituted by a C₂₋₇ alkylcarbonyl group, a C₁₋₆ alkyl group substituted by a mono-, di- or poly-halogeno C₂₋₇ alkylcarbonyl group, a C₁₋₆ alkyl group substituted by a C₃₋₇ alkenylcarbonyl group, a C₁₋₆ alkyl group substituted by a C₃₋₇ alkynylcarbonyl group, a C₁₋₆ alkyl group substituted by a C₂₋₅ alkylcarbonyl group substituted by a C₁₋₄ alkoxy group, a C₁₋₆ alkyl group substituted by a C₂₋₅ alkylcarbonyl group substituted by a C₁₋₄ alkylthio group, a C₁₋₆ alkyl group substituted by a C₂₋₅ alkylcarbonyl group substituted by a C₁₋₄ alkylsulfinyl group, a C₁₋₆ alkyl group substituted by a C₂₋₅ alkylcarbonyl group substituted by a C₁₋₄ alkylsulfonyl group, a C₂₋₆ alkenyl group substituted by a C₂₋₇ alkylcarbonyl group, a C₂₋₆ alkynyl group substituted by a C₂₋₇ alkylcarbonyl group, a C₁₋₆ alkyl group substituted by a C₁₋₆ alkylsulfamoyl group, a C₁₋₆ alkyl group substituted by a C₁₋₆ alkoxysulfamoyl group, a C₁₋₆ alkyl group substituted by a di(C₁₋₃ alkyl)sulfamoyl group, a C₁₋₆ alkyl group substituted by an N-(C₁₋₃ alkyl)-N-(C₁₋₃ alkoxy)sulfamoyl group, a C₁₋₆ alkyl group substituted by a C₂₋₇ alkylcarbamoyl group, a C₁₋₆ alkyl group substituted by a di(C₁₋₃ alkyl)carbamoyl group, a C₁₋₆ alkyl group substituted by a C₂₋₇ alkoxycarbamoyl group, a C₁₋₆ alkyl group substituted by an N-(C₁₋₃ alkyl)-N-(C₁₋₃ alkoxy)carbamoyl group, a C₁₋₆ alkyl group substituted by a C₁₋₆ alkylamino group, a C₁₋₆ alkyl group substituted by a C₁₋₆ alkoxyamino group, a C₁₋₆ alkyl group substituted by a di(C₁₋₃ alkyl)amino group, a C₁₋₆ alkyl group substituted by an N-(C₁₋₃ alkyl)-N-(C₁₋₃ alkoxy)amino group, a C₁₋₆ alkyl group substituted by an N-(C₂₋₇ alkylcarbonyl)-N-(C₁₋₆ alkyl)amino group, a C₁₋₆ alkyl group substituted by an N-(C₂₋₇ alkylcarbonyl)-N-(C₁₋₆ alkoxy)amino group, a C₁₋₆ alkyl group substituted by an N-(C₁₋₆ alkylsulfonyl)-N-(C₁₋₆ alkyl)amino group, a C₁₋₆ alkyl group substituted by an N-(C₁₋₆ alkylsulfonyl)-N-(C₁₋₆ alkoxy)amino group, a phenyl group (provided that such a phenyl group may be substituted by one or more substituents selected from the group consisting of a halogen atom, a trifluoromethyl group, a nitro group, a C₁₋₆ alkyl group, a C₁₋₆ alkoxy group and a C₂₋₇ alkoxycarbonyl group), a C₁₋₆ alkyl group substituted by a phenyl group (provided that such a phenyl group may be substituted by one or more substituents selected from the group consisting of a halogen atom, a trifluoromethyl group, a nitro group, a C₁₋₆ alkyl group, a C₁₋₆ alkoxy group and a C₂₋₇ alkoxycarbonyl group), a C₂₋₇ alkenyl group substituted by a phenyl group (provided that such a phenyl group may be substituted by one or more substituents selected from the group consisting of a halogen atom, a trifluoromethyl group, a nitro group, a C₁₋₆ alkyl group, a C₁₋₆ alkoxy group and a C₂₋₇ alkoxycarbonyl group), a C₂₋₆ alkynyl group substituted by a phenyl group (provided that such a phenyl group may be substituted by one or more substituents selected from the group consisting of a halogen atom, a trifluoromethyl group, a nitro group, a C₁₋₆ alkyl group, a C₁₋₆ alkoxy group and a C₂₋₇ alkoxycarbonyl group), a C₁₋₆ alkyl group substituted by a phenoxy group (provided that such a phenoxy group may be substituted by one or more substituents selected from the group consisting of a halogen atom, a trifluoromethyl group, a nitro group, a C₁₋₆ alkyl group, a C₁₋₆ alkoxy group and a C₂₋₇ alkoxycarbonyl group), a C₁₋₆ alkyl group substituted by a phenylthio group (provided that such a phenylthio group may be substituted by one or more substituents selected from the group consisting of a halogen atom, a trifluoromethyl group, a nitro group, a C₁₋₆ alkyl group, a C₁₋₆ alkoxy group and a C₂₋₇ alkoxycarbonyl group), a C₁₋₆ alkyl group substituted by a phenylsulfinyl group (provided that such a phenylsulfinyl group may be substituted by one or more substituents selected from the group consisting of a halogen atom, a trifluoromethyl group, a nitro group, a C₁₋₆ alkyl group, a C₁₋₆ alkoxy group and a C₂₋₇ alkoxycarbonyl group), a C₁₋₆ alkyl group substituted by a phenylsulfonyl group (provided that such a phenylsulfonyl group may be substituted by one or more substituents selected from the group consisting of a halogen atom, a trifluoromethyl group, a nitro group, a C₁₋₆ alkyl group, a C₁₋₆ alkoxy group and a C₂₋₇ alkoxycarbonyl group), a C₁₋₆ alkyl group substituted by a benzyloxy group (provided that the phenyl group of such a benzyloxy group may be substituted by one or more substituents selected from the group consisting of a halogen atom, a trifluoromethyl group, a nitro group, a C₁₋₆ alkyl group, a C₁₋₆ alkoxy group and a C₂₋₇ alkoxycarbonyl group), a C₁₋₆ alkyl group substituted by a benzylthio group (provided that the phenyl group of such a benzylthio group may be substituted by one or more substituents selected from the group consisting of a halogen atom, a trifluoromethyl group, a nitro group, a C₁₋₆ alkyl group, a C₁₋₆ alkoxy group and a C₂₋₇ alkoxycarbonyl group), a C₁₋₆ alkyl group substituted by a benzylsulfinyl group (provided that the phenyl group of such a benzylsulfinyl group may be substituted by one or more substituents selected from the group consisting of a halogen atom, a trifluoromethyl group, a nitro group, a C₁₋₆ alkyl group, a C₁₋₆ alkoxy group and a C₂₋₇ alkoxycarbonyl group), a C₁₋₆ alkyl group substituted by a benzylsulfonyl group (provided that the phenyl group of such a benzylsulfonyl group may be substituted by one or more substituents selected from the group consisting of a halogen atom, a trifluoromethyl group, a nitro group, a C₁₋₆ alkyl group, a C₁₋₆ alkoxy group and a C₂₋₇ alkoxycarbonyl group), a C₁₋₆ alkyl group substituted by a phenylcarbonyl group (provided that such a phenylcarbonyl group may be substituted by one or more substituents selected from the group consisting of a halogen atom, a trifluoromethyl group, a nitro group, a C₁₋₆ alkyl group, a C₁₋₆ alkoxy group and a C₂₋₇ alkoxycarbonyl group), a C₁₋₆ alkyl group substituted by a benzylcarbonyl group (provided that the phenyl group of such a benzylcarbonyl group may be substituted by one or more substituents selected from the group consisting of a halogen atom, a trifluoromethyl group, a nitro group, a C₁₋₆ alkyl group, a C₁₋₆ alkoxy group and a C₂₋₇ alkoxycarbonyl group), a C₁₋₆ alkyl group substituted by an amino group substituted by a C₁₋₄ alkylsulfonyl group, or a C₁₋₆ alkyl group substituted by an amino group substituted by a C₂₋₄ alkylcarbonyl group,
each of R^{e2}_{,} R^{e3}, R^{e6} and R^{e7} which are independent of one another, is a hydrogen atom, a C₁₋₆ alkyl group, a mono-, di- or poly-halogeno C₁₋₆ alkyl group, a C₁₋₆ alkoxy group, a mono-, di- or poly-halogeno C₁₋₆ alkoxy group, a C₁₋₆ alkylthio group, a C₁₋₆ alkylsulfinyl group, a C₁₋₆ alkylsulfonyl group, a C₂₋₇ alkoxycarbonyl group, a C₂₋₇ alkylcarbonyl group, a halogen atom, a nitro group, a cyano group, or a phenyl group (provided that such a phenyl group may be substituted by one or more substituents selected from the group consisting of a halogen atom, a trifluoromethyl group, a nitro group, a C₁₋₆ alkyl group, a C₁₋₆ alkoxy group and a C₂₋₇ alkoxycarbonyl group),
each of R^{e4}, R^{e5}, R^{e8}, R^{e9} and R^{e10} which are independent of one another, is a hydrogen atom, a C₁₋₈ alkyl group, a C₂₋₈ alkenyl group, a C₂₋₈ alkynyl group, or a phenyl group (provided that such a phenyl group may be substituted by one or more substituents selected from the group consisting of a halogen atom, a trifluoromethyl group, a nitro group, a C₁₋₆ alkyl group, a C₁₋₆ alkoxy group and a C₂₋₇ alkoxycarbonyl group),
R^{f1} is a C₁₋₈ alkyl group, a C₃₋₇ cycloalkyl group, a C₁₋₆ alkyl group substituted by a C₃₋₇ cycloalkyl group, a C₃₋₇ cycloalkenyl group, a C₁₋₆ alkyl group substituted by a C₃₋₇ cycloalkenyl group, a C₂₋₈ alkenyl group, a C₂₋₈ alkynyl group, a C₁₋₆ alkyl group substituted by a C₁₋₆ alkoxy group, a C₁₋₆ alkyl group substituted by a C₂₋₆ alkenyloxy group, a C₁₋₆ alkyl group substituted by a C₂₋₆ alkynyloxy group, a C₁₋₆ alkyl group substituted by a mono-, di- or poly-halogeno C₁₋₆ alkoxy group, a C₁₋₆ alkyl group substituted by a mono-, di- or poly-halogeno C₂₋₆ alkenyloxy group, a C₁₋₆ alkyl group substituted by a mono-, di- or poly-halogeno C₂₋₆ alkynyloxy group, a C₁₋₆ alkyl group substituted by a C₁₋₆ alkylthio group, a C₁₋₆ alkyl group substituted by a C₁₋₆ alkylsulfinyl group, a C₁₋₆ alkyl group substituted by a C₁₋₆ alkylsulfonyl group, a mono-, di- or poly-halogeno C₁₋₈ alkyl group, a mono-, di- or poly-halogeno C₂₋₈ alkenyl group, a mono-, di- or poly-halogeno C₂₋₈ alkynyl group, a C₁₋₆ alkyl group substituted by a cyano group, a C₂₋₆ alkenyl group substituted by a cyano group, a C₂₋₆ alkynyl group substituted by a cyano group, a C₁₋₆ alkyl group substituted by a nitro group, a C₂₋₆ alkenyl group substituted by a nitro group, a C₂₋₆ alkynyl group substituted by a nitro group, a C₁₋₆ alkyl group substituted by a C₂₋₇ alkoxycarbonyl group, a C₂₋₆ alkenyl group substituted by a C₂₋₇ alkoxycarbonyl group, a C₂₋₆ alkynyl group substituted by a C₂₋₇ alkoxycarbonyl group, a C₁₋₆ alkyl group substituted by a, C₂₋₇ alkylcarbonyl group, a C₁₋₆ alkyl group substituted by a mono-, di- or poly-halogeno C₂₋₇ alkylcarbonyl group, a C₁₋₆ alkyl group substituted by a C₃₋₇ alkenylcarbonyl group, a C₁₋₆ alkyl group substituted by a C₃₋₇ alkynylcarbonyl group, a C₁₋₆ alkyl group substituted by a C₂₋₅ alkylcarbonyl group substituted by a C₁₋₄ alkoxy group, a C₁₋₆ alkyl group substituted by a C₂₋₅ alkylcarbonyl group substituted by a C₁₋₄ alkylthio group, a C₁₋₆ alkyl group substituted by a C₂₋₅ alkylcarbonyl group substituted by a C₁₋₄ alkylsulfinyl group, a C₁₋₆ alkyl group substituted by a C₂₋₅ alkylcarbonyl group substituted by a C₁₋₄ alkylsulfonyl group, a C₂₋₆ alkenyl group substituted by a C₂₋₇ alkylcarbonyl group, a C₂₋₆ alkynyl group substituted by a C₂₋₇ alkylcarbonyl group, a C₁₋₆ alkyl group substituted by a C₁₋₆ alkylsulfamoyl group, a C₁₋₆ alkyl group substituted by a C₁₋₆ alkoxysulfamoyl group, a C₁₋₆ alkyl group substituted by a di(C₁₋₃ alkyl)sulfamoyl group, a C₁₋₆ alkyl group substituted by an N-(C₁₋₃ alkyl)-N-(C₁₋₃ alkoxy)sulfamoyl group, a C₁₋₆ alkyl group substituted by a C₂₋₇ alkylcarbamoyl group, a C₁₋₆ alkyl group substituted by a di(C₁₋₃ alkyl)carbamoyl group, a C₁₋₆ alkyl group substituted by a C₂₋₇ alkoxycarbamoyl group, a C₁₋₆ alkyl group substituted by an N-(C₁₋₃ alkyl)-N-(C₁₋₃ alkoxy)carbamoyl group, a C₁₋₆ alkyl group substituted by a C₁₋₆ alkylamino group, a C₁₋₆ alkyl group substituted by a C₁₋₆ alkoxyamino group, a C₁₋₆ alkyl group substituted by a di(C₁₋₃ alkyl)amino group, a C₁₋₆ alkyl group substituted by an N-(C₁₋₃ alkyl)-N-(C₁₋₃ alkoxy)amino group, a C₁₋₆ alkyl group substituted by an N-(C₂₋₇ alkylcarbonyl)-N-(C₁₋₆ alkyl)amino group, a C₁₋₆ alkyl group substituted by an N-(C₂₋₇ alkylcarbonyl)-N-(C₁₋₆ alkoxy)amino group, a C₁₋₆ alkyl group substituted by an N-(C₁₋₆ alkylsulfonyl)-N-(C₁₋₆ alkyl)amino group, a C₁₋₆ alkyl group substituted by an N-(C₁₋₆ alkylsulfonyl)-N-(C₁₋₆ alkoxy)amino group, a phenyl group (provided that such a phenyl group may be substituted by one or more substituents selected from the group consisting of a halogen atom, a trifluoromethyl group, a nitro group, a C₁₋₆ alkyl group, a C₁₋₆ alkoxy group and a C₂₋₇ alkoxycarbonyl group), a C₁₋₆ alkyl group substituted by a phenyl group (provided that such a phenyl group may be substituted by one or more substituents selected from the group consisting of a halogen atom, a trifluoromethyl group, a nitro group, a C₁₋₆ alkyl group, a C₁₋₆ alkoxy group and a C₂₋₇ alkoxycarbonyl group), a C₂₋₇ alkenyl group substituted by a phenyl group (provided that such a phenyl group may be substituted by one or more substituents selected from the group consisting of a halogen atom, a trifluoromethyl group, a nitro group, a C₁₋₆ alkyl group, a C₁₋₆ alkoxy group and a C₂₋₇ alkoxycarbonyl group), a C₂₋₆ alkynyl group substituted by a phenyl group (provided that such a phenyl group may be substituted by one or more substituents selected from the group consisting of a halogen atom, a trifluoromethyl group, a nitro group, a C₁₋₆ alkyl group, a C₁₋₆ alkoxy group and a C₂₋₇ alkoxycarbonyl group), a C₁₋₆ alkyl group substituted by a phenoxy group (provided that such a phenoxy group may be substituted by one or more substituents selected from the group consisting of a halogen atom, a trifluoromethyl group, a nitro group, a C₁₋₆ alkyl group, a C₁₋₆ alkoxy group and a C₂₋₇ alkoxycarbonyl group), a C₁₋₆ alkyl group substituted by a phenylthio group (provided that such a phenylthio group may be substituted by one or more substituents selected from the group consisting of a halogen atom, a trifluoromethyl group, a nitro group, a C₁₋₆ alkyl group, a C₁₋₆ alkoxy group and a C₂₋₇ alkoxycarbonyl group), a C₁₋₆ alkyl group substituted by a phenylsulfinyl group (provided that such a phenylsulfinyl group may be substituted by one or more substituents selected from the group consisting of a halogen atom, a trifluoromethyl group, a nitro group, a C₁₋₆ alkyl group, a C₁₋₆ alkoxy group and a C₂₋₇ alkoxycarbonyl group), a C₁₋₆ alkyl group substituted by a phenylsulfonyl group (provided that such a phenylsulfonyl group may be substituted by one or more substituents selected from the group consisting of a halogen atom, a trifluoromethyl group, a nitro group, a C₁₋₆ alkyl group, a C₁₋₆ alkoxy group and a C₂₋₇ alkoxycarbonyl group), a C₁₋₆ alkyl group substituted by a benzyloxy group (provided that the phenyl group of such a benzyloxy group may be substituted by one or more substituents selected from the group consisting of a halogen atom, a trifluoromethyl group, a nitro group, a C₁₋₆ alkyl group, a C₁₋₆ alkoxy group and a C₂₋₇ alkoxycarbonyl group), a C₁₋₆ alkyl group substituted by a benzylthio group (provided that the phenyl group of such a benzylthio group may be substituted by one or more substituents selected from the group consisting of a halogen atom, a trifluoromethyl group, a nitro group, a C₁₋₆ alkyl group, a C₁₋₆ alkoxy group and a C₂₋₇ alkoxycarbonyl group), a C₁₋₆ alkyl group substituted by a benzylsulfinyl group (provided that the phenyl group of such a benzylsulfinyl group may be substituted by one or more substituents selected from the group consisting of a halogen atom, a trifluoromethyl group, a nitro group, a C₁₋₆ alkyl group, a C₁₋₆ alkoxy group and a C₂₋₇ alkoxycarbonyl group), a C₁₋₆ alkyl group substituted by a benzylsulfonyl group (provided that the phenyl group of such a benzylsulfonyl group may be substituted by one or more substituents selected from the group consisting of a halogen atom, a trifluoromethyl group, a nitro group, a C₁₋₆ alkyl group, a C₁₋₆ alkoxy group and a C₂₋₇ alkoxycarbonyl group), a C₁₋₆ alkyl group substituted by a phenylcarbonyl group (provided that such a phenylcarbonyl group may be substituted by one or more substituents selected from the group consisting of a halogen atom, a trifluoromethyl group, a nitro group, a C₁₋₆ alkyl group, a C₁₋₆ alkoxy group and a C₂₋₇ alkoxycarbonyl group), a C₁₋₆ alkyl group substituted by a benzylcarbonyl group (provided that the phenyl group of such a benzylcarbonyl group may be substituted by one or more substituents selected from the group consisting of a halogen atom, a trifluoromethyl group, a nitro group, a C₁₋₆ alkyl group, a C₁₋₆ alkoxy group and a C₂₋₇ alkoxycarbonyl group), a C₁₋₆ alkyl group substituted by an amino group substituted by a C₁₋₄ alkylsulfonyl group, or a C₁₋₆ alkyl group substituted by an amino group substituted by a C₂₋₄ alkylcarbonyl group,
R^{f2} is a C₁₋₈ alkyl group, a C₂₋₈ alkenyl group, a C₂₋₈ alkynyl group, a C₁₋₆ alkyl group substituted by a C₁₋₆ alkylthio group, a C₁₋₆ alkyl group substituted by a C₁₋₆ alkylsulfinyl group, a C₁₋₆ alkyl group substituted by a C₁₋₆ alkylsulfonyl group, a C₁₋₆ alkyl group substituted by a C₁₋₆ alkoxy group, a C₁₋₆ alkyl group substituted by a C₂₋₇ alkoxycarbonyl group, a C₁₋₆ alkyl group substituted by a C₂₋₇ alkylcarbonyl group, a C₁₋₆ alkyl group substituted by a cyano group, a phenyl group (provided that such a phenyl group may be substituted by one or more substituents selected from the group consisting of a halogen atom, a trifluoromethyl group, a nitro group, a C₁₋₆ alkyl group, a C₁₋₆ alkoxy group and a C₂₋₇ alkoxycarbonyl group), or a C₁₋₆ alkyl group substituted by a phenyl group (provided that such a phenyl group may be substituted by one or more substituents selected from the group consisting of a halogen atom, a trifluoromethyl group, a nitro group, a C₁₋₆ alkyl group, a C₁₋₆ alkoxy group and a C₂₋₇ alkoxycarbonyl group),
each of R^{f3}, R^{f4}, R^{f7}, R^{f8}, R^{f11} and R^{f12} which are independent of one another, is a hydrogen atom, a C₁₋₆ alkyl group, a mono-, di- or poly-halogeno C₁₋₆ alkyl group, a C₁₋₆ alkoxy group, a mono-, di- or poly-halogeno C₁₋₆ alkoxy group, a C₁₋₆ alkylthio group, a C₁₋₆ alkylsulfinyl group, a C₁₋₆ alkylsulfonyl group, a C₂₋₇ alkoxycarbonyl group, a C₂₋₇ alkylcarbonyl group, a halogen atom, a nitro group, a cyano group, or a phenyl group (provided that such a phenyl group may be substituted by one or more substituents selected from the group consisting of a halogen atom, a trifluoromethyl group, a nitro group, a C₁₋₆ alkyl group, a C₁₋₆ alkoxy group and a C₂₋₇ alkoxycarbonyl group),
each of R^{f5}, R^{f6}, R^{f9}, R^{f10}, R^{f13}, R^{f14} and R^{f15} which are independent of one another, is a hydrogen atom, a C₁₋₈ alkyl group, a C₂₋₈ alkenyl group, a C₂₋₈ alkynyl group, or a phenyl group (provided that such a phenyl group may be substituted by one or more substituents selected from the group consisting of a halogen atom, a trifluoromethyl group, a nitro group, a C₁₋₆ alkyl group, a C₁₋₆ alkoxy group and a C₂₋₇ alkoxycarbonyl group),
R^{g1} is a C₁₋₈ alkyl group, a C₃₋₇ cycloalkyl group, a C₁₋₆ alkyl group substituted by a C₃₋₇ cycloalkyl group, a C₃₋₇ cycloalkenyl group, a C₁₋₆ alkyl group substituted by a C₃₋₇ cycloalkenyl group, a C₂₋₈ alkenyl group, a C₂₋₈ alkynyl group, a C₁₋₆ alkyl group substituted by a C₁₋₆ alkoxy group, a C₁₋₆ alkyl group substituted by a C₂₋₆ alkenyloxy group, a C₁₋₆ alkyl group substituted by a C₂₋₆ alkynyloxy group, a C₁₋₆ alkyl group substituted by a mono-, di- or poly-halogeno C₁₋₆ alkoxy group, a C₁₋₆ alkyl group substituted by a mono-, di- or poly-halogeno C₂₋₆ alkenyloxy group, a C₁₋₆ alkyl group substituted by a mono-, di- or poly-halogeno C₂₋₆ alkynyloxy group, a C₁₋₆ alkyl group substituted by a C₁₋₆ alkylthio group, a C₁₋₆ alkyl group substituted by a C₁₋₆ alkylsulfinyl group, a C₁₋₆ alkyl group substituted by a C₁₋₆ alkylsulfonyl group, a mono-, di- or poly-halogeno C₁₋₈ alkyl group, a mono-, di- or poly-halogeno C₂₋₈ alkenyl group, a mono-, di- or poly-halogeno C₂₋₈ alkynyl group, a C₁₋₆ alkyl group substituted by a cyano group, a C₂₋₆ alkenyl group substituted by a cyano group, a C₂₋₆ alkynyl group substituted by a cyano group, a C₁₋₆ alkyl group substituted by a nitro group, a C₂₋₆ alkenyl group substituted by a nitro group, a C₂₋₆ alkynyl group substituted by a nitro group, a C₁₋₆ alkyl group substituted by a C₂₋₇ alkoxycarbonyl group, a C₂₋₆ alkenyl group substituted by a C₂₋₇ alkoxycarbonyl group, a C₂₋₆ alkynyl group substituted by a C₂₋₇ alkoxycarbonyl group, a C₁₋₆ alkyl group substituted by a C₂₋₇ alkylcarbonyl group, a C₁₋₆ alkyl group substituted by a mono-, di- or poly-halogeno C₂₋₇ alkylcarbonyl group, a C₁₋₆ alkyl group substituted by a C₃₋₇ alkenylcarbonyl group, a C₁₋₆ alkyl group substituted by a C₃₋₇ alkynylcarbonyl group, a C₁₋₆ alkyl group substituted by a C₂₋₅ alkylcarbonyl group substituted by a C₁₋₄ alkoxy group, a C₁₋₆ alkyl group substituted by a C₂₋₅ alkylcarbonyl group substituted by a C₁₋₄ alkylthio group, a C₁₋₆ alkyl group substituted by a C₂₋₅ alkylcarbonyl group substituted by a C₁₋₄ alkylsulfinyl group, a C₁₋₆ alkyl group substituted by a C₂₋₅ alkylcarbonyl group substituted by a C₁₋₄ alkylsulfonyl group, a C₂₋₆ alkenyl group substituted by a C₂₋₇ alkylcarbonyl group, a C₂₋₆ alkynyl group substituted by a C₂₋₇ alkylcarbonyl group, a C₁₋₆ alkyl group substituted by a C₁₋₆ alkylsulfamoyl group, a C₁₋₆ alkyl group substituted by a C₁₋₆ alkoxysulfamoyl group, a C₁₋₆ alkyl group substituted by a di(C₁₋₃ alkyl)sulfamoyl group, a C₁₋₆ alkyl group substituted by an N-(C₁₋₃ alkyl)-N-(C₁₋₃ alkoxy)sulfamoyl group, a C₁₋₆ alkyl group substituted by a C₂₋₇ alkylcarbamoyl group, a C₁₋₆ alkyl group substituted by a di(C₁₋₃ alkyl)carbamoyl group, a C₁₋₆ alkyl group substituted by a C₂₋₇ alkoxycarbamoyl group, a C₁₋₆ alkyl group substituted by an N-(C₁₋₃ alkyl)-N-(C₁₋₃ alkoxy)carbamoyl group, a C₁₋₆ alkyl group substituted by a C₁₋₆ alkylamino group, a C₁₋₆ alkyl group substituted by a C₁₋₆ alkoxyamino group, a C₁₋₆ alkyl group substituted by a di(C₁₋₃ alkyl)amino group, a C₁₋₆ alkyl group substituted by an N-(C₁₋₃ alkyl)-N-(C₁₋₃ alkoxy)amino group, a C₁₋₆ alkyl group substituted by an N-(C₂₋₇ alkylcarbonyl)-N-(C₁₋₆ alkyl)amino group, a C₁₋₆ alkyl group substituted by an N-(C₂₋₇ alkylcarbonyl)-N-(C₁₋₆ alkoxy)amino group, a C₁₋₆ alkyl group substituted by an N-(C₁₋₆ alkylsulfonyl)-N-(C₁₋₆ alkyl)amino group, a C₁₋₆ alkyl group substituted by an N-(C₁₋₆ alkylsulfonyl)-N-(C₁₋₆ alkoxy)amino group, a C₁₋₆ alkyl group substituted by a phenyl group (provided that such a phenyl group may be substituted by one or more substituents selected from the group consisting of a halogen atom, a trifluoromethyl group, a nitro group, a C₁₋₆ alkyl group, a C₁₋₆ alkoxy group and a C₂₋₇ alkoxycarbonyl group), a C₂₋₇ alkenyl group substituted by a phenyl group (provided that such a phenyl group may be substituted by one or more substituents selected from the group consisting of a halogen atom, a trifluoromethyl group, a nitro group, a C₁₋₆ alkyl group, a C₁₋₆ alkoxy group and a C₂₋₇ alkoxycarbonyl group), a C₂₋₆ alkynyl group substituted by a phenyl group (provided that such a phenyl group may be substituted by one or more substituents selected from the group consisting of a halogen atom, a trifluoromethyl group, a nitro group, a C₁₋₆ alkyl group, a C₁₋₆ alkoxy group and a C₂₋₇ alkoxycarbonyl group), a C₁₋₆ alkyl group substituted by a phenoxy group (provided that such a phenoxy group may be substituted by one or more substituents selected from the group consisting of a halogen atom, a trifluoromethyl group, a nitro group, a C₁₋₆ alkyl group, a C₁₋₆ alkoxy group and a C₂₋₇ alkoxycarbonyl group), a C₁₋₆ alkyl group substituted by a phenylthio group (provided that such a phenylthio group may be substituted by one or more substituents selected from the group consisting of a halogen atom, a trifluoromethyl group, a nitro group, a C₁₋₆ alkyl group, a C₁₋₆ alkoxy group and a C₂₋₇ alkoxycarbonyl group), a C₁₋₆ alkyl group substituted by a phenylsulfinyl group (provided that such a phenylsulfinyl group may be substituted by one or more substituents selected from the group consisting of a halogen atom, a trifluoromethyl group, a nitro group, a C₁₋₆ alkyl group, a C₁₋₆ alkoxy group and a C₂₋₇ alkoxycarbonyl group), a C₁₋₆ alkyl group substituted by a phenylsulfonyl group (provided that such a phenylsulfonyl group may be substituted by one or more substituents selected from the group consisting of a halogen atom, a trifluoromethyl group, a nitro group, a C₁₋₆ alkyl group, a C₁₋₆ alkoxy group and a C₂₋₇ alkoxycarbonyl group), a C₁₋₆ alkyl group substituted by a benzyloxy group (provided that the phenyl group of such a benzyloxy group may be substituted by one or more substituents selected from the group consisting of a halogen atom, a trifluoromethyl group, a nitro group, a C₁₋₆ alkyl group, a C₁₋₆ alkoxy group and a C₂₋₇ alkoxycarbonyl group), a C₁₋₆ alkyl group substituted by a benzylthio group (provided that the phenyl group of such a benzylthio group may be substituted by one or more substituents selected from the group consisting of a halogen atom, a trifluoromethyl group, a nitro group, a C₁₋₆ alkyl group, a C₁₋₆ alkoxy group and a C₂₋₇ alkoxycarbonyl group), a C₁₋₆ alkyl group substituted by a benzylsulfinyl group (provided that the phenyl group of such a benzylsulfinyl group may be substituted by one or more substituents selected from the group consisting of a halogen atom, a trifluoromethyl group, a nitro group, a C₁₋₆ alkyl group, a C₁₋₆ alkoxy group and a C₂₋₇ alkoxycarbonyl group), a C₁₋₆ alkyl group substituted by a benzylsulfonyl group (provided that the phenyl group of such a benzylsulfonyl group may be substituted by one or more substituents selected from the group consisting of a halogen atom, a trifluoromethyl group, a nitro group, a C₁₋₆ alkyl group, a C₁₋₆ alkoxy group and a C₂₋₇ alkoxycarbonyl group), a C₁₋₆ alkyl group substituted by a phenylcarbonyl group (provided that such a phenylcarbonyl group may be substituted by one or more substituents selected from the group consisting of a halogen atom, a trifluoromethyl group, a nitro group, a C₁₋₆ alkyl group, a C₁₋₆ alkoxy group and a C₂₋₇ alkoxycarbonyl group), or a C₁₋₆ alkyl group substituted by a benzylcarbonyl group (provided that the phenyl group of such a benzylcarbonyl group may be substituted by one or more substituents selected from the group consisting of a halogen atom, a trifluoromethyl group, a nitro group, a C₁₋₆ alkyl group, a C₁₋₆ alkoxy group and a C₂₋₇ alkoxycarbonyl group),
each of R^{g2} and R^{g3} which are independent of each other, is a C₁₋₆ alkyl group, a C₂₋₆ alkenyl group, a C₂₋₆ alkynyl group, a mono-, di- or poly-halogeno C₁₋₆ alkyl group, a C₁₋₆ alkoxy group, a C₁₋₆ alkylsulfonyl group, a C₁₋₆ alkylsulfamoyl group, a di(C₁₋₃ alkyl)sulfamoyl group, a C₂₋₇ alkoxycarbonyl group, a C₂₋₇ alkylcarbonyl group, a C₂₋₇ alkylcarbamoyl group, a di(C₁₋₃ alkyl)carbamoyl group, a phenyl group (provided that such a phenyl group may be substituted by one or more substituents selected from the group consisting of a halogen atom, a trifluoromethyl group, a nitro group, a C₁₋₆ alkyl group, a C₁₋₆ alkoxy group and a C₂₋₇ alkoxycarbonyl group), or a benzyl group (provided that the phenyl group of such a benzyl group may be substituted by one or more substituents selected from the group consisting of a halogen atom, a trifluoromethyl group, a nitro group, a C₁₋₆ alkyl group, a C₁₋₆ alkoxy group and a C₂₋₇ alkoxycarbonyl group),
or R^{g2} and R^{g3} form a saturated 3- to 7-membered heterocyclic ring together with the nitrogen atom to which they are bonded,
X is an oxygen atom or a sulfur atom,
L is a hydrogen atom, a C₁₋₆ alkyl group, a C₂₋₆ alkenyl group or a C₂₋₆ alkynyl group,
G is 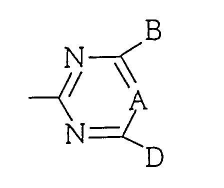
A is a CH group, or a nitrogen atom, and
each of B and D which are independent of each other, is a C₁₋₄ alkyl group, a C₁₋₄ alkoxy group, a mono-, di-or poly-halogeno C₁₋₄ alkyl group, a mono-, di- or poly-halogeno C₁₋₄ alkoxy group, a halogen atom, a C₁₋₄ alkylamino group, or a di(C₁₋₄ alkyl)amino group.

3. The iminosulfonylurea derivative or its salt according to Claim 2, wherein Q is

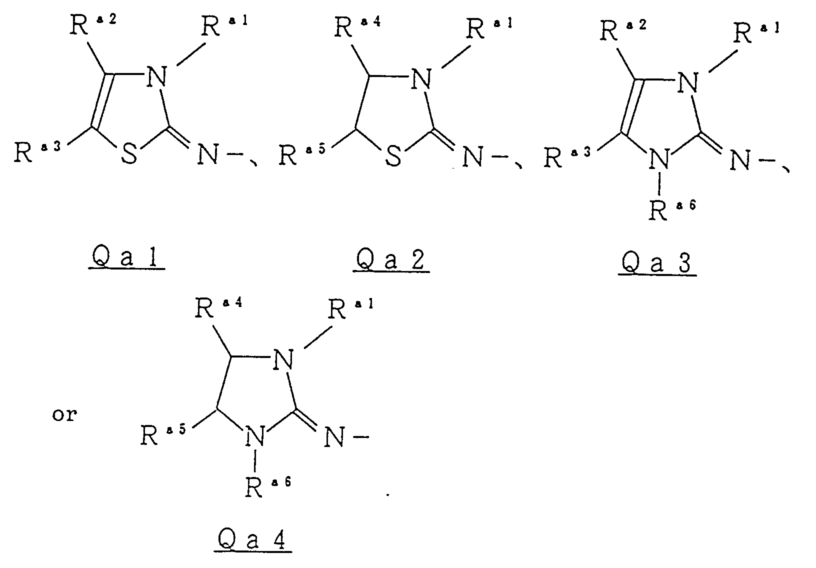

4. The iminosulfonylurea derivative or its salt according to Claim 2, wherein Q is

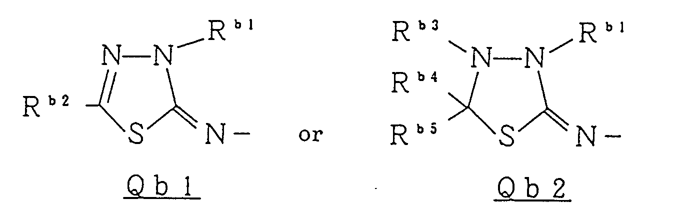

5. The iminosulfonylurea derivative or its salt according to Claim 2, wherein Q is

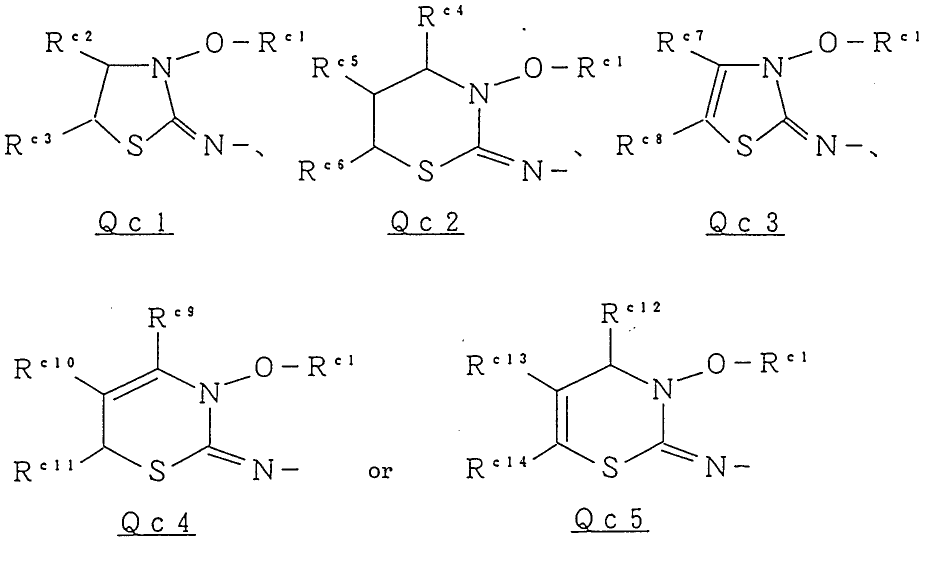

6. The iminosulfonylurea derivative or its salt according to Claim 2, wherein Q is

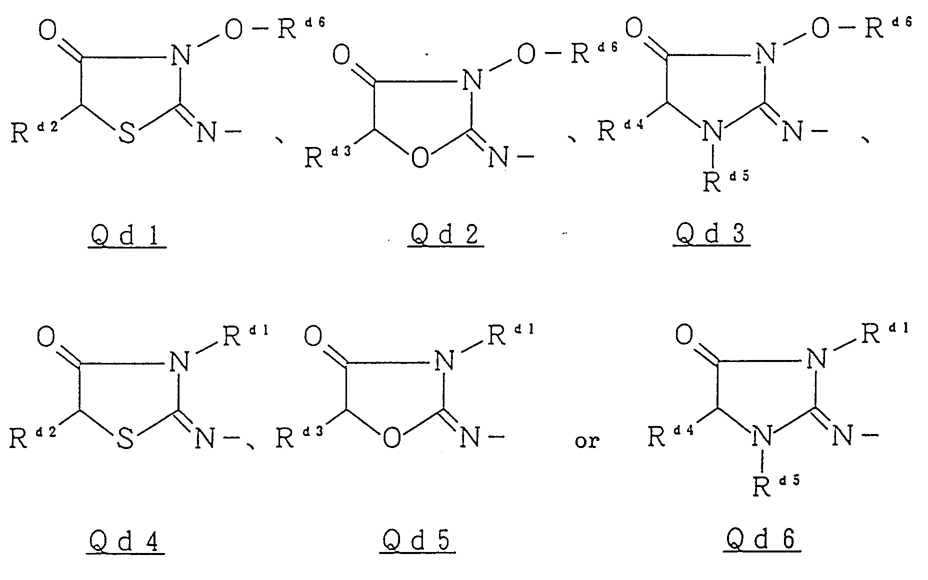

7. The iminosulfonylurea derivative or its salt according to Claim 2, wherein Q is

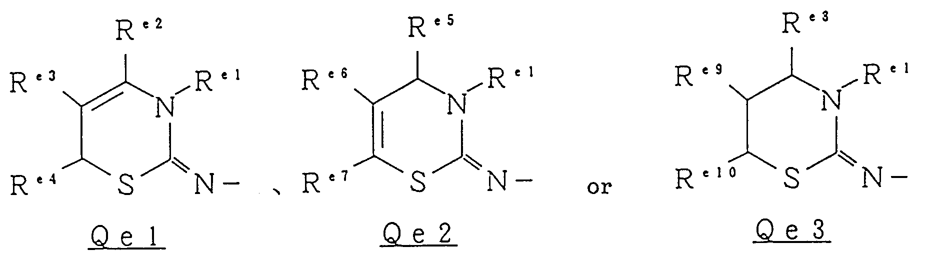

8. The iminosulfonylurea derivative or its salt according to Claim 2, wherein Q is

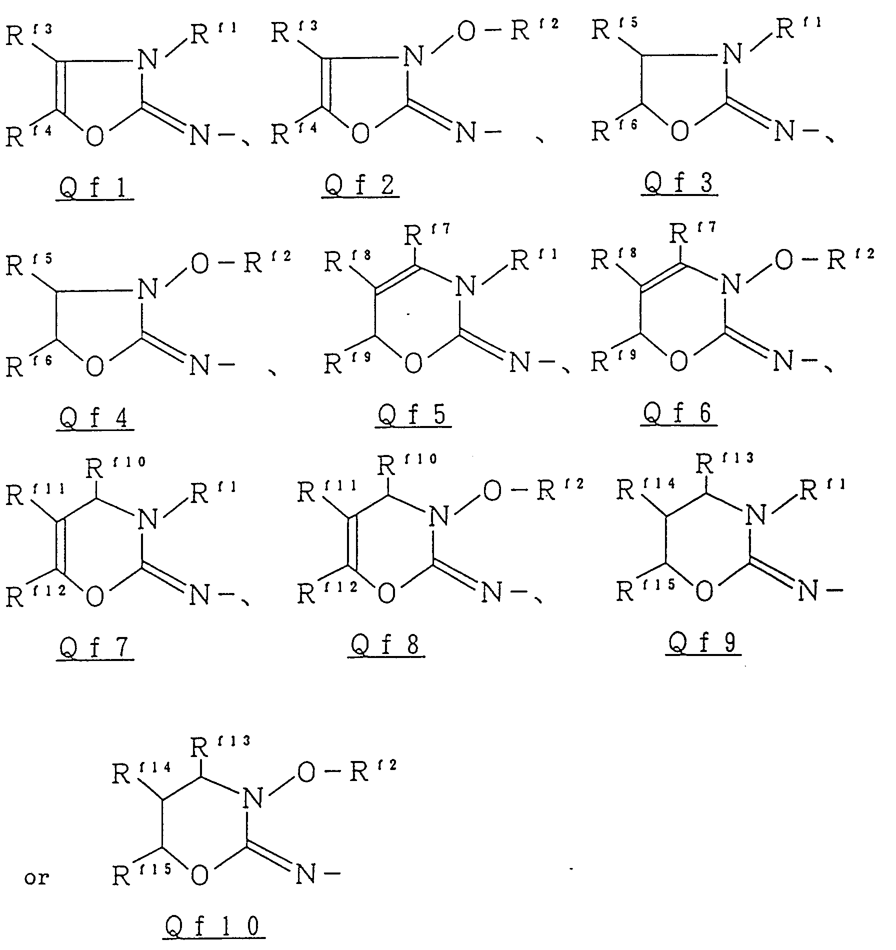

9. The iminosulfonylurea derivative or its salt according to Claim 2, wherein Q is

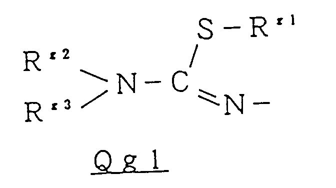

10. A herbicide which contains an iminosulfonylurea derivative defined in Claim 1, as an active ingredient.

11. A herbicide which contains an iminosulfonylurea derivative defined in Claim 2, as an active ingredient.

12. A herbicide which contains an iminosulfonylurea derivative defined in Claim 3, as an active ingredient.

13. A herbicide which contains an iminosulfonylurea derivative defined in Claim 4, as an active ingredient.

14. A herbicide which contains an iminosulfonylurea derivative defined in Claim 5, as an active ingredient.

15. A herbicide which contains an iminosulfonylurea derivative defined in Claim 6, as an active ingredient.

16. A herbicide which contains an iminosulfonylurea derivative defined in Claim 7, as an active ingredient.

17. A herbicide which contains an iminosulfonylurea derivative defined in Claim 8, as an active ingredient.

18. A herbicide which contains an iminosulfonylurea derivative defined in Claim 9, as an active ingredient.

19. A herbicidal and growth control method against weeds, which comprises applying a herbicidally effective amount of an iminosulfonylurea derivative defined in Claim 1.

20. A herbicidal and growth control method against weeds, which comprises applying a herbicidally effective amount of an iminosulfonylurea derivative defined in Claim 2.

21. A herbicidal and growth control method against weeds, which comprises applying a herbicidally effective amount of an iminosulfonylurea derivative defined in Claim 3.

22. A herbicidal and growth control method against weeds, which comprises applying a herbicidally effective amount of an iminosulfonylurea derivative defined in Claim 4.

23. A herbicidal and growth control method against weeds, which comprises applying a herbicidally effective amount of an iminosulfonylurea derivative defined in Claim 5.

24. A herbicidal and growth control method against weeds, which comprises applying a herbicidally effective amount of an iminosulfonylurea derivative defined in Claim 6.

25. A herbicidal and growth control method against weeds, which comprises applying a herbicidally effective amount of an iminosulfonylurea derivative defined in Claim 7.

26. A herbicidal and growth control method against weeds, which comprises applying a herbicidally effective amount of an iminosulfonylurea derivative defined in Claim 8.

27. A herbicidal and growth control method against weeds, which comprises applying a herbicidally effective amount of an iminosulfonylurea derivative defined in Claim 9.

28. The iminosulfonylurea derivative or its salt according to Claim 3, wherein R^{a1} is 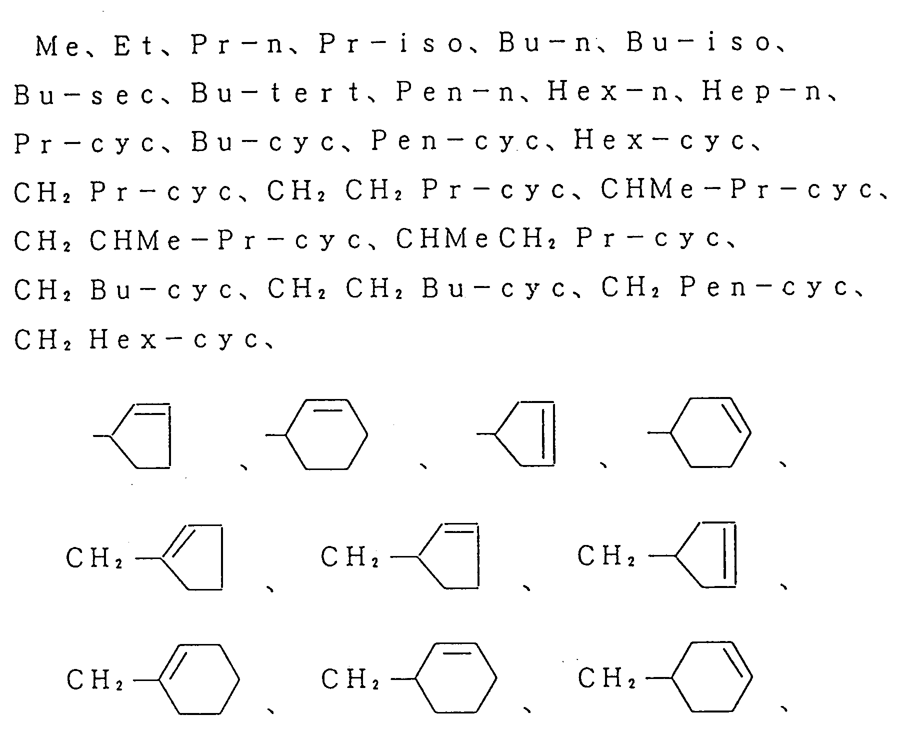 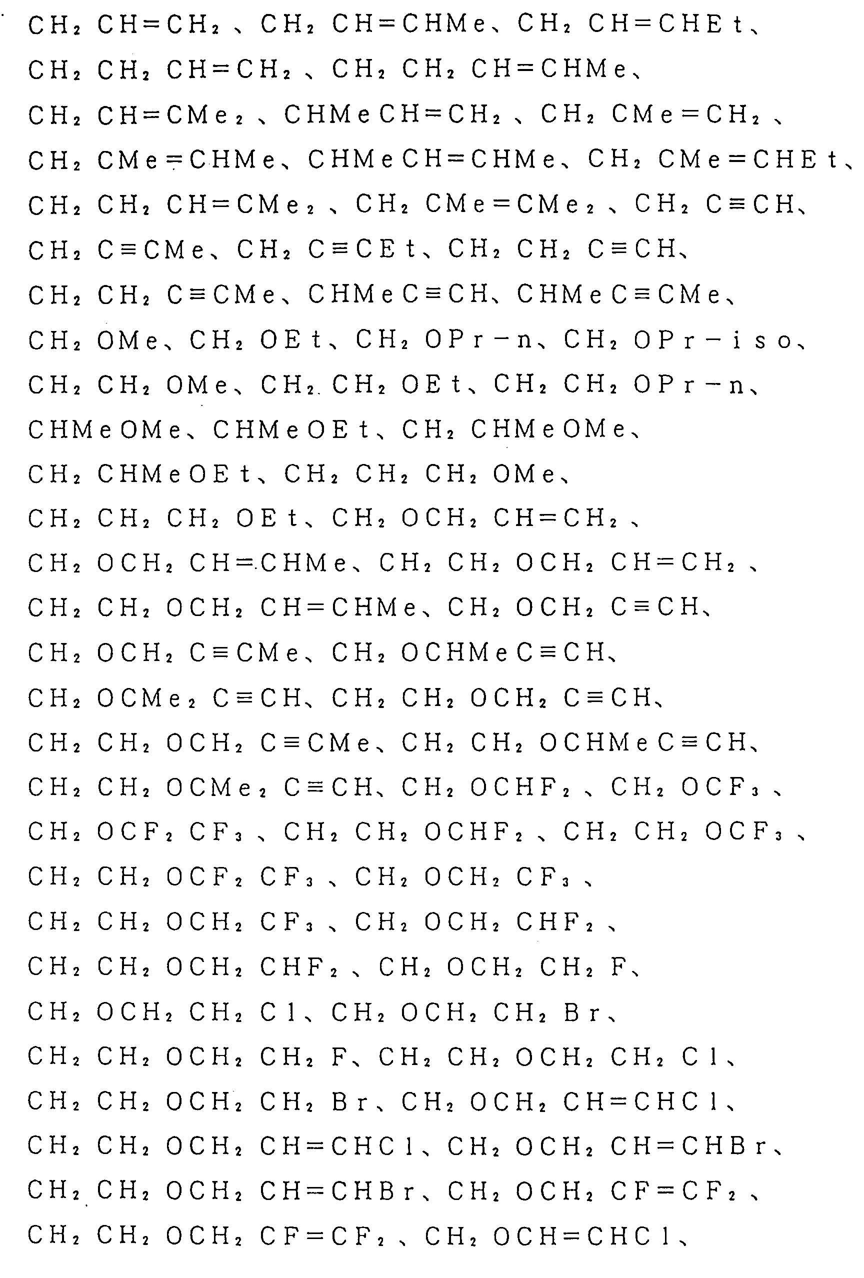 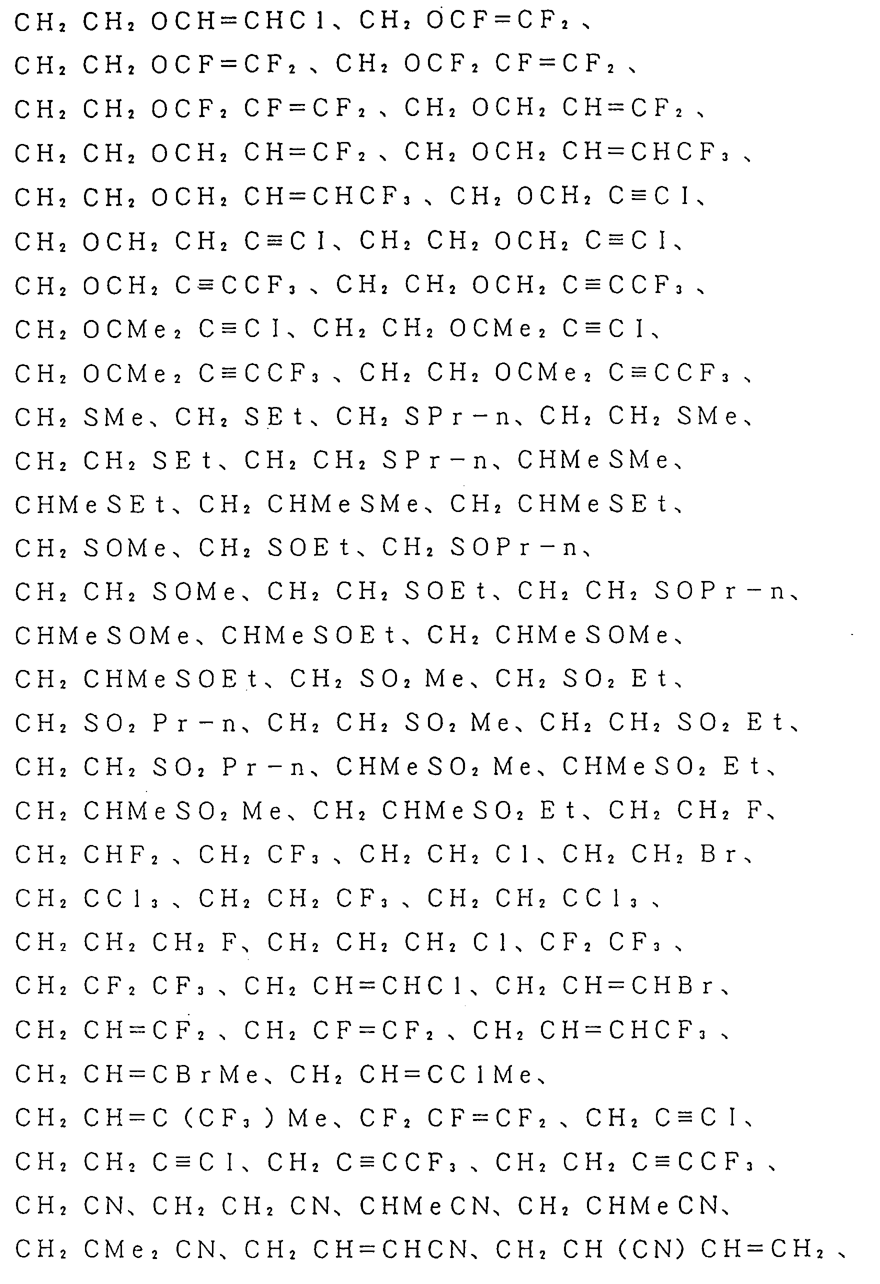 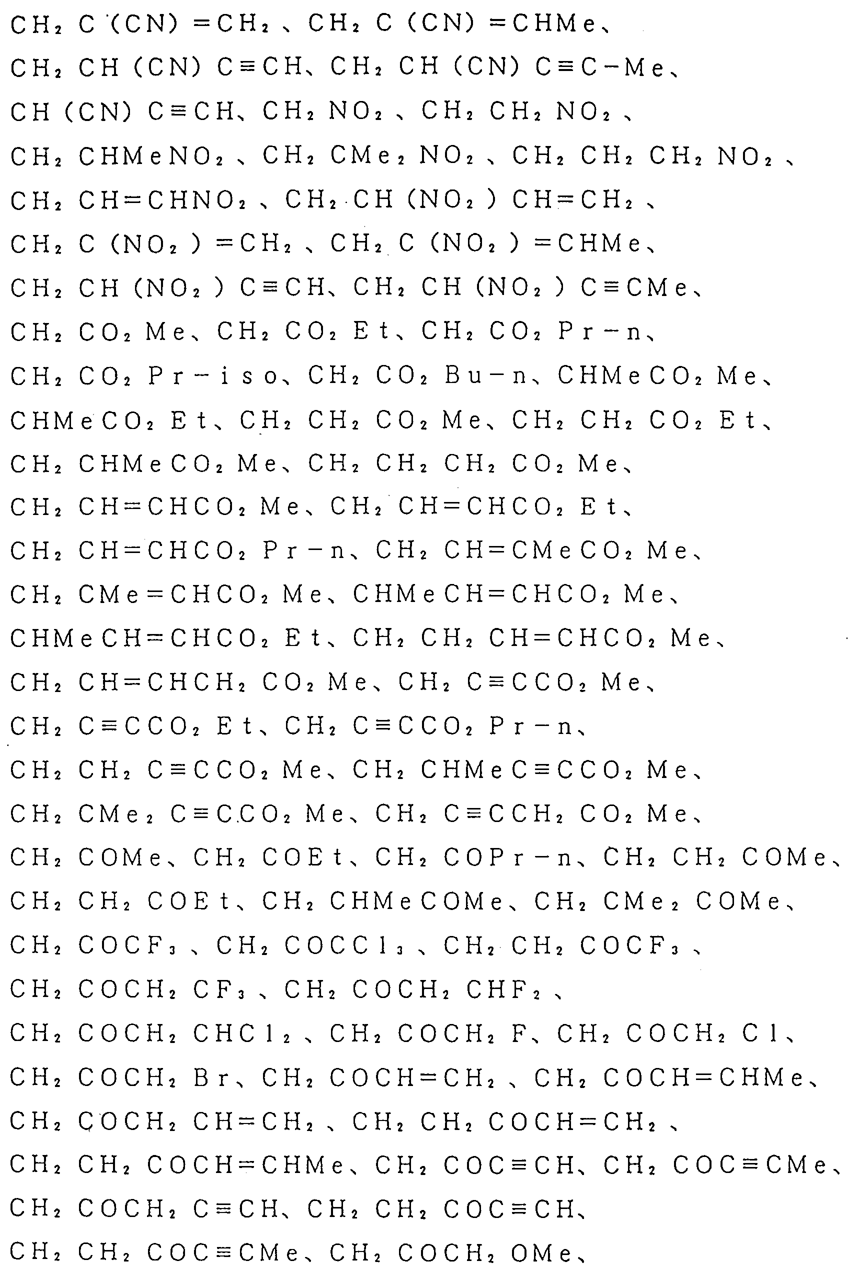 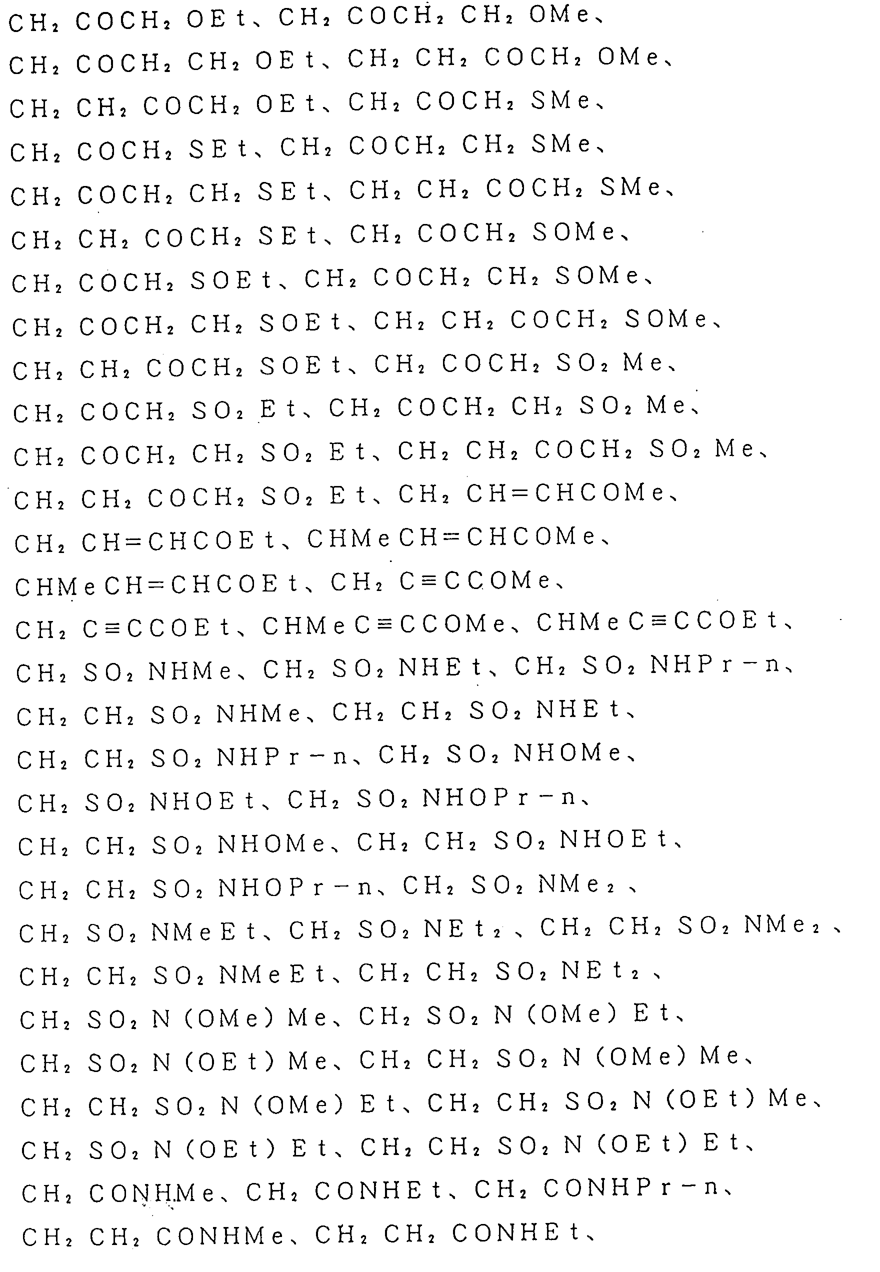 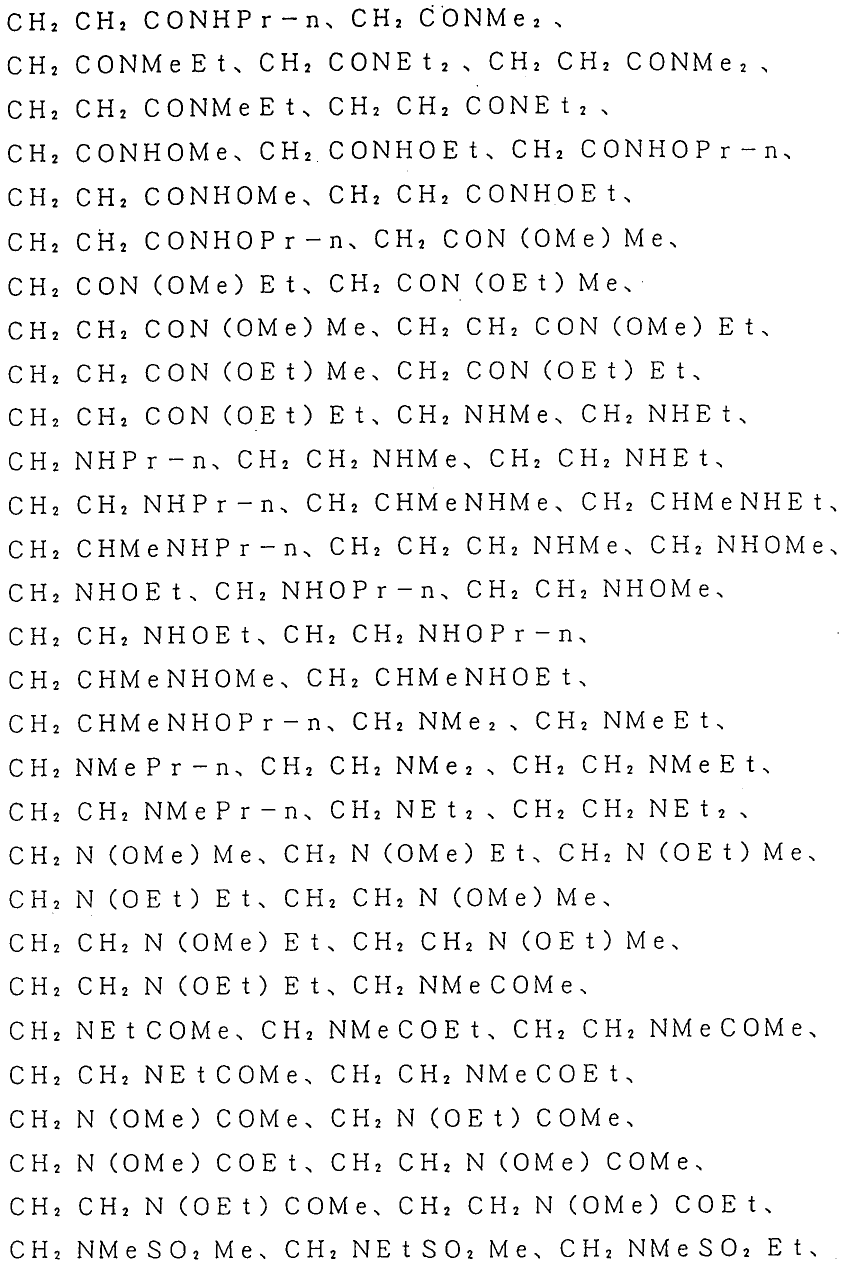 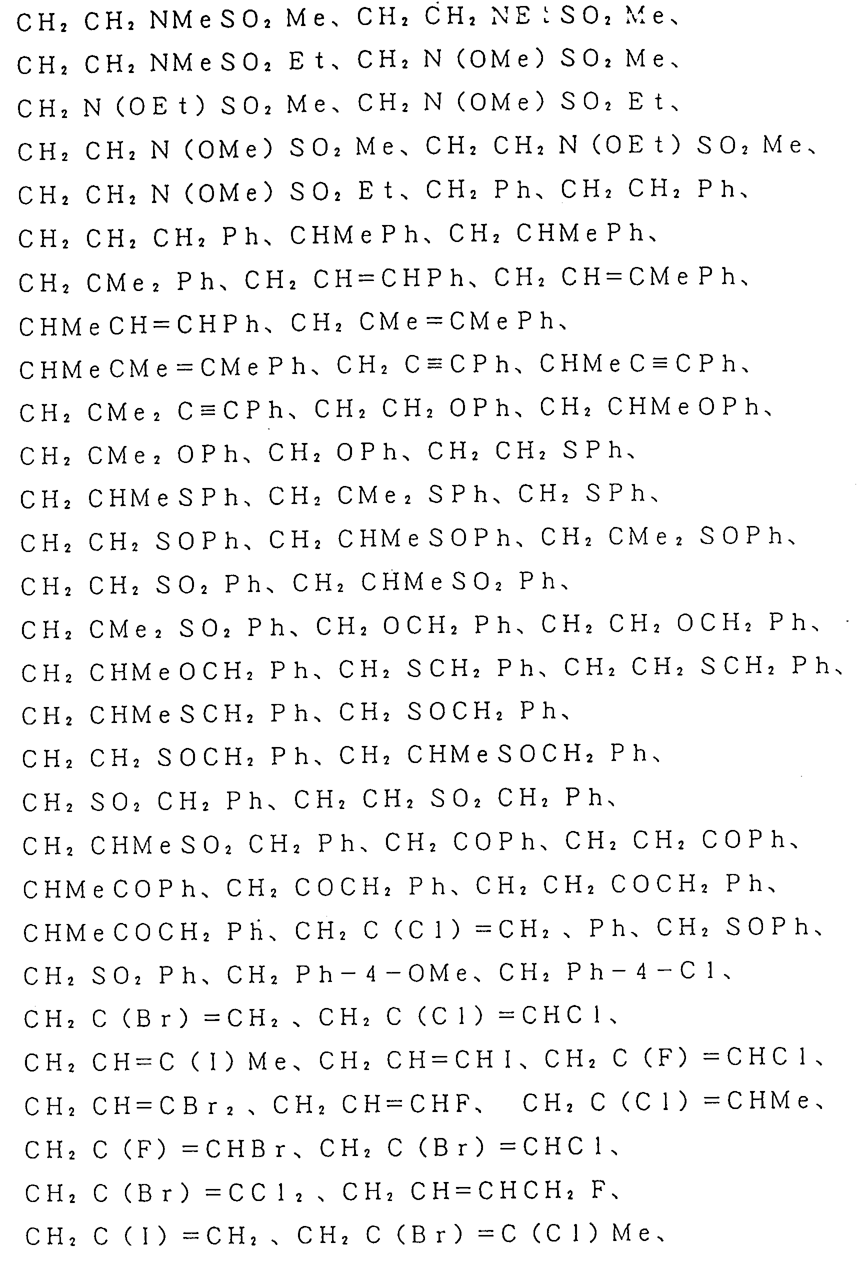 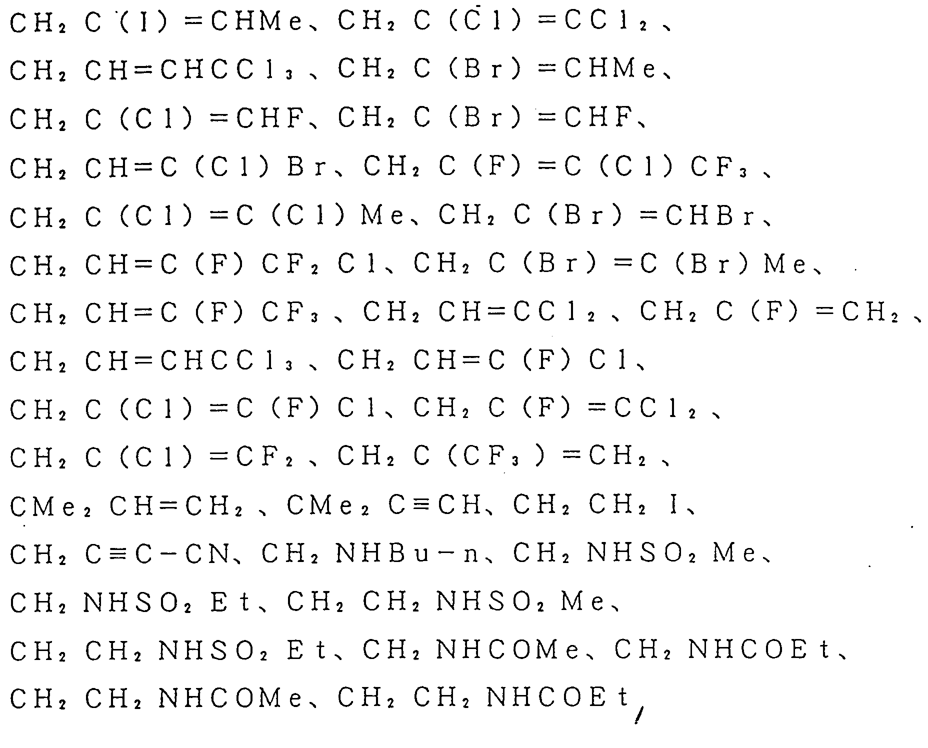 R^{a2} and R^{a3} is 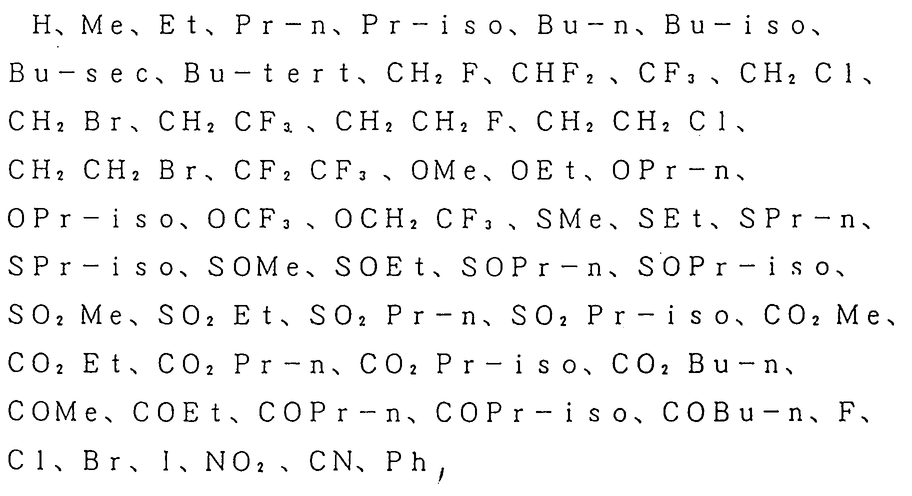 B and D is

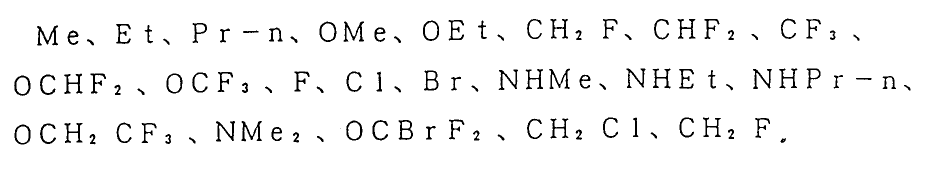

29. The iminosulfonylurea derivative or its salt according to Claim 3, wherein R^{a1} is

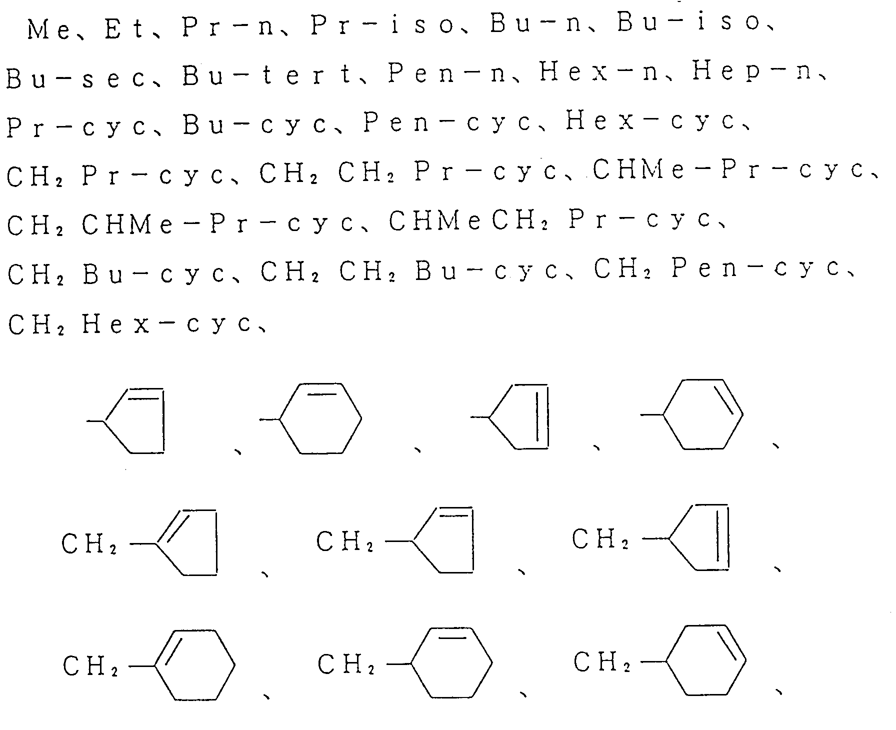

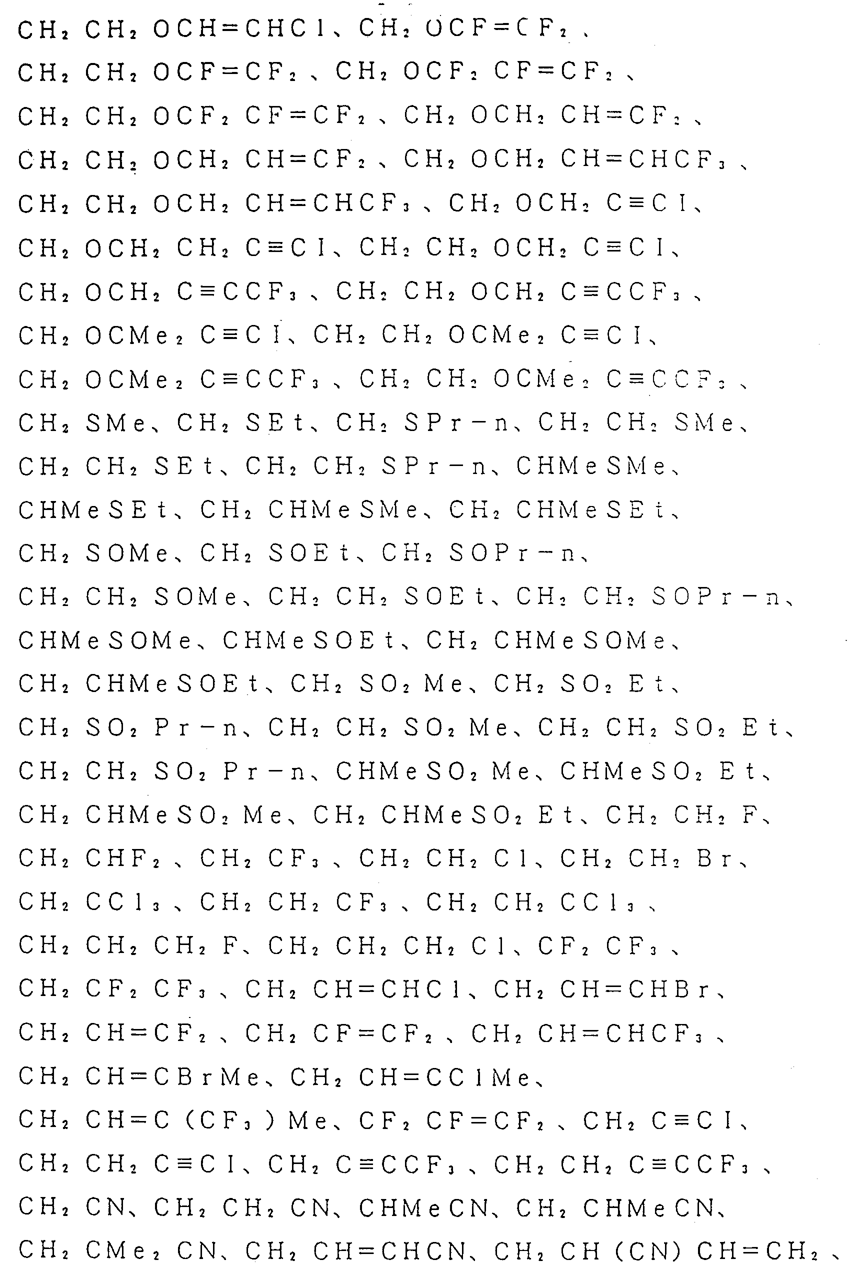

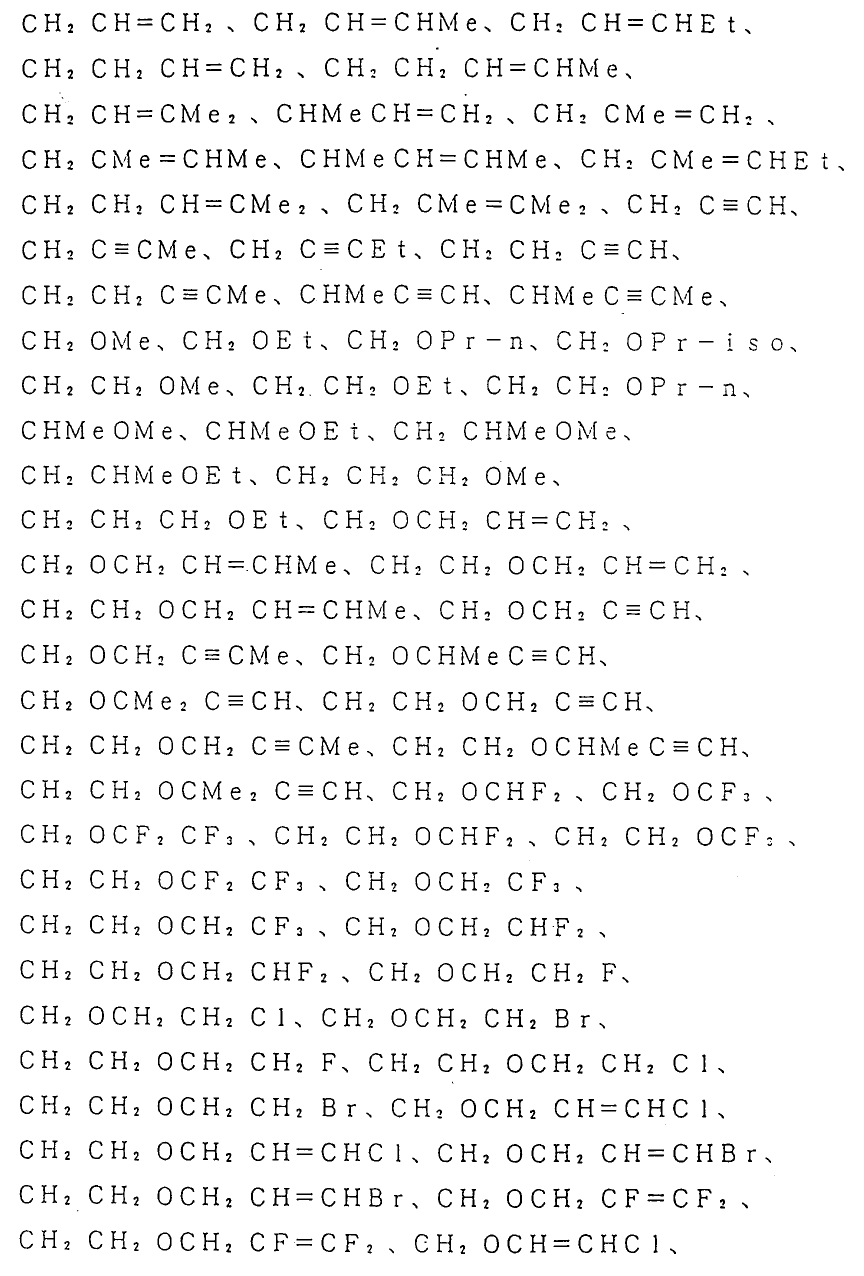

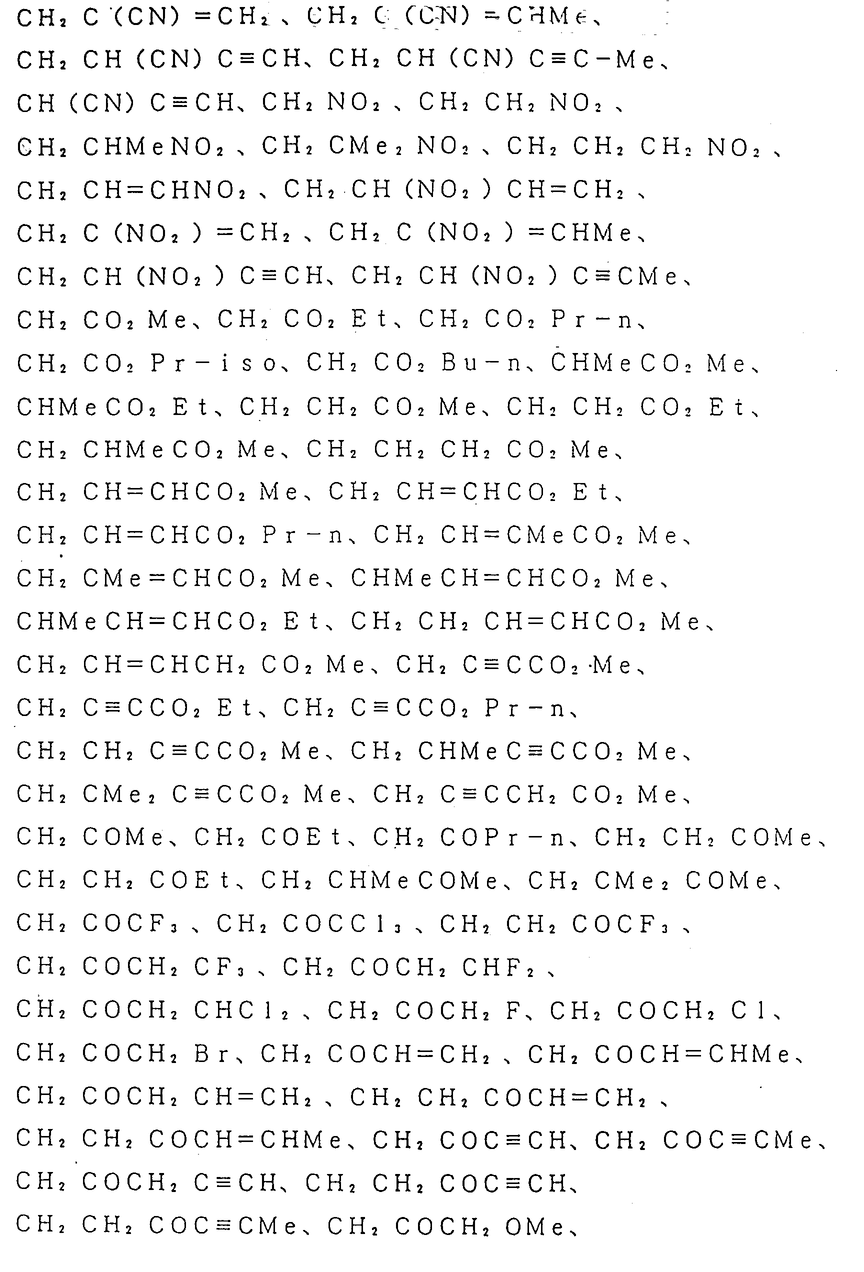

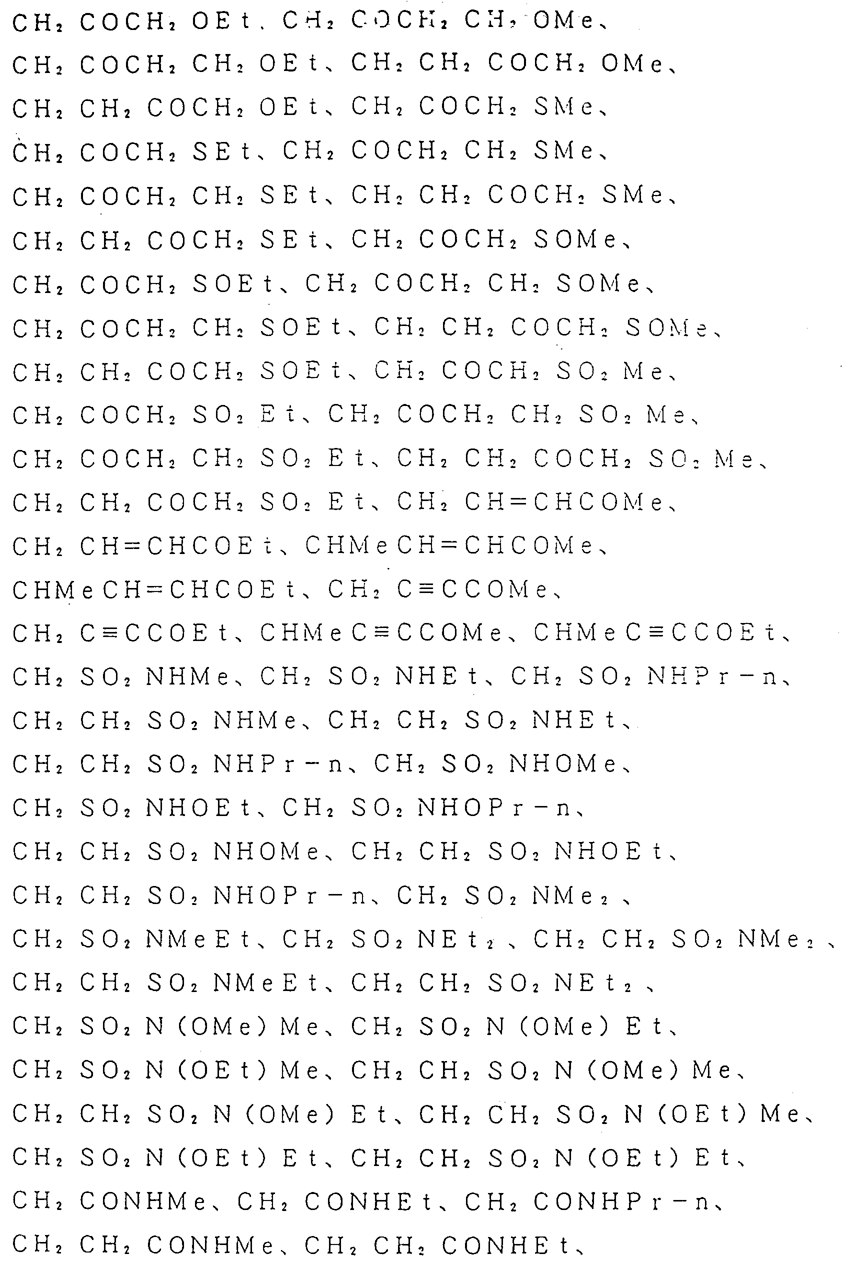

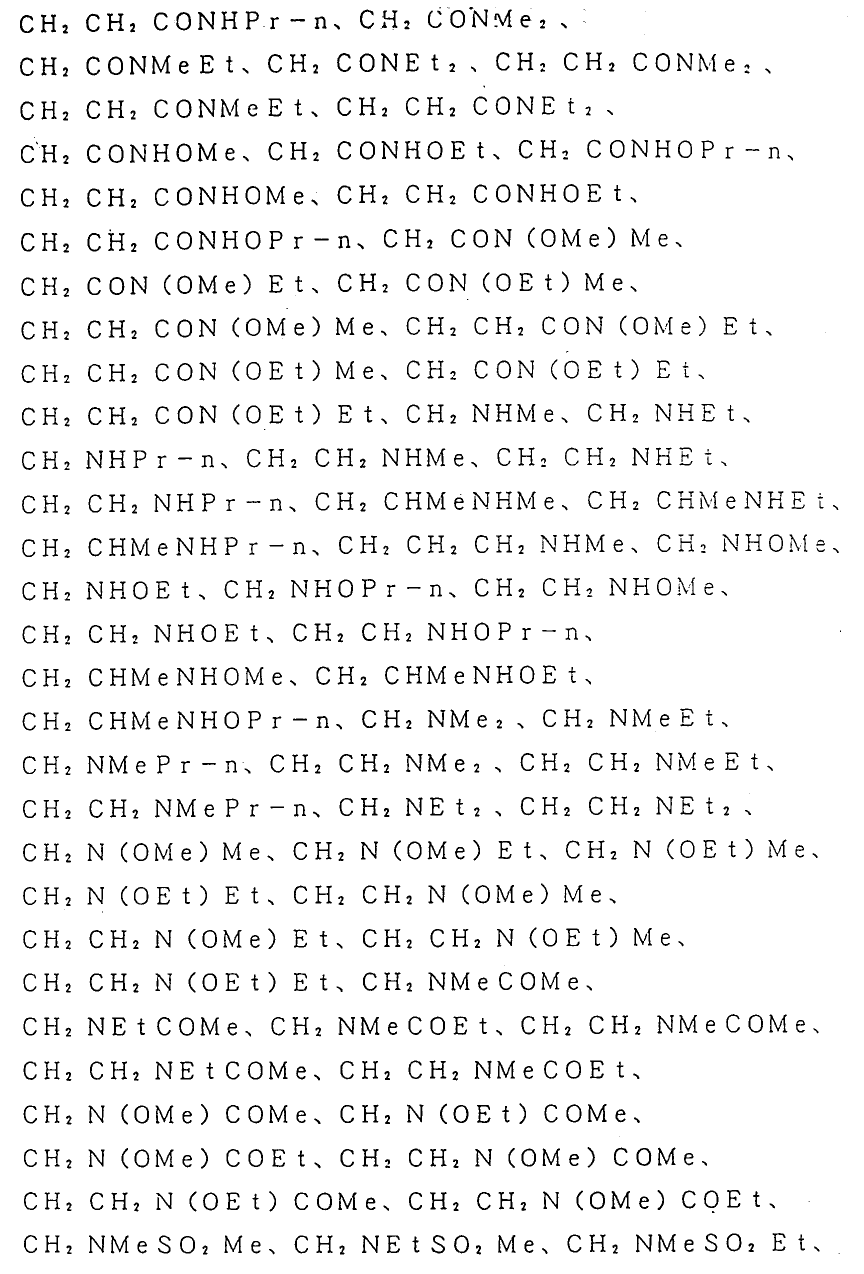

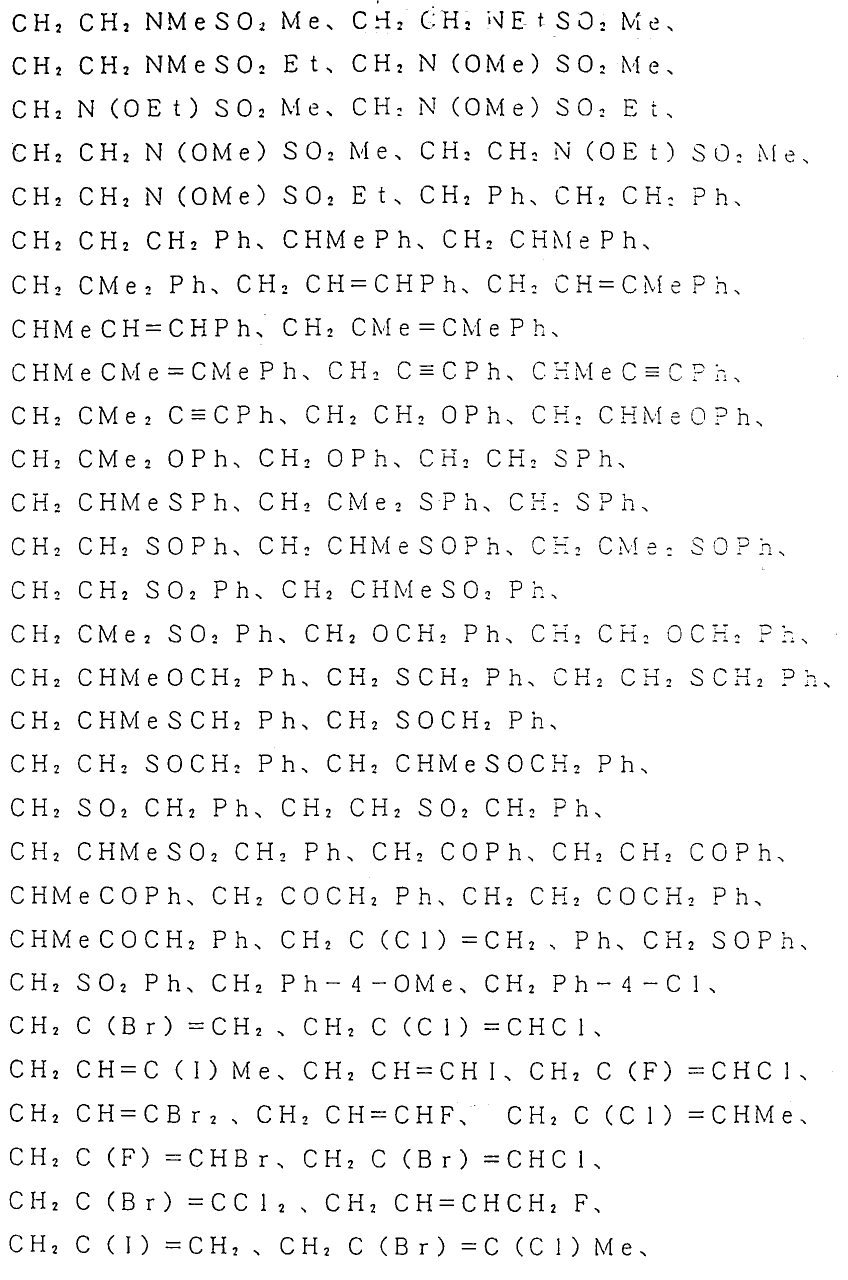

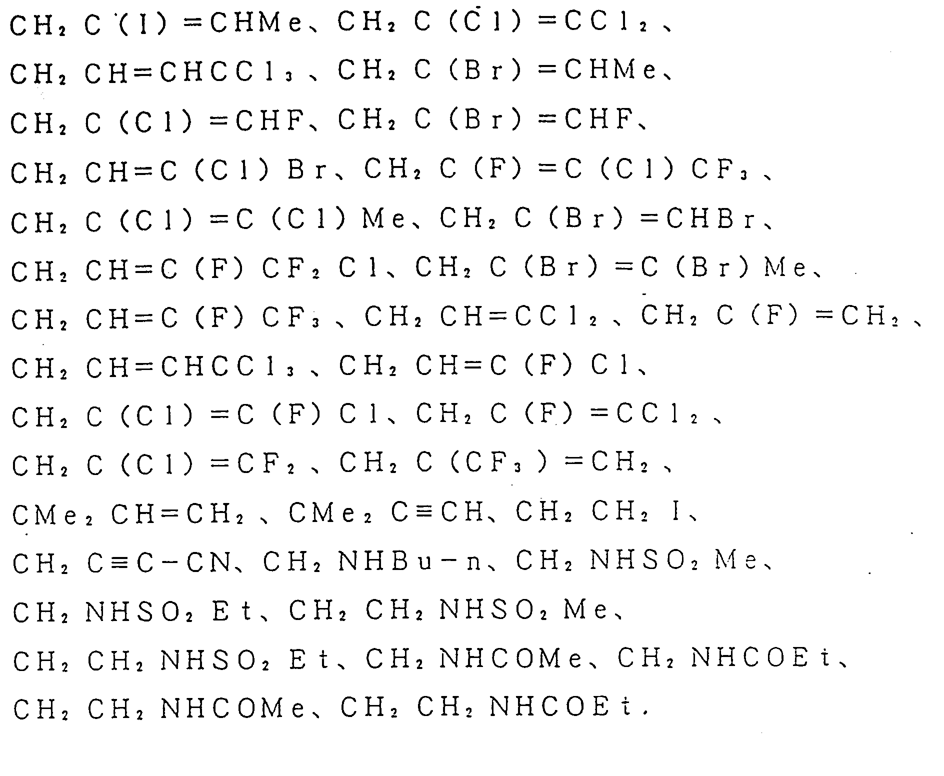

30. The iminosulfonylurea derivative or its salt according to Claim 3, wherein R^{a2} and R^{a3} is

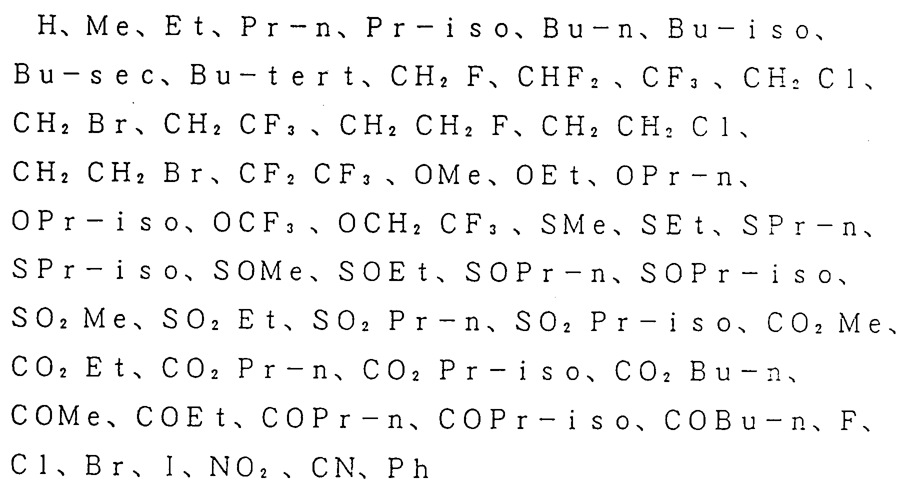

31. The iminosulfonylurea derivative or its salt according to Claim 3, wherein B and D is

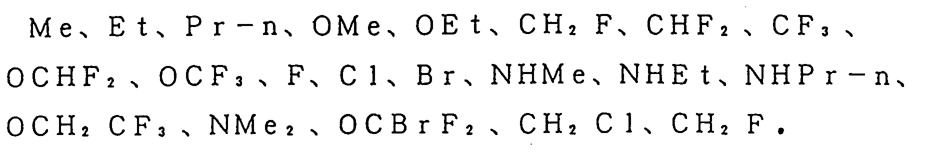

32. A herbicide which contains an iminosulfonylurea derivative defined in Claim 28.

33. A herbicide which contains an iminosulfonylurea derivative defined in Claim 29.

34. A herbicide which contains an iminosulfonylurea derivative defined in Claim 30.

35. A herbicide which contains an iminosulfonylurea derivative defined in Claim 31.

36. A herbicidal and growth control method against weeds, which comprises applying a herbicidally effective amount of an iminosulfonylurea derivative defined in Claim 28.

37. A herbicidal and growth control method against weeds, which comprises applying a herbicidally effective amount of an iminosulfonylurea derivative defined in Claim 29.

38. A herbicidal and growth control method against weeds, which comprises applying a herbicidally effective amount of an iminosulfonylurea derivative defined in Claim 30.

39. A herbicidal and growth control method against weeds, which comprises applying a herbicidally effective amount of an iminosulfonylurea derivative defined in Claim 31.

## Claims (Claims for the following Contracting State(s): ES)

1. A process for the preparation of an iminosulfonylurea derivative of the formula (1) or its salt: 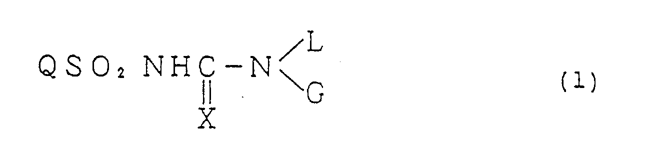
wherein Q is 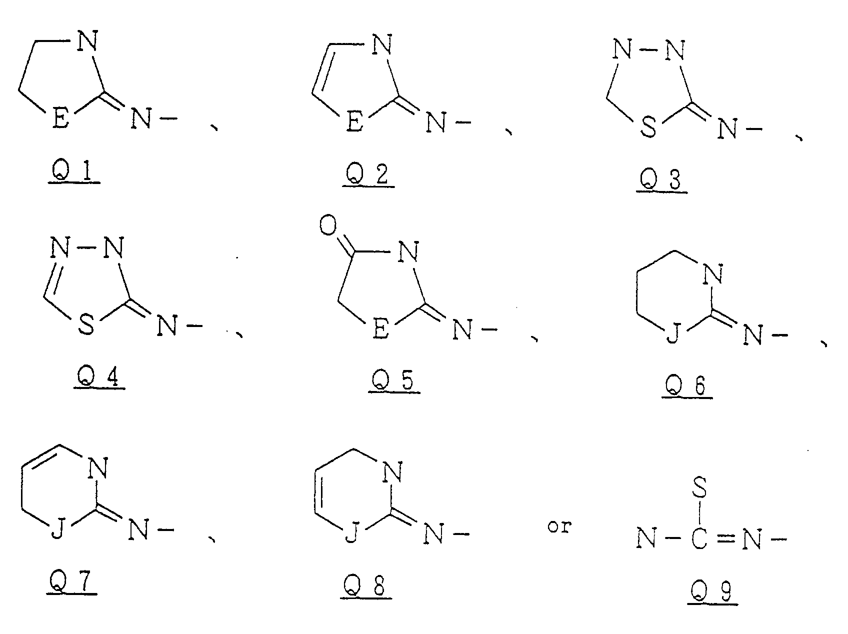 wherein in Q1, Q2 and Q5, E is a sulfur atom, an oxygen atom or a nitrogen atom mono-substituted by a substituent other than a hydrogen atom; in Q6, Q7 and Q8, J is a sulfur atom or an oxygen atom; in Q1 to Q 8, a nitrogen atom in the ring of Q is substituted by a substituent other than a hydrogen atom, and a carbon atom in the ring of Q is optionally substituted; and in Q9, the sulfur atom and the nitrogen atom on the carbon atom to which the imino group of Q is bonded, are substituted by substituents other than hydrogen atoms,
X is an oxygen atom or a sulfur atom,
L is a hydrogen atom, a C₁₋₆ alkyl group, a C₂₋₆ alkenyl group or a C₂₋₆ alkynyl group,
G is 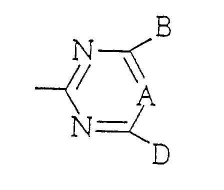
A is a CH group or a nitrogen atom, and
each of B and D which are independent of each other, is a C₁₋₄ alkyl group, a C₁₋₄ alkoxy group, a mono-, di- or polyhalogeno C₁₋₄ alkyl group, a mono-, di- or poly-halogeno C₁₋₄ alkoxy group, a halogen atom, a C₁₋₄ alkylamino group or a di(C₁₋₄ alkyl)amino group,
said process comprising:
a) reacting an amine of formula (2): 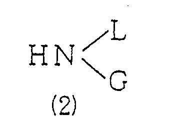 wherein G and L are as defined above, with chlorosulfonyl isocyanate in a solvent, and then reacting the product with an imine of formula (3) or (4):
Q-H (3)
or
Q-H · HZ (4)
wherein Q is as defined above and Z is a halogen atom, in the presence of a base, to obtain a compound of formula (1): 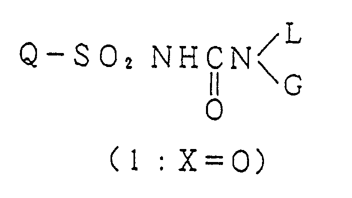 wherein Q, L and G are as defined above and X is an oxygen atom; or
b) reacting an imine of formula (3) or (4):
Q-H (3)
or
Q-H · HZ (4)
wherein Q and Z are as defined above, with an N-chlorosulfonyl carbamate of formula (5):
CℓSO₂ NHCO₂ Y (5)
wherein Y is a C₁₋₆ alkyl group or a phenyl group, in an amount of from 0.5 to 3.0 moles per mole of the imine (3) or (4) in the presence of a base and a solvent, to obtain a compound of formula (6): 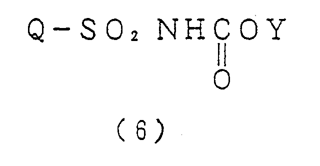 wherein Q and Y are as defined above, which is then heated with a compound of formula (2): 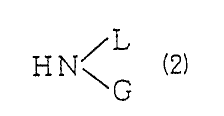 wherein L and G are as defined above, in a solvent, to obtain a compound of formula (1): 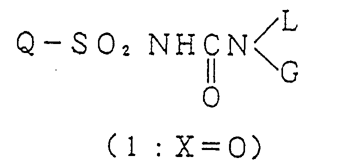 wherein Q, L and G are as defined above and X is an oxygen atom; or
c) reacting a substituted iminosulfonamide derivative of formula (7):
Q-SO₂ NH₂ (7)
wherein Q is as defined above, with a carbamate derivative of formula (8): 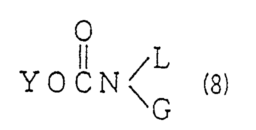 wherein Y, L and G are as defined above, in a solvent in the presence of an inorganic or organic base, to obtain a compound of formula (1): 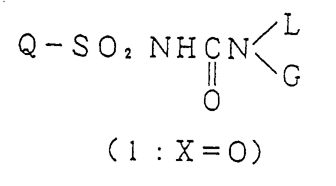 wherein Q, L and G are as defined above and X is an oxygen atom; or
d) reacting a substituted iminosulfonamide derivative of formula (7):
Q-SO₂ NH₂ (7)
wherein Q is as defined above, with chloroformic acid (thio)ester or carbonic acid (thio)ester in a solvent in the presence of a base, to obtain a compound of formula (6): 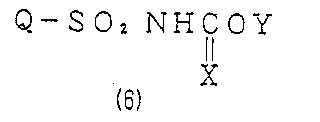 wherein Q, X and Y are as defined above, which is then heated together with a compound of formula (2): 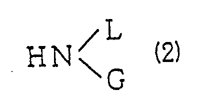 wherein L and G are as defined above, in a solvent, to obtain a compound of formula (1): 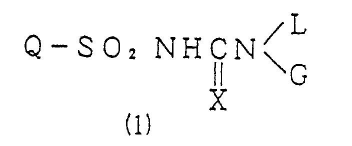 wherein Q, L, G and X are as defined above; or
e) reacting a substituted iminosulfonamide derivative of formula (7):
Q-SO₂ NH₂ (7)
wherein Q is as defined above, with an isothiocyanate derivative of formula (9):
S=C=N-G (9)
wherein G is as defined above, in a solvent in the presence of an inorganic or organic base, to obtain a compound of formula (1): 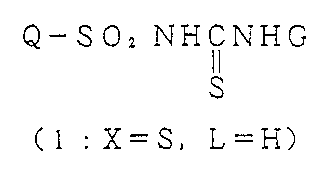 wherein Q and G are as defined above and X is sulfur and L is hydrogen.

2. The process for preparing an iminosulfonylurea derivative of formula (1) or its salt according to Claim 1: 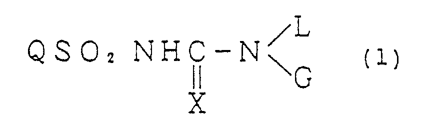
wherein Q is 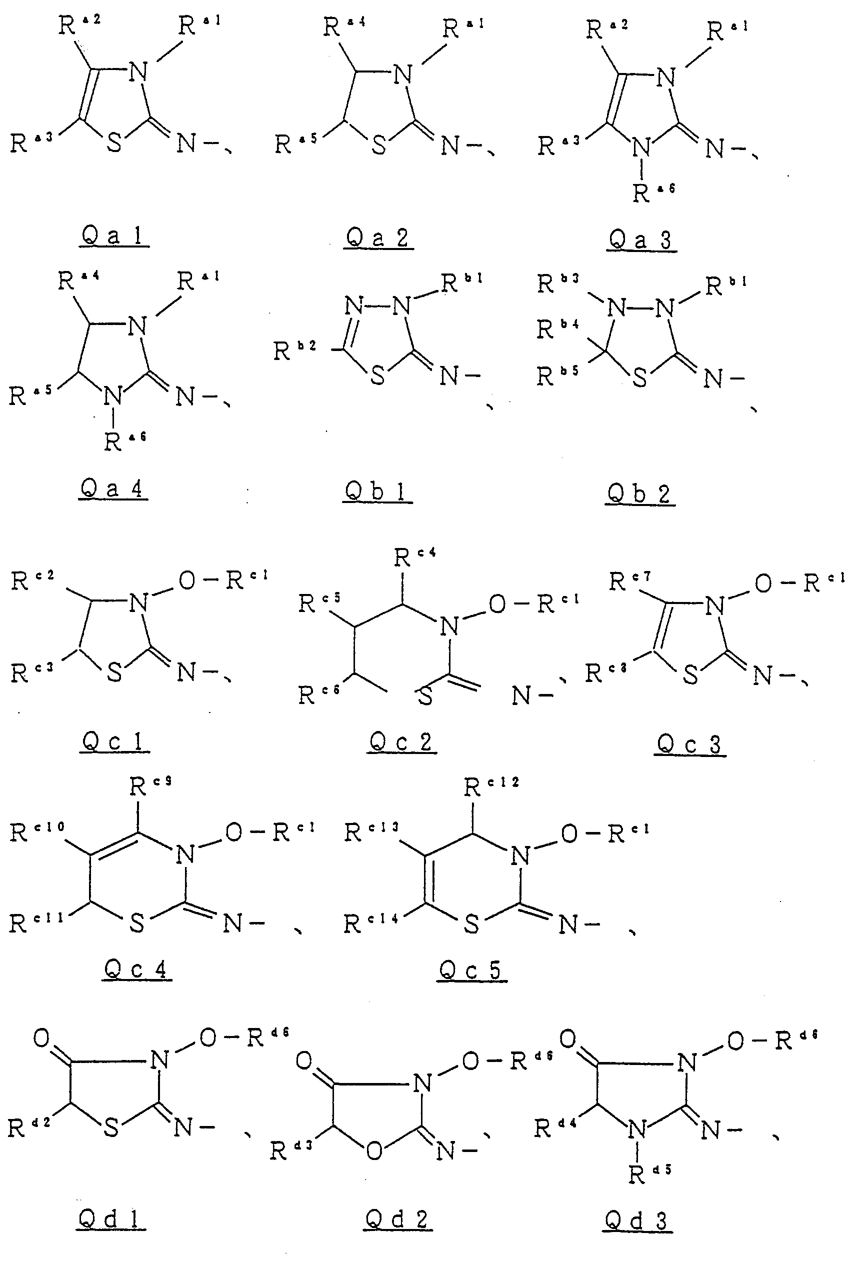 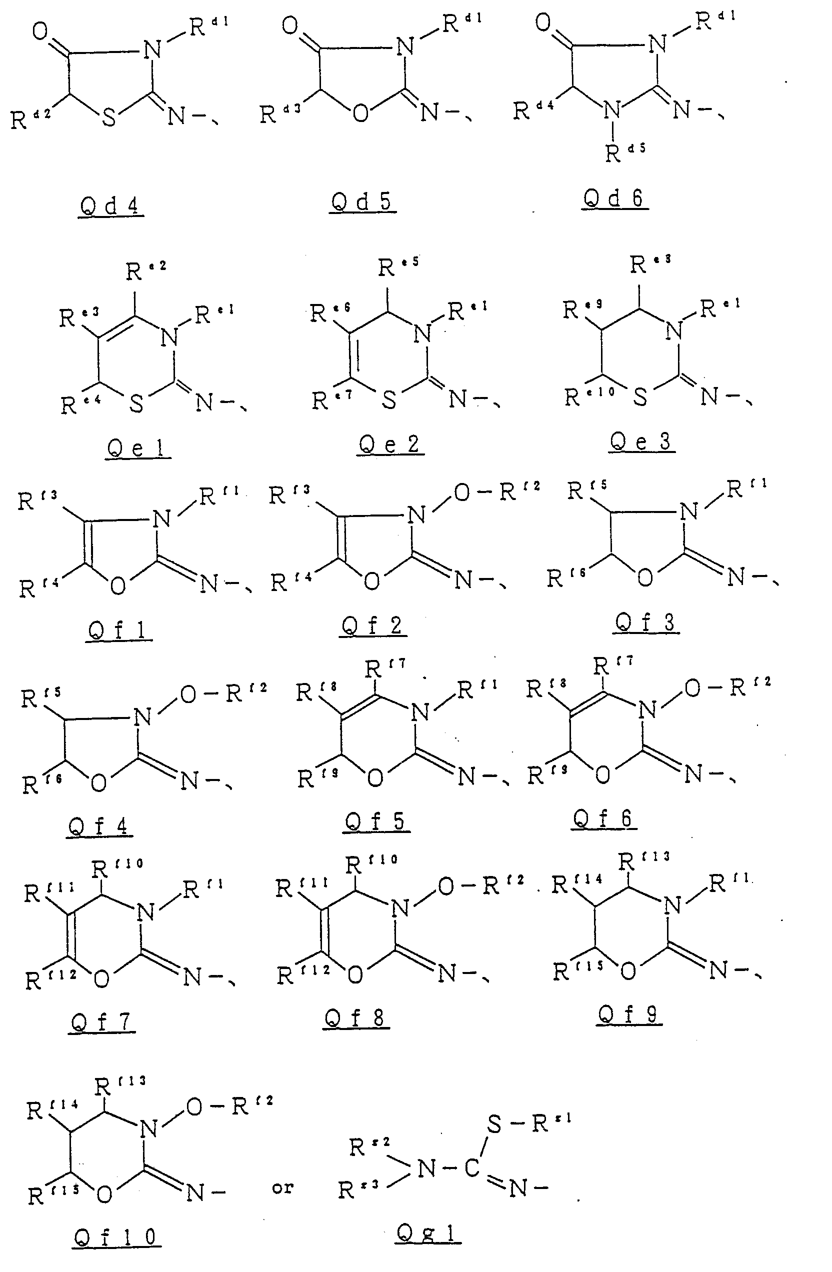
R^{a1} is a C₁₋₈ alkyl group, a C₃₋₇ cycloalkyl group, a C₁₋₆ alkyl group substituted by a C₃₋₇ cycloalkyl group, a C₃₋₇ cycloalkenyl group, a C₁₋₆ alkyl group substituted by a C₃₋₇ cycloalkenyl group, a C₂₋₈ alkenyl group, a C₂₋₈ alkynyl group, a C₁₋₆ alkyl group substituted by a C₁₋₆ alkoxy group, a C₁₋₆ alkyl group substituted by a C₂₋₆ alkenyloxy group, a C₁₋₆ alkyl group substituted by a C₂₋₆ alkynyloxy group, a C₁₋₆ alkyl group substituted by a mono-, di- or poly-halogeno C₁₋₆ alkoxy group, a C₁₋₆ alkyl group substituted by a mono-, di- or poly-halogeno C₂₋₆ alkenyloxy group, a C₁₋₆ alkyl group substituted by a mono-, di- or poly-halogeno C₂₋₆ alkynyloxy group, a C₁₋₆ alkyl group substituted by a C_{₁1-6} alkylthio group, a C₁₋₆ alkyl group substituted by a C₁₋₆ alkylsulfinyl group, a C₁₋₆ alkyl group substituted by a C₁₋₆ alkylsulfonyl group, a mono-, di- or poly-halogeno C₁₋₈ alkyl group, a mono-, di- or poly-halogeno C₂₋₈ alkenyl group, a mono-, di- or poly-halogeno C₂₋₈ alkynyl group, a C₁₋₆ alkyl group substituted by a cyano group, a C₂₋₆ alkenyl group substituted by a cyano group, a C₂₋₆ alkynyl group substituted by a cyano group, a C₁₋₆ alkyl group substituted by a nitro group, a C₂₋₆ alkenyl group substituted by a nitro group, a C₂₋₆ alkynyl group substituted by a nitro group, a C₁₋₆ alkyl group substituted by a C₂₋₇ alkoxycarbonyl group, a C₂₋₆ alkenyl group substituted by a C₂₋₇ alkoxycarbonyl group, a C₂₋₆ alkynyl group substituted by a C₂₋₇ alkoxycarbonyl group, a C₁₋₆ alkyl group substituted by a C₂₋₇ alkylcarbonyl group, a C₁₋₆ alkyl group substituted by a mono-, di- or poly-halogeno C₂₋₇ alkylcarbonyl group, a C₁₋₆ alkyl group substituted by a C₃₋₇ alkenylcarbonyl group, a C₁₋₆ alkyl group substituted by a C₃₋₇ alkynylcarbonyl group, a C₁₋₆ alkyl group substituted by a C₂₋₅ alkylcarbonyl group substituted by a C₁₋₄ alkoxy group, a C₁₋₆ alkyl group substituted by a C₂₋₅ alkylcarbonyl group substituted by a C₁₋₄ alkylthio group, a C₁₋₆ alkyl group substituted by a C₂₋₅ alkylcarbonyl group substituted by a C₁₋₄ alkylsulfinyl group, a C₁₋₆ alkyl group substituted by a C₂₋₅ alkylcarbonyl group substituted by a C₁₋₄ alkylsulfonyl group, a C₂₋₆ alkenyl group substituted by a C₂₋₇ alkylcarbonyl group, a C₂₋₆ alkynyl group substituted by a C₂₋₇ alkylcarbonyl group, a C₁₋₆ alkyl group substituted by a C₁₋₆ alkylsulfamoyl group, a C₁₋₆ alkyl group substituted by a C₁₋₆ alkoxysulfamoyl group, a C₁₋₆ alkyl group substituted by a di(C₁₋₃ alkyl)sulfamoyl group, a C₁₋₆ alkyl group substituted by an N-(C₁₋₃ alkyl)-N-(C₁₋₃ alkoxy)sulfamoyl group, a C₁₋₆ alkyl group substituted by a C₂₋₇ alkylcarbamoyl group, a C₁₋₆ alkyl group substituted by a di(C₁₋₃ alkyl)carbamoyl group, a C₁₋₆ alkyl group substituted by a C₂₋₇ alkoxycarbamoyl group, a C₁₋₆ alkyl group substituted by an N-(C₁₋₃ alkyl)-N-(C₁₋₃ alkoxy)carbamoyl group, a C₁₋₆ alkyl group substituted by a C₁₋₆ alkylamino group, a C₁₋₆ alkyl group substituted by a C₁₋₆ alkoxyamino group, a C₁₋₆ alkyl group substituted by a di(C₁₋₃ alkyl)amino group, a C₁₋₆ alkyl group substituted by an N-(C₁₋₃ alkyl)-N-(C₁₋₃ alkoxy)amino group, a C₁₋₆ alkyl group substituted by an N-(C₂₋₇ alkylcarbonyl)-N-(C₁₋₆ alkyl)amino group, a C₁₋₆ alkyl group substituted by an N-(C₂₋₇ alkylcarbonyl)-N-(C₁₋₆ alkoxy)amino group, a C₁₋₆ alkyl group substituted by an N-(C₁₋₆ alkylsulfonyl)-N-(C₁₋₆ alkyl)amino group, a C₁₋₆ alkyl group substituted by an N-(C₁₋₆ alkylsulfonyl)-N-(C₁₋₆ alkoxy)amino group, a phenyl group (provided that such a phenyl group may be substituted by one or more substituents selected from the group consisting of a halogen atom, a trifluoromethyl group, a nitro group, a C₁₋₆ alkyl group, a C₁₋₆ alkoxy group and a C₂₋₇ alkoxycarbonyl group), a C₁₋₆ alkyl group substituted by a phenyl group (provided that such a phenyl group may be substituted by one or more substituents selected from the group consisting of a halogen atom, a trifluoromethyl group, a nitro group, a C₁₋₆ alkyl group, a C₁₋₆ alkoxy group and a C₂₋₇ alkoxycarbonyl group), a C₂₋₇ alkenyl group substituted by a phenyl group (provided that such a phenyl group may be substituted by one or more substituents selected from the group consisting of a halogen atom, a trifluoromethyl group, a nitro group, a C₁₋₆ alkyl group, a C₁₋₆ alkoxy group and a C₂₋₇ alkoxycarbonyl group), a C₂₋₆ alkynyl group substituted by a phenyl group (provided that such a phenyl group may be substituted by one or more substituents selected from the group consisting of a halogen atom, a trifluoromethyl group, a nitro group, a C₁₋₆ alkyl group, a C₁₋₆ alkoxy group and a C₂₋₇ alkoxycarbonyl group), a C₁₋₆ alkyl group substituted by a phenoxy group (provided that such a phenoxy group may be substituted by one or more substituents selected from the group consisting of a halogen atom, a trifluoromethyl group, a nitro group, a C₁₋₆ alkyl group, a C₁₋₆ alkoxy group and a C₂₋₇ alkoxycarbonyl group), a C₁₋₆ alkyl group substituted by a phenylthio group (provided that such a phenylthio group may be substituted by one or more substituents selected from the group consisting of a halogen atom, a trifluoromethyl group, a nitro group, a C₁₋₆ alkyl group, a C₁₋₆ alkoxy group and a C₂₋₇ alkoxycarbonyl group), a C₁₋₆ alkyl group substituted by a phenylsulfinyl group (provided that such a phenylsulfinyl group may be substituted by one or more substituents selected from the group consisting of a halogen atom, a trifluoromethyl group, a nitro group, a C₁₋₆ alkyl group, a C₁₋₆ alkoxy group and a C₂₋₇ alkoxycarbonyl group), a C₁₋₆ alkyl group substituted by a phenylsulfonyl group (provided that such a phenylsulfonyl group may be substituted by one or more substituents selected from the group consisting of a halogen atom, a trifluoromethyl group, a nitro group, a C₁₋₆ alkyl group, a C₁₋₆ alkoxy group and a C₂₋₇ alkoxycarbonyl group), a C₁₋₆ alkyl group substituted by a benzyloxy group (provided that the phenyl group of such a benzyloxy group may be substituted by one or more substituents selected from the group consisting of a halogen atom, a trifluoromethyl group, a nitro group, a C₁₋₆ alkyl group, a C₁₋₆ alkoxy group and a C₂₋₇ alkoxycarbonyl group), a C₁₋₆ alkyl group substituted by a benzylthio group (provided that the phenyl group of such a benzylthio group may be substituted by one or more substituents selected from the group consisting of a halogen atom, a trifluoromethyl group, a nitro group, a C₁₋₆ alkyl group, a C₁₋₆ alkoxy group and a C₂₋₇ alkoxycarbonyl group), a C₁₋₆ alkyl group substituted by a benzylsulfinyl group (provided that the phenyl group of such a benzylsulfinyl group may be substituted by one or more substituents selected from the group consisting of a halogen atom, a trifluoromethyl group, a nitro group, a C₁₋₆ alkyl group, a C₁₋₆ alkoxy group and a C₂₋₇ alkoxycarbonyl group), a C₁₋₆ alkyl group substituted by a benzylsulfonyl group (provided that the phenyl group of such a benzylsulfonyl group may be substituted by one or more substituents selected from the group consisting of a halogen atom, a trifluoromethyl group, a nitro group, a C₁₋₆ alkyl group, a C₁₋₆ alkoxy group and a C₂₋₇ alkoxycarbonyl group), a C₁₋₆ alkyl group substituted by a phenylcarbonyl group (provided that such a phenylcarbonyl group may be substituted by one or more substituents selected from the group consisting of a halogen atom, a trifluoromethyl group, a nitro group, a C₁₋₆ alkyl group, a C₁₋₆ alkoxy group and a C₂₋₇ alkoxycarbonyl group), a C₁₋₆ alkyl group substituted by a benzylcarbonyl group (provided that the phenyl group of such a benzylcarbonyl group may be substituted by one or more substituents selected from the group consisting of a halogen atom, a trifluoromethyl group, a nitro group, a C₁₋₆ alkyl group, a C₁₋₆ alkoxy group and a C₂₋₇ alkoxycarbonyl group), a C₁₋₆ alkyl group substituted by an amino group substituted by a C₁₋₄ alkylsulfonyl group, or a C₁₋₆ alkyl group substituted by an amino group substituted by a C₂₋₄ alkylcarbonyl group,
each of R^{a2} and R^{a3} which are independent of each other, is a hydrogen atom, a C₁₋₆ alkyl group, a mono-, di- or poly-halogeno C₁₋₆ alkyl group, a C₁₋₆ alkoxy group, a mono-, di or poly-halogeno C₁₋₆ alkoxy group, a C₁₋₆ alkylthio group, a C₁₋₆ alkylsulfinyl group, a C₁₋₆ alkylsulfonyl group, a C₂₋₇ alkoxycarbonyl group, a C₂₋₇ alkylcarbonyl group, a halogen atom, a nitro group, a cyano group, or a phenyl group (provided that such a phenyl group may be substituted by one or more substituents selected from the group consisting of a halogen atom, a trifluoromethyl group, a nitro group, a C₁₋₆ alkyl group, a C₁₋₆ alkoxy group and a C₂₋₇ alkoxycarbonyl group),
each of R^{a4} and R^{a5} which are independent of each other, is a hydrogen atom, a C₁₋₈ alkyl group, a C₂₋₈ alkenyl group, a C₂₋₈ alkynyl group, or a phenyl group (provided that such a phenyl group may be substituted by one or more substituents selected from the group consisting of a halogen atom, a trifluoromethyl group, a nitro group, a C₁₋₆ alkyl group, a C₁₋₆ alkoxy group and a C₂₋₇ alkoxycarbonyl group),
R^{a6} is a C₁₋₈ alkyl group, a C₂₋₈ alkenyl group, a C₂₋₈ alkynyl group or a phenyl group (provided that such a phenyl group may be substituted by one or more substituents selected from the group consisting of a halogen atom, a trifluoromethyl group, a nitro group, a C₁₋₆ alkyl group, a C₁₋₆ alkoxy group and a C₂₋₇ alkoxycarbonyl group),
R^{b1} is a C₁₋₈ alkyl group, a C₃₋₇ cycloalkyl group, a C₁₋₆ alkyl group substituted by a C₃₋₇ cycloalkyl group, a C₃₋₇ cycloalkenyl group, a C₁₋₆ alkyl group substituted by a C₃₋₇ cycloalkenyl group, a C₂₋₈ alkenyl group, a C₂₋₈ alkynyl group, a C₁₋₆ alkyl group substituted by a C₁₋₆ alkoxy group, a C₁₋₆ alkyl group substituted by a C₂₋₆ alkenyloxy group, a C₁₋₆ alkyl group substituted by a C₂₋₆ alkynyloxy group, a C₁₋₆ alkyl group substituted by a mono-, di or poly-halogeno C₁₋₆ alkoxy group, a C₁₋₆ alkyl group substituted by a mono-, di- or poly-halogeno C₂₋₆ alkenyloxy group, a C₁₋₆ alkyl group substituted by a mono-, di- or poly-halogeno C₂₋₆ alkynyloxy group, a C₁₋₆ alkyl group substituted by a C₁₋₆ alkylthio group, a C₁₋₆ alkyl group substituted by a C₁₋₆ alkylsulfinyl group, a C₁₋₆ alkyl group substituted by a C₁₋₆ alkylsulfonyl group, a mono-, di- or poly-halogeno C₁₋₈ alkyl group, a mono-, di- or poly-halogeno C₂₋₈ alkenyl group, a mono-, di- or poly-halogeno C₂₋₈ alkynyl group, a C₁₋₆ alkyl group substituted by a cyano group, a C₂₋₆ alkenyl group substituted by a cyano group, a C₂₋₆ alkynyl group substituted by a cyano group, a C₁₋₆ alkyl group substituted by a nitro group, a C₂₋₆ alkenyl group substituted by a nitro group, a C₂₋₆ alkynyl group substituted by a nitro group, a C₁₋₆ alkyl group substituted by a C₂₋₇ alkoxycarbonyl group, a C₂₋₆ alkenyl group substituted by a C₂₋₇ alkoxycarbonyl group, a C₂₋₆ alkynyl group substituted by a C₂₋₇ alkoxycarbonyl group, a C₁₋₆ alkyl group substituted by a C₂₋₇ alkylcarbonyl group, a C₁₋₆ alkyl group substituted by a mono-, di- or poly-halogeno C₂₋₇ alkylcarbonyl group, a C₁₋₆ alkyl group substituted by a C₃₋₇ alkenylcarbonyl group, a C₁₋₆ alkyl group substituted by a C₃₋₇ alkynylcarbonyl group, a C₁₋₆ alkyl group substituted by a C₂₋₅ alkylcarbonyl group substituted by a C₁₋₄ alkoxy group, a C₁₋₆ alkyl group substituted by a C₂₋₅ alkylcarbonyl group substituted by a C₁₋₄ alkylthio group, a C₁₋₆ alkyl group substituted by a C₂₋₅ alkylcarbonyl group substituted by a C₁₋₄ alkylsulfinyl group, a C₁₋₆ alkyl group substituted by a C₂₋₅ alkylcarbonyl group substituted by a C₁₋₄ alkylsulfonyl group, a C₂₋₆ alkenyl group substituted by a C₂₋₇ alkylcarbonyl group, a C₂₋₆ alkynyl group substituted by a C₂₋₇ alkylcarbonyl group, a C₁₋₆ alkyl group substituted by a C₁₋₆ alkylsulfamoyl group, a C₁₋₆ alkyl group substituted by a C₁₋₆ alkoxysulfamoyl group, a C₁₋₆ alkyl group substituted by a di(C₁₋₃ alkyl)sulfamoyl group, a C₁₋₆ alkyl group substituted by an N-(C₁₋₃ alkyl)-N-(C₁₋₃ alkoxy)sulfamoyl group, a C₁₋₆ alkyl group substituted by a C₂₋₇ alkylcarbamoyl group, a C₁₋₆ alkyl group substituted by a di(C₁₋₃ alkyl)carbamoyl group, a C₁₋₆ alkyl group substituted by a C₂₋₇ alkoxycarbamoyl group, a C₁₋₆ alkyl group substituted by an N-(C₁₋₃ alkyl)-N-(C₁₋₃ alkoxy)carbamoyl group, a C₁₋₆ alkyl group substituted by a C₁₋₆ alkylamino group, a C₁₋₆ alkyl group substituted by a C₁₋₆ alkoxyamino group, a C₁₋₆ alkyl group substituted by a di(C₁₋₃ alkyl)amino group, a C₁₋₆ alkyl group substituted by an N-(C₁₋₃ alkyl)-N-(C₁₋₃ alkoxy)amino group, a C₁₋₆ alkyl group substituted by an N-(C₂₋₇ alkylcarbonyl)-N-(C₁₋₆ alkyl)amino group, a C₁₋₆ alkyl group substituted by an N-(C₂₋₇ alkylcarbonyl)-N-(C₁₋₆ alkoxy)amino group, a C₁₋₆ alkyl group substituted by an N-(C₁₋₆ alkylsulfonyl)-N-(C₁₋₆ alkyl)amino group, a C₁₋₆ alkyl group substituted by an N-(C₁₋₆ alkylsulfonyl)-N-(C₁₋₆ alkoxy)amino group, a phenyl group (provided that such a phenyl group may be substituted by one or more substituents selected from the group consisting of a halogen atom, a trifluoromethyl group, a nitro group, a C₁₋₆ alkyl group, a C₁₋₆ alkoxy group and a C₂₋₇ alkoxycarbonyl group), a C₁₋₆ alkyl group substituted by a phenyl group (provided that such a phenyl group may be substituted by one or more substituents selected from the group consisting of a halogen atom, a trifluoromethyl group, a nitro group, a C₁₋₆ alkyl group, a C₁₋₆ alkoxy group and a C₂₋₇ alkoxycarbonyl group), a C₂₋₇ alkenyl group substituted by a phenyl group (provided that such a phenyl group may be substituted by one or more substituents selected from the group consisting of a halogen atom, a trifluoromethyl group, a nitro group, a C₁₋₆ alkyl group, a C₁₋₆ alkoxy group and a C₂₋₇ alkoxycarbonyl group), a C₂₋₆ alkynyl group substituted by a phenyl group (provided that such a phenyl group may be substituted by one or more substituents selected from the group consisting of a halogen atom, a trifluoromethyl group, a nitro group, a C₁₋₆ alkyl group, a C₁₋₆ alkoxy group and a C₂₋₇ alkoxycarbonyl group), a C₁₋₆ alkyl group substituted by a phenoxy group (provided that such a phenoxy group may be substituted by one or more substituents selected from the group consisting of a halogen atom, a trifluoromethyl group, a nitro group, a C₁₋₆ alkyl group, a C₁₋₆ alkoxy group and a C₂₋₇ alkoxycarbonyl group), a C₁₋₆ alkyl group substituted by a phenylthio group (provided that such a phenylthio group may be substituted by one or more substituents selected from the group consisting of a halogen atom, a trifluoromethyl group, a nitro group, a C₁₋₆ alkyl group, a C₁₋₆ alkoxy group and a C₂₋₇ alkoxycarbonyl group), a C₁₋₆ alkyl group substituted by a phenylsulfinyl group (provided that such a phenylsulfinyl group may be substituted by one or more substituents selected from the group consisting of a halogen atom, a trifluoromethyl group, a nitro group, a C₁₋₆ alkyl group, a C₁₋₆ alkoxy group and a C₂₋₇ alkoxycarbonyl group), a C₁₋₆ alkyl group substituted by a phenylsulfonyl group (provided that such a phenylsulfonyl group may be substituted by one or more substituents selected from the group consisting of a halogen atom, a trifluoromethyl group, a nitro group, a C₁₋₆ alkyl group, a C₁₋₆ alkoxy group and a C₂₋₇ alkoxycarbonyl group), a C₁₋₆ alkyl group substituted by a benzyloxy group (provided that the phenyl group of such a benzyloxy group may be substituted by one or more substituents selected from the group consisting of a halogen atom, a trifluoromethyl group, a nitro group, a C₁₋₆ alkyl group, a C₁₋₆ alkoxy group and a C₂₋₇ alkoxycarbonyl group), a C₁₋₆ alkyl group substituted by a benzylthio group (provided that the phenyl group of such a benzylthio group may be substituted by one or more substituents selected from the group consisting of a halogen atom, a trifluoromethyl group, a nitro group, a C₁₋₆ alkyl group, a C₁₋₆ alkoxy group and a C₂₋₇ alkoxycarbonyl group), a C₁₋₆ alkyl group substituted by a benzylsulfinyl group (provided that the phenyl group of such a benzylsulfinyl group may be substituted by one or more substituents selected from the group consisting of a halogen atom, a trifluoromethyl group, a nitro group, a C₁₋₆ alkyl group, a C₁₋₆ alkoxy group and a C₂₋₇ alkoxycarbonyl group), a C₁₋₆ alkyl group substituted by a benzylsulfonyl group (provided that the phenyl group of such a benzylsulfonyl group may be substituted by one or more substituents selected from the group consisting of a halogen atom, a trifluoromethyl group, a nitro group, a C₁₋₆ alkyl group, a C₁₋₆ alkoxy group and a C₂₋₇ alkoxycarbonyl group), a C₁₋₆ alkyl group substituted by a phenylcarbonyl group (provided that such a phenylcarbonyl group may be substituted by one or more substituents selected from the group consisting of a halogen atom, a trifluoromethyl group, a nitro group, a C₁₋₆ alkyl group, a C₁₋₆ alkoxy group and a C₂₋₇ alkoxycarbonyl group), a C₁₋₆ alkyl group substituted by a benzylcarbonyl group (provided that the phenyl group of such a benzylcarbonyl group may be substituted by one or more substituents selected from the group consisting of a halogen atom, a trifluoromethyl group, a nitro group, a C₁₋₆ alkyl group, a C₁₋₆ alkoxy group and a C₂₋₇ alkoxycarbonyl group), a C₁₋₆ alkyl group substituted by an amino group substituted by a C₁₋₄ alkylsulfonyl group, or a C₁₋₆ alkyl group substituted by an amino group substituted by a C₂₋₄ alkylcarbonyl group,
R^{b2} is a hydrogen atom, a C₁₋₆ alkyl group, a mono-, di- or poly-halogeno C₁₋₆ alkyl group, a C₁₋₆ alkoxy group, a mono-, di- or poly-halogeno C₁₋₆ alkoxy group, a C₁₋₆ alkylthio group, a C₁₋₆ alkylsulfinyl group, a C₁₋₆ alkylsulfonyl group, a C₂₋₇ alkoxycarbonyl group, a C₂₋₇ alkylcarbonyl group, a halogen atom, a nitro group, a cyano group, or a phenyl group (provided that such a phenyl group may be substituted by one or more substituents selected from the group consisting of a halogen atom, a trifluoromethyl group, a nitro group, a C₁₋₆ alkyl group, a C₁₋₆ alkoxy group and a C₂₋₇ alkoxycarbonyl group),
R^{b3} is a C₁₋₆ alkyl group, a C₂₋₆ alkenyl group or a C₂₋₆ alkynyl group,
R^{b4} is a hydrogen atom, or a C₁₋₆ alkyl group,
R^{b5} is a hydrogen atom, or a C₁₋₆ alkyl group,
R^{c1} is a C₁₋₈ alkyl group, a C₃₋₇ cycloalkyl group, a C₁₋₆ alkyl group substituted by a C₃₋₇ cycloalkyl group, a C₃₋₇ cycloalkenyl group, a C₁₋₆ alkyl group substituted by a C₃₋₇ cycloalkenyl group, a C₂₋₈ alkenyl group, a C₂₋₈ alkynyl group, a C₁₋₆ alkyl group substituted by a C₁₋₆ alkoxy group, a C₁₋₆ alkyl group substituted by a C₂₋₆ alkenyloxy group, a C₁₋₆ alkyl group substituted by a C₂₋₆ alkynyloxy group, a C₁₋₆ alkyl group substituted by a mono-, di- or poly-halogeno C₁₋₆ alkoxy group, a C₁₋₆ alkyl group substituted by a mono-, di- or poly-halogeno C₂₋₆ alkenyloxy group, a C₁₋₆ alkyl group substituted by a mono-, di- or poly-halogeno C₂₋₆ alkynyloxy group, a C₁₋₆ alkyl group substituted by a C₁₋₆ alkylthio group, a C₁₋₆ alkyl group substituted by a C₁₋₆ alkylsulfinyl group, a C₁₋₆ alkyl group substituted by a C₁₋₆ alkylsulfonyl group, a mono-, di- or poly-halogeno C₁₋₈ alkyl group, a mono-, di- or poly-halogeno C₂₋₈ alkenyl group, a mono-, di- or poly-halogeno C₂₋₈ alkynyl group, a C₁₋₆ alkyl group substituted by a cyano group, a C₂₋₆ alkenyl group substituted by a cyano group, a C₂₋₆ alkynyl group substituted by a cyano group, a C₁₋₆ alkyl group substituted by a nitro group, a C₂₋₆ alkenyl group substituted by a nitro group, a C₂₋₆ alkynyl group substituted by a nitro group, a C₁₋₆ alkyl group substituted by a C₂₋₇ alkoxycarbonyl group, a C₂₋₆ alkenyl group substituted by a C₂₋₇ alkoxycarbonyl group, a C₂₋₆ alkynyl group substituted by a C₂₋₇ alkoxycarbonyl group, a C₁₋₆ alkyl group substituted by a C₂₋₇ alkylcarbonyl group, a C₁₋₆ alkyl group substituted by a mono-, di- or poly-halogeno C₂₋₇ alkylcarbonyl group, a C₁₋₆ alkyl group substituted by a C₃₋₇ alkenylcarbonyl group, a C₁₋₆ alkyl group substituted by a C₃₋₇ alkynylcarbonyl group, a C₁₋₆ alkyl group substituted by a C₂₋₅ alkylcarbonyl group substituted by a C₁₋₄ alkoxy group, a C₁₋₆ alkyl group substituted by a C₂₋₅ alkylcarbonyl group substituted by a C₁₋₄ alkylthio group, a C₁₋₆ alkyl group substituted by a C₂₋₅ alkylcarbonyl group substituted by a C₁₋₄ alkylsulfinyl group, a C₁₋₆ alkyl group substituted by a C₂₋₅ alkylcarbonyl group substituted by a C₁₋₄ alkylsulfonyl group, a C₂₋₆ alkenyl group substituted by a C₂₋₇ alkylcarbonyl group, a C₂₋₆ alkynyl group substituted by a C₂₋₇ alkylcarbonyl group, a C₁₋₆ alkyl group substituted by a C₁₋₆ alkylsulfamoyl group, a C₁₋₆ alkyl group substituted by a C₁₋₆ alkoxysulfamoyl group, a C₁₋₆ alkyl group substituted by a di(C₁₋₃ alkyl)sulfamoyl group, a C₁₋₆ alkyl group substituted by an N-(C₁₋₃ alkyl)-N-(C₁₋₃ alkoxy)sulfamoyl group, a C₁₋₆ alkyl group substituted by a C₂₋₇ alkylcarbamoyl group, a C₁₋₆ alkyl group substituted by a di(C₁₋₃ alkyl)carbamoyl group, a C₁₋₆ alkyl group substituted by a C₂₋₇ alkoxycarbamoyl group, a C₁₋₆ alkyl group substituted by an N-(C₁₋₃ alkyl)-N-(C₁₋₃ alkoxy)carbamoyl group, a C₁₋₆ alkyl group substituted by a C₁₋₆ alkylamino group, a C₁₋₆ alkyl group substituted by a C₁₋₆ alkoxyamino group, a C₁₋₆ alkyl group substituted by a di(C₁₋₃ alkyl)amino group, a C₁₋₆ alkyl group substituted by an N-(C₁₋₃ alkyl)-N-(C₁₋₃ alkoxy)amino group, a C₁₋₆ alkyl group substituted by an N-(C₂₋₇ alkylcarbonyl)-N-(C₁₋₆ alkyl)amino group, a C₁₋₆ alkyl group substituted by an N-(C₂₋₇ alkylcarbonyl)-N-(C₁₋₆ alkoxy)amino group, a C₁₋₆ alkyl group substituted by an N-(C₁₋₆ alkylsulfonyl)-N-(C₁₋₆ alkyl)amino group, a C₁₋₆ alkyl group substituted by an N-(C₁₋₆ alkylsulfonyl)-N-(C₁₋₆ alkoxy)amino group, a phenyl group (provided that such a phenyl group may be substituted by one or more substituents selected from the group consisting of a halogen atom, a trifluoromethyl group, a nitro group, a C₁₋₆ alkyl group, a C₁₋₆ alkoxy group and a C₂₋₇ alkoxycarbonyl group), a C₁₋₆ alkyl group substituted by a phenyl group (provided that such a phenyl group may be substituted by one or more substituents selected from the group consisting of a halogen atom, a trifluoromethyl group, a nitro group, a C₁₋₆ alkyl group, a C₁₋₆ alkoxy group and a C₂₋₇ alkoxycarbonyl group), a C₂₋₇ alkenyl group substituted by a phenyl group (provided that such a phenyl group may be substituted by one or more substituents selected from the group consisting of a halogen atom, a trifluoromethyl group, a nitro group, a C₁₋₆ alkyl group, a C₁₋₆ alkoxy group and a C₂₋₇ alkoxycarbonyl group), a C₂₋₆ alkynyl group substituted by a phenyl group (provided that such a phenyl group may be substituted by one or more substituents selected from the group consisting of a halogen atom, a trifluoromethyl group, a nitro group, a C₁₋₆ alkyl group, a C₁₋₆ alkoxy group and a C₂₋₇ alkoxycarbonyl group), a C₁₋₆ alkyl group substituted by a phenoxy group (provided that such a phenoxy group may be substituted by one or more substituents selected from the group consisting of a halogen atom, a trifluoromethyl group, a nitro group, a C₁₋₆ alkyl group, a C₁₋₆ alkoxy group and a C₂₋₇ alkoxycarbonyl group), a C₁₋₆ alkyl group substituted by a phenylthio group (provided that such a phenylthio group may be substituted by one or more substituents selected from the group consisting of a halogen atom, a trifluoromethyl group, a nitro group, a C₁₋₆ alkyl group, a C₁₋₆ alkoxy group and a C₂₋₇ alkoxycarbonyl group), a C₁₋₆ alkyl group substituted by a phenylsulfinyl group (provided that such a phenylsulfinyl group may be substituted by one or more substituents selected from the group consisting of a halogen atom, a trifluoromethyl group, a nitro group, a C₁₋₆ alkyl group, a C₁₋₆ alkoxy group and a C₂₋₇ alkoxycarbonyl group), a C₁₋₆ alkyl group substituted by a phenylsulfonyl group (provided that such a phenylsulfonyl group may be substituted by one or more substituents selected from the group consisting of a halogen atom, a trifluoromethyl group, a nitro group, a C₁₋₆ alkyl group, a C₁₋₆ alkoxy group and a C₂₋₇ alkoxycarbonyl group), a C₁₋₆ alkyl group substituted by a benzyloxy group (provided that the phenyl group of such a benzyloxy group may be substituted by one or more substituents selected from the group consisting of a halogen atom, a trifluoromethyl group, a nitro group, a C₁₋₆ alkyl group, a C₁₋₆ alkoxy group and a C₂₋₇ alkoxycarbonyl group), a C₁₋₆ alkyl group substituted by a benzylthio group (provided that the phenyl group of such a benzylthio group may be substituted by one or more substituents selected from the group consisting of a halogen atom, a trifluoromethyl group, a nitro group, a C₁₋₆ alkyl group, a C₁₋₆ alkoxy group and a C₂₋₇ alkoxycarbonyl group), a C₁₋₆ alkyl group substituted by a benzylsulfinyl group (provided that the phenyl group of such a benzylsulfinyl group may be substituted by one or more substituents selected from the group consisting of a halogen atom, a trifluoromethyl group, a nitro group, a C₁₋₆ alkyl group, a C₁₋₆ alkoxy group and a C₂₋₇ alkoxycarbonyl group), a C₁₋₆ alkyl group substituted by a benzylsulfonyl group (provided that the phenyl group or such a benzylsulfonyl group may be substituted by one or more substituents selected from the group consisting of a halogen atom, a trifluoromethyl group, a nitro group, a C₁₋₆ alkyl group, a C₁₋₆ alkoxy group and a C₂₋₇ alkoxycarbonyl group), a C₁₋₆ alkyl group substituted by a phenylcarbonyl group (provided that such a phenylcarbonyl group may be substituted by one or more substituents selected from the group consisting of a halogen atom, a trifluoromethyl group, a nitro group, a C₁₋₆ alkyl group, a C₁₋₆ alkoxy group and a C₂₋₇ alkoxycarbonyl group), a C₁₋₆ alkyl group substituted by a benzylcarbonyl group (provided that the phenyl group of such a benzylcarbonyl group may be substituted by one or more substituents selected from the group consisting of a halogen atom, a trifluoromethyl group, a nitro group, a C₁₋₆ alkyl group, a C₁₋₆ alkoxy group and a C₂₋₇ alkoxycarbonyl group), a C₁₋₆ alkyl group substituted by an amino group substituted by a C₁₋₄ alkylsulfonyl group, or a C₁₋₆ alkyl group substituted by an amino group substituted by a C₂₋₄ alkylcarbonyl group,
each of R^{c2}, R^{c3}, R^{c4}, R^{c5}, R^{c6}, R^{c11} and R^{c12} which are independent of one another, is a hydrogen atom, a C₁₋₈ alkyl group, a C₂₋₈ alkenyl group, a C₂₋₈ alkynyl group, or a phenyl group (provided that such a phenyl group may be substituted by one or more substituents selected from the group consisting of a halogen atom, a trifluoromethyl group, a nitro group, a C₁₋₆ alkyl group, a C₁₋₆ alkoxy group and a C₂₋₇ alkoxycarbonyl group),
each of R^{c7}, R^{c8}, R^{c9}, R^{c10}, R^{c13} and R^{c14} which are independent of one another, is a hydrogen atom, a C₁₋₆ alkyl group, a mono-, di- or poly-halogeno C₁₋₆ alkyl group, a C₁₋₆ alkoxy group, a mono, di- or poly-halogeno C₁₋₆ alkoxy group, a C₁₋₆ alkylthio group, a C₁₋₆ alkylsulfinyl group, a C₁₋₆ alkylsulfonyl group, a C₂₋₇ alkoxycarbonyl group, a C₂₋₇ alkylcarbonyl group, a halogen atom, a nitro group, a cyano group, or a phenyl group (provided that such a phenyl group may be substituted by one or more substituents selected from the group consisting of a halogen atom, a trifluoromethyl group, a nitro group, a C₁₋₆ alkyl group, a C₁₋₆ alkoxy group and a C₂₋₇ alkoxycarbonyl group),
R^{d1} is a C₁₋₈ alkyl group, a C₃₋₇ cycloalkyl group, a C₁₋₆ alkyl group substituted by a C₃₋₇ cycloalkyl group, a C₃₋₇ cycloalkenyl group, a C₁₋₆ alkyl group substituted by a C₃₋₇ cycloalkenyl group, a C₂₋₈ alkenyl group, a C₂₋₈ alkynyl group, a C₁₋₆ alkyl group substituted by a C₁₋₆ alkoxy group, a C₁₋₆ alkyl group substituted by a C₂₋₆ alkenyloxy group, a C₁₋₆ alkyl group substituted by a C₂₋₆ alkynyloxy group, a C₁₋₆ alkyl group substituted by a mono-, di or poly-halogeno C₁₋₆ alkoxy group, a C₁₋₆ alkyl group substituted by a mono-, di- or poly-halogeno C₂₋₆ alkenyloxy group, a C₁₋₆ alkyl group substituted by a mono-, di- or poly-halogeno C₂₋₆ alkynyloxy group, a C₁₋₆ alkyl group substituted by a C₁₋₆ alkylthio group, a C₁₋₆ alkyl group substituted by a C₁₋₆ alkylsulfinyl group, a C₁₋₆ alkyl group substituted by a C₁₋₆ alkylsulfonyl group, a mono-, di- or poly-halogeno C₁₋₈ alkyl group, a mono-, di- or poly-halogeno C₂₋₈ alkenyl group, a mono-, di- or poly-halogeno C₂₋₈ alkynyl group, a C₁₋₆ alkyl group substituted by a cyano group, a C₂₋₆ alkenyl group substituted by a cyano group, a C₂₋₆ alkynyl group substituted by a cyano group, a C₁₋₆ alkyl group substituted by a nitro group, a C₂₋₆ alkenyl group substituted by a nitro group, a C₂₋₆ alkynyl group substituted by a nitro group, a C₁₋₆ alkyl group substituted by a C₂₋₇ alkoxycarbonyl group, a C₂₋₆ alkenyl group substituted by a C₂₋₇ alkoxycarbonyl group, a C₂₋₆ alkynyl group substituted by a C₂₋₇ alkoxycarbonyl group, a C₁₋₆ alkyl group substituted by a C₂₋₇ alkylcarbonyl group, a C₁₋₆ alkyl group substituted by a mono-, di- or poly-halogeno C₂₋₇ alkylcarbonyl group, a C₁₋₆ alkyl group substituted by a C₃₋₇ alkenylcarbonyl group, a C₁₋₆ alkyl group substituted by a C₃₋₇ alkynylcarbonyl group, a C₁₋₆ alkyl group substituted by a C₂₋₅ alkylcarbonyl group substituted by a C₁₋₄ alkoxy group, a C₁₋₆ alkyl group substituted by a C₂₋₅ alkylcarbonyl group substituted by a C₁₋₄ alkylthio group, a C₁₋₆ alkyl group substituted by a C₂₋₅ alkylcarbonyl group substituted by a C₁₋₄ alkylsulfinyl group, a C₁₋₆ alkyl group substituted by a C₂₋₅ alkylcarbonyl group substituted by a C₁₋₄ alkylsulfonyl group, a C₂₋₆ alkenyl group substituted by a C₂₋₇ alkylcarbonyl group, a C₂₋₆ alkynyl group substituted by a C₂₋₇ alkylcarbonyl group, a C₁₋₆ alkyl group substituted by a C₁₋₆ alkylsulfamoyl group, a C₁₋₆ alkyl group substituted by a C₁₋₆ alkoxysulfamoyl group, a C₁₋₆ alkyl group substituted by a di(C₁₋₃ alkyl)sulfamoyl group, a C₁₋₆ alkyl group substituted by an N-(C₁₋₃ alkyl)-N-(C₁₋₃ alkoxy)sulfamoyl group, a C₁₋₆ alkyl group substituted by a C₂₋₇ alkylcarbamoyl group, a C₁₋₆ alkyl group substituted by a di(C₁₋₃ alkyl)carbamoyl group, a C₁₋₆ alkyl group substituted by a C₂₋₇ alkoxycarbamoyl group, a C₁₋₆ alkyl group substituted by an N-(C₁₋₃ alkyl)-N-(C₁₋₃ alkoxy)carbamoyl group, a C₁₋₆ alkyl group substituted by a C₁₋₆ alkylamino group, a C₁₋₆ alkyl group substituted by a C₁₋₆ alkoxyamino group, a C₁₋₆ alkyl group substituted by a di(C₁₋₃ alkyl)amino group, a C₁₋₆ alkyl group substituted by an N-(C₁₋₃ alkyl)-N-(C₁₋₃ alkoxy)amino group, a C₁₋₆ alkyl group substituted by an N-(C₂₋₇ alkylcarbonyl)-N-(C₁₋₆ alkyl)amino group, a C₁₋₆ alkyl group substituted by an N-(C₂₋₇ alkylcarbonyl)-N-(C₁₋₆ alkoxy)amino group, a C₁₋₆ alkyl group substituted by an N-(C₁₋₆ alkylsulfonyl)-N-(C₁₋₆ alkyl)amino group, a C₁₋₆ alkyl group substituted by an N-(C₁₋₆ alkylsulfonyl)-N-(C₁₋₆ alkoxy)amino group, a phenyl group (provided that such a phenyl group may be substituted by one or more substituents selected from the group consisting of a halogen atom, a trifluoromethyl group, a nitro group, a C₁₋₆ alkyl group, a C₁₋₆ alkoxy group and a C₂₋₇ alkoxycarbonyl group), a C₁₋₆ alkyl group substituted by a phenyl group (provided that such a phenyl group may be substituted by one or more substituents selected from the group consisting of a halogen atom, a trifluoromethyl group, a nitro group, a C₁₋₆ alkyl group, a C₁₋₆ alkoxy group and a C₂₋₇ alkoxycarbonyl group), a C₂₋₇ alkenyl group substituted by a phenyl group (provided that such a phenyl group may be substituted by one or more substituents selected from the group consisting of a halogen atom, a trifluoromethyl group, a nitro group, a C₁₋₆ alkyl group, a C₁₋₆ alkoxy group and a C₂₋₇ alkoxycarbonyl group), a C₂₋₆ alkynyl group substituted by a phenyl group (provided that such a phenyl group may be substituted by one or more substituents selected from the group consisting of a halogen atom, a trifluoromethyl group, a nitro group, a C₁₋₆ alkyl group, a C₁₋₆ alkoxy group and a C₂₋₇ alkoxycarbonyl group), a C₁₋₆ alkyl group substituted by a phenoxy group (provided that such a phenoxy group may be substituted by one or more substituents selected from the group consisting of a halogen atom, a trifluoromethyl group, a nitro group, a C₁₋₆ alkyl group, a C₁₋₆ alkoxy group and a C₂₋₇ alkoxycarbonyl group), a C₁₋₆ alkyl group substituted by a phenylthio group (provided that such a phenylthio group may be substituted by one or more substituents selected from the group consisting of a halogen atom, a trifluoromethyl group, a nitro group, a C₁₋₆ alkyl group, a C₁₋₆ alkoxy group and a C₂₋₇ alkoxycarbonyl group), a C₁₋₆ alkyl group substituted by a phenylsulfinyl group (provided that such a phenylsulfinyl group may be substituted by one or more substituents selected from the group consisting of a halogen atom, a trifluoromethyl group, a nitro group, a C₁₋₆ alkyl group, a C₁₋₆ alkoxy group and a C₂₋₇ alkoxycarbonyl group), a C₁₋₆ alkyl group substituted by a phenylsulfonyl group (provided that such a phenylsulfonyl group may be substituted by one or more substituents selected from the group consisting of a halogen atom, a trifluoromethyl group, a nitro group, a C₁₋₆ alkyl group, a C₁₋₆ alkoxy group and a C₂₋₇ alkoxycarbonyl group), a C₁₋₆ alkyl group substituted by a benzyloxy group (provided that the phenyl group of such a benzyloxy group may be substituted by one or more substituents selected from the group consisting of a halogen atom, a trifluoromethyl group, a nitro group, a C₁₋₆ alkyl group, a C₁₋₆ alkoxy group and a C₂₋₇ alkoxycarbonyl group), a C₁₋₆ alkyl group substituted by a benzylthio group (provided that the phenyl group of such a benzylthio group may be substituted by one or more substituents selected from the group consisting of a halogen atom, a trifluoromethyl group, a nitro group, a C₁₋₆ alkyl group, a C₁₋₆ alkoxy group and a C₂₋₇ alkoxycarbonyl group), a C₁₋₆ alkyl group substituted by a benzylsulfinyl group (provided that the phenyl group of such a benzylsulfinyl group may be substituted by one or more substituents selected from the group consisting of a halogen atom, a trifluoromethyl group, a nitro group, a C₁₋₆ alkyl group, a C₁₋₆ alkoxy group and a C₂₋₇ alkoxycarbonyl group), a C₁₋₆ alkyl group substituted by a benzylsulfonyl group (provided that the phenyl group of such a benzylsulfonyl group may be substituted by one or more substituents selected from the group consisting of a halogen atom, a trifluoromethyl group, a nitro group, a C₁₋₆ alkyl group, a C₁₋₆ alkoxy group and a C₂₋₇ alkoxycarbonyl group), a C₁₋₆ alkyl group substituted by a phenylcarbonyl group (provided that such a phenylcarbonyl group may be substituted by one or more substituents selected from the group consisting of a halogen atom, a trifluoromethyl group, a nitro group, a C₁₋₆ alkyl group, a C₁₋₆ alkoxy group and a C₂₋₇ alkoxycarbonyl group), a C₁₋₆ alkyl group substituted by a benzylcarbonyl group (provided that the phenyl group of such a benzylcarbonyl group may be substituted by one or more substituents selected from the group consisting of a halogen atom, a trifluoromethyl group, a nitro group, a C₁₋₆ alkyl group, a C₁₋₆ alkoxy group and a C₂₋₇ alkoxycarbonyl group), a C₁₋₆ alkyl group substituted by an amino group substituted by a C₁₋₄ alkylsulfonyl group, or a C₁₋₆ alkyl group substituted by an amino group substituted by a C₂₋₄ alkylcarbonyl group,
each of R^{d2}, R^{d3} and R^{d4} which are independent of one another, is a hydrogen atom, a C₁₋₈ alkyl group, a C₂₋₈ alkenyl group, a C₂₋₈ alkynyl group, or a phenyl group (provided that such a phenyl group may be substituted by one or more substituents selected from the group consisting of a halogen atom, a trifluoromethyl group, a nitro group, a C₁₋₆ alkyl group, a C₁₋₆ alkoxy group and a C₂₋₇ alkoxycarbonyl group),
R^{d5} is a C₁₋₈ alkyl group, a C₂₋₈ alkenyl group, a C₂₋₈ alkynyl group, or a phenyl group (provided that such a phenyl group may be substituted by one or more substituents selected from the group consisting of a halogen atom, a trifluoromethyl group, a nitro group, a C₁₋₆ alkyl group, a C₁₋₆ alkoxy group and a C₂₋₇ alkoxycarbonyl group),
R^{d6} is a C₁₋₈ alkyl group, a C₂₋₈ alkenyl group, a C₂₋₈ alkynyl group, a C₁₋₆ alkyl group substituted by a C₁₋₆ alkylthio group, a C₁₋₆ alkyl group substituted by a C₁₋₆ alkylsulfinyl group, a C₁₋₆ alkyl group substituted by a C₁₋₆ alkylsulfonyl group, a C₁₋₆ alkyl group substituted by a C₁₋₆ alkoxy group, a C₁₋₆ alkyl group substituted by a C₂₋₇ alkoxycarbonyl group, a C₁₋₆ alkyl group substituted by a C₂₋₇ alkylcarbonyl group, a C₁₋₆ alkyl group substituted by a cyano group, a phenyl group (provided that such a phenyl group may be substituted by one or more substituents selected from the group consisting of a halogen atom, a trifluoromethyl group, a nitro group, a C₁₋₆ alkyl group, a C₁₋₆ alkoxy group and a C₂₋₇ alkoxycarbonyl group), or a C₁₋₆ alkyl group substituted by a phenyl group (provided that such a phenyl group may be substituted by one or more substituents selected from the group consisting of a halogen atom, a trifluoromethyl group, a nitro group, a C₁₋₆ alkyl group, a C₁₋₆ alkoxy group and a C₂₋₇ alkoxycarbonyl group),
R^{e1} is a C₁₋₈ alkyl group, a C₃₋₇ cycloalkyl group, a C₁₋₆ alkyl group substituted by a C₃₋₇ cycloalkyl group, a C₃₋₇ cycloalkenyl group, a C₁₋₆ alkyl group substituted by a C₃₋₇ cycloalkenyl group, a C₂₋₈ alkenyl group, a C₂₋₈ alkynyl group, a C₁₋₆ alkyl group substituted by a C₁₋₆ alkoxy group, a C₁₋₆ alkyl group substituted by a C₂₋₆ alkenyloxy group, a C₁₋₆ alkyl group substituted by a C₂₋₆ alkynyloxy group, a C₁₋₆ alkyl group substituted by a mono-, di- or poly-halogeno C₁₋₆ alkoxy group, a C₁₋₆ alkyl group substituted by a mono-, di- or poly-halogeno C₂₋₆ alkenyloxy group, a C₁₋₆ alkyl group substituted by a mono-, di- or poly-halogeno C₂₋₆ alkynyloxy group, a C₁₋₆ alkyl group substituted by a C₁₋₆ alkylthio group, a C₁₋₆ alkyl group substituted by a C₁₋₆ alkylsulfinyl group, a C₁₋₆ alkyl group substituted by a C₁₋₆ alkylsulfonyl group, a mono-, di- or poly-halogeno C₁₋₈ alkyl group, a mono-, di- or poly-halogeno C₂₋₈ alkenyl group, a mono-, di- or poly-halogeno C₂₋₈ alkynyl group, a C₁₋₆ alkyl group substituted by a cyano group, a C₂₋₆ alkenyl group substituted by a cyano group, a C₂₋₆ alkynyl group substituted by a cyano group, a C₁₋₆ alkyl group substituted by a nitro group, a C₂₋₆ alkenyl group substituted by a nitro group, a C₂₋₆ alkynyl group substituted by a nitro group, a C₁₋₆ alkyl group substituted by a C₂₋₇ alkoxycarbonyl group, a C₂₋₆ alkenyl group substituted by a C₂₋₇ alkoxycarbonyl group, a C₂₋₆ alkynyl group substituted by a C₂₋₇ alkoxycarbonyl group, a C₁₋₆ alkyl group substituted by a C₂₋₇ alkylcarbonyl group, a C₁₋₆ alkyl group substituted by a mono-, di- or poly-halogeno C₂₋₇ alkylcarbonyl group, a C₁₋₆ alkyl group substituted by a C₃₋₇ alkenylcarbonyl group, a C₁₋₆ alkyl group substituted by a C₃₋₇ alkynylcarbonyl group, a C₁₋₆ alkyl group substituted by a C₂₋₅ alkylcarbonyl group substituted by a C₁₋₄ alkoxy group, a C₁₋₆ alkyl group substituted by a C₂₋₅ alkylcarbonyl group substituted by a C₁₋₄ alkylthio group, a C₁₋₆ alkyl group substituted by a C₂₋₅ alkylcarbonyl group substituted by a C₁₋₄ alkylsulfinyl group, a C₁₋₆ alkyl group substituted by a C₂₋₅ alkylcarbonyl group substituted by a C₁₋₄ alkylsulfonyl group, a C₂₋₆ alkenyl group substituted by a C₂₋₇ alkylcarbonyl group, a C₂₋₆ alkynyl group substituted by a C₂₋₇ alkylcarbonyl group, a C₁₋₆ alkyl group substituted by a C₁₋₆ alkylsulfamoyl group, a C₁₋₆ alkyl group substituted by a C₁₋₆ alkoxysulfamoyl group, a C₁₋₆ alkyl group substituted by a di(C₁₋₃ alkyl)sulfamoyl group, a C₁₋₆ alkyl group substituted by an N-(C₁₋₃ alkyl)-N-(C₁₋₃ alkoxy)sulfamoyl group, a C₁₋₆ alkyl group substituted by a C₂₋₇ alkylcarbamoyl group, a C₁₋₆ alkyl group substituted by a di(C₁₋₃ alkyl)carbamoyl group, a C₁₋₆ alkyl group substituted by a C₂₋₇ alkoxycarbamoyl group, a C₁₋₆ alkyl group substituted by an N-(C₁₋₃ alkyl)-N-(C₁₋₃ alkoxy)carbamoyl group, a C₁₋₆ alkyl group substituted by a C₁₋₆ alkylamino group, a C₁₋₆ alkyl group substituted by a C₁₋₆ alkoxyamino group, a C₁₋₆ alkyl group substituted by a di(C₁₋₃ alkyl)amino group, a C₁₋₆ alkyl group substituted by an N-(C₁₋₃ alkyl)-N-(C₁₋₃ alkoxy)amino group, a C₁₋₆ alkyl group substituted by an N-(C₂₋₇ alkylcarbonyl)-N-(C₁₋₆ alkyl)amino group, a C₁₋₆ alkyl group substituted by an N-(C₂₋₇ alkylcarbonyl)-N-(C₁₋₆ alkoxy)amino group, a C₁₋₆ alkyl group substituted by an N-(C₁₋₆ alkylsulfonyl)-N-(C₁₋₆ alkyl)amino group, a C₁₋₆ alkyl group substituted by an N-(C₁₋₆ alkylsulfonyl)-N-(C₁₋₆ alkoxy)amino group, a phenyl group (provided that such a phenyl group may be substituted by one or more substituents selected from the group consisting of a halogen atom, a trifluoromethyl group, a nitro group, a C₁₋₆ alkyl group, a C₁₋₆ alkoxy group and a C₂₋₇ alkoxycarbonyl group), a C₁₋₆ alkyl group substituted by a phenyl group (provided that such a phenyl group may be substituted by one or more substituents selected from the group consisting of a halogen atom, a trifluoromethyl group, a nitro group, a C₁₋₆ alkyl group, a C₁₋₆ alkoxy group and a C₂₋₇ alkoxycarbonyl group), a C₂₋₇ alkenyl group substituted by a phenyl group (provided that such a phenyl group may be substituted by one or more substituents selected from the group consisting of a halogen atom, a trifluoromethyl group, a nitro group, a C₁₋₆ alkyl group, a C₁₋₆ alkoxy group and a C₂₋₇ alkoxycarbonyl group), a C₂₋₆ alkynyl group substituted by a phenyl group (provided that such a phenyl group may be substituted by one or more substituents selected from the group consisting of a halogen atom, a trifluoromethyl group, a nitro group, a C₁₋₆ alkyl group, a C₁₋₆ alkoxy group and a C₂₋₇ alkoxycarbonyl group), a C₁₋₆ alkyl group substituted by a phenoxy group (provided that such a phenoxy group may be substituted by one or more substituents selected from the group consisting of a halogen atom, a trifluoromethyl group, a nitro group, a C₁₋₆ alkyl group, a C₁₋₆ alkoxy group and a C₂₋₇ alkoxycarbonyl group), a C₁₋₆ alkyl group substituted by a phenylthio group (provided that such a phenylthio group may be substituted by one or more substituents selected from the group consisting of a halogen atom, a trifluoromethyl group, a nitro group, a C₁₋₆ alkyl group, a C₁₋₆ alkoxy group and a C₂₋₇ alkoxycarbonyl group), a C₁₋₆ alkyl group substituted by a phenylsulfinyl group (provided that such a phenylsulfinyl group may be substituted by one or more substituents selected from the group consisting of a halogen atom, a trifluoromethyl group, a nitro group, a C₁₋₆ alkyl group, a C₁₋₆ alkoxy group and a C₂₋₇ alkoxycarbonyl group), a C₁₋₆ alkyl group substituted by a phenylsulfonyl group (provided that such a phenylsulfonyl group may be substituted by one or more substituents selected from the group consisting of a halogen atom, a trifluoromethyl group, a nitro group, a C₁₋₆ alkyl group, a C₁₋₆ alkoxy group and a C₂₋₇ alkoxycarbonyl group), a C₁₋₆ alkyl group substituted by a benzyloxy group (provided that the phenyl group of such a benzyloxy group may be substituted by one or more substituents selected from the group consisting of a halogen atom, a trifluoromethyl group, a nitro group, a C₁₋₆ alkyl group, a C₁₋₆ alkoxy group and a C₂₋₇ alkoxycarbonyl group), a C₁₋₆ alkyl group substituted by a benzylthio group (provided that the phenyl group of such a benzylthio group may be substituted by one or more substituents selected from the group consisting of a halogen atom, a trifluoromethyl group, a nitro group, a C₁₋₆ alkyl group, a C₁₋₆ alkoxy group and a C₂₋₇ alkoxycarbonyl group), a C₁₋₆ alkyl group substituted by a benzylsulfinyl group (provided that the phenyl group of such a benzylsulfinyl group may be substituted by one or more substituents selected from the group consisting of a halogen atom, a trifluoromethyl group, a nitro group, a C₁₋₆ alkyl group, a C₁₋₆ alkoxy group and a C₂₋₇ alkoxycarbonyl group), a C₁₋₆ alkyl group substituted by a benzylsulfonyl group (provided that the phenyl group of such a benzylsulfonyl group may be substituted by one or more substituents selected from the group consisting of a halogen atom, a trifluoromethyl group, a nitro group, a C₁₋₆ alkyl group, a C₁₋₆ alkoxy group and a C₂₋₇ alkoxycarbonyl group), a C₁₋₆ alkyl group substituted by a phenylcarbonyl group (provided that such a phenylcarbonyl group may be substituted by one or more substituents selected from the group consisting of a halogen atom, a trifluoromethyl group, a nitro group, a C₁₋₆ alkyl group, a C₁₋₆ alkoxy group and a C₂₋₇ alkoxycarbonyl group), a C₁₋₆ alkyl group substituted by a benzylcarbonyl group (provided that the phenyl group of such a benzylcarbonyl group may be substituted by one or more substituents selected from the group consisting of a halogen atom, a trifluoromethyl group, a nitro group, a C₁₋₆ alkyl group, a C₁₋₆ alkoxy group and a C₂₋₇ alkoxycarbonyl group), a C₁₋₆ alkyl group substituted by an amino group substituted by a C₁₋₄ alkylsulfonyl group, or a C₁₋₆ alkyl group substituted by an amino group substituted by a C₂₋₄ alkylcarbonyl group,
each of R^{e2}, R^{e3}, R^{e6} and R^{e7} which are independent of one another, is a hydrogen atom, a C₁₋₆ alkyl group, a mono-, di- or poly-halogeno C₁₋₆ alkyl group, a C₁₋₆ alkoxy group, a mono-, di- or poly-halogeno C₁₋₆ alkoxy group, a C₁₋₆ alkylthio group, a C₁₋₆ alkylsulfinyl group, a C₁₋₆ alkylsulfonyl group, a C₂₋₇ alkoxycarbonyl group, a C₂₋₇ alkylcarbonyl group, a halogen atom, a nitro group, a cyano group, or a phenyl group (provided that such a phenyl group may be substituted by one or more substituents selected from the group consisting of a halogen atom, a trifluoromethyl group, a nitro group, a C₁₋₆ alkyl group, a C₁₋₆ alkoxy group and a C₂₋₇ alkoxycarbonyl group),
each of R^{e4}, R^{e5}, R^{e8}, R^{e9} and R^{e10} which are independent of one another, is a hydrogen atom, a C₁₋₈ alkyl group, a C₂₋₈ alkenyl group, a C₂₋₈ alkynyl group, or a phenyl group (provided that such a phenyl group may be substituted by one or more substituents selected from the group consisting of a halogen atom, a trifluoromethyl group, a nitro group, a C₁₋₆ alkyl group, a C₁₋₆ alkoxy group and a C₂₋₇ alkoxycarbonyl group),
R^{f1} is a C₁₋₈ alkyl group, a C₃₋₇ cycloalkyl group, a C₁₋₆ alkyl group substituted by a C₃₋₇ cycloalkyl group, a C₃₋₇ cycloalkenyl group, a C₁₋₆ alkyl group substituted by a C₃₋₇ cycloalkenyl group, a C₂₋₈ alkenyl group, a C₂₋₈ alkynyl group, a C₁₋₆ alkyl group substituted by a C₁₋₆ alkoxy group, a C₁₋₆ alkyl group substituted by a C₂₋₆ alkenyloxy group, a C₁₋₆ alkyl group substituted by a C₂₋₆ alkynyloxy group, a C₁₋₆ alkyl group substituted by a mono-, di- or poly-halogeno C₁₋₆ alkoxy group, a C₁₋₆ alkyl group substituted by a mono-, di- or poly-halogeno C₂₋₆ alkenyloxy group, a C₁₋₆ alkyl group substituted by a mono-, di- or poly-halogeno C₂₋₆ alkynyloxy group, a C₁₋₆ alkyl group substituted by a C₁₋₆ alkylthio group, a C₁₋₆ alkyl group substituted by a C₁₋₆ alkylsulfinyl group, a C₁₋₆ alkyl group substituted by a C₁₋₆ alkylsulfonyl group, a mono-, di- or poly-halogeno C₁₋₈ alkyl group, a mono-, di- or poly-halogeno C₂₋₈ alkenyl group, a mono-, di- or poly-halogeno C₂₋₈ alkynyl group, a C₁₋₆ alkyl group substituted by a cyano group, a C₂₋₆ alkenyl group substituted by a cyano group, a C₂₋₆ alkynyl group substituted by a cyano group, a C₁₋₆ alkyl group substituted by a nitro group, a C₂₋₆ alkenyl group substituted by a nitro group, a C₂₋₆ alkynyl group substituted by a nitro group, a C₁₋₆ alkyl group substituted by a C₂₋₇ alkoxycarbonyl group, a C₂₋₆ alkenyl group substituted by a C₂₋₇ alkoxycarbonyl group, a C₂₋₆ alkynyl group substituted by a C₂₋₇ alkoxycarbonyl group, a C₁₋₆ alkyl group substituted by a C₂₋₇ alkylcarbonyl group, a C₁₋₆ alkyl group substituted by a mono-, di- or poly-halogeno C₂₋₇ alkylcarbonyl group, a C₁₋₆ alkyl group substituted by a C₃₋₇ alkenylcarbonyl group, a C₁₋₆ alkyl group substituted by a C₃₋₇ alkynylcarbonyl group, a C₁₋₆ alkyl group substituted by a C₂₋₅ alkylcarbonyl group substituted by a C₁₋₄ alkoxy group, a C₁₋₆ alkyl group substituted by a C₂₋₅ alkylcarbonyl group substituted by a C₁₋₄ alkylthio group, a C₁₋₆ alkyl group substituted by a C₂₋₅ alkylcarbonyl group substituted by a C₁₋₄ alkylsulfinyl group, a C₁₋₆ alkyl group substituted by a C₂₋₅ alkylcarbonyl group substituted by a C₁₋₄ alkylsulfonyl group, a C₂₋₆ alkenyl group substituted by a C₂₋₇ alkylcarbonyl group, a C₂₋₆ alkynyl group substituted by a C₂₋₇ alkylcarbonyl group, a C₁₋₆ alkyl group substituted by a C₁₋₆ alkylsulfamoyl group, a C₁₋₆ alkyl group substituted by a C₁₋₆ alkoxysulfamoyl group, a C₁₋₆ alkyl group substituted by a di(C₁₋₃ alkyl)sulfamoyl group, a C₁₋₆ alkyl group substituted by an N-(C₁₋₃ alkyl)-N-(C₁₋₃ alkoxy)sulfamoyl group, a C₁₋₆ alkyl group substituted by a C₂₋₇ alkylcarbamoyl group, a C₁₋₆ alkyl group substituted by a di(C₁₋₃ alkyl)carbamoyl group, a C₁₋₆ alkyl group substituted by a C₂₋₇ alkoxycarbamoyl group, a C₁₋₆ alkyl group substituted by an N-(C₁₋₃ alkyl)-N-(C₁₋₃ alkoxy)carbamoyl group, a C₁₋₆ alkyl group substituted by a C₁₋₆ alkylamino group, a C₁₋₆ alkyl group substituted by a C₁₋₆ alkoxyamino group, a C₁₋₆ alkyl group substituted by a di(C₁₋₃ alkyl)amino group, a C₁₋₆ alkyl group substituted by an N-(C₁₋₃ alkyl)-N-(C₁₋₃ alkoxy)amino group, a C₁₋₆ alkyl group substituted by an N-(C₂₋₇ alkylcarbonyl)-N-(C₁₋₆ alkyl)amino group, a C₁₋₆ alkyl group substituted by an N-(C₂₋₇ alkylcarbonyl)-N-(C₁₋₆ alkoxy)amino group, a C₁₋₆ alkyl group substituted by an N-(C₁₋₆ alkylsulfonyl)-N-(C₁₋₆ alkyl)amino group, a C₁₋₆ alkyl group substituted by an N-(C₁₋₆ alkylsulfonyl)-N-(C₁₋₆ alkoxy)amino group, a phenyl group (provided than such a phenyl group may be substituted by one or more substituents selected from the group consisting of a halogen atom, a trifluoromethyl group, a nitro group, a C₁₋₆ alkyl group, a C₁₋₆ alkoxy group and a C₂₋₇ alkoxycarbonyl group), a C₁₋₆ alkyl group substituted by a phenyl group (provided that such a phenyl group may be substituted by one or more substituents selected from the group consisting of a halogen atom, a trifluoromethyl group, a nitro group, a C₁₋₆ alkyl group, a C₁₋₆ alkoxy group and a C₂₋₇ alkoxycarbonyl group), a C₂₋₇ alkenyl group substituted by a phenyl group (provided that such a phenyl group may be substituted by one or more substituents selected from the group consisting of a halogen atom, a trifluoromethyl group, a nitro group, a C₁₋₆ alkyl group, a C₁₋₆ alkoxy group and a C₂₋₇ alkoxycarbonyl group), a C₂₋₆ alkynyl group substituted by a phenyl group (provided that such a phenyl group may be substituted by one or more substituents selected from the group consisting of a halogen atom, a trifluoromethyl group, a nitro group, a C₁₋₆ alkyl group, a C₁₋₆ alkoxy group and a C₂₋₇ alkoxycarbonyl group), a C₁₋₆ alkyl group substituted by a phenoxy group (provided that such a phenoxy group may be substituted by one or more substituents selected from the group consisting of a halogen atom, a trifluoromethyl group, a nitro group, a C₁₋₆ alkyl group, a C₁₋₆ alkoxy group and a C₂₋₇ alkoxycarbonyl group), a C₁₋₆ alkyl group substituted by a phenylthio group (provided that such a phenylthio group may be substituted by one or more substituents selected from the group consisting of a halogen atom, a trifluoromethyl group, a nitro group, a C₁₋₆ alkyl group, a C₁₋₆ alkoxy group and a C₂₋₇ alkoxycarbonyl group), a C₁₋₆ alkyl group substituted by a phenylsulfinyl group (provided that such a phenylsulfinyl group may be substituted by one or more substituents selected from the group consisting of a halogen atom, a trifluoromethyl group, a nitro group, a C₁₋₆ alkyl group, a C₁₋₆ alkoxy group and a C₂₋₇ alkoxycarbonyl group), a C₁₋₆ alkyl group substituted by a phenylsulfonyl group (provided that such a phenylsulfonyl group may be substituted by one or more substituents selected from the group consisting of a halogen atom, a trifluoromethyl group, a nitro group, a C₁₋₆ alkyl group, a C₁₋₆ alkoxy group and a C₂₋₇ alkoxycarbonyl group), a C₁₋₆ alkyl group substituted by a benzyloxy group (provided that the phenyl group of such a benzyloxy group may be substituted by one or more substituents selected from the group consisting of a halogen atom, a trifluoromethyl group, a nitro group, a C₁₋₆ alkyl group, a C₁₋₆ alkoxy group and a C₂₋₇ alkoxycarbonyl group), a C₁₋₆ alkyl group substituted by a benzylthio group (provided that the phenyl group of such a benzylthio group may be substituted by one or more substituents selected from the group consisting of a halogen atom, a trifluoromethyl group, a nitro group, a C₁₋₆ alkyl group, a C₁₋₆ alkoxy group and a C₂₋₇ alkoxycarbonyl group), a C₁₋₆ alkyl group substituted by a benzylsulfinyl group (provided that the phenyl group of such a benzylsulfinyl group may be substituted by one or more substituents selected from the group consisting of a halogen atom, a trifluoromethyl group, a nitro group, a C₁₋₆ alkyl group, a C₁₋₆ alkoxy group and a C₂₋₇ alkoxycarbonyl group), a C₁₋₆ alkyl group substituted by a benzylsulfonyl group (provided that the phenyl group of such a benzylsulfonyl group may be substituted by one or more substituents selected from the group consisting of a halogen atom, a trifluoromethyl group, a nitro group, a C₁₋₆ alkyl group, a C₁₋₆ alkoxy group and a C₂₋₇ alkoxycarbonyl group), a C₁₋₆ alkyl group substituted by a phenylcarbonyl group (provided that such a phenylcarbonyl group may be substituted by one or more substituents selected from the group consisting of a halogen atom, a trifluoromethyl group, a nitro group, a C₁₋₆ alkyl group, a C₁₋₆ alkoxy group and a C₂₋₇ alkoxycarbonyl group), a C₁₋₆ alkyl group substituted by a benzylcarbonyl group (provided that the phenyl group of such a benzylcarbonyl group may be substituted by one or more substituents selected from the group consisting of a halogen atom, a trifluoromethyl group, a nitro group, a C₁₋₆ alkyl group, a C₁₋₆ alkoxy group and a C₂₋₇ alkoxycarbonyl group), a C₁₋₆ alkyl group substituted by an amino group substituted by a C₁₋₄ alkylsulfonyl group, or a C₁₋₆ alkyl group substituted by an amino group substituted by a C₂₋₄ alkylcarbonyl group,
R^{f2} is a C₁₋₈ alkyl group, a C₂₋₈ alkenyl group, a C₂₋₈ alkynyl group, a C₁₋₆ alkyl group substituted by a C₁₋₆ alkylthio group, a C₁₋₆ alkyl group substituted by a C₁₋₆ alkylsulfinyl group, a C₁₋₆ alkyl group substituted by a C₁₋₆ alkylsulfonyl group, a C₁₋₆ alkyl group substituted by a C₁₋₆ alkoxy group, a C₁₋₆ alkyl group substituted by a C₂₋₇ alkoxycarbonyl group, a C₁₋₆ alkyl group substituted by a C₂₋₇ alkylcarbonyl group, a C₁₋₆ alkyl group substituted by a cyano group, a phenyl group (provided that such a phenyl group may be substituted by one or more substituents selected from the group consisting of a halogen atom, a trifluoromethyl group, a nitro group, a C₁₋₆ alkyl group, a C₁₋₆ alkoxy group and a C₂₋₇ alkoxycarbonyl group), or a C₁₋₆ alkyl group substituted by a phenyl group (provided that such a phenyl group may be substituted by one or more substituents selected from the group consisting of a halogen atom, a trifluoromethyl group, a nitro group, a C₁₋₆ alkyl group, a C₁₋₆ alkoxy group and a C₂₋₇ alkoxycarbonyl group),
each of R^{f3}, R^{f4}, R^{f7}, R^{f8}, R^{f11} and R^{f12} which are independent of one another, is a hydrogen atom, a C₁₋₆ alkyl group, a mono-, di- or poly-halogeno C₁₋₆ alkyl group, a C₁₋₆ alkoxy group, a mono-, di- or poly-halogeno C₁₋₆ alkoxy group, a C₁₋₆ alkylthio group, a C₁₋₆ alkylsulfinyl group, a C₁₋₆ alkylsulfonyl group, a C₂₋₇ alkoxycarbonyl group, a C₂₋₇ alkylcarbonyl group, a halogen atom, a nitro group, a cyano group, or a phenyl group (provided that such a phenyl group may be substituted by one or more substituents selected from the group consisting of a halogen atom, a trifluoromethyl group, a nitro group, a C₁₋₆ alkyl group, a C₁₋₆ alkoxy group and a C₂₋₇ alkoxycarbonyl group),
each of R^{f5}, R^{f6}, R^{f9}, R^{f10}, R^{f13}, R^{f14} and R^{f15} which are independent of one another, is a hydrogen atom, a C₁₋₈ alkyl group, a C₂₋₈ alkenyl group, a C₂₋₈ alkynyl group, or a phenyl group (provided that such a phenyl group may be substituted by one or more substituents selected from the group consisting of a halogen atom, a trifluoromethyl group, a nitro group, a C₁₋₆ alkyl group, a C₁₋₆ alkoxy group and a C₂₋₇ alkoxycarbonyl group),
R^{g1} is a C₁₋₈ alkyl group, a C₃₋₇ cycloalkyl group, a C₁₋₆ alkyl group substituted by a C₃₋₇ cycloalkyl group, a C₃₋₇ cycloalkenyl group, a C₁₋₆ alkyl group substituted by a C₃₋₇ cycloalkenyl group, a C₂₋₈ alkenyl group, a C₂₋₈ alkynyl group, a C₁₋₆ alkyl group substituted by a C₁₋₆ alkoxy group, a C₁₋₆ alkyl group substituted by a C₂₋₆ alkenyloxy group, a C₁₋₆ alkyl group substituted by a C₂₋₆ alkynyloxy group, a C₁₋₆ alkyl group substituted by a mono-, di- or poly-halogeno C₁₋₆ alkoxy group, a C₁₋₆ alkyl group substituted by a mono-, di- or poly-halogeno C₂₋₆ alkenyloxy group, a C₁₋₆ alkyl group substituted by a mono-, di- or poly-halogeno C₂₋₆ alkynyloxy group, a C₁₋₆ alkyl group substituted by a C₁₋₆ alkylthio group, a C₁₋₆ alkyl group substituted by a C₁₋₆ alkylsulfinyl group, a C₁₋₆ alkyl group substituted by a C₁₋₆ alkylsulfonyl group, a mono-, di- or poly-halogeno C₁₋₈ alkyl group, a mono-, di- or poly-halogeno C₂₋₈ alkenyl group, a mono-, di- or poly-halogeno C₂₋₈ alkynyl group, a C₁₋₆ alkyl group substituted by a cyano group, a C₂₋₆ alkenyl group substituted by a cyano group, a C₂₋₆ alkynyl group substituted by a cyano group, a C₁₋₆ alkyl group substituted by a nitro group, a C₂₋₆ alkenyl group substituted by a nitro group, a C₂₋₆ alkynyl group substituted by a nitro grouo, a C₁₋₆ alkyl group substituted by a C₂₋₇ alkoxycarbonyl group, a C₂₋₆ alkenyl group substituted by a C₂₋₇ alkoxycarbonyl group, a C₂₋₆ alkynyl group substituted by a C₂₋₇ alkoxycarbonyl group, a C₁₋₆ alkyl group substituted by a C₂₋₇ alkylcarbonyl group, a C₁₋₆ alkyl group substituted by a mono-, di- or poly-halogeno C₂₋₇ alkylcarbonyl group, a C₁₋₆ alkyl group substituted by a C₃₋₇ alkenylcarbonyl group, a C₁₋₆ alkyl group substituted by a C₃₋₇ alkynylcarbonyl group, a C₁₋₆ alkyl group substituted by a C₂₋₅ alkylcarbonyl group substituted by a C₁₋₄ alkoxy group, a C₁₋₆ alkyl group substituted by a C₂₋₅ alkylcarbonyl group substituted by a C₁₋₄ alkylthio group, a C₁₋₆ alkyl group substituted by a C₂₋₅ alkylcarbonyl group suostituted by a C₁₋₄ alkylsulfinyl group, a C₁₋₆ alkyl group substituted by a C₂₋₅ alkylcarbonyl group substituted by a C₁₋₄ alkylsulfonyl group, a C₂₋₆ alkenyl group substituted by a C₂₋₇ alkylcarbonyl group, a C₂₋₆ alkynyl group substituted by a C₂₋₇ alkylcarbonyl group, a C₁₋₆ alkyl group substituted by a C₁₋₆ alkylsulfamoyl group, a C₁₋₆ alkyl group substituted by a C₁₋₆ alkoxysulfamoyl group, a C₁₋₆ alkyl group substituted by a di(C₁₋₃ alkyl)sulfamoyl group, a C₁₋₆ alkyl group substituted by an N-(C₁₋₃ alkyl)-N-(C₁₋₃ alkoxy)sulfamoyl group, a C₁₋₆ alkyl group substituted by a C₂₋₇ alkylcarbamoyl group, a C₁₋₆ alkyl group substituted by a di(C₁₋₃ alkyl)carbamoyl group, a C₁₋₆ alkyl group substituted by a C₂₋₇ alkoxycarbamoyl group, a C₁₋₆ alkyl group substituted by an N-(C₁₋₃ alkyl)-N-(C₁₋₃ alkoxy)carbamoyl group, a C₁₋₆ alkyl group substituted by a C₁₋₆ alkylamino group, a C₁₋₆ alkyl group substituted by a C₁₋₆ alkoxyamino group, a C₁₋ ₆ alkyl group substituted by a di(C₁₋₃ alkyl)amino group, a C₁₋₆ alkyl group substituted by an N-(C₁₋₃ alkyl)-N-(C₁₋ ₃ alkoxy)amino group, a C₁₋₆ alkyl group substituted by an N-(C₂₋₇ alkylcarbonyl)-N-(C₁₋₆ alkyl)amino group, a C₁₋ ₆ alkyl group substituted by an N-(C₂₋₇ alkylcarbonyl)-N-(C₁₋₆ alkoxy)amino group, a C₁₋₆ alkyl group substituted by an N-(C₁₋₆ alkylsulfonyl)-N-(C₁₋₆ alkyl)amino group, a C₁₋₆ alkyl group substituted by an N-(C₁₋₆ alkylsulfonyl)-N-(C₁₋₆ alkoxy)amino group, a C₁₋₆ alkyl group substituted by a phenyl group (provided that such a phenyl group may be substituted by one or more substituents selected from the group consisting of a halogen atom, a trifluoromethyl group, a nitro group, a C₁₋₆ alkyl group, a C₁₋₆ alkoxy group and a C₂₋₇ alkoxycarbonyl group), a C₂₋₇ alkenyl group substituted by a phenyl group (provided that such a phenyl group may be substituted by one or more substituents selected from the group consisting of a halogen atom, a trifluoromethyl group, a nitro group, a C₁₋₆ alkyl group, a C₁₋₆ alkoxy group and a C₂₋₇ alkoxycarbonyl group), a C₂₋₆ alkynyl group substituted by a phenyl group (provided that such a phenyl group may be substituted by one or more substituents selected from the group consisting of a halogen atom, a trifluoromethyl group, a nitro group, a C₁₋₆ alkyl group, a C₁₋₆ alkoxy group and a C₂₋₇ alkoxycarbonyl group), a C₁₋₆ alkyl group substituted by a phenoxy group (provided that such a phenoxy group may be substituted by one or more substituents selected from the group consisting of a halogen atom, a trifluoromethyl group, a nitro group, a C₁₋₆ alkyl group, a C₁₋₆ alkoxy group and a C₂₋₇ alkoxycarbonyl group), a C₁₋₆ alkyl group substituted by a phenylthio group (provided that such a phenylthio group may be substituted by one or more substituents selected from the group consisting of a halogen atom, a trifluoromethyl group, a nitro group, a C₁₋₆ alkyl group, a C₁₋₆ alkoxy group and a C₂₋₇ alkoxycarbonyl group), a C₁₋₆ alkyl group substituted by a phenylsulfinyl group (provided that such a phenylsulfinyl group may be substituted by one or more substituents selected from the group consisting of a halogen atom, a trifluoromethyl group, a nitro group, a C₁₋₆ alkyl group, a C₁₋₆ alkoxy group and a C₂₋₇ alkoxycarbonyl group), a C₁₋₆ alkyl group substituted by a phenylsulfonyl group (provided that such a phenylsulfonyl group may be substituted by one or more substituents selected from the group consisting of a halogen atom, a trifluoromethyl group, a nitro group, a C₁₋₆ alkyl group, a C₁₋₆ alkoxy group and a C₂₋₇ alkoxycarbonyl group), a C₁₋₆ alkyl group substituted by a benzyloxy group (provided that the phenyl group of such a benzyloxy group may be substituted by one or more substituents selected from the group consisting of a halogen atom, a trifluoromethyl group, a nitro group, a C₁₋₆ alkyl group, a C₁₋₆ alkoxy group and a C₂₋₇ alkoxycarbonyl group), a C₁₋₆ alkyl group substituted by a benzylthio group (provided that the phenyl group of such a benzylthio group may be substituted by one or more substituents selected from the group consisting of a halogen atom, a trifluoromethyl group, a nitro group, a C₁₋₆ alkyl group, a C₁₋₆ alkoxy group and a C₂₋₇ alkoxycarbonyl group), a C₁₋₆ alkyl group substituted by a benzylsulfinyl group (provided that the phenyl group of such a benzylsulfinyl group may be substituted by one or more substituents selected from the group consisting of a halogen atom, a trifluoromethyl group, a nitro group, a C₁₋₆ alkyl group, a C₁₋₆ alkoxy group and a C₂₋₇ alkoxycarbonyl group), a C₁₋₆ alkyl group substituted by a benzylsulfonyl group (provided that the phenyl group of such a benzylsulfonyl group may be substituted by one or more substituents selected from the group consisting of a halogen atom, a trifluoromethyl group, a nitro group, a C₁₋₆ alkyl group, a C₁₋₆ alkoxy group and a C₂₋₇ alkoxycarbonyl group), a C₁₋₆ alkyl group substituted by a phenylcarbonyl group (provided that such a phenylcarbonyl group may be substituted by one or more substituents selected from the group consisting of a halogen atom, a trifluoromethyl group, a nitro group, a C₁₋₆ alkyl group, a C₁₋₆ alkoxy group and a C₂₋₇ alkoxycarbonyl group), or a C₁₋₆ alkyl group substituted by a benzylcarbonyl group (provided that the phenyl group of such a benzylcarbonyl group may be substituted by one or more substituents selected from the group consisting of a halogen atom, a trifluoromethyl group, a nitro group, a C₁₋₆ alkyl group, a C₁₋₆ alkoxy group and a C₂₋₇ alkoxycarbonyl group),
each of R^{g2} and R^{g3} which are independent of each other, is a C₁₋₆ alkyl group, a C₂₋₆ alkenyl group, a C₂₋₆ alkynyl group, a mono-, di- or poly-halogeno C₁₋₆ alkyl group, a C₁₋₆ alkoxy group, a C₁₋₆ alkylsulfonyl group, a C₁₋₆ alkylsulfamoyl group, a di(C₁₋₃ alkyl)sulfamoyl group, a C₂₋₇ alkoxycarbonyl group, a C₂₋₇ alkylcarbonyl group, a C₂₋₇ alkylcarbamoyl group, a di(C₁₋₃ alkyl)carbamoyl group, a phenyl group (provided that such a phenyl group may be substituted by one or more substituents selected from the group consisting or a halogen atom, a trifluoromethyl group, a nitro group, a C₁₋₆ alkyl group, a C₁₋₆ alkoxy group and a C₂₋₇ alkoxycarbonyl group), or a benzyl group (provided that the phenyl group of such a benzyl group may be substituted by one or more substituents selected from the group consisting of a halogen atom, a trifluoromethyl group, a nitro group, a C₁₋₆ alkyl group, a C₁₋₆ alkoxy group and a C₂₋₇ alkoxycarbonyl group),
or R^{g2} and R^{g3} form a saturated 3- to 7-membered heterocyclic ring together with the nitrogen atom to which they are bonded,
X is an oxygen atom or a sulfur atom,
L is a hydrogen atom, a C₁₋₆ alkyl group, a C₂₋₆ alkenyl group or a C₂₋₆ alkynyl group,
G is 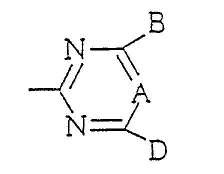
A is a CH group, or a nitrogen atom, and
each of B and D which are independent of each other, is a C₁₋₄ alkyl group, a C₁₋₄ alkoxy group, a mono-, di- or poly-halogeno C₁₋₄ alkyl group, a mono-, di- or poly-halogeno C₁₋₄ alkoxy group, a halogen atom, a C₁₋₄ alkylamino group, or a di(C₁₋₄ alkyl)amino group.

3. The process for preparing an iminosulfonylurea derivative or its salt according to Claim 2, wherein Q is

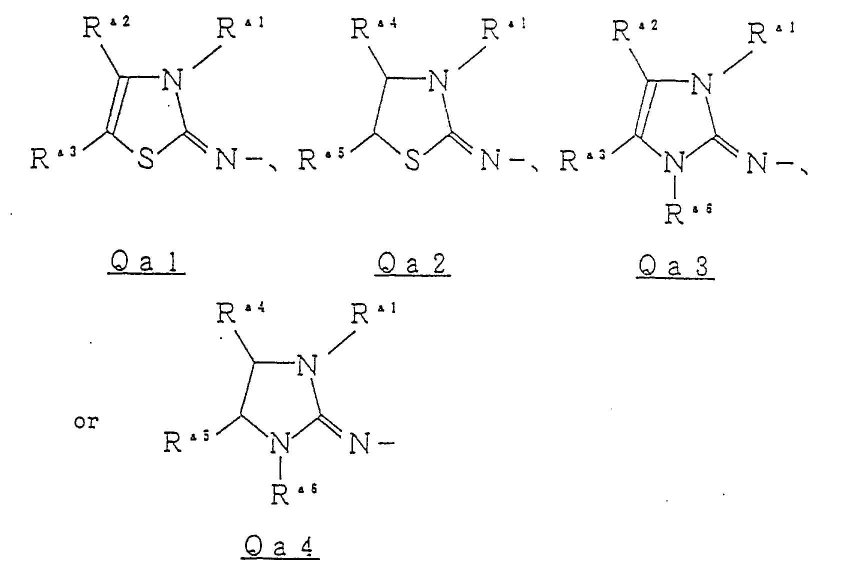

4. The process for preparing an iminosulfonylurea derivative or its salt according to Claim 2, wherein Q is

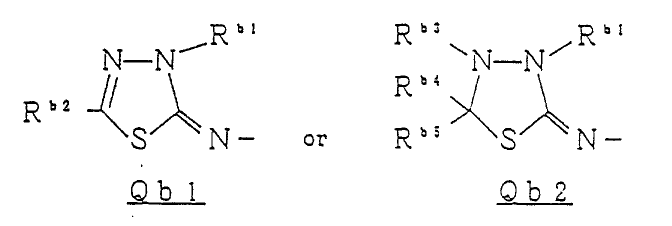

5. The process for preparing an iminosulfonylurea derivative or its salt according to Claim 2, wherein Q is

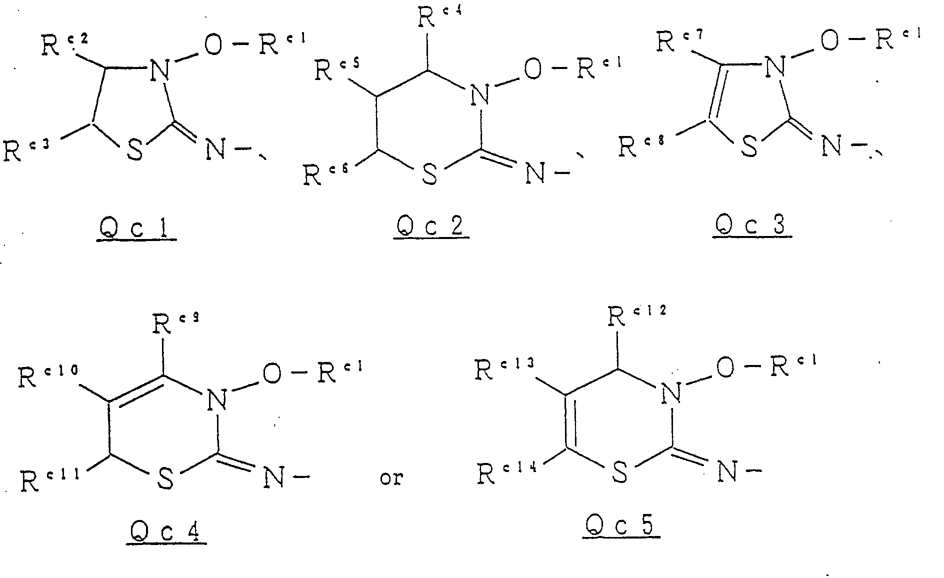

6. The process for preparing an iminosulfonylurea derivative or its salt according to Claim 2, wherein Q is

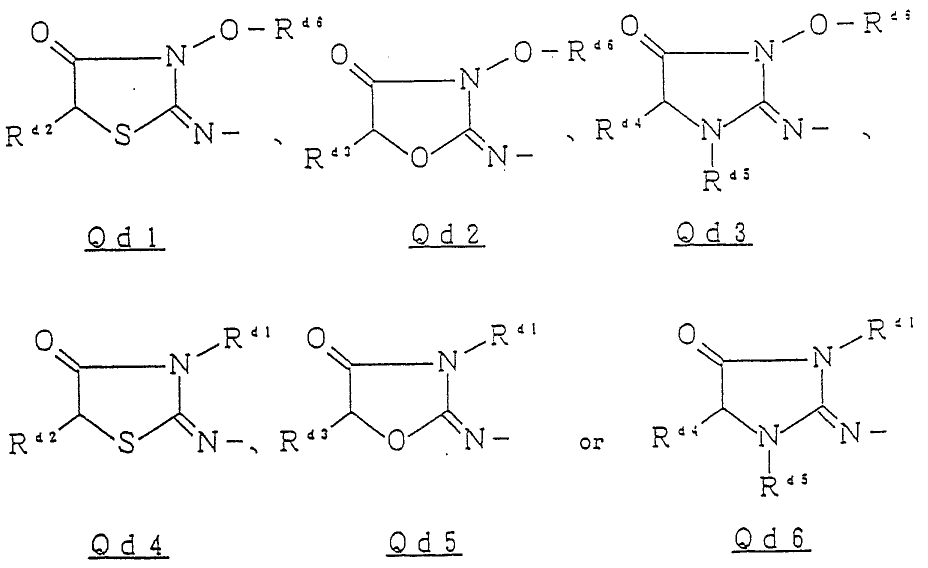

7. The process for preparing an iminosulfonylurea derivative or its salt according to Claim 2, wherein Q is

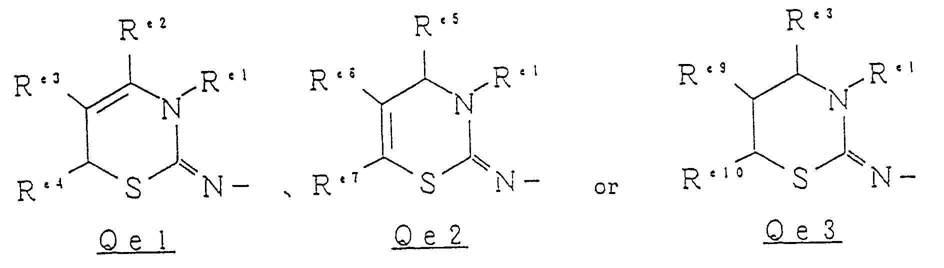

8. The process for preparing an iminosulfonylurea derivative or its salt according to Claim 2, wherein Q is

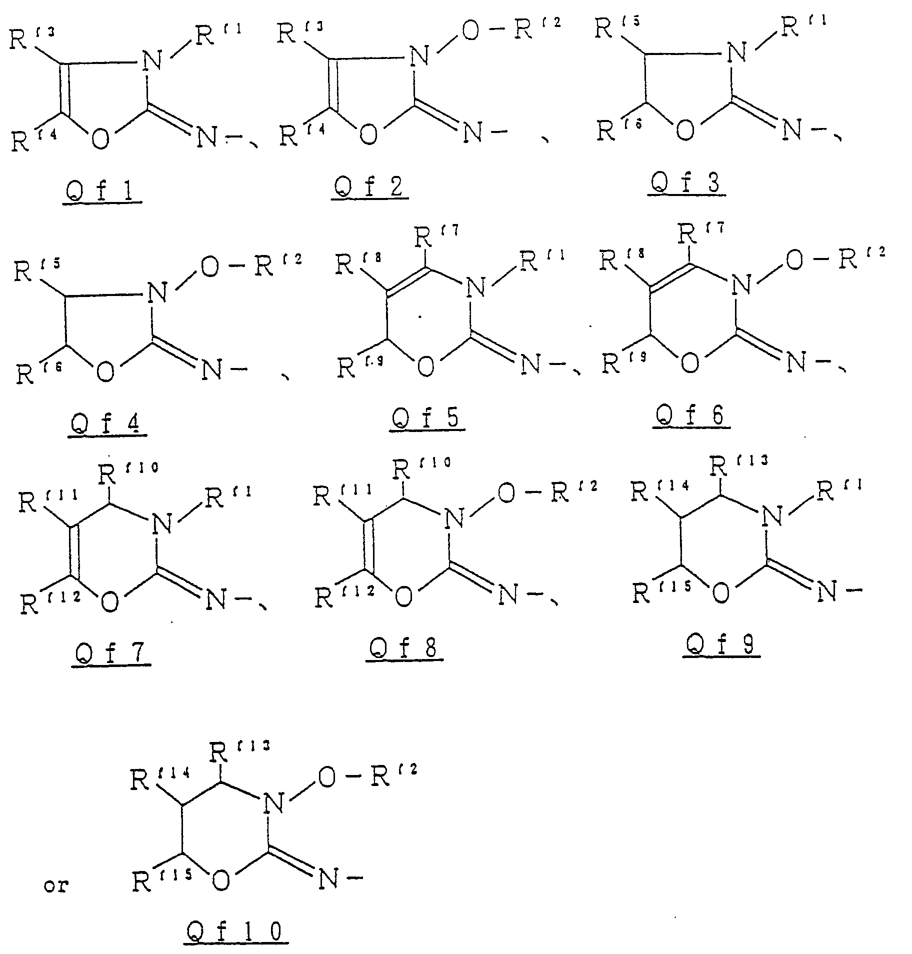

9. The process for preparing an iminosulfonylurea derivative or its salt according to Claim 2, wherein Q is

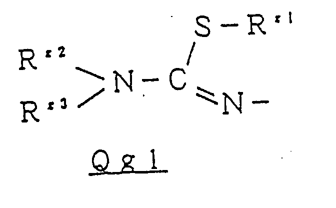

10. The process for preparing an iminosulfonylurea derivative or its salt according to Claim 3, wherein R^{a1} is 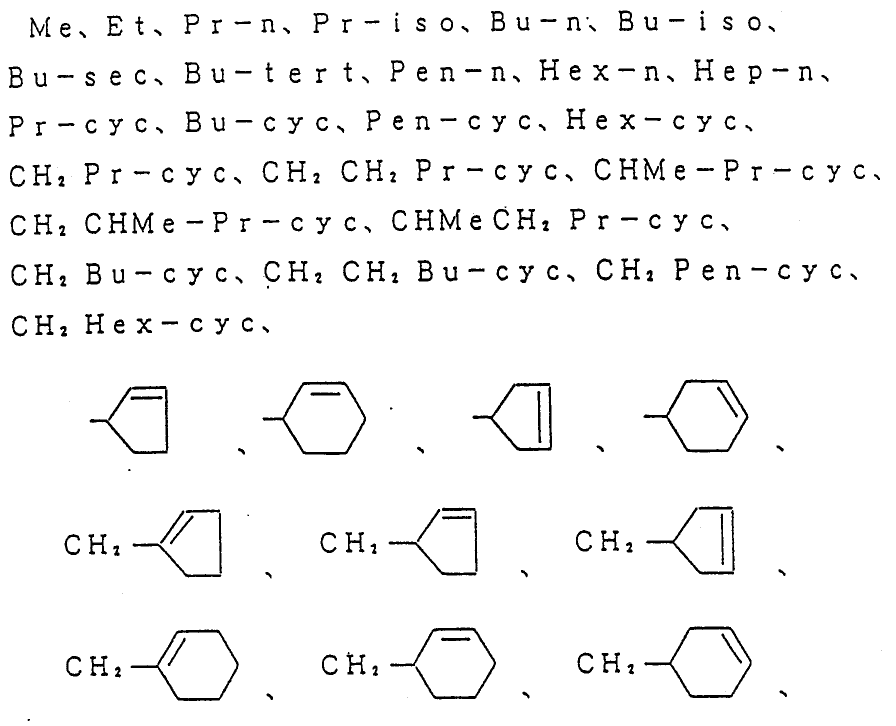 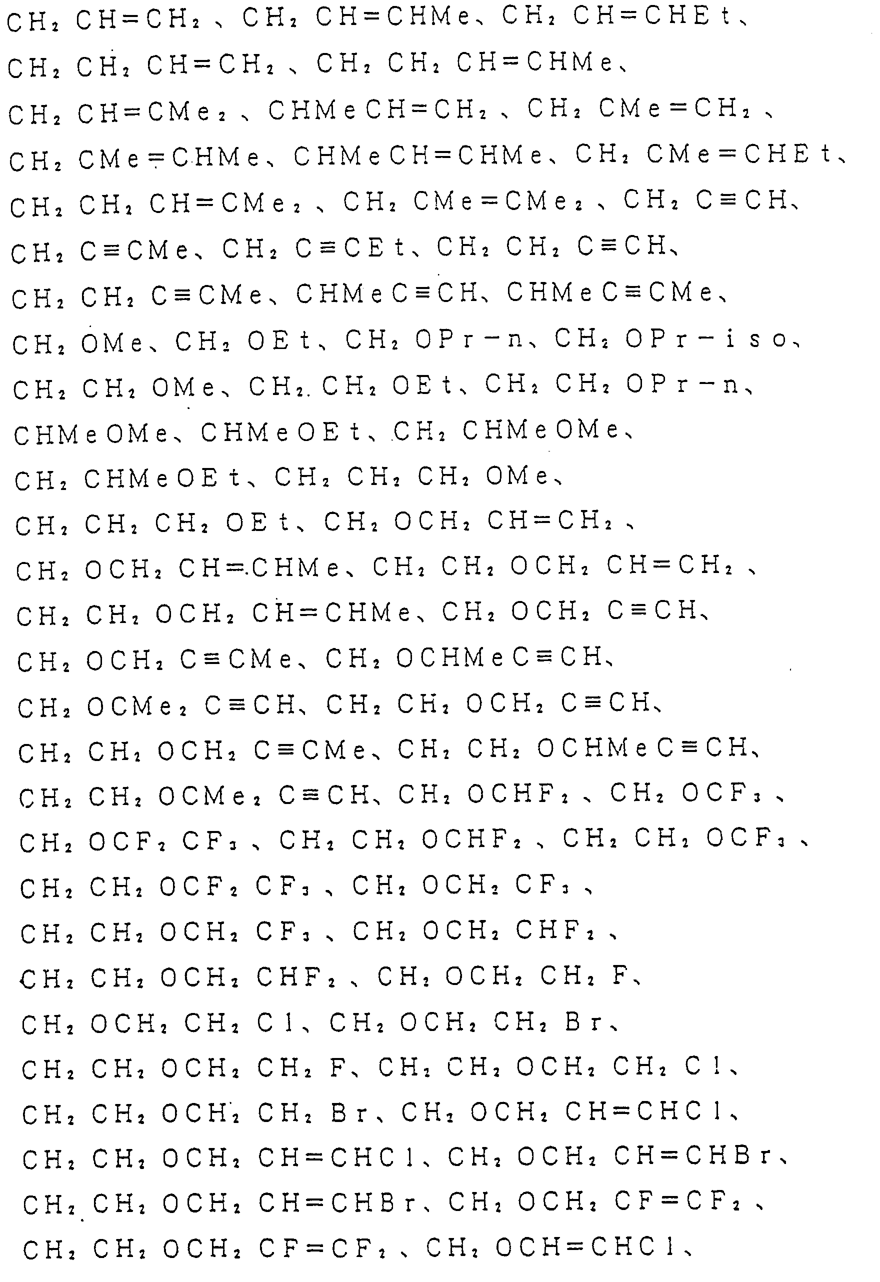 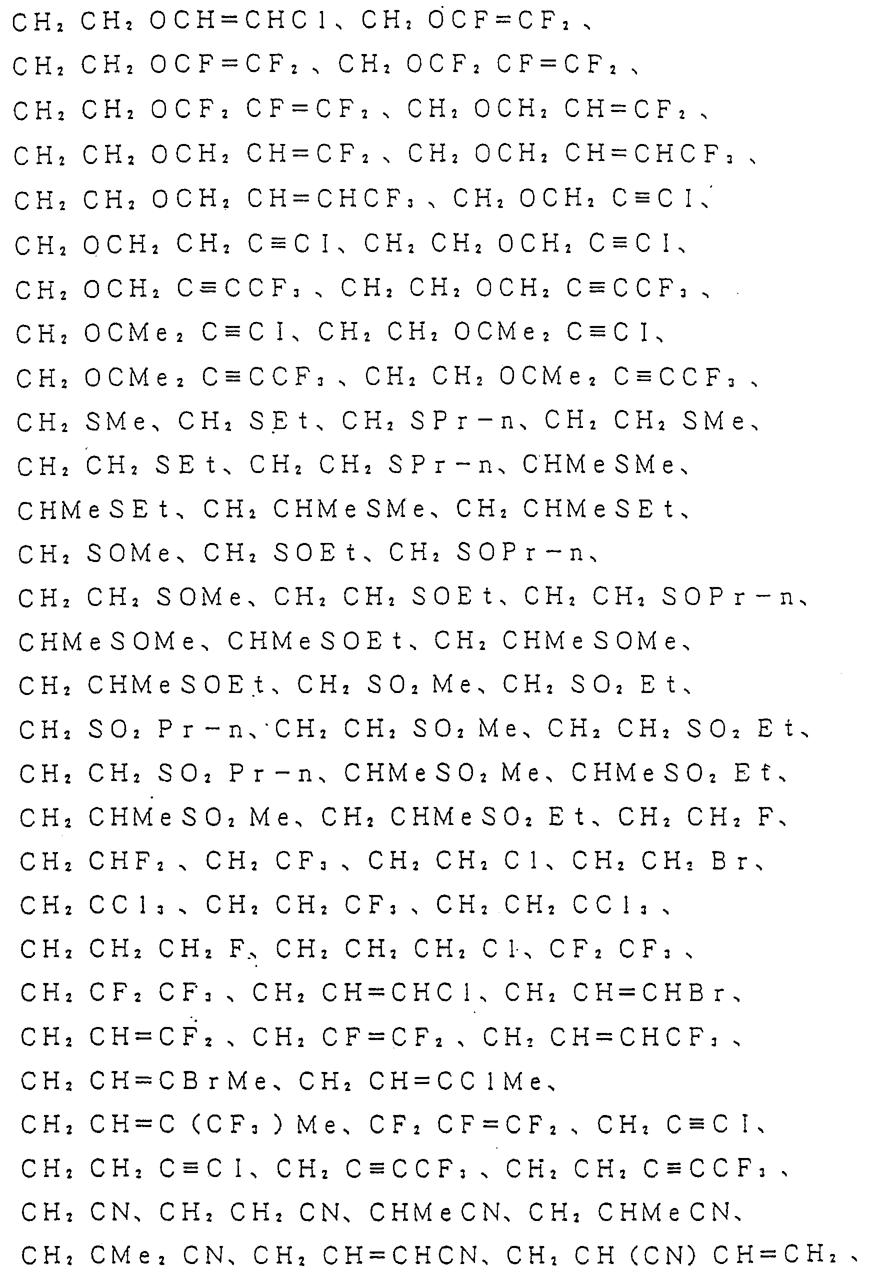 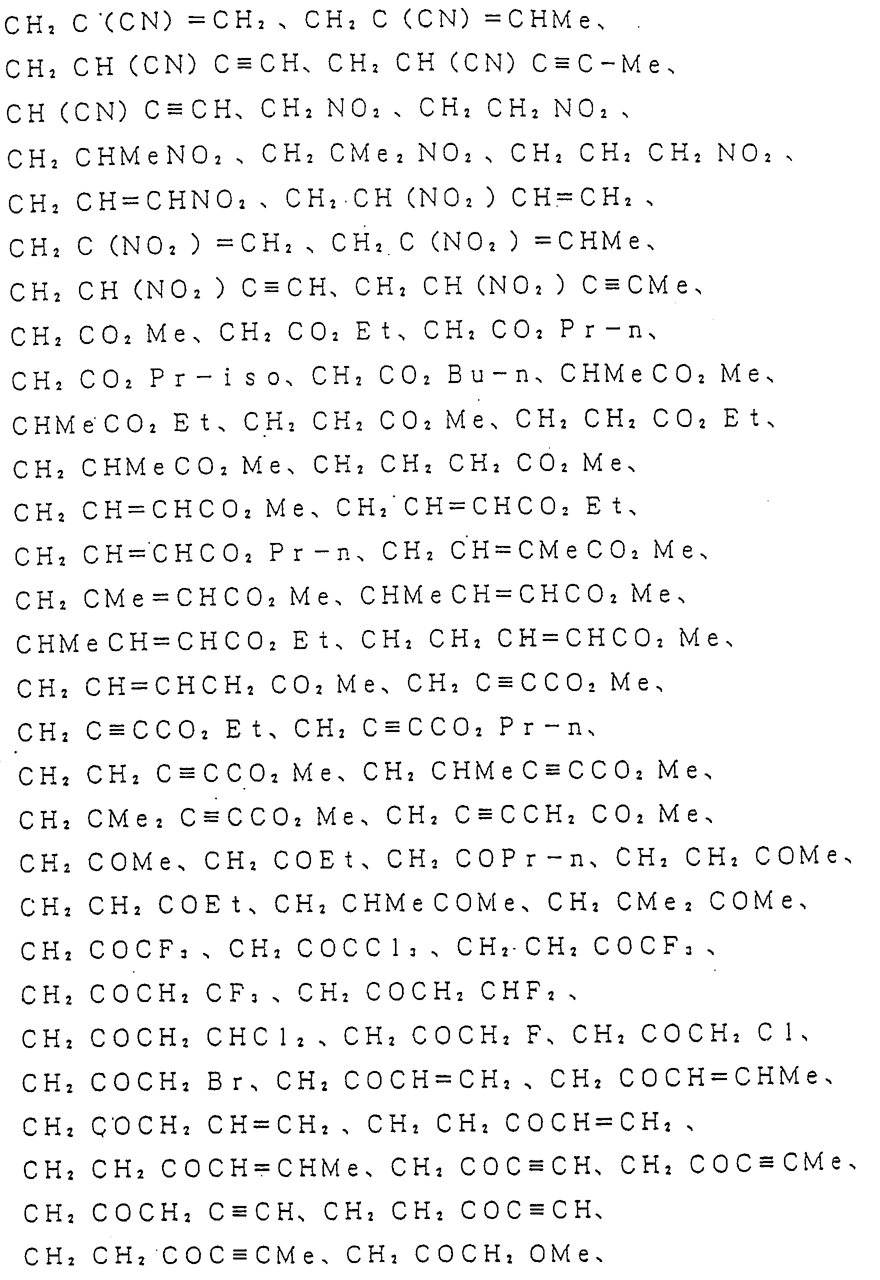 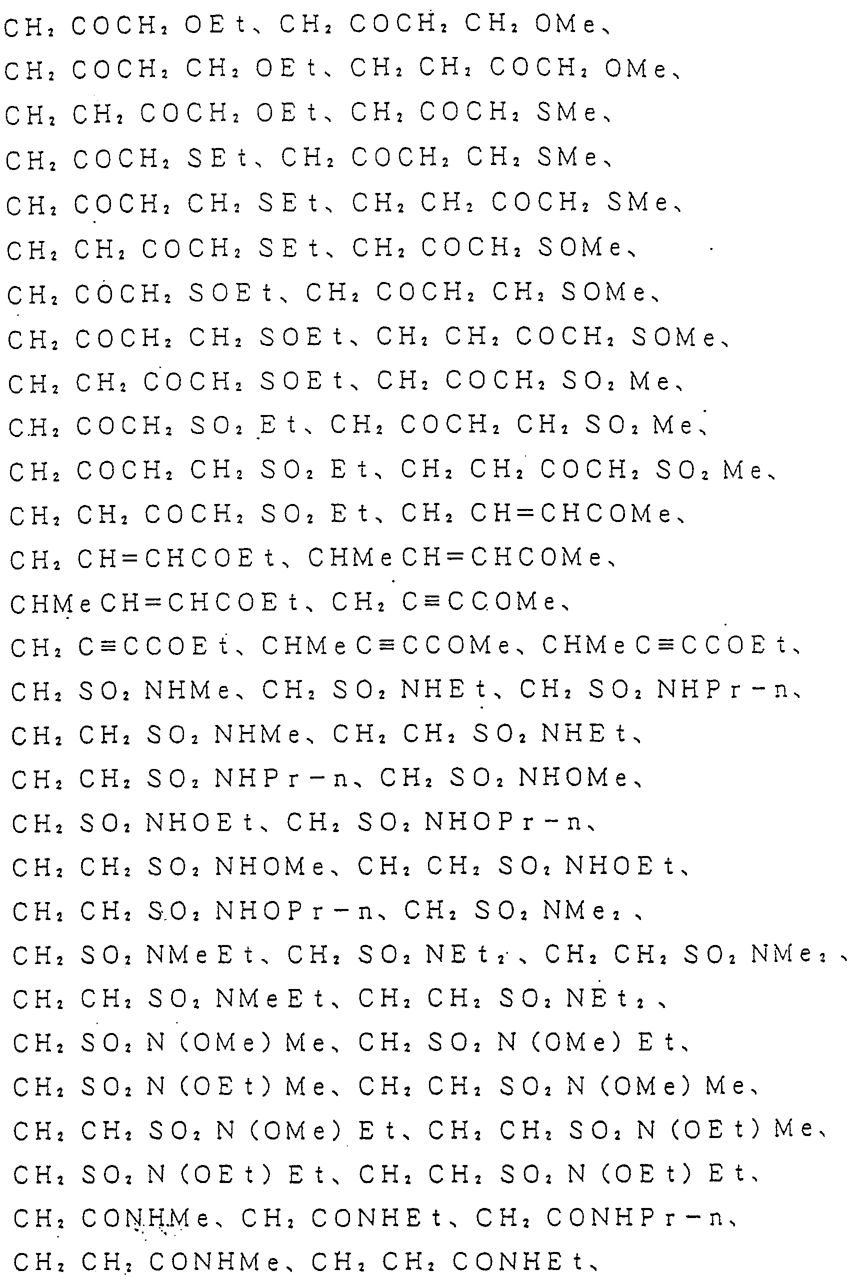 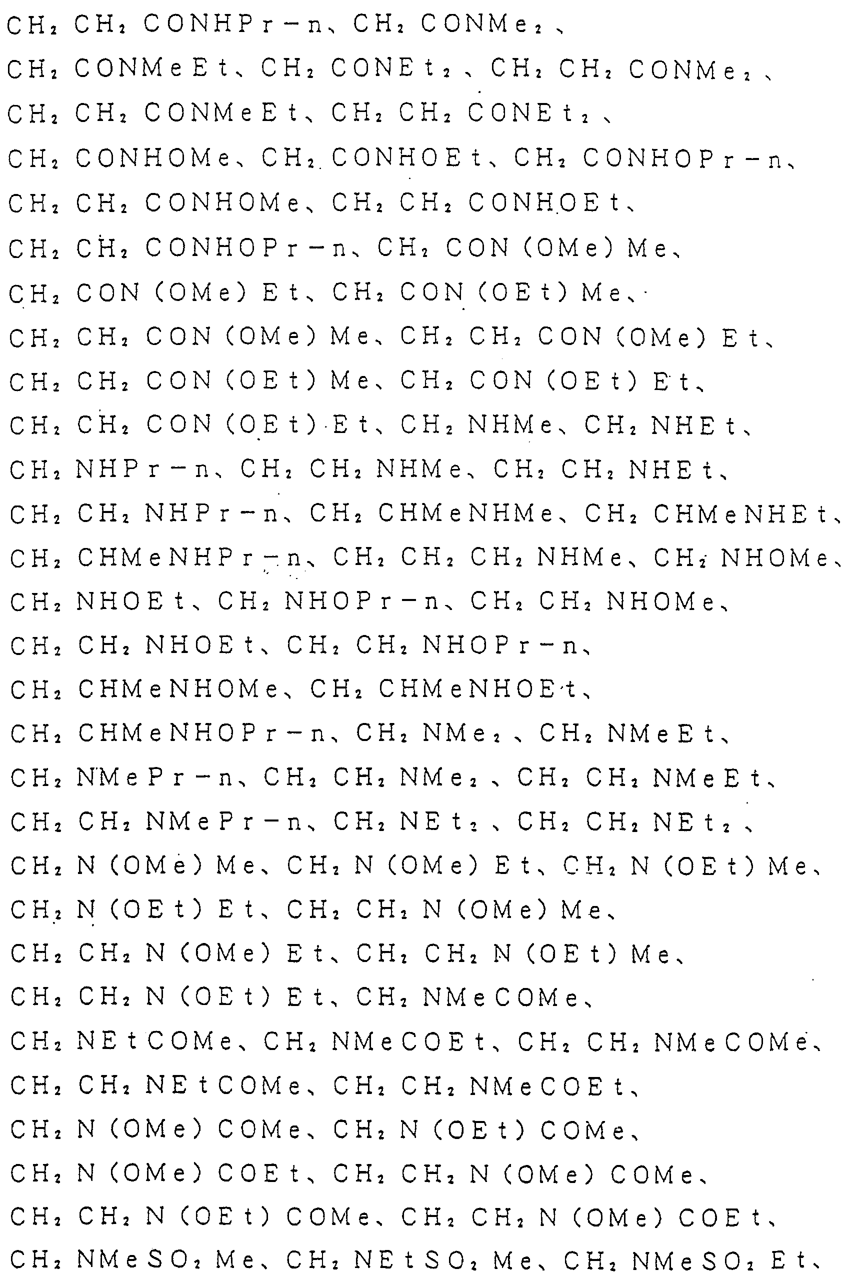 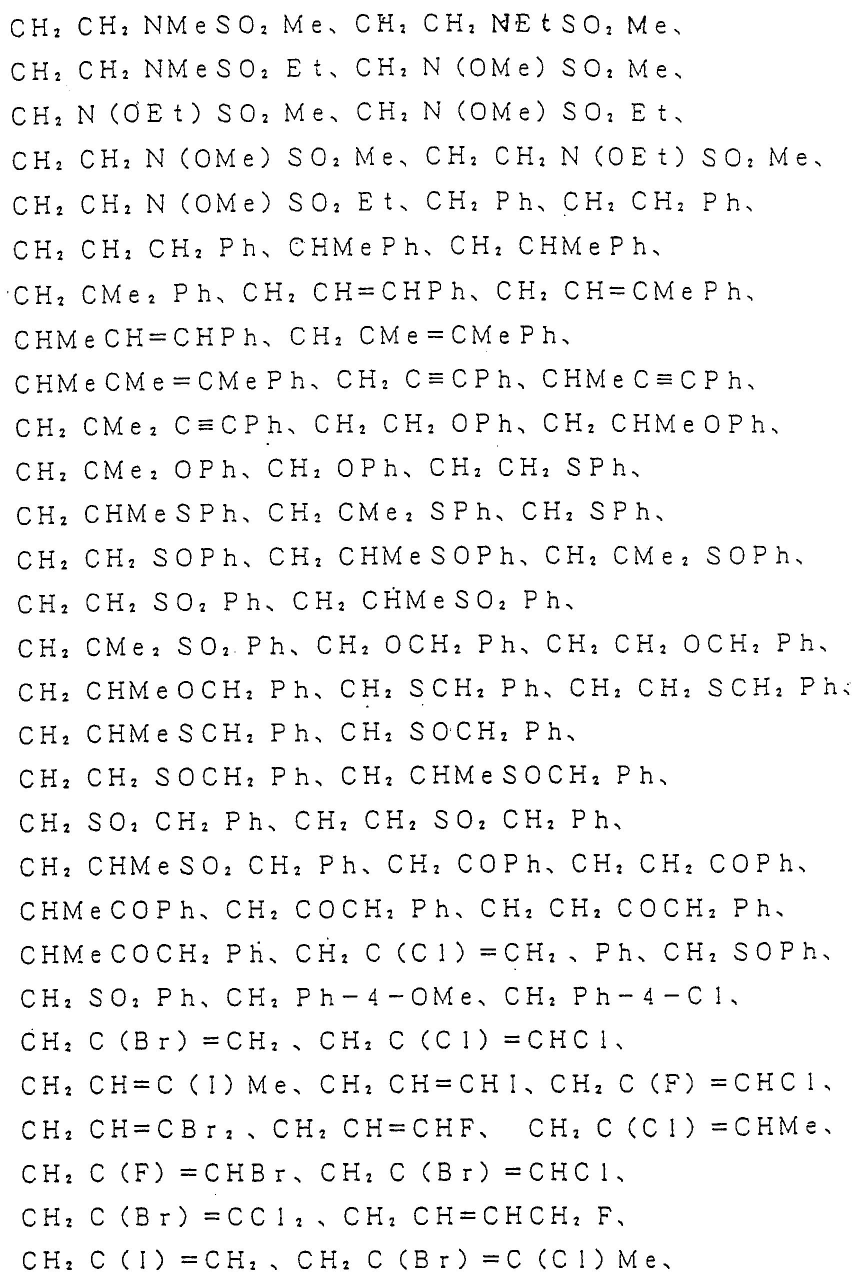 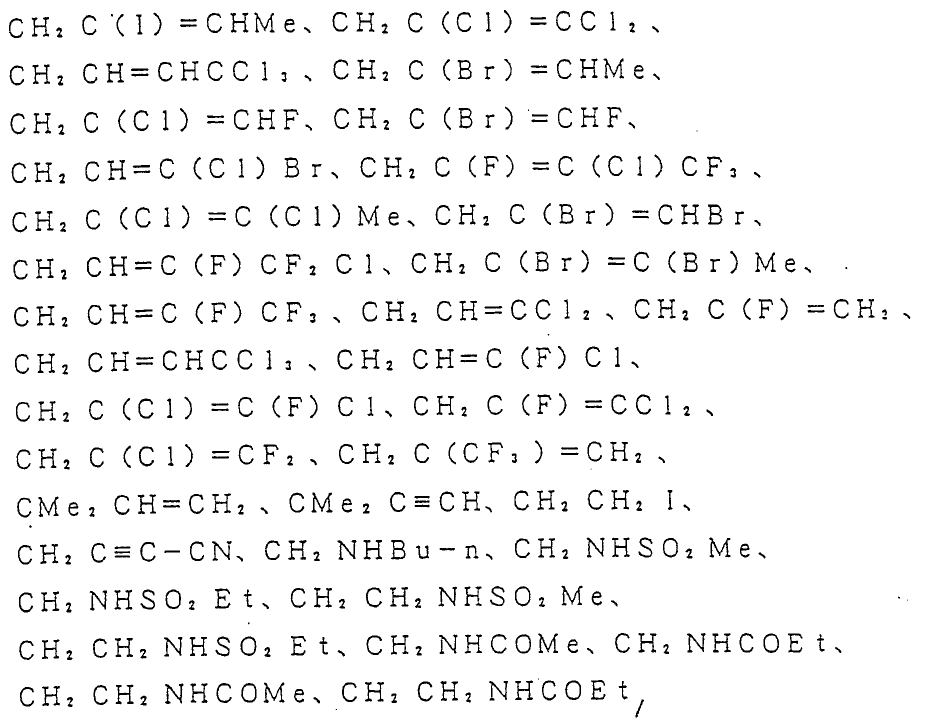 R^{a2} and R^{a3} is 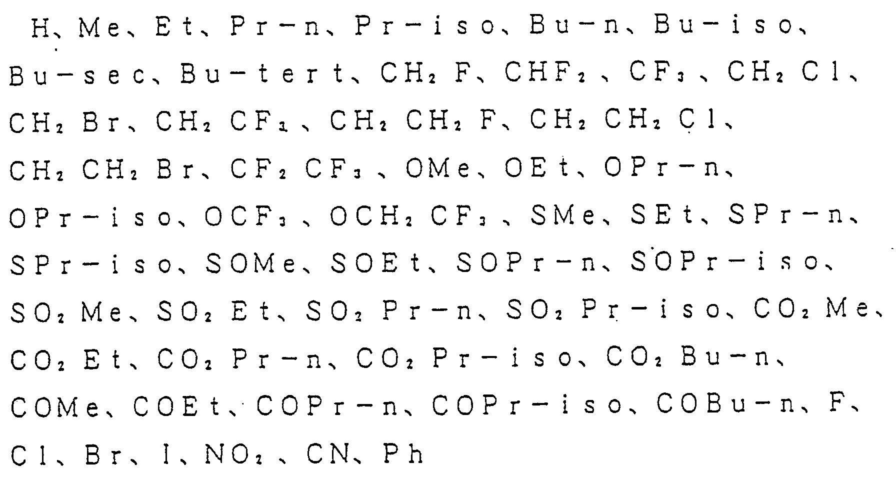 B and D is

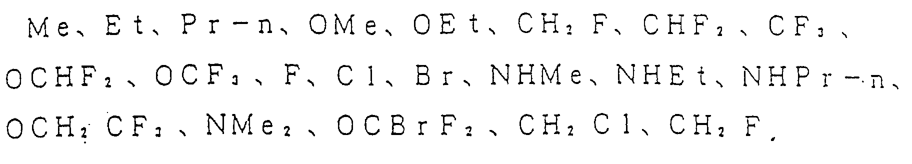

11. The process for preparing an iminosulfonylurea derivative or its salt according to Claim 3, wherein R^{a1} is

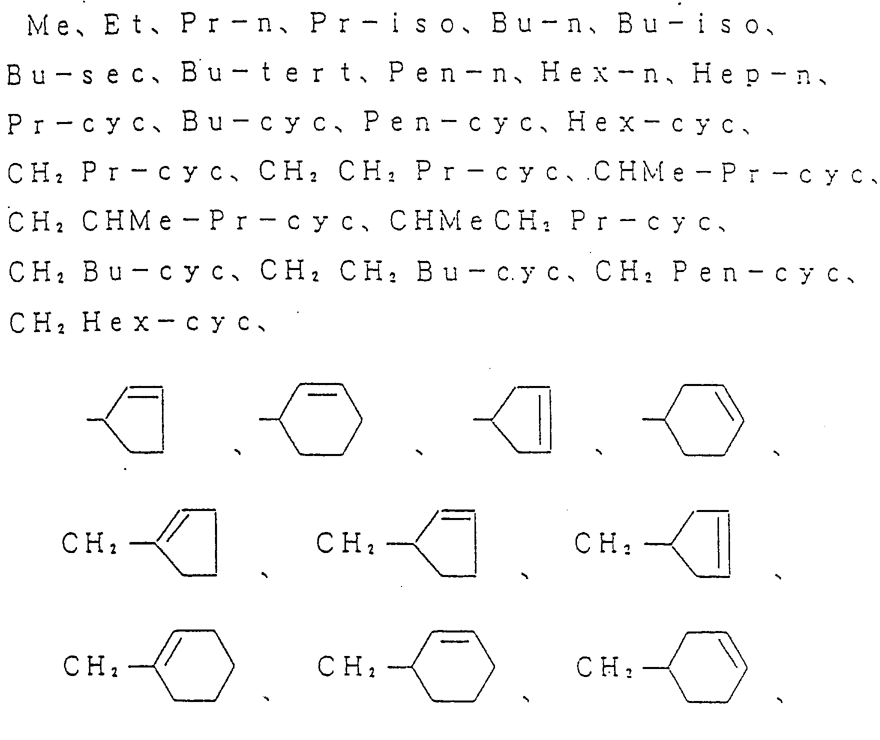

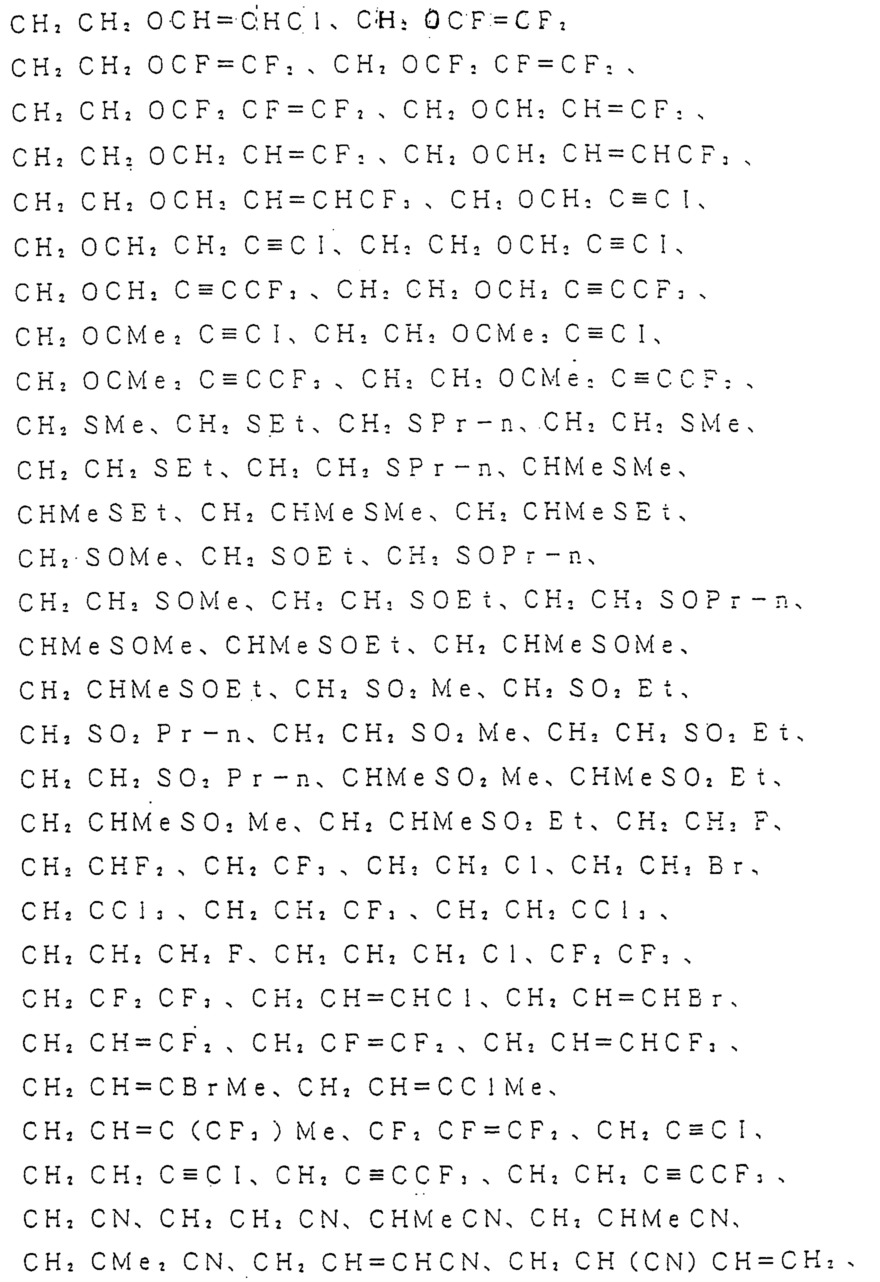

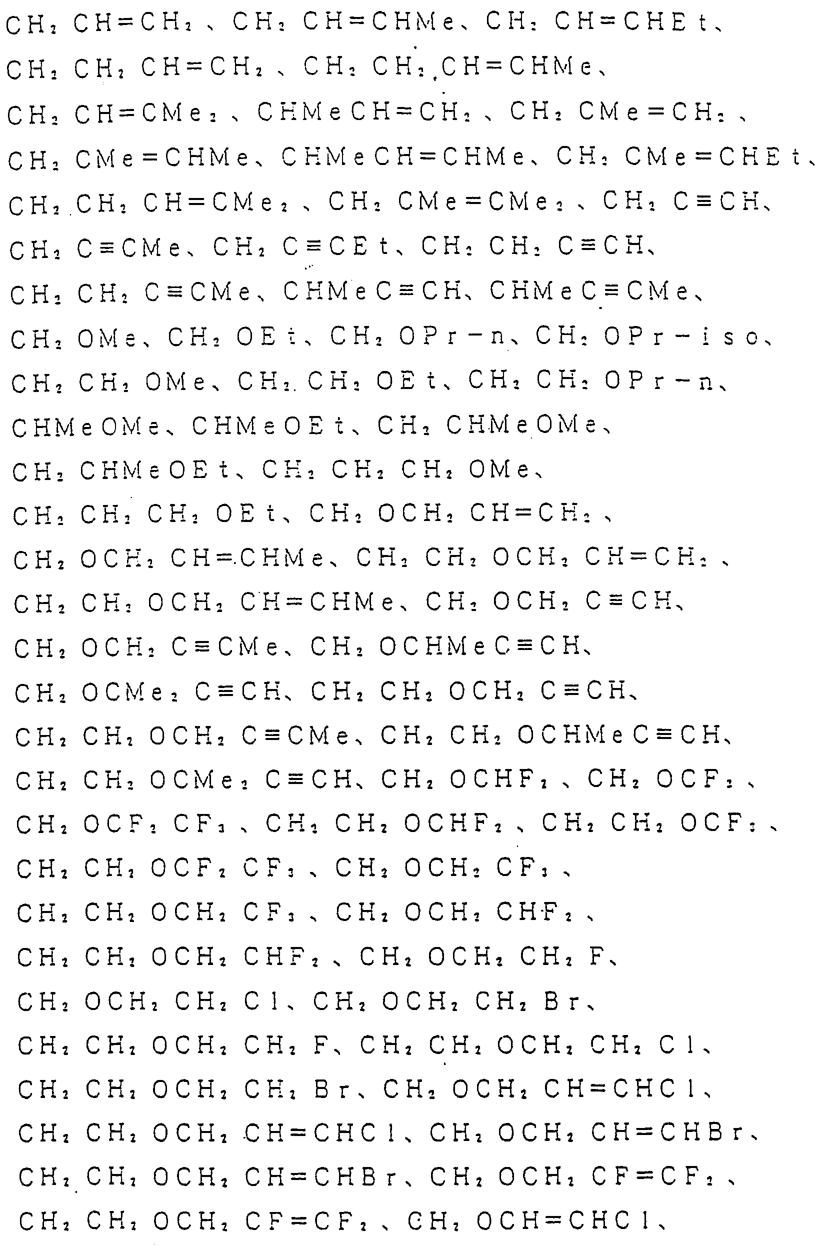

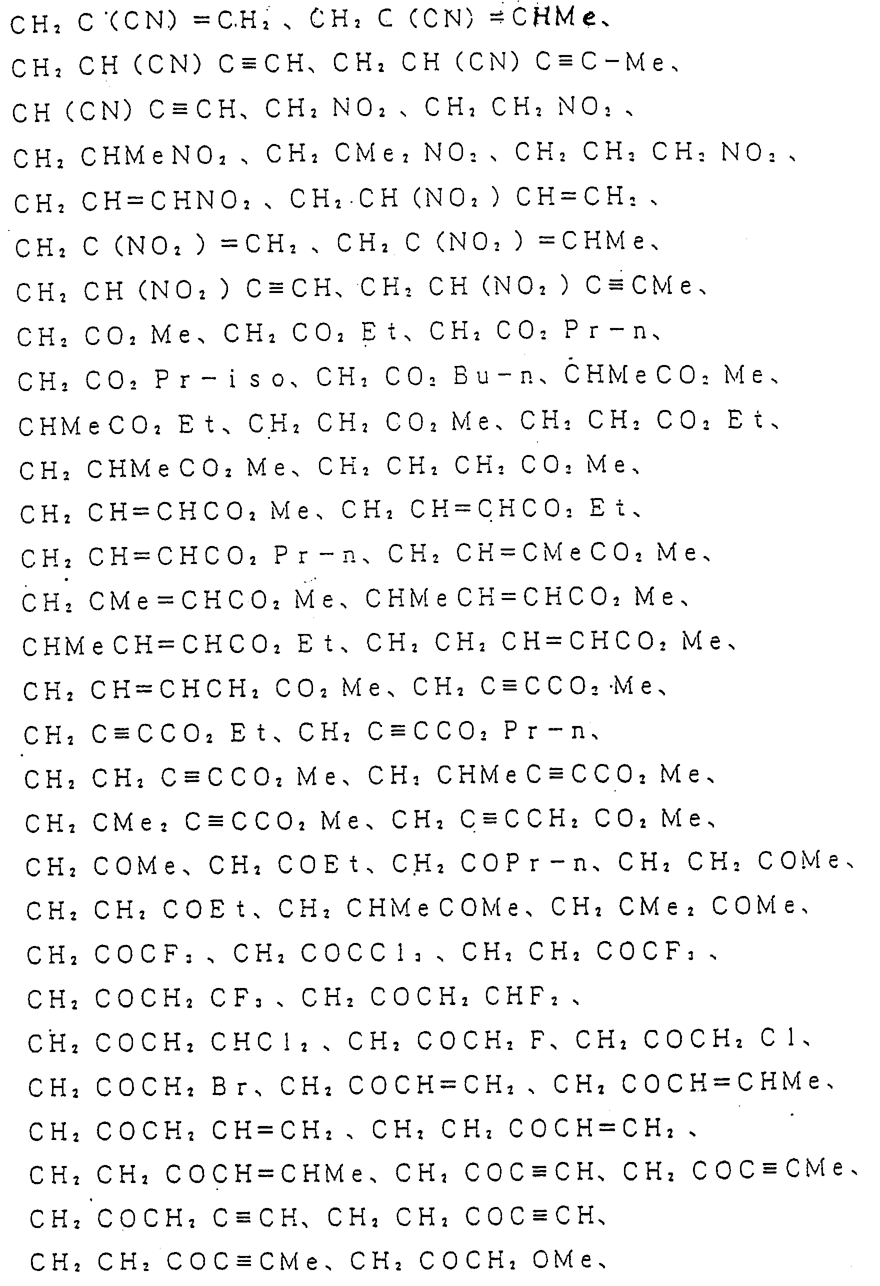

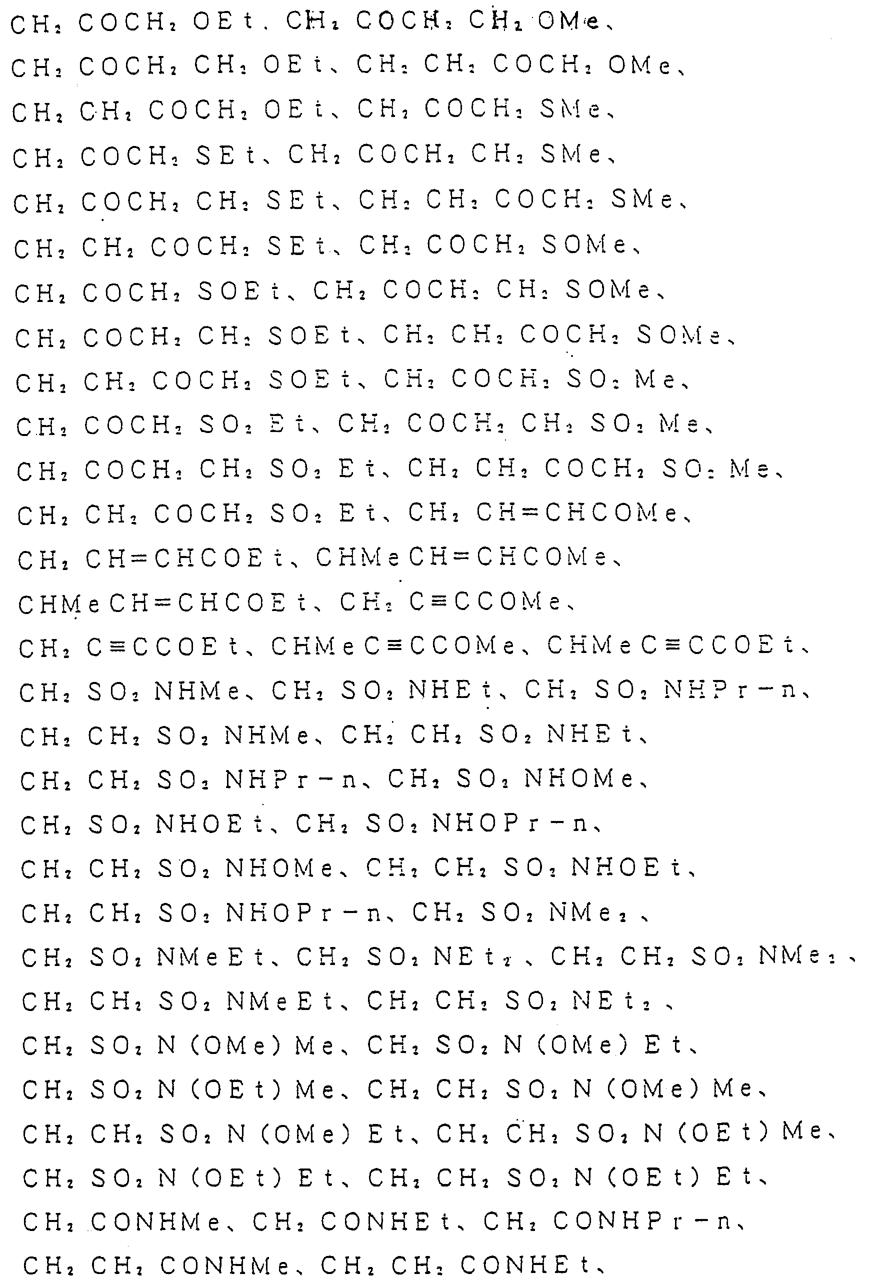

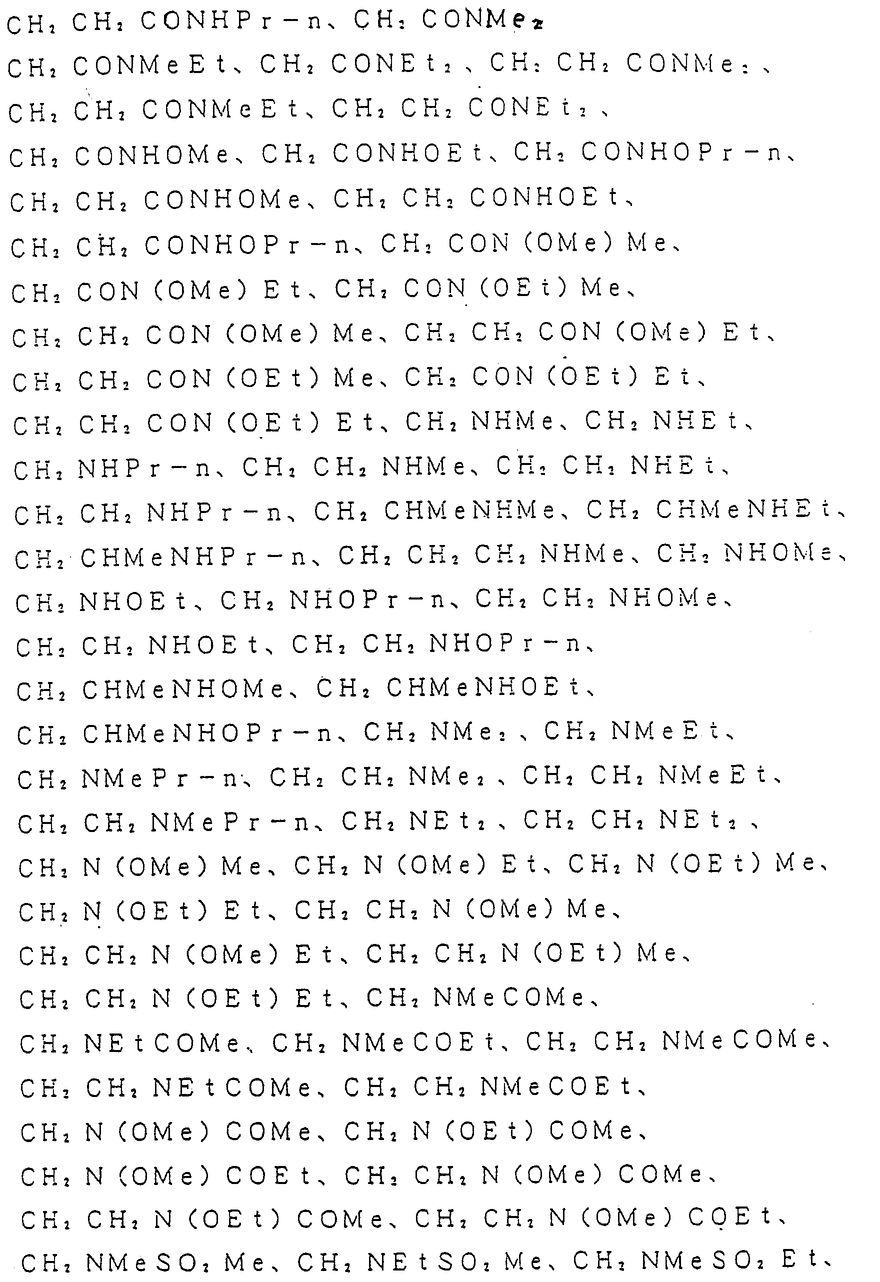

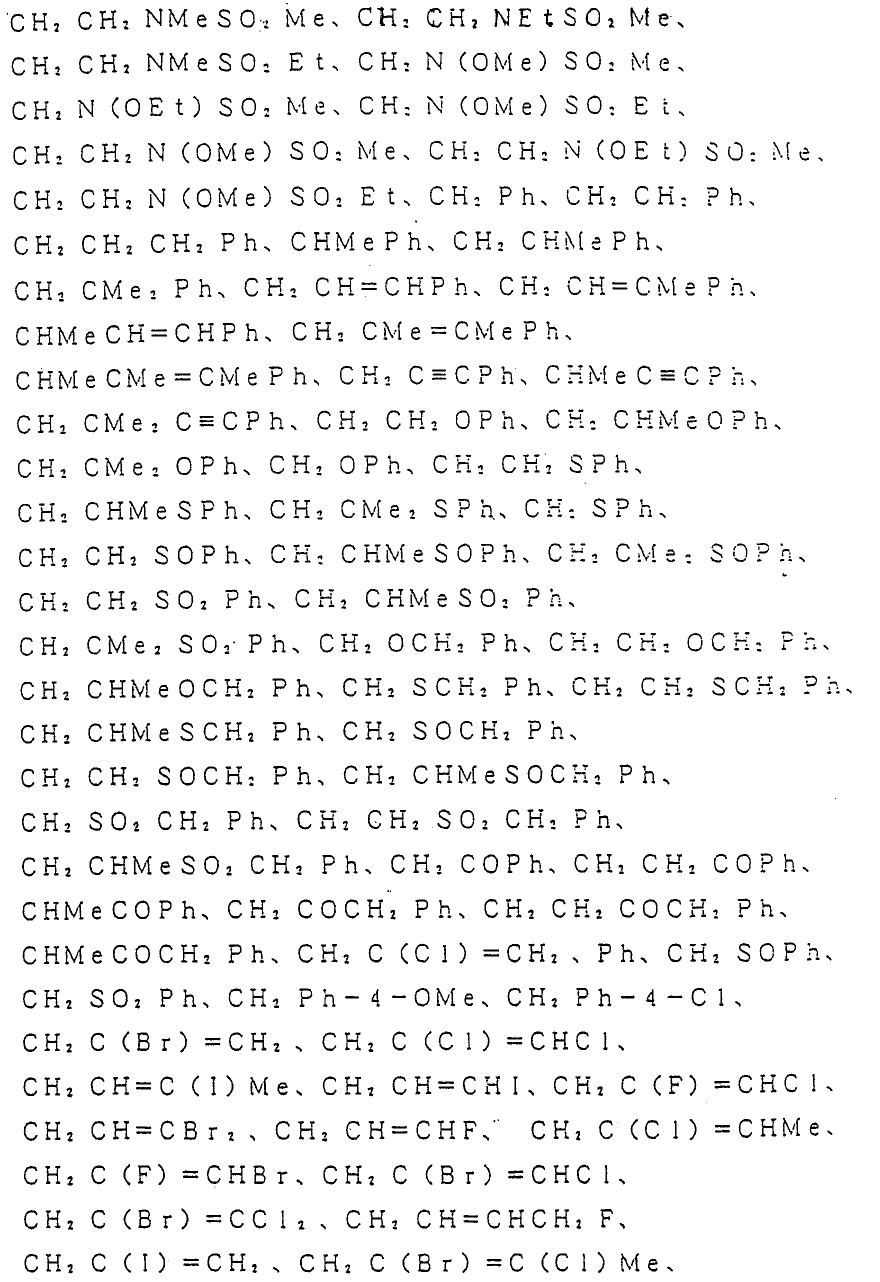

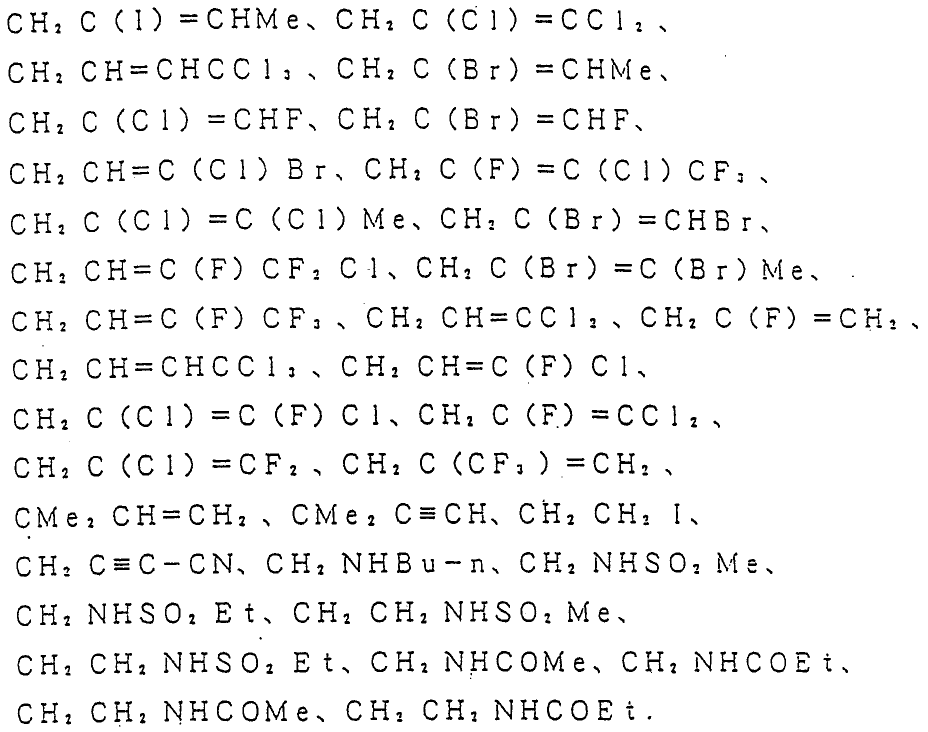

12. The process for preparing an iminosulfonylurea derivative or its salt according to Claim 3, wherein R^{a2} and R^{a3} is

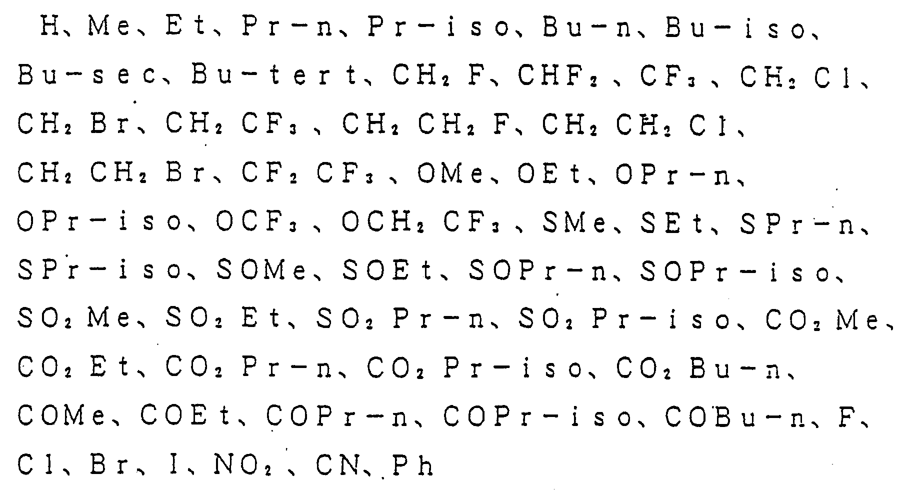

13. The process for preparing an iminosulfonylurea derivative or its salt according to Claim 3, wherein B and D is

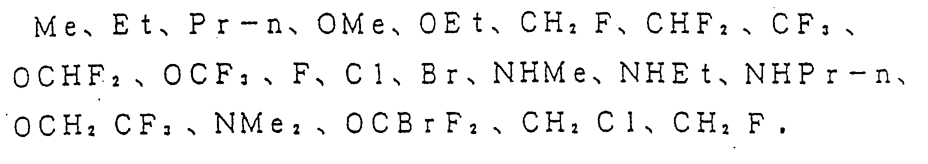

14. A process for the preparation of an herbicide which contains an iminosulfonylurea derivative as prepared in any of claims 1 to 13 said process comprising formulating said iminosulfonylurea derivative together with a suitable carrier.

15. A herbicidal and growth control method against weeds, which comprises applying a herbicidally effective amount of an iminosulfonylurea derivative as prepared in any of claims 1 to 13.

## Patentansprüche (Patentansprüche für folgende(n) Vertragsstaat(en): AT, BE, CH, DE, DK, FR, GB, IT, LI, LU, MC, NL, SE)

1. Iminosulfonyl-Harnstoffderivat der Formel (1) oder dessen Salz: 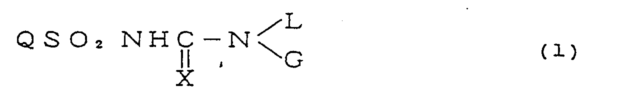
wobei Q für 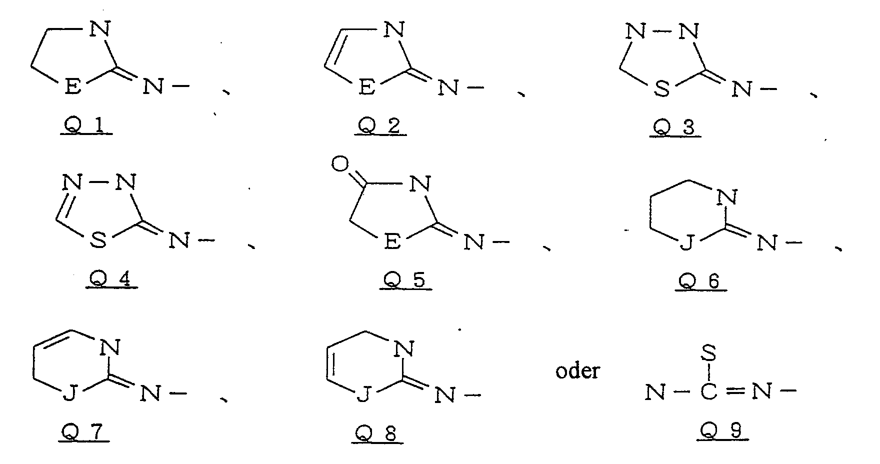 steht, wobei in Q1, Q2 und Q5 das E für ein Schwefelatom, ein Sauerstoffatom oder ein Stickstoffatom steht, das monosubstituiert ist, mit einem Substituenten, der nicht Wasserstoffatom ist; wobei in Q6, Q7 und Q8 das J für ein Schwefelatom oder ein Sauerstoffatom steht; wobei in Q1 bis Q8 ein Stickstoffatom im Ring von Q substituiert ist mit einem Substituenten, der kein Wasserstoffatom ist und ein Kohlenstoff im Ring von Q gegebenerfalls substituiert ist; und in Q9 das Schwefelatom und das Stickstoffatom an dem Kohlenstoffatom, an dem die Iminogruppe von Q gebunden ist, mit Substituenten, die keine Wasserstoffatome sind, substituiert sind,
X für ein Sauerstoffatom oder für ein Schwefelatom steht,
L für ein Wasserstoffatom, eine C₁₋₆-Alkylgruppe, eine C₂₋₆-Alkenylgruppe oder eine C₂₋₆-Alkinylgruppe steht,
G für 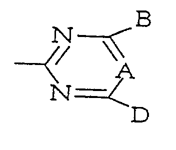
A für eine CH-Gruppe oder ein Stickstoffatom steht, und
jedes B und D, die unabhängig voneinander sind, für eine C₁₋₄-Alkylgruppe, eine C₁₋₄-Alkoxygruppe, eine Mono-, Di- oder Polyhalogen-C₁₋₄-Alkylgruppe, eine Mono-, Di- oder Polyhalogen-C₋₁₋₄-Alkoxygruppe, ein Halogenatom, eine C₋₁₋₄-Alkylaminogruppe oder eine Di(C₁₋₄-alkyl)aminogruppe stehen.

2. Iminosulfonyl-Harnstoffderivat der Formel (1) bzw. dessen Salz nach Anspruch 1: 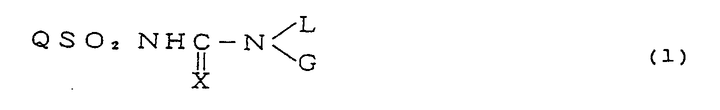
wobei Q für 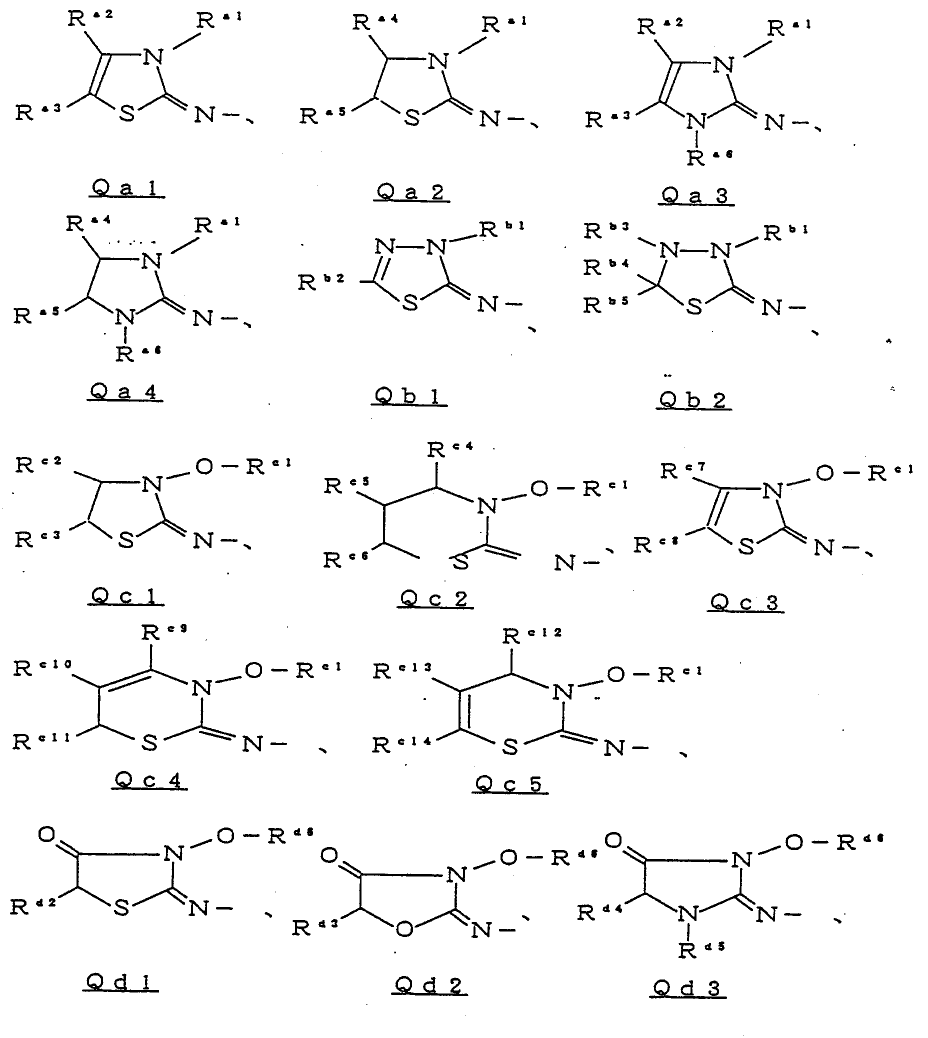 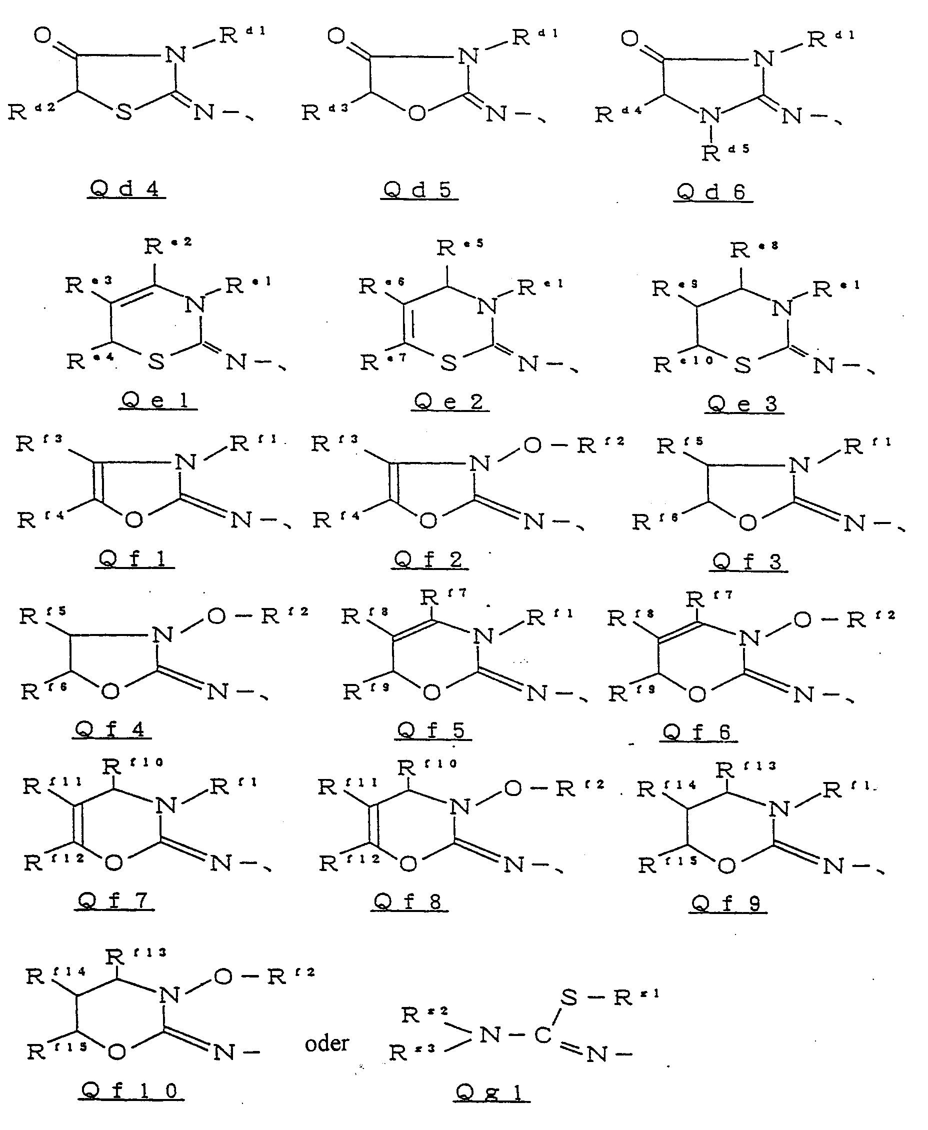
steht, R^{a1} für eine C₁₋₈-Alkylgruppe, eine C₃₋₇-Cycloalkylgruppe, eine mit einer C₃₋₇-Cycloalkylgruppe substituierte C₁₋₆-Alkylgruppe, eine C₃₋₇-Cycloalkenylgruppe, eine mit einer C₃₋₇-Cycloalkenylgruppe substituierte C₁₋₆-Alkylgruppe, eine C₂₋₈-Alkenylgruppe, eine C₂₋₈-Alkinylgruppe, eine mit einer C₁₋₆-Alkoxygruppe substituierte C₁₋₆-Alkylgruppe, eine mit einer C₂₋₆-Alkenyloxygruppe substituierte C₁₋₆-Alkylgruppe, eine mit einer C₂₋₆-Alkinyloxygruppe substituierte C₁₋₆-Alkylgruppe, eine mit einer Mono-, Di- oder Polyhalogen-C₁₋₆-alkoxygruppe substituierte C₁₋₆-Alkylgruppe, eine mit einer Mono-, Di- oder Polyhalogen-C₂₋₆-alkenyloxygruppe substituierte C₁₋₆-Alkylgruppe, eine mit einer Mono-, Di- und Polyhalogen-C₂₋₆-Alkinyloxygruppe substituierte C₁₋₆-Alkylgruppe, eine mit einer C₁₋₆-Alkylthiogruppe substituierte C₁₋₆-Alkylgruppe, eine mit einer C₁₋₆-Alkylsulfinylgruppe substituierte C₁₋₆-Alkylgruppe, eine mit einer C₁₋₆-Alkylsulfonylgruppe substituierte C₁₋₆-Alkylgruppe, eine Mono-, Di- oder Polyhalogen-C₁₋₈-Alkylgruppe, eine Mono-, Di- oder Polyhalogen-C₂₋₈-Alkenylgruppe, eine Mono-, Di- oder Polyhalogen-C₂₋₈-Alkinylgruppe, eine mit einer Cyanogruppe substituierte C₁₋₆-Alkylgruppe, eine mit einer Cyanogruppe substituierte C₂₋₆-Alkenylgruppe, eine mit einer Cyanogruppe substituierte C₂₋₆-Alkinylgruppe, eine mit einer Nitrogruppe substituierte C₁₋₆-Alkylgruppe, eine mit einer Nitrogruppe substituierte C₂₋₆-Alkenylgruppe, eine mit einer Nitrogruppe substituierte C₂₋₆-Alkinylgruppe, eine mit einer C₂₋₇-Alkoxycarbonylgruppe substituierte C₁₋₆-Alkylgruppe, einer mit einer C₂₋₆-Alkoxycarbonylgruppe substituierte C₂₋₆-Alkenylgruppe, eine mit einer C₂₋₇-Alkoxycarbonylgruppe substituierte C₂₋₇-Alkinylgruppe, eine mit einer C₂₋₇-Alkylcarbonylgruppe substituierte C₁₋₆-Alkylgruppe, eine mit einer Mono-, Di- oder Polyhalogen-C₂₋₇-alkylcarbonylgruppe substituierte C₁₋₆-Alkylgruppe, eine mit einer C₃₋₇-Alkenylcarbonylgruppe substituierte C₁₋₆-Alkylgruppe, eine mit einer C₃₋₇-Alkinylcarbonylgruppe substituierte C₁₋₆-Alkylgruppe, eine mit einer C₂₋₅-Alkylcarbonylgruppe, die mit einer C₁₋₄-Alkoxygruppe substituiert ist, substituierte C₁₋₆-Alkylgruppe, eine mit einer C₂₋₅-Alkylcarbonylgruppe, die mit einer C₁₋₄-Alkylthiogruppe substituiert ist, substituierte C₁₋₆-Alkylgruppe, eine mit einer C₂₋₅-Alkylcarbonylgruppe, die mit einer C₁₋₄-Alkylsulfinylgruppe substituiert ist, substituierte C₁₋₆-Alkylgruppe, eine mit einer C₂₋₅-Alkylcarbonylgruppe, die mit einer C₁₋₄-Alkylsulfonylgruppe substituiert ist, substituierte C₁₋₆-Alkylgruppe, eine mit einer C₂₋₇-Alkylcarbonylgruppe substituierte C₂₋₆-Alkenylgruppe, eine mit einer C₂₋₇-Alkylcarbonylgruppe substituierte C₂₋₆-Alkinylgruppe, eine mit einer C₁₋₆-Alkylsulfamoylgruppe substituierte C₁₋₆-Alkylgruppe, eine mit einer C₁₋₆-Alkoxysulfamoylgruppe substituierte C₁₋₆-Alkylgruppe, eine mit einer Di(C₁₋₃-alkyl)sulfamoylgruppe substituierte C₁₋₆-Alkylgruppe, eine mit einer N-(C₁₋₃-alkyl)-N-(C₁₋₃-alkoxy)sulfamoylgruppe substituierte C₁₋₆-Alkylgruppe, eine mit einer C₂₋₇-Alkylcarbamoylgruppe substituierte C₁₋₆-Alkylgruppe, eine mit einer Di(C₁₋₃-alkyl)carbamoylgruppe substituierte C₁₋₆-Alkylgruppe, eine mit einer C₂₋₇-Alkoxycarbamoylgruppe substituierte C₁₋₆-Alkylgruppe, eine mit einer N-(C₁₋₃-alkyl)-N-(C₁₋₃-alkoxy)carbamoylgruppe substiuierte C₁₋₆-Alkylgruppe, eine mit einer C₁₋₆-Alkylaminogruppe substituierte C₁₋₆-Alkylgruppe, eine mit einer C₁₋₆-Alkoxyaminogruppe substituierte C₁₋₆-Alkylgruppe, eine mit einer Di(C₁₋₃-alkyl)aminogruppe substituierte C₁₋₆-Alkylgruppe, eine mit einer N-(C₁₋₃-alkyl)-N-(C₁₋₃₋alkoxy)aminogruppe substituierte C₁₋₆-Alkylgruppe, eine mit einer N-(C₂₋₇-alkylcarbonyl)-N-(C₁₋₆-alkyl)aminogruppe substituierte C₁₋₆-Alkylgruppe, eine mit einer N-(C₂₋₇-Alkylcarbonyl)-N-(C₁₋₆-alkoxy)aminogruppe substituierte C₁₋₆-Alkylgruppe, eine mit einer N-(C₁₋₆-Alkylsulfonyl)-N-(C₁₋₆-alkyl)aminogruppe substituierte C₁₋₆-Alkylgruppe, eine mit einer N-(C₁₋₆-Alkylsulfonyl)-N-(C₁₋₆-alkoxy)aminogruppe substituierte C₁₋₆-Alkylgruppe, eine Phenylgruppe (mit der Maßgabe, daß eine solche Phenylgruppe substituiert sein kann mit einem oder mehreren Substituenten, die ausgewählt sind aus der Gruppe, bestehend aus einem Halogenatom, einer Trifluormethylgruppe, einer Nitrogruppe, einer C₁₋₆-Alkylgruppe, einer C₁₋₆-Alkoxygruppe und einer C₂₋₇-Alkoxycarbonylgruppe), eine mit einer Phenylgruppe substituierte C₁₋₆-Alkylgruppe (mit der Maßgabe, daß eine solche Phenylgruppe substituiert sein kann mit einem oder mehreren Substituenten, die ausgewählt sind aus der Gruppe, bestehend aus einem Halogenatom, einer Trifluormethylgruppe, einer Nitrogruppe, einer C₁₋₆-Alkylgruppe, einer C₁₋₆-Alkoxygruppe und einer C₂₋₇-Alkoxycarbonylgruppe), eine mit einer Phenylgruppe substituierte C₂₋₇-Alkenylgruppe (mit der Maßgabe, daß eine solche Phenylgruppe substituierte sein kann mit einem oder mehreren Substituenten, die ausgewählt sind aus der Gruppe, bestehend aus einem Halogenatom, eine Trifluormethylgruppe, einer Nitrogruppe, einer C₁₋₆-Alkylgruppe, einer C₁₋₆-Alkoxygruppe und einer C₂₋₇-Alkoxycarbonylgruppe), eine mit einer Phenylgruppe substituierte C₂₋₆-Alkinylgruppe (mit der Maßgabe, daß eine solche Phenylgruppe substituiert werden kann mit einem oder mehreren Substituenten, die ausgewählt sind aus der Gruppe, bestehend aus einem Halogenatom, einer Trifluormethylgruppe, einer Nitrogruppe, einer C₁₋₆-Alkylgruppe, einer C₁₋₆-Alkoxygruppe und einer C₂₋₇-Alkoxycarbonylgruppe), eine mit einer Phenoxygruppe substituierte C₁₋₆-Alkylgruppe (mit der Maßgabe, daß eine solche Phenoxygruppe substituiert sein kann mit einer oder mehreren Substituenten, die ausgewählt sind aus der Gruppe, bestehend aus einem Halogenatom, einer Trifluormethylgruppe, einer Nitrogruppe, einer C₁₋₆-Alkylgruppe, einer C₁₋₆-Alkoxygruppe und einer C₂₋₇-Alkoxycarbonylgruppe), einer mit einer Phenylthiogruppe substituierte C₁₋₆-Alkylgruppe (mit der Maßgabe, daß eine solche Phenylthiogruppe substituiert sein kann mit einem oder mehreren Substituenten, die ausgewählt sind aus der Gruppe, bestehend aus einem Halogenatom, einer Trifluormethylgruppe, einer Nitrogruppe, einer C₁₋₆-Alkylgruppe, einer C₁₋₆-Alkoxygruppe und einer C₂₋₇-Alkoxycarbonylgruppe), eine mit einer Phenylsulfinylgruppe substituierte C₁₋₆-Alkylgruppe (mit der Maßgabe, daß eine solche Phenylsulfinylgruppe substituiert sein kann mit einem oder mehreren Substituenten, die ausgewählt sind aus der Gruppe, bestehend aus einem Halogenatom, einer Trifluormethylgruppe, einer Nitrogruppe, einer C₁₋₆-Alkylgruppe, einer C₁₋₆-Alkoxygruppe und einer C₂₋₇-Alkoxycarbonylgruppe), eine mit einer Phenylsulfonylgruppe substituierte C₁₋₆-Alkylgruppe (mit der Maßgabe, daß eine solche Phenylsulfonylgruppe substituiert sein kann mit einem oder mehreren Substituenten, die ausgewählt sind aus der Gruppe, bestehend aus einem Halogenatom, einer Trifluormethylgruppe, einer Nitrogruppe, einer C₁₋₆-Alkylgruppe, einer C₁₋₆-Alkoxygruppe und einer C₂₋₇-Alkoxycarbonylgruppe), eine mit einer Benzyloxygruppe substituierte C₁₋₆-Alkylgruppe (mit der Maßgabe, daß die Phenylgruppe einer solchen Benzyloxygruppe substituiert sein kann mit einem oder mehreren Substituenten, die ausgewählt sind aus der Gruppe, bestehend aus einem Halogenatom, einer Trifluormethylgruppe, einer Nitrogruppe, einer C₁₋₆-Alkylgruppe, einer C₁₋₆-Alkoxygruppe und einer C₂₋₇-Alkoxycarbonylgruppe), eine mit einer Benzylthiogruppe substituierte C₁₋₆-Alkylgruppe (mit der Maßgabe, daß die Phenylgruppe einer solchen Benzylthiogruppe substituiert sein kann mit einem oder mehreren Substituenten, die ausgewählt sind aus der Gruppe, bestehend aus einem Halogenatom, einer Trifluormethylgruppe, einer Nitrogruppe, einer C₁₋₆-Alkylgruppe, einer C₁₋₆-Alkoxygruppe und einer C₂₋₇-Alkoxycarbonylgruppe), eine mit einer Benzylsulfinylgruppe substituierte C₁₋₆-Alkylgruppe (mit der Maßgabe, daß die Phenylgruppe einer solchen Benzylsulfinylgruppe substituiert sein kann mit einem oder mehreren Substituenten, die ausgewählt sind aus der Gruppe, bestehend aus einem Halogenatom, einer Trifluormethylgruppe, einer Nitrogruppe, einer C₁₋₆-Alkylgruppe, einer C₁₋₆-Alkoxygruppe und einer C₂₋₇-Alkoxycarbonylgruppe), eine mit einer Benzylsulfonylgruppe substituierte C₁₋₆-Alkylgruppe (mit der Maßgabe, daß die Phenylgruppe einer solchen Benzylsulfonylgruppe substituiert sein kann mit einem oder mehreren Substituenten, die ausgewählt sind aus der Gruppe, bestehend aus einem Halogenatom, einer Trifluormethylgruppe, einer Nitrogruppe, einer C₁₋₆-Alkylgruppe, einer C₁₋₆-Alkoxygruppe und einer C₂₋₇-Alkoxycarbonylgruppe), eine mit einer Phenylcarbonylgruppe substituierte C₁₋₆-Alkylgruppe (mit der Maßgabe, daß eine solche Phenylcarbonylgruppe substituiert sein kann mit einem oder mehreren Substituenten, die ausgewählt sind aus der Gruppe, bestehend aus einem Halogenatom, einer Trifluormethylgruppe, einer Nitrogruppe, einer C₁₋₆-Alkylgruppe, einer C₁₋₆-Alkoxygruppe und einer C₂₋₇-Alkoxycarbonylgruppe), eine mit einer Benzylcarbonylgruppe substituierte C₁₋₆-Alkylgruppe (mit der Maßgabe, daß die Phenylgruppe einer solchen Benzylcarbonylgruppe substituiert sein kann mit einem oder mehreren Substituenten, die ausgewählt sind aus der Gruppe, bestehend aus einem Halogenatom, einer Trifluormethylgruppe, einer Nitrogruppe, einer C₁₋₆-Alkylgruppe, einer C₁₋₆-Alkoxygruppe und einer C₂₋₇-Alkoxycarbonylgruppe), eine mit einer Aminogruppe, die mit einer C₁₋₄-Alkylsulfonylgruppe substituiert ist, substituierte C₁₋₆-Alkylgruppe, oder eine mit einer Aminogruppe, die mit einer C₂₋₄-Alkylcarbonylgruppe substituiert ist, substituierte C₁₋₆-Alkylgruppe steht,
R^{a2} und R^{a3}, die unabhängig voneinander sind, jeweils für ein Wasserstoffatom, eine C₁₋₆-Alkylgruppe, eine Mono-, Di- oder Polyhalogen-C₁₋₆-alkylgruppe, eine C₁₋₆-Alkoxygruppe, eine Mono-, Di- oder Polyhalogen-C₁₋₆-alkoxygruppe, eine C₁₋₆-Alkylthiogruppe, eine C₁₋₆-Alkylsulfinylgruppe, eine C₁₋₆-Alkylsulfonylgruppe, eine C₂₋₇-Alkoxycarbonylgruppe, eine C₂₋₇-Alkylcarbonylgruppe, ein Halogenatom, eine Nitrogruppe, eine Cyanogruppe oder eine Phenylgruppe (mit der Maßgabe, daß eine solche Phenylgruppe substituiert sein kann mit einem oder mehreren Substituenten, die ausgewählt sind aus der Gruppe, bestehend aus einem Halogenatom, einer Trifluormethylgruppe, einer Nitrogruppe, einer C₁₋₆-Alkylgruppe, einer C₁₋₆-Alkoxygruppe und einer C₂₋₇-Alkoxycarbonylgruppe) steht,
R^{a4} und R^{a5}, die unabhängig voneinander sind, jeweils für ein Wasserstoffatom, eine C₁₋₈-Alkylgruppe, eine C₂₋₈-Alkenylgruppe, eine C₂₋₈-Alkinylgruppe oder eine Phenylgruppe (mit der Maßgabe, daß eine solche Phenylgruppe substituiert sein kann mit einem oder mehreren Substituenten, die ausgewählt sind aus der Gruppe, bestehend aus einem Halogenatom, einer Trifluormethylgruppe, einer Nitrogruppe, einer C₁₋₆-Alkylgruppe, einer C₁₋₆-Alkoxygruppe und einer C₂₋₇-Alkoxycarbonylgruppe) steht,
R^{a6} für eine C₁₋₈-Alkylgruppe, eine C₂₋₈-Alkenylgruppe, eine C₂₋₈-Alkinylgruppe oder eine Phenylgruppe (mit der Maßgabe, daß eine solche Phenylgruppe substituiert sein kann mit einem oder mehreren Substituenten, die ausgewählt sind aus der Gruppe, bestehend aus einem Halogenatom, einer Trifluormethylgruppe, einer Nitrogruppe, einer C₁₋₆-Alkylgruppe, einer C₁₋₆-Alkoxygruppe und einer C₂₋₇-Alkoxycarbonylgruppe) steht,
R^{b1} für eine C₁₋₈-Alkylgruppe, eine C₃₋₇-Cycloalkylgruppe, eine mit einer C₃₋₇-Cycloalkylgruppe substituierte C₁₋₆-Alkylgruppe, eine C₃₋₇-Cycloalkenylgruppe, eine mit einer C₃₋₇-Alkenylgruppe substituierte C₁₋₆-Alkylgruppe, eine C₂₋₈-Alkenylgruppe, eine C₂₋₈-Alkinylgruppe, eine mit einer C₁₋₆-Alkoxygruppe substituierte C₁₋₆-Alkylgruppe, eine mit einer C₂₋₆-Alkenyloxygruppe substituierte C₁₋₆-Alkylgruppe, eine mit einer C₂₋₆-Alkinyloxygruppe substituierte C₁₋₆-Alkylgruppe, eine mit einer Mono-, Di- oder Polyhalogen-C₁₋₆-alkoxygruppe substituierte C₁₋₆-Alkylgruppe, eine mit einer Mono-, Di- oder Polyhalogen-C₂₋₆-Alkinyloxygruppe substituierte C₁₋₆-Alkylgruppe, eine mit einer Mono-, Di- oder Polyhalogen-C₂₋₆-alkinyloxygruppe substituierte C₁₋₆-Alkylgruppe, eine mit einer C₁₋₆-Alkylthiogruppe substituierte C₁₋₆-Alkylgruppe, eine mit einer C₁₋₆-Alkylsulfinylgruppe substituierte C₁₋₆-Alkylgruppe, eine mit einer C₁₋₆-Alkylsulfonylgruppe substituierte C₁₋₆-Alkylgruppe, eine Mono-, Di- oder Polyhalogen-C₁₋₈-Alkylgruppe, eine Mono-, Di- oder Polyhalogen-C₂₋₈-Alkenylgruppe, eine Mono-, Di- oder Polyhalogen-C₂₋₈-alkinylgruppe, eine mit einer Cyanogruppe substituierte C₁₋₆-Alkylgruppe, eine mit einer Cyanogruppe substituierte C₂₋₆-Alkinylgruppe, eine mit einer Cyanogruppe substituierte C₂₋₆-Alkinylgruppe, eine mit einer Nitrogruppe substituierte C₁₋₆-Alkylgruppe, eine mit einer Nitrogruppe substituierte C₂₋₆-Alkenylgruppe, eine mit einer Nitrogruppe substituierte C₂₋₆-Alkinylgruppe, eine mit einer C₂₋₇-Alkoxycarbonylgruppe substituierte C₁₋₆-Alkylgruppe, eine mit einer C₂₋₇-Alkoxycarbonylgruppe substituierte C₂₋₆-Alkenylgruppe, eine mit einer C₂₋₇-Alkoxycarbonylgruppe substituierte C₂₋₆-Alkinylgruppe, eine mit einer C₂₋₇-Alkylcarbonylgruppe substituierte C₁₋₆-Alkylgruppe, eine mit einer Mono-, Di- oder Polyhalogen-C₂₋₇-alkylcarbonylgruppe substituierte C₁₋₆-Alkylgruppe, eine mit einer C₃₋₇-Alkenylcarbonylgruppe substituierte C₁₋₆-Alkylgruppe, eine mit einer C₃₋₇-Alkinylcarbonylgruppe substituierte C₁₋₆-Alkylgruppe, eine mit einer C₂₋₅-Alkylcarbonylgruppe, die mit einer C₁₋₄-Alkoxygruppe substituiert ist, substituierte C₁₋₆-Alkylgruppe, eine mit einer C₂₋₅-Alkylcarbonylgruppe, die mit einer C₁₋₄-Alkylthiogruppe substituiert ist, substituierte C₁₋₆-Alkylgruppe, eine mit einer C₂₋₅-Alkylcarbonylgruppe, die mit einer C₁₋₄-Alkylsulfinylgruppe substituiert ist, substituierte C₁₋₆-Alkylgruppe, eine mit einer C₂₋₅-Alkylcarbonylgruppe, die mit einer C₁₋₄-Alkylsulfonylgruppe substituiert ist, substituierte C₁₋₆-Alkylgruppe, eine mit einer C₂₋₇-Alkylcarbonylgruppe substituierte C₂₋₆-Alkenylgruppe, eine mit einer C₂₋₇-Alkylcarbonylgruppe substituierte C₂₋₆-Alkinylgruppe, eine mit einer C₁₋₆-Alkylsulfamoylgruppe substituierte C₁₋₆-Alkylkgruppe, eine mit einer C₁₋₆-Alkoxysulfamoylgruppe substituierte C₁₋₆-Alkylgruppe, eine mit einer Di(C₁₋₃- alkyl)sulfamoylgruppe substituierte C₁₋₆-Alkylgruppe, eine mit einer N-(C₁₋₃-Alkyl)-N-(C₁₋₃-alkoxy)sulfamoylgruppe substituierte C₁₋₆-Alkylgruppe, eine mit einer C₂₋₇-Alkylcarbamoylgruppe substituierte C₁₋₆-Alkylgruppe, eine mit einer Di(C₁₋₃-alkyl)carbamoylgruppe substituierte C₁₋₆-Alkylgruppe, eine mit einer C₂₋₇-Alkoxycarbamoylgruppe substituierte C₁₋₆-Alkylgruppe, eine mit einer N-(C₁₋₃-Alkyl)-N-(C₁₋₃-alkoxy)carbamoylgruppe substituierte C₁₋₆-Alkylgruppe, eine mit einer C₁₋₆-Alkylaminogruppe substituierte C₁₋₆-Alkylgruppe, eine mit einer C₁₋₆-Alkoxyaminogruppe substituierte C₁₋₆-Alkylgruppe, eine mit einer Di(C₁₋₃-alkyl)aminogruppe substituierte C₁₋₆-Alkylgruppe, eine mit einer N-(C₁₋₃-Alkyl)-N-(C₁₋₃-alkoxy)aminogruppe substituierte C₁₋₆-Alkylgruppe, eine mit einer N-(C₂₋₇-Alkylcarbonyl)-N-(C₁₋₆-alkyl)aminogruppe substituierte C₁₋₆-Alkylgruppe, eine mit einer N-(C₂₋₇-Alkylcarbonyl)-N-(C₁₋₆-alkoxy)aminogruppe substituierte C₁₋₆-Alkylgruppe, eine mit einer N-(C₁₋₆-Alkylsulfonyl)-N-(C₁₋₆-alkyl)aminogruppe substituierte C₁₋₆-Alkylgruppe, eine mit einer N-(C₁₋₆-Alkylsulfonyl)-N-(C₁₋₆-alkoxy)aminogruppe substituierte C₁₋₆-Alkylgruppe, eine Phenylgruppe (mit der Maßgabe, daß eine solche Phenylgruppe substituiert sein kann mit einem oder mehreren Substituenten, die ausgewählt sind aus der Gruppe, bestehend aus einem Halogenatom, einer Trifluormethylgruppe, einer Nitrogruppe, einer C₁₋₆-Alkylgruppe, einer C₁₋₆-Alkoxygruppe und einer C₂₋₇-Alkoxycarbonylgruppe), eine mit einer Phenylgruppe substituierte C₁₋₆-Alkylgruppe (mit der Maßgabe, daß eine solche Phenylgruppe substituiert sein kann mit einem oder mehreren Substituenten, die ausgewählt sind aus der Gruppe, bestehend aus einem Halogenatom, einer Trifluormethylgruppe, einer Nitrogruppe, einer C₁₋₆-Alkylgruppe, einer C₁₋₆-Alkoxygruppe und einer C₂₋₇-Alkoxycarbonylgruppe), eine mit einer Phenylgruppe substituierte C₂₋₇-Alkenylgruppe (mit der Maßgabe, daß eine solche Phenylgruppe substituiert sein kann mit einem oder mehreren Substituenten, die ausgewählt sind aus der Gruppe, bestehend aus einem Halogenatom, einer Trifluormethylgruppe, einer Nitrogruppe, einer C₁₋₆-Alkylgruppe, einer C₁₋₆-Alkoxygruppe und einer C₂₋₇-Alkoxycarbonylgruppe), eine mit einer Phenylgruppe substituierte C₂₋₆-Alkinylgruppe (mit der Maßgabe, daß eine solche Phenylgruppe substituiert sein kann mit einem oder mehreren Substituenten, die ausgewählt sind aus der Gruppe, bestehend aus einem Halogenatom, einer Trifluormethylgruppe, einer Nitrogruppe, einer C₁₋₆-Alkylgruppe, einer C₁₋₆-Alkoxygruppe und einer C₂₋₇-Alkoxycarbonylgruppe), eine mit einer Phenoxygruppe substituierte C₁₋₆-Alkylgruppe (mit der Maßgabe, daß eine solche Phenoxygruppe substituiert sein kann mit einem oder mehreren Substituenten, die ausgewählt sind aus der Gruppe, bestehend aus einem Halogenatom, einer Trifluormethylgruppe, einer Nitrogruppe, einer C₁₋₆-Alkylgruppe, einer C₁₋₆-Alkoxygruppe und einer C₂₋₇-Alkoxycarbonylgruppe), eine mit einer Phenylthiogruppe substituierte C₁₋₆-Alkylgruppe (mit der Maßgabe, daß eine solche Phenylthiogruppe substituiert sein kann mit einem oder mehreren Substituenten, die ausgewählt sind aus der Gruppe, bestehend aus einem Halogenatom, einer Trifluormethylgruppe, einer Nitrogruppe, einer C₁₋₆-Alkylgruppe, einer C₁₋₆-Alkoxygruppe und einer C₂₋₇-Alkoxycarbonylgruppe), eine mit einer Phenylsulfinylgruppe substituierte C₁₋₆-Alkylgruppe (mit der Maßgabe, daß eine solche Phenylsulfinylgruppe substituiert sein kann mit einem oder mehreren Substituenten, die ausgewählt sind aus der Gruppe, bestehend aus einem Halogenatom, einer Trifluormethylgruppe, einer Nitrogruppe, einer C₁₋₆-Alkylgruppe, einer C₁₋₆-Alkoxygruppe und einer C₂₋₇-Alkoxycarbonylgruppe), eine mit einer Phenylsulfonylgruppe substituierte C₁₋₆-Alkylgruppe (mit der Maßgabe, daß eine solche Phenylsulfonylgruppe substituiert sein kann mit einem oder mehreren Substituenten, die ausgewählt sind aus der Gruppe, bestehend aus einem Halogenatom, einer Trifluormethylgruppe, einer Nitrogruppe, einer C₁₋₆-Alkylgruppe, einer C₁₋₆-Alkoxygruppe und einer C₂₋₇-Alkoxycarbonylgruppe), eine mit einer Benzyloxygruppe substituierte C₁₋₆-Alkylgruppe (mit der Maßgabe, daß die Phenylgruppe einer solchen Benzyloxygruppe substituiert sein kann mit einem oder mehreren Substituenten, die ausgewählt sind aus der Gruppe, bestehend aus einem Halogenatom, einer Trifluormethylgruppe, einer Nitrogruppe, einer C₁₋₆-Alkylgruppe, einer C₁₋₆-Alkoxygruppe und einer C₂₋₇-Alkoxycarbonylgruppe), eine mit einer Benzylthiogruppe substituierte C₁₋₆-Alkylgruppe (mit der Maßgabe, daß die Phenylgruppe einer solchen Benzylthiogruppe substituiert sein kann mit einem oder mehreren Substituenten, die ausgewählt sind aus der Gruppe, bestehend aus einem Halogenatom, einer Trifluormethylgruppe, einer Nitrogruppe, einer C₁₋₆-Alkylgruppe, einer C₁₋₆-Alkoxygruppe und einer C₂₋₇-Alkoxycarbonylgruppe), eine mit einer Benzylsulfinylgruppe substituierte C₁₋₆-Alkylgruppe (mit der Maßgabe, daß die Phenylgruppe einer solchen Benzylsulfinylgruppe substituiert sein kann mit einem oder mehreren Substituenten, die ausgewählt sind aus der Gruppe, bestehend aus einem Halogenatom, einer Trifluormethylgruppe, einer Nitrogruppe, einer C₁₋₆-Alkylgruppe, einer C₁₋₆-Alkoxygruppe und einer C₂₋₇-Alkoxycarbonylgruppe), eine mit einer Benzylsulfonylgruppe substituierte C₁₋₆-Alkylgruppe (mit der Maßgabe, daß die Phenylgruppe einer solchen Benzylsulfonylgruppe substituiert sein kann mit einem oder mehreren Substituenten, die ausgewählt sind aus der Gruppe, bestehend aus einem Halogenatom, einer Trifluormethylgruppe, einer Nitrogruppe, einer C₁₋₆-Alkylgruppe, einer C₁₋₆-Alkoxygruppe und einer C₂₋₇-Alkoxycarbonylgruppe), eine mit einer Phenylcarbonylgruppe substituierte C₁₋₆-Alkylgruppe (mit der Maßgabe, daß eine solche Phenylcarbonylgruppe substituiert sein kann mit einem oder mehreren Substituenten, die ausgewählt sind aus der Gruppe, bestehend aus einem Halogenatom, einer Trifluormethylgruppe, einer Nitrogruppe, einer C₁₋₆-Alkylgruppe, einer C₁₋₆-Alkoxygruppe und einer C₂₋₇-Alkoxycarbonylgruppe), eine mit einer Benzylcarbonylgruppe substituierte C₁₋₆-Alkylgruppe (mit der Maßgabe, daß die Phenylgruppe einer solchen Benzylcarbonylgruppe substituiert sein kann mit einem oder mehreren Substituenten, die ausgewählt sind aus der Gruppe, bestehend aus einem Halogenatom, einer Trifluormethylgruppe, einer Nitrogruppe, einer C₁₋₆-Alkylgruppe, einer C₁₋₆-Alkoxygruppe und einer C₂₋₇-Alkoxycarbonylgruppe), eine mit einer Aminogruppe, die mit einer C₁₋₄-Alkylsulfonylgruppe substituiert ist, substituierte C₁₋₆-Alkylgruppe, oder eine mit einer Aminogruppe, die mit einer C₂₋₄-Carbonylgruppe substituiert ist, substituierte C₁₋₆-Alkylgruppe steht,
R^{b2} für ein Wasserstoffatom, eine C₁₋₆-Alkylgruppe, eine Mono-, Di- oder Polyhalogen-C₁₋₆-alkylgruppe, eine C₁₋₆-Alkoxygruppe, eine Mono-, Di- oder Polyhalogen-C₁₋₆-alkoxygruppe, eine C₁₋₆-Alkylthiogruppe, eine C₁₋₆-Alkylsulfinylgruppe, eine C₁₋₆-Alkylsulfonylgruppe, eine C₂₋₇-Alkoxycarbonylgruppe, eine C₂₋₇-Alkylcarbonylgruppe, ein Halogenatom, eine Nitrogruppe, eine Cyanogruppe oder eine Phenylgruppe (mit der Maßgabe, daß eine solche Phenylgruppe substituiert sein kann mit einem oder mehreren Substituenten, die ausgewählt sind aus der Gruppe, bestehend aus einem Halogenatom, einer Trifluormethylgruppe, einer Nitrogruppe, einer C₁₋₆-Alkylgruppe, einer C₁₋₆-Alkoxygruppe und einer C₂₋₇-Alkoxycarbonylgruppe) steht,
R^{b3} für eine C₁₋₆-Alkylgruppe, eine C₂₋₆-Alkenylgruppe oder eine C₂₋₆-Alkinylgruppe steht,
R^{b4} für ein Wasserstoffatom oder eine C₁₋₆-Alkylgruppe steht,
R^{b5} für ein Wasserstoffatom oder eine C₁₋₆-Alkylgruppe steht,
R^{c1} für eine C₁₋₈-Alkylgruppe, eine C₃₋₇-Cycloalkylgruppe, eine mit einer C₃₋₇-Cycloalkylgruppe substituierte C₁₋₆-Alkylgruppe, eine C₃₋₇-Cycloalkenylgruppe, eine mit einer C₃₋₇-Cycloalkenylgruppe substituierte C₁₋₆-Alkylgruppe, eine C₂₋₈-Alkenylgruppe, eine C₂₋₈-Alkinylgruppe, eine mit einer C₁₋₆-Alkoxygruppe substituierte C₁₋₆-Alkylgruppe, eine mit einer C₂₋₆-Alkenyloxygruppe substituierte C₁₋₆-Gruppe, eine mit einer C₂₋₆-Alkinyloxygruppe substituierte C₁₋₆-Alkylgruppe, einer mit einer Mono-, Di- oder Polyhalogen-C₁₋₆-alkoxygruppe substituierte C₁₋₆-Alkylgruppe, eine mit einer Mono-, Di- oder Polyhalogen-C₂₋₆-alkenyloxygruppe substituierte C₁₋₆-Alkylgruppe, eine mit einer Mono-, Di- oder Polyhalogen-C₂₋₆-alkinyloxygruppe substituierte C₁₋₆-Alkylgruppe, eine mit einer C₁₋₆-Alkylthiogruppe substituierte C₁₋₆-Alkylgruppe, eine mit einer C₁₋₆-Alkylsulfinylgruppe substituierte C₁₋₆-Alkylgruppe, eine mit einer C₁₋₆-Alkylsulfonylgruppe substituierte C₁₋₆-Alkylgruppe, eine Mono-, Di- oder Polyhalogen-C₁₋₈-alkylgruppe, eine Mono-, Di- oder Polyhalogen-C₂₋₈alkenylgruppe, eine Mono-, Di- oder Polyhalogen-C₂₋₈-alkinylgruppe, eine mit einer Cyanogruppe substituierte C₁₋₆-Alkylgruppe, eine mit einer Cyanogruppe substituierte C₂₋₆-Alkenylgruppe, eine mit einer Cyanogruppe substituierte C₂₋₆-Alkinylgruppe, eine mit einer Nitrogruppe substituierte C₁₋₆-Alkylgruppe, eine mit einer Nitrogruppe substituierte C₂₋₆-Alkenylgruppe, eine mit einer Nitrogruppe substituierte C₂₋₆-Alkinylgruppe, eine mit einer C₂₋₇-Alkoxycarbonylgruppe substituierte C₁₋₆-Alkylgruppe, eine mit einer C₂₋₇-Alkoxycarbonylgruppe substituierte C₂₋₆-Alkenylgruppe, eine mit einer C₂₋₇-Alkoxycarbonylgruppe substituierte C₂₋₆-Alkinylgruppe, eine mit einer C₂₋₇-Alkylcarbonylgruppe substituierte C₁₋₆-Alkylgruppe, eine mit einer Mono-, Di- oder Polyhalogen-C₋₂₋₇-alkylcarbonylgruppe substituierte C₁₋₆-Alkylgruppe, eine mit einer C₃₋₇-Alkenylcarbonylgruppe substituierte C₁₋₆-Alkylgruppe, eine mit einer C₃₋₇-Alkinylcarbonylgruppe substituierte C₁₋₆-Alkylgruppe, eine mit einer C₂₋₅-Alkylcarbonylgruppe, die mit einer C₁₋₈-Alkoxygruppe substituiert ist, substituierte C₁₋₆-Alkylgruppe, eine mit einer C₂₋₅-Alkylcarbonylgruppe, die mit einer C₁₋₄-Alkylthiogruppe substituiert ist, substituierte C₁₋₆-Alkylgruppe, eine mit einer C₂₋₅-Alkylcarbonylgruppe, die mit einer C₁₋₄-Alkylsulfinylgruppe substituiert ist, substituierte C₁₋₆-Alkylgruppe, eine mit einer C₂₋₅-Alkylcarbonylgruppe, die mit einer C₁₋₄-Alkylsulfonylgruppe substituiert ist, substituierte C₁₋₆-Alkylgruppe, eine mit einer C₂₋₇-Alkylcarbonylgruppe substituierte C₂₋₆-Alkenylgruppe, eine mit einer C₂₋₇-Alkylcarbonylgruppe substituierte C₂₋₆-Alkinylgruppe, eine mit einer C₁₋₆-Alkylsulfamoylgruppe substituierte C₁₋₆-Alkylgruppe, eine mit einer C₁₋₆-Alkoxysulfamoylgruppe substituierte C₁₋₆-Alkylgruppe, eine mit einer Di(C₁₋₃-alkyl)sulfamoylgruppe substituierte C₁₋₆-Alkylgruppe, eine mit einer N-(C₁₋₃-alkyl)-N-(C₁₋₃-alkoxy)sulfamoylgruppe substituierte C₁₋₆-Alkylgruppe, eine mit einer C₂₋₇-Alkylcarbamoylgruppe substituierte C₁₋₆-Alkylgruppe, eine mit einer Di(C₁₋₃-alkyl)carbamoylgruppe substituierte C₁₋₆-Alkylgruppe, eine mit einer C₂₋₇-Alkoxycarbamoylgruppe substituierte C₁₋₆-Alkylgruppe, eine mit einer N-(C₁-₃-Alkyl)-N-(C₁₋₃-alkoxy)carbamoylgruppe substituierte C₁₋₆-Alkylgruppe, eine mit einer C₁₋₆₋Alkylaminogruppe substituierte C₁₋₆-Alkylgruppe, eine mit einer C₁₋₆-Alkoxyaminogruppe substituierte C₁₋₆-Alkylgruppe, eine mit einer Di(C₁₋₃-alkyl)aminogruppe substituierte C₁₋₆-Alkylgruppe, eine mit einer N-(C₁₋₃-alkyl)-N-(C₁₋₃-alkoxy)aminogruppe substituierte C₁₋₆-Alkylgruppe, eine mit einer N-(C₂₋₇-Alkylcarbonyl)-N-(C₁₋₆-alkyl)aminogruppe substituierte C₁₋₆-Alkylgruppe, eine mit einer N-(C₂₋₇-Alkylcarbonyl)-N-(C₁₋₆-alkoxy)aminogruppe substituierte C₁₋₆-Alkylgruppe, eine mit einer N-(C₁₋₆-Alkylsulfonyl)-N-(C₁₋₆-alkyl)aminogruppe substituierte C₁₋₆-Alkylgruppe, eine mit einer N-(C₁₋₆-Alkylsulfonyl)-N-(C₁₋₆-alkoxy)aminogruppe substituierte C₁₋₆-Alkylgruppe, eine Phenylgruppe (mit der Maßgabe, daß eine solche Phenylgruppe substituiert sein kann mit einem oder mehreren Substituenten, die ausgewählt sind aus der Gruppe, bestehend aus einem Halogenatom, einer Trifluormethylgruppe, einer Nitrogruppe, einer C₁₋₆-Alkylgruppe, einer C₁₋₆-Alkoxygruppe und einer C₂₋₇-Alkoxycarbonylgruppe), eine mit einer Phenylgruppe substituierte C₁₋₆-Alkylgruppe (mit der Maßgabe, daß eine solche Phenylgruppe substituiert sein kann mit einem oder mehreren Substituenten, die ausgewählt sind aus der Gruppe, bestehend aus einem Halogenatom, einer Trifluormethylgruppe, einer Nitrogruppe, einer C₁₋₆-Alkylgruppe, einer C₁₋₆-Alkoxygruppe und einer C₂₋₇-Alkoxycarbonylgruppe), eine mit einer Phenylgruppe substituierte C₂₋₇-Alkenylgruppe (mit der Maßgabe, daß eine solche Phenylgruppe substituiert sein kann mit einem oder mehreren Substituenten, die ausgewählt sind aus der Gruppe, bestehend aus einem Halogenatom, einer Trifluormethylgruppe, einer Nitrogruppe, einer C₁₋₆-Alkylgruppe, einer C₁₋₆-Alkoxygruppe und einer C₂₋₇-Alkoxycarbonylgruppe), eine mit einer Phenylgruppe substituierte C₂₋₆-Alkinylgruppe (mit der Maßgabe, daß eine solche Phenylgruppe substituiert sein kann mit einem oder mehreren Substituenten, die ausgewählt sind aus der Gruppe, bestehend aus einem Halogenatom, einer Trifluormethylgruppe, einer Nitrogruppe, einer C₁₋₆-Alkylgruppe, einer C₁₋₆-Alkoxygruppe und einer C₂₋₇-Alkoxycarbonylgruppe), eine mit einer Phenoxygruppe substituierte C₁₋₆-Alkylgruppe (mit der Maßgabe, daß eine solche Phenoxygruppe substituiert sein kann mit einem oder mehreren Substituenten, die ausgewählt sind aus der Gruppe, bestehend aus einem Halogenatom, einer Trifluormethylgruppe, einer Nitrogruppe, einer C₁₋₆-Alkylgruppe, einer C₁₋₆-Alkoxygruppe und einer C₂₋₇-Alkoxycarbonylgruppe), eine mit einer Phenylthiogruppe substituierte C₁₋₆-Alkylgruppe (mit der Maßgabe, daß eine solche Phenylthiogruppe substituiert sein kann mit einem oder mehreren Substituenten, die ausgewählt sind aus der Gruppe, bestehend aus einem Halogenatom, einer Trifluormethylgruppe, einer Nitrogruppe, einer C₁₋₆-Alkylgruppe, einer C₁₋₆-Alkoxygruppe und einer C₂₋₇-Alkoxycarbonylgruppe), eine mit einer Phenylsulfinylgruppe substituierte C₁₋₆-Alkylgruppe (mit der Maßgabe, daß eine solche Phenylsulfinylgruppe substituiert sein kann mit einem oder mehreren Substituenten, die ausgewählt sind aus der Gruppe, bestehend aus einem Halogenatom, einer Trifluormethylgruppe, einer Nitrogruppe, einer C₁₋₆-Alkylgruppe, einer C₁₋₆-Alkoxygruppe und einer C₂₋₇-Alkoxycarbonylgruppe), eine mit einer Phenylsulfonylgruppe substituierte C₁₋₆-Alkylgruppe (mit der Maßgabe, daß eine solche Phenylsulfonylgruppe substituiert sein kann mit einem oder mehreren Substituenten, die ausgewählt sind aus der Gruppe, bestehend aus einem Halogenatom, einer Trifluormethylgruppe, einer Nitrogruppe, einer C₁₋₆-Alkylgruppe, einer C₁₋₆-Alkoxygruppe und einer C₂₋₇-Alkoxycarbonylgruppe), eine mit einer Benzyloxygruppe substituierte C₁₋₆-Alkylgruppe (mit der Maßgabe, daß die Phenylgruppe einer solchen Benzyloxygruppe substituiert sein kann mit einem oder mehreren Substituenten, die ausgewählt sind aus der Gruppe, bestehend aus einem Halogenatom, einer Trifluormethylgruppe, einer Nitrogruppe, einer C₁₋₆-Alkylgruppe, einer C₁₋₆-Alkoxygruppe und einer C₂₋₇-Alkoxycarbonylgruppe), eine mit einer Benzylthiogruppe substituierte C₁₋₆-Alkylgruppe (mit der Maßgabe, daß die Phenylgruppe einer solchen Benzylthiogruppe substituiert sein kann mit einem oder mehreren Substituenten, die ausgewählt sind aus der Gruppe, bestehend aus einem Halogenatom, einer Trifluormethylgruppe, einer Nitrogruppe, einer C₁₋₆-Alkylgruppe, einer C₁₋₆-Alkoxygruppe und einer C₂₋₇-Alkoxycarbonylgruppe), eine mit einer Benzylsulfinylgruppe substituierte C₁₋₆-Alkylgruppe (mit der Maßgabe, daß die Phenylgruppe einer solchen Benzylsulfinylgruppe substituiert sein kann mit einem oder mehreren Substituenten, die ausgewählt sind aus der Gruppe, bestehend aus einem Halogenatom, einer Trifluormethylgruppe, einer Nitrogruppe, einer C₁₋₆-Alkylgruppe, einer C₁₋₆-Alkoxygruppe und einer C₂₋₇-Alkoxycarbonylgruppe), eine mit einer Benzylsulfonylgruppe substituierte C₁₋₆-Alkylgruppe (mit der Maßgabe, daß die Phenylgruppe einer solchen Benzylsulfonylgruppe substituiert sein kann mit einem oder mehreren Substituenten, die ausgewählt sind aus der Gruppe, bestehend aus einem Halogenatom, einer Trifluormethylgruppe, einer Nitrogruppe, einer C₁₋₆-Alkylgruppe, einer C₁₋₆-Alkoxygruppe und einer C₂₋₇-Alkoxycarbonylgruppe), eine mit einer Phenylcarbonylgruppe substituierte C₁₋₆-Alkylgruppe (mit der Maßgabe, daß eine solche Phenylcarbonylgruppe substituiert sein kann mit einem oder mehreren Substituenten, die ausgewählt sind aus der Gruppe, bestehend aus einem Halogenatom, einer Trifluormethylgruppe, einer Nitrogruppe, einer C₁₋₆-Alkylgruppe, einer C₁₋₆-Alkoxygruppe und einer C₂₋₇-Alkoxycarbonylgruppe), eine mit einer Benzylcarbonylgruppe substituierte C₁₋₆-Alkylgruppe (mit der Maßgabe, daß die Phenylgruppe einer solchen Benzylcarbonylgruppe substituiert sein kann mit einem oder mehreren Substituenten, die ausgewählt sind aus der Gruppe, bestehend aus einem Halogenatom, einer Trifluormethylgruppe, einer Nitrogruppe, einer C₁₋₆-Alkylgruppe, einer C₁₋₆-Alkoxygruppe und einer C₂₋₇-Alkoxycarbonylgruppe), eine mit einer Aminogruppe, die mit einer C₁₋₄-Alkylsulfonylgruppe substituiert ist, substituierte C₁₋₆-Alkylgruppe oder mit einer Aminogruppe, die mit einer C₂₋₄-Alkylcarbonylgruppe substituiert ist, substituierte C₁₋₆-Alkylgruppe steht
R^{c2}, R^{c3}, R^{c4}, R^{c5}, R^{c6}, R^{c11} und R^{c12}, die unabhängig voneinander sind, jeweils für ein Wasserstoffatom, eine C₁₋₈-Alkylgruppe, eine C₂₋₈-Alkenylgruppe, eine C₂₋₈-Alkinylgruppe oder eine Phenylgruppe (mit der Maßgabe, daß eine solche Phenylgruppe substituiert sein kann mit einem oder mehreren Substituenten, die ausgewählt sind aus der Gruppe, bestehend aus einem Halogenatom, einer Trifluormethylgruppe, einer Nitrogruppe, einer C₁₋₆-Alkylgruppe, einer C₁₋₆-Alkoxygruppe und einer C₂₋₇-Alkoxycarbonylgruppe) steht,
R^{c7}, R^{c8}, R^{c9}, R^{c10}, R^{c13} und R^{c14}, die unabhängig voneinander sind, jeweils für ein Wasserstoffatom, eine C₁₋₆-Alkylgruppe, eine Mono-, Di- oder Polyhalogen-C₁₋₆-alkylgruppe, eine C₁₋₆-Alkoxygruppe, eine Mono-, Di- oder Polyhalogen-C₁₋₆-alkoxygruppe, eine C₁₋₆-Alkylthiogruppe, eine C₁₋₆-Alkylsulfinylgruppe, eine C₁₋₆-Alkylsulfonylgruppe, eine C₂₋₇-Alkoxycarbonylgruppe, eine C₂₋₇-Alkylcarbonylgruppe, ein Halogenatom, eine Nitrogruppe, eine Cyanogruppe oder eine Phenylgruppe (mit der Maßgabe, daß eine solche Phenylgruppe substituiert sein kann mit einem oder mehreren Substituenten, die ausgewählt sind aus der Gruppe, bestehend aus einem Halogenatom, einer Trifluormethylgruppe, einer Nitrogruppe, einer C₁₋₆-Alkylgruppe, einer C₁₋₆-Alkoxygruppe und einer C₂₋₇-Alkoxycarbonylgruppe) steht,
R^{d1} für eine C₁₋₈-Alkylgruppe, eine C₃₋₇-Cycloalkylgruppe, eine mit einer C₃₋₇-Cycloalkylgruppe substituierte C₁₋₆-Alkylgruppe, eine C₃₋₇-Cycloalkenylgruppe, eine mit einer C₃₋₇-Cycloalkenylgruppe substituierte C₁₋₆-Alkylgruppe, eine C₂₋₈-Alkenylgruppe, eine C₂₋₈-Alkinylgruppe, eine mit einer C₁₋₆-Alkoxygruppe substituierte C₁₋₆-Alkylgruppe, eine mit einer C₂₋₆-Alkenyloxygruppe substituierte C₁₋₆-Alkylgruppe, eine mit einer C₂₋₆-Alkinyloxygruppe substituierte C₁₋₆-Alkylgruppe, eine mit einer Mono-, Di- oder Polyhalogen-C₁₋₆-alkoxygruppe substituierte C₁₋₆-Alkylgruppe, eine mit einer Mono-, Di- oder Polyhalogen-C₂₋₆-alkenyloxygruppe substituierte C₁₋₆-Alkylgruppe, eine mit einer Mono-, Di- oder Polyhalogen-C₂₋₆-alkinyloxygruppe substituierte C₁₋₆-Alkylgruppe, eine mit einer C₁₋₆-Alkylthiogruppe substituierte C₁₋₆-Alkylgruppe, eine mit einer C₁₋₆-Alkylsulfinylgruppe substituierte C₁₋₆-Alkylgruppe, eine mit einer C₁₋₆-Alkylsulfonylgruppe substituierte C₁₋₆-Alkylgruppe, eine Mono-, Di- oder Polyhalogen-C₁₋₈-alkylgruppe, eine Mono-, Di- oder Polyhalogen-C₂₋₈-alkenylgruppe, eine Mono-, Di- oder Polyhalogen-C₂₋₈-alkinylgruppe, eine mit einer Cyanogruppe substituierte C₁₋₆-Alkylgruppe, eine mit einer Cyanogruppe substituierte C₂₋₆-Alkenylgruppe, einer mit einer Cyanogruppe substituierte C₂₋₆-Alkinylgruppe, eine mit einer Nitrogruppe substituierte C₁₋₆-Alkylgruppe, eine mit einer Nitrogruppe substituierte C₂₋₆-Alkenylgruppe, eine mit einer Nitrogruppe substituierte C₂₋₆-Alkinylgruppe, eine mit einer C₂₋₇-Alkoxycarbonylgruppe substituierte C₁₋₆-Alkylgruppe, eine mit einer C₂₋₇-Alkoxycarbonylgruppe substituierte C₂₋₆-Alkenylgruppe, eine mit einer C₂₋₇-Alkoxycarbonylgruppe substituierte C₂₋₆-Alkinylgruppe, eine mit einer C₂₋₇-Alkylcarbonylgruppe substituierte C₁₋₆-Alkylgruppe, eine mit einer Mono-, Di- oder Polyhalogen-C₂₋₇-alkylcarbonylgruppe substituierte C₁₋₆-Alkylgruppe, eine mit einer C₃₋₇-Alkenylcarbonylgruppe substituierte C₁₋₆-Alkylgruppe, eine mit einer C₃₋₇-Alkinylcarbonylgruppe substituierte C₁₋₆-Alkylgruppe, eine mit einer C₂₋₅-Alkylcarbonylgruppe, die mit einer C₁₋₄-Alkoxygruppe substituiert ist, substituierte C₁₋₆-Alkylgruppe, eine mit einer C₂₋₅-Alkylcarbonylgruppe, die mit einer C₁₋₄-Alkylthiogruppe substituierte ist, substituierte C₁₋₆-Alkylgruppe, eine mit einer C₂₋₅-Alkylcarbonylgruppe, die mit einer C₁₋₄-Alkylsulfinylgruppe substituiert ist, substituierte C₁₋₆-Alkylgruppe, eine mit einer C₂₋₅-Alkylcarbonylgruppe, die mit einer C₁₋₄-Alkylsulfonylgruppe substituiert ist, substituierte C₁₋₆-Alkylgruppe, eine mit einer C₂₋₇-Alkylcarbonylgruppe substituierte C₂₋₆-Alkenylgruppe, eine mit einer C₂₋₇-Alkylcarbonylgruppe substituierte C₂₋₆-Alkinylgruppe, eine mit einer C₁₋₆-Alkylsulfamoylgruppe substituierte C₁₋₆-Alkylgruppe, eine mit einer C₁₋₆-Alkoxysulfamoylgruppe substituierte C₁₋₆-Alkylgruppe, eine mit einer Di(C₁₋₃-alkyl)sulfamoylgruppe substituierte C₁₋₆-Alkylgruppe, eine mit einer N-(C₁₋₃-alkyl)-N-(C₁₋₃-alkoxy)sulfamoylgruppe substituierte C₁₋₆-Alkylgruppe, eine mit einer C₂₋₇-Alkylcarbamoylgruppe substituierte C₁₋₆-Alkylgruppe, eine mit einer Di(C₁₋₃-alkyl)carbamoylgruppe substituierte C₁₋₆-Alkylgruppe, eine mit einer C₂₋₇-Alkoxycarbamoylgruppe substituierte C₁₋₆-Alkylgruppe, eine mit einer N-(C₁₋₃-alkyl)-N-(C₁₋₃-alkoxy)carbamoylgruppe substituierte C₁₋₆-Alkylgruppe, eine mit einer C₁₋₆-Alkylaminogruppe substituierte C₁₋₆-Alkylgruppe, eine mit einer C₁₋₆-Alkoxyaminogruppe substituierte C₁₋₆-Alkylgruppe, eine mit einer Di(C₁₋₃-alkyl)aminogruppe substituierte C₁₋₆-Alkylgruppe, einer mit einer N-(C₁₋₃-alkyl)-N-(C₁₋₃-alkoxy)aminogruppe substituierte C₁₋₆-Alkylgruppe, eine mit einer N-(C₂₋₇-alkylcarbonyl)-N-(C₁₋₆-alkyl)aminogruppe substituierte C₁₋₆-Alkylgruppe, eine mit einer N-(C₂₋₇-Alkylcarbonyl)-N-(C₁₋₆-alkoxy)aminogruppe substituierte C₁₋₆-Alkylgruppe, eine mit einer N-(C₁₋₆-Alkylsulfonyl)-N-(C₁₋₆-alkyl)aminogruppe substituierte C₁₋₆-Alkylgruppe, eine mit einer N₋(C₁₋₆-Alkylsulfonyl)-N-(C₁₋₆-alkoxy)aminogruppe substituierte C₁₋₆-Alkylgruppe, eine Phenylgruppe (mit der Maßgabe, daß eine solche Phenylgruppe substituiert sein kann mit einem oder mehreren Substituenten, die ausgewählt sind aus der Gruppe, bestehend aus einem Halogenatom, einer Trifluormethylgruppe, einer Nitrogruppe, einer C₁₋₆-Alkylgruppe, einer C₁₋₆-Alkoxygruppe und einer C₂₋₇-Alkoxycarbonylgruppe), eine mit einer Phenylgruppe substituierte C₁₋₆-Alkylgruppe (mit der Maßgabe, daß eine solche Phenylgruppe substituiert sein kann mit einem oder mehreren Substituenten, die ausgewählt sind aus der Gruppe, bestehend aus einem Halogenatom, einer Trifluormethylgruppe, einer Nitrogruppe, einer C₁₋₆-Alkylgruppe, einer C₁₋₆-Alkoxygruppe und einer C₂₋₇-Alkoxycarbonylgruppe), eine mit einer Phenylgruppe substituierte C₂₋₇-Alkenylgruppe (mit der Maßgabe, daß eine solche Phenylgruppe substituiert sein kann mit einem oder mehreren Substituenten, die ausgewählt sind aus der Gruppe, bestehend aus einem Halogenatom, einer Trifluormethylgruppe, einer Nitrogruppe, einer C₁₋₆-Alkylgruppe, einer C₁₋₆-Alkoxygruppe und einer C₂₋₇-Alkoxycarbonylgruppe), eine mit einer Phenylgruppe substituierte C₂₋₆-Alkinylgruppe (mit der Maßgabe, daß eine solche Phenylgruppe substituiert sein kann mit einem oder mehreren Substituenten, die ausgewählt sind aus der Gruppe, bestehend aus einem Halogenatom, einer Trifluormethylgruppe, einer Nitrogruppe, einer C₁₋₆-Alkylgruppe, einer C₁₋₆-Alkoxygruppe und einer C₂₋₇-Alkoxycarbonylgruppe), eine mit einer Phenoxygruppe substituierte C₁₋₆-Alkylgruppe (mit der Maßgabe, daß eine solche Phenoxygruppe substituiert sein kann mit einem oder mehreren Substituenten, die ausgewählt sind aus der Gruppe, bestehend aus einem Halogenatom, einer Trifluormethylgruppe, einer Nitrogruppe, einer C₁₋₆-Alkylgruppe, einer C₁₋₆-Alkoxygruppe und einer C₂₋₇-Alkoxycarbonylgruppe), eine mit einer Phenylthiogruppe substituierte C₁₋₆-Alkylgruppe (mit der Maßgabe, daß eine solche Phenylthiogruppe substituiert sein kann mit einem oder mehreren Substituenten, die ausgewählt sind aus der Gruppe, bestehend aus einem Halogenatom, einer Trifluormethylgruppe, einer Nitrogruppe, einer C₁₋₆-Alkylgruppe, einer C₁₋₆-Alkoxygruppe und einer C₂₋₇-Alkoxycarbonylgruppe), eine mit einer Phenylsulfinylgruppe substituierte C₁₋₆-Alkylgruppe (mit der Maßgabe, daß eine solche Phenylsulfinylgruppe substituiert sein kann mit einem oder mehreren Substituenten, die ausgewählt sind aus der Gruppe, bestehend aus einem Halogenatom, einer Trifluormethylgruppe, einer Nitrogruppe, einer C₁₋₆-Alkylgruppe, einer C₁₋₆-Alkoxygruppe und einer C₂₋₇-Alkoxycarbonylgruppe), eine mit einer Phenylsulfonylgruppe substituierte C₁₋₆-Alkylgruppe (mit der Maßgabe, daß eine solche Phenylsulfonylgruppe substituiert sein kann mit einem oder mehreren Substituenten, die ausgewählt sind aus der Gruppe, bestehend aus einem Halogenatom, einer Trifluormethylgruppe, einer Nitrogruppe, einer C₁₋₆-Alkylgruppe, einer C₁₋₆-Alkoxygruppe und einer C₂₋₇-Alkoxycarbonylgruppe), eine mit einer Benzyloxygruppe substituierte C₁₋₆-Alkylgruppe (mit der Maßgabe, daß die Phenylgruppe einer solchen Benzyloxygruppe substituiert sein kann mit einem oder mehreren Substituenten, die ausgewählt sind aus der Gruppe, bestehend aus einem Halogenatom, einer Trifluormethylgruppe, einer Nitrogruppe, einer C₁₋₆-Alkylgruppe, einer C₁₋₆-Alkoxygruppe und einer C₂₋₇-Alkoxycarbonylgruppe), eine mit einer Benzylthiogruppe substituierte C₁₋₆-Alkylgruppe (mit der Maßgabe, daß die Phenylgruppe einer solchen Benzylthiogruppe substituiert sein kann mit einem oder mehreren Substituenten, die ausgewählt sind aus der Gruppe, bestehend aus einem Halogenatom, einer Trifluormethylgruppe, einer Nitrogruppe, einer C₁₋₆-Alklgruppe, einer C₁₋₆-Alkoxygruppe und einer C₂₋₇-Alkoxycarbonylgruppe), eine mit einer Benzylsulfinylgruppe substituierte C₁₋₆-Alkylgruppe (mit der Maßgabe, daß die Phenylgruppe einer solchen Benzylsulfinylgruppe substituiert sein kann mit einem oder mehreren Substituenten, die ausgewählt sind aus der Gruppe, bestehend aus einem Halogenatom, einer Trifluormethylgruppe, einer Nitrogruppe, einer C₁₋₆-Alkylgruppe, einer C₁₋₆-Alkoxygruppe und einer C₂₋₇-Alkoxycarbonylgruppe), eine mit einer Benzylsulfonylgruppe substituierte C₁₋₆-Alkylgruppe (mit der Maßgabe, daß die Phenylgruppe einer solchen Benzylsulfonylgruppe substituiert sein kann mit einem oder mehreren Substituenten, die ausgewählt sind aus der Gruppe, bestehend aus einem Halogenatom, einer Trifluormethylgruppe, einer Nitrogruppe, einer C₁₋₆-Alkylgruppe, einer C₁₋₆-Alkoxygruppe und einer C₂₋₇-Alkoxycarbonylgruppe), eine mit einer Phenylcarbonylgruppe substituierte C₁₋₆-Alkylgruppe (mit der Maßgabe, daß eine solche Phenylcarbonylgruppe substituiert sein kann mit einem oder mehreren Substituenten, die ausgewählt sind aus der Gruppe, bestehend aus einem Halogenatom, einer Trifluormethylgruppe, einer Nitrogruppe, einer C₁₋₆-Alkylgruppe, einer C₁₋₆-Alkoxygruppe und einer C₂₋₇-Alkoxycarbonylgruppe), eine mit einer Benzylcarbonylgruppe substituierte C₁₋₆-Alkylgruppe (mit der Maßgabe, daß die Phenylgruppe einer solchen Benzylcarbonylgruppe substituiert sein kann mit einem oder mehreren Substituenten, die ausgewählt sind aus der Gruppe, bestehend aus einem Halogenatom, einer Trifluormethylgruppe, einer Nitrogruppe, einer C₁₋₆-Alkylgruppe, einer C₁₋₆-Alkoxygruppe und einer C₂₋₇-Alkoxycarbonylgruppe), eine mit einer Aminogruppe, die mit einer C₁₋₄-Alkylsulfonylgruppe substituiert ist, substituierte C₁₋₆-Alkylgruppe, oder mit einer Aminogruppe, die mit einer C₂₋₄-Alkylcarbonylgruppe substituiert ist, substituierte C₁₋₆-Alkylgruppe steht,
R^{d2}, R^{d3} und R^{d4}, die unabhängig voneinander sind, jeweils für ein Wasserstoffatom, eine C₁₋₈-Alkylgruppe, eine C₂₋₈-Alkenylgruppe, eine C₂₋₈-Alkinylgruppe oder eine Phenylgruppe (mit der Maßgabe, daß eine solche Phenylgruppe substituiert sein kann mit einem oder mehreren Substituenten, die ausgewählt sind aus der Gruppe, bestehend aus einem Halogenatom, einer Trifluormethylgruppe, einer Nitrogruppe, einer C₁₋₆-Alkylgruppe, einer C₁₋₆-Alkoxygruppe und einer C₂₋₇-Alkoxycarbonylgruppe) steht,
R^{d5} für eine C₁₋₈-Alkylgruppe, eine C₂₋₈-Alkenylgruppe, eine C₂₋₈-Alkinylgruppe oder eine Phenylgruppe (mit der Maßgabe, daß eine solche Phenylgruppe substituiert sein kann mit einem oder mehreren Substituenten, die ausgewählt sind aus der Gruppe, bestehend aus einem Halogenatom, einer Trifluormethylgruppe, einer Nitrogruppe, einer C₁₋₆-Alkylgruppe, einer C₁₋₆-Alkoxygruppe und einer C₂₋₇-Alkoxycarbonylgruppe) steht,
R^{d6} für eine C₁₋₈-Alkylgruppe, eine C₂₋₈-Alkenylgruppe, eine C₂₋₈-Alkinylgruppe, eine mit einer C₁₋₆-Alkylthiogruppe substituierte C₁₋₆-Alkylgruppe, eine mit einer C₁₋₆-Alkylsulfinylgruppe substituierte C₁₋₆-Alkylgruppe, eine mit einer C₁₋₆-Alkylsulfonylgruppe substituierte C₁₋₆-Alkylgruppe, eine mit einer C₁₋₆-Alkoxygruppe substituierte C₁₋₆-Alkylgruppe, eine mit einer C₂₋₇-Alkoxycarbonylgruppe substituierte C₁₋₆-Alkylgruppe, eine mit einer C₂₋₇-Alkylcarbonylgruppe substituierte C₁₋₆-Alkylgruppe, eine mit einer Cyanogruppe substituierte C₁₋₆-Alkylgruppe, eine Phenylgruppe (mit der Maßgabe, daß eine solche Phenylcarbonylgruppe substituiert sein kann mit einem oder mehreren Substituenten, die ausgewählt sind aus der Gruppe, bestehend aus einem Halogenatom, einer Trifluormethylgruppe, einer Nitrogruppe, einer C₁₋₆-Alkylgruppe, einer C₁₋₆-Alkoxygruppe und einer C₂₋₇-Alkoxycarbonylgruppe), oder eine mit einer Phenylgruppe substituierte C₁₋₆-Alkylgruppe (mit der Maßgabe, daß die eine solche Phenylgruppe substituiert sein kann mit einem oder mehreren Substituenten, die ausgewählt sind aus der Gruppe, bestehend aus einem Halogenatom, einer Trifluormethylgruppe, einer Nitrogruppe, einer C₁₋₆-Alkylgruppe, einer C₁₋₆-Alkoxygruppe und einer C₂₋₇-Alkoxycarbonylgruppe) steht,
R^{e1} für eine C₁₋₈-Alkylgruppe, eine C₃₋₇-Cycloalkylgruppe, eine mit einer C₃₋₇-Cycloalkylgruppe substituierte C₁₋₆-Alkylgruppe, eine C₃₋₇-Cycloalkenylgruppe, eine mit einer C₃₋₇-Cycloalkinylgruppe substituierte C₁₋₆-Alkylgruppe, eine C₂₋₈-Alkenylgruppe, eine C₂₋₈-Alkinylgruppe, eine mit einer C₁₋₆-Alkoxygruppe substituierte C₁₋₆-Alkylgruppe, eine mit einer C₂₋₆-Alkenyloxygruppe substituierte C₁₋₆-Alkylgruppe, eine mit einer C₂₋₆-Alkinyloxygruppe substituierte C₁₋₆-Alkylgruppe, eine mit einer Mono-, Di- oder Polyhalogen-C₁₋₆-alkoxygruppe substituierte C₁₋₆-Alkylgruppe, eine mit einer Mono-, Di- oder Polyhalogen-C₂₋₆-alkenyloxygruppe substituierte C₁₋₆-Alkylgruppe, eine mit einer Mono-, Di- oder Polyhalogen-C₂₋₆-alkinyloxygruppe substituierte C₁₋₆-Alkylgruppe, eine mit einer C₁₋₆-Alkylthiogruppe substituierte C₁₋₆-Alkylgruppe, eine mit einer C₁₋₆-Alkylsulfinylgruppe substituierte C₁₋₆-Alkylgruppe, eine mit einer C₁₋₆-Alkylsulfonylgruppe substituierte C₁₋₆-Alkylgruppe, eine Mono-, Di- oder Polyhalogen-C₁₋₈-alkylgruppe, eine Mono-, Di- oder Polyhalogen-C₂₋₈-alkenylgruppe, eine Mono-, Di- oder Polyhalogen-C₂₋₈-alkinylgruppe, eine mit einer Cyanogruppe substituierte C₁₋₆-Alkylgruppe, eine mit einer Cyanogruppe substituierte C₂₋₆-Alkenylgruppe, eine mit einer Cyanogruppe substituierte C₂₋₆-Alkinylgruppe, eine mit einer Nitrogruppe substituierte C₁₋₆-Alkylgruppe, eine mit einer Nitrogruppe substituierte C₂₋₆-Alkenylgruppe, eine mit einer Nitrogruppe substituierte C₂₋₆-Alkinylgruppe, eine mit einer C₂₋₇-Alkoxycarbonylgruppe substituierte C₁₋₆-Alkylgruppe, eine mit einer C₂₋₇-Alkoxycarbonylgruppe substituierte C₂₋₆-Alkenylgruppe, eine mit einer C₂₋₇-Alkoxycarbonylgruppe substituierte C₂₋₆-Alkinylgruppe, eine mit einer C₂₋₇-Alkylcarbonylgruppe substituierte C₁₋₆-Alkylgruppe, eine mit einer Mono-, Di- oder Polyhalogen-C₂₋₇-alkylcarbonylgruppe substituierte C₁₋₆-Alkylgruppe, eine mit einer C₃₋₇-Alkinylcarbonylgruppe substituierte C₁₋₆-Alkylgruppe, eine mit einer C₃₋₇-Alkinylcarbonylgruppe substituierte C₁₋₆-Alkylgruppe, eine mit einer C₂₋₅-Alkylcarbonylgruppe, die mit einer C₁₋₄-Alkoxygruppe substituiert ist, substituierte C₁₋₆-Alkylgruppe, eine mit einer C₂₋₅-Alkylcarbonylgruppe, die mit einer C₁₋₄-Alkylthiogruppe substituiert ist, substituierte C₁₋₆-Alkylgruppe, eine mit einer C₂₋₅-Alkylcarbonylgruppe, die mit einer C₁₋₄-Alkylsulfinylgruppe substituiert ist, substituierte C₁₋₆-Alkylgruppe, eine mit einer C₂₋₅-Alkylcarbonylgruppe, die mit einer C₁₋₄-Alkylsulfonylgruppe substituiert ist, substituierte C₁₋₆-Alkylgruppe, eine mit einer C₂₋₇-Alkylcarbonylgruppe substituierte C₂₋₆-Alkenylgruppe, eine mit einer C₂₋₇-Alkylcarbonylgruppe substituierte C₂₋₆-Alkinylgruppe, eine mit einer C₁₋₆-Alkylsulfamoylgruppe substituierte C₁₋₆-Alkylgruppe, eine mit einer C₁₋₆-Alkoxysulfamoylgruppe substituierte C₁₋₆-Alkylgruppe, eine mit einer Di(C₁₋₃-alkyl)sulfamoylgruppe substituierte C₁₋₆-Alkylgruppe, eine mit einer N-(C₁₋₃-Alkyl)-N-(C₁₋₃-alkoxy)sulfamoylgruppe substituierte C₁₋₆-Alkylgruppe, eine mit einer C₂₋₇-Alkylcarbamoylgruppe substituierte C₁₋₆-Alkylgruppe, eine mit einer Di(C₁₋₃-alkyl)carbamoylgruppe substituierte C₁₋₆-Alkylgruppe, eine mit einer C₂₋₇-Alkoxycarbamoylgruppe substituierte C₁₋₆-Alkylgruppe, eine mit einer N-(C₁₋₃-Alkyl)-N-(C₁₋₃-alkoxy)carbamoylgruppe substituierte C₁₋₆-Alkylgruppe, eine mit einer C₁₋₆-Alkylaminogruppe substituierte C₁₋₆-Alkylgruppe, eine mit einer C₁₋₆-Alkoxyaminogruppe substituierte C₁₋₆-Alkylgruppe, eine mit einer Di(C₁₋₃-alkyl)aminogruppe substituierte C₁₋₆-Alkylgruppe, eine mit einer N-(C₁₋₃-Alkyl)-N-(C₁₋₃-alkoxy)aminogruppe substituierte C₁₋₆-Alkylgruppe, eine mit einer N-(C₂₋₇-Alkylcarbonyl)-N-(C₁₋₆-alkyl)aminogruppe substituierte C₁₋₆-Alkylgruppe, eine mit einer N-(C₂₋₇-alkylcarbonyl)-N-(C₁₋₆-alkoxy)aminogruppe substituierte C₁₋₆-Alkylgruppe, eine mit einer N-(C₁₋₆-Alkylsulfonyl)-N-(C₁₋₆-alkyl)aminogruppe substituierte C₁₋₆-Alkylgruppe, eine mit einer N-(C₁₋₆-Alkylsulfonyl)-N-(C₁₋₆-alkoxy)aminogruppe substituierte C₁₋₆-Alkylgruppe, eine Phenylgruppe (mit der Maßgabe, daß eine solche Phenylgruppe substituiert sein kann mit einem oder mehreren Substituenten, die ausgewählt sind aus der Gruppe, bestehend aus einem Halogenatom, einer Trifluoromethylgruppe, einer Nitrogruppe, einer C₁₋₆-Alkylgruppe, einer C₁₋₆-Alkoxygruppe und einer C₂₋₇-Alkoxycarbonylgruppe), eine mit einer Phenylgruppe substituierte C₁₋₆-Alkylgruppe (mit der Maßgabe, daß eine solche Phenylgruppe substituiert sein kann mit einem oder mehreren Substituenten, die ausgewählt sind aus der Gruppe, bestehend aus einem Halogenatom, einer Trifluormethylgruppe, einer Nitrogruppe, einer C₁₋₆-Alkylgruppe, einer C₁₋₆-Alkoxygruppe und einer C₂₋₇-Alkoxycarbonylgruppe), eine mit einer Phenylgruppe substituierte C₂₋₇-Alkenylgruppe (mit der Maßgabe, daß eine solche Phenylgruppe substituiert sein kann mit einem oder mehreren Substituenten, die ausgewählt sind aus der Gruppe, bestehend aus einem Halogenatom, einer Trifluormethylgruppe, einer Nitrogruppe, einer C₁₋₆-Alkylgruppe, einer C₁₋₆-Alkoxygruppe und einer C₂₋₇-Alkoxycarbonylgruppe), eine mit einer Phenylgruppe substituierte C₂₋₆-Alkinylgruppe (mit der Maßgabe, daß eine solche Phenylgruppe substituiert sein kann mit einem oder mehreren Substituenten, die ausgewählt sind aus der Gruppe, bestehend aus einem Halogenatom, einer Trifluormethylgruppe, einer Nitrogruppe, einer C₁₋₆-Alkylgruppe, einer C₁₋₆-Alkoxygruppe und einer C₂₋₇-Alkoxycarbonylgruppe), einer mit einer Phenoxygruppe substituierte C₁₋₆-Alkylgruppe (mit der Maßgabe, daß eine solche Phenoxygruppe substituiert sein kann mit einem oder mehreren Substituenten, die ausgewählt sind aus der Gruppe, bestehend aus einem Halogenatom, einer Trifluormethylgruppe, einer Nitrogruppe, einer C₁₋₆-Alkylgruppe, einer C₁₋₆-Alkoxygruppe und einer C₂₋₇-Alkoxycarbonylgruppe), eine mit einer Phenylthiogruppe substituierte C₁₋₆-Alkylgruppe (mit der Maßgabe, daß eine solche Phenylthiogruppe substituiert sein kann mit einem oder mehreren Substituenten, die ausgewählt sind aus der Gruppe, bestehend aus einem Halogenatom, einer Trifluormethylgruppe, einer Nitrogruppe, einer C₁₋₆-Alkylgruppe, einer C₁₋₆-Alkoxygruppe und einer C₂₋₇-Alkoxycarbonylgruppe), eine mit einer Phenylsulfinylgruppe substituierte C₁₋₆-Alkylgruppe (mit der Maßgabe, daß eine solche Phenylsulfinylgruppe substituiert sein kann mit einem oder mehreren Substituenten, die ausgewählt sind aus der Gruppe, bestehend aus einem Halogenatom, einer Trifluormethylgruppe, einer Nitrogruppe, einer C₁₋₆-Alkylgruppe, einer C₁₋₆-Alkoxygruppe und einer C₂₋₇-Alkoxycarbonylgruppe), eine mit einer Phenylsulfonylgruppe substituierte C₁₋₆-Alkylgruppe (mit der Maßgabe, daß eine solche Phenylsulfonylgruppe substituiert sein kann mit einem oder mehreren Substituenten, die ausgewählt sind aus der Gruppe, bestehend aus einem Halogenatom, einer Trifluormethylgruppe, einer Nitrogruppe, einer C₁₋₆-Alkylgruppe, einer C₁₋₆-Alkoxygruppe und einer C₂₋₇-Alkoxycarbonylgruppe), eine mit einer Benzyloxygruppe substituierte C₁₋₆-Alkylgruppe (mit der Maßgabe, daß die Phenylgruppe einer solchen Benzyloxygruppe substituiert sein kann mit einem oder mehreren Substituenten, die ausgewählt sind aus der Gruppe, bestehend aus einem Halogenatom, einer Trifluormethylgruppe, einer Nitrogruppe, einer C₁₋₆-Alkylgruppe, einer C₁₋₆-Alkoxygruppe und einer C₂₋₇-Alkoxycarbonylgruppe), ein mit einer Benzylthiogruppe substituierte C₁₋₆-Alkylgruppe (mit der Maßgabe, daß die Phenylgruppe einer solchen Benzylthiogruppe substituiert sein kann mit einem oder mehreren Substituenten, die ausgewählt sind aus der Gruppe, bestehend aus einem Halogenatom, einer Tri- fluormethylgruppe, einer Nitrogruppe, einer C₁₋₆-Alkylgruppe, einer C₁₋₆-Alkoxygruppe und einer C₂₋₇-Alkoxycarbonylgruppe), eine mit einer Benzylsulfinylgruppe substituierte C₁₋₆-Alkylgruppe (mit der Maßgabe, daß die Phenylgruppe einer solchen Benzylsulfinylgruppe substituiert sein kann mit einem oder mehreren Substituenten, die ausgewählt sind aus der Gruppe, bestehend aus einem Halogenatom, einer Trifluormethylgruppe, einer Nitrogruppe, einer C₁₋₆-Alkylgruppe, einer C₁₋₆-Alkoxygruppe und einer C₂₋₇-Alkoxycarbonylgruppe), eine mit einer Benzylsulfonylgruppe substituierte C₁₋₆-Alkylgruppe (mit der Maßgabe, daß die Phenylgruppe eine solche Benzylsulfonylgruppe substituiert sein kann mit einem oder mehreren Substituenten, die ausgewählt sind aus der Gruppe, bestehend aus einem Halogenatom, einer Trifluormethylgruppe, einer Nitrogruppe, einer C₁₋₆-Alkylgruppe, einer C₁₋₆-Alkoxygruppe und einer C₂₋₇-Alkoxycarbonylgruppe), eine mit einer Phenylcarbonylgruppe substituierte C₁₋₆-Alkylgruppe (mit der Maßgabe, daß eine solche Phenylcarbonylgruppe substituiert sein kann mit einem oder mehreren Substituenten, die ausgewählt sind aus der Gruppe, bestehend aus einem Halogenatom, einer Trifluormethylgruppe, einer Nitrogruppe, einer C₁₋₆-Alkylgruppe, einer C₁₋₆-Alkoxygruppe und einer C₂₋₇-Alkoxycarbonylgruppe), eine mit einer Benzylcarbonylgruppe substituierte C₁₋₆-Alkylgruppe (mit der Maßgabe, daß die Phenylgruppe einer solchen Benzylcarbonylgruppe substituiert sein kann mit einem oder mehreren Substituenten, die ausgewählt sind aus der Gruppe, bestehend aus einem Halogenatom, einer Trifluormethylgruppe, einer Nitrogruppe, einer C₁₋₆-Alkylgruppe, einer C₁₋₆-Alkoxygruppe und einer C₂₋₇-Alkoxycarbonylgruppe), eine mit einer Aminogruppe, die mit einer C₁₋₄-Alkylsulfonylgruppe substituiert ist, substituierte C₁₋₆-Alkylgruppe, oder eine mit einer Aminogruppe, die mit einer C₂₋₄-Alkylcarbonylgruppe substituiert ist, substituierte C₁₋₆-Alkylgruppe steht,
R^{e2}, R^{e3}, R^{e6} und R^{e7}, die unabhängig voneinander sind, jeweils für ein Wasserstoffatom, eine C₁₋₆-Alkylgruppe, eine Mono-, Di- oder Polyhalogen-C₁₋₆-alkylgruppe, eine C₁₋₆-Alkoxygruppe, eine Mono-, Di- oder Polyhalogen-C₁₋₆-alkoxygruppe, eine C₁₋₆-Alkylthiogruppe, eine C₁₋₆-Alkylsulfinylgruppe, eine C₁₋₆-Alkylsulfonylgruppe, eine C₂₋₇-Alkoxycarbonylgruppe, eine C₂₋₇-Alkylcarbonylgruppe, ein Halogenatom, eine Nitrogruppe, eine Cyanogruppe oder eine Phenylgruppe (mit der Maßgabe, daß eine solche Phenylgruppe substituiert sein kann mit einem oder mehreren Substituenten, die ausgewählt sind aus der Gruppe, bestehend aus einem Halogenatom, einer Trifluormethylgruppe, einer Nitrogruppe, einer C₁₋₆-Alkylgruppe, einer C₁₋₆-Alkoxygruppe und einer C₂₋₇-Alkoxycarbonylgruppe) steht,
R^{e4}, R^{e5}, R^{e8}, R^{e9} und R^{e10}, die unabhängig voneinander sind, jeweils für ein Wasserstoffatom, eine C₁₋₈-Alkylgruppe, eine C₂₋₈-Alkenylgruppe, eine C₂₋₈-Alkinylgruppe oder eine Phenylgruppe (mit der Maßgabe, daß eine solche Phenylgruppe substituiert sein kann mit einem oder mehreren Substituenten, die ausgewählt sind aus der Gruppe, bestehend aus einem Halogenatom, einer Trifluormethylgruppe, einer Nitrogruppe, einer C₁₋₆-Alkylgruppe, einer C₁₋₆-Alkoxygruppe und einer C₂₋₇-Alkoxycarbonylgruppe) steht,
R^{f1} für eine C₁₋₈-Alkylgruppe, eine C₃₋₇-Cycloalkylgruppe, eine mit einer C₃₋₇-Cycloalkylgruppe substituierte C₁₋₆-Alkylgruppe, eine C₃₋₇-Cycloalkenylgruppe, eine mit einer C₃₋₇-Cycloalkenylgruppe substituierte C₁₋₆-Alkylgruppe, eine C₂₋₈-Alkenylgruppe, eine C₂₋₈-Alkinylgruppe, eine mit einer C₁₋₆-Alkoxygruppe substituierte C₁₋₆-Alkylgruppe, eine mit einer C₂₋₆-Alkenyloxygruppe substituierte C₁₋₆-Alkylgruppe, eine mit einer C₂₋₆-Alkinyloxygruppe substituierte C₁₋₆-Alkylgruppe, eine mit einer Mono-, Di- oder Polyhalogen-C₁₋₆-alkoxygruppe substituierte C₁₋₆-Alkylgruppe, eine mit einer Mono-, Di- oder Polyhalogen-C₂₋₆-alkenyloxygruppe substituierte c₁₋₆-Alkylgruppe, eine mit einer Mono-, Di- oder Polyhalogen-C₂₋₆-alkinyloxygruppe substituierte C₁₋₆-Alkylgruppe, eine mit einer C₁₋₆-Alkylthiogruppe substituierte C₁₋₆-Alkylgruppe, eine mit einer C₁₋₆-Alkylsulfinylgruppe substituierte C₁₋₆-Alkylgruppe, eine mit einer C₁₋₆-Alkylsulfonylgruppe substituierte C₁₋₆-Alkylgruppe, eine Mono-, Di- oder Polyhalogen-C₁₋₈-alkylgruppe, eine Mono-, Di- oder Polyhalogen-C₂₋₈-alkenylgruppe, eine Mono-, Di- oder Polyhalogen-C₂₋₈-alkinylgruppe, eine mit einer Cyanogruppe substituierte C₁₋₆-Alkylgruppe, eine mit einer Cyanogruppe substituierte C₂₋₆-Alkenylgruppe, eine mit einer Cyanogruppe substituierte C₂₋₆-Alkinylgruppe, eine mit einer Nitrogruppe substituierte C₁₋₆-Alkylgruppe, eine mit einer Nitrogruppe substituierte C₂₋₆-Alkenylgruppe, eine mit einer Nitrogruppe substituierte C₂₋₆-Alkinylgruppe, eine mit einer C₂₋₇-Alkoxycarbonylgruppe substituierte C₁₋₆-Alkylgruppe, eine mit einer C₂₋₇-Alkoxycarbonylgruppe substituierte C₂₋₆-Alkenylgruppe, eine mit einer C₂₋₇-Alkoxycarbonylgruppe substituierte C₂₋₆-Alkinylgruppe, eine mit einer C₋₂₋₇-Alkylcarbonylgruppe substituierte C₁₋₆-Alkylgruppe, eine mit einer Mono-, Di- oder Polyhalogen-C₋₂₋₇-alkylcarbonylgruppe substituierte C₁₋₆-Alkylgruppe, eine mit einer C₃₋₇-Alkenylcarbonylgruppe substituierte C₁₋₆-Alkylgruppe, eine mit einer C₃₋₇-Alkinylcarbonylgruppe substituierte C₁₋₆-Alkylgruppe, eine mit einer C₂₋₅-Alkylcarbonylgruppe, die mit einer C₁₋₄-Alkoxygruppe substituiert ist, substituierte C₁₋₆-Alkylgruppe, eine mit einer C₂₋₅-Alkylcarbonylgruppe, die mit einer C₁₋₄-Alkylthiogruppe substituiert ist, substituierte C₁₋₆-Alkylgruppe, eine mit einer C₂₋₅-Alkylcarbonylgruppe, die mit einer C₁₋₄-Alkylsulfinylgruppe substituiert ist, substituierte C₁₋₆-Alkylgruppe, eine mit einer C₂₋₅-Alkylcarbonylgruppe, die mit einer D₁₋₄-Alkylsulfonylgruppe substituiert ist, substituierte C₁₋₆-Alkylgruppe, eine mit einer C₂₋₇-Alkylcarbonylgruppe substituierte C₂₋₆-Alkenylgruppe, eine mit einer C₂₋₇-Alkylcarbonylgruppe substituierte C₂₋₆-Alkinylgruppe, eine mit einer C₁₋₆-Alkylsulfamoylgruppe substituierte C₁₋₆-Alkylgruppe, eine mit einer C₁₋₆-Alkoxysulfamoylgruppe substituierte C₁₋₆-Alkylgruppe, eine mit einer Di(C₁₋₃-alkyl)sulfamoylgruppe substituierte C₁₋₆-Alkylgruppe, eine mit einer N-(C₁₋₃-Alkyl)-N-(C₁₋₃-alkoxy)sulfamoylgruppe substituierte C₁₋₆-Alkylgruppe, eine mit einer C₂₋₇-Alkylcarbamoylgruppe substituierte C₁₋₆-Alkylgruppe, eine mit einer Di(C₁₋₃-alkyl)carbamoylgruppe substituierte C₁₋₆-Alkylgruppe, eine mit einer C₂₋₇-Alkoxycarbamoylgruppe substituierte C₁₋₆-Alkylgruppe, eine mit einer N-(C₁₋₃-Alkyl)-N-(C₁₋₃-alkoxy)carbamoylgruppe substituierte C₁₋₆-Alkylgruppe, eine mit einer C₁₋₆-Alkylaminogruppe substituierte C₁₋₆-Alkylgruppe, eine mit einer C₁₋₆-Alkoxyaminogruppe substituierte C₁₋₆-Alkylgruppe, eine mit einer Di(C₁₋₃-alkyl)aminogruppe substituierte C₁₋₆-Alkylgruppe, eine mit einer N-(C₁₋₃-Alkyl)-N-(C₁₋₃-alkoxy)aminogruppe substituierte C₁₋₆-Alkylgruppe, eine mit einer N-(C₂₋₇-Alkylcarbonyl)-N-(C₁₋₆-alkyl)aminogruppe substituierte C₁₋₆-Alkylgruppe, eine mit einer N-(C₂₋₇-Alkylcarbonyl)-N-(C₁₋₆-alkoxy)aminogruppe substituierte C₁₋₆-Alkylgruppe, eine mit einer N-(C₁₋₆-Alkylsulfonyl)-N-(C₁₋₆-alkyl)aminogruppe substituierte C₁₋₆-Alkylgruppe, eine mit einer N-(C₁₋₆-Alkylsulfonyl)-N-(C₁₋₆-alkoxy)aminogruppe substituierte C₁₋₆-Alkylgruppe, eine Phenylgruppe (mit der Maßgabe, daß eine solche Phenylgruppe substituiert sein kann mit einem oder mehreren Substituenten, die ausgewählt sind aus der Gruppe, bestehend aus einem Halogenatom, einer Trifluormethylgruppe, einer Nitrogruppe, einer C₁₋₅-Alkylgruppe, einer C₁₋₆-Alkoxygruppe und einer C₂₋₇-Alkoxycarbonylgruppe), eine mit einer Phenylgruppe substituierte C₁₋₆-Alkylgruppe (mit der Maßgabe, daß eine solche Phenylgruppe substituiert sein kann mit einem oder mehreren Substituenten, die ausgewählt sind aus der Gruppe, bestehend aus einem Halogenatom, einer Trifluormethylgruppe` einer Nitrogruppe, einer C₁₋₆-Alkylgruppe, einer C₁₋₆-Alkoxygruppe und einer C₂₋₇-Alkoxycarbonylgruppe), eine mit einer Phenylgruppe substituierte C₂₋₇-Alkenylgruppe (mit der Maßgabe, daß eine solche Phenylgruppe substituiert sein kann mit einem oder mehreren Substituenten, die ausgewählt sind aus der Gruppe, bestehend aus einem Halogenatom, einer Trifluormethylgruppe, einer Nitrogruppe, einer C₁₋₆-Alkylgruppe, einer C₁₋₆-Alkoxygruppe und einer C₂₋₇-Alkoxycarbonylgruppe), eine mit einer Phenylgruppe substituierte C₂₋₆-Alkinylgruppe (mit der Maßgabe, daß eine solche Phenylgruppe substituiert sein kann mit einem oder mehreren Substituenten, die ausgewählt sind aus der Gruppe, bestehend aus einem Halogenatom, einer Trifluormethylgruppe, einer Nitrogruppe, einer C₁₋₆-Alkylgruppe, einer C₁₋₆-Alkoxygruppe und einer C₂₋₇-Alkoxycarbonylgruppe), eine mit einer Phenoxygruppe substituierte C₁₋₆-Alkylgruppe (mit der Maßgabe, daß eine solche Phenoxygruppe substituiert sein kann mit einem oder mehreren Substituenten, die ausgewählt sind aus der Gruppe, bestehend aus einem Halogenatom, einer Trifluormethylgruppe, einer Nitrogruppe, einer C₁₋₆-Alkylgruppe, einer C₁₋₆-Alkoxygruppe und einer C₂₋₇-Alkoxycarbonylgruppe), eine mit einer Phenylthiogruppe substituierte C₁₋₆-Alkylgruppe (mit der Maßgabe, daß eine solche Phenylthiogruppe substituiert sein kann mit einem oder mehreren Substituenten, die ausgewählt sind aus der Gruppe, bestehend aus einem Halogenatom, einer Trifluormethylgruppe, einer Nitrogruppe, einer C₁₋₆-Alkylgruppe, einer C₁₋₆-Alkoxygruppe und einer C₂₋₇-Alkoxycarbonylgruppe), eine mit einer Phenysulfinylgruppe substituierte C₁₋₆-Alkylgruppe (mit der Maßgabe, daß eine solche Phenylsulfinylgruppe substituiert sein kann mit einem oder mehreren Substituenten, die ausgewählt sind aus der Gruppe, bestehend aus einem Halogenatom, einer Trifluormethylgruppe, einer Nitrogruppe, einer C₁₋₆-Alkylgruppe, einer C₁₋₆-Alkoxygruppe und einer C₂₋₇-Alkoxycarbonylgruppe), eine mit einer Phenylsulfonylgruppe substituierte C₁₋₆-Alkylgruppe (mit der Maßgabe, daß eine solche Phenylsulfonylgruppe substituiert sein kann mit einem oder mehreren Substituenten, die ausgewählt sind aus der Gruppe, bestehend aus einem Halogenatom, einer Trifluormethylgruppe, einer Nitrogruppe, einer C₁₋₆-Alkylgruppe, einer C₁₋₆-Alkoxygruppe und einer C₂₋₇-Alkoxycarbonylgruppe), eine mit einer Benzyloxygruppe substituierte C₁₋₆-Alkylgruppe (mit der Maßgabe, daß die Phenylgruppe einer solchen Benzyloxygruppe substituiert sein kann mit einem oder mehreren Substituenten, die ausgewählt sind aus der Gruppe, bestehend aus einem Halogenatom, einer Trifluormethylgruppe, einer Nitrogruppe, einer C₁₋₆-Alkylgruppe, einer C₁₋₆-Alkoxygruppe und einer C₂₋₇-Alkoxycarbonylgruppe), eine mit einer Benzylthiogruppe substituierte C₁₋₆-Alkylgruppe (mit der Maßgabe, daß die Phenylgruppe einer solchen Benzylthiogruppe substituiert sein kann mit einem oder mehreren Substituenten₇ die ausgewählt sind aus der Gruppe, bestehend aus einem Halogenatom, einer Trifluormethylgruppe, einer Nitrogruppe, einer C₁₋₆-Alkylgruppe, einer C₁₋₆-Alkoxygruppe und einer C₂₋₇-Alkoxycarbonylgruppe), eine mit einer Benzylsulfinylgruppe substituierte C₁₋₆-Alkylgruppe (mit der Maßgabe, daß die Phenylgruppe eine solche Benzylsulfinylgruppe substituiert sein kann mit einem oder mehreren Substituenten, die ausgewählt sind aus der Gruppe, bestehend aus einem Halogenatom, einer Tnfluormethylgruppe, einer Nitrogruppe, einer C₁₋₆-Alkylgruppe, einer C₁₋₆-Alkoxygruppe und einer C₂₋₇-Alkoxycarbonylgruppe), eine mit einer Benzylsulfonylgruppe substituierte C₁₋₆-Alkylgruppe (mit der Maßgabe, daß die Phenylgruppe eine solche Benzylsulfonylgruppe substituiert sein kann mit einem oder mehreren Substituenten, die ausgewählt sind aus der Gruppe, bestehend aus einem Halogenatom, einer Trifluormethylgruppe, einer Nitrogruppe, einer C₁₋₆-Alkylgruppe, einer C₁₋₆-Alkoxygruppe und einer C₂₋₇-Alkoxycarbonylgruppe), eine mit einer Phenylcarbonylgruppe substituierte C₁₋₆-Alkylgruppe (mit der Maßgabe, daß eine solche Phenylcarbonylgruppe substituiert sein kann mit einem oder mehreren Substituenten, die ausgewählt sind aus der Gruppe, bestehend aus einem Halogenatom, einer Trifluormethylgruppe, einer Nitrogruppe, einer C₁₋₆-Alkylgruppe, einer C₁₋₆-Alkoxygruppe und einer C₂₋₇-Alkoxycarbonylgruppe), eine mit einer Benzylcarbonylgruppe substituierte C₁₋₆-Alkylgruppe (mit der Maßgabe, daß die Phenylgruppe einer solchen Benzylcarbonylgruppe substituiert sein kann mit einem oder mehreren Substituenten, die ausgewählt sind aus der Gruppe, bestehend aus einem Halogenatom, einer Trifluormethylgruppe, einer Nitrogruppe, einer C₁₋₆-Alkylgruppe, einer C₁₋₆-Alkoxygruppe und einer C₂₋₇-Alkoxycarbonylgruppe), eine mit einer Aminogruppe, die mit einer C₁₋₄-Alkylsulfonylgruppe substituiert ist, substituierte C₁₋₆-Alkylgruppe, oder eine mit einer Aminogruppe, die mit einer C₂₋₄-Alkylcarbonylgruppe substituiert ist, substituierte C₁₋₆-Alkylgruppe steht,
R^{f2} für eine C₁₋₈-Alkylgruppe, eine C₂₋₈-Alkenylgruppe, eine C₂₋₈-Alkinylgruppe, mit einer C₁₋₆-Alkylthiogruppe substituierte C₁₋₆-Alkylgruppe, eine mit einer C₁₋₆-Alkylsulfinylgruppe substituierte C₁₋₆-Alkylgruppe, eine mit einer C₁₋₆-Alkylsulfonylgruppe substituierte C₁₋₆-Alkylgruppe, eine mit einer C₁₋₆-Alkoxygruppe substituierte C₁₋₆-Alkylgruppe, eine mit einer C₂₋₇-Alkoxycarbonylgruppe substituierte C₁₋₆-Alkylgruppe, eine mit einer C₂₋₇-Alkylcarbonylgruppe substituierte C₁₋₆-Alkylgruppe, eine mit einer Cyanogruppe substituierte C₁₋₆-Alkylgruppe, eine Phenylgruppe (mit der Maßgabe, daß eine solche Phenylgruppe substituiert sein kann mit einem oder mehreren Substituenten, die ausgewählt sind aus der Gruppe, bestehend aus einem Halogenatom, einer Trifluormethylgruppe, einer Nitrogruppe, einer C₁₋₆-Alkylgruppe, einer C₁₋₆-Alkoxygruppe und einer C₂₋₇-Alkoxycarbonylgruppe), oder eine mit einer Phenylgruppe substituierte C₁₋₆-Alkylgruppe (mit der Maßgabe, daß eine solche Phenylgruppe substituiert sein kann mit einem oder mehreren Substituenten, die ausgewählt sind aus der Gruppe, bestehend aus einem Halogenatom, einer Trifluormethylgruppe, einer Nitrogruppe, einer C₁₋₆-Alkylgruppe, einer C₁₋₆-Alkoxygruppe und einer C₂₋₇-Alkoxycarbonylgruppe) steht,
R^{f3}, R^{f4}, R^{f7}, R^{f8}, R^{f11} und R^{f12}, die unabhängig voneinander sind, jeweils für ein Wasserstoffatom, eine C₁₋₆-Alkylgruppe, eine Mono-, Di- oder Polyhalogen-C₁₋₆-alkylgruppe, eine C₁₋₆-Alkoxygruppe, eine Mono-, Di- oder Polyhalogen-C₁₋₆-alkoxygruppe, eine C₁₋₆-Alkylthiogruppe, eine C₁₋₆-Alkylsulfinylgruppe, eine C₁₋₆-Alkylsulfonylgruppe, eine C₂₋₇-Alkoxycarbonylgruppe, eine C₂₋₇-Alkylcarbonylgruppe, ein Halogenatom, eine Nitrogruppe, eine Cyanogruppe oder eine Phenylgruppe (mit der Maßgabe, daß eine solche Phenylgruppe substituiert sein kann mit einem oder mehreren Substituenten, die ausgewählt sind aus der Gruppe, bestehend aus einem Halogenatom, einer Trifluormethylgruppe, einer Nitrogruppe, einer C₁₋₆-Alkylgruppe, einer C₁₋₆-Alkoxygruppe und einer C₂₋₇-Alkoxycarbonylgruppe) steht,
R^{f5}, R^{f6}, R^{f9}, R^{f10}, R^{f13}, R^{f14} und R^{f15}, die unabhängig voneinander sind, jeweils für ein Wasserstoffatom, eine C₁₋₈-Alkylgruppe, eine C₂₋₈-Alkenylgruppe, eine C₂₋₈- Alkinylgruppe oder eine Phenylgruppe (mit der Maßgabe, daß eine solche Phenylgruppe substituiert sein kann mit einem oder mehreren Substituenten, die ausgewählt sind aus der Gruppe, bestehend aus einem Halogenatom, einer Trifluormethylgruppe, einer Nitrogruppe, einer C₁₋₆-Alkylgruppe, einer C₁₋₆-Alkoxygruppe und einer C₂₋₇-Alkoxycarbonylgruppe) steht,
R^{g1} für eine C₁₋₈-Alkylgruppe, eine C₃₋₇-Cycloalkylgruppe, eine mit einer C₃₋₇-Cycloalkylgruppe substituierte C₁₋₆-Alkylgruppe, eine C₃₋₇-Cycloalkenylgruppe, eine mit einer C₃₋₇-Cycloalkenylgruppe substituierte C₁₋₆-Alkylgruppe, eine C₂₋₈-Alkenylgrupp, eine C₂₋₈-Alkinylgruppe, eine mit einer C₁₋₆-Alkoxygruppe substituierte C₁₋₆-Alkylgruppe, eine mit einer C₂₋₆-Alkenyloxygruppe substituierte C₁₋₆-Alkylgruppe, eine mit einer C₂₋₆-Alkinyloxygruppe substituierte C₁₋₆-Alkylgruppe, eine mit einer Mono-, Di- oder Polyhalogen-C₁₋₆-alkoxygruppe substituierte C₁₋₆-Alkylgruppe, eine mit einer Mono-, Di- oder Polyhalogen-C₂₋₆-Alkenyloxygruppe substituierte C₁₋₆-Alkylgruppe, eine mit einer Mono-, Di- oder Polyhalogen-C₂₋₆-alkinyloxygruppe substituierte C₁₋₆-Alkylgruppe, eine mit einer C₁₋₆-Alkylthiogruppe substituierte C₁₋₆-Alkylgruppe, eine mit einer C₁₋₆-Alkylsulfinylgruppe substituierte C₁₋₆-Alkylgruppe, eine mit einer C₁₋₆-Alkylsulfonylgruppe substituierte C₁₋₆-Alkylgruppe, eine Mono-, Di- oder Polyhalogen-C₁₋₈-alkylgruppe, eine Mono-, Di- oder Polyhalogen-C₂₋₈-alkenylgruppe, eine Mono-, Di- oder Polyhalogen-C₂₋₈-alkinylgruppe, eine mit einer Cyanogruppe substituierte C₁₋₆-Alkylgruppe, eine mit einer Cyanogruppe substituierte C₂₋₆-Alkenylgruppe, eine mit einer Cyanogruppe substituierte C₂₋₆-Alkinylgruppe, eine mit einer Nitrogruppe substituierte C₁₋₆-Alkylgruppe, eine mit einer Nitrogruppe substituierte C₂₋₆-Alkenylgruppe, eine mit einer Nitrogruppe substituierte C₂₋₆-Alkinylgruppe, eine mit einer C₂₋₇-Alkoxycarbonylgruppe substituierte C₁₋₆-Alkylgruppe, eine mit einer C₂₋₇-Alkoxycarbonylgruppe substituierte C₂₋₆-Alkenylgtuppe, eine mit einer C₂₋₇-Alkoxycarbonylgruppe substituierte C₂₋₆-Alkinylgruppe, eine mit einer C₂₋₇-Alkylcarbonylgruppe substituierte C₁₋₆-Alkylgruppe, eine mit einer Mono-, Di- oder Polyhalogen-C₂₋₇-Alkylcarbonylgruppe substituierte C₁₋₆-Alkylgruppe, eine mit einer C₃₋₇-Alkenylcarbonylgruppe substituierte C₁₋₆-Alkylgruppe, eine mit einer C₃₋₇-Alkinylcarbonylgruppe substituierte C₁₋₆-Alkylgruppe, eine mit einer C₂₋₅-Alkylcarbonylgruppe, die mit einer C₁₋₄-Alkoxygruppe substituiert ist, substituierte C₁₋₆-Alkylgruppe, eine mit einer C₂₋₅-Alkylcarbonylgruppe, die mit einer C₁₋₄-Alkylthiogruppe substituiert ist, substituierte C₁₋₆-Alkylgruppe, eine mit einer C₂₋₅-Alkylcarbonylgruppe, die mit einer C₁₋₄-Alkylsulfinylgruppe substituiert ist, substituierte C₁₋₆-Alkylgruppe, eine mit einer C₂₋₅-Alkylcarbonylgruppe, die mit einer C₁₋₄-Alkylsulfonylgruppe substituiert ist, substituierte C₁₋₆-Alkylgruppe, eine mit einer C₂₋₇-Alkylcarbonylgruppe substituierte C₂₋₆-Alkenylgruppe, eine mit einer C₂₋₇-Alkylcarbonylgruppe substituierte C₂₋₆-Alkinylgruppe, eine mit einer C₁₋₆-Alkylsulfamoylgruppe substituierte C₁₋₆-Alkylgruppe, eine mit einer C₁₋₆-Alkoxysulfamoylgruppe substituierte C₁₋₆-Alkylgruppe, eine mit einer Di(C₁₋₃-alkyl)sulfamoylgruppe substituierte C₁₋₆-Alkylgruppe, eine mit einer N-(C₁₋₃-Alkyl)-N-(C₁₋₃-alkoxy)sulfamoylgruppe substituierte C₁₋₆-Alkylgruppe, eine mit einer C₂₋₇-Alkylcarbamoylgruppe substituierte C₁₋₆-Alkylgruppe, eine mit einer Di(C₁₋₃-alkyl)carbamoylgruppe substituierte C₁₋₆-Alkylgruppe, eine mit einer C₂₋₇-Alkoxycarbamoylgruppe substituierte C₁₋₆-Alkylgruppe, eine mit einer N-(C₁₋₃-Alkyl)-N-(C₁₋₃-alkoxy)carbamoylgruppe substituierte C₁₋₆-Alkylgruppe, eine mit einer C₁₋₆-Alkylaminogruppe substituierte C₁₋₆-Alkylgruppe, eine mit einer C₁₋₆-Alkoxyaminogruppe substituierte C₁₋₆-Alkylgruppe, eine mit einer Di(C₁₋₃-alkyl)aminogruppe substituierte C₁₋₆-Alkylgruppe, eine mit einer N-(C₁₋₃-Alkyl)-N-(C₁₋₃-alkoxy)aminogruppe substituierte C₁₋₆-Alkylgruppe, eine mit einer N-(C₂₋₇-Alkylcarbonyl)-N-(C₁₋₆-alkyl)aminogruppe substituierte C₁₋₆-Alkylgruppe, eine mit einer N-(C₂₋₇-Alkylcarbonyl)-N-(C₁₋₆-alkoxy)aminogruppe substituierte C₁₋₆-Alkylgruppe, eine mit einer N-(C₁₋₆-Alkylsulfonyl)-N-(C₁₋₆-alkyl)aminogruppe substituierte C₁₋₆-Alkylgruppe, eine mit einer N-(C₁₋₆-Alkylsulfonyl)-N-(C₁₋₆-alkoxy)amino substituierte C₁₋₆-Alkylgruppe, eine mit einer Phenylgruppe substituierte C₁₋₆-Alkylgruppe (mit der Maßgabe, daß eine solche Phenylgruppe substituiert sein kann mit einem oder mehreren Substituenten, die ausgewählt sind aus der Gruppe, bestehend aus einem Halogenatom, einer Trifluormethylgruppe, einer Nitrogruppe, einer C₁₋₆-Alkylgruppe, einer C₁₋₆-Alkoxygruppe und einer C₂₋₇-Alkoxycarbonylgruppe), eine mit einer Phenylgruppe substituierte C₂₋₇-Alkenylgruppe (mit der Maßgabe, daß eine solche Phenylgruppe substituiert sein kann mit einem oder mehreren Substituenten, die ausgewählt sind aus der Gruppe, bestehend aus einem Halogenatom, einer Trifluormethylgruppe, einer Nitrogruppe, einer C₁₋₆-Alkylgruppe, einer C₁₋₆-Alkoxygruppe und einer C₂₋₇-Alkoxycarbonylgruppe), eine mit einer Phenylgruppe substituierte C₂₋₆-Alkinylgruppe (mit der Maßgabe, daß eine solche Phenylgruppe substituiert sein kann mit einem oder mehreren Substituenten, die ausgewählt sind aus der Gruppe, bestehend aus einem Halogenatom, einer Trifluormethylgruppe, einer Nitrogruppe, einer C₁₋₆-Alkylgruppe, einer C₁₋₆-Alkoxygruppe und einer C₂₋₇-Alkoxycarbonylgruppe), eine mit einer Phenoxygruppe substituierte C₁₋₆-Alkylgruppe (mit der Maßgabe, daß eine solche Phenoxygruppe substituiert sein kann mit einem oder mehreren Substituenten, die ausgewählt sind aus der Gruppe, bestehend aus einem Halogenatom, einer Trifluormethylgruppe, einer Nitrogruppe, einer C₁₋₆-Alkylgruppe, einer C₁₋₆-Alkoxygruppe und einer C₂₋₇-Alkoxycarbonylgruppe), eine mit einer Phenylthiogruppe substituierte C₁₋₆-Alkylgruppe (mit der Maßgabe, daß eine solche Phenylthiogruppe substituiert sein kann mit einem oder mehreren Substituenten, die ausgewählt sind aus der Gruppe, bestehend aus einem Halogenatom, einer Trifluormethylgruppe, einer Nitrogruppe, einer C₁₋₆-Alkylgruppe, einer C₁₋₆-Alkoxygruppe und einer C₂₋₇-Alkoxycarbonylgruppe), eine mit einer Phenylsulfinylgruppe substituierte C₁₋₆-Alkylgruppe (mit der Maßgabe, daß eine solche Phenylsulfinylgruppe substituiert sein kann mit einem oder mehreren Substituenten, die ausgewählt sind aus der Gruppe, bestehend aus einem Halogenatom, einer Trifluormethylgruppe, einer Nitrogruppe, einer C₁₋₆-Alkylgruppe, einer C₁₋₆-Alkoxygruppe und einer C₂₋₇-Alkoxycarbonylgruppe), eine mit einer Phenylsulfonylgruppe substituierte C₁₋₆-Alkylgruppe (mit der Maßgabe, daß eine solche Phenylsulfonylgruppe substituiert sein kann mit einem oder mehreren Substituenten, die ausgewählt sind aus der Gruppe, bestehend aus einem Halogenatom, einer Trifluormethylgruppe, einer Nitrogruppe, einer C₁₋₆-Alkylgruppe, einer C₁₋₆-Alkoxygruppe und einer C₂₋₇-Alkoxycarbonylgruppe), eine mit einer Benzyloxygruppe substituierte C₁₋₆-Alkylgruppe (mit der Maßgabe, daß die Phenylgruppe einer solchen Benzyloxygruppe substituiert sein kann mit einem oder mehreren Substituenten, die ausgewählt sind aus der Gruppe, bestehend aus einem Halogenatom, einer Trifluormethylgruppe, einer Nitrogruppe, einer C₁₋₆-Alkylgruppe, einer C₁₋₆-Alkoxygruppe und einer C₂₋₇-Alkoxycarbonylgruppe), eine mit einer Benzylthiogruppe substituierte C₁₋₆-Alkylgruppe (mit der Maßgabe, daß die Phenylgruppe einer solchen Benzylthiogruppe substituiert sein kann mit einem oder mehreren Substituenten, die ausgewählt sind aus der Gruppe, bestehend aus einem Halogenatom, einer Trifluormethylgruppe, einer Nitrogruppe, einer C₁₋₆-Alkylgruppe, einer C₁₋₆-Alkoxygruppe und einer C₂₋₇-Alkoxycarbonylgruppe), eine mit einer Benzylsulfinylgruppe substituierte C₁₋₆-Alkylgruppe (mit der Maßgabe, daß die Phenylgruppe einer solchen Benzylsulfinylgruppe substituiert sein kann mit einem oder mehreren Substituenten, die ausgewählt sind aus der Gruppe, bestehend aus einem Halogenatom, einer Trifluormethylgruppe, einer Nitrogruppe, einer C₁₋₆-Alkylgruppe, einer C₁₋₆-Alkoxygruppe und einer C₂₋₇-Alkoxycarbonylgruppe), eine mit einer Benzylsulfonylgruppe substituierte C₁₋₆-Alkylgruppe (mit der Maßgabe, daß die Phenylgruppe solch einr Benzylsulfonylgruppe substituiert sein kann mit einem oder mehreren Substituenten, die ausgewählt sind aus der Gruppe, bestehend aus einem Halogenatom, einer Trifluormethylgruppe, einer Nitrogruppe, einer C₁₋₆-Alkylgruppe, einer C₁₋₆-Alkoxygruppe und einer C₂₋₇-Alkoxycarbonylgruppe), eine mit einer Phenylcarbonylgruppe substituierte C₁₋₆-Alkylgruppe (mit der Maßgabe, daß eine solche Phenylcarbonylgruppe substituiert sein kann mit einem oder mehreren Substituenten, die ausgewählt sind aus der Gruppe, bestehend aus einem Halogenatom, einer Trifluormethylgruppe, einer Nitrogruppe, einer C₁₋₆-Alkylgruppe, einer C₁₋₆-Alkoxygruppe und einer C₂₋₇-Alkoxycarbonylgruppe), oder eine mit einer Benzylcarbonylgruppe substituierte C₁₋₆-Alkylgruppe (mit der Maßgabe, daß die Phenylgruppe einer solchen Benzylcarbonylgruppe substituiert sein kann mit einem oder mehreren Substituenten, die ausgewählt sind aus der Gruppe, bestehend aus einem Halogenatom, einer Trifluormethylgruppe, einer Nitrogruppe, einer C₁₋₆-Alkylgruppe, einer C₁₋₆-Alkoxygruppe und einer C₂₋₇-Alkoxycarbonylgruppe) steht,
R^{g2} und R^{g3}, die unabhängig voneinander sind, jeweils für eine C₁₋₆-Alkylgruppe, eine C₂₋₆-Alkenylgruppe, eine C₂₋₆-Alkinylgruppe, eine Mono-, Di- oder Polyhalogen-C₁₋₆-alkylgruppe, eine C₁₋₆-Alkoxygruppe, eine C₁₋₆-Alkylsulfonylgruppe, eine C₁₋₆-Alkylsulfamoylgruppe, eine Di(C₁₋₃-alkyl)sulfamoylgruppe, eine C₂₋₇-Alkoxycarbonylgruppe, eine C₂₋₇-Alkylcarbonylgruppe, eine C₂₋₇-Alkylcarbamoylgruppe, eine Di- (C₁₋₃-alkyl)carbamoylgruppe, eine Phenylgruppe (mit der Maßgabe, daß eine solche Phenylgruppe substituiert sein kann mit einem oder mehreren Substituenten, die ausgewählt sind aus der Gruppe, bestehend aus einem Halogenatom, einer Trifluormethylgruppe, einer Nitrogroppe, einer C₁₋₆-Alkylgruppe, einer C₁₋₆-Alkoxygruppe und einer C₂₋₇-Alkoxycarbonylgruppe), oder eine Benzylgruppe (mit der Maßgabe, daß die Phenylgruppe einer solchen Benzylgruppe substituiert sein kann mit einem oder mehreren Substituenten, die ausgewählt sind aus der Gruppe, bestehend aus einem Halogenatom, einer Trifluormethylgruppe, einer Nitrogruppe, einer C₁₋₆-Alkylgruppe, einer C₁₋₆-Alkoxygruppe und einer C₂₋₇-Alkoxycarbonylgruppe) steht,
oder R^{g2} und R^{g3} zusammen mit dem Stickstoffatom, an das sie gebunden sind, einen 3- bis 7-gliedrigen heterocyclischen Ring bilden,
X für ein Sauerstoffatom und ein Schwefelatom steht,
L für ein Wasserstoffatom, eine C₁₋₆-Alkylgruppe, eine C₂₋₆-Alkenylgruppe oder eine C₂₋₆-Alkinylgruppe steht,
G für 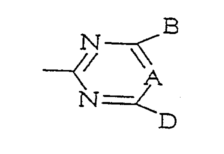 steht,
A für eine CH-Gruppe oder ein Stickstoffatom steht, und
B und D, die unabhängig voneinander sind, jeweils für eine C₁₋₄-Alkylgruppe, eine C₁₋₄-Alkoxygruppe, eine Mono-, Di- oder Polyhalogen-C₁₋₄-alkylgruppe, eine Mono-, Di- oder Polyhalogen-C₁₋₄-alkoxygruppe, ein Halogenatom, eine C₁₋₄-Alkylaminogruppe oder eine Di(C₁₋₄-alkyl)aminogruppe steht.

3. Iminosulfonyl-Harnstoffderivat oder dessen Salz nach Anspruch 2, wobei Q für 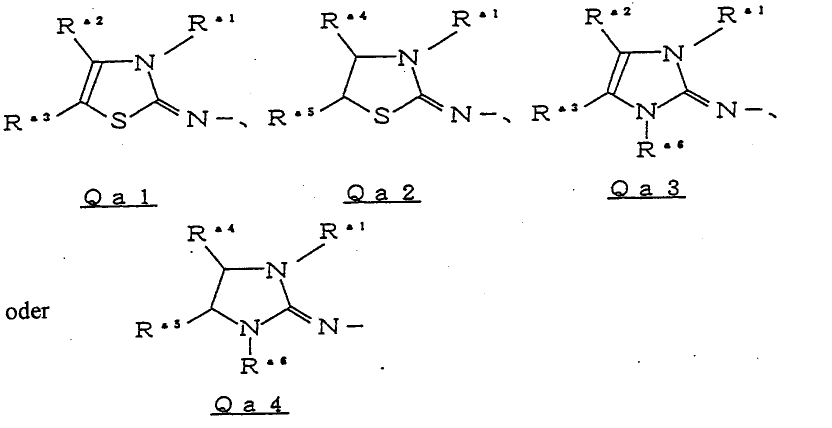 steht.

4. Iminosulfonyl-Harnstoffderivat oder dessen Salz nach Anspruch 2, wobei Q für 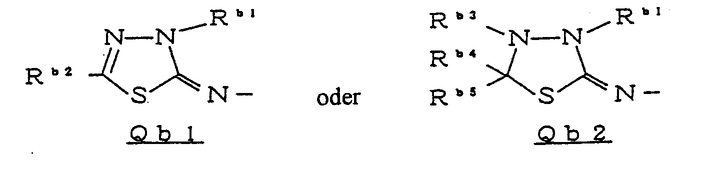 steht.

5. Iminosulfonyl-Harnstoffderivat oder dessen Salz nach Anspruch 2, wobei Q für 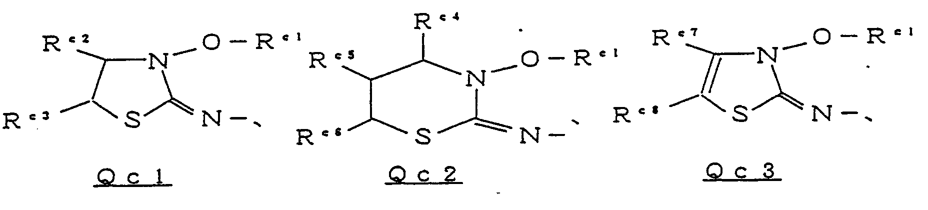 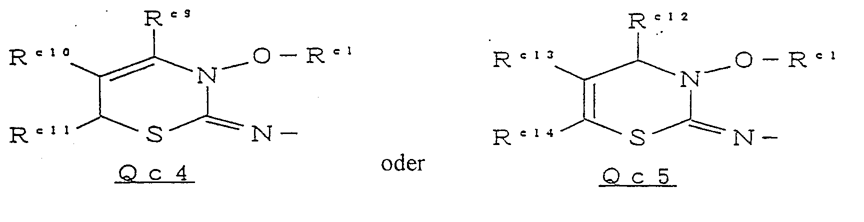 steht.

6. Iminosulfonyl-Harnstoffderivat oder dessen Salz nach Anspruch 2, wobei Q für 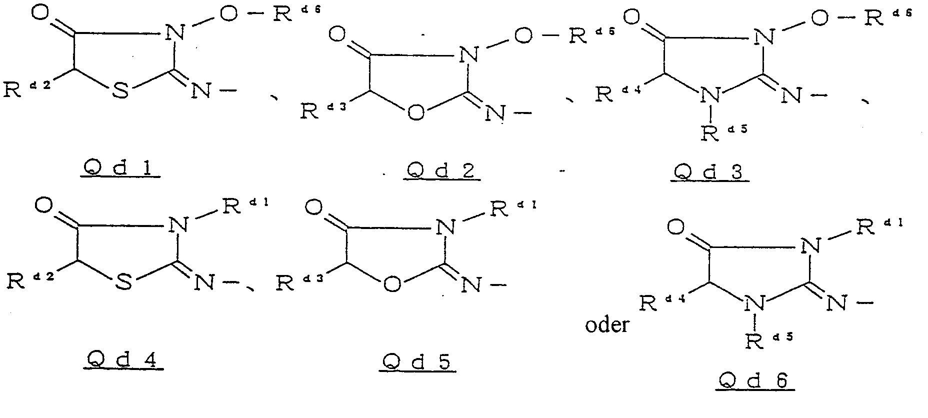 steht.

7. Iminosulfonyl-Harnstoffderivat oder dessen Salz nach Anspruch 2, wobei Q für 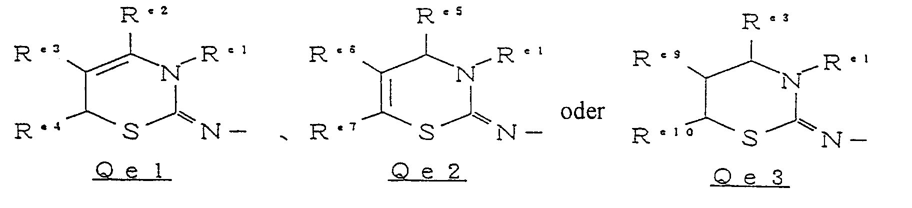 steht.

8. Iminosulfonyl-Harnstoffderivat oder dessen Salz nach Anspruch 2, wobei Q für 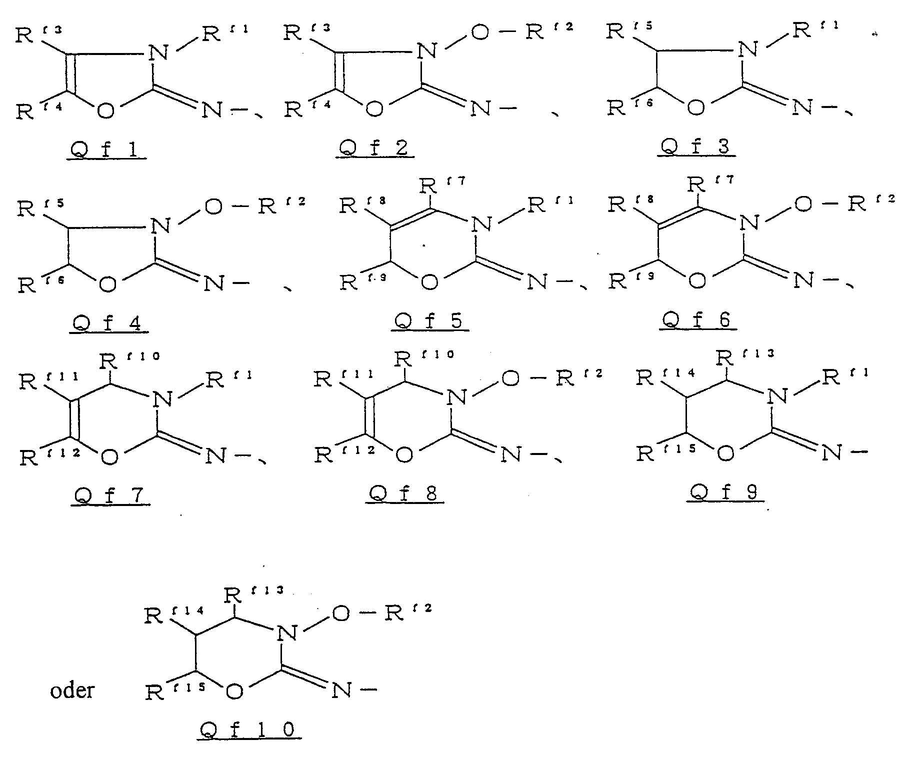 steht.

9. Iminosulfonyl-Harnstoffderivat oder dessen Salz nach Anspruch 2, wobei Q für 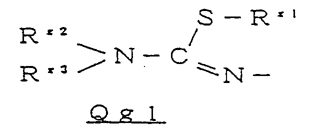 steht.

10. Herbizid, das ein Iminosulfonyl-Harnstoffderivat, wie es in Anspruch 1 definiert wird, als aktiven Bestandteil enthält.

11. Herbizid, das ein Iminosulfonyl-Harnstoffderivat, wie es in Anspruch 2 definiert wird, als aktiven Bestandteil enthält.

12. Herbizid, das ein Iminosulfonyl-Harnstoffderivat, wie es in Anspruch 3 definiert wird, als aktiven Bestandteil enthält.

13. Herbizid, das ein Iminosulfonyl-Harnstoffderivat, wie es in Anspruch 4 definiert wird, als aktiven Bestandteil enthält.

14. Herbizid, das ein Iminosulfonyl-Harnstoffderivat, wie es in Anspruch 5 definiert wird, als aktiven Bestandteil enthält.

15. Herbizid, das ein Iminosulfonyl-Harnstoffderivat, wie es in Anspruch 6 definiert wird, als aktiven Bestandteil enthält.

16. Herbizid, das ein Iminosulfonyl-Harnstoffderivat, wie es in Anspruch 7 definiert wird, als aktiven Bestandteil enthält.

17. Herbizid, das ein Iminosulfonyl-Harnstoffderivat, wie es in Anspruch 8 definiert wird, als aktiven Bestandteil enthält.

18. Herbizid, das ein Iminosulfonyl-Harnstoffderivat, wie es in Anspruch 9 definiert wird, als aktiven Bestandteil enthält.

19. Herbizides und wachstumsbekämpfendes Verfahren gegen Unkräuter, das eine Anwendung einer herbizidwirksamen Menge des Iminisulfonyl-Harnstoffderivats, wie es in Anspruch 1 definiert wird, umfaßt.

20. Herbizides und wachstumsbekämpfendes Verfahren gegen Unkräuter, das eine Anwendung einer herbizidwirksamen Menge des Iminisulfonyl-Harnstoffderivats, wie es in Anspruch 2 definiert wird, umfaßt.

21. Herbizides und wachstumsbekämpfendes Verfahren gegen Unkräuter, das eine Anwendung einer herbizidwirksamen Menge des Iminisulfonyl-Harnstoffderivats, wie es in Anspruch 3 definiert wird, umfaßt.

22. Herbizides und wachstumsbekämpfendes Verfahren gegen Unkräuter, das eine Anwendung einer herbizidwirksamen Menge des Iminisulfonyl-Harnstoffderivats, wie es in Anspruch 4 definiert wird, umfaßt.

23. Herbizides und wachstumsbekämpfendes Verfahren gegen Unkräuter, das eine Anwendung einer herbizidwirksamen Menge des Iminisulfonyl-Harnstoffderivats, wie es in Anspruch 5 definiert wird, umfaßt.

24. Herbizides und wachstumsbekämpfendes Verfahren gegen Unkräuter, das eine Anwendung einer herbizidwirksamen Menge des Iminisulfonyl-Harnstoffderivats, wie es in Anspruch 6 definiert wird, umfaßt.

25. Herbizides und wachstumsbekämpfendes Verfahren gegen Unkräuter, das eine Anwendung einer herbizidwirksamen Menge des Iminisulfonyl-Harnstoffderivats, wie es in Anspruch 7 definiert wird, umfaßt.

26. Herbizides und wachstumsbekämpfendes Verfahren gegen Unkräuter, das eine Anwendung einer herbizidwirksamen Menge des Iminisulfonyl-Harnstoffderivats, wie es in Anspruch 8 definiert wird, umfaßt.

27. Herbizides und wachstumsbekämpfendes Verfahren gegen Unkräuter, das eine Anwendung einer herbizidwirksamen Menge des Iminisulfonyl-Harnstoffderivats, wie es in Anspruch 9 definiert wird, umfaßt.

28. Iminosulfonyl-Harnstoffderivat oder dessen Salz nach Anspruch 3, wobei R^{a1} für 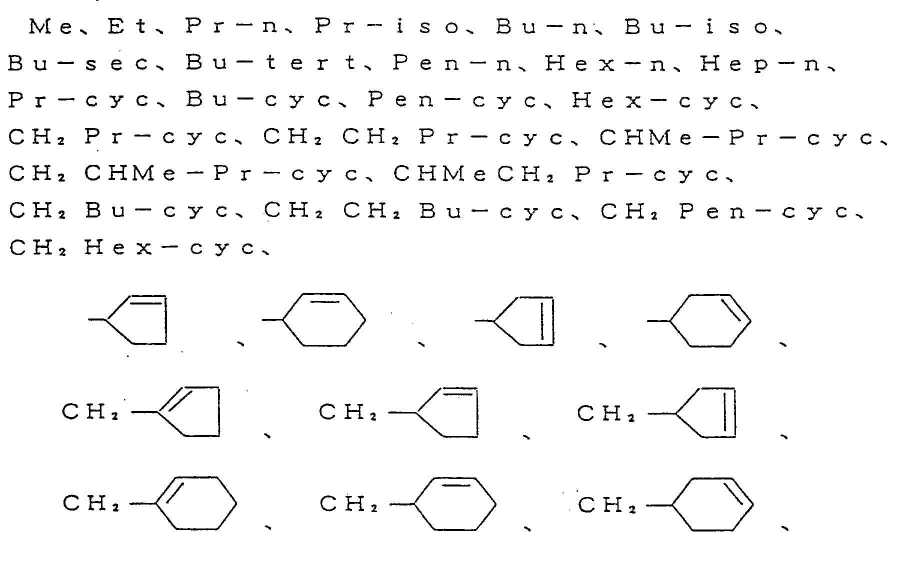 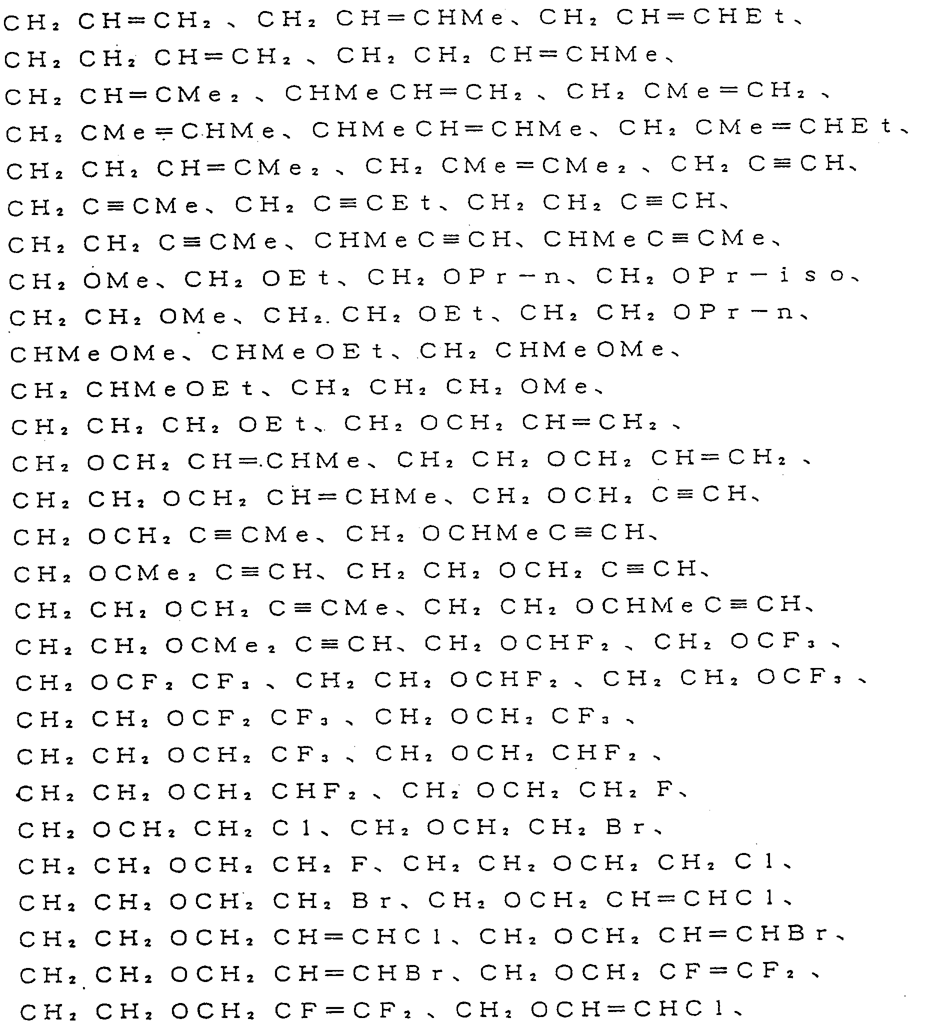 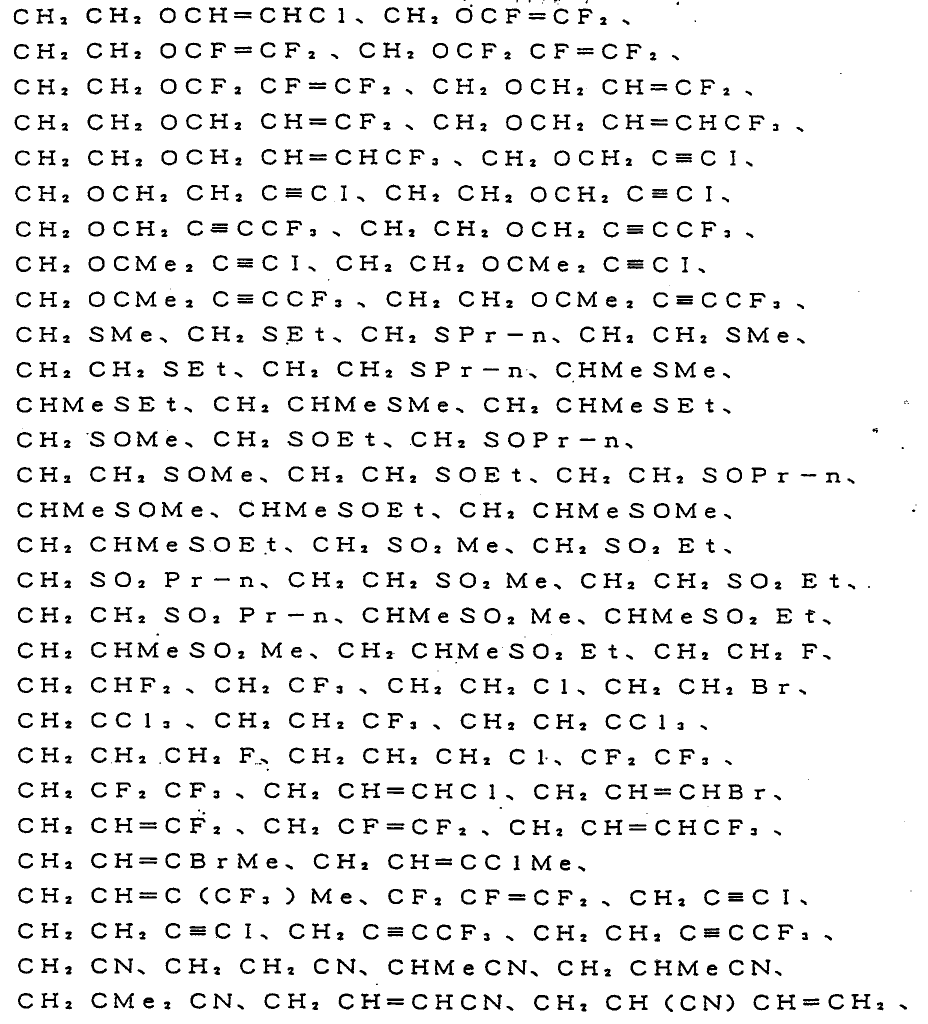 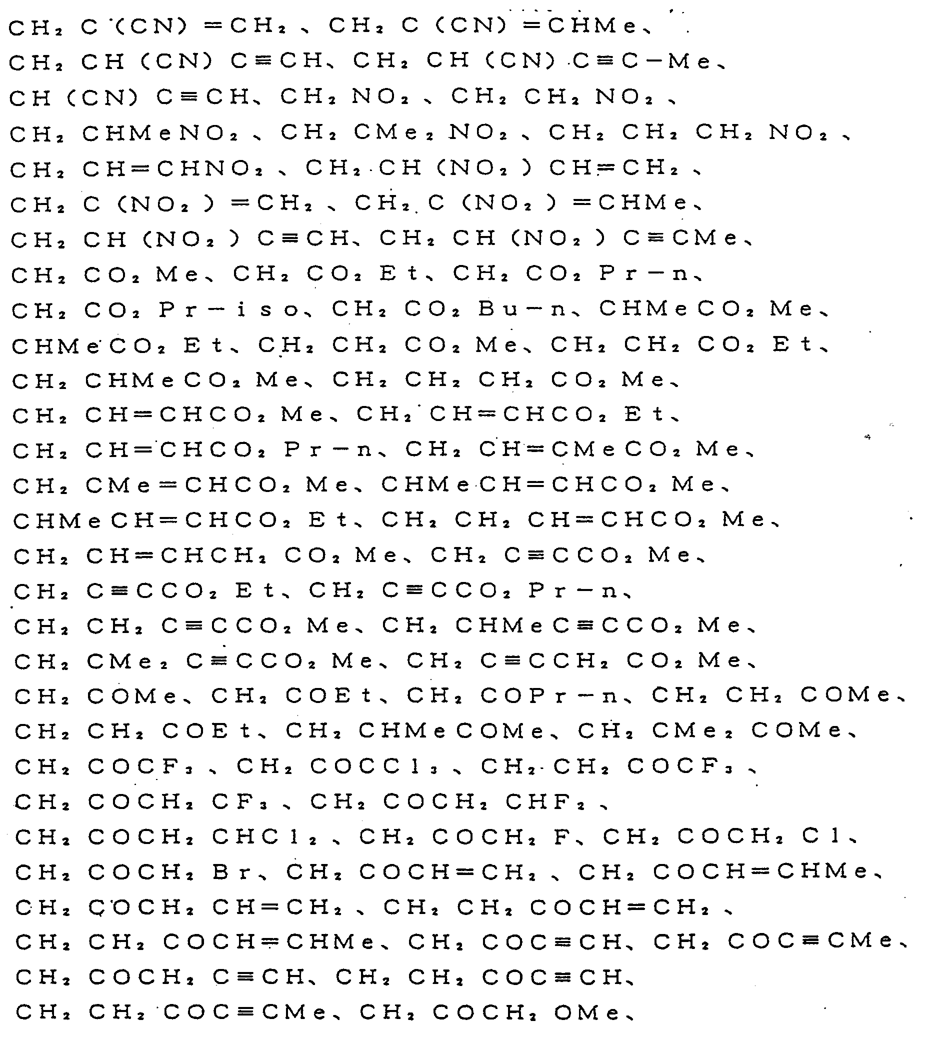 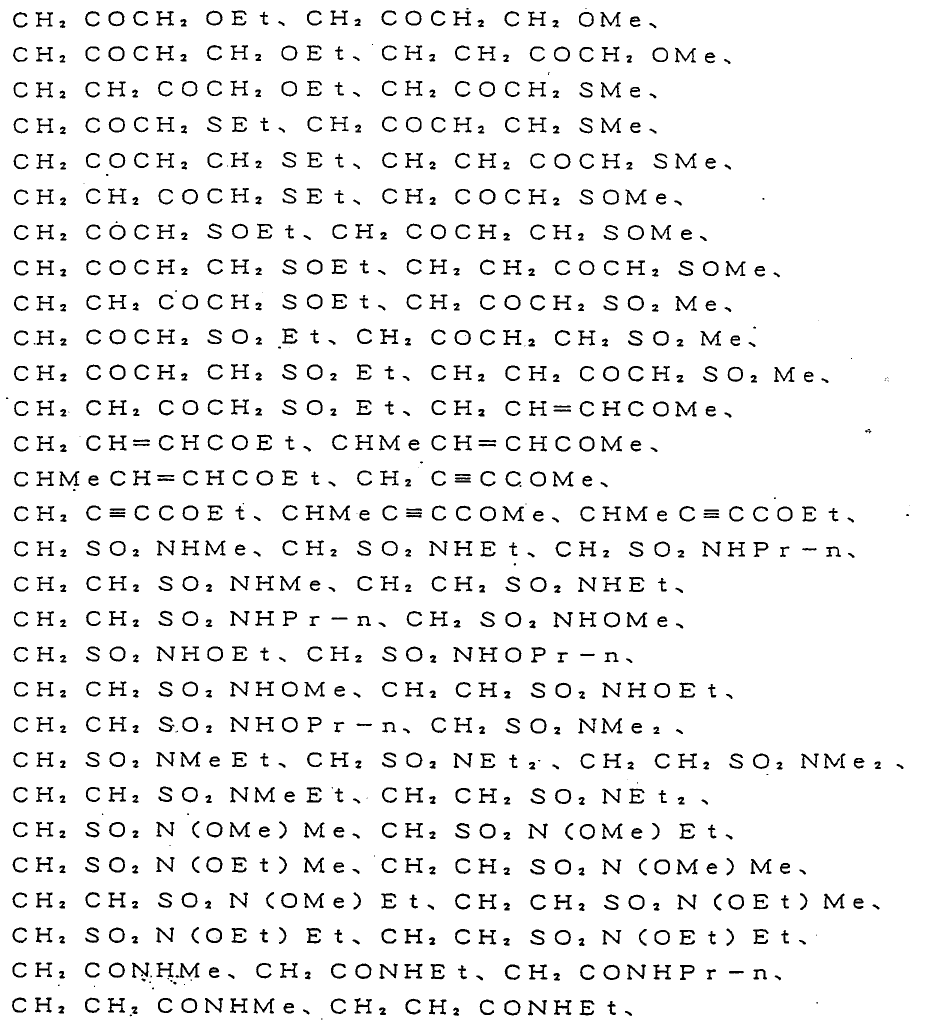 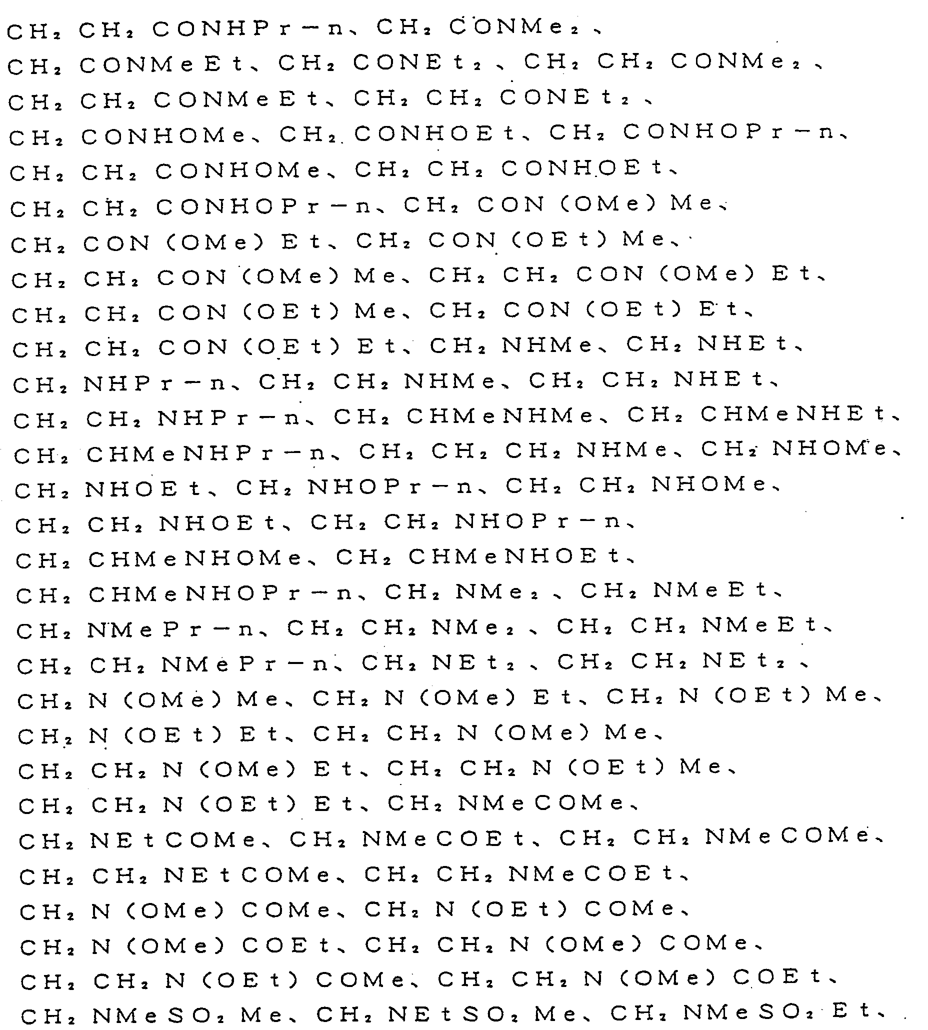 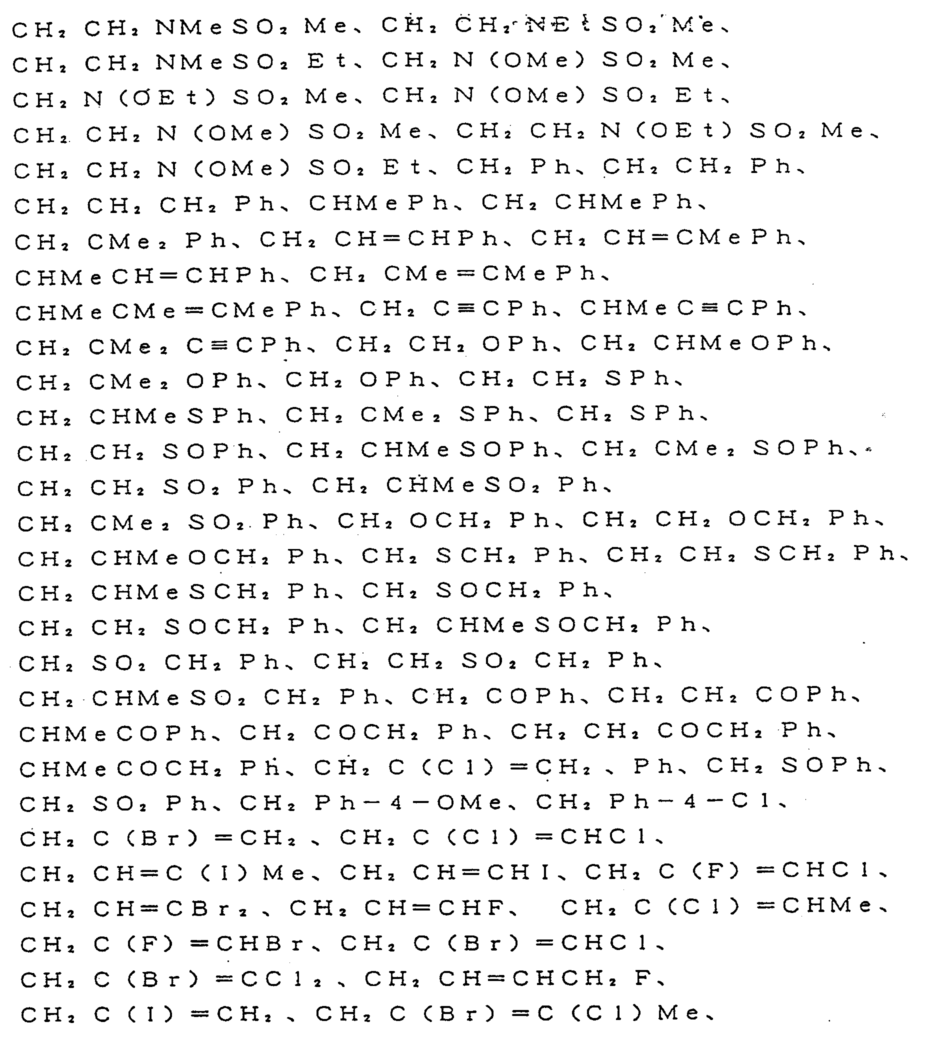 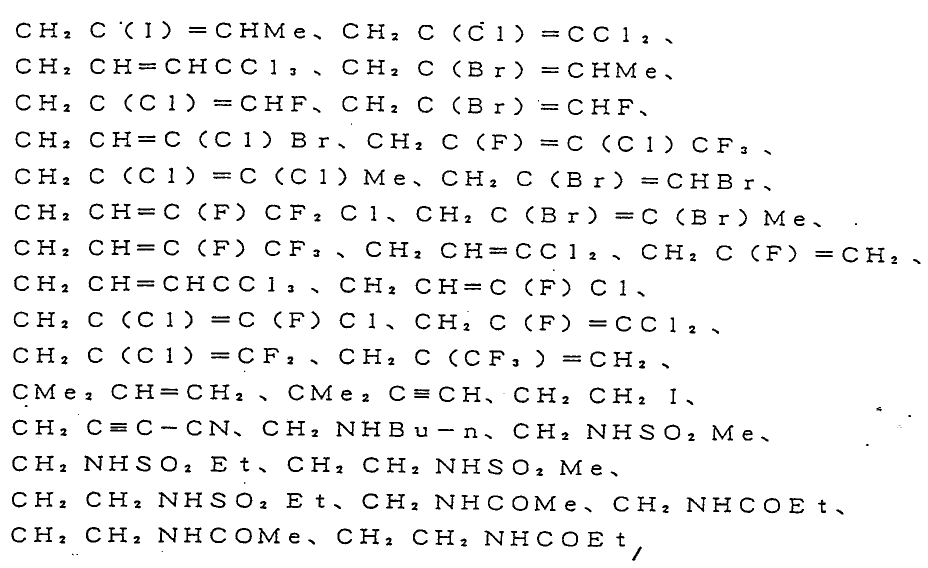 steht,
R^{a2} und R^{a3} für 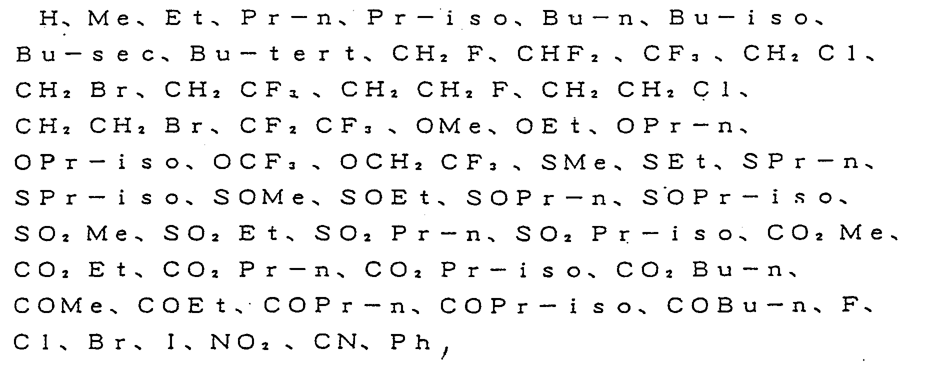 stehen,
B und D für 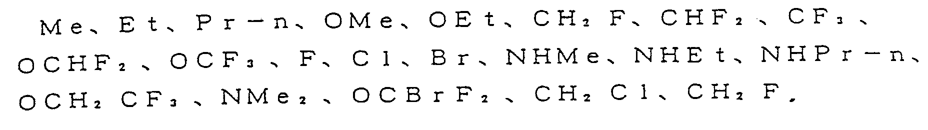 stehen.

29. Iminosulfonyl-Harnstoffderivat oder dessen Salz nach Anspruch 3, wobei R^{a1} für 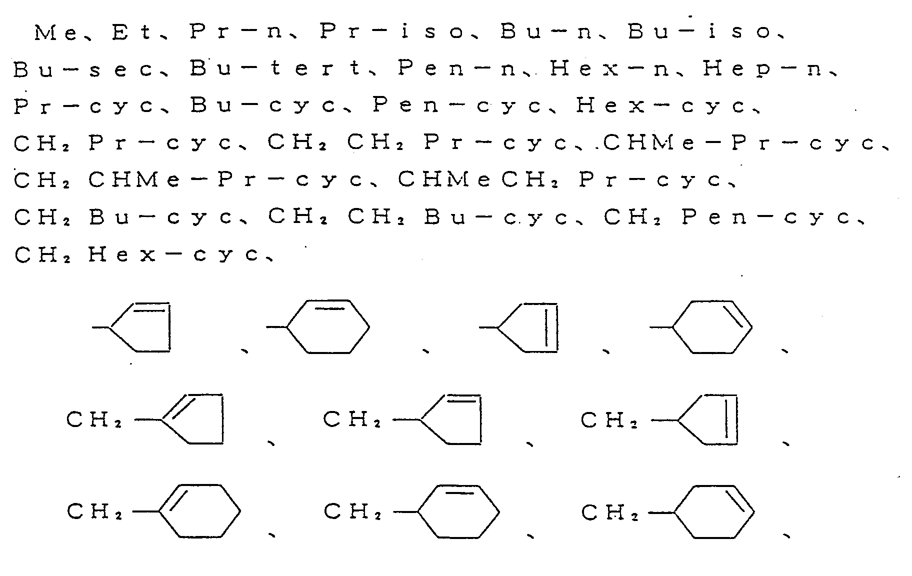 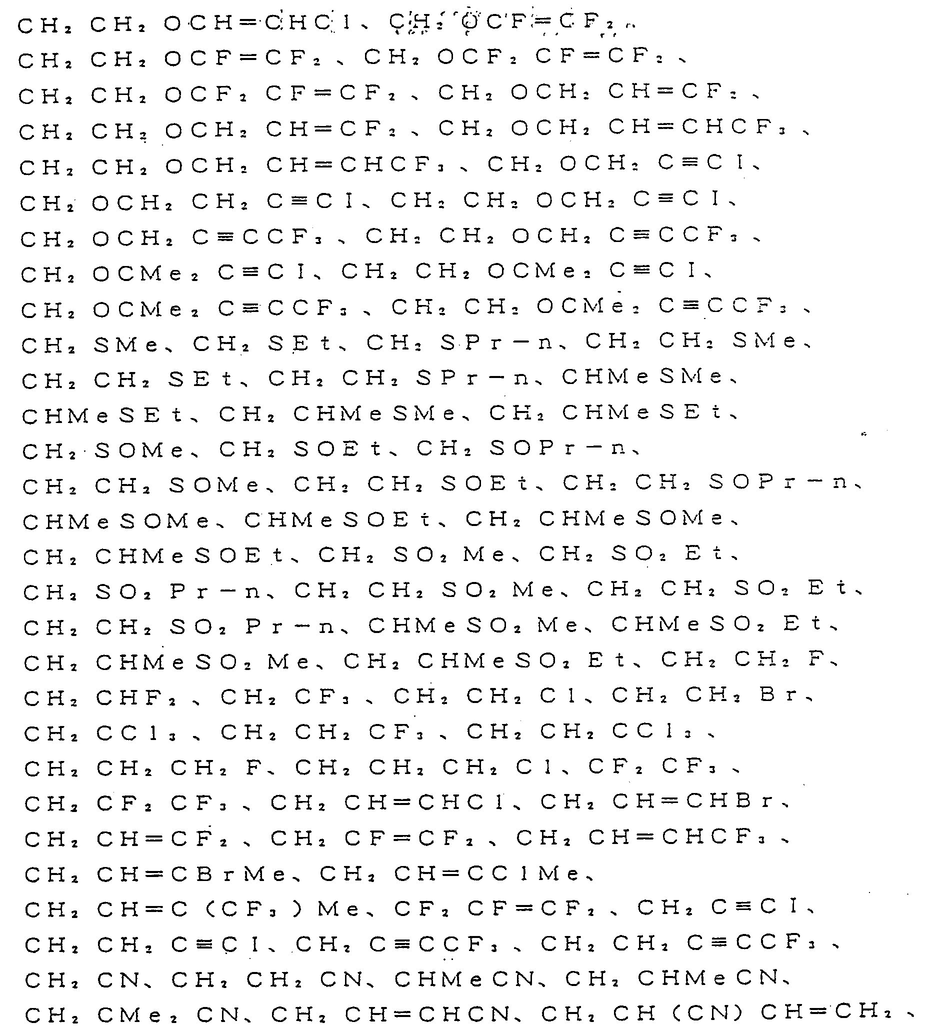 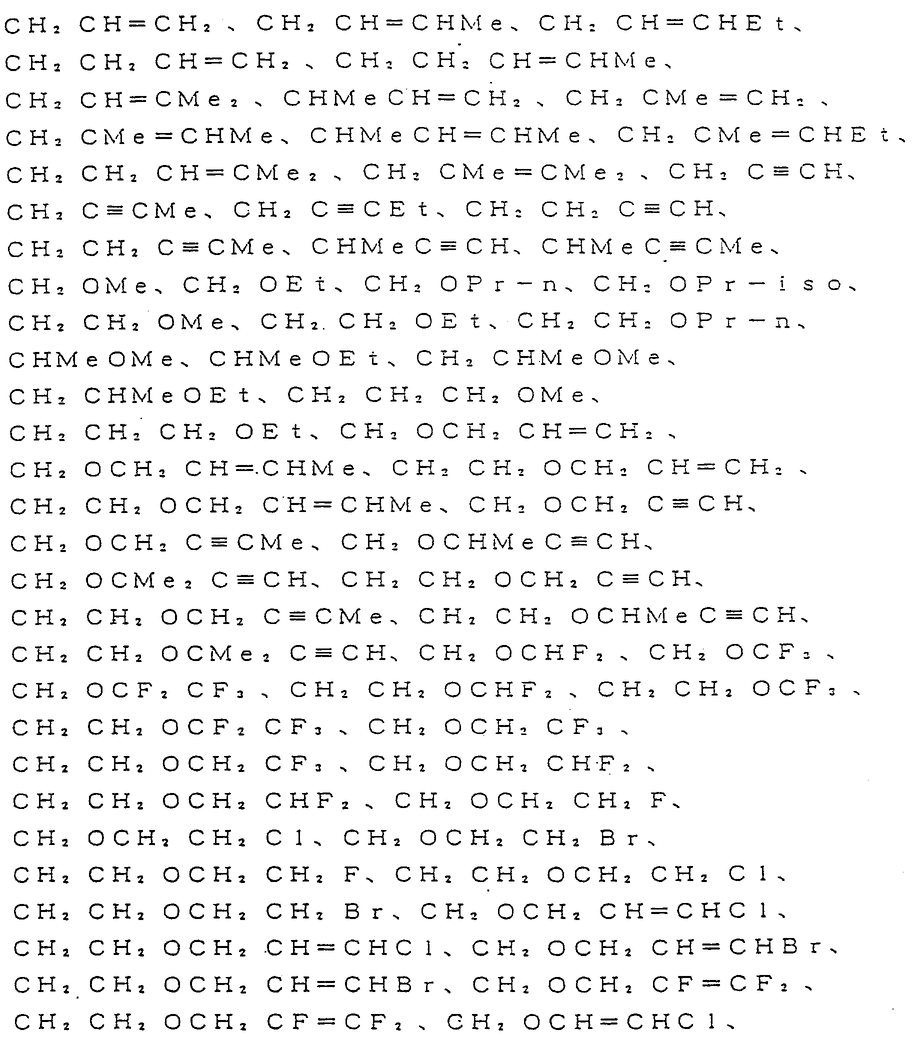 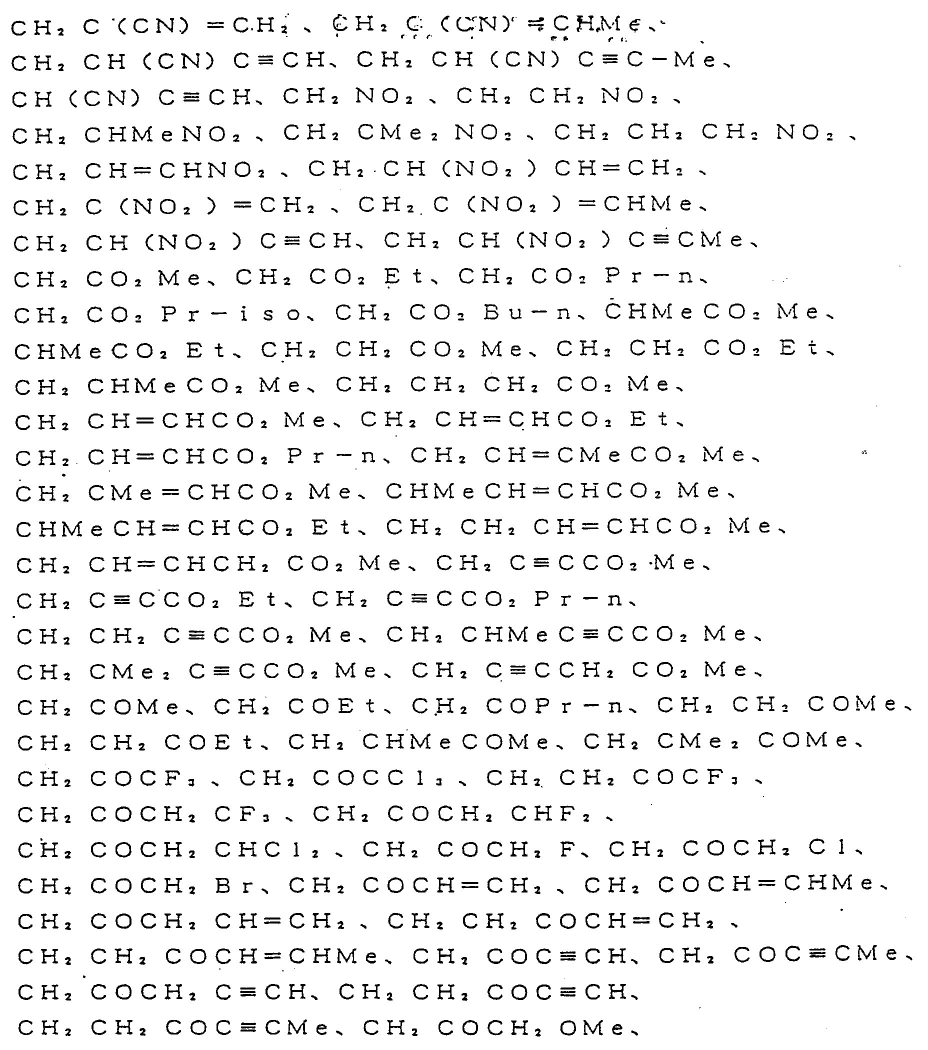 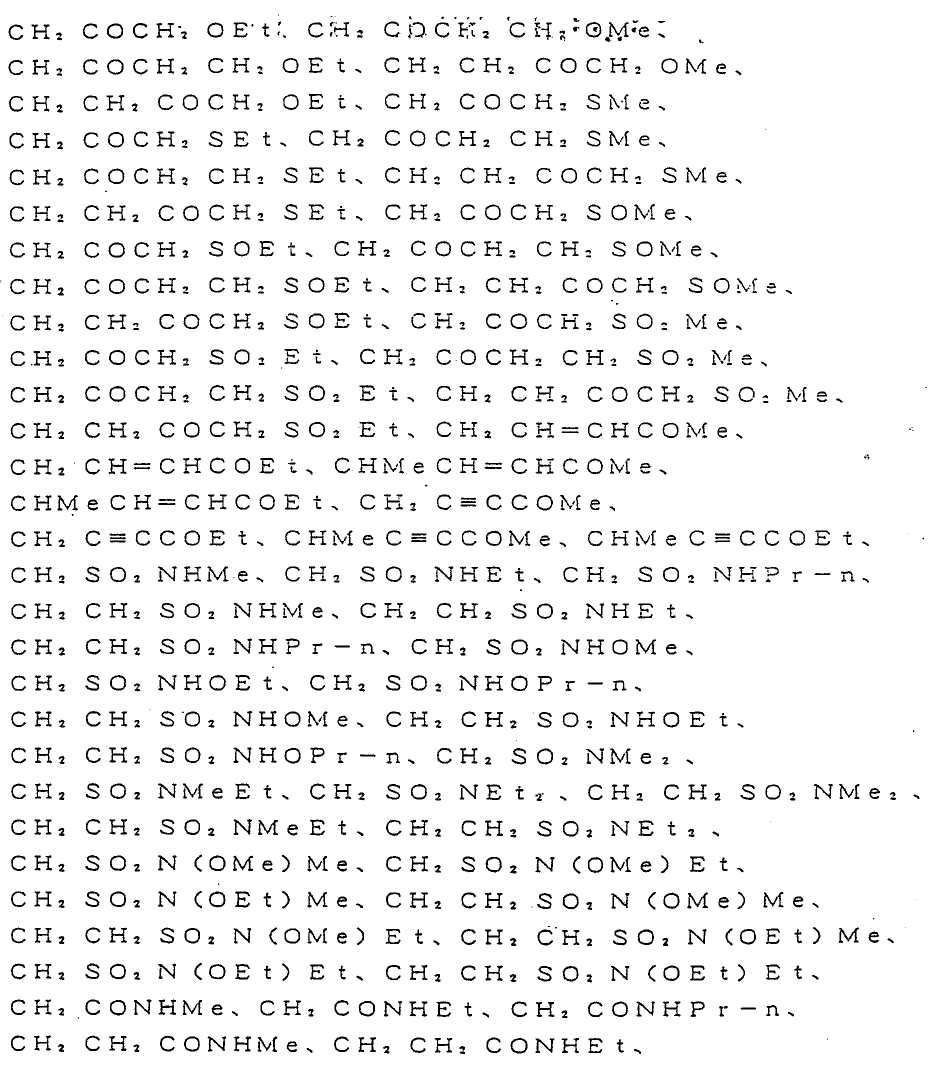 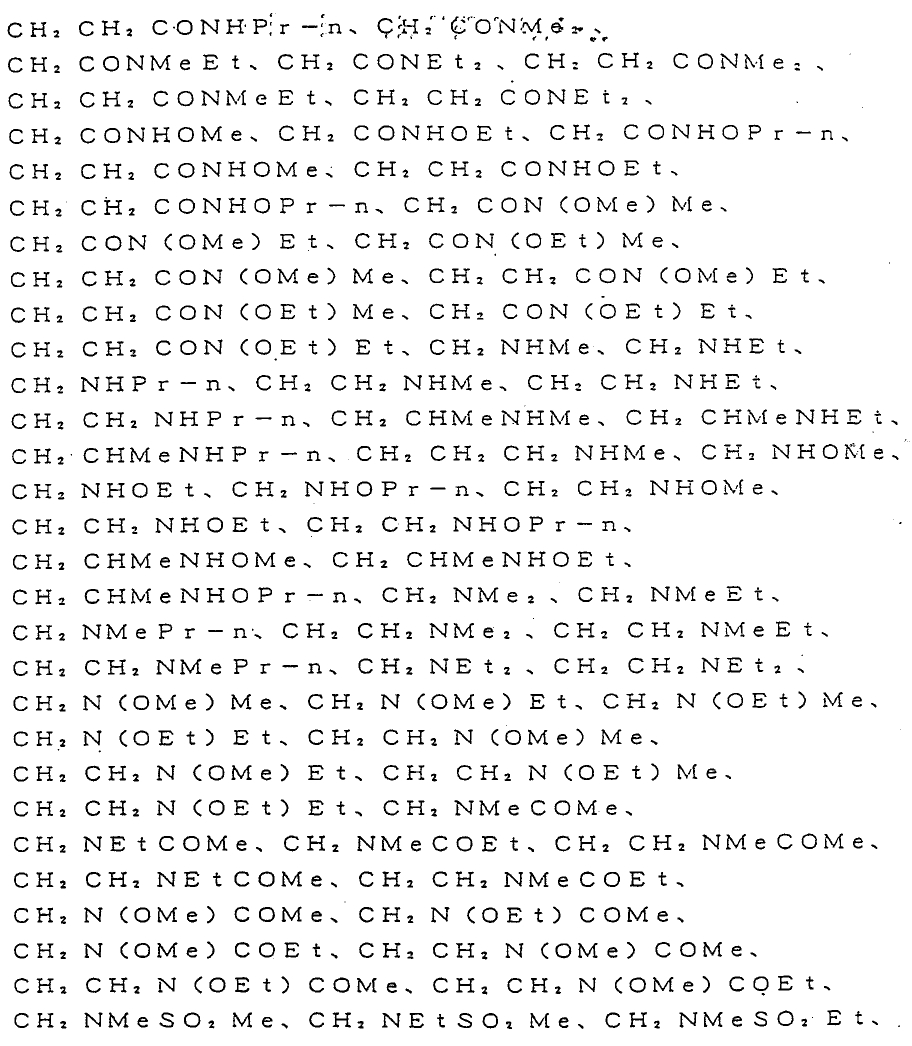 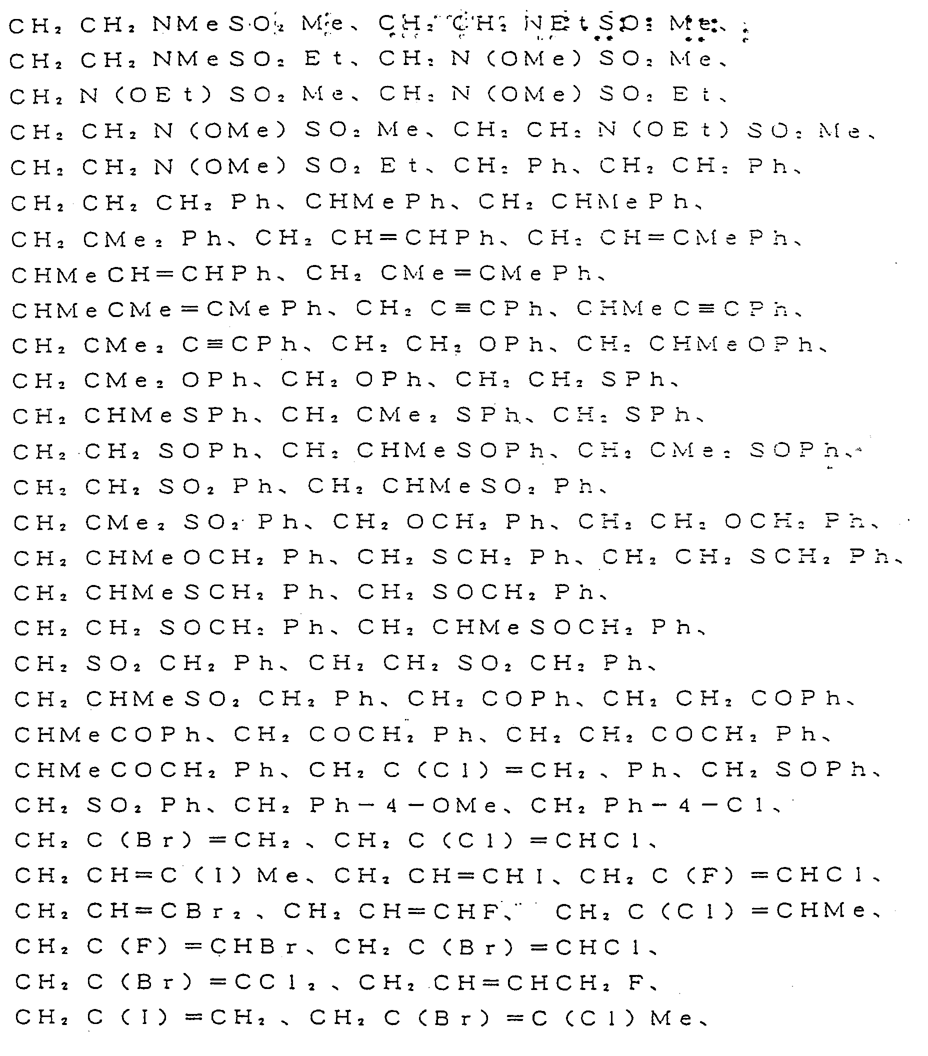 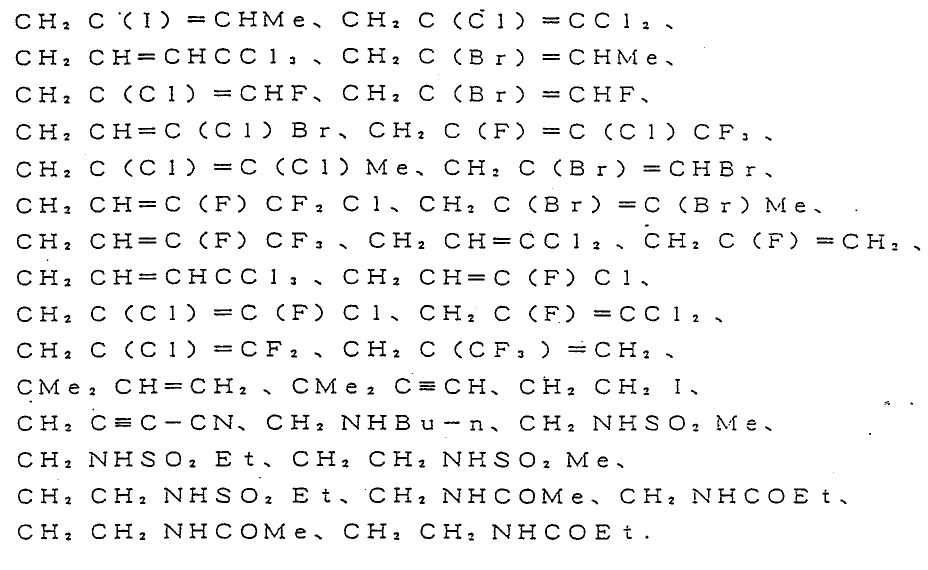 steht.

30. Iminosulfonyl-Harnstoffderivat oder dessen Salz nach Anspruch 3, wobei R^{a2} und R^{a3} für 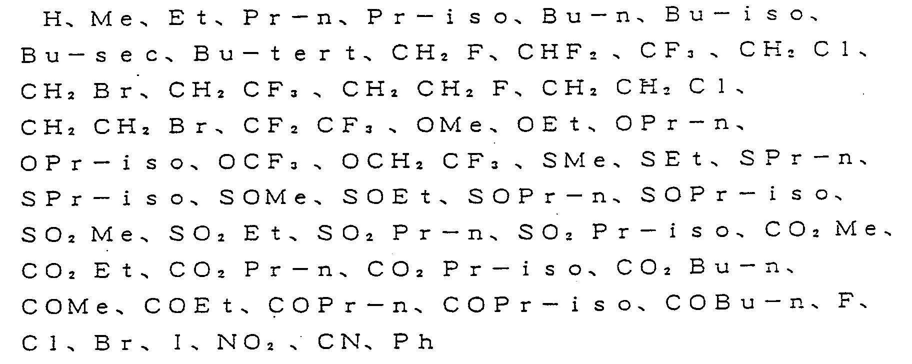 stehen.

31. Iminosulfonyl-Harnstoffderivat oder dessen Salz nach Anspruch 3, wobei B und D für 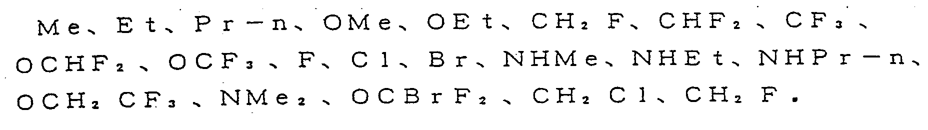 stehen.

32. Herbizid, das ein Iminosulfonyl-Harnstoffderivat, wie es in Anspruch 28 definiert wird, enthält.

33. Herbizid, das ein Iminosulfonyl-Harnstoffderivat, wie es in Anspruch 29 definiert wird, enthält.

34. Herbizid, das ein Iminosulfonyl-Harnstoffderivat, wie es in Anspruch 30 definiert wird, enthält.

35. Herbizid, das ein Iminosulfonyl-Harnstoffderivat, wie es in Anspruch 31 definiert wird, enthält.

36. Herbizides und wachstumsbekämpfendes Verfahren gegen Unkräuter, das eine Anwendung einer herbizidwirksamen Menge eines Iminosulfonyl-Harnstoffderivats, wie es in Anspruch 28 definiert wird, umfaßt.

37. Herbizides und wachstumsbekämpfendes Verfahren gegen Unkräuter, das eine Anwendung einer herbizidwirksamen Menge eines Iminosulfonyl-Harnstoffderivats, wie es in Anspruch 29 definiert wird, umfaßt.

38. Herbizides und wachstumsbekämpfendes Verfahren gegen Unkräuter, das eine Anwendung einer herbizidwirksamen Menge eines Iminosulfonyl-Harnstoffderivats, wie es in Anspruch 30 definiert wird, umfaßt.

39. Herbizides und wachstumsbekämpfendes Verfahren gegen Unkräuter, das eine Anwendung einer herbizidwirksamen Menge eines Iminosulfonyl-Harnstoffderivats, wie es in Anspruch 31 definiert wird, umfaßt.

## Patentansprüche (Patentansprüche für folgende(n) Vertragsstaat(en): ES)

1. Verfahren zur Herstellung eines Iminosulfonyl-Harnstoffderivats der Formel (1) oder dessen Salz: 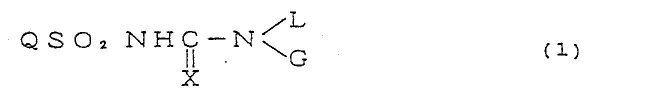
wobei Q für 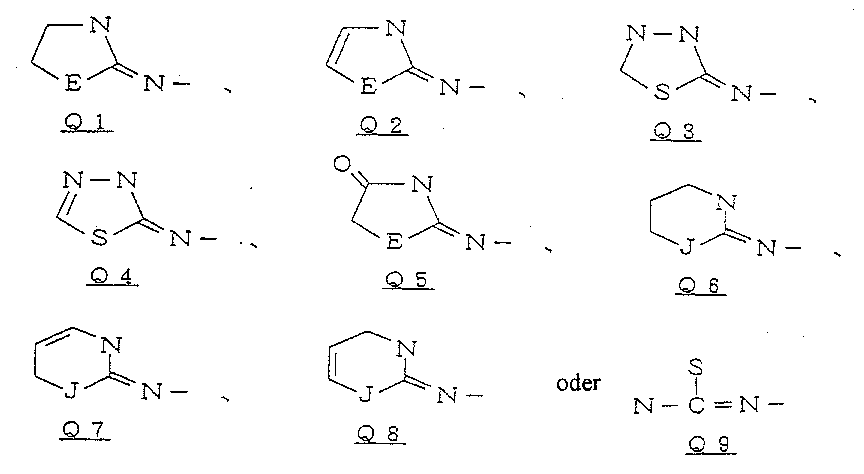 steht, wobei in Q1, Q2 und Q5 das E für ein Schwefelatom, ein Sauerstoffatom oder ein Stickstoffatom steht, das monosubstituiert ist, mit einem Substituenten, der nicht Wasserstoffatom ist; in Q6, Q7 und Q8 das J für ein Schwefelatom oder ein Sauerstoffatom steht; in Q1 bis Q8 ein Stickstoffatom im Ring von Q substituiert ist mit einem Substituenten, der kein Wasserstoffatom ist, und ein Kohlenstoff im Ring von Q gegebenenfalls substituiert ist; und in Q9 das Schwefelatom und das Stickstoffatom an dem Kohlenstoffatom, an dem die Iminogruppe von Q gebunden ist, mit Substituenten, die keine Wasserstoffatome sind, substituiert sind,
X für ein Sauerstoffatom oder für ein Schwefelatom steht,
L für ein Wasserstoffatom, eine C₁₋₆-Alkylgruppe, eine C₂₋₆-Alkenylgruppe oder eine C₂₋₆-Alkinylgruppe steht,
G für 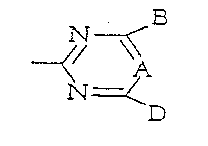 steht,
A für eine CH-Gruppe oder ein Stickstoffatom steht, und
jedes B und D, die unabhängig voneinander sind, jeweils für eine C₁₋₄-Alkylgruppe, eine C₁₋₄-Alkoxygruppe, eine Mono-, Di- oder Polyhalogen-C₁₋₄-Alkylgruppe, eine Mono-, Di- oder Polyhalogen-C₋₁₋₄-Alkoxygruppe, ein Halogenatom, eine C₋₁₋₄-Alkylaminogruppe oder eine Di(C₁₋₄-alkyl)aminogruppe steht,
wobei das Verfahren umfaßt:
a) Umsetzung eines Amins der Formel (2): 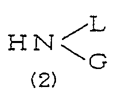 wobei G und L wie oben definiert sind, mit Chlorsulfonylisocyanat in einem Lösungsmittel und anschließend Umsetzung des Produkts mit einem Imin der Formel (3) oder (4):
Q-H (3)
oder
Q-H · HZ (4)
wobei Q wie oben definiert ist und Z für ein Halogenatom steht, in Gegenwart einer Base, um eine Verbindung der Formel (1) zu schaffen: 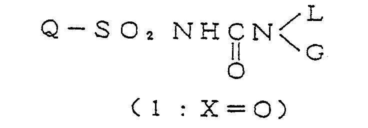 wobei Q, L und G wie oben definiert sind, und X für ein Sauerstoffatom steht; oder
b) Umsetzung eines Imins der Formel (3) oder (4):
Q-H (3)
oder
Q-H · HZ (4)
wobei Q und Z wie oben definiert sind, mit einem N-Chlorsulfonylcarbamat der Formel (5):
CℓSO₂ NHCO₂ Y (5)
wobei Y für eine C₁₋₆-Alkylgruppe oder eine Phenylgruppe steht, in einer Menge von 0,5 bis 3,0 Mol pro Mol des Imins (3) oder (4) in Gegenwart einer Base und eines Lösungsmittels, um eine Verbindung der Formel (6) zu schaffen: 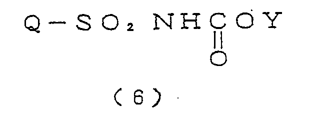 wobei Q und Y wie oben definiert sind, welche dann erhitzt wird mit einer Verbindung der Formel (2): 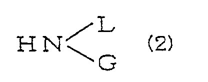 wobei L und G wie oben definiert sind, in einem Lösungsmittel, um eine Verbindung der Formel (1) zu schaffen: 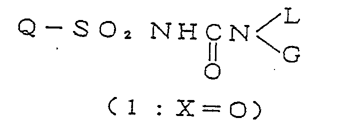 wobei Q, L und G wie oben definiert sind, und X für ein Sauerstoffatom steht; oder
c) Umsetzung eines substituierten Iminosulfonamidderivats der Formel (7):
Q-SO₂ NH₂ (7)
wobei Q wie oben definiert ist, mit einem Carbamatderivat der Formel (8): 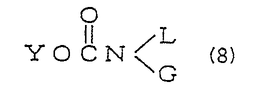 wobei Y, L und G wie oben definiert sind in einem Lösungsmittel in Gegenwart einer anorganischen und organischen Base, um eine Verbindung der Formel (1) zu schaffen: 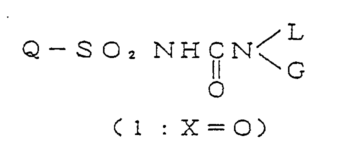 wobei Q, L und G wie oben definiert sind, und X für ein Sauerstoffatom steht; oder
d) Umsetzung eine substituierten Iminosulfonamidderivats der Formel (7):
Q-SO₂ NH₂ (7)
wobei Q wie oben definiert ist, mit einem Chlorameisensäure(thio)ester oder Kohlensäure(thio)ester in einem Lösungsmittel in Gegenwart einer Base, um eine Verbindung der Formel (6) zu schaffen: 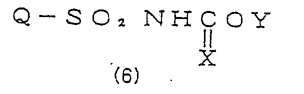 wobei Q, X und Y wie oben definiert sind, welche dann erhitzt wird zusammen mit einer Verbindung der Formel (2): 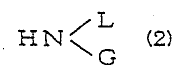 wobei L und G wie oben definiert sind, in einem Lösungsmittel, um eine Verbindung der Formel (1) zu schaffen: 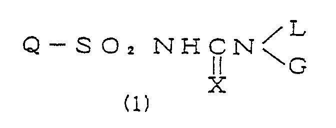 wobei Q, L, G und X wie oben definiert sind; oder
e) Umsetzung eines substituierten Iminosulfonamidderivats der Formel (7):
Q-SO₂ NH₂ (7)
wobei Q wie oben definiert ist, mit einem Isothiocyanatderivat der Formel (9):
S=C=N-G (9)
wobei G wie oben definiert ist, in einem Lösungsmittel in Gegenwart einer anorganischen oder organischen Base, um eine Verbindung der Formel (1) zu schaffen: 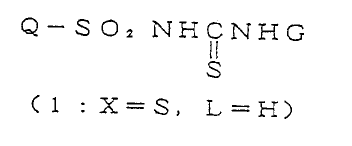 wobei Q und G wie oben definiert sind, und X für Schwefel steht und L für Wasserstoff steht.

2. Verfahren zur Herstellung eines Iminosulfonyl-Harnstoffderivats der Formel (1) oder dessen Salz nach Anspruch 1: 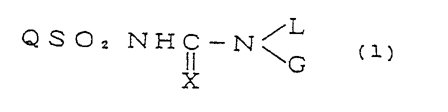
wobei Q für 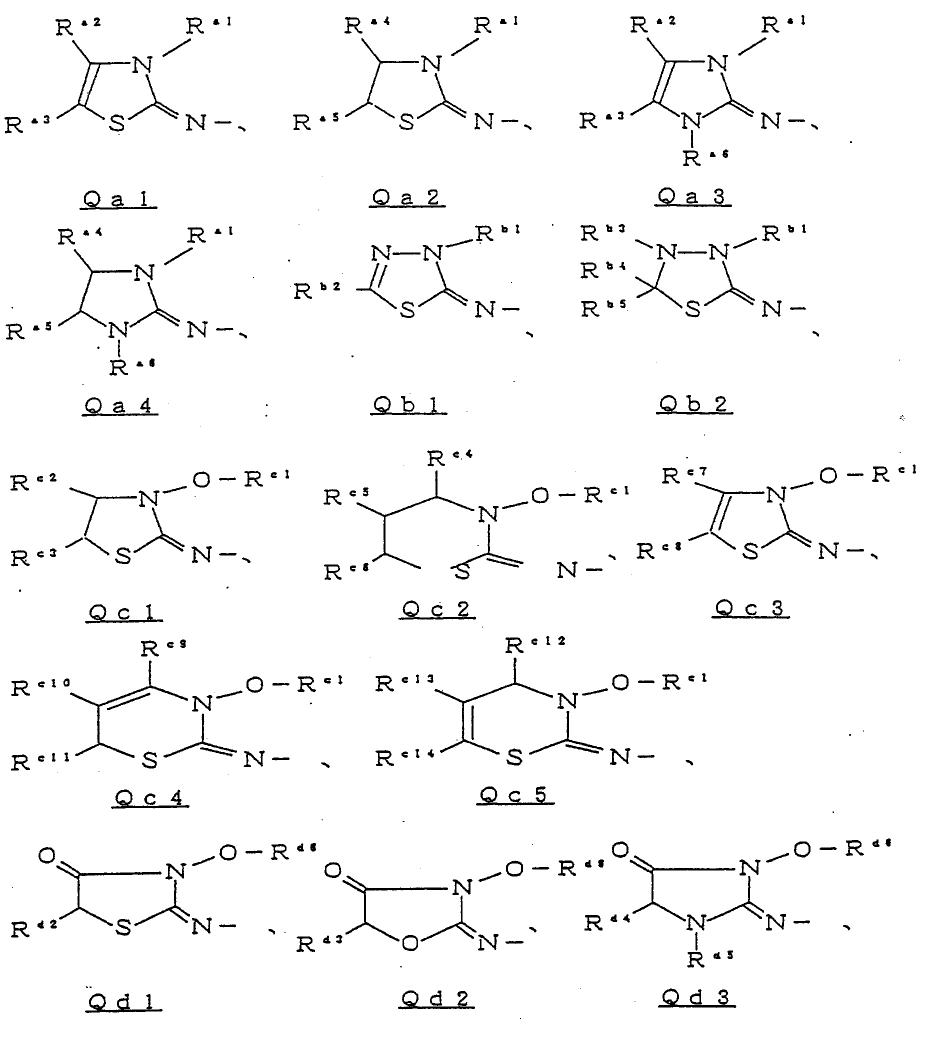 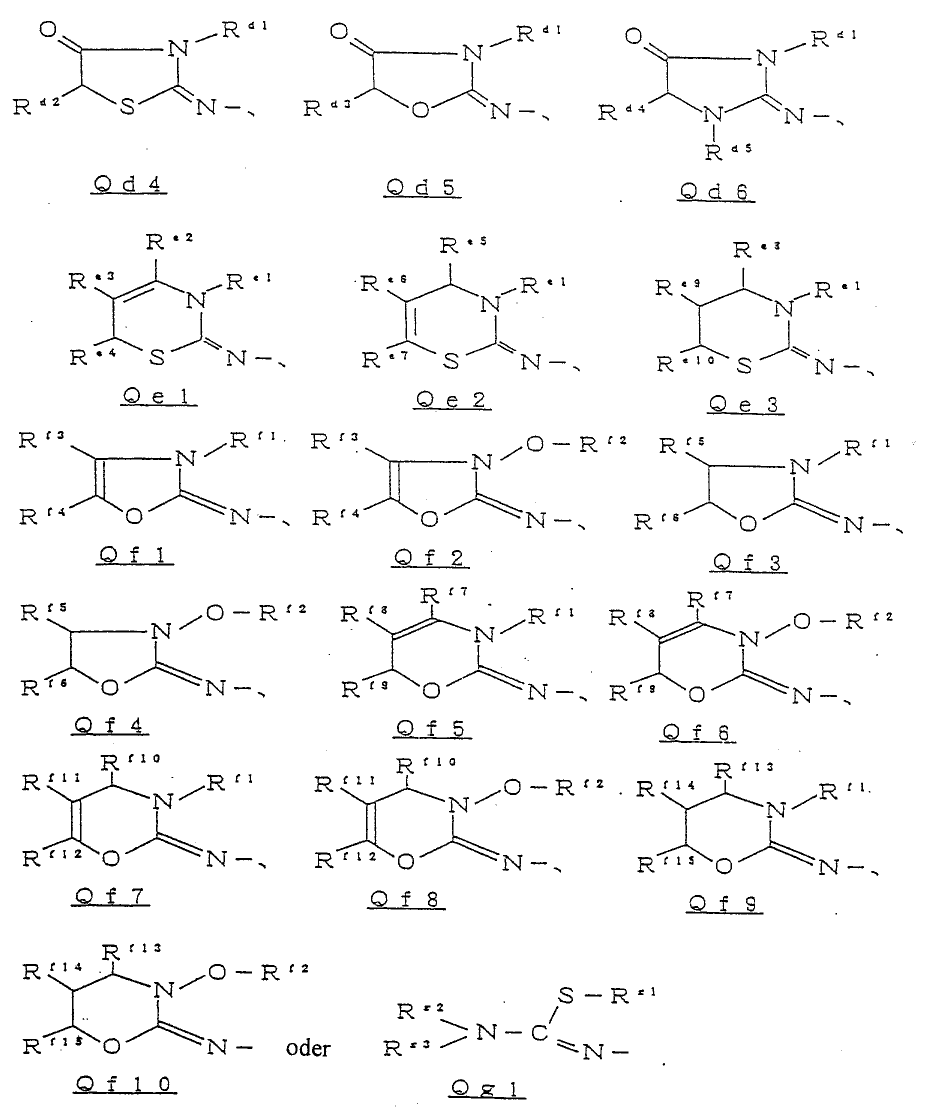
steht, R^{a1} für eine C₁₋₈-Alkylgruppe, eine C₃₋₇-Cycloalkylgruppe, eine mit einer C₃₋₇-Cycloalkylgruppe substituierte C₁₋₆-Alkylgruppe, eine C₃₋₇-Cycloalkenylgruppe, eine mit einer C₃₋₇-Cycloalkenylgruppe substituierte C₁₋₆-Alkylgruppe, eine C₂₋₈-Alkenylgruppe, eine C₂₋₈-Alkinylgruppe, eine mit einer C₁₋₆-Alkoxygruppe substituierte C₁₋₆-Alkylgruppe, eine mit einer C₂₋₆-Alkenyloxygruppe substituierte C₁₋₆-Alkylgruppe, eine mit einer C₂₋₆-Alkinyloxygruppe substituierte C₁₋₆-Alkylgruppe, eine mit einer Mono-, Di- oder Polyhalogen-C₁₋₆-alkoxygruppe substituierte C₁₋₆-Alkylgruppe, eine mit einer Mono-, Di- oder Polyhalogen-C₂₋₆-alkenyloxygruppe substituierte C₁₋₆-Alkylgruppe, eine mit einer Mono-, Di- und Polyhalogen-C₂₋₆-Alkinyloxygruppe substituierte C₁₋₆-Alkylgruppe, eine mit einer C₁₋₆-Alkylthiogruppe substituierte C₁₋₆-Alkylgruppe, eine mit einer C₁₋₆-Alkylsulfinylgruppe substituierte C₁₋₆-Alkylgruppe, eine mit einer C₁₋₆-Alkylsulfonylgruppe substituierte C₁₋₆-Alkylgruppe, eine Mono-, Di- oder Polyhalogen-C₁₋₈-Alkylgruppe, eine Mono-, Di- oder Polyhalogen-C₂₋₈-Alkenylgruppe, eine Mono-, Di- oder Polyhalogen-C₂₋₈-Alkinylgruppe, eine mit einer Cyanogruppe substituierte C₁₋₆-Alkylgruppe, eine mit einer Cyanogruppe substituierte C₂₋₆-Alkenylgruppe, eine mit einer Cyanogruppe substituierte C₂₋₆-Alkinylgruppe, eine mit einer Nitrogruppe substituierte C₁₋₆-Alkylgruppe, eine mit einer Nitrogruppe substituierte C₂₋₆-Alkenylgruppe, eine mit einer Nitrogruppe substituierte C₂₋₆-Alkinylgruppe, eine mit einer C₂₋₇-Alkoxycarbonylgruppe substituierte C₁₋₆-Alkylgruppe, einer mit einer C₂₋₆-Alkoxycarbonylgruppe substituierte C₂₋₆-Alkenylgruppe, eine mit einer C₂₋₇-Alkoxycarbonylgruppe substituierte C₂₋₇-Alkinylgruppe, eine mit einer C₂₋₇-Alkylcarbonylgruppe substituierte C₁₋₆-Alkylgruppe, eine mit einer Mono-, Di- oder Polyhalogen-C₂₋₇-alkylcarbonylgruppe substituierte C₁₋₆-Alkylgruppe, eine mit einer C₃₋₇-Alkenylcarbonylgruppe substituierte C₁₋₆-Alkylgruppe, eine mit einer C₃₋₇-Alkinylcarbonylgruppe substituierte C₁₋₆-Alkylgruppe, eine mit einer C₂₋₅-Alkylcarbonylgruppe, die mit einer C₁₋₄-Alkoxygruppe substituiert ist, substituierte C₁₋₆-Alkylgruppe, eine mit einer C₂₋₅-Alkylcarbonylgruppe, die mit einer C₁₋₄-Alkylthiogruppe substituiert ist, substituierte C₁₋₆-Alkylgruppe, eine mit einer C₂₋₅-Alkylcarbonylgruppe, die mit einer C₁₋₄-Alkylsulfinylgruppe substituiert ist, substituierte C₁₋₆-Alkylgruppe, eine mit einer C₂₋₅-Alkylcarbonylgruppe, die mit einer C₁₋₄-Alkylsulfonylgruppe substituiert ist, substituierte C₁₋₆-Alkylgruppe, eine mit einer C₂₋₇-Alkylcarbonylgruppe substituierte C₂₋₆-Alkenylgruppe, eine mit einer C₂₋₇-Alkylcarbonylgruppe substituierte C₂₋₆-Alkinylgruppe, eine mit einer C₁₋₆-Alkylsulfamoylgruppe substituierte C₁₋₆-Alkylgruppe, eine mit einer C₁₋₆-Alkoxysulfamoylgruppe substituierte C₁₋₆-Alkylgruppe, eine mit einer Di(C₁₋₃-alkyl)sulfamoylgruppe substituierte C₁₋₆-Alkylgruppe, eine mit einer N-(C₁₋₃-alkyl)-N-(C₁₋₃-alkoxy)sulfamoylgruppe substituierte C₁₋₆₋Alkylgruppe, eine mit einer C₂₋₇-Alkylcarbamoylgruppe substituierte C₁₋₆-Alkylgruppe, eine mit einer Di(C₁₋₃-alkyl)carbamoylgruppe substituierte C₁₋₆-Alkylgruppe, eine mit einer C₂₋₇-Alkoxycarbamoylgruppe substituierte C₁₋₆-Alkylgruppe, eine mit einer N-(C₁₋₃-alkyl)-N-(C₁₋₃-alkoxy)carbamoylgruppe substiuierte C₁₋₆-Alkylgruppe, eine mit einer C₁₋₆-Alkylaminogruppe substituierte C₁₋₆-Alkylgruppe, eine mit einer C₁₋₆-Alkoxyaminogruppe substituierte C₁₋₆-Alkylgruppe, eine mit einer Di(C₁₋₃-alkyl)aminogruppe substituierte C₁₋₆-Alkylgruppe, eine mit einer N-(C₁₋₃-alkyl)-N-(C₁₋₃-alkoxy)aminogruppe substituierte C₁₋₆-Alkylgruppe, eine mit einer N-(C₂₋₇-alkylcarbonyl)-N-(C₁₋₆-alkyl)aminogruppe substituierte C₁₋₆-Alkylgruppe, eine mit einer N-(C₂₋₇-Alkylcarbonyl)-N-(C₁₋₆-alkoxy)aminogruppe substituierte C₁₋₆-Alkylgruppe, eine mit einer N-(C₁₋₆-Alkylsulfonyl)-N-(C₁₋₆-alkyl)aminogruppe substituierte C₁₋₆-Alkylgruppe, eine mit einer N-(C₁₋₆-Alkylsulfonyl)-N-(C₁₋₆-alkoxy)aminogruppe substituierte C₁₋₆-Alkylgruppe, eine Phenylgruppe (mit der Maßgabe, daß eine solche Phenylgruppe substituiert sein kann mit einem oder mehreren Substituenten, die ausgewählt sind aus der Gruppe, bestehend aus einem Halogenatom, einer Trifluormethylgruppe, einer Nitrogruppe, einer C₁₋₆-Alkylgruppe, einer C₁₋₆-Alkoxygruppe und einer C₂₋₇-Alkoxycarbonylgruppe), eine mit einer Phenylgruppe substituierte C₁₋₆-Alkylgruppe (mit der Maßgabe, daß eine solche Phenylgruppe substituiert sein kann mit einem oder mehreren Substituenten, die ausgewählt sind aus der Gruppe, bestehend aus einem Halogenatom, einer Trifluormethylgruppe, einer Nitrogruppe, einer C₁₋₆-Alkylgruppe, einer C₁₋₆-Alkoxygruppe und einer C₂₋₇-Alkoxycarbonylgruppe), eine mit einer Phenylgruppe substituierte C₂₋₇-Alkenylgruppe (mit der Maßgabe, daß eine solche Phenylgruppe substituierte sein kann mit einem oder mehreren Substituenten, die ausgewählt sind aus der Gruppe, bestehend aus einem Halogenatom, eine Trifluormethylgruppe, einer Nitrogruppe, einer C₁₋₆-Alkylgruppe, einer C₁₋₆-Alkoxygruppe und einer C₂₋₇-Alkoxycarbonylgruppe), eine mit einer Phenylgruppe substituierte C₂₋₆-Alkinylgruppe (mit der Maßgabe, daß eine solche Phenylgruppe substituiert werden kann mit einem oder mehreren Substituenten, die ausgewählt sind aus der Gruppe, bestehend aus einem Halogenatom, einer Trifluormethylgruppe, einer Nitrogruppe, einer C₁₋₆-Alkylgruppe, einer C₁₋₆-Alkoxygruppe und einer C₂₋₇-Alkoxycarbonylgruppe), eine mit einer Phenoxygruppe substituierte C₁₋₆-Alkylgruppe (mit der Maßgabe, daß eine solche Phenoxygruppe substituiert sein kann mit einer oder mehreren Substituenten, die ausgewählt sind aus der Gruppe, bestehend aus einem Halogenatom, einer Trifluormethylgruppe, einer Nitrogruppe, einer C₁₋₆-Alkylgruppe, einer C₁₋₆-Alkoxygruppe und einer C₂₋₇-Alkoxycarbonylgruppe), einer mit einer Phenylthiogruppe substituierte C₁₋₆-Alkylgruppe (mit der Maßgabe, daß eine solche Phenylthiogruppe substituiert sein kann mit einem oder mehreren Substituenten, die ausgewählt sind aus der Gruppe, bestehend aus einem Halogenatom, einer Trifluormethylgruppe, einer Nitrogruppe, einer C₁₋₆-Alkylgruppe, einer C₁₋₆-Alkoxygruppe und einer C₂₋₇-Alkoxycarbonylgruppe), eine mit einer Phenylsulfinylgruppe substituierte C₁₋₆-Alkylgruppe (mit der Maßgabe, daß eine solche Phenylsulfinylgruppe substituiert sein kann mit einem oder mehreren Substituenten, die ausgewählt sind aus der Gruppe, bestehend aus einem Halogenatom, einer Trifluormethylgruppe, einer Nitrogruppe, einer C₁₋₆-Alkylgruppe, einer C₁₋₆-Alkoxygruppe und einer C₂₋₇-Alkoxycarbonylgruppe), eine mit einer Phenylsulfonylgruppe substituierte C₁₋₆-Alkylgruppe (mit der Maßgabe, daß eine solche Phenylsulfonylgruppe substituiert sein kann mit einem oder mehreren Substituenten, die ausgewählt sind aus der Gruppe, bestehend aus einem Halogenatom, einer Trifluormethylgruppe, einer Nitrogruppe, einer C₁₋₆-Alkylgruppe, einer C₁₋₆-Alkoxygruppe und einer C₂₋₇-Alkoxycarbonylgruppe), eine mit einer Benzyloxygruppe substituierte C₁₋₆-Alkylgruppe (mit der Maßgabe, daß die Phenylgruppe einer solchen Benzyloxygruppe substituiert sein kann mit einem oder mehreren Substituenten, die ausgewählt sind aus der Gruppe, bestehend aus einem Halogenatom, einer Trifluormethylgruppe, einer Nitrogruppe, einer C₁₋₆-Alkylgruppe, einer C₁₋₆-Alkoxygruppe und einer C₂₋₇-Alkoxycarbonylgruppe), eine mit einer Benzylthiogruppe substituierte C₁₋₆-Alkylgruppe (mit der Maßgabe, daß die Phenylgruppe einer solchen Benzylthiogruppe substituiert sein kann mit einem oder mehreren Substituenten, die ausgewählt sind aus der Gruppe, bestehend aus einem Halogenatom, einer Trifluormethylgruppe, einer Nitrogruppe, einer C₁₋₆-Alkylgruppe, einer C₁₋₆-Alkoxygruppe und einer C₂₋₇-Alkoxycarbonylgruppe), eine mit einer Benzylsulfinylgruppe substituierte C₁₋₆-Alkylgruppe (mit der Maßgabe, daß die Phenylgruppe einer solchen Benzylsulfinylgruppe substituiert sein kann mit einem oder mehreren Substituenten, die ausgewählt sind aus der Gruppe, bestehend aus einem Halogenatom, einer Trifluormethylgruppe, einer Nitrogruppe, einer C₁₋₆-Alkylgruppe, einer C₁₋₆-Alkoxygruppe und einer C₂₋₇-Alkoxycarbonylgruppe), eine mit einer Benzylsulfonylgruppe substituierte C₁₋₆-Alkylgruppe (mit der Maßgabe, daß die Phenylgruppe einer solchen Benzylsulfonylgruppe substituiert sein kann mit einem oder mehreren Substituenten, die ausgewählt sind aus der Gruppe, bestehend aus einem Halogenatom, einer Trifluormethylgruppe, einer Nitrogruppe, einer C₁₋₆-Alkylgruppe, einer C₁₋₆-Alkoxygruppe und einer C₂₋₇-Alkoxycarbonylgruppe), eine mit einer Phenylcarbonylgruppe substituierte C₁₋₆-Alkylgruppe (mit der Maßgabe, daß eine solche Phenylcarbonylgruppe substituiert sein kann mit einem oder mehreren Substituenten, die ausgewählt sind aus der Gruppe, bestehend aus einem Halogenatom, einer Trifluormethylgruppe, einer Nitrogruppe, einer C₁₋₆-Alkylgruppe, einer C₁₋₆-Alkoxygruppe und einer C₂₋₇-Alkoxycarbonylgruppe), eine mit einer Benzylcarbonylgruppe substituierte C₁₋₆-Alkylgruppe (mit der Maßgabe, daß die Phenylgruppe einer solchen Benzylcarbonylgruppe substituiert sein kann mit einem oder mehreren Substituenten, die ausgewählt sind aus der Gruppe, bestehend aus einem Halogenatom, einer Trifluormethylgruppe, einer Nitrogruppe, einer C₁₋₆-Alkylgruppe, einer C₁₋₆-Alkoxygruppe und einer C₂₋₇-Alkoxycarbonylgruppe), eine mit einer Aminogruppe, die mit einer C₁₋₄-Alkylsulfonylgruppe substituiert ist, substituierte C₁₋₆-Alkylgruppe, oder eine mit einer Aminogruppe, die mit einer C₂₋₄-Alkylcarbonylgruppe substituiert ist, substituierte C₁₋₆-Alkylgruppe steht,
R^{a2} und R^{a3}, die unabhängig voneinander sind, jeweils für ein Wasserstoffatom, eine C₁₋₆-Alkylgruppe, eine Mono-, Di- oder Polyhalogen-C₁₋₆-alkylgruppe, eine C₁₋₆-Alkoxygruppe, eine Mono-, Di- oder Polyhalogen-C₁₋₆-alkoxygruppe, eine C₁₋₆-Alkylthiogruppe, eine C₁₋₆-Alkylsulfinylgruppe, eine C₁₋₆-Alkylsulfonylgruppe, eine C₂₋₇-Alkoxycarbonylgruppe, eine C₂₋₇-Alkylcarbonylgruppe, ein Halogenatom, eine Nitrogruppe, eine Cyanogruppe oder eine Phenylgruppe (mit der Maßgabe, daß eine solche Phenylgruppe substituiert sein kann mit einem oder mehreren Substituenten, die ausgewählt sind aus der Gruppe, bestehend aus einem Halogenatom, einer Trifluormethylgruppe, einer Nitrogruppe, einer C₁₋₆-Alkylgruppe, einer C₁₋₆-Alkoxygruppe und einer C₂₋₇-Alkoxycarbonylgruppe) steht,
R^{a4} und R^{a5}, die unabhängig voneinander sind, jeweils für ein Wasserstoffatom, eine C₁₋₈-Alkylgruppe, eine C₂₋₈-Alkenylgruppe, eine C₂₋₈-Alkinylgruppe oder eine Phenylgruppe (mit der Maßgabe, daß eine solche Phenylgruppe substituiert sein kann mit einem oder mehreren Substituenten, die ausgewählt sind aus der Gruppe, bestehend aus einem Halogenatom, einer Trifluormethylgruppe, einer Nitrogruppe, einer C₁₋₆-Alkylgruppe, einer C₁₋₆-Alkoxygruppe und einer C₂₋₇-Alkoxycarbonylgruppe) steht,
R^{a6} für eine C₁₋₈-Alkylgruppe, eine C₂₋₈-Alkenylgruppe, eine C₂₋₈-Alkinylgruppe oder eine Phenylgruppe (mit der Maßgabe, daß eine solche Phenylgruppe substituiert sein kann mit einem oder mehreren Substituenten, die ausgewählt sind aus der Gruppe, bestehend aus einem Halogenatom, einer Trifluormethylgruppe, einer Nitrogruppe, einer C₁₋₆-Alkylgruppe, einer C₁₋₆-Alkoxygruppe und einer C₂₋₇-Alkoxycarbonylgruppe) steht,
R^{b1} für eine C₁₋₈-Alkylgruppe, eine C₃₋₇-Cycloalkylgruppe, eine mit einer C₃₋₇-Cycloalkylgruppe substituierte C₁₋₆-Alkylgruppe, eine C₃₋₇-Cycloalkenylgruppe, eine mit einer C₃₋₇-Alkenylgruppe substituierte C₁₋₆-Alkylgruppe, eine C₂₋₈-Alkenylgruppe, eine C₂₋₈-Alkinylgruppe, eine mit einer C₁₋₆-Alkoxygruppe substituierte C₁₋₆-Alkylgruppe, eine mit einer C₂₋₆-Alkenyloxygruppe substituierte C₁₋₆-Alkylgruppe, eine mit einer C₂₋₆-Alkinyloxygruppe substituierte C₁₋₆-Alkylgruppe, eine mit einer Mono-, Di- oder Polyhalogen-C₁₋₆-alkoxygruppe substituierte C₁₋₆-Alkylgruppe, eine mit einer Mono-, Di- oder Polyhalogen-C₂₋₆-Alkinyloxygruppe substituierte C₁₋₆-Alkylgruppe, eine mit einer Mono-, Di- oder Polyhalogen-C₂₋₆-alkinyloxygruppe substituierte C₁₋₆-Alkylgruppe, eine mit einer C₁₋₆-Alkylthiogruppe substituierte C₁₋₆-Alkylgruppe, eine mit einer C₁₋₆-Alkylsulfinylgruppe substituierte C₁₋₆-Alkylgruppe, eine mit einer C₁₋₆-Alkylsulfonylgruppe substituierte C₁₋₆-Alkylgruppe, eine Mono-, Di- oder Polyhalogen-C₁₋₈-Alkylgruppe, eine Mono-, Di- oder Polyhalogen-C₂₋₈-Alkenylgruppe, eine Mono-, Di- oder Polyhalogen-C₂₋₈-alkinylgruppe, eine mit einer Cyanogruppe substituierte C₁₋₆-Alkylgruppe, eine mit einer Cyanogruppe substituierte C₂₋₆-Alkinylgruppe, eine mit einer Cyanogruppe substituierte C₂₋₆-Alkinylgruppe, eine mit einer Nitrogruppe substituierte C₁₋₆-Alkylgruppe, eine mit einer Nitrogruppe substituierte C₂₋₆-Alkenylgruppe, eine mit einer Nitrogruppe substituierte C₂₋₆-Alkinylgruppe, eine mit einer C₂₋₇-Alkoxycarbonylgruppe substituierte C₁₋₆-Alkylgruppe, eine mit einer C₂₋₇-Alkoxycarbonylgruppe substituierte C₂₋₆-Alkenylgruppe, eine mit einer C₂₋₇-Alkoxycarbonylgruppe substituierte C₂₋₆-Alkinylgruppe, eine mit einer C₂₋₇-Alkylcarbonylgruppe substituierte C₁₋₆-Alkylgruppe, eine mit einer Mono-, Di- oder Polyhalogen-C₂₋₇-alkylcarbonylgruppe substituierte C₁₋₆-Alkylgruppe, eine mit einer C₃₋₇-Alkenylcarbonylgruppe substituierte C₁₋₆-Alkylgruppe, eine mit einer C₃₋₇-Alkinylcarbonylgruppe substituierte C₁₋₆-Alkylgruppe, eine mit einer C₂₋₅-Alkylcarbonylgruppe, die mit einer C₁₋₄-Alkoxygruppe substituiert ist, substituierte C₁₋₆-Alkylgruppe, eine mit einer C₂₋₅-Alkylcarbonylgruppe, die mit einer C₁₋₄-Alkylthiogruppe substituiert ist, substituierte C₁₋₆-Alkylgruppe, eine mit einer C₂₋₅-Alkylcarbonylgruppe, die mit einer C₁₋₄-Alkylsulfinylgruppe substituiert ist, substituierte C₁₋₆-Alkylgruppe, eine mit einer C₂₋₅-Alkylcarbonylgruppe, die mit einer C₁₋₄-Alkylsulfonylgruppe substituiert ist, substituierte C₁₋₆-Alkylgruppe, eine mit einer C₂₋₇-Alkylcarbonylgruppe substituierte C₂₋₆-Alkenylgruppe, eine mit einer C₂₋₇-Alkylcarbonylgruppe substituierte C₂₋₆-Alkinylgruppe, eine mit einer C₁₋₆-Alkylsulfamoylgruppe substituierte C₁₋₆-Alkylkgruppe, eine mit einer C₁₋₆-Alkoxysulfamoylgruppe substituierte C₁₋₆-Alkylgruppe, eine mit einer Di(C₁₋₃- alkyl)sulfamoylgruppe substituierte C₁₋₆-Alkylgruppe, eine mit einer N-(C₁₋₃-Alkyl)-N-(C₁₋₃-alkoxy)sulfamoylgruppe substituierte C₁₋₆-Alkylgruppe, eine mit einer C₂₋₇-Alkylcarbamoylgruppe substituierte C₁₋₆-Alkylgruppe, eine mit einer Di(C₁₋₃-alkyl)carbamoylgruppe substituierte C₁₋₆-Alkylgruppe, eine mit einer C₂₋₇-Alkoxycarbamoylgruppe substituierte C₁₋₆-Alkylgruppe, eine mit einer N-(C₁₋₃-Alkyl)-N-(C₁₋₃-alkoxy)carbamoylgruppe substituierte C₁₋₆-Alkylgruppe, eine mit einer C₁₋₆-Alkylaminogruppe substituierte C₁₋₆-Alkylgruppe, eine mit einer C₁₋₆-Alkoxyammogruppe substituierte C₁₋₆-Alkylgruppe, eine mit einer Di(C₁₋₃-alkyl)aminogruppe substituierte C₁₋₆-Alkylgruppe, eine mit einer N-(C₁₋₃-Alkyl)-N-(C₁₋₃-alkoxy)aminogruppe substituierte C₁₋₆-Alkylgruppe, eine mit einer N-(C₂₋₇-Alkylcarbonyl)-N-(C₁₋₆-alkyl)aminogruppe substituierte C₁₋₆-Alkylgruppe, eine mit einer N-(C₂₋₇-Alkylcarbonyl)-N-(C₁₋₆-alkoxy)aminogruppe substituierte C₁₋₆-Alkylgruppe, eine mit einer N-(C₁₋₆-Alkylsulfonyl)-N-(C₁₋₆-alkyl)aminogruppe substituierte C₁₋₆-Alkylgruppe, eine mit einer N-(C₁₋₆-Alkylsulfonyl)-N-(C₁₋₆-alkoxy)aminogruppe substituierte C₁₋₆-Alkylgruppe, eine Phenylgruppe (mit der Maßgabe, daß eine solche Phenylgruppe substituiert sein kann mit einem oder mehreren Substituenten, die ausgewählt sind aus der Gruppe, bestehend aus einem Halogenatom, einer Trifluormethylgruppe, einer Nitrogruppe, einer C₁₋₆-Alkylgruppe, einer C₁₋₆-Alkoxygruppe und einer C₂₋₇-Alkoxycarbonylgruppe), eine mit einer Phenylgruppe substituierte C₁₋₆-Alkylgruppe (mit der Maßgabe, daß eine solche Phenylgruppe substituiert sein kann mit einem oder mehreren Substituenten, die ausgewählt sind aus der Gruppe, bestehend aus einem Halogenatom, einer Trifluormethylgrvppe, einer Nitrogruppe, einer C₁₋₆-Alkylgruppe, einer C₁₋₆-Alkoxygruppe und einer C₂₋₇-Alkoxycarbonylgruppe), eine mit einer Phenylgruppe substituierte C₂₋₇-Alkenylgruppe (mit der Maßgabe, daß eine solche Phenylgruppe substituiert sein kann mit einem oder mehreren Substituenten, die ausgewählt sind aus der Gruppe, bestehend aus einem Halogenatom, einer Trifluormethylgruppe, einer Nitrogruppe, einer C₁₋₆-Alkylgruppe, einer C₁₋₆-Alkoxygruppe und einer C₂₋₇-Alkoxycarbonylgruppe), eine mit einer Phenylgruppe substituierte C₂₋₆-Alkinylgruppe (mit der Maßgabe, daß eine solche Phenylgruppe substituiert sein kann mit einem oder mehreren Substituenten, die ausgewählt sind aus der Gruppe, bestehend aus einem Halogenatom, einer Trifluormethylgruppe, einer Nitrogruppe, einer C₁₋₆-Alkylgruppe, einer C₁₋₆-Alkoxygruppe und einer C₂₋₇-Alkoxycarbonylgruppe), eine mit einer Phenoxygruppe substituierte C₁₋₆-Alkylgruppe (mit der Maßgabe, daß eine solche Phenoxygruppe substituiert sein kann mit einem oder mehreren Substituenten, die ausgewählt sind aus der Gruppe, bestehend aus einem Halogenatom, einer Trifluormethylgruppe, einer Nitrogruppe, einer C₁₋₆-Alkylgruppe, einer C₁₋₆-Alkoxygruppe und einer C₂₋₇-Alkoxycarbonylgruppe), eine mit einer Phenylthiogruppe substituierte C₁₋₆-Alkylgruppe (mit der Maßgabe, daß eine solche Phenylthiogruppe substituiert sein kann mit einem oder mehreren Substituenten, die ausgewählt sind aus der Gruppe, bestehend aus einem Halogenatom, einer Trifluormethylgruppe, einer Nitrogruppe, einer C₁₋₆-Alkylgruppe, einer C₁₋₆-Alkoxygruppe und einer C₂₋₇-Alkoxycarbonylgruppe), eine mit einer Phenylsulfinylgruppe substituierte C₁₋₆-Alkylgruppe (mit der Maßgabe, daß eine solche Phenylsulfinylgruppe substituiert sein kann mit einem oder mehreren Substituenten, die ausgewählt sind aus der Gruppe, bestehend aus einem Halogenatom, einer Trifluormethylgruppe, einer Nitrogruppe, einer C₁₋₆-Alkylgruppe, einer C₁₋₆-Alkoxygruppe und einer C₂₋₇-Alkoxycarbonylgruppe), eine mit einer Phenylsulfonylgruppe substituierte C₁₋₆-Alkylgruppe (mit der Maßgabe, daß eine solche Phenylsulfonylgruppe substituiert sein kann mit einem oder mehreren Substituenten, die ausgewählt sind aus der Gruppe, bestehend aus einem Halogenatom, einer Trifluormethylgruppe, einer Nitrogruppe, einer C₁₋₆-Alkylgruppe, einer C₁₋₆-Alkoxygruppe und einer C₂₋₇-Alkoxycarbonylgruppe), eine mit einer Benzyloxygruppe substituierte C₁₋₆-Alkylgruppe (mit der Maßgabe, daß die Phenylgruppe einer solchen Benzyloxygruppe substituiert sein kann mit einem oder mehreren Substituenten, die ausgewählt sind aus der Gruppe, bestehend aus einem Halogenatom, einer Trifluormethylgruppe, einer Nitrogruppe, einer C₁₋₆-Alkylgruppe, einer C₁₋₆-Alkoxygruppe und einer C₂₋₇-Alkoxycarbonylgruppe), eine mit einer Benzylthiogruppe substituierte C₁₋₆-Alkylgruppe (mit der Maßgabe, daß die Phenylgruppe einer solchen Benzylthiogruppe substituiert sein kann mit einem oder mehreren Substituenten, die ausgewählt sind aus der Gruppe, bestehend aus einem Halogenatom, einer Trifluormethylgruppe, einer Nitrogruppe, einer C₁₋₆-Alkylgruppe, einer C₁₋₆-Alkoxygruppe und einer C₂₋₇-Alkoxycarbonylgruppe), eine mit einer Benzylsulfinylgruppe substituierte C₁₋₆-Alkylgruppe (mit der Maßgabe, daß die Phenylgruppe einer solchen Benzylsulfinylgruppe substituiert sein kann mit einem oder mehreren Substituenten, die ausgewählt sind aus der Gruppe, bestehend aus einem Halogenatom, einer Trifluormethylgruppe, einer Nitrogruppe, einer C₁₋₆-Alkylgruppe, einer C₁₋₆-Alkoxygruppe und einer C₂₋₇-Alkoxycarbonylgruppe), eine mit einer Benzylsulfonylgruppe substituierte C₁₋₆-Alkylgruppe (mit der Maßgabe, daß die Phenylgruppe einer solchen Benzylsultonylgruppe substituiert sein kann mit einem oder mehreren Substituenten, die ausgewählt sind aus der Gruppe, bestehend aus einem Halogenatom, einer Trifluormethylgruppe, einer Nitrogruppe, einer C₁₋₆-Alkylgruppe, einer C₁₋₆-Alkoxygruppe und einer C₂₋₇-Alkoxycarbonylgruppe), eine mit einer Phenylcarbonylgruppe substituierte C₁₋₆-Alkylgruppe (mit der Maßgabe, daß eine solche Phenylcarbonylgruppe substituiert sein kann mit einem oder mehreren Substituenten, die ausgewählt sind aus der Gruppe, bestehend aus einem Halogenatom, einer Trifluormethylgruppe, einer Nitrogruppe, einer C₁₋₆-Alkylgruppe, einer C₁₋₆-Alkoxygruppe und einer C₂₋₇-Alkoxycarbonylgruppe), eine mit einer Benzylcarbonylgruppe substituierte C₁₋₆-Alkylgruppe (mit der Maßgabe, daß die Phenylgruppe einer solchen Benzylcarbonylgruppe substituiert sein kann mit einem oder mehreren Substituenten, die ausgewählt sind aus der Gruppe, bestehend aus einem Halogenatom, einer Trifluormethylgruppe, einer Nitrogruppe, einer C₁₋₆-Alkylgruppe, einer C₁₋₆-Alkoxygruppe und einer C₂₋₇-Alkoxycarbonylgruppe), eine mit einer Aminogruppe, die mit einer C₁₋₄-Alkylsulfonylgruppe substituiert ist, substituierte C₁₋₆-Alkylgruppe, oder eine mit einer Aminogruppe, die mit einer C₂₋₄-Carbonylgruppe substituiert ist, substituierte C₁₋₆-Alkylgruppe steht,
R^{b2} für ein Wasserstoffatom, eine C₁₋₆-Alkylgruppe, eine Mono-, Di- oder Polyhalogen-C₁₋₆-alkylgruppe, eine C₁₋₆-Alkoxygruppe, eine Mono-, Di- oder Polyhalogen-C₁₋₆-alkoxygruppe, eine C₁₋₆-Alkylthiogruppe, eine C₁₋₆-Alkylsulfinylgruppe, eine C₁₋₆-Alkylsulfonylgruppe, eine C₂₋₇-Alkoxycarbonylgruppe, eine C₂₋₇-Alkylcarbonylgruppe, ein Halogenatom, eine Nitrogruppe, eine Cyanogruppe oder eine Phenylgruppe (mit der Maßgabe, daß eine solche Phenylgruppe substituiert sein kann mit einem oder mehreren Substituenten, die ausgewählt sind aus der Gruppe, bestehend aus einem Halogenatom, einer Trifluormethylgruppe, einer Nitrogruppe, einer C₁₋₆-Alkylgruppe, einer C₁₋₆-Alkoxygruppe und einer C₂₋₇-Alkoxycarbonylgruppe) steht,
R^{b3} für eine C₁₋₆-Alkylgruppe, eine C₂₋₆-Alkenylgruppe oder eine C₂₋₆-Alkinylgruppe steht,
R^{b4} für ein Wasserstoffatom oder eine C₁₋₆-Alkylgruppe steht,
R^{b5} für ein Wasserstoffatom oder eine C₁₋₆-Alkylgruppe steht,
R^{c1} für eine C₁₋₈-Alkylgruppe, eine C₃₋₇-Cycloalkylgruppe, eine mit einer C₃₋₇-Cycloalkylgruppe substituierte C₁₋₆-Alkylgruppe, eine C₃₋₇-Cycloalkenylgruppe, eine mit einer C₃₋₇-Cycloalkenylgruppe substituierte C₁₋₆-Alkylgruppe, eine C₂₋₈-Alkenylgruppe, eine C₂₋₈-Alkinylgruppe, eine mit einer C₁₋₆-Alkoxygruppe substituierte C₁₋₆-Alkylgruppe, eine mit einer C₂₋₆-Alkenyloxygruppe substituierte C₁₋₆-Gruppe, eine mit einer C₂₋₆-Alkinyloxygruppe substituierte C₁₋₆-Alkylgruppe, einer mit einer Mono-, Di- oder Polyhalogen-C₁₋₆-alkoxygruppe substituierte C₁₋₆-Alkylgruppe, eine mit einer Mono-, Di- oder Polyhalogen-C₂₋₆-alkenyloxygruppe substituierte C₁₋₆-Alkylgruppe, eine mit einer Mono-, Di- oder Polyhalogen-C₂₋₆-alkinyloxygruppe substituierte C₁₋₆-Alkylgruppe, eine mit einer C₁₋₆-Alkylthiogruppe substituierte C₁₋₆-Alkylgruppe, eine mit einer C₁₋₆-Alkylsulfinylgruppe substituierte C₁₋₆-Alkylgruppe, eine mit einer C₁₋₆-Alkylsulfonylgruppe substituierte C₁₋₆-Alkylgruppe, eine Mono-, Di- oder Polyhalogen-C₁₋₈-alkylgruppe, eine Mono-, Di- oder Polyhalogen-C₂₋₈-alkenylgruppe, eine Mono-, Di- oder Polyhalogen-C₂₋₈-alkinylgruppe, eine mit einer Cyanogruppe substituierte C₁₋₆-Alkylgruppe, eine mit einer Cyanogruppe substituierte C₂₋₆-Alkenylgruppe, eine mit einer Cyanogruppe substituierte C₂₋₆-Alkinylgruppe, eine mit einer Nitrogruppe substituierte C₁₋₆-Alkylgruppe, eine mit einer Nitrogruppe substituierte C₂₋₆-Alkenylgruppe, eine mit einer Nitrogruppe substituierte C₂₋₆-Alkinylgruppe, eine mit einer C₂₋₇-Alkoxycarbonylgruppe substituierte C₁₋₆-Alkylgruppe, eine mit einer C₂₋₇-Alkoxycarbonylgruppe substituierte C₂₋₆-Alkenylgruppe, eine mit einer C₂₋₇-Alkoxycarbonylgruppe substituierte C₂₋₆-Alkinylgruppe, eine mit einer C₂₋₇-Alkylcarbonylgruppe substituierte C₁₋₆-Alkylgruppe, eine mit einer Mono-, Di- oder Polyhalogen-C₋₂₋₇-alkylcarbonylgruppe substituierte C₁₋₆-Alkylgruppe, eine mit einer C₃₋₇-Alkenylcarbonylgruppe substituierte C₁₋₆-Alkylgruppe, eine mit einer C₃₋₇-Alkinylcarbonylgruppe substituierte C₁₋₆-Alkylgruppe, eine mit einer C₂₋₅-Alkylcarbonylgruppe, die mit einer C₁₋₈-Alkoxygruppe substituiert ist, substituierte C₁₋₆-Alkylgruppe, eine mit einer C₂₋₅-Alkylcarbonylgruppe, die mit einer C₁₋₄-Alkylthiogruppe substituiert ist, substituierte C₁₋₆-Alkylgruppe, eine mit einer C₂₋₅-Alkylcarbonylgruppe, die mit einer C₁₋₄-Alkylsulfinylgruppe substituiert ist, substituierte C₁₋₆-Alkylgruppe, eine mit einer C₂₋₅-Alkylcarbonylgruppe, die mit einer C₁₋₄-Alkylsulfonylgruppe substituiert ist, substituierte C₁₋₆-Alkylgruppe, eine mit einer C₂₋₇-Alkylcarbonylgruppe substituierte C₂₋₆-Alkenylgruppe, eine mit einer C₂₋₇-Alkylcarbonylgruppe substituierte C₂₋₆-Alkinylgruppe, eine mit einer C₁₋₆-Alkylsulfamoylgruppe substituierte C₁₋₆-Alkylgruppe, eine mit einer C₁₋₆-Alkoxysulfamoylgruppe substituierte C₁₋₆-Alkylgruppe, eine mit einer Di(C₁₋₃-alkyl)sulfamoylgruppe substituierte C₁₋₆-Alkylgruppe, eine mit einer N-(C₁₋₃-alkyl)-N-(C₁₋₃-alkoxy)sulfamoylgruppe substituierte C₁₋₆-Alkylgruppe, eine mit einer C₂₋₇-Alkylcarbamoylgruppe substituierte C₁₋₆-Alkylgruppe, eine mit einer Di(C₁₋₃-alkyl)carbamoylgruppe substituierte C₁₋₆-Alkylgruppe, eine mit einer C₂₋₇-Alkoxycarbamoylgruppe substituierte C₁₋₆-Alkylgruppe, eine mit einer N-(C₁₋₃-Alkyl)-N-(C₁₋₃-alkoxy)carbamoylgruppe substituierte C₁₋₆-Alkylgruppe, eine mit einer C₁₋₆-Alkylaminogruppe substituierte C₁₋₆-Alkylgruppe, eine mit einer C₁₋₆-Alkoxyaminogruppe substituierte C₁₋₆-Alkylgruppe, eine mit einer Di(C₁₋₃-alkyl)aminogruppe substituierte C₁₋₆-Alkylgruppe, eine mit einer N-(C₁₋₃-alkyl)-N-(C₁₋₃-alkoxy)aminogruppe substituierte C₁₋₆-Alkylgruppe, eine mit einer N-(C₂₋₇-Alkylcarbonyl)-N-(C₁₋₆-alkyl)aminogruppe substituierte C₁₋₆-Alkylgruppe, eine mit einer N-(C₂₋₇-Alkylcarbonyl)-N-(C₁₋₆-alkoxy)aminogruppe substituierte C₁₋₆-Alkylgruppe, eine mit einer N-(C₁₋₆-Alkylsulfonyl)-N-(C₁₋₆-alkyl)aminogruppe substituierte C₁₋₆-Alkylgruppe, eine mit einer N-(C₁₋₆-Alkylsulfonyl)-N-(C₁₋₆-alkoxy)aminogruppe substituierte C₁₋₆-Alkylgruppe, eine Phenylgruppe (mit der Maßgabe, daß eine solche Phenylgruppe substituiert sein kann mit einem oder mehreren Substituenten, die ausgewählt sind aus der Gruppe, bestehend aus einem Halogenatom, einer Trifluormethylgruppe, einer Nitrogruppe, einer C₁₋₆-Alkylgruppe, einer C₁₋₆-Alkoxygruppe und einer C₂₋₇-Alkoxycarbonylgruppe), eine mit einer Phenylgruppe substituierte C₁₋₆-Alkylgruppe (mit der Maßgabe, daß eine solche Phenylgruppe substituiert sein kann mit einem oder mehreren Substituenten, die ausgewählt sind aus der Gruppe, bestehend aus einem Halogenatom, einer Trifluormethylgruppe, einer Nitrogruppe, einer C₁₋₆-Alkylgruppe, einer C₁₋₆-Alkoxygruppe und einer C₂₋₇-Alkoxycarbonylgruppe), eine mit einer Phenylgruppe substituierte C₂₋₇-Alkenylgruppe (mit der Maßgabe, daß eine solche Phenylgruppe substituiert sein kann mit einem oder mehreren Substituenten, die ausgewählt sind aus der Gruppe, bestehend aus einem Halogenatom, einer Trifluormethylgruppe, einer Nitrogruppe, einer C₁₋₆-Alkylgruppe, einer C₁₋₆-Alkoxygruppe und einer C₂₋₇-Alkoxycarbonylgruppe), eine mit einer Phenylgruppe substituierte C₂₋₆-Alkinylgruppe (mit der Maßgabe, daß eine solche Phenylgruppe substituiert sein kann mit einem oder mehreren Substituenten, die ausgewählt sind aus der Gruppe, bestehend aus einem Halogenatom, einer Trifluormethylgruppe, einer Nitrogruppe, einer C₁₋₆-Alkylgruppe, einer C₁₋₆-Alkoxygruppe und einer C₂₋₇-Alkoxycarbonylgruppe), eine mit einer Phenoxygruppe substituierte C₁₋₆-Alkylgruppe (mit der Maßgabe, daß eine solche Phenoxygruppe substituiert sein kann mit einem oder mehreren Substituenten, die ausgewählt sind aus der Gruppe, bestehend aus einen Halogenatom, einer Trifluormethylgruppe, einer Nitrogruppe, einer C₁₋₆-Alkylgruppe, einer C₁₋₆-Alkoxygruppe und einer C₂₋₇-Alkoxycarbonylgruppe), eine mit einer Phenylthiogruppe substituierte C₁₋₆-Alkylgruppe (mit der Maßgabe, daß eine solche Phenylthiogruppe substituiert sein kann mit einem oder mehreren Substituenten, die ausgewählt sind aus der Gruppe, bestehend aus einem Halogenatom, einer Trifluormethylgruppe, einer Nitrogruppe, einer C₁₋₆-Alkylgruppe, einer C₁₋₆-Alkoxygruppe und einer C₂₋₇-Alkoxycarbonylgruppe), eine mit einer Phenylsulfinylgruppe substituierte C₁₋₆-Alkylgruppe (mit der Maßgabe, daß eine solche Phenylsulfinylgruppe substituiert sein kann mit einem oder mehreren Substituenten, die ausgewählt sind aus der Gruppe, bestehend aus einem Halogenatom, einer Trifluormethylgruppe, einer Nitrogruppe, einer C₁₋₆-Alkylgruppe, einer C₁₋₆-Alkoxygruppe und einer C₂₋₇-Alkoxycarbonylgruppe), eine mit einer Phenylsulfonylgruppe substituierte C₁₋₆-Alkylgruppe (mit der Maßgabe, daß eine solche Phenylsulfonylgruppe substituiert sein kann mit einem oder mehreren Substituenten, die ausgewählt sind aus der Gruppe, bestehend aus einem Halogenatom, einer Trifluormethylgruppe, einer Nitrogruppe, einer C₁₋₆-Alkylgruppe, einer C₁₋₆-Alkoxygruppe und einer C₂₋₇-Alkoxycarbonylgruppe), eine mit einer Benzyloxygruppe substituierte C₁₋₆-Alkylgruppe (mit der Maßgabe, daß die Phenylgruppe einer solchen Benzyloxygruppe substituiert sein kann mit einem oder mehreren Substituenten, die ausgewählt sind aus der Gruppe, bestehend aus einem Halogenatom, einer Trifluormethylgruppe, einer Nitrogruppe, einer C₁₋₆-Alkylgruppe, einer C₁₋₆-Alkoxygruppe und einer C₂₋₇-Alkoxycarbonylgruppe), eine mit einer Benzylthiogruppe substituierte C₁₋₆ Alkylgruppe (mit der Maßgabe, daß die Phenylgruppe einer solchen Benzylthiogruppe substituiert sein kann mit einem oder mehreren Substituenten, die ausgewählt sind aus der Gruppe, bestehend aus einem Halogenatom, einer Trifluormethylgruppe, einer Nitrogruppe, einer C₁₋₆-Alkylgruppe, einer C₁₋₆-Alkoxygruppe und einer C₂₋₇-Alkoxycarbonylgruppe), eine mit einer Benzylsulfinylgruppe substituierte C₁₋₆-Alkylgruppe (mit der Maßgabe, daß die Phenylgruppe einer solchen Benzylsulfinylgruppe substituiert sein kann mit einem oder mehreren Substituenten, die ausgewählt sind aus der Gruppe, bestehend aus einem Halogenatom, einer Trifluormethylgruppe, einer Nitrogruppe, einer C₁₋₆-Alkylgruppe, einer C₁₋₆-Alkoxygruppe und einer C₂₋₇-Alkoxycarbonylgruppe), eine mit einer Benzylsulfonylgruppe substituierte C₁₋₆-Alkylgruppe (mit der Maßgabe, daß die Phenylgruppe einer solchen Benzylsulfonylgruppe substituiert sein kann mit einem oder mehreren Substituenten, die ausgewählt sind aus der Gruppe, bestehend aus einem Halogenatom, einer Trifluormethylgruppe, einer Nitrogruppe, einer C₁₋₆-Alkylgruppe, einer C₁₋₆-Alkoxygruppe und einer C₂₋₇-Alkoxycarbonylgruppe), eine mit einer Phenylcarbonylgruppe substituierte C₁₋₆-Alkylgruppe (mit der Maßgabe, daß eine solche Phenylcarbonylgruppe substituiert sein kann mit einem oder mehreren Substituenten, die ausgewählt sind aus der Gruppe, bestehend aus einem Halogenatom, einer Trifluormethylgruppe, einer Nitrogruppe, einer C₁₋₆-Alkylgruppe, einer C₁₋₆-Alkoxygruppe und einer C₂₋₇-Alkoxycarbonylgruppe), eine mit einer Benzylcarbonylgruppe substituierte C₁₋₆-Alkylgruppe (mit der Maßgabe, daß die Phenylgruppe einer solchen Benzylcarbonylgruppe substituiert sein kann mit einem oder mehreren Substituenten, die ausgewählt sind aus der Gruppe, bestehend aus einem Halogenatom, einer Trifluormethylgruppe, einer Nitrogruppe, einer C₁₋₆-Alkylgruppe, einer C₁₋₆-Alkoxygruppe und einer C₂₋₇-Alkoxycarbonylgruppe), eine mit einer Aminogruppe, die mit einer C₁₋₄-Alkylsulfonylgruppe substituiert ist, substituierte C₁₋₆-Alkylgruppe oder mit einer Aminogruppe, die mit einer C₂₋₄-Alkylcarbonylgruppe substituiert ist, substituierte C₁₋₆-Alkylgruppe steht
R^{c2}, R^{c3}, R^{c4}, R^{c5}, R^{c6}, R^{c11} und R^{c12}, die unabhängig voneinander sind, jeweils für ein Wasserstoffatom, eine C₁₋₈-Alkylgruppe, eine C₂₋₈-Alkenylgruppe, eine C₂₋₈-Alkinylgruppe oder eine Phenylgruppe (mit der Maßgabe, daß eine solche Phenylgruppe substituiert sein kann mit einem oder mehreren Substituenten, die ausgewählt sind aus der Gruppe, bestehend aus einem Halogenatom, einer Trifluormethylgruppe, einer Nitrogruppe, einer C₁₋₆-Alkylgruppe, einer C₁₋₆-Alkoxygruppe und einer C₂₋₇-Alkoxycarbonylgruppe) steht,
R^{c7}, R^{c8}, R^{c9}, R^{c10}, R^{c13} und R^{c14}, die unabhängig voneinander sind, jeweils für ein Wasserstoffatom, eine C₁₋₆-Alkylgruppe, eine Mono-, Di- oder Polyhalogen-C₁₋₆-alkylgruppe, eine C₁₋₆-Alkoxygruppe, eine Mono-, Di- oder Polyhalogen-C₁₋₆-alkoxygruppe, eine C₁₋₆-Alkylthlogruppe, eine C₁₋₆-Alkylsulfinylgruppe, eine C₁₋₆-Alkylsulfonylgruppe, eine C₂₋₇-Alkoxycarbonylgruppe, eine C₂₋₇-Alkylcarbonylgruppe, ein Halogenatom, eine Nitrogruppe, eine Cyanogruppe oder eine Phenylgruppe (mit der Maßgabe, daß eine solche Phenylgruppe substituiert sein kann mit einem oder mehreren Substituenten, die ausgewählt sind aus der Gruppe, bestehend aus einem Halogenatom, einer Trifluormethylgruppe, einer Nitrogruppe, einer C₁₋₆-Alkylgruppe, einer C₁₋₆-Alkoxygruppe und einer C₂₋₇-Alkoxycarbonylgruppe) steht,
R^{d1} für eine C₁₋₈-Alkylgruppe, eine C₃₋₇-Cycloalkylgruppe, eine mit einer C₃₋₇-Cycloalkylgruppe substituierte C₁₋₆-Alkylgruppe, eine C₃₋₇-Cycloalkenylgruppe, eine mit einer C₃₋₇-Cycloalkenylgruppe substituierte C₁₋₆-Alkylgruppe, eine C₂₋₈-Alkenylgruppe, eine C₂₋₈-Alkinylgruppe, eine mit einer C₁₋₆-Alkoxygruppe substituierte C₁₋₆-Alkylgruppe, eine mit einer C₂₋₆-Alkenyloxygruppe substituierte C₁₋₆-Alkylgruppe, eine mit einer C₂₋₆-Alkinyloxygruppe substituierte C₁₋₆-Alkylgruppe, eine mit einer Mono-, Di- oder Polyhalogen-C₁₋₆-alkoxygruppe substituierte C₁₋₆-Alkylgruppe, eine mit einer Mono-, Di- oder Polyhalogen-C₂₋₆-alkenyloxygruppe substituierte C₁₋₆-Alkylgruppe, eine mit einer Mono-, Di- oder Polyhalogen₋C₂₋₆-alkinyloxygruppe substituierte C₁₋₆-Alkylgruppe, eine mit einer C₁₋₆-Alkylthiogruppe substituierte C₁₋₆-Alkylgruppe, eine mit einer C₁₋₆-Alkylulfinylgruppe substituierte C₁₋₆-Alkylgruppe, eine mit einer C₁₋₆-Alkylsulfonylgruppe substituierte C₁₋₆-Alkylgruppe, eine Mono-, Di- oder Polyhalogen-C₁₋₈-alkylgruppe, eine Mono-, Di- oder Polyhalogen-C₂₋₈-alkenylgruppe, eine Mono-, Di- oder Polyhalogen-C₂₋₈-alkinylgruppe, eine mit einer Cyanogruppe substituierte C₁₋₆-Alkylgruppe, eine mit einer Cyanogruppe substituierte C₂₋₆-Alkenylgruppe, einer mit einer Cyanogruppe substituierte C₂₋₆-Alkinylgruppe, eine mit einer Nitrogruppe substituierte C₁₋₆-Alkylgruppe, eine mit einer Nitrogruppe substituierte C₂₋₆-Alkenylgruppe, eine mit einer Nitrogruppe substituierte C₂₋₆-Alkinylgruppe, eine mit einer C₂₋₇-Alkoxycarbonylgruppe substituierte C₁₋₆-Alkylgruppe, eine mit einer C₂₋₇-Alkoxycarbonylgruppe substituierte C₂₋₆-Alkenylgruppe, eine mit einer C₂₋₇-Alkoxycarbonylgruppe substituierte C₂₋₆-Alkinylgruppe, eine mit einer C₂₋₇-Alkylcarbonylgruppe substituierte C₁₋₆-Alkylgruppe, eine mit einer Mono-, Di- oder Polyhalogen-C₂₋₇-alkylcarbonylgruppe substituierte C₁₋₆-Alkylgruppe, eine mit einer C₃₋₇-Alkenylcarbonylgruppe substituierte C₁₋₆-Alkylgruppe, eine mit einer C₃₋₇-Alkinylcarbonylgruppe substituierte C₁₋₆-Alkylgruppe, eine mit einer C₂₋₅-Alkylcarbonylgruppe, die mit einer C₁₋₄-Alkoxygruppe substituiert ist, substituierte C₁₋₆-Alkylgruppe, eine mit einer C₂₋₅-Alkylcarbonylgruppe, die mit einer C₁₋₄-Alkylthiogruppe substituierte ist, substituierte C₁₋₆-Alkylgruppe, eine mit einer C₂₋₅-Alkylcarbonylgruppe, die mit einer C₁₋₄-Alkylsulfinylgruppe substituiert ist, substituierte C₁₋₆-Alkylgruppe, eine mit einer C₂₋₅-Alkylcarbonylgruppe, die mit einer C₁₋₄-Alkylsulfonylgruppe substituiert ist, substituierte C₁₋₆-Alkylgruppe, eine mit einer C₂₋₇-Alkylcarbonylgruppe substituierte C₂₋₆-Alkenylgruppe, eine mit einer C₂₋₇-Alkylcarbonylgruppe substituierte C₂₋₆-Alkinylgruppe, eine mit einer C₁₋₆-Alkylsulfamoylgruppe substituierte C₁₋₆-Alkylgruppe, eine mit einer C₁₋₆-Alkoxysulfamoylgruppe substituierte C₁₋₆-Alkylgruppe, eine mit einer Di(C₁₋₃-alkyl)sulfamoylgruppe substituierte C₁₋₆-Alkylgruppe, eine mit einer N-(C₁₋₃-alkyl)-N-(C₁₋₃-alkoxy)sulfamoylgruppe substituierte C₁₋₆-Alkylgruppe, eine mit einer C₂₋₇-Alkylcarbamoylgruppe substituierte C₁₋₆-Alkylgruppe, eine mit einer Di(C₁₋₃-alkyl)carbamoylgruppe substituierte C₁₋₆-Alkylgruppe, eine mit einer C₂₋₇-Alkoxycarbamoylgruppe substituierte C₁₋₆-Alkylgruppe, eine mit einer N-(C₁₋₃-alkyl)-N-(C₁₋₃-alkoxy)carbamoylgruppe substituierte C₁₋₆-Alkylgruppe, eine mit einer C₁₋₆-Alkylaminogruppe substituierte C₁₋₆-Alkylgruppe, eine mit einer C₁₋₆-Alkoxyaminogruppe substituierte C₁₋₆-Alkylgruppe, eine mit einer Di(C₁₋₃-alkyl)aminogruppe substituierte C₁₋₆-Alkylgruppe, einer mit einer N-(C₁₋₃-alkyl)-N-(C₁₋₃-alkoxy)aminogruppe substituierte C₁₋₆-Alkylgruppe, eine mit einer N-(C₂₋₇-alkylcarbonyl)-N-C₁₋₆-alkyl)aminogruppe substituierte C₁₋₆-Alkylgruppe, eine mit einer N-(C₂₋₇-Alkylcarbonyl)-N-(C₁₋₆-alkoxy)aminogruppe substituierte C₁₋₆-Alkylgruppe, eine mit einer N-(C₁₋₆-Alkylsulfonyl)-N-(C₁₋₆-alkyl)aminogruppe substituierte C₁₋₆-Alkylgruppe, eine mit einer N-(C₁₋₆-Alkylsulfonyl)-N-(C₁₋₆-alkoxy)aminogruppe substituierte C₁₋₆-Alkylgruppe, eine Phenylgruppe (mit der Maßgabe, daß eine solche Phenylgruppe substituiert sein kann mit einem oder mehreren Substituenten, die ausgewählt sind aus der Gruppe, bestehend aus einem Halogenatom, einer Trifluormethylgruppe, einer Nitrogruppe, einer C₁₋₆-Alkylgruppe, einer C₁₋₆-Alkoxygruppe und einer C₂₋₇-Alkoxycarbonylgruppe), eine mit einer Phenylgruppe substituierte C₁₋₆-Alkylgruppe (mit der Maßgabe, daß eine solche Phenylgruppe substituiert sein kann mit einem oder mehreren Substituenten, die ausgewählt sind aus der Gruppe, bestehend aus einem Halogenatom, einer Trifluormethylgruppe, einer Nitrogruppe, einer C₁₋₆-Alkylgruppe, einer C₁₋₆-Alkoxygruppe und einer C₂₋₇-Alkoxycarbonylgruppe), eine mit einer Phenylgruppe substituierte C₂₋₇-Alkenylgruppe (mit der Maßgabe, daß eine solche Phenylgruppe substituiert sein kann mit einem oder mehreren Substituenten, die ausgewählt sind aus der Gruppe, bestehend aus einem Halogenatom, einer Trifluormethylgruppe, einer Nitrogruppe, einer C₁₋₆-Alkylgruppe, einer C₁₋₆-Alkoxygruppe und einer C₂₋₇-Alkoxycarbonylgruppe), eine mit einer Phenylgruppe substituierte C₂₋₆-Alkinylgruppe (mit der Maßgabe, daß eine solche Phenylgruppe substituiert sein kann mit einem oder mehreren Substituenten, die ausgewählt sind aus der Gruppe, bestehend aus einem Halogenatom, einer Trifluormethylgruppe, einer Nitrogruppe, einer C₁₋₆-Alkylgruppe, einer C₁₋₆-Alkoxygruppe und einer C₂₋₇-Alkoxycarbonylgruppe), eine mit einer Phenoxygruppe substituierte C₁₋₆-Alkylgruppe (mit der Maßgabe, daß eine solche Phenoxygruppe substituiert sein kann mit einem oder mehreren Substituenten, die ausgewählt sind aus der Gruppe, bestehend aus einem Halogenatom, einer Trifluormethylgruppe, einer Nitrogruppe, einer C₁₋₆-Alkylgruppe, einer C₁₋₆-Alkoxygruppe und einer C₂₋₇-Alkoxycarbonylgruppe), eine mit einer Phenylthiogruppe substituierte C₁₋₆-Alkylgruppe (mit der Maßgabe, daß eine solche Phenylthiogruppe substituiert sein kann mit einem oder mehreren Substituenten, die ausgewählt sind aus der Gruppe, bestehend aus einem Halogenatom, einer Trifluormethylgruppe, einer Nitrogruppe, einer C₁₋₆-Alkylgruppe, einer C₁₋₆-Alkoxygruppe und einer C₂₋₇-Alkoxycarbonylgruppe), eine mit einer Phenylsulfinylgruppe substituierte C₁₋₆-Alkylgruppe (mit der Maßgabe, daß eine solche Phenylsulfinylgruppe substituiert sein kann mit einem oder mehreren Substituenten, die ausgewählt sind aus der Gruppe, bestehend aus einem Halogenatom, einer Trifluormethylgruppe, einer Nitrogruppe, einer C₁₋₆-Alkylgruppe, einer C₁₋₆-Alkoxygruppe und einer C₂₋₇-Alkoxycarbonylgruppe), eine mit einer Phenylsulfonylgruppe substituierte C₁₋₆-Alkylgruppe (mit der Maßgabe, daß eine solche Phenylsulfonylgruppe substituiert sein kann mit einem oder mehreren Substituenten, die ausgewählt sind aus der Gruppe, bestehend aus einem Halogenatom, einer Trifluormethylgruppe, einer Nitrogruppe, einer C₁₋₆-Alkylgruppe, einer C₁₋₆-Alkoxygruppe und einer C₂₋₇-Alkoxycarbonylgruppe), eine mit einer Benzyloxygruppe substituierte C₁₋₆-Alkylgruppe (mit der Maßgabe, daß die Phenylgruppe einer solchen Benzyloxygruppe substituiert sein kann mit einem oder mehreren Substituenten, die ausgewählt sind aus der Gruppe, bestehend aus einem Halogenatom, einer Trifluormethylgruppe, einer Nitrogruppe, einer C₁₋₆-Alkylgruppe, einer C₁₋₆-Alkoxygruppe und einer C₂₋₇-Alkoxycarbonylgruppe), eine mit einer Benzylthiogruppe substituierte C₁₋₆-Alkylgruppe (mit der Maßgabe, daß die Phenylgruppe einer solchen Benzylthiogruppe substituiert sein kann mit einem oder mehreren Substituenten, die ausgewählt sind aus der Gruppe, bestehend aus einem Halogenatom, einer Trifluormethylgruppe, einer Nitrogruppe, einer C₁₋₆-Alkylgruppe, einer C₁₋₆-Alkoxygruppe und einer C₂₋₇-Alkoxycarbonylgruppe), eine mit einer Benzylsulfinylgruppe substituierte C₁₋₆-Alkylgruppe (mit der Maßgabe, daß die Phenylgruppe einer solchen Benzylsulfinylgruppe substituiert sein kann mit einem oder mehreren Substituenten, die ausgewählt sind aus der Gruppe, bestehend aus einem Halogenatom, einer Trifluormethylgruppe, einer Nitrogruppe, einer C₁₋₆-Alkylgruppe, einer C₁₋₆-Alkoxygruppe und einer C₂₋₇-Alkoxycarbonylgruppe), eine mit einer Benzylsulfonylgruppe substituierte C₁₋₆-Alkylgruppe (mit der Maßgabe, daß die Phenylgruppe einer solchen Benzylsulfonylgruppe substituiert sein kann mit einem oder mehreren Substituenten, die ausgewählt sind aus der Gruppe, bestehend aus einem Halogenatom, einer Trifluormethylgruppe, einer Nitrogruppe, einer C₁₋₆-Alkylgruppe, einer C₁₋₆-Alkoxygruppe und einer C₂₋₇-Alkoxycarbonylgruppe), eine mit einer Phenylcarbonylgruppe substituierte C₁₋₆-Alkylgruppe (mit der Maßgabe, daß eine solche Phenylcarbonylgruppe substituiert sein kann mit einem oder mehrere Substituenten, die ausgewählt sind aus der Gruppe, bestehend aus einem Halogenatom, einer Trifluormethylgruppe, einer Nitrogruppe, einer C₁₋₆-Alkylgruppe, einer C₁₋₆-Alkoxygruppe und einer C₂₋₇-Alkoxycarbonylgruppe), eine mit einer Benzylcarbonylgruppe substituierte C₁₋₆-Alkylgruppe (mit der Maßgabe, daß die Phenylgruppe einer solchen Benzylcarbonylgruppe substituiert sein kann mit einem oder mehreren Substituenten, die ausgewählt sind aus der Gruppe, bestehend aus einem Halogenatom, einer Trifluormethylgruppe, einer Nitrogruppe, einer C₁₋₆-Alkylgruppe, einer C₁₋₆-Alkoxygruppe und einer C₂₋₇-Alkoxycarbonylgruppe), eine mit einer Aminogruppe, die mit einer C₁₋₄-Alkylsulfonylgruppe substituiert ist, substituierte C₁₋₆-Alkylgruppe, oder mit einer Aminogruppe, die mit einer C₂₋₄-Alkylcarbonylgruppe substituiert ist, substituierte C₁₋₆-Alkylgruppe steht,
R^{d2}, R^{d3} und R^{d4}, die unabhängig voneinander sind, jeweils für ein Wasserstoffatom, eine C₁₋₈-Alkylgruppe, eine C₂₋₈-Alkenylgruppe, eine C₂₋₈-Alkinylgruppe oder eine Phenylgruppe (mit der Maßgabe, daß eine solche Phenylgruppe substituiert sein kann mit einem oder mehreren Substituenten, die ausgewählt sind aus der Gruppe, bestehend aus einem Halogenatom, einer Trifluormethylgruppe, einer Nitrogruppe, einer C₁₋₆-Alkylgruppe, einer C₁₋₆-Alkoxygruppe und einer C₂₋₇-Alkoxycarbonylgruppe) steht,
R^{d5} für eine C₁₋₈-Alkylgruppe, eine C₂₋₈-Alkenylgruppe, eine C₂₋₈-Alkinylgruppe oder eine Phenylgruppe (mit der Maßgabe, daß eine solche Phenylgruppe substituiert sein kann mit einem oder mehreren Substituenten, die ausgewählt sind aus der Gruppe, bestehend aus einem Halogenatom, einer Trifluormethylgruppe, einer Nitrogruppe, einer C₁₋₆-Alkylgruppe, einer C₁₋₆-Alkoxygruppe und einer C₂₋₇-Alkoxycarbonylgruppe) steht,
R^{d6} für eine C₁₋₈-Alkylgruppe, eine C₂₋₈-Alkenylgruppe, eine C₂₋₈-Alkinylgruppe, eine mit einer C₁₋₆-Alkylthlogruppe substituierte C₁₋₆-Alkylgruppe, eine mit einer C₁₋₆-Alkylsulfinylgruppe substituierte C₁₋₆-Alkylgruppe, eine mit einer C₁₋₆-Alkylsulfonylgruppe substituierte C₁₋₆-Alkylgruppe, eine mit einer C₁₋₆-Alkoxygruppe substituierte C₁₋₆-Alkylgruppe, eine mit einer C₂₋₇-Alkoxycarbonylgruppe substituierte C₁₋₆-Alkylgruppe, eine mit einer C₂₋₇-Alkylcarbonylgruppe substituierte C₁₋₆-Alkylgruppe, eine mit einer Cyanogruppe substituierte C₁₋₆-Alkylgruppe, eine Phenylgruppe (mit der Maßgabe, daß eine solche Phenylcarbonylgruppe substituiert sein kann mit einem oder mehreren Substituenten, die ausgewählt sind aus der Gruppe, bestehend aus einem Halogenatom, einer Trifluormethylgruppe, einer Nitrogruppe, einer C₁₋₆-Alkylgruppe, einer C₁₋₆-Alkoxygruppe und einer C₂₋₇-Alkoxycarbonylgruppe), oder eine mit einer Phenylgruppe substituierte C₁₋₆-Alkylgruppe (mit der Maßgabe, daß die eine solche Phenylgruppe substituiert sein kann mit einem oder mehreren Substituenten, die ausgewählt sind aus der Gruppe, bestehend aus einem Halogenatom, einer Trifluormethylgruppe, einer Nitrogruppe, einer C₁₋₆-Alkylgruppe, einer C₁₋₆-Alkoxygruppe und einer C₂₋₇-Alkoxycarbonylgruppe) steht,
R^{e1} für eine C₁₋₈-Alkylgruppe, eine C₃₋₇-Cycloalkylgruppe, eine mit einer C₃₋₇-Cycloalkylgruppe substituierte C₁₋₆-Alkylgruppe, eine C₃₋₇-Cycloalkenylgruppe, eine mit einer C₃₋₇-Cycloalkinylgruppe substituierte C₁₋₆-Alkylgruppe, eine C₂₋₈-Alkenylgruppe, eine C₂₋₈-Alkinylgruppe, eine mit einer C₁₋₆-Alkoxygruppe substituierte C₁₋₆-Alkylgruppe, eine mit einer C₂₋₆-Alkenyloxygruppe substituierte C₁₋₆-Alkylgruppe, eine mit einer C₂₋₆-Alkinyloxygruppe substituierte C₁₋₆-Alkylgruppe, eine mit einer Mono-, Di- oder Polyhalogen-C₁₋₆-alkoxygruppe substituierte C₁₋₆-Alkylgruppe, eine mit einer Mono-, Di- oder Polyhalogen-C₂₋₆-alkenyloxygruppe substituierte C₁₋₆-Alkylgruppe, eine mit einer Mono-, Di- oder Polyhalogen-C₂₋₆-alkinyloxygruppe substituierte C₁₋₆-Alkylgruppe, eine mit einer C₁₋₆-Alkylthiogruppe substituierte C₁₋₆-Alkylgruppe, eine mit einer C₁₋₆-Alkylsulfinylgruppe substituierte C₁₋₆-Alkylgruppe, eine mit einer C₁₋₆-Alkylsulfonylgruppe substituierte C₁₋₆-Alkylgruppe, eine Mono-, Di- oder Polyhalogen-C₁₋₈-alkylgruppe, eine Mono-, Di- oder Polyhalogen-C₂₋₈-alkenylgruppe, eine Mono-, Di- oder Polyhalogen-C₂₋₈-alkinylgruppe, eine mit einer Cyanogruppe substituierte C₁₋₆-Alkylgruppe, eine mit einer Cyanogruppe substituierte C₂₋₆-Alkenylgruppe, eine mit einer Cyanogruppe substituierte C₂₋₆-Alkinylgruppe, eine mit einer Nitrogruppe substituierte C₁₋₆-Alkylgruppe, eine mit einer Nitrogruppe substituierte C₂₋₆-Alkenylgruppe, eine mit einer Nitrogruppe substituierte C₂₋₆-Alkinylgruppe, eine mit einer C₂₋₇-Alkoxycarbonylgruppe substituierte C₁₋₆-Alkylgruppe, eine mit einer C₂₋₇-Alkoxycarbonylgruppe substituierte C₂₋₆-Alkenylgruppe, eine mit einer C₂₋₇-Alkoxycarbonylgruppe substituierte C₂₋₆-Alkinylgruppe, eine mit einer C₂₋₇-Alkylcarbonylgruppe substituierte C₁₋₆-Alkylgruppe, eine mit einer Mono-, Di- oder Polyhalogen-C₂₋₇-alkylcarbonylgruppe substituierte C₁₋₆-Alkylgruppe, eine mit einer C₃₋₇-Alkinylcarbonylgruppe substituierte C₁₋₆-Alkylgruppe, eine mit einer C₃₋₇-Alkinylcarbonylgruppe substituierte C₁₋₈-Alkylgruppe, eine mit einer C₂₋₅-Alkylcarbonylgruppe, die mit einer C₁₋₄-Alkoxygruppe substituiert ist, substituierte C₁₋₆-Alkylgruppe, eine mit einer C₂₋₅-Alkylcarbonylgruppe, die mit einer C₁₋₄-Alkylthiogruppe substituiert ist, substituierte C₁₋₆-Alkylgruppe, eine mit einer C₂₋₅-Alkylcarbonylgruppe, die mit einer C₁₋₄-Alkylsulfinylgruppe substituiert ist, substituierte C₁₋₆-Alkylgruppe, eine mit einer C₂₋₅-Alkylcarbonylgruppe, die mit einer C₁₋₄-Alkylsulfonylgruppe substituiert ist, substituierte C₁₋₆-Alkylgruppe, eine mit einer C₂₋₇-Alkylcarbonylgruppe substituierte C₂₋₆-Alkenylgruppe, eine mit einer C₂₋₇-Alkylcarbonylgruppe substituierte C₂₋₆-Alkinylgruppe, eine mit einer C₁₋₆-Alkylsulfamoylgruppe substituierte C₁₋₆-Alkylgruppe, eine mit einer C₁₋₆-Alkoxysulfamoylgruppe substituierte C₁₋₆-Alkylgruppe, eine mit einer Di(C₁₋₃-alkyl)sulfamoylgruppe substituierte C₁₋₆-Alkylgruppe, eine mit einer N-(C₁₋₃-Alkyl)-N-(C₁₋₃-alkoxy)sulfamoylgruppe substituierte C₁₋₆-Alkylgruppe, eine mit einer C₂₋₇-Alkylcarbamoylgruppe substituierte C₁₋₆-Alkylgruppe, eine mit einer Di(C₁₋₃-alkyl)carbamoylgruppe substituierte C₁₋₆-Alkylgruppe, eine mit einer C₂₋₇-Alkoxycarbamoylgruppe substituierte C₁₋₆-Alkylgruppe, eine mit einer N-(C₁₋₃-Alkyl)-N-(C₁₋₃-alkoxy)carbamoylgruppe substituierte C₁₋₆-Alkylgruppe, eine mit einer C₁₋₆-Alkylaminogruppe substituierte C₁₋₆-Alkylgruppe, eine mit einer C₁₋₆-Alkoxyaminogruppe substituierte C₁₋₆-Alkylgruppe, eine mit einer Di(C₁₋₃-alkyl)aminogruppe substituierte C₁₋₆-Alkylgruppe, eine mit einer N-(C₁₋₃-Alkyl)-N-(C₁₋₃-alkoxy)aminogruppe substituierte C₁₋₆-Alkylgruppe, eine mit einer N-(C₂₋₇-Alkylcarbonyl)-N-(C₁₋₆-alkyl)aminogruppe substituierte C₁₋₆-Alkylgruppe, eine mit einer N-(C₂₋₇-alkylcarbonyl)-N-(C₁₋₆-alkoxy)aminogruppe substituierte C₁₋₆-Alkylgruppe, eine mit einer N-(C₁₋₆-Alkylsulfonyl)-N-(C₁₋₆-alkyl)aminogruppe substituierte C₁₋₆-Alkylgruppe, eine mit einer N-(C₁₋₆-Alkylsulfonyl)-N-(C₁₋₆-alkoxy)aminogruppe substituierte C₁₋₆-Alkylgruppe, eine Phenylgruppe (mit der Maßgabe, daß eine solche Phenylgruppe substituiert sein kann mit einem oder mehreren Substituenten, die ausgewählt sind aus der Gruppe, bestehend aus einem Halogenatom, einer Trifluormethylgruppe, einer Nitrogruppe, einer C₁₋₆-Alkylgruppe, einer C₁₋₆-Alkoxygruppe und einer C₂₋₇-Alkoxycarbonylgruppe), eine mit einer Phenylgruppe substituierte C₁₋₆-Alkylgruppe (mit der Maßgabe, daß eine solche Phenylgruppe substituiert sein kann mit einem oder mehreren Substituenten, die ausgewählt sind aus der Gruppe, bestehend aus einem Halogenatom, einer Trifluormethylgruppe, einer Nitrogruppe, einer C₁₋₆-Alkylgruppe, einer C₁₋₆-Alkoxygruppe und einer C₂₋₇-Alkoxycarbonylgruppe), eine mit einer Phenylgruppe substituierte C₂₋₇-Alkenylgruppe (mit der Maßgabe, daß eine solche Phenylgruppe substituiert sein kann mit einem oder mehreren Substituenten, die ausgewählt sind aus der Gruppe, bestehend aus einem Halogenatom, einer Trifluormethylgruppe, einer Nitrogruppe, einer C₁₋₆-Alkylgruppe, einer C₁₋₆-Alkoxygruppe und einer C₂₋₇-Alkoxycarbonylgruppe), eine mit einer Phenylgruppe substituierte C₂₋₆-Alkylgruppe (mit der Maßgabe, daß eine solche Phenylgruppe substituiert sein kann mit einem oder mehreren Substituenten, die ausgewählt sind aus der Gruppe, bestehend aus einem Halogenatom, einer Trifluormethylgruppe, einer Nitrogruppe, einer C₁₋₆-Alkylgruppe, einer C₁₋₆-Alkoxygruppe und einer C₂₋₇-Alkoxycarbonylgruppe), einer mit einer Phenoxygruppe substituierte C₁₋₆-Alkylgruppe (mit der Maßgabe, daß eine solche Phenoxygruppe substituiert sein kann mit einem oder mehreren Substituenten, die ausgewählt sind aus der Gruppe, bestehend aus einem Halogenatom, einer Trifluormethylgruppe, einer Nitrogruppe, einer C₁₋₆-Alkylgruppe, einer C₁₋₆-Alkoxygruppe und einer C₂₋₇-Alkoxycarbonylgruppe), eine mit einer Phenylthiogruppe substituierte C₁₋₆-Alkylgruppe (mit der Maßgabe, daß eine solche Phenylthiogruppe substituiert sein kann mit einem oder mehreren Substituenten, die ausgewählt sind aus der Gruppe, bestehend aus einem Halogenatom, einer Trifluormethylgruppe, einer Nitrogruppe, einer C₁₋₆-Alkylgruppe, einer C₁₋₆-Alkoxygruppe und einer C₂₋₇-Alkoxycarbonylgruppe), eine mit einer Phenylsulfinylgruppe substituierte C₁₋₆-Alkylgruppe (mit der Maßgabe, daß eine solche Phenylsulfinylgruppe substituiert sein kann mit einem oder mehreren Substituenten, die ausgewählt sind aus der Gruppe, bestehend aus einem Halogenatom, einer Trifluormethylgruppe, einer Nitrogruppe, einer C₁₋₆-Alkylgruppe, einer C₁₋₆-Alkoxygruppe und einer C₂₋₇-Alkoxycarbonylgruppe), eine mit einer Phenylsulfonylgruppe substituierte C₁₋₆-Alkylgruppe (mit der Maßgabe, daß eine solche Phenylsulfonylgruppe substituiert sein kann mit einem oder mehreren Substituenten, die ausgewählt sind aus der Gruppe, bestehend aus einem Halogenatom, einer Trifluormethylgruppe, einer Nitrogruppe, einer C₁₋₆-Alkylgruppe, einer C₁₋₆-Alkoxygruppe und einer C₂₋₇-Alkoxycarbonylgruppe), eine mit einer Benzyloxygruppe substituierte C₁₋₆-Alkylgruppe (mit der Maßgabe, daß die Phenylgruppe einer solchen Benzyloxygruppe substituiert sein kann mit einem oder mehreren Substituenten, die ausgewählt sind aus der Gruppe, bestehend aus einem Halogenatom, einer Trifluormethylgruppe, einer Nitrogruppe, einer C₁₋₆-Alkylgruppe, einer C₁-₆-Alkoxygruppe und einer C₂₋₇-Alkoxycarbonylgruppe), ein mit einer Benzylthiogruppe substituierte C₁₋₆-Alkylgruppe (mit der Maßgabe, daß die Phenylgruppe einer solchen Benzylthiogruppe substituiert sein kann mit einem oder mehreren Substituenten, die ausgewählt sind aus der Gruppe, bestehend aus einem Halogenatom, einer Tri- fluormethylgruppe, einer Nitrogruppe, einer C₁₋₆-Alkylgruppe, einer C₁₋₆-Alkoxygruppe und einer C₂₋₇-Alkoxycarbonylgruppe), eine mit einer Benzylsulfinylgruppe substituierte C₁₋₆-Alkylgruppe (mit der Maßgabe, daß die Phenylgruppe einer solchen Benzylsulfinylgruppe substituiert sein kann mit einem oder mehreren Substituenten, die ausgewählt sind aus der Gruppe, bestehend aus einem Halogenatom, einer Trifluormethylgruppe, einer Nitrogruppe, einer C₁₋₆-Alkylgruppe, einer C₁₋₆-Alkoxygruppe und einer C₂₋₇-Alkoxycarbonylgruppe), eine mit einer Benzylsulfonylgruppe substituierte C₁₋₆-Alkylgruppe (mit der Maßgabe, daß die Phenylgruppe eine solche Benzylsulfonylgruppe substituiert sein kann mit einem oder mehreren Substituenten, die ausgewählt sind aus der Gruppe, bestehend aus einem Halogenatom, einer Trifluormethylgruppe, einer Nitrogruppe, einer C₁₋₆-Alkylgruppe, einer C₁₋₆-Alkoxygruppe und einer C₂₋₇-Alkoxycarbonylgruppe), eine mit einer Phenylcarbonylgruppe substituierte C₁₋₆-Alkylgruppe (mit der Maßgabe, daß eine solche Phenylcarbonylgruppe substituiert sein kann mit einem oder mehreren Substituenten, die ausgewählt sind aus der Gruppe, bestehend aus einem Halogenatom, einer Trifluormethylgruppe, einer Nitrogruppe, einer C₁₋₆-Alkylgruppe, einer C₁₋₆-Alkoxygruppe und einer C₂₋₇-Alkoxycarbonylgruppe), eine mit einer Benzylcarbonylgruppe substituierte C₁₋₆-Alkylgruppe (mit der Maßgabe, daß die Phenylgruppe einer solchen Benzylcarbonylgruppe substituiert sein kann mit einem oder mehreren Substituenten, die ausgewählt sind aus der Gruppe, bestehend aus einem Halogenatom, einer Trifluormethylgruppe, einer Nitrogruppe, einer C₁₋₆-Alkylgruppe, einer C₁₋₆-Alkoxygruppe und einer C₂₋₇-Alkoxycarbonylgruppe), eine mit einer Aminogruppe, die mit einer C₁₋₄-Alkylsulfonylgruppe substituiert ist, substituierte C₁₋₆-Alkylgruppe, oder eine mit einer Aminogruppe, die mit einer C₂₋₄-Alkylcarbonylgruppe substituiert ist, substituierte C₁₋₆-Alkylgruppe steht,
R^{e2}, R^{e3}, R^{e6} und R^{e7}, die unabhängig voneinander sind, jeweils für ein Wasserstoffatom, eine C₁₋₆-Alkylgruppe, eine Mono-, Di- oder Polyhalogen-C₁₋₆-alkylgruppe, eine C₁₋₆-Alkoxygruppe, eine Mono-, Di- oder Polyhalogen-C₁₋₆-alkoxygruppe, eine C₁₋₆-Alkylthiogruppe, eine C₁₋₆-Alkylsulfinylgruppe, eine C₁₋₆-Alkylsulfonylgruppe, eine C₂₋₇-Alkoxycarbonylgruppe, eine C₂₋₇-Alkylcarbonylgruppe, ein Halogenatom, eine Nitrogruppe, eine Cyanogruppe oder eine Phenylgruppe (mit der Maßgabe, daß eine solche Phenylgruppe substituiert sein kann mit einem oder mehreren Substituenten, die ausgewählt sind aus der Gruppe, bestehend aus einem Halogenatom, einer Trifluormethylgruppe, einer Nitrogruppe, einer C₁₋₆-Alkylgruppe, einer C₁₋₆-Alkoxygruppe und einer C₂₋₇-Alkoxycarbonylgruppe) steht,
R^{e4}, R^{e5}, R^{e8}, R^{e9} und R^{e10}, die unabhängig voneinander sind, jeweils für ein Wasserstoffatom, eine C₁₋₈-Alkylgruppe, eine C₂₋₈-Alkenylgruppe, eine C₂₋₈-Alkinylgruppe oder eine Phenylgruppe (mit der Maßgabe, daß eine solche Phenylgruppe substituiert sein kann mit einem oder mehreren Substituenten, die ausgewählt sind aus der Gruppe, bestehend aus einem Halogenatom, einer Trifluormethylgruppe, einer Nitrogruppe, einer C₁₋₆-Alkylgruppe, einer C₁₋₆-Alkoxygruppe und einer C₂₋₇-Alkoxycarbonylgruppe) steht,
R^{f1} für eine C₁₋₈-Alkylgruppe, eine C₃₋₇-Cycloalkylgruppe, eine mit einer C₃₋₇-Cycloalkylgruppe substituierte C₁₋₆-Alkylgruppe, eine C₃₋₇-Cycloalkenylgruppe, eine mit einer C₃₋₇-Cycloalkenylgruppe substituierte C₁₋₆-Alkylgruppe, eine C₂₋₈-Alkenylgruppe, eine C₂₋₈-Alkinylgruppe, eine mit einer C₁₋₆-Alkoxygruppe substituierte C₁₋₆-Alkylgruppe, eine mit einer C₂₋₆-Alkenyloxygruppe substituierte C₁₋₆-Alkylgruppe, eine mit einer C₂₋₆-Alkinyloxygruppe substituierte C₁₋₆-Alkylgruppe, eine mit einer Mono-, Di- oder Polyhalogen-C₁₋₆-alkoxygruppe substituierte C₁₋₆-Alkylgruppe, eine mit einer Mono-, Di- oder Polyhalogen-C₂₋₆-alkenyloxygruppe substituierte c₁₋₆-Alkylgruppe, eine mit einer Mono-, Di- oder Polyhalogen-C₂₋₆-alkinyloxygruppe substituierte C₁₋₆-Alkylgruppe, eine mit einer C₁₋₆-Alkylthiogruppe substituierte C₁₋₆-Alkylgruppe, eine mit einer C₁₋₆-Alkylsulfinylgruppe substituierte C₁₋₆-Alkylgruppe, eine mit einer C₁₋₆-Alkylsulfonylgruppe substituierte C₁₋₆-Alkylgruppe, eine Mono-, Di- oder Polyhalogen-C₁₋₈-alkylgruppe, eine Mono-, Di- oder Polyhalogen-C₂₋₈-alkenylgruppe, eine Mono-, Di- oder Polyhalogen-C₂₋₈-alkinylgruppe, eine mit einer Cyanogruppe substituierte C₁₋₆-Alkylgruppe, eine mit einer Cyanogruppe substituierte C₂₋₆-Alkenylgruppe, eine mit einer Cyanogruppe substituierte C₂₋₆-Alkinylgruppe, eine mit einer Nitrogruppe substituierte C₁₋₆-Alkylgruppe, eine mit einer Nitrogruppe substituierte C₂₋₆-Alkenylgruppe, eine mit einer Nitrogruppe substituierte C₂₋₆-Alkinylgruppe, eine mit einer C₂₋₇-Alkoxycarbonylgruppe substituierte C₁₋₆-Alkylgruppe, eine mit einer C₂₋₇-Alkoxycarbonylgruppe substituierte C₂₋₆-Alkenylgruppe, eine mit einer C₂₋₇-Alkoxycarbonylgruppe substituierte C₂₋₆-Alkinylgruppe, eine mit einer C₂₋₇-Alkylcarbonylgruppe substituierte C₁₋₆-Alkylgruppe, eine mit einer Mono-, Di- oder Polyhalogen-C₂₋₇-alkylcarbonylgruppe substituierte C₁₋₆-Alkylgruppe, eine mit einer C₃₋₇-Alkenylcarbonylgruppe substituierte C₁₋₆-Alkylgruppe, eine mit einer C₃₋₇-Alkinylcarbonylgruppe substituierte C₁₋₆-Alkylgruppe, eine mit einer C₂₋₅-Alkylcarbonylgruppe, die mit einer C₁₋₄-Alkoxygruppe substituiert ist, substituierte C₁₋₆-Alkylgruppe, eine mit einer C₂₋₅-Alkylcarbonylgruppe, die mit einer C₁₋₄-Alkylthiogruppe substituiert ist, substituierte C₁₋₆-Alkylgruppe, eine mit einer C₂₋₅-Alkylcarbonylgruppe, die mit einer C₁₋₄-Alkylsulfinylgruppe substituiert ist, substituierte C₁₋₆-Alkylgruppe, eine mit einer C₂₋₅-Alkylcarbonylgruppe, die mit einer D₁₋₄-Alkylsulfonylgruppe substituiert ist, substituierte C₁₋₆-Alkylgruppe, eine mit einer C₂₋₇-Alkylcarbonylgruppe substituierte C₂₋₆-Alkenylgruppe, eine mit einer C₂₋₇-Alkylcarbonylgruppe substituierte C₂₋₆-Alkinylgruppe, eine mit einer C₁₋₆-Alkylsulfamoylgruppe substituierte C₁₋₆-Alkylgruppe, eine mit einer C₁₋₆-Alkoxysulfamoylgruppe substituierte C₁₋₆-Alkylgruppe, eine mit einer Di(C₁₋₃-alkyl)sulfamoylgruppe substituierte C₁₋₆-Alkylgruppe, eine mit einer N-(C₁₋₃-Alkyl)-N-(C₁₋₃-alkoxy)sulfamoylgruppe substituierte C₁₋₆-Alkylgruppe, eine mit einer C₂₋₇-Alkylcarbamoylgruppe substituierte C₁₋₆-Alkylgruppe, eine mit einer Di(C₁₋₃-alkyl)carbamoylgruppe substituierte C₁₋₆-Alkylgruppe, eine mit einer C₂₋₇-Alkoxycarbamoylgruppe substituierte C₁₋₆-Alkylgruppe, eine mit einer N-(C₁₋₃-Alkyl)-N-(C₁₋₃-alkoxy)carbamoylgruppe substituierte C₁₋₆-Alkylgruppe, eine mit einer C₁₋₆-Alkylaminogruppe substituierte C₁₋₆-Alkylgruppe, eine mit einer C₁₋₆-Alkoxyaminogruppe substituierte C₁₋₆-Alkylgruppe, eine mit einer Di(C₁₋₃-alkyl)aminogruppe substituierte C₁₋₆-Alkylgruppe, eine mit einer N-(C₁₋₃-Alkyl)-N-(C₁₋₃-alkoxy)aminogruppe substituierte C₁₋₆-Alkylgruppe, eine mit einer N-(C₂₋₇-Alkyl carbonyl)-N-(C₁₋₆-alkyl)aminogruppe substituierte C₁₋₆-Alkylgruppe, eine mit einer N-(C₂₋₇-Alkylcarbonyl)-N-(C₁₋₆-alkoxy)aminogruppe substituierte C₁₋₆-Alkylgruppe, eine mit einer N-(C₁₋₆-Alkylsulfonyl)-N-(C₁₋₆-alkyl)aminogruppe substituierte C₁₋₆-Alkylgruppe, eine mit einer N-(C₁₋₆-Alkylsulfonyl)-N-(C₁₋₆-alkoxy)aminogruppe substituierte C₁₋₆-Alkylgruppe, eine Phenylgruppe (mit der Maßgabe, daß eine solche Phenylgruppe substituiert sein kann mit einem oder mehreren Substituenten, die ausgewählt sind aus der Gruppe, bestehend aus einem Halogenatom, einer Trifluormethylgruppe, einer Nitrogruppe, einer C₁₋₆-Alkylgruppe, einer C₁₋₆-Alkoxygruppe und einer C₂₋₇-Alkoxycarbonylgruppe), eine mit einer Phenylgruppe substituierte C₁₋₆-Alkylgruppe (mit der Maßgabe, daß eine solche Phenylgruppe substituiert sein kann mit einem oder mehreren Substituenten, die ausgewählt sind aus der Gruppe, bestehend aus einem Halogenatom, einer Trifluormethylgruppe, einer Nitrogruppe, einer C₁₋₆-Alkylgruppe, einer C₁₋₆-Alkoxygruppe und einer C₂₋₇-Alkoxycarbonylgroppe), eine mit einer Phenylgruppe substituierte C₂₋₇-Alkenylgruppe (mit der Maßgabe, daß eine solche Phenylgruppe substituiert sein kann mit einem oder mehreren Substituenten, die ausgewählt sind aus der Gruppe, bestehend aus einem Halogenatom, einer Trifluormethylgruppe, einer Nitrogruppe, einer C₁₋₆-Alkylgruppe, einer C₁₋₆-Alkoxygruppe und einer C₂₋₇-Alkoxycarbonylgruppe), eine mit einer Phenylgruppe substituierte C₂₋₆-Alkinylgruppe (mit der Maßgabe, daß eine solche Phenylgruppe substituiert sein kann mit einem oder mehreren Substituenten, die ausgewählt sind aus der Gruppe, bestehend aus einem Halogenatom, einer Trifluormethylgruppe, einer Nitrogruppe, einer C₁₋₆-Alkylgruppe, einer C₁₋₆-Alkoxygruppe und einer C₂₋₇-Alkoxycarbonylgruppe), eine mit einer Phenoxygruppe substituierte C₁₋₆-Alkylgruppe (mit der Maßgabe, daß eine solche Phenoxygruppe substituiert sein kann mit einem oder mehreren Substituenten, die ausgewählt sind aus der Gruppe, bestehend aus einem Halogenatom, einer Trifluormethylgruppe, einer Nitrogruppe, einer C₁₋₆-Alkylgruppe, einer C₁₋₆-Alkoxygruppe und einer C₂₋₇-Alkoxycarbonylgruppe), eine mit einer Phenylthiogruppe substituierte C₁₋₆-Alkylgruppe (mit der Maßgabe, daß eine solche Phenylthiogruppe substituiert sein kann mit einem oder mehreren Substituenten, die ausgewählt sind aus der Gruppe, bestehend aus einem Halogenatom, einer Trifluormethylgruppe, einer Nitrogruppe, einer C₁₋₆-Alkylgruppe, einer C₁₋₆-Alkoxygruppe und einer C₂₋₇-Alkoxycarbonylgruppe), eine mit einer Phenysulfinylgruppe substituierte C₁₋₆-Alkylgruppe (mit der Maßgabe, daß eine solche Phenylsulfinylgruppe substituiert sein kann mit einem oder mehreren Substituenten, die ausgewählt sind aus der Gruppe, bestehend aus einem Halogenatom, einer Trifluormethylgruppe, einer Nitrogruppe, einer C₁₋₆-Alkylgruppe, einer C₁₋₆-Alkoxygruppe und einer C₂₋₇-Alkoxycarbonylgruppe), eine mit einer Phenylsulfonylgruppe substituierte C₁₋₆-Alkylgruppe (mit der Maßgabe, daß eine solche Phenylsulfonylgruppe substituiert sein kann mit einem oder mehreren Substituenten, die ausgewählt sind aus der Gruppe, bestehend aus einem Halogenatom, einer Trifluormethylgruppe, einer Nitrogruppe, einer C₁₋₆-Alkylgruppe, einer C₁₋₆-Alkoxygruppe und einer C₂₋₇-Alkocarbonylgruppe), eine mit einer Benzyloxygruppe substituierte C₁₋₆-Alkylgruppe (mit der Maßgabe, daß die Phenylgruppe einer solchen Benzyloxygruppe substituiert sein kann mit einem oder mehreren Substituenten, die ausgewählt sind aus der Gruppe, bestehend aus einem Halogenatom, einer Trifluormethylgruppe, einer Nitrogruppe, einer C₁₋₆-Alkylgruppe, einer C₁₋₆-Alkoxygruppe und einer C₂₋₇-Alkoxycarbonylgruppe), eine mit einer Benzylthiogruppe substituierte C₁₋₆-Alkylgruppe (mit der Maßgabe, daß die Phenylgruppe einer solchen Benzylthiogruppe substituiert sein kann mit einem oder mehreren Substituenten, die ausgewählt sind aus der Gruppe, bestehend aus einem Halogenatom, einer Trifluormethylgruppe, einer Nitrogruppe, einer C₁₋₆-Alkylgruppe, einer C₁₋₆-Alkoxygruppe und einer C₂₋₇-Alkoxycarbonylgruppe), eine mit einer Benzylsulfinylgruppe substituierte C₁₋₆-Alkylgruppe (mit der Maßgabe, daß die Phenylgruppe eine solche Benzylsulfinylgruppe substituiert sein kann mit einem oder mehreren Substituenten, die ausgewählt sind aus der Gruppe, bestehend aus einem Halogenatom, einer Trifluormethylgruppe, eIner Nitrogruppe, einer C₁₋₆-Alkylgruppe, einer C₁₋₆-Alkoxygruppe und einer C₂₋₇-Alkoxycarbonylgruppe), eine mit einer Benzylsulfonylgruppe substituierte C₁₋₆-Alkylgruppe (mit der Maßgabe, daß die Phenylgruppe eine solche Benzylsulfonylgruppe substituiert sein kann mit einem oder mehreren Substituenten, die ausgewählt sind aus der Gruppe, bestehend aus einem Halogenatom, einer Trifluormethylgruppe, einer Nitrogruppe, einer C₁₋₆-Alkylgruppe, einer C₁₋₆-Alkoxygruppe und einer C₂₋₇-Alkoxycarbonylgruppe), eine mit einer Phenylcarbonylgruppe substituierte C₁₋₆-Alkylgruppe (mit der Maßgabe, daß eine solche Phenylcarbonylgruppe substituiert sein kann mit einem oder mehreren Substituenten, die ausgewählt sind aus der Gruppe, bestehend aus einem Halogenatom, einer Trifluormethylgruppe, einer Nitrogruppe, einer C₁₋₆-Alkylgruppe, einer C₁₋₆-Alkoxygruppe und einer C₂₋₇-Alkoxycarbonylgruppe), eine mit einer Benzylcarbonylgruppe substituierte C₁₋₆-Alkylgruppe (mit der Maßgabe, daß die Phenylgruppe einer solchen Benzylcarbonylgruppe substituiert sein kann mit einem oder mehreren Substituenten, die ausgewählt sind aus der Gruppe, bestehend aus einem Halogenatom, einer Trifluormethylgruppe, einer Nitrogruppe, einer C₁₋₆-Alkylgruppe, einer C₁₋₆-Alkoxygruppe und einer C₂₋₇-Alkoxycarbonylgruppe), eine mit einer Aminogruppe, die mit einer C₁₋₄-Alkylsulfonylgruppe substituiert ist, substituierte C₁₋₆-Alkylgruppe, oder eine mit einer Aminogruppe, die mit einer C₂₋₄-Alkylcarbonylgruppe substituiert ist, substituierte C₁₋₆-Alkylgruppe steht,
R^{f2} für eine C₁₋₈-Alkylgruppe, eine C₂₋₈-Alkenylgruppe, eine C₂₋₈-Alkinylgruppe, mit einer C₁₋₆-Alkylthiogruppe substituierte C₁₋₆-Alkylgruppe, eine mit einer C₁₋₆-Alkylsulfinylgruppe substituierte C₁₋₆-Alkylgruppe, eine mit einer C₁₋₆-Alkylsulfonylgruppe substituierte C₁₋₆-Alkylgruppe, eine mit einer C₁₋₆-Alkoxygruppe substituierte C₁₋₆-Alkylgruppe, eine mit einer C₂₋₇-Alkoxycarbonylgruppe substituierte C₁₋₆-Alkylgruppe, eine mit einer C₂₋₇-Alkylcarbonylgruppe substituierte C₁₋₆-Alkylgruppe, eine mit einer Cyanogruppe substituierte C₁₋₆-Alkylgruppe, eine Phenylgruppe (mit der Maßgabe, daß eine solche Phenylgruppe substituiert sein kann mit einem oder mehreren Substituenten, die ausgewählt sind aus der Gruppe, bestehend aus einem Halogenatom, einer Trifluormethylgruppe, einer Nitrogruppe, einer C₁₋₆-Alkylgruppe, einer C₁₋₆-Alkoxygruppe und einer C₂₋₇-Alkoxycarbonylgruppe), oder eine mit einer Phenylgruppe substituierte C₁₋₆-Alkylgruppe (mit der Maßgabe, daß eine solche Phenylgruppe substituiert sein kann mit einem oder mehreren Substituenten, die ausgewählt sind aus der Gruppe, bestehend aus einem Halogenatom, einer Trifluormethylgruppe, einer Nitrogruppe, einer C₁₋₆-Alkylgruppe, einer C₁₋₆-Alkoxygruppe und einer C₂₋₇-Alkoxycarbonylgruppe) steht,
R^{f3}, R^{f4}, R^{f7}, R^{f8}, R^{f11} und R^{f12}, die unabhängig voneinander sind, jeweils für ein Wasserstoffatom, eine C₁₋₆-Alkylgruppe, eine Mono-, Di- oder Polyhalogen-C₁₋₆-alkylgruppe, eine C₁₋₆-Alkoxygruppe, eine Mono-, Di- oder Polyhalogen-C₁₋₆-alkoxygruppe, eine C₁₋₆-Alkylthiogruppe, eine C₁₋₆-Alkylsulfinylgruppe, eine C₁₋₆-Alkylsulfonylgruppe, eine C₂₋₇-Alkoxycarbonylgruppe, eine C₂₋₇-Alkylcarbonylgruppe, ein Halogenatom, eine Nitrogruppe, eine Cyanogruppe oder eine Phenylgruppe (mit der Maßgabe, daß eine solche Phenylgruppe substituiert sein kann mit einem oder mehreren Substituenten, die ausgewählt sind aus der Gruppe, bestehend aus einem Halogenatom, einer Trifluormethylgruppe, einer Nitrogruppe, einer C₁₋₆-Alkylgruppe, einer C₁₋₆-Alkoxygruppe und einer C₂₋₇-Alkoxycarbonylgruppe) steht,
R^{f5}, R^{f6}, R^{f9}, R^{f10}, R^{f13}, R^{f14} und R^{f15}, die unabhängig voneinander sind, jeweils für ein Wasserstoffatom, eine C₁₋₈-Alkylgruppe, eine C₂₋₈-Alkenylgruppe, eine C₂₋₈-Alkinylgruppe oder eine Phenylgruppe (mit der Maßgabe, daß eine solche Phenylgruppe substituiert sein kann mit einem oder mehreren Substituenten, die ausgewählt sind aus der Gruppe, bestehend aus einem Halogenatom, einer Trifluormethylgruppe, einer Nitrogruppe, einer C₁₋₆-Alkylgruppe, einer C₁₋₆-Alkoxygruppe und einer C₂₋₇-Alkoxycarbonylgruppe) steht,
R^{g1} für eine C₁₋₈-Alkylgruppe, eine C₃₋₇-Cycloalkylgruppe, eine mit einer C₃₋₇-Cycloalkylgruppe substituierte C₁₋₆-Alkylgruppe, eine C₃₋₇-Cycloalkenylgruppe, eine mit einer C₃₋₇-Cycloalkenylgruppe substituierte C₁₋₆-Alkylgruppe, eine C₂₋₈-Alkenylgrupp, eine C₂₋₈-Alkinylgruppe, eine mit einer C₁₋₆-Alkoxygruppe substituierte C₁₋₆-Alkylgruppe, eine mit einer C₂₋₆-Alkenyloxygruppe substituierte C₁₋₆-Alkylgruppe, eine mit einer C₂₋₆-Alkinyloxygruppe substituierte C₁₋₆-Alkylgruppe, eine mit einer Mono-, Di- oder Polyhalogen-C₁₋₆-alkoxygruppe substituierte C₁₋₆-Alkylgruppe, eine mit einer Mono-, Di- oder Polyhalogen-C₂₋₆-Alkenyloxygruppe substituierte C₁₋₆-Alkylgruppe, eine mit einer Mono-, Di- oder Polyhalogen-C₂₋₆-alkinyloxygruppe substituierte C₁₋₆-Alkylgruppe, eine mit einer C₁₋₆-Alkylthiogruppe substituierte C₁₋₆-Alkylgruppe, eine mit einer C₁₋₆-Alkylsulfinylgruppe substituierte C₁₋₆-Alkylgruppe, eine mit einer C₁₋₆-Alkylsulfonylgruppe substituierte C₁₋₆-Alkylgruppe, eine Mono-, Di- oder Polyhalogen-C₁₋₈-alkyl gruppe, eine Mono-, Di- oder Polyhalogen-C₂₋₈-alkenylgruppe, eine Mono-, Di- oder Polyhalogen-C₂₋₈-alkinylgruppe, eine mit einer Cyanogruppe substituierte C₁₋₆-Alkylgruppe, eine mit einer Cyanogruppe substituierte C₂₋₆-Alkenylgruppe, eine mit einer Cyanogruppe substituierte C₂₋₆-Alkinylgruppe, eine mit einer Nitrogruppe substituierte C₁₋₆-Alkylgruppe, eine mit einer Nitrogruppe substituierte C₂₋₆-Alkenylgruppe, eine mit einer Nitrogruppe substituierte C₂₋₆-Alkinylgruppe, eine mit einer C₂₋₇-Alkoxycarbonylgruppe substituierte C₁₋₆-Alkylgruppe, eine mit einer C₂₋₇-Alkoxycarbonylgruppe substituierte C₂₋₆-Alkenylgruppe, eine mit einer C₂₋₇-Alkoxycarbonylgruppe substituierte C₂₋₆-Alkinylgruppe, eine mit einer C₂₋₇-Alkylcarbonylgruppe substituierte C₁₋₆-Alkylgruppe, eine mit einer Mono-, Di- oder Polyhalogen-C₂₋₇-Alkylcarbonylgruppe substituierte C₁₋₆-Alkylgruppe, eine mit einer C₃₋₇-Alkenylcarbonylgruppe substituierte C₁₋₆-Alkylgruppe, eine mit einer C₃₋₇-Alkinylcarbonylgruppe substituierte C₁₋₆-Alkylgruppe, eine mit einer C₂₋₅-Alkylcarbonylgruppe, die mit einer C₁₋₄-Alkoxygruppe substituiert ist, substituierte C₁₋₆-Alkylgruppe, eine mit einer C₂₋₅-Alkylcarbonylgruppe, die mit einer C₁₋₄-Alkylthiogruppe substituiert ist, substituierte C₁₋₆-Alkylgruppe, eine mit einer C₂₋₅-Alkylcarbonylgruppe, die mit einer C₁₋₄-Alkylsulfinylgruppe substituiert ist, substituierte C₁₋₆-Alkylgruppe, eine mit einer C₂₋₅-Alkylcarbonylgruppe, die mit einer C₁₋₄-Alkylsulfonylgruppe substituiert ist, substituierte C₁₋₆-Alkylgruppe, eine mit einer C₂₋₇-Alkylcarbonylgruppe substituierte C₂₋₆-Alkenylgruppe, eine mit einer C₂₋₇-Alkylcarbonylgruppe substituierte C₂₋₆-Alkinylgruppe, eine mit einer C₁₋₆-Alkylsulfamoylgruppe substituierte C₁₋₆-Alkylgruppe, eine mit einer C₁₋₆-Alkoxysulfamoylgruppe substituierte C₁₋₆-Alkylgruppe, eine mit einer Di(₁₋₃-alkyl)sulfamoylgruppe substituierte C₁₋₆-Alkylgruppe, eine mit einer N-(C₁₋₃-Alkyl)-N-(C₁₋₃-alkoxy)sulfamoylgruppe substituierte C₁₋₆-Alkylgruppe, eine mit einer C₂₋₇-Alkylcarbamoylgruppe substituierte C₁₋₆-Alkylgruppe, eine mit einer Di(C₁₋₃-alkyl)carbamoylgruppe substituierte C₁₋₆-Alkylgruppe, eine mit einer C₂₋₇-Alkoxycarbamoylgruppe substituierte C₁₋₆-Alkylgruppe, eine mit einer N-(C₁₋₃-Alkyl)-N-(C₁₋₃-alkoxy)carbamoylgruppe substituierte C₁₋₆-Alkylgruppe, eine mit einer C₁₋₆-Alkylaminogruppe substituierte C₁₋₆-Alkylgruppe, eine mit einer C₁₋₆-Alkoxyaminogruppe substituierte C₁₋₆-Alkylgruppe, eine mit einer Di(C₁₋₃-alkyl)aminogruppe substituierte C₁₋₆-Alkylgruppe, eine mit einer N-(C₁₋₃-Alkyl)-N-(C₁₋₃-alkoxy)aminogruppe substituierte C₁₋₆-Alkylgruppe, eine mit einer N-(C₂₋₇-Alkylcarbonyl)-N-(C₁₋₆-alkyl)aminogruppe substituierte C₁₋₆-Alkylgruppe, eine mit einer N-(C₂₋₇-Alkylcarbonyl)-N-(C₁₋₆-alkoxy)aminogruppe substituierte C₁₋₆-Alkylgruppe, eine mit einer N-(C₁₋₆-Alkylsulfonyl)-N-(C₁₋₆-alkyl)aminogruppe substituierte C₁₋₆-Alkylgruppe, eine mit einer N-(C₁₋₆-Alkylsulfonyl)-N-(C₁₋₆-alkoxy)amino substituierte C₁₋₆-Alkylgruppe, eine mit einer Phenylgruppe substituierte C₁₋₆-Alkylgruppe (mit der Maßgabe, daß eine solche Phenylgruppe substituiert sein kann mit einem oder mehreren Substituenten, die ausgewählt sind aus der Gruppe, bestehend aus einem Halogenatom, einer Trifluormethylgruppe, einer Nitrogruppe, einer C₁₋₆-Alkylgruppe, einer C₁₋₆-Alkoxygruppe und einer C₂₋₇-Alkoxycarbonylgruppe), eine mit einer Phenylgruppe substituierte C₂₋₇-Alkenylgruppe (mit der Maßgabe, daß eine solche Phenylgruppe substituiert sein kann mit einem oder mehreren Substituenten, die ausgewählt sind aus der Gruppe, bestehend aus einem Halogenatom, einer Trifluormethylgruppe, einer Nitrogruppe, einer C₁₋₆-Alkylgruppe, einer C₁₋₆-Alkoxygruppe und einer C₂₋₇-Alkoxycarbonylgruppe), eine mit einer Phenylgruppe substituierte C₂₋₆-Alkinylgruppe (mit der Maßgabe, daß eine solche Phenylgruppe substituiert sein kann mit einem oder mehreren Substituenten, die ausgewählt sind aus der Gruppe, bestehend aus einem Halogenatom, einer Trifluormethylgruppe, einer Nitrogruppe, einer C₁₋₆-Alkylgruppe, einer C₁₋₆-Alkoxygruppe und einer C₂₋₇-Alkoxycarbonylgruppe), eine mit einer Phenoxygruppe substituierte C₁₋₆-Alkylgruppe (mit der Maßgabe, daß eine solche Phenoxygruppe substituiert sein kann mit einem oder mehreren Substituenten, die ausgewählt sind aus der Gruppe, bestehend aus einem Halogenatom, einer Trifluormethylgruppe, einer Nitrogruppe, einer C₁₋₆-Alkylgruppe, einer C₁₋₆-Alkoxygruppe und einer C₂₋₇-Alkoxycarbonylgruppe), eine mit einer Phenylthiogruppe substituierte C₁₋₆-Alkylgruppe (mit der Maßgabe, daß eine solche Phenylthiogruppe substituiert sein kann mit einem oder mehreren Substituenten, die ausgewählt sind aus der Gruppe, bestehend aus einem Halogenatom, einer Trifluormethylgruppe, einer Nitrogruppe, einer C₁₋₆-Alkylgruppe, einer C₁₋₆-Alkoxygruppe und einer C₂₋₇-Alkoxycarbonylgruppe), eine mit einer Phenylsulfinylgruppe substituierte C₁₋₆-Alkylgruppe (mit der Maßgabe, daß eine solche Phenylsulfinylgruppe substituiert sein kann mit einem oder mehreren Substituenten, die ausgewählt sind aus der Gruppe, bestehend aus einem Halogenatom, einer Trifluormethylgruppe, einer Nitrogruppe, einer C₁₋₆-Alkylgruppe, einer C₁₋₆-Alkoxygruppe und einer C₂₋₇-Alkoxycarbonylgruppe), eine mit einer Phenylsulfonylgruppe substituierte C₁₋₆-Alkylgruppe (mit der Maßgabe, daß eine solche Phenylsulfonylgruppe substituiert sein kann mit einem oder mehreren Substituenten, die ausgewählt sind aus der Gruppe, bestehend aus einem Halogenatom, einer Trifluormethylgruppe, einer Nitrogruppe, einer C₁₋₆-Alkylgruppe, einer C₁₋₆-Alkoxygruppe und einer C₂₋₇-Alkoxycarbonylgruppe), eine mit einer Benzyloxygruppe substituierte C₁₋₆-Alkylgruppe (mit der Maßgabe, daß die Phenylgruppe einer solchen Benzyloxygruppe substituiert sein kann mit einem oder mehreren Substituenten, die ausgewählt sind aus der Gruppe, bestehend aus einem Halogenatom, einer Trifluormethylgruppe, einer Nitrogruppe, einer C₁₋₆-Alkylgruppe, einer C₁₋₆-Alkoxygruppe und einer C₂₋₇-Alkoxycarbonylgruppe), eine mit einer Benzylthiogruppe substituierte C₁₋₆-Alkylgruppe (mit der Maßgabe, daß die Phenylgruppe einer solchen Benzylthiogruppe substituiert sein kann mit einem oder mehreren Substituenten, die ausgewählt sind aus der Gruppe, bestehend aus einem Halogenatom, einer Trifluormethylgruppe, einer Nitrogruppe, einer C₁₋₆-Alkylgruppe, einer C₁₋₆-Alkoxygruppe und einer C₂₋₇-Alkoxycarbonylgruppe), eine mit einer Benzylsulfinylgruppe substituierte C₁₋₆-Alkylgruppe (mit der Maßgabe, daß die Phenylgruppe einer solchen Benzylsulfinylgruppe substituiert sein kann mit einem oder mehreren Substituenten, die ausgewählt sind aus der Gruppe, bestehend aus einem Halogenatom, einer Trifluormethylgruppe, einer Nitrogruppe, einer C₁₋₆-Alkylgruppe, einer C₁₋₆-Alkoxygruppe und einer C₂₋₇-Alkoxycarbonylgruppe), eine mit einer Benzylsulfonylgruppe substituierte C₁₋₆-Alkylgruppe (mit der Maßgabe, daß die Phenylgruppe solch einr Benzylsulfonylgruppe substituiert sein kann mit einem oder mehreren Substituenten, die ausgewählt sind aus der Gruppe, bestehend aus einem Halogenatom, einer Trifluormethylgruppe, einer Nitrogruppe, einer C₁₋₆-Alkylgruppe, einer C₁₋₆-Alkoxygruppe und einer C₂₋₇-Alkoxycarbonylgruppe), eine mit einer Phenylcarbonylgruppe substituierte C₁₋₆-Alkylgruppe (mit der Maßgabe, daß eine solche Phenylcarbonylgruppe substituiert sein kann mit einem oder mehreren Substituenten, die ausgewählt sind aus der Gruppe, bestehend aus einem Halogenatom, einer Trifluormethylgruppe, einer Nitrogruppe, einer C₁₋₆-Alkylgruppe, einer C₁₋₆-Alkoxygruppe und einer C₂₋₇-Alkoxycarbonylgruppe), oder eine mit einer Benzylcarbonylgruppe substituierte C₁₋₆-Alkylgruppe (mit der Maßgabe, daß die Phenylgruppe einer solchen Benzylcarbonylgruppe substituiert sein kann mit einem oder mehreren Substituenten, die ausgewählt sind aus der Gruppe, bestehend aus einem Halogenatom, einer Trifluormethylgruppe, einer Nitrogruppe, einer C₁₋₆-Alkylgruppe, einer C₁₋₆-Alkoxygruppe und einer C₂₋₇-Alkoxycarbonylgruppe) steht,
R^{g2} und R^{g3}, die unabhängig voneinander sind, jeweils für eine C₁₋₆-Alkylgruppe, eine C₂₋₆-Alkenylgruppe, eine C₂₋₆-Alkinylgruppe, eine Mono-, Di- oder Polyhalogen-C₁₋₆-alkylgruppe, eine C₁₋₆-Alkoxygruppe, eine C₁₋₆-Alkylsulfonylgruppe, eine C₁₋₆-Alkylsulfamoylgruppe, eine Di(C₁₋₃-alkyl)sulfamoylgruppe, eine C₂₋₇-Alkoxycarbonylgruppe, eine C₂₋₇-Alkylcarbonylgruppe, eine C₂₋₇-Alkylcarbamoylgruppe, eine Di- (C₁₋₃-alkyl)carbamoylgruppe, eine Phenylgruppe (mit der Maßgabe, daß eine solche Phenylgruppe substituiert sein kann mit einem oder mehreren Substituenten, die ausgewählt sind aus der Gruppe, bestehend aus einem Halogenatom, einer Trifluormethylgruppe, einer Nitrogruppe, einer C₁₋₆-Alkylgruppe, einer C₁₋₆-Alkoxygruppe und einer C₂₋₇-Alkocycarbonylgruppe), oder eine Benzylgruppe (mit der Maßgabe, daß die Phenylgruppe einer solchen Benzylgruppe substituiert sein kann mit einem oder mehreren Substituenten, die ausgewählt sind aus der Gruppe, bestehend aus einem Halogenatom, einer Trifluormethylgruppe, einer Nitrogruppe, einer C₁₋₆-Alkylgruppe, einer C₁₋₆-Alkoxygruppe und einer C₂₋₇-Alkoxycarbonylgruppe) steht,
oder R^{g2} und R^{g3} zusammen mit dem Stickstoffatom, an das sie gebunden sind, einen 3- bis 7-gliedrigen heterocyclischen Ring bilden,
X für ein Sauerstoffatom und ein Schwefelatom steht,
L für ein Wasserstoffatom, eine C₁₋₆-Alkylgruppe, eine C₂₋₆-Alkenylgruppe oder eine C₂₋₆-Alkinylgruppe steht,
G für 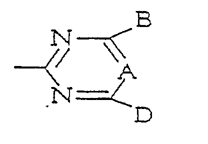 steht,
A für eine CH-Gruppe oder ein Stickstoffatom steht, und
B und D, die unabhängig voneinander sind, jeweils für eine C₁₋₄-Alkylgruppe, eine C₁₋₄-Alkoxygruppe, eine Mono-, Di- oder Polyhalogen-C₁₋₄-alkylgruppe, eine Mono-, Di- oder Polyhalogen-C₁₋₄-alkoxygruppe, ein Halogenatom, eine C₁₋₄-Alkylaminogruppe oder eine Di(C₁₋₄-alkyl)aminogruppe steht.

3. Verfahren zur Herstellung eines Iminosulfonyl-Harnstoffderivats oder dessen Salz nach Anspruch 2, wobei Q für 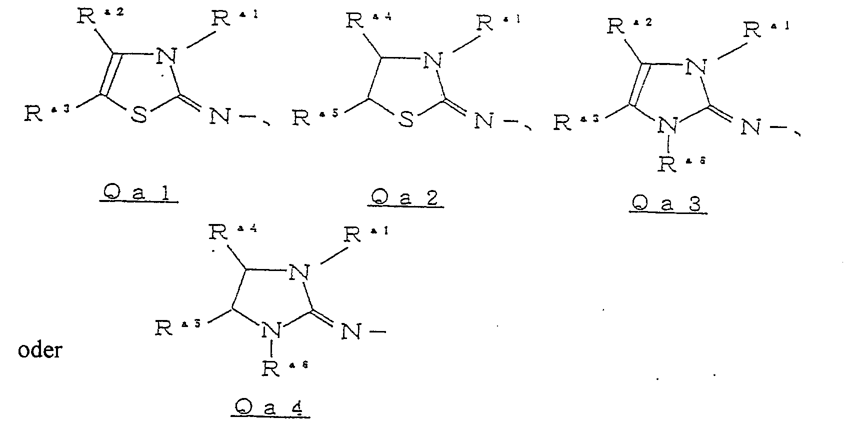 steht.

4. Verfahren zur Herstellung eines Iminosulfonyl-Harnstoffderivats oder dessen Salz nach Anspruch 2, wobei Q für 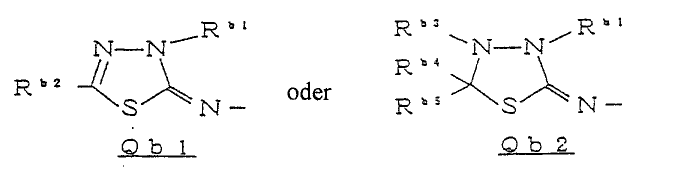 steht.

5. Verfahren zur Herstellung eines Iminosulfonyl-Harnstoffderivats oder dessen Salz nach Anspruch 2, wobei Q für 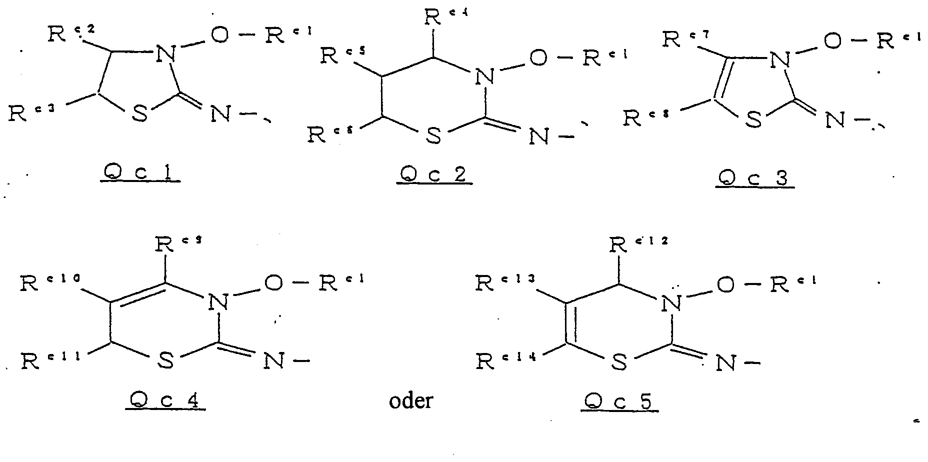 steht.

6. Verfahren zur Herstellung eines Iminosulfonyl-Harnstoffderivats oder dessen Salz nach Anspruch 2, wobei Q für 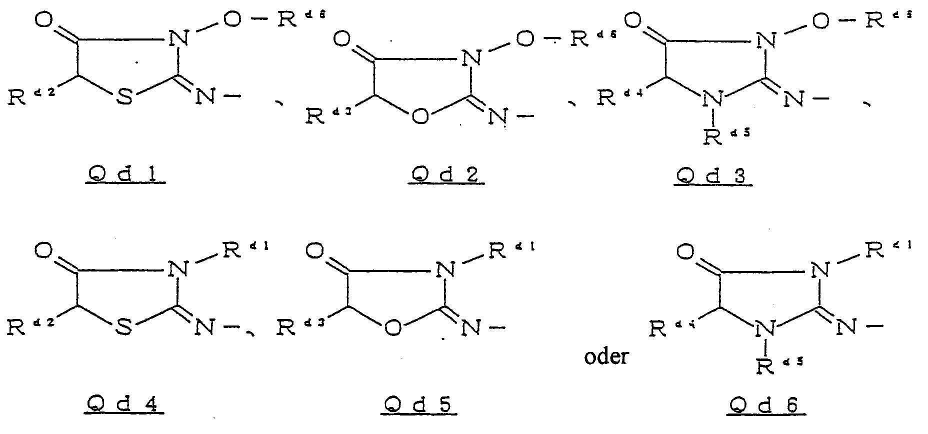 steht.

7. Verfahren zur Herstellung eines Iminosulfonyl-Harnstoffderivats oder dessen Salz nach Anspruch 2, wobei Q für 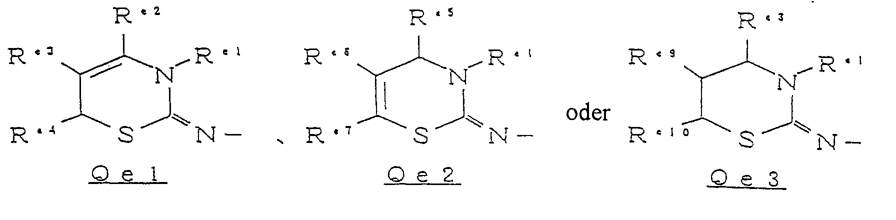 steht.

8. Verfahren zur Herstellung eines Iminosulfonyl-Harnstoffderivats oder dessen Salz nach Anspruch 2, wobei Q für 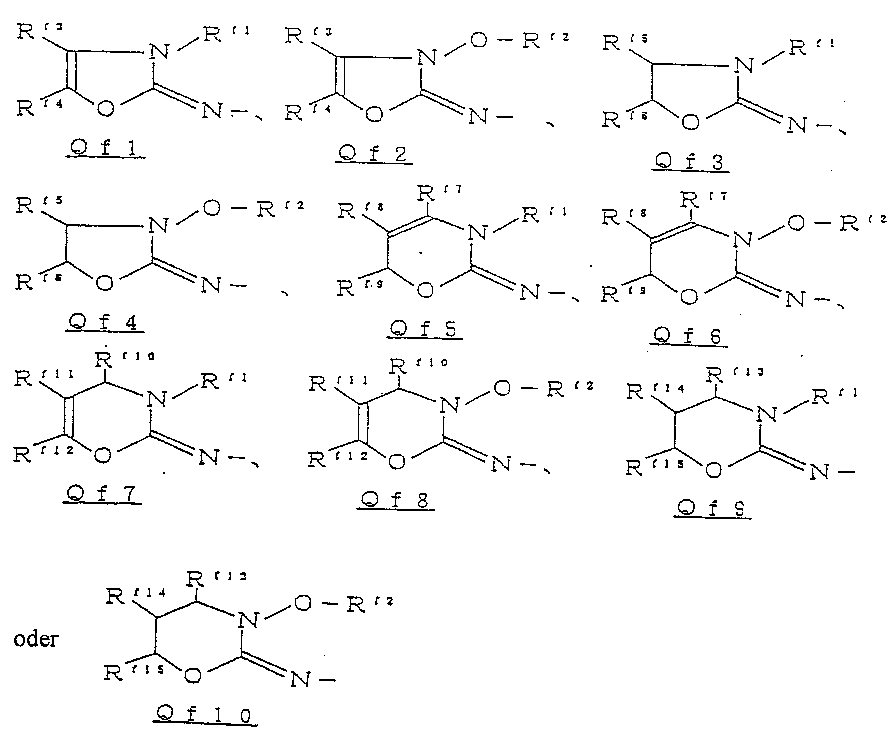 steht.

9. Verfahren zur Herstellung eines Iminosulfonyl-Harnstoffderivats oder dessen Salz nach Anspruch 2, wobei Q für 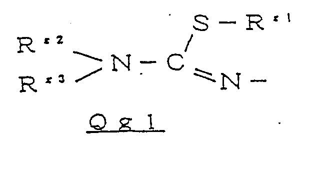 steht.

10. Verfahren zur Herstellung eines Iminosulfonyl-Harnstoffderivats oder eines Salzes davon nach Anspruch 3, wobei R^{a1} für 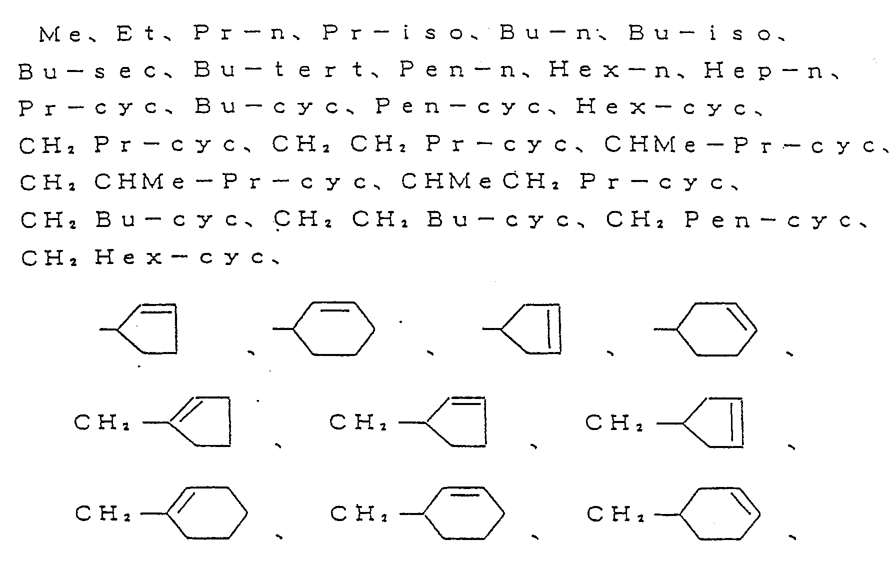 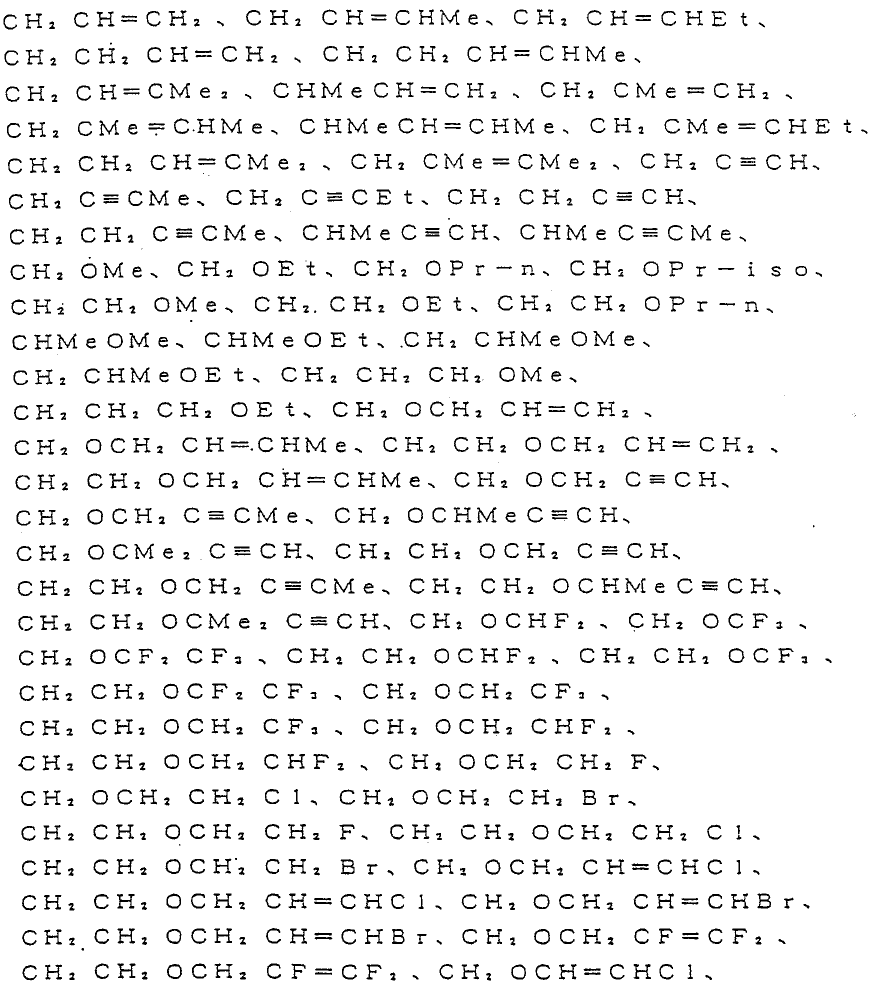 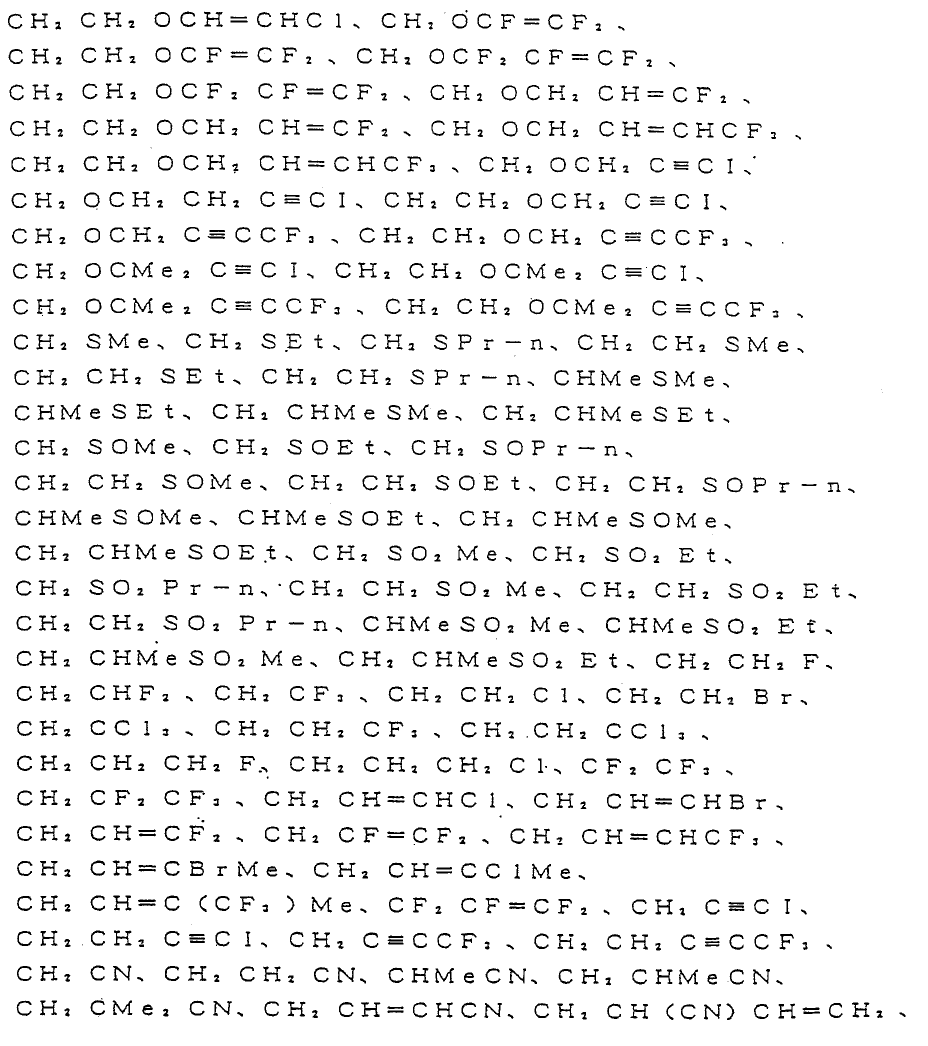 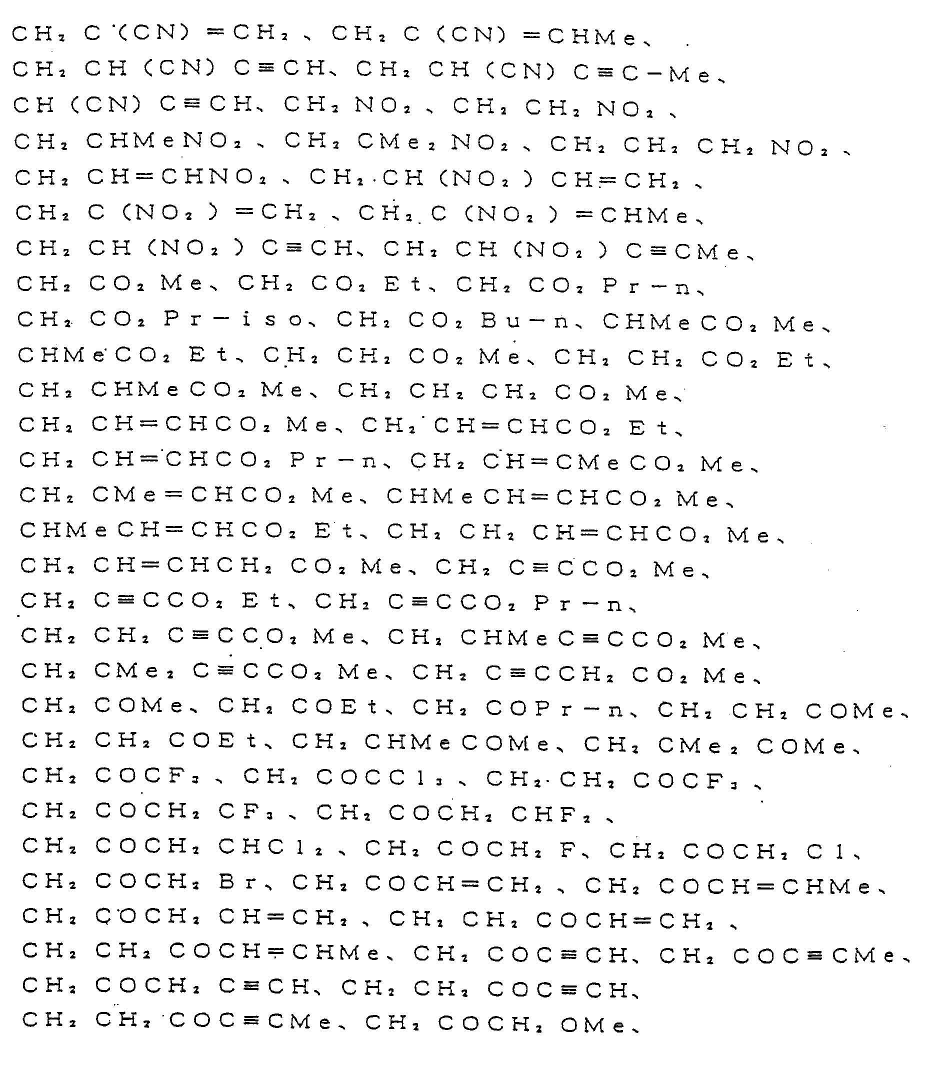 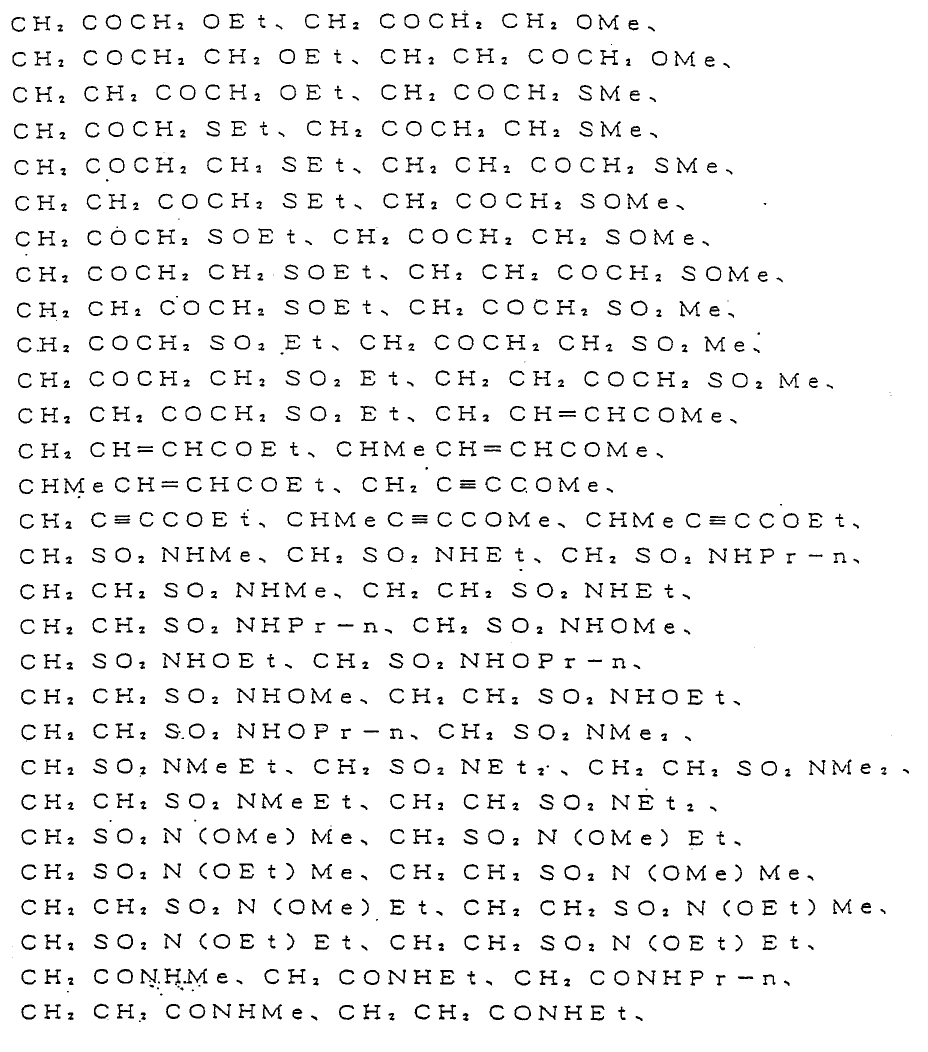 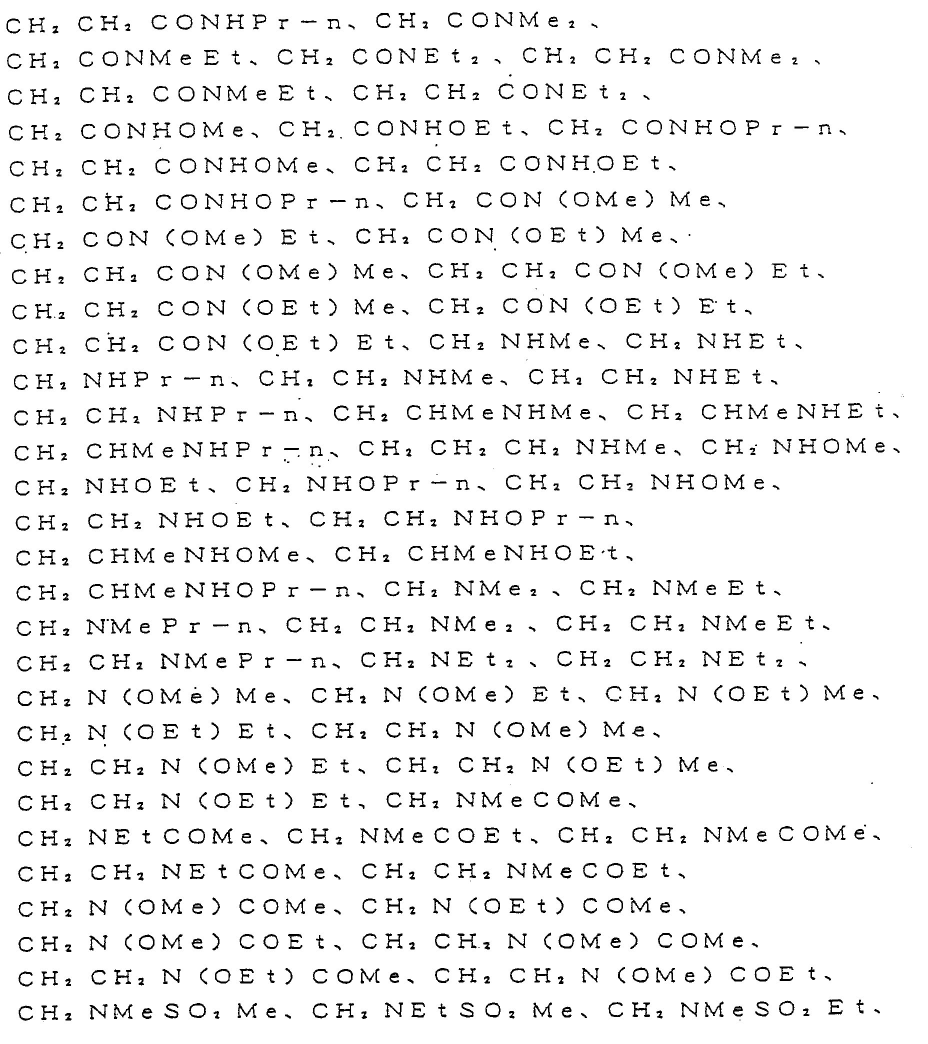 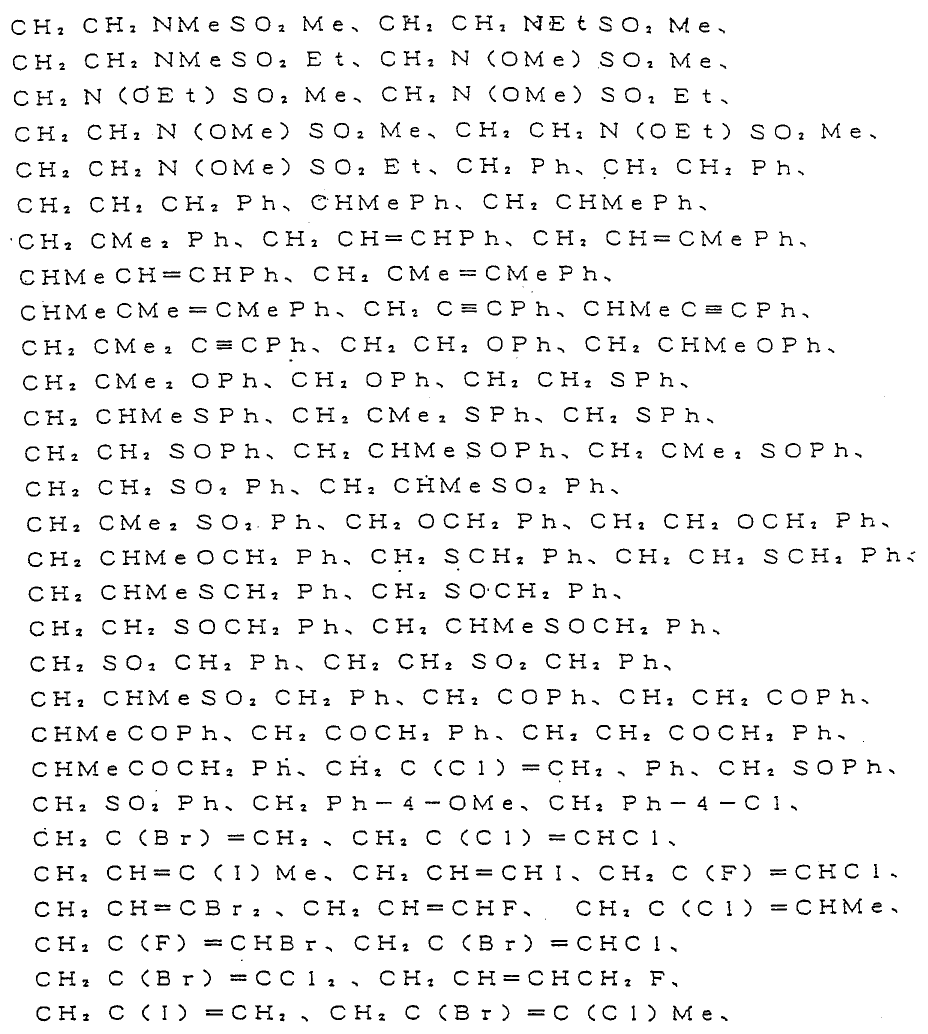 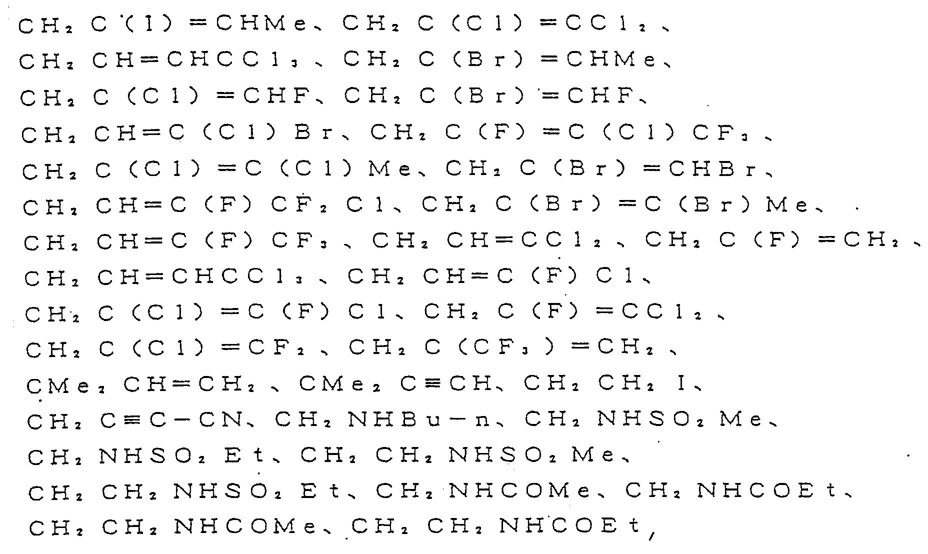 steht,
R^{a2} und R^{a³} für 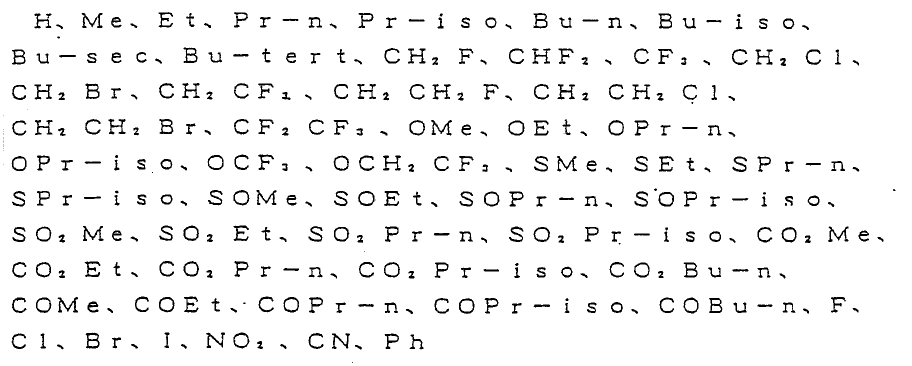 stehen,
B und D für 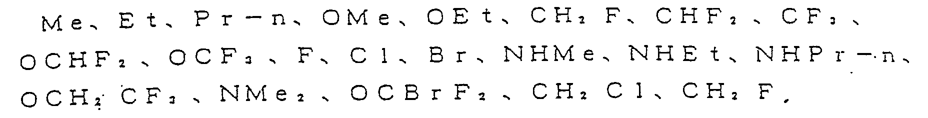 steht.

11. Verfahren zur Herstellung eines Iminosulfonyl-Harnstoffderivats oder eines Salzes davon nach Anspruch 3, wobei R^{a1} für 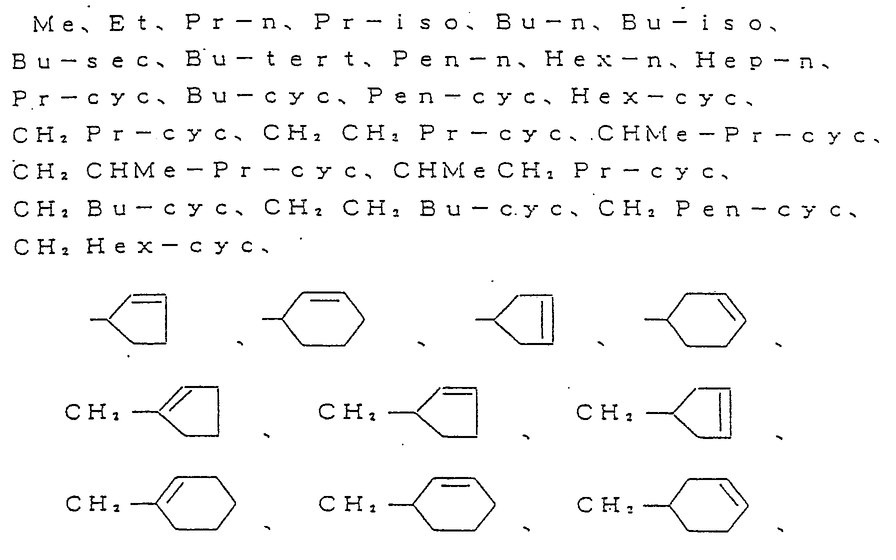 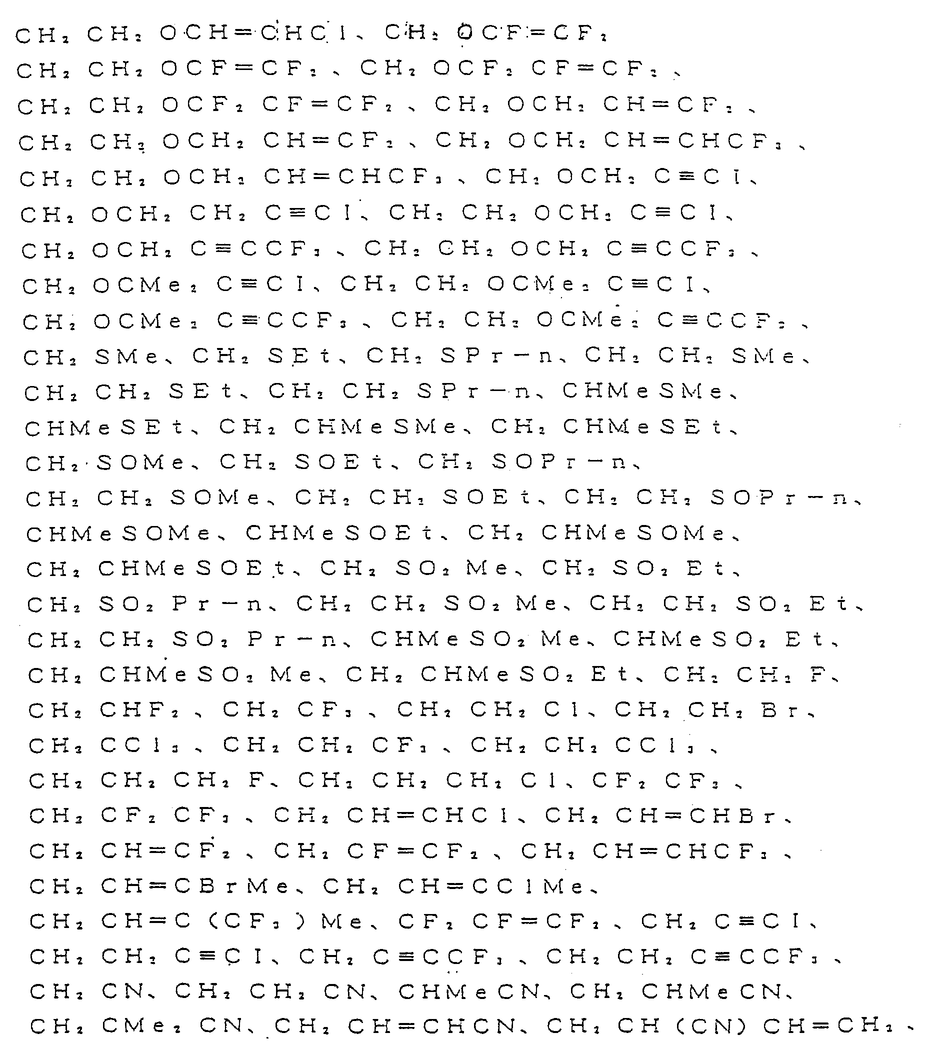 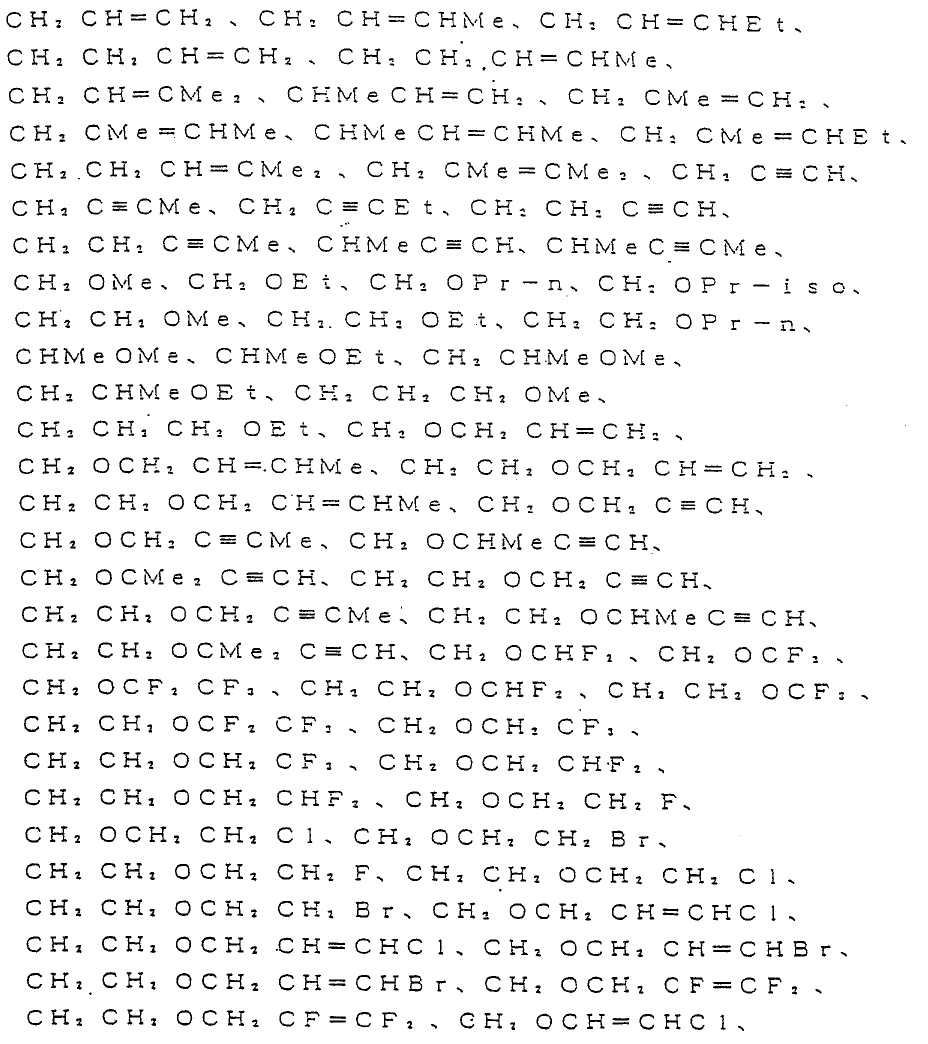 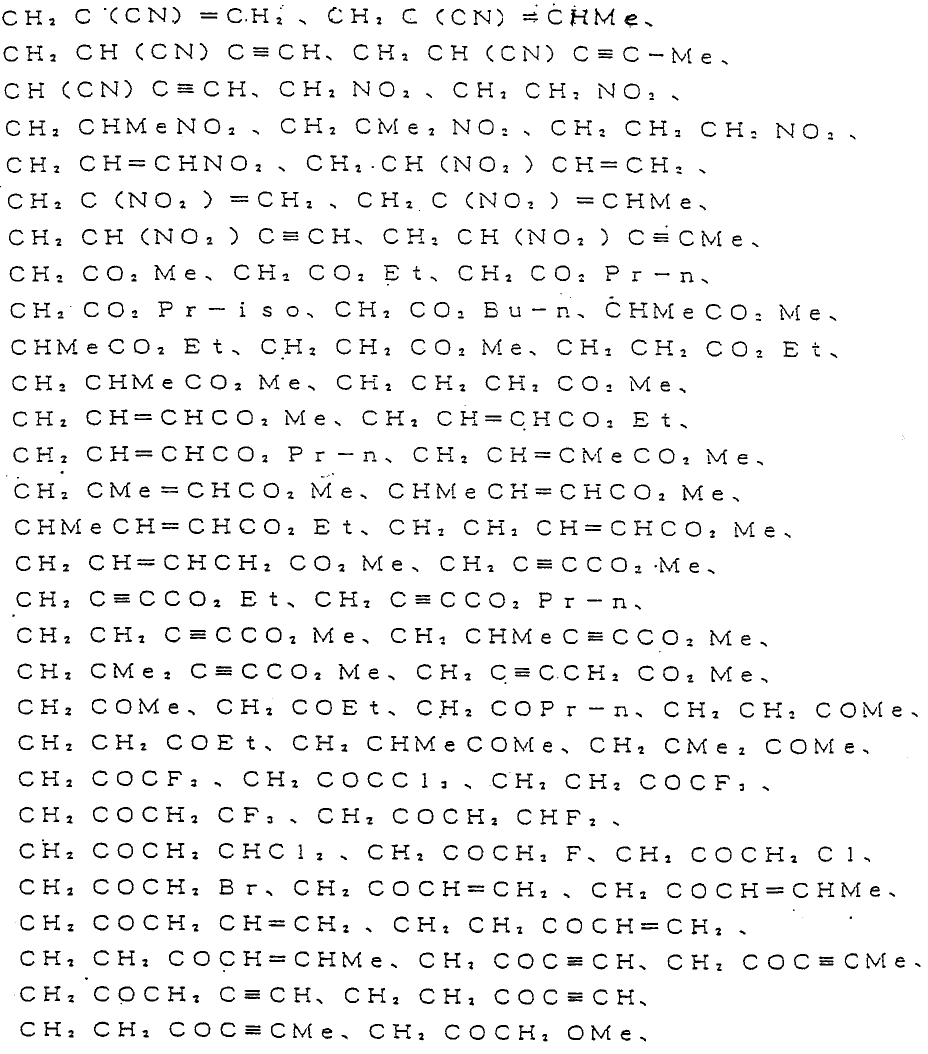 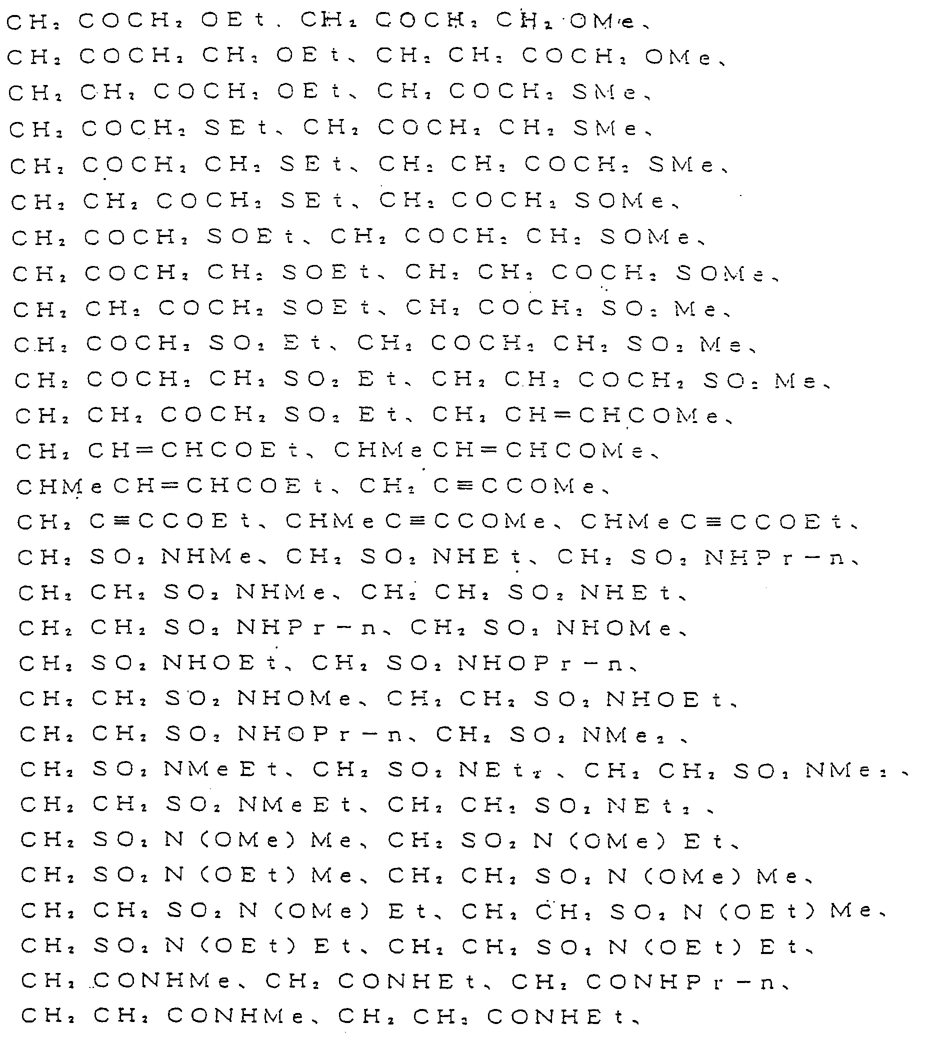 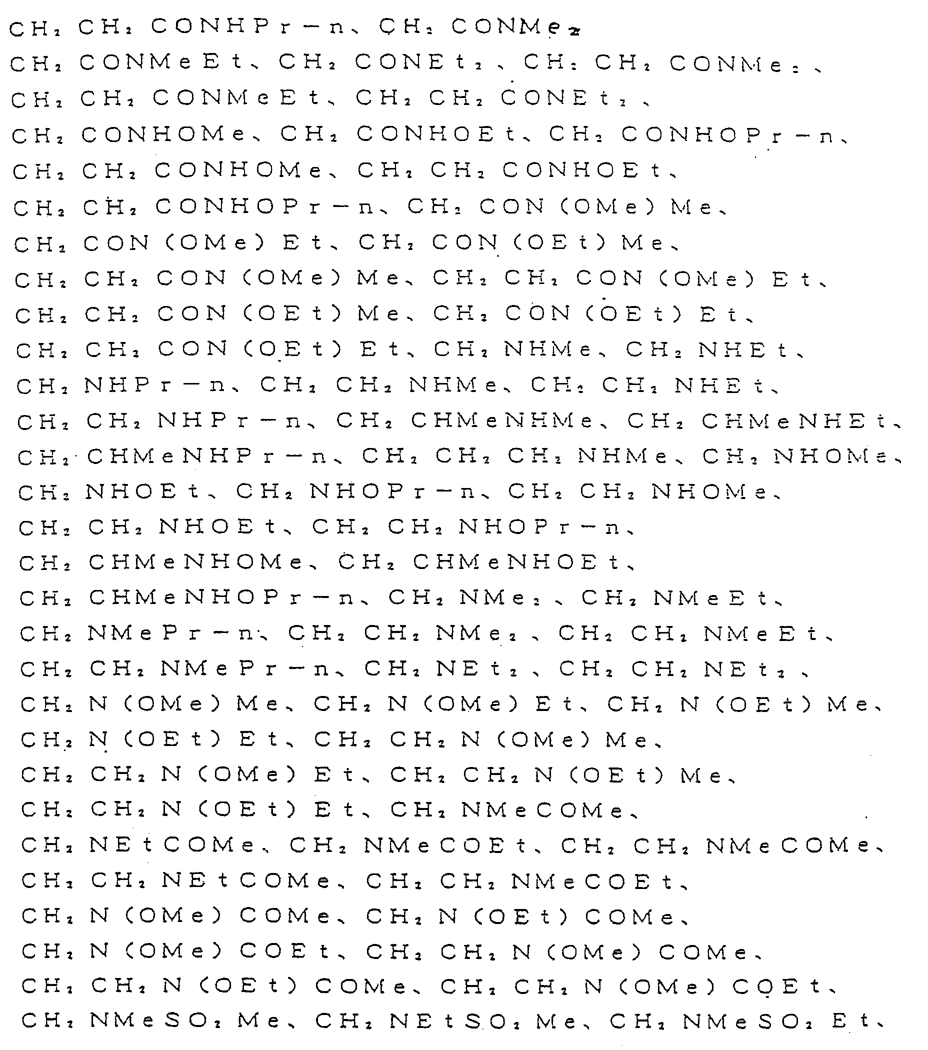 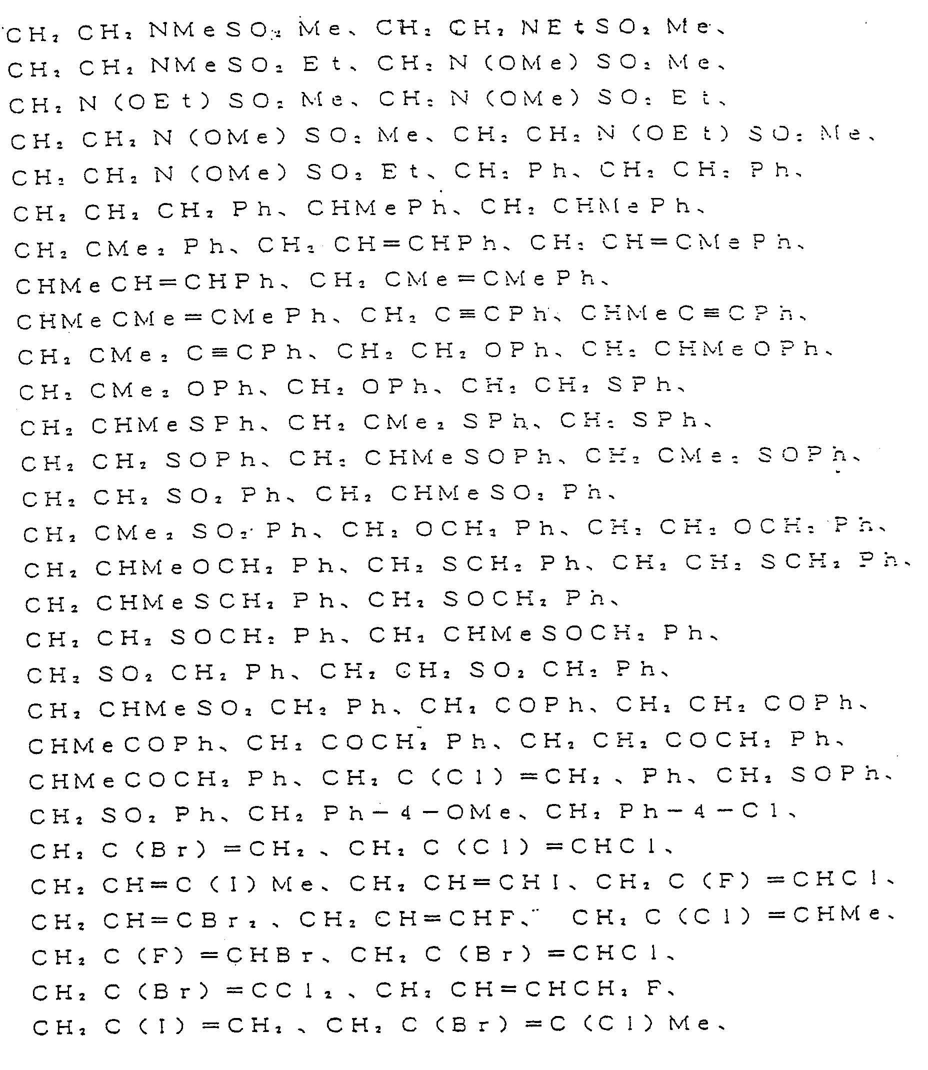 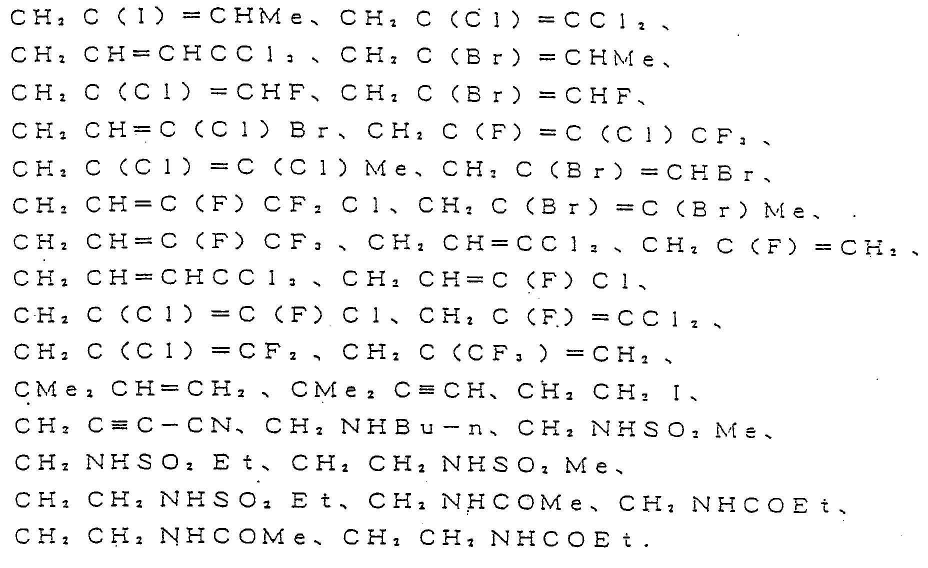 steht.

12. Verfahren zur Herstellung eines Iminosulfonyl-Harnstoffderivats oder eines Salzes davon nach Anspruch 3, wobei R^{a2} und R^{a3} für 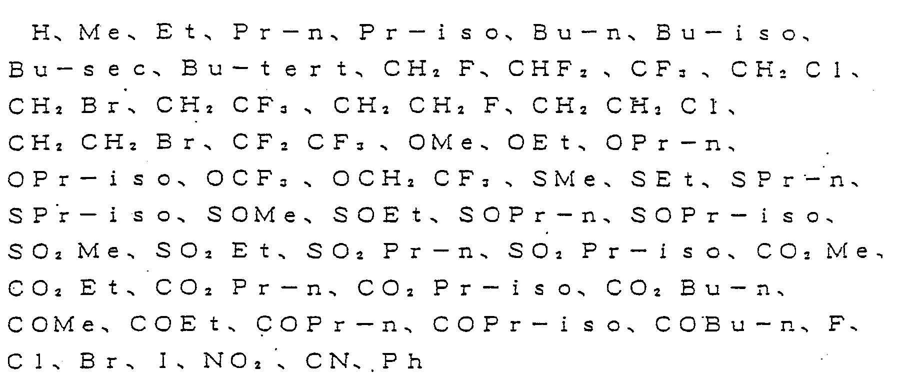 stehen.

13. Verfahren zuer Herstellungs eines Iminosulfonyl-Harnstoffderivats oder eines Salzes davon nach Anspruch 3, wobei B und D für 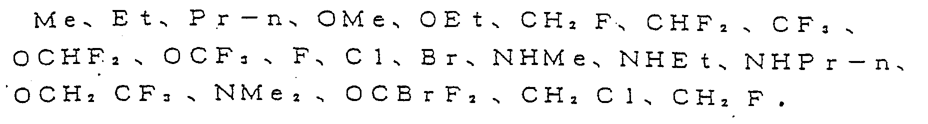 steht.

14. Verfahren zur Herstellung eines Herbizids, das ein Iminosulfonyl-Harnstoffderivat, wie es nach irgendeinem der Ansprüche 1 bis 13 herstellbar ist, wobei dieses Verfahren eine Formulierung des Iminosulfonyl-Harnstoffderivats zusammen mit einem geeigneten Träger umfaßt.

15. Herbizides und wachstumsbekämpfendes Verfahren gegen Unkräuter, das eine Anwendung einer herbizidwirksamen Menge eines Iminosulfonyl-Harnstoffderivats, das nach irgendeinem der Ansprüche 1 bis 13 herstellbar ist, umfaßt.

## Revendications (Revendications pour l'(les) Etat(s) contractant(s) suivant(s): AT, BE, CH, DE, DK, FR, GB, IT, LI, LU, MC, NL, SE)

1. Dérivé d'iminosulfonylurée de la formule (1) ou son sel : 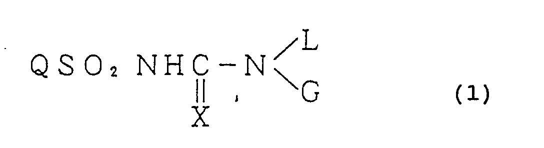 dans laquelle :
- Q représente : 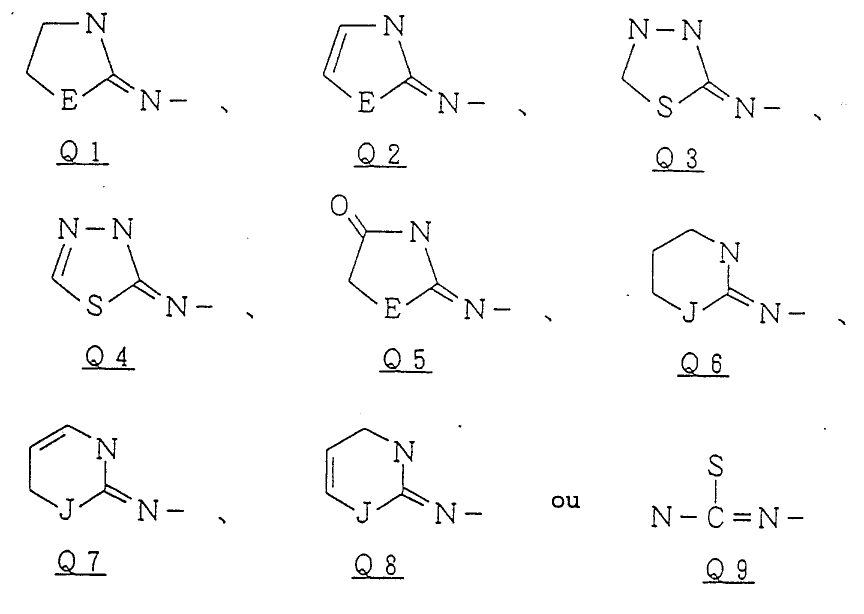 où :
- dans Q1, Q2 et Q5, E représente un atome de soufre, un atome d'oxygène ou un atome d'azote mono-substitué par un substituant autre qu'un atome d'hydrogène ;
- dans Q6, Q7 et Q8, J est un atome de soufre ou un atome d'oxygène ;
- dans Q1 à Q8, un atome d'azote dans le cycle de Q est substitué par un substituant autre qu'un atome d'hydrogène, et un atome de carbone dans le cycle de Q est facultativement substitué ; et
- dans Q9, l'atome de soufre et l'atome d'azote sur l'atome de carbone auquel le groupe imino de Q est lié, sont substitués par des substituants autres que des atomes d'hydrogène ;
- X représente un atome d'oxygène ou un atome de soufre ;
- L est un atome d'hydrogène, un groupe alkyle en C₁₋₆, un groupe alcényle en C₂₋₆ ou un groupe alcynyle en C₂₋₆ ;
- G représente : 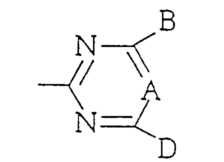
- A est un groupe CH ou un atome d'azote, et
- B et D, qui sont indépendants l'un de l'autre, représentent chacun un groupe alkyle en C₁₋₄, un groupe alcoxy en C₁₋₄, un groupe mono-, di- ou poly-halogéno alkyle en C₁₋₄, un groupe mono-, di- ou poly-halogéno alcoxy en C₁₋₄, un atome d'halogène, un groupe alkylamino en C₁₋₄ ou un groupe di(alkyl en C₁₋₄)amino.

2. Dérivé d'iminosulfonylurée de la formule (1) et son sel, selon la revendication 1 : 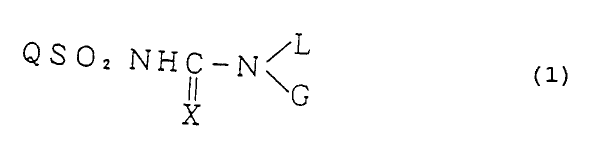 dans laquelle :
- Q représente : 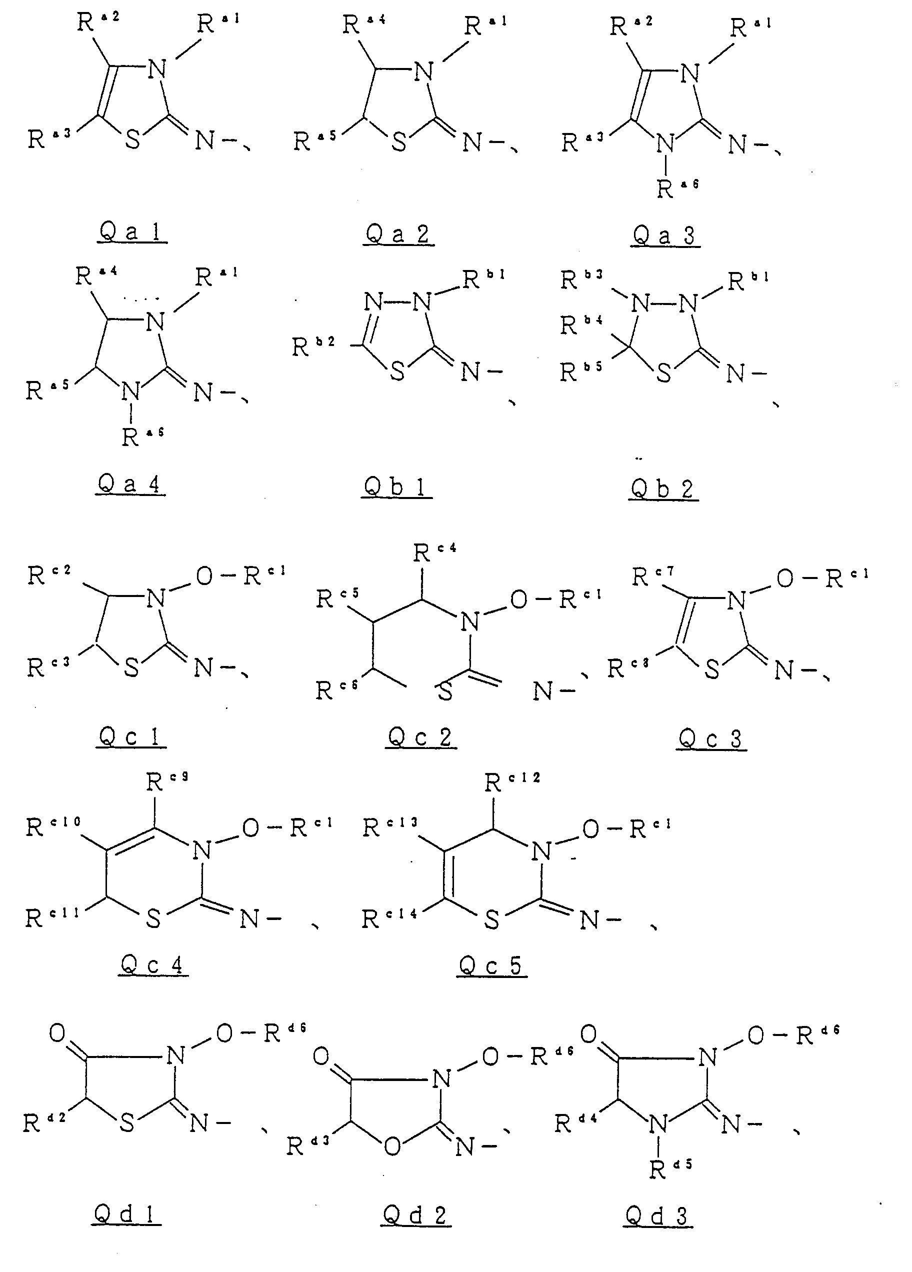 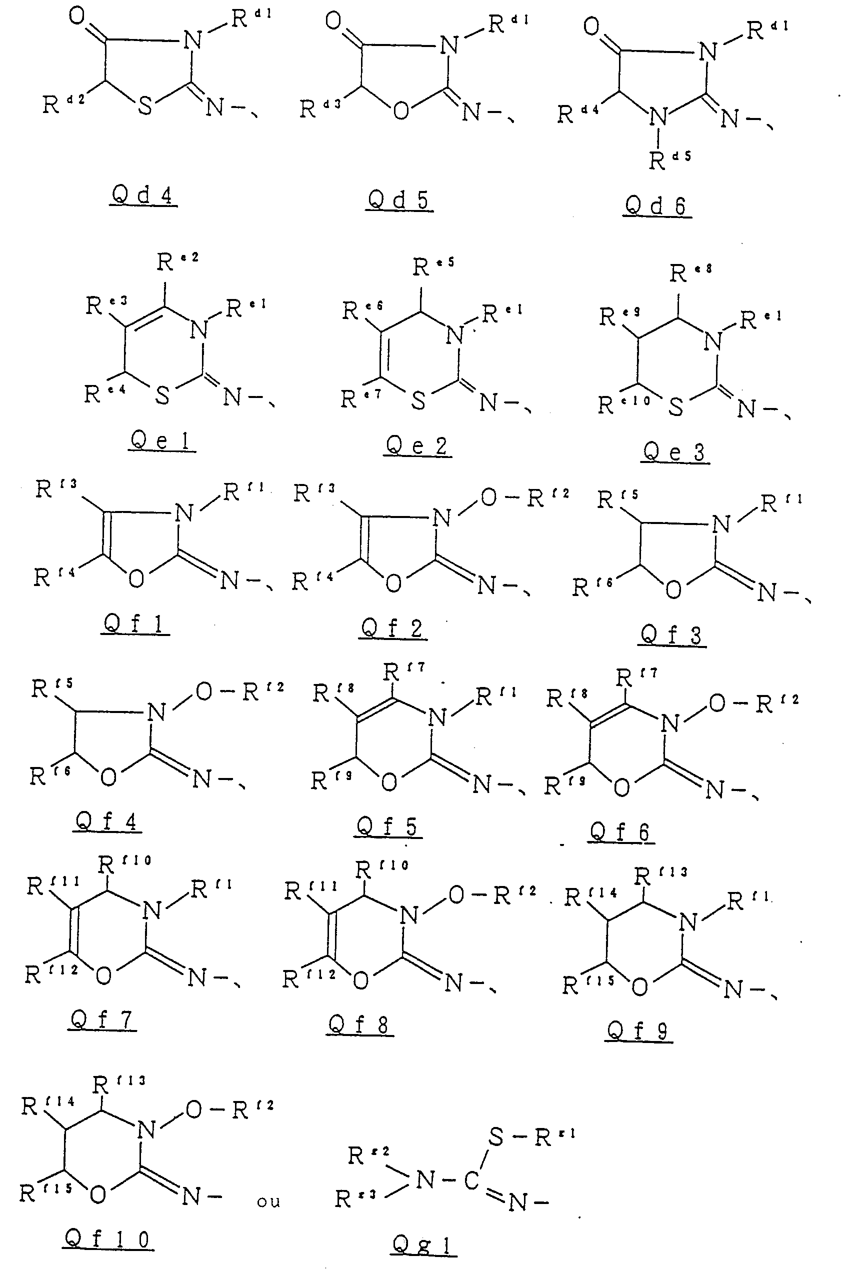
- R^{a1} est un groupe alkyle en C₁₋₈, un groupe cycloalkyle en C₃₋₇, un groupe alkyle en C₁₋₆ substitué par un groupe cycloalkyle en C₃₋₇, un groupe cycloalcényle en C₃₋₇, un groupe alkyle en C₁₋₆ substitué par un groupe cycloalcényle en C₃₋₇, un groupe alcényle en C₂₋₈, un groupe alcynyle en C₂₋₈, un groupe alkyle en C₁₋₆ substitué par un groupe alcoxy en C₁₋₆, un groupe alkyle en C₁₋₆ substitué par un groupe alcényloxy en C₂₋₆, un groupe alkyle en C₁₋₆ substitué par un groupe alcynyloxy en C₂₋₆, un groupe alkyle en C₁₋₆ substitué par un groupe mono-, di- ou poly-halogénoalcoxy en C₁₋₆, un groupe alkyle en C₁₋₆ substitué par un groupe mono-, di- ou poly-halogénoalcényloxy en C₂₋₆, un groupe alkyle en C₁₋₆ substitué par un groupe mono-, di- ou poly-halogénoalcynyloxy en C₂₋₆, un groupe alkyle en C₁₋₆ substitué par un groupe alkylthio en C₁₋₆, un groupe alkyle en C₁₋₆ substitué par un groupe alkylsulfinyle en C₁₋₆, un groupe alkyle en C₁₋₆ substitué par un groupe alkylsulfonyle en C₁₋₆, un groupe mono-, di- ou poly-halogénoalkyle en C₁₋₈, un groupe mono-, di-, ou poly-halogénoalcényle en C₂₋₈, un groupe mono-, di-, ou poly-halogénoalcynyle en C₂₋₈, un groupe alkyle en C₁₋₆ substitué par un groupe cyano, un groupe alcényle en C₂₋₆ substitué par un groupe cyano, un groupe alcynyle en C₂₋₆ substitué par un groupe cyano, un groupe alkyle en C₁₋₆ substitué par un groupe nitro, un groupe alcényle en C₂₋₆ substitué par un groupe nitro, un groupe alcynyle en C₂₋₆ substitué par un groupe nitro, un groupe alkyle en C₁₋₆ substitué par un groupe alcoxycarbonyle en C₂₋₇, un groupe alcényle en C₂₋₆ substitué par un groupe alcoxycarbonyle en C₂₋₇, un groupe alcynyle en C₂₋₆ substitué par un groupe alcoxycarbonyle en C₂₋₇, un groupe alkyle en C₁₋₆ substitué par un groupe alkylcarbonyle en C₂₋₇, un groupe alkyle en C₁₋₆ substitué par un groupe mono-, di- ou poly-halogénoalkylcarbonyle en C₂₋₇, un groupe alkyle en C₁₋₆ substitué par un groupe alcénylcarbonyle en C₃₋₇, un groupe alkyle en C₁₋₆ substitué par un groupe alcynylcarbonyle en C₃₋₇, un groupe alkyle en C₁₋₆ substitué par un groupe alkylcarbonyle en C₂₋₅ substitué par un groupe alcoxy en C₁₋₄, un groupe alkyle en C₁₋₆ substitué par un groupe alkylcarbonyle en C₂₋₅ substitué par un groupe alkylthio en C₁₋₄, un groupe alkyle en C₁₋₆ substitué par un groupe alkylcarbonyle en C₂₋₅ substitué par un groupe alkylsulfinyle en C₁₋₄, un groupe alkyle en C₁₋₆ substitué par un groupe alkylcarbonyle en C₂₋₅ substitué par un groupe alkylsulfonyle en C₁₋₄, un groupe alcényle en C₂₋₆ substitué par un groupe alkylcarbonyle en C₂₋₇, un groupe alcynyle en C₂₋₆ substitué par un groupe alkylcarbonyle en C₂₋₇, un groupe alkyle en C₁₋₆ substitué par un groupe alkylsulfamoyle en C₁₋₆, un groupe alkyle en C₁₋₆ substitué par un groupe alcoxysulfamoyle en C₁₋₆, un groupe alkyle en C₁₋₆ substitué par un groupe di(alkyl en C₁₋₃)sulfamoyle, un groupe alkyle en C₁₋₆ substitué par un groupe N-(alkyl en C₁₋₃)-N-(alcoxy en C₁₋₃)sulfamoyle, un groupe alkyle en C₁₋₆ substitué par un groupe alkylcarbamoyle en C₂₋₇, un groupe alkyle en C₁₋₆ substitué par un groupe di(alkyl en C₁₋₃)carbamoyle, un groupe alkyle en C₁₋₆ substitué par un groupe alcoxycarbamoyle en C₂₋₇, un groupe alkyle en C₁₋₆ substitué par un groupe N-(alkyl en C₁₋₃)-N-(alcoxy en C₁₋₃)carbamoyle, un groupe alkyle en C₁₋₆ substitué par un groupe alkylamino en C₁₋₆, un groupe alkyle en C₁₋₆ substitué par un groupe alcoxyamino en C₁₋₆, un groupe alkyle en C₁₋₆ substitué par un groupe di(alkyl en C₁₋₃)amino, un groupe alkyle en C₁₋₆ substitué par un groupe N-(alkyl en C₁₋₃)-N-(alcoxy en C₁₋₃)amino, un groupe alkyle en C₁₋₆ substitué par un groupe N-(alkylcarbonyl en C₂₋₇)-N-(alkyl en C₁₋₆)amino, un groupe alkyle en C₁₋₆ substitué par un groupe N-(alkylcarbonyl en C₂₋₇)-N-(alcoxy en C₁₋₆)-amino, un groupe alkyle en C₁₋₆ substitué par un groupe N-(alkylsulfonyl en C₁₋₆)-N-(alkyl en C₁₋₆)amino, un groupe alkyle en C₁₋₆ substitué par un groupe N-(alkylsulfonyl en C₁₋₆)-N-(alcoxy en C₁₋₆)amino, un groupe phényle (à la condition qu'un tel groupe phényle puisse être substitué par un ou plusieurs substituants choisis dans le groupe constitué par un atome d'halogène, un groupe trifluorométhyle, un groupe nitro, un groupe alkyle en C₁₋₆, un groupe alcoxy en C₁₋₆ et un groupe alcoxycarbonyle en C₂₋₇), un groupe alkyle en C₁₋₆ substitué par un groupe phényle (à la condition qu'un tel groupe phényle puisse être substitué par un ou plusieurs substituants choisis dans le groupe constitué par un atome d'halogène, un groupe trifluorométhyle, un groupe nitro, un groupe alkyle en C₁₋₆, un groupe alcoxy en C₁₋₆ et un groupe alcoxycarbonyle en C₂₋₇), un groupe alcényle en C₂₋₇ substitué par un groupe phényle (à la condition qu'un tel groupe phényle puisse être substitué par un ou plusieurs substituants choisis dans le groupe constitué par un atome d'halogène, un groupe trifluorométhyle, un groupe nitro, un groupe alkyle en C₁₋₆, un groupe alcoxy en C₁₋₆ et un groupe alcoxycarbonyle en C₂₋₇), un groupe alcynyle en C₂₋₆ substitué par un groupe phényle (à la condition qu'un tel groupe phényle puisse être substitué par un ou plusieurs substituants choisis dans le groupe constitué par un atome d'halogène, un groupe trifluorométhyle, un groupe nitro, un groupe alkyle en C₁₋₆, un groupe alcoxy en C₁₋₆ et un groupe alcoxycarbonyle en C₂₋₇), un groupe alkyle en C₁₋₆ substitué par un groupe phénoxy (à la condition qu'un tel groupe phénoxy puisse être substitué par un ou plusieurs substituants choisis dans le groupe constitué par un atome d'halogène, un groupe trifluorométhyle, un groupe nitro, un groupe alkyle en C₁₋₆, un groupe alcoxy en C₁₋₆ et un groupe alcoxycarbonyle en C₂₋₇), un groupe alkyle en C₁₋₆ substitué par un groupe phénylthio (à la condition qu'un tel groupe phénylthio puisse être substitué par un ou plusieurs substituants choisis dans le groupe constitué par un atome d'halogène, un groupe trifluorométhyle, un groupe nitro, un groupe alkyle en C₁₋₆, un groupe alcoxy en C₁₋₆ et un groupe alcoxycarbonyle en C₂₋₇), un groupe alkyle en C₁₋₆ substitué par un groupe phénylsulfinyle (à la condition qu'un tel groupe phénylsulfinyle puisse être substitué par un ou plusieurs substituants choisis dans le groupe constitué par un atome d'halogène, un groupe trifluorométhyle, un groupe nitro, un groupe alkyle en C₁₋₆, un groupe alcoxy en C₁₋₆ et un groupe alcoxycarbonyle en C₂₋₇), un groupe alkyle en C₁₋₆ substitué par un groupe phénylsulfonyle (à la condition qu'un tel groupe phénylsulfonyle puisse être substitué par un ou plusieurs substituants choisis dans le groupe constitué par un atome d'halogène, un groupe trifluorométhyle, un groupe nitro, un groupe alkyle en C₁₋₆, un groupe alcoxy en C₁₋₆ et un groupe alcoxycarbonyle en C₂₋₇), un groupe alkyle en C₁₋₆ substitué par un groupe benzyloxy (à la condition que le groupe phényle d'un tel groupe benzyloxy puisse être substitué par un ou plusieurs substituants choisis dans le groupe constitué par un atome d'halogène, un groupe trifluorométhyle, un groupe nitro, un groupe alkyle en C₁₋₆, un groupe alcoxy en C₁₋₆ et un groupe alcoxycarbonyle en C₂₋₇), un groupe alkyle en C₁₋₆ substitué par un groupe benzylthio (à la condition que le groupe phényle d'un tel groupe benzylthio puisse être substitué par un ou plusieurs substituants choisis dans le groupe constitué par un atome d'halogène, un groupe trifluorométhyle, un groupe nitro, un groupe alkyle en C₁₋₆, un groupe alcoxy en C₁₋₆ et un groupe alcoxycarbonyle en C₂₋₇), un groupe alkyle en C₁₋₆ substitué par un groupe benzylsulfinyle (à la condition que le groupe phényle d'un tel groupe benzylsulfinyle puisse être substitué par un ou plusieurs substituants choisis dans le groupe constitué par un atome d'halogène, un groupe trifluorométhyle, un groupe nitro, un groupe alkyle en C₁₋₆, un groupe alcoxy en C₁₋₆ et un groupe alcoxycarbonyle en C₂₋₇), un groupe alkyle en C₁₋₆ substitué par un groupe benzylsulfonyle (à la condition que le groupe phényle d'un tel groupe benzylsulfonyle puisse être substitué par un ou plusieurs substituants choisis dans le groupe constitué par un atome d'halogène, un groupe trifluorométhyle, un groupe nitro, un groupe alkyle en C₁₋₆, un groupe alcoxy en C₁₋₆ et un groupe alcoxycarbonyle en C₂₋₇), un groupe alkyle en C₁₋₆ substitué par un groupe phénylcarbonyle (à la condition qu'un tel groupe phénylcarbonyle puisse être substitué par un ou plusieurs substituants choisis dans le groupe constitué par un atome d'halogène, un groupe trifluorométhyle, un groupe nitro, un groupe alkyle en C₁₋₆, un groupe alcoxy en C₁₋₆ et un groupe alcoxycarbonyle en C₂₋₇), un groupe alkyle en C₁₋₆ substitué par un groupe benzylcarbonyle (à la condition que le groupe phényle d'un tel groupe benzylcarbonyle puisse être substitué par un ou plusieurs substituants choisis dans le groupe constitué par un atome d'halogène, un groupe trifluorométhyle, un groupe nitro, un groupe alkyle en C₁₋₆, un groupe alcoxy en C₁₋₆ et un groupe alcoxycarbonyle en C₂₋₇), un groupe alkyle en C₁₋₆ substitué par un groupe amino substitué par un groupe alkylsulfonyle en C₁₋₄, ou un groupe alkyle en C₁₋₆ substitué par un groupe amino substitué par un groupe alkylcarbonyle en C₂₋₄ ;
- R^{a2} et R^{a3}, qui sont indépendants l'un de l'autre, représentent chacun un atome d'hydrogène, un groupe alkyle en C₁₋₆, un groupe mono-, di- ou poly-halogénoalkyle en C₁₋₆, un groupe alcoxy en C₁₋₆, un groupe mono-, di- ou poly-halogénoalcoxy en C₁₋₆, un groupe alkylthio en C₁₋₆, un groupe alkylsulfinyle en C₁₋₆, un groupe alkylsulfonyle en C₁₋₆, un groupe alcoxycarbonyle en C₂₋₇, un groupe alkylcarbonyle en C₂₋₇, un atome d'halogène, un groupe nitro, un groupe cyano, ou un groupe phényle (à la condition qu'un tel groupe phényle puisse être substitué par un ou plusieurs substituants choisis dans le groupe constitué par un atome d'halogène, un groupe trifluorométhyle, un groupe nitro, un groupe alkyle en C₁₋₆, un groupe alcoxy en C₁₋₆ et un groupe alcoxycarbonyle en C₂₋₇) ;
- R^{a4} et R^{a5}, qui sont indépendants l'un de l'autre, représentent chacun un atome d'hydrogène, un groupe alkyle en C₁₋₈, un groupe alcényle en C₂₋₈, un groupe alcynyle en C₂₋₈, ou un groupe phényle (à la condition qu'un tel groupe phényle puisse être substitué par un ou plusieurs substituants choisis dans le groupe constitué par un atome d'halogène, un groupe trifluorométhyle, un groupe nitro, un groupe alkyle en C₁₋₆, un groupe alcoxy en C₁₋₆ et un groupe alcoxycarbonyle en C₂₋₇) ;
- R^{a6} est un groupe alkyle en C₁₋₈, un groupe alcényle en C₂₋₈, un groupe alcynyle en C₂₋₈ ou un groupe phényle (à la condition qu'un tel groupe phényle puisse être substitué par un ou plusieurs substituants choisis dans le groupe constitué par un atome d'halogène, un groupe trifluorométhyle, un groupe nitro, un groupe alkyle en C₁₋₆, un groupe alcoxy en C₁₋₆ et un groupe alcoxycarbonyle en C₂₋₇) ;
- R^{b1} est un groupe alkyle en C₁₋₈, un groupe cycloalkyle en C₃₋₇, un groupe alkyle en C₁₋₆ substitué par un groupe cycloalkyle en C₃₋₇, un groupe cycloalcényle en C₃₋₇, un groupe alkyle en C₁₋₆ substitué par un groupe cycloalcényle en C₃₋₇, un groupe alcényle en C₂₋₈, un groupe alcynyle en C₂₋₈, un groupe alkyle en C₁₋₆ substitué par un groupe alcoxy en C₁₋₆, un groupe alkyle en C₁₋₆ substitué par un groupe alcényloxy en C₂₋₆, un groupe alkyle en C₁₋₆ substitué par un groupe alcynyloxy en C₂₋₆, un groupe alkyle en C₁₋₆ substitué par un groupe mono-, di- ou poly-halogénoalcoxy en C₁₋₆, un groupe alkyle en C₁₋₆ substitué par un groupe mono-, di-, ou poly-halogénoalcényloxy en C₂₋₆, un groupe alkyle en C₁₋₆ substitué par un groupe mono-, di-, ou poly-halogénoalcynyloxy en C₂₋₆, un groupe alkyle en C₁₋₆ substitué par un groupe alkylthio en C₁₋₆, un groupe alkyle en C₁₋₆ substitué par un groupe alkylsufinyle en C₁₋₆, un groupe alkyle en C₁₋₆ substitué par un groupe alkylsulfonyle en C₁₋₆, un groupe mono-, di- ou poly-halogénoalkyle en C₁₋₈, un groupe mono-, di-, ou poly-halogénoalcényle en C₂₋₈, un groupe mono-, di- ou poly-halogénoalcynyle en C₂₋₈, un groupe alkyle en C₁₋₆ substitué par un groupe cyano, un groupe alcényle en C₂₋₆ substitué par un groupe cyano, un groupe alcynyle en C₂₋₆ substitué par un groupe cyano, un groupe alkyle en C₁₋₆ substitué par un groupe nitro, un groupe alcényle en C₂₋₆ substitué par un groupe nitro, un groupe alcynyle en C₂₋₆ substitué par un groupe nitro, un groupe alkyle en C₁₋₆ substitué par un groupe alcoxycarbonyle en C₂₋₇, un groupe alcényle en C₂₋₆ substitué par un groupe alcoxycarbonyle en C₂₋₇, un groupe alcynyle en C₂₋₆ substitué par un groupe alcoxycarbonyle en C₂₋₇, un groupe alkyle en C₁₋₆ substitué par un groupe alkylcarbonyle en C₂₋₇, un groupe alkyle en C₁₋₆ substitué par un groupe mono-, di- ou poly-halogénoalkylcarbonyle en C₂₋₇, un groupe alkyle en C₁₋₆ substitué par un groupe alcénylcarbonyle en C₃₋₇, un groupe alkyle en C₁₋₆ substitué par un groupe alcynylcarbonyle en C₃₋₇, un groupe alkyle en C₁₋₆ substitué par un groupe alkycarbonyle en C₂₋₅ substitué par un groupe alcoxy en C₁₋₄, un groupe alkyle en C₁₋₆ substitué par un groupe alkylcarbonyle en C₂₋₅ substitué par un groupe alkylthio en C₁₋₄, un groupe alkyle en C₁₋₆ substitué par un groupe alkylcarbonyle en C₂₋₅ substitué par un groupe alkylsulfinyle en C₁₋₄, un groupe alkyle en C₁₋₆ substitué par un groupe alkylcarbonyle en C₂₋₅ substitué par un groupe alkylsulfonyle en C₁₋₄, un groupe alcényle en C₂₋₆ substitué par un groupe alkylcarbonyle en C₂₋₇, un groupe alcynyle en C₂₋₆ substitué par un groupe alkylcarbonyle en C₂₋₇, un groupe alkyle en C₁₋₆ substitué par un groupe alkylsulfamoyle en C₁₋₆, un groupe alkyle en C₁₋₆ substitué par un groupe alcoxysulfamoyle en C₁₋₆, un groupe alkyle en C₁₋₆ substitué par un groupe di(alkyl en C₁₋₃)sulfamoyle, un groupe alkyle en C₁₋₆ substitué par un groupe N-(alkyl en C₁₋₃)-N-(alcoxy en C₁₋₃)sulfamoyle, un groupe alkyle en C₁₋₆ substitué par un groupe alkylcarbamoyle en C₂₋₇, un groupe alkyle en C₁₋₆ substitué par un groupe di(alkyl en C₁₋₃)carbamoyle, un groupe alkyle en C₁₋₆ substitué par un groupe alcoxycarbamoyle en C₂₋₇, un groupe alkyle en C₁₋₆ substitué par un groupe N-(alkyl en C₁₋₃)-N-(alcoxy en C₁₋₃)carbamoyle, un groupe alkyle en C₁₋₆ substitué par un groupe alkylamino en C₁₋₆, un groupe alkyle en C₁₋₆ substitué par un groupe alcoxyamino en C₁₋₆, un groupe alkyle en C₁₋₆ substitué par un groupe di(alkyl en C₁₋₃)amino, un groupe alkyle en C₁₋₆ substitué par un groupe N-(alkyl en C₁₋₃)-N-(alcoxy en C₁₋₃)amino, un groupe alkyle en C₁₋₆ substitué par un groupe N-(alkylcarbonyl en C₂₋₇)-N-(alkyl en C₁₋₆)amino, un groupe alkyle en C₁₋₆ substitué par un groupe N-(alkylcarbonyl en C₂₋₇)-N-(alcoxy en C₁₋₆)amino, un groupe alkyle en C₁₋₆ substitué par un groupe N-(alkylsulfonyl en C₁₋₆)-N-(alkyl en C₁₋₆)amino, un groupe alkyle en C₁₋₆ substitué par un groupe N-(alkylsulfonyl en C₁₋₆)-N-(alcoxy en C₁₋₆)amino, un groupe phényle (à la condition qu'un tel groupe phényle puisse être substitué par un ou plusieurs substituants choisis dans le groupe constitué par un atome d'halogène, un groupe trifluorométhyle, un groupe nitro, un groupe alkyle en C₁₋₆, un groupe alcoxy en C₁₋₆ et un groupe alcoxycarbonyle en C₂₋₇), un groupe alkyle en C₁₋₆ substitué par un groupe phényle (à la condition qu'un tel groupe phényle puisse être substitué par un ou plusieurs substituants choisis dans le groupe constitué par un atome d'halogène, un groupe trifluorométhyle, un groupe nitro, un groupe alkyle en C₁₋₆, un groupe alcoxy en C₁₋₆ et un groupe alcoxycarbonyle en C₂₋₇), un groupe alcényle en C₂₋₇ substitué par un groupe phényle (à la condition qu'un tel groupe phényle puisse être substitué par un ou plusieurs substituants choisis dans le groupe constitué par un atome d'halogène, un groupe trifluorométhyle, un groupe nitro, un groupe alkyle en C₁₋₆, un groupe alcoxy en C₁₋₆ et un groupe alcoxycarbonyle en C₂₋₇), un groupe alcynyle en C₂₋₆ substitué par un groupe phényle (à la condition qu'un tel groupe phényle puisse être substitué par un ou plusieurs substituants choisis dans le groupe constitué par un atome d'halogène, un groupe trifluorométhyle, un groupe nitro, un groupe alkyle en C₁₋₆, un groupe alcoxy en C₁₋₆ et un groupe alcoxycarbonyle en C₂₋₇), un groupe alkyle en C₁₋₆ substitué par un groupe phénoxy (à la condition qu'un tel groupe phénoxy puisse être substitué par un ou plusieurs substituants choisis dans le groupe constitué par un atome d'halogène, un groupe trifluorométhyle, un groupe nitro, un groupe alkyle en C₁₋₆, un groupe alcoxy en C₁₋₆ et un groupe alcoxycarbonyle en C₂₋₇), un groupe alkyle en C₁₋₆ substitué par un groupe phénylthio (à la condition qu'un tel groupe phénylthio puisse être substitué par un ou plusieurs substituants choisis dans le groupe constitué par un atome d'halogène, un groupe trifluorométhyle, un groupe nitro, un groupe alkyle en C₁₋₆, un groupe alcoxy en C₁₋₆ et un groupe alcoxycarbonyle en C₂₋₇), un groupe alkyle en C₁₋₆ substitué par un groupe phénylsulfinyle (à la condition qu'un tel groupe phénylsulfinyle puisse être substitué par un ou plusieurs substituants choisis dans le groupe constitué par un atome d'halogène, un groupe trifluorométhyle, un groupe nitro, un groupe alkyle en C₁₋₆, un groupe alcoxy en C₁₋₆ et un groupe alcoxycarbonyle en C₂₋₇), un groupe alkyle en C₁₋₆ substitué par un groupe phénylsulfonyle (à la condition qu'un tel groupe phénylsulfonyle puisse être substitué par un ou plusieurs substituants choisis dans le groupe constitué par un atome d'halogène, un groupe trifluorométhyle, un groupe nitro, un groupe alkyle en C₁₋₆, un groupe alcoxy en C₁₋₆ et un groupe alcoxycarbonyle en C₂₋₇), un groupe alkyle en C₁₋₆ substitué par un groupe benzyloxy (à la condition que le groupe phényle d'un tel groupe benzyloxy puisse être substitué par un ou plusieurs substituants choisis dans le groupe constitué par un atome d'halogène, un groupe trifluorométhyle, un groupe nitro, un groupe alkyle en C₁₋₆, un groupe alcoxy en C₁₋₆ et un groupe alcoxycarbonyle en C₂₋₇), un groupe alkyle en C₁₋₆ substitué par un groupe benzylthio (à la condition que le groupe phényle d'un tel groupe benzylthio puisse être substitué par un ou plusieurs substituants choisis dans le groupe constitué par un atome d'halogène, un groupe trifluorométhyle, un groupe nitro, un groupe alkyle en C₁₋₆, un groupe alcoxy en C₁₋₆ et un groupe alcoxycarbonyle en C₂₋₇), un groupe alkyle en C₁₋₆ substitué par un groupe benzylsulfinyle (à la condition que le groupe phényle d'un tel groupe benzylsulfinyle puisse être substitué par un ou plusieurs substituants choisis dans le groupe constitué par un atome d'halogène, un groupe trifluorométhyle, un groupe nitro, un groupe alkyle en C₁₋₆, un groupe alcoxy en C₁₋₆ et un groupe alcoxycarbonyle en C₂₋₇), un groupe alkyle en C₁₋₆ substitué par un groupe benzylsulfonyle (à la condition que le groupe phényle d'un tel groupe benzylsulfonyle puisse être substitué par un ou plusieurs substituants choisis dans le groupe constitué par un atome d'halogène, un groupe trifluorométhyle, un groupe nitro, un groupe alkyle en C₁₋₆, un groupe alcoxy en C₁₋₆ et un groupe alcoxycarbonyle en C₂₋₇), un groupe alkyle en C₁₋₆ substitué par un groupe phénylcarbonyle (à la condition qu'un tel groupe phénylcarbonyle puisse être substitué par un ou plusieurs substituants choisis dans le groupe constitué par un atome d'halogène, un groupe trifluorométhyle, un groupe nitro, un groupe alkyle en C₁₋₆, un groupe alcoxy en C₁₋₆ et un groupe alcoxycarbonyle en C₂₋₇), un groupe alkyle en C₁₋₆ substitué par un groupe benzylcarbonyle (à la condition que le groupe phényle d'un tel groupe benzylcarbonyle puisse être substitué par un ou plusieurs substituants choisis dans le groupe constitué par un atome d'halogène, un groupe trifluorométhyle, un groupe nitro, un groupe alkyle en C₁₋₆, un groupe alcoxy en C₁₋₆ et un groupe alcoxycarbonyle en C₂₋₇), un groupe alkyle en C₁₋₆ substitué par un groupe amino substitué par un groupe alkylsulfonyle en C₁₋₄ ou un groupe alkyle en C₁₋₆ substitué par un groupe amino substitué par un groupe alkylcarbonyle en C₂₋₄ ;
- R^{b2} est un atome d'hydrogène, un groupe alkyle en C₁₋₆, un groupe mono-, di- ou poly-halogénoalkyle en C₁₋₆, un groupe alcoxy en C₁₋₆, un groupe mono-, di- ou poly-halogénoalcoxy en C₁₋₆, un groupe alkylthio en C₁₋₆, un groupe alkylsulfinyle en C₁₋₆, un groupe alkylsulfonyle en C₁₋₆, un groupe alcoxycarbonyle en C₂₋₇, un groupe alkylcarbonyle en C₂₋₇, un atome d'halogène, un groupe nitro, un groupe cyano, ou un groupe phényle (à la condition qu'un tel groupe phényle puisse être substitué par un ou plusieurs substituants choisis dans le groupe constitué par un atome d'halogène, un groupe trifluorométhyle, un groupe nitro, un groupe alkyle en C₁₋₆, un groupe alcoxy en C₁₋₆ et un groupe alcoxycarbonyle en C₂₋₇) ;
- R^{b3} est un groupe alkyle en C₁₋₆, un groupe alcényle en C₂₋₆ ou un groupe alcynyle en C₂₋₆ ;
- R^{b4} est un atome d'hydrogène ou un groupe alkyle en C₁₋₆ ;
- R^{b5} est un atome d'hydrogène ou un groupe alkyle en C₁₋₆ ;
- R^{c1} est un groupe alkyle en C₁₋₈, un groupe cycloalkyle en C₃₋₇, un groupe alkyle en C₁₋₆ substitué par un groupe cycloalkyle en C₃₋₇, un groupe cycloalcényle en C₃₋₇, un groupe alkyle en C₁₋₆ substitué par un groupe cycloalcényle en C₃₋₇, un groupe alcényle en C₂₋₈, un groupe alcynyle en C₂₋₈, un groupe alkyle en C₁₋₆ substitué par un groupe alcoxy en C₁₋₆, un groupe alkyle en C₁₋₆ substitué par un groupe alcényloxy en C₂₋₆, un groupe alkyle en C₁₋₆ substitué par un groupe alcynyloxy en C₂₋₆, un groupe alkyle en C₁₋₆ substitué par un groupe mono-, di- ou poly-halogénoalcoxy en C₁₋₆, un groupe alkyle en C₁₋₆ substitué par un groupe mono-, di-, ou poly-halogénoalcényloxy en C₂₋₆, un groupe alkyle en C₁₋₆ substitué par un groupe mono-, di- ou poly-halogénoalcynyloxy en C₂₋₆, un groupe alkyle en C₁₋₆ substitué par un groupe alkylthio en C₁₋₆, un groupe alkyle en C₁₋₆ substitué par un groupe alkylsulfinyle en C₁₋₆, un groupe alkyle en C₁₋₆ substitué par un groupe alkylsulfonyle en C₁₋₆, un groupe mono-, di-, ou poly-halogénoalkyle en C₁₋₈, un groupe mono-, di- ou poly-halogénoalcényle en C₂₋₈, un groupe mono-, di- ou polyhalogénoalcynyle en C₂₋₈, un groupe alkyle en C₁₋₆ substitué par un groupe cyano, un groupe alcényle en C₂₋₆ substitué par un groupe cyano, un groupe alcynyle en C₂₋₆ substitué par un groupe cyano, un groupe alkyle en C₁₋₆ substitué par un groupe nitro, un groupe alcényle en C₂₋₆ substitué par un groupe nitro, un groupe alcynyle en C₂₋₆ substitué par un groupe nitro, un groupe alkyle en C₁₋₆ substitué par un groupe alcoxycarbonyle en C₂₋₇, un groupe alcényle en C₂₋₆ substitué par un groupe alcoxycarbonyle en C₂₋₇, un groupe alcynyle en C₂₋₆ substitué par un groupe alcoxycarbonvle en C₂₋₇, un groupe alkyle en C₁₋₆ substitué par un groupe alkylcarbonyle en C₂₋₇, un groupe alkyle en C₁₋₆ substitué par un groupe mono-, di-, ou poly-halogénoalkylcarbonyle en C₂₋₇, un groupe alkyle en C₁₋₆ substitué par un groupe alcénylcarbonyle en C₃₋₇, un groupe alkyle en C₁₋₆ substitué par un groupe alcynylcarbonyle en C₃₋₇, un groupe alkyle en C₁₋₆ substitué par un groupe alkylcarbonyle en C₂₋₅ substitué par un groupe alcoxy en C₁₋₄, un groupe alkyle en C₁₋₆ substitué par un groupe alkylcarbonyle en C₂₋₅ substitué par un groupe alkylthio en C₁₋₄, un groupe alkyle en C₁₋₆ substitué par un groupe alkylcarbonyle en C₂₋₅ substitué par un groupe alkylsulfinyle en C₁₋₄, un groupe alkyle en C₁₋₆ substitué par un groupe alkylcarbonyle en C₂₋₅ substitué par un groupe alkylsulfonyle en C₁₋₄, un groupe alcényle en C₂₋₆ substitué par un groupe alkylcarbonyle en C₂₋₇, un groupe alcynyle en C₂₋₆ substitué par un groupe alkylcarbonyle en C₂₋₇, un groupe alkyle en C₁₋₆ substitué par un groupe alkylsulfamoyle en C₁₋₆, un groupe alkyle en C₁₋₆ substitué par un groupe alcoxysulfamoyle en C₁₋₆, un groupe alkyle en C₁₋₆ substitué par un groupe di(alkyl en C₁₋₃) sulfamoyle, un groupe alkyle en C₁₋₆ substitué par un groupe N-(alkyl en C₁₋₃)-N-(alcoxy en C₁₋₃)sulfamoyle, un groupe alkyle en C₁₋₆ substitué par un groupe alkylcarbamoyle en C₂₋₇, un groupe alkyle en C₁₋₆ substitué par un groupe di(alkyl en C₁₋₃)carbamoyle, un groupe alkyle en C₁₋₆ substitué par un groupe alcoxycarbamoyle en C₂₋₇, un groupe alkyle en C₁₋₆ substitué par un groupe N-(alkyl en C₁₋₃)-N-(alcoxy en C₁₋₃)carbamoyle, un groupe alkyle en C₁₋₆ substitué par un groupe alkylamino en C₁₋₆, un groupe alkyle en C₁₋₆ substitué par un groupe alcoxyamino en C₁₋₆, un groupe alkyle en C₁₋₆ substitué par un groupe di(alkyl en C₁₋₃)amino, un groupe alkyle en C₁₋₆ substitué par un groupe N-(alkyl en C₁₋₃)-N-(alcoxy en C₁₋₃)amino, un groupe alkyle en C₁₋₆ substitué par un groupe N-(alkylcarbonyl en C₂₋₇)-N-(alkyl en C₁₋₆)amino, un groupe alkyle en C₁₋₆ substitué par un groupe N-(alkylcarbonyl en C₂₋₇)-N-(alcoxy en C₁₋₆)amino, un groupe alkyle en C₁₋₆ substitué par un groupe N-(alkylsulfonyl en C₁₋₆)-N-(alkyl en C₁₋₆)amino, un groupe alkyle en C₁₋₆ substitué par un groupe N-(alkylsulfonyl en C₁₋₆)-N-(alcoxy en C₁₋₆)amino, un groupe phényle (à la condition qu'un tel groupe phényle puisse être substitué par un ou plusieurs substituants choisis dans le groupe constitué par un atome d'halogène, un groupe trifluorométhyle, un groupe nitro, un groupe alkyle en C₁₋₆, un groupe alcoxy en C₁₋₆ et un groupe alcoxycarbonyle en C₂₋₇), un groupe alkyle en C₁₋₆ substitué par un groupe phényle (à la condition qu'un tel groupe phényle puisse être substitué par un ou plusieurs substituants choisis dans le groupe constitué par un atome d'halogène, un groupe trifluorométhyle, un groupe nitro, un groupe alkyle en C₁₋₆, un groupe alcoxy en C₁₋₆ et un groupe alcoxycarbonyle en C₂₋₇), un groupe alcényle en C₂₋₇ substitué par un groupe phényle (à la condition qu'un tel groupe phényle puisse être substitué par un ou plusieurs substituants choisis dans le groupe constitué par un atome d'halogène, un groupe trifluorométhyle, un groupe nitro, un groupe alkyle en C₁₋₆, un groupe alcoxy en C₁₋₆ et un groupe alcoxycarbonyle en C₂₋₇), un groupe alcynyle en C₂₋₆ substitué par un groupe phényle (à la condition qu'un tel groupe phényle puisse être substitué par un ou plusieurs substituants choisis dans le groupe constitué par un atome d'halogène, un groupe trifluorométhyle, un groupe nitro, un groupe alkyle en C₁₋₆, un groupe alcoxy en C₁₋₆ et un groupe alcoxycarbonyle en C₂₋₇), un groupe alkyle en C₁₋₆ substitué par un groupe phénoxy (à la condition qu'un tel groupe phénoxy puisse être substitué par un ou plusieurs substituants choisis dans le groupe constitué par un atome d'halogène, un groupe trifluorométhyle, un groupe nitro, un groupe alkyle en C₁₋₆, un groupe alcoxy en C₁₋₆ et un groupe alcoxy-carbonyle en C₂₋₇), un groupe alkyle en C₁₋₆ substitué par un groupe phénylthio (à la condition qu'un tel groupe phénylthio puisse être substitué par un ou plusieurs substituants choisis dans le groupe constitué par un atome d'halogène, un groupe trifluorométhyle, un groupe nitro, un groupe alkyle en C₁₋₆, un groupe alcoxy en C₁₋₆ et un groupe alcoxycarbonyle en C₂₋₇), un groupe alkyle en C₁₋₆ substitué par un groupe phénylsulfinyle (à la condition qu'un tel groupe phénylsulfinyle puisse être substitué par un ou plusieurs substituants choisis dans le groupe constitué par un atome d'halogène, un groupe trifluorométhyle, un groupe nitro, un groupe alkyle en C₁₋₆, un groupe alcoxy en C₁₋₆ et un groupe alcoxycarbonyle en C₂₋₇), un groupe alkyle en C₁₋₆ substitué par un groupe phénylsulfonyle (à la condition qu'un tel groupe phénylsulfonyle puisse être substitué par un ou plusieurs substituants choisis dans le groupe constitué par un atome d'halogène, un groupe trifluorométhyle, un groupe nitro, un groupe alkyle en C₁₋₆, un groupe alcoxy en C₁₋₆ et un groupe alcoxy-carbonyle en C₂₋₇₎, un groupe alkyle en C₁₋₆ substitué par un groupe benzyloxy (à la condition que le groupe phényle d'un tel groupe benzyloxy puisse être substitué par un ou plusieurs substituants choisis dans le groupe constitué par un atome d'halogène, un groupe trifluorométhyle, un groupe nitro, un groupe alkyle en C₁₋₆, un groupe alcoxy en C₁₋₆ et un groupe alcoxy-carbonyle en C₂₋₇), un groupe alkyle en C₁₋₆ substitué par un groupe benzylthio (à la condition que le groupe phényle d'un tel groupe benzylthio puisse être substitué par un ou plusieurs substituants choisis dans le groupe constitué par un atome d'halogène, un groupe trifluorométhyle, un groupe nitro, un groupe alkyle en C₁₋₆, un groupe alcoxy en C₁₋₆ et un groupe alcoxycarbonyle en C₂₋₇), un groupe alkyle en C₁₋₆ substitué par un groupe benzylsulfinyle (à la condition que le groupe phényle d'un tel groupe benzylsulfinyle puisse être substitué par un ou plusieurs substituants choisis dans le groupe constitué par un atome d'halogène, un groupe trifluorométhyle, un groupe nitro, un groupe alkyle en C₁₋₆, un groupe alcoxy en C₁₋₆ et un groupe alcoxycarbonyle en C₂₋₇), un groupe alkyle en C₁₋₆ substitué par un groupe benzylsulfonyle (à la condition que le groupe phényle d'un tel groupe benzylsulfonyle puisse être substitué par un ou plusieurs substituants choisis dans le groupe constitué par un atome d'halogène, un groupe trifluorométhyle, un groupe nitro, un groupe alkyle en C₁₋₆, un groupe alcoxy en C₁₋₆ et un groupe alcoxycarbonyle en C₂₋₇₎, un groupe alkyle en C₁₋₆ substitué par un groupe phénylcarbonyle (à la condition qu'un tel groupe phénylcarbonyle puisse être substitué par un ou plusieurs substituants choisis dans le groupe constitué par un atome d'halogène, un groupe trifluorométhyle, un groupe nitro, un groupe alkyle en C₁₋₆, un groupe alcoxy en C₁₋₆ et un groupe alcoxycarbonyle en C₂₋₇), un groupe alkyle en C₁₋₆ substitué par un groupe benzylcarbonyle (à la condition que le groupe phényle d'un tel groupe benzylcarbonyle puisse être substitué par un ou plusieurs substituants choisis dans le groupe constitué par un atome d'halogène, un groupe trifluorométhyle, un groupe nitro, un groupe alkyle en C₁₋₆, un groupe alcoxy en C₁₋₆ et un groupe alcoxycarbonyle en C₂₋₇), un groupe alkyle en C₁₋₆ substitué par un groupe amino substitué par un groupe alkylsulfonyle en C₁₋₄, ou un groupe alkyle en C₁₋₆ substitué par un groupe amino substitué par un groupe alkylcarbonyle en C₂₋₄ ;
- R^{c2}, R^{c3}, R^{c4}, R^{c5}, R^{c6}, R^{c11} et R^{c12}, qui sont indépendants l'un de l'autre, représentent chacun un atome d'hydrogène, un groupe alkyle en C₁₋₈, un groupe alcényle en C₂₋₈, un groupe alcynyle en C₂₋₈, ou un groupe phényle (à la condition qu'un tel groupe phényle puisse être substitué par un ou plusieurs substituants choisis dans le groupe constitué par un atome d'halogène, un groupe trifluorométhyle, un groupe nitro, un groupe alkyle en C₁₋₆, un groupe alcoxy en C₁₋₆ et un groupe alcoxycarbonyle en C₂₋₇) ;
- R^{c7}, R^{c8}, R^{c9}, R^{c10}, R^{c13} et R^{c14}, qui sont indépendants l'un de l'autre, représentent chacun un atome d'hydrogène, un groupe alkyle en C₁₋₆, un groupe mono-, di- ou poly-halogénoalkyle en C₁₋₆, un groupe alcoxy en C₁₋₆, un groupe mono-, di- ou poly-halogénoalcoxy en C₁₋₆, un groupe alkylthio en C₁₋₆, un groupe alkylsulfinyle en C₁₋₆, un groupe alkylsulfonyle en C₁₋₆, un groupe alcoxycarbonyle en C₂₋₇, un groupe alkylcarbonyle en C₂₋₇, un atome d'halogène, un groupe nitro, un groupe cyano ou un groupe phényle (à la condition qu'un tel groupe phényle puisse être substitué par un ou plusieurs substituants choisis dans le groupe constitué par un atome d'halogène, un groupe trifluorométhyle, un groupe nitro, un groupe alkyle en C₁₋₆, un groupe alcoxy en C₁₋₆ et un groupe alcoxycarbonyle en C₂₋₇) ;
- R^{d1} est un groupe alkyle en C₁₋₈, un groupe cycloalkyle en C₃₋₇, un groupe alkyle en C₁₋₆ substitué par un groupe cycloalkyle en C₃₋₇, un groupe cycloalcényle en C₃₋₇, un groupe alkyle en C₁₋₆ substitué par un groupe cycloalcényle en C₃₋₇, un groupe alcényle en C₂₋₈, un groupe alcynyle en C₂₋₈, un groupe alkyle en C₁₋₆ substitué par un groupe alcoxy en C₁₋₆, un groupe alkyle en C₁₋₆ substitué par un groupe alcényloxy en C₂₋₆, un groupe alkyle en C₁₋₆ substitué par un groupe alcynyloxy en C₂₋₆, un groupe alkyle en C₁₋₆ substitué par un groupe mono-, di- ou poly-halogénoalcoxy en C₁₋₆, un groupe alkyle en C₁₋₆ substitué par un groupe mono-, di- ou poly-halogénoalcényloxy en C₂₋₆, un groupe alkyle en C₁₋₆ substitué par un groupe mono-, di-, ou poly-halogénoalcynyloxy en C₂₋₆, un groupe alkyle en C₁₋₆ substitué par un groupe alkylthio en C₁₋₆, un groupe alkyle en C₁₋₆ substitué par un groupe alkylsulfinyle en C₁₋₆, un groupe alkyle en C₁₋₆ substitué par un groupe alkylsulfonyle en C₁₋₆ un groupe mono-, di- ou poly-halogénoalkyle en C₁₋₈, un groupe mono-, di- ou poly-halogénoalcényle en C₂₋₈, un groupe mono-, di- ou poly-halogénoalcynyle en C₂₋₈, un groupe alkyle en C₁₋₆ substitué par un groupe cyano, un groupe alcényle en C₂₋₆ substitué par un groupe cyano, un groupe alcynyle en C₂₋₆ substitué par un groupe cyano, un groupe alkyle en C₁₋₆ substitué par un groupe nitro, un groupe alcényle en C₂₋₆ substitué par un groupe nitro, un groupe alcynyle en C₂₋₆ substitué par un groupe nitro, un groupe alkyle en C₁₋₆ substitué par un groupe alcoxycarbonyle en C₂₋₇, un groupe alcényle en C₂₋₆ substitué par un groupe alcoxycarbonyle en C₂₋₇, un groupe alcynyle en C₂₋₆ substitué par un groupe alcoxycarbonyle en C₂₋₇, un groupe alkyle en C₁₋₆ substitué par un groupe alkylcarbonyle en C₂₋₇, un groupe alkyle en C₁₋₆ substitué par un groupe mono-, di- ou poly-halogénoalkylcarbonyle en C₂₋₇, un groupe alkyle en C₁₋₆ substitué par un groupe alcénylcarbonyle en C₃₋₇, un groupe alkyle en C₁₋₆ substitué par un groupe alcynyl-carbonyle en C₃₋₇, un groupe alkyle en C₁₋₆ substitué par un groupe alkylcarbonyle en C₂₋₅ substitué par un groupe alcoxy en C₁₋₄, un groupe alkyle en C₁₋₆ substitué par un groupe alkylcarbonyle en C₂₋₅ substitué par un groupe alkylthio en C₁₋₄, un groupe alkyle en C₁₋₆ substitué par un groupe alkylcarbonyle en C₂₋₅ substitué par un groupe alkylsulfinyle en C₁₋₄, un groupe alkyle en C₁₋₆ substitué par un groupe alkylcarbonyle en C₂₋₅ substitué par un groupe alkylsulfonyle en C₁₋₄, un groupe alcényle en C₂₋₆ substitué par un groupe alkylcarbonyle en C₂₋₇, un groupe alcynyle en C₂₋₆ substitué par un groupe alkylcarbonyle en C₂₋₇, un groupe alkyle en C₁₋₆ substitué par un groupe alkylsulfamoyle en C₁₋₆, un groupe alkyle en C₁₋₆ substitué par un groupe alcoxysulfamoyle en C₁₋₆, un groupe alkyle en C₁₋₆ substitué par un groupe di(alkyl en C₁₋₃)sulfamoyle, un groupe alkyle en C₁₋₆ substitué par un groupe N-(alkyl en C₁₋₃)-N-(alcoxy en C₁₋₃)sulfamoyle, un groupe alkyle en C₁₋₆ substitué par un groupe alkylcarbamoyle en C₂₋₇, un groupe alkyle en C₁₋₆ substitué par un groupe di(alkyl en C₁₋₃)carbamoyle, un groupe alkyle en C₁₋₆ substitué par un groupe alcoxy-carbamoyle en C₂₋₇, un groupe alkyle en C₁₋₆ substitué par un groupe N-(alkyl en C₁₋₃)-N-(alcoxy en C₁₋₃)carbamoyle, un groupe alkyle en C₁₋₆ substitué par un groupe alkylamino en C₁₋₆, un groupe alkyle en C₁₋₆ substitué par un groupe alcoxyamino en C₁₋₆, un groupe alkyle en C₁₋₆ substitué par un groupe di(alkyl en C₁₋₃)amino, un groupe alkyle en C₁₋₆ substitué par un groupe N-(alkyl en C₁₋₃)-N-(alcoxy en C₁₋₃)amino, un groupe alkyle en C₁₋₆ substitué par un groupe N-(alkylcarbonyl en C₂₋₇)-N-(alkyl en C₁₋₆)amino, un groupe alkyle en C₁₋₆ substitué par un groupe N-(alkylcarbonyl en C₂₋₇)-N-(alcoxy en C₁₋₆)amino, un groupe alkyle en C₁₋₆ substitué par un groupe N-(alkylsulfonyl en C₁₋₆)-N-(alkyl en C₁₋₆)amino, un groupe alkyle en C₁₋₆ substitué par un groupe N-(alkylsulfonyl en C₁₋₆)-N-(alcoxy en C₁₋₆)amino, un groupe phényle (à la condition qu'un tel groupe phényle puisse être substitué par un ou plusieurs substituants choisis dans le groupe constitué par un atome d'halogène, un groupe trifluorométhyle, un groupe nitro, un groupe alkyle en C₁₋₆, un groupe alcoxy en C₁₋₆ et un groupe alcoxycarbonyle en C₂₋₇), un groupe alkyle en C₁₋₆ substitué par un groupe phényle (à la condition qu'un tel groupe phényle puisse être substitué par un ou plusieurs substituants choisis dans le groupe constitué par un atome d'halogène, un groupe trifluorométhyle, un groupe nitro, un groupe alkyle en C₁₋₆, un groupe alcoxy en C₁₋₆ et un groupe alcoxycarbonyle en C₂₋₇), un groupe alcényle en C₂₋₇ substitué par un groupe phényle (à la condition qu'un tel groupe phényle puisse être substitué par un ou plusieurs substituants choisis dans le groupe constitué par un atome d'halogène, un groupe trifluorométhyle, un groupe nitro, un groupe alkyle en C₁₋₆, un groupe alcoxy en C₁₋₆ et un groupe alcoxycarbonyle en C₂₋₇), un groupe alcynyle en C₂₋₆ substitué par un groupe phényle (à la condition qu'un tel groupe phényle puisse être substitué par un ou plusieurs substituants choisis dans le groupe constitué par un atome d'halogène, un groupe trifluorométhyle, un groupe nitro, un groupe alkyle en C₁₋₆, un groupe alcoxy en C₁₋₆ et un groupe alcoxycarbonyle en C₂₋₇), un groupe alkyle en C₁₋₆ substitué par un groupe phénoxy (à la condition qu'un tel groupe phénoxy puisse être substitué par un ou plusieurs substituants choisis dans le groupe constitué par un atome d'halogène, un groupe trifluorométhyle, un groupe nitro, un groupe alkyle en C₁₋₆, un groupe alcoxy en C₁₋₆ et un groupe alcoxycarbonyle en C₂₋₇), un groupe alkyle en C₁₋₆ substitué par un groupe phénylthio (à la condition qu'un tel groupe phénylthio puisse être substitué par un ou plusieurs substituants choisis dans le groupe constitué par un atome d'halogène, un groupe trifluorométhyle, un groupe nitro, un groupe alkyle en C₁₋₆, un groupe alcoxy en C₁₋₆ et un groupe alcoxycarbonyle en C₂₋₇), un groupe alkyle en C₁₋₆ substitué par un groupe phénylsulfinyle (à la condition qu'un tel groupe phénylsulfinyle puisse être substitué par un ou plusieurs substituants choisis dans le groupe constitué par un atome d'halogène, un groupe trifluorométhyle, un groupe nitro, un groupe alkyle en C₁₋₆, un groupe alcoxy en C₁₋₆ et un groupe alcoxycarbonyle en C₂₋₇), un groupe alkyle en C₁₋₆ substitué par un groupe phénylsulfonyle (à la condition qu'un tel groupe phénylsulfonyle puisse être substitué par un ou plusieurs substituants choisis dans le groupe constitué par un atome d'halogène, un groupe trifluorométhyle, un groupe nitro, un groupe alkyle en C₁₋₆, un groupe alcoxy en C₁₋₆ et un groupe alcoxycarbonyle en C₂₋₇), un groupe alkyle en C₁₋₆ substitué par un groupe benzyloxy (à la condition que le groupe phényle d'un tel groupe benzyloxy puisse être substitué par un ou plusieurs substituants choisis dans le groupe constitué par un atome d'halogène, un groupe trifluorométhyle, un groupe nitro, un groupe alkyle en C₁₋₆, un groupe alcoxy en C₁₋₆ et un groupe alcoxycarbonyle en C₂₋₇), un groupe alkyle en C₁₋₆ substitué par un groupe benzylthio (à la condition que le groupe phényle d'un tel groupe benzylthio puisse être substitué par un ou plusieurs substituants choisis dans le groupe constitué par un atome d'halogène, un groupe trifluorométhyle, un groupe nitro, un groupe alkyle en C₁₋₆, un groupe alcoxy en C₁₋₆ et un groupe alcoxycarbonyle en C₂₋₇), un groupe alkyle en C₁₋₆ substitué par un groupe benzylsulfinyle (à la condition que le groupe phényle d'un tel groupe benzylsulfinyle puisse être substitué par un ou plusieurs substituants choisis dans le groupe constitué par un atome d'halogène, un groupe trifluorométhyle, un groupe nitro, un groupe alkyle en C₁₋₆, un groupe alcoxy en C₁₋₆ et un groupe alcoxycarbonyle en C₂₋₇), un groupe alkyle en C₁₋₆ substitué par un groupe benzylsulfonyle, (à la condition que le groupe phényle d'un tel groupe benzylsulfonyle puisse être substitué par un ou plusieurs substituants choisis dans le groupe constitué par un atome d'halogène, un groupe trifluorométhyle, un groupe nitro, un groupe alkyle en C₁₋₆, un groupe alcoxy en C₁₋₆ et un groupe alcoxycarbonyle en C₂₋₇), un groupe alkyle en C₁₋₆ substitué par un groupe phénylcarbonyle (à la condition qu'un tel groupe phénylcarbonyle puisse être substitué par un ou plusieurs substituants choisis dans le groupe constitué par un atome d'halogène, un groupe trifluorométhyle, un groupe nitro, un groupe alkyle en C₁₋₆, un groupe alcoxy en C₁₋₆ et un groupe alcoxycarbonyle en C₂₋₇), un groupe alkyle en C₁₋₆ substitué par un groupe benzylcarbonyle (à la condition que le groupe phényle d'un tel groupe benzylcarbonyle puisse être substitué par un ou plusieurs substituants choisis dans le groupe constitué par un atome d'halogène, un groupe trifluorométhyle, un groupe nitro, un groupe alkyle en C₁₋₆, un groupe alcoxy en C₁₋₆ et un groupe alcoxycarbonyle en C₂₋₇), un groupe alkyle en C₁₋₆ substitué par un groupe amino substitué par un groupe alkylsulfonyle en C₁₋₄ ou un groupe alkyle en C₁₋₆ substitué par un groupe amino substitué par un groupe alkylcarbonyle en C₂₋₄ ;
- R^{d2}, R^{d3} et R^{d4}, qui sont indépendants l'un de l'autre, représentent chacun un atome d'hydrogène, un groupe alkyle en C₁₋₈, un groupe alcényle en C₂₋₈, un groupe alcynyle en C₂₋₈, ou un groupe phényle (à la condition qu'un tel groupe phényle puisse être substitué par un ou plusieurs substituants choisis dans le groupe constitué par un atome d'halogène, un groupe trifluorométhyle, un groupe nitro, un groupe alkyle en C₁₋₆, un groupe alcoxy en C₁₋₆ et un groupe alcoxycarbonyle en C₂₋₇) ;
- R^{d5} est un groupe alkyle en C₁₋₈, un groupe alcényle en C₂₋₈, un groupe alcynyle en C₂₋₈, ou un groupe phényle (à la condition qu'un tel groupe phényle puisse être substitué par un ou plusieurs substituants choisis dans le groupe constitué par un atome d'halogène, un groupe trifluorométhyle, un groupe nitro, un groupe alkyle en C₁₋₆, un groupe alcoxy en C₁₋₆ et un groupe alcoxycarbonyle en C₂₋₇) ;
- R^{d6} est un groupe alkyle en C₁₋₈, un groupe alcényle en C₂₋₈, un groupe alcynyle en C₂₋₈, un groupe alkyle en C₁₋₆ substitué par un groupe alkylthio en C₁₋₆, un groupe alkyle en C₁₋₆ substitué par un groupe alkylsulfinyle en C₁₋₆, un groupe alkyle en C₁₋₆ substitué par un groupe alkylsulfonyle en C₁₋₆, un groupe alkyle en C₁₋₆ substitué par un groupe alcoxy en C₁₋₆, un groupe alkyle en C₁₋₆ substitué par un groupe alcoxycarbonyle en C₂₋₇, un groupe alkyle en C₁₋₆ substitué par un groupe alkylcarbonyle en C₂₋₇, un groupe alkyle en C₁₋₆ substitué par un groupe cyano, un groupe phényle (à la condition qu'un tel groupe phényle puisse être substitué par un ou plusieurs substituants choisis dans le groupe constitué par un atome d'halogène, un groupe trifluorométhyle, un groupe nitro, un groupe alkyle en C₁₋₆, un groupe alcoxy en C₁₋₆ et un groupe alcoxycarbonyle en C₂₋₇), ou un groupe alkyle en C₁₋₆ substitué par un groupe phényle (à la condition qu'un tel groupe phényle puisse être substitué par un ou plusieurs substituants choisis dans le groupe constitué par un atome d'halogène, un groupe trifluorométhyle, un groupe nitro, un groupe alkyle en C₁₋₆, un groupe alcoxy en C₁₋₆ et un groupe alcoxycarbonyle en C₂₋₇) ;
- R^{e1} est un groupe alkyle en C₁₋₈, un groupe cycloalkyle en C₃₋₇, un groupe alkyle en C₁₋₆ substitué par un groupe cycloalkyle en C₃₋₇, un groupe cycloalcényle en C₃₋₇, un groupe alkyle en C₁₋₆ substitué par un groupe cycloalcényle en C₃₋₇, un groupe alcényle en C₂₋₈, un groupe alcynyle en C₂₋₈, un groupe alkyle en C₁₋₆ substitué par un groupe alcoxy en C₁₋₆, un groupe alkyle en C₁₋₆ substitué par un groupe alcényloxy en C₂₋₆, un groupe alkyle en C₁₋₆ substitué par un groupe alcynyloxy en C₂₋₆, un groupe alkyle en C₁₋₆ substitué par un groupe mono-, di- ou poly-halogénoalcoxy en C_{1-6,} un groupe alkyle en C₁₋₆ substitué par un groupe mono-, di-, ou poly-halogénoalcényloxy en C₂₋₆, un groupe alkyle en C₁₋₆ substitué par un groupe mono-, di-, ou poly-halogénoalcynyloxy en C₂₋₆, un groupe alkyle en C₁₋₆ substitué par un groupe alkylthio en C₁₋₆, un groupe alkyle en C₁₋₆ substitué par un groupe alkylsulfinyle en C₁₋₆, un groupe alkyle en C₁₋₆ substitué par un groupe alkylsulfonyle en C₁₋₆, un groupe mono-, di- ou poly-halogénoalkyle en C₁₋₈, un groupe mono-, di- ou poly-halogénoalcényle en C₂₋₈, un groupe mono-, di- ou poly-halogénoalcynyle en C₂₋₈, un groupe alkyle en C₁₋₆ substitué par un groupe cyano, un groupe alcényle en C₂₋₆ substitué par un groupe cyano, un groupe alcynyle en C₂₋₆ substitué par un groupe cyano, un groupe alkyle en C₁₋₆ substitué par un groupe nitro, un groupe alcényle en C₂₋₆ substitué par un groupe nitro, un groupe alcynyle en C₂₋₆ substitué par un groupe nitro, un groupe alkyle en C₁₋₆ substitué par un groupe alcoxycarbonyle en C₂₋₇, un groupe alcényle en C₂₋₆ substitué par un groupe alcoxycarbonyle en C₂₋₇, un groupe alcynyle en C₂₋₆ substitué par un groupe alcoxycarbonyle en C₂₋₇, un groupe alkyle en C₁₋₆ substitué par un groupe alkylcarbonyle en C₂₋₇, un groupe alkyle en C₁₋₆ substitué par un groupe mono-, di-, ou poly-halogénoalkylcarbonyle en C₂₋₇, un groupe alkyle en C₁₋₆ substitué par un groupe alcénylcarbonyle en C₃₋₇, un groupe alkyle en C₁₋₆ substitué par un groupe alcynyl-carbonyle en C₃₋₇, un groupe alkyle en C₁₋₆ substitué par un groupe alkylcarbonyle en C₂₋₅ substitué par un groupe alcoxy en C₁₋₄, un groupe alkyle en C₁₋₆ substitué par un groupe alkylcarbonyle en C₂₋₅ substitué par un groupe alkylthio en C₁₋₄, un groupe alkyle en C₁₋₆ substitué par un groupe alkylcarbonyle en C₂₋₅ substitué par un groupe alkylsulfinyle en C₁₋₄, un groupe alkyle en C₁₋₆ substitué par un groupe alkylcarbonyle en C₂₋₅ substitué par un groupe alkylsulfonyle en C₁₋₄, un groupe alcényle en C₂₋₆ substitué par un groupe alkylcarbonyle en C₂₋₇, un groupe alcynyle en C₂₋₆ substitué par un groupe alkylcarbonyle en C₂₋₇, un groupe alkyle en C₁₋₆ substitué par un groupe alkylsulfamoyle en C₁₋₆, un groupe alkyle en C₁₋₆ substitué par un groupe alcoxysulfamoyle en C₁₋₆, un groupe alkyle en C₁₋₆ substitué par un groupe di(alkyl en C₁₋₃)sulfamoyle, un groupe alkyle en C₁₋₆ substitué par un groupe N-(alkyl en C₁₋₃)-N-(alcoxy en C₁₋₃)sulfamoyle, un groupe alkyle en C₁₋₆ substitué par un groupe alkylcarbamoyle en C₂₋₇, un groupe alkyle en C₁₋₆ substitué par un groupe di(alkyl en C₁₋₃)carbamoyle, un groupe alkyle en C₁₋₆ substitué par un groupe alcoxy-carbamoyle en C₂₋₇, un groupe alkyle en C₁₋₆ substitué par un groupe N-(alkyl en C₁₋₃)-N-(alcoxy en C₁₋₃)carbamoyle, un groupe alkyle en C₁₋₆ substitué par un groupe alkylamino en C₁₋₆, un groupe alkyle en C₁₋₆ substitué par un groupe alcoxyamino en C₁₋₆, un groupe alkyle en C₁₋₆ substitué par un groupe di(alkyl en C₁₋₃)amino, un groupe alkyle en C₁₋₆ substitué par un groupe N-(alkyl en C₁₋₃)-N-(alcoxy en C₁₋₃)amino, un groupe alkyle en C₁₋₆ substitué par un groupe N-(alkylcarbonyl en C₂₋₇)-N-(alkyl en C₁₋₆)amino, un groupe alkyle en C₁₋₆ substitué par un groupe N-(alkylcarbonyl en C₂₋₇)-N-(alcoxy en C₁₋₆)amino, un groupe alkyle en C₁₋₆ substitué par un groupe N-(alkylsulfonyl en C₁₋₆)-N-(alkyl en C₁₋₆)amino, un groupe alkyle en C₁₋₆ substitué par un groupe N-(alkylsulfonyl en C₁₋₆)-N-(alcoxy en C₁₋₆)amino, un groupe phényle (à la condition qu'un tel groupe phényle puisse être substitué par un ou plusieurs substituants choisis dans le groupe constitué par un atome d'halogène, un groupe trifluorométhyle, un groupe nitro, un groupe alkyle en C₁₋₆, un groupe alcoxy en C₁₋₆ et un groupe alcoxycarbonyle en C₂₋₇), un groupe alkyle en C₁₋₆ substitué par un groupe phényle (à la condition qu'un tel groupe phényle puisse être substitué par un ou plusieurs substituants choisis dans le groupe constitué par un atome d'halogène, un groupe trifluorométhyle, un groupe nitro, un groupe alkyle en C₁₋₆, un groupe alcoxy en C₁₋₆ et un groupe alcoxycarbonyle en C₂₋₇), un groupe alcényle en C₂₋₇ substitué par un groupe phényle (à la condition qu'un tel groupe phényle puisse être substitué par un ou plusieurs substituants choisis dans le groupe constitué par un atome d'halogène, un groupe trifluorométhyle, un groupe nitro, un groupe alkyle en C₁₋₆, un groupe alcoxy en C₁₋₆ et un groupe alcoxycarbonyle en C₂₋₇), un groupe alcynyle en C₂₋₆ substitué par un groupe phényle (à la condition qu'un tel groupe phényle puisse être substitué par un ou plusieurs substituants choisis dans le groupe constitué par un atome d'halogène, un groupe trifluorométhyle, un groupe nitro, un groupe alkyle en C₁₋₆, un groupe alcoxy en C₁₋₆ et un groupe alcoxycarbonyle en C₂₋₇), un groupe alkyle en C₁₋₆ substitué par un groupe phénoxy (à la condition qu'un tel groupe phénoxy puisse être substitué par un ou plusieurs substituants choisis dans le groupe constitué par un atome d'halogène, un groupe trifluorométhyle, un groupe nitro, un groupe alkyle en C₁₋₆, un groupe alcoxy en C₁₋₆ et un groupe alcoxy-carbonyle en C₂₋₇), un groupe alkyle en C₁₋₆ substitué par un groupe phénylthio (à la condition qu'un tel groupe phénylthio puisse être substitué par un ou plusieurs substituants choisis dans le groupe constitué par un atome d'halogène, un groupe trifluorométhyle, un groupe nitro, un groupe alkyle en C₁₋₆, un groupe alcoxy en C₁₋₆ et un groupe alcoxycarbonyle en C₂₋₇), un groupe alkyle en C₁₋₆ substitué par un groupe phénylsulfinyle (à la condition qu'un tel groupe phénylsulfinyle puisse être substitué par un ou plusieurs substituants choisis dans le groupe constitué par un atome d'halogène, un groupe trifluorométhyle, un groupe nitro, un groupe alkyle en C₁₋₆, un groupe alcoxy en C₁₋₆ et un groupe alcoxycarbonyle en C₂₋₇), un groupe alkyle en C₁₋₆ substitué par un groupe phénylsulfonyle (à la condition qu'un tel groupe phénylsulfonyle puisse être substitué par un ou plusieurs substituants choisis dans le groupe constitué par un atome d'halogène, un groupe trifluorométhyle, un groupe nitro, un groupe alkyle en C₁₋₆, un groupe alcoxy en C₁₋₆ et un groupe alcoxycarbonyle en C₂₋₇), un groupe alkyle en C₁₋₆ substitué par un groupe benzyloxy (à la condition que le groupe phényle d'un tel groupe benzyloxy puisse être substitué par un ou plusieurs substituants choisis dans le groupe constitué par un atome d'halogène, un groupe trifluorométhyle, un groupe nitro, un groupe alkyle en C₁₋₆, un groupe alcoxy en C₁₋₆ et un groupe alcoxycarbonyle en C₂₋₇), un groupe alkyle en C₁₋₆ substitué par un groupe benzylthio (à la condition que le groupe phényle d'un tel groupe benzylthio puisse être substitué par un ou plusieurs substituants choisis dans le groupe constitué par un atome d'halogène, un groupe trifluorométhyle, un groupe nitro, un groupe alkyle en C₁₋₆, un groupe alcoxy en C₁₋₆ et un groupe alcoxycarbonyle en C₂₋₇), un groupe alkyle en C₁₋₆ substitué par un groupe benzylsulfinyle (à la condition que le groupe phényle d'un tel groupe benzylsulfinyle puisse être substitué par un ou plusieurs substituants choisis dans le groupe constitué par un atome d'halogène, un groupe trifluorométhyle, un groupe nitro, un groupe alkyle en C₁₋₆, un groupe alcoxy en C₁₋₆ et un groupe alcoxycarbonyle en C₂₋₇), un groupe alkyle en C₁₋₆ substitué par un groupe benzylsulfonyle (à la condition que le groupe phényle d'un tel groupe benzylsulfonyle puisse être substitué par un ou plusieurs substituants choisis dans le groupe constitué par un atome d'halogène, un groupe trifluorométhyle, un groupe nitro, un groupe alkyle en C₁₋₆, un groupe alcoxy en C₁₋₆ et un groupe alcoxycarbonyle en C₂₋₇), un groupe alkyle en C₁₋₆ substitué par un groupe phénylcarbonyle (à la condition qu'un tel groupe phénylcarbonyle puisse être substitué par un ou plusieurs substituants choisis dans le groupe constitué par un atome d'halogène, un groupe trifluorométhyle, un groupe nitro, un groupe alkyle en C₁₋₆, un groupe alcoxy en C₁₋₆ et un groupe alcoxycarbonyle en C₂₋₇), un groupe alkyle en C₁₋₆ substitué par un groupe benzylcarbonyle (à la condition que le groupe phényle d'un tel groupe benzylcarbonyle puisse être substitué par un ou plusieurs substituants choisis dans le groupe constitué par un atome d'halogène, un groupe trifluorométhyle, un groupe nitro, un groupe alkyle en C₁₋₆, un groupe alcoxy en C₁₋₆ et un groupe alcoxycarbonyle en C₂₋₇), un groupe alkyle en C₁₋₆ substitué par un groupe amino substitué par un groupe alkylsulfonyle en C₁₋₄, ou un groupe alkyle en C₁₋₆ substitué par un groupe amino substitué par un groupe alkylcarbonyle en C₂₋₄ ;
- R^{e2}, R^{e3}, R^{e6} et R^{e7}, qui sont indépendants l'un de l'autre, représentent chacun un atome d'hydrogène, un groupe alkyle en C₁₋₆, un groupe mono-, di- ou poly-halogénoalkyle en C₁₋₆, un groupe alcoxy en C₁₋₆, un groupe mono-, di- ou poly-halogénoalcoxy en C₁₋₆, un groupe alkylthio en C₁₋₆, un groupe alkylsulfinyle en C₁₋₆, un groupe alkylsulfonyle en C₁₋₆, un groupe alcoxycarbonyle en C₂₋₇, un groupe alkylcarbonyle en C₂₋₇, un atome d'halogène, un groupe nitro, un groupe cyano, ou un groupe phényle (à la condition qu'un tel groupe phényle puisse être substitué par un ou plusieurs substituants choisis dans le groupe constitué par un atome d'halogène, un groupe trifluorométhyle, un groupe nitro, un groupe alkyle en C₁₋₆, un groupe alcoxy en C₁₋₆ et un groupe alcoxycarbonyle en C₂₋₇),
- R^{e4}, R^{e5}, R^{e8}, R^{e9} et R^{e10}, qui sont indépendants l'un de l'autre, représentent chacun un atome d'hydrogène, un groupe alkyle en C₁₋₈, un groupe alcényle en C₂₋₈, un groupe alcynyle en C₂₋₈, ou un groupe phényle (à la condition qu'un tel groupe phényle puisse être substitué par un ou plusieurs substituants choisis dans le groupe constitué par un atome d'halogène, un groupe trifluorométhyle, un groupe nitro, un groupe alkyle en C₁₋₆, un groupe alcoxy en C₁₋₆ et un groupe alcoxycarbonyle en C₂₋₇) ;
- R^{f1} est un groupe alkyle en C₁₋₈, un groupe cycloalkyle en C₃₋₇, un groupe alkyle en C₁₋₆ substitué par un groupe cycloalkyle en C₃₋₇, un groupe cycloalcényle en C₃₋₇, un groupe alkyle en C₁₋₆ substitué par un groupe cycloalcényle en C₃₋₇, un groupe alcényle en C₂₋₈, un groupe alcynyle en C₂₋₈, un groupe alkyle en C₁₋₆ substitué par un groupe alcoxy en C₁₋₆, un groupe alkyle en C₁₋₆ substitué par un groupe alcényloxy en C₂₋₆, un groupe alkyle en C₁₋₆ substitué par un groupe alcynyloxy en C₂₋₆, un groupe alkyle en C₁₋₆ substitué par un groupe mono-, di- ou poly-halogénoalcoxy en C₁₋₆, un groupe alkyle en C₁₋₆ substitué par un groupe mono-, di- ou poly-halogénoalcényloxy en C₂₋₆, un groupe alkyle en C₁₋₆ substitué par un groupe mono-, di- ou poly-halogénoalcynyloxy en C₂₋₆, un groupe alkyle en C₁₋₆ substitué par un groupe alkylthio en C₁₋₆, un groupe alkyle en C₁₋₆ substitué par un groupe alkylsulfinyle en C₁₋₆, un groupe alkyle en C₁₋₆ substitué par un groupe alkylsulfonyle en C₁₋₆, un groupe mono-, di- ou poly-halogénoalkyle en C₁₋₈, un groupe mono-, di- ou poly-halogénoalcényle en C₂₋₈, un groupe mono-, di- ou poly-halogénoalcynyle en C₂₋₈, un groupe alkyle en C₁₋₆ substitué par un groupe cyano, un groupe alcényle en C₂₋₆ substitué par un groupe cyano, un groupe alcynyle en C₂₋₆ substitué par un groupe cyano, un groupe alkyle en C₁₋₆ substitué par un groupe nitro, un groupe alcényle en C₂₋₆ substitué par un groupe nitro, un groupe alcynyle en C₂₋₆ substitué par un groupe nitro, un groupe alkyle en C₁₋₆ substitué par un groupe alcoxycarbonyle en C₂₋₇, un groupe alcényle en C₂₋₆ substitué par un groupe alcoxycarbonyle en C₂₋₇, un groupe alcynyle en C₂₋₆ substitué par un groupe alcoxycarbonyle en C₂₋₇, un groupe alkyle en C₁₋₆ substitué par un groupe alkylcarbonyle en C₂₋₇, un groupe alkyle en C₁₋₆ substitué par un groupe mono-, di- ou poly-halogénoalkylcarbonyle en C₂₋₇, un groupe alkyle en C₁₋₆ substitué par un groupe alcénylcarbonyle en C₃₋₇, un groupe alkyle en C₁₋₆ substitué par un groupe alcynylcarbonyle en C₃₋₇, un groupe alkyle en C₁₋₆ substitué par un groupe alkylcarbonyle en C₂₋₅ substitué par un groupe alcoxy en C₁₋₄, un groupe alkyle en C₁₋₆ substitué par un groupe alkylcarbonyle en C₂₋₅ substitué par un groupe alkylthio en C₁₋₄, un groupe alkyle en C₁₋₆ substitué par un groupe alkylcarbonyle en C₂₋₅ substitué par un groupe alkylsulfinyle en C₁₋₄, un groupe alkyle en C₁₋₆ substitué par un groupe alkylcarbonyle en C₂₋₅ substitué par un groupe alkylsulfonyle en C₁₋₄, un groupe alcényle en C₂₋₆ substitué par un groupe alkylcarbonyle en C₂₋₇, un groupe alcynyle en C₂₋₆ substitué par un groupe alkylcarbonyle en C₂₋₇, un groupe alkyle en C₁₋₆ substitué par un groupe alkylsulfamoyle en C₁₋₆, un groupe alkyle en C₁₋₆ substitué par un groupe alcoxysulfamoyle en C₁₋₆, un groupe alkyle en C₁₋₆ substitué par un groupe di(alkyl en C₁₋₃)sulfamoyle, un groupe alkyle en C₁₋₆ substitué par un groupe N-(alkyl en C₁₋₃)-N-(alcoxy en C₁₋₃)sulfamoyle, un groupe alkyle en C₁₋₆ substitué par un groupe alkylcarbamoyle en C₂₋₇, un groupe alkyle en C₁₋₆ substitué par un groupe di(alkyl en C₁₋₃)carbamoyle, un groupe alkyle en C₁₋₆ substitué par un groupe alcoxycarbamoyle en C₂₋₇, un groupe alkyle en C₁₋₆ substitué par un groupe N-(alkyl en C₁₋₃)-N-(alcoxy en C₁₋₃)carbamoyle, un groupe alkyle en C₁₋₆ substitué par un groupe alkylamino en C₁₋₆, un groupe alkyle en C₁₋₆ substitué par un groupe alcoxyamino en C₁₋₆, un groupe alkyle en C₁₋₆ substitué par un groupe di(alkyl en C₁₋₃)amino, un groupe alkyle en C₁₋₆ substitué par un groupe N-(alkyl en C₁₋₃)-N-(alcoxy en C₁₋₃)amino, un groupe alkyle en C₁₋₆ substitué par un groupe N-(alkylcarbonyl en C₂₋₇)-N-(alkyl en C₁₋₆)amino, un groupe alkyle en C₁₋₆ substitué par un groupe N-(alkylcarbonyl en C₂₋₇)-N-(alcoxy en C₁₋₆)amino, un groupe alkyle en C₁₋₆ substitué par un groupe N-(alkylsulfonyl en C₁₋₆)-N-(alkyl en C₁₋₆)amino, un groupe alkyle en C₁₋₆ substitué par un groupe N-(alkylsulfonyl en C₁₋₆)-N-(alcoxy en C₁₋₆)amino, un groupe phényle (à la condition qu'un tel groupe phényle puisse être substitué par un ou plusieurs substituants choisis dans le groupe constitué par un atome d'halogène, un groupe trifluorométhyle, un groupe nitro, un groupe alkyle en C₁₋₆, un groupe alcoxy en C₁₋₆ et un groupe alcoxycarbonyle en C₂₋₇), un groupe alkyle en C₁₋₆ substitué par un groupe phényle (à la condition qu'un tel groupe phényle puisse être substitué par un ou plusieurs substituants choisis dans le groupe constitué par un atome d'halogène, un groupe trifluorométhyle, un groupe nitro, un groupe alkyle en C₁₋₆, un groupe alcoxy en C₁₋₆ et un groupe alcoxycarbonyle en C₂₋₇), un groupe alcényle en C₂₋₇ substitué par un groupe phényle (à la condition qu'un tel groupe phényle puisse être substitué par un ou plusieurs substituants choisis dans le groupe constitué par un atome d'halogène, un groupe trifluorométhyle, un groupe nitro, un groupe alkyle en C₁₋₆, un groupe alcoxy en C₁₋₆ et un groupe alcoxycarbonyle en C₂₋₇), un groupe alcynyle en C₂₋₆ substitué par un groupe phényle (à la condition qu'un tel groupe phényle puisse être substitué par un ou plusieurs substituants choisis dans le groupe constitué par un atome d'halogène, un groupe trifluorométhyle, un groupe nitro, un groupe alkyle en C₁₋₆, un groupe alcoxy en C₁₋₆ et un groupe alcoxycarbonyle en C₂₋₇), un groupe alkyle en C₁₋₆ substitué par un groupe phénoxy (à la condition qu'un tel groupe phénoxy puisse être substitué par un ou plusieurs substituants choisis dans le groupe constitué par un atome d'halogène, un groupe trifluorométhyle, un groupe nitro, un groupe alkyle en C₁₋₆, un groupe alcoxy en C₁₋₆ et un groupe alcoxy-carbonyle en C₂₋₇), un groupe alkyle en C₁₋₆ substitué par un groupe phénylthio (à la condition qu'un tel groupe phénylthio puisse être substitué par un ou plusieurs substituants choisis dans le groupe constitué par un atome d'halogène, un groupe trifluorométhyle, un groupe nitro, un groupe alkyle en C₁₋₆, un groupe alcoxy en C₁₋₆ et un groupe alcoxycarbonyle en C₂₋₇), un groupe alkyle en C₁₋₆ substitué par un groupe phénylsulfinyle (à la condition qu'un tel groupe phénylsulfinyle puisse être substitué par un ou plusieurs substituants choisis dans le groupe constitué par un atome d'halogène, un groupe trifluorométhyle, un groupe nitro, un groupe alkyle en C₁₋₆, un groupe alcoxy en C₁₋₆ et un groupe alcoxycarbonyle en C₂₋₇), un groupe alkyle en C₁₋₆ substitué par un groupe phénylsulfonyle (à la condition qu'un tel groupe phénylsulfonyle puisse être substitué par un ou plusieurs substituants choisis dans le groupe constitué par un atome d'halogène, un groupe trifluorométhyle, un groupe nitro, un groupe alkyle en C₁₋₆, un groupe alcoxy en C₁₋₆ et un groupe alcoxy-carbonyle en C₂₋₇), un groupe alkyle en C₁₋₆ substitué par un groupe benzyloxy (à la condition que le groupe phényle d'un tel groupe benzyloxy puisse être substitué par un ou plusieurs substituants choisis dans le groupe constitué par un atome d'halogène, un groupe trifluorométhyle, un groupe nitro, un groupe alkyle en C₁₋₆, un groupe alcoxy en C₁₋₆ et un groupe alcoxy-carbonyle en C₂₋₇), un groupe alkyle en C₁₋₆ substitué par un groupe benzylthio (à la condition que le groupe phényle d'un tel groupe benzylthio puisse être substitué par un ou plusieurs substituants choisis dans le groupe constitué par un atome d'halogène, un groupe trifluorométhyle, un groupe nitro, un groupe alkyle en C₁₋₆, un groupe alcoxy en C₁₋₆ et un groupe alcoxycarbonyle en C₂₋₇), un groupe alkyle en C₁₋₆ substitué par un groupe benzylsulfinyle (à la condition que le groupe phényle d'un tel groupe benzylsulfinyle puisse être substitué par un ou plusieurs substituants choisis dans le groupe constitué par un atome d'halogène, un groupe trifluorométhyle, un groupe nitro, un groupe alkyle en C₁₋₆, un groupe alcoxy en C₁₋₆ et un groupe alcoxycarbonyle en C₂₋₇), un groupe alkyle en C₁₋₆ substitué par un groupe benzylsulfonyle (à la condition que le groupe phényle d'un tel groupe benzylsulfonyle puisse être substitué par un ou plusieurs substituants choisis dans le groupe constitué par un atome d'halogène, un groupe trifluorométhyle, un groupe nitro, un groupe alkyle en C₁₋₆, un groupe alcoxy en C₁₋₆ et un groupe alcoxycarbonyle en C₂₋₇), un groupe alkyle en C₁₋₆ substitué par un groupe phénylcarbonyle (à la condition qu'un tel groupe phénylcarbonyle puisse être substitué par un ou plusieurs substituants choisis dans le groupe constitué par un atome d'halogène, un groupe trifluorométhyle, un groupe nitro, un groupe alkyle en C₁₋₆, un groupe alcoxy en C₁₋₆ et un groupe alcoxycarbonyle en C₂₋₇), un groupe alkyle en C₁₋₆ substitué par un groupe benzylcarbonyle (à la condition que le groupe phényle d'un tel groupe benzylcarbonyle puisse être substitué par un ou plusieurs substituants choisis dans le groupe constitué par un atome d'halogène, un groupe trifluorométhyle, un groupe nitro, un groupe alkyle en C₁₋₆, un groupe alcoxy en C₁₋₆ et un groupe alcoxycarbonyle en C₂₋₇), un groupe alkyle en C₁₋₆ substitué par un groupe amino substitué par un groupe alkylsulfonyle en C₁₋₄, ou un groupe alkyle en C₁₋₆ substitué par un groupe amino substitué par un groupe alkylcarbonyle en C₂₋₄ ;
- R^{f2} est un groupe alkyle en C₁₋₈, un groupe alcényle en C₂₋₈, un groupe alcynyle en C₂₋₈, un groupe alkyle en C₁₋₆ substitué par un groupe alkylthio en C₁₋₆, un groupe alkyle en C₁₋₆ substitué par un groupe alkylsulfinyle en C₁₋₆, un groupe alkyle en C₁₋₆ substitué par un groupe alkylsulfonyle en C₁₋₆, un groupe alkyle en C₁₋₆ substitué par un groupe alcoxy en C₁₋₆, un groupe alkyle en C₁₋₆ substitué par un groupe alcoxycarbonyle en C₂₋₇, un groupe alkyle en C₁₋₆ substitué par un groupe alkylcarbonyle en C₂₋₇, un groupe alkyle en C₁₋₆ substitué par un groupe cyano, un groupe phényle (à la condition qu'un tel groupe phényle puisse être substitué par un ou plusieurs substituants choisis dans le groupe constitué par un atome d'halogène, un groupe trifluorométhyle, un groupe nitro, un groupe alkyle en C₁₋₆, un groupe alcoxy en C₁₋₆ et un groupe alcoxycarbonyle en C₂₋₇), ou un groupe alkyle en C₁₋₆ substitué par un groupe phényle (à la condition qu'un tel groupe phényle puisse être substitué par un ou plusieurs substituants choisis dans le groupe constitué par un atome d'halogène, un groupe trifluorométhyle, un groupe nitro, un groupe alkyle en C₁₋₆, un groupe alcoxy en C₁₋₆ et un groupe alcoxycarbonyle en C₂₋₇) ;
- R^{f3}, R^{f4}, R^{f7}, R^{f8}, R^{f11} et R^{f12}, qui sont indépendants l'un de l'autre, représentent chacun un atome d'hydrogène, un groupe alkyle en C₁₋₆, un groupe mono-, di- ou poly-halogénoalkyle en C₁₋₆, un groupe alcoxy en C₁₋₆, un groupe mono-, di- ou poly-halogénoalcoxy en C₁₋₆, un groupe alkylthio en C₁₋₆, un groupe alkylsulfinyle en C₁₋₆, un groupe alkylsulfonyle en C₁₋₆, un groupe alcoxycarbonyle en C₂₋₇, un groupe alkylcarbonyle en C₂₋₇, un atome d'halogène, un groupe nitro, un groupe cyano, ou un groupe phényle (à la condition qu'un tel groupe phényle puisse être substitué par un ou plusieurs substituants choisis dans le groupe constitué par un atome d'halogène, un groupe trifluorométhyle, un groupe nitro, un groupe alkyle en C₁₋₆, un groupe alcoxy en C₁₋₆ et un groupe alcoxycarbonyle en C₂₋₇) ;
- R^{f5}, R^{f6}, R^{f9}, R^{f10}, R^{f13}, R^{f14} et R^{f15}, qui sont indépendants l'un de l'autre, représentent chacun un atome d'hydrogène, un groupe alkyle en C₁₋₈, un groupe alcényle en C₂₋₈, un groupe alcynyle en C₂₋₈, ou un groupe phényle (à la condition qu'un tel groupe phényle puisse être substitué par un ou plusieurs substituants choisis dans le groupe constitué par un atome d'halogène, un groupe trifluorométhyle, un groupe nitro, un groupe alkyle en C₁₋₆, un groupe alcoxy en C₁₋₆ et un groupe alcoxycarbonyle en C₂₋₇) ;
- R^{g1} est un groupe alkyle en C₁₋₈, un groupe cycloalkyle en C₃₋₇, un groupe alkyle en C₁₋₆ substitué par un groupe cycloalkyle en C₃₋₇, un groupe cycloalcényle en C₃₋₇, un groupe alkyle en C₁₋₆ substitué par un groupe cycloalcényle en C₃₋₇, un groupe alcényle en C₂₋₈, un groupe alcynyle en C₂₋₈, un groupe alkyle en C₁₋₆ substitué par un groupe alcoxy en C₁₋₆, un groupe alkyle en C₁₋₆ substitué par un groupe alcényloxy en C₂₋₆, un groupe alkyle en C₁₋₆ substitué par un groupe alcynyloxy en C₂₋₆, un groupe alkyle en C₁₋₆ substitué par un groupe mono-, di- ou poly-halogénoalcoxy en C₁₋₆, un groupe alkyle en C₁₋₆ substitué par un groupe mono-, di- ou poly-halogénoalcényloxy en C₂₋₆, un groupe alkyle en C₁₋₆ substitué par un groupe mono-, di- ou poly-halogénoalcynyloxy en C₂₋₆, un groupe alkyle en C₁₋₆ substitué par un groupe alkylthio en C₁₋₆, un groupe alkyle en C₁₋₆ substitué par un groupe alkylsulfinyle en C₁₋₆, un groupe alkyle en C₁₋₆ substitué par un groupe alkylsulfonyle en C₁₋₆, un groupe mono-, di- ou poly-halogénoalkyle en C₁₋₈, un groupe mono-, di- ou poly-halogénoalcényle en C₂₋₈, un groupe mono-, di- ou poly-halogénoalcynyle en C₂₋₈, un groupe alkyle en C₁₋₆ substitué par un groupe cyano, un groupe alcényle en C₂₋₆ substitué par un groupe cyano, un groupe alcynyle en C₂₋₆ substitué par un groupe cyano, un groupe alkyle en C₁₋₆ substitué par un groupe nitro, un groupe alcényle en C₂₋₆ substitué par un groupe nitro, un groupe alcynyle en C₂₋₆ substitué par un groupe nitro, un groupe alkyle en C₁₋₆ substitué par un groupe alcoxycarbonyle en C₂₋₇, un groupe alcényle en C₂₋₆ substitué par un groupe alcoxycarbonyle en C₂₋₇, un groupe alcynyle en C₂₋₆ substitué par un groupe alcoxycarbonyle en C₂₋₇, un groupe alkyle en C₁₋₆ substitué par un groupe alkylcarbonyle en C₂₋₇, un groupe alkyle en C₁₋₆ substitué par un groupe mono-, di- ou poly-halogénoalkylcarbonyle en C₂₋₇, un groupe alkyle en C₁₋₆ substitué par un groupe alcénylcarbonyle en C₃₋₇, un groupe alkyle en C₁₋₆ substitué par un groupe alcynylcarbonyle en C₃₋₇, un groupe alkyle en C₁₋₆ substitué par un groupe alkylcarbonyle en C₂₋₅ substitué par un groupe alcoxy en C₁₋₄, un groupe alkyle en C₁₋₆ substitué par un groupe alkylcarbonyle en C₂₋₅ substitué par un groupe alkylthio en C₁₋₄, un groupe alkyle en C₁₋₆ substitué par un groupe alkylcarbonyle en C₂₋₅ substitué par un groupe alkylsulfinyle en C₁₋₄, un groupe alkyle en C₁₋₆ substitué par un groupe alkylcarbonyle en C₂₋₅ substitué par un groupe alkylsulfonyle en C₁₋₄, un groupe alcényle en C₂₋₆ substitué par un groupe alkylcarbonyle en C₂₋₇, un groupe alcynyle en C₂₋₆ substitué par un groupe alkylcarbonyle en C₂₋₇, un groupe alkyle en C₁₋₆ substitué par un groupe alkylsulfamoyle en C₁₋₆, un groupe alkyle en C₁₋₆ substitué par un groupe alcoxysulfamoyle en C₁₋₆, un groupe alkyle en C₁₋₆ substitué par un groupe di(alkyl en C₁₋₃)sulfamoyle, un groupe alkyle en C₁₋₆ substitué par un groupe N-(alkyl en C₁₋₃)-N-(alcoxy en C₁₋₃)sulfamoyle, un groupe alkyle en C₁₋₆ substitué par un groupe alkylcarbamoyle en C₂₋₇, un groupe alkyle en C₁₋₆ substitué par un groupe di(alkyl en C₁₋₃)carbamoyle, un groupe alkyle en C₁₋₆ substitué par un groupe alcoxycarbamoyle en C₂₋₇, un groupe alkyle en C₁₋₆ substitué par un groupe N-(alkyl en C₁₋₃)-N-(alcoxy en C₁₋₃)carbamoyle, un groupe alkyle en C₁₋₆ substitué par un groupe alkylamino en C₁₋₆, un groupe alkyle en C₁₋₆ substitué par un groupe alcoxyamino en C₁₋₆, un groupe alkyle en C₁₋₆ substitué par un groupe di(alkyl en C₁₋₃)amino, un groupe alkyle en C₁₋₆ substitué par un groupe N-(alkyl en C₁₋₃)-N-(alcoxy en C₁₋₃)amino, un groupe alkyle en C₁₋₆ substitué par un groupe N-(alkylcarbonyl en C₂₋₇)-N-(alkyl en C₁₋₆)amino, un groupe alkyle en C₁₋₆ substitué par un groupe N-(alkylcarbonyl en C₂₋₇)-N-(alcoxy en C₁₋₆)amino, un groupe alkyle en C₁₋₆ substitué par un groupe N-(alkylsulfonyl en C₁₋₆)-N-(alkyl en C₁₋₆)amino, un groupe alkyle en C₁₋₆ substitué par un groupe N-(alkylsulfonyl en C₁₋₆)-N-(alcoxy en C₁₋₆)amino, un groupe alkyle en C₁₋₆ substitué par un groupe phényle (à la condition qu'un tel groupe phényle puisse être substitué par un ou plusieurs substituants choisis dans le groupe constitué par un atome d'halogène, un groupe trifluorométhyle, un groupe nitro, un groupe alkyle en C₁₋₆, un groupe alcoxy en C₁₋₆ et un groupe alcoxycarbonyle en C₂₋₇), un groupe alcényle en C₂₋₇ substitué par un groupe phényle (à la condition qu'un tel groupe phényle puisse être substitué par un ou plusieurs substituants choisis dans le groupe constitué par un atome d'halogène, un groupe trifluorométhyle, un groupe nitro, un groupe alkyle en C₁₋₆, un groupe alcoxy en C₁₋₆ et un groupe alcoxycarbonyle en C₂₋₇), un groupe alcynyle en C₂₋₆ substitué par un groupe phényle (à la condition qu'un tel groupe phényle puisse être substitué par un ou plusieurs substituants choisis dans le groupe constitué par un atome d'halogène, un groupe trifluorométhyle, un groupe nitro, un groupe alkyle en C₁₋₆, un groupe alcoxy en C₁₋₆ et un groupe alcoxy-carbonyle en C₂₋₇), un groupe alkyle en C₁₋₆ substitué par un groupe phénoxy (à la condition qu'un tel groupe phénoxy puisse être substitué par un ou plusieurs substituants choisis dans le groupe constitué par un atome d'halogène, un groupe trifluorométhyle, un groupe nitro, un groupe alkyle en C₁₋₆, un groupe alcoxy en C₁₋₆ et un groupe alcoxycarbonyle en C₂₋₇), un groupe alkyle en C₁₋₆ substitué par un groupe phénylthio (à la condition qu'un tel groupe phénylthio puisse être substitué par un ou plusieurs substituants choisis dans le groupe constitué par un atome d'halogène, un groupe trifluorométhyle, un groupe nitro, un groupe alkyle en C₁₋₆, un groupe alcoxy en C₁₋₆ et un groupe alcoxy-carbonyle en C₂₋₇), un groupe alkyle en C₁₋₆ substitué par un groupe phénylsulfinyle (à la condition qu'un tel groupe phénylsulfinyle puisse être substitué par un ou plusieurs substituants choisis dans le groupe constitué par un atome d'halogène, un groupe trifluorométhyle, un groupe nitro, un groupe alkyle en C₁₋₆, un groupe alcoxy en C₁₋₆ et un groupe alcoxycarbonyle en C₂₋₇), un groupe alkyle en C₁₋₆ substitué par un groupe phénylsulfonyle (à la condition qu'un tel groupe phénylsulfonyle puisse être substitué par un ou plusieurs substituants choisis dans le groupe constitué par un atome d'halogène, un groupe trifluorométhyle, un groupe nitro, un groupe alkyle en C₁₋₆, un groupe alcoxy en C₁₋₆ et un groupe alcoxycarbonyle en C₂₋₇), un groupe alkyle en C₁₋₆ substitué par un groupe benzyloxy (à la condition que le groupe phényle d'un tel groupe benzyloxy puisse être substitué par un ou plusieurs substituants choisis dans le groupe constitué par un atome d'halogène, un groupe trifluorométhyle, un groupe nitro, un groupe alkyle en C₁₋₆, un groupe alcoxy en C₁₋₆ et un groupe alcoxycarbonyle en C₂₋₇), un groupe alkyle en C₁₋₆ substitué par un groupe benzylthio (à la condition que le groupe phényle d'un tel groupe benzylthio puisse être substitué par un ou plusieurs substituants choisis dans le groupe constitué par un atome d'halogène, un groupe trifluorométhyle, un groupe nitro, un groupe alkyle en C₁₋₆, un groupe alcoxy en C₁₋₆ et un groupe alcoxycarbonyle en C₂₋₇), un groupe alkyle en C₁₋₆ substitué par un groupe benzylsulfinyle (à la condition que le groupe phényle d'un tel groupe benzylsulfinyle puisse être substitué par un ou plusieurs substituants choisis dans le groupe constitué par un atome d'halogène, un groupe trifluorométhyle, un groupe nitro, un groupe alkyle en C₁₋₆, un groupe alcoxy en C₁₋₆ et un groupe alcoxycarbonyle en C₂₋₇), un groupe alkyle en C₁₋₆ substitué par un groupe benzylsulfonyle (à la condition que le groupe phényle d'un tel groupe benzylsulfonyle puisse être substitué par un ou plusieurs substituants choisis dans le groupe constitué par un atome d'halogène, un groupe trifluorométhyle, un groupe nitro, un groupe alkyle en C₁₋₆, un groupe alcoxy en C₁₋₆ et un groupe alcoxycarbonyle en C₂₋₇), un groupe alkyle en C₁₋₆ substitué par un groupe phénylcarbonyle (à la condition qu'un tel groupe phénylcarbonyle puisse être substitué par un ou plusieurs substituants choisis dans le groupe constitué par un atome d'halogène, un groupe trifluorométhyle, un groupe nitro, un groupe alkyle en C₁₋₆, un groupe alcoxy en C₁₋₆ et un groupe alcoxycarbonyle en C₂₋₇), ou un groupe alkyle en C₁₋₆ substitué par un groupe benzylcarbonyle (à la condition que le groupe phényle d'un tel groupe benzylcarbonyle puisse être substitué par un ou plusieurs substituants choisis dans le groupe constitué par un atome d'halogène, un groupe trifluorométhyle, un groupe nitro, un groupe alkyle en C₁₋₆, un groupe alcoxy en C₁₋₆ et un groupe alcoxycarbonyle en C₂₋₇),
- R^{g2} et R^{g3}, qui sont indépendants l'un de l'autre, représentent chacun un groupe alkyle en C₁₋₆, un groupe alcényle en C₂₋₆, un groupe alcynyle en C₂₋₆, un groupe mono-, di- ou poly-halogénoalkyle en C₁₋₆, un groupe alcoxy en C₁₋₆, un groupe alkylsulfonyle en C₁₋₆, un groupe alkylsulfamoyle en C₁₋₆, un groupe di(alkyl en C₁₋₃)sulfamoyle, un groupe alcoxycarbonyle en C₂₋₇, un groupe alkylcarbonyle en C₂₋₇, un groupe alkylcarbamoyle en C₂₋₇, un groupe di(alkyl en C₁₋₃)carbamoyle, un groupe phényle (à la condition qu'un tel groupe phényle puisse être substitué par un ou plusieurs substituants choisis dans le groupe constitué par un atome d'halogène, un groupe trifluorométhyle, un groupe nitro, un groupe alkyle en C₁₋₆, un groupe alcoxy en C₁₋₆ et un groupe alcoxycarbonyle en C₂₋₇), ou un groupe benzyle (à la condition que le groupe phényle d'un tel groupe benzyle puisse être substitué par un ou plusieurs substituants choisis dans le groupe constitué par un atome d'halogène, un groupe trifluorométhyle, un groupe nitro, un groupe alkyle en C₁₋₆, un groupe alcoxy en C₁₋₆ et un groupe alcoxycarbonyle en C₂₋₇) ; ou
- R^{g2} et R^{g3} forment un groupe hétérocyclique saturé, à 3 à 7 chaînons, conjointement avec l'atome d'azote auxquels ils sont liés ;
- X est un atome d'oxygène ou un atome de soufre ;
- L est un atome d'hydrogène, un groupe alkyle en C₁₋₆, un groupe alcényle en C₂₋₆ ou un groupe alcynyle en C₂₋₆ ;
- G représente : 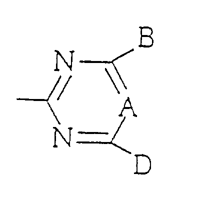
- A est un groupe CH ou un atome d'azote, et
- D et B, qui sont indépendants l'un de l'autre, représentent chacun un groupe alkyle en C₁₋₄, un groupe alcoxy en C₁₋₄, un groupe mono-, di- ou poly-halogénoalkyle en C₁₋₄, un groupe mono-, di- ou poly-halogénoalcoxy en C₁₋₄, un atome d'halogène, un groupe alkylamino en C₁₋₄ ou un groupe di(alkyl en C₁₋₄)amino.

3. Dérivé d'iminosulfonylurée ou son sel, selon la revendication 2, dans lequel Q représente :

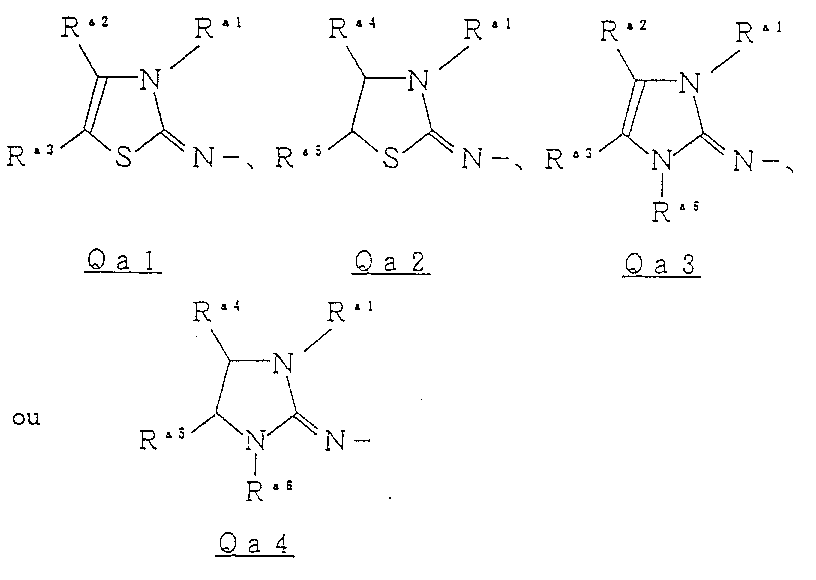

4. Dérivé d'iminosulfonylurée ou son sel, selon la revendication 2, dans lequel Q représente :

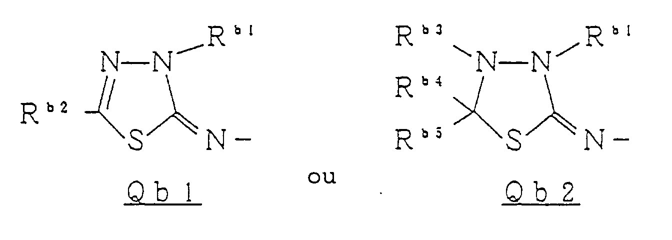

5. Dérivé d'iminosulfonylurée ou son sel, selon la revendication 2, dans lequel Q représente :

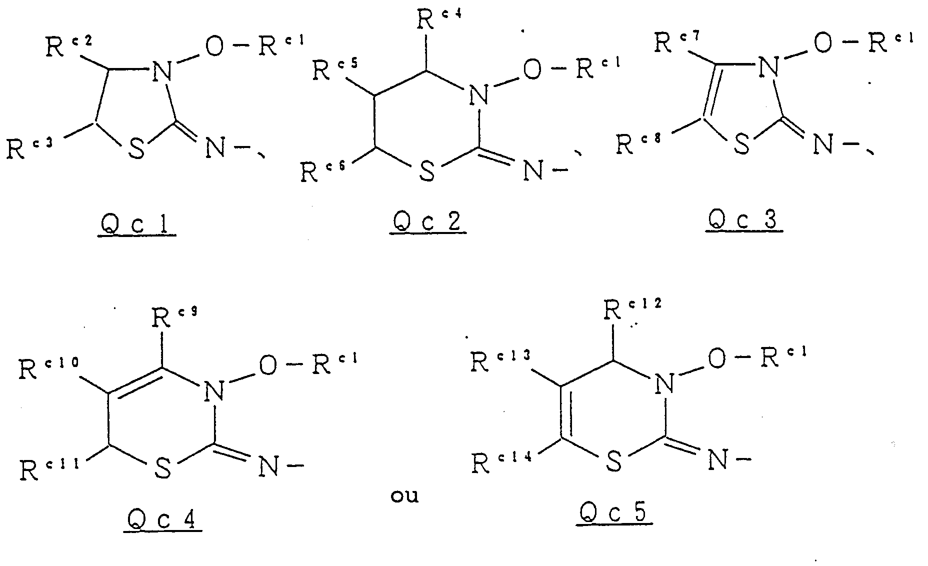

6. Dérivé d'iminosulfonylurée ou son sel, selon la revendication 2, dans lequel Q représente :

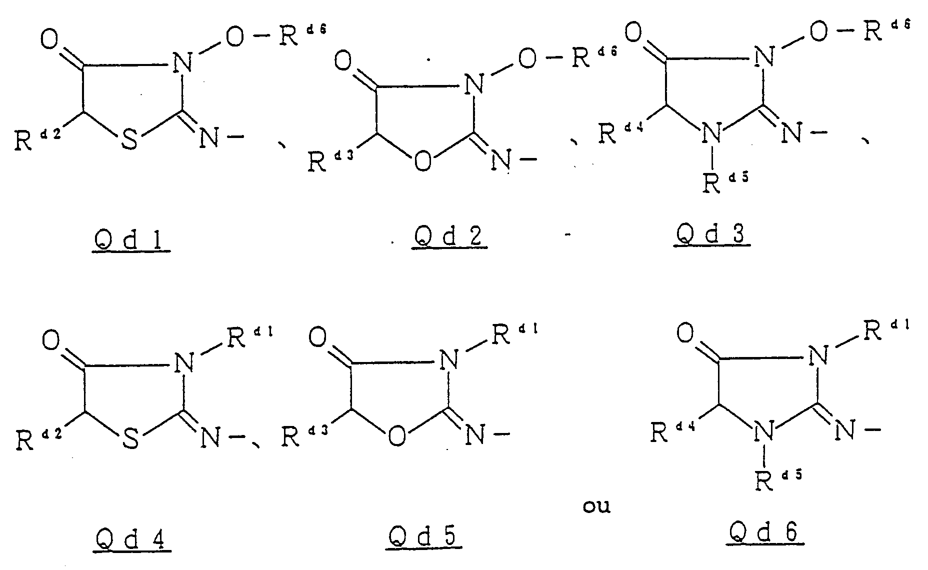

7. Dérivé d'iminosulfonylurée ou son sel, selon la revendication 2, dans lequel Q représente :

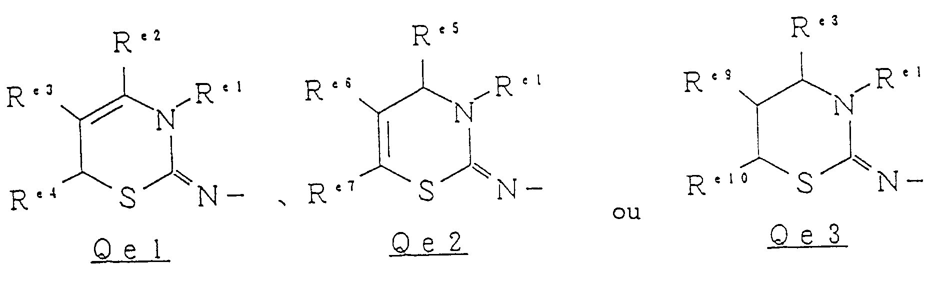

8. Dérivé d'iminosulfonylurée ou son sel, selon la revendication 2, dans lequel Q représente :

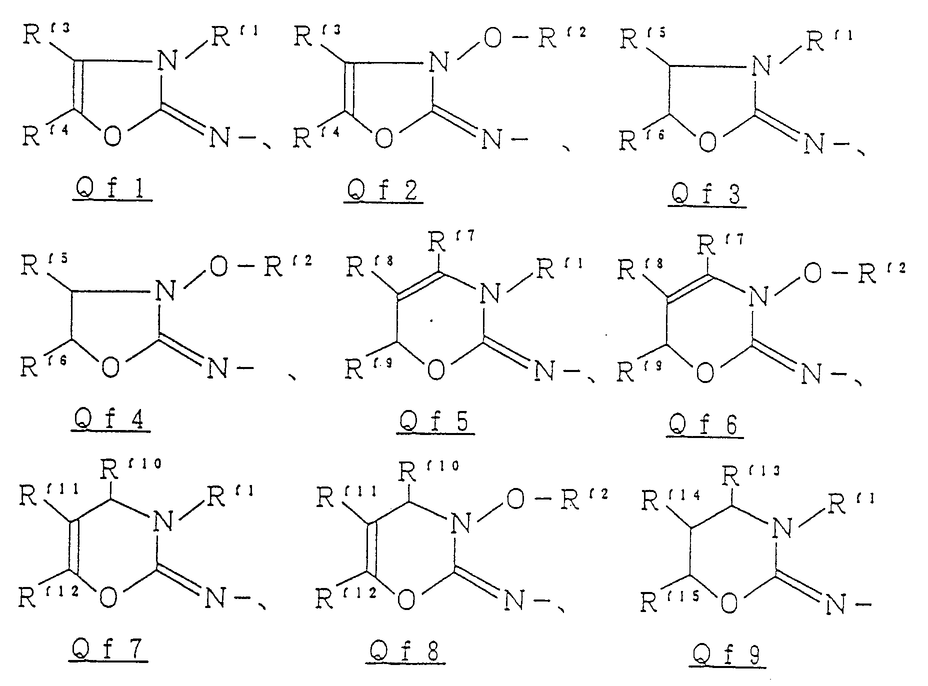

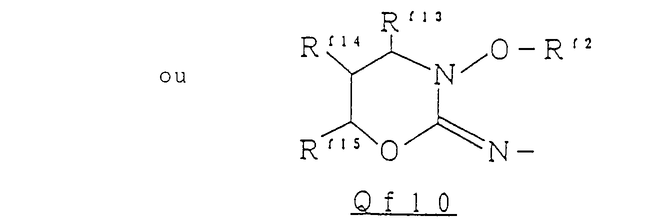

9. Dérivé d'iminosulfonylurée ou son sel, selon la revendication 2, dans lequel Q représente :

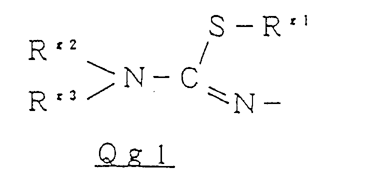

10. Herbicide qui contient un dérivé d'iminosulfonylurée tel que défini à la revendication 1, en tant qu'ingrédient actif.

11. Herbicide qui contient un dérivé d'iminosulfonylurée tel que défini à la revendication 2, en tant qu'ingrédient actif.

12. Herbicide qui contient un dérivé d'iminosulfonylurée tel que défini à la revendication 3, en tant qu'ingrédient actif.

13. Herbicide qui contient un dérivé d'iminosulfonylurée tel que défini à la revendication 4, en tant qu'ingrédient actif.

14. Herbicide qui contient un dérivé d'iminosulfonylurée tel que défini à la revendication 5, en tant qu'ingrédient actif.

15. Herbicide qui contient un dérivé d'iminosulfonylurée tel que défini à la revendication 6, en tant qu'ingrédient actif.

16. Herbicide qui contient un dérivé d'iminosulfonylurée tel que défini à la revendication 7, en tant qu'ingrédient actif.

17. Herbicide qui contient un dérivé d'iminosulfonylurée tel que défini à la revendication 8, en tant qu'ingrédient actif.

18. Herbicide qui contient un dérivé d'iminosulfonylurée tel que défini à la revendication 9, en tant qu'ingrédient actif.

19. Procédé herbicide et de lutte contre la croissance à l'encontre de mauvaises herbes, qui comprend l'application d'une quantité efficace du point de vue herbicide d'un dérivé d'iminosulfonylurée tel que défini à la revendication 1.

20. Procédé herbicide et de lutte contre la croissance à l'encontre des mauvaises herbes, qui comprend l'application d'une quantité efficace du point de vue herbicide d'un dérivé d'iminosulfonylurée tel que défini à la revendication 2.

21. Procédé herbicide et de lutte contre la croissance à l'encontre des mauvaises herbes, qui comprend l'application d'une quantité efficace du point de vue herbicide d'un dérivé d'iminosulfonylurée tel que défini à la revendication 3.

22. Procédé herbicide et de lutte contre la croissance à l'encontre des mauvaises herbes, qui comprend l'application d'une quantité efficace du point de vue herbicide d'un dérivé d'iminosulfonylurée tel que défini à la revendication 4.

23. Procédé herbicide et de lutte contre la croissance à l'encontre des mauvaises herbes, qui comprend l'application d'une quantité efficace du point de vue herbicide d'un dérivé d'iminosulfonylurée tel que défini à la revendication 5.

24. Procédé herbicide et de lutte contre la croissance à l'encontre des mauvaises herbes, qui comprend l'application d'une quantité efficace du point de vue herbicide d'un dérivé d'iminosulfonylurée tel que défini à la revendication 6.

25. Procédé herbicide et de lutte contre la croissance à l'encontre des mauvaises herbes, qui comprend l'application d'une quantité efficace du point de vue herbicide d'un dérivé d'iminosulfonylurée tel que défini à la revendication 7.

26. Procédé herbicide et de lutte contre la croissance à l'encontre des mauvaises herbes, qui comprend l'application d'une quantité efficace du point de vue herbicide d'un dérivé d'iminosulfonylurée tel que défini à la revendication 8.

27. Procédé herbicide et de lutte contre la croissance à l'encontre des mauvaises herbes, qui comprend l'application d'une quantité efficace du point de vue herbicide d'un dérivé d'iminosulfonylurée tel que défini à la revendication 9.

28. Dérivé d'iminosulfonylurée ou son sel, selon la revendication 3, dans lequel :
- R^{a1} représente : 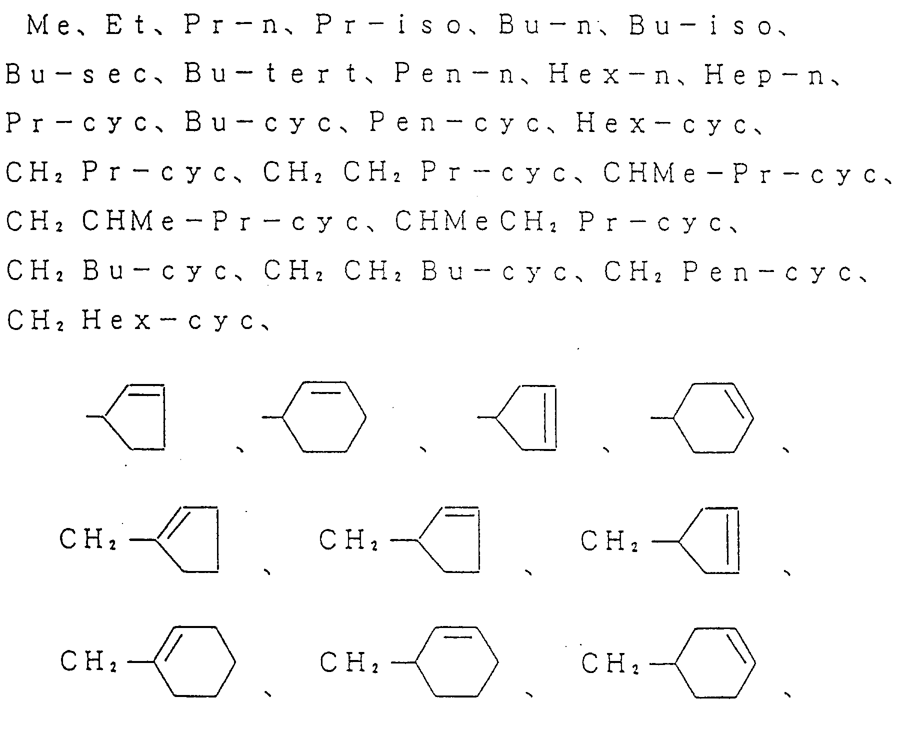 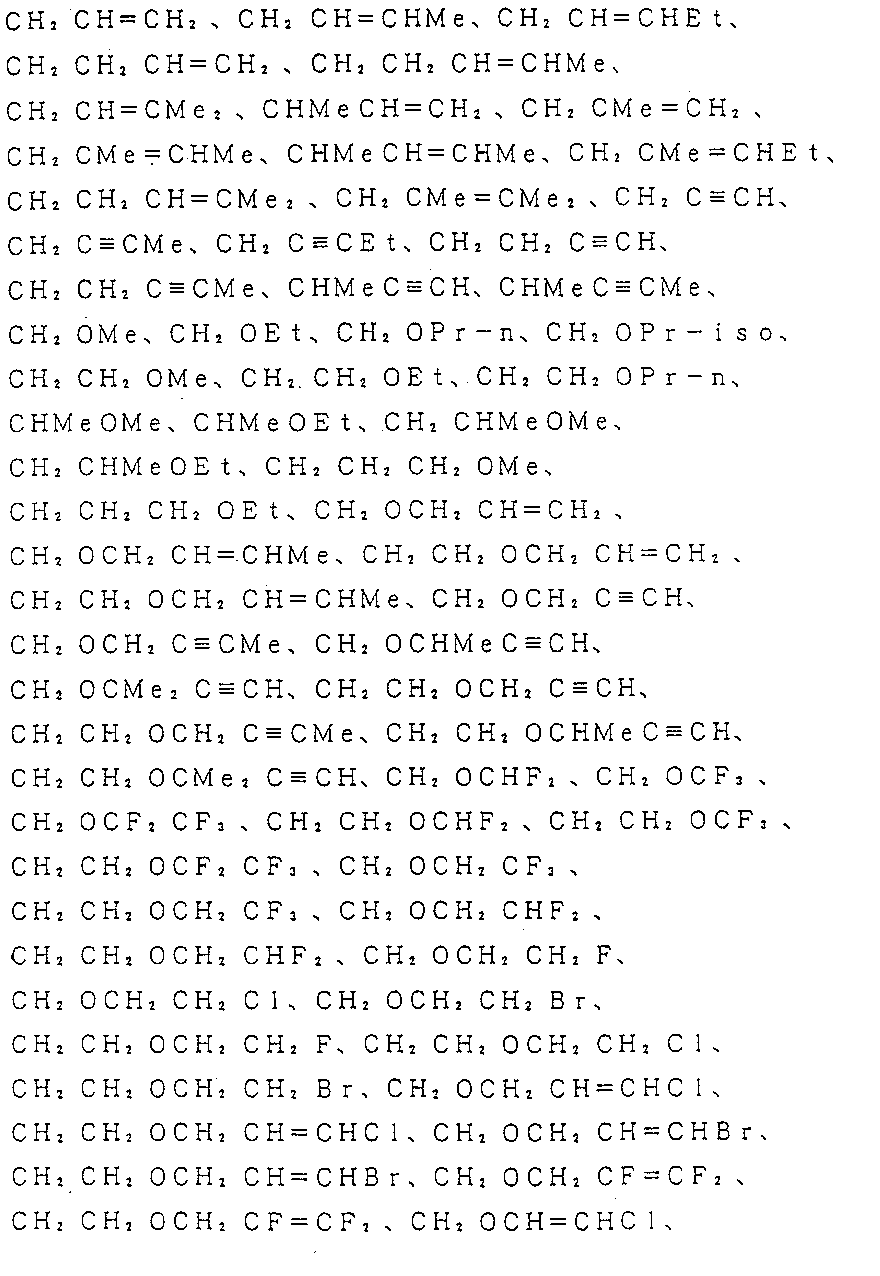 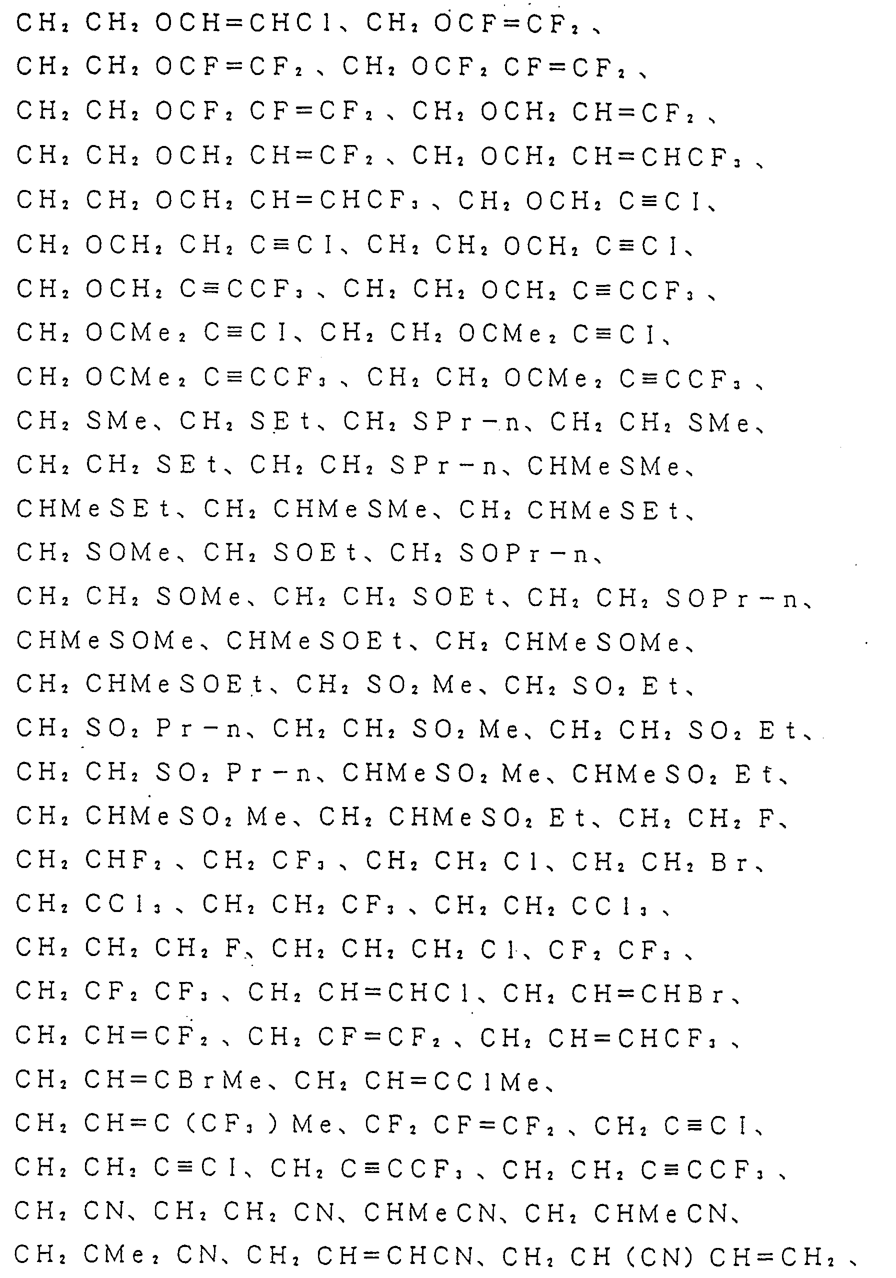 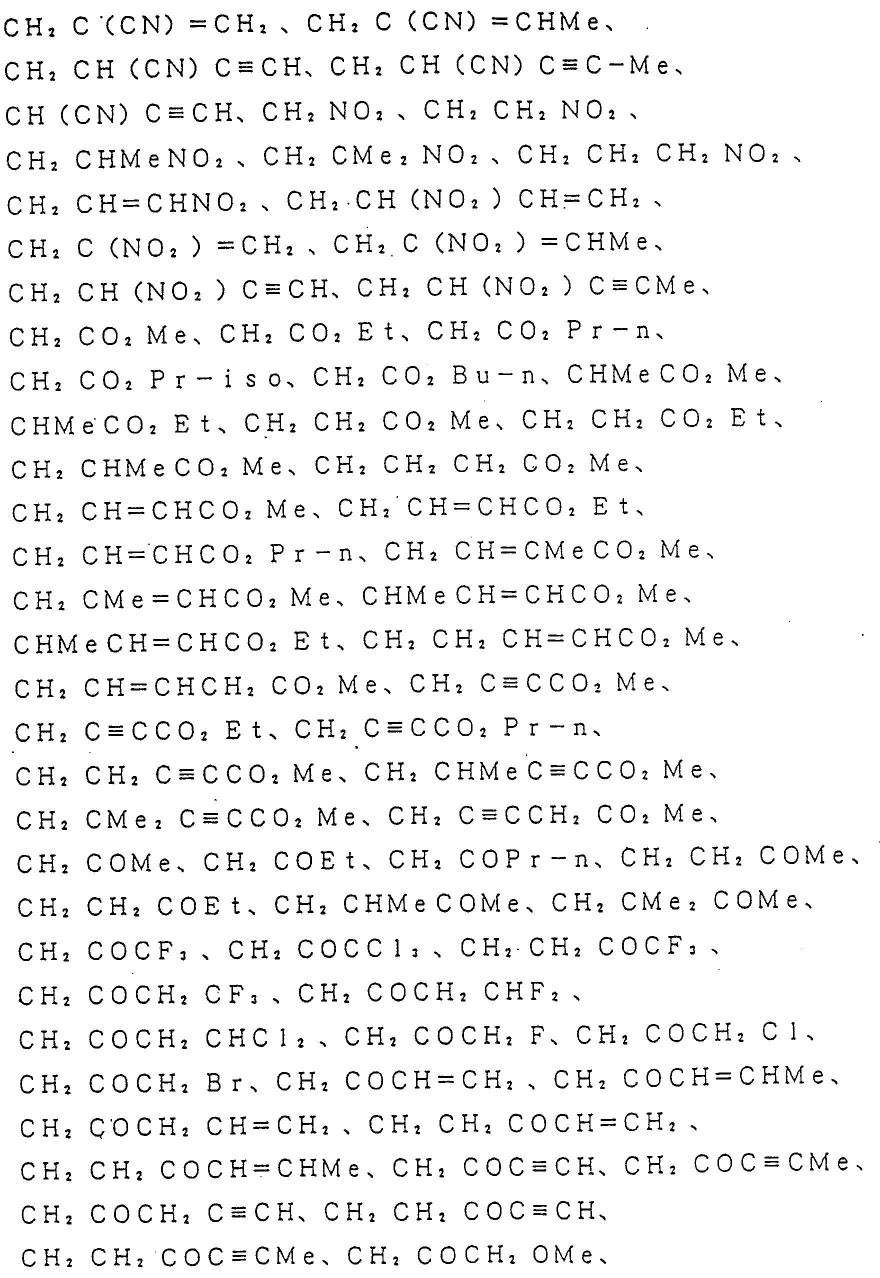 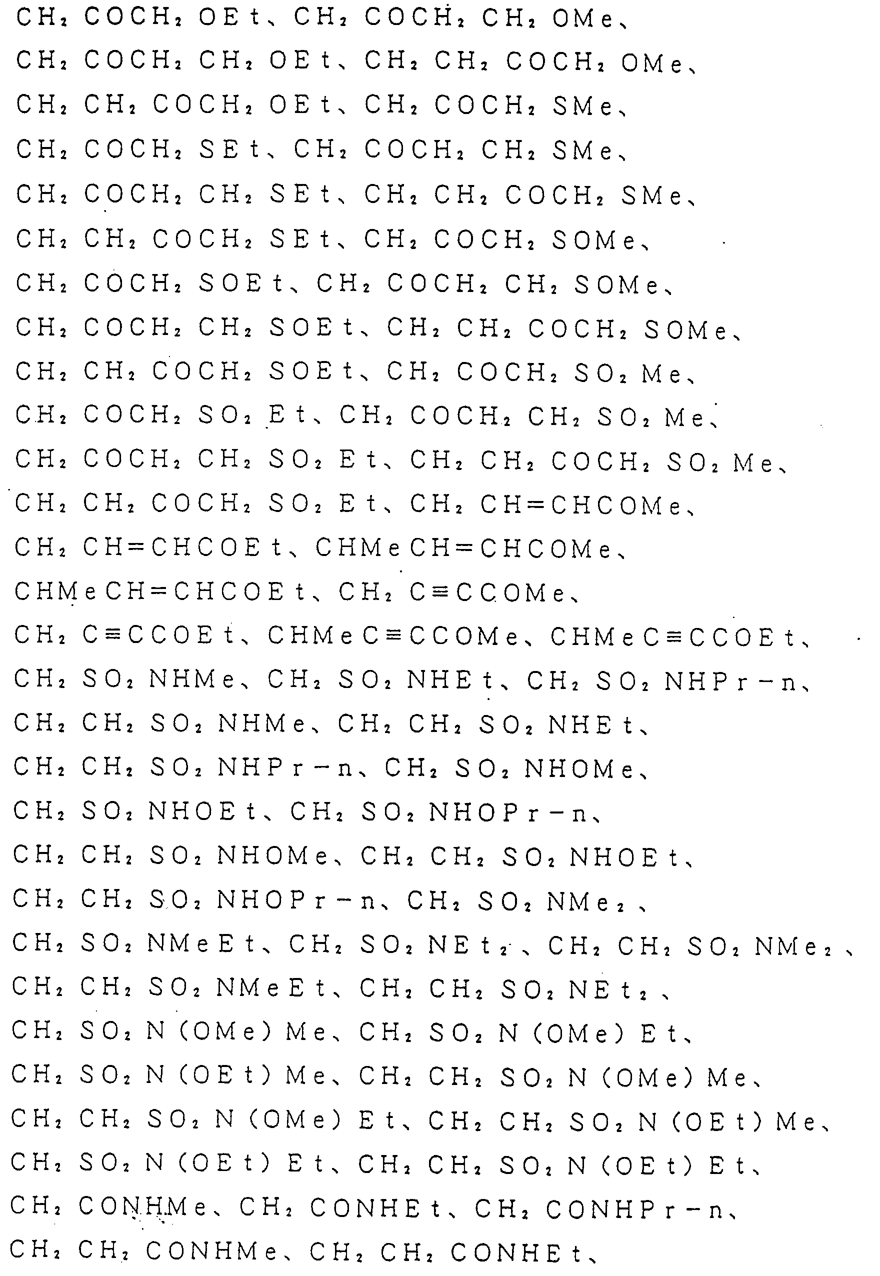 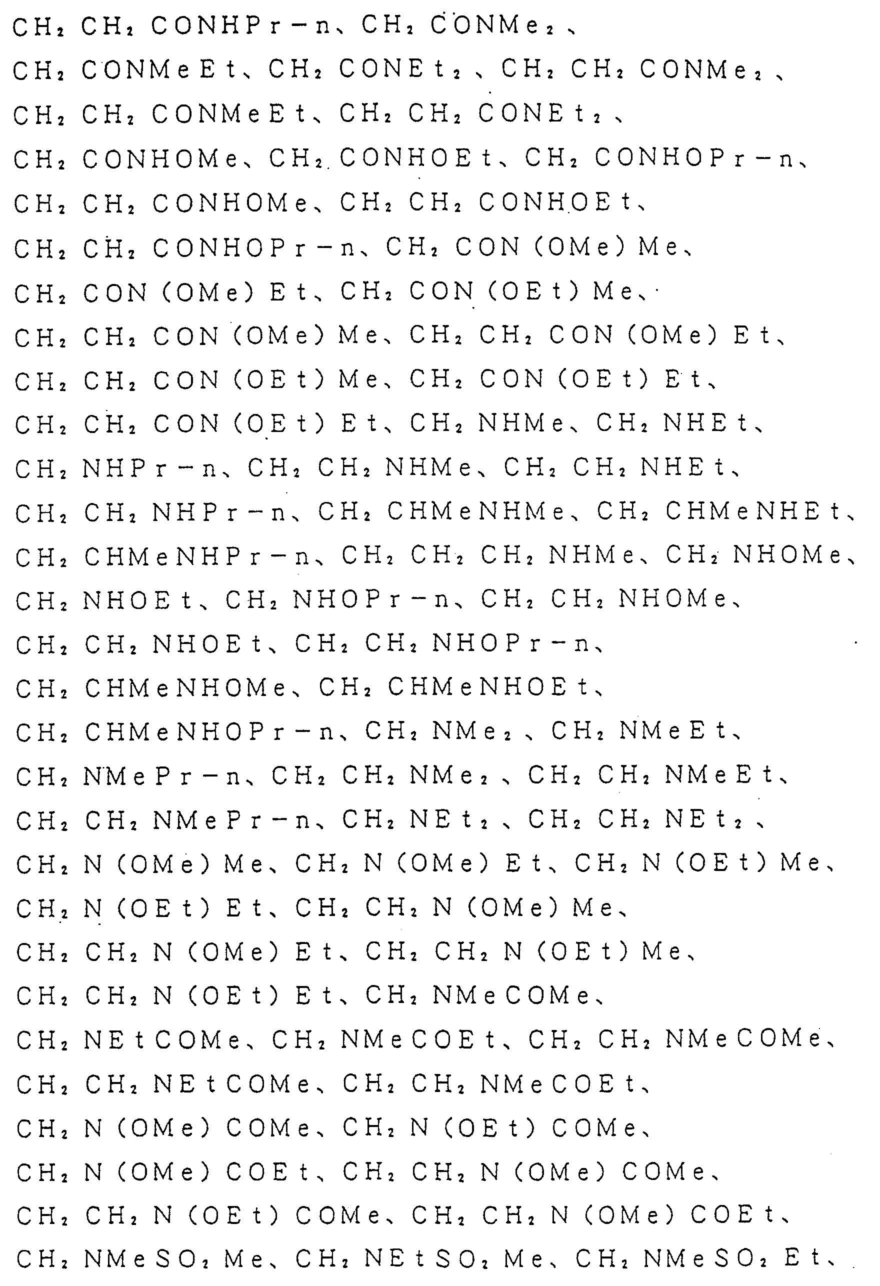 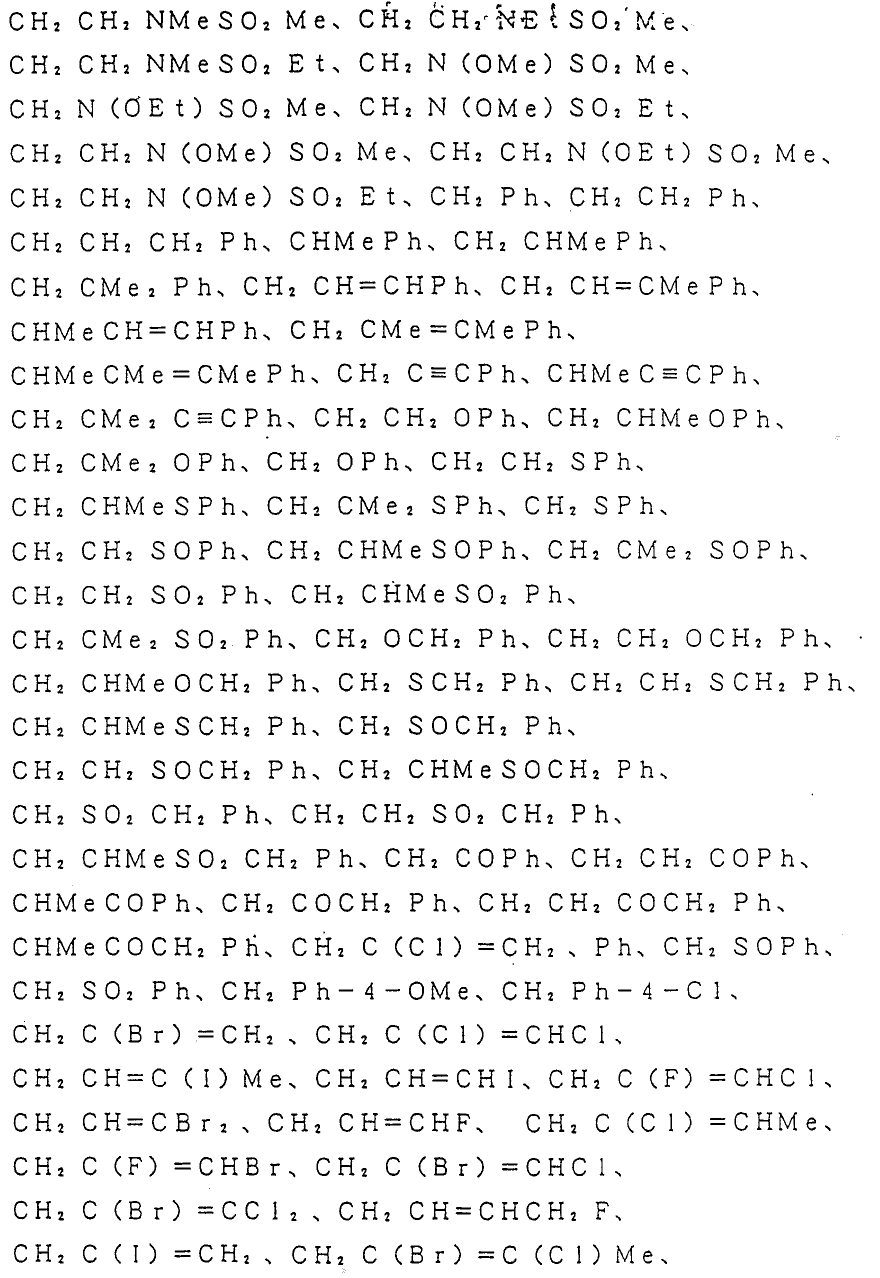 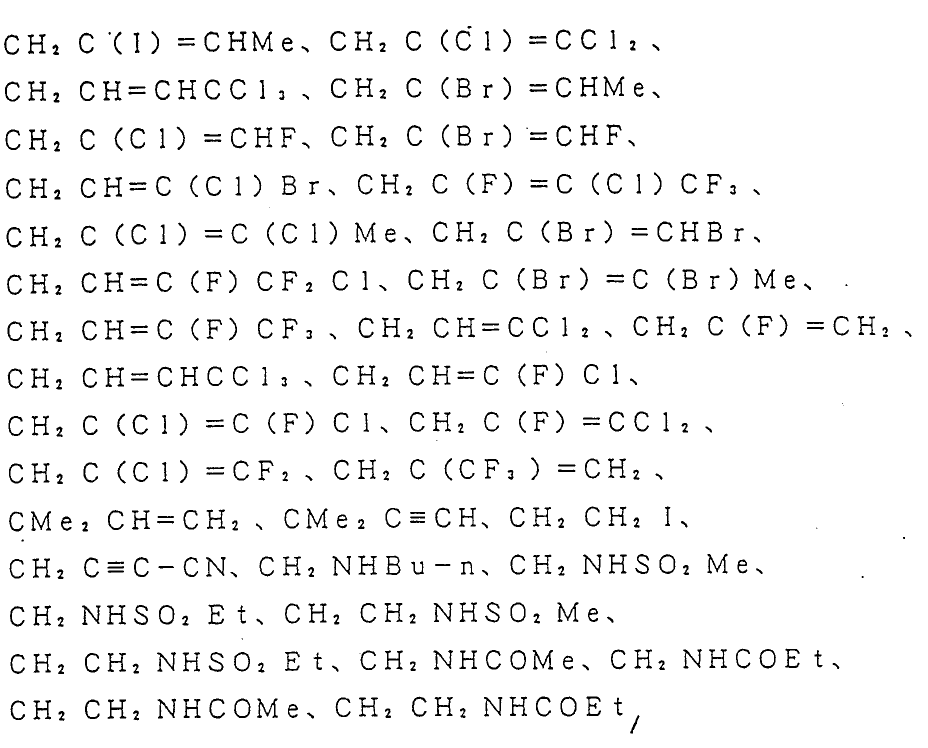
- R^{a2} et R^{a3} représentent : 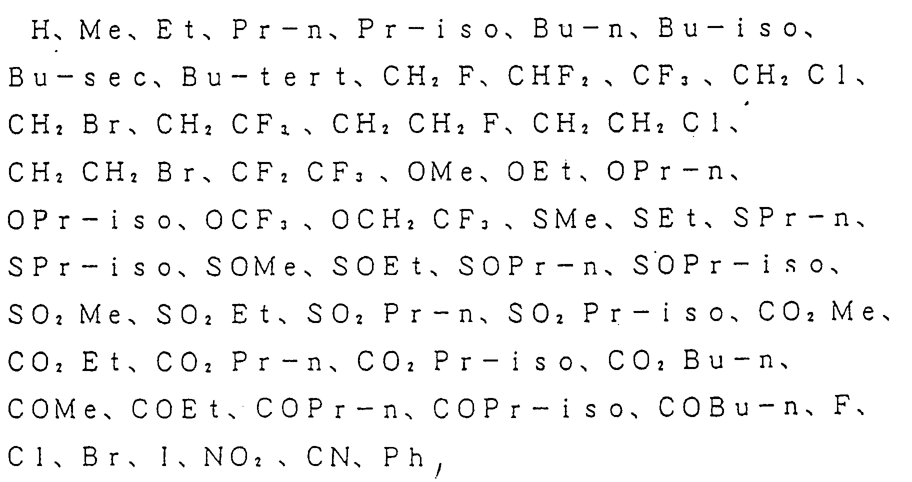
- B et D représentent :

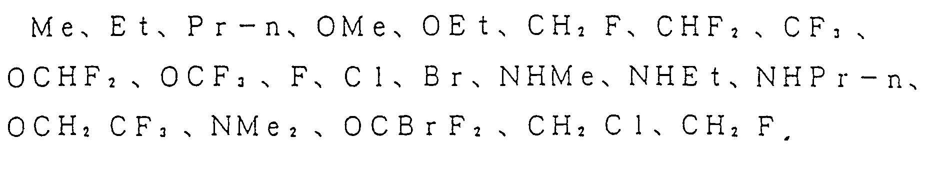

29. Dérivé d'iminosulfonylurée ou son sel, selon la revendication 3, dans lequel :
- R^{a1} représente :

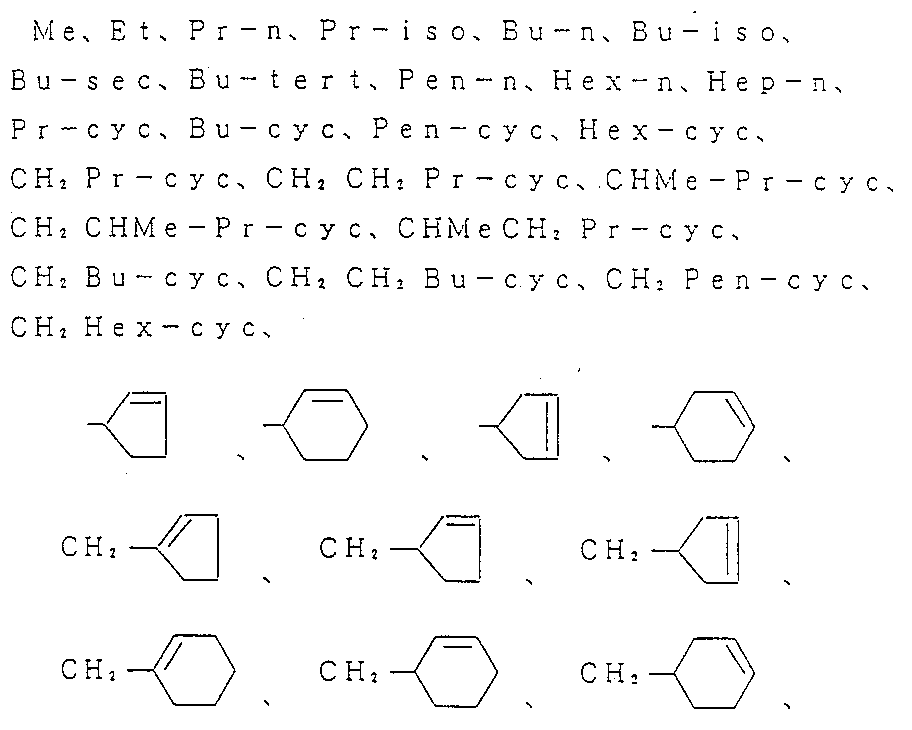

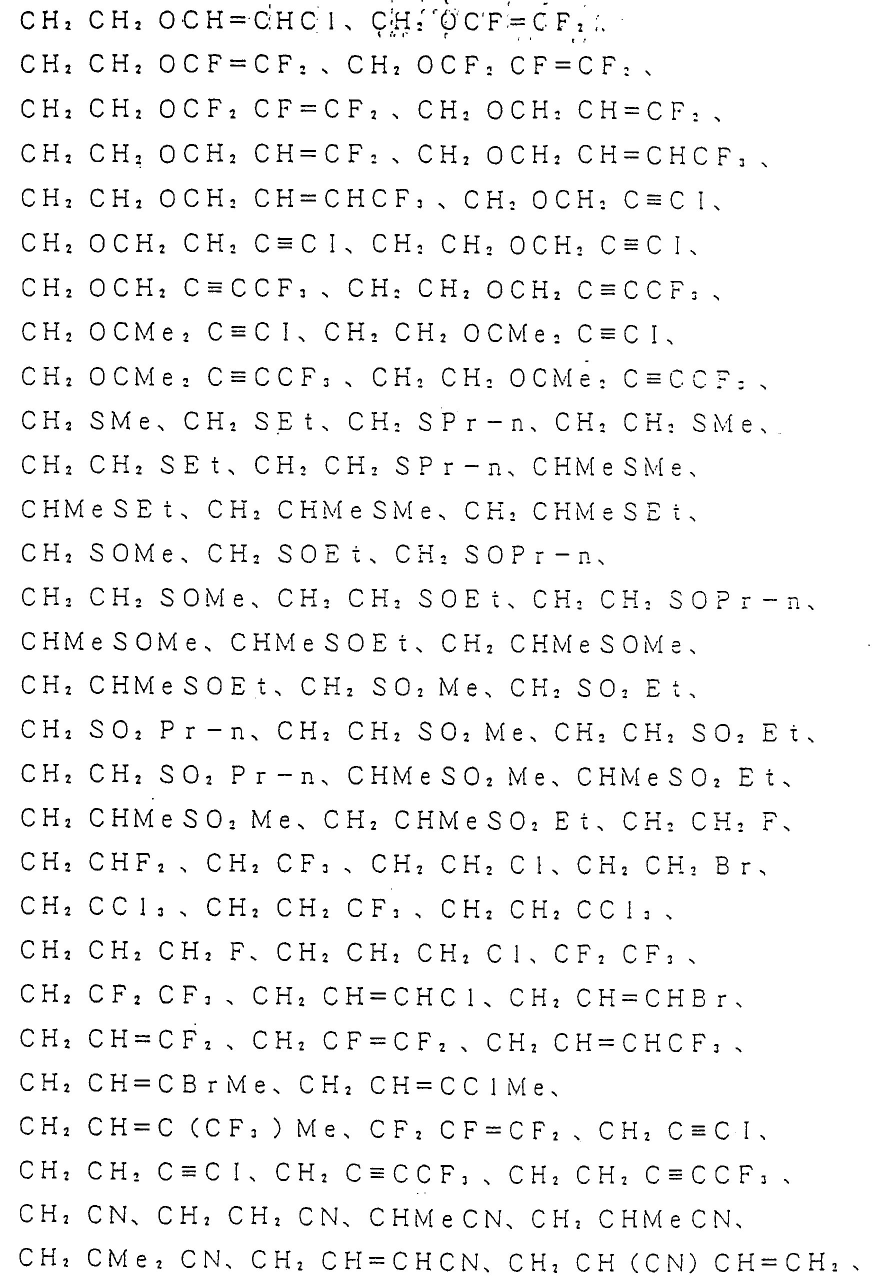

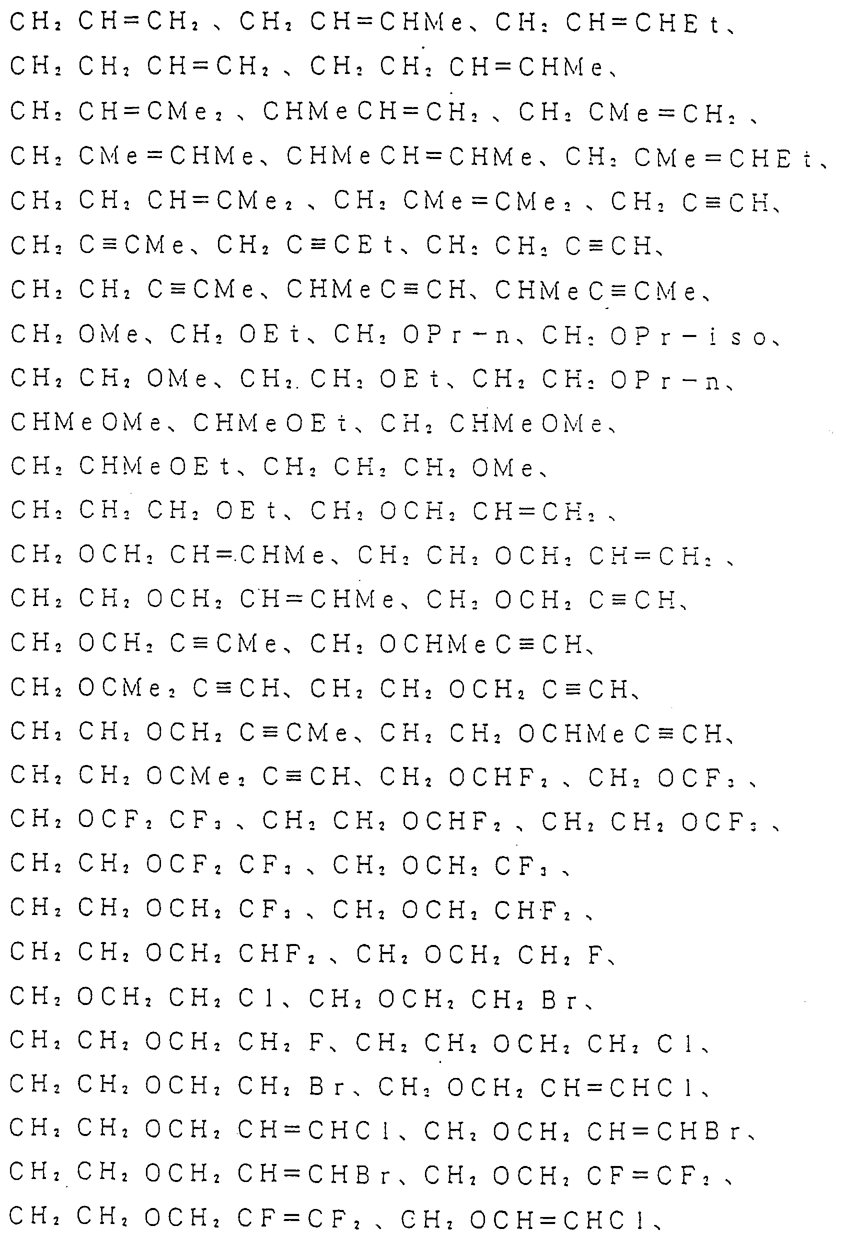

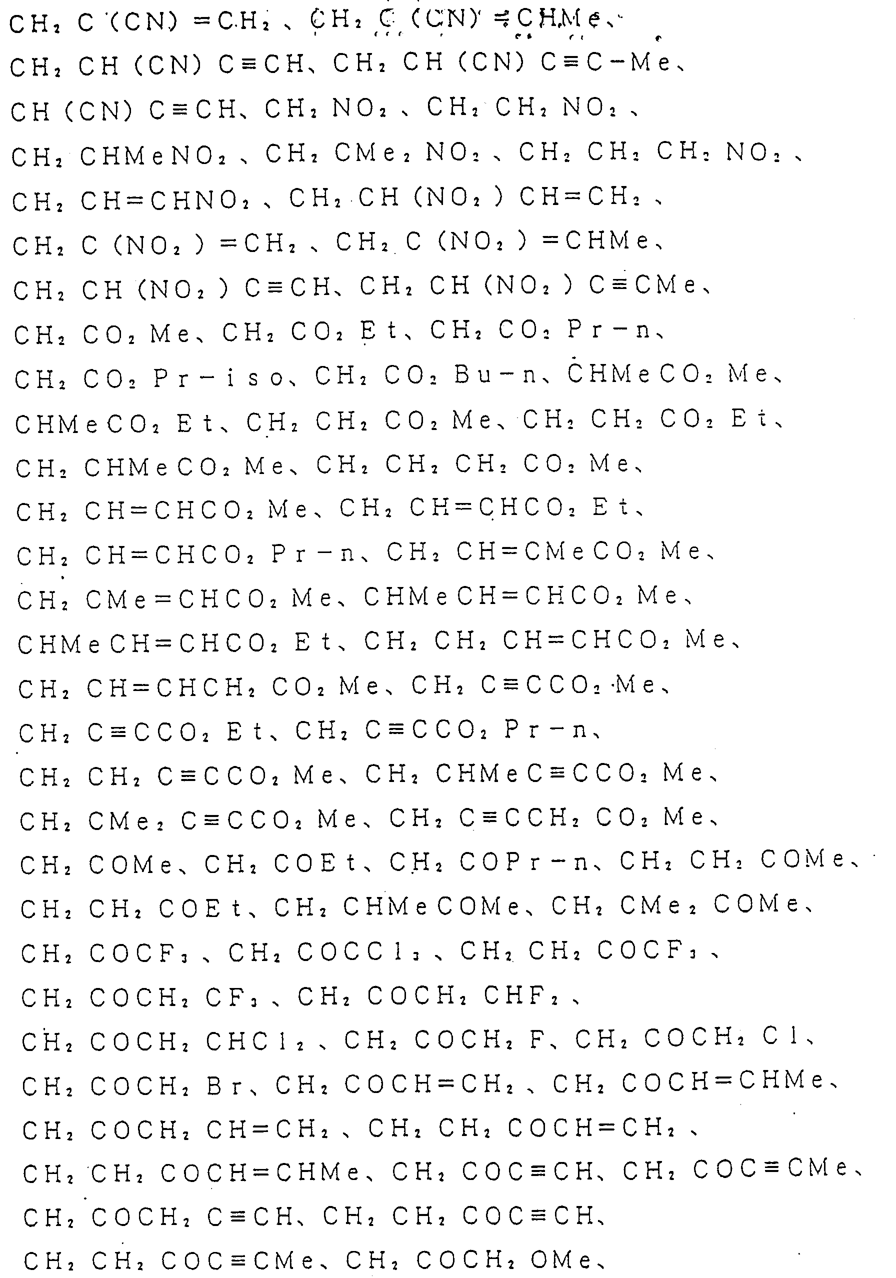

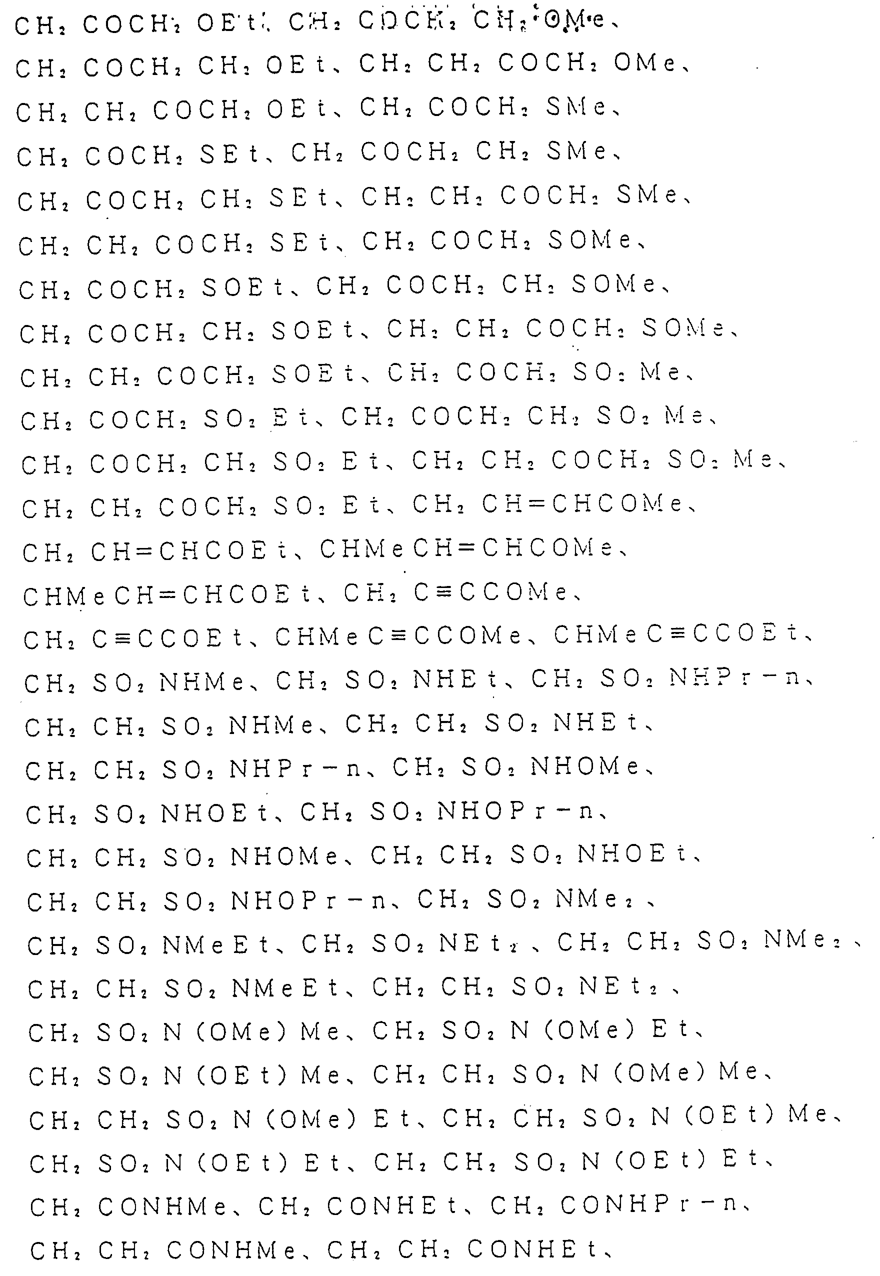

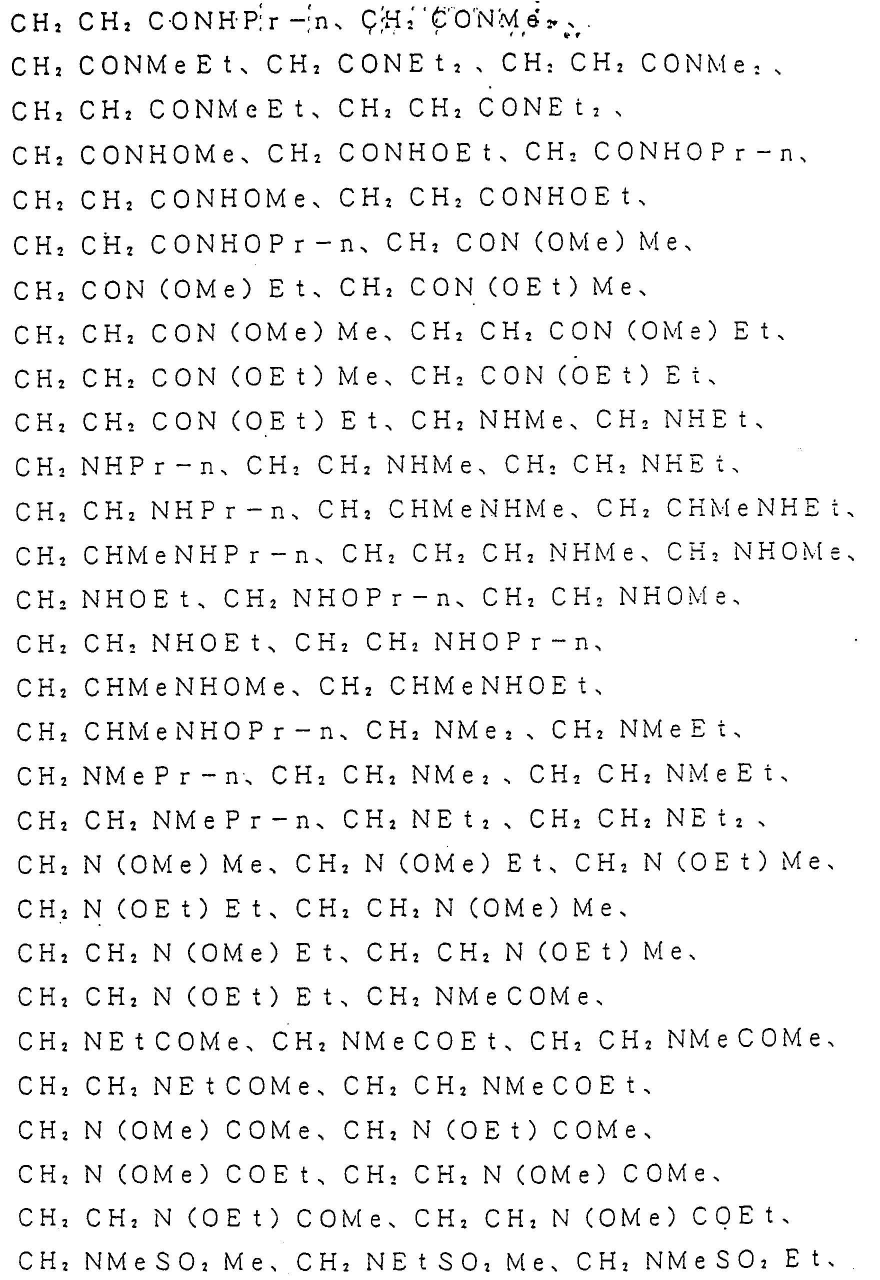

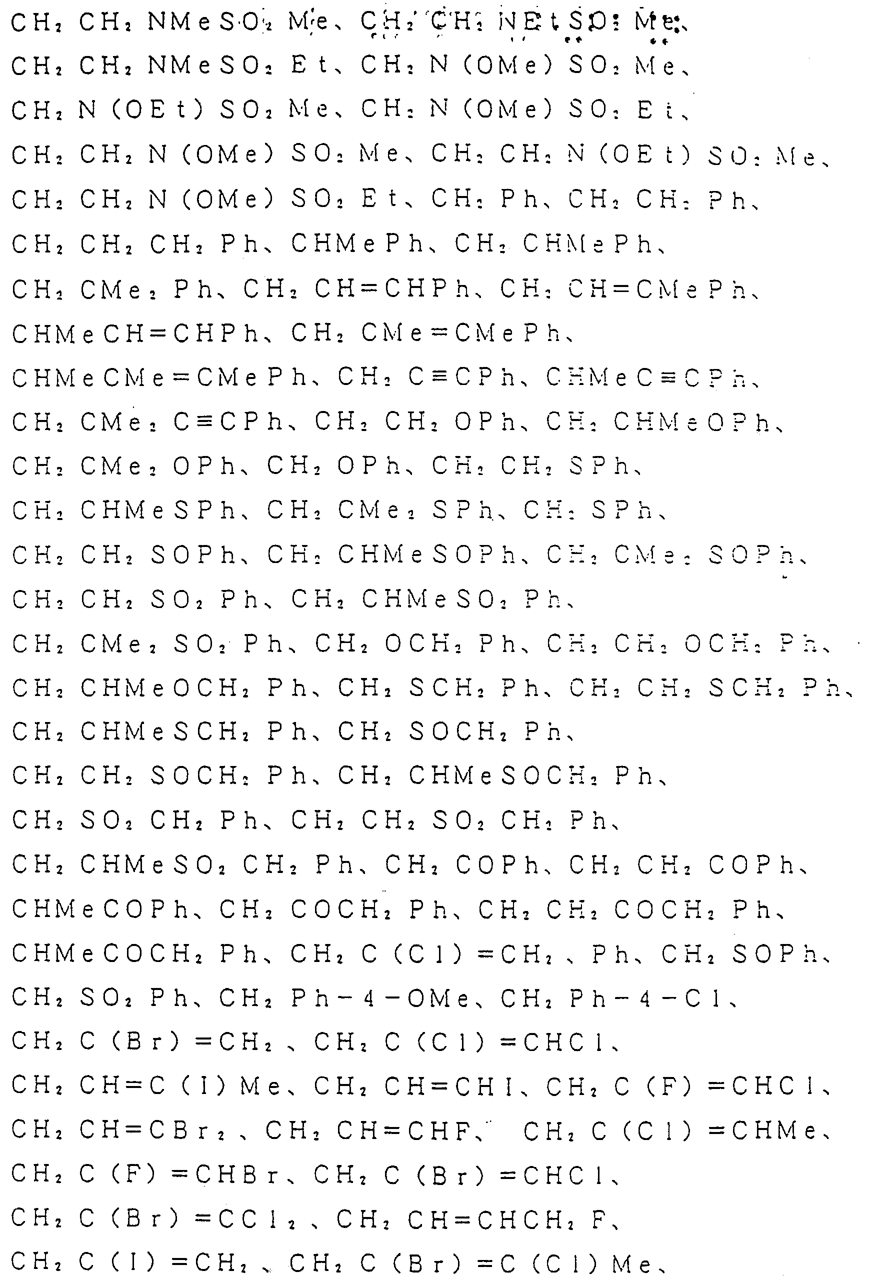

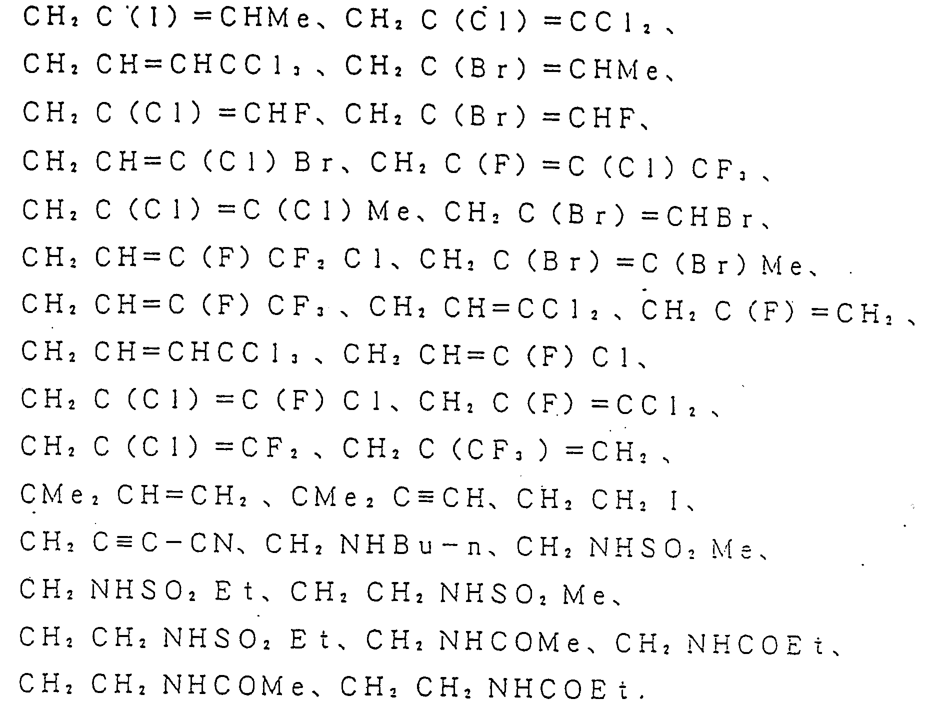

30. Dérivé d'iminosulfonylurée ou son sel, suivant la revendication 3, dans lequel :
- R^{a2} et R^{a3} représentent :

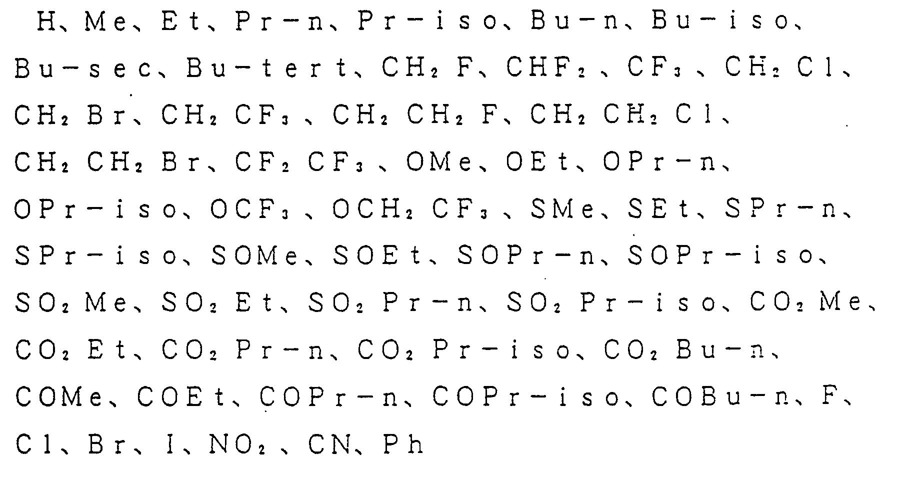

31. Dérivé d'iminosulfonylurée ou son sel, selon la revendication 3, dans lequel B et D représentent :

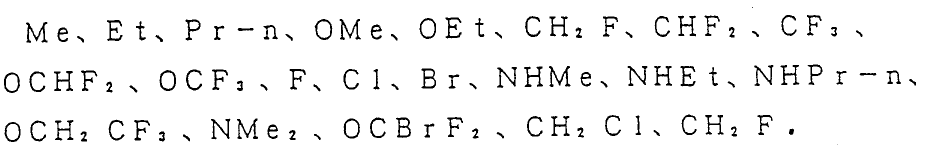

32. Herbicide qui contient un dérivé d'iminosulfonylurée tel que défini à la revendication 28.

33. Herbicide qui contient un dérivé d'iminosulfonylurée tel que défini à la revendication 29.

34. Herbicide qui contient un dérivé d'iminosulfonylurée tel que défini à la revendication 30.

35. Herbicide qui contient un dérivé d'iminosulfonylurée tel que défini à la revendication 31.

36. Procédé herbicide et de lutte contre la croissance à l'encontre des mauvaises herbes, qui comprend l'application d'une quantité efficace du point de vue herbicide d'un dérivé d'iminosulfonylurée tel que défini à la revendication 28.

37. Procédé herbicide et de lutte contre la croissance à l'encontre des mauvaises herbes, qui comprend l'application d'une quantité efficace du point de vue herbicide d'un dérivé d'iminosulfonylurée tel que défini à la revendication 29.

38. Procédé herbicide et de lutte contre la croissance à l'encontre des mauvaises herbes, qui comprend l'application d'une quantité efficace du point de vue herbicide d'un dérivé d'iminosulfonylurée tel que défini à la revendication 30.

39. Procédé herbicide et de lutte contre la croissance à l'encontre des mauvaises herbes, qui comprend l'application d'une quantité efficace du point de vue herbicide d'un dérivé d'iminosulfonylurée tel que défini à la revendication 31.

## Revendications (Revendications pour l'(les) Etat(s) contractant(s) suivant(s): ES)

1. Procédé de préparation d'un dérivé d'iminosulfonylurée de la formule (1) ou de son sel : 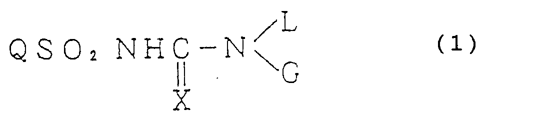 dans laquelle :
- Q représente : 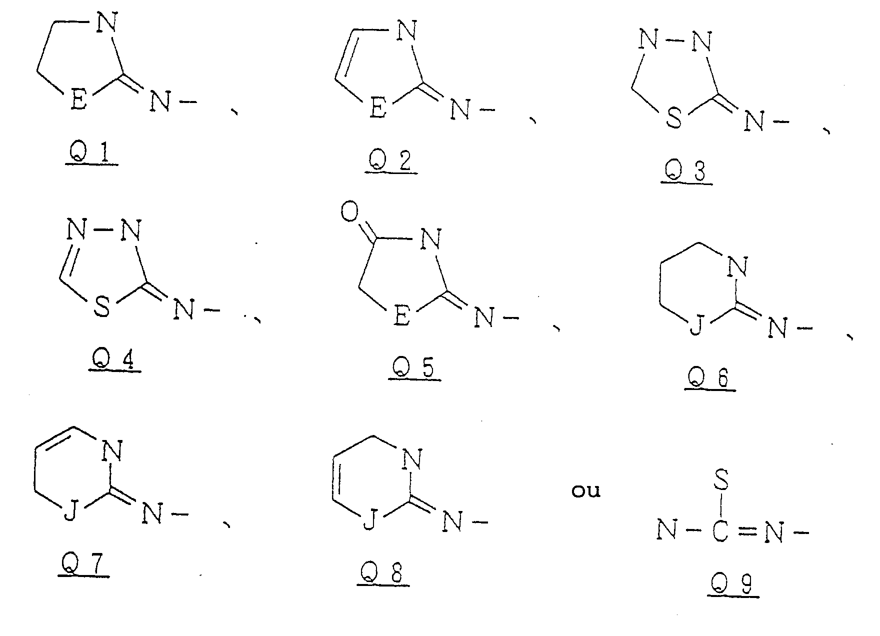 où :
- dans Q1, Q2 et Q5, E représente un atome de soufre, un atome d'oxygène ou un atome d'azote mono-substitué par un substituant autre qu'un atome d'hydrogène ;
- dans Q6, Q7 et Q8, J est un atome de soufre ou un atome d'oxygène ;
- dans Q1 à Q8, un atome d'azote dans le cycle de Q est substitué par un substituant autre qu'un atome d'hydrogène, et un atome de carbone dans le cycle de Q est facultativement substitué ; et
- dans Q9, l'atome de soufre et l'atome d'azote sur l'atome de carbone auquel le groupe imino de Q est lié, sont substitués par des substituants autres que des atomes d'hydrogène ;
- X représente un atome d'oxygène ou un atome de soufre ;
- L est un atome d'hydrogène, un groupe alkyle en C₁₋₆, un groupe alcényle en C₂₋₆ ou un groupe alcynyle en C₂₋₆ ;
- G représente : 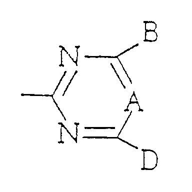
- A est un groupe CH ou un atome d'azote ; et
- B et D, qui sont indépendants l'un de l'autre, représentent chacun un groupe alkyle en C₁₋₄, un groupe alcoxy en C₁₋₄, un groupe mono-, di- ou poly-halogéno alkyle en C₁₋₄, un groupe mono-, di- ou poly-halogéno alcoxy en C₁₋₄, un atome d'halogène, un groupe alkylamino en C₁₋₄ ou un groupe di(alkyl en C₁₋₄)amino,
ledit procédé comprenant :
(a) la réaction d'une amine de formule (2) : 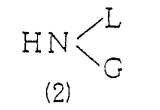 dans laquelle G et L sont tels que définis ci-dessus, avec de l'isocyanate de chlorosulfonyle dans un solvant, puis la réaction du produit avec une imine de formule (3) ou (4) :
Q-H (3)
ou
Q-H · HZ (4)
où :
- Q est tel que défini ci-dessus ; et
- Z est un atome d'halogène,
en présence d'une base, pour obtenir un composé de formule (1) : 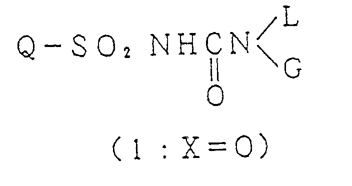 dans laquelle :
- Q, L et G sont tels que définis ci-dessus ; et
- X est un atome d'oxygène ; ou
(b) la réaction d'une imine de formule (3) ou (4) :
Q-H (3)
ou
Q-H · HZ (4)
où Q et Z sont tels que définis ci-dessus,
avec un carbamate de N-chlorosulfonyle de formule (5) :
CℓSO₂ NHCO₂ Y (5)
dans laquelle Y est un groupe alkyle en C₁₋₆ ou un groupe phényle,
dans une quantité de 0,5 à 3,0 moles par mole de l'imine (3) ou (4) en présence d'une base ou d'un solvant, pour obtenir un composé de formule (6) : 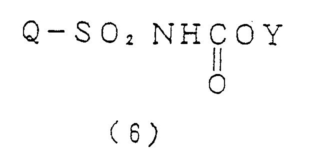 où Q et Y sont tels que définis ci-dessus,
lequel est ensuite chauffé avec un composé de formule (2) : 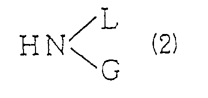 dans laquelle L et G sont tels que définis ci-dessus, dans un solvant, pour obtenir un composé de formule (1) : 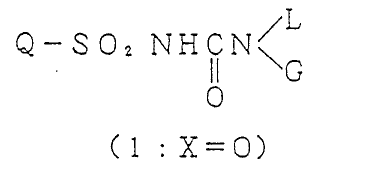 dans laquelle :
- Q, L et G sont tels que définis ci-dessus ; et
- X est un atome d'oxygène ; ou
(c) la réaction d'un dérivé d'iminosulfonamide substitué de formule (7) :
Q-SO₂ NH₂ (7)
dans laquelle Q est tel que défini ci-dessus,
avec un dérivé carbamate de formule (8) : 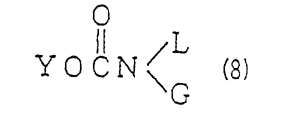 dans laquelle Y, L et G sont tels que définis ci-dessus,
dans un solvant en présence d'une base minérale ou organique, pour obtenir un composé de formule (1) : 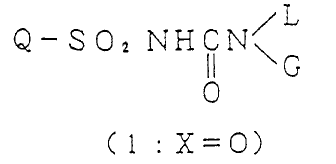 dans laquelle :
- Q, L et G sont tels que définis ci-dessus ; et
- X est un atome d'oxygène ; ou
(d) la réaction d'un dérivé d'iminosulfonamide substitué de formule (7) :
Q-SO₂ NH₂ (7)
dans laquelle Q est tel que défini ci-dessus,
avec un (thio)ester de l'acide chloroformique ou un (thio)ester de l'acide carbonique dans un solvant en présence d'une base, pour obtenir un composé de formule (6) : 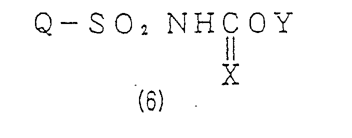 dans laquelle Q, X et Y sont tels que définis ci-dessus,
lequel est ensuite chauffé conjointement avec un composé de formule (2) : 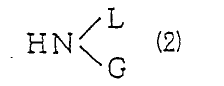 dans laquelle L et G sont tels que définis ci-dessus,
dans un solvant, pour obtenir un composé de formule (1) : 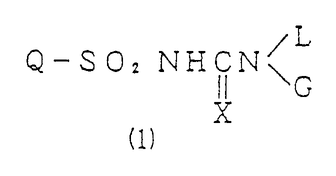 dans laquelle Q, L, G et X sont tels que définis ci-dessus ; ou
(e) la réaction d'un dérivé d'iminosulfonamide substitué de formule (7) :
Q-SO₂ NH₂ (7)
dans laquelle Q est tel que défini ci-dessus,
avec un dérivé d'isothiocyanate de formule (9) :
S=C=N-G (9)
dans laquelle G est tel que défini ci-dessus,
dans un solvant en présence d'une base minérale ou organique, pour obtenir un composé de formule (1) : 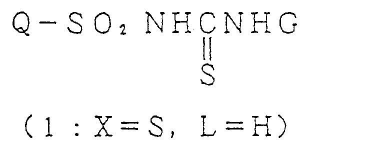 dans laquelle :
- Q et G sont tels que définis ci-dessus ; et
- X représente soufre et L représente hydrogène.

2. Procédé de préparation d'un dérivé d'iminosulfonylurée de la formule (1) ou de son sel, selon la revendication 1 : 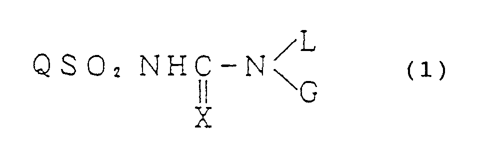 dans laquelle :
- Q représente : 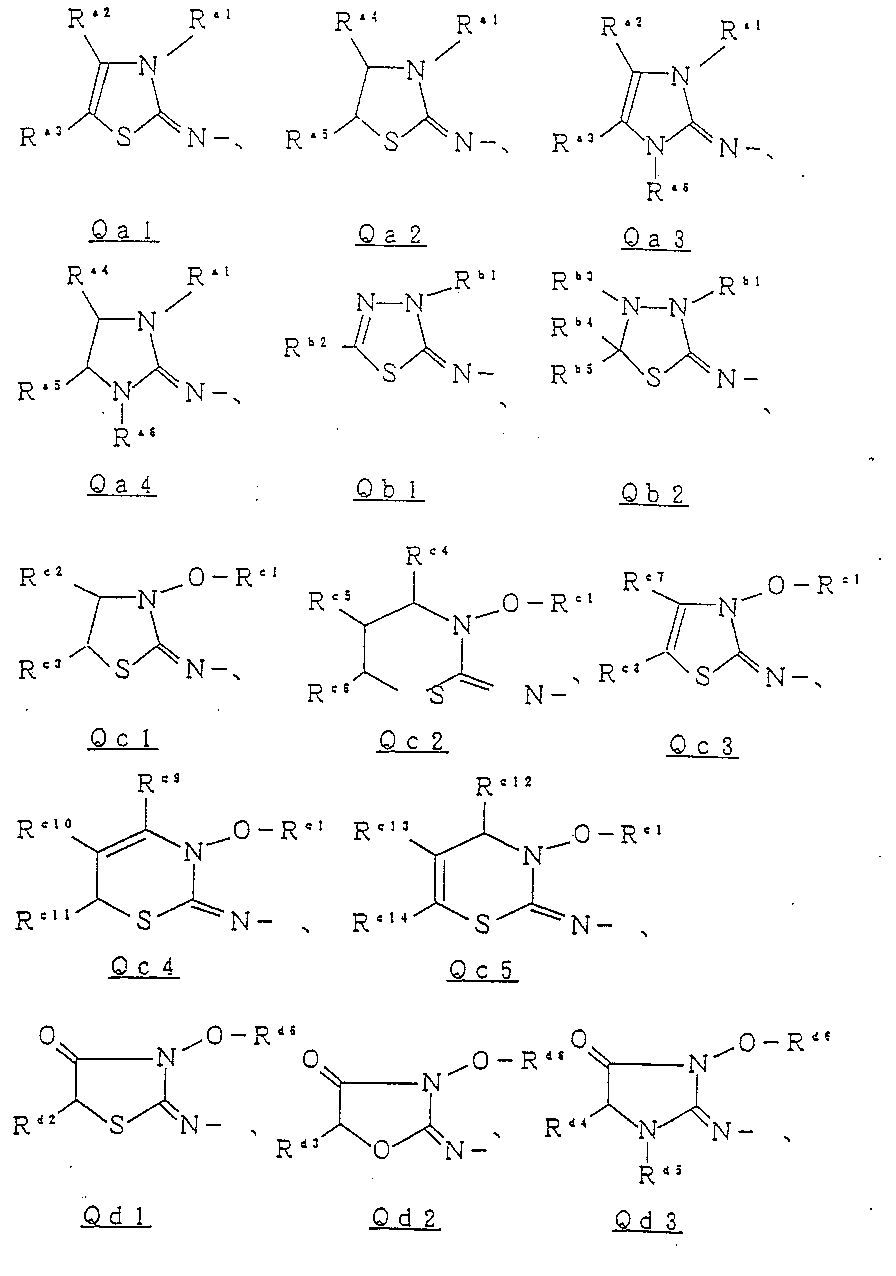 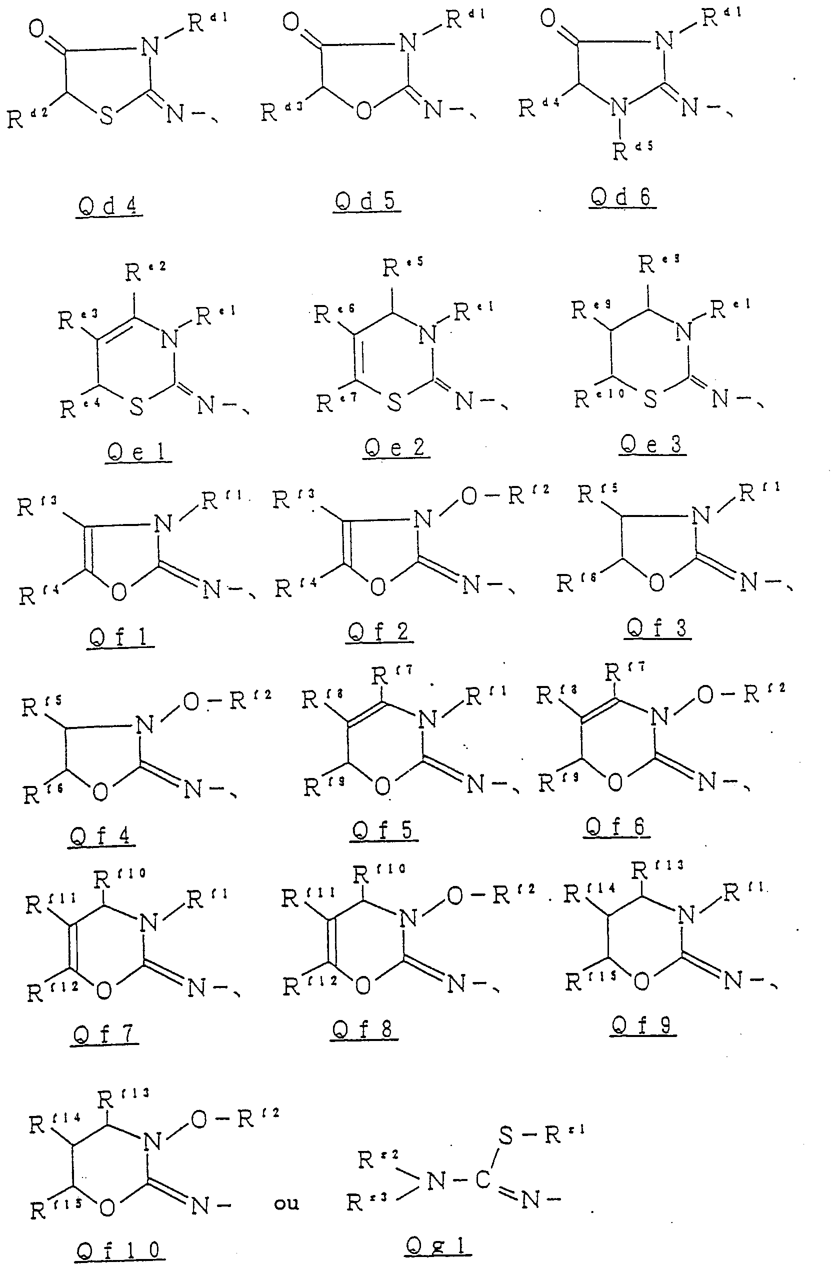
- R^{a1} est un groupe alkyle en C₁₋₈, un groupe cycloalkyle en C₃₋₇, un groupe alkyle en C₁₋₆ substitué par un groupe cycloalkyle en C₃₋₇, un groupe cycloalcényle en C₃₋₇, un groupe alkyle en C₁₋₆ substitué par un groupe cycloalcényle en C₃₋₇, un groupe alcényle en C₂₋₈, un groupe alcynyle en C₂₋₈, un groupe alkyle en C₁₋₆ substitué par un groupe alcoxy en C₁₋₆, un groupe alkyle en C₁₋₆ substitué par un groupe alcényloxy en C₂₋₆, un groupe alkyle en C₁₋₆ substitué par un groupe alcynyloxy en C₂₋₆, un groupe alkyle en C₁₋₆ substitué par un groupe mono-, di- ou poly-halogénoalcoxy en C₁₋₆, un groupe alkyle en C₁₋₆ substitué par un groupe mono-, di- ou poly-halogénoalcényloxy en C₂₋₆, un groupe alkyle en C₁₋₆ substitué par un groupe mono-, di- ou poly-halogénoalcynyloxy en C₂₋₆, un groupe alkyle en C₁₋₆ substitué par un groupe alkylthio en C₁₋₆, un groupe alkyle en C₁₋₆ substitué par un groupe alkylsulfinyle en C₁₋₆, un groupe alkyle en C₁₋₆ substitué par un groupe alkylsulfonyle en C₁₋₆, un groupe mono-, di- ou poly-halogénoalkyle en C₁₋₈, un groupe mono-, di-, ou poly-halogénoalcényle en C₂₋₈, un groupe mono-, di-, ou poly-halogénoalcynyle en C₂₋₈, un groupe alkyle en C₁₋₆ substitué par un groupe cyano, un groupe alcényle en C₂₋₆ substitué par un groupe cyano, un groupe alcynyle en C₂₋₆ substitué par un groupe cyano, un groupe alkyle en C₁₋₆ substitué par un groupe nitro, un groupe alcényle en C₂₋₆ substitué par un groupe nitro, un groupe alcynyle en C₂₋₆ substitué par un groupe nitro, un groupe alkyle en C₁₋₆ substitué par un groupe alcoxycarbonyle en C₂₋₇, un groupe alcényle en C₂₋₆ substitué par un groupe alcoxycarbonyle en C₂₋₇, un groupe alcynyle en C₂₋₆ substitué par un groupe alcoxycarbonyle en C₂₋₇, un groupe alkyle en C₁₋₆ substitué par un groupe alkylcarbonyle en C₂₋₇, un groupe alkyle en C₁₋₆ substitué par un groupe mono-, di- ou poly-halogénoalkylcarbonyle en C₂₋₇, un groupe alkyle en C₁₋₆ substitué par un groupe alcénylcarbonyle en C₃₋₇, un groupe alkyle en C₁₋₆ substitué par un groupe alcynylcarbonyle en C₃₋₇, un groupe alkyle en C₁₋₆ substitué par un groupe alkylcarbonyle en C₂₋₅ substitué par un groupe alcoxy en C₁₋₄, un groupe alkyle en C₁₋₆ substitué par un groupe alkylcarbonyle en C₂₋₅ substitué par un groupe alkylthio en C₁₋₄, un groupe alkyle en C₁₋₆ substitué par un groupe alkylcarbonyle en C₂₋₅ substitué par un groupe alkylsulfinyle en C₁₋₄, un groupe alkyle en C₁₋₆ substitué par un groupe alkylcarbonyle en C₂₋₅ substitué par un groupe alkylsulfonyle en C₁₋₄, un groupe alcényle en C₂₋₆ substitué par un groupe alkylcarbonyle en C₂₋₇, un groupe alcynyle en C₂₋₆ substitué par un groupe alkylcarbonyle en C₂₋₇, un groupe alkyle en C₁₋₆ substitué par un groupe alkylsulfamoyle en C₁₋₆, un groupe alkyle en C₁₋₆ substitué par un groupe alcoxysulfamoyle en C₁₋₆, un groupe alkyle en C₁₋₆ substitué par un groupe di(alkyl en C₁₋₃)sulfamoyle, un groupe alkyle en C₁₋₆ substitué par un groupe N-(alkyl en C₁₋₃)-N-(alcoxy en C₁₋₃)sulfamoyle, un groupe alkyle en C₁₋₆ substitué par un groupe alkylcarbamoyle en C₂₋₇, un groupe alkyle en C₁₋₆ substitué par un groupe di(alkyl en C₁₋₃)carbamoyle, un groupe alkyle en C₁₋₆ substitué par un groupe alcoxycarbamoyle en C₂₋₇, un groupe alkyle en C₁₋₆ substitué par un groupe N-(alkyl en C₁₋₃)-N-(alcoxy en C₁₋₃)carbamoyle, un groupe alkyle en C₁₋₆ substitué par un groupe alkylamino en C₁₋₆, un groupe alkyle en C₁₋₆ substitué par un groupe alcoxyamino en C₁₋₆, un groupe alkyle en C₁₋₆ substitué par un groupe di(alkyl en C₁₋₃)amino, un groupe alkyle en C₁₋₆ substitué par un groupe N-(alkyl en C₁₋₃)-N-(alcoxy en C₁₋₃)amino, un groupe alkyle en C₁₋₆ substitué par un groupe N-(alkylcarbonyl en C₂₋₇)-N-(alkyl en C₁₋₆)amino, un groupe alkyle en C₁₋₆ substitué par un groupe N-(alkylcarbonyl en C₂₋₇)-N-(alcoxy en C₁₋₆)-amino, un groupe alkyle en C₁₋₆ substitué par un groupe N-(alkylsulfonyl en C₁₋₆)-N-(alkyl en C₁₋₆)amino, un groupe alkyle en C₁₋₆ substitué par un groupe N-(alkylsulfonyl en C₁₋₆)-N-(alcoxy en C₁₋₆)amino, un groupe phényle (à la condition qu'un tel groupe phényle puisse être substitué par un ou plusieurs substituants choisis dans le groupe constitué par un atome d'halogène, un groupe trifluorométhyle, un groupe nitro, un groupe alkyle en C₁₋₆, un groupe alcoxy en C₁₋₆ et un groupe alcoxycarbonyle en C₂₋₇), un groupe alkyle en C₁₋₆ substitué par un groupe phényle (à la condition qu'un tel groupe phényle puisse être substitué par un ou plusieurs substituants choisis dans le groupe constitué par un atome d'halogène, un groupe trifluorométhyle, un groupe nitro, un groupe alkyle en C₁₋₆, un groupe alcoxy en C₁₋₆ et un groupe alcoxycarbonyle en C₂₋₇), un groupe alcényle en C₂₋₇ substitué par un groupe phényle (à la condition qu'un tel groupe phényle puisse être substitué par un ou plusieurs substituants choisis dans le groupe constitué par un atome d'halogène, un groupe trifluorométhyle, un groupe nitro, un groupe alkyle en C₁₋₆, un groupe alcoxy en C₁₋₆ et un groupe alcoxycarbonyle en C₂₋₇), un groupe alcynyle en C₂₋₆ substitué par un groupe phényle (à la condition qu'un tel groupe phényle puisse être substitué par un ou plusieurs substituants choisis dans le groupe constitué par un atome d'halogène, un groupe trifluorométhyle, un groupe nitro, un groupe alkyle en C₁₋₆, un groupe alcoxy en C₁₋₆ et un groupe alcoxycarbonyle en C₂₋₇), un groupe alkyle en C₁₋₆ substitué par un groupe phénoxy (à la condition qu'un tel groupe phénoxy puisse être substitué par un ou plusieurs substituants choisis dans le groupe constitué par un atome d'halogène, un groupe trifluorométhyle, un groupe nitro, un groupe alkyle en C₁₋₆, un groupe alcoxy en C₁₋₆ et un groupe alcoxycarbonyle en C₂₋₇), un groupe alkyle en C₁₋₆ substitué par un groupe phénylthio (à la condition qu'un tel groupe phénylthio puisse être substitué par un ou plusieurs substituants choisis dans le groupe constitué par un atome d'halogène, un groupe trifluorométhyle, un groupe nitro, un groupe alkyle en C₁₋₆, un groupe alcoxy en C₁₋₆ et un groupe alcoxycarbonyle en C₂₋₇), un groupe alkyle en C₁₋₆ substitué par un groupe phénylsulfinyle (à la condition qu'un tel groupe phénylsulfinyle puisse être substitué par un ou plusieurs substituants choisis dans le groupe constitué par un atome d'halogène, un groupe trifluorométhyle, un groupe nitro, un groupe alkyle en C₁₋₆, un groupe alcoxy en C₁₋₆ et un groupe alcoxycarbonyle en C₂₋₇), un groupe alkyle en C₁₋₆ substitué par un groupe phénylsulfonyle (à la condition qu'un tel groupe phénylsulfonyle puisse être substitué par un ou plusieurs substituants choisis dans le groupe constitué par un atome d'halogène, un groupe trifluorométhyle, un groupe nitro, un groupe alkyle en C₁₋₆, un groupe alcoxy en C₁₋₆ et un groupe alcoxycarbonyle en C₂₋₇), un groupe alkyle en C₁₋₆ substitué par un groupe benzyloxy (à la condition que le groupe phényle d'un tel groupe benzyloxy puisse être substitué par un ou plusieurs substituants choisis dans le groupe constitué par un atome d'halogène, un groupe trifluorométhyle, un groupe nitro, un groupe alkyle en C₁₋₆, un groupe alcoxy en C₁₋₆ et un groupe alcoxycarbonyle en C₂₋₇), un groupe alkyle en C₁₋₆ substitué par un groupe benzylthio (à la condition que le groupe phényle d'un tel groupe benzylthio puisse être substitué par un ou plusieurs substituants choisis dans le groupe constitué par un atome d'halogène, un groupe trifluorométhyle, un groupe nitro, un groupe alkyle en C₁₋₆, un groupe alcoxy en C₁₋₆ et un groupe alcoxycarbonyle en C₂₋₇), un groupe alkyle en C₁₋₆ substitué par un groupe benzylsulfinyle (à la condition que le groupe phényle d'un tel groupe benzylsulfinyle puisse être substitué par un ou plusieurs substituants choisis dans le groupe constitué par un atome d'halogène, un groupe trifluoromethyle, un groupe nitro, un groupe alkyle en C₁₋₆, un groupe alcoxy en C₁₋₆ et un groupe alcoxycarbonyle en C₂₋₇), un groupe alkyle en C₁₋₆ substitué par un groupe benzylsulfonyle (à la condition que le groupe phényle d'un tel groupe benzylsulfonyle puisse être substitué par un ou plusieurs substituants choisis dans le groupe constitué par un atome d'halogène, un groupe trifluorométhyle, un groupe nitro, un groupe alkyle en C₁₋₆, un groupe alcoxy en C₁₋₆ et un groupe alcoxycarbonyle en C₂₋₇), un groupe alkyle en C₁₋₆ substitué par un groupe phénylcarbonyle (à la condition qu'un tel groupe phénylcarbonyle puisse être substitué par un ou plusieurs substituants choisis dans le groupe constitué par un atome d'halogène, un groupe trifluorométhyle, un groupe nitro, un groupe alkyle en C₁₋₆, un groupe alcoxy en C₁₋₆ et un groupe alcoxycarbonyle en C₂₋₇), un groupe alkyle en C₁₋₆ substitué par un groupe benzylcarbonyle (à la condition que le groupe phényle d'un tel groupe benzylcarbonyle puisse être substitué par un ou plusieurs substituants choisis dans le groupe constitué par un atome d'halogène, un groupe trifluorométhyle, un groupe nitro, un groupe alkyle en C₁₋₆, un groupe alcoxy en C₁₋₆ et un groupe alcoxycarbonyle en C₂₋₇), un groupe alkyle en C₁₋₆ substitué par un groupe amino substitué par un groupe alkylsulfonyle en C₁₋₄, ou un groupe alkyle en C₁₋₆ substitué par un groupe amino substitué par un groupe alkylcarbonyle en C₂₋₄ ;
- R^{a2} et R^{a3}, qui sont indépendants l'un de l'autre, représentent chacun un atome d'hydrogène, un groupe alkyle en C₁₋₆, un groupe mono-, di- ou poly-halogénoalkyle en C₁₋₆, un groupe alcoxy en C₁₋₆, un groupe mono-, di- ou poly-halogénoalcoxy en C₁₋₆, un groupe alkylthio en C₁₋₆, un groupe alkylsulfinyle en C₁₋₆, un groupe alkylsulfonyle en C₁₋₆, un groupe alcoxycarbonyle en C₂₋₇, un groupe alkylcarbonyle en C₂₋₇, un atome d'halogène, un groupe nitro, un groupe cyano, ou un groupe phényle (à la condition qu'un tel groupe phényle puisse être substitué par un ou plusieurs substituants choisis dans le groupe constitué par un atome d'halogène, un groupe trifluorométhyle, un groupe nitro, un groupe alkyle en C₁₋₆, un groupe alcoxy en C₁₋₆ et un groupe alcoxycarbonyle en C₂₋₇) ;
- R^{a4} et R^{a5}, qui sont indépendants l'un de l'autre, représentent chacun un atome d'hydrogène, un groupe alkyle en C₁₋₈, un groupe alcényle en C₂₋₈, un groupe alcynyle en C₂₋₈, ou un groupe phényle (à la condition qu'un tel groupe phényle puisse être substitué par un ou plusieurs substituants choisis dans le groupe constitué par un atome d'halogène, un groupe trifluorométhyle, un groupe nitro, un groupe alkyle en C₁₋₆, un groupe alcoxy en C₁₋₆ et un groupe alcoxycarbonyle en C₂₋₇) ;
- R^{a6} est un groupe alkyle en C₁₋₈, un groupe alcényle en C₂₋₈, un groupe alcynyle en C₂₋₈ ou un groupe phényle (à la condition qu'un tel groupe phényle puisse être substitué par un ou plusieurs substituants choisis dans le groupe constitué par un atome d'halogène, un groupe trifluorométhyle, un groupe nitro, un groupe alkyle en C₁₋₆, un groupe alcoxy en C₁₋₆ et un groupe alcoxycarbonyle en C₂₋₇) ;
- R^{b1} est un groupe alkyle en C₁₋₈, un groupe cycloalkyle en C₃₋₇, un groupe alkyle en C₁₋₆ substitué par un groupe cycloalkyle en C₃₋₇, un groupe cycloalcényle en C₃₋₇, un groupe alkyle en C₁₋₆ substitué par un groupe cycloalcényle en C₃₋₇, un groupe alcényle en C₂₋₈, un groupe alcynyle en C₂₋₈, un groupe alkyle en C₁₋₆ substitué par un groupe alcoxy en C₁₋₆, un groupe alkyle en C₁₋₆ substitué par un groupe alcényloxy en C₂₋₆, un groupe alkyle en C₁₋₆ substitué par un groupe alcynyloxy en C₂₋₆, un groupe alkyle en C₁₋₆ substitué par un groupe mono-, di- ou poly-halogénoalcoxy en C₁₋₆, un groupe alkyle en C₁₋₆ substitué par un groupe mono-, di-, ou poly-halogénoalcényloxy en C₂₋₆, un groupe alkyle en C₁₋₆ substitué par un groupe mono-, di-, ou poly-halogénoalcynyloxy en C₂₋₆, un groupe alkyle en C₁₋₆ substitué par un groupe alkylthio en C₁₋₆, un groupe alkyle en C₁₋₆ substitué par un groupe alkylsufinyle en C₁₋₆, un groupe alkyle en C₁₋₆ substitué par un groupe alkylsulfonyle en C₁₋₆, un groupe mono-, di- ou poly-halogénoalkyle en C₁₋₈, un groupe mono-, di-, ou poly-halogénoalcényle en C₂₋₈, un groupe mono-, di- ou poly-halogénoalcynyle en C₂₋₈, un groupe alkyle en C₁₋₆ substitué par un groupe cyano, un groupe alcényle en C₂₋₆ substitué par un groupe cyano, un groupe alcynyle en C₂₋₆ substitué par un groupe cyano, un groupe alkyle en C₁₋₆ substitué par un groupe nitro, un groupe alcényle en C₂₋₆ substitué par un groupe nitro, un groupe alcynyle en C₂₋₆ substitué par un groupe nitro, un groupe alkyle en C₁₋₆ substitué par un groupe alcoxycarbonyle en C₂₋₇, un groupe alcényle en C₂₋₆ substitué par un groupe alcoxycarbonyle en C₂₋₇, un groupe alcynyle en C₂₋₆ substitué par un groupe alcoxycarbonyle en C₂₋₇, un groupe alkyle en C₁₋₆ substitué par un groupe alkylcarbonyle en C₂₋₇, un groupe alkyle en C₁₋₆ substitué par un groupe mono-, di- ou poly-halogénoalkylcarbonyle en C₂₋₇, un groupe alkyle en C₁₋₆ substitué par un groupe alcénylcarbonyle en C₃₋₇, un groupe alkyle en C₁₋₆ substitué par un groupe alcynylcarbonyle en C₃₋₇, un groupe alkyle en C₁₋₆ substitué par un groupe alkycarbonyle en C₂₋₅ substitué par un groupe alcoxy en C₁₋₄, un groupe alkyle en C₁₋₆ substitué par un groupe alkylcarbonyle en C₂₋₅ substitué par un groupe alkylthio en C₁₋₄, un groupe alkyle en C₁₋₆ substitué par un groupe alkylcarbonyle en C₂₋₅ substitué par un groupe alkylsulfinyle en C₁₋₄, un groupe alkyle en C₁₋₆ substitué par un groupe alkylcarbonyle en C₂₋₅ substitué par un groupe alkylsulfonyle en C₁₋₄, un groupe alcényle en C₂₋₆ substitué par un groupe alkylcarbonyle en C₂₋₇, un groupe alcynyle en C₂₋₆ substitué par un groupe alkylcarbonyle en C₂₋₇, un groupe alkyle en C₁₋₆ substitué par un groupe alkylsulfamoyle en C₁₋₆, un groupe alkyle en C₁₋₆ substitué par un groupe alcoxysulfamoyle en C₁₋₆, un groupe alkyle en C₁₋₆ substitué par un groupe di(alkyl en C₁₋₃)sulfamoyle, un groupe alkyle en C₁₋₆ substitué par un groupe N-(alkyl en C₁₋₃)-N-(alcoxy en C₁₋₃)sulfamoyle, un groupe alkyle en C₁₋₆ substitué par un groupe alkylcarbamoyle en C₂₋₇, un groupe alkyle en C₁₋₆ substitué par un groupe di(alkyl en C₁₋₃)carbamoyle, un groupe alkyle en C₁₋₆ substitué par un groupe alcoxycarbamoyle en C₂₋₇, un groupe alkyle en C₁₋₆ substitué par un groupe N-(alkyl en C₁₋₃)-N-(alcoxy en C₁₋₃)carbamoyle, un groupe alkyle en C₁₋₆ substitué par un groupe alkylamino en C₁₋₆, un groupe alkyle en C₁₋₆ substitué par un groupe alcoxyamino en C₁₋₆, un groupe alkyle en C₁₋₆ substitué par un groupe di(alkyl en C₁₋₃)amino, un groupe alkyle en C₁₋₆ substitué par un groupe N-(alkyl en C₁₋₃)-N-(alcoxy en C₁₋₃)amino, un groupe alkyle en C₁₋₆ substitué par un groupe N-(alkylcarbonyl en C₂₋₇)-N-(alkyl en C₁₋₆)amino, un groupe alkyle en C₁₋₆ substitué par un groupe N-(alkylcarbonyl en C₂₋₇)-N-(alcoxy en C₁₋₆)amino, un groupe alkyle en C₁₋₆ substitué par un groupe N-(alkylsulfonyl en C₁₋₆)-N-(alkyl en C₁₋₆)amino, un groupe alkyle en C₁₋₆ substitué par un groupe N-(alkylsulfonyl en C₁₋₆)-N-(alcoxy en C₁₋₆)amino, un groupe phényle (à la condition qu'un tel groupe phényle puisse être substitué par un ou plusieurs substituants choisis dans le groupe constitué par un atome d'halogène, un groupe trifluorométhyle, un groupe nitro, un groupe alkyle en C₁₋₆, un groupe alcoxy en C₁₋₆ et un groupe alcoxycarbonyle en C₂₋₇), un groupe alkyle en C₁₋₆ substitué par un groupe phényle (à la condition qu'un tel groupe phényle puisse être substitué par un ou plusieurs substituants choisis dans le groupe constitué par un atome d'halogène, un groupe trifluorométhyle, un groupe nitro, un groupe alkyle en C₁₋₆, un groupe alcoxy en C₁₋₆ et un groupe alcoxycarbonyle en C₂₋₇), un groupe alcényle en C₂₋₇ substitué par un groupe phényle (à la condition qu'un tel groupe phényle puisse être substitué par un ou plusieurs substituants choisis dans le groupe constitué par un atome d'halogène, un groupe trifluorométhyle, un groupe nitro, un groupe alkyle en C₁₋₆, un groupe alcoxy en C₁₋₆ et un groupe alcoxycarbonyle en C₂₋₇), un groupe alcynyle en C₂₋₆ substitué par un groupe phényle (à la condition qu'un tel groupe phényle puisse être substitué par un ou plusieurs substituants choisis dans le groupe constitué par un atome d'halogène, un groupe trifluorométhyle, un groupe nitro, un groupe alkyle en C₁₋₆, un groupe alcoxy en C₁₋₆ et un groupe alcoxycarbonyle en C₂₋₇), un groupe alkyle en C₁₋₆ substitué par un groupe phénoxy (à la condition qu'un tel groupe phénoxy puisse être substitué par un ou plusieurs substituants choisis dans le groupe constitué par un atome d'halogène, un groupe trifluorométhyle, un groupe nitro, un groupe alkyle en C₁₋₆, un groupe alcoxy en C₁₋₆ et un groupe alcoxycarbonyle en C₂₋₇), un groupe alkyle en C₁₋₆ substitué par un groupe phénylthio (à la condition qu'un tel groupe phénylthio puisse être substitué par un ou plusieurs substituants choisis dans le groupe constitué par un atome d'halogène, un groupe trifluorométhyle, un groupe nitro, un groupe alkyle en C₁₋₆, un groupe alcoxy en C₁₋₆ et un groupe alcoxycarbonyle en C₂₋₇), un groupe alkyle en C₁₋₆ substitué par un groupe phénylsulfinyle (à la condition qu'un tel groupe phénylsulfinyle puisse être substitué par un ou plusieurs substituants choisis dans le groupe constitué par un atome d'halogène, un groupe trifluorométhyle, un groupe nitro, un groupe alkyle en C₁₋₆, un groupe alcoxy en C₁₋₆ et un groupe alcoxycarbonyle en C₂₋₇), un groupe alkyle en C₁₋₆ substitué par un groupe phénylsulfonyle (à la condition qu'un tel groupe phénylsulfonyle puisse être substitué par un ou plusieurs substituants choisis dans le groupe constitué par un atome d'halogène, un groupe trifluorométhyle, un groupe nitro, un groupe alkyle en C₁₋₆, un groupe alcoxy en C₁₋₆ et un groupe alcoxycarbonyle en C₂₋₇), un groupe alkyle en C₁₋₆ substitué par un groupe benzyloxy (à la condition que le groupe phényle d'un tel groupe benzyloxy puisse être substitué par un ou plusieurs substituants choisis dans le groupe constitué par un atome d'halogène, un groupe trifluorométhyle, un groupe nitro, un groupe alkyle en C₁₋₆, un groupe alcoxy en C₁₋₆ et un groupe alcoxycarbonyle en C₂₋₇), un groupe alkyle en C₁₋₆ substitué par un groupe benzylthio (à la condition que le groupe phényle d'un tel groupe benzylthio puisse être substitué par un ou plusieurs substituants choisis dans le groupe constitué par un atome d'halogène, un groupe trifluorométhyle, un groupe nitro, un groupe alkyle en C₁₋₆, un groupe alcoxy en C₁₋₆ et un groupe alcoxycarbonyle en C₂₋₇), un groupe alkyle en C₁₋₆ substitué par un groupe benzylsulfinyle (à la condition que le groupe phényle d'un tel groupe benzylsulfinyle puisse être substitué par un ou plusieurs substituants choisis dans le groupe constitué par un atome d'halogène, un groupe trifluorométhyle, un groupe nitro, un groupe alkyle en C₁₋₆, un groupe alcoxy en C₁₋₆ et un groupe alcoxycarbonyle en C₂₋₇), un groupe alkyle en C₁₋₆ substitué par un groupe benzylsulfonyle (à la condition que le groupe phényle d'un tel groupe benzylsulfonyle puisse être substitué par un ou plusieurs substituants choisis dans le groupe constitué par un atome d'halogène, un groupe trifluorométhyle, un groupe nitro, un groupe alkyle en C₁₋₆, un groupe alcoxy en C₁₋₆ et un groupe alcoxycarbonyle en C₂₋₇), un groupe alkyle en C₁₋₆ substitué par un groupe phénylcarbonyle (à la condition qu'un tel groupe phénylcarbonyle puisse être substitué par un ou plusieurs substituants choisis dans le groupe constitué par un atome d'halogène, un groupe trifluorométhyle, un groupe nitro, un groupe alkyle en C₁₋₆, un groupe alcoxy en C₁₋₆ et un groupe alcoxycarbonyle en C₂₋₇), un groupe alkyle en C₁₋₆ substitué par un groupe benzylcarbonyle (à la condition que le groupe phényle d'un tel groupe benzylcarbonyle puisse être substitué par un ou plusieurs substituants choisis dans le groupe constitué par un atome d'halogène, un groupe trifluorométhyle, un groupe nitro, un groupe alkyle en C₁₋₆, un groupe alcoxy en C₁₋₆ et un groupe alcoxycarbonyle en C₂₋₇), un groupe alkyle en C₁₋₆ substitué par un groupe amino substitué par un groupe alkylsulfonyle en C₁₋₄ ou un groupe alkyle en C₁₋₆ substitué par un groupe amino substitué par un groupe alkylcarbonyle en C₂₋₄ ;
- R^{b2} est un atome d'hydrogène, un groupe alkyle en C₁₋₆, un groupe mono-, di- ou poly-halogénoalkyle en C₁₋₆, un groupe alcoxy en C₁₋₆, un groupe mono-, di- ou poly-halogénoalcoxy en C₁₋₆, un groupe alkylthio en C₁₋₆, un groupe alkylsulfinyle en C₁₋₆, un groupe alkylsulfonyle en C₁₋₆, un groupe alcoxycarbonyle en C₂₋₇, un groupe alkylcarbonyle en C₂₋₇, un atome d'halogène, un groupe nitro, un groupe cyano, ou un groupe phényle (à la condition qu'un tel groupe phényle puisse être substitué par un ou plusieurs substituants choisis dans le groupe constitué par un atome d'halogène, un groupe trifluorométhyle, un groupe nitro, un groupe alkyle en C₁₋₆, un groupe alcoxy en C₁₋₆ et un groupe alcoxycarbonyle en C₂₋₇) ;
- R^{b3} est un groupe alkyle en C₁₋₆, un groupe alcényle en C₂₋₆ ou un groupe alcynyle en C₂₋₆ ;
- R^{b4} est un atome d'hydrogène ou un groupe alkyle en C₁₋₆ ;
- R^{b5} est un atome d'hydrogène ou un groupe alkyle en C₁₋₆ ;
- R^{c1} est un groupe alkyle en C₁₋₈, un groupe cycloalkyle en C₃₋₇, un groupe alkyle en C₁₋₆ substitué par un groupe cycloalkyle en C₃₋₇, un groupe cycloalcényle en C₃₋₇, un groupe alkyle en C₁₋₆ substitué par un groupe cycloalcényle en C₃₋₇, un groupe alcényle en C₂₋₈, un groupe alcynyle en C₂₋₈, un groupe alkyle en C₁₋₆ substitué par un groupe alcoxy en C₁₋₆, un groupe alkyle en C₁₋₆ substitué par un groupe alcényloxy en C₂₋₆, un groupe alkyle en C₁₋₆ substitué par un groupe alcynyloxy en C₂₋₆, un groupe alkyle en C₁₋₆ substitué par un groupe mono-, di- ou poly-halogénoalcoxy en C₁₋₆, un groupe alkyle en C₁₋₆ substitué par un groupe mono-, di-, ou poly-halogénoalcényloxy en C₂₋₆, un groupe alkyle en C₁₋₆ substitué par un groupe mono-, di- ou poly-halogénoalcynyloxy en C₂₋₆, un groupe alkyle en C₁₋₆ substitué par un groupe alkylthio en C₁₋₆, un groupe alkyle en C₁₋₆ substitué par un groupe alkylsulfinyle en C₁₋₆, un groupe alkyle en C₁₋₆ substitué par un groupe alkylsulfonyle en C₁₋₆, un groupe mono-, di-, ou poly-halogénoalkyle en C₁₋₈, un groupe mono-, di- ou poly-halogénoalcényle en C₂₋₈, un groupe mono-, di- ou poly-halogénoalcynyle en C₂₋₈, un groupe alkyle en C₁₋₆ substitué par un groupe cyano, un groupe alcényle en C₂₋₆ substitué par un groupe cyano, un groupe alcynyle en C₂₋₆ substitué par un groupe cyano, un groupe alkyle en C₁₋₆ substitué par un groupe nitro, un groupe alcényle en C₂₋₆ substitué par un groupe nitro, un groupe alcynyle en C₂₋₆ substitué par un groupe nitro, un groupe alkyle en C₁₋₆ substitué par un groupe alcoxycarbonyle en C₂₋₇, un groupe alcényle en C₂₋₆ substitué par un groupe alcoxycarbonyle en C₂₋₇, un groupe alcynyle en C₂₋₆ substitué par un groupe alcoxycarbonyle en C₂₋₇, un groupe alkyle en C₁₋₆ substitué par un groupe alkylcarbonyle en C₂₋₇, un groupe alkyle en C₁₋₆ substitué par un groupe mono-, di-, ou poly-halogénoalkylcarbonyle en C₂₋₇, un groupe alkyle en C₁₋₆ substitué par un groupe alcénylcarbonyle en C₃₋₇, un groupe alkyle en C₁₋₆ substitué par un groupe alcynylcarbonyle en C₃₋₇, un groupe alkyle en C₁₋₆ substitué par un groupe alkylcarbonyle en C₂₋₅ substitué par un groupe alcoxy en C₁₋₄, un groupe alkyle en C₁₋₆ substitué par un groupe alkylcarbonyle en C₂₋₅ substitué par un groupe alkylthio en C₁₋₄, un groupe alkyle en C₁₋₆ substitué par un groupe alkylcarbonyle en C₂₋₅ substitué par un groupe alkylsulfinyle en C₁₋₄, un groupe alkyle en C₁₋₆ substitué par un groupe alkylcarbonyle en C₂₋₅ substitué par un groupe alkylsulfonyle en C₁₋₄, un groupe alcényle en C₂₋₆ substitué par un groupe alkylcarbonyle en C₂₋₇, un groupe alcynyle en C₂₋₆ substitué par un groupe alkylcarbonyle en C₂₋₇, un groupe alkyle en C₁₋₆ substitué par un groupe alkylsulfamoyle en C₁₋₆, un groupe alkyle en C₁₋₆ substitué par un groupe alcoxysulfamoyle en C₁₋₆, un groupe alkyle en C₁₋₆ substitué par un groupe di(alkyl en C₁₋₃)sulfamoyle, un groupe alkyle en C₁₋₆ substitué par un groupe N-(alkyl en C₁₋₃)-N-(alcoxy en C₁₋₃)sulfamoyle, un groupe alkyle en C₁₋₆ substitué par un groupe alkylcarbamoyle en C₂₋₇, un groupe alkyle en C₁₋₆ substitué par un groupe di(alkyl en C₁₋₃)carbamoyle, un groupe alkyle en C₁₋₆ substitué par un groupe alcoxycarbamoyle en C₂₋₇, un groupe alkyle en C₁₋₆ substitué par un groupe N-(alkyl en C₁₋₃)-N-(alcoxy en C₁₋₃)carbamoyle, un groupe alkyle en C₁₋₆ substitué par un groupe alkylamino en C₁₋₆, un groupe alkyle en C₁₋₆ substitué par un groupe alcoxyamino en C₁₋₆, un groupe alkyle en C₁₋₆ substitué par un groupe di(alkyl en C₁₋₃)amino, un groupe alkyle en C₁₋₆ substitué par un groupe N-(alkyl en C₁₋₃)-N-(alcoxy en C₁₋₃)amino, un groupe alkyle en C₁₋₆ substitué par un groupe N-(alkylcarbonyl en C₂₋₇)-N-(alkyl en C₁₋₆)amino, un groupe alkyle en C₁₋₆ substitué par un groupe N-(alkylcarbonyl en C₂₋₇)-N-(alcoxy en C₁₋₆)amino, un groupe alkyle en C₁₋₆ substitué par un groupe N-(alkylsulfonyl en C₁₋₆)-N-(alkyl en C₁₋₆)amino, un groupe alkyle en C₁₋₆ substitué par un groupe N-(alkylsulfonyl en C₁₋₆)-N-(alcoxy en C₁₋₆)amino, un groupe phényle (à la condition qu'un tel groupe phényle puisse être substitué par un ou plusieurs substituants choisis dans le groupe constitué par un atome d'halogène, un groupe trifluorométhyle, un groupe nitro, un groupe alkyle en C₁₋₆, un groupe alcoxy en C₁₋₆ et un groupe alcoxycarbonyle en C₂₋₇), un groupe alkyle en C₁₋₆ substitué par un groupe phényle (à la condition qu'un tel groupe phényle puisse être substitué par un ou plusieurs substituants choisis dans le groupe constitué par un atome d'halogène, un groupe trifluorométhyle, un groupe nitro, un groupe alkyle en C₁₋₆, un groupe alcoxy en C₁₋₆ et un groupe alcoxycarbonyle en C₂₋₇), un groupe alcényle en C₂₋₇ substitué par un groupe phényle (à la condition qu'un tel groupe phényle puisse être substitué par un ou plusieurs substituants choisis dans le groupe constitué par un atome d'halogène, un groupe trifluorométhyle, un groupe nitro, un groupe alkyle en C₁₋₆, un groupe alcoxy en C₁₋₆ et un groupe alcoxycarbonyle en C₂₋₇), un groupe alcynyle en C₂₋₆ substitué par un groupe phényle (à la condition qu'un tel groupe phényle puisse être substitué par un ou plusieurs substituants choisis dans le groupe constitué par un atome d'halogène, un groupe trifluorométhyle, un groupe nitro, un groupe alkyle en C₁₋₆, un groupe alcoxy en C₁₋₆ et un groupe alcoxycarbonyle en C₂₋₇), un groupe alkyle en C₁₋₆ substitué par un groupe phénoxy (à la condition qu'un tel groupe phénoxy puisse être substitué par un ou plusieurs substituants choisis dans le groupe constitué par un atome d'halogène, un groupe trifluorométhyle, un groupe nitro, un groupe alkyle en C₁₋₆, un groupe alcoxy en C₁₋₆ et un groupe alcoxy-carbonyle en C₂₋₇), un groupe alkyle en C₁₋₆ substitué par un groupe phénylthio (à la condition qu'un tel groupe phénylthio puisse être substitué par un ou plusieurs substituants choisis dans le groupe constitué par un atome d'halogène, un groupe trifluorométhyle, un groupe nitro, un groupe alkyle en C₁₋₆, un groupe alcoxy en C₁₋₆ et un groupe alcoxycarbonyle en C₂₋₇), un groupe alkyle en C₁₋₆ substitué par un groupe phénylsulfinyle (à la condition qu'un tel groupe phénylsulfinyle puisse être substitué par un ou plusieurs substituants choisis dans le groupe constitué par un atome d'halogène, un groupe trifluorométhyle, un groupe nitro, un groupe alkyle en C₁₋₆, un groupe alcoxy en C₁₋₆ et un groupe alcoxycarbonyle en C₂₋₇), un groupe alkyle en C₁₋₆ substitué par un groupe phénylsulfonyle (à la condition qu'un tel groupe phénylsulfonyle puisse être substitué par un ou plusieurs substituants choisis dans le groupe constitué par un atome d'halogène, un groupe trifluorométhyle, un groupe nitro, un groupe alkyle en C₁₋₆, un groupe alcoxy en C₁₋₆ et un groupe alcoxy-carbonyle en C₂₋₇₎, un groupe alkyle en C₁₋₆ substitué par un groupe benzyloxy (à la condition que le groupe phényle d'un tel groupe benzyloxy puisse être substitué par un ou plusieurs substituants choisis dans le groupe constitué par un atome d'halogène, un groupe trifluorométhyle, un groupe nitro, un groupe alkyle en C₁₋₆, un groupe alcoxy en C₁₋₆ et un groupe alcoxy-carbonyle en C₂₋₇), un groupe alkyle en C₁₋₆ substitué par un groupe benzylthio (à la condition que le groupe phényle d'un tel groupe benzylthio puisse être substitué par un ou plusieurs substituants choisis dans le groupe constitué par un atome d'halogène, un groupe trifluorométhyle, un groupe nitro, un groupe alkyle en C₁₋₆, un groupe alcoxy en C₁₋₆ et un groupe alcoxycarbonyle en C₂₋₇), un groupe alkyle en C₁₋₆ substitué par un groupe benzylsulfinyle (à la condition que le groupe phényle d'un tel groupe benzylsulfinyle puisse être substitué par un ou plusieurs substituants choisis dans le groupe constitué par un atome d'halogène, un groupe trifluorométhyle, un groupe nitro, un groupe alkyle en C₁₋₆, un groupe alcoxy en C₁₋₆ et un groupe alcoxycarbonyle en C₂₋₇), un groupe alkyle en C₁₋₆ substitué par un groupe benzylsulfonyle (à la condition que le groupe phényle d'un tel groupe benzylsulfonyle puisse être substitué par un ou plusieurs substituants choisis dans le groupe constitué par un atome d'halogène, un groupe trifluorométhyle, un groupe nitro, un groupe alkyle en C₁₋₆, un groupe alcoxy en C₁₋₆ et un groupe alcoxycarbonyle en C₂₋₇₎, un groupe alkyle en C₁₋₆ substitué par un groupe phénylcarbonyle (à la condition qu'un tel groupe phénylcarbonyle puisse être substitué par un ou plusieurs substituants choisis dans le groupe constitué par un atome d'halogène, un groupe trifluorométhyle, un groupe nitro, un groupe alkyle en C₁₋₆, un groupe alcoxy en C₁₋₆ et un groupe alcoxycarbonyle en C₂₋₇), un groupe alkyle en C₁₋₆ substitué par un groupe benzylcarbonyle (à la condition que le groupe phényle d'un tel groupe benzylcarbonyle puisse être substitué par un ou plusieurs substituants choisis dans le groupe constitué par un atome d'halogène, un groupe trifluorométhyle, un groupe nitro, un groupe alkyle en C₁₋₆, un groupe alcoxy en C₁₋₆ et un groupe alcoxycarbonyle en C₂₋₇), un groupe alkyle en C₁₋₆ substitué par un groupe amino substitué par un groupe alkylsulfonyle en C₁₋₄, ou un groupe alkyle en C₁₋₆ substitué par un groupe amino substitué par un groupe alkylcarbonyle en C₂₋₄ ;
- R^{c2}, R^{c3}, R^{c4}, R^{c5}, R^{c6}, R^{c11} et R^{c12}, qui sont indépendants l'un de l'autre, représentent chacun un atome d'hydrogène, un groupe alkyle en C₁₋₈, un groupe alcényle en C₂₋₈, un groupe alcynyle en C₂₋₈, ou un groupe phényle (à la condition qu'un tel groupe phényle puisse être substitué par un ou plusieurs substituants choisis dans le groupe constitué par un atome d'halogène, un groupe trifluorométhyle, un groupe nitro, un groupe alkyle en C₁₋₆, un groupe alcoxy en C₁₋₆ et un groupe alcoxycarbonyle en C₂₋₇) ;
- R^{c7}, R^{c8}, R^{c9}, R^{c10}, R^{c13} et R^{c14}, qui sont indépendants l'un de l'autre, représentent chacun un atome d'hydrogène, un groupe alkyle en C₁₋₆, un groupe mono-, di- ou poly-halogénoalkyle en C₁₋₆, un groupe alcoxy en C₁₋₆, un groupe mono-, di- ou poly-halogénoalcoxy en C₁₋₆, un groupe alkylthio en C₁₋₆, un groupe alkylsulfinyle en C₁₋₆, un groupe alkylsulfonyle en C₁₋₆, un groupe alcoxycarbonyle en C₂₋₇, un groupe alkylcarbonyle en C₂₋₇, un atome d'halogène, un groupe nitro, un groupe cyano ou un groupe phényle (à la condition qu'un tel groupe phényle puisse être substitué par un ou plusieurs substituants choisis dans le groupe constitué par un atome d'halogène, un groupe trifluorométhyle, un groupe nitro, un groupe alkyle en C₁₋₆, un groupe alcoxy en C₁₋₆ et un groupe alcoxycarbonyle en C₂₋₇) ;
- R^{d1} est un groupe alkyle en C₁₋₈, un groupe cycloalkyle en C₃₋₇, un groupe alkyle en C₁₋₆ substitué par un groupe cycloalkyle en C₃₋₇, un groupe cycloalcényle en C₃₋₇, un groupe alkyle en C₁₋₆ substitué par un groupe cycloalcényle en C₃₋₇, un groupe alcényle en C₂₋₈, un groupe alcynyle en C₂₋₈, un groupe alkyle en C₁₋₆ substitué par un groupe alcoxy en C₁₋₆, un groupe alkyle en C₁₋₆ substitué par un groupe alcényloxy en C₂₋₆, un groupe alkyle en C₁₋₆ substitué par un groupe alcynyloxy en C₂₋₆, un groupe alkyle en C₁₋₆ substitué par un groupe mono-, di- ou poly-halogénoalcoxy en C₁₋₆, un groupe alkyle en C₁₋₆ substitué par un groupe mono-, di- ou poly-halogénoalcényloxy en C₂₋₆, un groupe alkyle en C₁₋₆ substitué par un groupe mono-, di-, ou poly-halogénoalcynyloxy en C₂₋₆, un groupe alkyle en C₁₋₆ substitué par un groupe alkylthio en C₁₋₆, un groupe alkyle en C₁₋₆ substitué par un groupe alkylsulfinyle en C₁₋₆, un groupe alkyle en C₁₋₆ substitué par un groupe alkylsulfonyle en C₁₋₆, un groupe mono-, di- ou poly-halogénoalkyle en C₁₋₈, un groupe mono-, di- ou poly-halogénoalcényle en C₂₋₈, un groupe mono-, di- ou poly-halogénoalcynyle en C₂₋₈, un groupe alkyle en C₁₋₆ substitué par un groupe cyano, un groupe alcényle en C₂₋₆ substitué par un groupe cyano, un groupe alcynyle en C₂₋₆ substitué par un groupe cyano, un groupe alkyle en C₁₋₆ substitué par un groupe nitro, un groupe alcényle en C₂₋₆ substitué par un groupe nitro, un groupe alcynyle en C₂₋₆ substitué par un groupe nitro, un groupe alkyle en C₁₋₆ substitué par un groupe alcoxycarbonyle en C₂₋₇, un groupe alcényle en C₂₋₆ substitué par un groupe alcoxycarbonyle en C₂₋₇, un groupe alcynyle en C₂₋₆ substitué par un groupe alcoxycarbonyle en C₂₋₇, un groupe alkyle en C₁₋₆ substitué par un groupe alkylcarbonyle en C₂₋₇, un groupe alkyle en C₁₋₆ substitué par un groupe mono-, di- ou poly-halogénoalkylcarbonyle en C₂₋₇, un groupe alkyle en C₁₋₆ substitué par un groupe alcénylcarbonyle en C₃₋₇, un groupe alkyle en C₁₋₆ substitué par un groupe alcynyl-carbonyle en C₃₋₇, un groupe alkyle en C₁₋₆ substitué par un groupe alkylcarbonyle en C₂₋₅ substitué par un groupe alcoxy en C₁₋₄, un groupe alkyle en C₁₋₆ substitué par un groupe alkylcarbonyle en C₂₋₅ substitué par un groupe alkylthio en C₁₋₄, un groupe alkyle en C₁₋₆ substitué par un groupe alkylcarbonyle en C₂₋₅ substitué par un groupe alkylsulfinyle en C₁₋₄, un groupe alkyle en C₁₋₆ substitué par un groupe alkylcarbonyle en C₂₋₅ substitué par un groupe alkylsulfonyle en C₁₋₄, un groupe alcényle en C₂₋₆ substitué par un groupe alkylcarbonyle en C₂₋₇, un groupe alcynyle en C₂₋₆ substitué par un groupe alkylcarbonyle en C₂₋₇, un groupe alkyle en C₁₋₆ substitué par un groupe alkylsulfamoyle en C₁₋₆, un groupe alkyle en C₁₋₆ substitué par un groupe alcoxysulfamoyle en C₁₋₆, un groupe alkyle en C₁₋₆ substitué par un groupe di(alkyl en C₁₋₃)sulfamoyle, un groupe alkyle en C₁₋₆ substitué par un groupe N-(alkyl en C₁₋₃)-N-(alcoxy en C₁₋₃)sulfamoyle, un groupe alkyle en C₁₋₆ substitué par un groupe alkylcarbamoyle en C₂₋₇, un groupe alkyle en C₁₋₆ substitué par un groupe di(alkyl en C₁₋₃)carbamoyle, un groupe alkyle en C₁₋₆ substitué par un groupe alcoxy-carbamoyle en C₂₋₇, un groupe alkyle en C₁₋₆ substitué par un groupe N-(alkyl en C₁₋₃)-N-(alcoxy en C₁₋₃)carbamoyle, un groupe alkyle en C₁₋₆ substitué par un groupe alkylamino en C₁₋₆, un groupe alkyle en C₁₋₆ substitué par un groupe alcoxyamino en C₁₋₆, un groupe alkyle en C₁₋₆ substitué par un groupe di(alkyl en C₁₋₃)amino, un groupe alkyle en C₁₋₆ substitué par un groupe N-(alkyl en C₁₋₃)-N-(alcoxy en C₁₋₃)amino, un groupe alkyle en C₁₋₆ substitué par un groupe N-(alkylcarbonyl en C₂₋₇)-N-(alkyl en C₁₋₆)amino, un groupe alkyle en C₁₋₆ substitué par un groupe N-(alkylcarbonyl en C₂₋₇)-N-(alcoxy en C₁₋₆)amino, un groupe alkyle en C₁₋₆ substitué par un groupe N-(alkylsulfonyl en C₁₋₆)-N-(alkyl en C₁₋₆)amino, un groupe alkyle en C₁₋₆ substitué par un groupe N-(alkylsulfonyl en C₁₋₆)-N-(alcoxy en C₁₋₆)amino, un groupe phényle (à la condition qu'un tel groupe phényle puisse être substitué par un ou plusieurs substituants choisis dans le groupe constitué par un atome d'halogène, un groupe trifluorométhyle, un groupe nitro, un groupe alkyle en C₁₋₆, un groupe alcoxy en C₁₋₆ et un groupe alcoxycarbonyle en C₂₋₇), un groupe alkyle en C₁₋₆ substitué par un groupe phényle (à la condition qu'un tel groupe phényle puisse être substitué par un ou plusieurs substituants choisis dans le groupe constitué par un atome d'halogène, un groupe trifluorométhyle, un groupe nitro, un groupe alkyle en C₁₋₆, un groupe alcoxy en C₁₋₆ et un groupe alcoxycarbonyle en C₂₋₇), un groupe alcényle en C₂₋₇ substitué par un groupe phényle (à la condition qu'un tel groupe phényle puisse être substitué par un ou plusieurs substituants choisis dans le groupe constitué par un atome d'halogène, un groupe trifluorométhyle, un groupe nitro, un groupe alkyle en C₁₋₆, un groupe alcoxy en C₁₋₆ et un groupe alcoxycarbonyle en C₂₋₇), un groupe alcynyle en C₂₋₆ substitué par un groupe phényle (à la condition qu'un tel groupe phényle puisse être substitué par un ou plusieurs substituants choisis dans le groupe constitué par un atome d'halogène, un groupe trifluorométhyle, un groupe nitro, un groupe alkyle en C₁₋₆, un groupe alcoxy en C₁₋₆ et un groupe alcoxycarbonyle en C₂₋₇), un groupe alkyle en C₁₋₆ substitué par un groupe phénoxy (à la condition qu'un tel groupe phénoxy puisse être substitué par un ou plusieurs substituants choisis dans le groupe constitué par un atome d'halogène, un groupe trifluorométhyle, un groupe nitro, un groupe alkyle en C₁₋₆, un groupe alcoxy en C₁₋₆ et un groupe alcoxycarbonyle en C₂₋₇), un groupe alkyle en C₁₋₆ substitué par un groupe phénylthio (à la condition qu'un tel groupe phénylthio puisse être substitué par un ou plusieurs substituants choisis dans le groupe constitué par un atome d'halogène, un groupe trifluorométhyle, un groupe nitro, un groupe alkyle en C₁₋₆, un groupe alcoxy en C₁₋₆ et un groupe alcoxycarbonyle en C₂₋₇), un groupe alkyle en C₁₋₆ substitué par un groupe phénylsulfinyle (à la condition qu'un tel groupe phénylsulfinyle puisse être substitué par un ou plusieurs substituants choisis dans le groupe constitué par un atome d'halogène, un groupe trifluorométhyle, un groupe nitro, un groupe alkyle en C₁₋₆, un groupe alcoxy en C₁₋₆ et un groupe alcoxycarbonyle en C₂₋₇), un groupe alkyle en C₁₋₆ substitué par un groupe phénylsulfonyle (à la condition qu'un tel groupe phénylsulfonyle puisse être substitué par un ou plusieurs substituants choisis dans le groupe constitué par un atome d'halogène, un groupe trifluorométhyle, un groupe nitro, un groupe alkyle en C₁₋₆, un groupe alcoxy en C₁₋₆ et un groupe alcoxycarbonyle en C₂₋₇), un groupe alkyle en C₁₋₆ substitué par un groupe benzyloxy (à la condition que le groupe phényle d'un tel groupe benzyloxy puisse être substitué par un ou plusieurs substituants choisis dans le groupe constitué par un atome d'halogène, un groupe trifluorométhyle, un groupe nitro, un groupe alkyle en C₁₋₆, un groupe alcoxy en C₁₋₆ et un groupe alcoxycarbonyle en C₂₋₇), un groupe alkyle en C₁₋₆ substitué par un groupe benzylthio (à la condition que le groupe phényle d'un tel groupe benzylthio puisse être substitué par un ou plusieurs substituants choisis dans le groupe constitué par un atome d'halogène, un groupe trifluorométhyle, un groupe nitro, un groupe alkyle en C₁₋₆, un groupe alcoxy en C₁₋₆ et un groupe alcoxycarbonyle en C₂₋₇), un groupe alkyle en C₁₋₆ substitué par un groupe benzylsulfinyle (à la condition que le groupe phényle d'un tel groupe benzylsulfinyle puisse être substitué par un ou plusieurs substituants choisis dans le groupe constitué par un atome d'halogène, un groupe trifluorométhyle, un groupe nitro, un groupe alkyle en C₁₋₆, un groupe alcoxy en C₁₋₆ et un groupe alcoxycarbonyle en C₂₋₇), un groupe alkyle en C₁₋₆ substitué par un groupe benzylsulfonyle, (à la condition que le groupe phényle d'un tel groupe benzylsulfonyle puisse être substitué par un ou plusieurs substituants choisis dans le groupe constitué par un atome d'halogène, un groupe trifluorométhyle, un groupe nitro, un groupe alkyle en C₁₋₆, un groupe alcoxy en C₁₋₆ et un groupe alcoxycarbonyle en C₂₋₇), un groupe alkyle en C₁₋₆ substitué par un groupe phénylcarbonyle (à la condition qu'un tel groupe phénylcarbonyle puisse être substitué par un ou plusieurs substituants choisis dans le groupe constitué par un atome d'halogène, un groupe trifluorométhyle, un groupe nitro, un groupe alkyle en C₁₋₆, un groupe alcoxy en C₁₋₆ et un groupe alcoxycarbonyle en C₂₋₇), un groupe alkyle en C₁₋₆ substitué par un groupe benzylcarbonyle (à la condition que le groupe phényle d'un tel groupe benzylcarbonyle puisse être substitué par un ou plusieurs substituants choisis dans le groupe constitué par un atome d'halogène, un groupe trifluorométhyle, un groupe nitro, un groupe alkyle en C₁₋₆, un groupe alcoxy en C₁₋₆ et un groupe alcoxycarbonyle en C₂₋₇), un groupe alkyle en C₁₋₆ substitué par un groupe amino substitué par un groupe alkylsulfonyle en C₁₋₄ ou un groupe alkyle en C₁₋₆ substitué par un groupe amino substitué par un groupe alkylcarbonyle en C₂₋₄ ;
- R^{d2}, R^{d3} et R^{d4}, qui sont indépendants l'un de l'autre, représentent chacun un atome d'hydrogène, un groupe alkyle en C₁₋₈, un groupe alcényle en C₂₋₈, un groupe alcynyle en C₂₋₈, ou un groupe phényle (à la condition qu'un tel groupe phényle puisse être substitué par un ou plusieurs substituants choisis dans le groupe constitué par un atome d'halogène, un groupe trifluorométhyle, un groupe nitro, un groupe alkyle en C₁₋₆, un groupe alcoxy en C₁₋₆ et un groupe alcoxycarbonyle en C₂₋₇) ;
- R^{d5} est un groupe alkyle en C₁₋₈, un groupe alcényle en C₂₋₈, un groupe alcynyle en C₂₋₈, ou un groupe phényle (à la condition qu'un tel groupe phényle puisse être substitué par un ou plusieurs substituants choisis dans le groupe constitué par un atome d'halogène, un groupe trifluorométhyle, un groupe nitro, un groupe alkyle en C₁₋₆, un groupe alcoxy en C₁₋₆ et un groupe alcoxycarbonyle en C₂₋₇) ;
- R^{d6} est un groupe alkyle en C₁₋₈, un groupe alcényle en C₂₋₈, un groupe alcynyle en C₂₋₈, un groupe alkyle en C₁₋₆ substitué par un groupe alkylthio en C₁₋₆, un groupe alkyle en C₁₋₆ substitué par un groupe alkylsulfinyle en C₁₋₆, un groupe alkyle en C₁₋₆ substitué par un groupe alkylsulfonyle en C₁₋₆, un groupe alkyle en C₁₋₆ substitué par un groupe alcoxy en C₁₋₆, un groupe alkyle en C₁₋₆ substitué par un groupe alcoxycarbonyle en C₂₋₇, un groupe alkyle en C₁₋₆ substitué par un groupe alkylcarbonyle en C₂₋₇, un groupe alkyle en C₁₋₆ substitué par un groupe cyano, un groupe phényle (à la condition qu'un tel groupe phényle puisse être substitué par un ou plusieurs substituants choisis dans le groupe constitué par un atome d'halogéne, un groupe trifluorométhyle, un groupe nitro, un groupe alkyle en C₁₋₆, un groupe alcoxy en C₁₋₆ et un groupe alcoxycarbonyle en C₂₋₇), ou un groupe alkyle en C₁₋₆ substitué par un groupe phényle (à la condition qu'un tel groupe phényle puisse être substitué par un ou plusieurs substituants choisis dans le groupe constitué par un atome d'halogène, un groupe trifluorométhyle, un groupe nitro, un groupe alkyle en C₁₋₆, un groupe alcoxy en C₁₋₆ et un groupe alcoxycarbonyle en C₂₋₇) ;
- R^{e1} est un groupe alkyle en C₁₋₈, un groupe cycloalkyle en C₃₋₇, un groupe alkyle en C₁₋₆ substitué par un groupe cycloalkyle en C₃₋₇, un groupe cycloalcényle en C₃₋₇, un groupe alkyle en C₁₋₆ substitué par un groupe cycloalcényle en C₃₋₇, un groupe alcényle en C₂₋₈, un groupe alcynyle en C₂₋₈, un groupe alkyle en C₁₋₆ substitué par un groupe alcoxy en C₁₋₆, un groupe alkyle en C₁₋₆ substitué par un groupe alcényloxy en C₂₋₆, un groupe alkyle en C₁₋₆ substitué par un groupe alcynyloxy en C₂₋₆, un groupe alkyle en C₁₋₆ substitué par un groupe mono-, di- ou poly-halogénoalcoxy en C₁₋₆ un groupe alkyle en C₁₋₆ substitué par un groupe mono-, di-, ou poly-halogénoalcényloxy en C₂₋₆, un groupe alkyle en C₁₋₆ substitué par un groupe mono-, di-, ou poly-halogénoalcynyloxy en C₂₋₆, un groupe alkyle en C₁₋₆ substitué par un groupe alkylthio en C₁₋₆, un groupe alkyle en C₁₋₆ substitué par un groupe alkylsulfinyle en C₁₋₆, un groupe alkyle en C₁₋₆ substitué par un groupe alkylsulfonyle en C₁₋₆, un groupe mono-, di- ou poly-halogénoalkyle en C₁₋₈, un groupe mono-, di- ou poly-halogénoalcényle en C₂₋₈, un groupe mono-, di- ou poly-halogénoalcynyle en C₂₋₈, un groupe alkyle en C₁₋₆ substitué par un groupe cyano, un groupe alcényle en C₂₋₆ substitué par un groupe cyano, un groupe alcynyle en C₂₋₆ substitué par un groupe cyano, un groupe alkyle en C₁₋₆ substitué par un groupe nitro, un groupe alcényle en C₂₋₆ substitué par un groupe nitro, un groupe alcynyle en C₂₋₆ substitué par un groupe nitro, un groupe alkyle en C₁₋₆ substitué par un groupe alcoxycarbonyle en C₂₋₇, un groupe alcényle en C₂₋₆ substitué par un groupe alcoxycarbonyle en C₂₋₇, un groupe alcynyle en C₂₋₆ substitué par un groupe alcoxycarbonyle en C₂₋₇, un groupe alkyle en C₁₋₆ substitué par un groupe alkylcarbonyle en C₂₋₇, un groupe alkyle en C₁₋₆ substitué par un groupe mono-, di-, ou poly-halogénoalkylcarbonyle en C₂₋₇, un groupe alkyle en C₁₋₆ substitué par un groupe alcénylcarbonyle en C₃₋₇, un groupe alkyle en C₁₋₆ substitué par un groupe alcynyl-carbonyle en C₃₋₇, un groupe alkyle en C₁₋₆ substitué par un groupe alkylcarbonyle en C₂₋₅ substitué par un groupe alcoxy en C₁₋₄, un groupe alkyle en C₁₋₆ substitué par un groupe alkylcarbonyle en C₂₋₅ substitué par un groupe alkylthio en C₁₋₄, un groupe alkyle en C₁₋₆ substitué par un groupe alkylcarbonyle en C₂₋₅ substitué par un groupe alkylsulfinyle en C₁₋₄, un groupe alkyle en C₁₋₆ substitué par un groupe alkylcarbonyle en C₂₋₅ substitué par un groupe alkylsulfonyle en C₁₋₄, un groupe alcényle en C₂₋₆ substitué par un groupe alkylcarbonyle en C₂₋₇, un groupe alcynyle en C₂₋₆ substitué par un groupe alkylcarbonyle en C₂₋₇, un groupe alkyle en C₁₋₆ substitué par un groupe alkylsulfamoyle en C₁₋₆, un groupe alkyle en C₁₋₆ substitué par un groupe alcoxysulfamoyle en C₁₋₆, un groupe alkyle en C₁₋₆ substitué par un groupe di(alkyl en C₁₋₃)sulfamoyle, un groupe alkyle en C₁₋₆ substitué par un groupe N-(alkyl en C₁₋₃)-N-(alcoxy en C₁₋₃)sulfamoyle, un groupe alkyle en C₁₋₆ substitué par un groupe alkylcarbamoyle en C₂₋₇, un groupe alkyle en C₁₋₆ substitué par un groupe di(alkyl en C₁₋₃)carbamoyle, un groupe alkyle en C₁₋₆ substitué par un groupe alcoxy-carbamoyle en C₂₋₇, un groupe alkyle en C₁₋₆ substitué par un groupe N-(alkyl en C₁₋₃)-N-(alcoxy en C₁₋₃)carbamoyle, un groupe alkyle en C₁₋₆ substitué par un groupe alkylamino en C₁₋₆, un groupe alkyle en C₁₋₆ substitué par un groupe alcoxyamino en C₁₋₆, un groupe alkyle en C₁₋₆ substitué par un groupe di(alkyl en C₁₋₃)amino, un groupe alkyle en C₁₋₆ substitué par un groupe N-(alkyl en C₁₋₃)-N-(alcoxy en C₁₋₃)amino, un groupe alkyle en C₁₋₆ substitué par un groupe N-(alkylcarbonyl en C₂₋₇)-N-(alkyl en C₁₋₆)amino, un groupe alkyle en C₁₋₆ substitué par un groupe N-(alkylcarbonyl en C₂₋₇)-N-(alcoxy en C₁₋₆)amino, un groupe alkyle en C₁₋₆ substitué par un groupe N-(alkylsulfonyl en C₁₋₆)-N-(alkyl en C₁₋₆)amino, un groupe alkyle en C₁₋₆ substitué par un groupe N-(alkylsulfonyl en C₁₋₆)-N-(alcoxy en C₁₋₆)amino, un groupe phényle (à la condition qu'un tel groupe phényle puisse être substitué par un ou plusieurs substituants choisis dans le groupe constitué par un atome d'halogène, un groupe trifluorométhyle, un groupe nitro, un groupe alkyle en C₁₋₆, un groupe alcoxy en C₁₋₆ et un groupe alcoxycarbonyle en C₂₋₇), un groupe alkyle en C₁₋₆ substitué par un groupe phényle (à la condition qu'un tel groupe phényle puisse être substitué par un ou plusieurs substituants choisis dans le groupe constitué par un atome d'halogène, un groupe trifluorométhyle, un groupe nitro, un groupe alkyle en C₁₋₆, un groupe alcoxy en C₁₋₆ et un groupe alcoxycarbonyle en C₂₋₇), un groupe alcényle en C₂₋₇ substitué par un groupe phényle (à la condition qu'un tel groupe phényle puisse être substitué par un ou plusieurs substituants choisis dans le groupe constitué par un atome d'halogène, un groupe trifluorométhyle, un groupe nitro, un groupe alkyle en C₁₋₆, un groupe alcoxy en C₁₋₆ et un groupe alcoxycarbonyle en C₂₋₇), un groupe alcynyle en C₂₋₆ substitué par un groupe phényle (à la condition qu'un tel groupe phényle puisse être substitué par un ou plusieurs substituants choisis dans le groupe constitué par un atome d'halogène, un groupe trifluorométhyle, un groupe nitro, un groupe alkyle en C₁₋₆, un groupe alcoxy en C₁₋₆ et un groupe alcoxycarbonyle en C₂₋₇), un groupe alkyle en C₁₋₆ substitué par un groupe phénoxy (à la condition qu'un tel groupe phénoxy puisse être substitué par un ou plusieurs substituants choisis dans le groupe constitué par un atome d'halogène, un groupe trifluorométhyle, un groupe nitro, un groupe alkyle en C₁₋₆, un groupe alcoxy en C₁₋₆ et un groupe alcoxy-carbonyle en C₂₋₇), un groupe alkyle en C₁₋₆ substitué par un groupe phénylthio (à la condition qu'un tel groupe phénylthio puisse être substitué par un ou plusieurs substituants choisis dans le groupe constitué par un atome d'halogène, un groupe trifluorométhyle, un groupe nitro, un groupe alkyle en C₁₋₆, un groupe alcoxy en C₁₋₆ et un groupe alcoxycarbonyle en C₂₋₇), un groupe alkyle en C₁₋₆ substitué par un groupe phénylsulfinyle (à la condition qu'un tel groupe phénylsulfinyle puisse être substitué par un ou plusieurs substituants choisis dans le groupe constitué par un atome d'halogène, un groupe trifluorométhyle, un groupe nitro, un groupe alkyle en C₁₋₆, un groupe alcoxy en C₁₋₆ et un groupe alcoxycarbonyle en C₂₋₇), un groupe alkyle en C₁₋₆ substitué par un groupe phénylsulfonyle (à la condition qu'un tel groupe phénylsulfonyle puisse être substitué par un ou plusieurs substituants choisis dans le groupe constitué par un atome d'halogène, un groupe trifluorométhyle, un groupe nitro, un groupe alkyle en C₁₋₆, un groupe alcoxy en C₁₋₆ et un groupe alcoxycarbonyle en C₂₋₇), un groupe alkyle en C₁₋₆ substitué par un groupe benzyloxy (à la condition que le groupe phényle d'un tel groupe benzyloxy puisse être substitué par un ou plusieurs substituants choisis dans le groupe constitué par un atome d'halogène, un groupe trifluorométhyle, un groupe nitro, un groupe alkyle en C₁₋₆, un groupe alcoxy en C₁₋₆ et un groupe alcoxycarbonyle en C₂₋₇), un groupe alkyle en C₁₋₆ substitué par un groupe benzylthio (à la condition que le groupe phényle d'un tel groupe benzylthio puisse être substitué par un ou plusieurs substituants choisis dans le groupe constitué par un atome d'halogène, un groupe trifluorométhyle, un groupe nitro, un groupe alkyle en C₁₋₆, un groupe alcoxy en C₁₋₆ et un groupe alcoxycarbonyle en C₂₋₇), un groupe alkyle en C₁₋₆ substitué par un groupe benzylsulfinyle (à la condition que le groupe phényle d'un tel groupe benzylsulfinyle puisse être substitué par un ou plusieurs substituants choisis dans le groupe constitué par un atome d'halogène, un groupe trifluorométhyle, un groupe nitro, un groupe alkyle en C₁₋₆, un groupe alcoxy en C₁₋₆ et un groupe alcoxycarbonyle en C₂₋₇), un groupe alkyle en C₁₋₆ substitué par un groupe benzylsulfonyle (à la condition que le groupe phényle d'un tel groupe benzylsulfonyle puisse être substitué par un ou plusieurs substituants choisis dans le groupe constitué par un atome d'halogène, un groupe trifluorométhyle, un groupe nitro, un groupe alkyle en C₁₋₆, un groupe alcoxy en C₁₋₆ et un groupe alcoxycarbonyle en C₂₋₇), un groupe alkyle en C₁₋₆ substitué par un groupe phénylcarbonyle (à la condition qu'un tel groupe phénylcarbonyle puisse être substitué par un ou plusieurs substituants choisis dans le groupe constitué par un atome d'halogène, un groupe trifluorométhyle, un groupe nitro, un groupe alkyle en C₁₋₆, un groupe alcoxy en C₁₋₆ et un groupe alcoxycarbonyle en C₂₋₇), un groupe alkyle en C₁₋₆ substitué par un groupe benzylcarbonyle (à la condition que le groupe phényle d'un tel groupe benzylcarbonyle puisse être substitué par un ou plusieurs substituants choisis dans le groupe constitué par un atome d'halogène, un groupe trifluorométhyle, un groupe nitro, un groupe alkyle en C₁₋₆, un groupe alcoxy en C₁₋₆ et un groupe alcoxycarbonyle en C₂₋₇), un groupe alkyle en C₁₋₆ substitué par un groupe amino substitué par un groupe alkylsulfonyle en C₁₋₄, ou un groupe alkyle en C₁₋₆ substitué par un groupe amino substitué par un groupe alkylcarbonyle en C₂₋₄ ;
- R^{e2}, R^{e3}, R^{e6} et R^{e7}, qui sont indépendants l'un de l'autre, représentent chacun un atome d'hydrogène, un groupe alkyle en C₁₋₆, un groupe mono-, di- ou poly-halogénoalkyle en C₁₋₆, un groupe alcoxy en C₁₋₆, un groupe mono-, di- ou poly-halogénoalcoxy en C₁₋₆, un groupe alkylthio en C₁₋₆, un groupe alkylsulfinyle en C₁₋₆, un groupe alkylsulfonyle en C₁₋₆, un groupe alcoxycarbonyle en C₂₋₇, un groupe alkylcarbonyle en C₂₋₇, un atome d'halogène, un groupe nitro, un groupe cyano, ou un groupe phényle (à la condition qu'un tel groupe phényle puisse être substitué par un ou plusieurs substituants choisis dans le groupe constitué par un atome d'halogène, un groupe trifluorométhyle, un groupe nitro, un groupe alkyle en C₁₋₆, un groupe alcoxy en C₁₋₆ et un groupe alcoxycarbonyle en C₂₋₇),
- R^{e4}, R^{e5}, R^{e8}, R^{e9} et R^{e10}, qui sont indépendants l'un de l'autre, représentent chacun un atome d'hydrogène, un groupe alkyle en C₁₋₈, un groupe alcényle en C₂₋₈, un groupe alcynyle en C₂₋₈, ou un groupe phényle (à la condition qu'un tel groupe phényle puisse être substitué par un ou plusieurs substituants choisis dans le groupe constitué par un atome d'halogène, un groupe trifluorométhyle, un groupe nitro, un groupe alkyle en C₁₋₆, un groupe alcoxy en C₁₋₆ et un groupe alcoxycarbonyle en C₂₋₇) ;
- R^{f1} est un groupe alkyle en C₁₋₈, un groupe cycloalkyle en C₃₋₇, un groupe alkyle en C₁₋₆ substitué par un groupe cycloalkyle en C₃₋₇, un groupe cycloalcényle en C₃₋₇, un groupe alkyle en C₁₋₆ substitué par un groupe cycloalcényle en C₃₋₇, un groupe alcényle en C₂₋₈, un groupe alcynyle en C₂₋₈, un groupe alkyle en C₁₋₆ substitué par un groupe alcoxy en C₁₋₆, un groupe alkyle en C₁₋₆ substitué par un groupe alcényloxy en C₂₋₆, un groupe alkyle en C₁₋₆ substitué par un groupe alcynyloxy en C₂₋₆, un groupe alkyle en C₁₋₆ substitué par un groupe mono-, di- ou poly-halogénoalcoxy en C₁₋₆, un groupe alkyle en C₁₋₆ substitué par un groupe mono-, di- ou poly-halogénoalcényloxy en C₂₋₆, un groupe alkyle en C₁₋₆ substitué par un groupe mono-, di- ou poly-halogénoalcynyloxy en C₂₋₆, un groupe alkyle en C₁₋₆ substitué par un groupe alkylthio en C₁₋₆, un groupe alkyle en C₁₋₆ substitué par un groupe alkylsulfinyle en C₁₋₆, un groupe alkyle en C₁₋₆ substitué par un groupe alkylsulfonyle en C₁₋₆, un groupe mono-, di- ou poly-halogénoalkyle en C₁₋₈, un groupe mono-, di- ou poly-halogénoalcényle en C₂₋₈, un groupe mono-, di- ou poly-halogénoalcynyle en C₂₋₈, un groupe alkyle en C₁₋₆ substitué par un groupe cyano, un groupe alcényle en C₂₋₆ substitué par un groupe cyano, un groupe alcynyle en C₂₋₆ substitué par un groupe cyano, un groupe alkyle en C₁₋₆ substitué par un groupe nitro, un groupe alcényle en C₂₋₆ substitué par un groupe nitro, un groupe alcynyle en C₂₋₆ substitué par un groupe nitro, un groupe alkyle en C₁₋₆ substitué par un groupe alcoxycarbonyle en C₂₋₇, un groupe alcényle en C₂₋₆ substitué par un groupe alcoxycarbonyle en C₂₋₇, un groupe alcynyle en C₂₋₆ substitué par un groupe alcoxycarbonyle en C₂₋₇, un groupe alkyle en C₁₋₆ substitué par un groupe alkylcarbonyle en C₂₋₇, un groupe alkyle en C₁₋₆ substitué par un groupe mono-, di- ou poly-halogénoalkylcarbonyle en C₂₋₇, un groupe alkyle en C₁₋₆ substitué par un groupe alcénylcarbonyle en C₃₋₇, un groupe alkyle en C₁₋₆ substitué par un groupe alcynylcarbonyle en C₃₋₇, un groupe alkyle en C₁₋₆ substitué par un groupe alkylcarbonyle en C₂₋₅ substitué par un groupe alcoxy en C₁₋₄, un groupe alkyle en C₁₋₆ substitué par un groupe alkylcarbonyle en C₂₋₅ substitué par un groupe alkylthio en C₁₋₄, un groupe alkyle en C₁₋₆ substitué par un groupe alkylcarbonyle en C₂₋₅ substitué par un groupe alkylsulfinyle en C₁₋₄, un groupe alkyle en C₁₋₆ substitué par un groupe alkylcarbonyle en C₂₋₅ substitué par un groupe alkylsulfonyle en C₁₋₄, un groupe alcényle en C₂₋₆ substitué par un groupe alkylcarbonyle en C₂₋₇, un groupe alcynyle en C₂₋₆ substitué par un groupe alkylcarbonyle en C₂₋₇, un groupe alkyle en C₁₋₆ substitué par un groupe alkylsulfamoyle en C₁₋₆, un groupe alkyle en C₁₋₆ substitué par un groupe alcoxysulfamoyle en C₁₋₆, un groupe alkyle en C₁₋₆ substitué par un groupe di(alkyl en C₁₋₃)sulfamoyle, un groupe alkyle en C₁₋₆ substitué par un groupe N-(alkyl en C₁₋₃)-N-(alcoxy en C₁₋₃)sulfamoyle, un groupe alkyle en C₁₋₆ substitué par un groupe alkylcarbamoyle en C₂₋₇, un groupe alkyle en C₁₋₆ substitué par un groupe di(alkyl en C₁₋₃)carbamoyle, un groupe alkyle en C₁₋₆ substitué par un groupe alcoxycarbamoyle en C₂₋₇, un groupe alkyle en C₁₋₆ substitué par un groupe N-(alkyl en C₁₋₃)-N-(alcoxy en C₁₋₃)carbamoyle, un groupe alkyle en C₁₋₆ substitué par un groupe alkylamino en C₁₋₆, un groupe alkyle en C₁₋₆ substitué par un groupe alcoxyamino en C₁₋₆, un groupe alkyle en C₁₋₆ substitué par un groupe di(alkyl en C₁₋₃)amino, un groupe alkyle en C₁₋₆ substitué par un groupe N-(alkyl en C₁₋₃)-N-(alcoxy en C₁₋₃)amino, un groupe alkyle en C₁₋₆ substitué par un groupe N-(alkylcarbonyl en C₂₋₇)-N-(alkyl en C₁₋₆)amino, un groupe alkyle en C₁₋₆ substitué par un groupe N-(alkylcarbonyl en C₂₋₇)-N-(alcoxy en C₁₋₆)amino, un groupe alkyle en C₁₋₆ substitué par un groupe N-(alkylsulfonyl en C₁₋₆)-N-(alkyl en C₁₋₆)amino, un groupe alkyle en C₁₋₆ substitué par un groupe N-(alkylsulfonyl en C₁₋₆)-N-(alcoxy en C₁₋₆)amino, un groupe phényle (à la condition qu'un tel groupe phényle puisse être substitué par un ou plusieurs substituants choisis dans le groupe constitué par un atome d'halogène, un groupe trifluorométhyle, un groupe nitro, un groupe alkyle en C₁₋₆, un groupe alcoxy en C₁₋₆ et un groupe alcoxycarbonyle en C₂₋₇), un groupe alkyle en C₁₋₆ substitué par un groupe phényle (à la condition qu'un tel groupe phényle puisse être substitué par un ou plusieurs substituants choisis dans le groupe constitué par un atome d'halogène, un groupe trifluorométhyle, un groupe nitro, un groupe alkyle en C₁₋₆, un groupe alcoxy en C₁₋₆ et un groupe alcoxycarbonyle en C₂₋₇), un groupe alcényle en C₂₋₇ substitué par un groupe phényle (à la condition qu'un tel groupe phényle puisse être substitué par un ou plusieurs substituants choisis dans le groupe constitué par un atome d'halogène, un groupe trifluorométhyle, un groupe nitro, un groupe alkyle en C₁₋₆, un groupe alcoxy en C₁₋₆ et un groupe alcoxycarbonyle en C₂₋₇), un groupe alcynyle en C₂₋₆ substitué par un groupe phényle (à la condition qu'un tel groupe phényle puisse être substitué par un ou plusieurs substituants choisis dans le groupe constitué par un atome d'halogène, un groupe trifluorométhyle, un groupe nitro, un groupe alkyle en C₁₋₆, un groupe alcoxy en C₁₋₆ et un groupe alcoxycarbonyle en C₂₋₇), un groupe alkyle en C₁₋₆ substitué par un groupe phénoxy (à la condition qu'un tel groupe phénoxy puisse être substitué par un ou plusieurs substituants choisis dans le groupe constitué par un atome d'halogène, un groupe trifluorométhyle, un groupe nitro, un groupe alkyle en C₁₋₆, un groupe alcoxy en C₁₋₆ et un groupe alcoxy-carbonyle en C₂₋₇), un groupe alkyle en C₁-₆ substitué par un groupe phénylthio (à la condition qu'un tel groupe phénylthio puisse être substitué par un ou plusieurs substituants choisis dans le groupe constitué par un atome d'halogène, un groupe trifluorométhyle, un groupe nitro, un groupe alkyle en C₁₋₆, un groupe alcoxy en C₁₋₆ et un groupe alcoxycarbonyle en C₂₋₇), un groupe alkyle en C₁₋₆ substitué par un groupe phénylsulfinyle (à la condition qu'un tel groupe phénylsulfinyle puisse être substitué par un ou plusieurs substituants choisis dans le groupe constitué par un atome d'halogène, un groupe trifluorométhyle, un groupe nitro, un groupe alkyle en C₁₋₆, un groupe alcoxy en C₁₋₆ et un groupe alcoxycarbonyle en C₂₋₇), un groupe alkyle en C₁₋₆ substitué par un groupe phénylsulfonyle (à la condition qu'un tel groupe phénylsulfonyle puisse être substitué par un ou plusieurs substituants choisis dans le groupe constitué par un atome d'halogène, un groupe trifluorométhyle, un groupe nitro, un groupe alkyle en C₁₋₆, un groupe alcoxy en C₁₋₆ et un groupe alcoxy-carbonyle en C₂₋₇), un groupe alkyle en C₁₋₆ substitué par un groupe benzyloxy (à la condition que le groupe phényle d'un tel groupe benzyloxy puisse être substitué par un ou plusieurs substituants choisis dans le groupe constitué par un atome d'halogène, un groupe trifluorométhyle, un groupe nitro, un groupe alkyle en C₁₋₆, un groupe alcoxy en C₁₋₆ et un groupe alcoxy-carbonyle en C₂₋₇), un groupe alkyle en C₁₋₆ substitué par un groupe benzylthio (à la condition que le groupe phényle d'un tel groupe benzylthio puisse être substitué par un ou plusieurs substituants choisis dans le groupe constitué par un atome d'halogène, un groupe trifluorométhyle, un groupe nitro, un groupe alkyle en C₁₋₆, un groupe alcoxy en C₁₋₆ et un groupe alcoxycarbonyle en C₂₋₇), un groupe alkyle en C₁₋₆ substitué par un groupe benzylsulfinyle (à la condition que le groupe phényle d'un tel groupe benzylsulfinyle puisse être substitué par un ou plusieurs substituants choisis dans le groupe constitué par un atome d'halogène, un groupe trifluorométhyle, un groupe nitro, un groupe alkyle en C₁₋₆, un groupe alcoxy en C₁₋₆ et un groupe alcoxycarbonyle en C₂₋₇), un groupe alkyle en C₁₋₆ substitué par un groupe benzylsulfonyle (à la condition que le groupe phényle d'un tel groupe benzylsulfonyle puisse être substitué par un ou plusieurs substituants choisis dans le groupe constitué par un atome d'halogène, un groupe trifluorométhyle, un groupe nitro, un groupe alkyle en C₁₋₆, un groupe alcoxy en C₁₋₆ et un groupe alcoxycarbonyle en C₂₋₇), un groupe alkyle en C₁₋₆ substitué par un groupe phénylcarbonyle (à la condition qu'un tel groupe phénylcarbonyle puisse être substitué par un ou plusieurs substituants choisis dans le groupe constitué par un atome d'halogène, un groupe trifluorométhyle, un groupe nitro, un groupe alkyle en C₁₋₆, un groupe alcoxy en C₁₋₆ et un groupe alcoxycarbonyle en C₂₋₇), un groupe alkyle en C₁₋₆ substitué par un groupe benzylcarbonyle (à la condition que le groupe phényle d'un tel groupe benzylcarbonyle puisse être substitué par un ou plusieurs substituants choisis dans le groupe constitué par un atome d'halogène, un groupe trifluorométhyle, un groupe nitro, un groupe alkyle en C₁₋₆, un groupe alcoxy en C₁₋₆ et un groupe alcoxycarbonyle en C₂₋₇), un groupe alkyle en C₁₋₆ substitué par un groupe amino substitué par un groupe alkylsulfonyle en C₁₋₄, ou un groupe alkyle en C₁₋₆ substitué par un groupe amino substitué par un groupe alkylcarbonyle en C₂₋₄ ;
- R^{f2} est un groupe alkyle en C₁₋₈, un groupe alcényle en C₂₋₈, un groupe alcynyle en C₂₋₈, un groupe alkyle en C₁₋₆ substitué par un groupe alkylthio en C₁₋₆, un groupe alkyle en C₁₋₆ substitué par un groupe alkylsulfinyle en C₁₋₆, un groupe alkyle en C₁₋₆ substitué par un groupe alkylsulfonyle en C₁₋₆, un groupe alkyle en C₁₋₆ substitué par un groupe alcoxy en C₁₋₆, un groupe alkyle en C₁₋₆ substitué par un groupe alcoxycarbonyle en C₂₋₇, un groupe alkyle en C₁₋₆ substitué par un groupe alkylcarbonyle en C₂₋₇, un groupe alkyle en C₁₋₆ substitué par un groupe cyano, un groupe phényle (à la condition qu'un tel groupe phényle puisse être substitué par un ou plusieurs substituants choisis dans le groupe constitué par un atome d'halogène, un groupe trifluorométhyle, un groupe nitro, un groupe alkyle en C₁₋₆, un groupe alcoxy en C₁₋₆ et un groupe alcoxycarbonyle en C₂₋₇), ou un groupe alkyle en C₁₋₆ substitué par un groupe phényle (à la condition qu'un tel groupe phényle puisse être substitué par un ou plusieurs substituants choisis dans le groupe constitué par un atome d'halogène, un groupe trifluorométhyle, un groupe nitro, un groupe alkyle en C₁₋₆, un groupe alcoxy en C₁₋₆ et un groupe alcoxycarbonyle en C₂₋₇) ;
- R^{f3}, R^{f4}, R^{f7}, R^{f8}, R^{f11} et R^{f12}, qui sont indépendants l'un de l'autre, représentent chacun un atome d'hydrogène, un groupe alkyle en C₁₋₆, un groupe mono-, di- ou poly-halogénoalkyle en C₁₋₆, un groupe alcoxy en C₁₋₆, un groupe mono-, di- ou poly-halogénoalcoxy en C₁₋₆, un groupe alkylthio en C₁₋₆, un groupe alkylsulfinyle en C₁₋₆, un groupe alkylsulfonyle en C₁₋₆, un groupe alcoxycarbonyle en C₂₋₇, un groupe alkylcarbonyle en C₂₋₇, un atome d'halogène, un groupe nitro, un groupe cyano, ou un groupe phényle (à la condition qu'un tel groupe phényle puisse être substitué par un ou plusieurs substituants choisis dans le groupe constitué par un atome d'halogène, un groupe trifluorométhyle, un groupe nitro, un groupe alkyle en C₁₋₆, un groupe alcoxy en C₁₋₆ et un groupe alcoxycarbonyle en C₂₋₇) ;
- R^{f5}, R^{f6}, R^{f9}, R^{f10}, R^{f13}, R^{f14} et R^{f15}, qui sont indépendants l'un de l'autre, représentent chacun un atome d'hydrogène, un groupe alkyle en C₁₋₈, un groupe alcényle en C₂₋₈, un groupe alcynyle en C₂₋₈, ou un groupe phényle (à la condition qu'un tel groupe phényle puisse être substitué par un ou plusieurs substituants choisis dans le groupe constitué par un atome d'halogène, un groupe trifluorométhyle, un groupe nitro, un groupe alkyle en C₁₋₆, un groupe alcoxy en C₁₋₆ et un groupe alcoxycarbonyle en C₂₋₇) ;
- R^{g1} est un groupe alkyle en C₁₋₈, un groupe cycloalkyle en C₃₋₇, un groupe alkyle en C₁₋₆ substitué par un groupe cycloalkyle en C₃₋₇, un groupe cycloalcényle en C₃₋₇, un groupe alkyle en C₁₋₆ substitué par un groupe cycloalcényle en C₃₋₇, un groupe alcényle en C₂₋₈, un groupe alcynyle en C₂₋₈, un groupe alkyle en C₁₋₆ substitué par un groupe alcoxy en C₁₋₆, un groupe alkyle en C₁₋₆ substitué par un groupe alcényloxy en C₂₋₆, un groupe alkyle en C₁₋₆ substitué par un groupe alcynyloxy en C₂₋₆, un groupe alkyle en C₁₋₆ substitué par un groupe mono-, di- ou poly-halogénoalcoxy en C₁₋₆, un groupe alkyle en C₁₋₆ substitué par un groupe mono-, di- ou poly-halogénoalcényloxy en C₂₋₆, un groupe alkyle en C₁₋₆ substitué par un groupe mono-, di- ou poly-halogénoalcynyloxy en C₂₋₆, un groupe alkyle en C₁₋₆ substitué par un groupe alkylthio en C₁₋₆, un groupe alkyle en C₁₋₆ substitué par un groupe alkylsulfinyle en C₁₋₆, un groupe alkyle en C₁₋₆ substitué par un groupe alkylsulfonyle en C₁₋₆, un groupe mono-, di- ou poly-halogénoalkyle en C₁₋₈, un groupe mono-, di- ou poly-halogénoalcényle en C₂₋₈, un groupe mono-, di- ou poly-halogénoalcynyle en C₂₋₈, un groupe alkyle en C₁₋₆ substitué par un groupe cyano, un groupe alcényle en C₂₋₆ substitué par un groupe cyano, un groupe alcynyle en C₂₋₆ substitué par un groupe cyano, un groupe alkyle en C₁₋₆ substitué par un groupe nitro, un groupe alcényle en C₂₋₆ substitué par un groupe nitro, un groupe alcynyle en C₂₋₆ substitué par un groupe nitro, un groupe alkyle en C₁₋₆ substitué par un groupe alcoxycarbonyle en C₂₋₇, un groupe alcényle en C₂₋₆ substitué par un groupe alcoxycarbonyle en C₂₋₇, un groupe alcynyle en C₂₋₆ substitué par un groupe alcoxycarbonyle en C₂₋₇, un groupe alkyle en C₁₋₆ substitué par un groupe alkylcarbonyle en C₂₋₇, un groupe alkyle en C₁₋₆ substitué par un groupe mono-, di- ou poly-halogénoalkylcarbonyle en C₂₋₇, un groupe alkyle en C₁₋₆ substitué par un groupe alcénylcarbonyle en C₃₋₇, un groupe alkyle en C₁₋₆ substitué par un groupe alcynylcarbonyle en C₃₋₇, un groupe alkyle en C₁₋₆ substitué par un groupe alkylcarbonyle en C₂₋₅ substitué par un groupe alcoxy en C₁₋₄, un groupe alkyle en C₁₋₆ substitué par un groupe alkylcarbonyle en C₂₋₅ substitué par un groupe alkylthio en C₁₋₄, un groupe alkyle en C₁₋₆ substitué par un groupe alkylcarbonyle en C₂₋₅ substitué par un groupe alkylsulfinyle en C₁₋₄, un groupe alkyle en C₁₋₆ substitué par un groupe alkylcarbonyle en C₂₋₅ substitué par un groupe alkylsulfonyle en C₁₋₄, un groupe alcényle en C₂₋₆ substitué par un groupe alkylcarbonyle en C₂₋₇, un groupe alcynyle en C₂₋₆ substitué par un groupe alkylcarbonyle en C₂₋₇, un groupe alkyle en C₁₋₆ substitué par un groupe alkylsulfamoyle en C₁₋₆, un groupe alkyle en C₁₋₆ substitué par un groupe alcoxysulfamoyle en C₁₋₆, un groupe alkyle en C₁₋₆ substitué par un groupe di(alkyl en C₁₋₃)sulfamoyle, un groupe alkyle en C₁₋₆ substitué par un groupe N-(alkyl en C₁₋₃)-N-(alcoxy en C₁₋₃)sulfamoyle, un groupe alkyle en C₁₋₆ substitué par un groupe alkylcarbamoyle en C₂₋₇, un groupe alkyle en C₁₋₆ substitué par un groupe di(alkyl en C₁₋₃)carbamoyle, un groupe alkyle en C₁₋₆ substitué par un groupe alcoxycarbamoyle en C₂₋₇, un groupe alkyle en C₁₋₆ substitué par un groupe N-(alkyl en C₁₋₃)-N-(alcoxy en C₁₋₃)carbamoyle, un groupe alkyle en C₁₋₆ substitué par un groupe alkylamino en C₁₋₆, un groupe alkyle en C₁₋₆ substitué par un groupe alcoxyamino en C₁₋₆, un groupe alkyle en C₁₋₆ substitué par un groupe di(alkyl en C₁₋₃)amino, un groupe alkyle en C₁₋₆ substitué par un groupe N-(alkyl en C₁₋₃)-N-(alcoxy en C₁₋₃)amino, un groupe alkyle en C₁₋₆ substitué par un groupe N-(alkylcarbonyl en C₂₋₇)-N-(alkyl en C₁₋₆)amino, un groupe alkyle en C₁₋₆ substitué par un groupe N-(alkylcarbonyl en C₂₋₇)-N-(alcoxy en C₁₋₆)amino, un groupe alkyle en C₁₋₆ substitué par un groupe N-(alkylsulfonyl en C₁₋₆)-N-(alkyl en C₁₋₆)amino, un groupe alkyle en C₁₋₆ substitué par un groupe N-(alkylsulfonyl en C₁₋₆)-N-(alcoxy en C₁₋₆)amino, un groupe alkyle en C₁₋₆ substitué par un groupe phényle (à la condition qu'un tel groupe phényle puisse être substitué par un ou plusieurs substituants choisis dans le groupe constitué par un atome d'halogène, un groupe trifluorométhyle, un groupe nitro, un groupe alkyle en C₁₋₆, un groupe alcoxy en C₁₋₆ et un groupe alcoxycarbonyle en C₂₋₇), un groupe alcényle en C₂₋₇ substitué par un groupe phényle (à la condition qu'un tel groupe phényle puisse être substitué par un ou plusieurs substituants choisis dans le groupe constitué par un atome d'halogène, un groupe trifluorométhyle, un groupe nitro, un groupe alkyle en C₁₋₆, un groupe alcoxy en C₁₋₆ et un groupe alcoxycarbonyle en C₂₋₇), un groupe alcynyle en C₂₋₆ substitué par un groupe phényle (à la condition qu'un tel groupe phényle puisse être substitué par un ou plusieurs substituants choisis dans le groupe constitué par un atome d'halogène, un groupe trifluorométhyle, un groupe nitro, un groupe alkyle en C₁₋₆, un groupe alcoxy en C₁₋₆ et un groupe alcoxy-carbonyle en C₂₋₇), un groupe alkyle en C₁₋₆ substitué par un groupe phénoxy (à la condition qu'un tel groupe phénoxy puisse être substitué par un ou plusieurs substituants choisis dans le groupe constitué par un atome d'halogène, un groupe trifluorométhyle, un groupe nitro, un groupe alkyle en C₁₋₆, un groupe alcoxy en C₁₋₆ et un groupe alcoxycarbonyle en C₂₋₇), un groupe alkyle en C₁₋₆ substitué par un groupe phénylthio (à la condition qu'un tel groupe phénylthio puisse être substitué par un ou plusieurs substituants choisis dans le groupe constitué par un atome d'halogéne, un groupe trifluorométhyle, un groupe nitro, un groupe alkyle en C₁₋₆, un groupe alcoxy en C₁₋₆ et un groupe alcoxy-carbonyle en C₂₋₇), un groupe alkyle en C₁₋₆ substitué par un groupe phénylsulfinyle (à la condition qu'un tel groupe phénylsulfinyle puisse être substitué par un ou plusieurs substituants choisis dans le groupe constitué par un atome d'halogène, un groupe trifluorométhyle, un groupe nitro, un groupe alkyle en C₁₋₆, un groupe alcoxy en C₁₋₆ et un groupe alcoxycarbonyle en C₂₋₇), un groupe alkyle en C₁₋₆ substitué par un groupe phénylsulfonyle (à la condition qu'un tel groupe phénylsulfonyle puisse être substitué par un ou plusieurs substituants choisis dans le groupe constitué par un atome d'halogène, un groupe trifluorométhyle, un groupe nitro, un groupe alkyle en C₁₋₆, un groupe alcoxy en C₁₋₆ et un groupe alcoxycarbonyle en C₂₋₇), un groupe alkyle en C₁₋₆ substitué par un groupe benzyloxy (à la condition que le groupe phényle d'un tel groupe benzyloxy puisse être substitué par un ou plusieurs substituants choisis dans le groupe constitué par un atome d'halogène, un groupe trifluorométhyle, un groupe nitro, un groupe alkyle en C₁₋₆, un groupe alcoxy en C₁₋₆ et un groupe alcoxycarbonyle en C₂₋₇), un groupe alkyle en C₁₋₆ substitué par un groupe benzylthio (à la condition que le groupe phényle d'un tel groupe benzylthio puisse être substitué par un ou plusieurs substituants choisis dans le groupe constitué par un atome d'halogène, un groupe trifluorométhyle, un groupe nitro, un groupe alkyle en C₁₋₆, un groupe alcoxy en C₁₋₆ et un groupe alcoxycarbonyle en C₂₋₇), un groupe alkyle en C₁₋₆ substitué par un groupe benzylsulfinyle (à la condition que le groupe phényle d'un tel groupe benzylsulfinyle puisse être substitué par un ou plusieurs substituants choisis dans le groupe constitué par un atome d'halogène, un groupe trifluorométhyle, un groupe nitro, un groupe alkyle en C₁₋₆, un groupe alcoxy en C₁₋₆ et un groupe alcoxycarbonyle en C₂₋₇), un groupe alkyle en C₁₋₆ substitué par un groupe benzylsulfonyle (à la condition que le groupe phényle d'un tel groupe benzylsulfonyle puisse être substitué par un ou plusieurs substituants choisis dans le groupe constitué par un atome d'halogène, un groupe trifluorométhyle, un groupe nitro, un groupe alkyle en C₁₋₆, un groupe alcoxy en C₁₋₆ et un groupe alcoxycarbonyle en C₂₋₇), un groupe alkyle en C₁₋₆ substitué par un groupe phénylcarbonyle (à la condition qu'un tel groupe phénylcarbonyle puisse être substitué par un ou plusieurs substituants choisis dans le groupe constitué par un atome d'halogène, un groupe trifluorométhyle, un groupe nitro, un groupe alkyle en C₁₋₆, un groupe alcoxy en C₁₋₆ et un groupe alcoxycarbonyle en C₂₋₇), ou un groupe alkyle en C₁₋₆ substitué par un groupe benzylcarbonyle (à la condition que le groupe phényle d'un tel groupe benzylcarbonyle puisse être substitué par un ou plusieurs substituants choisis dans le groupe constitué par un atome d'halogène, un groupe trifluorométhyle, un groupe nitro, un groupe alkyle en C₁₋₆, un groupe alcoxy en C₁₋₆ et un groupe alcoxycarbonyle en C₂₋₇),
- R^{g2} et R^{g3}, qui sont indépendants l'un de l'autre, représentent chacun un groupe alkyle en C₁₋₆, un groupe alcényle en C₂₋₆, un groupe alcynyle en C₂₋₆, un groupe mono-, di- ou poly-halogénoalkyle en C₁₋₆, un groupe alcoxy en C₁₋₆, un groupe alkylsulfonyle en C₁₋₆, un groupe alkylsulfamoyle en C₁₋₆, un groupe di(alkyl en C₁₋₃)sulfamoyle, un groupe alcoxycarbonyle en C₂₋₇, un groupe alkylcarbonyle en C₂₋₇, un groupe alkylcarbamoyle en C₂₋₇, un groupe di(alkyl en C₁₋₃)carbamoyle, un groupe phényle (à la condition qu'un tel groupe phényle puisse être substitué par un ou plusieurs substituants choisis dans le groupe constitué par un atome d'halogène, un groupe trifluorométhyle, un groupe nitro, un groupe alkyle en C₁₋₆, un groupe alcoxy en C₁₋₆ et un groupe alcoxycarbonyle en C₂₋₇), ou un groupe benzyle (à la condition que le groupe phényle d'un tel groupe benzyle puisse être substitué par un ou plusieurs substituants choisis dans le groupe constitué par un atome d'halogène, un groupe trifluorométhyle, un groupe nitro, un groupe alkyle en C₁₋₆, un groupe alcoxy en C₁₋₆ et un groupe alcoxycarbonyle en C₂₋₇) ; ou
- R^{g2} et R^{g3} forment un groupe hétérocyclique saturé, à 3 à 7 chaînons, conjointement avec l'atome d'azote auxquels ils sont liés ;
- X est un atome d'oxygène ou un atome de soufre ;
- L est un atome d'hydrogène, un groupe alkyle en C₁₋₆, un groupe alcényle en C₂₋₆ ou un groupe alcynyle en C₂₋₆ ;
- G représente : 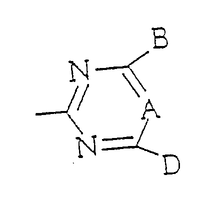
- A est un groupe CH ou un atome d'azote, et
- D et B, qui sont indépendants l'un de l'autre, représentent chacun un groupe alkyle en C₁₋₄, un groupe alcoxy en C₁₋₄, un groupe mono-, di- ou poly-halogénoalkyle en C₁₋₄, un groupe mono-, di- ou poly-halogénoalcoxy en C₁₋₄, un atome d'halogène, un groupe alkylamino en C₁₋₄ ou un groupe di(alkyl en C₁₋₄)amino.

3. Procédé de préparation d'un dérivé d'iminosulfonylurée ou de son sel, selon la revendication 2, dans lequel Q représente :

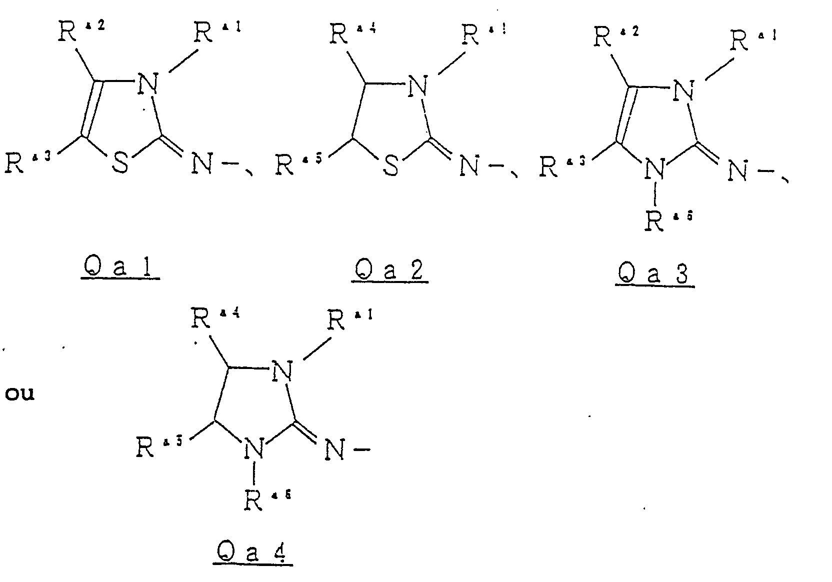

4. Procédé de préparation d'un dérivé d'iminosulfonylurée ou de son sel, selon la revendication 2, dans lequel Q représente :

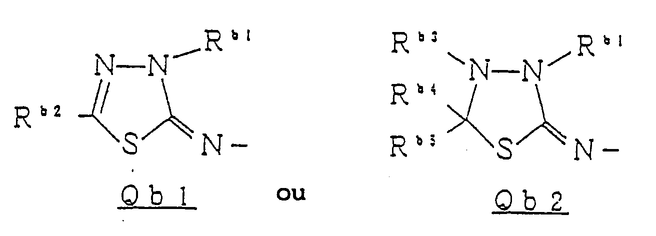

5. Procédé de préparation d'un dérivé d'iminosulfonylurée ou de son sel, selon la revendication 2, dans lequel Q représente :

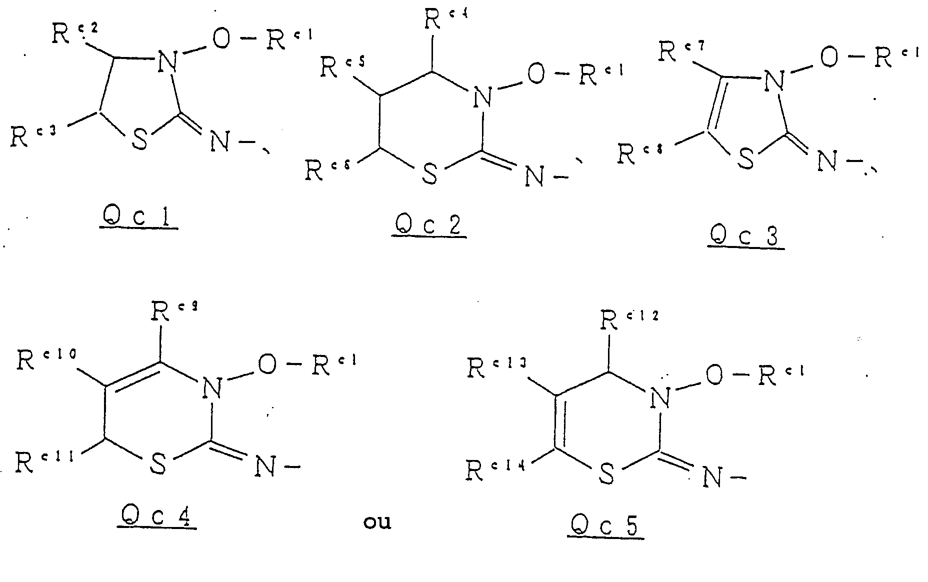

6. Procédé de préparation d'un dérivé d'iminosulfonylurée ou de son sel, selon la revendication 2, dans lequel Q représente :

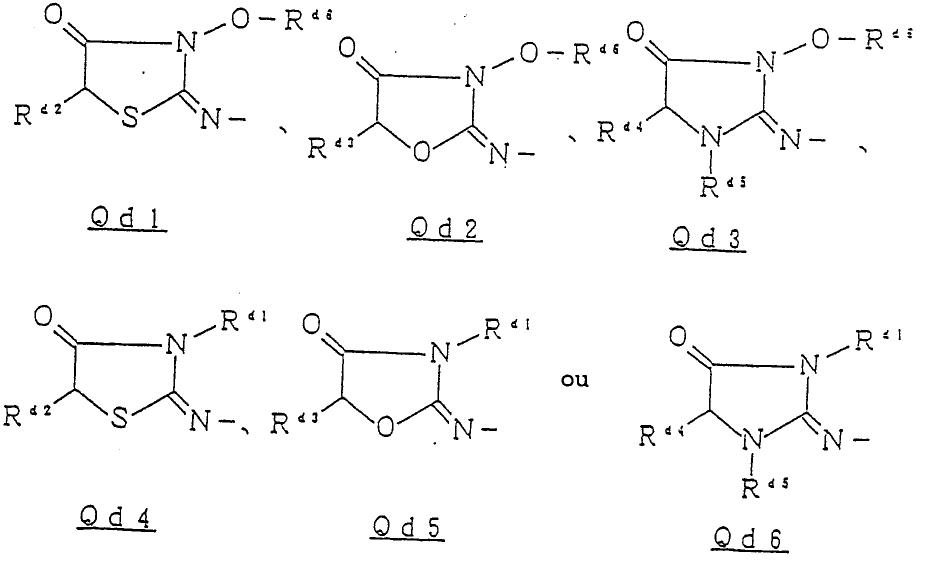

7. Procédé de préparation d'un dérivé d'iminosulfonylurée ou de son sel, selon la revendication 2, dans lequel Q représente :

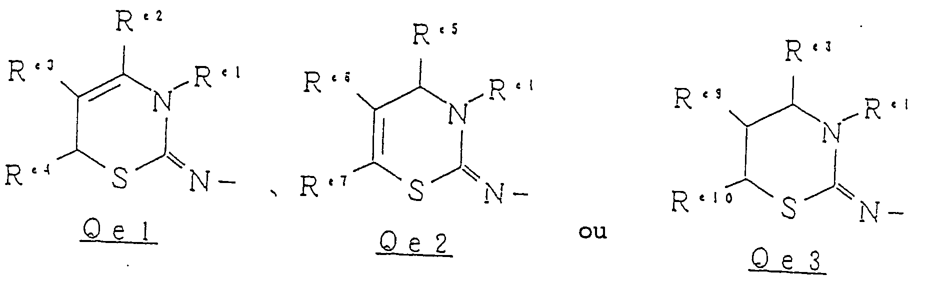

8. Procédé de préparation d'un dérivé d'iminosulfonylurée ou de son sel, selon la revendication 2, dans lequel Q représente :

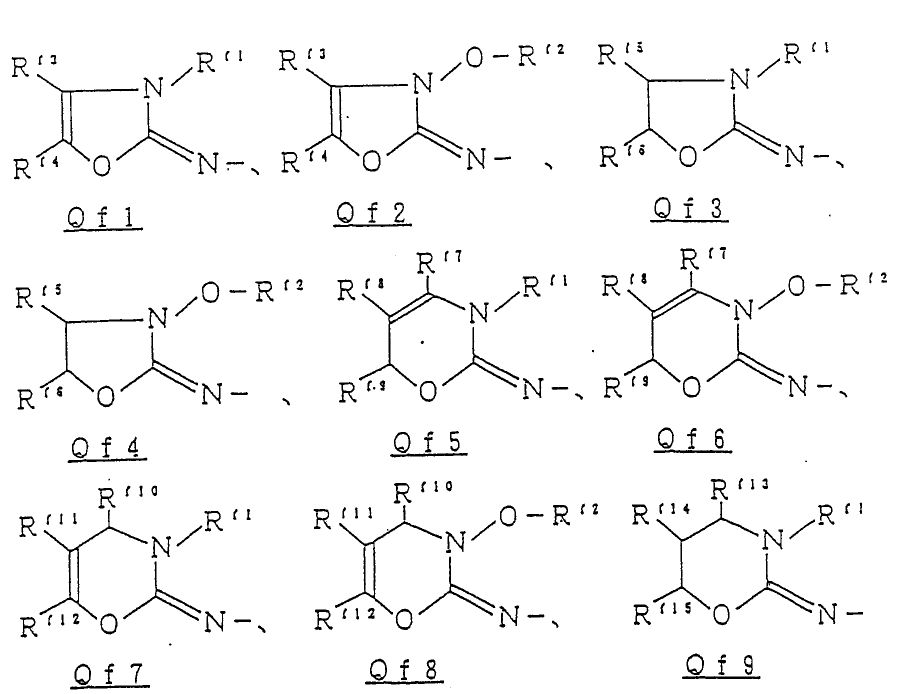

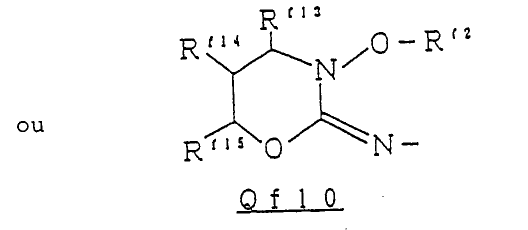

9. Procédé de préparation d'un dérivé d'iminosulfonylurée ou de son sel, selon la revendication 2, dans lequel Q représente :

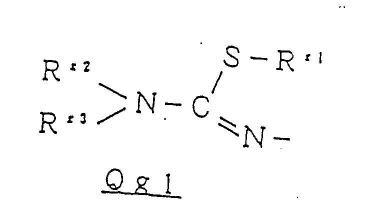

10. Procédé de préparation d'un dérivé d'iminosulfonylurée ou de son sel, selon la revendication 3, dans lequel :
- R^{a1} représente : 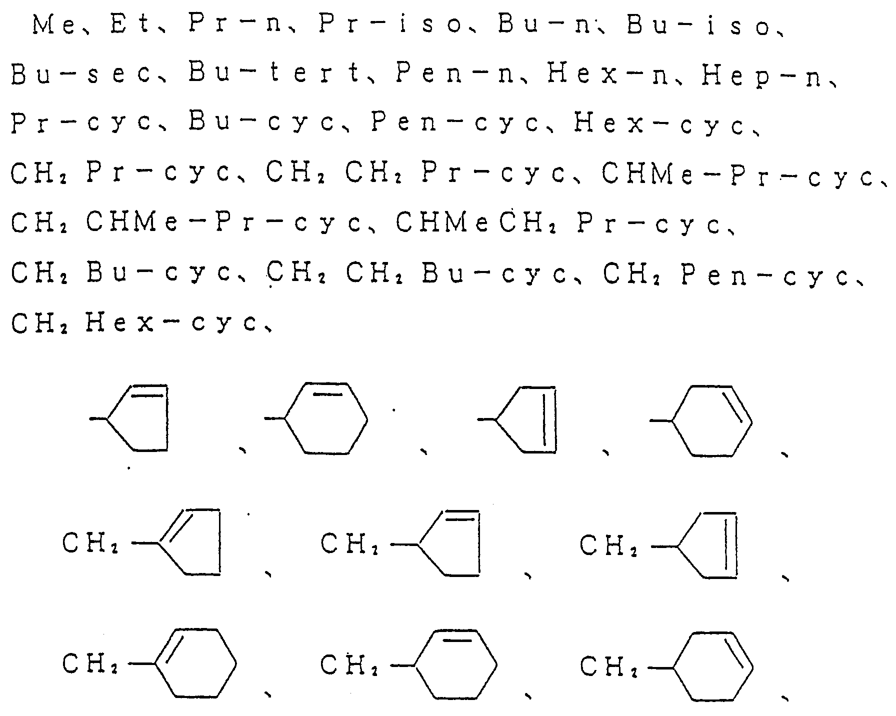 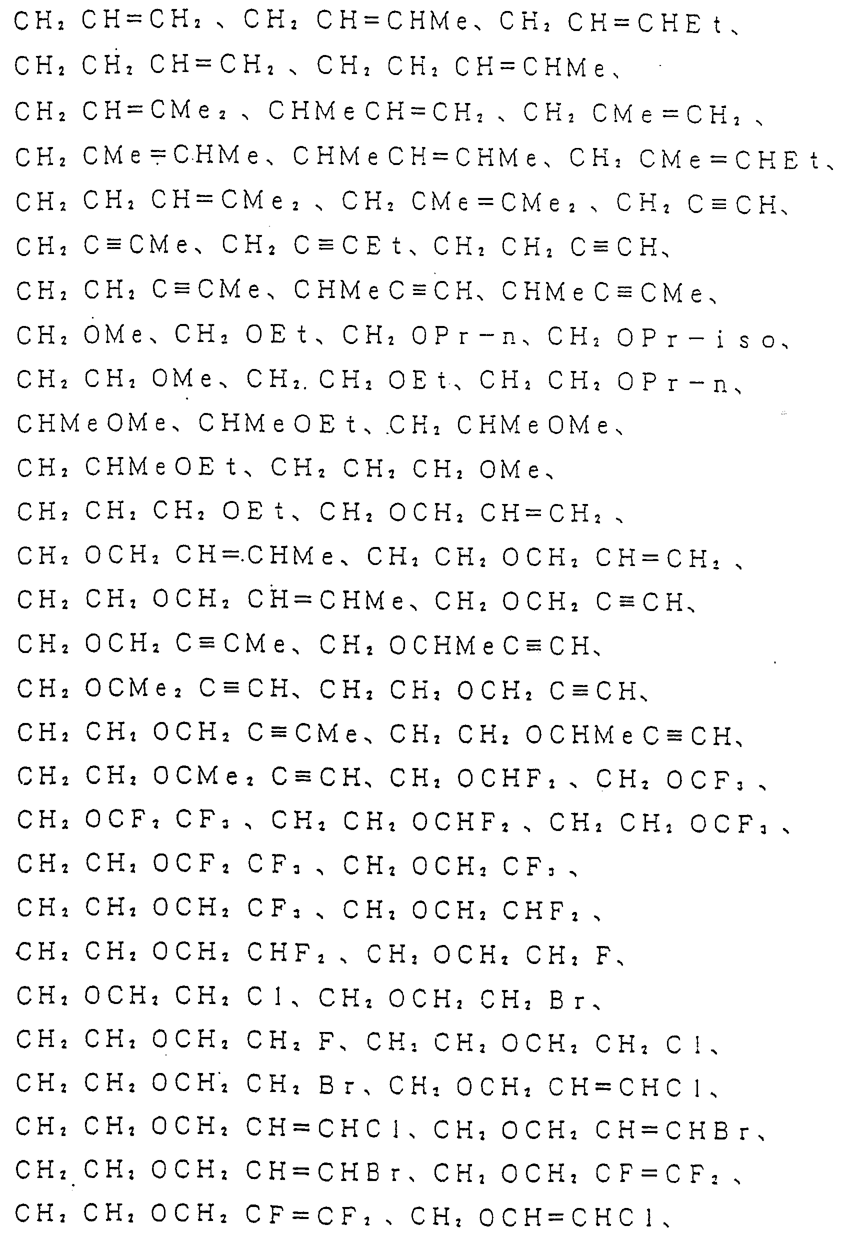 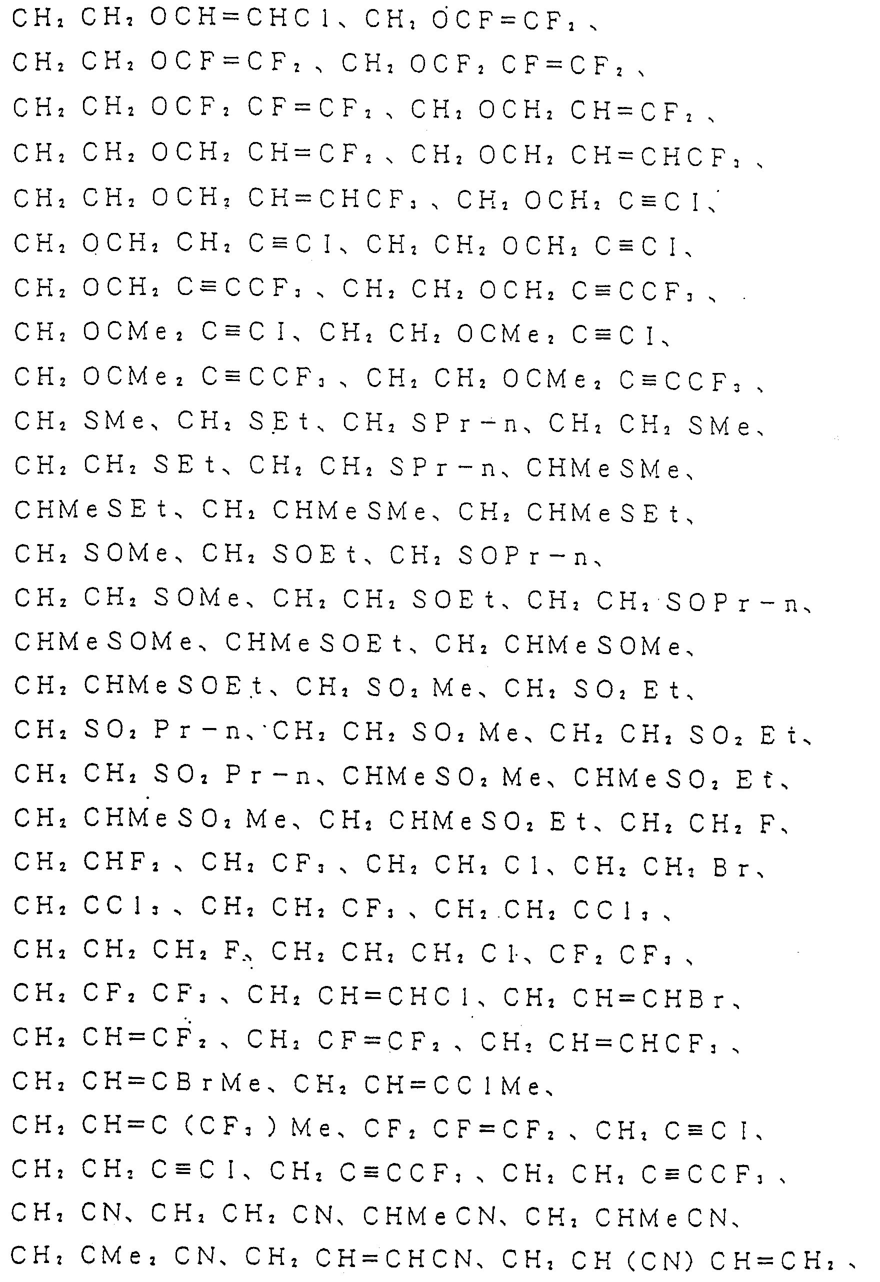 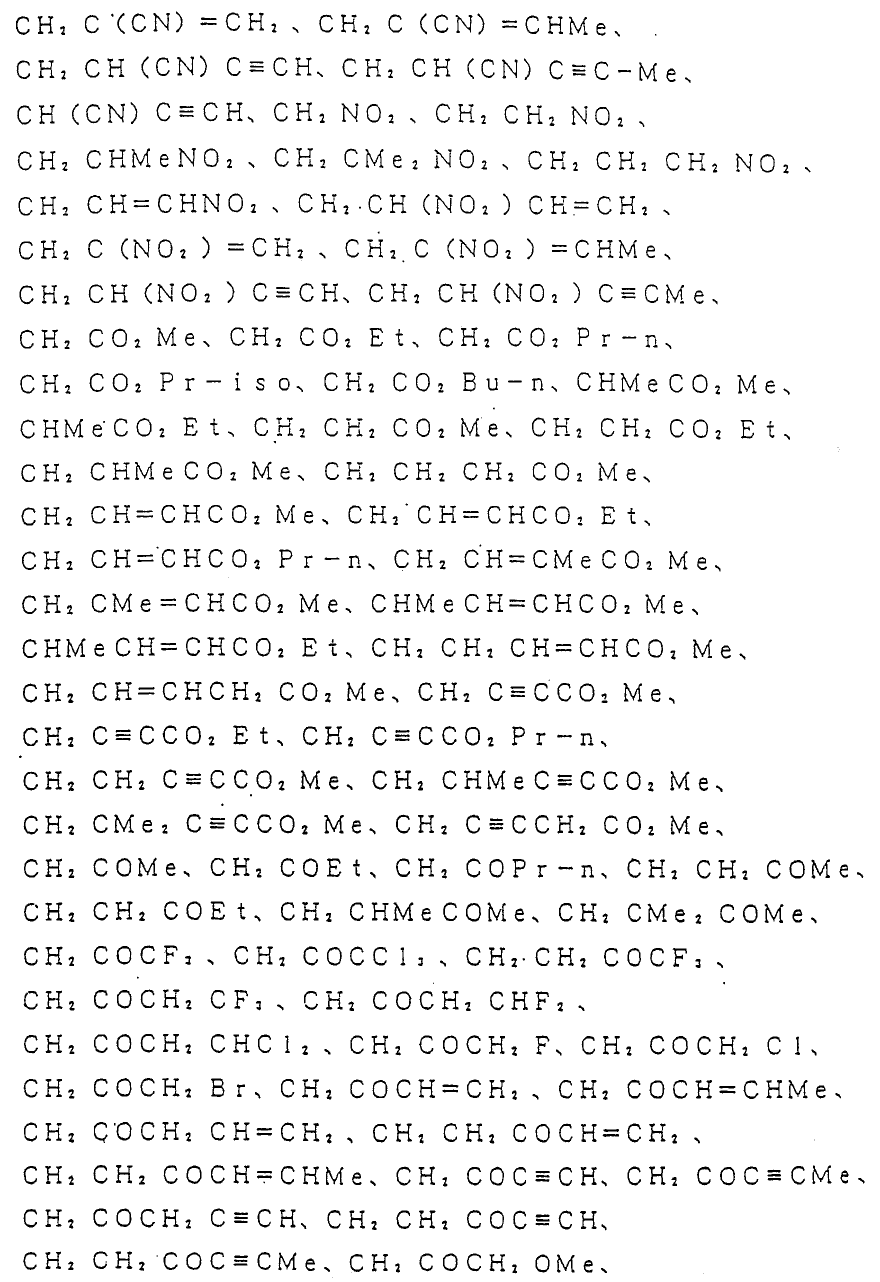 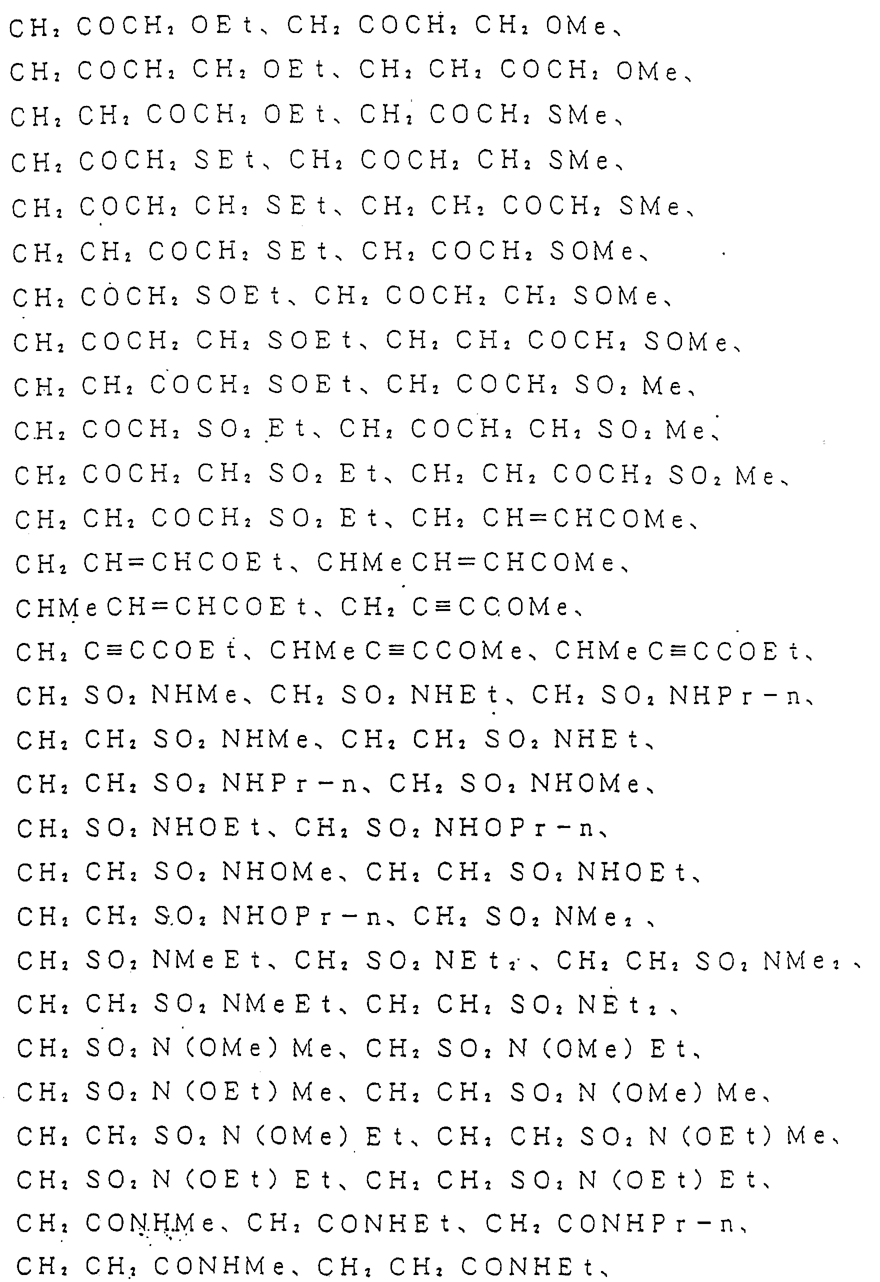 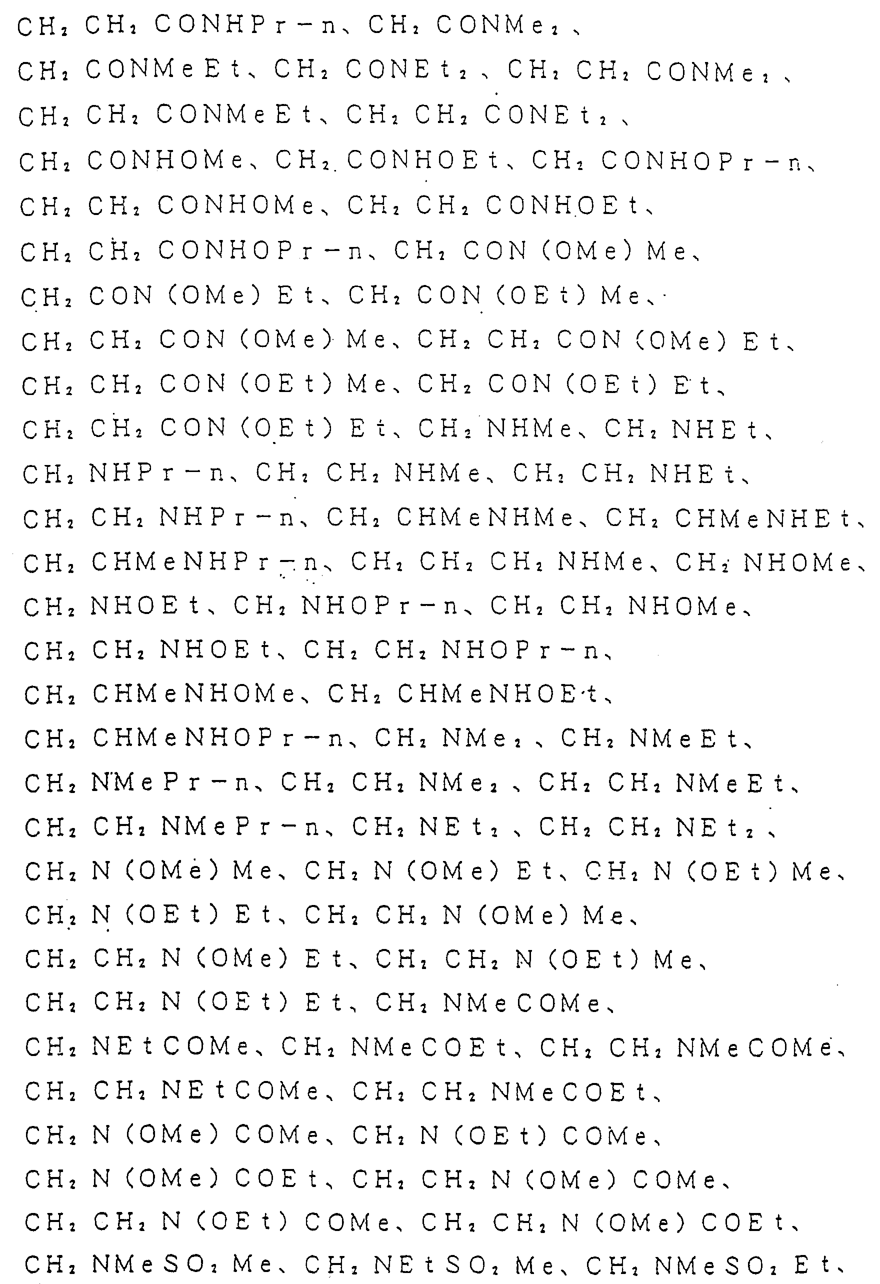 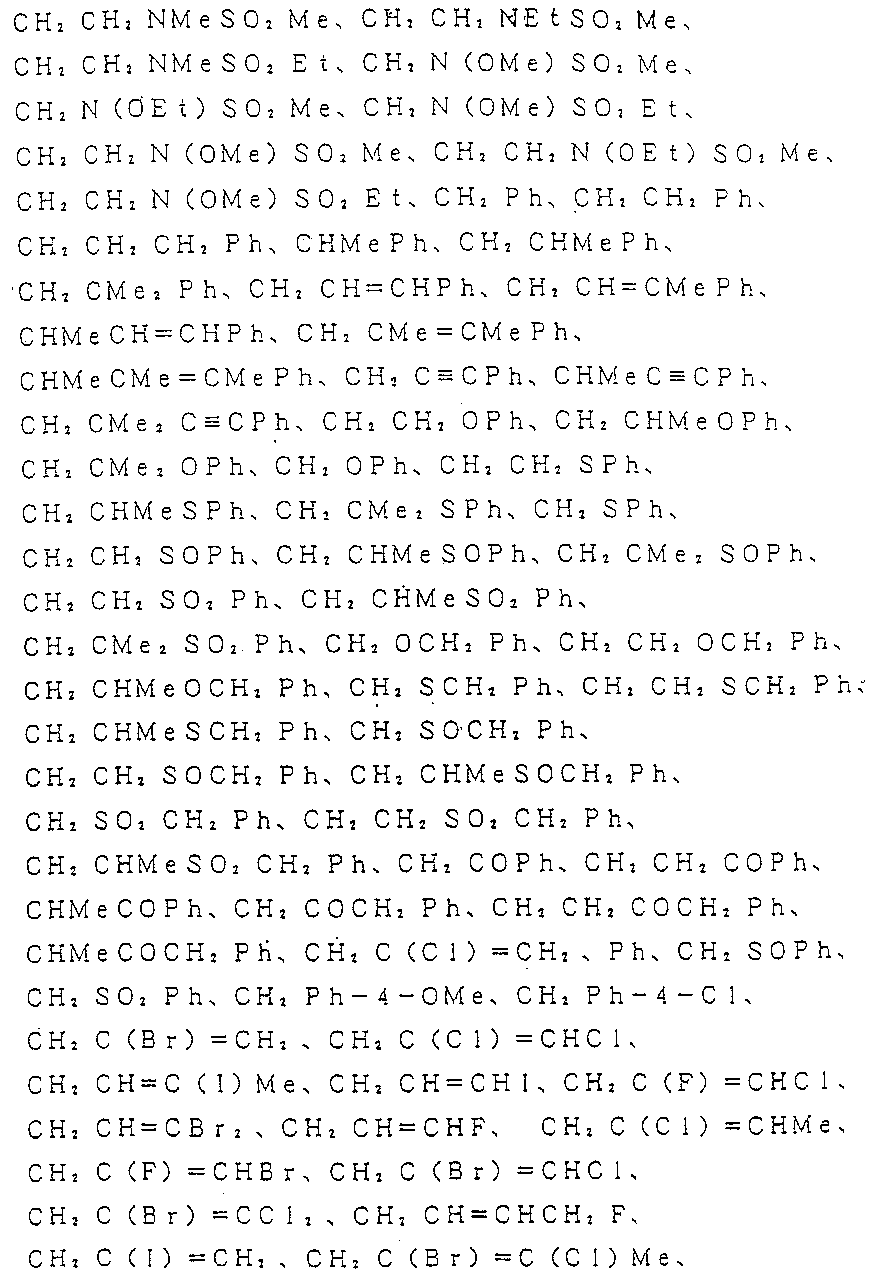 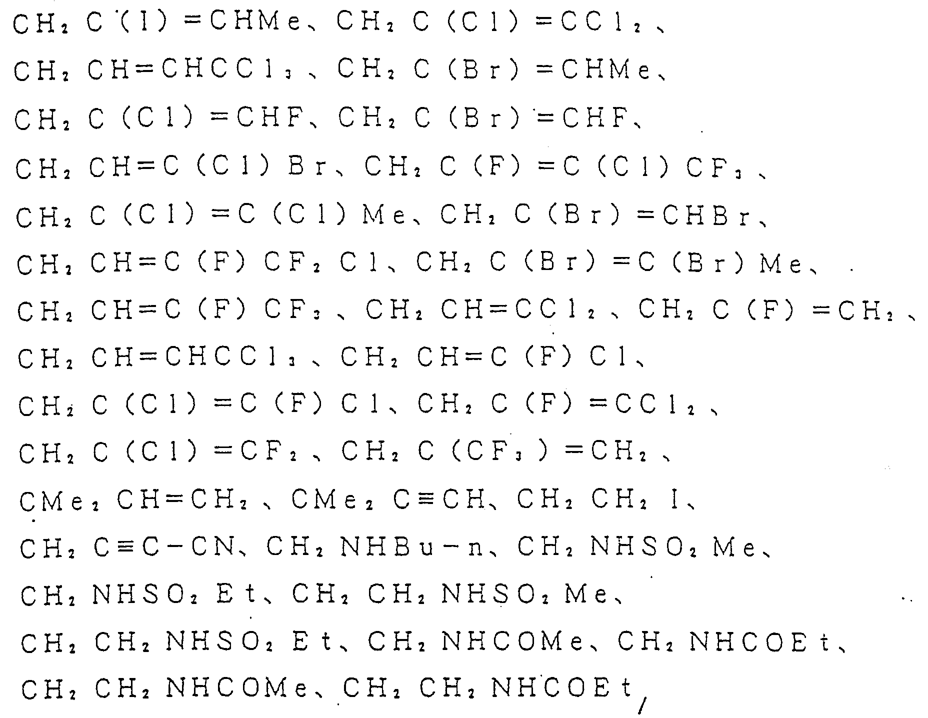
- R^{a2} et R^{a3} représentent : 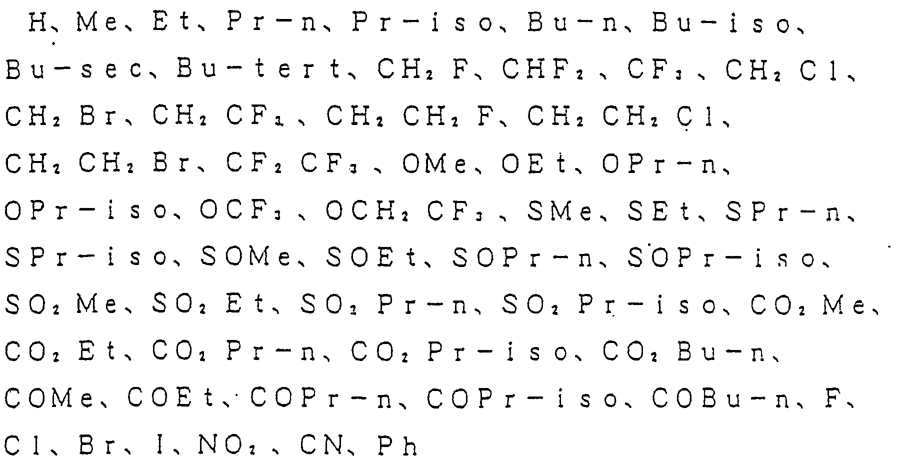
- B et D représentent :

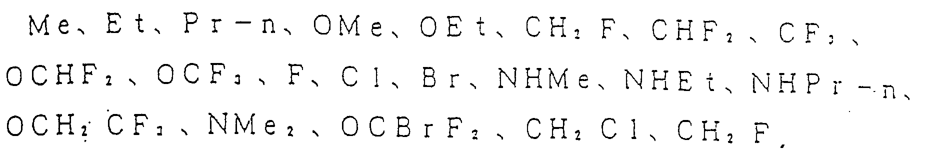

11. Procédé de préparation d'un dérivé d'iminosulfonylurée ou de son sel, selon la revendication 3, dans lequel :
- R^{a1} représente :

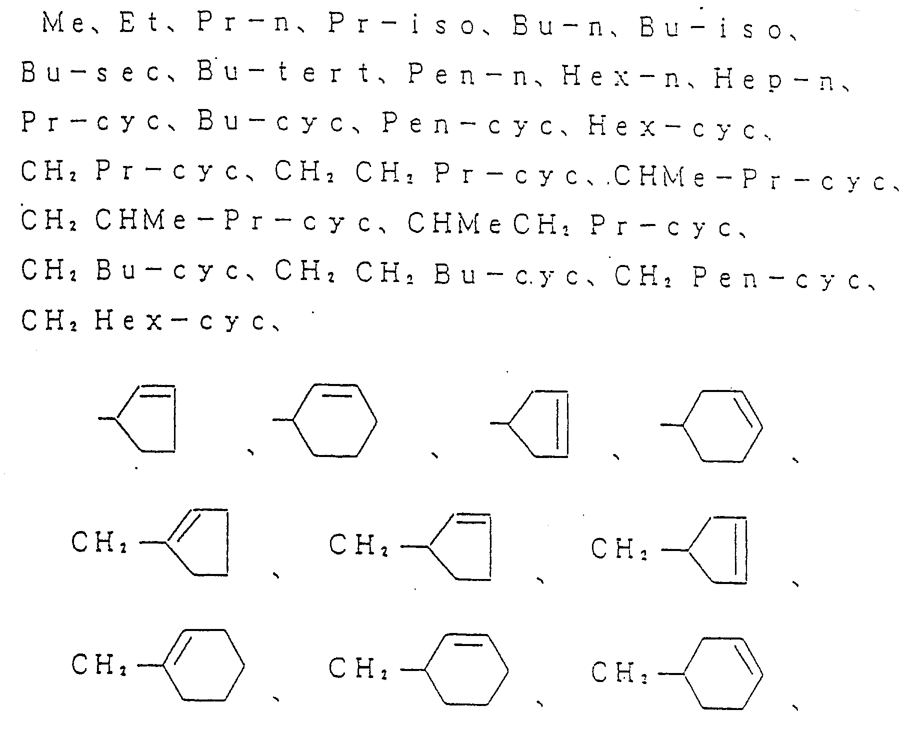

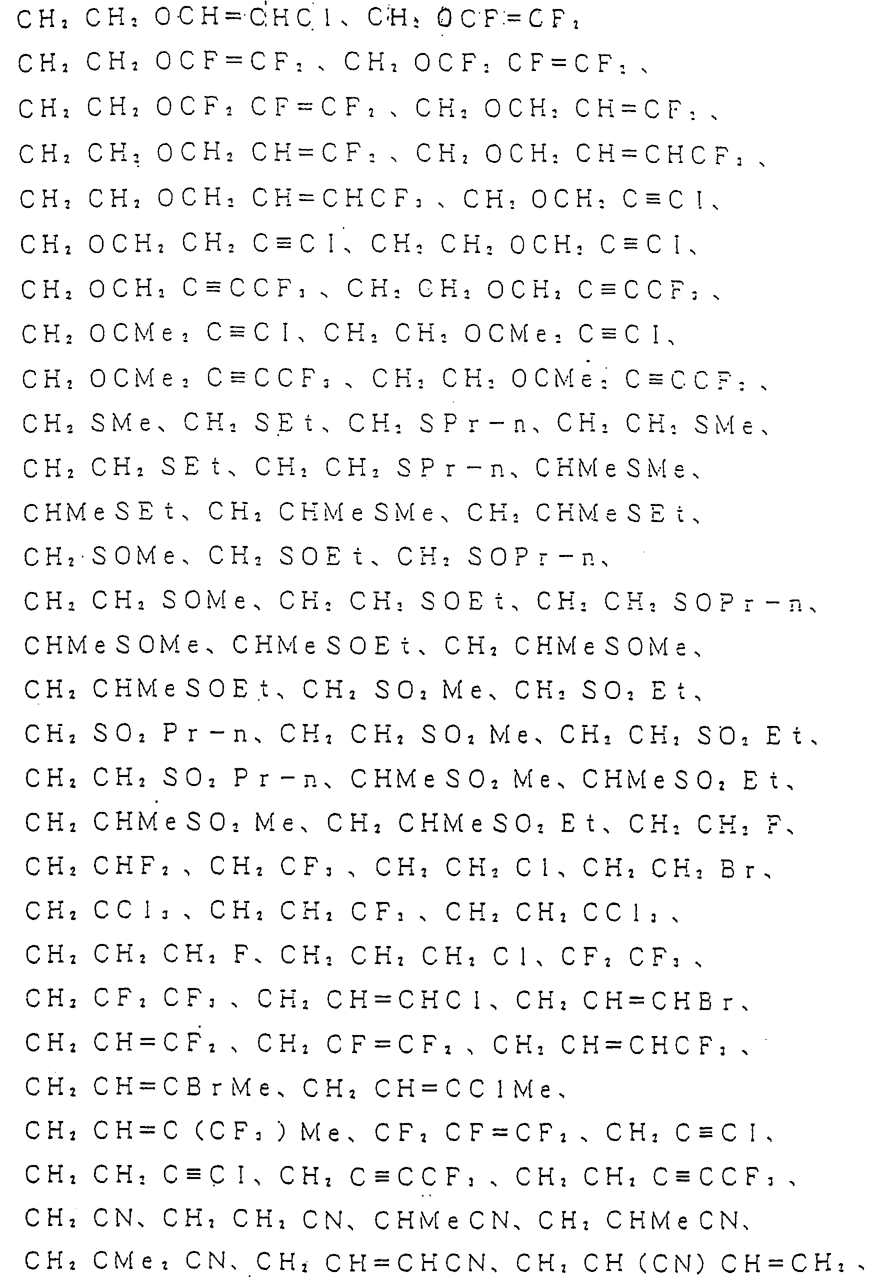

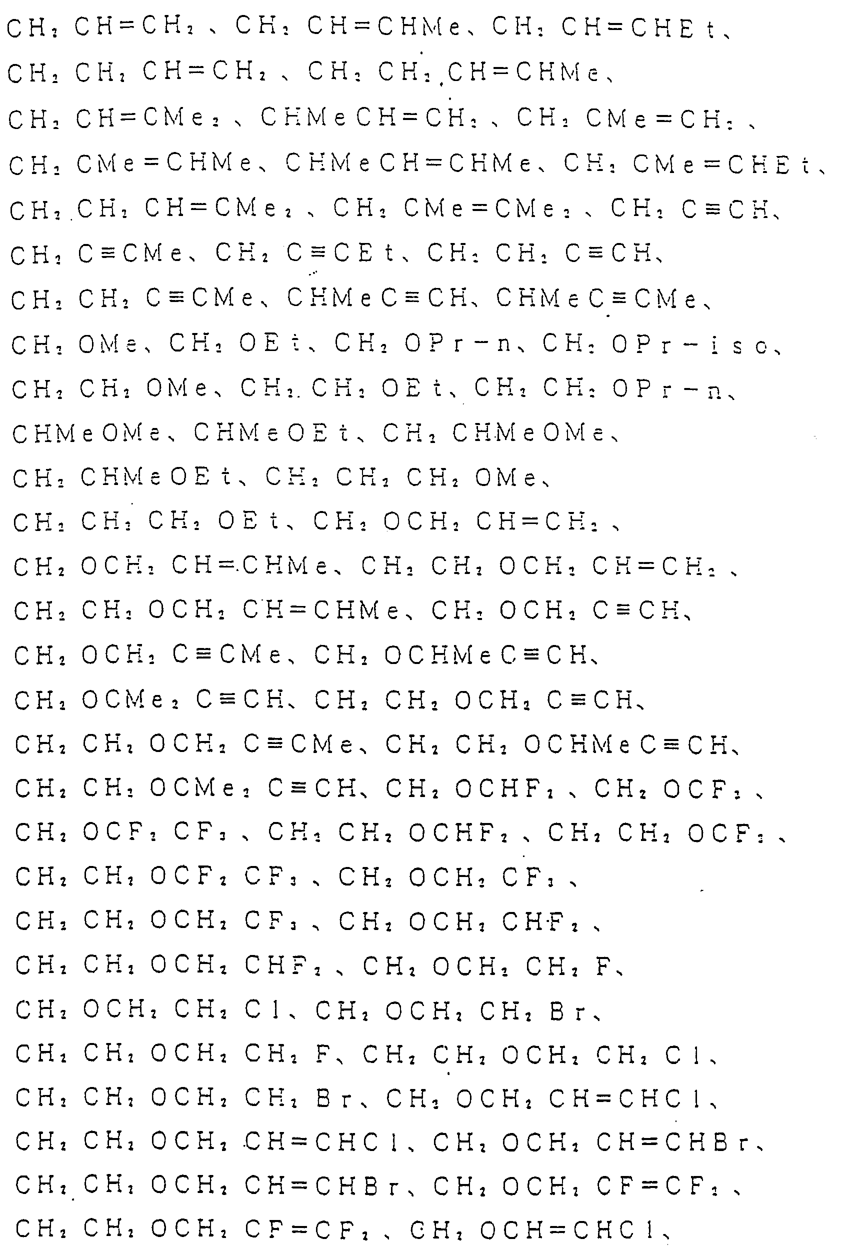

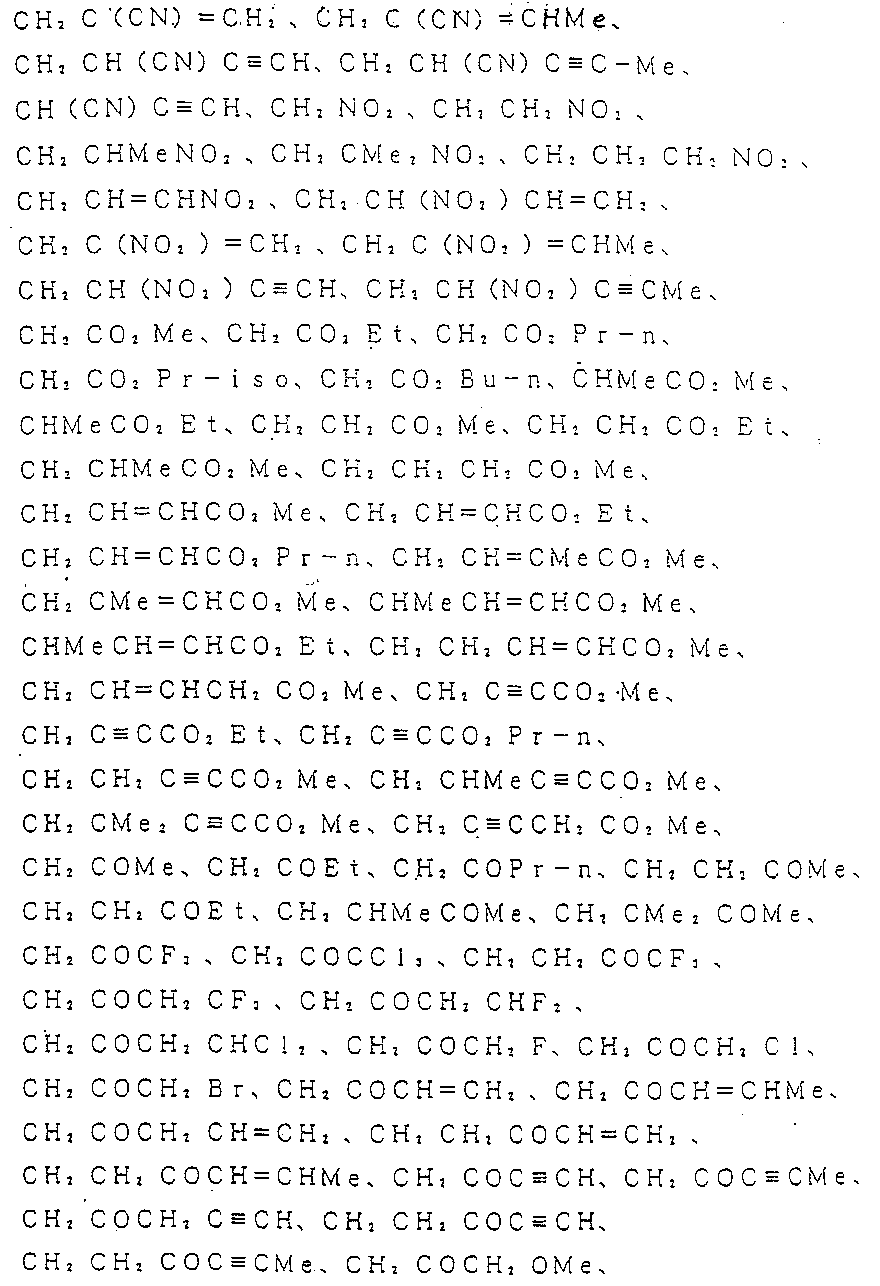

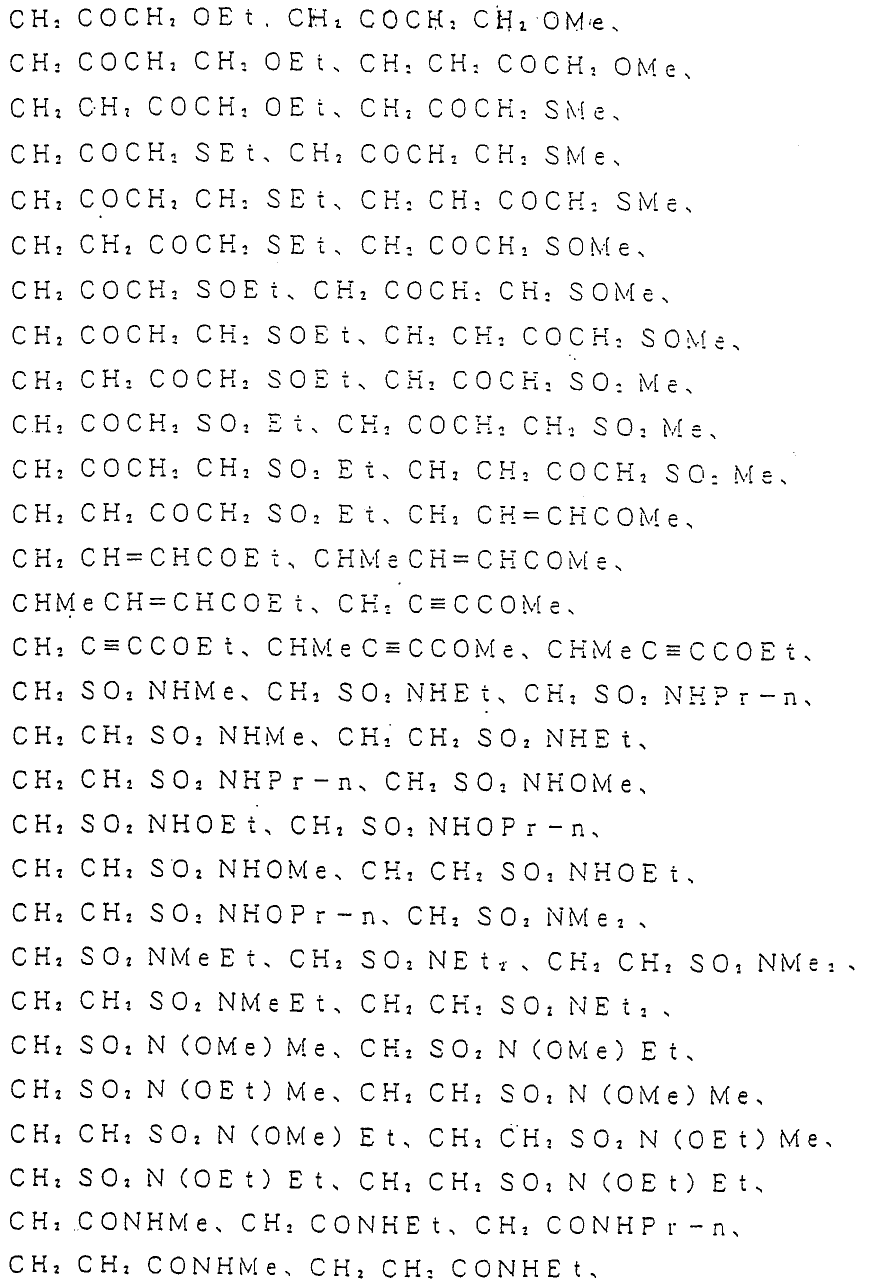

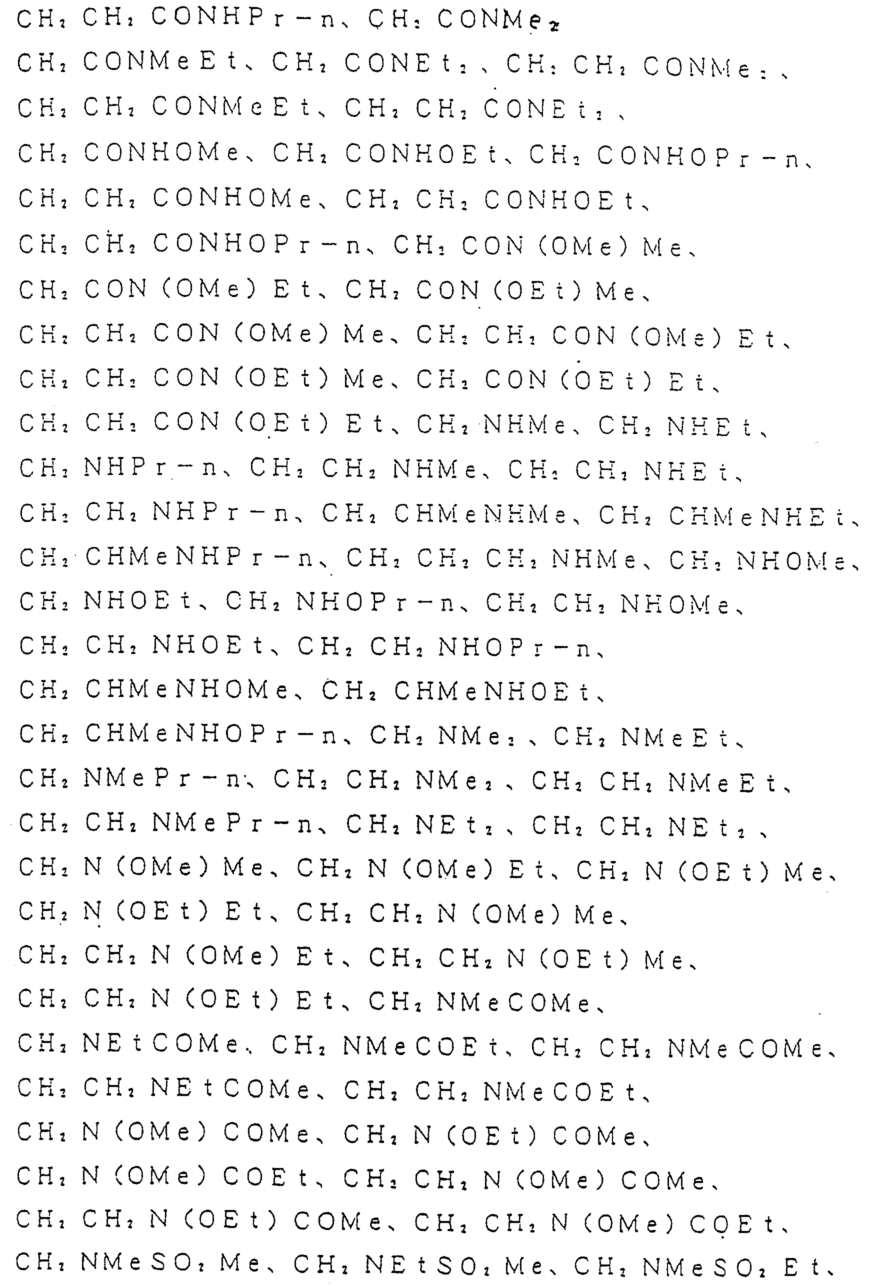

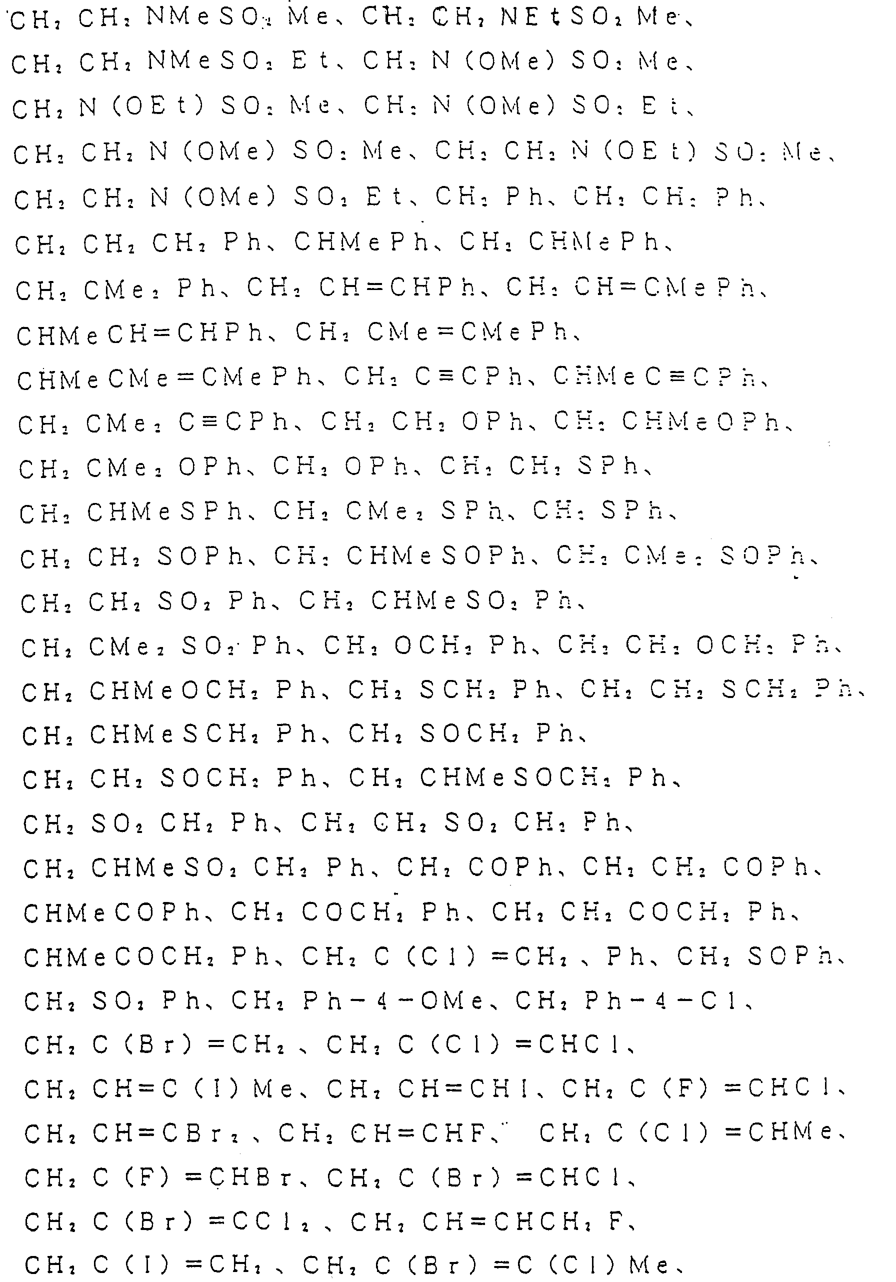

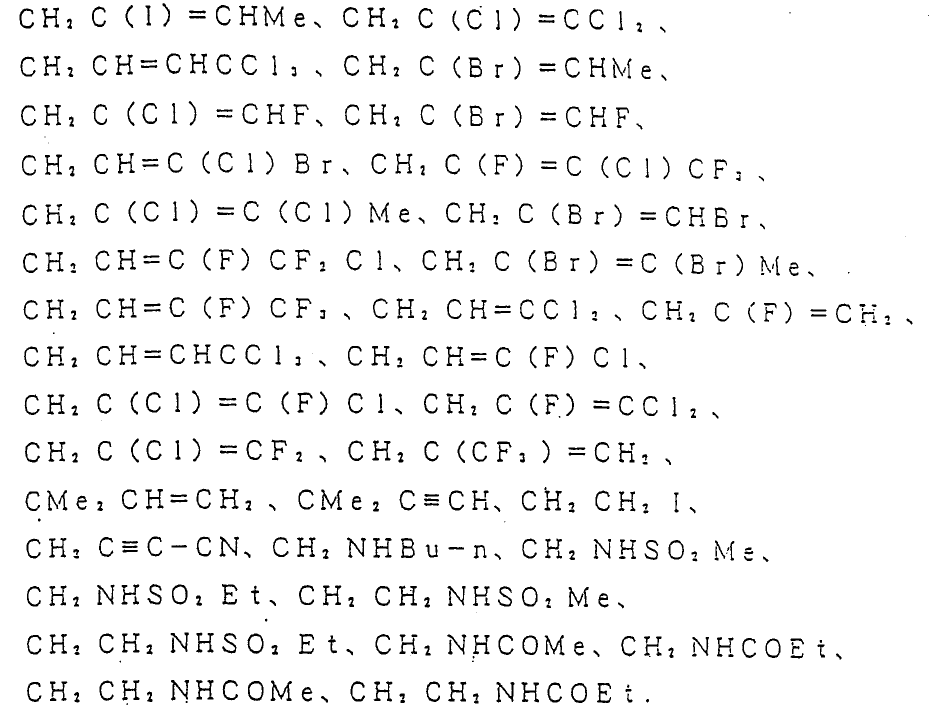

12. Procédé de préparation d'un dérivé d'iminosulfonylurée ou de son sel, suivant la revendication 3, dans lequel :
- R^{a2} et R^{a3} représentent :

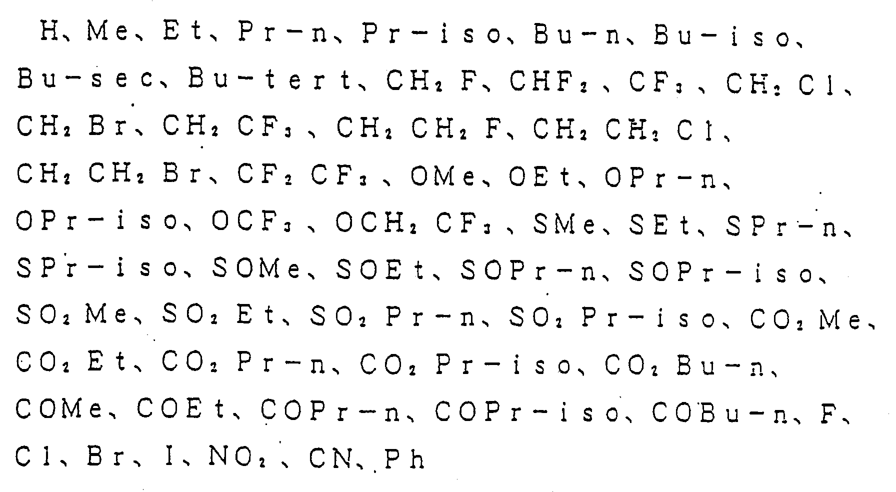

13. Procédé de préparation d'un dérivé d'iminosulfonylurée ou de son sel, selon la revendication 3, dans lequel B et D représentent :

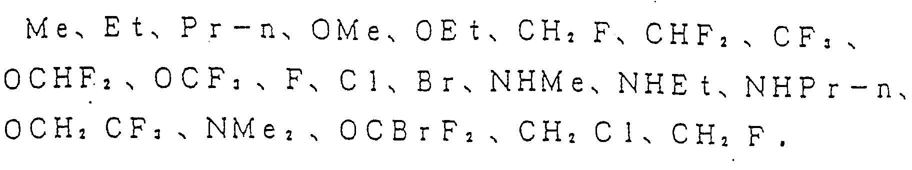

14. Procédé de préparation d'un herbicide qui contient un dérivé d'iminosulfonylurée tel que préparé selon l'une des revendications 1 à 13, ledit procédé comprenant la formulation dudit dérivé d'iminosulfonylurée conjointement avec un support approprié.

15. Procédé herbicide et de lutte contre la croissance à l'encontre des mauvaises herbes, qui comprend l'application d'une quantité efficace du point de vue herbicide d'un dérivé d'iminosulfonylurée tel que fabriqué à l'une des revendications 1 à 13.
